# EUROPEAN PATENT SPECIFICATION

(11) **EP 3 704 094 B1**
(45) Date of publication and mention of the grant of the patent: **11.03.2026**
(21) Application number: 18812422.6
(22) Date of filing: 02.11.2018
(51) Int. Cl.: C07D 213/73, C07D 317/64, C07D 231/56, C07C 311/13, C07D 401/04, C07D 237/14, C07D 237/16, C07D 405/08, C07D 241/18, C07D 471/04, C07D 261/08, C07D 261/20, C07C 235/22, C07D 271/113, C07D 213/30, C07D 213/66, A61K 31/44, A61P 35/00

(54) **MODULATORS OF THE INTEGRATED STRESS PATHWAY**
MODULATOREN EINES INTEGRIERTEN SPANNUNGSWEGS
MODULATEURS DE LA VOIE DE RÉPONSE INTÉGRÉE AU STRESS

(30) Priority: 02.11.2017 US 201762580726 P; 14.03.2018 US 201862643059 P
(43) Date of publication of application: 09.09.2020
(73) Proprietor: Calico Life Sciences LLC, South San Francisco, CA 94080 (US); AbbVie Inc., North Chicago, IL 60064 (US)
(72) Inventor: MARTIN, Kathleen Ann, San Francisco, CA 94118 (US); SIDRAUSKI, Carmela, Saratoga, CA 95070 (US); PLIUSHCHEV, Marina A., Vernon Hills, IL 60061 (US); FROST, Jennifer M., Gurnee, IL 60031 (US); TONG, Yunsong, Libertyville, IL 60048 (US); BLACK, Lawrence A., Lahaina, HI 96761 (US); XU, Xiangdong, Buffalo Grove, IL 60089 (US); SHI, Lei, Vernon Hills, IL 60061 (US); ZHANG, Qingwei, Libertyville, IL 60048 (US); CHUNG, Seungwon, Libertyville, IL 60048 (US); XIONG, Zhaoming, Buffalo Grove, IL 60089 (US); SWEIS, Ramzi Farah, Lake Bluff, IL 60044 (US); DART, Michael J., Highland Park, IL 60035 (US); BROWN, Brian S., Evanston, IL 60202 (US); MURAUSKI, Kathleen, Chicago, IL 60640 (US)
(74) Representative: Heller, Benjamin Henry
(86) International application number: PCT/US2018/058949
(87) International publication number: WO 2019/090069

(56) References cited:
- WO-A1-2012/088365
- WO-A1-2017/193034
- WO-A1-2017/193063
- CN-A- 106 349 130
- GB-A- 1 026 506
- US-A- 3 264 351
- US-A1- 2016 096 800
- US-A1- 2016 318 856
- PUBCHEM COMPOUND,, 30 November 2012 (2012-11-30), XP002770924
- PUBCHEM COMPOUND,, 23 February 2016 (2016-02-23), XP002770925
- PUBCHEM COMPOUND,, 23 March 2015 (2015-03-23), XP002770920

## Description

### CROSS-REFERENCE TO RELATED APPLICATIONS

This application claims priority to U.S. Provisional Application No. 62/580,726, filed November 2, 2017 and U.S. Provisional Application No. 62/643,059, filed March 14, 2018.

### BACKGROUND

In metazoa, diverse stress signals converge at a single phosphorylation event at serine 51 of a common effector, the translation initiation factor eIF2α. This step is carried out by four eIF2α kinases in mammalian cells: PERK, which responds to an accumulation of unfolded proteins in the endoplasmic reticulum (ER), GCN2 to amino acid starvation and UV light, PKR to viral infection and metabolic stress, and HRI to heme deficiency. This collection of signaling pathways has been termed the "integrated stress response" (ISR), as they converge on the same molecular event. eIF2α phosphorylation results in an attenuation of translation with consequences that allow cells to cope with the varied stresses (Wek, R.C. et al, Biochem Soc Trans (2006) 34(Pt 1):7-11).

eIF2 (which is comprised of three subunits, α, β and γ) binds GTP and the initiator Met-tRNA to form the ternary complex (eIF2-GTP-Met-tRNAᵢ), which, in turn, associates with the 40S ribosomal subunit scanning the 5'UTR of mRNAs to select the initiating AUG codon. Upon phosphorylation of its α-subunit, eIF2 becomes a competitive inhibitor of its GTP-exchange factor (GEF), eIF2B (Hinnebusch, A.G. and Lorsch, J.R. Cold Spring Harbor Perspect Biol (2012) 4(10)). The tight and nonproductive binding of phosphorylated eIF2 to eIF2B prevents loading of the eIF2 complex with GTP, thus blocking ternary complex formation and reducing translation initiation (Krishnamoorthy, T. et al, Mol Cell Biol (2001) 21(15):5018-5030). Because eIF2B is less abundant than eIF2, phosphorylation of only a small fraction of the total eIF2 has a dramatic impact on eIF2B activity in cells.

eIF2B is a complex molecular machine, composed of five different subunits, eIF2B1 through eIF2B5. eIF2B5 catalyzes the GDP/GTP exchange reaction and, together with a partially homologous subunit eIF2B3, constitutes the "catalytic core" (Williams, D.D. et al, J Biol Chem (2001) 276:24697-24703). The three remaining subunits (eIF2B1, eIF2B2, and eIF2B4) are also highly homologous to one another and form a "regulatory sub-complex" that provides binding sites for eIF2B's substrate eIF2 (Dev, K. et al, Mol Cell Biol (2010) 30:5218-5233). The exchange of GDP with GTP in eIF2 is catalyzed by its dedicated guanine nucleotide exchange factor (GEF) eIF2B. eIF2B exists as a decamer (B1₂ B2₂ B3₂ B4₂ B5₂) or dimer of two pentamers in cells (Gordiyenko, Y. et al, Nat Commun (2014) 5:3902; Wortham, N.C. et al, FASEB J (2014) 28:2225-2237). Molecules such as ISRIB interact with and stabilize the eIF2B dimer conformation, thereby enhancing intrinsic GEF activity and making cells less sensitive to the cellular effects of phosphorylation of eIF2□ (Sidrauski, C. et al, eLife (2015) e07314; Sekine, Y. et al, Science (2015) 348:1027-1030). As such, small molecule therapeutics that can modulate eIF2B activity may have the potential to attenuate the PERK branch of the UPR and the overall ISR, and therefore may be used in the prevention and/or treatment of various diseases, such as a neurodegenerative disease, a leukodystrophy, cancer, an inflammatory disease, a musculoskeletal disease, or a metabolic disease.

### SUMMARY OF THE INVENTION

The present invention features compounds suitable for the modulation of eIF2B (e.g., activation of eIF2B) and the attenuation of the ISR signaling pathway. The compounds of the invention are specified in the appended claims. Embodiments not encompassed by the claims are provided for reference purposes. The references to methods of treatment in the subsequent paragraphs of this description are to be interpreted as references to the compounds, pharmaceutical compositions and medicaments for use in a method for treatment of the human (or animal) body by therapy.

In some embodiments, the present description features an eIF2B modulator (e.g., an eIF2B activator) comprising a compound of Formula (I) or Formula (II), or a pharmaceutically acceptable salt, solvate, hydrate, tautomer, ester, N-oxide or stereoisomer thereof. In other embodiments, the present invention features methods of using a compound of Formula (I) or Formula (II), or a pharmaceutically acceptable salt, solvate, hydrate, tautomer, ester, N-oxide or stereoisomer thereof for the treatment of a disease or disorder, e.g., a neurodegenerative disease, a leukodystrophy, cancer, an inflammatory disease, a musculoskeletal disease, a metabolic disease, or a disease or disorder associated with impaired function of eIF2B or components in the ISR pathway (e.g., eIF2 pathway).

In one aspect, the present description features a compound of Formula (I): 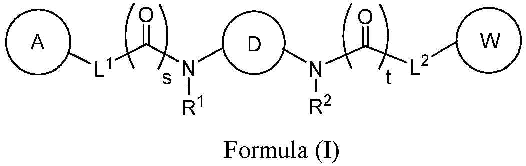 or a pharmaceutically acceptable salt, solvate, hydrate, tautomer, ester, N-oxide or stereoisomer thereof, wherein D is a bridged bicyclic cycloalkyl, bridged bicyclic heterocyclyl, bridged bicyclic cycloalkenyl, or cubanyl, wherein each bridged bicyclic cycloalkyl, bridged bicyclic heterocyclyl, bridged bicyclic cycloalkenyl, or cubanyl is optionally substituted with 1-4 R^{X} groups; and wherein if the bridged bicyclic heterocyclyl contains a substitutable nitrogen moiety, the substitutable nitrogen moiety may be optionally substituted by R^{N1}; L¹ and L² are each independently C₁-C₆ alkylene, C₂-C₆ alkenylene, 2-7-membered heteroalkylene, O, or NR^{C}, wherein each C₁-C₆ alkylene, C₂-C₆ alkenylene, or 2-7-membered heteroalkylene is optionally substituted with 1-5 R^{L}; R¹ and R² are each independently hydrogen, C₁-C₆ alkyl, C₁-C₆ alkoxy-C₂-C₆ alkyl, hydroxy-C₂-C₆ alkyl, silyloxy-C₂-C₆ alkyl, G¹-O-C₂-C₆ alkyl, HO₂C-C₁-C₆ alkyl, or C₁-C₆ alkyl-C(O)₂-C₁-C₆ alkyl; each R^{L} is independently selected from the group consisting of C₁-C₆ alkyl, hydroxy-C₁-C₆ alkyl, hydroxy-C₁-C₆ alkoxy, halo-C₁-C₆ alkyl, amino-C₁-C₆ alkyl, cyano-C₁-C₆ alkyl, C₁-C₆ alkoxy-C₁-C₆ alkyl, HO₂C-C₁-C₆ alkyl, C₁-C₆ alkyl-C(O)₂-C₁-C₆ alkyl, oxo, halo, cyano, -OR^{A}, -NR^{B}R^{C}, -NR^{B}R^{CC}, -NR^{B}C(O)R^{D}, -C(O)NR^{B}R^{C}, -C(O)R^{D}, - C(O)OH, -C(O)OR^{D}, -SR^{E}, -S(O)R^{D}, -S(O)₂R^{D}, -OS(O)R^{D}, -OS(O)₂R^{D}, and G²; or 2 geminal R^{L} groups together with the carbon to which they are attached form a cyclopropyl moiety; R^{N1} is selected from the group consisting of hydrogen, C₁-C₆ alkyl, hydroxy-C₂-C₆ alkyl, halo-C₂-C₆ alkyl, amino-C₂-C₆ alkyl, cyano-C₂-C₆ alkyl, C₁-C₆ alkoxy-C₁-C₆ alkyl, HO₂C-C₁-C₆ alkyl, C₁-C₆ alkyl-C(O)₂-C₁-C₆ alkyl, phenoxy-C₁-C₆ alkyl (wherein phenoxy is optionally substituted with 1-3 halogens), -C(O)NR^{B}R^{C}, -C(O)R^{D}, -C(O)OR^{D}, and -S(O)₂R^{D}; A and W are each independently aryl or 5-6-membered heteroaryl, wherein each phenyl or 5-6-membered heteroaryl is optionally substituted with 1-5 R^{Y}; each R^{X} is independently selected from the group consisting of C₁-C₆ alkyl, hydroxy-C₁-C₆ alkyl, halo-C₁-C₆ alkyl, amino-C₁-C₆ alkyl, cyano-C₁-C₆ alkyl, C₁-C₆ alkoxy-C₁-C₆ alkyl, C₁-C₆ alkyl-C₁-C₆ alkoxy, C₁-C₆ alkoxy-C₁-C₆ alkoxy, oxo, halo, cyano, -OR^{A}, -NR^{B}R^{C}, -NR^{B}R^{CC}, -NR^{B}C(O)R^{D}, -C(O)NR^{B}R^{C}, -C(O)R^{D}, - C(O)OH, -C(O)OR^{D}, =CHC(O)OR^{D}, =CHC(O)OH, -SR^{E}, -S(O)R^{D}, -S(O)₂R^{D}, -OS(O)R^{D}, - OS(O)₂R^{D}, and G²; or 2 geminal R^{X} groups together with the carbon to which they are attached form an oxirane moiety; each R^{Y} is independently selected from the group consisting of hydrogen, C₁-C₆ alkyl, O-C₃-C₆ cycloalkyl, C₁-C₆ alkoxy-C₁-C₆ alkyl, hydroxy-C₁-C₆ alkyl, hydroxy-C₁-C₆ alkoxy, halo-C₁-C₆ alkyl, halo-C₁-C₆ alkoxy, amino-C₁-C₆ alkyl, cyano-C₁-C₆ alkyl, oxo, halo, cyano, -OR^{A}, -NR^{B}R^{C}, -NR^{B}R^{CC}, -NR^{B}C(O)R^{D}, -C(O)NR^{B}R^{C}, -C(O)R^{D}, - C(O)OH, -C(O)OR^{D}, -S(R^{F})ₘ, -S(O)R^{D}, -S(O)₂R^{D}, and G¹; or 2 R^{Y} groups on adjacent atoms, together with the atoms to which they are attached form a 3-7-membered fused cycloalkyl, heterocyclyl, aryl, or heteroaryl ring optionally substituted with 1-5 R^{X}; each G¹ and G² is independently C₃-C₆ cycloalkyl, 4-7-membered heterocyclyl, aryl, or 5-6-membered heteroaryl, wherein each C₃-C₆ cycloalkyl, 4-7-membered heterocyclyl, aryl, or 5-6-membered heteroaryl is optionally substituted with 1-3 R^{Z}; each R^{Z} is independently selected from the group consisting of C₁-C₆ alkyl, hydroxy-C₁-C₆ alkyl, halo-C₁-C₆ alkyl, halo, cyano, -OR^{A}, -NR^{B}R^{C}, - NR^{B}C(O)R^{D}, -C(O)NR^{B}R^{C}, -C(O)R^{D}, -C(O)OH, -C(O)OR^{D}, and -S(O)₂R^{D}; each R^{A} is independently hydrogen, C₁-C₆ alkyl, halo-C₁-C₆ alkyl, -C(O)NR^{B}R^{C}, -C(O)R^{D}, or -C(O)OR^{D}; each of R^{B} and R^{C} is independently hydrogen, C₁-C₆ alkyl, or halo-C₁-C₆ alkyl; R^{B} and R^{C} together with the atom to which they are attached form a 3-7-membered heterocyclyl ring optionally substituted with 1-3 R^{Z}; each R^{CC} is independently selected from the group consisting of hydroxy-C₁-C₆ alkyl, halo-C₁-C₆ alkyl, HO₂C-C₁-C₆ alkyl, C₁-C₆ alkyl-C(O)₂-C₁-C₆ alkyl, (CO)-C₁-C₆alkyl-OH, (CO)-C₁-C₆alkyl-C₁-C₆alkoxy and 4-6 membered heterocyclyl; wherein the heterocyclyl may optionally be substituted with 1-3 R^{Z}: each R^{D} is independently C₁-C₆ alkyl, 2-7-membered heteroalkyl, hydroxy-C₁-C₆ alkyl, or halo-C₁-C₆ alkyl, wherein each C₁-C₆ alkyl, 2-7-membered heteroalkyl, hydroxy-C₁-C₆ alkyl, or halo-C₁-C₆ alkyl is optionally substituted with 1-5 R^{G}; each R^{E} is independently hydrogen, C₁-C₆ alkyl, or halo-C₁-C₆ alkyl; each R^{F} is independently hydrogen, C₁-C₆ alkyl, or halo; each R^{G} is independently aryl or 5-6 membered heteroaryl, wherein each aryl or 5-6 membered heteroaryl is optionally substituted with 1-5 R^{H}; each R^{H} is independently C₁-C₆ alkyl or halo-C₁-C₆ alkyl; m is 1 when R^{F} is hydrogen or C₁-C₆ alkyl, 3 when R^{F} is C₁-C₆ alkyl, or 5 when R^{F} is halo; t is 0 or 1; and s is 0 or 1.

In some embodiments, D is a bridged bicyclic cycloalkyl, a bridged bicyclic heterocyclyl, or cubanyl, each of which is optionally substituted with 1-4 R^{X} groups. In some embodiments, D is a bridged 5-8 membered bicyclic cycloalkyl or heterocyclyl, or cubanyl, each of which is optionally substituted with 1-4 R^{X} groups. In some embodiments, D is selected from cubane, bicyclo[1.1.1]pentane, bicyclo[2.2.2]octane, bicyclo[2.2.2]oct-2-ene, bicyclo[2.1.1]hexane, bicyclo[3.1.1]heptane, bicyclo[2.2.1]heptane, bicyclo[3.2.1]octane, or 2-azabicyclo[2.2.2]octane, each of which is optionally substituted with 1-4 R^{X} groups. In some embodiments, D is selected from cubane, bicyclo[1.1.1]pentane, bicyclo[2.2.2]octane, bicyclo[2.1.1]hexane, or bicyclo[3.1.1]heptane, each of which is optionally substituted with 1-4 R^{X} groups. In some embodiments, D is selected from: 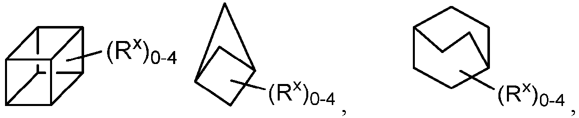 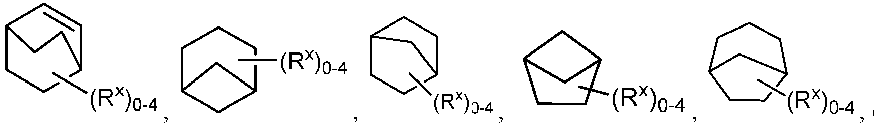 or 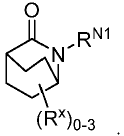 In some embodiments, D is selected from: 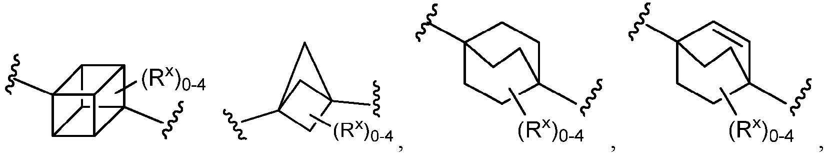 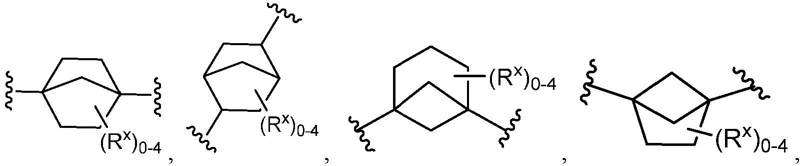 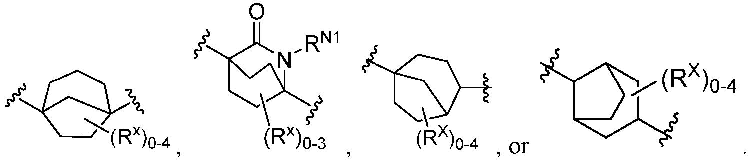 In some embodiments, D is substituted with 1 or 2 R^{X}. In some embodiments, R^{X} is independently C₁-C₆ alkyl, hydroxy-C₁-C₆ alkyl, C₁-C₆ alkoxy-C₁-C₆ alkyl, C₁-C₆ alkoxy-C₁-C₆ alkoxy, oxo, halo, cyano, -OR^{A}, -OS(O)₂R^{D}, -S(O)₂R^{D}, -SR^{E}, NR^{B}C(O)R^{D}, -C(O)NR^{B}R^{C}, -C(O)R^{D}, -C(O)OH, - C(O)OR^{D}, =CHC(O)OH, =CHC(O)OR^{D}, NR^{B}R^{C}, NR^{B}R^{CC}, or G²; or 2 geminal R^{X} groups together with the carbon to which they are attached form an oxirane moiety. In some embodiments, R^{X} is independently CH₃, -CH₂OH, -CH₂OCH₃, -CH₂CH₂CH₃, -C(CH₃)₂OH, -O-CH₂-O-CH₃, CH₂CH₂OH, oxo, fluoro, bromo, OH, cyano, OCH₃, NH₂, N(H)CH₂CF₃, N(H)CH₂CH₂OH, N(CH₃)₂, N(CH₃)CH₂CH₂OH, N(CH₃)CH₂CO₂H, N(CH₃)CH₂CH₂CO₂H, NHC(O)CH₃, OC(O)CH₃, C(O)NH₂, OS(O)₂CH₃, -S(O)₂CH₃, -S(O)₂ CH₂CH₃, C(O)OH, C(O)OCH₃, OC(O)R^{D}, -C(O)CH₃, =CHC(O)OH, =CHC(O)OCH₂CH₃, -SCH₃, 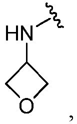 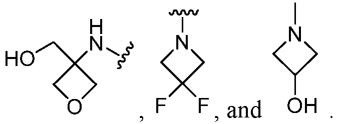 In some embodiments, G² is aryl or 5-6 membered heteroaryl. In some embodiments, G² is oxadiazolyl or tetrazolyl.

In some embodiments, D is substituted with 0 R^{X}. In some embodiments, D is

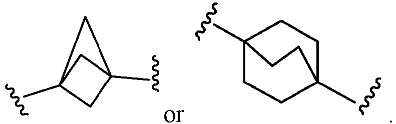

In some embodiments, at least one of L¹ and L² is independently 2-7-membered heteroalkylene optionally substituted by 1-5 R^{L}. In some embodiments, one of L¹ and L² is independently C₁-C₆ alkylene or C₂-C₆ alkenylene and the other of L¹ and L² is independently 2-7-membered heteroalkylene, and wherein each C₁-C₆ alkylene, C₂-C₆ alkenylene, and 2-7-membered heteroalkylene is optionally substituted by 1-5 R^{L}.

In some embodiments, each R^{L} is independently C₁-C₆ alkyl, OH, oxo, -C(O)R^{D}, cyano, HO₂C-C₁-C₆ alkyl, C₁-C₆ alkyl-C(O)₂-C₁-C₆ alkyl, -C(O)OH, -NR^{B}R^{C}, -NR^{B}R^{CC}, - NR^{B}C(O)R^{D}, hydroxy-C₁-C₆ alkyl, or hydroxy-C₁-C₆ alkoxy. each R^{L} is independently CH₃, oxo, or C(O)CH₃.

In some embodiments, each of L¹ and L² is independently selected from CH₂O-*, CH₂CH₂-*, CH₂CH₂CH₂-*, CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, NHCH₂-*, CH₂NH-*, CH₂N(CH₃)-*, CH₂N(CH₃)C(O)-*, CH₂N(C(O)CH₃)-*, CH₂CH(OH)-*, CH(OH)-*, CH(OH)CH₂CH₂-*, CH₂CH(OH)-*, CH₂NHC(O)-*, NHC(O)OCH₂-*, O-*, NH-*, S(O)₂CH-*, S(O)₂CH₂CH₂-*, S(O)₂CH₂CH₂O-*, CH₂C(O)-*, O-CH₂CH₂*, CH₂N(CH₂CO₂H)-*, CH(CO₂H)CH₂CH₂O-*, CH₂N(CH₂CO₂C(CH₃)₃)-*, CH(CN)CH₂O-*, CH₂CH(NH(CH₃))-*,CH(OCH₂CH₂OH)-*, CH₂CH(NH(C(O)CH₂OH))-*, CH₂CH(NH₂)CH₂*,CH(CH₂CH₂OH)O-* or 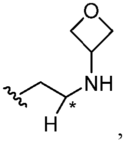 and "-*" indicates the attachment point to A and W, respectively. In some embodiments, L¹ is independently selected from CH₂O-*, CH₂CH₂O-* or CH=CH-*, L² is independently selected from CH₂O-*, CH₂CH₂-*, CH₂CH₂CH₂-*, CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, NHCH₂-*, CH₂NH-*, CH₂N(CH₃)-*, CH₂N(CH₃)C(O)-*, CH₂N(C(O)CH₃)-*, CH₂CH(OH)-*, CH(OH)-*, CH(OH)CH₂CH₂-*, CH₂CH(OH)-*, CH₂NHC(O)-*, NHC(O)OCH₂-*, O-*, NH-*, S(O)₂CH-*, S(O)₂CH₂CH₂-*, S(O)₂CH₂CH₂O-*, CH₂C(O)-*, CH₂N(CH₂CO₂H)-*, CH(CO₂H)CH₂CH₂O-*, CH₂N(CH₂CO₂C(CH₃)₃)-*, CH(CN)CH₂O-*, CH₂CH(NH(CH₃))-*,CH(OCH₂CH₂OH)-*, CH₂CH(NH(C(O)CH₂OH))-*, CH₂CH(NH₂)CH₂*,CH(CH₂CH₂OH)O-*, CH₂C(CH₃)₂O*- or 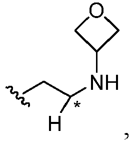 and "-*" indicates the attachment point to A and W, respectively.

In some embodiments, t is 1 and s is 1. In some embodiments, s is 1 and t is 0. In some embodiments, s is 1 and t is 0.

In some embodiments, R¹ and R² are each independently hydrogen, C₁-C₆ alkyl, hydroxyl-C₂-C₆ alkyl, or silyloxy-C₂-C₆ alkyl. one of R¹ and R² is independently hydrogen and the other of R¹ and R² is independently hydrogen, C₁-C₆ alkyl, C₂-C₆ hydroxyl-C₂-C₆ alkyl, silyloxy-C₂-C₆ alkyl, HO₂C-C₁-C₆ alkyl, or C₁-C₆ alkyl-C(O)₂-C₁-C₆ alkyl. wherein R¹ and R² are each independently hydrogen, *-CH₃, *-CH₂CH₂OH, *-CH₂CH₂OSi(CH₃)₂C(CH₃)₃, *-CH₂CO₂H, or *-CH₂C(O)₂C(CH₃), and "*-" indicates the attachment point to the nitrogen atom. one of R¹ and R² is independently hydrogen and the other of R¹ and R² is independently hydrogen, *-CH₃, *-CH₂CH₂OH, *-CH₂CH₂OSi(CH₃)₂C(CH₃)₃, *-CH₂CO₂H, or *-CH₂C(O)₂C(CH₃), and "*-" indicates the attachment point to the nitrogen atom. In some embodiments, R¹ and R² are each independently hydrogen.

In some embodiments, each A and W is independently phenyl or 5-6-membered heteroaryl optionally substituted with 1-5 R^{Y}. In some embodiments, each of A and W is independently phenyl, pyridyl, pyrazinyl, pyridazinyl, pyridazinonyl, triazinyl, pyrazolyl, pyrimidinyl, triazolyl, oxadiazolyl, oxadiazolonyl, thiazolyl, imidazolyl, pyrimidin-2(1H)-onyl, 1H-benzo[d]imidazolyl, pyrazolo[1,5-a]pyridine, 1H-indazolyl or isoxazolyl, each of which is optionally substituted with 1-5 R^{Y} groups. In some embodiments, each of A and W is independently selected from: 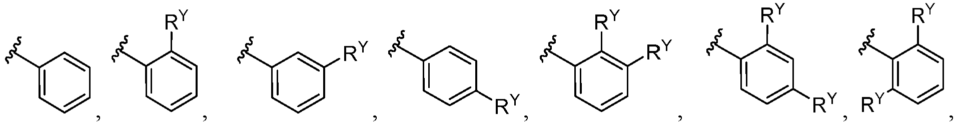 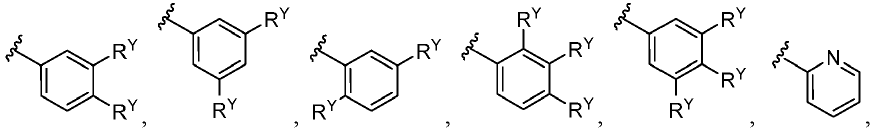 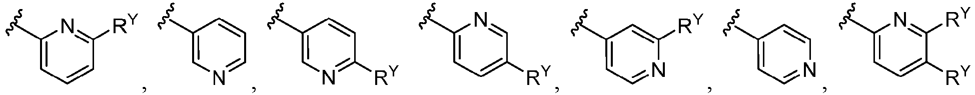 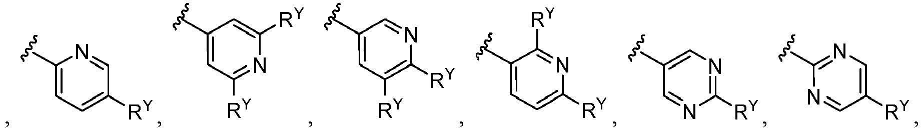 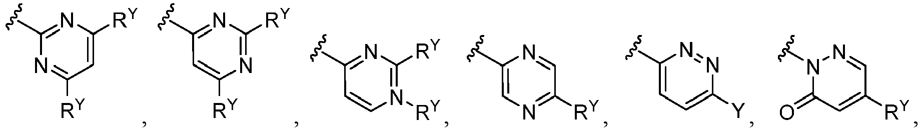 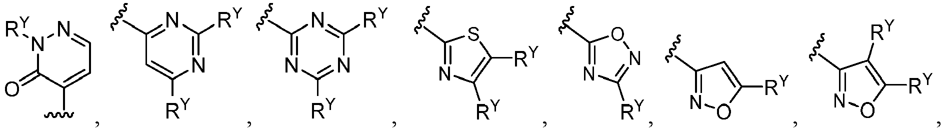 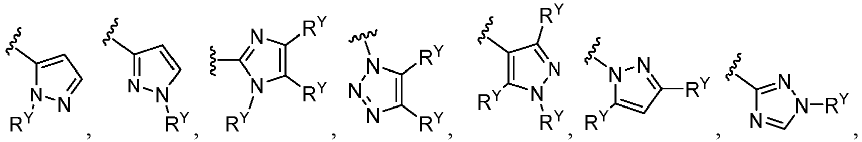 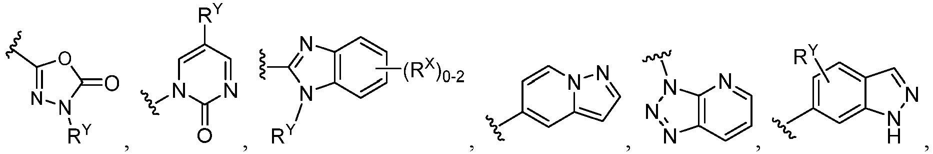 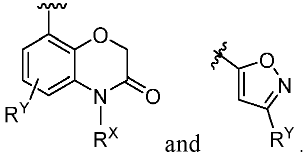 In some embodiments, A is phenyl and W is phenyl or 5-6-membered heteroaryl, each of A and W is optionally substituted with 1-5 R^{Y}, and each R^{Y} is independently C₁-C₆ alkyl, O-C₃-C₆ cycloalkyl, C₁-C₆ alkoxy-C₁-C₆ alkyl, hydroxy-C₁-C₆ alkyl, halo-C₁-C₆ alkyl, halo-C₁-C₆ alkoxy, hydroxy-C₁-C₆ alkoxy, amino-C₁-C₆ alkyl, cyano-C₁-C₆ alkyl, halo, cyano, -OR^{A}, -NR^{E}R^{C}, - NR^{B}R^{CC}, -NR^{B}C(O)R^{D}, -C(O)R^{D}, -C(O)OH, -C(O)OR^{D}, -S(R^{F})ₘ,-S(O)₂R^{D}, or G¹. In some embodiments, A is phenyl and W is phenyl, pyridyl, pyrazinyl, pyridazinyl, pyridazinonyl, pyrimidinyl, triazinyl, pyrazolyl, triazolyl, oxadiazolyl, oxadiazolonyl, thiazolyl, imidazolyl, or isoxazolyl, each of which is optionally substituted with 1-5 R^{Y} groups.

In some embodiments, A is selected from:

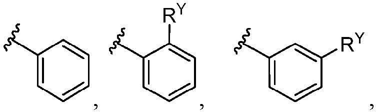

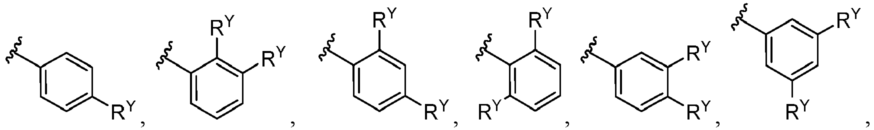

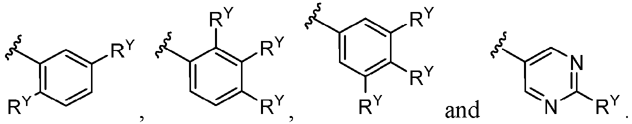

In some embodiments, W is selected from:

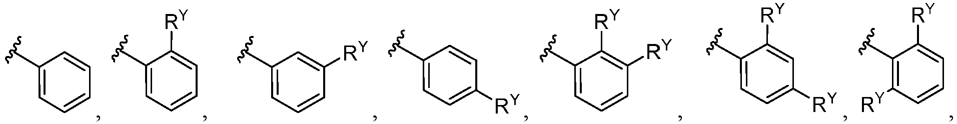

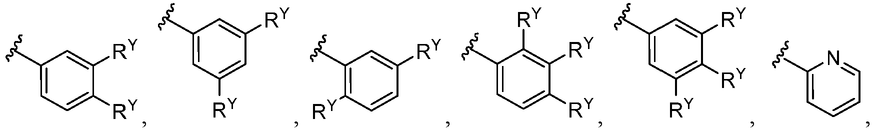

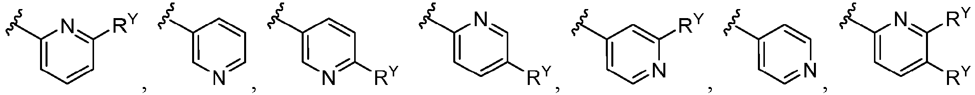

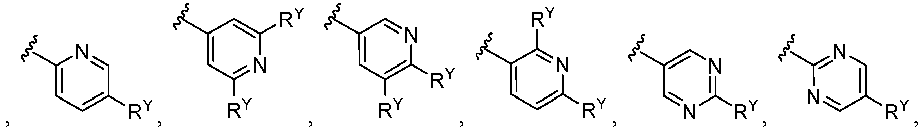

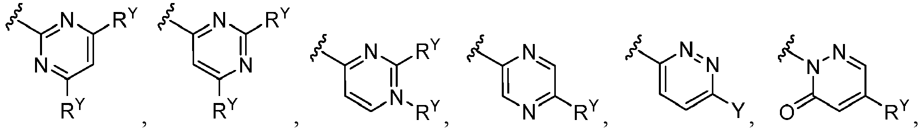

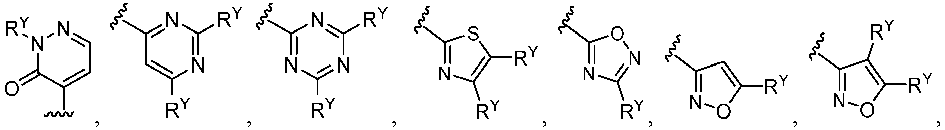

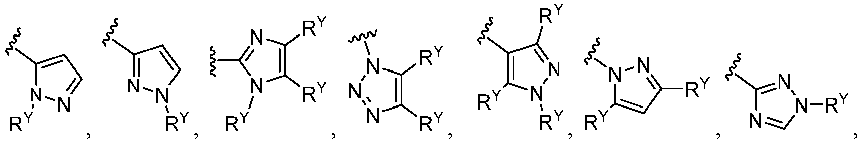

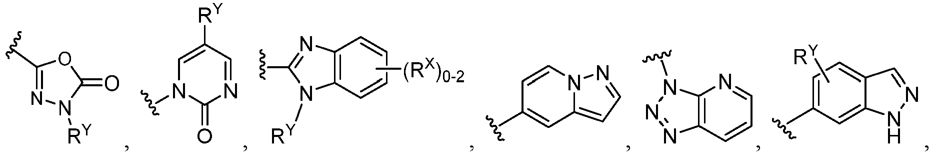

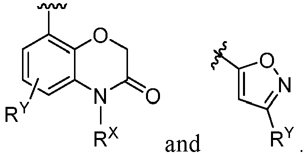

In some embodiments, each R^{Y} is independently hydrogen, bromo, chloro, fluoro, iodo, cyano, CF₃, CHF₂, CH₂CF₃, CH₃, CH₂CH₃, OH, CH₂OH, C(CH₃)₂OH, OCH₃, OCH₂CH₃, OCHF₂, OCF₃, OCH₂CF₃, OCH₂CH₂OH, CH₂OCH₃, S(O)₂CH₃, S(O)₂CH₂CH₂CH₃, CN, N(CH₃)₂, SF₅, SCH₃, NH₂, NH(C(O)CH₂OH), NH(CH₂CH₂OH), NH(CO)CH₂OCH₃, C(CH)₃, CH(CH₃)₂, CH₂CN, CH₂NH₂, CH(OH)CH₃, C(OH)(CH₃)CF₃, S(O)₂CH₃, C(O)CH₃, C(O)OCH₃, C(O)OH, OCHF₂, G¹, or

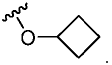

In some embodiments, each A and W is independently substituted with 2 R^{Y} on adjacent atoms, and the 2 R^{Y}, together with the atoms to which they are attached, form a 3-7-membered fused cycloalkyl, 3-7-membered fused heterocyclyl, fused aryl, or 5-6-membered fused heteroaryl ring optionally substituted with 1-5 R^{X}. In some embodiments, the 2 R^{Y} together with the atoms to which they are attached form a phenyl, pyridyl, pyrazolyl, pyrrolyl, isoxazolyl, thiophenyl, furanyl, dioxanyl, dioxolanyl, pyrrolidin-2-onyl, or morpholin-3-onyl ring, each of which is optionally substituted with 1-5 R^{X}. In some embodiments, each R^{X} is independently C₁-C₆ alkyl, OR^{A}, or halo. In some embodiments, each R^{X} is independently CH₃ OR^{A}, or fluoro.

In some embodiments, G¹ is cyclopropyl, isoxazolyl, piperidinyl, phenyl, or pyrazolyl, each of which is optionally substituted with 1-5 R^{Z}. In some embodiments, each R^{Z} is independently C₁-C₆ alkyl or halo.

In one aspect, the present description features a compound of Formula (I-b): 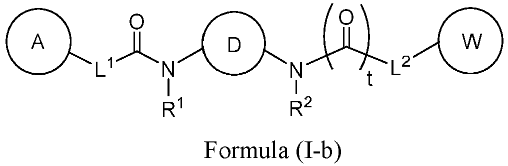 or a pharmaceutically acceptable salt, solvate, hydrate, tautomer, ester, N-oxide or stereoisomer thereof, wherein D is (1,2,3,4,6,7)-cubane, bicyclo[1.1.1]pentane, bicyclo[2.2.2]octane, bicyclo[2.1.1]hexane, bicyclo[2.2.1]heptane, bicyclo[3.1.1]heptane, bicyclo[3.2.1]octane, or 2-azabicyclo[2.2.2]octane, each of which is optionally substituted with 1-4 R^{X} groups; and wherein 2-azabicyclo[2.2.2]octane is substituted on nitrogen by hydrogen or CH₃; L¹ and L² are each independently CH₂O-*, CH₂CH₂-*, CH₂CH₂CH₂-*, CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, NHCH₂-*, CH₂NH-*, CH₂N(CH₃)-*, CH₂N(CH₃)C(O)-*, CH₂N(C(O)CH₃)-*, CH₂CH(OH)-*, CH(OH)-*, CH(OH)CH₂CH₂-*, CH₂CH(OH)-*, CH₂NHC(O)-*, NHC(O)OCH₂-*, O-*, NH-*, S(O)₂CH-*, S(O)₂CH₂CH₂-*, S(O)₂CH₂CH₂O-*, or CH₂C(O)-*, and "-*" indicates the attachment point to A and W, respectively; R¹ and R² are each independently hydrogen, CH₃, CH₂CH₂OH, or CH₂CH₂OSi(CH₃)₂C(CH₃)₃; A and W are each independently isoxazolyl, phenyl, pyridyl, pyrazinyl, pyridazinyl, pyridazinonyl, pyrimidinyl, triazinyl, thiazolyl, triazolyl, oxadiazolyl, or oxadiazolonyl, each of which is optionally substituted with 1-5 R^{Y} groups; each R^{X} is independently selected from CH₃, -CH₂OH, -C(CH₃)₂OH, oxo, fluoro, bromo, OH, cyano, OCH₃, NH₂, N(CH₃)₂, NHC(O)CH₃, OC(O)CH₃, C(O)NH₂, OS(O)₂CH₃, -S(O)₂CH₃, -S(O)₂ CH₂CH₃, C(O)OH, OC(O)R^{D}, -C(O)CH₃, -SCH₃, or G²; each R^{Y} is independently bromo, chloro, fluoro, iodo, CF₃, CHF₂, CH₂CF₃, CH₃, CH₂CH₃, OH, CH₂OH, C(CH₃)₂OH, OCH₃, OCH₂CH₃, OCF₃, S(O)₂CH₃, S(O)₂CH₂CH₂CH₃, CN, N(CH₃)₂, SF₅, SCH₃, NH₂, C(CH)₃, CH(CH₃)₂, CH₂CN, CH₂NH₂, CH(OH)CH₃, C(OH)(CH₃)CF₃, S(O)₂CH₃, C(O)CH₃, C(O)OCH₃, C(O)OH, OCHF₂ or G¹; or 2 R^{Y} groups on adjacent atoms, together with the atoms to which they are attached form a pyrazolyl, pyrrolyl, isoxazolyl, thiophenyl, furanyl, or dioxolanyl ring, each of which is optionally substituted with 1-2 R^{X}; G¹ and G² are cyclopropyl, isoxazolyl, phenyl, piperidinyl, oxadiazolyl, or tetrazolyl, or pyrazolyl, each of which is optionally substituted with 1-2 R^{Z}; each R^{D} is CH₂O optionally substituted with 1-5 R^{G}; each R^{G} is independently pyridyl optionally substituted with 1-5 R^{H}; each R^{H} is independently CF₃; each R^{Z} is independently CH₃; and t is 0 or 1.

In some embodiments, the compound of Formula (I) is a compound of Formula (I-c): 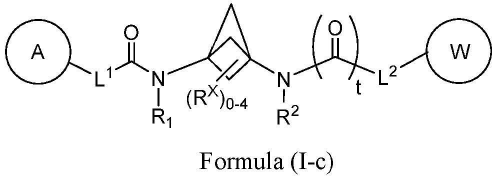 or a pharmaceutically acceptable salt, solvate, hydrate, tautomer, ester, N-oxide or stereoisomer thereof.

In some embodiments, the compound of Formula (I) is a compound of Formula (I-d): 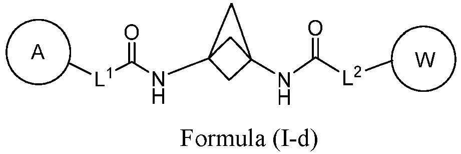 or a pharmaceutically acceptable salt, solvate, hydrate, tautomer, ester, N-oxide or stereoisomer thereof.

In some embodiments, the compound of Formula (I) is a compound of Formula (I-e): 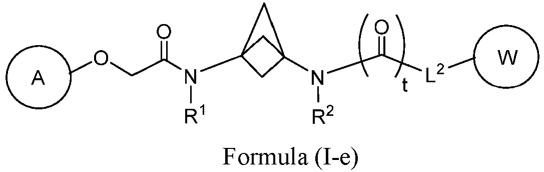 or a pharmaceutically acceptable salt, solvate, hydrate, tautomer, ester, N-oxide or stereoisomer thereof.

In some embodiments, the compound of Formula (I) is a compound of Formula (I-f): 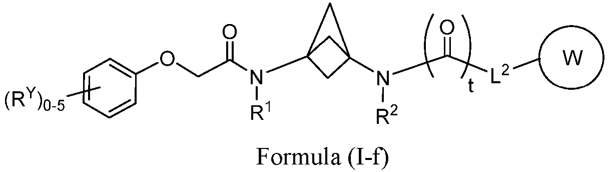 or a pharmaceutically acceptable salt, solvate, hydrate, tautomer, ester, N-oxide or stereoisomer thereof.

In some embodiments, the compound of Formula (I) is a compound of Formula (I-g): 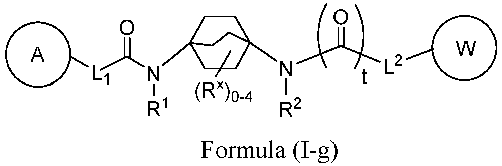 or a pharmaceutically acceptable salt, solvate, hydrate, tautomer, ester, N-oxide or stereoisomer thereof.

In some embodiments, the compound of Formula (I) is a compound of Formula (I-h): 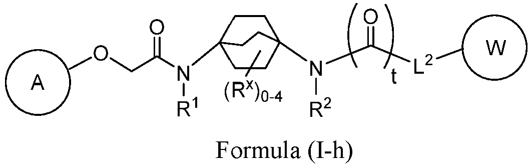 or a pharmaceutically acceptable salt, solvate, hydrate, tautomer, ester, N-oxide or stereoisomer thereof.

In some embodiments, the compound of Formula (I) is a compound of Formula (I-i): 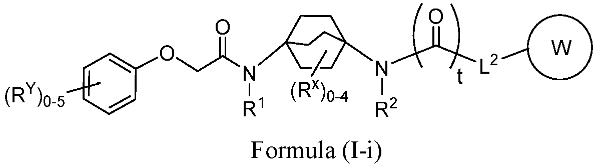 or a pharmaceutically acceptable salt, solvate, hydrate, tautomer, ester, N-oxide or stereoisomer thereof.

In some embodiments, the compound of Formula (I) is a compound of Formula (I-j): 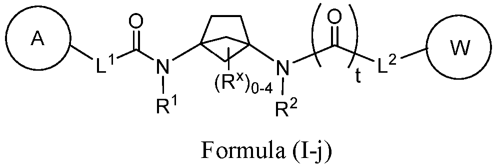 or a pharmaceutically acceptable salt, solvate, hydrate, tautomer, ester, N-oxide or stereoisomer thereof.

In some embodiments, the compound of Formula (I) is a compound of Formula (I-k): 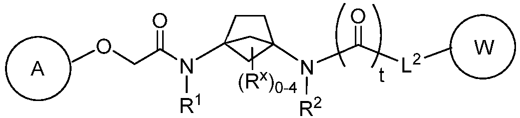 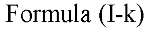 or a pharmaceutically acceptable salt, solvate, hydrate, tautomer, ester, N-oxide or stereoisomer thereof.

In some embodiments, the compound of Formula (I) is a compound of Formula (I-l): 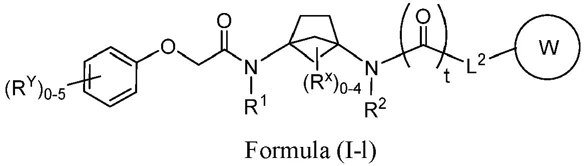 or a pharmaceutically acceptable salt, solvate, hydrate, tautomer, ester, N-oxide or stereoisomer thereof.

Also disclosed herein is a compound of Formula (II): 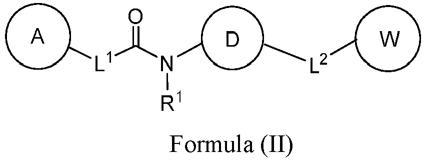 or a pharmaceutically acceptable salt, solvate, hydrate, tautomer, ester, N-oxide or stereoisomer thereof, wherein:
D is a bridged bicyclic cycloalkyl, bridged bicyclic heterocyclyl, or cubanyl, wherein each bridged bicyclic cycloalkyl, bridged bicyclic heterocyclyl, or cubanyl is optionally substituted with 1-4 R^{X} groups; and wherein if the bridged bicyclic heterocyclyl contains a substitutable nitrogen moiety, the substitutable nitrogen moiety may be optionally substituted by R^{N1};
L¹ is C₁-C₆ alkylene, C₂-C₆ alkenylene, 2-7-membered heteroalkylene, O, or NR^{C}, wherein C₁-C₆ alkylene, C₂-C₆ alkenylene, or 2-7-membered heteroalkylene is optionally substituted with 1-5 R^{X};
L² is -(C₀-C₂ alkylene)-O-(C₀-C₂ alkylene)-;
R¹ is hydrogen, C₁-C₆ alkyl, C₁-C₆ alkoxy-C₂-C₆ alkyl, hydroxy-C₂-C₆ alkyl, and silyloxy-C₂-C₆ alkyl;
R^{N1} is selected from the group consisting of hydrogen, C₁-C₆ alkyl, hydroxy-C₂-C₆ alkyl, halo-C₂-C₆ alkyl, amino-C₂-C₆ alkyl, cyano-C₂-C₆ alkyl, -C(O)NR^{B}R^{C}, -C(O)R^{D}, -C(O)OR^{D}, and -S(O)₂R^{D};
A and W are each independently phenyl or 5-6-membered heteroaryl, wherein each phenyl or 5-6-membered heteroaryl is optionally substituted with 1-5 R^{Y};
each R^{X} is independently selected from the group consisting of C₁-C₆ alkyl, hydroxy-C₁-C₆ alkyl, halo-C₁-C₆ alkyl, amino-C₁-C₆ alkyl, cyano-C₁-C₆ alkyl, C₁-C₆ alkoxy-C₁-C₆ alkyl, oxo, halo, cyano, -OR^{A}, -NR^{B}R^{C}, -NR^{B}C(O)R^{D}, -C(O)NR^{B}R^{C}, -C(O)R^{D}, -C(O)OH, - C(O)OR^{D}, -SR^{E}, -S(O)R^{D}, -S(O)₂R^{D}, -OS(O)R^{D}, -OS(O)₂R^{D}, and G²;
each R^{Y} is independently selected from the group consisting of hydrogen, C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, oxo, halo, cyano, -OR^{A}, -NR^{B}R^{C}, -NR^{B}C(O)R^{D}, -C(O)NR^{B}R^{C}, -C(O)R^{D}, -C(O)OH, - C(O)OR^{D}, -S(R^{F})ₘ, -S(O)R^{D}, -S(O)₂R^{D}, and G¹; or
2 R^{Y} groups on adjacent atoms, together with the atoms to which they are attached form a 3-7-membered fused cycloalkyl, heterocyclyl, aryl, or heteroaryl ring optionally substituted with 1-5 R^{X};
each G¹ and G² is independently C₃-C₆ cycloalkyl, 4-7-membered heterocyclyl, aryl, or 5-6-membered heteroaryl, wherein each C₃-C₆ cycloalkyl, 4-7-membered heterocyclyl, aryl, or 5-6-membered heteroaryl is optionally substituted with 1-3 R^{Z};
each R^{Z} is independently selected from the group consisting of C₁-C₆ alkyl, hydroxy-C₁-C₆ alkyl, halo-C₁-C₆ alkyl, halo, cyano, -OR^{A}, -NR^{B}R^{C}, -NR^{B}C(O)R^{D}, -C(O)NR^{B}R^{C}, -C(O)R^{D}, -C(O)OH, -C(O)OR^{D}, and -S(O)₂R^{D};
each R^{A} is independently hydrogen, C₁-C₆ alkyl, halo-C₁-C₆ alkyl, -C(O)NR^{B}R^{C}, - C(O)R^{D}, or -C(O)OR^{D};
each of R^{B} and R^{C} is independently hydrogen or C₁-C₆ alkyl; or
R^{B} and R^{C} together with the atom to which they are attached form a 3-7-membered heterocyclyl ring optionally substituted with 1-3 R^{Z};
each R^{D} is independently C₁-C₆ alkyl, 2-7-membered heteroalkyl, or halo-C₁-C₆ alkyl, wherein each C₁-C₆ alkyl, 2-7-membered heteroalkyl, or halo-C₁-C₆ alkyl is optionally substituted with 1-5 R^{G};
each R^{E} is independently hydrogen, C₁-C₆ alkyl, or halo-C₁-C₆ alkyl;
each R^{F} is independently hydrogen, C₁-C₆ alkyl, or halo;
each R^{G} is independently aryl or 5-6 membered heteroaryl, wherein each aryl or 5-6 membered heteroaryl is optionally substituted with 1-5 R^{H};
each R^{H} is independently C₁-C₆ alkyl or halo-C₁-C₆ alkyl;
m is 1 when R^{F} is hydrogen or C₁-C₆ alkyl, 3 when R^{F} is C₁-C₆ alkyl, or 5 when R^{F} is halo; and; and
t is 0 or 1.

In some embodiments D is selected from the group consisting of 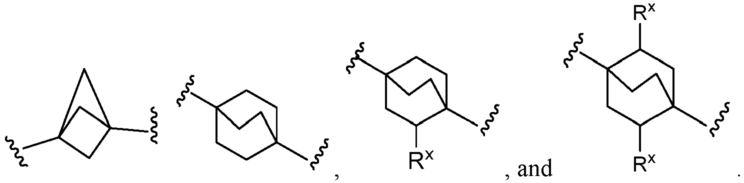 In some embodiments, each R^{X} is independently selected from the group consisting of oxo, -OR^{A} (*e.g.,* OH or OCH₃), -C(O)OH, -C(O)OR^{D} (*e.g.,* -C(O)OCH₃), halo, and hydroxy-C₁-C₆ alkyl.

In some embodiments, L¹ is CH₂O-* or CH₂OCH₂-*; wherein "-*" indicates the attachment point to A. In some embodiments, L² is selected from the group consisting of O-*, OCH₂-*, CH₂O-*, OCH₂CH₂-*, CH₂OCH₂-*, and CH₂CH₂O-*; wherein "-*" indicates the attachment point to W. In some embodiments, R¹ is hydrogen or CH₃.

In some embodiments, A is selected from the group consisting of:

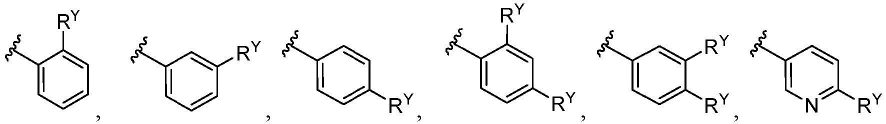

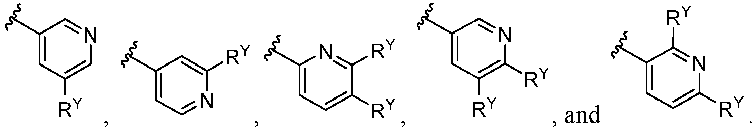

In some embodiments, W is selected from the group consisting of: 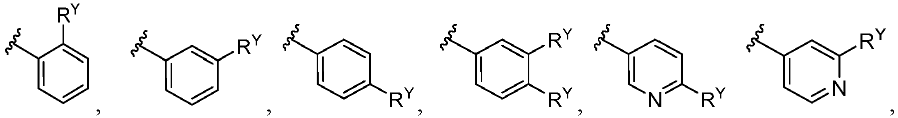 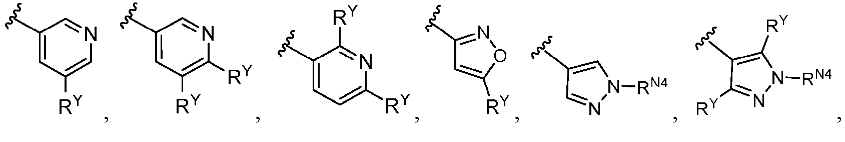 and 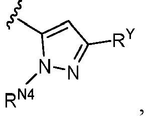 wherein R^{N4} is hydrogen or CH₃.

In some embodiments, wherein each R^{Y} is independently hydrogen, chloro, fluoro, CF₃, CHF₂, CH₃, CH₂CH₃, CH(CH₃)₂, OCH₃, OCF₃, OCH(CH₃)₂, or CN.

In some embodiments, the compound of Formula (II) is a compound of Formula (II-a): 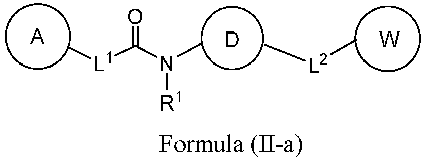 or a pharmaceutically acceptable salt, solvate, hydrate, tautomer, ester, N-oxide or stereoisomer thereof, wherein:
D is bicyclo[1.1.1]pentanyl or bicyclo[2.2.2]octanyl, each of which is optionally substituted with 1-4 R^{X} groups;
L¹ is CH₂O-* or CH₂OCH₂-*, wherein "-*" indicates the attachment point to A;
L² is selected from the group consisting of O-*, OCH₂-*, CH₂O-*, OCH₂CH₂-*, CH₂OCH₂-*, and CH₂CH₂O-*; wherein "-*" indicates the attachment point to W;
A is phenyl or pyridyl, each of which is optionally substituted with 1-5 R^{Y} groups;
W is phenyl, pyridyl, isoxazolyl, or pyrazolyl, each of which is optionally substituted on one or more available carbons with 1-5 R^{Y} groups; and wherein pyrazolyl may be optionally substituted on an available nitrogen with hydrogen or CH₃;
each R^{X} is independently fluoro, oxo, OH, OCH₃, C(O)OH, or C(O)OCH₃;
each R^{Y} is independently chloro, fluoro, CF₃, CH₃, CH₂CH₃, CH(CH₃)₂, OCH₃, OCH(CH₃)₂, or CN; or
2 R^{Y} groups on adjacent atoms, together with the atoms to which they are attached form a furanyl, pyrrolyl, or dioxolanyl ring, each of which is optionally substituted with 1-2 R^{X}; and
R¹ is hydrogen.

In some embodiments, the compound of Formula (I) or (II) is selected from a compound set forth in Table 1 or a pharmaceutically acceptable salt, solvate, hydrate, tautomer, ester, N-oxide or stereoisomer thereof.

In some embodiments, the compound of Formula (I) or (II) or a pharmaceutically acceptable salt, solvate, hydrate, tautomer, ester, N-oxide or stereoisomer thereof is formulated as a pharmaceutically acceptable composition comprising a disclosed compound and a pharmaceutically acceptable carrier.

In another aspect, the present description features a compound of Formula (I) or Formula (II), or a pharmaceutically acceptable salt, solvate, hydrate, tautomer, ester, N-oxide or stereoisomer thereof, or a composition thereof, for use in a method of treating a neurodegenerative disease, a leukodystrophy, a cancer, an inflammatory disease, an autoimmune disease, a viral infection, a skin disease, a fibrotic disease, a hemoglobin disease, a kidney disease, a hearing loss condition, an ocular disease, a musculoskeletal disease, a metabolic disease, or a mitochondrial disease, or a disease or disorder associated with impaired function of eIF2B or components in the ISR pathway (e.g., eIF2 pathway) in a subject.

In some embodiments, the method comprises the treatment of a neurodegenerative disease. In some embodiments, the neurodegenerative disease comprises a leukodystrophy, a leukoencephalopathy, a hypomyelinating or demyelinating disease, an intellectual disability syndrome, a cognitive impairment, a glial cell dysfunction, or a brain injury (e.g., a traumatic brain injury or toxin induced brain injury). In some embodiments, the neurodegenerative disease comprises vanishing white matter disease, childhood ataxia with CNS hypo myelination, Alzheimer's disease, amyotrophic lateral sclerosis, Creutzfeldt-Jakob disease, frontotemporal dementia, Gerstmann-Straussler-Scheinker disease, Huntington's disease, dementia (e.g., HIV-associated dementia or Lewy body dementia), kuru, multiple sclerosis, Parkinson's disease, or a prion disease.

In some embodiments, the method comprises the treatment of cancer. In some embodiments, the cancer comprises pancreatic cancer, breast cancer, multiple myeloma, or a cancer of the secretory cells.

In some embodiments, the method comprises the treatment of an inflammatory disease. In some embodiments, the inflammatory disease comprises postoperative cognitive dysfunction, arthritis (e.g., rheumatoid arthritis, psoriatic arthritis, or juvenile idiopathic arthritis), systemic lupus erythematosus (SLE), myasthenia gravis, diabetes (e.g., juvenile onset diabetes or diabetes mellitus type 1), Guillain-Barre syndrome, Hashimoto's encephalitis, Hashimoto's thyroiditis, ankylosing spondylitis, psoriasis, Sjogren's syndrome, vasculitis, glomerulonephritis, autoimmune thyroiditis, Behcet's disease, Crohn's disease, ulcerative colitis, bullous pemphigoid, sarcoidosis, ichthyosis, Graves' ophthalmopathy, inflammatory bowel disease, Addison's disease, vitiligo, asthma (e.g., allergic asthma), acne vulgaris, celiac disease, chronic prostatitis, pelvic inflammatory disease, reperfusion injury, sarcoidosis, transplant rejection, interstitial cystitis, atherosclerosis, or atopic dermatitis.

In some embodiments, the method comprises the treatment of a musculoskeletal disease. In some embodiments, the musculoskeletal disease comprises muscular dystrophy (e.g., Duchenne muscular dystrophy, Becker muscular dystrophy, distal muscular dystrophy, congenital muscular dystrophy, Emery-Dreifuss muscular dystrophy, facioscapulohumeral muscular dystrophy, or myotonic muscular dystrophy), multiple sclerosis, amyotropic lateral sclerosis, primary lateral sclerosis, progressive muscular atrophy, progressive bulbar palsy, pseudobulbar palsy, spinal muscular atrophy, progressive spinobulbar muscular atrophy, spinal cord spasticity, spinal muscle atrophy, myasthenia gravis, neuralgia, fibromyalgia, Machado-Joseph disease, cramp fasciculation syndrome, Freidrich's ataxia, a muscle wasting disorder (e.g., muscle atrophy, sarcopenia, cachexia), an inclusion body myopathy, motor neuron disease, or paralysis.

In some embodiments, the method comprises the treatment of a metabolic disease. In some embodiments, the metabolic disease comprises non-alcoholic steatohepatitis (NASH), non-alcoholic fatty liver disease (NAFLD), liver fibrosis, obesity, heart disease, atherosclerosis, arthritis, cystinosis, diabetes (e.g., Type I diabetes, Type II diabetes, or gestational diabetes), phenylketonuria, proliferative retinopathy, or Kearns-Sayre disease.

In some embodiments, the method comprises the treatment of a mitochondrial disease. In some embodiments, the mitochondrial disease is associated with, or is a result of, or is caused by mitochondrial dysfunction, one or more mitochondrial protein mutations, or one or more mitochondrial DNA mutations. In some embodiments, the mitochondrial disease is a mitochondrial myopathy. In some embodiments, the mitochondrial disease is selected from the group consisting of Barth syndrome, chronic progressive external ophthalmoplegia (cPEO), Kearns-Sayre syndrome (KSS), Leigh syndrome (e.g., MILS, or maternally inherited Leigh syndrome), mitochondrial DNA depletion syndromes (MDDS, e.g., Alpers syndrome), mitochondrial encephalomyopathy (e.g., mitochondrial encephalomyopathy, lactic acidosis, and stroke-like episodes (MELAS)), mitochondrial neurogastrointestinal encephalomyopathy (MNGIE), myoclonus epilepsy with ragged red fibers (MERRF), neuropathy, ataxia, retinitis pigmentosa (NARP), Leber's hereditary optic neuropathy (LHON), and Pearson syndrome.

In another aspect, the present description features a compound of Formula (I) or Formula (II), or a pharmaceutically acceptable salt, solvate, hydrate, tautomer, ester, N-oxide or stereoisomer thereof, or a composition thereof, for use in a method of treating a disease or disorder related to modulation (e.g., a decrease) in eIF2B activity or level, modulation (e.g., a decrease) of eIF2α activity or level, modulation (e.g., an increase) in eIF2α phosphorylation, modulation (e.g., an increase) of phosphorylated eIF2α pathway activity, or modulation (e.g., an increase) of ISR activity in a subject. In some embodiments, the disease may be caused by a mutation to a gene or protein sequence related to a member of the eIF2 pathway (e.g., the eIF2α signaling pathway or ISR pathway).

In another aspect, the present description features a compound of formula (I) or formula (II) for use in a method of treating cancer in a subject in combination with an immunotherapeutic agent.

### DETAILED DESCRIPTION OF THE INVENTION

The present invention features compounds of Formula (II) or (II-a), or a pharmaceutically acceptable salt thereof as defined in appended claims 1 and 4. The invention also features compounds of appended claim 5. These compounds are suitable for use, e.g., in the modulation (e.g., activation) of eIF2B and the attenuation of the ISR signaling pathway.

### Definitions

### Chemical Definitions

Definitions of specific functional groups and chemical terms are described in more detail below. The chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987.

The abbreviations used herein have their conventional meaning within the chemical and biological arts. The chemical structures and formulae set forth herein are constructed according to the standard rules of chemical valency known in the chemical arts.

Compounds described herein can comprise one or more asymmetric centers, and thus can exist in various isomeric forms, e.g., enantiomers and/or diastereomers. For example, the compounds described herein can be in the form of an individual enantiomer, diastereomer or geometric isomer, or can be in the form of a mixture of stereoisomers, including racemic mixtures and mixtures enriched in one or more stereoisomer. Isomers can be isolated from mixtures by methods known to those skilled in the art, including chiral high pressure liquid chromatography (HPLC) and the formation and crystallization of chiral salts; or preferred isomers can be prepared by asymmetric syntheses. See, for example, Jacques et al., Enantiomers, Racemates and Resolutions (Wiley Interscience, New York, 1981); Wilen et al., Tetrahedron 33:2725 (1977); Eliel, Stereochemistry of Carbon Compounds (McGraw-Hill, NY, 1962); and Wilen, Tables of Resolving Agents and Optical Resolutions p. 268 (E.L. Eliel, Ed., Univ. of Notre Dame Press, Notre Dame, IN 1972). The invention additionally encompasses compounds described herein as individual isomers substantially free of other isomers, and alternatively, as mixtures of various isomers.

As used herein a pure enantiomeric compound is substantially free from other enantiomers or stereoisomers of the compound (i.e., in enantiomeric excess). In other words, an "S" form of the compound is substantially free from the "R" form of the compound and is, thus, in enantiomeric excess of the "R" form. The term "enantiomerically pure" or "pure enantiomer" denotes that the compound comprises more than 75% by weight, more than 80% by weight, more than 85% by weight, more than 90% by weight, more than 91% by weight, more than 92% by weight, more than 93% by weight, more than 94% by weight, more than 95% by weight, more than 96% by weight, more than 97% by weight, more than 98% by weight, more than 99% by weight, more than 99.5% by weight, or more than 99.9% by weight, of the enantiomer. In certain embodiments, the weights are based upon total weight of all enantiomers or stereoisomers of the compound.

In the compositions provided herein, an enantiomerically pure compound can be present with other active or inactive ingredients. For example, a pharmaceutical composition comprising enantiomerically pure R-compound can comprise, for example, about 90% excipient and about 10% enantiomerically pure R-compound. In certain embodiments, the enantiomerically pure R-compound in such compositions can, for example, comprise, at least about 95% by weight R-compound and at most about 5% by weight S-compound, by total weight of the compound. For example, a pharmaceutical composition comprising enantiomerically pure S-compound can comprise, for example, about 90% excipient and about 10% enantiomerically pure S-compound. In certain embodiments, the enantiomerically pure S-compound in such compositions can, for example, comprise, at least about 95% by weight S-compound and at most about 5% by weight R-compound, by total weight of the compound. In certain embodiments, the active ingredient can be formulated with little or no excipient or carrier.

Compound described herein may also comprise one or more isotopic substitutions. For example, H may be in any isotopic form, including ¹H, ²H (D or deuterium), and ³H (T or tritium); C may be in any isotopic form, including ¹²C, ¹³C, and ¹⁴C; O may be in any isotopic form, including ¹⁶O and ¹⁸O; and the like.

The articles "a" and "an" may be used herein to refer to one or to more than one (*i.e.* at least one) of the grammatical objects of the article. By way of example "an analogue" means one analogue or more than one analogue.

When a range of values is listed, it is intended to encompass each value and sub-range within the range. For example "C₁-C₆ alkyl" is intended to encompass, C₁, C₂, C₃, C₄, C₅, C₆, C₁-C₆, C₁-C₅, C₁-C₄, C₁-C₃, C₁-C₂, C₂-C₆, C₂-C₅, C₂-C₄, C₂-C₃, C₃-C₆, C₃-C₅, C₃-C₄, C₄-C₆, C₄-C₅, and C₅-C₆ alkyl.

The following terms are intended to have the meanings presented therewith below and are useful in understanding the description and intended scope of the present invention.

"Alkyl" refers to a radical of a straight-chain or branched saturated hydrocarbon group having from 1 to 20 carbon atoms ("C₁-C₂₀ alkyl"). In some embodiments, an alkyl group has 1 to 12 carbon atoms ("C₁-C₁₂ alkyl"). In some embodiments, an alkyl group has 1 to 8 carbon atoms ("C₁-C₈ alkyl"). In some embodiments, an alkyl group has 1 to 6 carbon atoms ("C₁-C₆ alkyl"). In some embodiments, an alkyl group has 1 to 5 carbon atoms ("C₁-C₅ alkyl"). In some embodiments, an alkyl group has 1 to 4 carbon atoms ("C₁-C₄alkyl"). In some embodiments, an alkyl group has 1 to 3 carbon atoms ("C₁-C₃ alkyl"). In some embodiments, an alkyl group has 1 to 2 carbon atoms ("C₁-C₂ alkyl"). In some embodiments, an alkyl group has 1 carbon atom ("C₁ alkyl"). In some embodiments, an alkyl group has 2 to 6 carbon atoms ("C₂-C₆alkyl"). Examples of C₁-C₆alkyl groups include methyl (C₁), ethyl (C₂), n-propyl (C₃), isopropyl (C₃), n-butyl (C₄), tert-butyl (C₄), sec-butyl (C₄), iso-butyl (C₄), n-pentyl (C₅), 3-pentanyl (C₅), amyl (C₅), neopentyl (C₅), 3-methyl-2-butanyl (C₅), tertiary amyl (C₅), and n-hexyl (C₆). Additional examples of alkyl groups include n-heptyl (C₇), n-octyl (C₈) and the like. Common alkyl abbreviations include Me (-CH₃), Et (-CH₂CH₃), iPr (-CH(CH₃)₂), nPr (-CH₂CH₂CH₃), n-Bu (-CH₂CH₂CH₂CH₃), or i-Bu (-CH₂CH(CH₃)₂).

The term "alkylene," by itself or as part of another substituent, means, unless otherwise stated, a divalent radical derived from an alkyl, as exemplified, but not limited by, - CH₂CH₂CH₂CH₂-. Typically, an alkyl (or alkylene) group will have from 1 to 24 carbon atoms, with those groups having 10 or fewer carbon atoms being preferred in the present invention. The term "alkenylene," by itself or as part of another substituent, means, unless otherwise stated, a divalent radical derived from an alkene. An alkylene group may be described as, e.g., a C₁-C₆-membered alkylene, wherein the term "membered" refers to the non-hydrogen atoms within the moiety.

"Alkenyl" refers to a radical of a straight-chain or branched hydrocarbon group having from 2 to 20 carbon atoms, one or more carbon-carbon double bonds, and no triple bonds ("C₂-C₂₀ alkenyl"). In some embodiments, an alkenyl group has 2 to 10 carbon atoms ("C₂-C₁₀ alkenyl"). In some embodiments, an alkenyl group has 2 to 8 carbon atoms ("C₂-C₈ alkenyl"). In some embodiments, an alkenyl group has 2 to 6 carbon atoms ("C₂-C₆ alkenyl"). In some embodiments, an alkenyl group has 2 to 5 carbon atoms ("C₂-C₅ alkenyl"). In some embodiments, an alkenyl group has 2 to 4 carbon atoms ("C₂-C₄ alkenyl"). In some embodiments, an alkenyl group has 2 to 3 carbon atoms ("C₂-C₃ alkenyl"). In some embodiments, an alkenyl group has 2 carbon atoms ("C₂ alkenyl"). The one or more carbon-carbon double bonds can be internal (such as in 2-butenyl) or terminal (such as in 1-butenyl). Examples of C₂-C₄ alkenyl groups include ethenyl (C₂), 1-propenyl (C₃), 2-propenyl (C₃), 1-butenyl (C₄), 2-butenyl (C₄), butadienyl (C₄), and the like. Examples of C₂-C₆ alkenyl groups include the aforementioned C₂₋₄ alkenyl groups as well as pentenyl (C₅), pentadienyl (C₅), hexenyl (C₆), and the like. Additional examples of alkenyl include heptenyl (C₇), octenyl (C₈), octatrienyl (C₈), and the like.

"Aryl" refers to a radical of a monocyclic or polycyclic (*e.g.,* bicyclic or tricyclic) 4n+2 aromatic ring system (*e.g*., having 6, 10, or 14 π electrons shared in a cyclic array) having 6-14 ring carbon atoms and zero heteroatoms provided in the aromatic ring system ("C₆-C₁₄ aryl"). In some embodiments, an aryl group has six ring carbon atoms ("C₆ aryl"; *e.g.,* phenyl). In some embodiments, an aryl group has ten ring carbon atoms ("C₁₀ aryl"; *e.g.,* naphthyl such as 1-naphthyl and 2-naphthyl). In some embodiments, an aryl group has fourteen ring carbon atoms ("C₁₄ aryl"; *e.g.,* anthracyl). An aryl group may be described as, e.g., a C₆-C₁₀-membered aryl, wherein the term "membered" refers to the non-hydrogen ring atoms within the moiety. Aryl groups include, but are not limited to, phenyl, naphthyl, indenyl, and tetrahydronaphthyl.

Examples of representative substituted aryls include the following 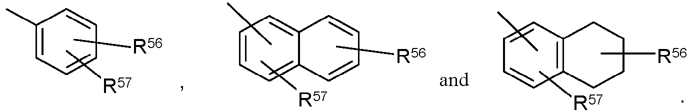 wherein one of R⁵⁶ and R⁵⁷ may be hydrogen and at least one of R⁵⁶ and R⁵⁷ is each independently selected from C₁-C₈ alkyl, halo-C₁-C₈ alkyl, 4-10 membered heterocyclyl, alkanoyl, alkoxy-C₁-C₈ alkyl, heteroaryloxy, alkylamino, arylamino, heteroarylamino, NR⁵⁸COR⁵⁹, ⁵⁸SOR⁵⁹ NR⁵⁸SO₂R⁵⁹, C(O)Oalkyl, C(O)Oaryl, CONR⁵⁸R⁵⁹, CONR⁵⁸OR⁵⁹, NR⁵⁸R⁵⁹, SO₂NR⁵⁸R⁵⁹, S-alkyl, S(O)-alkyl, S(O)₂-alkyl, S-aryl, S(O)-aryl, S(O₂)-aryl; or R⁵⁶ and R⁵⁷ may be joined to form a cyclic ring (saturated or unsaturated) from 5 to 8 atoms, optionally containing one or more heteroatoms selected from the group N, O, or S.

Other representative aryl groups having a fused heterocyclyl group include the following: 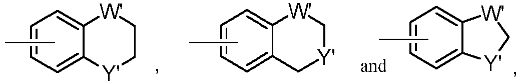 wherein each W' is selected from C(R⁶⁶)₂, NR⁶⁶, O, and S; and each Y' is selected from carbonyl, NR⁶⁶, O and S; and R⁶⁶ is independently hydrogen, C₁-C₈ alkyl, C₃-C₁₀ cycloalkyl, 4-10 membered heterocyclyl, C₆-C₁₀ aryl, and 5-10 membered heteroaryl.

An "arylene" and a "heteroarylene," alone or as part of another substituent, mean a divalent radical derived from an aryl and heteroaryl, respectively. Non-limiting examples of heteroaryl groups include pyridinyl, pyrimidinyl, thiophenyl, thienyl, furanyl, indolyl, benzoxadiazolyl, benzodioxolyl, benzodioxanyl, thianaphthanyl, pyrrolopyridinyl, indazolyl, quinolinyl, quinoxalinyl, pyridopyrazinyl, quinazolinonyl, benzoisoxazolyl, imidazopyridinyl, benzofuranyl, benzothienyl, benzothiophenyl, phenyl, naphthyl, biphenyl, pyrrolyl, pyrazolyl, imidazolyl, pyrazinyl, oxazolyl, isoxazolyl, thiazolyl, furylthienyl, pyridyl, pyrimidyl, benzothiazolyl, purinyl, benzimidazolyl, isoquinolyl, thiadiazolyl, oxadiazolyl, pyrrolyl, diazolyl, triazolyl, tetrazolyl, benzothiadiazolyl, isothiazolyl, pyrazolopyrimidinyl, pyrrolopyrimidinyl, benzotriazolyl, benzoxazolyl, or quinolyl. The examples above and divalent radicals of each heteroaryl example above are non-limiting examples of heteroarylene.

"Halo" or "halogen," independently or as part of another substituent, mean, unless otherwise stated, a fluorine (F), chlorine (Cl), bromine (Br), or iodine (I) atom. The term "halide" by itself or as part of another substituent, refers to a fluoride, chloride, bromide, or iodide atom. In certain embodiments, the halo group is either fluorine or chlorine.

Additionally, terms such as "haloalkyl" are meant to include monohaloalkyl and polyhaloalkyl. For example, the term "halo-C₁-C₆ alkyl" includes, but is not limited to, fluoromethyl, difluoromethyl, trifluoromethyl, 2,2,2-trifluoroethyl, 4-chlorobutyl, 3-bromopropyl, and the like.

The term "heteroalkyl," by itself or in combination with another term, means, unless otherwise stated, a non-cyclic stable straight or branched chain, or combinations thereof, including at least one carbon atom and at least one heteroatom selected from the group consisting of O, N, P, Si, and S, and wherein the nitrogen and sulfur atoms may optionally be oxidized, and the nitrogen heteroatom may optionally be quaternized. The heteroatom(s) O, N, P, S, and Si may be placed at any interior position of the heteroalkyl group or at the position at which the alkyl group is attached to the remainder of the molecule. Exemplary heteroalkyl groups include, but are not limited to: -CH₂-CH₂-O-CH₃, -CH₂-CH₂-NH-CH₃, -CH₂-CH₂-N(CH₃)-CH₃, -CH₂-S-CH₂-CH₃, -CH₂-CH₂, -S(O)₂, -S(O)-CH₃, -S(O)₂-CH₂, -CH₂-CH₂-S(O)₂-CH₃, -CH=CH-O-CH₃, -Si(CH₃)₃, -CH₂-CH=N-OCH₃, -CH=CH-N(CH₃)-CH₃, -O-CH₃, and -O-CH₂-CH₃. Up to two or three heteroatoms may be consecutive, such as, for example, -CH₂-NH-OCH₃ and -CH₂-O-Si(CH₃)₃. Where "heteroalkyl" is recited, followed by recitations of specific heteroalkyl groups, such as +CH₂O, -NR^{B}R^{C}, or the like, it will be understood that the terms heteroalkyl and -CH₂O or -NR^{B}R^{C} are not redundant or mutually exclusive. Rather, the specific heteroalkyl groups are recited to add clarity. Thus, the term "heteroalkyl" should not be interpreted herein as excluding specific heteroalkyl groups, such as -CH₂O, -NR^{B}R^{C}, or the like.

Similarly, the term "heteroalkylene," by itself or as part of another substituent, means, unless otherwise stated, a divalent radical derived from heteroalkyl, as exemplified, but not limited by, -CH₂O- and -CH₂CH₂O-. A heteroalkylene group may be described as, e.g., a 2-7-membered heteroalkylene, wherein the term "membered" refers to the non-hydrogen atoms within the moiety. For heteroalkylene groups, heteroatoms can also occupy either or both of the chain termini (e.g., alkyleneoxy, alkylenedioxy, alkyleneamino, alkylenediamino, and the like). Still further, for alkylene and heteroalkylene linking groups, no orientation of the linking group is implied by the direction in which the formula of the linking group is written. For example, the formula -C(O)₂R'- may represent both -C(O)₂R'- and -R'C(O)₂-.

"Heteroaryl" refers to a radical of a 5-10 membered monocyclic or bicyclic 4n+2 aromatic ring system (*e.g.,* having 6 or 10 π electrons shared in a cyclic array) having ring carbon atoms and 1-4 ring heteroatoms provided in the aromatic ring system, wherein each heteroatom is independently selected from nitrogen, oxygen and sulfur ("5-10 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. Bicyclic heteroaryl groups wherein one ring does not contain a heteroatom (*e.g.,* indolyl, quinolinyl, carbazolyl, and the like) the point of attachment can be on either ring, *i.e.,* either the ring bearing a heteroatom (*e.g.,* 2-indolyl) or the ring that does not contain a heteroatom (*e.g.,* 5-indolyl). A heteroaryl group may be described as, e.g., a 6-10-membered heteroaryl, wherein the term "membered" refers to the non-hydrogen ring atoms within the moiety.

In some embodiments, a heteroaryl group is a 5-10 membered aromatic ring system having ring carbon atoms and 1-4 ring heteroatoms provided in the aromatic ring system, wherein each heteroatom is independently selected from nitrogen, oxygen, and sulfur ("5-10 membered heteroaryl"). In some embodiments, a heteroaryl group is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). In some embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). In some embodiments, the 5-6 membered heteroaryl has 1-3 ring heteroatoms selected from nitrogen, oxygen, and sulfur. In some embodiments, the 5-6 membered heteroaryl has 1-2 ring heteroatoms selected from nitrogen, oxygen, and sulfur. In some embodiments, the 5-6 membered heteroaryl has 1 ring heteroatom selected from nitrogen, oxygen, and sulfur.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

Examples of representative heteroaryls include the following formulae: 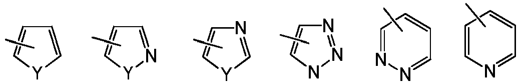 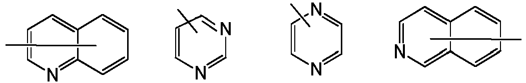 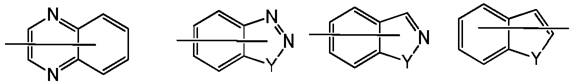 wherein each Y is selected from carbonyl, N, NR⁶⁵, O, and S; and R⁶⁵ is independently hydrogen, C₁-C₈ alkyl, C₃-C₁₀ cycloalkyl, 4-10 membered heterocyclyl, C₆-C₁₀ aryl, and 5-10 membered heteroaryl.

"Cycloalkyl" refers to a radical of a non-aromatic cyclic hydrocarbon group having from 3 to 10 ring carbon atoms ("C₃-C₁₀ cycloalkyl") and zero heteroatoms in the non-aromatic ring system. In some embodiments, a cycloalkyl group has 3 to 8 ring carbon atoms ("C₃-C₈cycloalkyl"). In some embodiments, a cycloalkyl group has 3 to 6 ring carbon atoms ("C₃-C₆ cycloalkyl"). In some embodiments, a cycloalkyl group has 3 to 6 ring carbon atoms ("C₃-C₆ cycloalkyl"). In some embodiments, a cycloalkyl group has 5 to 10 ring carbon atoms ("C₅-C₁₀ cycloalkyl"). A cycloalkyl group may be described as, e.g., a C₄-C₇-membered cycloalkyl, wherein the term "membered" refers to the non-hydrogen ring atoms within the moiety. Exemplary C₃-C₆ cycloalkyl groups include, without limitation, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), and the like. Exemplary C₃-C₈ cycloalkyl groups include, without limitation, the aforementioned C₃-C₆ cycloalkyl groups as well as cycloheptyl (C₇), cycloheptenyl (C₇), cycloheptadienyl (C₇), cycloheptatrienyl (C₇), cyclooctyl (C₈), cyclooctenyl (C₈), cubanyl (C₈), bicyclo[1.1.1]pentanyl (C₅), bicyclo[2.2.2]octanyl (C₈), bicyclo[2.1.1]hexanyl (C₆), bicyclo[3.1.1]heptanyl (C₇), and the like. Exemplary C₃-C₁₀ cycloalkyl groups include, without limitation, the aforementioned C₃-C₈ cycloalkyl groups as well as cyclononyl (C₉), cyclononenyl (C₉), cyclodecyl (C₁₀), cyclodecenyl (C₁₀), octahydro-1H-indenyl (C₉), decahydronaphthalenyl (C₁₀), spiro[4.5]decanyl (C₁₀), and the like. As the foregoing examples illustrate, in certain embodiments, the cycloalkyl group is either monocyclic ("monocyclic cycloalkyl") or contain a fused, bridged or spiro ring system such as a bicyclic system ("bicyclic cycloalkyl") and can be saturated or can be partially unsaturated. "Cycloalkyl" also includes ring systems wherein the cycloalkyl ring, as defined above, is fused with one or more aryl groups wherein the point of attachment is on the cycloalkyl ring, and in such instances, the number of carbons continue to designate the number of carbons in the cycloalkyl ring system.

In some embodiments, "cycloalkyl" is a monocyclic, saturated cycloalkyl group having from 3 to 10 ring carbon atoms ("C₃-C₁₀ cycloalkyl"). In some embodiments, a cycloalkyl group has 3 to 8 ring carbon atoms ("C₃-C₈ cycloalkyl"). In some embodiments, a cycloalkyl group has 3 to 6 ring carbon atoms ("C₃-C₆ cycloalkyl"). In some embodiments, a cycloalkyl group has 5 to 6 ring carbon atoms ("C₅-C₆ cycloalkyl"). In some embodiments, a cycloalkyl group has 5 to 10 ring carbon atoms ("C₅-C₁₀ cycloalkyl"). Examples of C₅-C₆ cycloalkyl groups include cyclopentyl (C₅) and cyclohexyl (C₅). Examples of C₃-C₆ cycloalkyl groups include the aforementioned C₅-C₆ cycloalkyl groups as well as cyclopropyl (C₃) and cyclobutyl (C₄). Examples of C₃-C₈ cycloalkyl groups include the aforementioned C₃-C₆ cycloalkyl groups as well as cycloheptyl (C₇) and cyclooctyl (C₈). Unless otherwise specified, each instance of a cycloalkyl group is independently unsubstituted (an "unsubstituted cycloalkyl") or substituted (a "substituted cycloalkyl") with one or more substituents. In certain embodiments, the cycloalkyl group is unsubstituted C₃-C₁₀ cycloalkyl. In certain embodiments, the cycloalkyl group is substituted C₃-C₁₀ cycloalkyl.

"Heterocyclyl" or "heterocyclic" refers to a radical of a 3- to 10-membered non-aromatic ring system having ring carbon atoms and 1 to 4 ring heteroatoms, wherein each heteroatom is independently selected from nitrogen, oxygen, sulfur, boron, phosphorus, and silicon ("3-10 membered heterocyclyl"). In heterocyclyl groups that contain one or more nitrogen atoms, the point of attachment can be a carbon or nitrogen atom, as valency permits. A heterocyclyl group can either be monocyclic ("monocyclic heterocyclyl") or a fused, bridged or spiro ring system such as a bicyclic system ("bicyclic heterocyclyl"), and can be saturated or can be partially unsaturated. Heterocyclyl bicyclic ring systems can include one or more heteroatoms in one or both rings. "Heterocyclyl" also includes ring systems wherein the heterocyclyl ring, as defined above, is fused with one or more cycloalkyl groups wherein the point of attachment is either on the cycloalkyl or heterocyclyl ring, or ring systems wherein the heterocyclyl ring, as defined above, is fused with one or more aryl or heteroaryl groups, wherein the point of attachment is on the heterocyclyl ring, and in such instances, the number of ring members continue to designate the number of ring members in the heterocyclyl ring system. A heterocyclyl group may be described as, e.g., a 3-7-membered heterocyclyl, wherein the term "membered" refers to the non-hydrogen ring atoms, i.e., carbon, nitrogen, oxygen, sulfur, boron, phosphorus, and silicon, within the moiety.

In some embodiments, a heterocyclyl group is a 5-10 membered non-aromatic ring system having ring carbon atoms and 1-4 ring heteroatoms, wherein each heteroatom is independently selected from nitrogen, oxygen, sulfur, boron, phosphorus, and silicon ("5-10 membered heterocyclyl"). In some embodiments, a heterocyclyl group is a 5-8 membered non-aromatic ring system having ring carbon atoms and 1-4 ring heteroatoms, wherein each heteroatom is independently selected from nitrogen, oxygen, and sulfur ("5-8 membered heterocyclyl"). In some embodiments, a heterocyclyl group is a 5-6 membered non-aromatic ring system having ring carbon atoms and 1-4 ring heteroatoms, wherein each heteroatom is independently selected from nitrogen, oxygen, and sulfur ("5-6 membered heterocyclyl"). In some embodiments, the 5-6 membered heterocyclyl has 1-3 ring heteroatoms selected from nitrogen, oxygen, and sulfur. In some embodiments, the 5-6 membered heterocyclyl has 1-2 ring heteroatoms selected from nitrogen, oxygen, and sulfur. In some embodiments, the 5-6 membered heterocyclyl has one ring heteroatom selected from nitrogen, oxygen, and sulfur.

Exemplary 3-membered heterocyclyl groups containing one heteroatom include, without limitation, azirdinyl, oxiranyl, thiorenyl. Exemplary 4-membered heterocyclyl groups containing one heteroatom include, without limitation, azetidinyl, oxetanyl and thietanyl. Exemplary 5-membered heterocyclyl groups containing one heteroatom include, without limitation, tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocyclyl groups containing two heteroatoms include, without limitation, dioxolanyl, oxasulfuranyl, disulfuranyl, and oxazolidin-2-one. Exemplary 5-membered heterocyclyl groups containing three heteroatoms include, without limitation, triazolinyl, oxadiazolinyl, and thiadiazolinyl. Exemplary 6-membered heterocyclyl groups containing one heteroatom include, without limitation, piperidinyl, tetrahydropyranyl, dihydropyridinyl, and thianyl. Exemplary 6-membered heterocyclyl groups containing two heteroatoms include, without limitation, piperazinyl, morpholinyl, dithianyl, dioxanyl. Exemplary 6-membered heterocyclyl groups containing two heteroatoms include, without limitation, triazinanyl. Exemplary 7-membered heterocyclyl groups containing one heteroatom include, without limitation, azepanyl, oxepanyl and thiepanyl. Exemplary 8-membered heterocyclyl groups containing one heteroatom include, without limitation, azocanyl, oxecanyl and thiocanyl. Exemplary 5-membered heterocyclyl groups fused to a C₆ aryl ring (also referred to herein as a 5,6-bicyclic heterocyclic ring) include, without limitation, indolinyl, isoindolinyl, dihydrobenzofuranyl, dihydrobenzothienyl, benzoxazolinonyl, and the like. Exemplary 6-membered heterocyclyl groups fused to an aryl ring (also referred to herein as a 6,6-bicyclic heterocyclic ring) include, without limitation, tetrahydroquinolinyl, tetrahydroisoquinolinyl, and the like.

Particular examples of heterocyclyl groups are shown in the following illustrative examples: 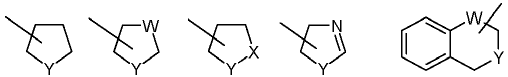 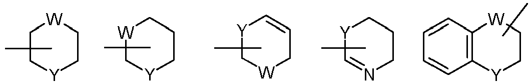 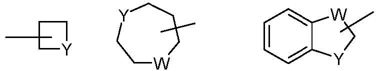 wherein each W is selected from CR⁶⁷, C(R⁶⁷)₂, NR⁶⁷, O, and S; and each Y is selected from NR⁶⁷, O, and S; and R⁶⁷ is independently hydrogen, C₁-C₈ alkyl, C₃-C₁₀ cycloalkyl, 4-10 membered heterocyclyl, C₆-C₁₀ aryl, and 5-10-membered heteroaryl. These heterocyclyl rings may be optionally substituted with one or more groups selected from the group consisting of acyl, acylamino, acyloxy, alkoxy, alkoxycarbonyl, alkoxycarbonylamino, amino, substituted amino, aminocarbonyl (e.g., amido), aminocarbonylamino, aminosulfonyl, sulfonylamino, aryl, aryloxy, azido, carboxyl, cyano, cycloalkyl, halogen, hydroxy, keto, nitro, thiol, -S-alkyl, -S-aryl, -S(O)-alkyl, -S(O)-aryl, -S(O)₂-alkyl, and -S(O)₂-aryl. Substituting groups include carbonyl or thiocarbonyl which provide, for example, lactam and urea derivatives.

"Nitrogen-containing heterocyclyl" group means a 4- to 7- membered non-aromatic cyclic group containing at least one nitrogen atom, for example, but without limitation, morpholine, piperidine (*e.g.* 2-piperidinyl, 3-piperidinyl and 4-piperidinyl), pyrrolidine (*e.g. 2-*pyrrolidinyl and 3-pyrrolidinyl), azetidine, pyrrolidone, imidazoline, imidazolidinone, 2-pyrazoline, pyrazolidine, piperazine, and N-alkyl piperazines such as N-methyl piperazine. Particular examples include azetidine, piperidone and piperazone.

"Amino" refers to the radical -NR⁷⁰R⁷¹, wherein R⁷⁰ and R⁷¹ are each independently hydrogen, C₁-C₈ alkyl, C₃-C₁₀ cycloalkyl, 4-10 membered heterocyclyl, C₆-C₁₀ aryl, and 5-10-membered heteroaryl. In some embodiments, amino refers to NH₂.

"Cyano" refers to the radical -CN.

"Hydroxy" refers to the radical -OH.

In general, the term "substituted", whether preceded by the term "optionally" or not, means that at least one hydrogen present on a group (*e.g.,* a carbon or nitrogen atom) is replaced with a permissible substituent, *e.g.,* a substituent which upon substitution results in a stable compound, *e.g.,* a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. The term "substituted" is contemplated to include substitution with all permissible substituents of organic compounds, such as any of the substituents described herein that result in the formation of a stable compound. The present invention contemplates any and all such combinations in order to arrive at a stable compound. For purposes of this invention, heteroatoms such as nitrogen may have hydrogen substituents and/or any suitable substituent as described herein which satisfy the valencies of the heteroatoms and results in the formation of a stable moiety.

Two or more substituents may optionally be joined to form aryl, heteroaryl, cycloalkyl, or heterocycloalkyl groups. Such so-called ring-forming substituents are typically, though not necessarily, found attached to a cyclic base structure. In one embodiment, the ring-forming substituents are attached to adjacent members of the base structure. For example, two ring-forming substituents attached to adjacent members of a cyclic base structure create a fused ring structure. In another embodiment, the ring-forming substituents are attached to a single member of the base structure. For example, two ring-forming substituents attached to a single member of a cyclic base structure create a spirocyclic structure. In yet another embodiment, the ring-forming substituents are attached to non-adjacent members of the base structure.

A "counterion" or "anionic counterion" is a negatively charged group associated with a cationic quaternary amino group in order to maintain electronic neutrality. Exemplary counterions include halide ions (*e.g.,* F⁻, Cl⁻, Br⁻, I⁻), NO₃⁻, ClO₄⁻, OH⁻, H₂PO₄⁻, HSO₄⁻, sulfonate ions (*e.g.,* methansulfonate, trifluoromethanesulfonate, p-toluenesulfonate, benzenesulfonate, 10-camphor sulfonate, naphthalene-2-sulfonate, naphthalene-1-sulfonic acid-5-sulfonate, ethan-1-sulfonic acid-2-sulfonate, and the like), and carboxylate ions (*e.g.,* acetate, ethanoate, propanoate, benzoate, glycerate, lactate, tartrate, glycolate, and the like).

The term "pharmaceutically acceptable salts" is meant to include salts of the active compounds that are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, e.g., Berge et al, Journal of Pharmaceutical Science 66: 1-19 (1977)). Certain specific compounds of the present invention contain both basic and acidic functionalities that allow the compounds to be converted into either base or acid addition salts. Other pharmaceutically acceptable carriers known to those of skill in the art are suitable for the present invention. Salts tend to be more soluble in aqueous or other protonic solvents that are the corresponding free base forms. In other cases, the preparation may be a lyophilized powder in a first buffer, e.g., in 1 mM-50 mM histidine, 0. l%-2% sucrose, 2%-7% mannitol at a pH range of 4.5 to 5.5, that is combined with a second buffer prior to use.

Thus, the compounds of the present invention may exist as salts, such as with pharmaceutically acceptable acids. The present invention includes such salts. Examples of such salts include hydrochlorides, hydrobromides, sulfates, methanesulfonates, nitrates, maleates, acetates, citrates, fumarates, tartrates (e.g., (+)-tartrates, (-)-tartrates, or mixtures thereof including racemic mixtures), succinates, benzoates, and salts with amino acids such as glutamic acid. These salts may be prepared by methods known to those skilled in the art.

The neutral forms of the compounds are preferably regenerated by contacting the salt with a base or acid and isolating the parent compound in the conventional manner. The parent form of the compound differs from the various salt forms in certain physical properties, such as solubility in polar solvents.

In addition to salt forms, the present description provides compounds, which are in a prodrug form. Prodrugs of the compounds described herein are those compounds that readily undergo chemical changes under physiological conditions to provide the compounds of the present invention. Additionally, prodrugs can be converted to the compounds of the present invention by chemical or biochemical methods in an ex vivo environment. For example, prodrugs can be slowly converted to the compounds of the present invention when placed in a transdermal patch reservoir with a suitable enzyme or chemical reagent.

Certain compounds of the present invention can exist in unsolvated forms as well as solvated forms, including hydrated forms. In general, the solvated forms are equivalent to unsolvated forms and are encompassed within the scope of the present invention. Certain compounds of the present invention may exist in multiple crystalline or amorphous forms. In general, all physical forms are equivalent for the uses of the compounds contemplated by the present invention and are intended to be within the scope of the present invention.

As used herein, the term "salt" refers to acid or base salts of the compounds used in the methods of the present invention. Illustrative examples of acceptable salts are mineral acid (hydrochloric acid, hydrobromic acid, phosphoric acid, and the like) salts, organic acid (acetic acid, propionic acid, glutamic acid, citric acid and the like) salts, quaternary ammonium (methyl iodide, ethyl iodide, and the like) salts.

Certain compounds of the present invention possess asymmetric carbon atoms (optical or chiral centers) or double bonds; the enantiomers, racemates, diastereomers, tautomers, geometric isomers, stereoisometric forms that may be defined, in terms of absolute stereochemistry, as (R)- or (S)- or, as (D)- or (L)- for amino acids, and individual isomers are encompassed within the scope of the present invention. The compounds of the present invention do not include those which are known in art to be too unstable to synthesize and/or isolate. The present invention is meant to include compounds in racemic and optically pure forms. Optically active (R)- and (S)-, or (D)- and (L)-isomers may be prepared using chiral synthons or chiral reagents, or resolved using conventional techniques. When the compounds described herein contain olefinic bonds or other centers of geometric asymmetry, and unless specified otherwise, it is intended that the compounds include both E and Z geometric isomers.

As used herein, the term "isomers" refers to compounds having the same number and kind of atoms, and hence the same molecular weight, but differing in respect to the structural arrangement or configuration of the atoms.

The term "tautomer," as used herein, refers to one of two or more structural isomers which exist in equilibrium and which are readily converted from one isomeric form to another.

It will be apparent to one skilled in the art that certain compounds of this invention may exist in tautomeric forms, all such tautomeric forms of the compounds being within the scope of the invention.

The terms "treating" or "treatment" refers to any indicia of success in the treatment or amelioration of an injury, disease, pathology or condition, including any objective or subjective parameter such as abatement; remission; diminishing of symptoms or making the injury, pathology or condition more tolerable to the patient; slowing in the rate of degeneration or decline; making the final point of degeneration less debilitating; improving a patient's physical or mental well-being. The treatment or amelioration of symptoms can be based on objective or subjective parameters; including the results of a physical examination, neuropsychiatric exams, and/or a psychiatric evaluation. For example, certain methods herein treat cancer (e.g. pancreatic cancer, breast cancer, multiple myeloma, cancers of secretory cells), neurodegenerative diseases (e.g. Alzheimer's disease, Parkinson's disease, frontotemporal dementia), leukodystrophies (e.g., vanishing white matter disease, childhood ataxia with CNS hypo-myelination), postsurgical cognitive dysfunction, traumatic brain injury, stroke, spinal cord injury, intellectual disability syndromes, inflammatory diseases, musculoskeletal diseases, metabolic diseases, or diseases or disorders associated with impaired function of eIF2B or components in a signal transduction or signaling pathway including the ISR and decreased eIF2 pathway activity). For example certain methods herein treat cancer by decreasing or reducing or preventing the occurrence, growth, metastasis, or progression of cancer or decreasing a symptom of cancer; treat neurodegeneration by improving mental wellbeing, increasing mental function, slowing the decrease of mental function, decreasing dementia, delaying the onset of dementia, improving cognitive skills, decreasing the loss of cognitive skills, improving memory, decreasing the degradation of memory, decreasing a symptom of neurodegeneration or extending survival; treat vanishing white matter disease by reducing a symptom of vanishing white matter disease or reducing the loss of white matter or reducing the loss of myelin or increasing the amount of myelin or increasing the amount of white matter; treat childhood ataxia with CNS hypo-myelination by decreasing a symptom of childhood ataxia with CNS hypo-myelination or increasing the level of myelin or decreasing the loss of myelin; treat an intellectual disability syndrome by decreasing a symptom of an intellectual disability syndrome, treat an inflammatory disease by treating a symptom of the inflammatory disease; treat a musculoskeletal disease by treating a symptom of the musculoskeletal disease; or treat a metabolic disease by treating a symptom of the metabolic disease. Symptoms of a disease, disorder, or condition described herein (e.g., cancer a neurodegenerative disease, a leukodystrophy, an inflammatory disease, a musculoskeletal disease, a metabolic disease, or a condition or disease associated with impaired function of eIF2B or components in a signal transduction pathway including the eIF2 pathway, eIF2□ phosphorylation. or ISR pathway) would be known or may be determined by a person of ordinary skill in the art. The term "treating" and conjugations thereof, include prevention of an injury, pathology, condition, or disease (e.g. preventing the development of one or more symptoms of a disease, disorder, or condition described herein).

An "effective amount" is an amount sufficient to accomplish a stated purpose (e.g. achieve the effect for which it is administered, treat a disease, reduce enzyme activity, increase enzyme activity, or reduce one or more symptoms of a disease or condition). An example of an "effective amount" is an amount sufficient to contribute to the treatment, prevention, or reduction of a symptom or symptoms of a disease, which could also be referred to as a "therapeutically effective amount." A "prophylactically effective amount" of a drug is an amount of a drug that, when administered to a subject, will have the intended prophylactic effect, e.g., preventing or delaying the onset (or reoccurrence) of an injury, disease, pathology or condition, or reducing the likelihood of the onset (or reoccurrence) of an injury, disease, pathology, or condition, or their symptoms. The full prophylactic effect does not necessarily occur by administration of one dose, and may occur only after administration of a series of doses. Thus, a prophylactically effective amount may be administered in one or more administrations. The exact amounts will depend on the purpose of the treatment, and will be ascertainable by one skilled in the art using known techniques (see, e.g., Lieberman, Pharmaceutical Dosage Forms (vols. 1-3, 1992); Lloyd, The Art, Science and Technology of Pharmaceutical Compounding (1999); Pickar, Dosage Calculations (1999); and Remington: The Science and Practice of Pharmacy, 20th Edition, 2003, Gennaro, Ed., Lippincott, Williams & Wilkins).

A "reduction" of a symptom or symptoms (and grammatical equivalents of this phrase) means decreasing of the severity or frequency of the symptom(s), or elimination of the symptom(s).

The term "associated" or "associated with" in the context of a substance or substance activity or function associated with a disease (e.g., a disease or disorder described herein, e.g., cancer, a neurodegenerative disease, a leukodystrophy, an inflammatory disease, a musculoskeletal disease, a metabolic disease, or a disease or disorder associated with impaired function of eIF2B or components in a signal transduction pathway including the eIF2 pathway, eIF2□ phosphorylation. or ISR pathway) means that the disease is caused by (in whole or in part), or a symptom of the disease is caused by (in whole or in part) the substance or substance activity or function. For example, a symptom of a disease or condition associated with an impaired function of the eIF2B may be a symptom that results (entirely or partially) from a decrease in eIF2B activity (e.g. decrease in eIF2B activity or levels, increase in eIF2α phosphorylation or activity of phosphorylated eIF2α or reduced eIF2 activity or increase in activity of phosphorylated eIF2α signal transduction or the ISR signalling pathway). As used herein, what is described as being associated with a disease, if a causative agent, could be a target for treatment of the disease. For example, a disease associated with decreased eIF2 activity or eIF2 pathway activity, may be treated with an agent (e.g., compound as described herein) effective for increasing the level or activity of eIF2 or eIF2 pathway or a decrease in phosphorylated eIF2α activity or the ISR pathway. For example, a disease associated with phosphorylated eIF2α may be treated with an agent (e.g., compound as described herein) effective for decreasing the level of activity of phosphorylated eIF2α or a downstream component or effector of phosphorylated eIF2α. For example, a disease associated with eIF2α, may be treated with an agent (e.g., compound as described herein) effective for increasing the level of activity of eIF2 or a downstream component or effector of eIF2.

"Control" or "control experiment" is used in accordance with its plain ordinary meaning and refers to an experiment in which the subjects or reagents of the experiment are treated as in a parallel experiment except for omission of a procedure, reagent, or variable of the experiment. In some instances, the control is used as a standard of comparison in evaluating experimental effects.

"Contacting" is used in accordance with its plain ordinary meaning and refers to the process of allowing at least two distinct species (e.g. chemical compounds including biomolecules, or cells) to become sufficiently proximal to react, interact or physically touch. It should be appreciated, however, that the resulting reaction product can be produced directly from a reaction between the added reagents or from an intermediate from one or more of the added reagents which can be produced in the reaction mixture. The term "contacting" may include allowing two species to react, interact, or physically touch, wherein the two species may be a compound as described herein and a protein or enzyme (e.g. eIF2B, eIF2α, or a component of the eIF2 pathway or ISR pathway). In some embodiments contacting includes allowing a compound described herein to interact with a protein or enzyme that is involved in a signaling pathway (e.g. eIF2B, eIF2α, or a component of the eIF2 pathway or ISR pathway).

As defined herein, the term "inhibition", "inhibit", "inhibiting" and the like in reference to a protein-inhibitor (e.g., antagonist) interaction means negatively affecting (e.g., decreasing) the activity or function of the protein relative to the activity or function of the protein in the absence of the inhibitor. In some embodiments, inhibition refers to reduction of a disease or symptoms of disease. In some embodiments, inhibition refers to a reduction in the activity of a signal transduction pathway or signaling pathway. Thus, inhibition includes, at least in part, partially or totally blocking stimulation, decreasing, preventing, or delaying activation, or inactivating, desensitizing, or down-regulating signal transduction or enzymatic activity or the amount of a protein. In some embodiments, inhibition refers to a decrease in the activity of a signal transduction pathway or signaling pathway (e.g., eIF2B, eIF2α, or a component of the eIF2 pathway, pathway activated by eIF2α phosphorylation, or ISR pathway). Thus, inhibition may include, at least in part, partially or totally decreasing stimulation, decreasing or reducing activation, or inactivating, desensitizing, or down-regulating signal transduction or enzymatic activity or the amount of a protein increased in a disease (e.g. eIF2B, eIF2α, or a component of the eIF2 pathway or ISR pathway, wherein each is associated with cancer, a neurodegenerative disease, a leukodystrophy, an inflammatory disease, a musculoskeletal disease, or a metabolic disease). Inhibition may include, at least in part, partially or totally decreasing stimulation, decreasing or reducing activation, or deactivating, desensitizing, or down-regulating signal transduction or enzymatic activity or the amount of a protein (e.g. eIF2B, eIF2α, or component of the eIF2 pathway or ISR pathway) that may modulate the level of another protein or increase cell survival (e.g., decrease in phosphorylated eIF2α pathway activity may increase cell survival in cells that may or may not have an increase in phosphorylated eIF2α pathway activity relative to a non-disease control or decrease in eIF2α pathway activity may increase cell survival in cells that may or may not have an increase in eIF2α pathway activity relative to a non-disease control).

As defined herein, the term "activation", "activate", "activating" and the like in reference to a protein-activator (e.g. agonist) interaction means positively affecting (e.g. increasing) the activity or function of the protein (e.g. eIF2B, eIF2α, or component of the eIF2 pathway or ISR pathway) relative to the activity or function of the protein in the absence of the activator (e.g. compound described herein). In some embodiments, activation refers to an increase in the activity of a signal transduction pathway or signaling pathway (e.g. eIF2B, eIF2α, or component of the eIF2 pathway or ISR pathway). Thus, activation may include, at least in part, partially or totally increasing stimulation, increasing or enabling activation, or activating, sensitizing, or up-regulating signal transduction or enzymatic activity or the amount of a protein decreased in a disease (e.g. level of eIF2B, eIF2α, or component of the eIF2 pathway or ISR pathway associated with cancer, a neurodegenerative disease, a leukodystrophy, an inflammatory disease, a musculoskeletal disease, or a metabolic disease). Activation may include, at least in part, partially or totally increasing stimulation, increasing or enabling activation, or activating, sensitizing, or up-regulating signal transduction or enzymatic activity or the amount of a protein (e.g., eIF2B, eIF2α, or component of the eIF2 pathway or ISR pathway) that may modulate the level of another protein or increase cell survival (e.g., increase in eIF2α activity may increase cell survival in cells that may or may not have a reduction in eIF2α activity relative to a non-disease control).

The term "modulation" refers to an increase or decrease in the level of a target molecule or the function of a target molecule. In some embodiments, modulation of eIF2B, eIF2α, or a component of the eIF2 pathway or ISR pathway may result in reduction of the severity of one or more symptoms of a disease associated with eIF2B, eIF2α, or a component of the eIF2 pathway or ISR pathway (e.g., cancer, a neurodegenerative disease, a leukodystrophy, an inflammatory disease, a musculoskeletal disease, or a metabolic disease) or a disease that is not caused by eIF2B, eIF2α, or a component of the eIF2 pathway or ISR pathway but may benefit from modulation of eIF2B, eIF2α, or a component of the eIF2 pathway or ISR pathway (e.g., decreasing in level or level of activity of eIF2B, eIF2α or a component of the eIF2 pathway).

The term "modulator" as used herein refers to modulation of (e.g., an increase or decrease in) the level of a target molecule or the function of a target molecule. In embodiments, a modulator of eIF2B, eIF2α, or component of the eIF2 pathway or ISR pathway is an anti-cancer agent. In embodiments, a modulator of eIF2B, eIF2α, or component of the eIF2 pathway or ISR pathway is a neuroprotectant. In embodiments, a modulator of eIF2B, eIF2α, or component of the eIF2 pathway or ISR pathway is a memory enhancing agent. In embodiments, a modulator of eIF2B, eIF2α, or component of the eIF2 pathway or ISR pathway is a memory enhancing agent (e.g., a long term memory enhancing agent). In embodiments, a modulator of eIF2B, eIF2α, or component of the eIF2 pathway or ISR pathway is an anti-inflammatory agent. In some embodiments, a modulator of eIF2B, eIF2α, or component of the eIF2 pathway or ISR pathway is a pain-relieving agent.

"Patient" or "subject in need thereof refers to a living organism suffering from or prone to a disease or condition that can be treated by administration of a compound or pharmaceutical composition, as provided herein. Non-limiting examples include humans, other mammals, bovines, rats, mice, dogs, monkeys, goat, sheep, cows, deer, and other non-mammalian animals. In some embodiments, a patient is human. In some embodiments, a patient is a domesticated animal. In some embodiments, a patient is a dog. In some embodiments, a patient is a parrot. In some embodiments, a patient is livestock animal. In some embodiments, a patient is a mammal. In some embodiments, a patient is a cat. In some embodiments, a patient is a horse. In some embodiments, a patient is bovine. In some embodiments, a patient is a canine. In some embodiments, a patient is a feline. In some embodiments, a patient is an ape. In some embodiments, a patient is a monkey. In some embodiments, a patient is a mouse. In some embodiments, a patient is an experimental animal. In some embodiments, a patient is a rat. In some embodiments, a patient is a hamster. In some embodiments, a patient is a test animal. In some embodiments, a patient is a newborn animal. In some embodiments, a patient is a newborn human. In some embodiments, a patient is a newborn mammal. In some embodiments, a patient is an elderly animal. In some embodiments, a patient is an elderly human. In some embodiments, a patient is an elderly mammal. In some embodiments, a patient is a geriatric patient.

"Disease", "disorder" or "condition" refers to a state of being or health status of a patient or subject capable of being treated with a compound, pharmaceutical composition, or method provided herein. In some embodiments, the compounds and methods described herein comprise reduction or elimination of one or more symptoms of the disease, disorder, or condition, e.g., through administration of a compound of Formula (I) or Formula (II), or a pharmaceutically acceptable salt thereof.

The term "signaling pathway" as used herein refers to a series of interactions between cellular and optionally extra-cellular components (e.g. proteins, nucleic acids, small molecules, ions, lipids) that conveys a change in one component to one or more other components, which in turn may convey a change to additional components, which is optionally propagated to other signaling pathway components.

"Pharmaceutically acceptable excipient" and "pharmaceutically acceptable carrier" refer to a substance that aids the administration of an active agent to and absorption by a subject and can be included in the compositions of the present description without causing a significant adverse toxicological effect on the patient. Non-limiting examples of pharmaceutically acceptable excipients include water, NaCl, normal saline solutions, lactated Ringer's, normal sucrose, normal glucose, binders, fillers, disintegrants, lubricants, coatings, sweeteners, flavors, salt solutions (such as Ringer's solution), alcohols, oils, gelatins, carbohydrates such as lactose, amylose or starch, fatty acid esters, hydroxymethycellulose, polyvinyl pyrrolidine, and colors, and the like. Such preparations can be sterilized and, if desired, mixed with auxiliary agents such as lubricants, preservatives, stabilizers, wetting agents, emulsifiers, salts for influencing osmotic pressure, buffers, coloring, and/or aromatic substances and the like that do not deleteriously react with the compounds of the invention. One of skill in the art will recognize that other pharmaceutical excipients are useful in the present invention.

The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

As used herein, the term "administering" means oral administration, administration as a suppository, topical contact, intravenous, parenteral, intraperitoneal, intramuscular, intralesional, intrathecal, intracranial, intranasal or subcutaneous administration, or the implantation of a slow-release device, e.g., a mini-osmotic pump, to a subject. Administration is by any route, including parenteral and transmucosal (e.g., buccal, sublingual, palatal, gingival, nasal, vaginal, rectal, or transdermal). Parenteral administration includes, e.g., intravenous, intramuscular, intra-arterial, intradermal, subcutaneous, intraperitoneal, intraventricular, and intracranial. Other modes of delivery include, but are not limited to, the use of liposomal formulations, intravenous infusion, transdermal patches, etc. By "co-administer" it is meant that a composition described herein is administered at the same time, just prior to, or just after the administration of one or more additional therapies (e.g., anti-cancer agent, chemotherapeutic, or treatment for a neurodegenerative disease). The compound of the invention can be administered alone or can be coadministered to the patient. Coadministration is meant to include simultaneous or sequential administration of the compound individually or in combination (more than one compound or agent). Thus, the preparations can also be combined, when desired, with other active substances (e.g. to reduce metabolic degradation).

The term "eIF2B" as used herein refers to the heteropentameric eukaryotic translation initiation factor 2B. eIF2B is composed of five subunits: eIF2B1, eIF2B2, eIF2B3, eIF2B4 and eIF2B5. eIF2B1 refers to the protein associated with Entrez gene 1967, OMIM 606686, Uniprot Q14232, and/or RefSeq (protein) NP_001405. eIF2B2 refers to the protein associated with Entrez gene 8892, OMIM 606454, Uniprot P49770, and/or RefSeq (protein) NP_055054. eIF2B3 refers to the protein associated with Entrez gene 8891, OMIM 606273, Uniprot Q9NR50, and/or RefSeq (protein) NP_065098. eIF2B4 refers to the protein associated with Entrez gene 8890, OMIM 606687, Uniprot Q9UI10, and/or RefSeq (protein) NP_751945. eIF2B5 refers to the protein associated with Entrez gene 8893, OMIM 603945, Uniprot Q13144, and/or RefSeq (protein) NP_003898.

The terms "eIF2alpha", "eIF2a"or "eIF2α" are interchangeable and refer to the protein "eukaryotic translation initiation factor 2 alpha subunit eIF2S1". In embodiments, "eIF2alpha", "eIF2a"or "eIF2α" refer to the human protein. Included in the terms eIF2alpha", "eIF2a"or "eIF2α" are the wildtype and mutant forms of the protein. In embodiments, " eIF2alpha", "eIF2a"or "eIF2α" refer to the protein associated with Entrez Gene 1965, OMIM 603907, UniProt P05198, and/or RefSeq (protein) NP_004085. In embodiments, the reference numbers immediately above refer to the protein and associated nucleic acids known as of the date of filing of this application.

### Compounds

In one aspect, the present description features a compound of Formula (I): 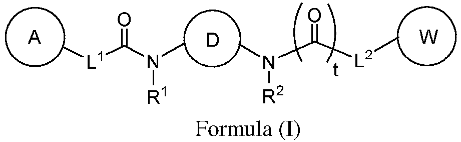 or a pharmaceutically acceptable salt, solvate, hydrate, tautomer, ester, N-oxide or stereoisomer thereof, wherein D is a bridged bicyclic cycloalkyl, bridged bicyclic heterocyclyl, bridged bicyclic cycloalkenyl, or cubanyl, wherein each bridged bicyclic cycloalkyl, bridged bicyclic heterocyclyl, bridged bicyclic cycloalkenyl, or cubanyl is optionally substituted with 1-4 R^{X} groups; and wherein if the bridged bicyclic heterocyclyl contains a substitutable nitrogen moiety, the substitutable nitrogen moiety may be optionally substituted by R^{N1}; L¹ and L² are each independently C₁-C₆ alkylene, C₂-C₆ alkenylene, 2-7-membered heteroalkylene, O, or NR^{C}, wherein each C₁-C₆ alkylene, C₂-C₆ alkenylene, or 2-7-membered heteroalkylene is optionally substituted with 1-5 R^{L}; R¹ and R² are each independently hydrogen, C₁-C₆ alkyl, C₁-C₆ alkoxy-C₂-C₆ alkyl, hydroxy-C₂-C₆ alkyl, silyloxy-C₂-C₆ alkyl, G¹-O-C₂-C₆ alkyl, HO₂C-C₁-C₆ alkyl, or C₁-C₆ alkyl-C(O)₂-C₁-C₆ alkyl; each R^{L} is independently selected from the group consisting of C₁-C₆ alkyl, hydroxy-C₁-C₆ alkyl, hydroxy-C₁-C₆ alkoxy, halo-C₁-C₆ alkyl, amino-C₁-C₆ alkyl, cyano-C₁-C₆ alkyl, C₁-C₆ alkoxy-C₁-C₆ alkyl, HO₂C-C₁-C₆ alkyl, C₁-C₆ alkyl-C(O)₂-C₁-C₆ alkyl, oxo, halo, cyano, -OR^{A}, -NR^{B}R^{C}, -NR^{B}R^{CC}, -NR^{B}C(O)R^{D}, -C(O)NR^{B}R^{C}, -C(O)R^{D}, - C(O)OH, -C(O)OR^{D}, -SR^{E}, -S(O)R^{D}, -S(O)₂R^{D}, -OS(O)R^{D}, -OS(O)₂R^{D}, and G²; or 2 geminal R^{L} groups together with the carbon to which they are attached form a cyclopropyl moiety; R^{N1} is selected from the group consisting of hydrogen, C₁-C₆ alkyl, hydroxy-C₂-C₆ alkyl, halo-C₂-C₆ alkyl, amino-C₂-C₆ alkyl, cyano-C₂-C₆ alkyl, C₁-C₆ alkoxy-C₁-C₆ alkyl, HO₂C-C₁-C₆ alkyl, C₁-C₆ alkyl-C(O)₂-C₁-C₆ alkyl, phenoxy-C₁-C₆ alkyl (wherein phenoxy is optionally substituted with 1-3 halogens), -C(O)NR^{B}R^{C}, -C(O)R^{D}, -C(O)OR^{D}, and -S(O)₂R^{D}; A and W are each independently aryl or 5-6-membered heteroaryl, wherein each phenyl or 5-6-membered heteroaryl is optionally substituted with 1-5 R^{Y}; each R^{X} is independently selected from the group consisting of C₁-C₆ alkyl, hydroxy-C₁-C₆ alkyl, halo-C₁-C₆ alkyl, amino-C₁-C₆ alkyl, cyano-C₁-C₆ alkyl, C₁-C₆ alkoxy-C₁-C₆ alkyl, C₁-C₆ alkyl-C₁-C₆ alkoxy, C₁-C₆ alkoxy-C₁-C₆ alkoxy, oxo, halo, cyano, -OR^{A}, -NR^{B}R^{C}, -NR^{B}R^{CC}, -NR^{B}C(O)R^{D}, -C(O)NR^{B}R^{C}, -C(O)R^{D}, - C(O)OH, -C(O)OR^{D}, =CHC(O)OR^{D}, =CHC(O)OH, -SR^{E}, -S(O)R^{D}, -S(O)₂R^{D}, -OS(O)R^{D}, - OS(O)₂R^{D}, and G²; or 2 geminal R^{X} groups together with the carbon to which they are attached form an oxirane moiety; each R^{Y} is independently selected from the group consisting of hydrogen, C₁-C₆ alkyl, O-C₃-C₆ cycloalkyl, C₁-C₆ alkoxy-C₁-C₆ alkyl, hydroxy-C₁-C₆ alkyl, hydroxy-C₁-C₆ alkoxy, halo-C₁-C₆ alkyl, halo-C₁-C₆ alkoxy, amino-C₁-C₆ alkyl, cyano-C₁-C₆ alkyl, oxo, halo, cyano, -OR^{A}, -NR^{B}R^{C}, -NR^{B}R^{CC}, -NR^{B}C(O)R^{D}, -C(O)NR^{B}R^{C}, -C(O)R^{D}, - C(O)OH, -C(O)OR^{D}, -S(R^{F})ₘ, -S(O)R^{D}, -S(O)₂R^{D}, and G¹; or 2 R^{Y} groups on adjacent atoms, together with the atoms to which they are attached form a 3-7-membered fused cycloalkyl, heterocyclyl, aryl, or heteroaryl ring optionally substituted with 1-5 R^{X}; each G¹ is independently C₃-C₆ cycloalkyl, 4-7-membered heterocyclyl, aryl, or 5-6-membered heteroaryl, wherein each C₃-C₆ cycloalkyl, 4-7-membered heterocyclyl, aryl, or 5-6-membered heteroaryl is optionally substituted with 1-3 R^{Z}; each R^{Z} is independently selected from the group consisting of C₁-C₆ alkyl, hydroxy-C₁-C₆ alkyl, halo-C₁-C₆ alkyl, halo, cyano, -OR^{A}, -NR^{B}R^{C}, -NR^{B}C(O)R^{D}, - C(O)NR^{B}R^{C}, -C(O)R^{D}, -C(O)OH, -C(O)OR^{D}, and -S(O)₂R^{D}; each R^{A} is independently hydrogen, C₁-C₆ alkyl, halo-C₁-C₆ alkyl, -C(O)NR^{B}R^{C}, -C(O)R^{D}, or -C(O)OR^{D}; each of R^{B} and R^{C} is independently hydrogen, C₁-C₆ alkyl, or halo-C₁-C₆ alkyl; R^{B} and R^{C} together with the atom to which they are attached form a 3-7-membered heterocyclyl ring optionally substituted with 1-3 R^{Z}; each R^{CC} is independently selected from the group consisting of hydroxy-C₁-C₆ alkyl, halo-C₁-C₆ alkyl, HO₂C-C₁-C₆ alkyl, C₁-C₆ alkyl-C(O)₂-C₁-C₆ alkyl, (CO)-C₁-C₆alkyl-OH, (CO)-C₁-C₆alkyl-C₁-C₆alkoxy and 4-6 membered heterocyclyl; wherein the heterocyclyl may optionally be substituted with 1-3 R^{Z}: each R^{D} is independently C₁-C₆ alkyl, 2-7-membered heteroalkyl, hydroxy-C₁-C₆ alkyl, or halo-C₁-C₆ alkyl, wherein each C₁-C₆ alkyl, 2-7-membered heteroalkyl, hydroxy-C₁-C₆ alkyl, or halo-C₁-C₆ alkyl is optionally substituted with 1-5 R^{G}; each R^{E} is independently hydrogen, C₁-C₆ alkyl, or halo-C₁-C₆ alkyl; each R^{F} is independently hydrogen, C₁-C₆ alkyl, or halo; each R^{G} is independently aryl or 5-6 membered heteroaryl, wherein each aryl or 5-6 membered heteroaryl is optionally substituted with 1-5 R^{H}; each R^{H} is independently C₁-C₆ alkyl or halo-C₁-C₆ alkyl; m is 1 when R^{F} is hydrogen or C₁-C₆ alkyl, 3 when R^{F} is C₁-C₆ alkyl, or 5 when R^{F} is halo; t is 0 or 1; and s is 0 or 1.

In some embodiments, D is a bridged bicyclic cycloalkyl, a bridged bicyclic heterocyclyl, or cubanyl, each of which is optionally substituted with 1-4 R^{X} groups. In some embodiments, D is a bridged 5-8 membered bicyclic cycloalkyl, a bridged bicyclic heterocyclyl, or cubanyl, each of which is optionally substituted with 1-4 R^{X} groups. In some embodiments, D is selected from cubane, bicyclo[1.1.1]pentane, bicyclo[2.2.2]octane, bicyclo[2.2.2]oct-2-ene, bicyclo[2.1.1]hexane, bicyclo[3.1.1]heptane, bicyclo[2.2.1]heptane, bicyclo[3.2.1]octane, or 2-azabicyclo[2.2.2]octane, each of which is optionally substituted with 1-4 R^{X} groups. In some embodiments, D is selected from cubane, bicyclo[1.1.1]pentane, bicyclo[2.2.2]octane, bicyclo[2.1.1]hexane, or bicyclo[3.1.1]heptane, each of which is optionally substituted with 1-4 R^{X} groups. In some embodiments, D is selected from: 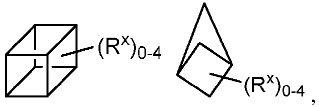 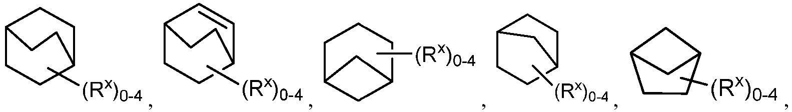 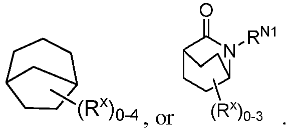 In some embodiments, D is selected from: 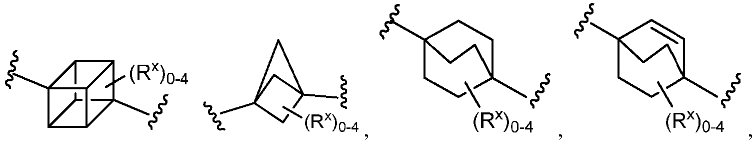 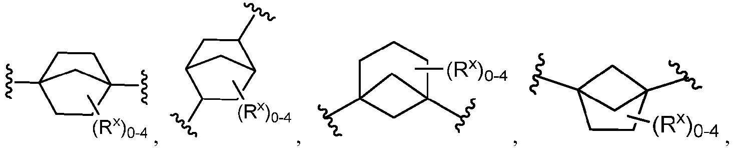 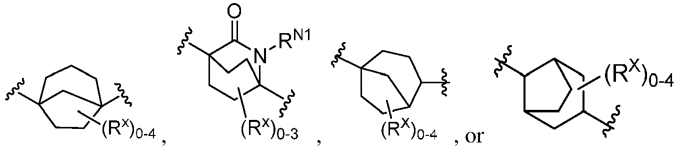 In some embodiments, D is selected from: 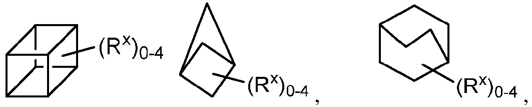 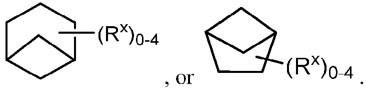 In some embodiments, D is selected from: 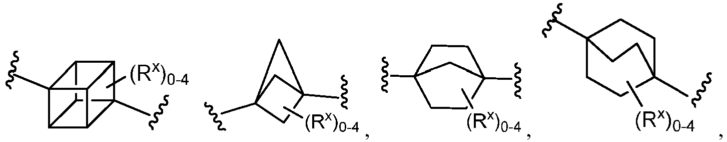 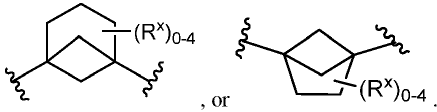 In some embodiments, D is selected from: 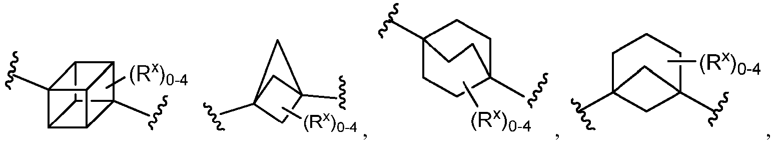 or 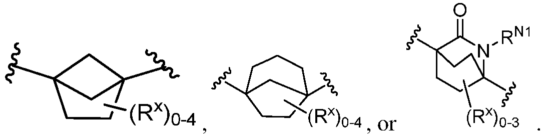 In some embodiments, D is substituted with 1 or 2 R^{X}. In some embodiments, R^{X} is independently C₁-C₆ alkyl, hydroxy-C₁-C₆ alkyl, C₁-C₆ alkoxy-C₁-C₆ alkyl, C₁-C₆ alkoxy-C₁-C₆ alkoxy, oxo, halo, cyano, -OR^{A}, - OS(O)₂R^{D}, -S(O)₂R^{D}, -SR^{E}, NR^{B}C(O)R^{D}, -C(O)NR^{B}R^{C}, -C(O)R^{D}, -C(O)OH, -C(O)OR^{D}, =CHC(O)OH, =CHC(O)OR^{D}, NR^{B}R^{C}, NR^{B}R^{CC}, or G²; or 2 geminal R^{X} groups together with the carbon to which they are attached form an oxirane moiety. In some embodiments, R^{X} is independently CH₃, -CH₂OH, -CH₂OCH₃, -CH₂CH₂CH₃, -C(CH₃)₂OH, -O-CH₂-O-CH₃, CH₂CH₂OH, oxo, fluoro, bromo, OH, cyano, OCH₃, NH₂, N(H)CH₂CF₃, N(H)CH₂CH₂OH, N(CH₃)₂, N(CH₃)CH₂CH₂OH, N(CH₃)CH₂CO₂H, N(CH₃)CH₂CH₂CO₂H, NHC(O)CH₃, OC(O)CH₃, C(O)NH₂, OS(O)₂CH₃, -S(O)₂CH₃, -S(O)₂ CH₂CH₃, C(O)OH, C(O)OCH₃, OC(O)R^{D}, -C(O)CH₃, =CHC(O)OH, =CHC(O)OCH₂CH₃, -SCH₃, 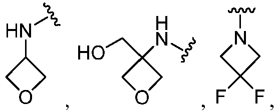 and 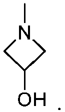 In some embodiments, R^{X} is oxo, -OR^{A}, or NR^{B}R^{C} (*e.g.,* oxo, OH, OCH₃, N(CH₃)₂, or OC(O)R^{D}). In some embodiments, G² is aryl or 5-6 membered heteroaryl. In some embodiments, G² is oxadiazolyl or tetrazolyl.

In some embodiments, D is substituted with 0 R^{X}. In some embodiments, D is

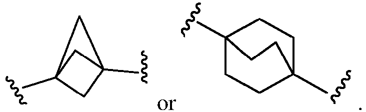

In some embodiments, at least one of L¹ and L² is independently 2-7-membered heteroalkylene, O, or NR^{C}, wherein heteroalkylene is optionally substituted by 1-5 R^{L}. In some embodiments, at least one of L¹ and L² is independently 2-7-membered heteroalkylene optionally substituted by 1-5 R^{L}. In some embodiments, both L¹ and L² are independently 2-7-membered heteroalkylene optionally substituted by 1-5 R^{L}. In some embodiments, one of L¹ and L² is independently C₁-C₆ alkylene or C₂-C₆ alkenylene and the other of L¹ and L² is independently 2-7-membered heteroalkylene, and wherein each C₁-C₆ alkylene, C₂-C₆ alkenylene, and 2-7-membered heteroalkylene is optionally substituted by 1-5 R^{L}. In some embodiments, both of L¹ and L² are C₁-C₆ alkylene or C₂-C₆ alkenylene, and wherein each C₁-C₆ alkylene, and C₂-C₆ alkenylene is optionally substituted by 1-5 R^{L}. In some embodiments, both of L¹ and L² are C₂-C₆ alkenylene, optionally substituted by 1-5 R^{L}.

In some embodiments, each R^{L} is independently C₁-C₆ alkyl, hydroxy-C₁-C₆ alkyl, oxo, or -C(O)R^{D} (*e.g.,* CH₃, OH, oxo, CH₂OH, CH₂OCH₃, or C(O)CH₃). In some embodiments, each R^{L} is independently C₁-C₆ alkyl, OH, oxo, -C(O)R^{D}, cyano, HO₂C-C₁-C₆ alkyl, C₁-C₆ alkyl-C(O)₂-C₁-C₆ alkyl, -C(O)OH, -NR^{B}R^{C} -NR^{B}R^{CC}, -NR^{B}C(O)R^{D}, hydroxy-C₁-C₆ alkyl, or hydroxy-C₁-C₆ alkoxy. In some embodiments, each R^{L} is independently CH₃, oxo, or C(O)CH₃.

In some embodiments, each of L¹ and L² is independently selected from CH₂O-*, CH₂CH₂-*, CH₂CH₂CH₂-*, CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, NHCH₂-*, CH₂NH-*, CH₂N(CH₃)-*, CH₂N(CH₃)C(O)-*, CH₂N(C(O)CH₃)-*, CH₂CH(OH)-*, CH(OH)-*, CH(OH)CH₂CH₂-*, CH₂CH(OH)-*, CH₂NHC(O)-*, NHC(O)OCH₂-*, O-*, NH-*, S(O)₂CH-*, S(O)₂CH₂CH₂-*, S(O)₂CH₂CH₂O-*, CH₂C(O)-*, O-CH₂CH₂*, CH₂N(CH₂CO₂H)-*, CH(CO₂H)CH₂CH₂O-*, CH₂N(CH₂CO₂C(CH₃)₃)-*, CH(CN)CH₂O-*, CH₂CH(NH(CH₃))-*,CH(OCH₂CH₂OH)-*, CH₂CH(NH(C(O)CH₂OH))-*, CH₂CH(NH₂)CH₂*,CH(CH₂CH₂OH)O-* or 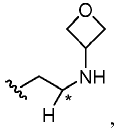 and "-*" indicates the attachment point to A and W, respectively. In some embodiments, each of L¹ and L² is independently selected from CH₂O-*,CH₂CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, CH₂NH-*, CH₂N(CH₃)-*, CH₂N(CH₃)C(O)-*, CH₂N(C(O)CH₃)-*, CH₂CH(OH)-*, NHC(O)OCH₂-*, or CH₂C(O)-*, and "-*" indicates the attachment point to A and W. respectively. In some embodiments, L¹ is independently selected from CH₂O-* and CH=CH-*, L² is independently selected from CH₂O-*, CH₂CH₂-*, CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, NHCH₂-*, CH₂NH-*, CH₂N(CH₃)-*, CH₂N(CH₃)C(O)-*, CH₂N(C(O)CH₃)-*, CH₂CH(OH)-*, CH(OH)-*, CH(OH)CH₂CH₂-*, CH₂CH(OH)-*, CH₂NHC(O)-*, -NHC(O)OCH₂-*, O-*, NH-*, S(O)₂CH₂-*, S(O)₂CH₂CH₂-*, S(O)₂CH₂CH₂O-*, or CH₂C(O)-*, and "-*" indicates the attachment point to A and W, respectively. In some embodiments, L¹ is independently selected from CH₂O-*,CH₂CH₂O-* or CH=CH-*, L² is independently selected from CH₂O-*,CH₂CH₂-*, CH₂CH₂CH₂-*, CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, NHCH₂-*, CH₂NH-*, CH₂N(CH₃)-*, CH₂N(CH₃)C(O)-*, CH₂N(C(O)CH₃)-*, CH₂CH(OH)-*, CH(OH)-*, CH(OH)CH₂CH₂-*, CH₂CH(OH)-*, CH₂NHC(O)-*, NHC(O)OCH₂-*, O-*, NH-*, S(O)₂CH-*, S(O)₂CH₂CH₂-*, S(O)₂CH₂CH₂O-*, CH₂C(O)-*, CH₂N(CH₂CO₂H)-*, CH(CO₂H)CH₂CH₂O-*, CH₂N(CH₂CO₂C(CH₃)₃)-*, CH(CN)CH₂O-*, CH₂CH(NH(CH₃))-*,CH(OCH₂CH₂OH)-*, CH₂CH(NH(C(O)CH₂OH))-*, CH₂CH(NH₂)CH₂*,CH(CH₂CH₂OH)O-*, CH₂C(CH₃)₂O*- or 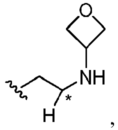 and "-*" indicates the attachment point to A and W, respectively.

In some embodiments, t is 1 and s is 1. In some embodiments, s is 1 and t is 0. In some embodiments, s is 0 and t is 0.

In some embodiments, R¹ and R² are each independently hydrogen, C₁-C₆ alkyl, hydroxyl-C₂-C₆ alkyl, or silyloxy-C₂-C₆ alkyl. In some embodiments, one of R¹ and R² is independently hydrogen and the other of R¹ and R² is independently hydrogen, C₁-C₆ alkyl, C₂-C₆ hydroxyl-C₂-C₆ alkyl, silyloxy-C₂-C₆ alkyl, HO₂C-C₁-C₆ alkyl, or C₁-C₆ alkyl-C(O)₂-C₁-C₆ alkyl. In some embodiments, R¹ and R² are each independently hydrogen, *-CH₃, *-CH₂CH₂OH, *-CH₂CH₂OSi(CH₃)₂C(CH₃)₃, *-CH₂CO₂H, or *-CH₂C(O)₂C(CH₃), and "*-" indicates the attachment point to the nitrogen atom. In some embodiments, one of R¹ and R² is independently hydrogen and the other of R¹ and R² is independently hydrogen, *-CH₃, *-CH₂CH₂OH, or *-CH₂CH₂OSi(CH₃)₂C(CH₃)₃, and "*-" indicates the attachment point to the nitrogen atom. one of R¹ and R² is independently hydrogen and the other of R¹ and R² is independently hydrogen, *-CH₃, *-CH₂CH₂OH, *-CH₂CH₂OSi(CH₃)₂C(CH₃)₃, *-CH₂CO₂H, or *-CH₂C(O)₂C(CH₃), and "*-" indicates the attachment point to the nitrogen atom. In some embodiments, R¹ and R² are each independently hydrogen.

In some embodiments, A is phenyl and W is independently phenyl or 5-6-membered heteroaryl. In some embodiments, each A and W is independently phenyl. In some embodiments, A is phenyl and W is 5-6-membered heteroaryl.

In some embodiments, W is a monocyclic 5-6-membered heteroaryl. In some embodiments, 2 R^{Y} groups on adjacent atoms of W, together with the atoms to which they are attached form a 3-7-membered fused cycloalkyl or heterocyclyl optionally substituted with 1-5 R^{X} forming a bicyclic heteroaryl. In some embodiments, W is a 10-membered heteroaryl, a 9-membered heteroaryl, a 6-membered heteroaryl, or a 5-membered heteroaryl. In some embodiments, W is a heteroaryl containing nitrogen, oxygen or sulfur as allowed by valence.

In some embodiments, each A and W is independently phenyl or 5-6-membered heteroaryl optionally substituted with 1-5 R^{Y}, and each R^{Y} is independently C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, halo, cyano, -OR^{A}, -NR^{B}R^{C}, -C(O)R^{D}, -C(O)OH, -C(O)OR^{D}, -S(R^{F})ₘ,-S(O)₂R^{D}, or G¹. In some embodiments, each of A and W is independently phenyl, pyridyl, pyrazinyl, pyridazinyl, pyridazinonyl, triazinyl, pyrazolyl, pyrimidinyl, triazolyl, oxadiazolyl, oxadiazolonyl, thiazolyl, imidazolyl, pyrimidin-2(1H)-onyl, 1H-benzo[d]imidazolyl, pyrazolo[1,5-a]pyridine, 1H-indazolyl or isoxazolyl, each of which is optionally substituted with 1-5 R^{Y} groups. In some embodiments, each of A and W is independently phenyl, pyridyl, pyrazinyl, pyridazinyl, pyridazinonyl, oxadiazolyl, or oxadiazolonyl, each of which is optionally substituted with 1-5 R^{Y} groups. In some embodiments, each of A and W is independently selected from: 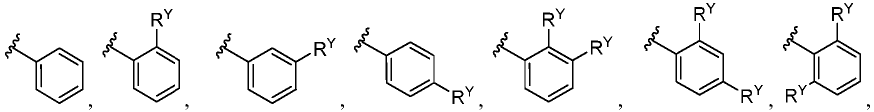 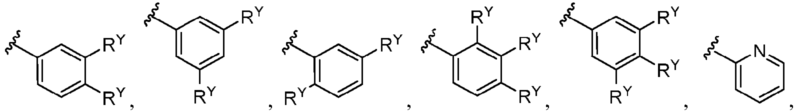 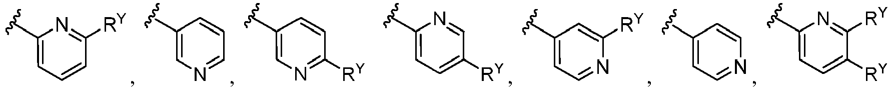 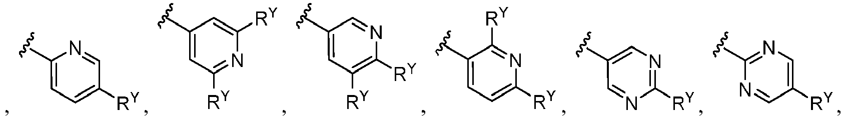 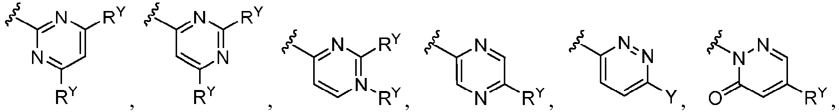 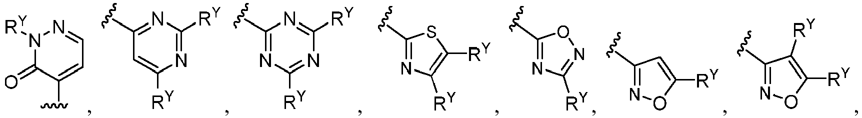 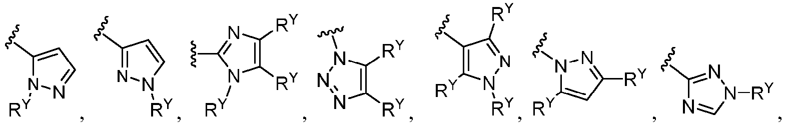 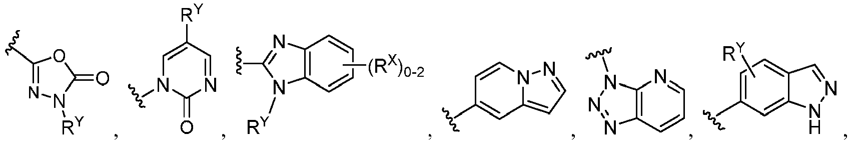 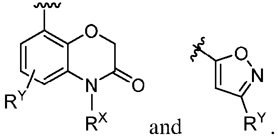 In some embodiments, each of A and W is independently selected from: 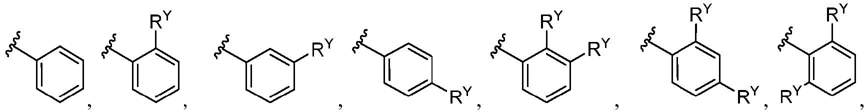 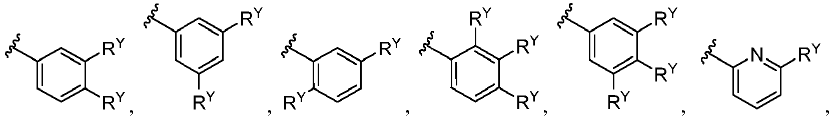 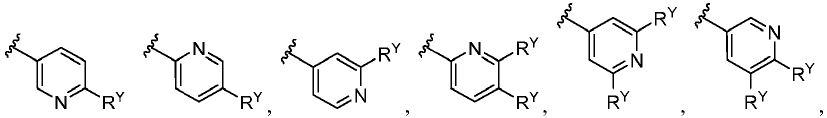 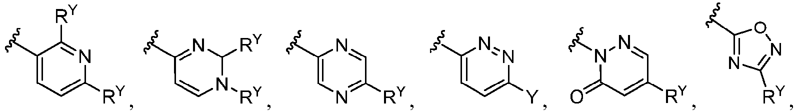 and 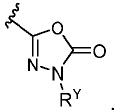 In some embodiments, A is phenyl and W is phenyl or 5-6-membered heteroaryl, each of A and W is optionally substituted with 1-5 R^{Y}, and each R^{Y} is independently C₁-C₆ alkyl, O-C₃-C₆ cycloalkyl, C₁-C₆ alkoxy-C₁-C₆ alkyl, hydroxy-C₁-C₆ alkyl, halo-C₁-C₆ alkyl, halo-C₁-C₆ alkoxy, hydroxy-C₁-C₆ alkoxy, amino-C₁-C₆ alkyl, cyano-C₁-C₆ alkyl, halo, cyano, -OR^{A}, - NR^{B}R^{C}, -NR^{B}R^{CC}, -NR^{B}C(O)R^{D}, -C(O)R^{D}, -C(O)OH, -C(O)OR^{D}, -S(R^{F})ₘ,-S(O)₂R^{D}, or G¹. In some embodiments, A is phenyl and W is phenyl, pyridyl, pyrazinyl, pyridazinyl, pyridazinonyl, pyrimidinyl, triazinyl, pyrazolyl, triazolyl, oxadiazolyl, oxadiazolonyl, thiazolyl, imidazolyl, or isoxazolyl, each of which is optionally substituted with 1-5 R^{Y} groups.

In some embodiments, A is selected from:

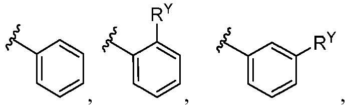

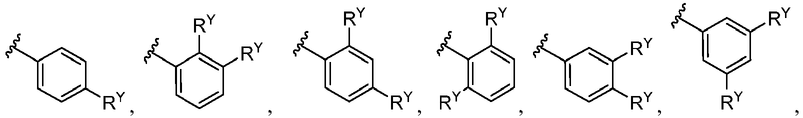

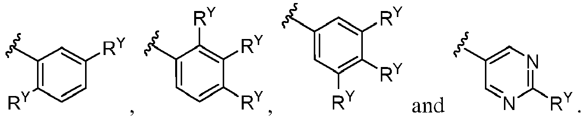

In some embodiments, W is selected from:

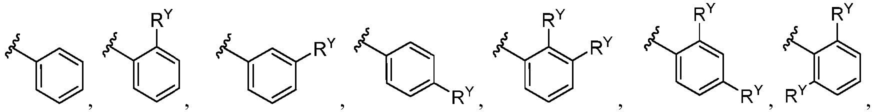

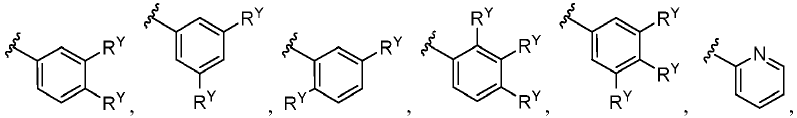

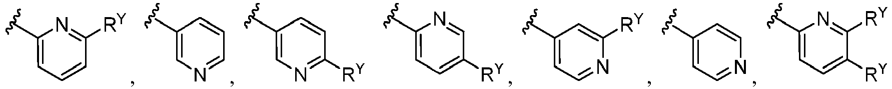

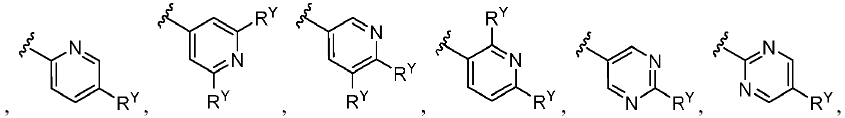

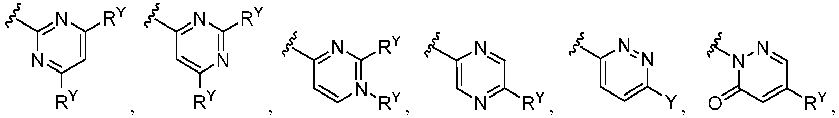

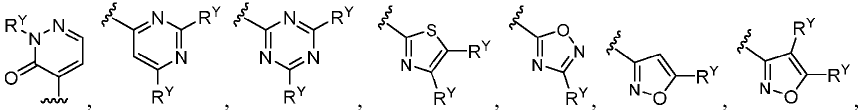

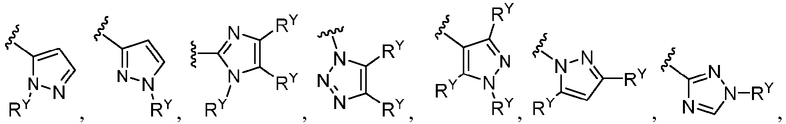

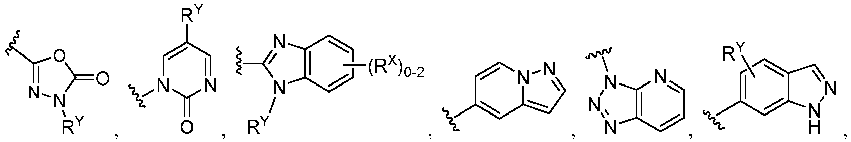

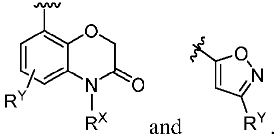

In some embodiments, A is phenyl and W is phenyl or 5-6-membered heteroaryl. In some embodiments, each of A and W is optionally substituted with 1-5 R^{Y}, and each R^{Y} is independently C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, halo, cyano, -OR^{A}, NR^{B}R^{C}, -C(O)R^{D}, -C(O)OH, -C(O)OR^{D},-S(R^{F})ₘ,-S(O)₂R^{D}, or G¹.

In some embodiments, each R^{Y} is independently hydrogen, bromo, chloro, fluoro, iodo, cyano, CF₃, CHF₂, CH₂CF₃, CH₃, CH₂CH₃, OH, CH₂OH, C(CH₃)₂OH, OCH₃, OCH₂CH₃, OCHF₂, OCF₃, OCH₂CF₃, OCH₂CH₂OH, CH₂OCH₃, S(O)₂CH₃, S(O)₂CH₂CH₂CH₃, CN, N(CH₃)₂, SF₅, SCH₃, NH₂, NH(C(O)CH₂OH), NH(CH₂CH₂OH), NH(CO)CH₂OCH₃, C(CH)₃, CH(CH₃)₂, CH₂CN, CH₂NH₂, CH(OH)CH₃, C(OH)(CH₃)CF₃, S(O)₂CH₃, C(O)CH₃, C(O)OCH₃, C(O)OH, OCHF₂, G¹, or

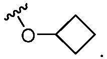

In some embodiments, each R^{Y} is independently chloro, fluoro, iodo, CF₃, CH₃, CH₂CH₃, OCH₃, S(O)₂CH₃, CN, N(CH₃)₂, SF₅, NH₂, C(CH)₃, CH(CH₃)₂, CH₂CN, CH₂NH₂, CH(OH)CH₃, C(O)CH₃, C(O)OCH₃, C(O)OH, OCHF₂ or G¹.

In some embodiments, each A and W is independently substituted with 2 R^{Y} on adjacent atoms, and the 2 R^{Y}, together with the atoms to which they are attached, form a 3-7-membered fused cycloalkyl, 3-7-membered fused heterocyclyl, fused aryl, or 5-6-membered fused heteroaryl ring optionally substituted with 1-5 R^{X}. In some embodiments, the 2 R^{Y} together with the atoms to which they are attached form a phenyl, pyridyl, pyrazolyl, pyrrolyl, isoxazolyl, thiophenyl, furanyl, dioxanyl, dioxolanyl, pyrrolidin-2-onyl, or morpholin-3-onyl ring, each of which is optionally substituted with 1-5 R^{X}. In some embodiments, each R^{X} is independently C₁-C₆ alkyl, OR^{A}, or halo. (*e.g.,* CH₃ or fluoro). In some embodiments, each R^{X} is independently CH₃, OHor fluoro.

In some embodiments, G¹ is cyclopropyl, isoxazolyl, piperidinyl, phenyl, or pyrazolyl, each of which is optionally substituted with 1-5 R^{Z}. In some embodiments, G¹ is cyclopropyl, isoxazolyl, or pyrazolyl, each of which is optionally substituted with 1-5 R^{Z}. In some embodiments, each R^{Z} is independently C₁-C₆ alkyl or halo. In some embodiments, each R^{Z} is independently C₁-C₆ alkyl (e.g., CH₃). In another aspect, the present description features a compound of Formula (I-a): 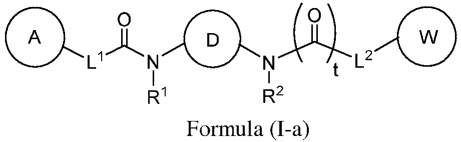 or a pharmaceutically acceptable salt, solvate, hydrate, tautomer, ester, N-oxide or stereoisomer thereof, wherein D is a bridged bicyclic cycloalkyl, bridged bicyclic heterocyclyl, or cubanyl, wherein each bridged bicyclic cycloalkyl, bridged bicyclic heterocyclyl, or cubanyl is optionally substituted with 1-4 R^{X} groups; L¹ and L² are each independently C₁-C₆ alkylene, C₂-C₆ alkenylene, or 2-7-membered heteroalkylene, wherein each C₁-C₆ alkylene, C₂-C₆ alkenylene, or 2-7-membered heteroalkylene is optionally substituted with 1-5 R^{X}; R¹ and R² are each independently hydrogen, C₁-C₆ alkyl, C₁-C₆ alkoxy-C₂-C₆ alkyl, hydroxy-C₂-C₆ alkyl, silyloxy-C₂-C₆ alkyl; A and W are each independently phenyl or 5-6-membered heteroaryl, wherein each phenyl or 5-6-membered heteroaryl is optionally substituted with 1-5 R^{Y}; each R^{X} is independently selected from the group consisting of C₁-C₆ alkyl, hydroxy-C₁-C₆ alkyl, halo-C₁-C₆ alkyl, amino-C₁-C₆ alkyl, cyano-C₁-C₆ alkyl, oxo, halo, cyano, -OR^{A}, NR^{B}R^{C}, - NR^{B}C(O)R^{D}, -C(O)NR^{B}R^{C}, -C(O)R^{D}, -C(O)OH, -C(O)OR^{D}, -SR^{E}, -S(O)R^{D}, and -S(O)₂R^{D}; each R^{Y} is independently selected from the group consisting of hydrogen, C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, oxo, halo, cyano, -OR^{A}, -NR^{B}R^{C}, -NR^{B}C(O)R^{D}, -C(O)NR^{B}R^{C}, -C(O)R^{D}, -C(O)OH, -C(O)OR^{D}, - S(R^{F})ₘ, -S(O)R^{D}, -S(O)₂R^{D}, and G¹; or 2 R^{Y} groups on adjacent atoms, together with the atoms to which they are attached form a 3-7-membered fused cycloalkyl, heterocyclyl, aryl, or heteroaryl ring optionally substituted with 1-5 R^{X}; each G¹ is independently C₃-C₆ cycloalkyl, 4-7-membered heterocyclyl, aryl, or 5-6-membered heteroaryl, wherein each C₃-C₆ cycloalkyl, 4-7-membered heterocyclyl, aryl, or 5-6-membered heteroaryl is optionally substituted with 1-3 R^{Z}; each R^{Z} is independently selected from the group consisting of C₁-C₆ alkyl, hydroxy-C₁-C₆ alkyl, halo-C₁-C₆ alkyl, halo, cyano, -OR^{A}, -NR^{B}R^{C}, -NR^{B}C(O)R^{D}, -C(O)NR^{B}R^{C}, -C(O)R^{D}, - C(O)OH, -C(O)OR^{D}, and -S(O)₂R^{D}; each R^{A} is independently hydrogen, C₁-C₆ alkyl, halo-C₁-C₆ alkyl, -C(O)NR^{B}R^{C}, -C(O)R^{D}, -C(O)OH, or -C(O)OR^{D}; each of R^{B} and R^{C} is independently hydrogen or C₁-C₆ alkyl; or R^{B} and R^{C} together with the atom to which they are attached form a 3-7-membered heterocyclyl ring optionally substituted with 1-3 R^{Z}; each R^{D} is independently C₁-C₆ alkyl, 2-7-membered heteroalkyl, or halo-C₁-C₆ alkyl, wherein each C₁-C₆ alkyl, 2-7-membered heteroalkyl, or halo-C₁-C₆ alkyl is optionally substituted with 1-5 R^{G}; each R^{E} is independently hydrogen, C₁-C₆ alkyl, or halo-C₁-C₆ alkyl; each R^{F} is independently hydrogen, C₁-C₆ alkyl, or halo; each R^{G} is independently aryl or 5-6 membered heteroaryl, wherein each aryl or 5-6 membered heteroaryl is optionally substituted with 1-5 R^{H}; each R^{H} is independently C₁-C₆ alkyl or halo-C₁-C₆ alkyl; m is 1 when R^{F} is hydrogen or C₁-C₆ alkyl, 3 when R^{F} is C₁-C₆ alkyl, or 5 when R^{F} is halo; and; and t is 0 or 1.

In some embodiments, D is a bridged bicyclic cycloalkyl or cubanyl, each of which is optionally substituted with 1-4 R^{X} groups. In some embodiments, D is a bridged 5-8 membered bicyclic cycloalkyl or cubanyl, each of which is optionally substituted with 1-4 R^{X} groups. In some embodiments, D is selected from cubane, bicyclo[1.1.1]pentane, bicyclo[2.2.2]octane, bicyclo[2.1.1]hexane, or bicyclo[3.1.1]heptane, each of which is optionally substituted with 1-4 R^{X} groups. In some embodiments, D is selected from: 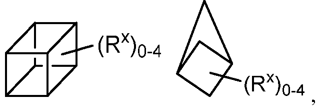 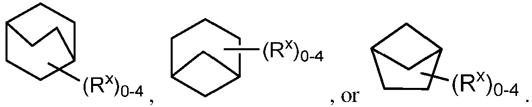 In some embodiments, D is selected from: 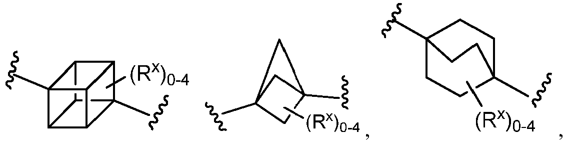 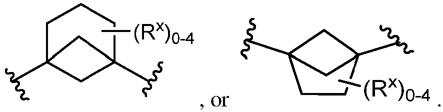 In some embodiments, D is substituted with 1 R^{X}. In some embodiments, R^{X} is oxo, -OR^{A}, or NR^{B}R^{C} (*e.g.,* oxo, OH, OCH₃, N(CH₃)₂, or OC(O)R^{D}). In some embodiments, D is substituted with 0 R^{X}. In some embodiments, D is

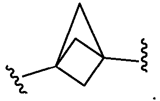

In some embodiments, at least one of L¹ and L² is independently 2-7-membered heteroalkylene optionally substituted by 1-5 R^{X}. In some embodiments, both L¹ and L² are independently 2-7-membered heteroalkylene optionally substituted by 1-5 R^{X}. In some embodiments, one of L¹ and L² is independently C₁-C₆ alkylene or C₂-C₆ alkenylene and the other of L¹ and L² is independently 2-7-membered heteroalkylene, and wherein each C₁-C₆ alkylene, C₂-C₆ alkenylene, and 2-7-membered heteroalkylene is optionally substituted by 1-5 R^{X}. In some embodiments, each R^{X} is independently C₁-C₆ alkyl, oxo, or -C(O)R^{D} (*e.g.,* CH₃, oxo, or C(O)CH₃). In some embodiments, each of L¹ and L² is independently selected from CH₂O-*,CH₂CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, CH₂NH-*, CH₂N(CH₃)-*, CH₂N(CH₃)C(O)-*, CH₂N(C(O)CH₃)-*, CH₂CH(OH)-*, NHC(O)OCH₂-*, or CH₂C(O)-*, and "-*" indicates the attachment point to A and W, respectively. In some embodiments, L¹ is CH₂O-*,L² is independently selected from CH₂O-*,CH₂CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, CH₂NH-*, CH₂N(CH₃)-*, CH₂N(CH₃)C(O)-*, CH₂N(C(O)CH₃)-*, CH₂CH(OH)-*, NHC(O)OCH₂-*, or CH₂C(O)-*, and "-*" indicates the attachment point to A and W. respectively.

In some embodiments, t is 1. In some embodiments, t is 0.

In some embodiments, R¹ and R² are each independently hydrogen, C₁-C₆ alkyl, hydroxyl-C₂-C₆ alkyl, or silyloxy-C₂-C₆ alkyl. In some embodiments, one of R¹ and R² is independently hydrogen and the other of R¹ and R² is independently hydrogen, C₁-C₆ alkyl, hydroxyl-C₂-C₆ alkyl, or silyloxy-C₂-C₆ alkyl. In some embodiments, R¹ and R² are each independently hydrogen, *-CH₃, *-CH₂CH₂OH, or *-CH₂CH₂OSi(CH₃)₂C(CH₃)₃, and "*-" indicates the attachment point to the nitrogen atom. In some embodiments, one of R¹ and R² is independently hydrogen and the other of R¹ and R² is independently hydrogen, *-CH₃, *-CH₂CH₂OH, or *-CH₂CH₂OSi(CH₃)₂C(CH₃)₃, and "*-" indicates the attachment point to the nitrogen atom. In some embodiments, R¹ and R² are each independently hydrogen.

In some embodiments, each A and W is independently a phenyl or 5-6-membered heteroaryl optionally substituted with 1-5 R^{Y} groups, and each R^{Y} is independently C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, halo, cyano, -OR^{A}, -NR^{B}R^{C}, -C(O)R^{D}, -C(O)OH, -C(O)OR^{D}, -S(R^{F})ₘ,-S(O)₂R^{D}, or G¹. In some embodiments, each of A and W is independently phenyl, pyridyl, pyrazinyl, pyridazinyl, pyridazinonyl, oxadiazolyl, or oxadiazolonyl, each of which is optionally substituted with 1-5 R^{Y} groups. In some embodiments, each of A and W is independently selected from: 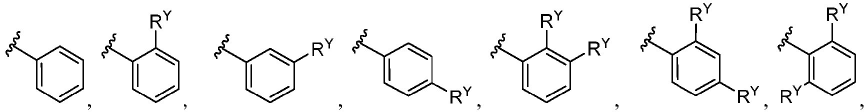 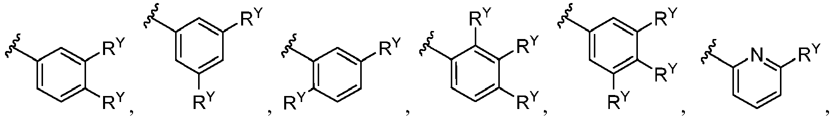 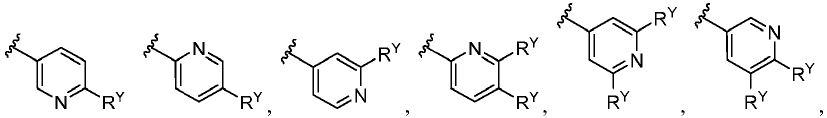 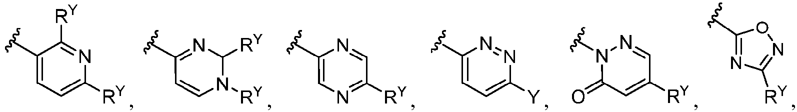 and 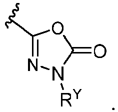 In some embodiments, A is phenyl and W is phenyl or 5-6-membered heteroaryl, each of A and W is optionally substituted with 1-5 R^{Y}, and each R^{Y} is independently C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, halo, cyano, -OR^{A}, -NR^{B}R^{C}, -C(O)R^{D}, -C(O)OH, -C(O)OR^{D}, -S(R^{F})ₘ,-S(O)₂R^{D}, or G¹. In some embodiments, A is phenyl and W is phenyl, pyridyl, pyrazinyl, pyridazinyl, pyridazinonyl, oxadiazolyl, or oxadiazolonyl, each of which is optionally substituted with 1-5 R^{Y}.

In some embodiments, A is selected from: 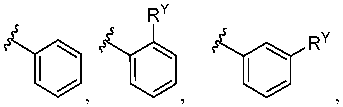 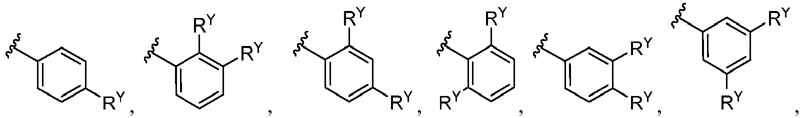 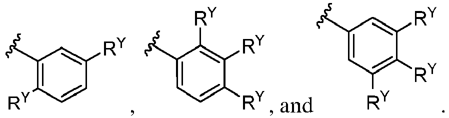 In some embodiments, In some embodiments, W is selected from:

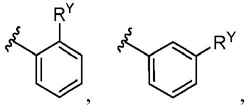

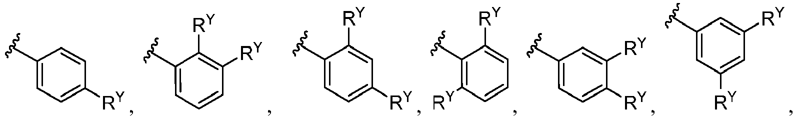

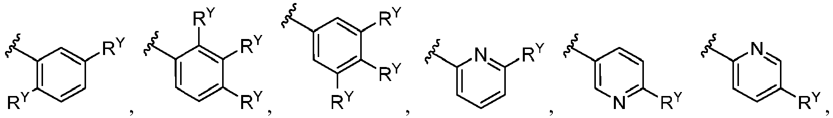

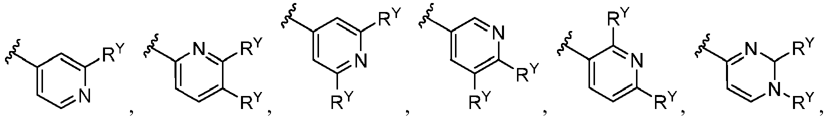

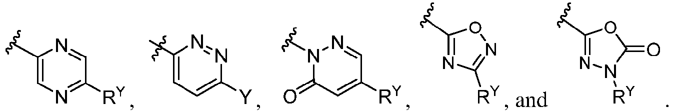

In some embodiments, each R^{Y} is independently chloro, fluoro, iodo, CF₃, CH₃, CH₂CH₃, OCH₃, S(O)₂CH₃, CN, N(CH₃)₂, SF₅, NH₂, C(CH)₃, CH(CH₃)₂, CH₂CN, CH₂NH₂, CH(OH)CH₃, C(O)CH₃, C(O)OCH₃, C(O)OH, OCHF₂ or G¹.

In some embodiments, each A and W is independently substituted with 2 R^{Y} on adjacent atoms, and the 2 R^{Y}, together with the atoms to which they are attached, form a 3-7-membered fused cycloalkyl, 3-7-membered fused heterocyclyl, fused aryl, or 5-6-membered fused heteroaryl ring optionally substituted with 1-5 R^{X}. In some embodiments, 2 R^{Y} together with the atoms to which they are attached form a pyrazolyl, pyrrolyl, isoxazolyl, furanyl, or dioxolanyl ring, each of which is optionally substituted with 1-5 R^{X}. In some embodiments, each R^{X} is independently C₁-C₆ alkyl or halo (*e.g.,* CH₃ or fluoro).

In some embodiments, G¹ is cyclopropyl, isoxazolyl, or pyrazolyl, each of which is optionally substituted with 1-5 R^{Z}. In some embodiments, each R^{Z} is independently C₁-C₆ alkyl (e.g., CH₃).

In some embodiments, the compound of Formula (I) is a compound of Formula (I-b): 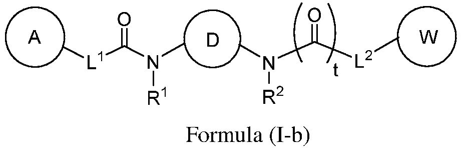
or a pharmaceutically acceptable salt, solvate, hydrate, tautomer, ester, N-oxide or
stereoisomer thereof,
wherein D is (1,2,3,4,6,7)-cubane, bicyclo[1.1.1]pentane, bicyclo[2.2.2]octane, bicyclo[2.1.1]hexane, bicyclo[2.2.1]heptane, bicyclo[3.1.1]heptane, bicyclo[3.2.1]octane, or 2-azabicyclo[2.2.2]octane, each of which is optionally substituted with 1-4 R^{X} groups; and wherein 2-azabicyclo[2.2.2]octane is substituted on nitrogen by hydrogen or CH₃; L¹ and L² are each independently CH₂O-*,CH₂CH₂-*, CH₂CH₂CH₂-*, CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, NHCH₂-*, CH₂NH-*, CH₂N(CH₃)-*, CH₂N(CH₃)C(O)-*, CH₂N(C(O)CH₃)-*, CH₂CH(OH)-*, CH(OH)-*, CH(OH)CH₂CH₂-*, CH₂CH(OH)-*, CH₂NHC(O)-*, NHC(O)OCH₂-*, O-*, NH-*, S(O)₂CH-*, S(O)₂CH₂CH₂-*, S(O)₂CH₂CH₂O-*, or CH₂C(O)-*, and "-*" indicates the attachment point to A and W, respectively; R¹ and R² are each independently hydrogen, CH₃, CH₂CH₂OH, or CH₂CH₂OSi(CH₃)₂C(CH₃)₃; A and W are each independently isoxazolyl, phenyl, pyridyl, pyrazinyl, pyridazinyl, pyridazinonyl, pyrimidinyl, triazinyl, thiazolyl, triazolyl, oxadiazolyl, or oxadiazolonyl, each of which is optionally substituted with 1-5 R^{Y} groups; each R^{X} is independently selected from CH₃, -CH₂OH, -C(CH₃)₂OH, oxo, fluoro, bromo, OH, cyano, OCH₃, NH₂, N(CH₃)₂, NHC(O)CH₃, OC(O)CH₃, C(O)NH₂, OS(O)₂CH₃, -S(O)₂CH₃, -S(O)₂ CH₂CH₃, C(O)OH, OC(O)R^{D}, -C(O)CH₃, -SCH₃, or G²; each R^{Y} is independently bromo, chloro, fluoro, iodo, CF₃, CHF₂, CH₂CF₃, CH₃, CH₂CH₃, OH, CH₂OH, C(CH₃)₂OH, OCH₃, OCH₂CH₃, OCF₃, S(O)₂CH₃, S(O)₂CH₂CH₂CH₃, CN, N(CH₃)₂, SF₅, SCH₃, NH₂, C(CH)₃, CH(CH₃)₂, CH₂CN, CH₂NH₂, CH(OH)CH₃, C(OH)(CH₃)CF₃, S(O)₂CH₃, C(O)CH₃, C(O)OCH₃, C(O)OH, OCHF₂ or G¹; or 2 R^{Y} groups on adjacent atoms, together with the atoms to which they are attached form a pyrazolyl, pyrrolyl, isoxazolyl, thiophenyl, furanyl, or dioxolanyl ring, each of which is optionally substituted with 1-2 R^{X}; G¹ and G² are cyclopropyl, isoxazolyl, phenyl, piperidinyl, oxadiazolyl, or tetrazolyl, or pyrazolyl, each of which is optionally substituted with 1-2 R^{Z}; each R^{D} is CH₂O optionally substituted with 1-5 R^{G}; each R^{G} is independently pyridyl optionally substituted with 1-5 R^{H}; each R^{H} is independently CF₃; each R^{Z} is independently CH₃; and t is 0 or 1.

In some embodiments, the compound of Formula (I) is a compound of Formula (I-c): 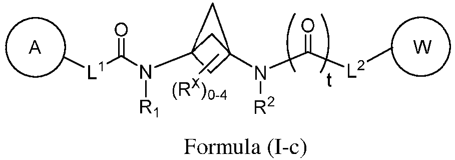 or a pharmaceutically acceptable salt, solvate, hydrate, tautomer, ester, N-oxide or stereoisomer thereof, wherein each of L¹, L², R¹, R², A, W, R^{X}, and t is defined as for Formula (I).

In some embodiments, R^{X} is C₁-C₆ alkyl, oxo, halo, cyano, -OR^{A}, -OS(O)₂R^{D}, - S(O)₂R^{D}, -SR^{E}, NR^{B}C(O)R^{D}, -C(O)NR^{B}R^{C}, -C(O)R^{D}, -C(O)OH, NR^{B}R^{C}, or G² (*e.g.*, CH₃, oxo, fluoro, OH, cyano, OCH₃, NH₂, N(CH₃)₂, NHC(O)CH₃, OC(O)CH₃, C(O)NH₂, OS(O)₂CH₃, - S(O)₂CH₃, -S(O)₂ CH₂CH₃, C(O)OH, OC(O)R^{D}, -C(O)CH₃, or -SCH₃). In some embodiments, R^{X} is oxo, -OR^{A}, or NR^{B}R^{C} (*e.g.*, oxo, OH, OCH₃, N(CH₃)₂, or OC(O)R^{D}). In some embodiments, G² is aryl or 5-6 membered heteroaryl (e.g., oxadiazolyl, or tetrazolyl).

In some embodiments, at least one of L¹ and L² is independently 2-7-membered heteroalkylene, O, or NR^{C}, wherein heteroalkylene is optionally substituted by 1-5 R^{X}. In some embodiments, at least one of L¹ and L² is independently 2-7-membered heteroalkylene optionally substituted by 1-5 R^{X}. In some embodiments, both L¹ and L² are independently 2-7-membered heteroalkylene optionally substituted by 1-5 R^{X}. In some embodiments, one of L¹ and L² is independently C₁-C₆ alkylene or C₂-C₆ alkenylene and the other of L¹ and L² is independently 2-7-membered heteroalkylene, and wherein each C₁-C₆ alkylene, C₂-C₆ alkenylene, and 2-7-membered heteroalkylene is optionally substituted by 1-5 R^{X}. In some embodiments, both of L¹ and L² are C₁-C₆ alkylene or C₂-C₆ alkenylene, and wherein each C₁-C₆ alkylene, and C₂-C₆ alkenylene is optionally substituted by 1-5 R^{X}. In some embodiments, both of L¹ and L² are C₂-C₆ alkenylene, optionally substituted by 1-5 R^{X}.

In some embodiments, R^{X} is C₁-C₆ alkyl, oxo, halo, cyano, -OR^{A}, -OS(O)₂R^{D}, - S(O)₂R^{D}, -SR^{E}, NR^{B}C(O)R^{D}, -C(O)NR^{B}R^{C}, -C(O)R^{D}, -C(O)OH, NR^{B}R^{C}, or G² (*e.g.*, CH₃, oxo, fluoro, OH, cyano, OCH₃, NH₂, N(CH₃)₂, NHC(O)CH₃, OC(O)CH₃, C(O)NH₂, OS(O)₂CH₃, - S(O)₂CH₃, -S(O)₂ CH₂CH₃, C(O)OH, OC(O)R^{D}, -C(O)CH₃, or -SCH₃). In some embodiments, R^{X} is oxo, -OR^{A}, or NR^{B}R^{C} (*e.g.,* oxo, OH, OCH₃, N(CH₃)₂, or OC(O)R^{D}). In some embodiments, G² is aryl or 5-6 membered heteroaryl (e.g., oxadiazolyl, or tetrazolyl).

In some embodiments, each of L¹ and L² is independently selected from CH₂O-*, CH₂CH₂-*, CH₂CH₂CH₂-*, CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, NHCH₂-*, CH₂NH-*, CH₂N(CH₃)-*, CH₂N(CH₃)C(O)-*, CH₂N(C(O)CH₃)-*, CH₂CH(OH)-*, CH(OH)-*, CH(OH)CH₂CH₂-*, CH₂CH(OH)-*, CH₂NHC(O)-*, NHC(O)OCH₂-*, O-*, NH-*, S(O)₂CH-*, S(O)₂CH₂CH₂-*, S(O)₂CH₂CH₂O-*, or CH₂C(O)-*, and "-*" indicates the attachment point to A and W, respectively. In some embodiments, each of L¹ and L² is independently selected from CH₂O-*, CH₂CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, CH₂NH-*, CH₂N(CH₃)-*, CH₂N(CH₃)C(O)-*, CH₂N(C(O)CH₃)-*, CH₂CH(OH)-*, NHC(O)OCH₂-*, or CH₂C(O)-*, and "-*" indicates the attachment point to A and W, respectively. In some embodiments, L¹ is independently selected from CH₂O-* and CH=CH-*, L² is independently selected from CH₂O-*, CH₂CH₂-*, CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, NHCH₂-*, CH₂NH-*, CH₂N(CH₃)-*, CH₂N(CH₃)C(O)-*, CH₂N(C(O)CH₃)-*, CH₂CH(OH)-*, CH(OH)-*, CH(OH)CH₂CH₂-*, CH₂CH(OH)-*, CH₂NHC(O)-*, - NHC(O)OCH₂-*, O-*, NH-*, S(O)₂CH₂-*, S(O)₂CH₂CH₂-*, S(O)₂CH₂CH₂O-*, or CH₂C(O)-*, and "-*" indicates the attachment point to A and W, respectively. In some embodiments, L¹ is CH₂O-*, L² is independently selected from CH₂O-*, CH₂CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, CH₂NH-*, CH₂N(CH₃)-*, CH₂N(CH₃)C(O)-*, CH₂N(C(O)CH₃)-*, CH₂CH(OH)-*, NHC(O)OCH₂-*, or CH₂C(O)-*, and "-*" indicates the attachment point to A and W, respectively.

In some embodiments, t is 1. In some embodiments, t is 0.

In some embodiments, R¹ and R² are each independently hydrogen, C₁-C₆ alkyl, hydroxyl-C₂-C₆ alkyl, or silyloxy-C₂-C₆ alkyl. In some embodiments, one of R¹ and R² is independently hydrogen and the other of R¹ and R² is independently hydrogen, C₁-C₆ alkyl, hydroxyl-C₂-C₆ alkyl, or silyloxy-C₂-C₆ alkyl. In some embodiments, R¹ and R² are each independently hydrogen, *-CH₃, *-CH₂CH₂OH, or *-CH₂CH₂OSi(CH₃)₂C(CH₃)₃, and "*-" indicates the attachment point to the nitrogen atom. In some embodiments, one of R¹ and R² is independently hydrogen and the other of R¹ and R² is independently hydrogen, *-CH₃, *-CH₂CH₂OH, or *-CH₂CH₂OSi(CH₃)₂C(CH₃)₃, and "*-" indicates the attachment point to the nitrogen atom. In some embodiments, R¹ and R² are each independently hydrogen.

In some embodiments, A is phenyl and W is independently phenyl or 5-6-membered heteroaryl. In some embodiments, each A and W is independently phenyl. In some embodiments, A is phenyl and W is 5-6-membered heteroaryl.

In some embodiments, W is a monocyclic 5-6-membered heteroaryl. In some embodiments, 2 R^{Y} groups on adjacent atoms of W, together with the atoms to which they are attached form a 3-7-membered fused cycloalkyl or heterocyclyl optionally substituted with 1-5 R^{X} forming a bicyclic heteroaryl. In some embodiments, W is a 10-membered heteroaryl, a 9-membered heteroaryl, a 6-membered heteroaryl, or a 5-membered heteroaryl. In some embodiments, W is a heteroaryl containing nitrogen, oxygen or sulfur as allowed by valence.

In some embodiments, each A and W is independently phenyl or 5-6-membered heteroaryl optionally substituted with 1-5 R^{Y}, and each R^{Y} is independently C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, halo, cyano, -OR^{A}, -NR^{B}R^{C}, -C(O)R^{D}, -C(O)OH, -C(O)OR^{D}, -S(R^{F})ₘ,-S(O)₂R^{D}, or G¹. In some embodiments, each of A and W is independently phenyl, pyridyl, pyrazinyl, pyridazinyl, pyridazinonyl, triazinyl, triazolyl, oxadiazolyl, or oxadiazolonyl, each of which is optionally substituted with 1-5 R^{Y} groups In some embodiments, each of A and W is independently phenyl, pyridyl, pyrazinyl, pyridazinyl, pyridazinonyl, oxadiazolyl, or oxadiazolonyl, each of which is optionally substituted with 1-5 R^{Y} groups. In some embodiments, each of A and W is independently selected from:

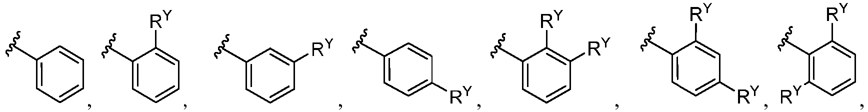

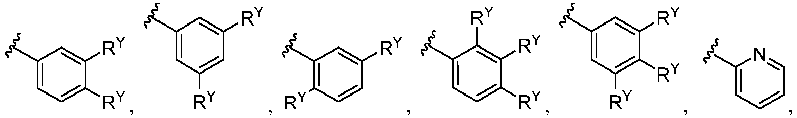

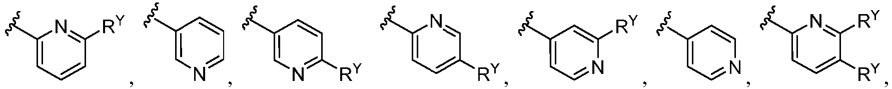

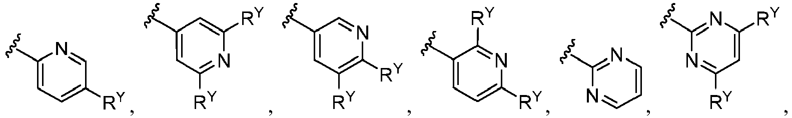

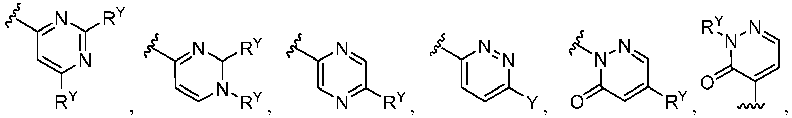

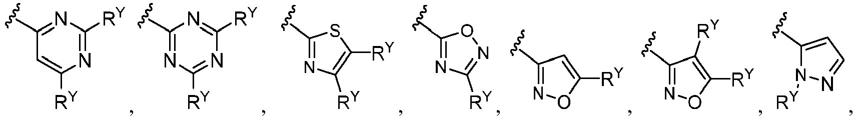

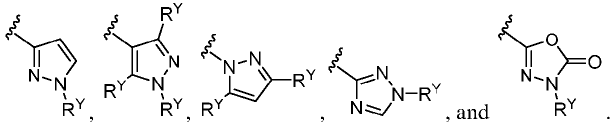

In some embodiments, each of A and W is independently selected from: 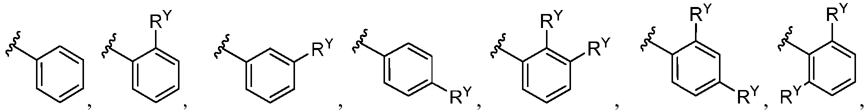 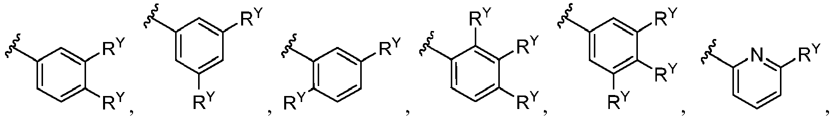 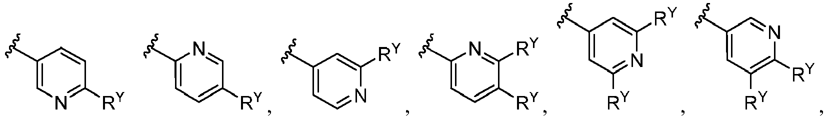 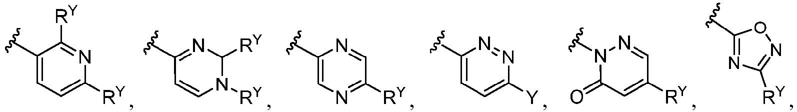 and 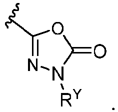 In some embodiments, A is phenyl and W is phenyl or 5-6-membered heteroaryl, each of A and W is optionally substituted with 1-5 R^{Y}, and each R^{Y} is independently C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, halo, cyano, -OR^{A}, -NR^{B}R^{C}, -C(O)R^{D}, -C(O)OH, -C(O)OR^{D}, -S(R^{F})ₘ,-S(O)₂R^{D}, or G¹. In some embodiments, A is phenyl and W is phenyl, pyridyl, pyrazinyl, pyridazinyl, pyridazinonyl, oxadiazolyl, or oxadiazolonyl, each of which is optionally substituted with 1-5 R^{Y}.

In some embodiments, A is selected from:

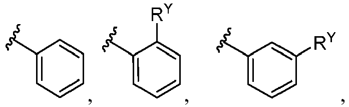

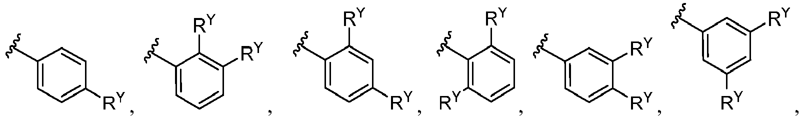

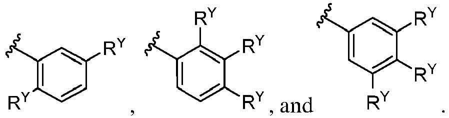

In some embodiments, W is selected from:

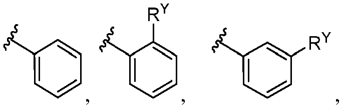

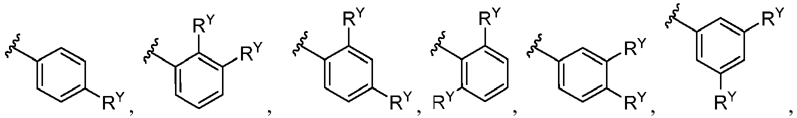

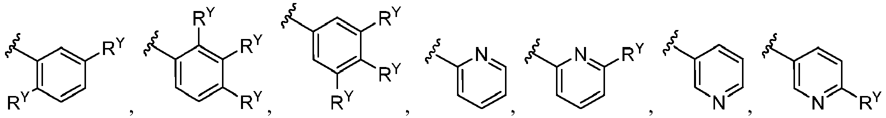

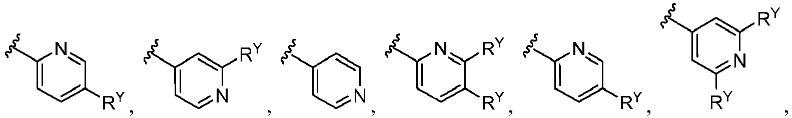

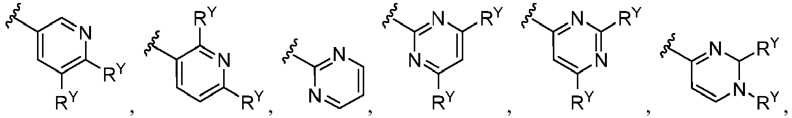

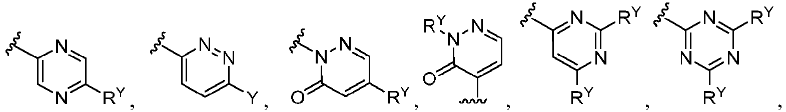

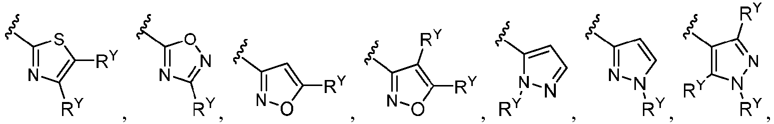

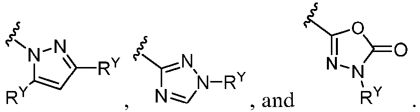

In some embodiments, A is phenyl and W is phenyl or 5-6-membered heteroaryl. In some embodiments, each of A and W is optionally substituted with 1-5 R^{Y}, and each R^{Y} is independently C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, halo, cyano, -OR^{A} NR^{B}R^{C}, -C(O)R^{D}, -C(O)OH, -C(O)OR^{D}, - S(R^{F})ₘ,-S(O)₂R^{D}, or G¹.

In some embodiments, each R^{Y} is independently chloro, fluoro, iodo, CF₃, CHF₂, CH₂CF₃, CH₃, CH₂CH₃, C(CH₃)₂OH, OCH₃, OCH₂CH₃, OCF₃, S(O)₂CH₃, S(O)₂CH₂CH₂CH₃, CN, N(CH₃)₂, SF₅, SCH₃, NH₂, C(CH)₃, CH(CH₃)₂, CH₂CN, CH₂NH₂, CH(OH)CH₃, C(OH)(CH₃)CF₃, S(O)₂CH₃, C(O)CH₃, C(O)OCH₃, C(O)OH, OCHF₂ or G¹.

In some embodiments, each R^{Y} is independently chloro, fluoro, iodo, CF₃, CH₃, CH₂CH₃, OCH₃, S(O)₂CH₃, CN, N(CH₃)₂, SF₅, NH₂, C(CH)₃, CH(CH₃)₂, CH₂CN, CH₂NH₂, CH(OH)CH₃, C(O)CH₃, C(O)OCH₃, C(O)OH, OCHF₂ or G¹.

In some embodiments, each A and W is independently substituted with 2 R^{Y} on adjacent atoms, and the 2 R^{Y}, together with the atoms to which they are attached, form a 3-7-membered fused cycloalkyl, 3-7-membered fused heterocyclyl, fused aryl, or 5-6-membered fused heteroaryl ring optionally substituted with 1-5 R^{X}. In some embodiments, 2 R^{Y} together with the atoms to which they are attached form a pyrazolyl, pyrrolyl, isoxazolyl, thiophenyl, furanyl, or dioxolanyl ring, each of which is optionally substituted with 1-5 R^{X}. In some embodiments, each R^{X} is independently C₁-C₆ alkyl or halo (*e.g.*, CH₃ or fluoro).

In some embodiments, G¹ is cyclopropyl, isoxazolyl, piperidinyl, phenyl, or pyrazolyl, each of which is optionally substituted with 1-5 R^{Z}. In some embodiments, G¹ is cyclopropyl, isoxazolyl, or pyrazolyl, each of which is optionally substituted with 1-5 R^{Z}. In some embodiments, each R^{Z} is independently C₁-C₆ alkyl (e.g., CH₃) or halo (e.g., chloro). In some embodiments, each R^{Z} is independently C₁-C₆ alkyl (e.g., CH₃).

In some embodiments, the compound of Formula (I) is a compound of Formula (I-c): 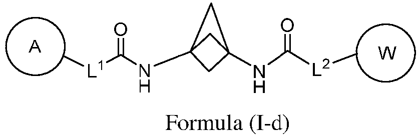 or a pharmaceutically acceptable salt, solvate, hydrate, tautomer, ester, N-oxide or stereoisomer thereof, wherein each of L¹, L², A, and W, is defined as for Formula (I).

In some embodiments, the compound of Formula (I) is a compound of Formula (I-e): 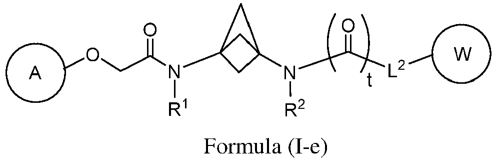 or a pharmaceutically acceptable salt, solvate, hydrate, tautomer, ester, N-oxide or stereoisomer thereof, wherein each of L², A, W, R¹, R² and t is defined as for Formula (I).

In some embodiments, L² is C₁-C₆ alkylene, C₂-C₆ alkenylene, or 2-7-membered heteroalkylene optionally substituted by 1-5 R^{X}. In some embodiments, L² is selected from CH₂O-*, CH₂CH₂-*, CH₂CH₂CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, CH₂NH-*, CH₂N(CH₃)-*, CH₂N(CH₃)C(O)-*, CH₂N(C(O)CH₃)-*, CH₂CH(OH)-*, NHC(O)OCH₂-*, NH-*, S(O)₂CH-*, or CH₂C(O)-*, and "-*" indicates the attachment point to W.

C₁-C₆ alkyl, oxo, halo, cyano, -OR^{A}, -OS(O)₂R^{D}, -S(O)₂R^{D}, -SR^{E}, NR^{B}C(O)R^{D}, - C(O)NR^{B}R^{C}, -C(O)R^{D}, -C(O)OH, NR^{B}R^{C}, or G² (*e.g.*, CH₃, oxo, fluoro, OH, cyano, OCH₃, NH₂, N(CH₃)₂, NHC(O)CH₃, OC(O)CH₃, C(O)NH₂, OS(O)₂CH₃, -S(O)₂CH₃, -S(O)₂ CH₂CH₃, C(O)OH, OC(O)R^{D}, -C(O)CH₃, or -SCH₃).

In some embodiments, L² is selected from CH₂O-*, CH₂CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, CH₂NH-*, CH₂N(CH₃)-*, CH₂N(CH₃)C(O)-*, CH₂N(C(O)CH₃)-*, CH₂CH(OH)-*, NHC(O)OCH₂-*, or CH₂C(O)-*, and "-*" indicates the attachment point to W. In some embodiments, L² is selected from CH₂O-*, CH₂CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, CH₂NH-*, CH₂N(CH₃)-*, CH₂N(CH₃)C(O)-*, CH₂N(C(O)CH₃)-*, CH₂CH(OH)-*, NHC(O)OCH₂-*, or CH₂C(O)-*, and "-*" indicates the attachment point to W.

In some embodiments, t is 1. In some embodiments, t is 0.

In some embodiments, R¹ and R² are each independently hydrogen, C₁-C₆ alkyl, hydroxyl-C₂-C₆ alkyl, or silyloxy-C₂-C₆ alkyl. In some embodiments, one of R¹ and R² is independently hydrogen and the other of R¹ and R² is independently hydrogen, C₁-C₆ alkyl, hydroxyl-C₂-C₆ alkyl, or silyloxy-C₂-C₆ alkyl. In some embodiments, R¹ and R² are each independently hydrogen, *-CH₃, *-CH₂CH₂OH, or *-CH₂CH₂OSi(CH₃)₂C(CH₃)₃, and "*-" indicates the attachment point to the nitrogen atom. In some embodiments, one of R¹ and R² is independently hydrogen and the other of R¹ and R² is independently hydrogen, *-CH₃, *-CH₂CH₂OH, or *-CH₂CH₂OSi(CH₃)₂C(CH₃)₃, and "*-" indicates the attachment point to the nitrogen atom. In some embodiments, R¹ and R² are each independently hydrogen.

In some embodiments, each A and W is independently a phenyl or heteroaryl optionally substituted with 1-5 R^{Y} groups. In some embodiments, each A and W is independently a phenyl or 5-6-membered heteroaryl optionally substituted with 1-5 R^{Y} groups, and each R^{Y} is independently C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, halo, cyano, -OR^{A}, -NR^{B}R^{C}, -C(O)R^{D}, -C(O)OH, -C(O)OR^{D}, - S(R^{F})ₘ,-S(O)₂R^{D}, or G¹. In some embodiments, each of A and W is independently phenyl, pyridyl, pyrazinyl, pyridazinyl, pyridazinonyl, oxadiazolyl, or oxadiazolonyl, each of which is optionally substituted with 1-5 R^{Y} groups. In some embodiments, each of A and W is independently selected from: 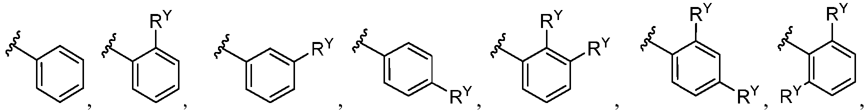 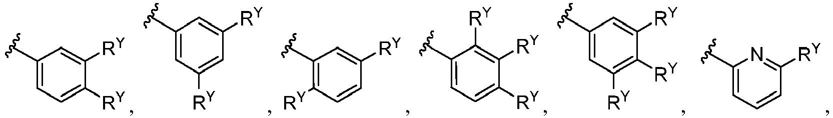 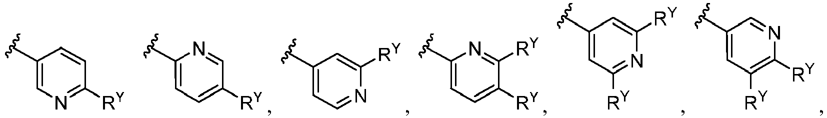 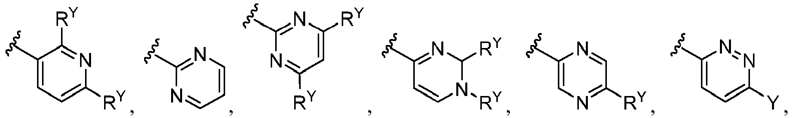 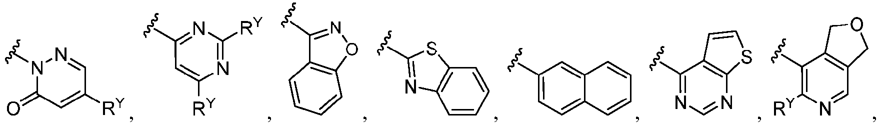 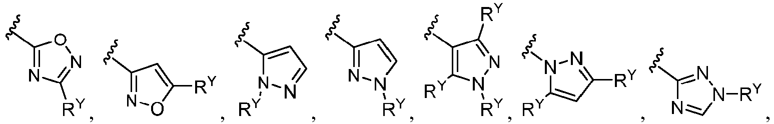 and

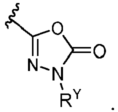

In some embodiments, each of A and W is independently selected from: 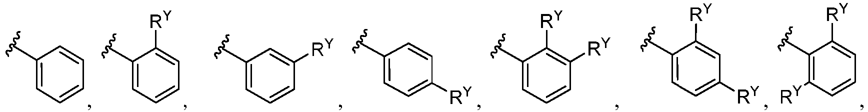 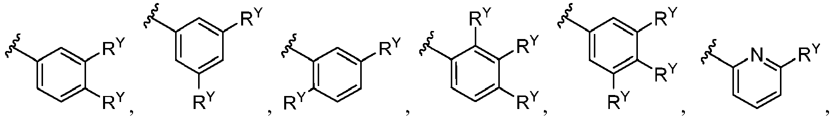 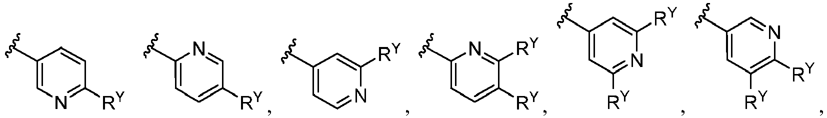 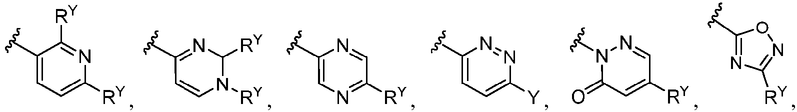 and 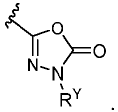 In some embodiments, A is phenyl and W is phenyl or 5-6-membered heteroaryl, each of A and W is optionally substituted with 1-5 R^{Y}, and each R^{Y} is independently C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, halo, cyano, -OR^{A}, -NR^{B}R^{C}, -C(O)R^{D}, -C(O)OH, -C(O)OR^{D}, -S(R^{F})ₘ,-S(O)₂R^{D}, or G¹. In some embodiments, A is phenyl and W is phenyl, pyridyl, pyrazinyl, pyridazinyl, pyridazinonyl, oxadiazolyl, or oxadiazolonyl, each of which is optionally substituted with 1-5 R^{Y}.

In some embodiments, A is selected from:

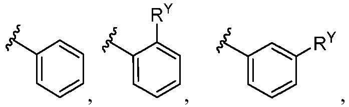

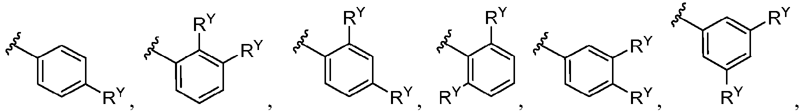

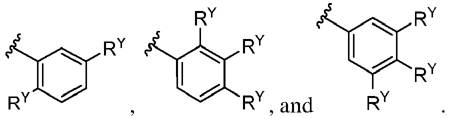

In some embodiments, In some embodiments, W is selected from:

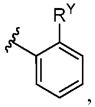

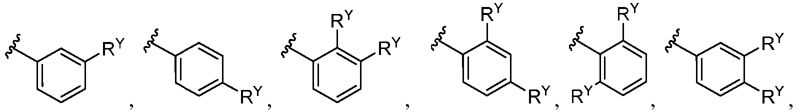

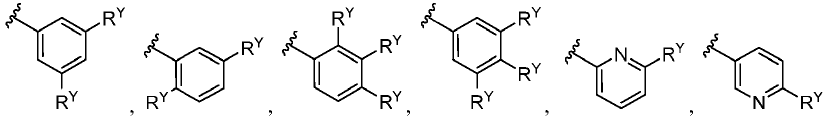

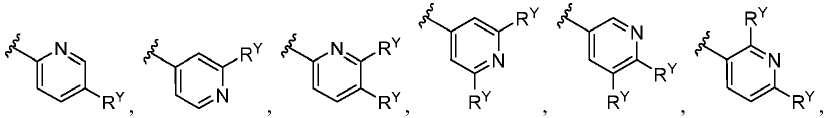

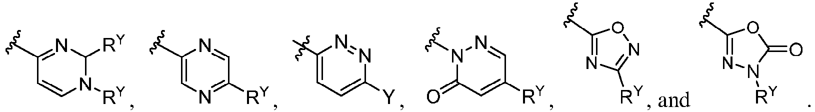

In some embodiments, each R^{Y} is independently chloro, fluoro, iodo, CF₃, CHF₂, CH₂CF₃, CH₃, CH₂CH₃, C(CH₃)₂OH, OCH₃, OCH₂CH₃, OCF₃, S(O)₂CH₃, S(O)₂CH₂CH₂CH₃, CN, N(CH₃)₂, SF₅, SCH₃, NH₂, C(CH)₃, CH(CH₃)₂, CH₂CN, CH₂NH₂, CH(OH)CH₃, C(OH)(CH₃)CF₃, S(O)₂CH₃, C(O)CH₃, C(O)OCH₃, C(O)OH, OCHF₂ or G¹.

In some embodiments, each R^{Y} is independently chloro, fluoro, iodo, CF₃, CH₃, CH₂CH₃, OCH₃, S(O)₂CH₃, CN, N(CH₃)₂, SF₅, NH₂, C(CH)₃, CH(CH₃)₂, CH₂CN, CH₂NH₂, CH(OH)CH₃, C(O)CH₃, C(O)OCH₃, C(O)OH, OCHF₂ or G¹.

In some embodiments, each A and W is independently substituted with 2 R^{Y} on adjacent atoms, and the 2 R^{Y}, together with the atoms to which they are attached, form a 3-7-membered fused cycloalkyl, 3-7-membered fused heterocyclyl, fused aryl, or 5-6-membered fused heteroaryl ring optionally substituted with 1-5 R^{X}. In some embodiments, 2 R^{Y} together with the atoms to which they are attached form a pyrazolyl, pyrrolyl, isoxazolyl, furanyl, or dioxolanyl ring, each of which is optionally substituted with 1-5 R^{X}. In some embodiments, each R^{X} is independently C₁-C₆ alkyl or halo (*e.g.*, CH₃ or fluoro).

In some embodiments, G¹ is cyclopropyl, isoxazolyl, piperidinyl, phenyl, or pyrazolyl, each of which is optionally substituted with 1-5 R^{Z}. In some embodiments, each R^{Z} is independently C₁-C₆ alkyl (e.g., CH₃) or halo (e.g., chloro).

In some embodiments, the compound of Formula (I) is a compound of Formula (I-f): 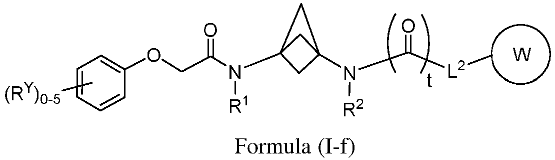 or a pharmaceutically acceptable salt, solvate, hydrate, tautomer, ester, N-oxide or stereoisomer thereof, wherein each of L², W, R^{Y}, R¹, R² and t is defined as for Formula (I).

In some embodiments, L² is C₁-C₆ alkylene, C₂-C₆ alkenylene, or 2-7-membered heteroalkylene optionally substituted by 1-5 R^{X}. In some embodiments, L² is 2-7-membered heteroalkylene, O, or NR^{C}, optionally substituted by 1-5 R^{X}. In some embodiments, L² is selected from CH₂O-*, CH₂CH₂-*, CH₂CH₂CH₂-*, CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, NHCH₂-*, CH₂NH-*, CH₂N(CH₃)-*, CH₂N(CH₃)C(O)-*, CH₂N(C(O)CH₃)-*, CH₂CH(OH)-*, CH(OH)-*, CH(OH)CH₂CH₂-*, CH₂CH(OH)-*, CH₂NHC(O)-*, NHC(O)OCH₂-*, O-*, NH-*, S(O)₂CH-*, S(O)₂CH₂CH₂-*, S(O)₂CH₂CH₂O-*, or CH₂C(O)-*, and "-*" indicates the attachment point to W.

In some embodiments, each R^{X} is independently C₁-C₆ alkyl, oxo, halo, cyano, -OR^{A}, - OS(O)₂R^{D}, -S(O)₂R^{D}, -SR^{E}, NR^{B}C(O)R^{D}, -C(O)NR^{B}R^{C}, -C(O)R^{D}, -C(O)OH, NR^{B}R^{C}, or G² (*e.g.*, CH₃, oxo, fluoro, OH, cyano, OCH₃, NH₂, N(CH₃)₂, NHC(O)CH₃, OC(O)CH₃, C(O)NH₂, OS(O)₂CH₃, -S(O)₂CH₃, -S(O)₂ CH₂CH₃, C(O)OH, OC(O)R^{D}, -C(O)CH₃, or -SCH₃).

In some embodiments, L² is selected from CH₂O-*, CH₂CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, CH₂NH-*, CH₂N(CH₃)-*, CH₂N(CH₃)C(O)-*, CH₂N(C(O)CH₃)-*, CH₂CH(OH)-*, NHC(O)OCH₂-*, or CH₂C(O)-*, and "-*" indicates the attachment point to W. In some embodiments, L² is selected from CH₂O-*, CH₂CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, CH₂NH-*, CH₂N(CH₃)-*, CH₂N(CH₃)C(O)-*, CH₂N(C(O)CH₃)-*, CH₂CH(OH)-*, NHC(O)OCH₂-*, or CH₂C(O)-*, and "-*" indicates the attachment point to W.

In some embodiments, t is 1. In some embodiments, t is 0.

In some embodiments, R¹ and R² are each independently hydrogen, C₁-C₆ alkyl, hydroxyl-C₂-C₆ alkyl, or silyloxy-C₂-C₆ alkyl. In some embodiments, one of R¹ and R² is independently hydrogen and the other of R¹ and R² is independently hydrogen, C₁-C₆ alkyl, hydroxyl-C₂-C₆ alkyl, or silyloxy-C₂-C₆ alkyl. In some embodiments, R¹ and R² are each independently hydrogen, *-CH₃, *-CH₂CH₂OH, or *-CH₂CH₂OSi(CH₃)₂C(CH₃)₃, and "*-" indicates the attachment point to the nitrogen atom. In some embodiments, one of R¹ and R² is independently hydrogen and the other of R¹ and R² is independently hydrogen, *-CH₃, *-CH₂CH₂OH, or *-CH₂CH₂OSi(CH₃)₂C(CH₃)₃, and "*-" indicates the attachment point to the nitrogen atom. In some embodiments, R¹ and R² are each independently hydrogen.

In some embodiments, each A and W is independently phenyl or 5-6-membered heteroaryl optionally substituted with 1-5 R^{Y}, and each R^{Y} is independently C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, halo, cyano, -OR^{A}, -NR^{B}R^{C}, -C(O)R^{D}, -C(O)OH, -C(O)OR^{D}, -S(R^{F})ₘ,-S(O)₂R^{D}, or G¹. In some embodiments, each of A and W is independently phenyl, pyridyl, pyrazinyl, pyridazinyl, pyridazinonyl, triazinyl, triazolyl, oxadiazolyl, or oxadiazolonyl, each of which is optionally substituted with 1-5 R^{Y} groups In some embodiments, each of A and W is independently phenyl, pyridyl, pyrazinyl, pyridazinyl, pyridazinonyl, oxadiazolyl, or oxadiazolonyl, each of which is optionally substituted with 1-5 R^{Y} groups. In some embodiments, each of A and W is independently selected from: 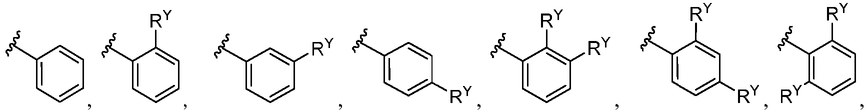 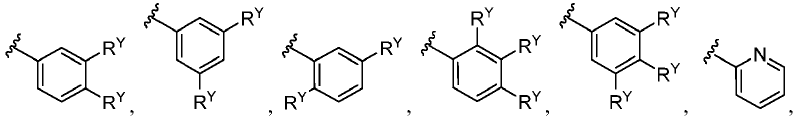 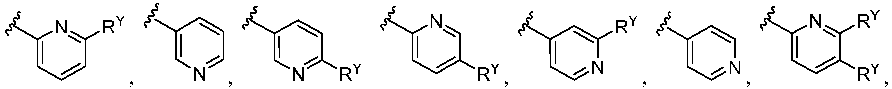 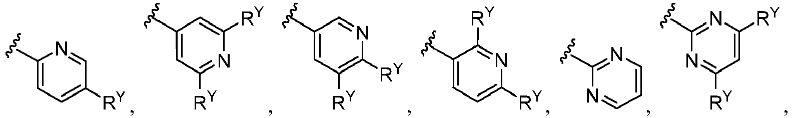 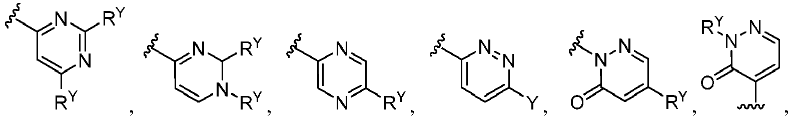 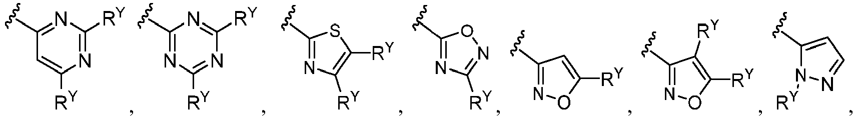 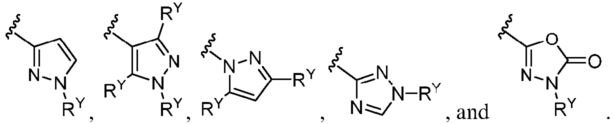 In some embodiments, each of A and W is independently selected from: 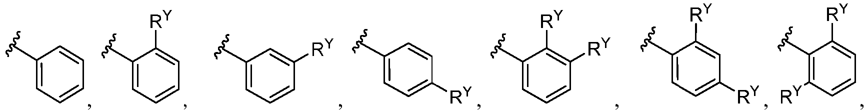 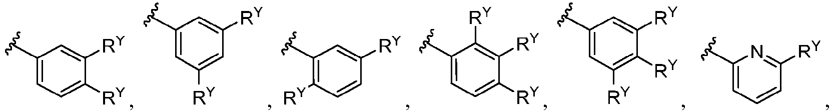 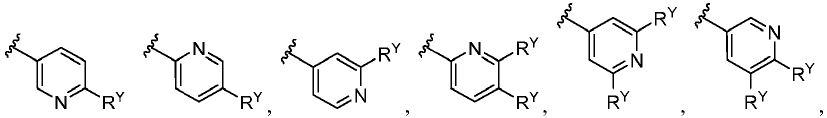 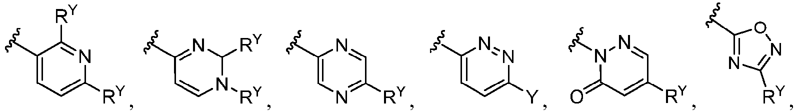 and 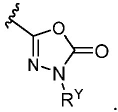 In some embodiments, A is phenyl and W is phenyl or 5-6-membered heteroaryl, each of A and W is optionally substituted with 1-5 R^{Y}, and each R^{Y} is independently C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, halo, cyano, -OR^{A}, -NR^{B}R^{C}, -C(O)R^{D}, -C(O)OH, -C(O)OR^{D}, -S(R^{F})ₘ,-S(O)₂R^{D}, or G¹. In some embodiments, A is phenyl and W is phenyl, pyridyl, pyrazinyl, pyridazinyl, pyridazinonyl, oxadiazolyl, or oxadiazolonyl, each of which is optionally substituted with 1-5 R^{Y}.

In some embodiments, A is selected from:

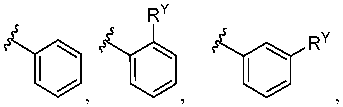

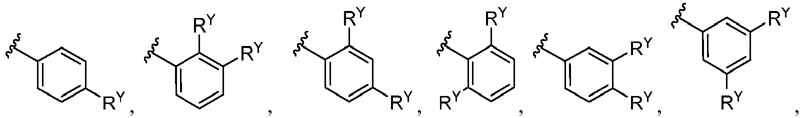

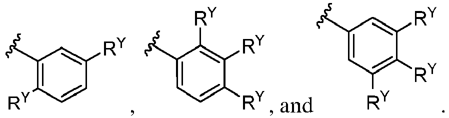

In some embodiments, W is selected from:

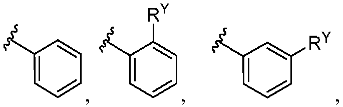

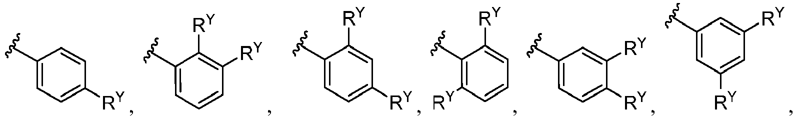

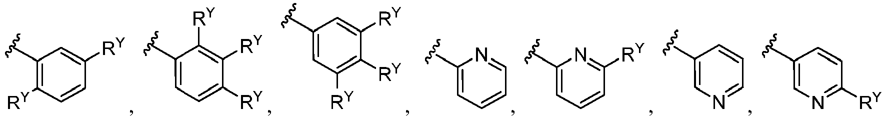

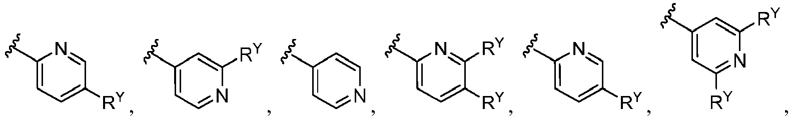

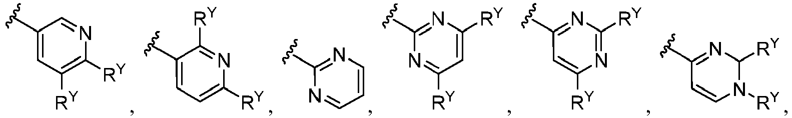

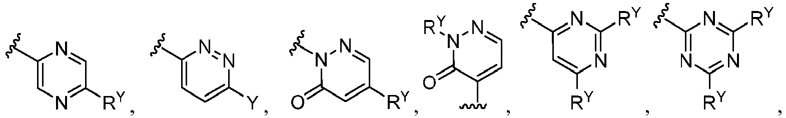

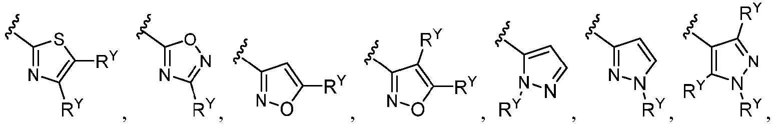

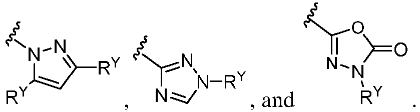

In some embodiments, A is phenyl and W is phenyl or 5-6-membered heteroaryl. In some embodiments, each of A and W is optionally substituted with 1-5 R^{Y}, and each R^{Y} is independently C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, halo, cyano, -OR^{A}, NR^{B}R^{C}, -C(O)R^{D}, -C(O)OH, -C(O)OR^{D}, - S(R^{F})ₘ,-S(O)₂R^{D}, or G¹.

In some embodiments, each R^{Y} is independently chloro, fluoro, iodo, CF₃, CHF₂, CH₂CF₃, CH₃, CH₂CH₃, C(CH₃)₂OH, OCH₃, OCH₂CH₃, OCF₃, S(O)₂CH₃, S(O)₂CH₂CH₂CH₃, CN, N(CH₃)₂, SF₅, SCH₃, NH₂, C(CH)₃, CH(CH₃)₂, CH₂CN, CH₂NH₂, CH(OH)CH₃, C(OH)(CH₃)CF₃, S(O)₂CH₃, C(O)CH₃, C(O)OCH₃, C(O)OH, OCHF₂ or G¹.

In some embodiments, each R^{Y} is independently chloro, fluoro, iodo, CF₃, CH₃, CH₂CH₃, OCH₃, S(O)₂CH₃, CN, N(CH₃)₂, SF₅, NH₂, C(CH)₃, CH(CH₃)₂, CH₂CN, CH₂NH₂, CH(OH)CH₃, C(O)CH₃, C(O)OCH₃, C(O)OH, OCHF₂ or G¹.

In some embodiments, each A and W is independently substituted with 2 R^{Y} on adjacent atoms, and the 2 R^{Y}, together with the atoms to which they are attached, form a 3-7-membered fused cycloalkyl, 3-7-membered fused heterocyclyl, fused aryl, or 5-6-membered fused heteroaryl ring optionally substituted with 1-5 R^{X}. In some embodiments, 2 R^{Y} together with the atoms to which they are attached form a pyrazolyl, pyrrolyl, isoxazolyl, thiophenyl, furanyl, or dioxolanyl ring, each of which is optionally substituted with 1-5 R^{X}. In some embodiments, each R^{X} is independently C₁-C₆ alkyl or halo (*e.g.*, CH₃ or fluoro).

In some embodiments, G¹ is cyclopropyl, isoxazolyl, piperidinyl, phenyl, or pyrazolyl, each of which is optionally substituted with 1-5 R^{Z}. In some embodiments, G¹ is cyclopropyl, isoxazolyl, or pyrazolyl, each of which is optionally substituted with 1-5 R^{Z}. In some embodiments, each R^{Z} is independently C₁-C₆ alkyl (e.g., CH₃) or halo (e.g., chloro). In some embodiments, each R^{Z} is independently C₁-C₆ alkyl (e.g., CH₃).

In some embodiments, the compound of Formula (I) is a compound of Formula (I-g): 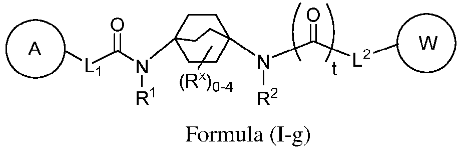 or a pharmaceutically acceptable salt, solvate, hydrate, tautomer, ester, N-oxide or stereoisomer thereof, wherein each of L¹, L², R¹, R², A, W, R^{X}, and t is defined as for Formula (I).

In some embodiments, the compound of Formula (I) is a compound of Formula (I-h): 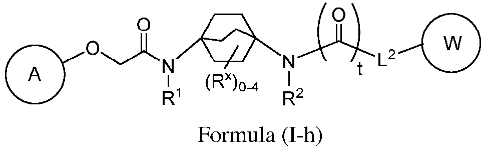 or a pharmaceutically acceptable salt, solvate, hydrate, tautomer, ester, N-oxide or stereoisomer thereof, wherein each of L², R¹, R², A, W, R^{X}, and t is defined as for Formula (I).

In some embodiments, the compound of Formula (I) is a compound of Formula (I-i): 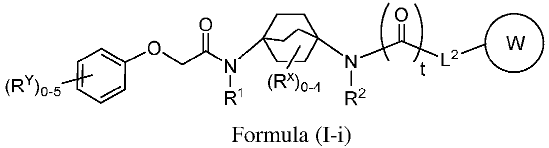 or a pharmaceutically acceptable salt, solvate, hydrate, tautomer, ester, N-oxide or stereoisomer thereof, wherein each of L², R¹, R², W, R^{X}, R^{Y}, and t is defined as for Formula (I).

In some embodiments, L² is C₁-C₆ alkylene, C₂-C₆ alkenylene, or 2-7-membered heteroalkylene optionally substituted by 1-5 R^{X}. In some embodiments, L² is 2-7-membered heteroalkylene, O, or NR^{C}, optionally substituted by 1-5 R^{X}. In some embodiments, L² is selected from CH₂O-*, CH₂CH₂-*, CH₂CH₂CH₂-*, CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, NHCH₂-*, CH₂NH-*, CH₂N(CH₃)-*, CH₂N(CH₃)C(O)-*, CH₂N(C(O)CH₃)-*, CH₂CH(OH)-*, CH(OH)-*, CH(OH)CH₂CH₂-*, CH₂CH(OH)-*, CH₂NHC(O)-*, NHC(O)OCH₂-*, O-*, NH-*, S(O)₂CH-*, S(O)₂CH₂CH₂-*, S(O)₂CH₂CH₂O-*, or CH₂C(O)-*, and "-*" indicates the attachment point to W.

In some embodiments, each R^{X} is independently C₁-C₆ alkyl, oxo, halo, cyano, -OR^{A}, - OS(O)₂R^{D}, -S(O)₂R^{D}, -SR^{E}, NR^{B}C(O)R^{D}, -C(O)NR^{B}R^{C}, -C(O)R^{D}, -C(O)OH, NR^{B}R^{C}, or G² (*e.g.*, CH₃, oxo, fluoro, OH, cyano, OCH₃, NH₂, N(CH₃)₂, NHC(O)CH₃, OC(O)CH₃, C(O)NH₂, OS(O)₂CH₃, -S(O)₂CH₃, -S(O)₂ CH₂CH₃, C(O)OH, OC(O)R^{D}, -C(O)CH₃, or -SCH₃).

In some embodiments, L² is selected from CH₂O-*, CH₂CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, CH₂NH-*, CH₂N(CH₃)-*, CH₂N(CH₃)C(O)-*, CH₂N(C(O)CH₃)-*, CH₂CH(OH)-*, NHC(O)OCH₂-*, or CH₂C(O)-*, and "-*" indicates the attachment point to W. In some embodiments, L² is selected from CH₂O-*, CH₂CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, CH₂NH-*, CH₂N(CH₃)-*, CH₂N(CH₃)C(O)-*, CH₂N(C(O)CH₃)-*, CH₂CH(OH)-*, NHC(O)OCH₂-*, or CH₂C(O)-*, and "-*" indicates the attachment point to W.

In some embodiments, t is 1. In some embodiments, t is 0.

In some embodiments, R¹ and R² are each independently hydrogen, C₁-C₆ alkyl, hydroxyl-C₂-C₆ alkyl, or silyloxy-C₂-C₆ alkyl. In some embodiments, one of R¹ and R² is independently hydrogen and the other of R¹ and R² is independently hydrogen, C₁-C₆ alkyl, hydroxyl-C₂-C₆ alkyl, or silyloxy-C₂-C₆ alkyl. In some embodiments, R¹ and R² are each independently hydrogen, *-CH₃, *-CH₂CH₂OH, or *-CH₂CH₂OSi(CH₃)₂C(CH₃)₃, and "*-" indicates the attachment point to the nitrogen atom. In some embodiments, one of R¹ and R² is independently hydrogen and the other of R¹ and R² is independently hydrogen, *-CH₃, *-CH₂CH₂OH, or *-CH₂CH₂OSi(CH₃)₂C(CH₃)₃, and "*-" indicates the attachment point to the nitrogen atom. In some embodiments, R¹ and R² are each independently hydrogen.

In some embodiments, each A and W is independently phenyl or 5-6-membered heteroaryl optionally substituted with 1-5 R^{Y}, and each R^{Y} is independently C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, halo, cyano, -OR^{A}, -NR^{B}R^{C}, -C(O)R^{D}, -C(O)OH, -C(O)OR^{D}, -S(R^{F})ₘ,-S(O)₂R^{D}, or G¹. In some embodiments, each of A and W is independently phenyl, pyridyl, pyrazinyl, pyridazinyl, pyridazinonyl, triazinyl, triazolyl, oxadiazolyl, or oxadiazolonyl, each of which is optionally substituted with 1-5 R^{Y} groups In some embodiments, each of A and W is independently phenyl, pyridyl, pyrazinyl, pyridazinyl, pyridazinonyl, oxadiazolyl, or oxadiazolonyl, each of which is optionally substituted with 1-5 R^{Y} groups. In some embodiments, each of A and W is independently selected from: 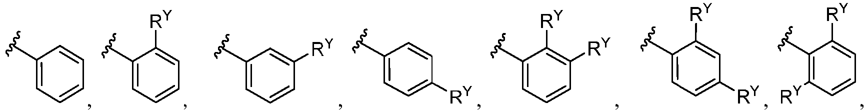 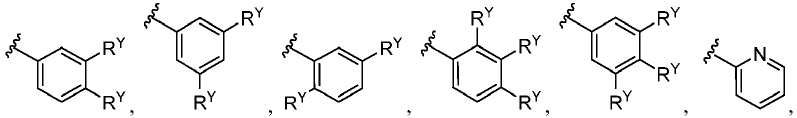 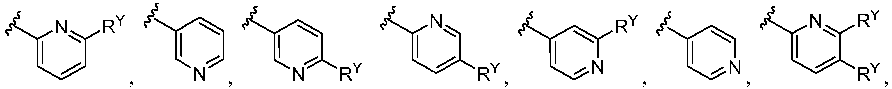 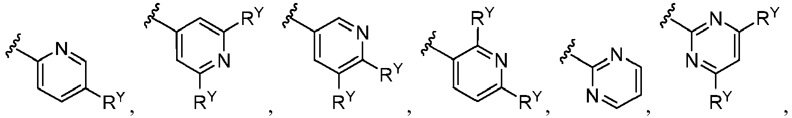 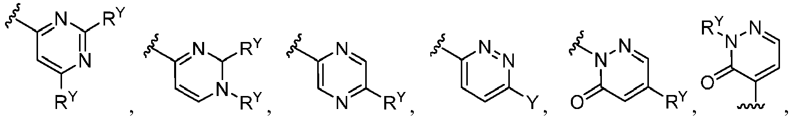 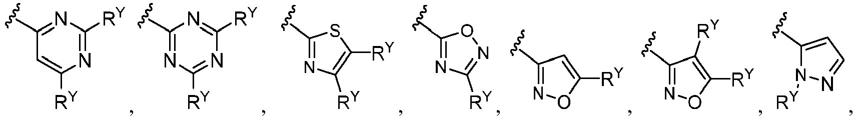 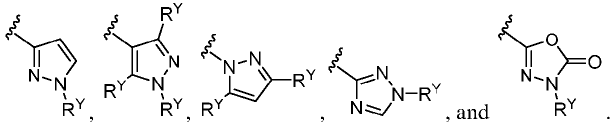 In some embodiments, each of A and W is independently selected from: 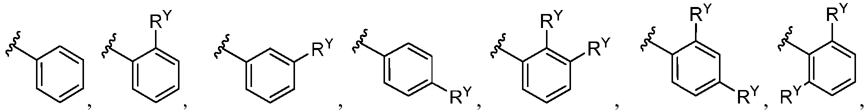 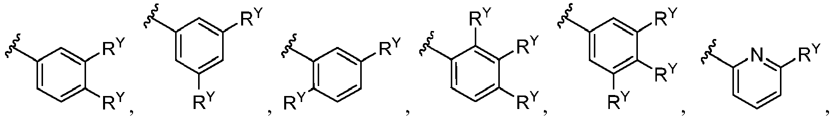 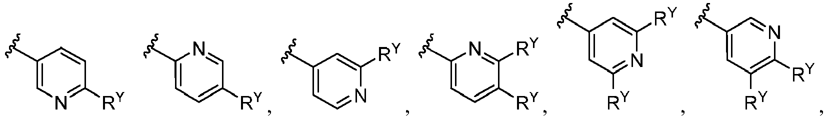 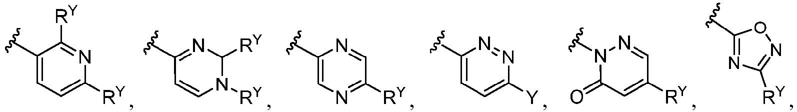 and 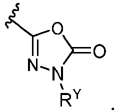 In some embodiments, A is phenyl and W is phenyl or 5-6-membered heteroaryl, each of A and W is optionally substituted with 1-5 R^{Y}, and each R^{Y} is independently C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, halo, cyano, -OR^{A}, -NR^{B}R^{C}, -C(O)R^{D}, -C(O)OH, -C(O)OR^{D}, -S(R^{F})ₘ,-S(O)₂R^{D}, or G¹. In some embodiments, A is phenyl and W is phenyl, pyridyl, pyrazinyl, pyridazinyl, pyridazinonyl, oxadiazolyl, or oxadiazolonyl, each of which is optionally substituted with 1-5 R^{Y}.

In some embodiments, A is selected from:

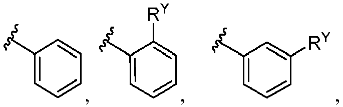

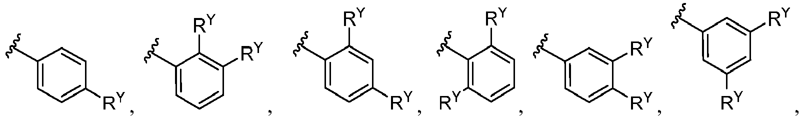

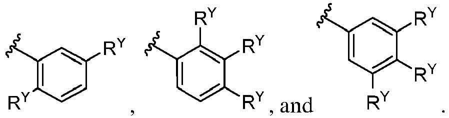

In some embodiments, W is selected from:

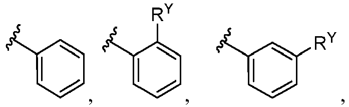

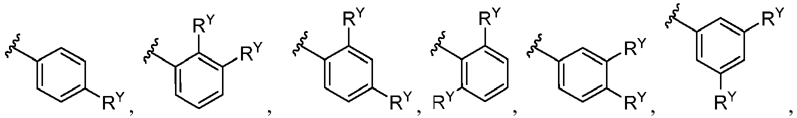

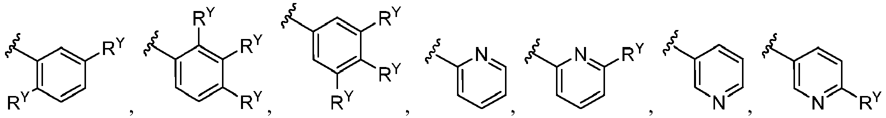

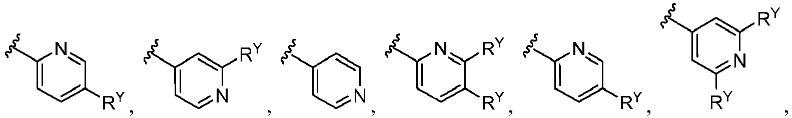

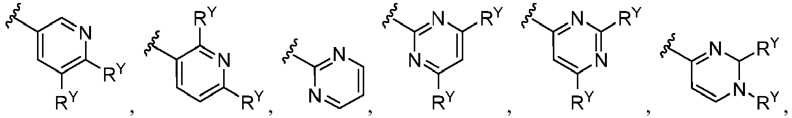

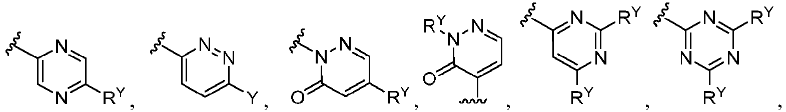

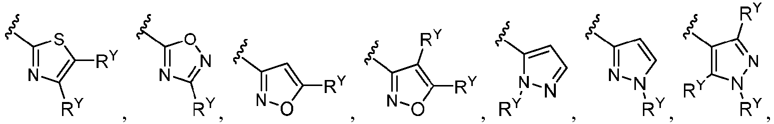

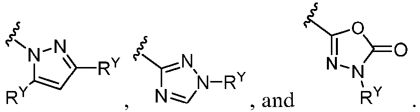

In some embodiments, A is phenyl and W is phenyl or 5-6-membered heteroaryl. In some embodiments, each of A and W is optionally substituted with 1-5 R^{Y}, and each R^{Y} is independently C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, halo, cyano, -OR^{A}, NR^{B}R^{C}, -C(O)R^{D}, -C(O)OH, -C(O)OR^{D}, - S(R^{F})ₘ,-S(O)₂R^{D}, or G¹.

In some embodiments, each R^{Y} is independently chloro, fluoro, iodo, CF₃, CHF₂, CH₂CF₃, CH₃, CH₂CH₃, C(CH₃)₂OH, OCH₃, OCH₂CH₃, OCF₃, S(O)₂CH₃, S(O)₂CH₂CH₂CH₃, CN, N(CH₃)₂, SF₅, SCH₃, NH₂, C(CH)₃, CH(CH₃)₂, CH₂CN, CH₂NH₂, CH(OH)CH₃, C(OH)(CH₃)CF₃, S(O)₂CH₃, C(O)CH₃, C(O)OCH₃, C(O)OH, OCHF₂ or G¹.

In some embodiments, each R^{Y} is independently chloro, fluoro, iodo, CF₃, CH₃, CH₂CH₃, OCH₃, S(O)₂CH₃, CN, N(CH₃)₂, SF₅, NH₂, C(CH)₃, CH(CH₃)₂, CH₂CN, CH₂NH₂, CH(OH)CH₃, C(O)CH₃, C(O)OCH₃, C(O)OH, OCHF₂ or G¹.

In some embodiments, each A and W is independently substituted with 2 R^{Y} on adjacent atoms, and the 2 R^{Y}, together with the atoms to which they are attached, form a 3-7-membered fused cycloalkyl, 3-7-membered fused heterocyclyl, fused aryl, or 5-6-membered fused heteroaryl ring optionally substituted with 1-5 R^{X}. In some embodiments, 2 R^{Y} together with the atoms to which they are attached form a pyrazolyl, pyrrolyl, isoxazolyl, thiophenyl, furanyl, or dioxolanyl ring, each of which is optionally substituted with 1-5 R^{X}. In some embodiments, each R^{X} is independently C₁-C₆ alkyl or halo (*e.g.*, CH₃ or fluoro).

In some embodiments, G¹ is cyclopropyl, isoxazolyl, piperidinyl, phenyl, or pyrazolyl, each of which is optionally substituted with 1-5 R^{Z}. In some embodiments, G¹ is cyclopropyl, isoxazolyl, or pyrazolyl, each of which is optionally substituted with 1-5 R^{Z}. In some embodiments, each R^{Z} is independently C₁-C₆ alkyl (e.g., CH₃) or halo (e.g., chloro). In some embodiments, each R^{Z} is independently C₁-C₆ alkyl (e.g., CH₃).

In some embodiments, the compound of Formula (I) is a compound of Formula (I-j): 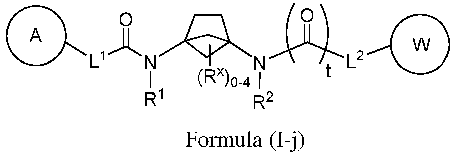 or a pharmaceutically acceptable salt, solvate, hydrate, tautomer, ester, N-oxide or stereoisomer thereof, wherein each of L¹, L², R¹, R², A, W, R^{X}, and t is defined as for Formula (I).

In some embodiments, the compound of Formula (I) is a compound of Formula (I-k): 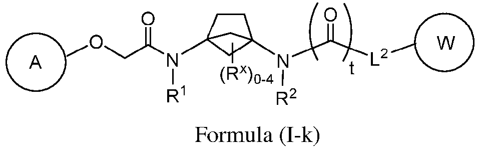 or a pharmaceutically acceptable salt, solvate, hydrate, tautomer, ester, N-oxide or stereoisomer thereof, wherein each of L², R¹, R² A, W, R^{X}, and t is defined as for Formula (I).

In some embodiments, the compound of Formula (I) is a compound of Formula (I-1): 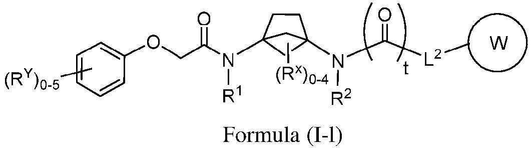 or a pharmaceutically acceptable salt, solvate, hydrate, tautomer, ester, N-oxide or stereoisomer thereof, wherein each of L², R¹, R² W, R^{X}, R^{Y}, and t is defined as for Formula (I).

In some embodiments, at least one of L¹ and L² is independently 2-7-membered heteroalkylene optionally substituted by 1-5 R^{X}. In some embodiments, both L¹ and L² are independently 2-7-membered heteroalkylene optionally substituted by 1-5 R^{X}. In some embodiments, one of L¹ and L² is independently C₁-C₆ alkylene or C₂-C₆ alkenylene and the other of L¹ and L² is independently 2-7-membered heteroalkylene, and wherein each C₁-C₆ alkylene, C₂-C₆ alkenylene, and 2-7-membered heteroalkylene is optionally substituted by 1-5 R^{X}. In some embodiments, each R^{X} is independently C₁-C₆ alkyl, oxo, or -C(O)R^{D} (*e.g.*, CH₃, oxo, or C(O)CH₃). In some embodiments, each of L¹ and L² is independently selected from CH₂-*, CH₂CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, CH₂NH-*, CH₂N(CH₃)-*, CH₂N(CH₃)C(O)-*, CH₂N(C(O)CH₃)-*, CH₂CH(OH)-*, NHC(O)OCH₂-*, or CH₂C(O)-*, and "-*" indicates the attachment point to A and W, respectively. In some embodiments, L¹ is CH₂-*, L² is independently selected from CH₂-*, CH₂CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, CH₂NH-*, CH₂N(CH₃)-*, CH₂N(CH₃)C(O)-*, CH₂N(C(O)CH₃)-*, CH₂CH(OH)-*, NHC(O)OCH₂-*, or CH₂C(O)-*, and "-*" indicates the attachment point to A and W. respectively.

In some embodiments, L² is selected from CH₂-*, CH₂CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, CH₂NH-*, CH₂N(CH₃)-*, CH₂N(CH₃)C(O)-*, CH₂N(C(O)CH₃)-*, CH₂CH(OH)-*, NHC(O)OCH₂-*, or CH₂C(O)-*, and "-*" indicates the attachment point to W. In some embodiments, L² is selected from CH₂-*, CH₂CH₂-*, CH₂C(O)-*, CH=CH-*, CH₂CH₂O-*, CH₂OCH₂-*, CH₂OCH₂CH₂-*, CH₂CH₂CH₂O-*, CH₂CH₂OCH₂-*, CH₂NH-*, CH₂N(CH₃)-*, CH₂N(CH₃)C(O)-*, CH₂N(C(O)CH₃)-*, CH₂CH(OH)-*, NHC(O)OCH₂-*, or CH₂C(O)-*, and "-*" indicates the attachment point to W.

In some embodiments, each R^{X} is independently C₁-C₆ alkyl, oxo, halo, cyano, -OR^{A}, - OS(O)₂R^{D}, -S(O)₂R^{D}, -SR^{E}, NR^{B}C(O)R^{D}, -C(O)NR^{B}R^{C}, -C(O)R^{D}, -C(O)OH, NR^{B}R^{C}, or G² (*e.g.*, CH₃, oxo, fluoro, OH, cyano, OCH₃, NH₂, N(CH₃)₂, NHC(O)CH₃, OC(O)CH₃, C(O)NH₂, OS(O)₂CH₃, -S(O)₂CH₃, -S(O)₂ CH₂CH₃, C(O)OH, OC(O)R^{D}, -C(O)CH₃, or -SCH₃).

In some embodiments, t is 1. In some embodiments, t is 0.

In some embodiments, R¹ and R² are each independently hydrogen, C₁-C₆ alkyl, hydroxyl-C₁-C₆ alkyl, or silyloxy-C₁-C₆ alkyl. In some embodiments, one of R¹ and R² is independently hydrogen and the other of R¹ and R² is independently hydrogen, C₁-C₆ alkyl, C₁-C₆ hydroxyl-C₁-C₆ alkyl, or silyloxy-C₁-C₆ alkyl. In some embodiments, R¹ and R² are each independently hydrogen, *-CH₃, *-CH₂CH₂OH, or *-CH₂CH₂OSi(CH₃)₂C(CH₃)₃, and "*-" indicates the attachment point to the nitrogen atom. In some embodiments, one of R¹ and R² is independently hydrogen and the other of R¹ and R² is independently hydrogen, *-CH₃, *- CH₂CH₂OH, or *-CH₂CH₂OSi(CH₃)₂C(CH₃)₃, and "*-" indicates the attachment point to the nitrogen atom. In some embodiments, R¹ and R² are each independently hydrogen.

In some embodiments, A is phenyl and W is independently phenyl or 5-6-membered heteroaryl. In some embodiments, each A and W is independently phenyl. In some embodiments, A is phenyl and W is 5-6-membered heteroaryl.

In some embodiments, W is a monocyclic 5-6-membered heteroaryl. In some embodiments, 2 R^{Y} groups on adjacent atoms of W, together with the atoms to which they are attached form a 3-7-membered fused cycloalkyl or heterocyclyl optionally substituted with 1-5 R^{X} forming a bicyclic heteroaryl. In some embodiments, W is a 10-membered heteroaryl, a 9-membered heteroaryl, a 6-membered heteroaryl, or a 5-membered heteroaryl. In some embodiments, W is a heteroaryl containing nitrogen, oxygen or sulfur as allowed by valence.

In some embodiments, each A and W is independently phenyl or 5-6-membered heteroaryl optionally substituted with 1-5 R^{Y}, and each R^{Y} is independently C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, halo, cyano, -OR^{A}, -NR^{B}R^{C}, -C(O)R^{D}, -C(O)OH, -C(O)OR^{D}, -S(R^{F})ₘ,-S(O)₂R^{D}, or G¹. In some embodiments, each of A and W is independently phenyl, pyridyl, pyrazinyl, pyridazinyl, pyridazinonyl, triazinyl, triazolyl, oxadiazolyl, or oxadiazolonyl, each of which is optionally substituted with 1-5 R^{Y} groups In some embodiments, each of A and W is independently phenyl, pyridyl, pyrazinyl, pyridazinyl, pyridazinonyl, oxadiazolyl, or oxadiazolonyl, each of which is optionally substituted with 1-5 R^{Y} groups. In some embodiments, each of A and W is independently selected from: 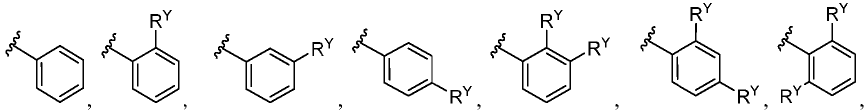 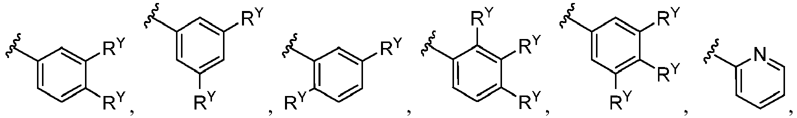 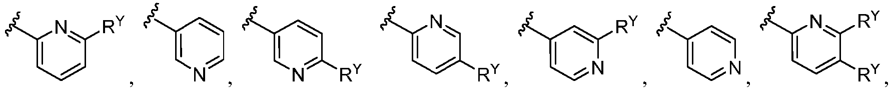 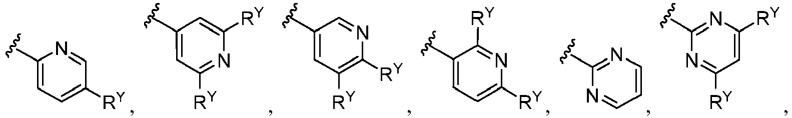 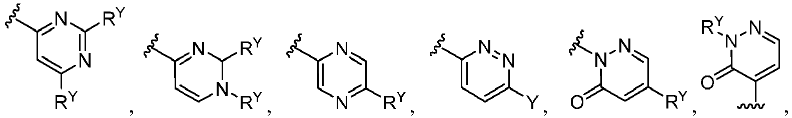 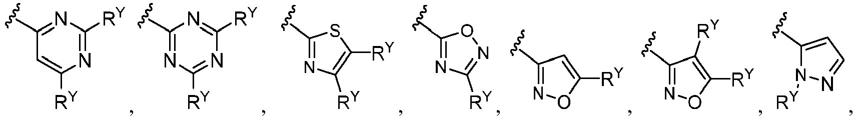 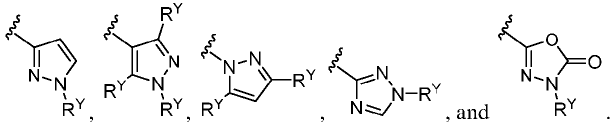 In some embodiments, each of A and W is independently selected from: 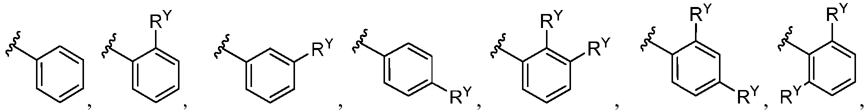 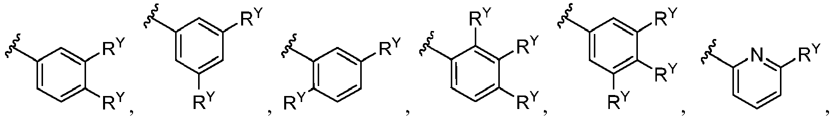 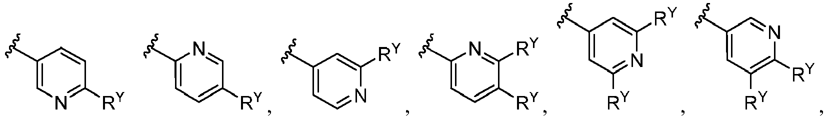 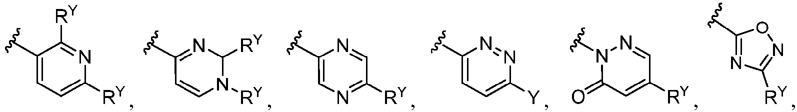 and 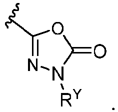 In some embodiments, A is phenyl and W is phenyl or 5-6-membered heteroaryl, each of A and W is optionally substituted with 1-5 R^{Y}, and each R^{Y} is independently C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, halo, cyano, -OR^{A}, -NR^{B}R^{C}, -C(O)R^{D}, -C(O)OH, -C(O)OR^{D}, -S(R^{F})ₘ,-S(O)₂R^{D}, or G¹. In some embodiments, A is phenyl and W is phenyl, pyridyl, pyrazinyl, pyridazinyl, pyridazinonyl, oxadiazolyl, or oxadiazolonyl, each of which is optionally substituted with 1-5 R^{Y}.

In some embodiments, A is selected from:

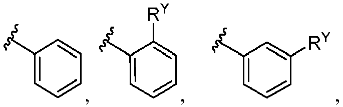

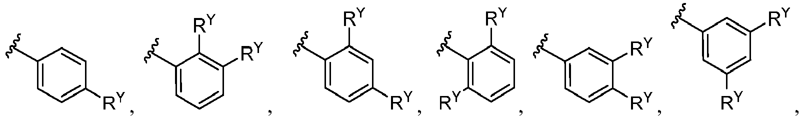

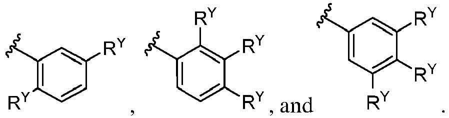

In some embodiments, W is selected from:

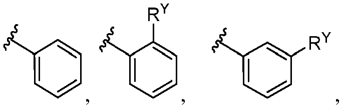

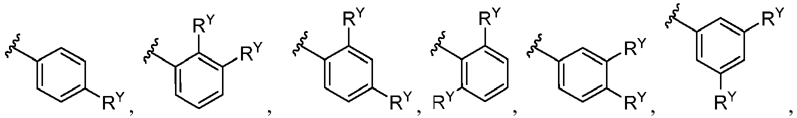

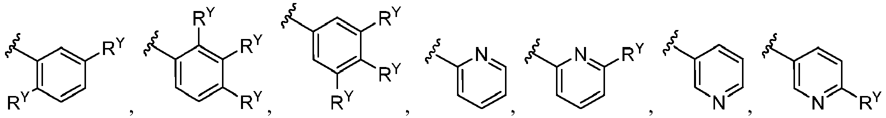

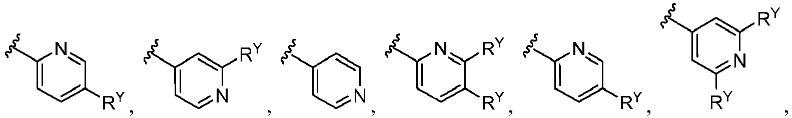

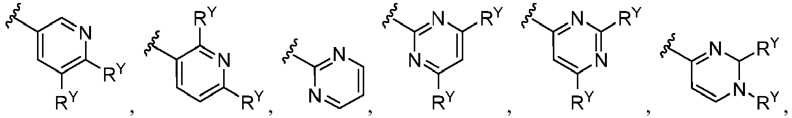

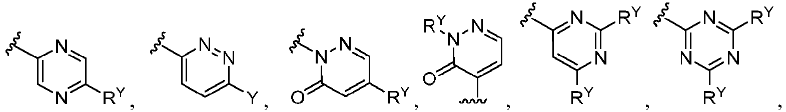

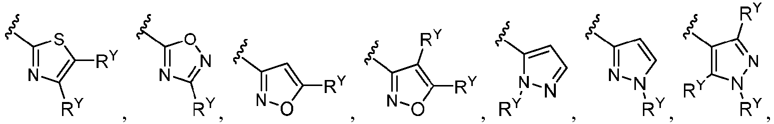

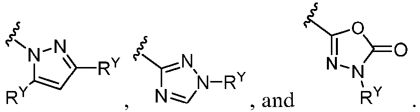

In some embodiments, A is phenyl and W is phenyl or 5-6-membered heteroaryl. In some embodiments, each of A and W is optionally substituted with 1-5 R^{Y}, and each R^{Y} is independently C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, halo, cyano, -OR^{A}, NR^{B}R^{C}, -C(O)R^{D}, -C(O)OH, -C(O)OR^{D}, - S(R^{F})ₘ,-S(O)₂R^{D}, or G¹.

In some embodiments, each R^{Y} is independently chloro, fluoro, iodo, CF₃, CHF₂, CH₂CF₃, CH₃, CH₂CH₃, C(CH₃)₂OH, OCH₃, OCH₂CH₃, OCF₃, S(O)₂CH₃, S(O)₂CH₂CH₂CH₃, CN, N(CH₃)₂, SF₅, SCH₃, NH₂, C(CH)₃, CH(CH₃)₂, CH₂CN, CH₂NH₂, CH(OH)CH₃, C(OH)(CH₃)CF₃, S(O)₂CH₃, C(O)CH₃, C(O)OCH₃, C(O)OH, OCHF₂ or G¹.

In some embodiments, each R^{Y} is independently chloro, fluoro, iodo, CF₃, CH₃, CH₂CH₃, OCH₃, S(O)₂CH₃, CN, N(CH₃)₂, SF₅, NH₂, C(CH)₃, CH(CH₃)₂, CH₂CN, CH₂NH₂, CH(OH)CH₃, C(O)CH₃, C(O)OCH₃, C(O)OH, OCHF₂ or G¹.

In some embodiments, each A and W is independently substituted with 2 R^{Y} on adjacent atoms, and the 2 R^{Y}, together with the atoms to which they are attached, form a 3-7-membered fused cycloalkyl, 3-7-membered fused heterocyclyl, fused aryl, or 5-6-membered fused heteroaryl ring optionally substituted with 1-5 R^{X}. In some embodiments, 2 R^{Y} together with the atoms to which they are attached form a pyrazolyl, pyrrolyl, isoxazolyl, thiophenyl, furanyl, or dioxolanyl ring, each of which is optionally substituted with 1-5 R^{X}. In some embodiments, each R^{X} is independently C₁-C₆ alkyl or halo (*e.g.*, CH₃ or fluoro).

In some embodiments, G¹ is cyclopropyl, isoxazolyl, piperidinyl, phenyl, or pyrazolyl, each of which is optionally substituted with 1-5 R^{Z}. In some embodiments, G¹ is cyclopropyl, isoxazolyl, or pyrazolyl, each of which is optionally substituted with 1-5 R^{Z}. In some embodiments, each R^{Z} is independently C₁-C₆ alkyl (e.g., CH₃) or halo (e.g., chloro). In some embodiments, each R^{Z} is independently C₁-C₆ alkyl (e.g., CH₃).

In some embodiments, the compound of Formula (I) (e.g., a compound of Formula (I-a), (I-b), (I-c), (I-d), (I-e), (I-f), (I-g), (I-h), (I-i), (I-j), (I-k) or (I-1)) or a pharmaceutically acceptable salt thereof is formulated as a pharmaceutically acceptable composition comprising a disclosed compound and a pharmaceutically acceptable carrier.

In some embodiments, the compound of Formula (I) (e.g., a compound of Formula (I-a), (I-b), (I-c), (I-d), (I-e), (I-f), (I-g), (I-h), (I-i), (I-j), (I-k) or (I-1)) is selected from a compound set forth in Table 1 or a pharmaceutically acceptable salt, solvate, hydrate, tautomer, ester, N-oxide or stereoisomer thereof.

Also disclosed herein is a compound of Formula (II): 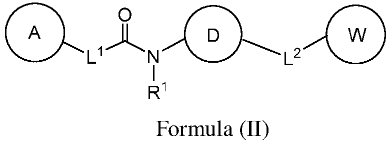 or a pharmaceutically acceptable salt, solvate, hydrate, tautomer, ester, N-oxide or stereoisomer thereof, wherein:
D is a bridged bicyclic cycloalkyl, bridged bicyclic heterocyclyl, or cubanyl, wherein each bridged bicyclic cycloalkyl, bridged bicyclic heterocyclyl, or cubanyl is optionally substituted with 1-4 R^{X} groups; and wherein if the bridged bicyclic heterocyclyl contains a substitutable nitrogen moiety, the substitutable nitrogen moiety may be optionally substituted by R^{N1};
L¹ is C₁-C₆ alkylene, C₂-C₆ alkenylene, 2-7-membered heteroalkylene, O, or NR^{C}, wherein C₁-C₆ alkylene, C₂-C₆ alkenylene, or 2-7-membered heteroalkylene is optionally substituted with 1-5 R^{X};
L² is -(C₀-C₂ alkylene)-O-(C₀-C₂ alkylene)-;
R¹ is hydrogen, C₁-C₆ alkyl, C₁-C₆ alkoxy-C₂-C₆ alkyl, hydroxy-C₂-C₆ alkyl, and silyloxy-C₂-C₆ alkyl;
R^{N1} is selected from the group consisting of hydrogen, C₁-C₆ alkyl, hydroxy-C₂-C₆ alkyl, halo-C₂-C₆ alkyl, amino-C₂-C₆ alkyl, cyano-C₂-C₆ alkyl, -C(O)NR^{B}R^{C}, -C(O)R^{D}, -C(O)OR^{D}, and -S(O)₂R^{D};
A and W are each independently phenyl or 5-6-membered heteroaryl, wherein each phenyl or 5-6-membered heteroaryl is optionally substituted with 1-5 R^{Y};
each R^{X} is independently selected from the group consisting of C₁-C₆ alkyl, hydroxy-C₁-C₆ alkyl, halo-C₁-C₆ alkyl, amino-C₁-C₆ alkyl, cyano-C₁-C₆ alkyl, C₁-C₆ alkoxy-C₁-C₆ alkyl, oxo, halo, cyano, -OR^{A}, -NR^{B}R^{C}, -NR^{B}C(O)R^{D}, -C(O)NR^{B}R^{C}, -C(O)R^{D}, -C(O)OH, - C(O)OR^{D}, -SR^{E}, -S(O)R^{D}, -S(O)₂R^{D}, -OS(O)R^{D}, -OS(O)₂R^{D}, and G²;
each R^{Y} is independently selected from the group consisting of hydrogen, C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, oxo, halo, cyano, -OR^{A}, -NR^{B}R^{C}, -NR^{B}C(O)R^{D}, -C(O)NR^{B}R^{C}, -C(O)R^{D}, -C(O)OH, - C(O)OR^{D}, -S(R^{F})ₘ, -S(O)R^{D}, -S(O)₂R^{D}, and G¹; or
2 R^{Y} groups on adjacent atoms, together with the atoms to which they are attached form a 3-7-membered fused cycloalkyl, heterocyclyl, aryl, or heteroaryl ring optionally substituted with 1-5 R^{X};
each G¹ and G² is independently C₃-C₆ cycloalkyl, 4-7-membered heterocyclyl, aryl, or 5-6-membered heteroaryl, wherein each C₃-C₆ cycloalkyl, 4-7-membered heterocyclyl, aryl, or 5-6-membered heteroaryl is optionally substituted with 1-3 R^{Z};
each R^{Z} is independently selected from the group consisting of C₁-C₆ alkyl, hydroxy-C₁-C₆ alkyl, halo-C₁-C₆ alkyl, halo, cyano, -OR^{A}, -NR^{B}R^{C}, -NR^{B}C(O)R^{D}, -C(O)NR^{B}R^{C}, -C(O)R^{D}, -C(O)OH, -C(O)OR^{D}, and -S(O)₂R^{D};
each R^{A} is independently hydrogen, C₁-C₆ alkyl, halo-C₁-C₆ alkyl, -C(O)NR^{B}R^{C}, - C(O)R^{D}, or -C(O)OR^{D};
each of R^{B} and R^{C} is independently hydrogen or C₁-C₆ alkyl; or
R^{B} and R^{C} together with the atom to which they are attached form a 3-7-membered heterocyclyl ring optionally substituted with 1-3 R^{Z};
each R^{D} is independently C₁-C₆ alkyl, 2-7-membered heteroalkyl, or halo-C₁-C₆ alkyl, wherein each C₁-C₆ alkyl, 2-7-membered heteroalkyl, or halo-C₁-C₆ alkyl is optionally substituted with 1-5 R^{G};
each R^{E} is independently hydrogen, C₁-C₆ alkyl, or halo-C₁-C₆ alkyl;
each R^{F} is independently hydrogen, C₁-C₆ alkyl, or halo;
each R^{G} is independently aryl or 5-6 membered heteroaryl, wherein each aryl or 5-6 membered heteroaryl is optionally substituted with 1-5 R^{H};
each R^{H} is independently C₁-C₆ alkyl or halo-C₁-C₆ alkyl;
m is 1 when R^{F} is hydrogen or C₁-C₆ alkyl, 3 when R^{F} is C₁-C₆ alkyl, or 5 when R^{F} is halo; and; and
t is 0 or 1.

In some embodiments D is selected from the group consisting of 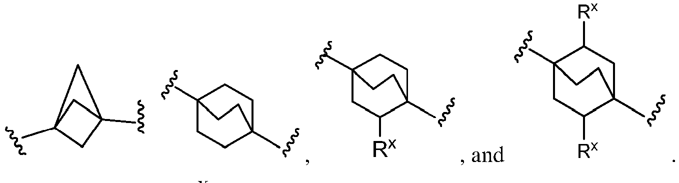 In some embodiments, each R^{X} is independently selected from the group consisting of oxo, -OR^{A} (*e.g.*, OH or OCH₃), -C(O)OH, -C(O)OR^{D} (*e.g.*, -C(O)OCH₃), halo, and hydroxy-C₁-C₆ alkyl.

In some embodiments, L¹ is CH₂O-* or CH₂OCH₂-*; wherein "-*" indicates the attachment point to A. In some embodiments, L² is selected from the group consisting of O-*, OCH₂-*, CH₂-*, OCH₂CH₂-*, CH₂OCH₂-*, and CH₂CH₂O-*; wherein "-*" indicates the attachment point to W. In some embodiments, R¹ is hydrogen or CH₃.

In some embodiments, A is selected from the group consisting of:

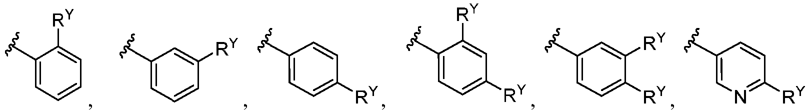

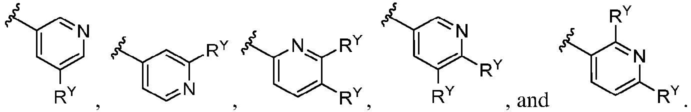

In some embodiments, W is selected from the group consisting of: 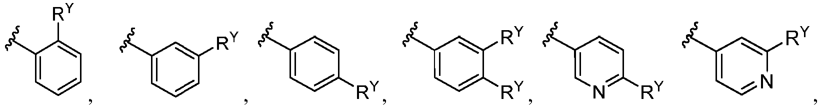 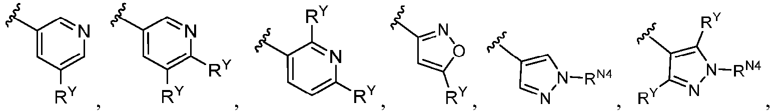 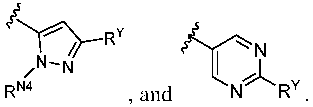 wherein R^{N4} is hydrogen or CH₃.

In some embodiments, each R^{Y} is independently hydrogen, chloro, fluoro, CF₃, CHF₂, CH₃, CH₂CH₃, CH(CH₃)₂, OCH₃, OCF₃, OCH(CH₃)₂, or CN.

In some embodiments, the compound of Formula (II) is a compound of Formula (II-a): 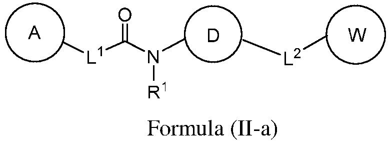 or a pharmaceutically acceptable salt, solvate, hydrate, tautomer, ester, N-oxide or stereoisomer thereof, wherein:
D is bicyclo[1.1.1]pentanyl or bicyclo[2.2.2]octanyl, each of which is optionally substituted with 1-4 R^{X} groups;
L¹ is CH₂O-* or CH₂OCH₂-*, wherein "-*" indicates the attachment point to A;
L² is selected from the group consisting of O-*, OCH₂-*, CH₂-*, OCH₂CH₂-*, CH₂OCH₂-*, and CH₂CH₂O-*; wherein "-*" indicates the attachment point to W;
A is phenyl or pyridyl, each of which is optionally substituted with 1-5 R^{Y} groups;
W is phenyl, pyridyl, isoxazolyl, or pyrazolyl, each of which is optionally substituted on one or more available carbons with 1-5 R^{Y} groups; and wherein pyrazolyl may be optionally substituted on an available nitrogen with hydrogen or CH₃;
each R^{X} is independently fluoro, oxo, OH, OCH₃, C(O)OH, or C(O)OCH₃;
each R^{Y} is independently chloro, fluoro, CF₃, CH₃, CH₂CH₃, CH(CH₃)₂, OCH₃, OCH(CH₃)₂, or CN; or
2 R^{Y} groups on adjacent atoms, together with the atoms to which they are attached form a furanyl, pyrrolyl, or dioxolanyl ring, each of which is optionally substituted with 1-2 R^{X}; and
R¹ is hydrogen.

In some embodiments, the compound of Formula (II) (e.g., a compound of Formula (II-a) or a pharmaceutically acceptable salt thereof is formulated as a pharmaceutically acceptable composition comprising a disclosed compound and a pharmaceutically acceptable carrier.

In some embodiments, the compound of Formula (II) (e.g., a compound of Formula (II-a) is selected from a compound set forth in Table 1 or a pharmaceutically acceptable salt, solvate, hydrate, tautomer, ester, N-oxide or stereoisomer thereof.

**Table 1: Exemplary compounds (compounds 551-619 are compounds of the invention as defined in appended claim 5, all other compounds are reference compounds)**

| **Compound Number** | **Structure** | **Compound Number** | **Structure** |
|---|---|---|---|
| **100** | 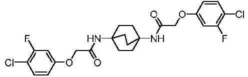 | **101** | 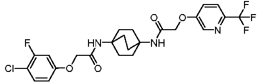 |
| **102** | 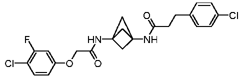 | **103** | 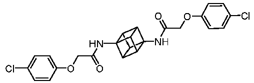 |
| **104** | 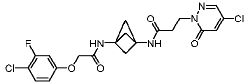 | **105** | 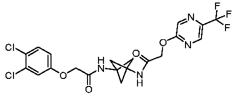 |
| **106** | 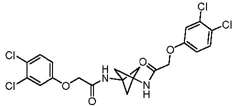 | **107** | 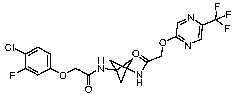 |
| **108** | 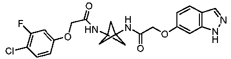 | **109** | 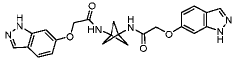 |
| **110** | 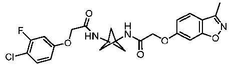 | **111** | 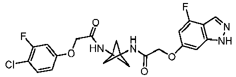 |
| **112** | 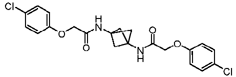 | **113** | 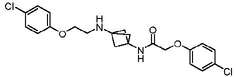 |
| **114** | 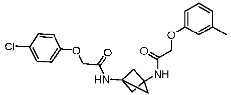 | **115** | 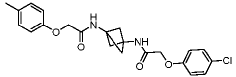 |
| **116** | 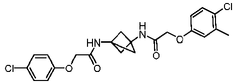 | **117** | 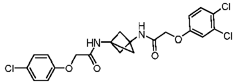 |
| **118** | 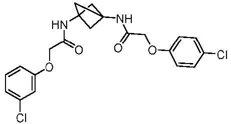 | **119** | 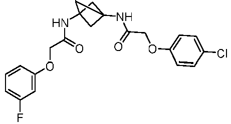 |
| **120** | 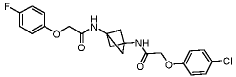 | **121** | 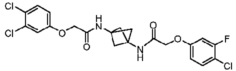 |
| **122** | 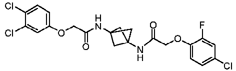 | **123** | 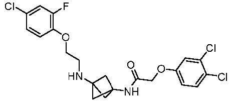 |
| **124** | 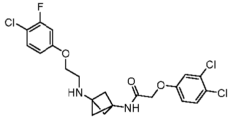 | **125** | 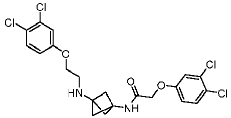 |
| **126** | 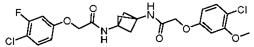 | **127** | 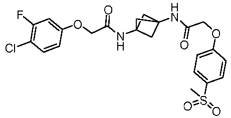 |
| **128** | 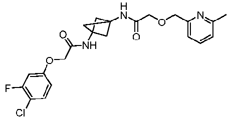 | **129** | 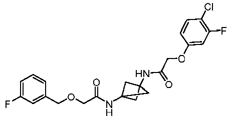 |
| **130** | 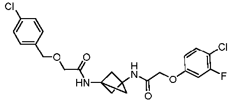 | **131** | 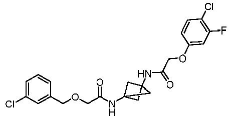 |
| **132** | 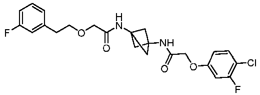 | **133** | 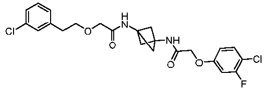 |
| **134** | 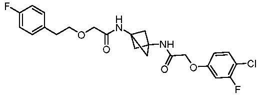 | **135** | 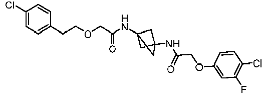 |
| **136** | 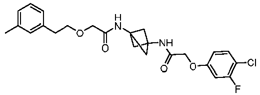 | **137** | 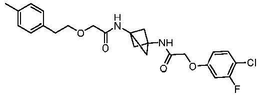 |
| **138** | 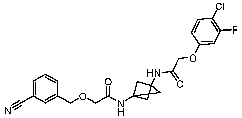 | **139** | 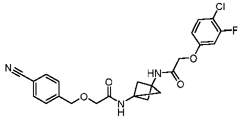 |
| **140** | 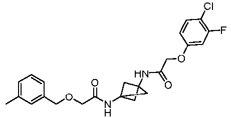 | **141** | 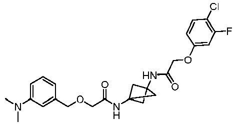 |
| **142** | 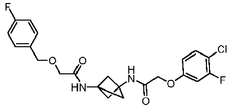 | **143** | 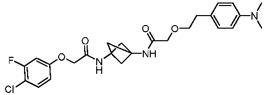 |
| **144** | 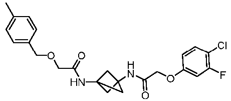 | **145** | 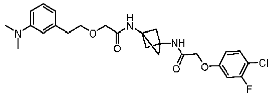 |
| **146** | 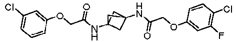 | **147** | 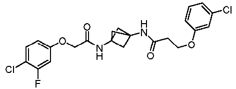 |
| **148** | 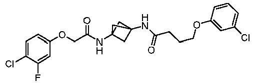 | **149** | 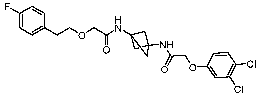 |
| **150** | 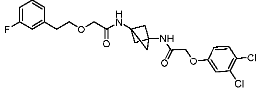 | **151** | 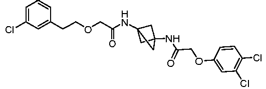 |
| **152** | 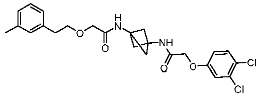 | **153** | 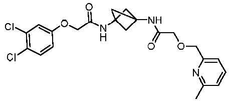 |
| **154** | 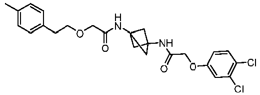 | **155** | 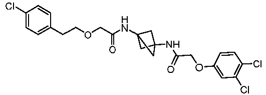 |
| **156** | 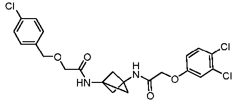 | **157** | 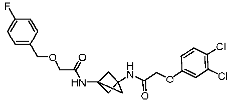 |
| **158** | 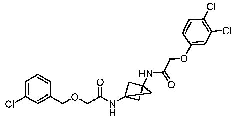 | **159** | 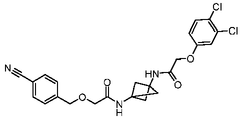 |
| **160** | 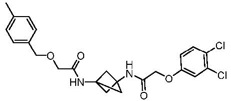 | **161** | 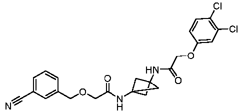 |
| **162** | 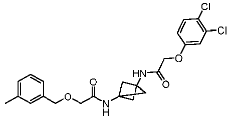 | **163** | 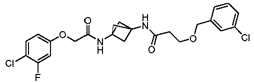 |
| **164** | 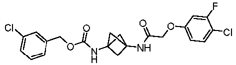 | **165** | 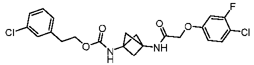 |
| **166** | 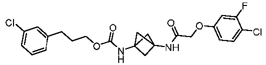 | **167** | 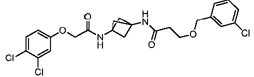 |
| **168** | 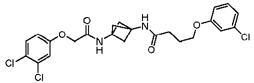 | **169** | 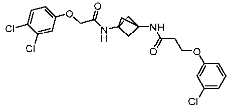 |
| **170** | 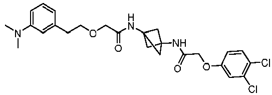 | **171** | 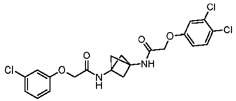 |
| **172** | 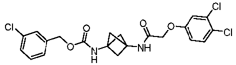 | **173** | 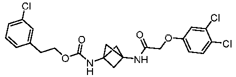 |
| **174** | 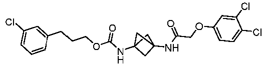 | **175** | 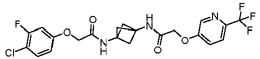 |
| **176** | 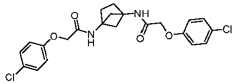 | **177** | 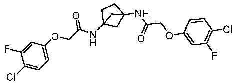 |
| **178** | 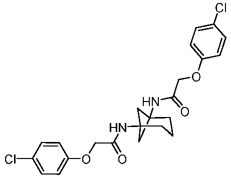 | **179** | 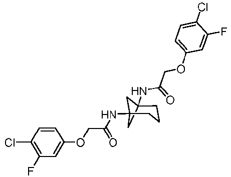 |
| **180** | 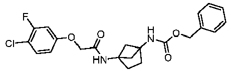 | **181** | 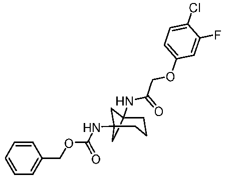 |
| **182** | 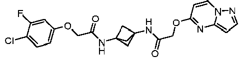 | **183** | 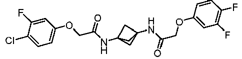 |
| **184** | 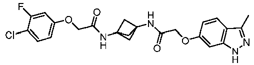 | **185** | 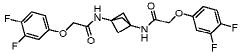 |
| **186** | 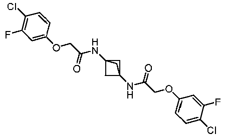 | **187** | 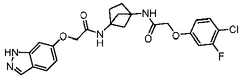 |
| **188** | 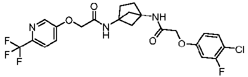 | **189** | 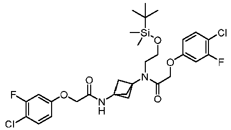 |
| **190** | 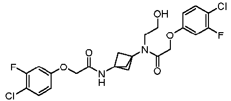 | **191** | 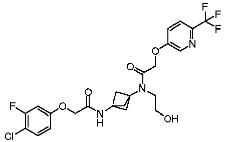 |
| **192** | 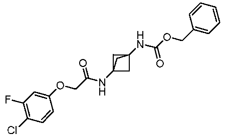 | **193** | 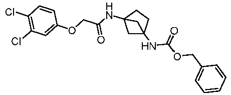 |
| **194** | 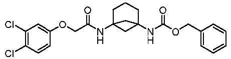 | **195** | 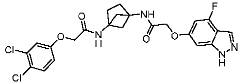 |
| **196** | 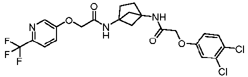 | **197** | 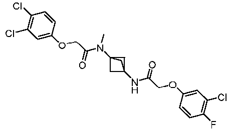 |
| **198** | 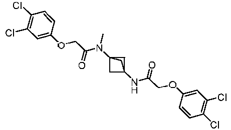 | **199** | 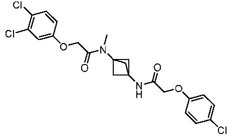 |
| **200** | 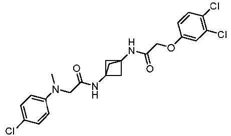 | **201** | 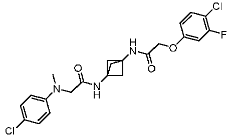 |
| **202** | 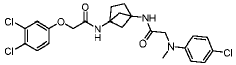 | **203** | 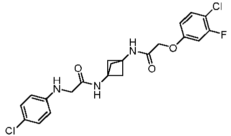 |
| **204** | 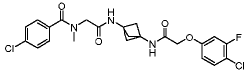 | **205** | 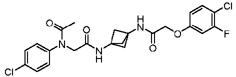 |
| **206** | 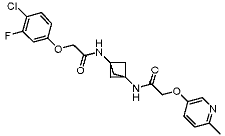 | **207** | 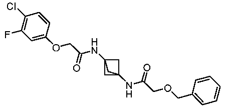 |
| **208** | 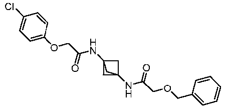 | **209** | 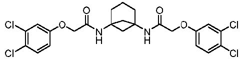 |
| **210** | 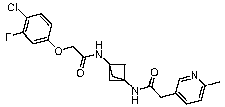 | **211** | 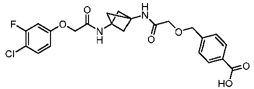 |
| **212** | 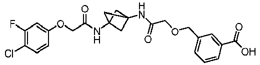 | **213** | 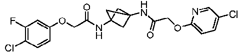 |
| **214** | 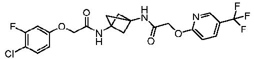 | **215** | 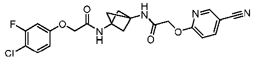 |
| **216** | 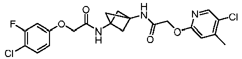 | **217** | 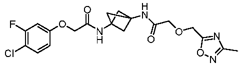 |
| **218** | 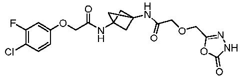 | **219** | 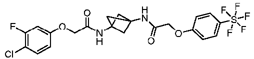 |
| **220** | 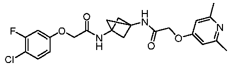 | **221** | 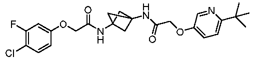 |
| **222** | 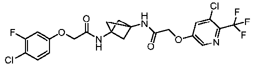 | **223** | 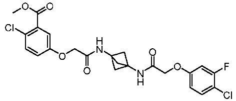 |
| **224** | 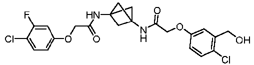 | **225** | 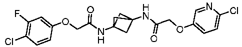 |
| **226** | 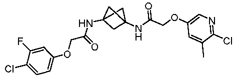 | **227** | 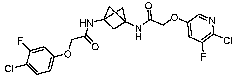 |
| **228** | 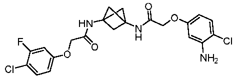 | **229** | 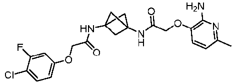 |
| **230** | 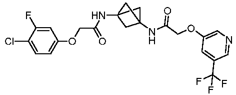 | **231** | 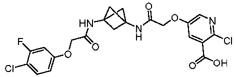 |
| **232** | 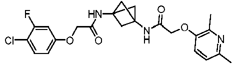 | **233** | 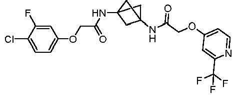 |
| **234** | 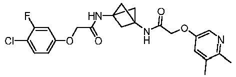 | **235** | 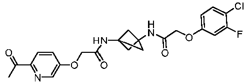 |
| **236** | 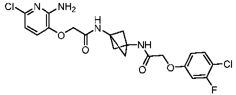 | **237** | 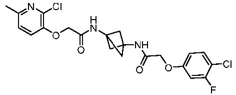 |
| **238** | 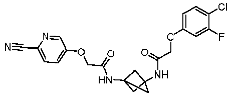 | **239** | 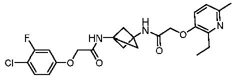 |
| **240** | 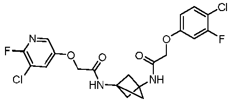 | **241** | 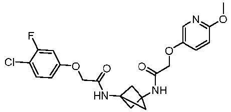 |
| **242** | 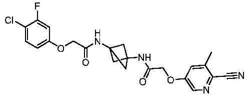 | **243** | 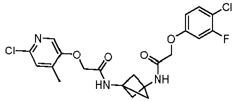 |
| **244** | 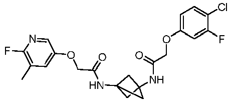 | **245** | 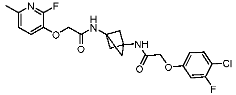 |
| **246** | 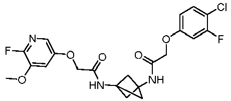 | **247** | 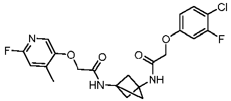 |
| **248** | 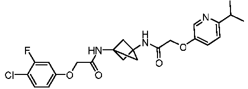 | **249** | 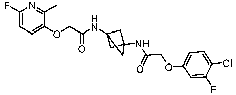 |
| **250** | 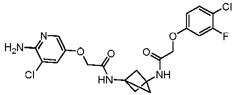 | **251** | 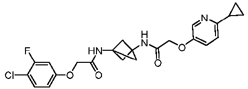 |
| **252** | 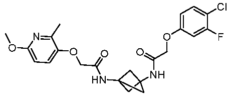 | **253** | 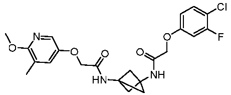 |
| **254** | 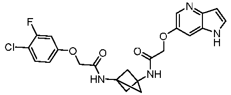 | **255** | 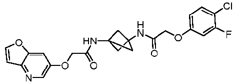 |
| **256** | 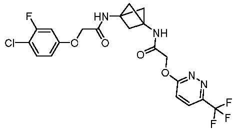 | **257** | 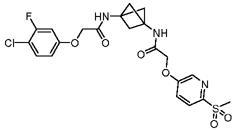 |
| **258** | 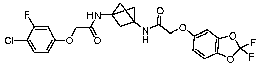 | **259** | 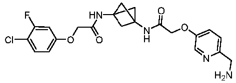 |
| **260** | 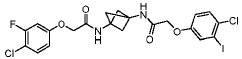 | **261** | 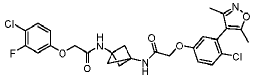 |
| **262** | 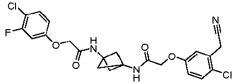 | **263** | 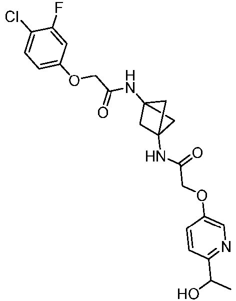 |
| **264** | 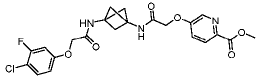 | **265** | 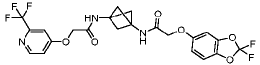 |
| **266** | 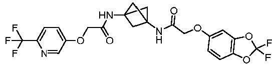 | **267** | 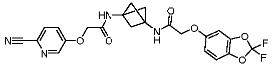 |
| **268** | 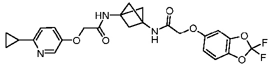 | **269** | 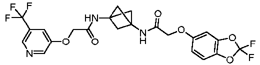 |
| **270** | 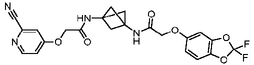 | **271** | 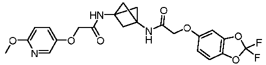 |
| **272** | 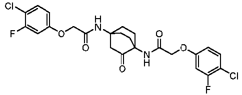 | **273** | 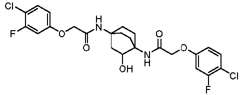 |
| **274** | 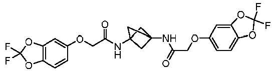 | **275** | 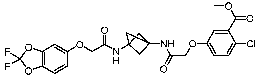 |
| **276** | 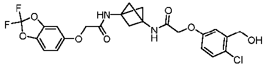 | **277** | 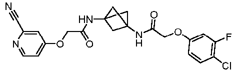 |
| **278** | 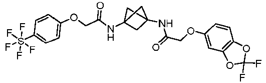 | **279** | 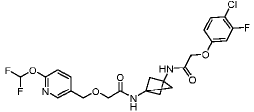 |
| **280** | 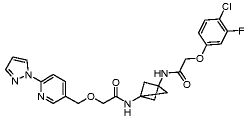 | **281** | 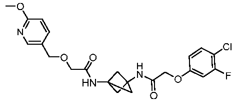 |
| **282** | 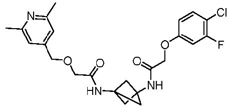 | **283** | 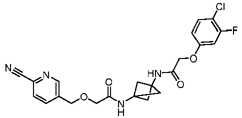 |
| **284** | 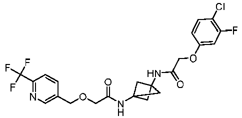 | **285** | 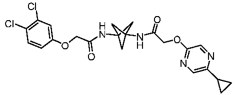 |
| **286** | 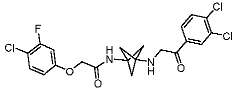 | **287** | 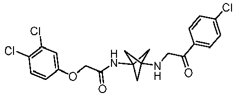 |
| **288** | 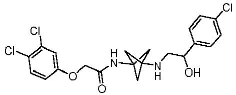 | **289** | 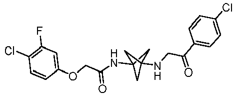 |
| **290** | 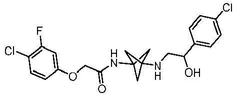 | **291** | 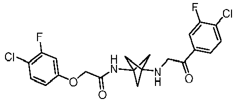 |
| **292** | 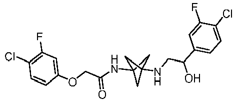 | **293** | 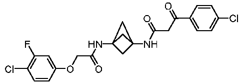 |
| **294** | 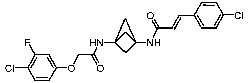 | **295** | 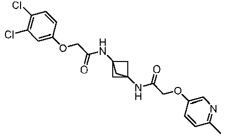 |
| **296** | 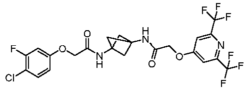 | **297** | 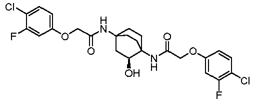 |
| **298** | 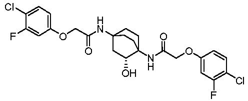 | **299** | 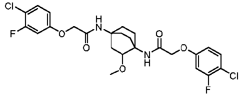 |
| **300** | 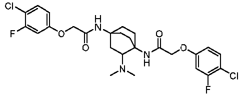 | **301** | 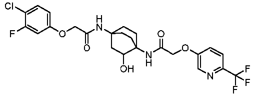 |
| **302** | 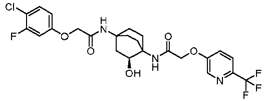 | **303** | 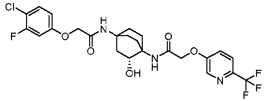 |
| **304** | 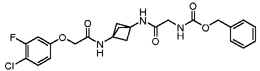 | **305** | 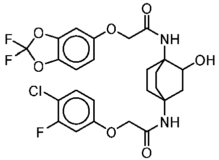 |
| **306** | 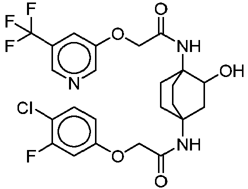 | **307** | 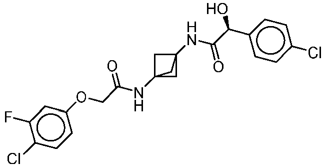 |
| **308** | 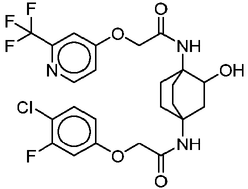 | **309** | 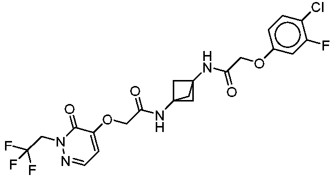 |
| **310** | 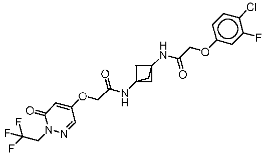 | **311** | 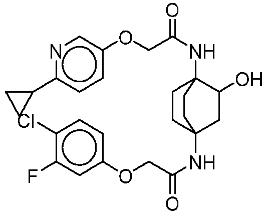 |
| **312** | 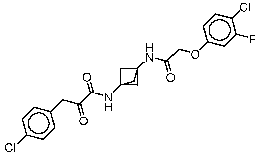 | **313** | 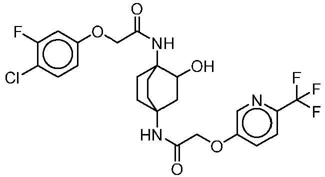 |
| **314** | 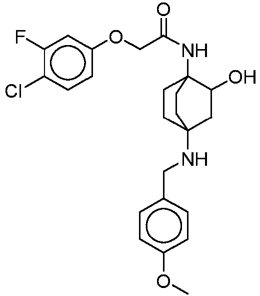 | **315** | 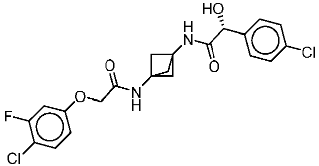 |
| **316** | 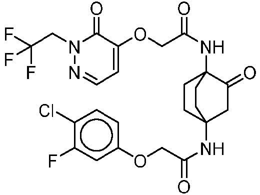 | **317** | 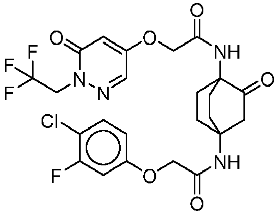 |
| **318** | 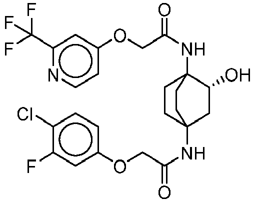 | **319** | 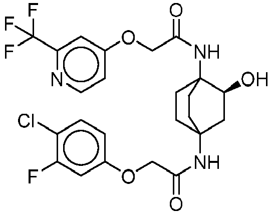 |
| **320** | 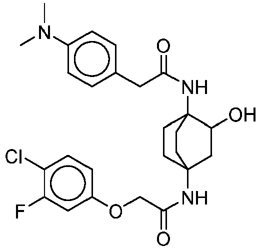 | **321** | 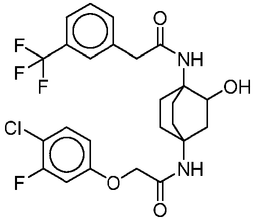 |
| **322** | 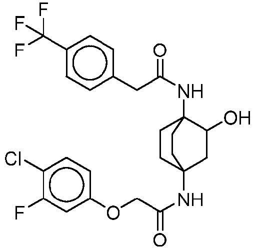 | **323** | 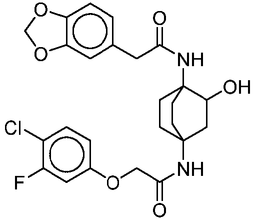 |
| **324** | 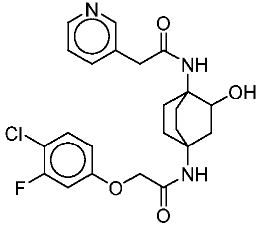 | **325** | 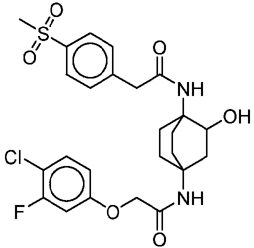 |
| **326** | 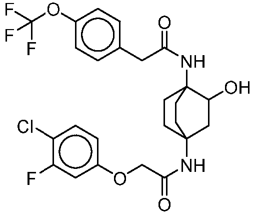 | **327** | 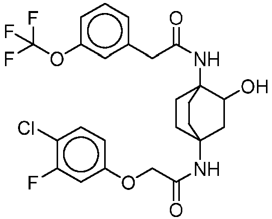 |
| **328** | 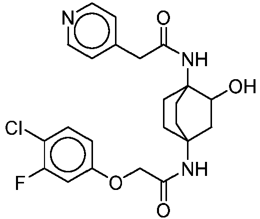 | **329** | 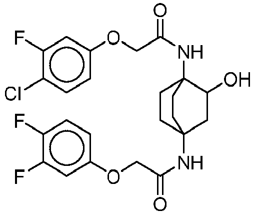 |
| **330** | 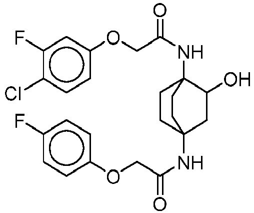 | **331** | 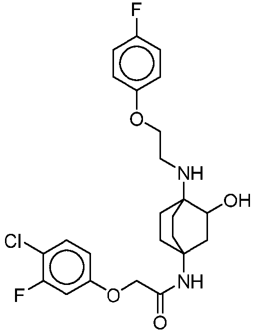 |
| **332** | 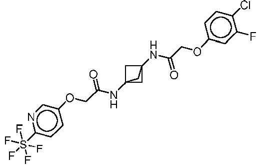 | **333** | 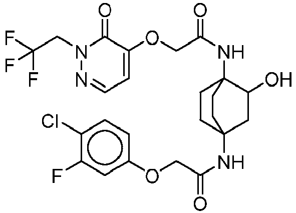 |
| **334** | 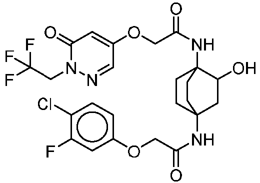 | **335** | 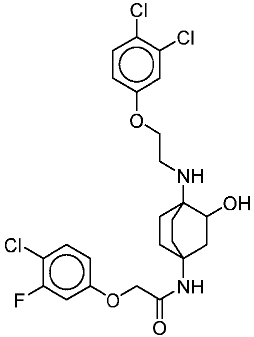 |
| **336** | 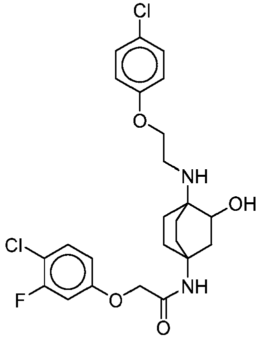 | **337** | 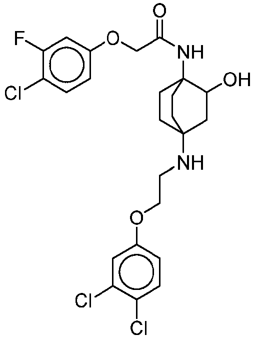 |
| **338** | 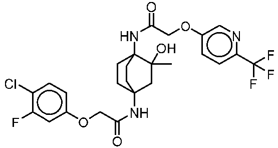 | **339** | 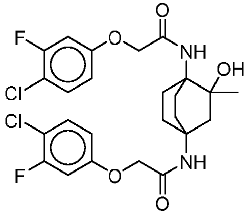 |
| **340** | 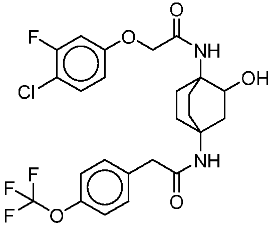 | **341** | 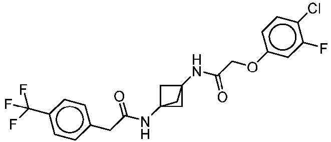 |
| **342** | 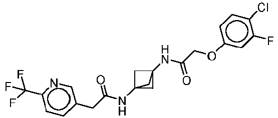 | **343** | 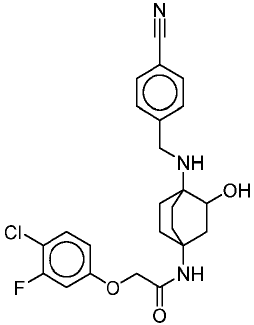 |
| **344** | 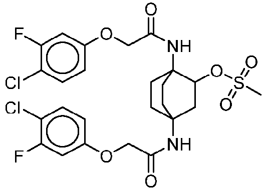 | **345** | 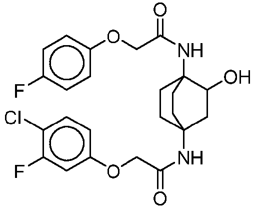 |
| **346** | 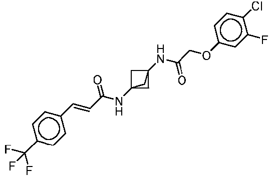 | **347** | 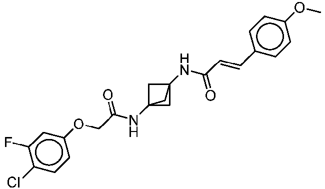 |
| **348** | 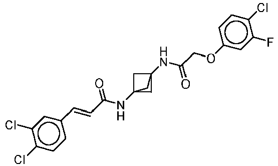 | **349** | 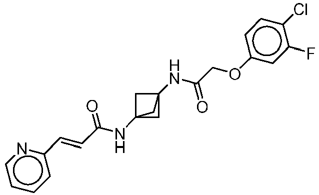 |
| **350** | 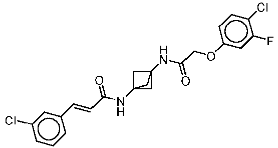 | **351** | 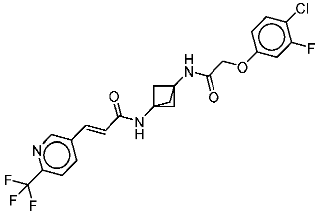 |
| **352** | 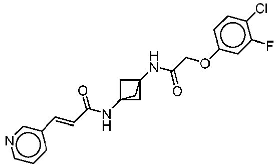 | **353** | 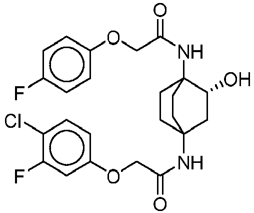 |
| **354** | 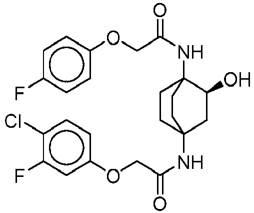 | **355** | 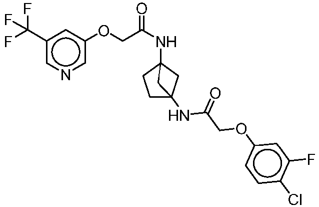 |
| **356** | 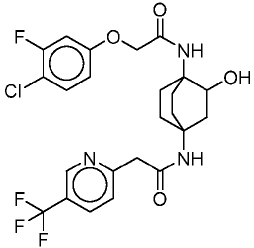 | **357** | 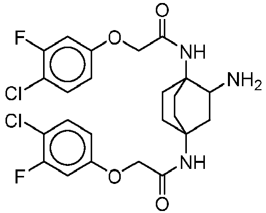 |
| **358** | 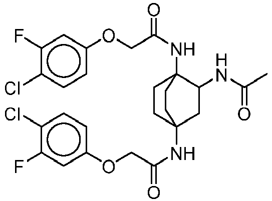 | **359** | 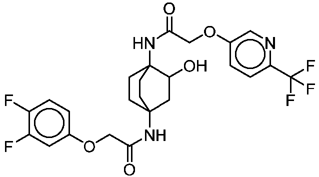 |
| **360** | 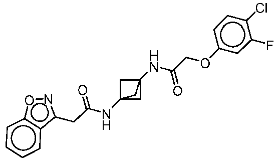 | **361** | 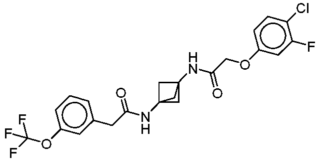 |
| **362** | 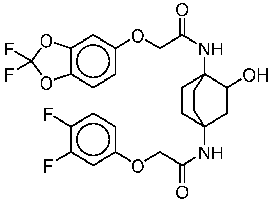 | **363** | 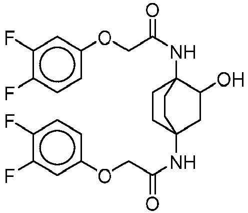 |
| **364** | 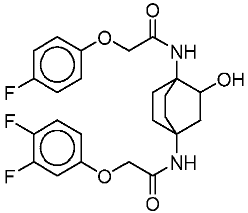 | **365** | 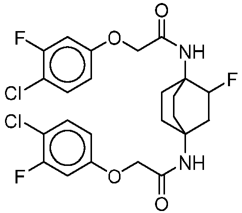 |
| **366** | 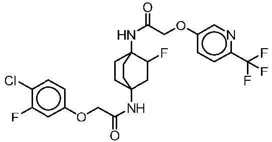 | **367** | 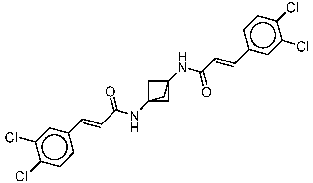 |
| **368** | 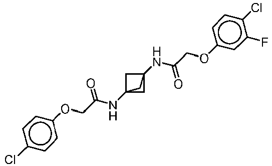 | **369** | 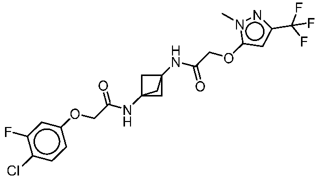 |
| **370** | 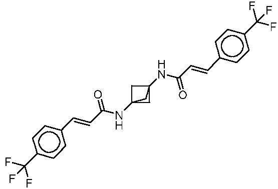 | **371** | 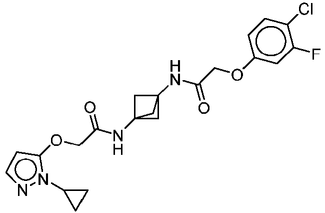 |
| **372** | 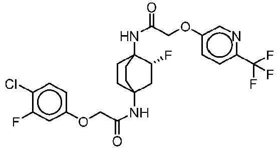 | **373** | 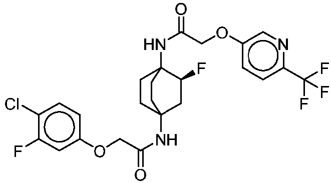 |
| **374** | 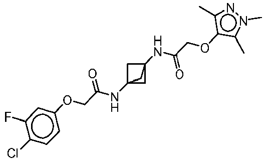 | **375** | 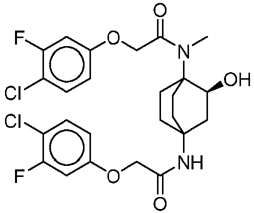 |
| **376** | 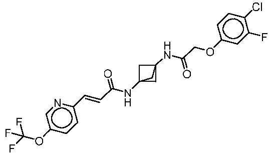 | **377** | 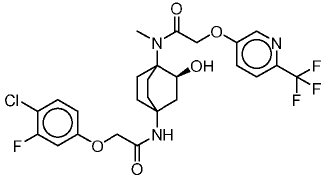 |
| **378** | 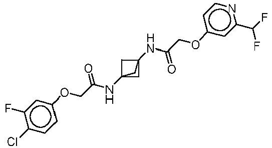 | **379** | 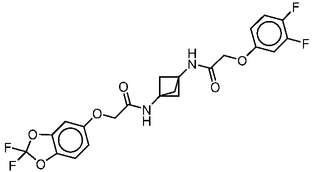 |
| **380** | 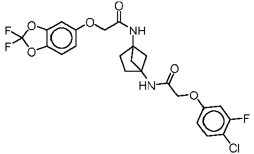 | **381** | 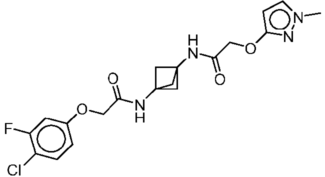 |
| **382** | 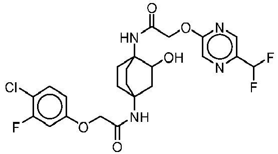 | **383** | 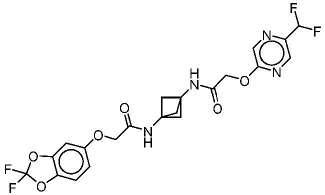 |
| **384** | 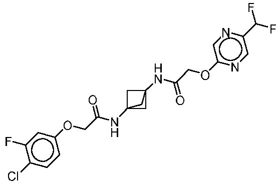 | **385** | 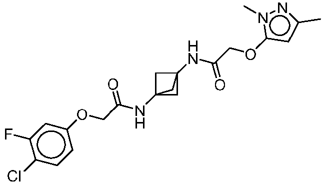 |
| **386** | 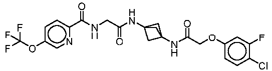 | **387** | 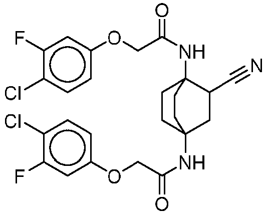 |
| **388** | 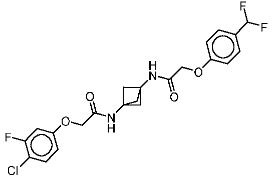 | **389** | 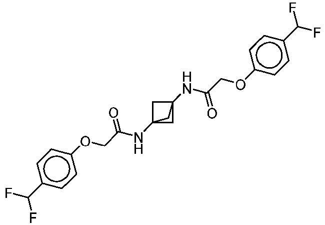 |
| **390** | 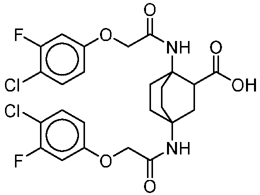 | **391** | 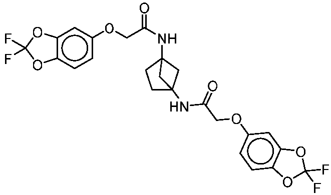 |
| **392** | 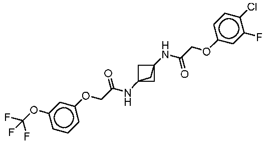 | **393** | 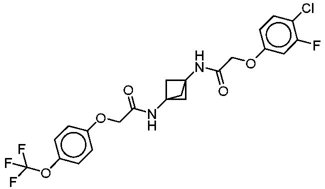 |
| **394** | 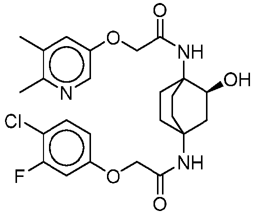 | **395** | 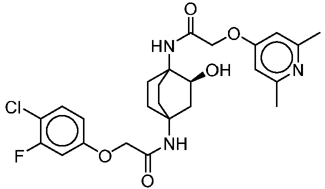 |
| **396** | 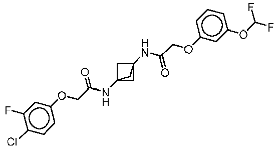 | **397** | 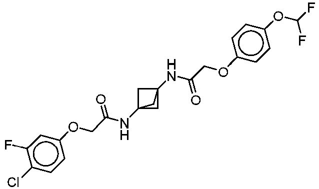 |
| **398** | 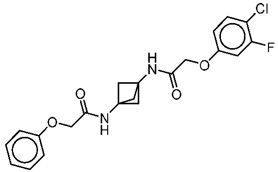 | **399** | 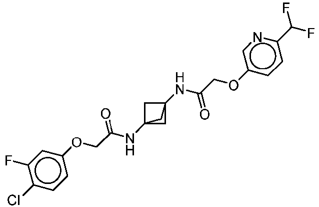 |
| **400** | 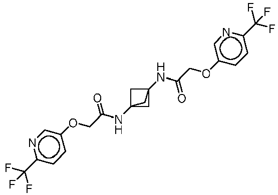 | **401** | 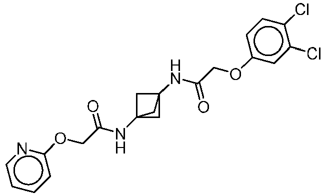 |
| **402** | 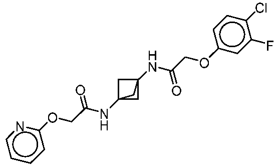 | **403** | 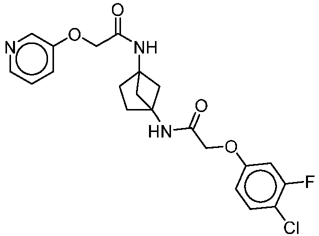 |
| **404** | 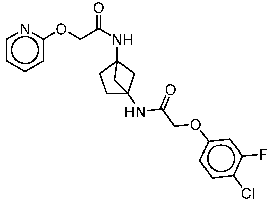 | **405** | 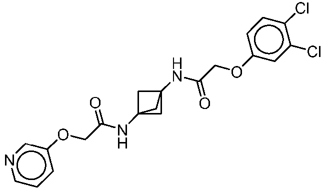 |
| **406** | 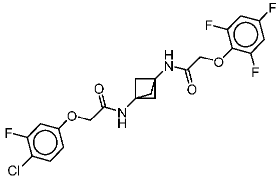 | **407** | 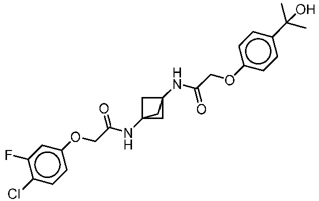 |
| **408** | 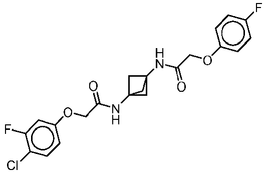 | **409** | 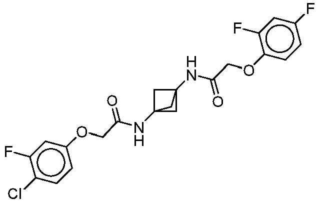 |
| **410** | 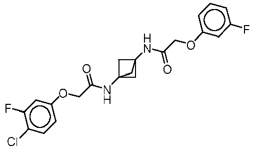 | **411** | 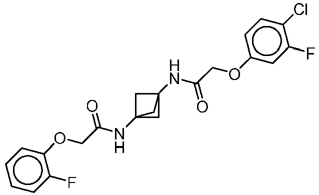 |
| **412** | 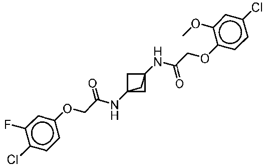 | **413** | 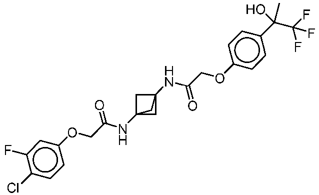 |
| **414** | 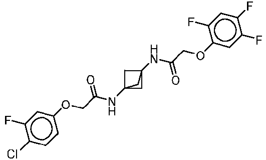 | **415** | 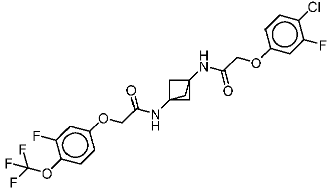 |
| **416** | 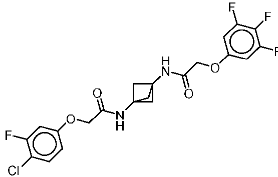 | **417** | 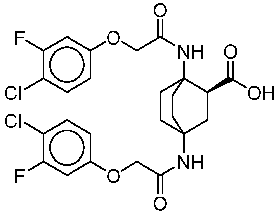 |
| **418** | 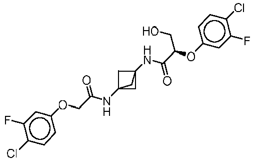 | **419** | 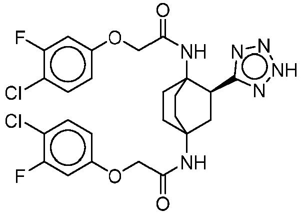 |
| **420** | 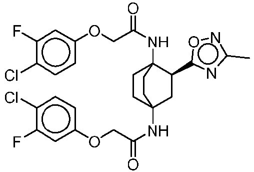 | **421** | 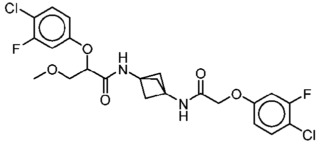 |
| **422** | 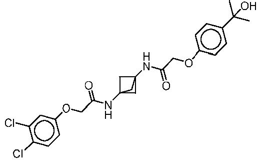 | **423** | 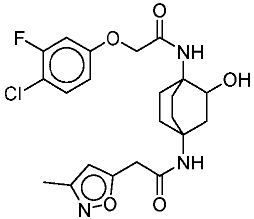 |
| **424** | 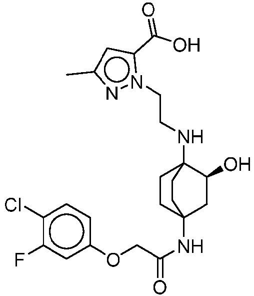 | **425** | 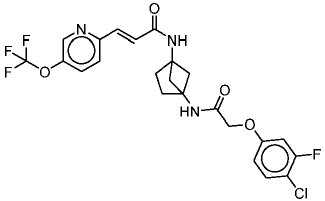 |
| **426** | 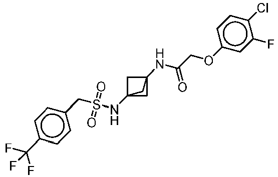 | **427** | 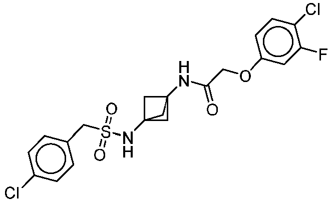 |
| **428** | 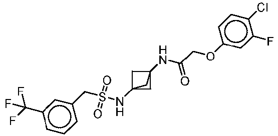 | **429** | 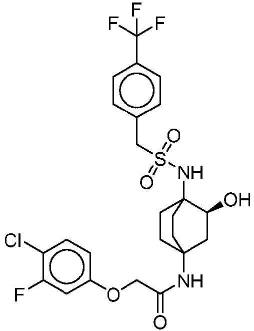 |
| **430** | 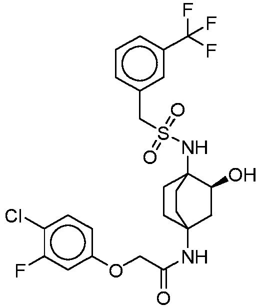 | **431** | 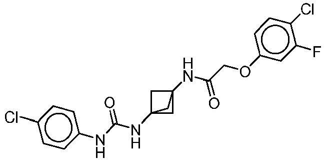 |
| **432** | 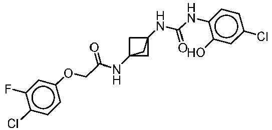 | **433** | 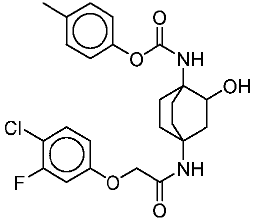 |
| **434** | 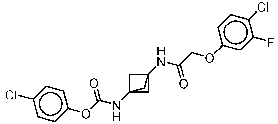 | **435** | 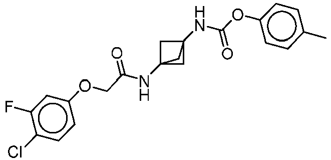 |
| | | | |
| **436** | 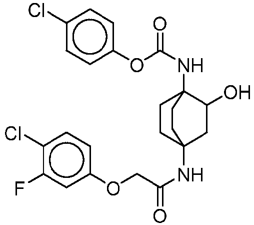 | **437** | 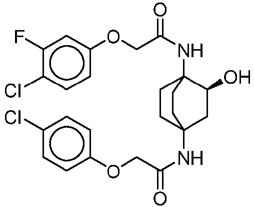 |
| **438** | 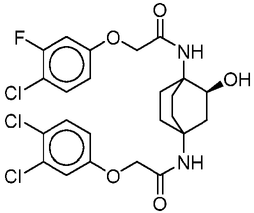 | **439** | 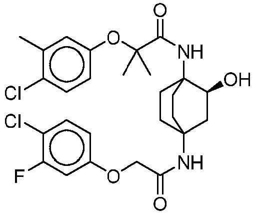 |
| **440** | 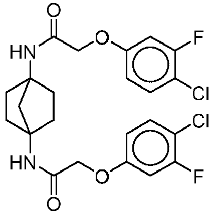 | **441** | 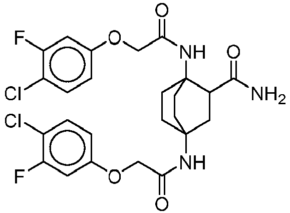 |
| **442** | 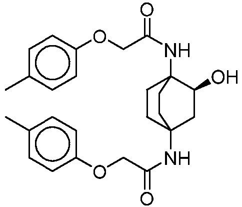 | **443** | 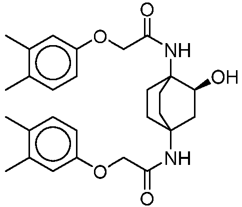 |
| **444** | 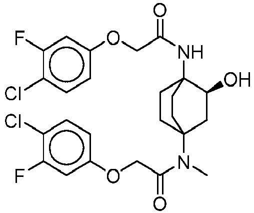 | **445** | 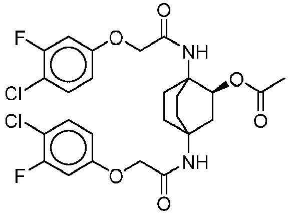 |
| **446** | 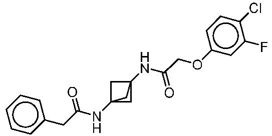 | **447** | 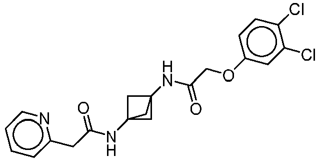 |
| **448** | 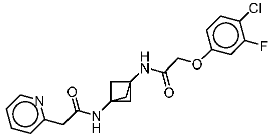 | **449** | 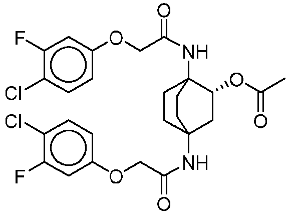 |
| **450** | 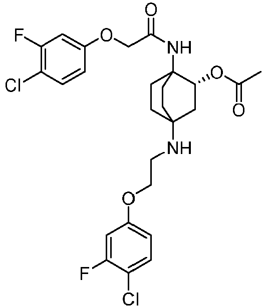 | **451** | 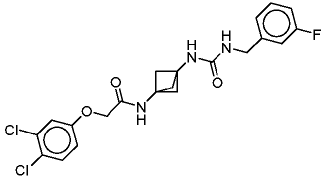 |
| **452** | 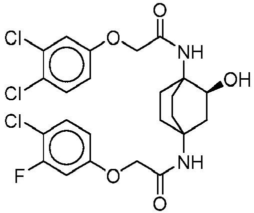 | **453** | 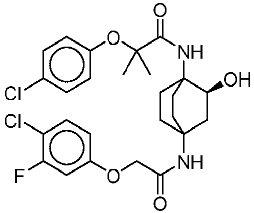 |
| **454** | 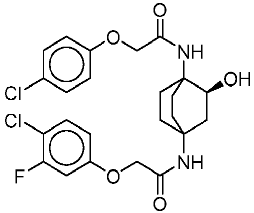 | **455** | 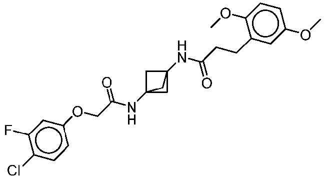 |
| **456** | 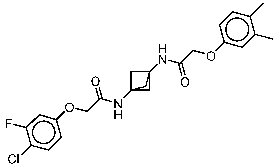 | **457** | 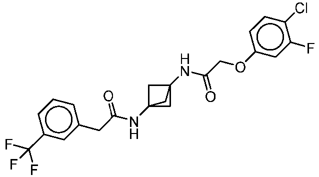 |
| **458** | 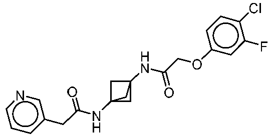 | **459** | 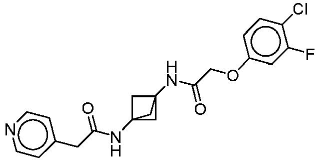 |
| **460** | 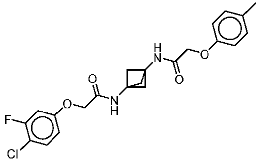 | **461** | 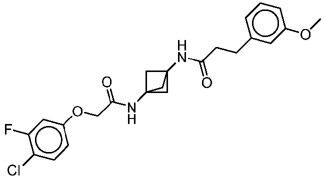 |
| **462** | 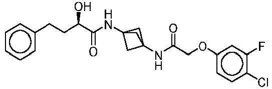 | **463** | 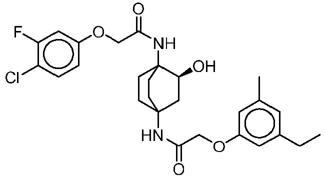 |
| **464** | 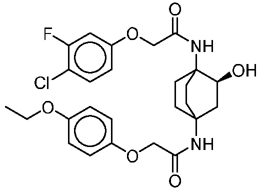 | **465** | 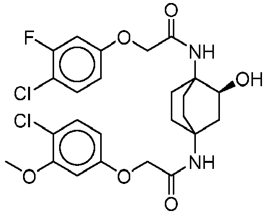 |
| **466** | 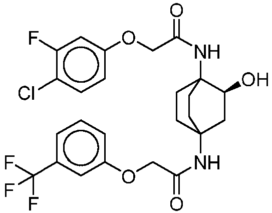 | **467** | 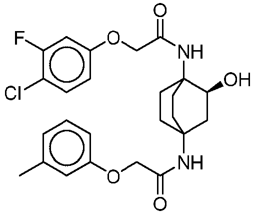 |
| **468** | 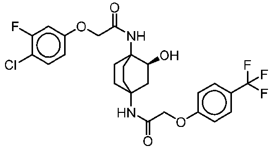 | **469** | 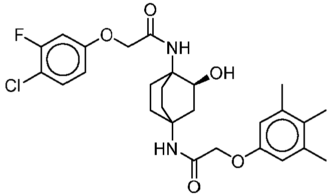 |
| **470** | 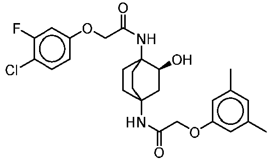 | **471** | 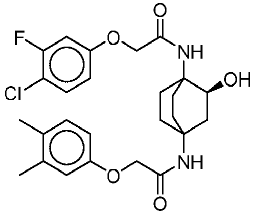 |
| **472** | 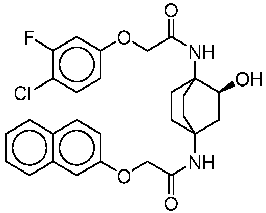 | **473** | 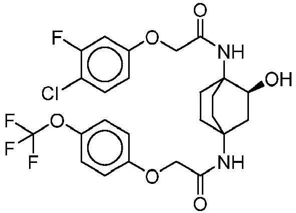 |
| **474** | 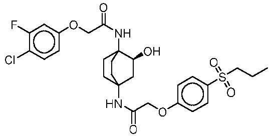 | **475** | 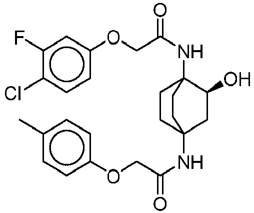 |
| **476** | 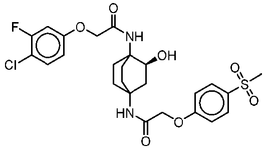 | **477** | 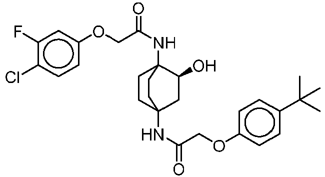 |
| **478** | 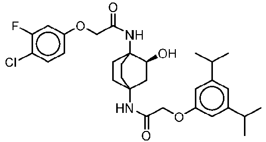 | **479** | 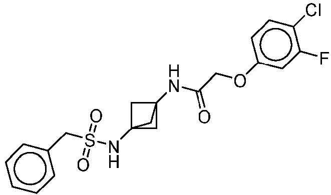 |
| | | | |
| **480** | 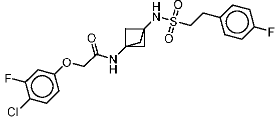 | **481** | 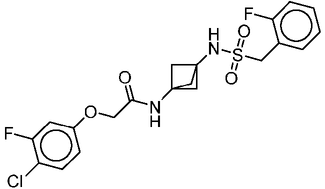 |
| **482** | 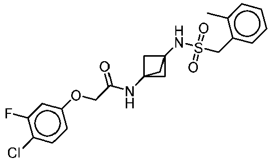 | **483** | 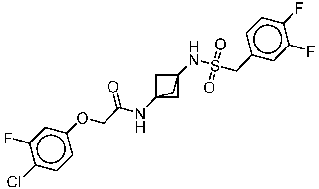 |
| **484** | 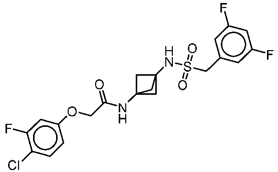 | **485** | 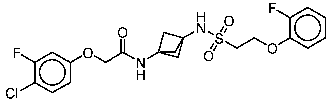 |
| **486** | 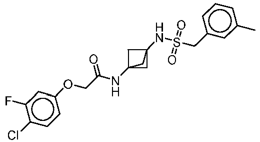 | **487** | 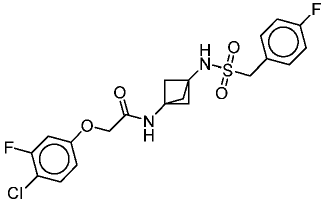 |
| **488** | 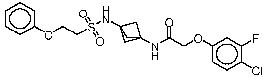 | **489** | 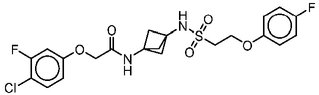 |
| **490** | 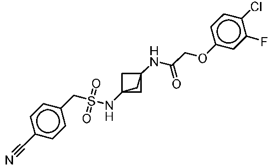 | **491** | 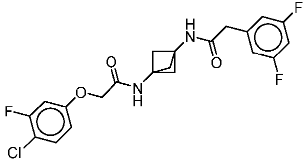 |
| **492** | 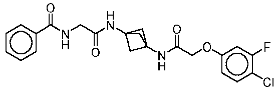 | **493** | 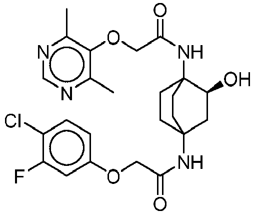 |
| **494** | 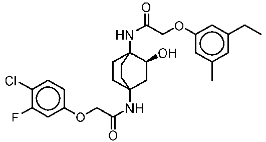 | **495** | 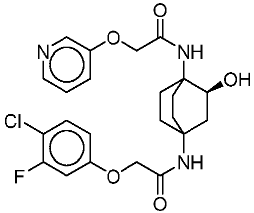 |
| **496** | 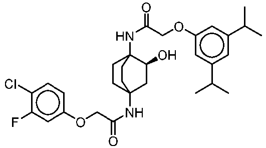 | **497** | 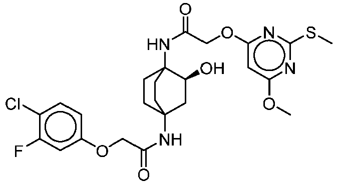 |
| **498** | 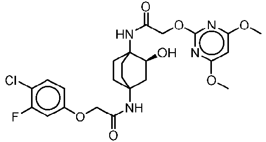 | **499** | 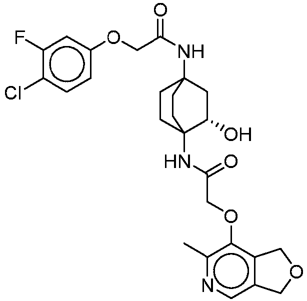 |
| **500** | 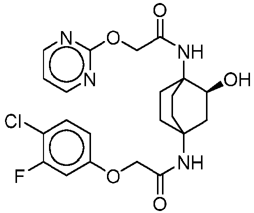 | **501** | 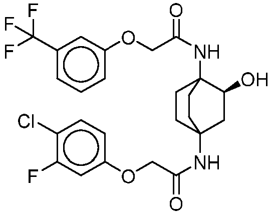 |
| **502** | 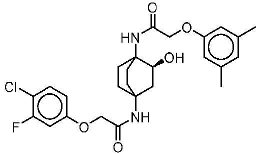 | **503** | 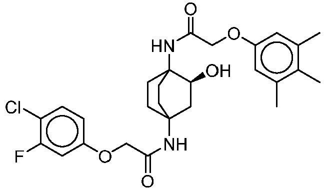 |
| **504** | 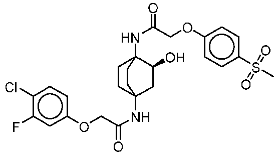 | **505** | 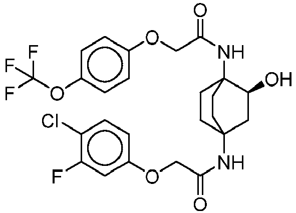 |
| **506** | 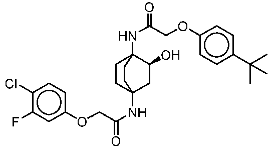 | **507** | 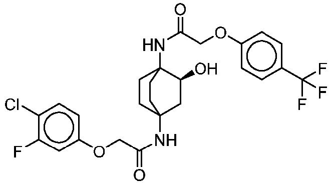 |
| **508** | 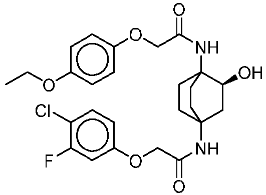 | **509** | 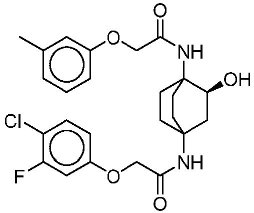 |
| **510** | 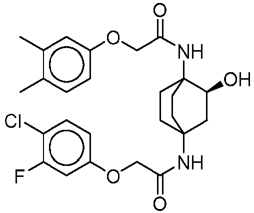 | **511** | 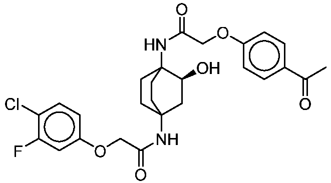 |
| **512** | 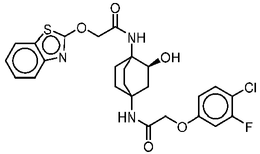 | **513** | 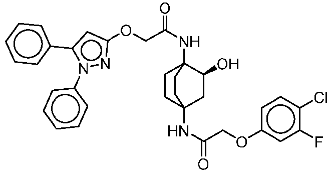 |
| **514** | 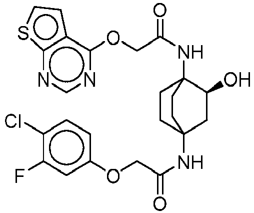 | **515** | 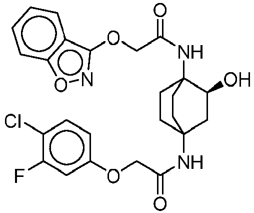 |
| **516** | 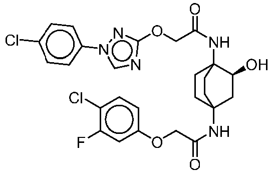 | **517** | 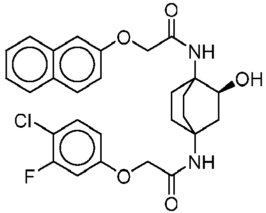 |
| **518** | 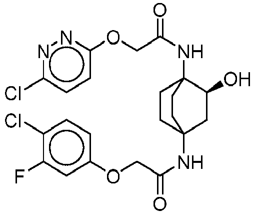 | **519** | 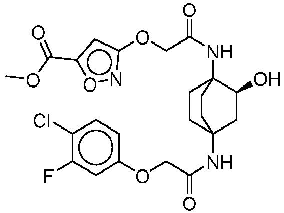 |
| **520** | 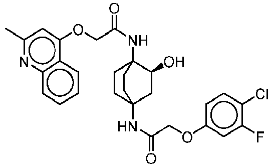 | **521** | 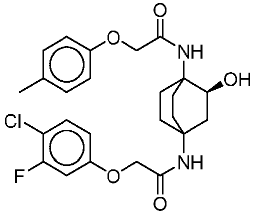 |
| **522** | 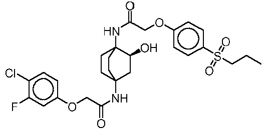 | **523** | 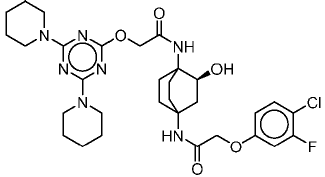 |
| **524** | 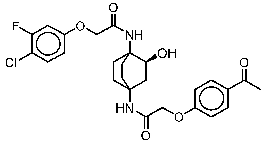 | **525** | 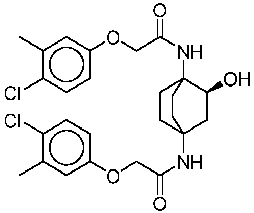 |
| **526** | 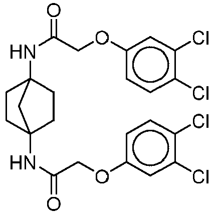 | **527** | 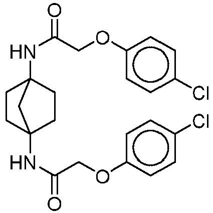 |
| **528** | 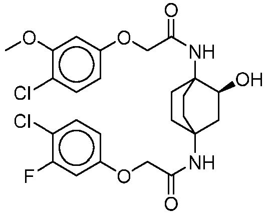 | **529** | 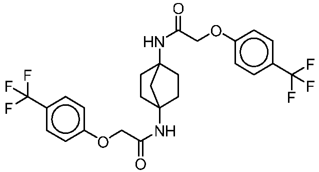 |
| **530** | 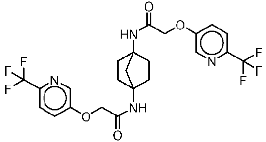 | **531** | 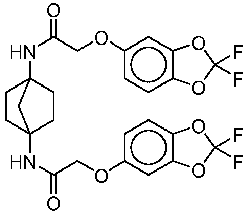 |
| **532** | 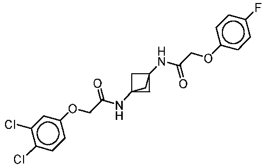 | **533** | 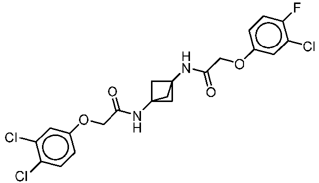 |
| **534** | 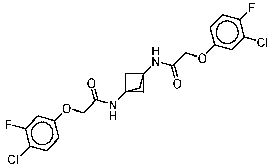 | **535** | 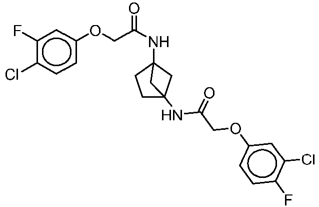 |
| **536** | 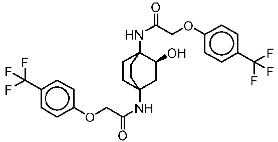 | **537** | 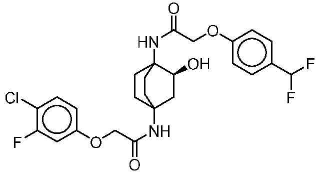 |
| **538** | 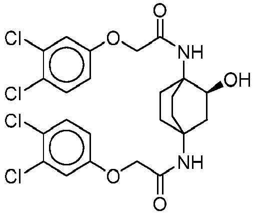 | **539** | 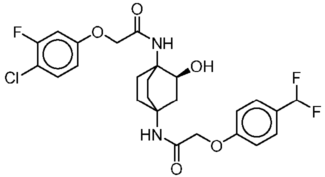 |
| **540** | 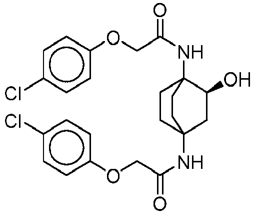 | **541** | 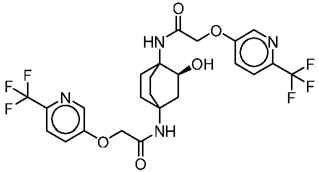 |
| **542** | 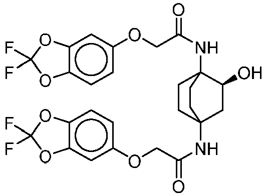 | **543** | 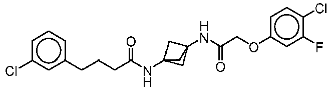 |
| **544** | 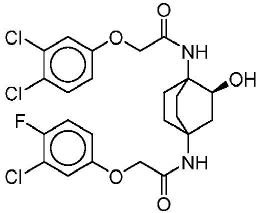 | **545** | 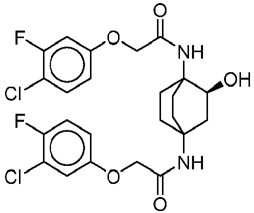 |
| **546** | 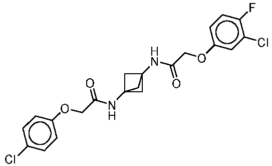 | **547** | 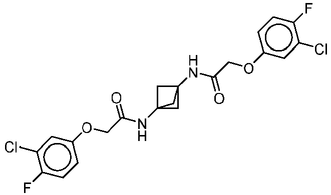 |
| **548** | 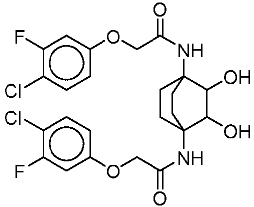 | **549** | 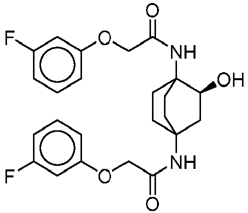 |
| **550** | 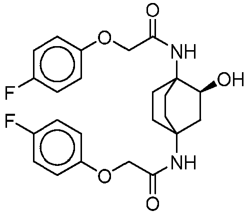 | **551** | 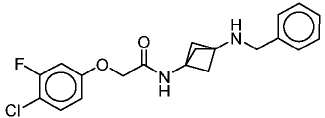 |
| **552** | 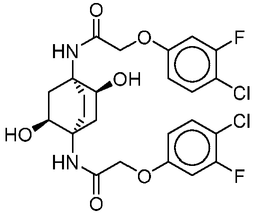 | **553** | 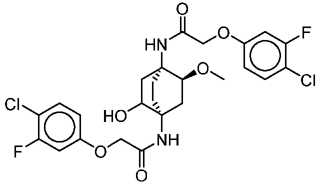 |
| **554** | 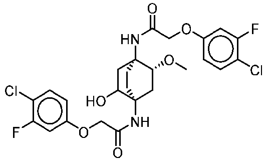 | **555** | 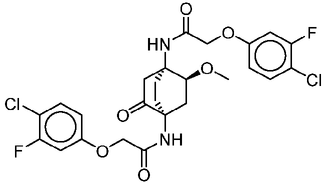 |
| **556** | 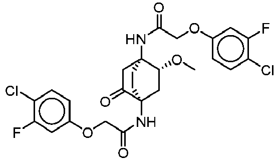 | **557** | 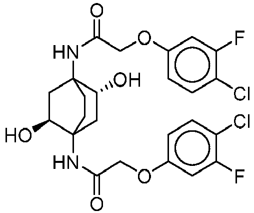 |
| **558** | 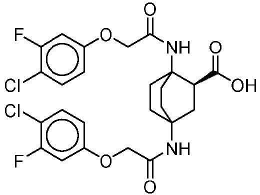 | **559** | 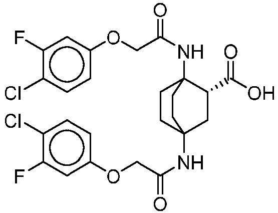 |
| **560** | 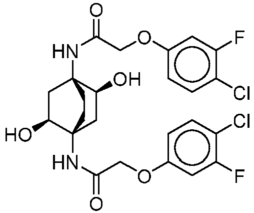 | **561** | 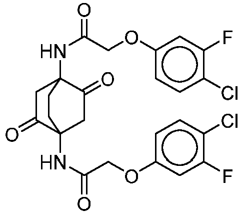 |
| **562** | 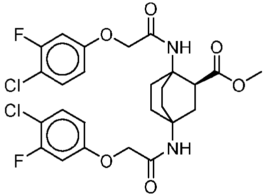 | **563** | 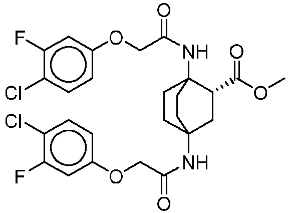 |
| **564** | 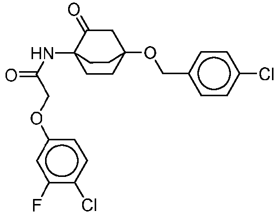 | **565** | 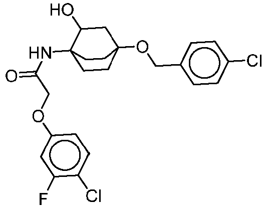 |
| **566** | 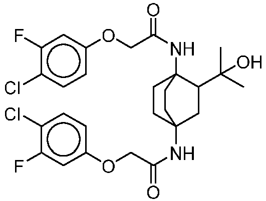 | **567** | 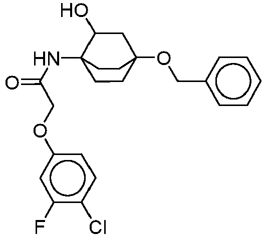 |
| **568** | 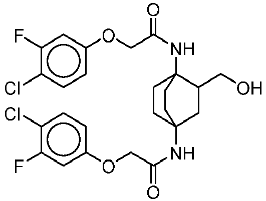 | **569** | 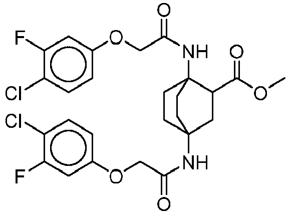 |
| **570** | 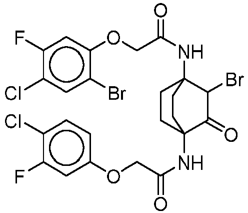 | **571** | 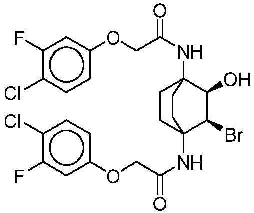 |
| **572** | 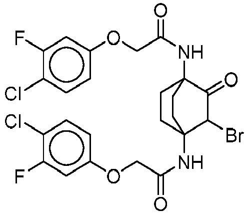 | **573** | 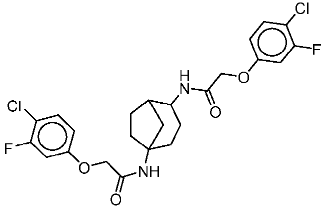 |
| **574** | 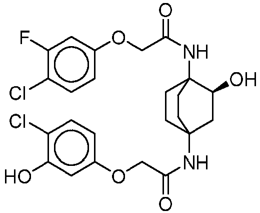 | **575** | 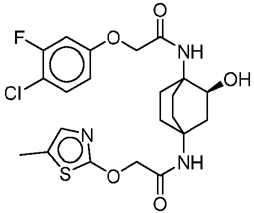 |
| **576** | 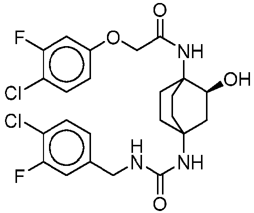 | **577** | 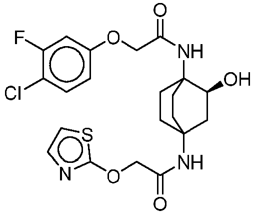 |
| **578** | 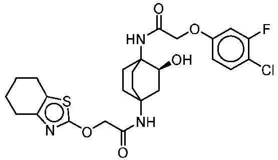 | **579** | 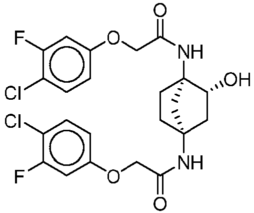 |
| **580** | 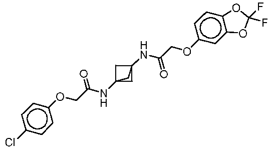 | **581** | 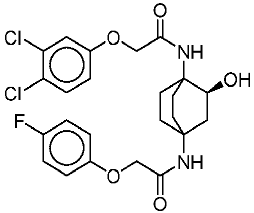 |
| **582** | 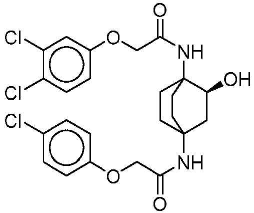 | **583** | 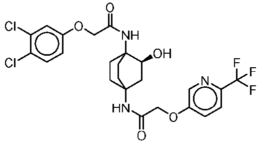 |
| **584** | 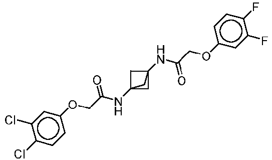 | **585** | 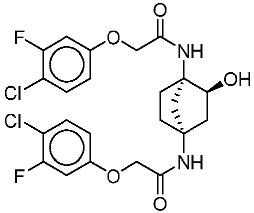 |
| **586** | 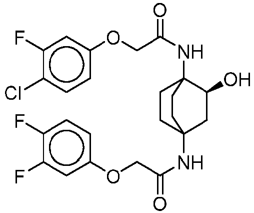 | **587** | 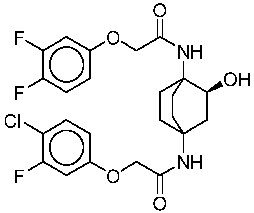 |
| **588** | 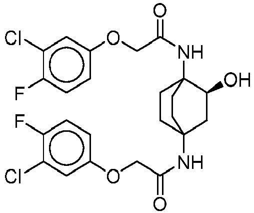 | **589** | 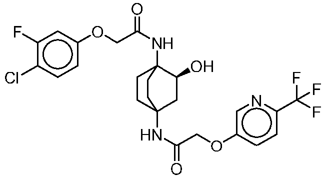 |
| **590** | 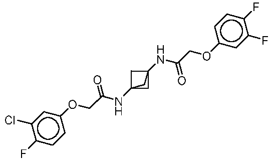 | **591** | 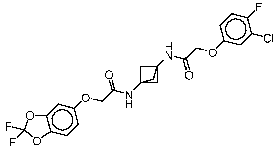 |
| **592** | 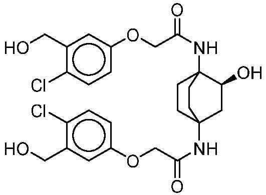 | **593** | 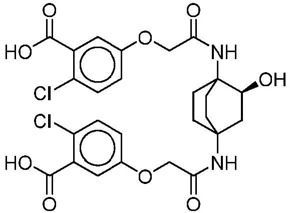 |
| **594** | 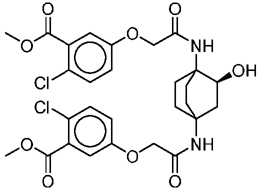 | **595** | 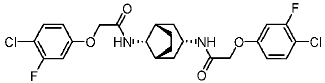 |
| **596** | 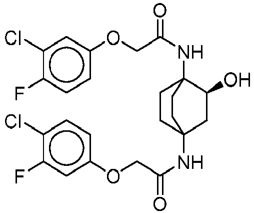 | **597** | 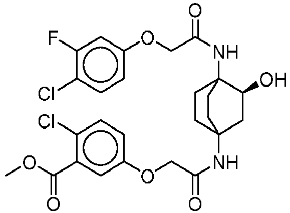 |
| **598** | 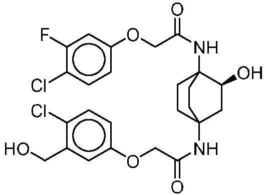 | **599** | 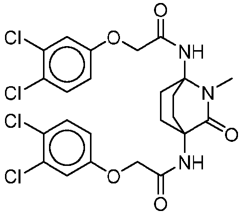 |
| **600** | 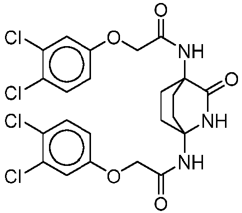 | **601** | 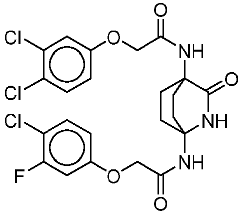 |
| **602** | 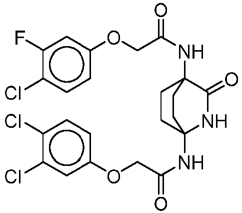 | **603** | 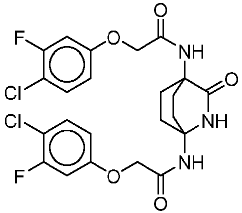 |
| **604** | 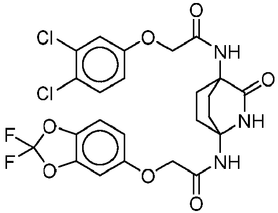 | **605** | 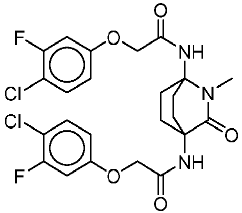 |
| **606** | 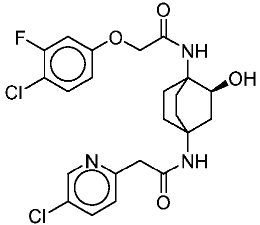 | **607** | 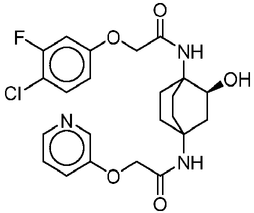 |
| **608** | 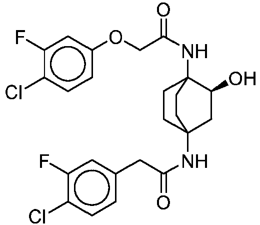 | **609** | 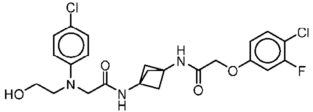 |
| **610** | 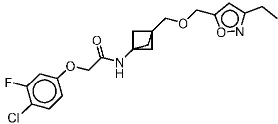 | **611** | 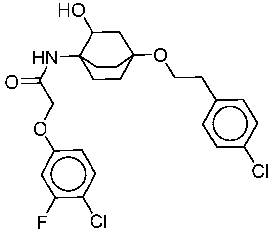 |
| **612** | 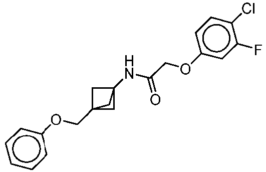 | **613** | 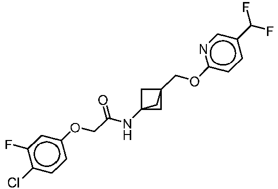 |
| **614** | 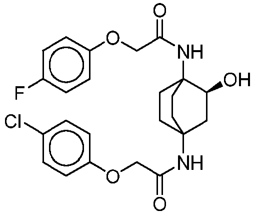 | **615** | 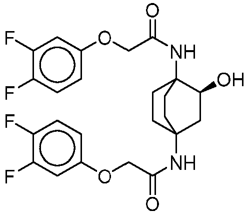 |
| **616** | 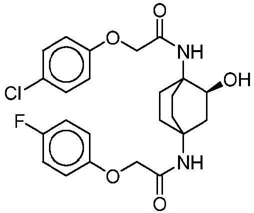 | **617** | 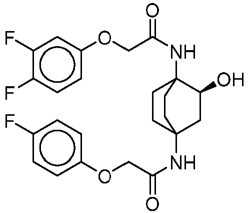 |
| **618** | 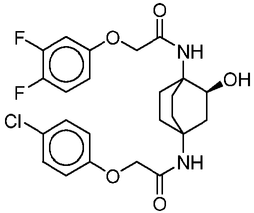 | **619** | 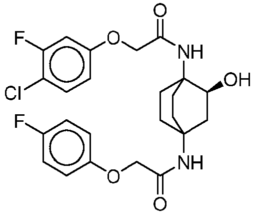 |
| **620** | 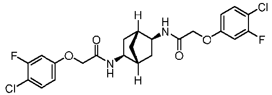 | **621** | 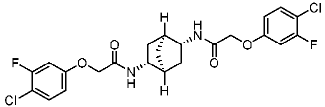 |
| **622** | 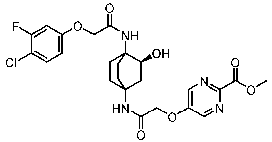 | **623** | 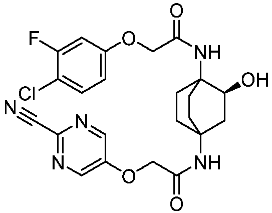 |
| **624** | 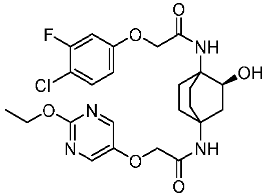 | **625** | 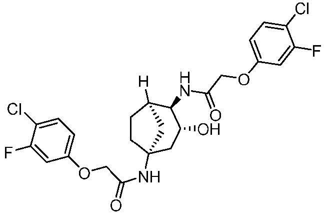 |
| **626** | 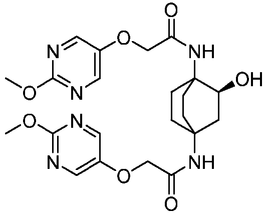 | **627** | 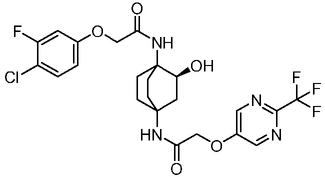 |
| **628** | 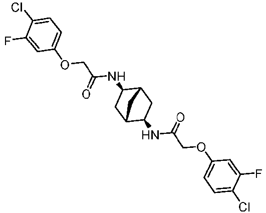 | **629** | 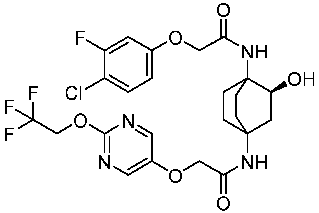 |
| **630** | 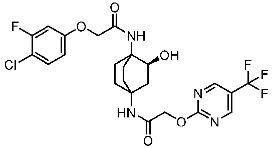 | **631** | 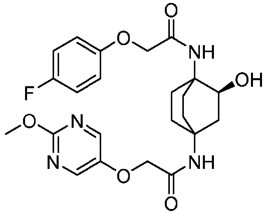 |
| **632** | 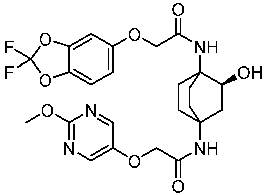 | **633** | 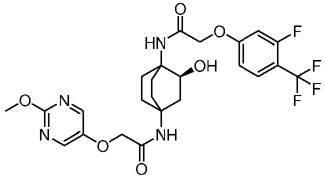 |
| **634** | 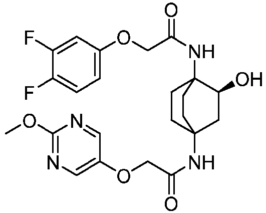 | **635** | 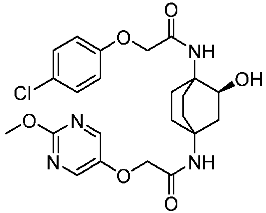 |
| **636** | 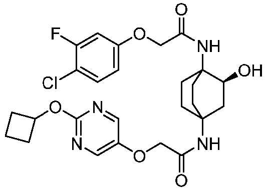 | **637** | 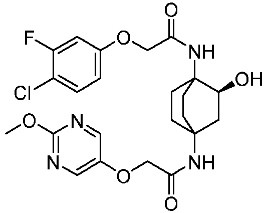 |
| **638** | 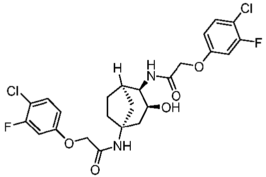 | **639** | 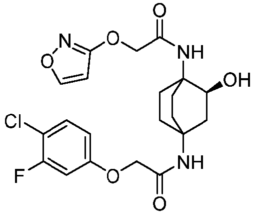 |
| **640** | 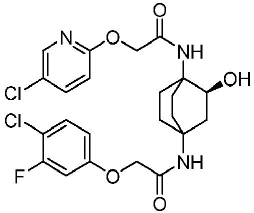 | **641** | 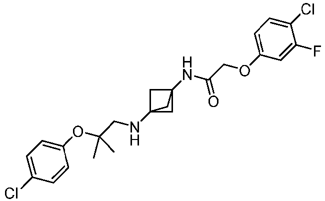 |
| **642** | 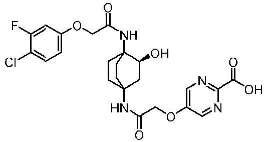 | **643** | 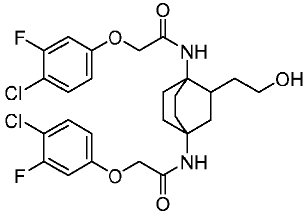 |
| **644** | 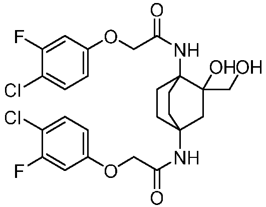 | **645** | 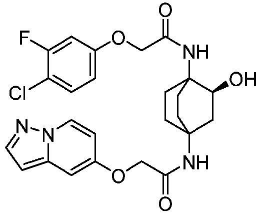 |
| **646** | 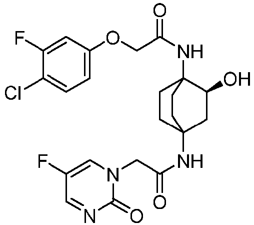 | **647** | 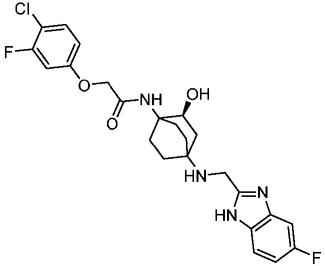 |
| **648** | 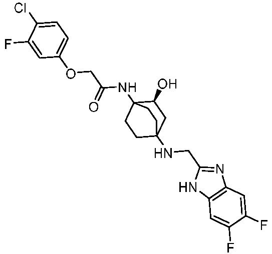 | **649** | 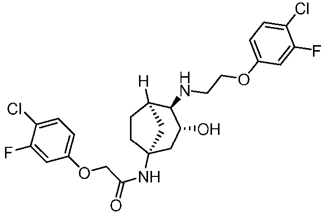 |
| **650** | 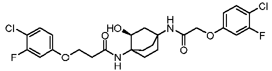 | **651** | 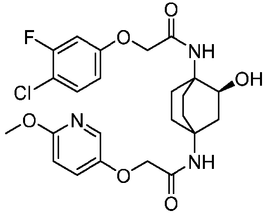 |
| **652** | 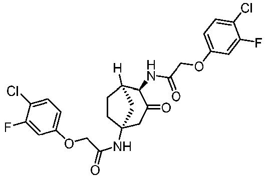 | **653** | 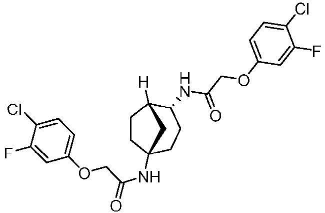 |
| **654** | 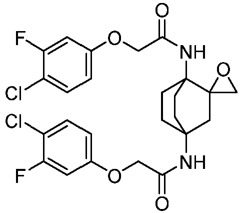 | **655** | 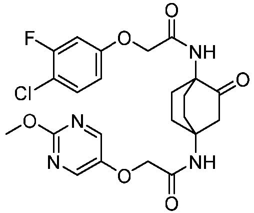 |
| **656** | 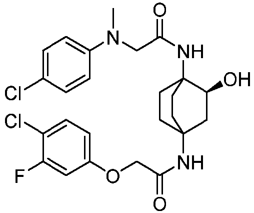 | **657** | 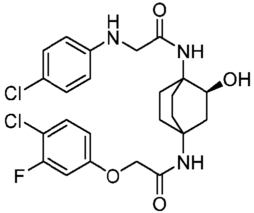 |
| **658** | 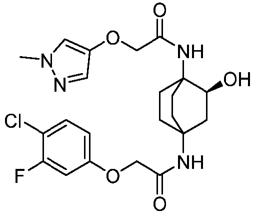 | **659** | 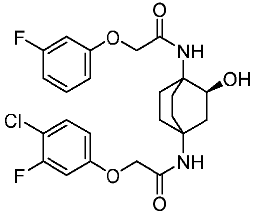 |
| **660** | 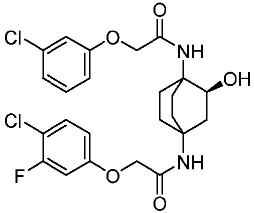 | **661** | 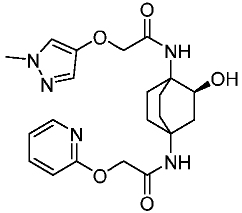 |
| **662** | 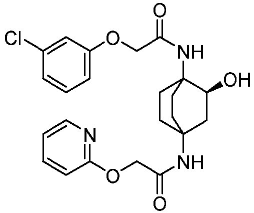 | **663** | 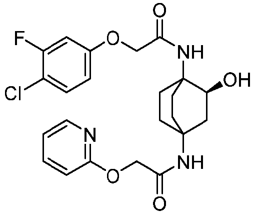 |
| **664** | 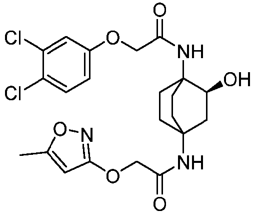 | **665** | 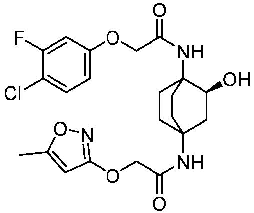 |
| **666** | 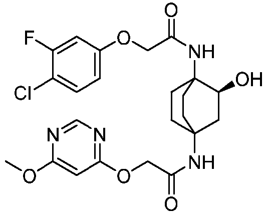 | **667** | 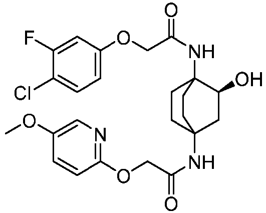 |
| **668** | 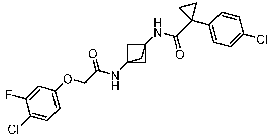 | **669** | 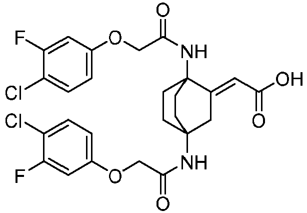 |
| **670** | 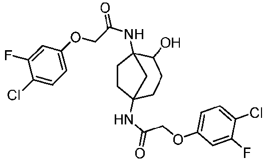 | **671** | 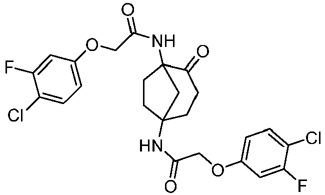 |
| **672** | 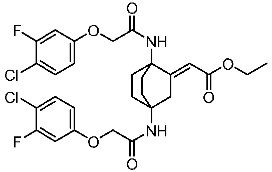 | **673** | 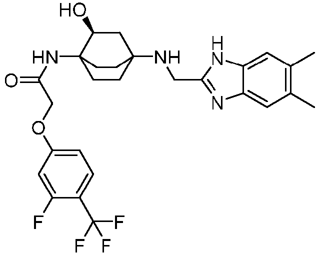 |
| **674** | 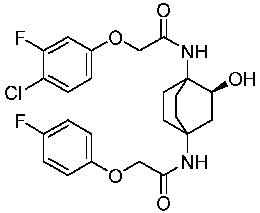 | **675** | 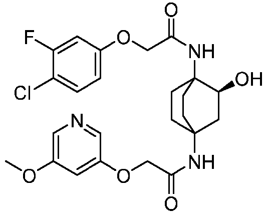 |
| **676** | 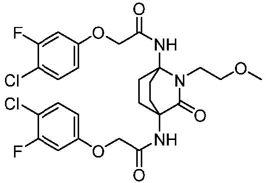 | **677** | 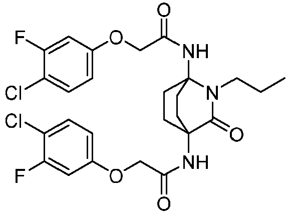 |
| **678** | 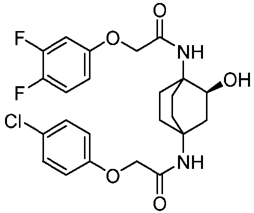 | **679** | 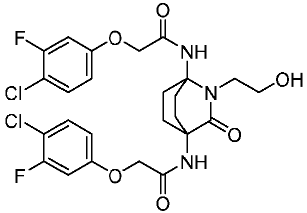 |
| **680** | 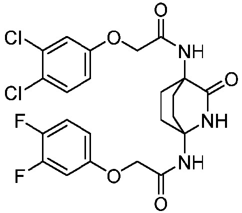 | **681** | 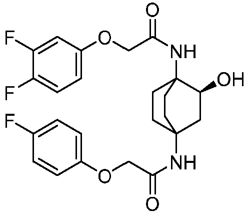 |
| **682** | 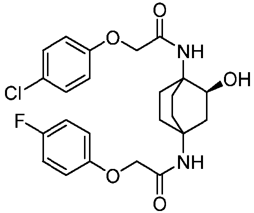 | **683** | 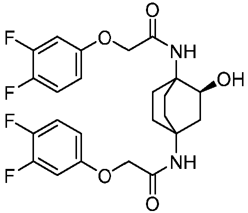 |
| **684** | 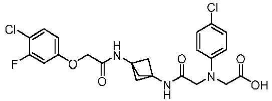 | **685** | 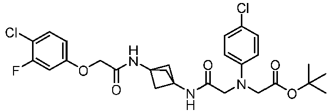 |
| **686** | 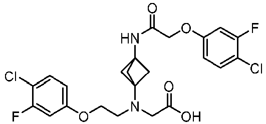 | **687** | 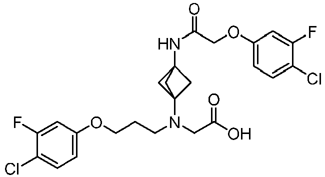 |
| **688** | 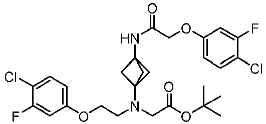 | **689** | 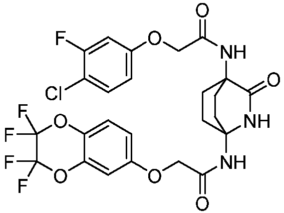 |
| **690** | 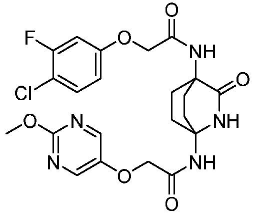 | **691** | 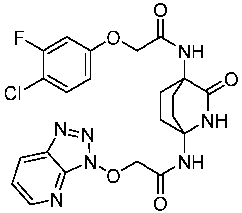 |
| **692** | 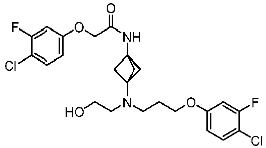 | **693** | 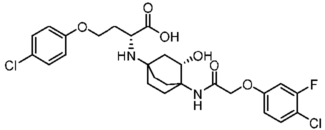 |
| **694** | 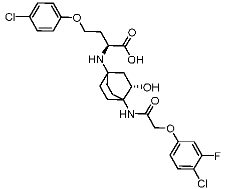 | **695** | 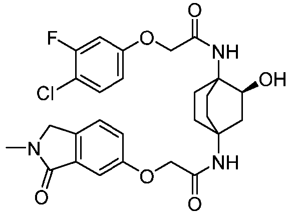 |
| **696** | 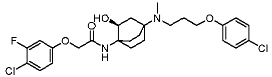 | **697** | 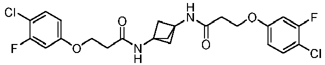 |
| **698** | 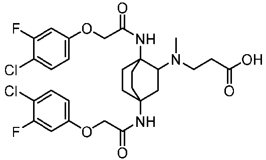 | **699** | 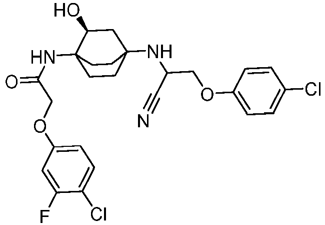 |
| **700** | 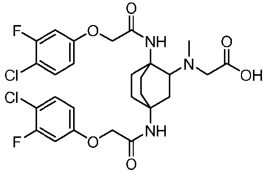 | **701** | 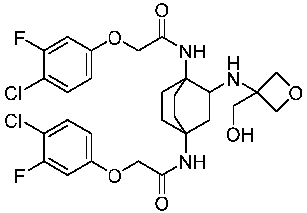 |
| **702** | 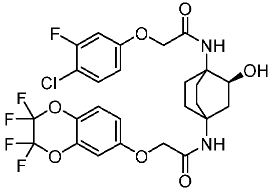 | **703** | 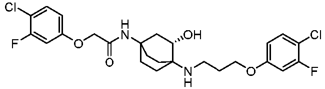 |
| **704** | 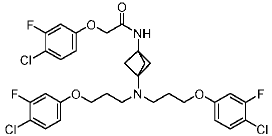 | **705** | 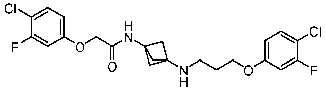 |
| **706** | 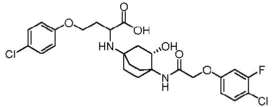 | **707** | 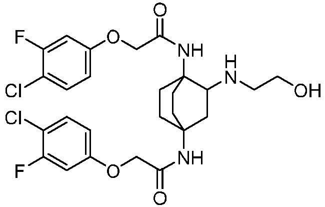 |
| **708** | 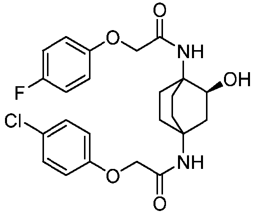 | **709** | 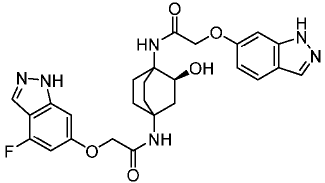 |
| **710** | 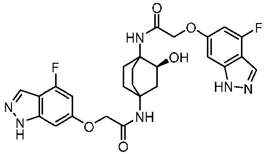 | **711** | 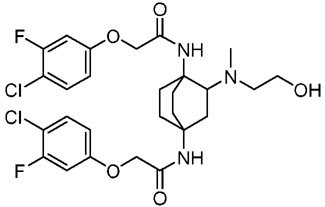 |
| **712** | 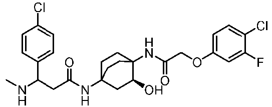 | **713** | 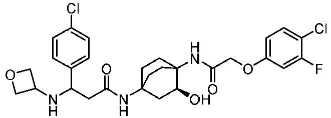 |
| **714** | 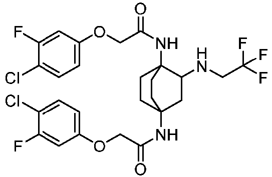 | **715** | 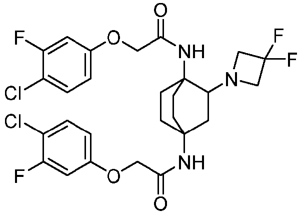 |
| **716** | 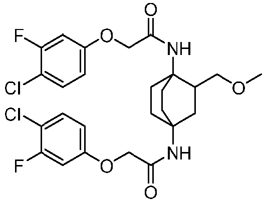 | **717** | 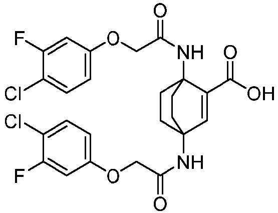 |
| **718** | 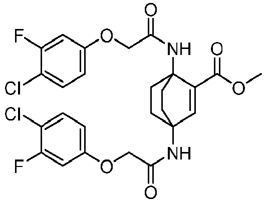 | **719** | 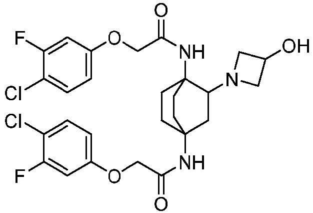 |
| **720** | 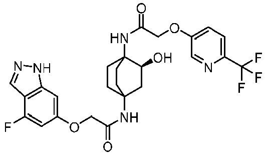 | **721** | 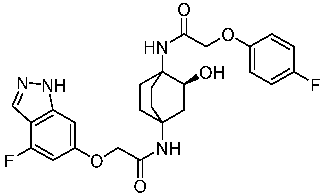 |
| **722** | 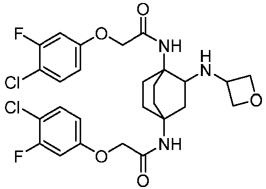 | **723** | 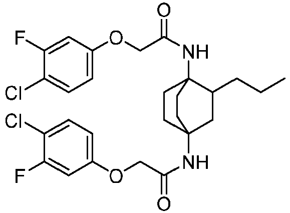 |
| **724** | 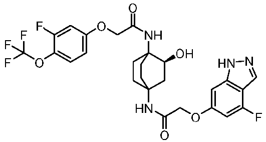 | **725** | 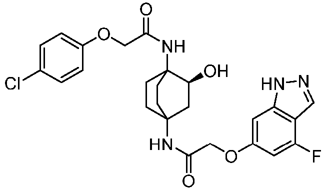 |
| **726** | 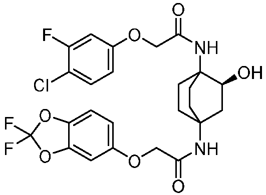 | **727** | 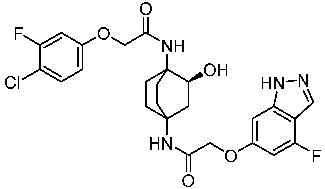 |
| **728** | 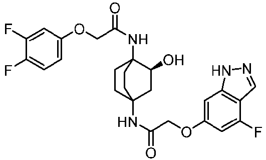 | **729** | 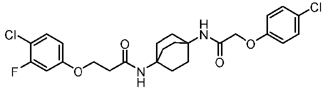 |
| **730** | 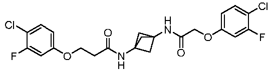 | **731** | 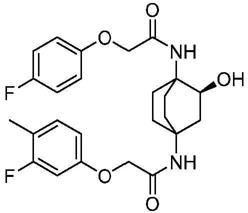 |
| **732** | 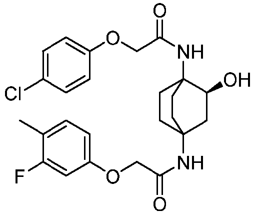 | **733** | 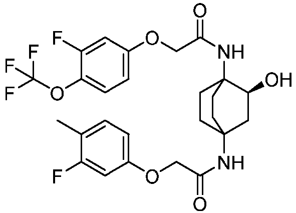 |
| **734** | 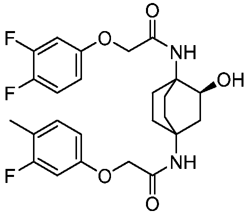 | **735** | 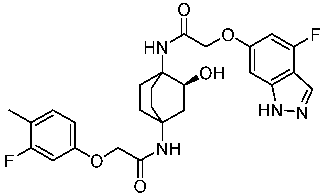 |
| **736** | 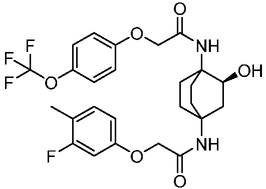 | **737** | 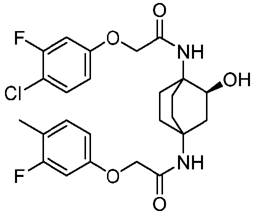 |
| **738** | 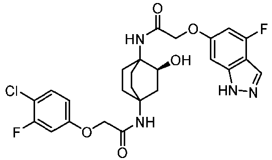 | **739** | 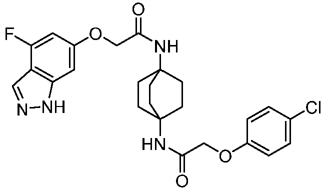 |
| **740** | 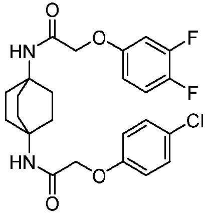 | **741** | 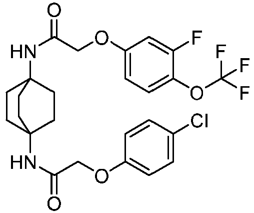 |
| **742** | 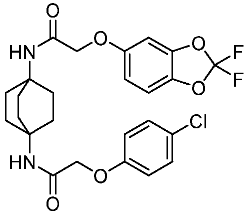 | **743** | 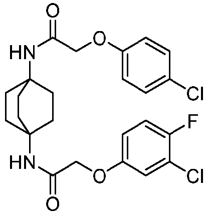 |
| **744** | 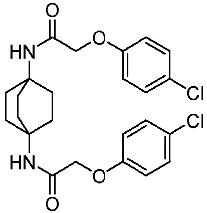 | **745** | 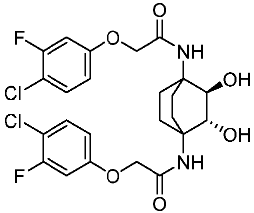 |
| **746** | 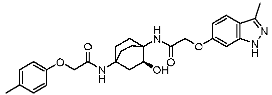 | **747** | 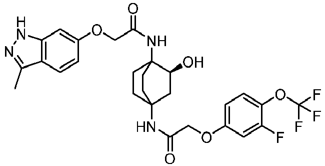 |
| **748** | 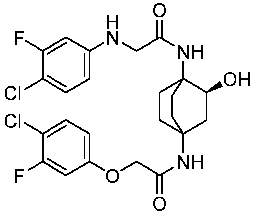 | **749** | 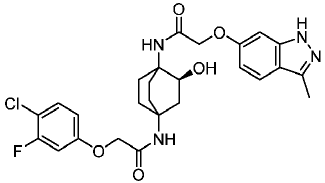 |
| **750** | 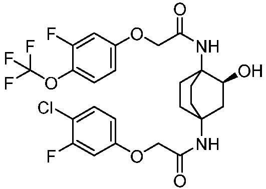 | **751** | 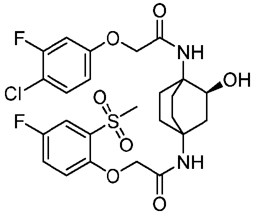 |
| **752** | 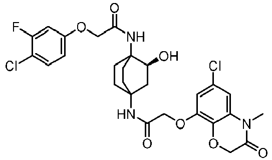 | **753** | 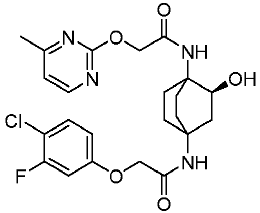 |
| **754** | 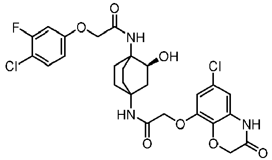 | **755** | 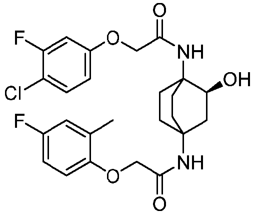 |
| **756** | 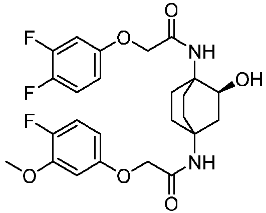 | **757** | 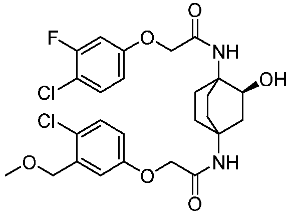 |
| **758** | 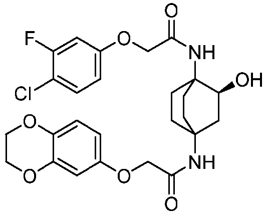 | **759** | 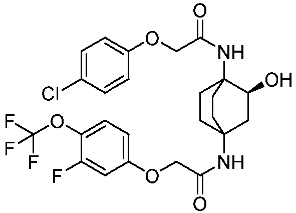 |
| **760** | 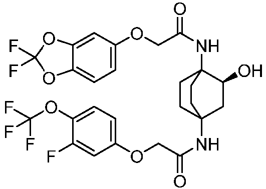 | **761** | 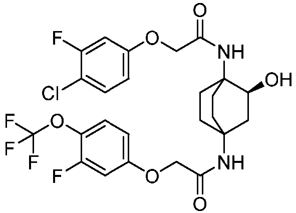 |
| **762** | 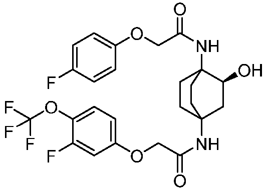 | **763** | 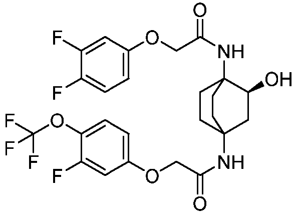 |
| **764** | 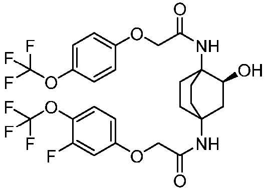 | **765** | 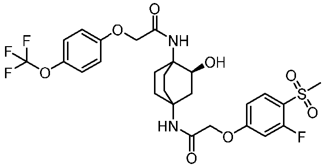 |
| **766** | 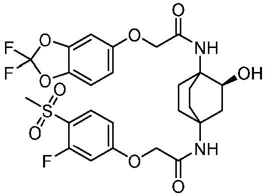 | **767** | 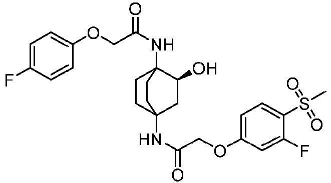 |
| **768** | 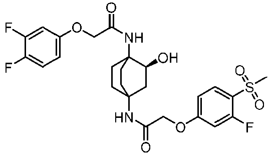 | **769** | 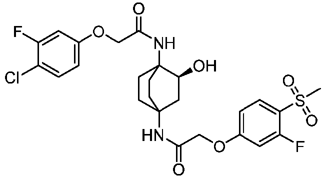 |
| **770** | 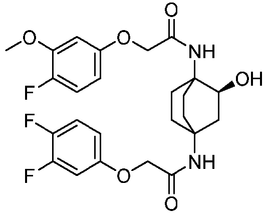 | **771** | 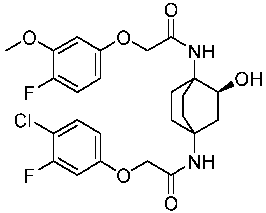 |
| **772** | 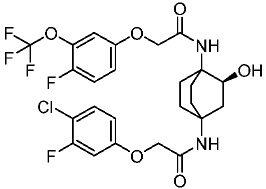 | **773** | 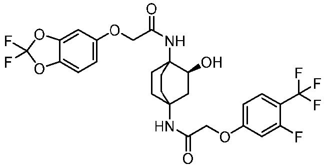 |
| **774** | 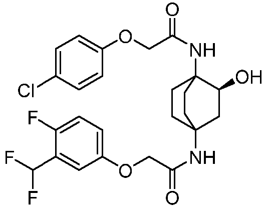 | **775** | 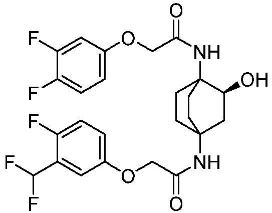 |
| **776** | 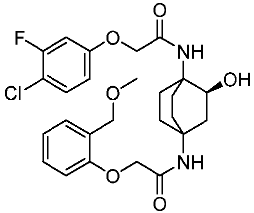 | **777** | 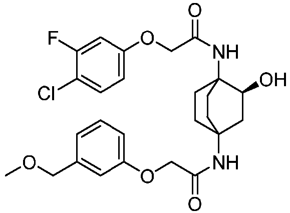 |
| **778** | 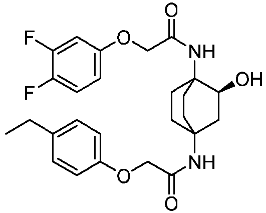 | **779** | 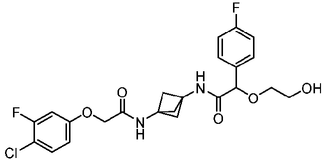 |
| **780** | 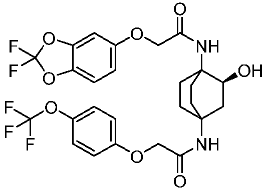 | **781** | 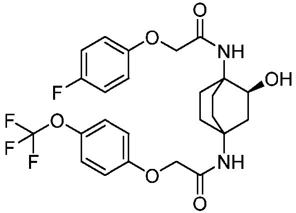 |
| **782** | 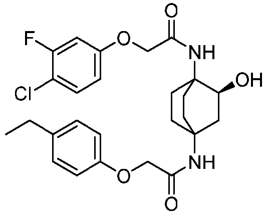 | **783** | 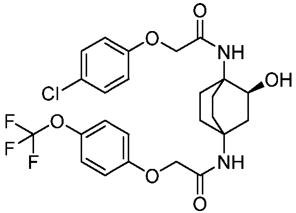 |
| **784** | 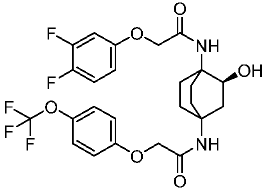 | **785** | 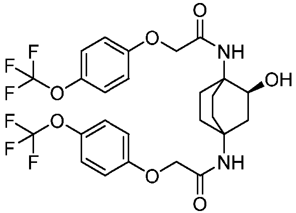 |
| **786** | 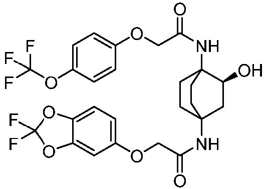 | **787** | 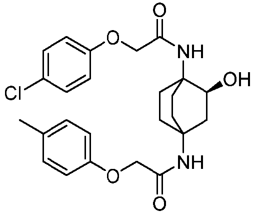 |
| **788** | 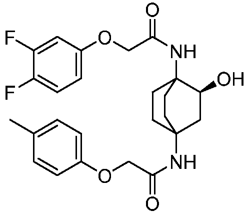 | **789** | 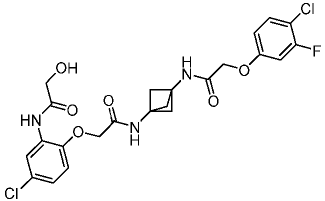 |
| **790** | 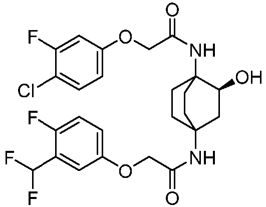 | **791** | 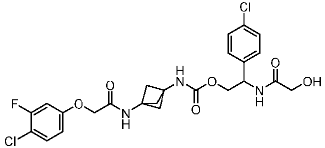 |
| **792** | 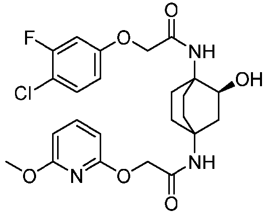 | **793** | 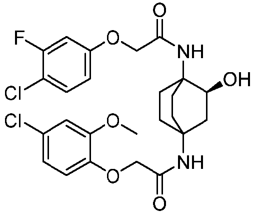 |
| **794** | 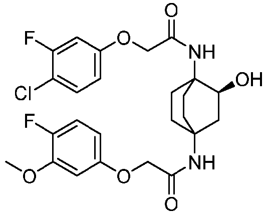 | **795** | 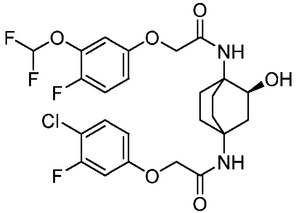 |
| **796** | 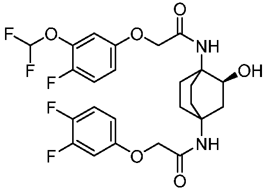 | **797** | 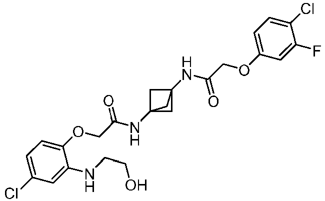 |
| **798** | 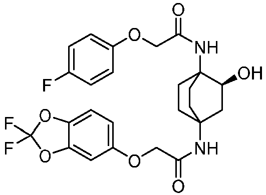 | **799** | 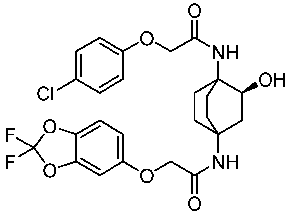 |
| **800** | 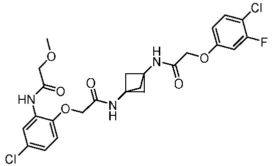 | **801** | 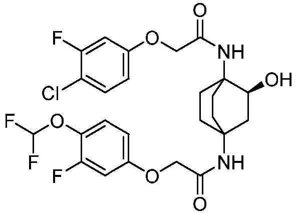 |
| **802** | 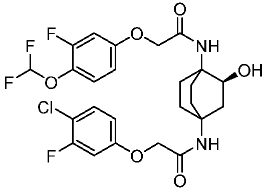 | **803** | 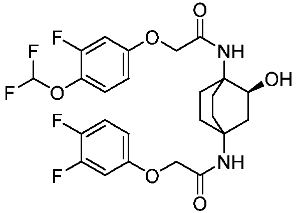 |
| **804** | 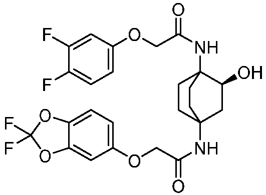 | **805** | 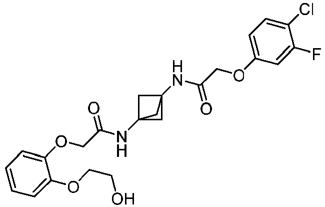 |
| **806** | 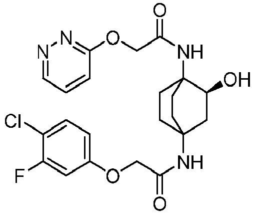 | **807** | 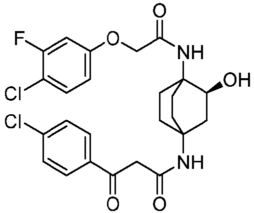 |
| **808** | 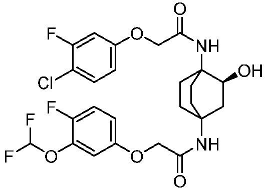 | **809** | 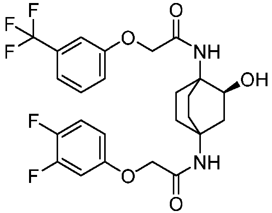 |
| **810** | 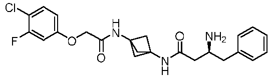 | **811** | 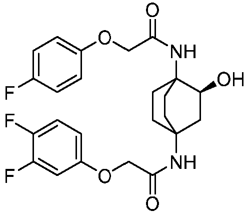 |
| **812** | 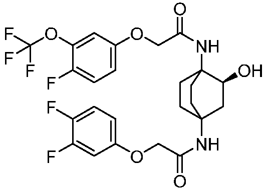 | **813** | 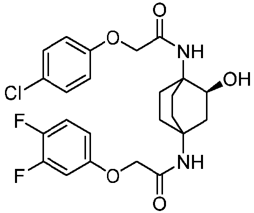 |
| **814** | 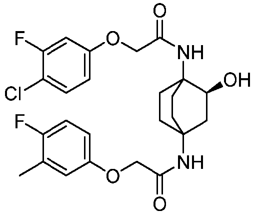 | **815** | 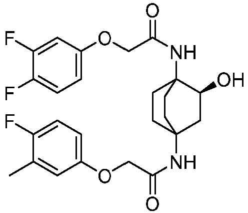 |
| **816** | 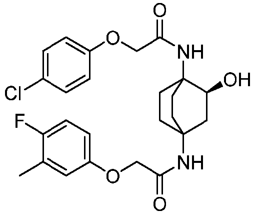 | **817** | 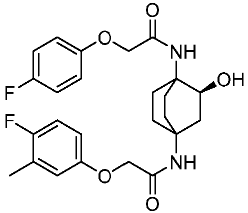 |
| **818** | 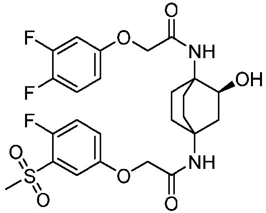 | **819** | 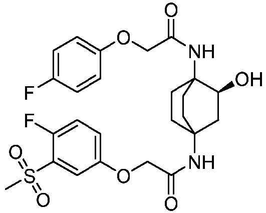 |
| **820** | 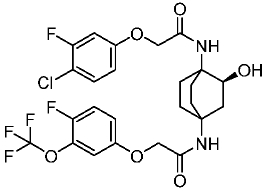 | **821** | 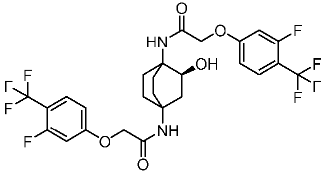 |
| 822 | 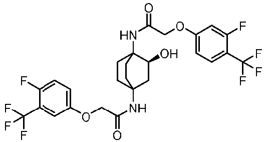 | 823 | 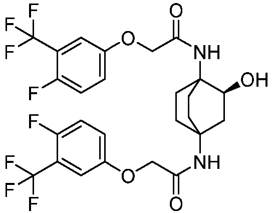 |
| 824 | 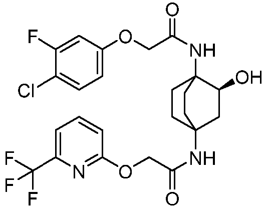 | 825 | 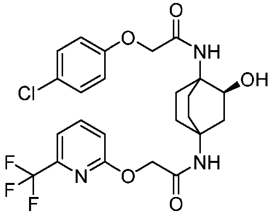 |
| 826 | 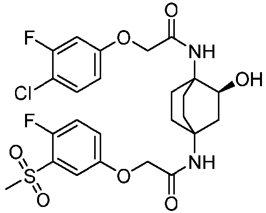 | 827 | 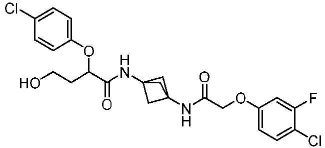 |
| 828 | 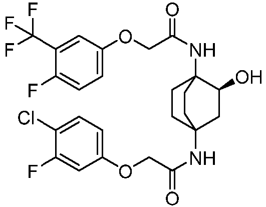 | 829 | 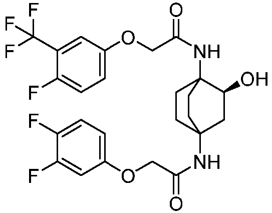 |
| **830** | 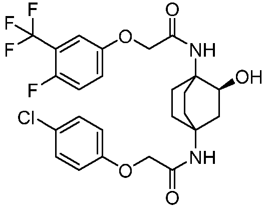 | **831** | 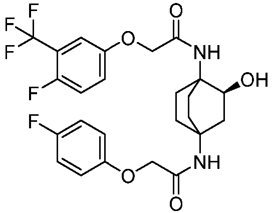 |
| **832** | 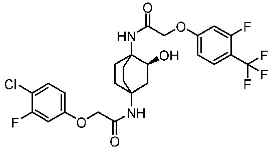 | **833** | 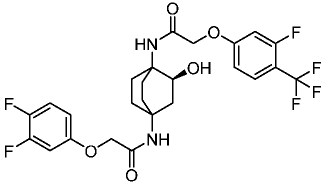 |
| **834** | 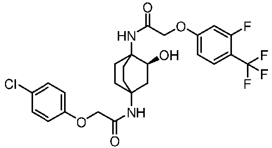 | **835** | 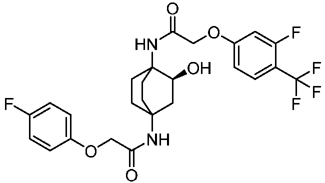 |
| **836** | 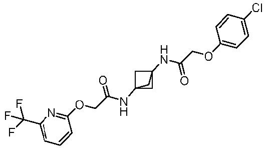 | **837** | 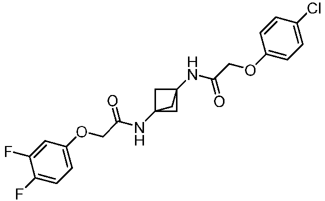 |
| **838** | 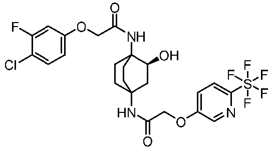 | **839** | 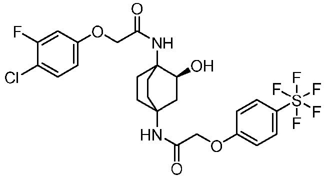 |
| **840** | 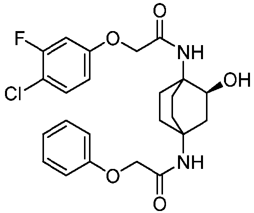 | **841** | 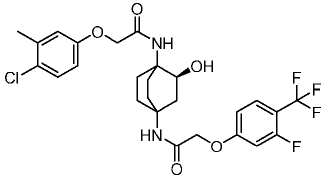 |
| **842** | 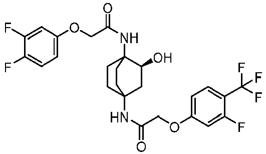 | **843** | 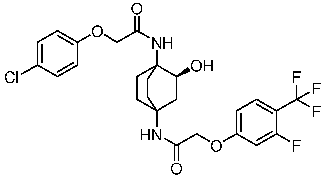 |
| **844** | 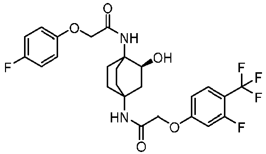 | **845** | 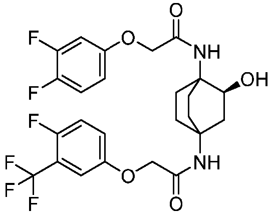 |
| **846** | 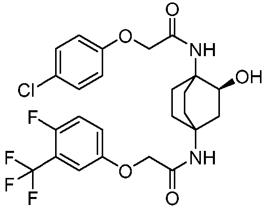 | **847** | 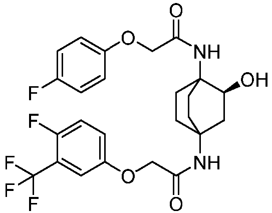 |
| **848** | 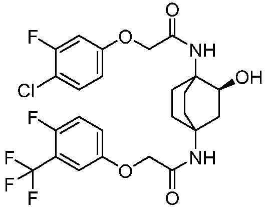 | **849** | 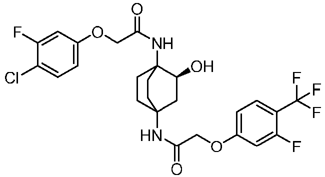 |
| | | | |

### Methods of Making Exemplary Compounds

The compounds of the invention may be better understood in connection with the following synthetic schemes and methods which illustrate a means by which the compounds can be prepared. The compounds of this invention can be prepared by a variety of synthetic procedures. Representative synthetic procedures are shown in, but not limited to, Schemes 1-29. The variables A, D, W, L¹, L², R¹, and R² are defined as detailed herein, e.g., in the Summary

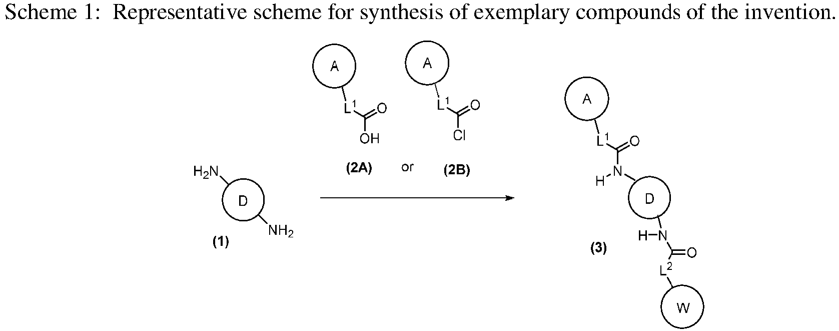

As shown in Scheme 1, compounds of formula (3), when A and W are the same and L¹ and L² are the same, and which are representative of compounds of formula (I), can be prepared from compounds of formula (1). Carboxylic acids of formula (2A) can be coupled with amines of formula (1) under amide bond forming conditions to provide compounds of formula (3). Examples of conditions known to generate amides from a mixture of a carboxylic acid and an amine include, but are not limited to, adding a coupling reagent such as, but not limited to, *N-*(3-dimethylaminopropyl)-*N*'-ethylcarbodiimide or 1-(3-dimethylaminopropyl)-3-ethylcarbodiimide (EDC, EDAC or EDCI) or the corresponding hydrochloride salt, 1,3-dicyclohexylcarbodiimide (DCC), bis(2-oxo-3-oxazolidinyl)phosphinic chloride (BOPCl), *N*-[(dimethylamino)-1*H*-1,2,3-triazolo-[4,5-*b*]pyridin-1-ylmethylene]-*N*-methylmethanaminium hexafluorophosphate *N*-oxide or 2-(7-azabenzotriazol-1-yl)-*N,N,N',N*'-tetramethyluronium hexafluorophosphate or 1-[bis(dimethylamino)methylene]-1H-1,2,3-triazolo[4,5-b]pyridinium 3-oxid hexafluorophosphate (HATU), *O*-(benzotriazol-1-yl)-*N,N,N',N'*-tetramethyluronium tetrafluoroborate (TBTU), 2-(1*H-*benzo[*d*][1,2,3]triazol-1-yl)-1,1,3,3-tetramethylisouronium hexafluorophosphate(V) (HBTU), and 2,4,6-tripropyl-1,3,5,2,4,6-trioxatriphosphinane 2,4,6-trioxide (T3P^{®}). The coupling reagents may be added as a solid, a solution, or as the reagent bound to a solid support resin.

In addition to the coupling reagents, auxiliary-coupling reagents may facilitate the coupling reaction. Auxiliary coupling reagents that are often used in the coupling reactions include but are not limited to (dimethylamino)pyridine (DMAP), 1-hydroxy-7-azabenzotriazole (HOAT) and 1-hydroxybenzotriazole (HOBT). The reaction may be carried out optionally in the presence of a base such as, but not limited to, triethylamine, *N,N*-diisopropylethylamine or pyridine. The coupling reaction may be carried out in solvents such as, but not limited to, tetrahydrofuran, *N,N*-dimethylformamide, *N,N*-dimethylacetamide, dimethyl sulfoxide, dichloromethane, and ethyl acetate. The reactions may be carried out at ambient temperature or heated. The heating can be accomplished either conventionally or with microwave irradiation.

Alternatively, acid chlorides of formula (2B) can be reacted with amines of formula (1), optionally in the presence of a base for example, a tertiary amine base such as, but not limited to, triethylamine or *N,N*-diisopropylethylamine or an aromatic base such as pyridine, at room temperature or heated in a solvent such as, but not limited to, dichloromethane to provide amides of formula (3). Amines of formula (1) can also be coupled with acid chlorides of formula (2B) in a mixture of water and dichloromethane in the presence of a base such as but not limited to sodium hydroxide.

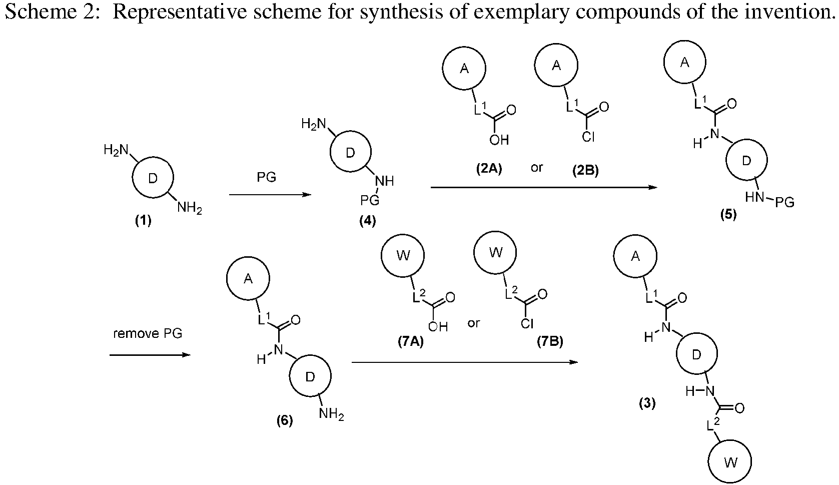

As shown in Scheme 2, compounds of formula (3), when A and W are the same or different and L¹ and L² are the same or different, and which are representative of compounds of formula (I), can be prepared from compounds of formula (1). Amines of formula (1) can be protected with a suitable protecting group (PG) to provide compounds of formula (4). For example, amines of formula (1) can be treated with di-*tert*-butyl dicarbonate at ambient temperature in a solvent such as, but not limited to, tetrahydrofuran to provide compounds of formula (4) wherein PG is C(O)OC(CH₃)₃. Carboxylic acids of formula (2A) or acid chlorides of formula (2B) can be coupled with amines of formula (4) under amide bond forming conditions described in Scheme 1 to provide compounds of formula (5). The protecting group (PG) in formula (5) can be removed to provide compounds of formula (6). For example, BOC protecting groups can be removed using an acid such as, but not limited to, trifluoroacetic acid or hydrochloric acid in a solvent such as, but not limited to, methanol, 1,4-dioxane or dichloromethane, or mixtures thereof. The reaction may be performed at ambient or an elevated temperature. Carboxylic acids of formula (7A) or acid chlorides of formula (7B) can be coupled with amines of formula (6) under amide bond forming conditions described in Scheme 1 to provide compounds of formula (3), which are representative of compounds of formula (I).

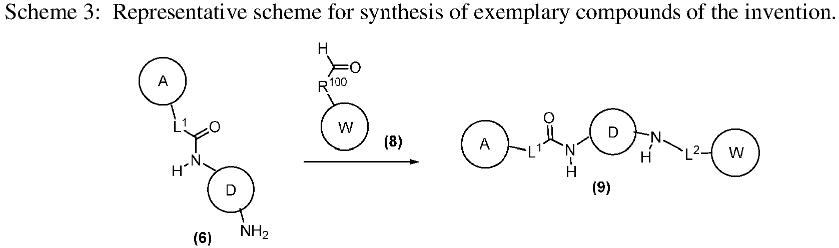

As shown in Scheme 3, compounds of formula (9), which are representative of compounds of formula (I) when t is 0, can be prepared from compounds of formula (6). Compounds of formula (6), which can be prepared as described in Scheme 2, can be reacted with an aldehyde of formula (8), wherein R¹⁰⁰ is absent or is alkylene or heteroalkylene, in the presence of a reducing agent such as, but not limited to, sodium triacetoxyborohydride or sodium cyanoborohydride, to provide compounds of formula (9). The reaction is typically performed at ambient temperature in a solvent such as, but not limited to, 1,2-dichloroethane, dichloromethane, methanol, ethanol, tetrahydrofuran, acetonitrile, or mixtures thereof.

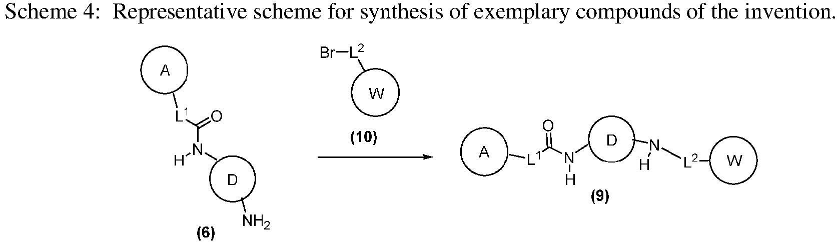

Alternatively, compounds of formula (9), which are representative of compounds of formula (I) when t is 0, can be prepared from compounds of formula (6) as shown in Scheme 4. Amines of formula (6) can be reacted with bromides of formula (10), in the presence of a base such as, but not limited to, potassium carbonate, to provide compounds of formula (9). The reaction is typically performed at an elevated temperature in a solvent such as, but not limited to, *N*,*N*-dimethylformamide or dimethyl sulfoxide.

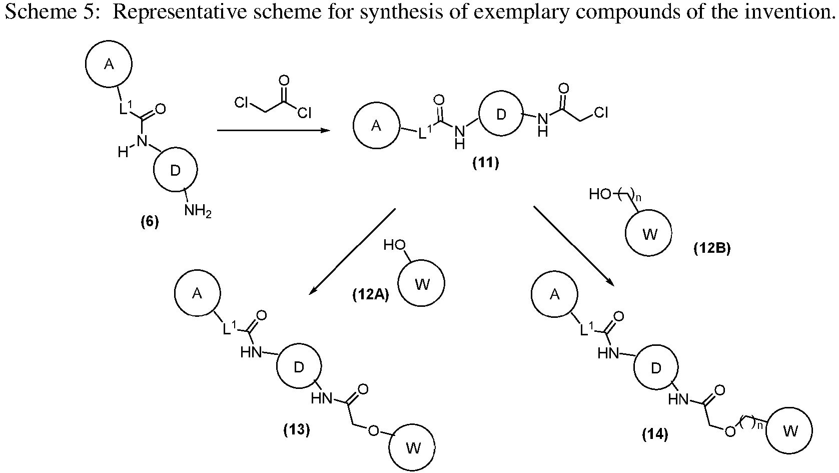

Compounds of formula (13) and compounds of formula (14), which are representative of compounds of formula (I) wherein t is 1, can be prepared as shown in Scheme 5. Amines of formula (6), which can be prepared as described in Scheme 2, can be treated with 2-chloroacetyl chloride in the presence of a base such as, but not limited to, potassium carbonate, to provide compounds of formula (11). The addition is typically performed at low temperature before warming up to ambient temperature in a solvent such as, but not limited to, tetrahydrofuran, water, or mixtures thereof. Alcohols of formula (12A) can be reacted with compounds of formula (11) in the presence of a strong base, such as but not limited to sodium hydride, to provide compounds of formula (13). The reaction is typically performed at ambient temperature in a solvent such as but not limited to *N*,*N*-dimethylformamide. Alternatively, alcohols of formula (12A) can be reacted with compounds of formula (11) in the presence of a base, such as but not limited to potassium carbonate, optionally with the addition of a catalytic amount of potassium iodide, to provide compounds of formula (13). The reaction is typically performed at an elevated temperature, optionally in a microwave, and in a solvent such as, but not limited to, acetonitrile, acetone, or mixtures thereof. Alcohols of formula (12B), wherein n is 1-6, can be reacted with compounds of formula (11) in the presence of a strong base, such as but not limited to sodium hydride, to provide compounds of formula (14). The reaction is typically performed at ambient temperature in a solvent, such as but not limited to, *N*,*N*-dimethylformamide.

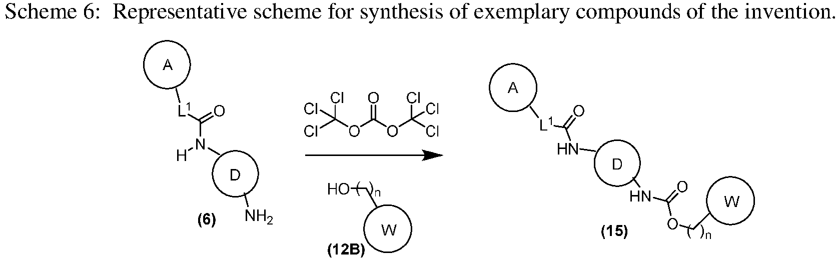

A shown in Scheme 6, compounds of formula (15), which are representative of compounds of formula (I) wherein t is 1 and L² is C₂-C₇ heteroalkylene, can be prepared from compounds of formula (6). Amines of formula (6) can be treated with bis(trichloromethyl) carbonate, followed by alcohols of formula (12B), to provide compounds of formula (15). The reaction is typically performed at ambient temperature in a solvent such as but not limited to tetrahydrofuran.

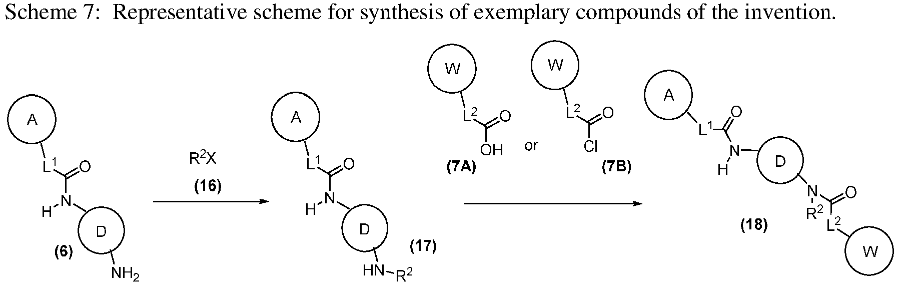

Compounds of formula (18), which are representative of compounds of formula (I) wherein t is 1 and R² is C₁-C₆ alkyl, C₁-C₆ alkoxy-C₁-C₆ alkyl, hydroxy-C₁-C₆ alkyl, silyloxy-C₁-C₆ alkyl, or C₁-C₆ alkyl-C(O)₂-C₁-C₆ alkyl can be prepared from compounds of formula (6) as shown in Scheme 7. Amines of formula (6) can be alkylated with an alkylating agent of formula (16) wherein X is a halide, in the presence of a base such as, but not limited to, potassium carbonate, to provide compounds of formula (17). The reaction is typically performed at an elevated temperature in a solvent such as, but not limited to, *N*,*N*-dimethylformamide. Carboxylic acids of formula (7A) or acid chlorides of formula (7B) can be coupled with amines of formula (17) under amide bond forming conditions described in Scheme 1 to provide compounds of formula (18).

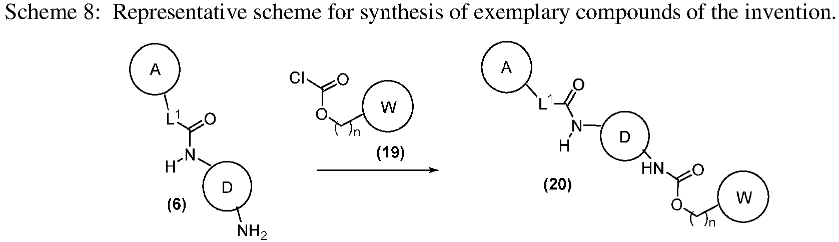

As shown in Scheme 8, compounds of formula (20), which are representative of compounds of formula (I) wherein t is 1, can be prepared from amines of formula (6). Amines of formula (6) can be reacted with chloroformates of formula (19) in the presence of a base such as, but not limited to, *N*,*N*-diisopropylethylamine, to provide compounds of formula (20). The reaction is typically performed at ambient temperature in a solvent such as but not limited to toluene, dichloromethane, or mixtures thereof.

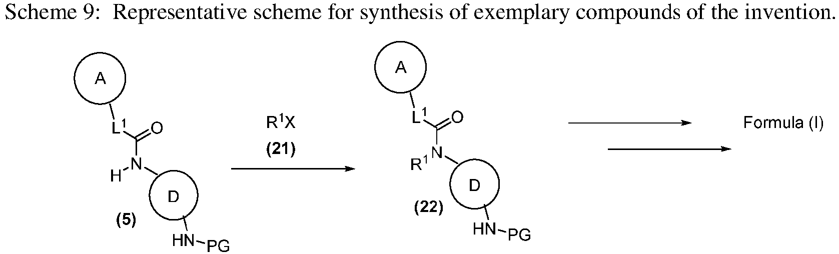

Compounds of formula (I), wherein R¹ is C₁-C₆ alkyl, C₁-C₆ alkoxy-C₂-C₆ alkyl, hydroxy-C₂-C₆ alkyl, or silyloxy-C₂-C₆ alkyl, can be prepared from compounds of formula (5) as shown in Scheme 9. Amines of formula (5), which can be prepared as described in Scheme 2, can be alkylated with an alkylating agent of formula (21), wherein X is a halide, in the presence of a base such as but not limited to sodium hydride, to provide compounds of formula (22). The reaction is typically performed at ambient temperature in a solvent such as, but not limited to, tetrahydrofuran, *N*,*N*-dimethylacetamide, *N*,*N*-dimethylformamide, or mixtures thereof. After removal of the protecting group (PG), compounds of formula (22) can be reacted with carboxylic acids of formula (7A) or acid chlorides of formula (7B) under amide bond forming conditions described in Scheme 2 to provide compounds of formula (I).

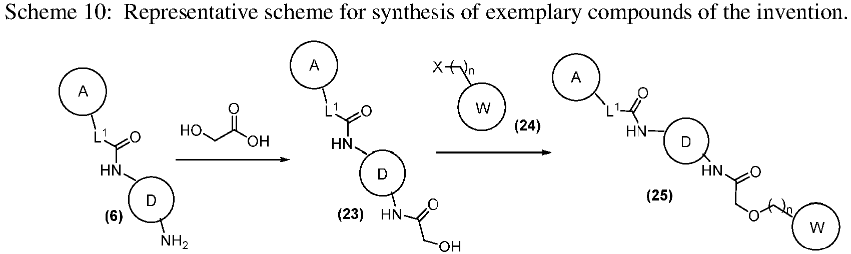

As shown in Scheme 10, compounds of formula (25), which are representative of compounds of formula (I) wherein t is 1, can be prepared from compounds of formula (6). Amines of formula (6), which can be prepared as described in Scheme 2, can be reacted with 2-hydroxyacetic acid to provide compounds of formula (23) under amide bond forming conditions described in Scheme 2. Compounds of formula (23) can be alkylated with an alkylating agent of formula (24), wherein X is a halide and n is 0-5, in the presence of a base such as but not limited to sodium hydride, to provide compounds of formula (25). The reaction is typically performed at an elevated temperature in a solvent such as, but not limited to, tetrahydrofuran *N,N-*dimethylformamide, or mixtures thereof.

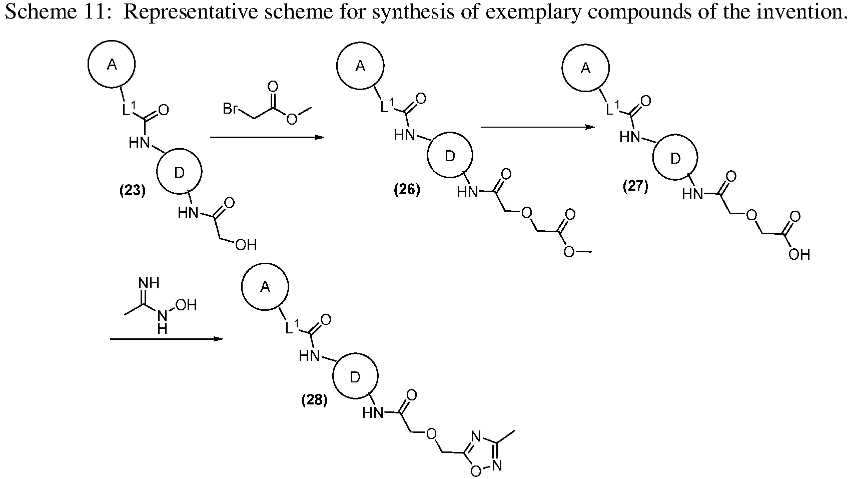

Compounds of formula (28), which are representative of compounds of formula (I),wherein t is 1, can be prepared from compounds of formula (23) as shown in Scheme 11. Compounds of formula (23), which can be prepared as described in Scheme 10, can be reacted with methyl 2-bromoacetate in the presence of a base such as, but not limited to, cesium carbonate to provide compounds of formula (26). The reaction is typically performed at ambient temperature in a solvent such as, but not limited to, tetrahydrofuran. Compounds of formula (26) can be treated with aqueous lithium hydroxide to provide compounds of formula (27). The reaction is typically performed at ambient temperature in a solvent such as, but not limited to, tetrahydrofuran, methanol, or mixtures thereof. Compounds of formula (27) can be treated with *N*-hydroxyacetimidamide in the presence of a coupling agent such as, but not limited to, 1-[bis(dimethylamino)methylene]-1H-1,2,3-triazolo[4,5-b]pyridinium 3-oxid hexafluorophosphate (HATU), and a base such as, but not limited to, triethylamine, to provide compounds of formula (28). The reaction is typically performed at an elevated temperature in a solvent such as but not limited to acetonitrile.

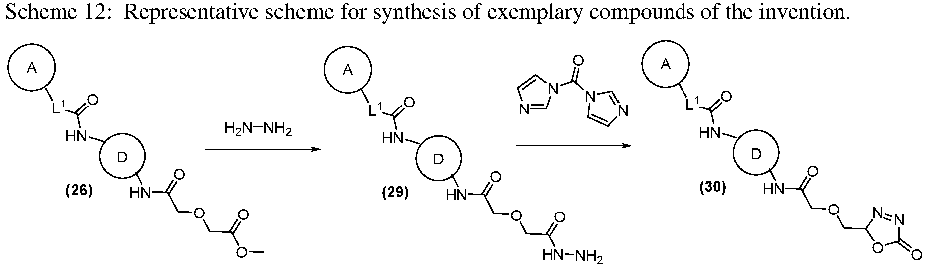

As shown in Scheme 12, compounds of formula (30), which are representative of compounds of formula (I), can be prepared from compounds of formula (26). Compounds of formula (26), which can be prepared as described in Scheme 11, can be treated with hydrazine monohydrate, to provide compounds of formula (29). The reaction is typically performed at an elevated temperature in a solvent such as, but not limited to, ethanol. Compounds of formula (29) can be treated with 1,1'-carbonyldiimidazole, to provide compounds of formula (30). The reaction is typically performed at an elevated temperature in a solvent such as but not limited to 1,4-dioxane.

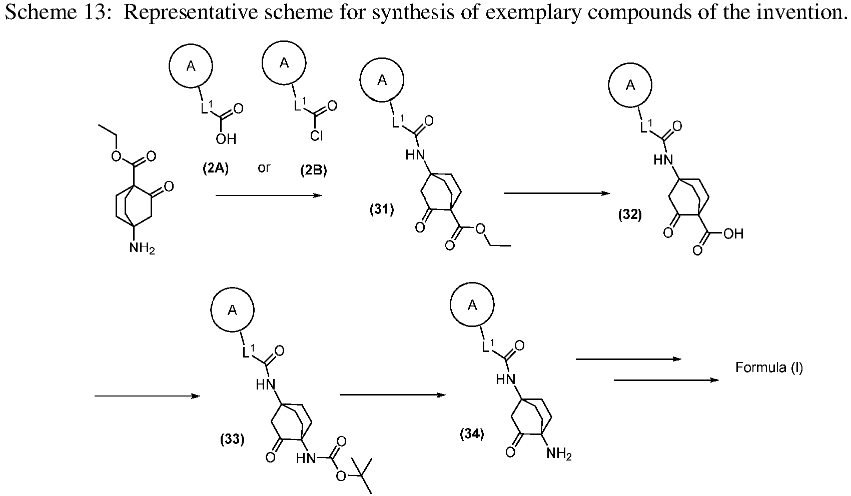

Scheme 13 describes the synthesis of compounds of formula (I) wherein D is a 2-oxobicyclo[2.2.2]octan-1-yl core. Ethyl 4-amino-2-oxobicyclo[2.2.2]octane-1-carboxylate, which can be prepared as described herein, can be reacted with carboxylic acids of formula (2A) or acid chlorides of formula (2B) under amide bond forming conditions described in Scheme 1 to provide compounds of formula (31). Compounds of formula (31) can be treated with a methanolic solution of sodium hydroxide at ambient temperature to provide compounds of formula (32). Acids of formula (32) can be treated with diphenylphosphoryl azide, in the presence of a base such as but not limited to triethylamine, followed by treatment with *tert-*butanol, to provide compounds of formula (33). The reaction is typically performed at an elevated temperature in a solvent such as, but not limited to, toluene. Compounds of formula (33) can be treated with an acid such as, but not limited to, hydrochloric acid at ambient temperature in a solvent such as, but not limited to, 1,4-dioxane, to provide compounds of formula (34). Compounds of formula (34) can be reacted with carboxylic acids of formula (7A) or acid chlorides of formula (7B) under amide bond forming conditions described in Scheme 2 to provide compounds of formula (I).

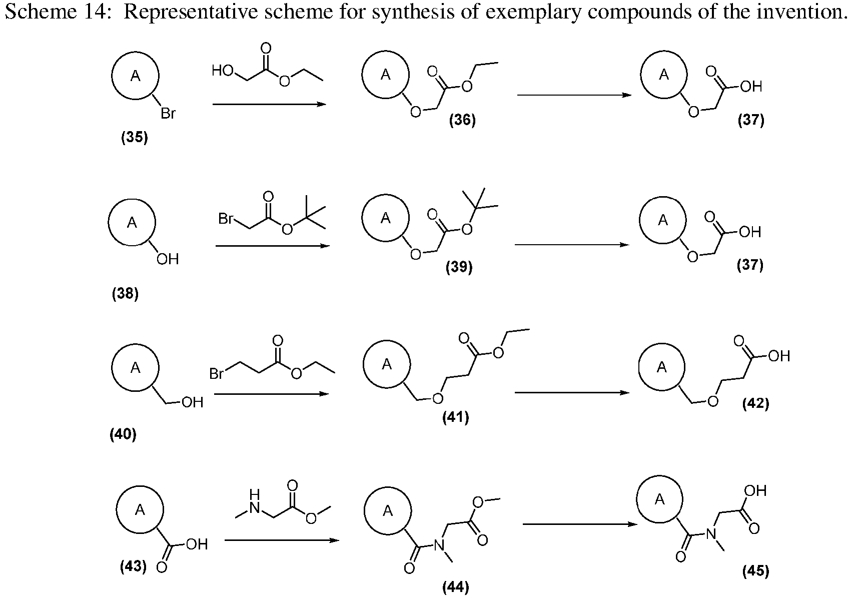

Scheme 14 describes the synthesis of carboxylic acids (37), (42), and (45), which are representative of acids of formula (2A) and formula (7A).

Compounds of formula (35) can be reacted with ethyl 2-hydroxyacetate in the presence of a strong base such as, but not limited to, potassium *tert*-butoxide to provide compounds of formula (36). The reaction is typically performed at ambient temperature in a solvent such as, but not limited to, tetrahydrofuran. Compounds of formula (36) can be treated with aqueous lithium hydroxide to provide compounds of formula (37). The reaction is typically performed at ambient temperature in a solvent such as, but not limited to, tetrahydrofuran.

Alcohols of formula (38) can be reacted with *tert-*butyl 2-bromoacetate in the presence of a base such as, but not limited to, potassium carbonate to provide compounds of formula (39). The reaction is typically performed at an elevated temperature in a solvent such as, but not limited to, *N*,*N*-dimethylformamide. Compounds of formula (39) can be treated with an acid such as, but not limited to, hydrochloric acid to provide compounds of formula (37). The reaction is typically performed at ambient temperature in a solvent such as, but not limited to, 1,4-dioxane.

Compounds of formula (40) can be reacted with ethyl 3-bromopropanoate in the presence of a strong base such as, but not limited to, sodium hydride, to provide compounds of formula (41). The addition is typically performed at low temperature before warming to ambient temperature, in a solvent such as, but not limited to, tetrahydrofuran. Compounds of formula (41) can be treated with aqueous sodium hydroxide to provide compounds of formula (42). The reaction is typically performed at ambient temperature in a solvent such as, but not limited to, tetrahydrofuran.

Carboxylic acids of formula (43) can be reacted with sarcosine methyl ester, under amide bond forming conditions as described in Scheme 1, to provide compounds of formula (44). Compounds of formula (44) can be treated with aqueous sodium hydroxide to provide compounds of formula (45). The reaction is typically performed at ambient temperature in a solvent such as, but not limited to, ethanol.

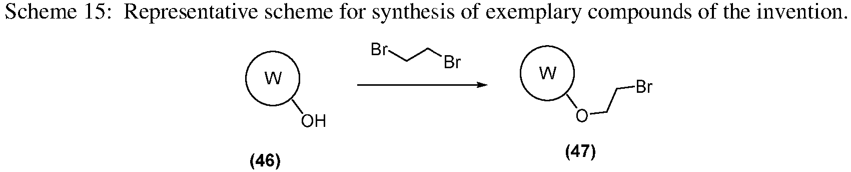

As shown in Scheme 15, bromides of formula (47), which are representative of compounds of formula (10) and (24), can be prepared from alcohols of formula (46). Compounds of formula (46) can be reacted with 1,2-dibromoethane in the presence of a base such as, but not limited to, potassium carbonate, to provide compounds of formula (47). The reaction is typically performed at elevated temperature in a solvent such as, but not limited to, acetonitrile. 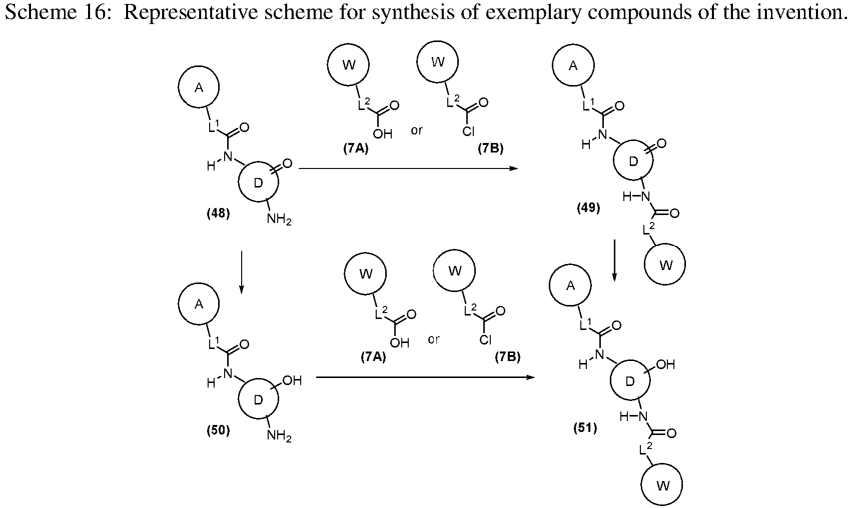 As shown in Scheme 16, compounds of formula (49), wherein A and W are the same or different and L¹ and L² are the same or different, and which are representative of compounds of formula (I), can be prepared from compounds of formula (48). Carboxylic acids of formula (7A) or acid chlorides of formula (7B) can be coupled with amines of formula (48) under amide bond forming conditions described in Scheme 1 to provide compounds of formula (49). Ketones of formula (48) can also be reduced in the presence of a reducing agent such as, but not limited to sodium borohydride in solvents such as a mixture of methanol and dichloromethane to give alcohols of formula (50). Carboxylic acids of formula (7A) or acid chlorides of formula (7B) can be coupled with amines of formula (50) under amide bond forming conditions described in Scheme 1 to provide compounds of formula (51). Ketones of formula (49) can also be reduced in the presence of a reducing agent such as, but not limited to sodium borohydride in solvents such as a mixture of methanol and dichloromethane to give alcohols of formula (51). Compounds of formula (48), formula (49), formula (50) and formula (51) can be further derivatized as illustrated in the Examples below.

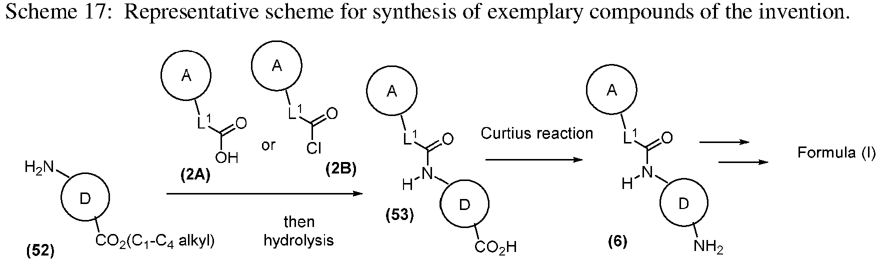

As shown in Scheme 17, compounds of formula (52) can be transformed to compounds of formula (6) which in turn through the methods described in Schemes 2-8 and 10 can be converted to compounds of formula (I). Accordingly, carboxylic acids of formula (2A) or acid chlorides of formula (2B) can be coupled with amines of formula (50) under amide bond forming conditions described in Scheme 1 followed by ester hydrolysis using conditions known to one of skill in the art to provide compounds of formula (53). Compounds of formula (53) can be reacted under Curtius reaction conditions such as treatment with diphenylphosphoryl azide and triethylamine in heated toluene followed by acid hydrolysis to give compounds of formula (6).

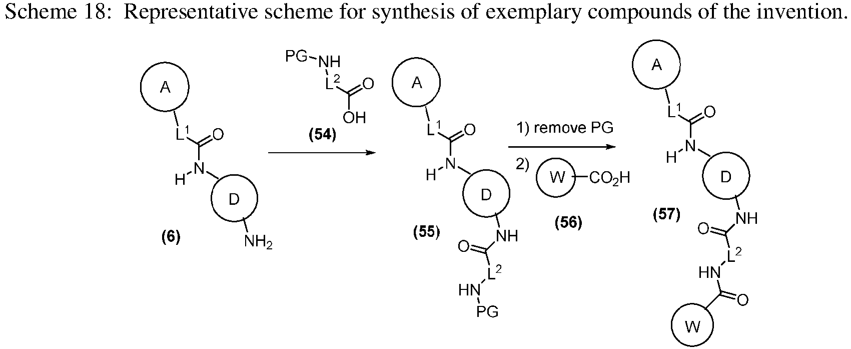

As shown in Scheme 18, compounds of formula (6) can be converted to compounds of formula (57) which are representative of compounds of formula (I). Accordingly, compounds of formula (6) can be coupled with protected amino acids of formula (54), wherein PG is suitable amine protecting group, using the amide bond coupling conditions described in Scheme 1 to give compounds of formula (55). The protecting group, PG, in compounds of formula (55) can be removed under conditions known to one of skill in the art to expose a primary amine that can be coupled with carboxylic acids of formula (56) using the amide bond coupling conditions described in Scheme 1 to give compounds of formula (57).

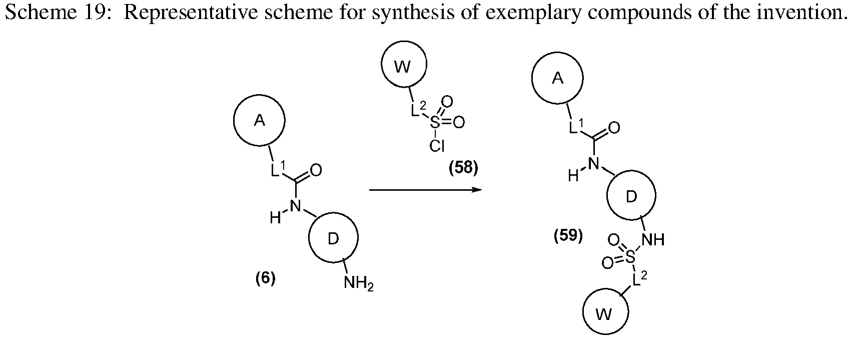

As shown in Scheme 19, compounds of formula (6) can be converted to compounds of formula (59) which are representative of compounds of formula (I). Compounds of formula (6) can be reacted with sulfonyl chlorides of formula (58) in the presence of a base, such as triethylamine, in an optionally warmed solvent, such as but not limited to *N,N-*dimethylformamide, to give compounds of formula (59).

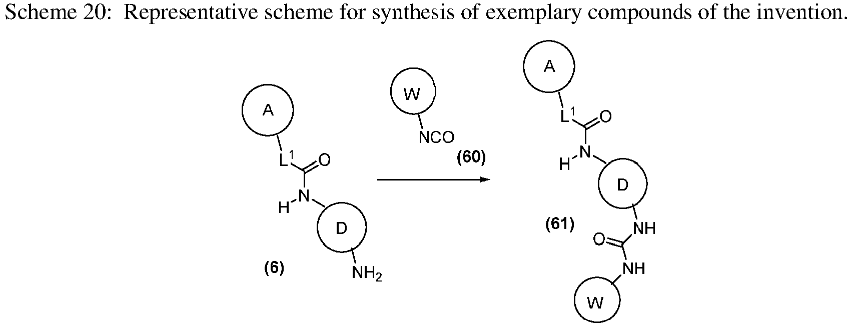

As shown in Scheme 20, compounds of formula (6) can be converted to compounds of formula (61) which are representative of compounds of formula (I). Compounds of formula (6) can be reacted with isocyanates of formula (60) in the presence of pyridine to give compounds of formula (61).

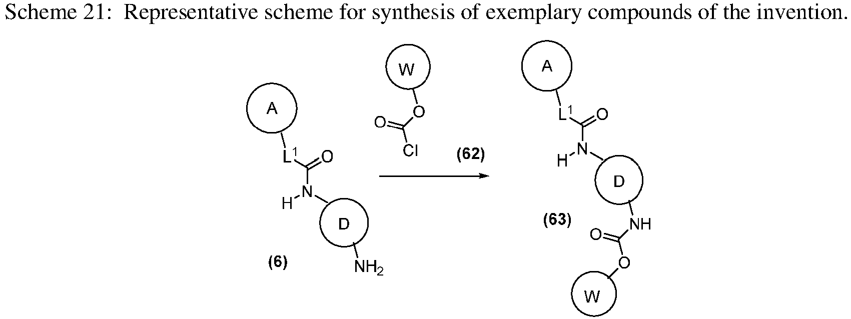

As shown in Scheme 21, compounds of formula (6) can be converted to compounds of formula (63) which are representative of compounds of formula (I). Compounds of formula (6) can be reacted with carbanochloridates of formula (62) in the presence of a base, such as *N,N-*diisopropylethylamine, in a solvent, such as tetrahydrofuran, to give compounds of formula (63).

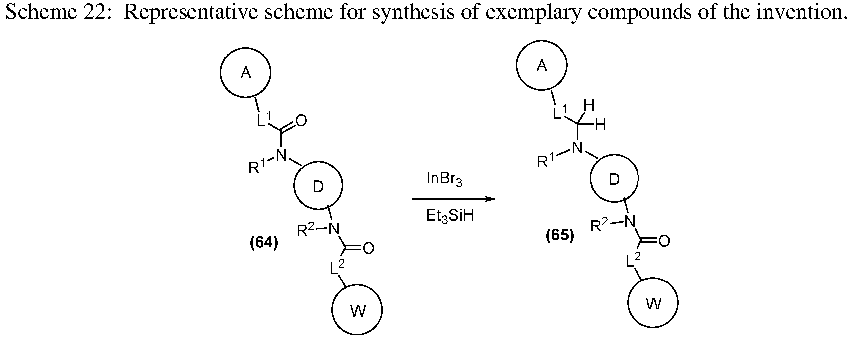

As shown in Scheme 22, compounds of formula (64) can be transformed to compounds of formula (65) which are representative of compounds of formula (I). Compounds of formula (64) can be reduced with indium(III) bromide and triethylsilane (Et₃SiH) in warmed dichloromethane to give compounds of formula (65).

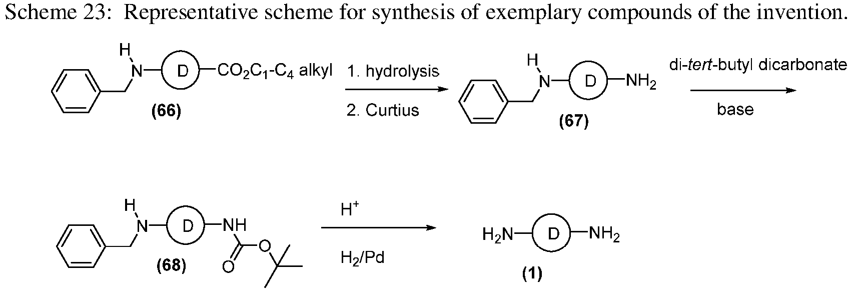

As shown in Scheme 23, compounds of formula (66) can be converted to compounds of formula (1). Accordingly, the ester moiety of compounds of formula (66) can be hydrolyzed under conditions known to one of skill in the art to give the corresponding carboxylic acids. The carboxylic acids can be treated under Curtius reaction conditions to complete the transformation to compounds of formula (67). Compounds of formula (67) can be reacted with di-tert-butyl dicarbonate in the presence of a base to give the orthogonally protected bis-amine, (68). Compounds of formula (68) can be converted to compounds of formula (1) under catalytic hydrogenation conditions in the presence of an acid, such as 4 M hydrochloric acid, in a solvent such as warmed dioxane. Compounds of formula (1) can be used as described in Scheme 1 or Scheme 2.

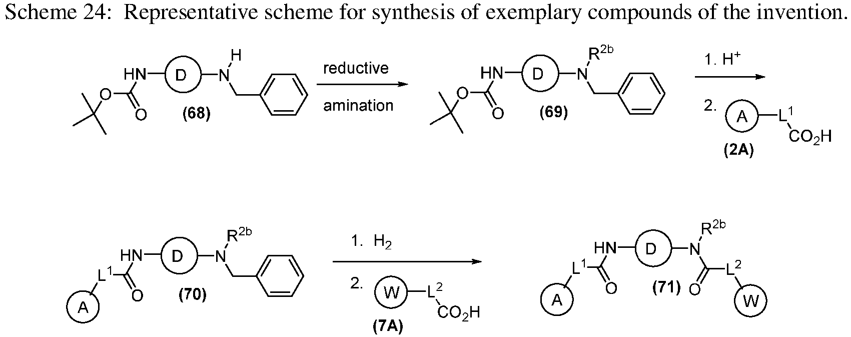

As shown in Scheme 24, compounds of formula (68) can be converted to compounds of formula (71). Compounds of formula (68) can be reductively aminated to compounds of formula (69), wherein R^{2b} is optionally substituted C₁-C₆ alkyl. Compounds of formula (69) can be treated under acidic conditions known to one of skill in the art to selectively remove the *tert-*butoxy carbonyl protecting group and then couple the exposed amine with compounds of formula (2A) using amide bond forming reaction conditions described in Scheme 1 to give compounds of formula (70). Alternatively, acid chlorides of formula (2B) can be coupled with the amines also as described in Scheme 1. The benzyl protecting group of compounds of formula (70) can be removed under catalytic hydrogenation conditions, and then the revealed amine can be coupled with carboxylic acids of formula (7A) to give compounds of formula (71). Compounds of formula (71) can also be obtained by reaction with the corresponding acid chloride with the previously mentioned revealed amine using conditions also described in Scheme 1. Compounds of formula (71) are representative of compounds of formula (I).

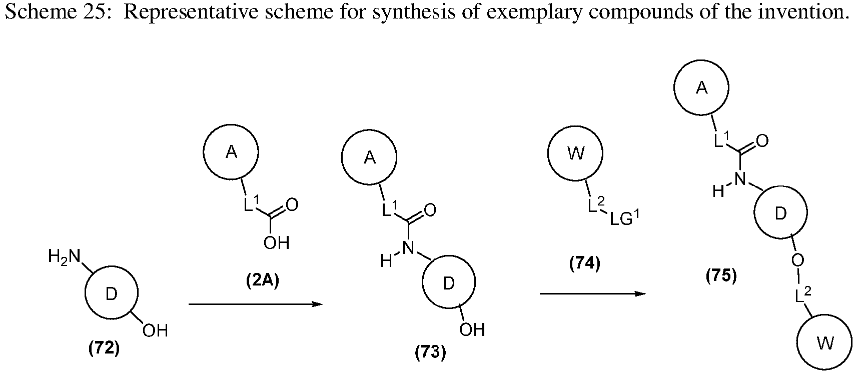

As shown in Scheme 25, compounds of formula (72) can be converted to compounds of formula (75). Carboxylic acids of formula (2A) can be coupled with amines of formula (72) under amide bond forming conditions as discussed for Scheme 1 to provide compounds of formula (73). Compounds of formula (73) can be reacted with compounds of formula (74), wherein LG¹ is a leaving group, e.g., halogen or sulfonate, under nucleophilic substitution reaction conditions to give compounds of formula (75). Compounds of formula (73) can be reacted with compounds of formula (74) in the presence of a tertiary amine base, such as 2,6-di-tert-butylpyridine, in a solvent such as dichloromethane to give compounds of formula (75). When LG¹ is a halogen, a silver reagent such as silver trifluoromethanesulfonate may be used to increase the reactivity of the halide during nucleophilic substitution compounds of formula (74) such as benzyl halides, for example. Compounds of formula (75) are representative of compounds of Formula (I).

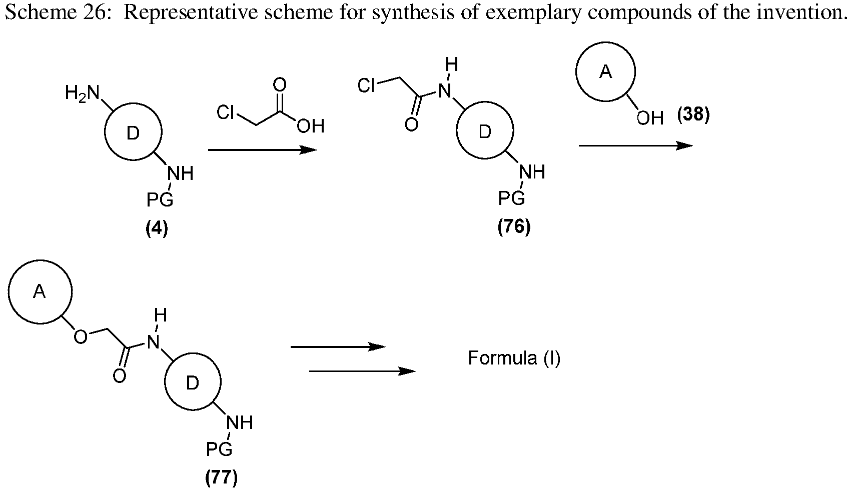

As shown in Scheme 26, compounds of formula (4) can be transformed to compounds of formula (77) which in turn through the last two steps described in Schemes 2 can be converted to compounds of formula (I). Compounds of formula (4), wherein PG is suitable amine protecting group, can be reacted with 2-chloroacetic acid under the amide bond forming conditions described in Scheme 1 to give compound of formula (76). Compounds of formula (76) can be reacted with compounds of formula (38) in the presence of potassium iodide and a base such as potassium carbonate in a heated solvent such as but not limited to acetone to give compounds of formula (77). The heating may be achieved conventionally or with microwave irradiation. After removal of the protecting group (PG) under conditions known to one of skill in the art and dependent upon the particular protecting group from compounds of formula (77), reaction of the resultant amine with carboxylic acids of formula (7A) or acid chlorides of formula (7B) under amide bond forming conditions described in Scheme 1 can provide compounds of formula (I).

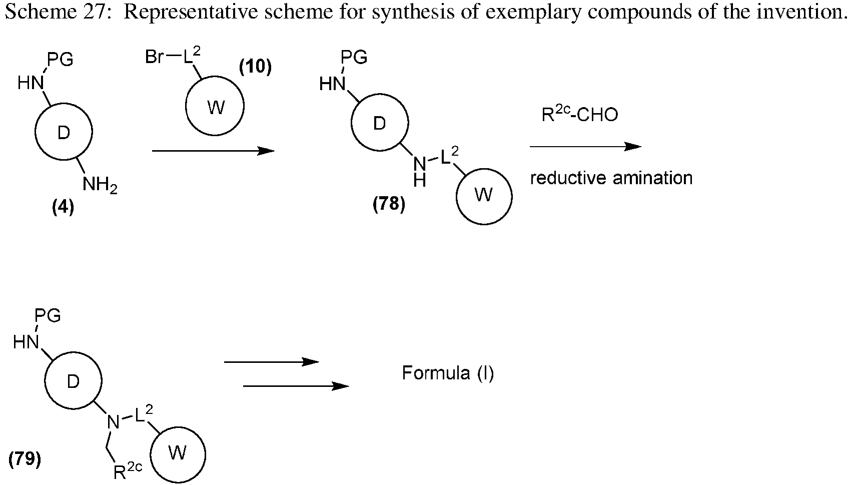

As shown in Scheme 27, compounds of formula (4) can be transformed to compounds of formula (79) which in turn through the last two steps described in Schemes 2 can be converted to compounds of formula (I). Compounds of formula (4), wherein PG is suitable amine protecting group, can be reacted with compounds of formula (10) in the presence of a base such as but not limited to cesium carbonate in an optionally warmed solvent such as but not limited to *N,N-*dimethylformamide to give compounds of formula (78). Compounds of formula (78) can be reacted with aldehydes, R^{2c}-CHO, wherein R^{2c} is hydrogen, C₁-C₅ alkyl, C₁-C₆ alkoxy-C₂-C₅ alkyl, hydroxy-C₂-C₅ alkyl, silyloxy-C₂-C₅ alkyl, or C₁-C₅ alkyl-C(O)₂-C₁-C₆ alkyl; under reductive amination conditions such as in the presence of an acid such as trifluoroacetic acid in the presence of sodium borohydride in a solvent such as methanol to give compounds of formula (79). After removal of the protecting group (PG) under conditions known to one of skill in the art and dependent upon the particular protecting group from compounds of formula (79), reaction of the resultant amine with carboxylic acids of formula (2A) or acid chlorides of formula (2B) under amide bond forming conditions described in Scheme 1 can provide compounds of formula (I).

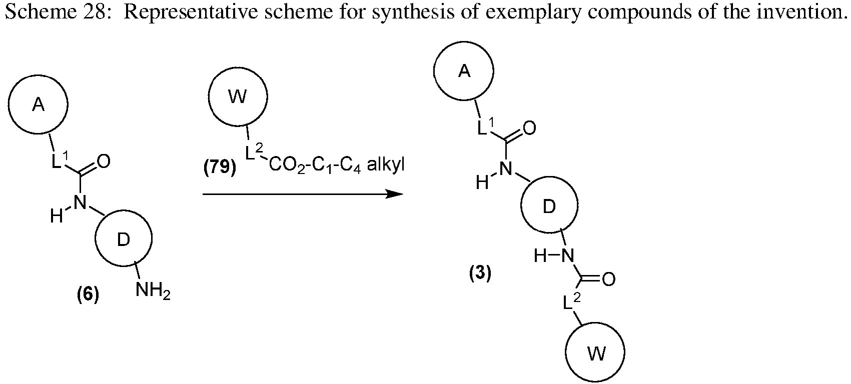

As shown in Scheme 28, compounds of formula (6) can be converted to compounds of formula (3) in one step. Accordingly, amines of formula (6) can be reacted with esters of formula (79) in a heated solvent such as but not limited to toluene to give compounds of formula (3). Compounds of formula (3) are representative of compounds of Formula (I).

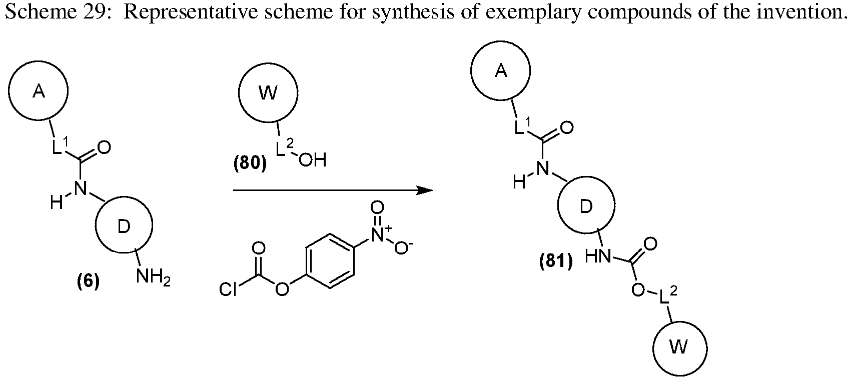

As shown in Scheme 29, compounds of formula (6) can be converted to compounds of formula (81). Accordingly, amines of formula (6) can be reacted with alcohols of formula (80) and 4-nitrophenyl carbonochloridate in a heated solvent such as but not limited to acetonitrile in the presence of a tertiary amine base such as Hunig's base and an aromatic amine base such as pyridine to give compounds of formula (81). The heating may be accomplished conventionally or through microwave irradiation. Compounds of formula (81) are representative of compounds of Formula (I).

### Pharmaceutical Compositions

The present description features pharmaceutical compositions comprising a compound of Formula (I) or Formula (II), or a pharmaceutically acceptable salt, solvate, hydrate, tautomer, ester, N-oxide or stereoisomer thereof. In some embodiments, the pharmaceutical composition further comprises a pharmaceutically acceptable excipient. In some embodiments, the compound of Formula (I) or Formula (II), or a pharmaceutically acceptable salt, solvate, hydrate, tautomer, stereoisomer thereof is provided in an effective amount in the pharmaceutical composition. In some embodiments, the effective amount is a therapeutically effective amount. In certain embodiments, the effective amount is a prophylactically effective amount.

Pharmaceutical compositions described herein can be prepared by any method known in the art of pharmacology. In general, such preparatory methods include the steps of bringing the compound of Formula (I) or Formula (II), (the "active ingredient") into association with a carrier and/or one or more other accessory ingredients, and then, if necessary and/or desirable, shaping and/or packaging the product into a desired single- or multi-dose unit. Pharmaceutical compositions can be prepared, packaged, and/or sold in bulk, as a single unit dose, and/or as a plurality of single unit doses. As used herein, a "unit dose" is a discrete amount of the pharmaceutical composition comprising a predetermined amount of the active ingredient. The amount of the active ingredient is generally equal to the dosage of the active ingredient which would be administered to a subject and/or a convenient fraction of such a dosage such as, for example, one-half or one-third of such a dosage.

Relative amounts of a compound of Formula (I) or Formula (II), the pharmaceutically acceptable excipient, and/or any additional ingredients in a pharmaceutical composition of the description will vary, depending upon the identity, size, and/or condition of the subject treated and further depending upon the route by which the composition is to be administered. By way of example, the composition may comprise between 0.1% and 100% (w/w) of a compound of Formula (I) or Formula (II).

The term "pharmaceutically acceptable excipient" refers to a non-toxic carrier, adjuvant, diluent, or vehicle that does not destroy the pharmacological activity of the compound with which it is formulated. Pharmaceutically acceptable excipients useful in the manufacture of the pharmaceutical compositions of the description are any of those that are well known in the art of pharmaceutical formulation and include inert diluents, dispersing and/or granulating agents, surface active agents and/or emulsifiers, disintegrating agents, binding agents, preservatives, buffering agents, lubricating agents, and/or oils. Pharmaceutically acceptable excipients useful in the manufacture of the pharmaceutical compositions of the description include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

Compositions of the present description may be administered orally, parenterally (including subcutaneous, intramuscular, intravenous and intradermal), by inhalation spray, topically, rectally, nasally, buccally, vaginally or via an implanted reservoir. In some embodiments, provided compounds or compositions are administrable intravenously and/or orally.

The term "parenteral" as used herein includes subcutaneous, intravenous, intramuscular, intraocular, intravitreal, intra-articular, intra-synovial, intrasternal, intrathecal, intrahepatic, intraperitoneal intralesional and intracranial injection or infusion techniques. Preferably, the compositions are administered orally, subcutaneously, intraperitoneally or intravenously. Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

Pharmaceutically acceptable compositions of this description may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added. In some embodiments, a provided oral formulation is formulated for immediate release or sustained/delayed release. In some embodiments, the composition is suitable for buccal or sublingual administration, including tablets, lozenges and pastilles. A compound of Formula (I) or Formula (II) may also be in micro-encapsulated form.

The compositions of the present description can be delivered by transdermally, by a topical route, formulated as applicator sticks, solutions, suspensions, emulsions, gels, creams, ointments, pastes, jellies, paints, powders, and aerosols. Oral preparations include tablets, pills, powder, dragees, capsules, liquids, lozenges, cachets, gels, syrups, slurries, suspensions, etc., suitable for ingestion by the patient. Solid form preparations include powders, tablets, pills, capsules, cachets, suppositories, and dispersible granules. Liquid form preparations include solutions, suspensions, and emulsions, for example, water or water/propylene glycol solutions. The compositions of the present invention may additionally include components to provide sustained release and/or comfort. Such components include high molecular weight, anionic mucomimetic polymers, gelling polysaccharides and finely-divided drug carrier substrates. These components are discussed in greater detail in U.S. Patent Nos. 4,911,920; 5,403,841; 5,212, 162; and 4,861,760. The compositions of the present description can also be delivered as microspheres for slow release in the body. For example, microspheres can be administered via intradermal injection of drug-containing microspheres, which slowly release subcutaneously (see Rao, J. Biomater Sci. Polym. Ed. 7:623-645, 1995; as biodegradable and injectable gel formulations (see, e.g., Gao Pharm. Res.12:857-863, 1995); or, as microspheres for oral administration (see, e.g., Eyles, J. Pharm. Pharmacol. 49:669-674, 1997). In another embodiment, the formulations of the compositions of the present description can be delivered by the use of liposomes which fuse with the cellular membrane or are endocytosed, i.e., by employing receptor ligands attached to the liposome, that bind to surface membrane protein receptors of the cell resulting in endocytosis. By using liposomes, particularly where the liposome surface carries receptor ligands specific for target cells, or are otherwise preferentially directed to a specific organ, one can focus the delivery of the compositions of the present invention into the target cells in vivo. (See, e.g., Al-Muhammed, J. Microencapsul. 13:293-306, 1996; Chonn, Curr. Opin. Biotechnol. 6:698-708, 1995; Ostro, J. Hosp. Pharm. 46: 1576-1587, 1989). The compositions of the present invention can also be delivered as nanoparticles.

Alternatively, pharmaceutically acceptable compositions of this description may be administered in the form of suppositories for rectal administration. Pharmaceutically acceptable compositions of this description may also be administered topically, especially when the target of treatment includes areas or organs readily accessible by topical application, including diseases of the eye, the skin, or the lower intestinal tract. Suitable topical formulations are readily prepared for each of these areas or organs.

In some embodiments, in order to prolong the effect of a drug, it is often desirable to slow the absorption of the drug from subcutaneous or intramuscular injection. This can be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Although the descriptions of pharmaceutical compositions provided herein are principally directed to pharmaceutical compositions which are suitable for administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with ordinary experimentation.

Compounds provided herein, e.g., a compound of Formula (I) or Formula (II), or a pharmaceutically acceptable salt, solvate, hydrate, tautomer, ester, N-oxide or stereoisomer thereof are typically formulated in dosage unit form, e.g., single unit dosage form, for ease of administration and uniformity of dosage. It will be understood, however, that the total daily usage of the compositions of the present description will be decided by the attending physician within the scope of sound medical judgment. The specific therapeutically effective dose level for any particular subject or organism will depend upon a variety of factors including the disease being treated and the severity of the disorder; the activity of the specific active ingredient employed; the specific composition employed; the age, body weight, general health, sex and diet of the subject; the time of administration, route of administration, and rate of excretion of the specific active ingredient employed; the duration of the treatment; drugs used in combination or coincidental with the specific active ingredient employed; and like factors well known in the medical arts.

The exact amount of a compound required to achieve an effective amount will vary from subject to subject, depending, for example, on species, age, and general condition of a subject, severity of the side effects or disorder, identity of the particular compound(s), mode of administration, and the like. The desired dosage can be delivered three times a day, two times a day, once a day, every other day, every third day, every week, every two weeks, every three weeks, or every four weeks. In certain embodiments, the desired dosage can be delivered using multiple administrations (e.g., two, three, four, five, six, seven, eight, nine, ten, eleven, twelve, thirteen, fourteen, or more administrations).

In certain embodiments, an effective amount of a compound of Formula (I) or Formula (II), or a pharmaceutically acceptable salt, solvate, hydrate, tautomer, ester, N-oxide or stereoisomer thereof for administration one or more times a day may comprise about 0.0001 mg to about 5000 mg, e.g., from about 0.0001 mg to about 4000 mg, about 0.0001 mg to about 2000 mg, about 0.0001 mg to about 1000 mg, about 0.001 mg to about 1000 mg, about 0.01 mg to about 1000 mg, about 0.1 mg to about 1000 mg, about 1 mg to about 1000 mg, about 1 mg to about 100 mg, about 10 mg to about 1000 mg, or about 100 mg to about 1000 mg, of a compound per unit dosage form.

In certain embodiments, a compound of Formula (I) or Formula (II), or a pharmaceutically acceptable salt, solvate, hydrate, tautomer, ester, N-oxide or stereoisomer thereof may be at dosage levels sufficient to deliver from about 0.001 mg/kg to about 1000 mg/kg, e.g., about 0.001 mg/kg to about 500 mg/kg, about 0.01 mg/kg to about 250 mg/kg, about 0.1 mg/kg to about 100 mg/kg, about 0.1 mg/kg to about 50 mg/kg, about 0.1 mg/kg to about 40 mg/kg, about 0.1 mg/kg to about 25 mg/kg, about 0.01 mg/kg to about 10 mg/kg, about 0.1 mg/kg to about 10 mg/kg, or about 1 mg/kg to about 50 mg/kg, of subject body weight per day, one or more times a day, to obtain the desired therapeutic effect.

It will be appreciated that dose ranges as described herein provide guidance for the administration of provided pharmaceutical compositions to an adult. The amount to be administered to, for example, a child or an adolescent can be determined by a medical practitioner or person skilled in the art and can be lower or the same as that administered to an adult.

It will be also appreciated that a compound or composition, e.g., a compound of Formula (I) or Formula (II), or a pharmaceutically acceptable salt, solvate, hydrate, tautomer, ester, N-oxide or stereoisomer thereof as described herein, can be administered in combination with one or more additional pharmaceutical agents. The compounds or compositions can be administered in combination with additional pharmaceutical agents that improve their bioavailability, reduce and/or modify their metabolism, inhibit their excretion, and/or modify their distribution within the body. It will also be appreciated that the therapy employed may achieve a desired effect for the same disorder, and/or it may achieve different effects.

The compound or composition can be administered concurrently with, prior to, or subsequent to, one or more additional pharmaceutical agents, which may be useful as, *e.g.*, combination therapies. Pharmaceutical agents include therapeutically active agents. Pharmaceutical agents also include prophylactically active agents. Each additional pharmaceutical agent may be administered at a dose and/or on a time schedule determined for that pharmaceutical agent. The additional pharmaceutical agents may also be administered together with each other and/or with the compound or composition described herein in a single dose or administered separately in different doses. The particular combination to employ in a regimen will take into account compatibility of the inventive compound with the additional pharmaceutical agents and/or the desired therapeutic and/or prophylactic effect to be achieved. In general, it is expected that the additional pharmaceutical agents utilized in combination be utilized at levels that do not exceed the levels at which they are utilized individually. In some embodiments, the levels utilized in combination will be lower than those utilized individually.

Exemplary additional pharmaceutical agents include, but are not limited to, antiproliferative agents, anti-cancer agents, anti-diabetic agents, anti-inflammatory agents, immunosuppressant agents, and pain-relieving agents. Pharmaceutical agents include small organic molecules such as drug compounds (e.g., compounds approved by the U.S. Food and Drug Administration as provided in the Code of Federal Regulations (CFR)), peptides, proteins, carbohydrates, monosaccharides, oligosaccharides, polysaccharides, nucleoproteins, mucoproteins, lipoproteins, synthetic polypeptides or proteins, small molecules linked to proteins, glycoproteins, steroids, nucleic acids, DNAs, RNAs, nucleotides, nucleosides, oligonucleotides, antisense oligonucleotides, lipids, hormones, vitamins, and cells.

Pharmaceutical compositions provided by the present description include compositions wherein the active ingredient (e.g., compounds described herein, including embodiments or examples) is contained in a therapeutically effective amount, i.e., in an amount effective to achieve its intended purpose. The actual amount effective for a particular application will depend, inter alia, on the condition being treated. When administered in methods to treat a disease, such compositions will contain an amount of active ingredient effective to achieve the desired result, e.g., modulating the activity of a target molecule (e.g. eIF2B, eIF2 or component of eIF2α signal transduction pathway or component of phosphorylated eIF2α pathway or the ISR pathway), and/or reducing, eliminating, or slowing the progression of disease symptoms (e.g. symptoms of cancer a neurodegenerative disease, a leukodystrophy, an inflammatory disease, a musculoskeletal disease, a metabolic disease, or a disease or disorder associated with impaired function of eIF2B, eIF2α or a component of the eIF2 pathway or ISR pathway). Determination of a therapeutically effective amount of a compound of the invention is well within the capabilities of those skilled in the art, especially in light of the detailed disclosure herein.

The dosage and frequency (single or multiple doses) administered to a mammal can vary depending upon a variety of factors, for example, whether the mammal suffers from another disease, and its route of administration; size, age, sex, health, body weight, body mass index, and diet of the recipient; nature and extent of symptoms of the disease being treated (e.g. a symptom of cancer, a neurodegenerative disease, a leukodystrophy, an inflammatory disease, a musculoskeletal disease, a metabolic disease, or a disease or disorder associated with impaired function of eIF2B, eIF2 α, or a component of the eIF2 pathway or ISR pathway), kind of concurrent treatment, complications from the disease being treated or other health-related problems. Other therapeutic regimens or agents can be used in conjunction with the methods and compounds of Applicants' invention. Adjustment and manipulation of established dosages (e.g., frequency and duration) are well within the ability of those skilled in the art.

For any compound described herein, the therapeutically effective amount can be initially determined from cell culture assays. Target concentrations will be those concentrations of active compound(s) that are capable of achieving the methods described herein, as measured using the methods described herein or known in the art.

As is well known in the art, therapeutically effective amounts for use in humans can also be determined from animal models. For example, a dose for humans can be formulated to achieve a concentration that has been found to be effective in animals. The dosage in humans can be adjusted by monitoring compounds effectiveness and adjusting the dosage upwards or downwards, as described above. Adjusting the dose to achieve maximal efficacy in humans based on the methods described above and other methods is well within the capabilities of the ordinarily skilled artisan.

Dosages may be varied depending upon the requirements of the patient and the compound being employed. The dose administered to a patient, in the context of the present description should be sufficient to affect a beneficial therapeutic response in the patient over time. The size of the dose also will be determined by the existence, nature, and extent of any adverse side-effects. Determination of the proper dosage for a particular situation is within the skill of the practitioner. Generally, treatment is initiated with smaller dosages which are less than the optimum dose of the compound. Thereafter, the dosage is increased by small increments until the optimum effect under circumstances is reached. Dosage amounts and intervals can be adjusted individually to provide levels of the administered compound effective for the particular clinical indication being treated. This will provide a therapeutic regimen that is commensurate with the severity of the individual's disease state.

Utilizing the teachings provided herein, an effective prophylactic or therapeutic treatment regimen can be planned that does not cause substantial toxicity and yet is effective to treat the clinical symptoms demonstrated by the particular patient. This planning should involve the careful choice of active compound by considering factors such as compound potency, relative bioavailability, patient body weight, presence and severity of adverse side effects, preferred mode of administration and the toxicity profile of the selected agent.

Also encompassed by the description are kits (e.g., pharmaceutical packs). The inventive kits may be useful for preventing and/or treating a disease (e.g., cancer, a neurodegenerative disease, a leukodystrophy, an inflammatory disease, a musculoskeletal disease, a metabolic disease, or other disease or condition described herein).

The kits provided may comprise an inventive pharmaceutical composition or compound and a container *(e.g.,* a vial, ampule, bottle, syringe, and/or dispenser package, or other suitable container). In some embodiments, provided kits may optionally further include a second container comprising a pharmaceutical excipient for dilution or suspension of an inventive pharmaceutical composition or compound. In some embodiments, the inventive pharmaceutical composition or compound provided in the container and the second container are combined to form one unit dosage form.

Thus, in one aspect, provided are kits including a first container comprising a compound of Formula (I) or Formula (II), or a pharmaceutically acceptable salt, solvate, hydrate, tautomer, ester, N-oxide or stereoisomer thereof, or a pharmaceutical composition thereof. In certain embodiments, the kits are useful in preventing and/or treating a proliferative disease in a subject. In certain embodiments, the kits further include instructions for administering a compound of Formula (I) or Formula (II), or a pharmaceutically acceptable salt, solvate, hydrate, tautomer, ester, N-oxide or stereoisomer thereof, or a pharmaceutical composition thereof, to a subject to prevent and/or treat a disease described herein.

### Methods of Treatment

The present description features compounds, compositions, and methods comprising a compound of Formula (I) or Formula (II), or a pharmaceutically acceptable salt, solvate, hydrate, tautomer, ester, *N*-oxide or stereoisomer thereof. In some embodiments, the compounds, compositions, and methods are used in the prevention or treatment of a disease, disorder, or condition. Exemplary diseases, disorders, or conditions include, but are not limited to a neurodegenerative disease, a leukodystrophy, a cancer, an inflammatory disease, an autoimmune disease, a viral infection, a skin disease, a fibrotic disease, a hemoglobin disease, a kidney disease, a hearing loss condition, an ocular disease, a disease with mutations that leads to UPR induction, a malaria infection, a musculoskeletal disease, a metabolic disease, or a mitochondrial disease.

In some embodiments, the disease, disorder, or condition is related to (e.g., caused by) modulation of (e.g., a decrease in) eIF2B activity or level, eIF2α activity or level, or a component of the eIF2 pathway or ISR pathway. In some embodiments, the disease, disorder, or condition is related to modulation of a signaling pathway related to a component of the eIF2 pathway or ISR pathway (e.g., phosphorylation of a component of the eIF2 pathway or ISR pathway). In some embodiments, the disease, disorder, or condition is related to (e.g., caused by) neurodegeneration. In some embodiments, the disease, disorder, or condition is related to (e.g., caused by) neural cell death or dysfunction. In some embodiments, the disease, disorder, or condition is related to (e.g., caused by) glial cell death or dysfunction. In some embodiments, the disease, disorder, or condition is related to (e.g., caused by) an increase in the level or activity of eIF2B, eIF2α, or a component of the eIF2 pathway or ISR pathway. In some embodiments, the disease, disorder, or condition is related to (e.g., caused by) a decrease in the level or activity of eIF2B, eIF2α, or a component of the eIF2 pathway or ISR pathway.

In some embodiments, the disease may be caused by a mutation to a gene or protein sequence related to a member of the eIF2 pathway (e.g., eIF2B, eIF2α, or other component). Exemplary mutations include an amino acid mutation in the eIF2B1, eIF2B2, eIF2B3, eIF2B4, eIF2B5 subunits. In some embodiments, an amino acid mutation (e.g., an amino acid substitution, addition, or deletion) in a particular protein that may result in a structural change, e.g., a conformational or steric change, that affects the function of the protein. For example, in some embodiments, amino acids in and around the active site or close to a binding site (e.g., a phosphorylation site, small molecule binding site, or protein-binding site) may be mutated such that the activity of the protein is impacted. In some instances, the amino acid mutation (e.g., an amino acid substitution, addition, or deletion) may be conservative and may not substantially impact the structure or function of a protein. For example, in certain cases, the substitution of a serine residue with a threonine residue may not significantly impact the function of a protein. In other cases, the amino acid mutation may be more dramatic, such as the substitution of a charged amino acid (e.g., aspartic acid or lysine) with a large, nonpolar amino acid (e.g., phenylalanine or tryptophan) and therefore may have a substantial impact on protein function. The nature of the mutations that affect the structure of function of a gene or protein may be readily identified using standard sequencing techniques, e.g., deep sequencing techniques that are well known in the art. In some embodiments, a mutation in a member of the eIF2 pathway may affect binding or activity of a compound of Formula (I) or Formula (II), or a pharmaceutically acceptable salt, solvate, hydrate, tautomer, ester, N-oxide or stereoisomer thereof and thereby modulate treatment of a particular disease, disorder, or condition, or a symptom thereof.

In some embodiments, an eIF2 protein may comprise an amino acid mutation (e.g., an amino acid substitution, addition, or deletion) at an alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, or valine residue. In some embodiments, an eIF2 protein may comprise an amino acid substitution at an alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, or valine residue. In some embodiments, an eIF2 protein may comprise an amino acid addition at an alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, or valine residue. In some embodiments, an eIF2 protein may comprise an amino acid deletion at an alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, or valine residue.

In some embodiments, the eIF2 protein may comprise an amino acid mutation (e.g., an amino acid substitution, addition, or deletion) at an alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, or valine residue in the eIF2B 1, eIF2B2, eIF2B3, eIF2B4, eIF2B5 subunits. In some embodiments, the eIF2 protein may comprise an amino acid substitution at an alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, or valine residue in the eIF2B 1, eIF2B2, eIF2B3, eIF2B4, eIF2B5 subunits. In some embodiments, the eIF2 protein may comprise an amino acid addition at an alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, or valine residue in the eIF2B 1, eIF2B2, eIF2B3, eIF2B4, eIF2B5 subunits. In some embodiments, the eIF2 protein may comprise an amino acid deletion at an alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, or valine residue in the eIF2B 1, eIF2B2, eIF2B3, eIF2B4, eIF2B5 subunits. Exemplary mutations include V183F (eIF2B1 subunit), H341Q (eIF2B3), I346T (eIF2B3), R483W (eIF2B4), R113H (eIF2B5), and R195H (eIF2B5).

In some embodiments, an amino acid mutation (e.g., an amino acid substitution, addition, or deletion) in a member of the eIF2 pathway (e.g., an eIF2B protein subunit) may affect binding or activity of a compound of Formula (I) or Formula (II), or a pharmaceutically acceptable salt, solvate, hydrate, tautomer, ester, N-oxide or stereoisomer thereof and thereby modulate treatment of a particular disease, disorder, or condition, or a symptom thereof.

### Neurodegenerative Disease

In some embodiments, the compound of Formula (I) or Formula (II), or a pharmaceutically acceptable salt, solvate, hydrate, tautomer, ester, N-oxide or stereoisomer thereof is used to treat a neurodegenerative disease. As used herein, the term "neurodegenerative disease" refers to a disease or condition in which the function of a subject's nervous system becomes impaired. Examples of a neurodegenerative disease that may be treated with a compound, pharmaceutical composition, or method described herein include Alexander's disease, Alper's disease, Alzheimer's disease, Amyotrophic lateral sclerosis (ALS), Ataxia telangiectasia, Batten disease (also known as Spielmeyer-Vogt-Sjogren-Batten disease), Bovine spongiform encephalopathy (BSE), Canavan disease, Cockayne syndrome, Corticobasal degeneration, Creutzfeldt-Jakob disease, Dystonia, frontotemporal dementia (FTD), Gerstmann-Straussler-Scheinker syndrome, Huntington's disease, HIV-associated dementia, Kennedy's disease, Krabbe disease, kuru, Lewy body dementia, Machado-Joseph disease (Spinocerebellar ataxia type 3), Multiple system atrophy, Multisystem proteinopathy, Narcolepsy, Neuroborreliosis, Parkinson's disease, Pelizaeus-Merzbacher Disease, Pick's disease, Primary lateral sclerosis, Prion diseases, Refsum's disease, Sandhoff disease, Schilder's disease, Subacute combined degeneration of spinal cord secondary to Pernicious Anaemia, Schizophrenia, Spinocerebellar ataxia (multiple types with varying characteristics, e.g., Spinocerebellar ataxia type 2 or Spinocerebellar ataxia type 8), Spinal muscular atrophy, Steele-Richardson-Olszewski disease, progressive supranuclear palsy, corticobasal degeneration, adrenoleukodystrophy, X-linked adrenoleukodystrophy, cerebral adrenoleukodystrophy, Pelizaeus-Merzbacher Disease, Krabbe disease, leukodystrophy due to mutation in DARS2 gene (sometimes known as lukoencephalopathy with brainstem and spinal cord involvement and lactate elevation (LBSL), DARS2-related spectrum disorders, or Tabes dorsalis.

In some embodiments, the neurodegenerative disease comprises vanishing white matter disease, childhood ataxia with CNS hypo-myelination, a leukodystrophy, a leukoencephalopathy, a hypomyelinating or demyelinating disease, an intellectual disability syndrome (e.g., Fragile X syndrome), Alzheimer's disease, amyotrophic lateral sclerosis (ALS), Creutzfeldt-Jakob disease, frontotemporal dementia (FTD), Gerstmann-Straussler-Scheinker disease, Huntington's disease, dementia (e.g., HIV-associated dementia or Lewy body dementia), kuru, multiple sclerosis, Parkinson's disease, or a prion disease.

In some embodiments, the neurodegenerative disease comprises vanishing white matter disease, childhood ataxia with CNS hypo-myelination, a leukodystrophy, a leukoencephalopathy, a hypomyelinating or demyelinating disease, or an intellectual disability syndrome (e.g., Fragile X syndrome).

In some embodiments, the neurodegenerative disease comprises a psychiatric disease such as agoraphobia, Alzheimer's disease, anorexia nervosa, amnesia, anxiety disorder, attention deficit disorder, bipolar disorder, body dysmorphic disorder, bulimia nervosa, claustrophobia, depression, delusions, Diogenes syndrome, dyspraxia, insomnia, Munchausen's syndrome, narcolepsy, narcissistic personality disorder, obsessive-compulsive disorder, psychosis, phobic disorder, schizophrenia, seasonal affective disorder, schizoid personality disorder, sleepwalking, social phobia, substance abuse, tardive dyskinesia, Tourette syndrome, or trichotillomania.

In some embodiments, the compound of Formula (I) or Formula (II), or a pharmaceutically acceptable salt, solvate, hydrate, tautomer, ester, N-oxide or stereoisomer thereof is used to treat vanishing white matter disease. Exemplary methods of treating vanishing white matter disease include, but are not limited to, reducing or eliminating a symptom of vanishing white matter disease, reducing the loss of white matter, reducing the loss of myelin, increasing the amount of myelin, or increasing the amount of white matter in a subject.

In some embodiments, the compound of Formula (I) or Formula (II), or a pharmaceutically acceptable salt, solvate, hydrate, tautomer, ester, N-oxide or stereoisomer thereof is used to treat childhood ataxia with CNS hypo-myelination. Exemplary methods of treating childhood ataxia with CNS hypo-myelination include, but are not limited to, reducing or eliminating a symptom of childhood ataxia with CNS hypo-myelination, increasing the level of myelin, or decreasing the loss of myelin in a subject.

In some embodiments, the compound of Formula (I) or Formula (II), or a pharmaceutically acceptable salt, solvate, hydrate, tautomer, ester, N-oxide or stereoisomer thereof is used to treat an intellectual disability syndrome (e.g., Fragile X syndrome). Exemplary methods of treating an intellectual disability syndrome include, but are not limited to, reducing or eliminating a symptom of an intellectual disability syndrome.

In some embodiments, the compound of Formula (I) or Formula (II), or a pharmaceutically acceptable salt, solvate, hydrate, tautomer, ester, N-oxide or stereoisomer thereof is used to treat neurodegeneration. Exemplary methods of treating neurodegeneration include, but are not limited to, improvement of mental wellbeing, increasing mental function, slowing the decrease of mental function, decreasing dementia, delaying the onset of dementia, improving cognitive skills, decreasing the loss of cognitive skills, improving memory, decreasing the degradation of memory, or extending survival.

In some embodiments, the compound of Formula (I) or Formula (II), or a pharmaceutically acceptable salt, solvate, hydrate, tautomer, ester, N-oxide or stereoisomer thereof is used to treat a leukoencephalopathy or demyelinating disease. Exemplary leukoencephalopathies include, but are not limited to, progressive multifocal leukoencephalopathy, toxic leukoencephalopathy, leukoencephalopathy with vanishing white matter, leukoencephalopathy with neuroaxonal spheroids, reversible posterior leukoencephalopathy syndrome, hypertensive leukoencephalopathy, megalencephalic leukoencephalopathy with subcortical cysts, Charcot-Marie-Tooth disorder, and Devic's disease. A leukoencephalopathy may comprise a demyelinating disease, which may be inherited or acquired. In some embodiments, an acquired demyelinating disease may be an inflammatory demyelinating disease (e.g., an infectious inflammatory demyelinating disease or a non-infectious inflammatory demyelinating disease), a toxic demyelinating disease, a metabolic demyelinating disease, a hypoxic demyelinating disease, a traumatic demyelinating disease, or an ischemic demyelinating disease (e.g., Binswanger's disease). Exemplary methods of treating a leukoencephalopathy or demyelinating disease include, but are not limited to, reducing or eliminating a symptom of a leukoencephalopathy or demyelinating disease, reducing the loss of myelin, increasing the amount of myelin, reducing the loss of white matter in a subject, or increasing the amount of white matter in a subject.

In some embodiments, the compound of Formula (I) or Formula (II), or a pharmaceutically acceptable salt, solvate, hydrate, tautomer, ester, *N*-oxide or stereoisomer thereof is used to treat a traumatic injury or a toxin-induced injury to the nervous system (e.g., the brain). Exemplary traumatic brain injuries include, but are not limited to, a brain abscess, concussion, ischemia, brain bleeding, cranial fracture, diffuse axonal injury, locked-in syndrome, or injury relating to a traumatic force or blow to the nervous system or brain that causes damage to an organ or tissue. Exemplary toxin-induced brain injuries include, but are not limited to, toxic encephalopathy, meningitis (e.g. bacterial meningitis or viral meningitis), meningoencephalitis, encephalitis (e.g., Japanese encephalitis, eastern equine encephalitis, West Nile encephalitis), Guillan-Barre syndrome, Sydenham's chorea, rabies, leprosy, neurosyphilis, a prion disease, or exposure to a chemical (e.g., arsenic, lead, toluene, ethanol, manganese, fluoride, dichlorodiphenyltrichloroethane (DDT), dichlorodiphenyldichloroethylene (DDE), tetrachloroethylene, a polybrominated diphenyl ether, a pesticide, a sodium channel inhibitor, a potassium channel inhibitor, a chloride channel inhibitor, a calcium channel inhibitor, or a blood brain barrier inhibitor).

In other embodiments, the compound of Formula (I) or Formula (II), or a pharmaceutically acceptable salt, solvate, hydrate, tautomer, ester, N-oxide or stereoisomer thereof is used to improve memory in a subject. Induction of memory has been shown to be facilitated by decreased and impaired by increased eIF2α phosphorylation. Regulators of translation, such as compounds disclosed herein (e.g. a compound of Formula (I) or Formula (II)), could serve as therapeutic agents that improve memory in human disorders associated with memory loss such as Alzheimer's disease and in other neurological disorders that activate the UPR or ISR in neurons and thus could have negative effects on memory consolidation such as Parkinson's disease, schizophrenia, amyotrophic lateral sclerosis (ALS) and prion diseases. In addition, a mutation in eIF2γ that disrupts complex integrity linked intellectual disability (intellectual disability syndrome or ID) to impaired translation initiation in humans. Hence, two diseases with impaired eIF2 function, ID and VWM, display distinct phenotypes but both affect mainly the brain and impair learning. In some embodiments, the disease or condition is unsatisfactory memory (e.g., working memory, long term memory, short term memory, or memory consolidation).

In still other embodiments, the compound of Formula (I) or Formula (II), or a pharmaceutically acceptable salt, solvate, hydrate, tautomer, ester, N-oxide or stereoisomer thereof is used in a method to improve memory in a subject (e.g., working memory, long term memory, short term memory, or memory consolidation). In some embodiments, the subject is human. In some embodiments, the subject is a non-human mammal. In some embodiments, the subject is a domesticated animal. In some embodiments, the subject is a dog. In some embodiments, the subject is a bird. In some embodiments, the subject is a horse. In embodiments, the patient is a bovine. In some embodiments, the subject is a primate.

### Cancer

In some embodiments, the compound of Formula (I) or Formula (II), or a pharmaceutically acceptable salt, solvate, hydrate, tautomer, or stereoisomer thereof is used to treat cancer. As used herein, "cancer" refers to human cancers and carcinomas, sarcomas, adenocarcinomas, lymphomas, leukemias, melanomas, etc., including solid and lymphoid cancers, kidney, breast, lung, bladder, colon, ovarian, prostate, pancreas, stomach, brain, head and neck, skin, uterine, testicular, glioma, esophagus, liver cancer, including hepatocarcinoma, lymphoma, including B-acute lymphoblastic lymphoma, non-Hodgkin's lymphomas (e.g., Burkitt's, Small Cell, and Large Cell lymphomas), Hodgkin's lymphoma, leukemia (including AML, ALL, and CML), and/or multiple myeloma. In some further instances, "cancer" refers to lung cancer, breast cancer, ovarian cancer, leukemia, lymphoma, melanoma, pancreatic cancer, sarcoma, bladder cancer, bone cancer, brain cancer, cervical cancer, colon cancer, esophageal cancer, gastric cancer, liver cancer, head and neck cancer, kidney cancer, myeloma, thyroid cancer, prostate cancer, metastatic cancer, or carcinoma.

As used herein, the term "cancer" refers to all types of cancer, neoplasm or malignant tumors found in mammals, including leukemia, lymphoma, carcinomas and sarcomas. Exemplary cancers that may be treated with a compound, pharmaceutical composition, or method provided herein include lymphoma, sarcoma, bladder cancer, bone cancer, brain tumor, cervical cancer, colon cancer, esophageal cancer, gastric cancer, head and neck cancer, kidney cancer, myeloma, thyroid cancer, leukemia, prostate cancer, breast cancer (e.g., ER positive, ER negative, chemotherapy resistant, herceptin resistant, HER2 positive, doxorubicin resistant, tamoxifen resistant, ductal carcinoma, lobular carcinoma, primary, metastatic), ovarian cancer, pancreatic cancer, liver cancer (e.g., hepatocellular carcinoma), lung cancer (e.g., non-small cell lung carcinoma, squamous cell lung carcinoma, adenocarcinoma, large cell lung carcinoma, small cell lung carcinoma, carcinoid, sarcoma), glioblastoma multiforme, glioma, or melanoma. Additional examples include, cancer of the thyroid, endocrine system, brain, breast, cervix, colon, head & neck, liver, kidney, lung, non-small cell lung, melanoma, mesothelioma, ovary, sarcoma, stomach, uterus or Medulloblastoma (e.g., WNT-dependent pediatric medulloblastoma), Hodgkin's Disease, Non-Hodgkin's Lymphoma, multiple myeloma, neuroblastoma, glioma, glioblastoma multiforme, ovarian cancer, rhabdomyosarcoma, primary thrombocytosis, primary macroglobulinemia, primary brain tumors, cancer, malignant pancreatic insulanoma, malignant carcinoid, urinary bladder cancer, premalignant skin lesions, testicular cancer, lymphomas, thyroid cancer, neuroblastoma, esophageal cancer, genitourinary tract cancer, malignant hypercalcemia, endometrial cancer, adrenal cortical cancer, neoplasms of the endocrine or exocrine pancreas, medullary thyroid cancer, medullary thyroid carcinoma, melanoma, colorectal cancer, papillary thyroid cancer, hepatocellular carcinoma, Paget' s Disease of the Nipple, Phyllodes Tumors, Lobular Carcinoma, Ductal Carcinoma, cancer of the pancreatic stellate cells, cancer of the hepatic stellate cells, or prostate cancer.

The term "leukemia" refers broadly to progressive, malignant diseases of the blood-forming organs and is generally characterized by a distorted proliferation and development of leukocytes and their precursors in the blood and bone marrow. Leukemia is generally clinically classified on the basis of (1) the duration and character of the disease-acute or chronic; (2) the type of cell involved; myeloid (myelogenous), lymphoid (lymphogenous), or monocytic; and (3) the increase or non-increase in the number abnormal cells in the blood-leukemic or aleukemic (subleukemic). Exemplary leukemias that may be treated with a compound, pharmaceutical composition, or method provided herein include, for example, acute nonlymphocytic leukemia, chronic lymphocytic leukemia, acute granulocytic leukemia, chronic granulocytic leukemia, acute promyelocytic leukemia, adult T-cell leukemia, aleukemic leukemia, a leukocythemic leukemia, basophylic leukemia, blast cell leukemia, bovine leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocyte leukemia, micromyeloblastic leukemia, monocytic leukemia, myeloblasts leukemia, myelocytic leukemia, myeloid granulocytic leukemia, myelomonocytic leukemia, Naegeli leukemia, plasma cell leukemia, multiple myeloma, plasmacytic leukemia, promyelocytic leukemia, Rieder cell leukemia, Schilling's leukemia, stem cell leukemia, subleukemic leukemia, or undifferentiated cell leukemia.

The term "sarcoma" generally refers to a tumor which is made up of a substance like the embryonic connective tissue and is generally composed of closely packed cells embedded in a fibrillar or homogeneous substance. Sarcomas that may be treated with a compound, pharmaceutical composition, or method provided herein include a chondrosarcoma, fibrosarcoma, lymphosarcoma, melanosarcoma, myxosarcoma, osteosarcoma, Abemethy's sarcoma, adipose sarcoma, liposarcoma, alveolar soft part sarcoma, ameloblastic sarcoma, botryoid sarcoma, chloroma sarcoma, chorio carcinoma, embryonal sarcoma, Wilms' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented hemorrhagic sarcoma, immunoblastic sarcoma of B cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, synovial sarcoma, or telangiectaltic sarcoma.

The term "melanoma" is taken to mean a tumor arising from the melanocytic system of the skin and other organs. Melanomas that may be treated with a compound, pharmaceutical composition, or method provided herein include, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma, subungal melanoma, or superficial spreading melanoma.

The term "carcinoma" refers to a malignant new growth made up of epithelial cells tending to infiltrate the surrounding tissues and give rise to metastases. Exemplary carcinomas that may be treated with a compound, pharmaceutical composition, or method provided herein include, for example, medullary thyroid carcinoma, familial medullary thyroid carcinoma, acinar carcinoma, acinous carcinoma, adenocystic carcinoma, adenoid cystic carcinoma, carcinoma adenomatosum, carcinoma of adrenal cortex, alveolar carcinoma, alveolar cell carcinoma, basal cell carcinoma, basaloid carcinoma, basosquamous cell carcinoma, bronchioalveolar carcinoma, bronchiolar carcinoma, bronchogenic carcinoma, cerebriform carcinoma, cholangiocellular carcinoma, chorionic carcinoma, colloid carcinoma, comedo carcinoma, corpus carcinoma, cribriform carcinoma, carcinoma en cuirasse, carcinoma cutaneum, cylindrical carcinoma, cylindrical cell carcinoma, duct carcinoma, ductal carcinoma, carcinoma durum, embryonal carcinoma, encephaloid carcinoma, epidermoid carcinoma, carcinoma epitheliale adenoides, exophytic carcinoma, carcinoma ex ulcere, carcinoma fibrosum, gelatiniforni carcinoma, gelatinous carcinoma, giant cell carcinoma, carcinoma gigantocellulare, glandular carcinoma, granulosa cell carcinoma, hair-matrix carcinoma, hematoid carcinoma, hepatocellular carcinoma, Hurthle cell carcinoma, hyaline carcinoma, hypernephroid carcinoma, infantile embryonal carcinoma, carcinoma in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lobular carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidermoid carcinoma, carcinoma mucosum, mucous carcinoma, carcinoma myxomatodes, nasopharyngeal carcinoma, oat cell carcinoma, carcinoma ossificans, osteoid carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signet-ring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticum, carcinoma telangiectodes, transitional cell carcinoma, carcinoma tuberosum, tubular carcinoma, tuberous carcinoma, verrucous carcinoma, or carcinoma villosum.

In some embodiments, the compound of Formula (I) or Formula (II) or a pharmaceutically acceptable salt, solvate, hydrate, tautomer, ester, N-oxide or stereoisomer thereof is used to treat pancreatic cancer, breast cancer, multiple myeloma, cancers of secretory cells. For example certain methods herein treat cancer by decreasing or reducing or preventing the occurrence, growth, metastasis, or progression of cancer. In some embodiments, the methods described herein may be used to treat cancer by decreasing or eliminating a symptom of cancer. In some embodiments, the compound of Formula (I) or Formula (II) or a pharmaceutically acceptable salt, solvate, hydrate, tautomer, ester, N-oxide or stereoisomer thereof may be used as a single agent in a composition or in combination with another agent in a composition to treat a cancer described herein (e.g., pancreatic cancer, breast cancer, multiple myeloma, cancers of secretory cells).

In some embodiments, the compounds (compounds described herein, *e.g.,* a compound of Formula (I) or Formula (II)) and compositions (e.g., compositions comprising a compound described herein, *e.g.,* a compound of Formula (I) or Formula (II))) are used with a cancer immunotherapy (*e.g.*, a checkpoint blocking antibody) to treat a subject (*e.g.*, a human subject), *e.g.*, suffering from a disease or disorder described herein (*e.g.*, abnormal cell growth, *e.g.*, cancer (*e.g.*, a cancer described herein)). The methods described herein comprise administering a compound described herein, e.g., a compound of Formula (I) or Formula (II) and an immunotherapy to a subject having abnormal cell growth such as cancer. Exemplary immunotherapies include, but are not limited to the following.

In some embodiments, the immunotherapeutic agent is a compound (*e.g.*, a ligand, an antibody) that inhibits the immune checkpoint blockade pathway. In some embodiments, the immunotherapeutic agent is a compound that inhibits the indoleamine 2,3-dioxygenase (IDO) pathway. In some embodiments, the immunotherapeutic agent is a compound that agonizes the STING pathway. Cancer immunotherapy refers to the use of the immune system to treat cancer. Three groups of immunotherapy used to treat cancer include cell-based, antibody-based, and cytokine therapies. All groups exploit cancer cells' display of subtly different structures (*e.g*., molecular structure; antigens, proteins, molecules, carbohydrates) on their surface that can be detected by the immune system. Cancer immunotherapy (i.e., anti-tumor immunotherapy or anti-tumor immunotherapeutics) includes but is not limited to, immune checkpoint antibodies (e.g., PD-1 antibodies, PD-L1 antibodies, PD-L2 antibodies, CTLA-4 antibodies, TIM3 antibodies, LAG3 antibodies, TIGIT antibodies); and cancer vaccines (i.e., anti-tumor vaccines or vaccines based on neoantigens such as a peptide or RNA vaccine).

Cell-based therapies (*e.g*., cancer vaccines), usually involve the removal of immune cells from a subject suffering from cancer, either from the blood or from a tumor. Immune cells specific for the tumor will be activated, grown, and returned to a subject suffering from cancer where the immune cells provide an immune response against the cancer. Cell types that can be used in this way are *e.g.*, natural killer cells, lymphokine-activated killer cells, cytotoxic T-cells, dendritic cells, CAR-T therapies (i.e., chimeric antigen receptor T-cells which are T-cells engineered to target specific antigens), TIL therapy (i.e., administration of tumor-infiltrating lymphocytes), TCR gene therapy, protein vaccines, and nucleic acid vaccines. An exemplary cell-based therapy is Provenge. In some embodiments, the cell-based therapy is a CAR-T therapy.

Interleukin-2 and interferon-alpha are examples of cytokines, proteins that regulate and coordinate the behavior of the immune system.

### Cancer Vaccines with Neoantigens

Neoantigens are antigens encoded by tumor-specific mutated genes. Technological innovations have made it possible to dissect the immune response to patient-specific neoantigens that arise as a consequence of tumor-specific mutations, and emerging data suggest that recognition of such neoantigens is a major factor in the activity of clinical immunotherapies. These observations indicate that neoantigen load may form a biomarker in cancer immunotherapy. Many novel therapeutic approaches are being developed that selectively enhance T cell reactivity against this class of antigens. One approach to target neoantigens is ***via*** cancer vaccine. These vaccines can be developed using peptides or RNA, e.g., synthetic peptides or synthetic RNA.

Antibody therapies are antibody proteins produced by the immune system and that bind to a target antigen on the surface of a cell. Antibodies are typically encoded by an immunoglobulin gene or genes, or fragments thereof. In normal physiology antibodies are used by the immune system to fight pathogens. Each antibody is specific to one or a few proteins, and those that bind to cancer antigens are used, *e.g.*, for the treatment of cancer. Antibodies are capable of specifically binding an antigen or epitope. (Fundamental Immunology, 3rd Edition, W.E., Paul, ed., Raven Press, N.Y. (1993). Specific binding occurs to the corresponding antigen or epitope even in the presence of a heterogeneous population of proteins and other biologics. Specific binding of an antibody indicates that it binds to its target antigen or epitope with an affinity that is substantially greater than binding to irrelevant antigens. The relative difference in affinity is often at least 25% greater, more often at least 50% greater, most often at least 100% greater. The relative difference can be at least 2-fold, at least 5-fold, at least 10-fold, at least 25-fold, at least 50-fold, at least 100-fold, or at least 1000-fold, for example.

Exemplary types of antibodies include without limitation human, humanized, chimeric, monoclonal, polyclonal, single chain, antibody binding fragments, and diabodies. Once bound to a cancer antigen, antibodies can induce antibody-dependent cell-mediated cytotoxicity, activate the complement system, prevent a receptor interacting with its ligand or deliver a payload of chemotherapy or radiation, all of which can lead to cell death. Exemplary antibodies for the treatment of cancer include but are not limited to, Alemtuzumab, Bevacizumab, Bretuximab vedotin, Cetuximab, Gemtuzumab ozogamicin, Ibritumomab tiuxetan, Ipilimumab, Ofatumumab, Panitumumab, Rituximab, Tositumomab, Trastuzumab, Nivolumab, Pembrolizumab, Avelumab, durvalumab and pidilizumab.

### Checkpoint blocking antibodies

The methods described herein comprise, in some embodiments, treating a human subject suffering from a disease or disorder described herein, the method comprising administering a composition comprising a cancer immunotherapy (*e.g*., an immunotherapeutic agent). In some embodiments, the immunotherapeutic agent is a compound (*e.g*., an inhibitor or antibody) that inhibits the immune checkpoint blockade pathway. Immune checkpoint proteins, under normal physiological conditions, maintain self-tolerance (*e.g*., prevent autoimmunity) and protect tissues from damage when the immune system is responding to *e.g.*, pathogenic infection. Immune checkpoint proteins can be dysregulated by tumors as an important immune resistance mechanism. (Pardoll, Nature Rev. Cancer, 2012, 12, 252-264). Agonists of co-stimulatory receptors or antagonists of inhibitory signals (*e.g*., immune checkpoint proteins), provide an amplification of antigen-specific T-cell responses. Antibodies that block immune checkpoints do not target tumor cells directly but typically target lymphocyte receptors or their ligands to enhance endogenous antitumor activity.

Exemplary checkpoint blocking antibodies include but are not limited to, anti-CTLA-4, anti-PD-1, anti-LAG3 (i.e., antibodies against lymphocyte activation gene 3), and anti-TIM3 (i.e., antibodies against T-cell membrane protein 3). Exemplary anti-CTLA-4 antibodies include but are not limited to, ipilimumab and tremelimumab. Exemplary anti-PD-1 ligands include but are not limited to, PD-L1 (i.e., B7-H1 and CD274) and PD-L2 (i.e., B7-DC and CD273). Exemplary anti-PD-1 antibodies include but are not limited to, nivolumab (i.e., MDX-1106, BMS-936558, or ONO-4538)), CT-011, AMP-224, pembrolizumab (trade name Keytruda), and MK-3475. Exemplary PD-L1-specific antibodies include but are not limited to, BMS936559 (i.e., MDX-1105), MEDI4736 and MPDL-3280A. Exemplary checkpoint blocking antibodies also include but are not limited to, IMP321 and MGA271.

T-regulatory cells (*e.g.*, CD4+, CD25+, or T-reg) are also involved in policing the distinction between self and non-self (*e.g*., foreign) antigens, and may represent an important mechanism in suppression of immune response in many cancers. T-reg cells can either emerge from the thymus (i.e., "natural T-reg") or can differentiate from mature T-cells under circumstances of peripheral tolerance induction (i.e., "induced T-reg"). Strategies that minimize the action of T-reg cells would therefore be expected to facilitate the immune response to tumors. (Sutmuller, van Duivernvoorde et al., 2001).

### IDO pathway inhibitors

The IDO pathway regulates immune response by suppressing T cell function and enabling local tumor immune escape. IDO expression by antigen-presenting cells (APCs) can lead to tryptophan depletion, and resulting antigen-specific T cell energy and regulatory T cell recruitment. Some tumors even express IDO to shield themselves from the immune system. A compound that inhibits IDO or the IDO pathway thereby activating the immune system to attack the cancer (e.g., tumor in a subject). Exemplary IDO pathway inhibitors include indoximod, epacadostat and EOS200271.

### STING pathway agonists

Stimulator of interferon genes (STING) is an adaptor protein that plays an important role in the activation of type I interferons in response to cytosolic nucleic acid ligands. Evidence indicates involvement of the STING pathway in the induction of antitumor immune response. It has been shown that activation of the STING-dependent pathway in cancer cells can result in tumor infiltration with immune cells and modulation of the anticancer immune response. STING agonists are being developed as a class of cancer therapeutics. Exemplary STING agonists include MK-1454 and ADU-S100.

### Co-stimulatory antibodies

The methods described herein comprise, in some embodiments, treating a human subject suffering from a disease or disorder described herein, the method comprising administering a composition comprising a cancer immunotherapy (*e.g*., an immunotherapeutic agent). In some embodiments, the immunotherapeutic agent is a co-stimulatory inhibitor or antibody. In some embodiments, the methods described herein comprise depleting or activating anti-4-1BB, anti-OX40, anti-GITR, anti-CD27 and anti-CD40, and variants thereof.

Inventive methods of the present description contemplate single as well as multiple administrations of a therapeutically effective amount of a compound as described herein. Compounds, *e.g*., a compound as described herein, can be administered at regular intervals, depending on the nature, severity and extent of the subject's condition. In some embodiments, a compound described herein is administered in a single dose. In some embodiments, a compound described herein is administered in multiple doses.

### Inflammatory Disease

In some embodiments, the compound of Formula (I) or Formula (II), or a pharmaceutically acceptable salt, solvate, hydrate, tautomer, ester, *N*-oxide or stereoisomer thereof is used to treat an inflammatory disease. As used herein, the term "inflammatory disease" refers to a disease or condition characterized by aberrant inflammation (e.g. an increased level of inflammation compared to a control such as a healthy person not suffering from a disease). Examples of inflammatory diseases include postoperative cognitive dysfunction, arthritis (e.g., rheumatoid arthritis, psoriatic arthritis, juvenile idiopathic arthritis), systemic lupus erythematosus (SLE), myasthenia gravis, juvenile onset diabetes, diabetes mellitus type 1, Guillain-Barre syndrome, Hashimoto's encephalitis, Hashimoto's thyroiditis, ankylosing spondylitis, psoriasis, Sjogren's syndrome, vasculitis, glomerulonephritis, auto-immune thyroiditis, Behcet's disease, Crohn's disease, ulcerative colitis, bullous pemphigoid, sarcoidosis, ichthyosis, Graves' ophthalmopathy, inflammatory bowel disease, Addison's disease, Vitiligo, asthma (e.g., allergic asthma), acne vulgaris, celiac disease, chronic prostatitis, inflammatory bowel disease, pelvic inflammatory disease, reperfusion injury, sarcoidosis, transplant rejection, interstitial cystitis, atherosclerosis, and atopic dermatitis. Proteins associated with inflammation and inflammatory diseases (e.g. aberrant expression being a symptom or cause or marker of the disease) include interleukin-6 (IL-6), interleukin-8 (IL-8), interleukin- 18 (IL-18), TNF-a (tumor necrosis factor-alpha), and C-reactive protein (CRP).

In some embodiments, the inflammatory disease comprises postoperative cognitive dysfunction, arthritis (e.g., rheumatoid arthritis, psoriatic arthritis, or juvenile idiopathic arthritis), systemic lupus erythematosus (SLE), myasthenia gravis, diabetes (e.g., juvenile onset diabetes or diabetes mellitus type 1), Guillain-Barre syndrome, Hashimoto's encephalitis, Hashimoto's thyroiditis, ankylosing spondylitis, psoriasis, Sjogren's syndrome, vasculitis, glomerulonephritis, auto-immune thyroiditis, Behcet's disease, Crohn's disease, ulcerative colitis, bullous pemphigoid, sarcoidosis, ichthyosis, Graves' ophthalmopathy, inflammatory bowel disease, Addison's disease, vitiligo, asthma (e.g., allergic asthma), acne vulgaris, celiac disease, chronic prostatitis, pelvic inflammatory disease, reperfusion injury, sarcoidosis, transplant rejection, interstitial cystitis, atherosclerosis, or atopic dermatitis.

In some embodiments, the inflammatory disease comprises postoperative cognitive dysfunction, which refers to a decline in cognitive function (e.g. memory or executive function (e.g. working memory, reasoning, task flexibility, speed of processing, or problem solving)) following surgery.

In other embodiments, the method of treatment is a method of prevention. For example, a method of treating postsurgical cognitive dysfunction may include preventing postsurgical cognitive dysfunction or a symptom of postsurgical cognitive dysfunction or reducing the severity of a symptom of postsurgical cognitive dysfunction by administering a compound described herein prior to surgery.

In some embodiments, the compound of Formula (I) or Formula (II), or a pharmaceutically acceptable salt, solvate, hydrate, tautomer, ester, N-oxide or stereoisomer thereof is used to treat an inflammatory disease (e.g., an inflammatory disease described herein) by decreasing or eliminating a symptom of the disease. In some embodiments, the compound of Formula (I) or Formula (II), or a pharmaceutically acceptable salt, solvate, hydrate, tautomer, ester, N-oxide or stereoisomer thereof may be used as a single agent in a composition or in combination with another agent in a composition to treat an inflammatory disease (e.g., an inflammatory disease described herein).

### Musculoskeletal Diseases

In some embodiments, the compound of Formula (I) or Formula (II), or a pharmaceutically acceptable salt, solvate, hydrate, tautomer, ester, *N*-oxide or stereoisomer thereof is used to treat a musculoskeletal disease. As used herein, the term "musculoskeletal disease" refers to a disease or condition in which the function of a subject's musculoskeletal system (e.g., muscles, ligaments, tendons, cartilage, or bones) becomes impaired. Exemplary musculoskeletal diseases that may be treated with a compound of Formula (I) or Formula (II), or a pharmaceutically acceptable salt, solvate, hydrate, tautomer, ester, *N*-oxide or stereoisomer thereof include muscular dystrophy (e.g., Duchenne muscular dystrophy, Becker muscular dystrophy, distal muscular dystrophy, congenital muscular dystrophy, Emery-Dreifuss muscular dystrophy, facioscapulohumeral muscular dystrophy, myotonic muscular dystrophy type 1, or myotonic muscular dystrophy type 2), limb girdle muscular dystrophy, multisystem proteinopathy, rhizomelic chondrodysplasia punctata, X-linked recessive chondrodysplasia punctata, Conradi-Hünermann syndrome, Autosomal dominant chondrodysplasia punctata, stress induced skeletal disorders (e.g., stress induced osteoporosis), multiple sclerosis, amyotrophic lateral sclerosis (ALS), primary lateral sclerosis, progressive muscular atrophy, progressive bulbar palsy, pseudobulbar palsy, spinal muscular atrophy, progressive spinobulbar muscular atrophy, spinal cord spasticity, spinal muscle atrophy, myasthenia gravis, neuralgia, fibromyalgia, Machado-Joseph disease, Paget's disease of bone, cramp fasciculation syndrome, Freidrich's ataxia, a muscle wasting disorder (e.g., muscle atrophy, sarcopenia, cachexia), an inclusion body myopathy, motor neuron disease, or paralysis.

In some embodiments, the compound of Formula (I) or Formula (II), or a pharmaceutically acceptable salt, solvate, hydrate, tautomer, ester, *N*-oxide or stereoisomer thereof is used to treat a musculoskeletal disease (e.g., a musculoskeletal disease described herein) by decreasing or eliminating a symptom of the disease. In some embodiments, the method of treatment comprises treatment of muscle pain or muscle stiffness associated with a musculoskeletal disease. In some embodiments, the compound of Formula (I) or Formula (II), or a pharmaceutically acceptable salt, solvate, hydrate, tautomer, ester, N-oxide or stereoisomer thereof may be used as a single agent in a composition or in combination with another agent in a composition to treat a musculoskeletal disease (e.g., a musculoskeletal disease described herein).

### Metabolic Diseases

In some embodiments, the compound of Formula (I) or Formula (II), or a pharmaceutically acceptable salt, solvate, hydrate, tautomer, ester, N-oxide or stereoisomer thereof is used to treat metabolic disease. As used herein, the term "metabolic disease" refers to a disease or condition affecting a metabolic process in a subject. Exemplary metabolic diseases that may be treated with a compound of Formula (I) or Formula (II), or a pharmaceutically acceptable salt, solvate, hydrate, tautomer, ester, *N*-oxide or stereoisomer thereof include non-alcoholic steatohepatitis (NASH), non-alcoholic fatty liver disease (NAFLD), liver fibrosis, obesity, heart disease, atherosclerosis, arthritis, cystinosis, diabetes (e.g., Type I diabetes, Type II diabetes, or gestational diabetes), phenylketonuria, proliferative retinopathy, or Kearns-Sayre disease.

In some embodiments, the compound of Formula (I) or Formula (II), or a pharmaceutically acceptable salt, solvate, hydrate, tautomer, ester, *N*-oxide or stereoisomer thereof is used to treat a metabolic disease (e.g., a metabolic disease described herein) by decreasing or eliminating a symptom of the disease. In some embodiments, the method of treatment comprises decreasing or eliminating a symptom comprising elevated blood pressure, elevated blood sugar level, weight gain, fatigue, blurred vision, abdominal pain, flatulence, constipation, diarrhea, jaundice, and the like. In some embodiments, the compound of Formula (I) or Formula (II), or a pharmaceutically acceptable salt, solvate, hydrate, tautomer, ester, N-oxide or stereoisomer thereof may be used as a single agent in a composition or in combination with another agent in a composition to treat a metabolic disease (e.g., a musculoskeletal disease described herein).

### Mitochondrial Diseases

In some embodiments, the compound of Formula (I) or Formula (II) or a pharmaceutically acceptable salt, solvate, hydrate, tautomer, ester, N-oxide or stereoisomer thereof is used to treat mitochondrial disease. As used herein, the term "mitochondrial disease" refers to a disease or condition affecting the mitochondria in a subject. In some embodiments, the mitochondrial disease is associated with, or is a result of, or is caused by mitochondrial dysfunction, one or more mitochondrial protein mutations, or one or more mitochondrial DNA mutations. In some embodiments, the mitochondrial disease is a mitochondrial myopathy. In some embodiments, mitochondrial diseases, e.g., the mitochondrial myopathy, that may be treated with a compound of Formula (I) or Formula (II) or a pharmaceutically acceptable salt, solvate, hydrate, tautomer, ester, *N*-oxide or stereoisomer thereof include, e.g., Barth syndrome, chronic progressive external ophthalmoplegia (cPEO), Kearns-Sayre syndrome (KSS), Leigh syndrome (e.g., MILS, or maternally inherited Leigh syndrome), mitochondrial DNA depletion syndromes (MDDS, e.g., Alpers syndrome), mitochondrial encephalomyopathy (e.g., mitochondrial encephalomyopathy, lactic acidosis, and stroke-like episodes (MELAS)), mitochondrial neurogastrointestinal encephalomyopathy (MNGIE), myoclonus epilepsy with ragged red fibers (MERRF), neuropathy, ataxia, retinitis pigmentosa (NARP), Leber's hereditary optic neuropathy (LHON), and Pearson syndrome.

In some embodiments, the compound of Formula (I) or Formula (II) or a pharmaceutically acceptable salt, solvate, hydrate, tautomer, ester, N-oxide or stereoisomer thereof is used to treat a mitochondrial disease described herein by decreasing or eliminating a symptom of the disease. In some embodiments, the compound of Formula (I) or Formula (II) or a pharmaceutically acceptable salt, solvate, hydrate, tautomer, ester, N-oxide or stereoisomer thereof may be used as a single agent in a composition or in combination with another agent in a composition to treat a mitochondrial disease described herein.

### Hearing Loss

In some embodiments, the compound of Formula (I) or Formula (II) or a pharmaceutically acceptable salt, solvate, hydrate, tautomer, ester, *N*-oxide or stereoisomer thereof is used to treat hearing loss. As used herein, the term "hearing loss" or "hearing loss condition" may broadly encompass any damage to the auditory systems, organs, and cells or any impairment of an animal subject's ability to hear sound, as measured by standard methods and assessments known in the art, for example otoacoustic emission testing, pure tone testing, and auditory brainstem response testing. Exemplary hearing loss conditions that may be treated with a compound of Formula (I) or Formula (II), or a pharmaceutically acceptable salt, solvate, hydrate, tautomer, ester, N-oxide or stereoisomer thereof include, but are not limited to, mitochondrial nonsyndromic hearing loss and deafness, hair cell death, age-related hearing loss, noise-induced hearing loss, genetic or inherited hearing loss, hearing loss experienced as a result of ototoxic exposure, hearing loss resulting from disease, and hearing loss resulting from trauma. In some embodiments, mitochondrial nonsyndromic hearing loss and deafness is a MT-RNR1-related hearing loss. In some embodiments, the MT-RNR1-related hearing loss is the result of amino glycoside ototoxicity. In some embodiments, mitochondrial nonsyndromic hearing loss and deafness is a MT-TS1-related hearing loss. In some embodiments, mitochondrial nonsyndromic hearing loss and deafness is characterized by sensorineural hearing loss.

In some embodiments, the compound of Formula (I) or Formula (II) or a pharmaceutically acceptable salt, solvate, hydrate, tautomer, ester, N-oxide or stereoisomer thereof is used to treat a hearing loss condition described herein by decreasing or eliminating a symptom of the disease. In some embodiments, the compound of Formula (I) or Formula (II) or a pharmaceutically acceptable salt, solvate, hydrate, tautomer, ester, N-oxide or stereoisomer thereof may be used as a single agent in a composition or in combination with another agent in a composition to treat a hearing loss condition described herein.

### Ocular Disease

In some embodiments, the compound of Formula (I) or Formula (II) or a pharmaceutically acceptable salt, solvate, hydrate, tautomer, ester, N-oxide or stereoisomer thereof is used to treat eye disease. As used herein, the term "ocular disease" may refer to a disease or condition in which the function of a subject's eye becomes impaired. Exemplary ocular diseases and conditions that may be treated with a compound of Formula (I) or Formula (II), or a pharmaceutically acceptable salt, solvate, hydrate, tautomer, ester, *N*-oxide or stereoisomer thereof include cataracts, glaucoma, endoplasmic reticulum (ER) stress, autophagy deficiency, age-related macular degeneration (AMD), or diabetic retinopathy.

In some embodiments, the compound of Formula (I) or Formula (II) or a pharmaceutically acceptable salt, solvate, hydrate, tautomer, ester, *N*-oxide or stereoisomer thereof is used to treat an ocular disease or condition described herein by decreasing or eliminating a symptom of the disease. In some embodiments, the compound of Formula (I) or Formula (II) or a pharmaceutically acceptable salt, solvate, hydrate, tautomer, ester, *N*-oxide or stereoisomer thereof may be used as a single agent in a composition or in combination with another agent in a composition to treat an ocular disease or condition described herein.

### Kidney Diseases

In some embodiments, the compound of Formula (I) or Formula (II) or a pharmaceutically acceptable salt, solvate, hydrate, tautomer, ester, *N*-oxide or stereoisomer thereof is used to treat kidney disease. As used herein, the term "kidney disease" may refer to a disease or condition in which the function of a subject's kidneys becomes impaired. Exemplary kidney diseases that may be treated with a compound of Formula (I) or Formula (II), or a pharmaceutically acceptable salt, solvate, hydrate, tautomer, ester, N-oxide or stereoisomer thereof include Abderhalden-Kaufmann-Lignac syndrome (Nephropathic Cystinosis), Abdominal Compartment Syndrome, Acetaminophen-induced Nephrotoxicity, Acute Kidney Failure/Acute Kidney Injury, Acute Lobar Nephronia, Acute Phosphate Nephropathy, Acute Tubular Necrosis, Adenine Phosphoribosyltransferase Deficiency, Adenovirus Nephritis, Alagille Syndrome, Alport Syndrome, Amyloidosis, ANCA Vasculitis Related to Endocarditis and Other Infections, Angiomyolipoma, Analgesic Nephropathy, Anorexia Nervosa and Kidney Disease, Angiotensin Antibodies and Focal Segmental Glomerulosclerosis, Antiphospholipid Syndrome, Anti-TNF-α Therapy-related Glomerulonephritis, APOL1 Mutations, Apparent Mineralocorticoid Excess Syndrome, Aristolochic Acid Nephropathy, Chinese Herbal Nephropathy, Balkan Endemic Nephropathy, Arteriovenous Malformations and Fistulas of the Urologic Tract, Autosomal Dominant Hypocalcemia, Bardet-Biedl Syndrome, Bartter Syndrome, Bath Salts and Acute Kidney Injury, Beer Potomania, Beeturia, β-Thalassemia Renal Disease, Bile Cast Nephropathy, BK Polyoma Virus Nephropathy in the Native Kidney, Bladder Rupture, Bladder Sphincter Dyssynergia, Bladder Tamponade, Border-Crossers' Nephropathy, Bourbon Virus and Acute Kidney Injury, Burnt Sugarcane Harvesting and Acute Renal Dysfunction, Byetta and Renal Failure, C1q Nephropathy, C3 Glomerulopathy, C3 Glomerulopathy with Monoclonal Gammopathy, C4 Glomerulopathy, Calcineurin Inhibitor Nephrotoxicity, Callilepsis Laureola Poisoning. Cannabinoid Hyperemesis Acute Renal Failure, Cardiorenal syndrome, Carfilzomib-Indiced Renal Injury, CFHR5 nephropathy, Charcot-Marie-Tooth Disease with Glomerulopathy, Chinese Herbal Medicines and Nephrotoxicity, Cherry Concentrate and Acute Kidney Injury, Cholesterol Emboli, Churg-Strauss syndrome, Chyluria, Ciliopathy, Cocaine and the Kidney, Cold Diuresis, Colistin Nephrotoxicity, Collagenofibrotic Glomerulopathy, Collapsing Glomerulopathy, Collapsing Glomerulopathy Related to CMV, Combination Antiretroviral (cART) Related-Nephropathy, Congenital Anomalies of the Kidney and Urinary Tract (CAKUT), Congenital Nephrotic Syndrome, Congestive Renal Failure, Conorenal syndrome (Mainzer-Saldino Syndrome or Saldino-Mainzer Disease), Contrast Nephropathy, Copper Sulphate Intoxication, Cortical Necrosis, Crizotinib-related Acute Kidney Injury, Cryocrystalglobulinemia, Cryoglobuinemia, Crystalglobulin-Induced Nephropathy, Crystal-Induced Acute Kidney injury, Crystal-Storing Histiocytosis, Cystic Kidney Disease, Acquired, Cystinuria, Dasatinib-Induced Nephrotic-Range Proteinuria, Dense Deposit Disease (MPGN Type 2), Dent Disease (X-linked Recessive Nephrolithiasis), DHA Crystalline Nephropathy, Dialysis Disequilibrium Syndrome, Diabetes and Diabetic Kidney Disease, Diabetes Insipidus, Dietary Supplements and Renal Failure, Diffuse Mesangial Sclerosis, Diuresis, Djenkol Bean Poisoning (Djenkolism), Down Syndrome and Kidney Disease, Drugs of Abuse and Kidney Disease, Duplicated Ureter, EAST syndrome, Ebola and the Kidney, Ectopic Kidney, Ectopic Ureter, Edema, Swelling, Erdheim-Chester Disease, Fabry's Disease, Familial Hypocalciuric Hypercalcemia, Fanconi Syndrome, Fraser syndrome, Fibronectin Glomerulopathy, Fibrillary Glomerulonephritis and Immunotactoid Glomerulopathy, Fraley syndrome, Fluid Overload, Hypervolemia, Focal Segmental Glomerulosclerosis, Focal Sclerosis, Focal Glomerulosclerosis, Galloway Mowat syndrome, Giant Cell (Temporal) Arteritis with Kidney Involvement, Gestational Hypertension, Gitelman Syndrome, Glomerular Diseases, Glomerular Tubular Reflux, Glycosuria, Goodpasture Syndrome, Green Smoothie Cleanse Nephropathy, HANAC Syndrome, Harvoni (Ledipasvir with Sofosbuvir)-Induced Renal Injury, Hair Dye Ingestion and Acute Kidney Injury, Hantavirus Infection Podocytopathy, Heat Stress Nephropathy, Hematuria (Blood in Urine), Hemolytic Uremic Syndrome (HUS), Atypical Hemolytic Uremic Syndrome (aHUS), Hemophagocytic Syndrome, Hemorrhagic Cystitis, Hemorrhagic Fever with Renal Syndrome (HFRS, Hantavirus Renal Disease, Korean Hemorrhagic Fever, Epidemic Hemorrhagic Fever, Nephropathis Epidemica), Hemosiderinuria, Hemosiderosis related to Paroxysmal Nocturnal Hemoglobinuria and Hemolytic Anemia, Hepatic Glomerulopathy, Hepatic Veno-Occlusive Disease, Sinusoidal Obstruction Syndrome, Hepatitis C-Associated Renal Disease, Hepatocyte Nuclear Factor 1β-Associated Kidney Disease, Hepatorenal Syndrome, Herbal Supplements and Kidney Disease, High Altitude Renal Syndrome, High Blood Pressure and Kidney Disease, HIV-Associated Immune Complex Kidney Disease (HIVICK), HIV-Associated Nephropathy (HIVAN), HNF1B-related Autosomal Dominant Tubulointerstitial Kidney Disease, Horseshoe Kidney (Renal Fusion), Hunner's Ulcer, Hydroxychloroquine-induced Renal Phospholipidosis, Hyperaldosteronism, Hypercalcemia, Hyperkalemia, Hypermagnesemia, Hypernatremia, Hyperoxaluria, Hyperphosphatemia, Hypocalcemia, Hypocomplementemic Urticarial Vasculitic Syndrome, Hypokalemia, Hypokalemia-induced renal dysfunction, Hypokalemic Periodic Paralysis, Hypomagnesemia, Hyponatremia, Hypophosphatemia, Hypophosphatemia in Users of Cannabis, Hypertension, Hypertension, Monogenic, Iced Tea Nephropathy, Ifosfamide Nephrotoxicity, IgA Nephropathy, IgG4 Nephropathy, Immersion Diuresis, Immune-Checkpoint Therapy-Related Interstitial Nephritis, Infliximab-Related Renal Disease, Interstitial Cystitis, Painful Bladder Syndrome (Questionnaire), Interstitial Nephritis, Interstitial Nephritis, Karyomegalic, Ivemark's syndrome, JC Virus Nephropathy, Joubert Syndrome, Ketamine-Associated Bladder Dysfunction, Kidney Stones, Nephrolithiasis, Kombucha Tea Toxicity, Lead Nephropathy and Lead-Related Nephrotoxicity, Lecithin Cholesterol Acyltransferase Deficiency (LCAT Deficiency), Leptospirosis Renal Disease, Light Chain Deposition Disease, Monoclonal Immunoglobulin Deposition Disease, Light Chain Proximal Tubulopathy, Liddle Syndrome, Lightwood-Albright Syndrome, Lipoprotein Glomerulopathy, Lithium Nephrotoxicity, LMX1B Mutations Cause Hereditary FSGS, Loin Pain Hematuria, Lupus, Systemic Lupus Erythematosis, Lupus Kidney Disease, Lupus Nephritis, Lupus Nephritis with Antineutrophil Cytoplasmic Antibody Seropositivity, Lupus Podocytopathy, Lyme Disease-Associated Glomerulonephritis, Lysinuric Protein Intolerance, Lysozyme Nephropathy, Malarial Nephropathy, Malignancy-Associated Renal Disease, Malignant Hypertension, Malakoplakia, McKittrick-Wheelock Syndrome, MDMA (Molly; Ecstacy; 3,4-Methylenedioxymethamphetamine) and Kidney Failure, Meatal Stenosis, Medullary Cystic Kidney Disease, Urolodulin-Associated Nephropathy, Juvenile Hyperuricemic Nephropathy Type 1, Medullary Sponge Kidney, Megaureter, Melamine Toxicity and the Kidney, MELAS Syndrome, Membranoproliferative Glomerulonephritis, Membranous Nephropathy, Membranous-like Glomerulopathy with Masked IgG Kappa Deposits, MesoAmerican Nephropathy, Metabolic Acidosis, Metabolic Alkalosis, Methotrexate-related Renal Failure, Microscopic Polyangiitis, Milk-alkalai syndrome, Minimal Change Disease, Monoclonal Gammopathy of Renal Significance, Dysproteinemia, Mouthwash Toxicity, MUC1 Nephropathy, Multicystic dysplastic kidney, Multiple Myeloma, Myeloproliferative Neoplasms and Glomerulopathy, Nail-patella Syndrome, NARP Syndrome, Nephrocalcinosis, Nephrogenic Systemic Fibrosis, Nephroptosis (Floating Kidney, Renal Ptosis), Nephrotic Syndrome, Neurogenic Bladder, 9/11 and Kidney Disease, Nodular Glomerulosclerosis, Non-Gonococcal Urethritis, Nutcracker syndrome, Oligomeganephronia, Orofaciodigital Syndrome, Orotic Aciduria, Orthostatic Hypotension, Orthostatic Proteinuria, Osmotic Diuresis, Osmotic Nephrosis, Ovarian Hyperstimulation Syndrome, Oxalate Nephropathy, Page Kidney, Papillary Necrosis, Papillorenal Syndrome (Renal-Coloboma Syndrome, Isolated Renal Hypoplasia), PARN Mutations and Kidney Disease, Parvovirus B19 and the Kidney, The Peritoneal-Renal Syndrome, POEMS Syndrome, Posterior Urethral Valve, Podocyte Infolding Glomerulopathy, Post-infectious Glomerulonephritis, Post-streptococcal Glomerulonephritis, Post-infectious Glomerulonephritis, Atypical, Post-Infectious Glomerulonephritis (IgA-Dominant), Mimicking IgA Nephropathy, Polyarteritis Nodosa, Polycystic Kidney Disease, Posterior Urethral Valves, Post-Obstructive Diuresis, Preeclampsia, Propofol infusion syndrome, Proliferative Glomerulonephritis with Monoclonal IgG Deposits (Nasr Disease), Propolis (Honeybee Resin) Related Renal Failure, Proteinuria (Protein in Urine), Pseudohyperaldosteronism, Pseudohypobicarbonatemia, Pseudohypoparathyroidism, Pulmonary-Renal Syndrome, Pyelonephritis (Kidney Infection), Pyonephrosis, Pyridium and Kidney Failure, Radiation Nephropathy, Ranolazine and the Kidney, Refeeding syndrome, Reflux Nephropathy, Rapidly Progressive Glomerulonephritis, Renal Abscess, Peripnephric Abscess, Renal Agenesis, Renal Arcuate Vein Microthrombi-Associated Acute Kidney Injury, Renal Artery Aneurysm, Renal Artery Dissection, Spontaneous, Renal Artery Stenosis, Renal Cell Cancer, Renal Cyst, Renal Hypouricemia with Exercise-induced Acute Renal Failure, Renal Infarction, Renal Osteodystrophy, Renal Tubular Acidosis, Renin Mutations and Autosomal Dominant Tubulointerstitial Kidney Disease, Renin Secreting Tumors (Juxtaglomerular Cell Tumor), Reset Osmostat, Retrocaval Ureter, Retroperitoneal Fibrosis, Rhabdomyolysis, Rhabdomyolysis related to Bariatric Sugery, Rheumatoid Arthritis-Associated Renal Disease, Sarcoidosis Renal Disease, Salt Wasting, Renal and Cerebral, Schistosomiasis and Glomerular Disease, Schimke immuno-osseous dysplasia, Scleroderma Renal Crisis, Serpentine Fibula-Polycystic Kidney Syndrome, Exner Syndrome, Sickle Cell Nephropathy, Silica Exposure and Chronic Kidney Disease, Sri Lankan Farmers' Kidney Disease, Sjögren's Syndrome and Renal Disease, Synthetic Cannabinoid Use and Acute Kidney Injury, Kidney Disease Following Hematopoietic Cell Transplantation, Kidney Disease Related to Stem Cell Transplantation, TAFRO Syndrome, Tea and Toast Hyponatremia, Tenofovir-Induced Nephrotoxicity, Thin Basement Membrane Disease, Benign Familial Hematuria, Thrombotic Microangiopathy Associated with Monoclonal Gammopathy, Trench Nephritis, Trigonitis, Tuberculosis, Genitourinary, Tuberous Sclerosis, Tubular Dysgenesis, Immune Complex Tubulointerstitial Nephritis Due to Autoantibodies to the Proximal Tubule Brush Border, Tumor Lysis Syndrome, Uremia, Uremic Optic Neuropathy, Ureteritis Cystica, Ureterocele, Urethral Caruncle, Urethral Stricture, Urinary Incontinence, Urinary Tract Infection, Urinary Tract Obstruction, Urogenital Fistula, Uromodulin-Associated Kidney Disease, Vancomycin-Associated Cast Nephropathy, Vasomotor Nephropathy, Vesicointestinal Fistula, Vesicoureteral Reflux, VGEF Inhibition and Renal Thrombotic Microangiopathy, Volatile Anesthetics and Acute Kidney Injury, Von Hippel-Lindau Disease, Waldenstrom's Macroglobulinemic Glomerulonephritis, Warfarin-Related Nephropathy, Wasp Stings and Acute Kidney Injury, Wegener's Granulomatosis, Granulomatosis with Polyangiitis, West Nile Virus and Chronic Kidney Disease, Wunderlich syndrome, Zellweger Syndrome, or Cerebrohepatorenal Syndrome.

In some embodiments, the compound of Formula (I) or Formula (II) or a pharmaceutically acceptable salt, solvate, hydrate, tautomer, ester, N-oxide or stereoisomer thereof is used to treat a kidney disease described herein by decreasing or eliminating a symptom of the disease. In some embodiments, the compound of Formula (I) or Formula (II) or a pharmaceutically acceptable salt, solvate, hydrate, tautomer, ester, N-oxide or stereoisomer thereof may be used as a single agent in a composition or in combination with another agent in a composition to treat a kidney disease described herein.

### Skin Diseases

In some embodiments, the compound of Formula (I) or Formula (II) or a pharmaceutically acceptable salt, solvate, hydrate, tautomer, ester, *N*-oxide or stereoisomer thereof is used to treat a skin disease. As used herein, the term "skin disease" may refer to a disease or condition affecting the skin. Exemplary skin diseases that may be treated with a compound of Formula (I) or Formula (II), or a pharmaceutically acceptable salt, solvate, hydrate, tautomer, ester, *N*-oxide or stereoisomer thereof include acne, alopecia areata, basal cell carcinoma, Bowen's disease, congenital erythropoietic porphyria, contact dermatitis, Darier's disease, disseminated superficial actinic porokeratosis, dystrophic epidermolysis bullosa, eczema (atopic eczema), extra-mammary Paget's disease, epidermolysis bullosa simplex, erythropoietic protoporphyria, fungal infections of nails, Hailey-Hailey disease, herpes simplex, hidradenitis suppurativa, hirsutism, hyperhidrosis, ichthyosis, impetigo, keloids, keratosis pilaris, lichen planus, lichen sclerosus, melanoma, melasma, mucous membrane pemphigoid, pemphigoid, pemphigus vulgaris, pityriasis lichenoides, pityriasis rubra pilaris, plantar warts (verrucas), polymorphic light eruption, psoriasis, plaque psoriasis, pyoderma gangrenosum, rosacea, scabies, scleroderma, shingles, squamous cell carcinoma, sweet's syndrome, urticaria and angioedema and vitiligo.

In some embodiments, the compound of Formula (I) or Formula (II) or a pharmaceutically acceptable salt, solvate, hydrate, tautomer, ester, *N*-oxide or stereoisomer thereof is used to treat a skin disease described herein by decreasing or eliminating a symptom of the disease. In some embodiments, the compound of Formula (I) or Formula (II) or a pharmaceutically acceptable salt, solvate, hydrate, tautomer, ester, *N*-oxide or stereoisomer thereof may be used as a single agent in a composition or in combination with another agent in a composition to treat a skin disease described herein.

### Fibrotic Diseases

In some embodiments, the compound of Formula (I) or Formula (II) or a pharmaceutically acceptable salt, solvate, hydrate, tautomer, ester, *N*-oxide or stereoisomer thereof is used to treat a fibrotic disease. As used herein, the term "fibrotic disease" may refer to a disease or condition that is defined by the accumulation of excess extracellular matrix components. Exemplary fibrotic diseases that may be treated with a compound of Formula (I) or Formula (II), or a pharmaceutically acceptable salt, solvate, hydrate, tautomer, ester, N-oxide or stereoisomer thereof include adhesive capsulitis, arterial stiffness, arthrofibrosis, atrial fibrosis, cardiac fibrosis, cirrhosis, congenital hepatic fibrosis, Crohn's disease, cystic fibrosis, Dupuytren's contracture, endomyocardial fibrosis, glial scar, hepatitis C, hypertrophic cardiomyopathy, hypersensitivity pneumonitis, idiopathic pulmonary fibrosis, idiopathic interstitial pneumonia, interstitial lung disease, keloid, mediastinal fibrosis, myelofibrosis, nephrogenic systemic fibrosis, non-alcoholic fatty liver disease, old myocardial infarction, Peyronie's disease, pneumoconiosis, pneumonitis, progressive massive fibrosis, pulmonary fibrosis, radiation-induced lung injury, retroperitoneal fibrosis, scleroderma/systemic sclerosis, silicosis and ventricular remodeling.

In some embodiments, the compound of Formula (I) or Formula (II) or a pharmaceutically acceptable salt, solvate, hydrate, tautomer, ester, *N*-oxide or stereoisomer thereof is used to treat a fibrotic disease described herein by decreasing or eliminating a symptom of the disease. In some embodiments, the compound of Formula (I) or Formula (II) or a pharmaceutically acceptable salt, solvate, hydrate, tautomer, ester, *N*-oxide or stereoisomer thereof may be used as a single agent in a composition or in combination with another agent in a composition to treat a fibrotic disease described herein.

### Hemoglobin Disorders

In some embodiments, the compound of Formula (I) or Formula (II) or a pharmaceutically acceptable salt, solvate, hydrate, tautomer, ester, *N*-oxide or stereoisomer thereof is used to treat a hemoglobin disease. As used herein, the terms "hemoglobin disease" or "hemoglobin disorder" may refer to a disease or condition characterized by an abnormal production or structure of the hemoglobin protein. Exemplary hemoglobin diseases that may be treated with a compound of Formula (I) or Formula (II), or a pharmaceutically acceptable salt, solvate, hydrate, tautomer, ester, N-oxide or stereoisomer thereof include "dominant" β-thalassemia, acquired (toxic) methemoglobinemia, carboxyhemoglobinemia, congenital Heinz body hemolytic anemia, HbH disease, HbS/β-thalassemia, HbE/β-thalassemia, HbSC disease, homozygous α⁺-thalassemia (phenotype of α⁰-thalassemia), Hydrops fetalis with Hb Bart's, sickle cell anemia/disease, sickle cell trait, sickle β-thalassemia disease, α⁺-thalassemia, α⁰-thalassemia, α-Thalassemia associated with myelodysplastic syndromes, α-Thalassemia with mental retardation syndrome (ATR), β⁰-Thalassemia, β⁺-Thalassemia, δ-Thalassemia, γ-Thalassemia, β-Thalassemia major, β-Thalassemia intermedia, δβ-Thalassemia, and εγδβ-Thalassemia.

In some embodiments, the compound of Formula (I) or Formula (II) or a pharmaceutically acceptable salt, solvate, hydrate, tautomer, ester, N-oxide or stereoisomer thereof is used to treat a hemoglobin disease described herein by decreasing or eliminating a symptom of the disease. In some embodiments, the compound of Formula (I) or Formula (II) or a pharmaceutically acceptable salt, solvate, hydrate, tautomer, ester, *N*-oxide or stereoisomer thereof may be used as a single agent in a composition or in combination with another agent in a composition to treat a hemoglobin disease described herein.

### Autoimmune Diseases

In some embodiments, the compound of Formula (I) or Formula (II) or a pharmaceutically acceptable salt, solvate, hydrate, tautomer, ester, *N*-oxide or stereoisomer thereof is used to treat an autoimmune disease. As used herein, the term "autoimmune disease" may refer to a disease or condition in which the immune system of a subject attacks and damages the tissues of said subject. Exemplary kidney diseases that may be treated with a compound of Formula (I) or Formula (II), or a pharmaceutically acceptable salt, solvate, hydrate, tautomer, ester, N-oxide or stereoisomer thereof include Achalasia, Addison's disease, Adult Still's disease, Agammaglobulinemia, Alopecia areata, Amyloidosis, Ankylosing spondylitis, Anti-GBM/Anti-TBM nephritis, Antiphospholipid syndrome, Autoimmune angioedema, Autoimmune dysautonomia, Autoimmune encephalomyelitis, Autoimmune hepatitis, Autoimmune inner ear disease (AIED), Autoimmune myocarditis, Autoimmune oophoritis, Autoimmune orchitis, Autoimmune pancreatitis, Autoimmune retinopathy, Autoimmune urticaria, Axonal & neuronal neuropathy (AMAN), Baló disease, Behcet's disease, Benign mucosal pemphigoid, Bullous pemphigoid, Castleman disease (CD), Celiac disease, Chagas disease, Chronic inflammatory demyelinating polyneuropathy (CIDP), Chronic recurrent multifocal osteomyelitis (CRMO), Churg-Strauss Syndrome (CSS) or Eosinophilic Granulomatosis (EGPA), Cicatricial pemphigoid, Cogan's syndrome, Cold agglutinin disease, Congenital heart block, Coxsackie myocarditis, CREST syndrome, Crohn's disease, Dermatitis herpetiformis, Dermatomyositis, Devic's disease (neuromyelitis optica), Discoid lupus, Dressler's syndrome, Endometriosis, Eosinophilic esophagitis (EoE), Eosinophilic fasciitis, Erythema nodosum, Essential mixed cryoglobulinemia, Evans syndrome, Fibromyalgia, Fibrosing alveolitis, Giant cell arteritis (temporal arteritis), Giant cell myocarditis, Glomerulonephritis, Goodpasture's syndrome, Granulomatosis with Polyangiitis, Graves' disease, Guillain-Barre syndrome, Hashimoto's thyroiditis, Hemolytic anemia, Henoch-Schonlein purpura (HSP), Herpes gestationis or pemphigoid gestationis (PG), Hidradenitis Suppurativa (HS) (Acne Inversa), Hypogammalglobulinemia, IgA Nephropathy, IgG4-related sclerosing disease, Immune thrombocytopenic purpura (ITP), Inclusion body myositis (IBM), Interstitial cystitis (IC), Juvenile arthritis, Juvenile diabetes (Type 1 diabetes), Juvenile myositis (JM), Kawasaki disease, Lambert-Eaton syndrome, Leukocytoclastic vasculitis, Lichen planus, Lichen sclerosus, Ligneous conjunctivitis, Linear IgA disease (LAD), Lupus, Lyme disease chronic, Meniere's disease, Microscopic polyangiitis (MPA), Mixed connective tissue disease (MCTD), Mooren's ulcer, Mucha-Habermann disease, Multifocal Motor Neuropathy (MMN) or MMNCB, Multiple sclerosis, Myasthenia gravis, Myositis, Narcolepsy, Neonatal Lupus, Neuromyelitis optica, Neutropenia, Ocular cicatricial pemphigoid, Optic neuritis, Palindromic rheumatism (PR), PANDAS, Paraneoplastic cerebellar degeneration (PCD), Paroxysmal nocturnal hemoglobinuria (PNH), Parry Romberg syndrome, Pars planitis (peripheral uveitis), Parsonnage-Turner syndrome,, Pemphigus, Peripheral neuropathy, Perivenous encephalomyelitis, Pernicious anemia (PA), POEMS syndrome, Polyarteritis nodosa, Polyglandular syndrome type I, Polyglandular syndrome type II, Polyglandular syndrome type III, Polymyalgia rheumatica, Polymyositis, Postmyocardial infarction syndrome, Postpericardiotomy syndrome, Primary biliary cirrhosis, Primary sclerosing cholangitis, Progesterone dermatitis, Psoriasis, Psoriatic arthritis, Pure red cell aplasia (PRCA), Pyoderma gangrenosum, Raynaud's phenomenon, Reactive Arthritis, Reflex sympathetic dystrophy, Relapsing polychondritis, Restless legs syndrome (RLS), Retroperitoneal fibrosis, Rheumatic fever, Rheumatoid arthritis, Sarcoidosis, Schmidt syndrome, Scleritis, Scleroderma, Sjögren's syndrome, Sperm & testicular autoimmunity, Stiff person syndrome (SPS), Subacute bacterial endocarditis (SBE), Susac's syndrome, Sympathetic ophthalmia (SO), Takayasu's arteritis, Temporal arteritis/Giant cell arteritis, Thrombocytopenic purpura (TTP), Tolosa-Hunt syndrome (THS), Transverse myelitis, Type 1 diabetes, Ulcerative colitis (UC), Undifferentiated connective tissue disease (UCTD), Uveitis, Vasculitis, Vitiligo, Vogt-Koyanagi-Harada Disease, and Wegener's granulomatosis (or Granulomatosis with Polyangiitis (GPA)).

In some embodiments, the compound of Formula (I) or Formula (II) or a pharmaceutically acceptable salt, solvate, hydrate, tautomer, ester, *N*-oxide or stereoisomer thereof is used to treat an autoimmune disease described herein by decreasing or eliminating a symptom of the disease. In some embodiments, the compound of Formula (I) or Formula (II) or a pharmaceutically acceptable salt, solvate, hydrate, tautomer, ester, *N*-oxide or stereoisomer thereof may be used as a single agent in a composition or in combination with another agent in a composition to treat an autoimmune disease described herein.

### Viral Infections

In some embodiments, the compound of Formula (I) or Formula (II) or a pharmaceutically acceptable salt, solvate, hydrate, tautomer, ester, *N*-oxide or stereoisomer thereof is used to treat a viral infection. Exemplary viral infections that may be treated with a compound of Formula (I) or Formula (II), or a pharmaceutically acceptable salt, solvate, hydrate, tautomer, ester, *N*-oxide or stereoisomer thereof include influenza, human immunodeficiency virus (HIV) and herpes.

In some embodiments, the compound of Formula (I) or Formula (II) or a pharmaceutically acceptable salt, solvate, hydrate, tautomer, ester, *N*-oxide or stereoisomer thereof is used to treat a viral infection described herein by decreasing or eliminating a symptom of the disease. In some embodiments, the compound of Formula (I) or Formula (II) or a pharmaceutically acceptable salt, solvate, hydrate, tautomer, ester, *N*-oxide or stereoisomer thereof may be used as a single agent in a composition or in combination with another agent in a composition to treat a viral infection described herein.

### Malaria Infection

In some embodiments, the compound of Formula (I) or Formula (II) or a pharmaceutically acceptable salt, solvate, hydrate, tautomer, ester, *N*-oxide or stereoisomer thereof is used to treat a malaria. As used herein, the term "malaria" may refer to a parasitic disease of protozoan of the plasmodium genus that causes infection of red blood cells (RBCs). Exemplary forms of malaria infection that may be treated with a compound of Formula (I) or Formula (II), or a pharmaceutically acceptable salt, solvate, hydrate, tautomer, ester, N-oxide or stereoisomer thereof include infection caused by Plasmodium vivax, Plasmodium ovale, Plasmodium malariae and Plasmodium falciparum. In some embodiments, the malaria infection that may be treated with a compound of Formula (I) or Formula (II), or a pharmaceutically acceptable salt, solvate, hydrate, tautomer, ester, *N*-oxide or stereoisomer thereof is resistant/recrudescent malaria.

In some embodiments, the compound of Formula (I) or Formula (II) or a pharmaceutically acceptable salt, solvate, hydrate, tautomer, ester, *N*-oxide or stereoisomer thereof is used to treat a malaria infection described herein by decreasing or eliminating a symptom of the disease. In some embodiments, the compound of Formula (I) or Formula (II) or a pharmaceutically acceptable salt, solvate, hydrate, tautomer, ester, *N*-oxide or stereoisomer thereof may be used as a single agent in a composition or in combination with another agent in a composition to treat a malaria infection described herein.

### Diseases with Mutations Leading to Unfolded Protein Response (UPR) Induction

In some embodiments, the compound of Formula (I) or Formula (II) or a pharmaceutically acceptable salt, solvate, hydrate, tautomer, ester, *N*-oxide or stereoisomer thereof is used to treat a disease with mutations that leads to UPR induction. Exemplary disease with mutations that lead to UPR induction include Marinesco-Sjogren syndrome, neuropathic pain, diabetic neuropathic pain, noise induced hearing loss, non-syndromic sensorineural hearing loss, age-related hearing loss, Wolfram syndrome, Darier White disease, Usher syndrome, collagenopathies, Thin basement nephropathy, Alport syndrome, skeletal chondrodysplasia, metaphyseal chondrodysplasia type Schmid, and Pseudochondrodysplasia.

In some embodiments, the compound of Formula (I) or Formula (II) or a pharmaceutically acceptable salt, solvate, hydrate, tautomer, ester, *N*-oxide or stereoisomer thereof is used to treat a disease with mutations that leads to UPR induction described herein by decreasing or eliminating a symptom of the disease. In some embodiments, the compound of Formula (I) or Formula (II) or a pharmaceutically acceptable salt, solvate, hydrate, tautomer, ester, *N*-oxide or stereoisomer thereof may be used as a single agent in a composition or in combination with another agent in a composition to treat a disease with mutations that leads to UPR induction described herein.

### Methods of Modulating Protein Production

In another aspect, disclosed herein is a method of modulating the expression of eIF2B, eIF2α, a component of the eIF2 pathway, component of the ISR pathway or any combination thereof in a cell, the method comprising contacting the cell with an effective amount of a compound of Formula (I) or Formula (II), or a pharmaceutically acceptable salt, solvate, hydrate, tautomer, ester, *N*-oxide or stereoisomer thereof, thereby modulating the expression of eIF2B, eIF2α, a component of the eIF2 pathway, component of the ISR pathway or any combination thereof in the cell. In some embodiments, contacting the compound of Formula (I) or Formula (II), or a pharmaceutically acceptable salt, solvate, hydrate, tautomer, ester, *N*-oxide or stereoisomer thereof with the cell increases the expression of eIF2B, eIF2α, a component of the eIF2 pathway, component of the ISR pathway or any combination thereof in the cell. In some embodiments, contacting the compound of Formula (I) or Formula (II), or a pharmaceutically acceptable salt, solvate, hydrate, tautomer, ester, *N*-oxide or stereoisomer thereof with the cell decreases the expression of eIF2B, eIF2α, a component of the eIF2 pathway, component of the ISR pathway or any combination thereof in the cell.

In another aspect, disclosed herein is a method of preventing or treating a condition, disease or disorder described herein in a patient in need thereof, the method comprising administering to the patient an effective amount of a compound of Formula (I) or Formula (II), or a pharmaceutically acceptable salt, solvate, hydrate, tautomer, ester, *N*-oxide or stereoisomer thereof, wherein the compound of Formula (I) or Formula (II), or a pharmaceutically acceptable salt, solvate, hydrate, tautomer, ester, *N*-oxide or stereoisomer thereof modulates the expression of eIF2B, eIF2α, a component of the eIF2 pathway, component of the ISR pathway or any combination thereof by the patient's cells, thereby treating the condition, disease or disorder. In some embodiments, the condition, disease or disorder is characterized by aberrant expression of eIF2B, eIF2α, a component of the eIF2 pathway, component of the ISR pathway or any combination thereof by the patient's cells. In some embodiments, the compound of Formula (I) or Formula (II), or a pharmaceutically acceptable salt, solvate, hydrate, tautomer, ester, *N*-oxide or stereoisomer thereof increases the expression of eIF2B, eIF2α, a component of the eIF2 pathway, component of the ISR pathway or any combination thereof by the patient's cells, thereby treating the condition, disease or disorder. In some embodiments, the compound of Formula (I) or Formula (II), or a pharmaceutically acceptable salt, solvate, hydrate, tautomer, ester, *N*-oxide or stereoisomer thereof decreases the expression of eIF2B, eIF2α, a component of the eIF2 pathway, component of the ISR pathway or any combination thereof by the patient's cells, thereby treating the condition, disease or disorder.

In another aspect, disclosed herein is a method of modulating the activity of eIF2B, eIF2α, a component of the eIF2 pathway, component of the ISR pathway or any combination thereof in a cell, the method comprising contacting the cell with an effective amount of a compound of Formula (I) or Formula (II), or a pharmaceutically acceptable salt, solvate, hydrate, tautomer, ester, *N*-oxide or stereoisomer thereof, thereby modulating the activity of eIF2B, eIF2α, a component of the eIF2 pathway, component of the ISR pathway or any combination thereof in the cell. In some embodiments, contacting the compound of Formula (I) or Formula (II), or a pharmaceutically acceptable salt, solvate, hydrate, tautomer, ester, *N*-oxide or stereoisomer thereof with the cell increases the activity of eIF2B, eIF2α, a component of the eIF2 pathway, component of the ISR pathway or any combination thereof in the cell. In some embodiments, contacting the compound of Formula (I) or Formula (II), or a pharmaceutically acceptable salt, solvate, hydrate, tautomer, ester, *N*-oxide or stereoisomer thereof with the cell decreases the activity of eIF2B, eIF2α, a component of the eIF2 pathway, component of the ISR pathway or any combination thereof in the cell.

In another aspect, disclosed herein is a method of preventing or treating a condition, disease or disorder described herein in a patient in need thereof, the method comprising administering to the patient an effective amount of a compound of Formula (I) or Formula (II), or a pharmaceutically acceptable salt, solvate, hydrate, tautomer, ester, *N*-oxide or stereoisomer thereof, wherein the compound of Formula (I) or Formula (II), or a pharmaceutically acceptable salt, solvate, hydrate, tautomer, ester, *N*-oxide or stereoisomer thereof modulates the activity of eIF2B, eIF2α, a component of the eIF2 pathway, component of the ISR pathway or any combination thereof by the patients cells, thereby treating the condition, disease or disorder. In some embodiments, the condition, disease or disorder is characterized by aberrant activity of eIF2B, eIF2α, a component of the eIF2 pathway, component of the ISR pathway or any combination thereof in the patient's cells. In some embodiments, the compound of Formula (I) or Formula (II), or a pharmaceutically acceptable salt, solvate, hydrate, tautomer, ester, N-oxide or stereoisomer thereof increases the activity of eIF2B, eIF2α, a component of the eIF2 pathway, component of the ISR pathway or any combination thereof in the patient's cells, thereby treating the condition, disease or disorder. In some embodiments, the compound of Formula (I) or Formula (II), or a pharmaceutically acceptable salt, solvate, hydrate, tautomer, ester, N-oxide or stereoisomer thereof decreases the activity of eIF2B, eIF2α, a component of the eIF2 pathway, component of the ISR pathway or any combination thereof in the patient's cells, thereby treating the condition, disease or disorder.

In some embodiments, administering an effective amount of a compound of Formula (I) or Formula (II), or a pharmaceutically acceptable salt, solvate, hydrate, tautomer, ester, *N*-oxide or stereoisomer thereof, wherein the compound of Formula (I) or Formula (II), or a pharmaceutically acceptable salt, solvate, hydrate, tautomer, ester, *N*-oxide or stereoisomer thereof modulates both the expression and the activity of eIF2B, eIF2α, a component of the eIF2 pathway, component of the ISR pathway or any combination thereof in the patients cells, thereby treating the condition, disease or disorder.

In some embodiments, the compound of Formula (I) or Formula (II) is chemically modified, prior to (ex vivo) or after (in vivo) contacting with a cell, forming a biologically active compound that modulates the expression and/or activity of eIF2B, eIF2α, a component of the eIF2 pathway, component of the ISR pathway or any combination thereof in the cell. In some embodiments, the compound of Formula (I) or Formula (II) is metabolized by the patient forming a biologically active compound that modulates the expression and/or activity of eIF2B, eIF2α, a component of the eIF2 pathway, component of the ISR pathway or any combination thereof in the patients cells, thereby treating a condition, disease or disorder disclosed herein. In some embodiments, the biologically active compound is the compound of formula (II).

In one aspect, disclosed herein is a method of treating a disease related to a modulation of eIF2B activity or levels, eIF2α activity or levels, or the activity or levels of a component of the eIF2 pathway or the ISR pathway in a patient in need thereof, comprising administering to the patient an effective amount of a compound of Formula (I) or Formula (II). In some embodiments, the modulation comprises an increase in eIF2B activity or levels, increase in eIF2α activity or levels, or increase in activity or levels of a component of the eIF2 pathway or the ISR pathway. In some embodiments, the disease may be caused by a mutation to a gene or protein sequence related to a member of the eIF2 pathway (e.g., the eIF2α signaling pathway).

### Methods of Increasing Protein Activity and Production

In another aspect, the compound of Formula (I) or Formula (II), or a pharmaceutically acceptable salt, solvate, hydrate, tautomer, ester, N-oxide or stereoisomer thereof may be useful in applications where increasing production output of eIF2B, eIF2α, a component of the eIF2 pathway, a component of the ISR pathway or any combination thereof is desirable, such as in vitro cell free systems for protein production.

In some embodiments, the present description features a method of increasing expression of eIF2B, eIF2α, a component of the eIF2 pathway, a component of the ISR pathway or any combination thereof by a cell or in vitro expression system, the method comprising contacting the cell or in vitro expression system with an effective amount of a compound of Formula (I) or Formula (II), or a pharmaceutically acceptable salt, solvate, hydrate, tautomer, ester, *N*-oxide or stereoisomer thereof. In some embodiments, the method is a method of increasing the expression of eIF2B, eIF2α, a component of the eIF2 pathway, a component of the ISR pathway or any combination thereof by a cell comprising contacting the cell with an effective amount of a compound described herein (e.g., the compound of Formula (I) or Formula (II), or a pharmaceutically acceptable salt, solvate, hydrate, tautomer, ester, *N*-oxide or stereoisomer thereof). In other embodiments, the method is a method of increasing the expression of eIF2B, eIF2α, a component of the eIF2 pathway, a component of the ISR pathway or any combination thereof by an in vitro protein expression system comprising contacting the in vitro expression system with a compound described herein (e.g. the compound of Formula (I) or Formula (II), or a pharmaceutically acceptable salt, solvate, hydrate, tautomer, ester, *N*-oxide or stereoisomer thereof). In some embodiments, contacting the cell or in vitro expression system with an effective amount of a compound of Formula (I) or Formula (II), or a pharmaceutically acceptable salt, solvate, hydrate, tautomer, ester, *N*-oxide or stereoisomer thereof increases expression of eIF2B, eIF2α, a component of the eIF2 pathway, a component of the ISR pathway or any combination thereof in the cell or in vitro expression system by about 1%, about 2%, about 3%, about 4%, about 5%, about 6%, about 7%, about 8%, about 9%, about 10%, about 15%, about 20%, about 25%, about 30%, about 40%, about 45%, about 50%, about 60%, about 65%, about 70%, about 75%, about 80%, about 85%, about 90%, about 95%, or about 100%. In some embodiments, contacting the cell or in vitro expression system with an effective amount of a compound of Formula (I) or Formula (II), or a pharmaceutically acceptable salt, solvate, hydrate, tautomer, ester, *N-*oxide or stereoisomer thereof increases expression of eIF2B, eIF2α, a component of the eIF2 pathway, a component of the ISR pathway or any combination thereof in the cell or in vitro expression system by about 1-fold, about 2-fold, about 3-fold, about 4-fold, about 5-fold, about 6-fold, about 7-fold, about 8-fold, about 9-fold, about 10-fold, about 20-fold, about 30-fold, about 40-fold, about 50-fold, about 60-fold, about 70-fold, about 80-fold, about 90-fold, about 100-fold, about 200-fold, about 300-fold, about 400-fold, about 500-fold, about 600-fold about 700-fold, about 800-fold, about 900-fold, about 1000-fold, about 10000-fold, about 100000-fold, or about 1000000-fold.

In some embodiments, the present description features a method of increasing the expression of eIF2B, eIF2α, a component of the eIF2 pathway, a component of the ISR pathway or any combination thereof by a patient cells, the method comprising administering to the patient an effective amount of a compound of Formula (I) or Formula (II), or a pharmaceutically acceptable salt, solvate, hydrate, tautomer, ester, *N*-oxide or stereoisomer thereof, wherein the patient has been diagnosed with a disease, disorder, or condition disclosed herein and wherein the disease, disorder or condition is characterized by aberrant expression of eIF2B, eIF2α, a component of the eIF2 pathway, a component of the ISR pathway or any combination thereof (e.g., a leukodystrophy, a leukoencephalopathy, a hypomyelinating or demyelinating disease, muscle-wasting disease, or sarcopenia). In some embodiments, administering to the patient in need thereof an effective amount of a compound of Formula (I) or Formula (II), or a pharmaceutically acceptable salt, solvate, hydrate, tautomer, ester, *N*-oxide or stereoisomer thereof increases the expression of eIF2B, eIF2α, a component of the eIF2 pathway, a component of the ISR pathway or any combination thereof by the patients cells about 1%, about 2%, about 3%, about 4%, about 5%, about 6%, about 7%, about 8%, about 9%, about 10%, about 15%, about 20%, about 25%, about 30%, about 40%, about 45%, about 50%, about 60%, about 65%, about 70%, about 75%, about 80%, about 85%, about 90%, about 95%, or about 100%, thereby treating the disease, disorder or condition. In some embodiments, administering to the patient in need thereof an effective amount of a compound of Formula (I) or Formula (II), or a pharmaceutically acceptable salt, solvate, hydrate, tautomer, ester, *N*-oxide or stereoisomer thereof increases expression of eIF2B, eIF2α, a component of the eIF2 pathway, a component of the ISR pathway or any combination thereof by the patients cells about 1-fold, about 2-fold, about 3-fold, about 4-fold, about 5-fold, about 6-fold, about 7-fold, about 8-fold, about 9-fold, about 10-fold, about 20-fold, about 30-fold, about 40-fold, about 50-fold, about 60-fold, about 70-fold, about 80-fold, about 90-fold, about 100-fold, about 200-fold, about 300-fold, about 400-fold, about 500-fold, about 600-fold about 700-fold, about 800-fold, about 900-fold, about 1000-fold, about 10000-fold, about 100000-fold, or about 1000000-fold, thereby treating the disease, disorder or condition.

In another aspect, the compound of Formula (I) or Formula (II), or a pharmaceutically acceptable salt, solvate, hydrate, tautomer, ester, *N*-oxide or stereoisomer thereof may be useful in applications where increasing the activity of eIF2B, eIF2α, a component of the eIF2 pathway, a component of the ISR pathway or any combination thereof is desirable.

In some embodiments, the present description features a method of increasing the activity of eIF2B, eIF2α, a component of the eIF2 pathway, a component of the ISR pathway or any combination thereof in a cell, the method comprising contacting the cell with an effective amount of a compound of Formula (I) or Formula (II), or a pharmaceutically acceptable salt, solvate, hydrate, tautomer, ester, *N*-oxide or stereoisomer thereof. In some embodiments, contacting the cell with an effective amount of a compound of Formula (I) or Formula (II), or a pharmaceutically acceptable salt, solvate, hydrate, tautomer, ester, *N*-oxide or stereoisomer thereof increases the activity of eIF2B, eIF2α, a component of the eIF2 pathway, a component of the ISR pathway or any combination thereof in the cell by about 1%, about 2%, about 3%, about 4%, about 5%, about 6%, about 7%, about 8%, about 9%, about 10%, about 15%, about 20%, about 25%, about 30%, about 40%, about 45%, about 50%, about 60%, about 65%, about 70%, about 75%, about 80%, about 85%, about 90%, about 95%, or about 100%. In some embodiments, contacting the cell with an effective amount of a compound of Formula (I) or Formula (II), or a pharmaceutically acceptable salt, solvate, hydrate, tautomer, ester, *N*-oxide or stereoisomer thereof increases the activity of eIF2B, eIF2α, a component of the eIF2 pathway, a component of the ISR pathway or any combination thereof in the cell by about 1-fold, about 2-fold, about 3-fold, about 4-fold, about 5-fold, about 6-fold, about 7-fold, about 8-fold, about 9-fold, about 10-fold, about 20-fold, about 30-fold, about 40-fold, about 50-fold, about 60-fold, about 70-fold, about 80-fold, about 90-fold, about 100-fold, about 200-fold, about 300-fold, about 400-fold, about 500-fold, about 600-fold about 700-fold, about 800-fold, about 900-fold, about 1000-fold, about 10000-fold, about 100000-fold, or about 1000000-fold.

In some embodiments, the present description features a method of increasing the activity of eIF2B, eIF2α, a component of the eIF2 pathway, a component of the ISR pathway or any combination thereof in a patient in need thereof, the method comprising administering to the patient an effective amount of a compound of Formula (I) or Formula (II), or a pharmaceutically acceptable salt, solvate, hydrate, tautomer, ester, *N*-oxide or stereoisomer thereof, wherein the patient has been diagnosed with a disease, disorder, or condition disclosed herein and wherein the disease, disorder or condition is characterized by lowered levels of protein activity. In some embodiments, administering to the patient in need thereof an effective amount of a compound of Formula (I) or Formula (II), or a pharmaceutically acceptable salt, solvate, hydrate, tautomer, ester, *N*-oxide or stereoisomer thereof increases the activity of eIF2B, eIF2α, a component of the eIF2 pathway, a component of the ISR pathway or any combination thereof in the patient by about 1%, about 2%, about 3%, about 4%, about 5%, about 6%, about 7%, about 8%, about 9%, about 10%, about 15%, about 20%, about 25%, about 30%, about 40%, about 45%, about 50%, about 60%, about 65%, about 70%, about 75%, about 80%, about 85%, about 90%, about 95%, or about 100%, thereby treating the disease, disorder or condition. In some embodiments, administering to the patient in need thereof an effective amount of a compound of Formula (I) or Formula (II), or a pharmaceutically acceptable salt, solvate, hydrate, tautomer, ester, *N*-oxide or stereoisomer thereof increases the activity of eIF2B, eIF2α, a component of the eIF2 pathway, a component of the ISR pathway or any combination thereof in the patient by about 1-fold, about 2-fold, about 3-fold, about 4-fold, about 5-fold, about 6-fold, about 7-fold, about 8-fold, about 9-fold, about 10-fold, about 20-fold, about 30-fold, about 40-fold, about 50-fold, about 60-fold, about 70-fold, about 80-fold, about 90-fold, about 100-fold, about 200-fold, about 300-fold, about 400-fold, about 500-fold, about 600-fold about 700-fold, about 800-fold, about 900-fold, about 1000-fold, about 10000-fold, about 100000-fold, or about 1000000-fold, thereby treating the disease, disorder or condition.

In some embodiments, the compound of Formula (I) or Formula (II) is chemically modified, prior to (ex vivo) or after (in vivo) contacting with the cell or in vitro expression system, forming a biologically active compound that increases the expression and/or activity of eIF2B, eIF2α, a component of the eIF2 pathway, component of the ISR pathway or any combination thereof in the cells and/or in vitro expression system. In some embodiments, the compound of Formula (I) or Formula (II) is metabolized by the patient forming a biologically active compound that increases the expression and/or activity of eIF2B, eIF2α, a component of the eIF2 pathway, component of the ISR pathway or any combination thereof in the patients cells, thereby treating a condition, disease or disorder disclosed herein. In some embodiments, the biologically active compound is the compound of formula (II).

### Methods of Decreasing Protein Activity and Production

In another aspect, the compound of Formula (I) or Formula (II), or a pharmaceutically acceptable salt, solvate, hydrate, tautomer, ester, *N*-oxide or stereoisomer thereof may be useful in applications where decreasing production output of eIF2B, eIF2α, a component of the eIF2 pathway, a component of the ISR pathway or any combination thereof is desirable.

In some embodiments, the present description features a method of decreasing expression of eIF2B, eIF2α, a component of the eIF2 pathway, a component of the ISR pathway or any combination thereof in a cell, the method comprising contacting the cells with an effective amount of a compound of Formula (I) or Formula (II), or a pharmaceutically acceptable salt, solvate, hydrate, tautomer, ester, *N*-oxide or stereoisomer thereof. In some embodiments, contacting the cells with an effective amount of a compound of Formula (I) or Formula (II), or a pharmaceutically acceptable salt, solvate, hydrate, tautomer, ester, *N-*oxide or stereoisomer thereof decreases expression of eIF2B, eIF2α, a component of the eIF2 pathway, a component of the ISR pathway or any combination thereof in the cell by about 1%, about 2%, about 3%, about 4%, about 5%, about 6%, about 7%, about 8%, about 9%, about 10%, about 15%, about 20%, about 25%, about 30%, about 40%, about 45%, about 50%, about 60%, about 65%, about 70%, about 75%, about 80%, about 85%, about 90%, about 95%, or about 100%.

In some embodiments, the present description features a method of decreasing the expression of eIF2B, eIF2α, a component of the eIF2 pathway, a component of the ISR pathway or any combination thereof in a patient in need thereof, the method comprising administering to the patient an effective amount of a compound of Formula (I) or Formula (II), or a pharmaceutically acceptable salt, solvate, hydrate, tautomer, ester, N-oxide or stereoisomer thereof, wherein the patient has been diagnosed with a disease, disorder, or condition described herein and wherein the disease, disorder or condition is characterized by increased levels of protein production. In some embodiments, administering to the patient in need thereof an effective amount of a compound of Formula (I) or Formula (II), or a pharmaceutically acceptable salt, solvate, hydrate, tautomer, ester, N-oxide or stereoisomer thereof decreases the expression of eIF2B, eIF2α, a component of the eIF2 pathway, a component of the ISR pathway or any combination thereof in the patient by about 1%, about 2%, about 3%, about 4%, about 5%, about 6%, about 7%, about 8%, about 9%, about 10%, about 15%, about 20%, about 25%, about 30%, about 40%, about 45%, about 50%, about 60%, about 65%, about 70%, about 75%, about 80%, about 85%, about 90%, about 95%, or about 100%, thereby treating the disease, disorder or condition.

In another aspect, the compound of Formula (I) or Formula (II), or a pharmaceutically acceptable salt, solvate, hydrate, tautomer, ester, *N*-oxide or stereoisomer thereof may be useful in applications where decreasing the activity of eIF2B, eIF2α, a component of the eIF2 pathway, a component of the ISR pathway or any combination thereof is desirable.

In some embodiments, the present description features a method of decreasing the activity of eIF2B, eIF2α, a component of the eIF2 pathway, a component of the ISR pathway or any combination thereof in a cell, the method comprising contacting the cell with an effective amount of a compound of Formula (I) or Formula (II), or a pharmaceutically acceptable salt, solvate, hydrate, tautomer, ester, *N*-oxide or stereoisomer thereof. In some embodiments, contacting the cell with an effective amount of a compound of Formula (I) or Formula (II), or a pharmaceutically acceptable salt, solvate, hydrate, tautomer, ester, *N*-oxide or stereoisomer thereof decreases the activity of eIF2B, eIF2α, a component of the eIF2 pathway, a component of the ISR pathway or any combination thereof in the cell by about 1%, about 2%, about 3%, about 4%, about 5%, about 6%, about 7%, about 8%, about 9%, about 10%, about 15%, about 20%, about 25%, about 30%, about 40%, about 45%, about 50%, about 60%, about 65%, about 70%, about 75%, about 80%, about 85%, about 90%, about 95%, or about 100%, thereby treating the disease, disorder or condition.

In some embodiments, the present invention features a method of decreasing the activity of eIF2B, eIF2α, a component of the eIF2 pathway, a component of the ISR pathway or any combination thereof in a patient in need thereof, the method comprising administering to the patient an effective amount of a compound of Formula (I) or Formula (II), or a pharmaceutically acceptable salt, solvate, hydrate, tautomer, ester*, N-*oxide or stereoisomer thereof, wherein the patient has been diagnosed with a disease, disorder, or condition described herein and wherein the disease, disorder or condition is characterized by increased levels of protein activity. In some embodiments, administering to the patient in need thereof an effective amount of a compound of Formula (I) or Formula (II), or a pharmaceutically acceptable salt, solvate, hydrate, tautomer, ester, *N*-oxide or stereoisomer thereof decreases the activity of eIF2B, eIF2α, a component of the eIF2 pathway, a component of the ISR pathway or any combination thereof in the patient by about 1%, about 2%, about 3%, about 4%, about 5%, about 6%, about 7%, about 8%, about 9%, about 10%, about 15%, about 20%, about 25%, about 30%, about 40%, about 45%, about 50%, about 60%, about 65%, about 70%, about 75%, about 80%, about 85%, about 90%, about 95%, or about 100%, thereby treating the disease, disorder or condition.

In some embodiments, the compound of Formula (I) or Formula (II) is chemically modified, prior to (ex vivo) or after (in vivo) contacting with a cell, forming a biologically active compound that decreases the expression and/or activity of eIF2B, eIF2α, a component of the eIF2 pathway, component of the ISR pathway or any combination thereof in the cell. In some embodiments, the compound of Formula (I) or Formula (II) is metabolized by the patient forming a biologically active compound that decreases the expression and/or activity of eIF2B, eIF2α, a component of the eIF2 pathway, component of the ISR pathway or any combination thereof in the patients cells, thereby treating a condition, disease or disorder disclosed herein. In some embodiments, the biologically active compound is the compound of Formula (I) or Formula (II).

In some embodiments, the compounds set forth herein are provided as pharmaceutical compositions including a compound of Formula (I) or Formula (II) or a pharmaceutically acceptable salt, solvate, hydrate, tautomer, ester, N-oxide or stereoisomer thereof and a pharmaceutically acceptable excipient. In embodiments of the method, a compound of Formula (I) or Formula (II) or a pharmaceutically acceptable salt, solvate, hydrate, tautomer, ester, N-oxide or stereoisomer thereof, is co-administered with a second agent (e.g. therapeutic agent). In other embodiments of the method, a compound of Formula (I) or Formula (II) or a pharmaceutically acceptable salt, solvate, hydrate, tautomer, ester, N-oxide or stereoisomer thereof, is co-administered with a second agent (e.g. therapeutic agent), which is administered in a therapeutically effective amount. In embodiments, the second agent is an agent for improving memory.

### Combination Therapy

In one aspect, the present description features a pharmaceutical composition comprising a compound of Formula (I) or Formula (II), or a pharmaceutically acceptable salt, solvate, hydrate, tautomer, ester, N-oxide or stereoisomer thereof as well as a second agent (e.g. a second therapeutic agent). In some embodiments, the pharmaceutical composition includes a second agent (e.g. a second therapeutic agent) in a therapeutically effective amount. In some embodiments, the second agent is an agent for treating cancer, a neurodegenerative disease, a leukodystrophy, an inflammatory disease, a musculoskeletal disease, a metabolic disease, or a disease or disorder associated with impaired function of eIF2B, eIF2α, or a component of the eIF2 pathway or ISR pathway.

The compounds described herein can be used in combination with one another, with other active agents known to be useful in treating cancer, a neurodegenerative disease, an inflammatory disease, a musculoskeletal disease, a metabolic disease, or a disease or disorder associated with impaired function of eIF2B, eIF2α, or a component of the eIF2 pathway or ISR pathway or with adjunctive agents that may not be effective alone, but may contribute to the efficacy of the active agent.

In some embodiments, co-administration includes administering one active agent within 0.5, 1, 2, 4, 6, 8, 10, 12, 16, 20, or 24 hours of a second active agent. Co-administration includes administering two active agents simultaneously, approximately simultaneously (e.g., within about 1, 5, 10, 15, 20, or 30 minutes of each other), or sequentially in any order. In some embodiments, co-administration can be accomplished by co-formulation, i.e., preparing a single pharmaceutical composition including both active agents. In other embodiments, the active agents can be formulated separately. In another embodiment, the active and/or adjunctive agents may be linked or conjugated to one another. In some embodiments, the compounds described herein may be combined with treatments for a cancer, a neurodegenerative disease, a leukodystrophy, an inflammatory disease, a musculoskeletal disease, a metabolic disease, or a disease or disorder associated with impaired function of eIF2B, eIF2α, or a component of the eIF2 pathway or ISR pathway.

In embodiments, the second agent is an anti-cancer agent. In embodiments, the second agent is a chemotherapeutic. In embodiments, the second agent is an agent for improving memory. In embodiments, the second agent is an agent for treating a neurodegenerative disease. In embodiments, the second agent is an agent for treating a leukodystrophy. In embodiments, the second agent is an agent for treating vanishing white matter disease. In embodiments, the second agent is an agent for treating childhood ataxia with CNS hypo-myelination. In embodiments, the second agent is an agent for treating an intellectual disability syndrome. In embodiments, the second agent is an agent for treating pancreatic cancer. In embodiments, the second agent is an agent for treating breast cancer. In embodiments, the second agent is an agent for treating multiple myeloma. In embodiments, the second agent is an agent for treating myeloma. In embodiments, the second agent is an agent for treating a cancer of a secretory cell. In embodiments, the second agent is an agent for reducing eIF2α phosphorylation. In embodiments, the second agent is an agent for inhibiting a pathway activated by eIF2α phosphorylation. In embodiments, the second agent is an agent for inhibiting a pathway activated by eIF2α. In embodiments, the second agent is an agent for inhibiting the integrated stress response. In embodiments, the second agent is an anti-inflammatory agent. In embodiments, the second agent is an agent for treating postsurgical cognitive dysfunction. In embodiments, the second agent is an agent for treating traumatic brain injury. In embodiments, the second agent is an agent for treating a musculoskeletal disease. In embodiments, the second agent is an agent for treating a metabolic disease. In embodiments, the second agent is an antidiabetic agent.

### Anti-cancer agents

"Anti-cancer agent" is used in accordance with its plain ordinary meaning and refers to a composition (e.g. compound, drug, antagonist, inhibitor, modulator) having antineoplastic properties or the ability to inhibit the growth or proliferation of cells. In some embodiments, an anti-cancer agent is a chemotherapeutic. In some embodiments, an anti-cancer agent is an agent identified herein having utility in methods of treating cancer. In some embodiments, an anticancer agent is an agent approved by the FDA or similar regulatory agency of a country other than the USA, for treating cancer. Examples of anti-cancer agents include, but are not limited to, MEK (e.g. MEK1, MEK2, or MEK1 and MEK2) inhibitors (e.g. XL518, CI- 1040, PD035901, selumetinib/ AZD6244, GSK1120212/ trametinib, GDC-0973, ARRY-162, ARRY-300, AZD8330, PD0325901, U0126, PD98059, TAK-733, PD318088, AS703026, BAY 869766), alkylating agents (e.g., cyclophosphamide, ifosfamide, chlorambucil, busulfan, melphalan, mechlorethamine, uramustine, thiotepa, nitrosoureas, nitrogen mustards (e.g., mechloroethamine, cyclophosphamide, chlorambucil, meiphalan), ethylenimine and methylmelamines (e.g., hexamethlymelamine, thiotepa), alkyl sulfonates (e.g., busulfan), nitrosoureas (e.g., carmustine, lomusitne, semustine, streptozocin), triazenes (decarbazine), anti-metabolites (e.g., 5-azathioprine, leucovorin, capecitabine, fludarabine, gemcitabine, pemetrexed, raltitrexed, folic acid analog (e.g., methotrexate), or pyrimidine analogs (e.g., fluorouracil, floxouridine, Cytarabine), purine analogs (e.g., mercaptopurine, thioguanine, pentostatin), etc.), plant alkaloids (e.g., vincristine, vinblastine, vinorelbine, vindesine, podophyllotoxin, paclitaxel, docetaxel, etc.), topoisomerase inhibitors (e.g., irinotecan, topotecan, amsacrine, etoposide (VP 16), etoposide phosphate, teniposide, etc.), antitumor antibiotics (e.g., doxorubicin, adriamycin, daunorubicin, epirubicin, actinomycin, bleomycin, mitomycin, mitoxantrone, plicamycin, etc.), platinum-based compounds (e.g. cisplatin, oxaloplatin, carboplatin), anthracenedione (e.g., mitoxantrone), substituted urea (e.g., hydroxyurea), methyl hydrazine derivative (e.g., procarbazine), adrenocortical suppressant (e.g., mitotane, aminoglutethimide), epipodophyllotoxins (e.g., etoposide), antibiotics (e.g., daunorubicin, doxorubicin, bleomycin), enzymes (e.g., L-asparaginase), inhibitors of mitogen-activated protein kinase signaling (e.g. U0126, PD98059, PD184352, PD0325901, ARRY-142886, SB239063, SP600125, BAY 43-9006, wortmannin, or LY294002, Syk inhibitors, mTOR inhibitors, antibodies (e.g., rituxan), gossyphol, genasense, polyphenol E, Chlorofusin, all trans-retinoic acid (ATRA), bryostatin, tumor necrosis factor-related apoptosis-inducing ligand (TRAIL), 5-aza-2'-deoxycytidine, all trans retinoic acid, doxorubicin, vincristine, etoposide, gemcitabine, imatinib (Gleevec.RTM.), geldanamycin, 17-N-Allylamino-17-Demethoxygeldanamycin (17-AAG), flavopiridol, LY294002, bortezomib, trastuzumab, BAY 1 1-7082, PKC412, PD184352, 20-epi-1, 25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein- 1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1 ; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorins; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; 9-dioxamycin; diphenyl spiromustine; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; 06-benzylguanine; octreotide; okicenone; oligonucleotides; onapristone; ondansetron; ondansetron; oracin; oral cytokine inducer; ormaplatin; osaterone; oxaliplatin; oxaunomycin; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylerie conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone Bl; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen-binding protein; sizofuran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; vitaxin; vorozole; zanoterone; zeniplatin; zilascorb; zinostatin stimalamer, Adriamycin, Dactinomycin, Bleomycin, Vinblastine, Cisplatin, acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cladribine; crisnatol mesylate; cyclophosphamide; cytarabine; dacarbazine; daunorubicin hydrochloride; decitabine; dexormaplatin; dezaguanine; dezaguanine mesylate; diaziquone; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; fluorocitabine; fosquidone; fostriecin sodium; gemcitabine; gemcitabine hydrochloride; hydroxyurea; idarubicin hydrochloride; ifosfamide; iimofosine; interleukin II (including recombinant interleukin II, or rlL.sub.2), interferon alfa-2a; interferon alfa-2b; interferon alfa-nl; interferon alfa-n3; interferon beta-la; interferon gamma-lb; iprop latin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazoie; nogalamycin; ormaplatin; oxisuran; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride, agents that arrest cells in the G2-M phases and/or modulate the formation or stability of microtubules, (e.g. Taxol (i.e. paclitaxel), Taxotere, compounds comprising the taxane skeleton, Erbulozole (i.e. R-55104), Dolastatin 10 (i.e. DLS-10 and NSC-376128), Mivobulin isethionate (i.e. as CI-980), Vincristine, NSC-639829, Discodermolide (i.e. as NVP-XX-A-296), ABT-751 (Abbott, i.e. E-7010), Altorhyrtins (e.g. Altorhyrtin A and Altorhyrtin C), Spongistatins (e.g. Spongistatin 1, Spongistatin 2, Spongistatin 3, Spongistatin 4, Spongistatin 5, Spongistatin 6, Spongistatin 7, Spongistatin 8, and Spongistatin 9), Cemadotin hydrochloride (i.e. LU-103793 and SC-D-669356), Epothilones (e.g. Epothilone A, Epothilone B, Epothilone C (i.e. desoxyepothilone A or dEpoA), Epothilone D (i.e. KOS-862, dEpoB, and desoxyepothilone B), Epothilone E, Epothilone F, Epothilone B N-oxide, Epothilone A N-oxide, 16-aza-epothilone B, 21 -aminoepothilone B (i.e. BMS-310705), 21-hydroxyepothilone D (i.e. Desoxyepothilone F and dEpoF), 26-fluoroepothilone, Auristatin PE (i.e. NSC-654663), Soblidotin (i.e. TZT-1027), LS-4559-P (Pharmacia, i.e. LS-4577), LS-4578 (Pharmacia, i.e. LS-477-P), LS-4477 (Pharmacia), LS-4559 (Pharmacia), RPR-1 12378 (Aventis), Vincristine sulfate, DZ-3358 (Daiichi), FR-182877 (Fujisawa, i.e. WS-9885B), GS-164 (Takeda), GS-198 (Takeda), KAR-2 (Hungarian Academy of Sciences), BSF-223651 (BASF, i.e. ILX-651 and LU-223651), SAH-49960 (Lilly/Novartis), SDZ-268970 (Lilly/Novartis), AM-97 (Armad/Kyowa Hakko), AM- 132 (Armad), AM- 138 (Armad/Kyowa Hakko), IDN-5005 (Indena), Cryptophycin 52 (i.e. LY-355703), AC-7739 (Ajinomoto, i.e. AVE-8063A and CS-39.HC1), AC-7700 (Ajinomoto, i.e. AVE-8062, AVE-8062A, CS-39-L-Ser.HCl, and RPR-258062A), Vitilevuamide, Tubulysin A, Canadensol, Centaureidin (i.e. NSC-106969), T-138067 (Tularik, i.e. T-67, TL-138067 and TI- 138067), COBRA-1 (Parker Hughes Institute, i.e. DDE-261 and WHI-261), H10 (Kansas State University), H16 (Kansas State University), Oncocidin A 1 (i.e. BTO-956 and DIME), DDE- 313 (Parker Hughes Institute), Fijianolide B, Laulimalide, SPA-2 (Parker Hughes Institute), SPA-1 (Parker Hughes Institute, i.e. SPIKET-P), 3-IAABU (Cytoskeleton/Mt. Sinai School of Medicine, i.e. MF-569), Narcosine (also known as NSC-5366), Nascapine, D-24851 (Asta Medica), A-105972 (Abbott), Hemiasterlin, 3-BAABU (Cytoskeleton/Mt. Sinai School of Medicine, i.e. MF-191), TMPN (Arizona State University), Vanadocene acetylacetonate, T- 138026 (Tularik), Monsatrol, Inanocine (i.e. NSC-698666), 3-IAABE (Cytoskeleton/Mt. Sinai School of Medicine), A-204197 (Abbott), T-607 (Tularik, i.e. T-900607), RPR-115781 (Aventis), Eleutherobins (such as Desmethyleleutherobin, Desaetyleleutherobin, lsoeleutherobin A, and Z-Eleutherobin), Caribaeoside, Caribaeolin, Halichondrin B, D-64131 (Asta Medica), D-68144 (Asta Medica), Diazonamide A, A-293620 (Abbott), NPI-2350 (Nereus), Taccalonolide A. TUB-245 (Aventis), A-259754 (Abbott), Diozostatin, (-)-Phenylahistin (i.e. NSCL-96F037), D-68838 (Asta Medica), D-68836 (Asta Medica), Myoseverin B, D-43411 (Zentaris, i.e. D-81862), A-289099 (Abbott), A-318315 (Abbott), HTI-286 (i.e. SPA-110, trifluoroacetate salt) (Wyeth), D-82317 (Zentaris), D-82318 (Zentaris), SC-12983 (NCI), Resverastatin phosphate sodium, BPR-OY-007 (National Health Research Institutes), and SSR-25041 1 (Sanofi), steroids (e.g., dexamethasone), finasteride, aromatase inhibitors, gonadotropin-releasing hormone agonists (GnRH) such as goserelin or leuprolide, adrenocorticosteroids (e.g., prednisone), progestins (e.g., hydroxyprogesterone caproate, megestrol acetate, medroxyprogesterone acetate), estrogens (e.g., diethlystilbestrol, ethinyl estradiol), antiestrogen (e.g., tamoxifen), androgens (e.g., testosterone propionate, fluoxymesterone), antiandrogen (e.g., flutamide), immunostimulants (e.g., Bacillus Calmette-Guerin (BCG), levamisole, interleukin-2, alpha-interferon, etc.), monoclonal antibodies (e.g., anti-CD20, anti-HER2, anti-CD52, anti-HLA-DR, and anti-VEGF monoclonal antibodies), immunotoxins (e.g., anti-CD33 monoclonal antibody-calicheamicin conjugate, anti-CD22 monoclonal antibody-pseudomonas exotoxin conjugate, etc.), radioimmunotherapy (e.g., anti-CD20 monoclonal antibody conjugated to ^{U 1}In, ⁹⁰Y, or ¹³¹I, etc. ), triptolide, homoharringtonine, dactinomycin, doxorubicin, epirubicin, topotecan, itraconazole, vindesine, cerivastatin, vincristine, deoxyadenosine, sertraline, pitavastatin, irinotecan, clofazimine, 5-nonyloxytryptamine, vemurafenib, dabrafenib, erlotinib, gefitinib, EGFR inhibitors, epidermal growth factor receptor (EGFR)-targeted therapy or therapeutic (e.g. gefitinib (Iressa^{™}), erlotinib (Tarceva^{™}), cetuximab (Erbitux^{™}), lapatinib (Tykerb^{™}), panitumumab (Vectibix^{™}), vandetanib (Caprelsa^{™}), afatinib/BIBW2992, CI-1033/canertinib, neratinib/HKI-272, CP-724714, TAK-285, AST-1306, ARRY334543, ARRY-380, AG-1478, dacomitinib/PF299804, OSI-420/desmethyl erlotinib, AZD8931, AEE788, pelitinib/EKB-569, CUDC-101, WZ8040, WZ4002, WZ3146, AG-490, XL647, PD153035, BMS-599626), sorafenib, imatinib, sunitinib, dasatinib, or the like.

"Chemotherapeutic" or "chemotherapeutic agent" is used in accordance with its plain ordinary meaning and refers to a chemical composition or compound having antineoplastic properties or the ability to inhibit the growth or proliferation of cells.

Additionally, the compounds described herein can be co-administered with conventional immunotherapeutic agents including, but not limited to, immunostimulants (e.g., Bacillus Calmette-Guerin (BCG), levamisole, interleukin-2, alpha- interferon, etc.), monoclonal antibodies (e.g., anti-CD20, anti-HER2, anti-CD52, anti-HLA-DR, and anti-VEGF monoclonal antibodies), immunotoxins (e.g., anti-CD33 monoclonal antibody-calicheamicin conjugate, anti-CD22 monoclonal antibody -pseudomonas exotoxin conjugate, etc.), and radioimmunotherapy (e.g., anti-CD20 monoclonal antibody conjugated to ^{m}In, ⁹⁰Y, or ¹³¹I, etc.).

In a further embodiment, the compounds described herein can be co-administered with conventional radiotherapeutic agents including, but not limited to, radionuclides such as ⁴⁷Sc, ⁶⁴Cu, ⁶⁷Cu, ⁸⁹Sr, ⁸⁶Y, ⁸⁷Y, ⁹⁰Y, ¹⁰⁵Rh, ^{m}Ag, ^{m}In, ^{117m}Sn, ¹⁴⁹Pm, ¹⁵³Sm, ¹⁶⁶Ho, ¹⁷⁷Lu, ¹⁸⁶Re, ¹⁸⁸Re, ²¹¹At, and ²¹²Bi, optionally conjugated to antibodies directed against tumor antigens.

### Additional Agents

In some embodiments, the second agent for use in combination with a compound (e.g., a compound of Formula (I)) or Formula (II), or composition thereof described herein is an agent for use in treating a neurodegenerative disease, a leukodystrophy, an inflammatory disease, a musculoskeletal disease, or a metabolic disease. In some embodiments, a second agent for use in combination with a compound (e.g., a compound of Formula (I) or Formula (II)) or composition thereof described herein is an agent approved by the FDA or similar regulatory agency of a country other than the USA, for treating a disease, disorder, or condition described herein.

In some embodiments, a second agent for use in treating a neurodegenerative disease, a leukodystrophy, an inflammatory disease, a musculoskeletal disease, or a metabolic disease includes, but is not limited to, an anti-psychotic drug, anti-depressive drug, anti-anxiety drug, analgesic, a stimulant, a sedative, a pain reliever, an anti-inflammatory agent, a benzodiazepine, a cholinesterase inhibitor, a non-steroidal anti-inflammatory drug (NSAID), a corticosteroid, a MAO inhibitor, a beta-blocker, a calcium channel blocker, an antacid, or other agent. Exemplary second agents may include donepezil, galantamine, rivastigmine, memantine, levodopa, dopamine, pramipexole, ropinirole, rotigotine, doxapram, oxazepam, quetiapine, selegiline, rasagiline, entacapone, benztropine, trihexyphenidyl, riluzole, diazepam, chlorodiazepoxide, lorazepam, alprazolam, buspirone, gepirone, ispapirone, hydroxyzine, propranolol, hydroxyzine, midazolam, trifluoperazine, methylphenidate, atomoxetine, methylphenidate, pemoline, perphenazine, divalproex, valproic acid, sertraline, fluoxetine, citalopram, escitalopram, paroxetine, fluvoxamine, trazodone, desvenlafaxine, duloxetine, venlafaxine, amitriptyline, amoxapine, clomipramine, desipramine, imipramine, nortriptyline, protriptyline, trimipramine, maprotiline, bupropion, nefazodone, vortioxetine, lithium, clozapine, fluphenazine, haloperidol, paliperidone, loxapine, thiothixene, pimozide, thioridazine, risperidone, aspirin, ibuprofen, naproxen, acetaminophen, azathioprine, methotrexate, mycophenolic acid, leflunomide, dibenzoylmethane, cilostazol, pentoxifylline, duloxetine, a cannabinoid (e.g, nabilone), simethicone, magaldrate, aluminum salts, calcium salts, sodium salts, magnesium salts, alginic acid, acarbose, albiglutide, alogliptin, metformin, insulin, lisinopril, atenolol, atorvastatin, fluvastatin, lovastatin, pitavastatin, simvastatin, rosuvastatin, and the like.

Naturally derived agents or supplements may also be used in conjunction with a compound of Formula (I) or Formula (II), or a composition thereof to treat a neurodegenerative disease, an inflammatory disease, a musculoskeletal disease, or a metabolic disease. Exemplary naturally derived agents or supplements include omega-3 fatty acids, carnitine, citicoline, curcumin, gingko, vitamin E, vitamin B (e.g., vitamin B5, vitamin B6, or vitamin B12), huperzine A, phosphatidylserine, rosemary, caffeine, melatonin, chamomile, St. John's wort, tryptophan, and the like.

### EXAMPLES

In order that the invention described herein may be more fully understood, the following examples are set forth. The synthetic and biological examples described in this application are offered to illustrate the compounds, pharmaceutical compositions, and methods provided herein and are not to be construed in any way as limiting their scope.

### Synthetic Protocols

The compounds provided herein can be prepared from readily available starting materials using modifications to the specific synthesis protocols set forth below that would be well known to those of skill in the art. It will be appreciated that where typical or preferred process conditions (i.e., reaction temperatures, times, mole ratios of reactants, solvents, pressures, etc.) are given, other process conditions can also be used unless otherwise stated. Optimum reaction conditions may vary with the particular reactants or solvents used, but such conditions can be determined by those skilled in the art by routine optimization procedures. General scheme relating to methods of making exemplary compounds of the invention and reference compounds are additionally described in the section entitled Methods of Making Compounds.

Additionally, as will be apparent to those skilled in the art, conventional protecting groups may be necessary to prevent certain functional groups from undergoing undesired reactions. The choice of a suitable protecting group for a particular functional group as well as suitable conditions for protection and deprotection are well known in the art. For example, numerous protecting groups, and their introduction and removal, are described in Greene et al., Protecting Groups in Organic Synthesis, Second Edition, Wiley, New York, 1991, and references cited therein.

### Abbreviations

APCI for atmospheric pressure chemical ionization; DCI for desorption chemical ionization; DMSO for dimethyl sulfoxide; ESI for electrospray ionization; HPLC for high performance liquid chromatography; LC/MS for liquid chromatography/mass spectrometry; MS for mass spectrum; NMR for nuclear magnetic resonance; psi for pounds per square inch; and TLC for thin-layer chromatography.

### Example 1: N,N'-(bicyclo[2.2.2]octane-1,4-diyl)bis[2-(4-chloro-3-fluorophenoxy)acetamide] (Compound 100)

A 50 mL round bottom flask, equipped with a magnetic stir bar, was charged with bicyclo[2.2.2]octane-1,4-diamine dihydrochloride (PharmaBlock, CAS#2277-93-2, 100 mg, 0.455 mmol), 2-(4-chloro-3-fluorophenoxy)acetic acid (205 mg, 1.001 mmol), and (1-cyano-2-ethoxy-2-oxoethylidenaminooxy)dimethylamino-morpholino-carbenium hexafluorophosphate (COMU^{®}, 485 mg, 1.092 mmol). The flask contents were placed under a dry nitrogen atmosphere, and then *N*,*N*-dimethylformamide (10 mL) was added via syringe. The stirred suspension was chilled to 0 °C, and then *N*,*N*-diisopropylethylamine (0.66 mL, 3.78 mmol) was introduced dropwise via syringe (reaction mixture turned bright yellow). The reaction mixture was allowed to warm to ambient temperature and stirred for 3 days. The reaction mixture was diluted with water and a white, insoluble solid was collected by filtration. The solid was treated with methanol, and then collected by filtration. The title compound was thus obtained as a white solid (93.5 mg, 40% yield). ¹H NMR (DMSO-*d*₆) *δ* ppm 7.51-7.44 (m, 4H), 7.02 (dd, J = 11.4, 2.9 Hz, 2H), 6.81 (ddd, J = 9.0, 2.8, 1.2 Hz, 2H), 4.43 (s, 4H), 1.90 (s, 12H). MS (+ESI) *m*/*z* 513 (M+H)⁺, MS (-ESI) *m*/*z* 511 (M-H)⁻.

### Example 2: 2-(4-chloro-3-fluorophenoxy)-N-[4-(2-{[6-(trifluoromethyl)pyridin-3-yl]oxy}acetamido)bicyclo[2.2.2]octan-1-yl]acetamide (Compound 101)

### Example 2A: tert-butyl (4-aminobicyclo[2.2.2]octan-1-yl)carbamate

Bicyclo[2.2.2]octane-1,4-diamine dihydrochloride (PharmaBlock, CAS#2277-93-2, 200 mg, 1.43 mmol) was dissolved in methanol (5 mL). The solution was basified with 50% aqueous sodium hydroxide. After stirring for 15 minutes (slight exotherm), the mixture was diluted with water and brine and extracted with dichloromethane (3 x 150 mL). The combined organic layers were dried (Na₂SO₄) and filtered. The filtrate was concentrated under reduced pressure to give the free base as a white solid. The free base, bicyclo[2.2.2]octane-1,4-diamine (176 mg, 1.255 mmol), di-*tert*-butyl dicarbonate (274 mg, 1.255 mmol), and tetrahydrofuran (100 mL) were stirred at ambient temperature for 17 hours. The reaction mixture was concentrated under reduced pressure, and the residue was partitioned between ethyl acetate and aqueous sodium carbonate. The organic layer was washed with brine, then dried (MgSO₄) and filtered. The filtrate was concentrated under reduced pressure to provide the title intermediate as an off-white solid (258 mg, 86% yield). ¹H NMR (methanol-*d*₄) *δ* ppm 1.91-1.85 (m, 7H), 1.65-1.60 (m, 2H), 1.40 (s, 12H). MS (DCI-NH₃) *m*/*z* 241 (M+H)⁺.

### Example 2B: tert-butyl (4-(2-(4-chloro-3-fluorophenoxy)acetamido)bicyclo[2.2.2]octan-1-yl)carbamate

A 50 mL round bottom flask, equipped with a magnetic stir bar, was charged with 2-(4-chloro-3-fluorophenoxy)acetic acid (234 mg, 1.144 mmol), *tert-*butyl (4-aminobicyclo[2.2.2]octan-1-yl)carbamate (Example 2A, 250 mg, 1.040 mmol), and COMU^{®} (535 mg, 1.248 mmol). The flask contents were placed under a dry nitrogen atmosphere and *N*,*N*-dimethylformamide (4 mL) was introduced via syringe. The reaction mixture was then stirred at ambient temperature as *N*,*N-*diisopropylethylamine (0.545 mL, 3.12 mmol) was added dropwise via syringe. The reaction mixture was stirred at ambient temperature for 19 hours. The reaction mixture was diluted with water (pH = 10). An insoluble beige solid was collected by filtration and rinsed thoroughly with water. The material was purified by column chromatography on an Analogix^{®} IntelliFlash^{™}-310 (Isco RediSep^{®} 40 g silica gel cartridge, 70:30 to 0:100 heptane/ethyl acetate). Fractions #15-31 were combined and concentrated under reduced pressure to give the title intermediate as a white solid (69.5 mg, 15.65% yield). ¹H NMR (CDCl₃) *δ* ppm 7.31 (t, J = 8.6 Hz, 1H), 6.73 (dd, J = 10.3, 2.9 Hz, 1H), 6.64 (ddd, J = 8.9, 2.9, 1.2 Hz, 1H), 6.07 (s, 1H), 4.32 (s, 1H), 4.31 (s, 2H), 2.05-1.91 (m, 12H), 1.42 (s, 9H). MS (+ESI) *m*/*z* 426 (M+H)⁺, *m*/*z* 853 (2M+H)⁺. MS (-ESI) *m*/*z* 425 (M-H)⁻.

### Example 2C: N-(4-aminobicyclo[2.2.2]octan-1-yl)-2-(4-chloro-3-fluorophenoxy)acetamide hydrochloride

A 4 mL vial, equipped with a magnetic stir bar, was charged with *tert-*butyl (4-(2-(4-chloro-3-fluorophenoxy)acetamido)bicyclo[2.2.2]octan-1-yl)carbamate (Example 2B, 69 mg, 0.162 mmol). Methanol (1 mL) was added, and the resulting solution was stirred at ambient temperature while 4 M HCl in dioxane (1.2 mL, 4.80 mmol) was added via syringe. The reaction mixture was stirred at ambient temperature for 89 hours. Volatiles were removed under reduced pressure to give the title intermediate as a white solid (58.3 mg, 99% yield). ¹H NMR (methanol-*d*₄) *δ* ppm 7.36 (t, J = 8.7 Hz, 1H), 6.89 (dd, J = 11.0, 2.9 Hz, 1H), 6.79 (ddd, J = 9.0, 2.9, 1.3 Hz, 1H), 4.43 (s, 2H), 2.15-2.08 (m, 6H), 1.94 1.87 (m, 6H). MS (+ESI) *m*/*z 327* (M+H)⁺. MS (-ESI) *m*/*z* 325 (M-H)⁻.

### Example 2D: 2-(4-chloro-3-fluorophenoxy)-N-[4-(2-{[6-(trifluoromethyl)pyridin-3-yl]oxy}acetamido)bicyclo[2.2.2]octan-1-yl]acetamide

A 4 mL vial, equipped with a magnetic stir bar, was charged with *N-*(4-aminobicyclo[2.2.2]octan-1-yl)-2-(4-chloro-3-fluorophenoxy)acetamide hydrochloride (Example 2C, 25 mg, 0.069 mmol), 2-((6-(trifluoromethyl)pyridin-3-yl)oxy)acetic acid (18.26 mg, 0.083 mmol), and COMU(^{®} (41.3 mg, 0.096 mmol). The vial was sealed with a septum screw cap, and the contents were placed under a dry nitrogen atmosphere. *N*,*N-*Dimethylformamide (0.5 mL) was introduced via syringe, and the stirred reaction mixture was treated dropwise with *N,N-*diisopropylethylamine (0.1 mL, 0.573 mmol). The reaction mixture was stirred at ambient temperature for 19 hours. An aliquot was partitioned between water and ethyl acetate. The organic layer was checked by TLC (80:20 ethyl acetate/heptane). A major new spot with R_{f} higher than either starting material was evident. LC/MS confirmed that this major new material had the correct mass for the title compound. The bulk of the reaction was diluted with water and extracted twice with ethyl acetate. The combined organic layers were washed twice with brine, then dried (MgSO₄) and filtered. The filtrate was concentrated under reduced pressure to give a pale yellow solid. This crude solid was purified by column chromatography on an Analogix^{®} IntelliFlash^{™}-310 (Isco RediSep^{®} 12 g silica gel cartridge, 70:30 heptane/ethyl acetate) to give a white solid that was stirred with *tert-*butyl methyl ether. The solvent was decanted away, and the solid was dried on a rotary evaporator to provide the title compound as a white solid (11.0 mg, 30.2% yield). ¹H NMR (CDCl₃) *δ* ppm 8.43 (d, J = 2.9 Hz, 1H), 7.67 (d, J = 8.7 Hz, 1H), 7.34-7.28 (m, 2H), 6.73 (dd, J = 10.3, 2.9 Hz, 1H), 6.65 (ddd, J = 8.9, 2.9, 1.3 Hz, 1H), 6.10 (d, J = 2.8 Hz, 2H), 4.45 (s, 2H), 4.33 (s, 2H), 2.08 (s, 12H). MS (+ESI) *m*/*z* 530 (M+H)⁺. MS (-ESI) *m*/*z* 528 (M-H)⁻.

### Example 3: N-{3-[2-(4-chloro-3-fluorophenoxy)acetamido]bicyclo[1.1.1]pentan-1-yl}-3-(4-chlorophenyl)propanamide (Compound 102)

### Example 3A : 3-(4-chlorophenyl)propanoyl chloride

A 50 mL round bottom flask, equipped with a magnetic stir bar, was charged with white crystals of 3-(4-chlorophenyl)propanoic acid (Aldrich, CAS# 2019-34-3, 100 mg, 0.542 mmol). The flask was closed with a septum attached to a bubbler. Anhydrous dichloromethane (2 mL) was introduced via syringe to give a solution that was stirred at ambient temperature. Oxalyl chloride (0.142 mL, 1.625 mmol) was added via syringe, followed by *N*,*N*-dimethylformamide (0.042 µL, 0.542 µmol) at which point gas evolution was evident. The reaction mixture was stirred at ambient temperature for 1 hour. Volatiles were removed under reduced pressure to give a pale yellow oil that was used in the next step.

### Example 3B: N-{3-[2-(4-chloro-3-fluorophenoxy)acetamido]bicyclo[1.1.1]pentan-1-yl}-3-(4-chlorophenyl)propanamide

Example 3A was redissolved in dichloromethane (3 mL), and then a suspension of *N-*(3-aminobicyclo[1.1.1]pentan-1-yl)-2-(4-chloro-3-fluorophenoxy)acetamide hydrochloride (Example 112A, 174 mg, 0.542 mmol) in dichloromethane (10 mL) was added to the reaction mixture. This mixture was stirred at ambient temperature under a dry nitrogen atmosphere and triethylamine (0.302 mL, 2.167 mmol) was introduced via syringe. The reaction mixture was stirred at ambient temperature for 20.5 hours. The reaction mixture was treated with aqueous citric acid. The organic layer was washed with brine, then dried (MgSO₄), and filtered. The filtrate was concentrated under reduced pressure to give a beige solid that was treated with *tert-*butyl methyl ether. The insoluble, cream-colored solid was collected by filtration and was purified by column chromatography on an Analogix^{®} IntelliFlash^{™}-310 (Isco RediSep^{®} 24 g silica gel cartridge, 90:10 to 85:15 dichloromethane/acetone, wavelength monitored: 220 nm) to give the title compound as a white solid (97.9 mg, 40% yield). ¹H NMR (DMSO-*d*₆) *δ* ppm 8.68 (s, 1H), 8.40 (s, 1H), 7.49 (t, J = 8.9 Hz, 1H), 7.34-7.30 (m, 2H), 7.23-7.19 (m, 2H), 7.07 (dd, J = 11.4, 2.8 Hz, 1H), 6.85 (ddd, J = 9.0, 2.9, 1.2 Hz, 1H), 4.47 (s, 2H), 2.77 (dd, J = 8.6, 6.9 Hz, 2H), 2.31 (dd, J = 8.5, 7.1 Hz, 2H), 2.20 (s, 6H). MS (+ESI) *m*/*z* 451 (M+H)⁺. MS (-ESI) *m*/*z* 449 (M-H)⁻.

### Example 4: N,N'-(pentacyclo[4.2.0.0^{2,5}.0^{3,8}.0^{4,7}]octane-1,4-diyl)bis[2-(4-chlorophenoxy)-acetamide] (Compound 103)

### Example 4A: cubane-1,4-diamine dihydrochloride

A 50 mL round bottom flask, equipped with a magnetic stir bar, was charged with cubane-1,4-dicarboxylic acid (Aldrich, CAS# 32846-66-5, 800 mg, 4.16 mmol), triethylamine (1.16 mL, 8.32 mmol), diphenylphosphoryl azide (1.8 mL, 8.35 mmol), and *t*-butanol (12.8 mL). The flask was fitted with a reflux condenser equipped with a calcium sulfate drying tube, and the reaction mixture was stirred at reflux for 16 hours. The reaction mixture was allowed to cool to ambient temperature, and then poured into saturated aqueous sodium bicarbonate (50 mL). The precipitate was collected by filtration and washed with water. The solid was dissolved in a hot mixture of dichloromethane, tetrahydrofuran, ethyl acetate, and ethanol. This warm solution was dried (MgSO₄) and filtered. The filtrate was concentrated under reduced pressure to give a beige solid that was treated with ether and collected by filtration. The crude, bis-(*tert*-butoxycarbonyl)-protected intermediate was suspended in methanol (30 mL) and treated with 4 M HCl in dioxane (30 mL, 120 mmol, 47.4 equivalents). The reaction mixture was stirred at ambient temperature for 4 hours. Volatiles were removed under reduced pressure to give a pale brown solid that was washed with diethyl ether and then with ethyl acetate. The solid was dissolved in hot methanol and treated with acetone to induce precipitation. The title intermediate solid was collected by filtration (125 mg, 14.5% yield). ¹H NMR (methanol-*d*₄) *δ* ppm 4.23 (s, 6H). MS (DCI-NH₃) *m*/*z* 135 (M+H)⁺, *m*/*z* 152 (M+NH₄)⁺, *m*/*z* 169 (M+NH₄+NH₃)⁺.

### Example 4B: NN'-(pentacyclo[4.2.0.0^{2,5}.0^{3,8}.0^{1,7}]octane-1,4-diyl)bis[2-(4-chlorophenoxy)acetamide]

A 50 mL round bottom flask, equipped with a magnetic stir bar, was charged with cubane-1,4-diamine dihydrochloride (Example 4A, 77 mg, 0.372 mmol). The flask contents were placed under a dry nitrogen atmosphere, then a solution of 2-(4-chlorophenoxy)acetyl chloride (Aldrich, CAS# 4122-68-3, 160 mg, 0.781 mmol) in dichloromethane (4 mL) was introduced via syringe. This stirred suspension was the treated with triethylamine (0.4 mL, 2.87 mmol). The reaction mixture was stirred at ambient temperature under a dry nitrogen atmosphere for 17 hours. Volatiles were removed under reduced pressure, and the solid residue was partitioned between *tert-*butyl methyl ether and ice water. Material that was insoluble in either layer was suspected to be product and was collected by filtration. This crude, beige solid was dissolved in a warm mixture of tetrahydrofuran and ethanol. Silica gel (1.2 g) was added, and the solvent was removed in vacuo. This mixture adsorbed to silica gel was placed at the top of a Practichem 4 g silica gel cartridge that had the top 1.6 g of silica gel removed. The cartridge was reassembled and connected to the top of an Isco RediSep^{®} 24 g silica gel cartridge and the assembly was eluted with 100:0 to 90:10 dichloromethane/acetone on an Analogix^{®} IntelliFlash^{™}-310 (wavelength monitored: 220 nm) to provide the title compound as a white solid (25.6 mg, 14.6% yield). ¹H NMR (DMSO-*d*₆) *δ* ppm 8.82 (s, 2H), 7.40-7.30 (m, 4H), 6.98 (d, J = 8.9 Hz, 4H), 4.49 (s, 4H), 3.96 (s, 6H). MS (+ESI) *m*/*z* 471 (M+H)⁺. MS (-ESI) *m*/*z* 469 (M-H)⁻.

### Example 5: N-{3-[2-(4-chloro-3-fluorophenoxy)acetamido]bicyclo[1.1.1]pentan-1-yl}-3-(4-chloro-6-oxopyridazin-1(6H)-yl)propanamide (Compound 104)

### Example 5A: methyl 3-(4-chloro-6-oxopyridazin-1(6H)-yl)propanoate

A 50 mL round bottom flask, equipped with a magnetic stir bar, was charged with 5-chloropyridazin-3(2*H*)-one (Maybridge, CAS# 660425-07-0, 350 mg, 2.68 mmol) and cesium carbonate (1310 mg, 4.02 mmol). The vial was sealed with a septum and placed under a dry nitrogen atmosphere, and then *N*,*N*-dimethylformamide (7 mL) was introduced via syringe. The reaction mixture was vigorously stirred at ambient temperature while methyl 3-bromopropanoate (0.351 mL, 3.22 mmol) was added via syringe. The reaction mixture was stirred at ambient temperature for 22 hours. The reaction mixture was diluted with water, neutralized with aqueous citric acid, and extracted with ethyl acetate (twice). The combined organic layers were washed with brine, then dried (MgSO₄), and filtered. The filtrate was concentrated under reduced pressure to give a yellow oil that was purified by column chromatography on an Analogix^{®} IntelliFlash^{™}-310 (Isco RediSep^{®} 40 g silica gel cartridge, 70:30 to 65:35 heptane/ethyl acetate) to give the title intermediate as a clear, colorless oil (479 mg, 82% yield). ¹H NMR (CDCl₃) *δ* ppm 7.72 (d, J = 2.4 Hz, 1H), 6.96 (d, J = 2.4 Hz, 1H), 4.42 (t, J = 7.1 Hz, 2H), 3.70 (s, 3H), 2.83 (t, J = 7.1 Hz, 2H). MS (DCI-NH₃) *m*/*z* 217 (M+H)⁺, *m*/*z* 234 (M+NH₄)⁺.

### Example 5B: 3-(4-chloro-6-oxopyridazin-1(6H)-yl)propanoic acid

A 50 mL round bottom flask, equipped with a magnetic stir bar, was charged with methyl 3-(4-chloro-6-oxopyridazin-1(6*H*)-yl)propanoate (Example 5A, 100 mg, 0.462 mmol). Dioxane (2.3 mL) was added, and the resulting solution was stirred at ambient temperature while sulfuric acid, 5 N aqueous (2.3 mL, 11.50 mmol) was added. The reaction mixture was stirred at 50 °C for 17.5 hours. The reaction mixture was concentrated under reduced pressure, and the oily residue was dissolved in dichloromethane. The solution was dried (Na₂SO₄) and filtered. The filtrate was concentrated under reduced pressure to give the title intermediate as a white solid (33.5 mg, 35.8% yield). ¹H NMR (CDCl₃) *δ* ppm 7.76 (d, J = 2.4 Hz, 1H), 7.01 (d, J = 2.4 Hz, 1H), 4.44 (t, J = 7.0 Hz, 2H), 2.89 (t, J = 7.0 Hz, 2H). MS (DCI-NH₃) *m*/*z* 203 (M+H)⁺, *m*/*z 220* (M+NH₄)⁺.

### Example 5C: 3-(4-chloro-6-oxopyridazin-1(6H)-yl)propanoyl chloride

A 4 mL vial, equipped with a magnetic stir bar, was charged with 3-(4-chloro-6-oxopyridazin-1(6*H*)-yl)propanoic acid (Example 5B, 47.7 mg, 0.235 mmol). The vial was sealed with a septum screw cap vented to a bubbler, and the vial contents were placed under a dry nitrogen atmosphere. Dichloromethane (1 mL) was introduced via syringe, and the stirred reaction mixture was treated with oxalyl chloride (0.1 mL, 1.142 mmol) and catalytic *N,N-*dimethylformamide (0.018 µL, 0.235 µmol). After stirring at ambient temperature for 45 minutes, volatiles were removed under reduced pressure, and the resulting crude acid chloride intermediate was used in the following step.

### Example 5D: N-{3-[2-(4-chloro-3-fluorophenoxy)acetamido]bicyclo[1.1.1]pentan-1-yl}-3-(4-chloro-6-oxopyridazin-1(6H)-yl)propanamide

The acid chloride intermediate, Example 5C, was redissolved in dichloromethane (1 mL). Solid *N-*(3-aminobicyclo[1.1.1]pentan-1-yl)-2-(4-chloro-3-fluorophenoxy)acetamide hydrochloride (Example 112A, 76 mg, 0.235 mmol) was added, and the vial was resealed with the septum screw cap vented to a bubbler. Triethylamine (0.2 mL, 1.435 mmol) was added dropwise via syringe (reaction mixture turns dark). The reaction mixture was vigorously stirred at ambient temperature for 16 hours. The dichloromethane was evaporated, and the residue was treated with pH = 4 water. Insoluble material was collected by filtration and rinsed with water. The remaining solid was purified by column chromatography on an Analogix^{®} IntelliFlash^{™}-310 (Isco RediSep^{®} 12 g silica gel cartridge, 95:5 to 70:30 dichloromethane/acetone, wavelength monitored: 220 nm) to provide 46.9 mg (42.4% yield) of the title compound as a cream-colored solid. ¹H NMR (DMSO-*d*₆) *δ* ppm 8.68 (s, 1H), 8.54 (s, 1H), 8.05 (d, J = 2.4 Hz, 1H), 7.49 (t, J = 8.9 Hz, 1H), 7.25 (d, J = 2.4 Hz, 1H), 7.07 (dd, J = 11.4, 2.9 Hz, 1H), 6.85 (ddd, J = 9.0, 2.9, 1.2 Hz, 1H), 4.47 (s, 2H), 4.19 (t, J = 7.3 Hz, 2H), 2.48 (t, J = 7.3 Hz, 2H), 2.20 (s, 6H). MS (+ESI) *m*/*z* 469 (M+H)⁺. MS (-ESI) *m*/*z* 467 (M-H)⁻.

### Example 6: 2-(3,4-dichlorophenoxy)-N-[3-(2-{[5-(trifluoromethyl)pyrazin-2-yl]oxy}acetamido)bicyclo[1.1.1]pentan-1-yl]acetamide (Compound 105)

### Example 6A: Ethyl 2-((5-(trifluoromethyl)pyrazin-2-yl)oxy)acetate

To a solution of ethyl 2-hydroxyacetate (0.167 mL, 1.762 mmol) in tetrahydrofuran (4 mL) at room temperature was added potassium *tert*-butoxide (1.850 mL, 1.850 mmol). After 10 minutes, 2-bromo-5-(trifluoromethyl)pyrazine (0.2g, 0.881 mmol) in tetrahydrofuran (4 mL) was added. The mixture was stirred at room temperature overnight. The reaction mixture was quenched by addition of water (10 mL), and extracted with ethyl acetate (3 x 30 mL). The organic phase was dried with MgSO₄, filtered and concentrated under reduced pressure. The residue was used in the next step without further purification (215 mg, 0.859 mmol, 98% yield). ¹H NMR (400 MHz, DMSO-*d*₆) *δ* ppm 8.76 (dd, J = 1.4, 0.7 Hz, 1H), 8.63 (d, J = 1.2 Hz, 1H), 5.09 (s, 2H), 4.16 (q, J = 7.1 Hz, 2H), 1.19 (t, J = 7.1 Hz, 3H). ¹⁹F NMR (376 MHz, DMSO-d6) *δ* ppm -65.37 MS (ESI+) *m*/*z* 251 (M+H)⁺.

### Example 6B: 2-((5-(trifluoromethyl)pyrazin-2-yl)oxy)acetic acid

To a solution of ethyl 2-((5-(trifluoromethyl)pyrazin-2-yl)oxy)acetate (215 mg, 0.859 mmol)(crude from previous reaction) in tetrahydrofuran (4 mL) were added lithium hydroxide (82 mg, 3.44 mmol) and water (1.00 mL). The mixture was stirred at room temperature for 2 hours. The reaction mixture diluted with water (2 mL) and extracted with ethyl acetate. The water layer was separated and acidified with 2 N HCl (aq.) to pH=3. The aqueous mixture was extracted with CH₂Cl₂ (2 x 20 mL). The combined organic fractions were dried with MgSO₄, and concentrated under reduced pressure to give the title compound (150mg, 0.675 mmol, 79% yield). ¹H NMR (400 MHz, DMSO-*d*₆) *δ* ppm 13.09 (s, 1H), 8.76 (t, J = 1.0 Hz, 1H), 8.60 (d, J = 1.2 Hz, 1H), 5.01 (s, 2H). ¹⁹F NMR (376 MHz, DMSO-*d*₆) *δ* ppm -64.84. MS (ESI+) *m*/*z 223* (M+H)⁺.

### Example 6C: N-(3-aminobicyclo[1.1.1]pentan-1-yl)-2-(3,4-dichlorophenoxy)acetamide hydrochloride

To Example 22A (3.45 g, 15 mmol) in dichloromethane (10 mL)/methanol (1 ml) was added 4 N HCl in dioxane (53.8 mL, 215 mmol). The mixture was stirred at ambient temperature for 1 hour and then concentrated to give 2.91 g of the title compound (100% yield) as a white solid. ¹H NMR (400 MHz, DMSO-*d*₆) *δ* ppm 8.90 (m, 4H), 7.55 (d, J = 8, 1H), 7.22 (d, J = 2, 1H), 6.98 (dd, J = 8, 2, 1H), 4.50 (s, 2H), 2.23 (s, 6H). MS (ESI+) *m*/*z* 301 (M+H)⁺.

### Example 6D: 2-(3,4-dichlorophenoxy)-N-[3-(2-{[5-(trifluoromethyl)pyrazin-2-yl]oxy}acetamido)bicyclo[1.1.1)pentan-1-yl)acetamide

To a suspension of *N*-(3-aminobicyclo[1.1.1]pentan-1-yl)-2-(3,4-dichlorophenoxy)acetamide hydrochloride (0.05 g, 0.148 mmol, Example 6C) in *N,N-*dimethylformamide (1mL) were added *N*,*N*-diisopropylethylamine (0.078 mL, 0.444 mmol) and 2-((5-(trifluoromethyl)pyrazin-2-yl)oxy)acetic acid (0.036 g, 0.163 mmol, Example 6B), followed by 1-[bis(dimethylamino)methylene]-1*H*-1,2,3-triazolo[4,5-b]pyridinium 3-oxid hexafluorophosphate (0.062 g, 0.163 mmol, HATU). The reaction mixture was stirred 1 hour at room temperature. The reaction mixture was then diluted with water and extracted with ethyl acetate. The combined organic layers were washed with brine. The organic layer was dried with MgSO₄, filtered and concentrated under reduced pressure. The residue was purified by flash column chromatography on silica gel (24 g) and eluted with heptane and ethyl acetate (0 to 100%) to give 25 mg of the title compound (33.4% yield) as a white solid. ¹H NMR (501 MHz, DMSO-*d*₆) *δ* ppm 8.77 (s, 1H), 8.74 (d, J = 1.2 Hz, 1H), 8.71 (s, 1H), 8.55 (d, J = 1.3 Hz, 1H), 7.54 (d, J = 8.9 Hz, 1H), 7.25 (d, J = 2.9 Hz, 1H), 6.98 (dd, J = 9.0, 2.9 Hz, 1H), 4.85 (s, 2H), 4.48 (s, 2H), 2.24 (s, 6H). ¹⁹F NMR (376 MHz, DMSO-*d*₆) *δ* ppm -64.74. MS (ESI⁺) *m*/*z* 552 (M+NH₄)⁺.

### Example 7: N,N'-(bicyclo[1.1.1]pentane-1,3-diyl)bis[2-(3,4-dichlorophenoxy)acetamide] (Compound 106)

To a suspension of *N*-(3-aminobicyclo[1.1.1]pentan-1-yl)-2-(3,4-dichlorophenoxy)acetamide hydrochloride (50 mg, 0.148 mmol, Example 6C) in tetrahydrofuran (1 mL)/*N*,*N*-dimethylformamide (0.1mL) were added *N*,*N-*diisopropylethylamine (0.078 mL, 0.444 mmol) and 2-(3,4-dichlorophenoxy)acetic acid (36.0 mg, 0.163 mmol), followed by 1-[bis(dimethylamino)methylene]-1*H*-1,2,3-triazolo[4,5-b]pyridinium 3-oxid hexafluorophosphate (61.9 mg, 0.163 mmol, HATU). The reaction mixture was stirred 2 hours at room temperature. The white solid was filtered and dried to give 40mg of the title compound (53.6% yield) as a white solid. ¹H NMR (400 MHz, DMSO-*d*₆) *δ* ppm 8.71 (s, 2H), 7.54 (d, J = 8.9 Hz, 2H), 7.26 (d, J = 2.9 Hz, 2H), 6.98 (dd, J = 9.0, 3.0 Hz, 2H), 4.49 (s, 4H), 2.27 (s, 6H). MS (ESI⁺) *m*/*z 505* (M+H)⁺, MS (ESI⁺) *m*/*z* 546 (M+41)⁺.

### Example 8: 2-(4-chloro-3-fluorophenoxy)-N-[3-(2-{[5-(trifluoromethyl)pyrazin-2-yl]oxy}acetamido)bicyclo[1.1.1]pentan-1-yl]acetamide (Compound 107)

To a suspension of Example 112A (30 mg, 0.093 mmol) in *N*,*N*-dimethylformamide (0.8 mL) were added *N*,*N*-diisopropylethylamine (0.049 mL, 0.280 mmol) and 2-((5-(trifluoromethyl)pyrazin-2-yl)oxy)acetic acid (22.82 mg, 0.103 mmol, Example 6B), followed by 1-[bis(dimethylamino)methylene]-1*H*-1,2,3-triazolo[4,5-b]pyridinium 3-oxid hexafluorophosphate (39.1 mg, 0.103 mmol, HATU). The reaction mixture was stirred overnight at room temperature, diluted with water and extracted with ethyl acetate. The combined organic layers were washed with saturated aqueous NaCl. The organic layer was dried with MgSO₄, filtered and concentrated under reduced pressure. The residue was purified by HPLC (Waters XBridge^{™} C18 5 µm OBD^{™} column, 50 x 100 mm, flow rate 90 mL/minute, 5-95% gradient of CH₃CN in buffer (0.1% CF₃CO₂H/H₂O to give 30 mg of the title compound (65.7% yield) as a white solid. ¹H NMR (400 MHz, DMSO-*d*₆) *δ* ppm 8.78 - 8.72 (m, 2H), 8.70 (s, 1H), 8.56 - 8.53 (m, 1H), 7.49 (t, J = 8.9 Hz, 1H), 7.06 (dd, J = 11.4, 2.8 Hz, 1H), 6.84 (ddd, J = 9.0, 2.9, 1.2 Hz, 1H), 4.85 (s, 2H), 4.47 (s, 2H), 2.24 (s, 6H). MS (ESI⁺) *m*/*z* 506 (M+NH₄)⁺.

### Example 9: 2-(4-chloro-3-fluorophenoxy)-N-(3-{2-[(1H-indazol-6-yl)oxy]acetamido}-bicyclo[1.1.1]pentan-1-yl)acetamide (Compound 108)

### Example 9A: tert-butyl (3-(2-(4-chloro-3-fluorophenoxy)acetamido)bicyclo[1.1.1]pentan-1-yl)carbamate

To a solution of 2-(4-chloro-3-fluorophenoxy)acetic acid (Aldlab Chemicals, 2.01 g, 9.84 mmol) in *N*,*N*-dimethylformamide (25 mL) was added *N*-ethyl-N-isopropylpropan-2-amine (3.96 mL, 22.7 mmol) followed by 2-(3*H*-[1,2,3]triazolo[4,5-b]pyridin-3-yl)-1,1,3,3-tetramethylisouronium hexafluorophosphate(V) (3.02 g, 7.94 mmol). This mixture was stirred at ambient temperature for 5 minutes, and then *tert-*butyl (3-aminobicyclo[1.1.1]pentan-1-yl)carbamate (PharmaBlock, 1.5 g, 7.57 mmol) was added. The mixture was allowed to stir at ambient temperature for 16 hours. The reaction mixture was quenched with saturated, aqueous NH₄Cl (20 mL) and then washed with CH₂Cl₂ (25 mL). The aqueous layer was extracted with CH₂Cl₂ (3 x 5 mL), and the combined organic fractions were dried over anhydrous Na₂SO₄, filtered, concentrated under reduced pressure. The residue was purified by column chromatography (SiO₂, 10% ethyl acetate/heptanes to 80% ethyl acetate/heptanes) to give the title compound (2.65 g, 6.89 mmol, 91% yield). MS (ESI⁺) *m*/*z* 402 (M+NH₄)⁺.

### Example 9B: N-(3-aminobicyclo[1.1.1]pentan-1-yl)-2-(4-chloro-3-fluorophenoxy)acetamide-2 Trifluoroacetic acid

To a solution of the product of Example 9A (0.79 g, 2.05 mmol) in CH₂Cl₂ (7 mL) at ambient temperature was added trifluoroacetic acid (3.16 mL, 41.1 mmol). The mixture was allowed to stir at ambient temperature for 3 hours. The mixture was concentrated under reduced pressure and azeotroped with toluene to give the title compound (1.06 g, 2.07 mmol, 100% yield) which was carried on to the next step without purification. MS (ESI⁺) *m*/*z* 285 (M+H)⁺.

### Example 9C: tert-butyl 2-((1H-indazol-5-yl)oxy)acetate

To a solution of 6-hydroxy-1H-indazole (0.89 g, 6.64 mmol) and *tert-*butyl bromoacetate (1.07 mL, 7.30 mmol) in dioxane (20 mL) was added potassium bis(trimethylsilyl)amide (1 M in tetrahydrofuran, 7.96 mL, 7.96 mmol). The mixture was then allowed to stir at ambient temperature for 14 hours. The mixture was quenched with saturated, aqueous NH₄Cl (5 mL) and diluted with CH₂Cl₂ (5 mL). The layers were separated, and the aqueous layer was extracted with CH₂Cl₂ (3 x 5 mL). The combined organic fractions were dried over anhydrous Na₂SO₄, filtered, and concentrated under reduced pressure. The residue was purified via column chromatography (SiO₂, 5% ethyl acetate/heptanes to 100% ethyl acetate) to give the title compound (0.56 g, 2.26 mmol, 34% yield). MS (ESI⁺) *m*/*z* 249 (M+H)⁺.

### Example 9D: 2-((1H-indazol-6-yl)oxy)acetic acid - Trifluoroacetic acid

To a solution of the product of Example 9C (0.56 g, 2.26 mmol) in CH₂Cl₂ (10 mL) at ambient temperature was added trifluoroacetic acid (3.92 mL, 50.9 mmol). The mixture was allowed to stir at ambient temperature for 3 hours, and then it was concentrated under reduced pressure and azeotroped with toluene to give the title compound (0.85 g, 2.36 mmol, >100% yield) which was carried on to the next step without purification. MS (ESI⁺) m/z 193 (M+H)⁺.

### Example 9E: 2-(4-chloro-3-fluorophenoxy)-N-(3-{2-[(1H-indazol-6-yl)oxy]acetamido}-bicyclo[1.1.1]pentan-1-yl)acetamide

To a mixture of the product of Example 9B (0.1 g, 0.195 mmol) and the product of Example 9D (0.090 g, 0.293 mmol) in dimethylacetamide (3 mL) was added N-ethyl-N-isopropylpropan-2-amine (0.136 mL, 0.780 mmol) followed by 2-(3H-[1,2,3]triazolo[4,5-*b*]pyridin-3-yl)-1,1,3,3-tetramethylisouronium hexafluorophosphate(V) (0.078 g, 0.205 mmol). This mixture was allowed to stir at ambient temperature for 16 hours, and then it was quenched with saturated, aqueous NaHCO₃ (10 mL) and diluted with CH₂Cl₂ (10 mL). The layers were separated, and the aqueous layer was extracted with CH₂Cl₂ (3 x 3 mL). The combined organic fractions were dried over anhydrous Na₂SO₄, filtered, and concentrated under reduced pressure. The residue was purified via HPLC (Waters XBridge^{™} C18 5 µm OBD^{™} column, 50 x 100 mm, flow rate 90 mL/minute, 20-100% gradient of methanol in buffer (0.025 M aqueous ammonium bicarbonate, adjusted to pH 10 with ammonium hydroxide)) to give the title compound (0.04, 0.087 mmol, 45% yield). ¹H NMR (400 MHz, DMSO-*d*₆) *δ* ppm 12.82 (s, 1H), 8.71 (d, *J* = 7.7 Hz, 2H), 7.92 (s, 1H), 7.62 (d, *J =* 8.8 Hz, 1H), 7.48 (t, *J =* 8.9 Hz, 1H), 7.05 (dd, *J =* 11.4, 2.9 Hz, 1H), 6.88 (s, 1H), 6.83 (ddt, *J* = 7.5, 4.6, 1.7 Hz, 2H), 4.46 (d, *J =* 3.4 Hz, 4H), 2.26 (s, 6H). MS (ESI⁺) *m*/*z* 459 (M+H)⁺.

### Example 10: N,N'-(bicyclo[1.1.1]pentane-1,3-diyl)bis{2-[(1H-indazol-6-yl)oxy]acetamide} (Compound 109)

### Example 10A: tert-butyl (3-(2-((1H-indazol-6-yl)oxy)acetamido)bicyclo[1.1.1]pentan-1-yl)carbamate

To a solution of the product of Example 9D (0.20 g, 0.56 mmol) in dimethylacetamide (4 mL) was added *N*-ethyl-*N*-isopropylpropan-2-amine (0.26 mL, 1.51 mmol) followed by 2-(3*H-*[1,2,3]triazolo[4,5-*b*]pyridin-3-yl)-1,1,3,3-tetramethylisouronium hexafluorophosphate(V) (0.201 g, 0.530 mmol). This mixture was stirred at ambient temperature for 2 minutes then *tert-*butyl (3-aminobicyclo[1.1.1]pentan-1-yl)carbamate (PharmaBlock, 0.10 g, 0.504 mmol) was added. The mixture was allowed to stir at ambient temperature for 16 hours, and then it was quenched with saturated, aqueous NH₄Cl (10 mL), diluted with CH₂Cl₂ (15 mL), and the layers were separated. The aqueous layer was extracted with CH₂Cl₂ (3 x 5 mL), and the combined organic fractions were dried over anhydrous Na₂SO₄, filtered, and concentrated under reduced pressure. The residue was purified by HPLC (Waters XBridge^{™} C18 5 µm OBD^{™} column, 50 x 100 mm, flow rate 90 mL/minute, 20-100% gradient of methanol in buffer (0.025 M aqueous ammonium bicarbonate, adjusted to pH 10 with ammonium hydroxide)) to give the title compound (0.12 g, 0.33 mmol, 65% yield). MS (ESI⁺) *m*/*z* 373 (M+H)⁺.

### Example 10B: 2-((1H-indazol-6-yl)oxy)-N-(3-aminobicyclo[1.1.1]pentan-1-yl)acetamide-3-trifluoroacetic acid

To a solution of the product of Example 10A (0.12 g, 0.33 mmol) in CH₂Cl₂ (3 mL) at ambient temperature was added trifluoroacetic acid (0.51 mL, 6.6 mmol). This mixture was allowed to stir at ambient temperature for 3 hours, and then it was concentrated under reduced pressure and azeotroped with toluene to give the title compound (0.22 g, 0.36 mmol, >100% yield) which was carried on to the next step without purification. MS (ESI⁺) *m*/*z* 273 (M+H)⁺.

### Example 10C: N,N'-(bicyclo[1.1.1]pentane-1,3-diyl)bis{2-[(1H-indazol-6-yl)oxy]acetamide)

To a mixture of the product of Example 10B (0.10 g, 0.16 mmol) and the product of Example 9D (0.055 g, 0.18 mmol) in dimethylacetamide (2 mL) was added *N*-ethyl-*N-*isopropylpropan-2-amine (0.17 mL, 0.98 mmol) followed by 2-(3*H*-[1,2,3]triazolo[4,5-*b*]pyridin-3-yl)-1,1,3,3-tetramethylisouronium hexafluorophosphate(V) (0.065 g, 0.17 mmol). This mixture was allowed to stir at ambient temperature for 16 hours, and it was directly purified via HPLC (Waters XBridge^{™} C18 5 µm OBD^{™} column, 50 x 100 mm, flow rate 90 mL/minute, 20-100% gradient of methanol in buffer (0.025 M aqueous ammonium bicarbonate, adjusted to pH 10 with ammonium hydroxide)) to give the title compound (0.055 g, 0.12 mmol, 76% yield). ¹H NMR (501 MHz, DMSO-*d*₆) *δ* ppm 12.82 (s, 2H), 8.72 (s, 2H), 7.92 (t, J = 1.3 Hz, 2H), 7.62 (d, J = 8.8 Hz, 2H), 6.88 (d, J = 2.1 Hz, 2H), 6.82 (dd, J = 8.8, 2.1 Hz, 2H), 4.47 (s, 4H), 2.27 (s, 6H). MS (ESI⁺) *m*/*z* 445 (M-H)⁺.

### Example 11: 2-(4-chloro-3-fluorophenoxy)-N-(3-{2-[(3-methyl-1,2-benzoxazol-6-yl)oxy]acetamidolbicyclo[1.1.1]pentan-1-yl)acetamide (Compound 110)

### Example 11A: tert-butyl 2-((3-methylbenzo[d]isoxazol-6-yl)oxy)acetate

A mixture of 5-hydroxy-3-methylbenzo[*d*]isoxazole (Chontech, 1.0 g, 6.70 mmol), potassium carbonate (1.85 g, 13.4 mmol) and *tert-*butyl bromoacetate (1.03 mL, 7.04 mmol) in *N*,*N*-dimethylformamide (20 mL) was warmed to 65 °C and was allowed to stir for 16 hours. The mixture was then quenched with saturated, aqueous NaHCO₃ (10 mL) and diluted with ethyl acetate (10 mL). The layers were separated, and the aqueous layer was extracted with ethyl acetate (3 x 5 mL). The combined organic fractions were dried over anhydrous Na₂SO₄, filtered, and concentrated under reduced pressure. The residue was purified via column chromatography (SiO₂, 5% ethyl acetate/heptanes to 100% ethyl acetate) to give the title compound (1.45 g, 5.51 mmol, 82% yield). MS (ESI⁺) *m*/*z* 264 (M+H)⁺.

### Example 11B: 2-((3-methylbenzo[d]isoxazol-6-yl)oxy)acetic acid

To a solution of the product of Example 11A (1.7 g, 6.46 mmol) in CH₂Cl₂ (25 mL) at ambient temperature was added trifluoroacetic acid (7.46 mL, 97 mmol). The mixture was allowed to stir at ambient temperature for 3 hours. The mixture was concentrated under reduced pressure, and the residue was azeotroped with toluene to give the title compound (1.68 g, 6.49 mmol, 100% yield) which was carried on in the next step without purification. MS (ESI⁺) *m*/*z* 208 (M+H)⁺.

### Example 11C: 2-(4-chloro-3-fluorophenoxy)-N-(3-{2-[(3-methyl-1,2-benzoxazol-6-yl)oxy]acetamido}bicyclo[1.1.1]pentan-1-yl)acetamide

To a mixture of the product of Example 9B (0.20 g, 0.390 mmol) and the product of Example 11B (0.204 g, 0.51 mmol) in dimethylacetamide (3 mL) was added *N*-ethyl-*N-*isopropylpropan-2-amine (0.272 mL, 1.56 mmol) followed by 2-(3*H*-[1,2,3]triazolo[4,5-*b*]pyridin-3-yl)-1,1,3,3-tetramethylisouronium hexafluorophosphate(V) (0.156 g, 0.410 mmol). This mixture was allowed to stir at ambient temperature for 16 hours then was quenched with saturated, aqueous NaHCO₃ (10 mL) and diluted with CH₂Cl₂ (10 mL). The layers were separated, and the aqueous layer was extracted with CH₂Cl₂ (3 x 3 mL). The combined organic fractions were dried over anhydrous Na₂SO₄, filtered, and concentrated under reduced pressure. The residue was purified via HPLC (Waters XBridge^{™} C18 5 µm OBD^{™} column, 50 x 100 mm, flow rate 90 mL/minute, 20-100% gradient of methanol in buffer (0.025 M aqueous ammonium bicarbonate, adjusted to pH 10 with ammonium hydroxide)) to give the title compound (0.18 g, 0.38 mmol, 97% yield). ¹H NMR (400 MHz, DMSO-*d*₆) *δ* ppm 8.67 (d, *J* = 2.0 Hz, 2H), 7.52 (d, *J* = 8.9 Hz, 1H), 7.46 (t, *J =* 8.9 Hz, 1H), 7.17 (d, *J =* 2.6 Hz, 1H), 7.04 (dd, *J=* 11.4, 2.8 Hz, 1H), 6.96 (dd, *J =* 8.8, 2.6 Hz, 1H), 6.82 (ddd, *J =* 9.0, 2.9, 1.2 Hz, 1H), 4.44 (d, *J =* 6.6 Hz, 4H), 2.54 (s, 3H), 2.24 (s, 6H). MS (ESI⁺) *m*/*z* 474 (M+H)⁺.

### Example 12: 2-(4-chloro-3-fluorophenoxy)-N-(3-{2-[(4-fluoro-1H-indazol-6-yl)oxy]acetamido}-bicyclo[1.1.1]pentan-1-yl)acetamide (Compound 111)

### Example 12A: tert-butyl 2-((4-fluoro-1H-indazol-6-yl)oxy)acetate

A mixture of 4-fluoro-1*H*-indazol-6-ol (ArkPharm, Inc., 1.0 g, 6.57 mmol), potassium carbonate (1.82 g, 13.2 mmol) and *tert-*butyl bromoacetate (1.01 mL, 6.90 mmol) in *N,N-*dimethylformamide (15 mL) was warmed to 65 °C and was allowed to stir for 16 hours. The mixture was allowed to cool to ambient temperature and was quenched with saturated, aqueous NaHCO₃ (10 mL) and diluted with ethyl acetate (10 mL). The layers were separated, and the aqueous layer was extracted with ethyl acetate (3 x 5 mL). The combined organic fractions were dried over anhydrous Na₂SO₄, filtered, concentrated under reduced pressure. The residue was purified via column chromatography (SiO₂, 5% ethyl acetate/heptanes to 100% ethyl acetate) to give the title compound (0.81 g, 3.04 mmol, 46% yield). MS (ESI⁺) *m*/*z* 267 (M+H)⁺.

### Example 12B: 2-((4-fluoro-1H-indazol-6-yl)oxy)acetic acid

To a solution of the product of Example 12A (0.81 g, 3.04 mmol) in CH₂Cl₂ (5 mL) at ambient temperature was added trifluoroacetic acid (2.34 mL, 30.4 mmol). This mixture was allowed to stir at ambient temperature for 4 hours and then was concentrated under reduced pressure. The residue was azeotroped with toluene to give solids which were re-precipitated from ethyl acetate/heptanes to give the title compound (1.31 g, 2.99 mmol, 98% yield). ¹H NMR (400 MHz, DMSO-*d*₆) *δ* ppm 8.03 (s, 1H), 6.72 (t, *J =* 1.3 Hz, 1H), 6.60 (dd, *J =* 11.7, 1.8 Hz, 1H), 4.75 (s, 2H).

### Example 12C: 2-(4-chloro-3-fluorophenoxy)-N-(3-{2-[(4-fluoro-1H-indazol-6-yl)oxy]acetamido}bicyclo[1.1.1]pentan-1-yl)acetamide

To a mixture of the product of Example 9B (0.15 g, 0.29 mmol) and the product of Example 12B (0.14 g, 0.32 mmol) in *N*,*N*-dimethylformamide (3 mL) was added *N*-ethyl-*N-*isopropylpropan-2-amine (0.20 mL, 1.17 mmol) followed by 2-(3*H*-[1,2,3]triazolo[4,5-*b*]pyridin-3-yl)-1,1,3,3-tetramethylisouronium hexafluorophosphate(V) (0.18 g, 0.31 mmol). This mixture was allowed to stir at ambient temperature for 16 hours then was quenched with saturated, aqueous NaHCO₃ (10 mL) and diluted with CH₂Cl₂ (10 mL). The layers were separated, and the aqueous layer was extracted with CH₂Cl₂ (3 x 3 mL). The combined organic fractions were dried over anhydrous Na₂SO₄, filtered, and concentrated under reduced pressure. The residue was purified via column chromatography (SiO₂, 75% ethyl acetate/heptanes) to give the title compound (0.11 g, 0.23 mmol, 79% yield). ¹H NMR (500 MHz, DMSO-*d*₆) *δ* ppm 13.19 (s, 1H), 8.75 (d, *J* = 12.0 Hz, 2H), 8.06 (s, 1H), 7.50 (t, *J* = 8.9 Hz, 1H), 7.08 (dd, *J=* 11.4, 2.8 Hz, 1H), 6.86 (ddd, *J =* 8.9, 2.9, 1.2 Hz, 1H), 6.78 (t, *J =* 1.4 Hz, 1H), 6.69 (dd, *J =* 11.6, 1.8 Hz, 1H), 4.52 (s, 2H), 4.49 (s, 2H), 2.28 (s, 6H). MS (ESI⁺) *m*/*z* 475 (M+H)⁺.

### Example 13: N,N'-(bicyclo[1.1.1]pentane-1,3-diyl)bis[2-(4-chlorophenoxy)acetamide] (Compound 112)

Bicyclo[1.1.1]pentane-1,3-diamine dihydrochloride (Pharmablock, 2.588 g, 15.13 mmol) in tetrahydrofuran/water (1/1, 60 mL) was treated with potassium carbonate (10.45 g, 76 mmol), cooled to 0°C and then treated with 2-(4-chlorophenoxy)acetyl chloride (4.72 mL, 30.3 mmol). The reaction mixture was stirred at ambient temperature for 2 hours. The precipitate was collected by filtration, washed with water and hexane, and air dried to provide 5.635 g (86%) of the title compound. ¹H NMR (400 MHz, DMSO-*d*₆) *δ* ppm 8.66 (s, 2H), 7.42 - 7.26 (m, 4H), 7.00 - 6.87 (m, 4H), 4.39 (s, 4H), 2.23 (s, 6H). MS (APCI) *m*/*z* 436 (M+H)⁺.

### Example 14: 2-(4-chlorophenoxy)-N-(3-{[2-(4-chlorophenoxy)ethyl]amino}bicyclo-[1.1.1]pentan-1-yl)acetamide (Compound 113)

### Example 14A: tert-butyl (3-(2-(4-chlorophenoxy)acetamido)bicyclo[1.1.1]pentan-1-yl)carbamate

*tert-*Butyl (3-aminobicyclo[1.1.1]pentan-1-yl)carbamate hydrochloride (Pharmablock, 0.469 g, 2 mmol) in tetrahydrofuran/water (1/1, 6 mL) was treated with potassium carbonate (0.732 g, 5.30 mmol), cooled to 0 °C, and then treated with 2-(4-chlorophenoxy)acetyl chloride (0.312 mL, 2 mmol). The reaction mixture was stirred at ambient temperature for 2 hours. The resultant precipitate was collected by filtration, washed with water and hexane, and air dried to provide 0.471 g (57.4%) of the title compound. MS (APCI) *m*/*z* 367 (M+H)⁺.

### Example 14B: N-(3-aminobicyclo[1.1.1]pentan-1-yl)-2-(4-chlorophenoxy)acetamide

A solution of Example 14A (0.471 g, 1.148 mmol) in dioxane (3 mL) was treated with 4 N HCl in dioxane (3 mL) and stirred at 25 °C for 20 hours. The reaction mixture was concentrated to provide 0.347 g (100%) of the title compound. MS (APCI) *m*/*z* 267 (M+H)⁺.

### Example 14C: 2-(4-chlorophenoxy)-N-(3-{[2-(4-chlorophenoxy)ethyl]amino}bicyclo-[1.1.1]pentan-1-yl)acetamide

A solution of Example 14B (0.1 g, 0.33 mmol) and 2-(4-chlorophenoxy)acetaldehyde (0.051 g, 0.3 mmol) in methanolic pH4 buffer (2 mL) was stirred at ambient temperature for 1 hour and then treated with sodium cyanoborohydride (0.062 g, 0.99 mmol). The reaction mixture was stirred for 20 hours and then partitioned between dichloromethane (20 mL) and water (20 mL). The aqueous layer was extracted with dichloromethane (3 x 20 mL). The combined organic layers were washed with brine (2 x 30 mL), dried (Na₂SO₄), filtered and concentrated under reduced pressure. The residue was purified by HPLC (Phenomenex^{®} Luna^{®} C18(2) 5 µm 100 Å AXIA^{™} column 250 mm × 21.2 mm, flow rate 25 mL/minute, 10-80% gradient of acetonitrile in buffer (0.1% trifluoroacetic acid in water)) to provide 0.076 g (60%) of the title compound. ¹H NMR (501 MHz, DMSO-*d*₆) *δ* ppm 8.83 (s, 1H), 7.41 - 7.25 (m, 4H), 7.06 - 6.92 (m, 4H), 4.44 (s, 2H), 4.15 (t, *J =* 5.1 Hz, 2H), 3.28 - 3.24 (m, 2H), 2.24 (s, 6H). MS (APCI) *m*/*z* 422 (M+H)⁺.

### Example 15: 2-(4-chlorophenoxy)-N-{3-[2-(3-methylphenoxy)acetamido]bicyclo[1.1.1]-pentan-1-yl}acetamide (Compound 114)

To a solution of 2-(m-tolyloxy)acetic acid (13.7 mg, 0.0801 mmol) in *N,N-*dimethylacetamide (0.5 mL) was added *N*,*N*-diisopropylethylamine (0.052 mL, 0.299 mmol), 1-[bis(dimethylamino)methylene]-1*H*-1,2,3-triazolo[4,5-b]pyridinium 3-oxid hexafluorophosphate (71.3 mg, 0.187 mmol) and Example 14B (20.1 mg, 0.0701 mmol). The reaction was stirred at ambient temperature for 18 hours. The crude reaction was purified by HPLC (2-coupled C8 5 µm 100 Å columns 30 mm × 75 mm each, flow rate of 50 mL/minute, 5-90% gradient of acetonitrile in buffer (0.1% trifluoroacetic acid in water)). ¹H NMR (400 MHz, DMSO-*d*₆) *δ* ppm 8.74 (d, J = 17.9 Hz, 2H), 7.39 - 7.27 (m, 2H), 7.16 (t, J = 8.1 Hz, 1H), 7.05 - 6.91 (m, 2H), 6.86 - 6.64 (m, 3H), 4.39 (d, J = 16.7 Hz, 4H), 2.26 (m, 9H). MS (APCI) *m*/*z* 415.370 (M+H)⁺.

### Example 16: 2-(4-chlorophenoxy)-N-{3-[2-(4-methylphenoxy)acetamido]bicyclo-[1.1.1]pentan-1-yl}acetamide (Compound 115)

The title compound was prepared using the method described in Example 15 by replacing 2-(m-tolyloxy)acetic acid with 2-(p-tolyloxy)acetic acid (13.7 mg, 0.0801 mmol). ¹H NMR (400 MHz, DMSO-*d*₆) *δ* ppm 7.38 - 7.27 (m, 2H), 7.12 - 7.05 (m, 2H), 7.00 - 6.94 (m, 2H), 6.86 - 6.81 (m, 2H), 4.38 (d, J = 24.1 Hz, 4H), 2.26 (s, 6H), 2.22 (s, 3H). MS (APCI) *m*/*z* 415.330 (M+H)⁺.

### Example 17: 2-(4-chloro-3-methylphenoxy)-N-{3-[2-(4-chlorophenoxy)acetamido]bicyclo-[1.1.1]pentan-1-yl}acetamide (Compound 116)

The title compound was prepared using the method described in Example 15 by replacing 2-(m-tolyloxy)acetic acid with 2-(4-chloro-3-methylphenoxy)acetic acid (16.6 mg, 0.0801 mmol). ¹H NMR (400 MHz, DMSO-*d*₆) *δ* ppm 8.76 (d, J = 7.9 Hz, 2H), 7.37 - 7.25 (m, 3H), 6.97 (m, 3H), 6.97 (dd, J = 9.5, 2.8 Hz, 1H), 4.40 (d, J = 8.2 Hz, 4H), 2.27 (m, J = 5.1 Hz, 9H). MS (APCI) *m*/*z* 449.2 (M+H)⁺.

### Example 18: 2-(4-chlorophenoxy)-N-{3-[2-(3,4-dichlorophenoxy)acetamido]bicyclo-[1.1.1]pentan-1-yl}acetamide (Compound 117)

The title compound was prepared using the method described in Example 15 by replacing 2-(m-tolyloxy)acetic acid with 2-(3,4-dichlorophenoxy)acetic acid (18.2 mg, 0.0801 mmol). ¹H NMR (400 MHz, DMSO-*d*₆) *δ* ppm 7.52 (dd, J = 8.9, 4.6 Hz, 2H), 7.36 - 7.30 (m, 2H), 7.23 (t, J = 3.3 Hz, 1H), 7.00 - 6.94 (m, 4H), 4.44 (d, J = 20.4 Hz, 4H), 2.26 (s, 6H). MS (APCI) *m*/*z* 469.230 (M+H)⁺.

### Example 19: 2-(4-chlorophenoxy)-N-{3-[2-(3-chlorophenoxy)acetamido]bicyclo-[1.1.1]pentan-1-yl}acetamide (Compound 118)

The title compound was prepared using the method described in Example 15 by replacing 2-(m-tolyloxy)acetic acid with 2-(3-chlorophenoxy)acetic acid (15.4 mg, 0.0801 mmol). ¹H NMR (400 MHz, DMSO-*d*₆) *δ* ppm 8.78 (d, J = 5.1 Hz, 2H), 7.36 - 7.27 (m, 3H), 7.06 - 6.88 (m, 6H), 4.43 (d, J = 12.4 Hz, 4H), 2.27 (s, 6H). MS (APCI) *m*/*z* 435.280 (M+H)⁺.

### Example 20: 2-(4-chlorophenoxy)-N-{3-[2-(3-fluorophenoxy)acetamido]-bicyclo[1.1.1]pentan-1-yl}acetamide (Compound 119)

The title compound was prepared using the method described in Example 15 by replacing 2-(m-tolyloxy)acetic acid with 2-(3-fluorophenoxy)acetic acid (14.1 mg, 0.0801 mmol). ¹H NMR (400 MHz, DMSO-*d*₆) *δ* ppm 7.37 - 7.28 (m, 3H), 7.00 - 6.93 (m, 2H), 6.85 - 6.75 (m, 3H), 4.43 (d, J = 8.7 Hz, 4H), 2.27 (s, 6H). MS (APCI) *m*/*z* 419.280 (M+H)⁺.

### Example 21: 2-(4-chlorophenoxy)-N-{3-[2-(4-fluorophenoxy)acetamido]bicyclo-[1.1.1]pentan-1-yl}acetamide (Compound 120)

The title compound was prepared using the method described in Example 15 by replacing 2-(m-tolyloxy)acetic acid with 2-(4-fluorophenoxy)acetic acid (14.1 mg, 0.0801 mmol). ¹H NMR (400 MHz, DMSO-*d*₆) *δ* ppm 8.76 (d, J = 6.9 Hz, 1H), 7.37 - 7.28 (m, 2H), 7.17 - 7.06 (m, 2H), 7.02 - 6.92 (m, 4H), 4.40 (d, J = 10.8 Hz, 4H), 2.26 (s, 6H). MS (APCI) *m*/*z* 419.260 (M+H)⁺.

### Example 22: 2-(4-chloro-3-fluorophenoxy)-N-{3-[2-(3,4-dichlorophenoxy)acetamido]-bicyclo[1.1.1]pentan-1-yl}acetamide (Compound 121)

### Example 22A: tert-butyl (3-(2-(3,4-dichlorophenoxy)acetamido)bicyclo[1.1.1]pentan-1-yl)carbamate

To a solution of 2-(3,4-dichlorophenoxy)acetic acid (3.53 g, 15.98 mmol) and *tert-*butyl (3-aminobicyclo[1.1.1]pentan-1-yl)carbamate (Pharmablock, 3.2 g, 14.53 mmol) in *N,N-*dimethylformamide (50 mL) was added *N*,*N*-diisopropylethylamine (12.69 mL, 72.6 mmol) and fluoro-*N,N,N,N*-tetramethylformamidinium hexafluorophosphate (8.28 g, 21.79 mmol) at ambient temperature under nitrogen. The resulting mixture was stirred, diluted with water (300 mL) and extracted with ethyl acetate (3 x 200 mL). The combined organic layer was washed with brine (3 x 100 mL), dried (Na₂SO₄), filtered, and concentrated under reduced pressure. The residue was treated with methyl *tert-*butyl ether (15 mL) and dried under high vacuum to provide 4.2 g (72.3%) of the title compound as a yellow solid. MS (APCI) *m*/*z* 402 (M+H)⁺.

### Example 22B: N-(3-aminobicyclo[1.1.1]pentan-1-yl)-2-(3,4-dichlorophenoxy)acetamide

To a solution of Example 22A (5.5 g, 13.57 mmol) in dichloromethane (100 mL) was added trifluoroacetic acid (30 mL, 389 mmol) at 0 °C. The mixture was stirred at ambient temperature for 12 hours. The mixture was concentrated under reduced pressure, and the residue was diluted with water (300 mL). The aqueous phase was adjusted to pH= 8 with saturated NaHCO₃, and extracted with dichloromethane (4 x 150 mL). The combined organic layer was dried (Na₂SO₄), filtered, and concentrated under reduced pressure to provide 4 g (87%) of the title compound as off white solid. MS (APCI) *m*/*z* 302 (M+H)⁺.

### Example 22C: 2-(4-chloro-3-fluorophenoxy)-N-{3-[2-(3,4-dichlorophenoxy)acetamido]-bicyclo[1.1.1]pentan-1-yljacetamide

To a solution of 2-(4-chloro-3-fluorophenoxy)acetic acid (0.033 g, 0.161 mmol) in *N,N-*dimethylformamide (1 mL) was added N,N-diisopropylethylamine (0.064 mL, 0.366 mmol), 1-[bis(dimethylamino)methylene]-1H-1,2,3-triazolo[4,5-b]pyridinium 3-oxid hexafluorophosphate (0.061 g, 0.161 mmol) and Example 22B (0.044 g, 0.146 mmol). The reaction mixture was stirred at room temperature for 18 hours and concentrated. HPLC purification (Phenomenex^{®} Luna^{®} C18(2) 5 µm 100 Å AXIA^{™} column 250 mm × 21.2 mm, flow rate 25 mL/minute, 10-80% gradient of acetonitrile in buffer (0.1% trifluoroacetic acid in water)) afforded the title compound. ¹H NMR (501 MHz, DMSO-*d*₆) *δ* ppm 8.72 (s, 2H), 7.55 (d, *J =* 8.9 Hz, 1H), 7.49 (t, *J =* 8.9 Hz, 1H), 7.26 (d, *J = 2.9* Hz, 1H), 7.07 (dd, *J =* 11.4, 2.8 Hz, 1H), 6.99 (dd, *J =* 8.9, 2.9 Hz, 1H), 6.85 (ddd, *J* = 9.0, 2.8, 1.2 Hz, 1H), 4.49 (s, 2H), 4.48 (s, 2H), 2.27 (s, 6H). MS (APCI) *m*/*z* 489 (M+H)⁺.

### Example 23: 2-(4-chloro-2-fluorophenoxy)-N-{3-[2-(3,4-dichlorophenoxy)acetamido]-bicyclo[1.1.1]pentan-1-yl}acetamide (Compound 122)

The title compound was prepared using the method described in Example 22C by replacing 2-(4-chloro-3-fluorophenoxy)acetic acid with 2-(4-chloro-2-fluorophenoxy)acetic acid (33 mg, 0.161 mmol). ¹H NMR (400 MHz, DMSO-*d*₆) *δ* ppm 8.76 - 8.66 (m, 2H), 7.54 (d, *J =* 9.0 Hz, 1H), 7.45 (dd, *J =* 11.2, 2.5 Hz, 1H), 7.26 (d, *J = 2.9* Hz, 1H), 7.24 - 7.18 (m, 1H), 7.08 (t, *J=* 9.0 Hz, 1H), 6.98 (dd, *J =* 8.9, 2.9 Hz, 1H), 4.54 (s, 2H), 4.48 (s, 2H), 2.25 (s, 6H). MS (APCI) *m*/*z* 489 (M+H)⁺.

### Example 24: N-(3-{[2-(4-chloro-2-fluorophenoxy)ethyl]amino}bicyclo[1.1.1]pentan-1-yl)-2-(3,4-dichlorophenoxy)acetamide (Compound 123)

### Example 24A: 1-(2-bromoethoxy)-4-chloro-2-fluorobenzene

A suspension of 4-chloro-2-fluorophenol (0.88 g, 6 mmol) and potassium carbonate (1.244 g, 9 mmol) in acetonitrile (20 mL) was treated with 1,2-dibromoethane (2.56 mL, 24 mmol) and stirred at 90 °C for 2 days. The reaction mixture was concentrated, washed with water and extracted twice with dichloromethane. The combined organic extracts were dried (Na₂SO₄), filtered, and then concentrated under reduced pressure to provide the title compound. MS (APCI) *m*/*z* 254 (M+H)⁺.

### Example 24B: N-(3-{[2-(4-chloro-2-fluorophenoxy)ethyl]amino]bicyclo[1.1.1]pentan-1-yl)-2-(3,4-dichlorophenoxy)acetamide

A suspension of Example 24A (0.05 g, 0.148 mmol) and Example 22B (0.038 g, 0.148 mmol) in *N*,*N*-dimethylformamide (1 mL) was treated with potassium carbonate (0.051 g, 0.37 mmol) and stirred at 90 °C for 18 hours. The reaction mixture was concentrated and purified on HPLC (Phenomenex^{®} Luna^{®} C18(2) 5 µm 100 Å AXIA^{™} column 250 mm × 21.2 mm, flow rate 25 mL/minute, 10-80% gradient of acetonitrile in buffer (0.1% trifluoroacetic acid in water)) to provide the title compound. ¹H NMR (400 MHz, DMSO-*d*₆) *δ* ppm 8.89 (s, 1H), 7.56 (d, *J =* 8.9 Hz, 1H), 7.49 (dd, *J =* 11.0, 1.9 Hz, 1H), 7.33 - 7.21 (m, 3H), 6.99 (dd, *J =* 8.9, 2.9 Hz, 1H), 4.53 (s, 2H), 4.27 (t, *J =* 5.0 Hz, 2H), 2.29 (s, 6H). MS (APCI) *m*/*z* 475 (M+H)⁺.

### Example 25: N-(3-{[2-(4-chloro-3-fluorophenoxy)ethyl]amino}bicyclo[1.1.1]pentan-1-yl)-2-(3,4-dichlorophenoxy)acetamide (Compound 124)

### Example 25A: 1-(2-bromoethoxy)-4-chloro-3-fluorobenzene

The title compound was prepared using the method described in Example 24A by replacing 4-chloro-2-fluorophenol with 4-chloro-3-fluorophenol (0.88 g, 6 mmol). MS (APCI) *m*/*z* 254 (M+H)⁺.

### Example 25B: N-(3-{[2-(4-chloro-3-fluorophenoxy)ethyl]amino)bicyclo[1.1.1]pentan-1-yl)-2-(3,4-dichlorophenoxy)acetamide

The title compound was prepared using the method described in Example 24B by replacing 1-(2-bromoethoxy)-4-chloro-2-fluorobenzene with 1-(2-bromoethoxy)-4-chloro-3-fluorobenzene (0.05 g, 0.148 mmol). ¹H NMR (400 MHz, DMSO-*d*₆) *δ* ppm 8.91 (s, 1H), 7.59 - 7.50 (m, 2H), 7.26 (d, *J =* 2.9 Hz, 1H), 7.13 (dd, *J =* 11.3, 2.9 Hz, 1H), 6.99 (dd, *J =* 8.9, 2.9 Hz, 1H), 6.90 (ddd, *J* = 9.0, 2.9, 1.2 Hz, 1H), 4.53 (s, 2H), 4.23 (t, *J =* 4.9 Hz, 2H), 2.31 (s, 6H). MS (APCI) *m*/*z* 475 (M+H)⁺.

### Example 26: 2-(3,4-dichlorophenoxy)-N-(3-{[2-(3,4-dichlorophenoxy)ethyl]amino}-bicyclo[1.1.1]pentan-1-yl)acetamide (Compound 125)

### Example 26A: 4-(2-bromoethoxy)-1,2-dichlorobenzene

The title compound was prepared using the method described in Example 24A by replacing 4-chloro-2-fluorophenol with 3,4-dichlorophenol (0.88 g, 6 mmol). MS (APCI) *m*/*z* 270 (M+H)⁺.

### Example 26B: 2-(3,4-dichlorophenoxy)-N-(3-[[2-(3,4-dichlorophenoxy)ethyl]amino]-bicyclo[1.1.1]pentan-1-yl)acetamide

The title compound was prepared using the method described in Example 24B by replacing 1-(2-bromoethoxy)-4-chloro-2-fluorobenzene with 4-(2-bromoethoxy)-1,2-dichlorobenzene (0.05 g, 0.148 mmol). ¹H NMR (501 MHz, DMSO-*d*₆) *δ* ppm 8.90 (s, 1H), 7.57 (dd, *J =* 12.2, 8.9 Hz, 2H), 7.28 (dd, *J =* 18.4, 2.9 Hz, 2H), 7.01 (ddd, *J =* 18.0, 8.9, 2.9 Hz, 2H), 4.53 (s, 2H), 4.24 (t, *J =* 5.0 Hz, 2H), 2.30 (s, 6H); ). MS (APCI) *m*/*z* 491 (M+H)⁺.

### Example 27: 2-(4-chloro-3-fluorophenoxy)-N-{3-[2-(4-chloro-3-methoxyphenoxy)acetamido]-bicyclo[1.1.1]pentan-1-yl}acetamide (Compound 126)

### Example 27A: tert-butyl 2-(4-chloro-3-methoxyphenoxy)acetate

A solution of 4-chloro-3-methoxyphenol (1g, 6.31 mmol) in *N*,*N*-dimethylformamide (10 mL) was treated with *tert*-butyl 2-bromoacetate (1.024 mL, 6.94 mmol) and potassium carbonate (1.743 g, 12.61 mmol) and heated at 65 °C for 2 hours. The reaction mixture was diluted with ethyl acetate and washed with water twice. The organic extract was dried (Na₂SO₄), filtered and concentrated to provide 1.72 g (100%) of the title compound. MS (APCI) *m*/*z* 273 (M+H)⁺.

### Example 27B: 2-(4-chloro-3-methoxyphenoxy)acetic acid

A solution of Example 27A (1.72 g, 6.31 mmol) in dioxane (8 mL) was treated with 4 N HCl in dioxane (8 mL) and stirred at 25 °C for 4 hours. The reaction mixture was concentrated to provide the title compound (1.365 g, 100%). MS (APCI) *m*/*z* 173 (M+H)⁺.

### Example 27C: tert-butyl (3-(2-(4-chloro-3-fluorophenoxy)acetamido)bicyclo[1.1.1]pentan-1-yl)carbamate

To solution of 2-(4-chloro-3-fluorophenoxy)acetic acid (6.09 g, 29.8 mmol) and *tert-*butyl (3-aminobicyclo[1.1.1]pentan-1-yl)carbamate (Pharmablock, 5.9 g, 29.8 mmol) in *N,N-*dimethylformamide (70 mL) was added N,N-diisopropylethylamine (15.59 mL, 89 mmol) and 2-(7-aza-1*H*-benzotriazole-1-yl)-1,1,3,3-tetramethyluronium hexafluorophosphate (16.97 g, 44.6 mmol) under nitrogen. The resulting mixture was stirred at ambient temperature for 12 hours, diluted with water (300 mL), and extracted with ethyl acetate (3 x 200 mL). The combined organic layer was washed with brine (3 x 100 mL), dried (Na₂SO₄), filtered, and concentrated under reduced pressure. The residue was treated with methyl *tert-*butyl ether (15 mL), and the resultant solid was dried under high vacuum to provide 6.07 g (53%) of the title compound as a white solid. MS (APCI) *m*/*z* 385 (M+H)⁺.

### Example 27D: N-(3-aminobicyclo[1.1.1]pentan-1-yl)-2-(4-chloro-3-fluorophenoxy)acetamide

To solution of Example 27C (9 g, 23.39 mmol) in dichloromethane (100 mL) was added trifluoroacetic acid (30 mL, 389 mmol) at 0 °C. The mixture was stirred at ambient temperature for 12 hours. The mixture was concentrated under reduced pressure, and the residue was diluted with water (300 mL). The aqueous phase was adjusted to pH= 8 with NaHCO₃ and then extracted with dichloromethane (4 x 150 mL). The combined organic layer was dried (Na₂SO₄) and concentrated under reduced pressure to provide 6 g (90%) of the title compound as a white solid. MS (APCI) *m*/*z* 285 (M+H)⁺

### Example 27E: 2-(4-chloro-3-fluorophenoxy)-N-[3-[2-(4-chloro-3-methoxyphenoxy)acetamido]-bicyclo[1.1.1]pentan-1-yl}acetamide

To solution of Example 27B (0.037 g, 0.171 mmol) and Example 27D (0.05 g, 0.156 mmol) in *N*,*N*-dimethylformamide (1 mL) was added *N*,*N*-diisopropylethylamine (0.07 mL, 0.39 mmol) and 2-(7-aza-1*H*-benzotriazole-1-yl)-1,1,3,3-tetramethyluronium hexafluorophosphate (0.065 g, 0.171 mmol) under nitrogen. The resulting mixture was stirred at ambient temperature for 12 hours and concentrated. The residue was taken into methanol (1 mL)/dimethyl sulfoxide (1 mL), and the precipitate was collected by filtration. The precipitate was washed with methanol (2 mL) and dried under high vacuum to provide the title compound (0.02 g, 27%) as a white solid. ¹H NMR (400 MHz, DMSO-*d*₆) *δ* ppm 8.71 (s, 1H), 8.69 (s, 1H), 7.50 (t, *J =* 8.8 Hz, 1H), 7.31 (d, *J =* 8.7 Hz, 1H), 7.07 (dd, *J =* 11.4, 2.9 Hz, 1H), 6.85 (dd, *J =* 8.9, 2.7 Hz, 1H), 6.76 (d, *J =* 2.7 Hz, 1H), 6.54 (dd, *J =* 8.7, 2.7 Hz, 1H), 4.48 (s, 2H), 4.44 (s, 2H), 3.83 (s, 3H), 2.27 (s, 6H). MS (APCI) *m*/*z* 484 (M+H)⁺.

### Example 28: 2-(4-chloro-3-fluorophenoxy)-N-(3-{2-[4-(methanesulfonyl)-phenoxy]acetamido}-bicyclo[1.1.1]pentan-1-yl)acetamide (Compound 127)

### Example 28A: 2-chloro-N-(3-(2-(4-chloro-3-fluorophenoxy)acetamido)bicyclo[1.1.1]pentan-1-yl)acetamide

A solution of Example 27D (0.93 g, 2.90 mmol) in tetrahydrofuran (5 mL) and water (5 mL) was treated with potassium carbonate (1 g, 7.24 mmol), cooled to 0 °C and treated with 2-chloroacetyl chloride (0.254 mL, 3.19 mmol). The reaction mixture was stirred at 25 °C for 2 hours and filtered. The precipitate was washed with water and dried in a vacuum oven to provide the title compound (0.853 g, 82%). MS (APCI) *m*/*z* 362 (M+H)⁺.

### Example 28B: 2-(4-chloro-3-fluorophenoxy)-N-(3-{2-[4-(methanesulfonyl)phenoxy]acetamido}bicyclo[1.1.1]pentan-1-yl)acetamide

A suspension of Example 28A (0.03 g, 0.083 mmol), 4-(methylsulfonyl)phenol (28.6 mg, 0.166 mmol), potassium carbonate (0.023 g, 0.166 mmol) and potassium iodide (0.002 g, 0.009 mmol) in acetonitrile (1 mL) was heated in microwave reactor (Personal Chemistry, 300 W) oven at 140 °C for 45 minutes. The reaction mixture was filtered, washed with acetonitrile and the filtrate was concentrated. The residue was purified on HPLC (Phenomenex^{®} Luna^{®} C18(2) 5 µm 100 Å AXIA^{™} column 250 mm × 21.2 mm, flow rate 25 mL/minute, 10-80% gradient of acetonitrile in buffer (0.1% trifluoroacetic acid in water)) to provide 0.021 g (51%) of the title compound. ¹H NMR (400 MHz, DMSO-*d*₆) *δ* ppm 8.77 (s, 1H), 8.71 (s, 1H), 7.92 - 7.81 (m, 2H), 7.49 (t, *J = 8.9* Hz, 1H), 7.19 - 7.14 (m, 1H), 7.07 (dd, *J =* 11.4, 2.8 Hz, 2H), 6.85 (ddd, *J* = 9.0, 2.9, 1.2 Hz, 1H), 4.57 (s, 2H), 4.48 (s, 2H), 3.16 (s, 3H), 2.27 (s, 6H). MS (APCI) *m*/*z* 498 (M+H)⁺.

### Example 29: 2-(4-chloro-3-fluorophenoxy)-N-(3-{2-[(6-methylpyridin-2-yl)methoxy]acetamido}bicyclo[1.1.1]pentan-1-yl)acetamide (Compound 128)

A solution of (6-methylpyridin-2-yl) methanol (39 mg, 6.5 equivalent, 0.31 mmol) in dimethylformamide (0.3 mL) was treated with sodium hydride (10 mg, 0.36 mmol), and the mixture was stirred for 30 minutes. A solution of Example 28A (18 mg, 0.048 mmol) in dimethylformamide (0.250 mL) was added to the mixture. The reaction mixture was stirred for 1 hour, filtered and concentrated to dryness. The residue was dissolved in 1:1 dimethyl sulfoxide/methanol and purified by reverse phase HPLC (2-coupled C8 5 µm 100 Å columns 30 mm × 75 mm each, flow rate of 50 mL/minute, 5-90% gradient of acetonitrile in buffer (0.1% trifluoroacetic acid in water)) to provide the title compound. ¹H NMR (500 MHz, DMSO-*d*₆) *δ* ppm 8.34 (t, J = 7.9 Hz, 1H), 7.77 (dd, J = 22.4, 7.9 Hz, 2H), 7.47 (t, J = 8.9 Hz, 1H), 7.04 (dd, J = 11.3, 2.8 Hz, 1H), 6.84 (ddd, J = 9.0, 2.9, 1.2 Hz, 1H), 4.82 (s, 2H), 4.45 (s, 2H), 4.04 (s, 2H), 2.69 (s, 3H), 2.26 (s, 6H). MS (APCI+) *m*/*z* 448.3 (M+H)⁺.

### Example 30: 2-(4-chloro-3-fluorophenoxy)-N-(3-{2-[(3-fluorophenyl)methoxy]acetamido}-bicyclo[1.1.1]pentan-1-yl)acetamide (Compound 129)

The title compound was prepared according to the method described in Example 29 replacing (6-methylpyridin-2-yl) methanol with (3-fluorophenyl)methanol (40 mg, 0.31 mmol). ¹H NMR (500 MHz, DMSO-*d*₆) *δ* ppm 8.79 (s, 1H), 8.47 (s, 1H), 7.47 (t, J = 8.9 Hz, 1H), 7.43 - 7.35 (m, 1H), 7.24 - 7.16 (m, 2H), 7.16 - 7.07 (m, 1H), 7.04 (dd, J = 11.3, 2.9 Hz, 1H), 6.84 (ddd, J = 9.0, 2.9, 1.2 Hz, 1H), 4.53 (s, 2H), 4.45 (s, 2H), 3.85 (s, 2H), 2.24 (s, 6H). MS (APCI+) *m*/*z* 451.3 (M+H)⁺.

### Example 31: 2-(4-chloro-3-fluorophenoxy)-N-(3-{2-[(4-chlorophenyl)methoxy]acetamido}-bicyclo[1.1.1]pentan-1-yl)acetamide (Compound 130)

The title compound was prepared according to the method described in Example 29 replacing (6-methylpyridin-2-yl) methanol with (4-chlorophenyl)methanol (45 mg, 0.31 mmol). ¹H NMR (500 MHz, DMSO-*d*₆) *δ* ppm 7.48 (d, J = 8.8 Hz, 1H), 7.46 - 7.36 (m, 4H), 7.11 - 7.03 (m, 1H), 6.90 - 6.83 (m, 1H), 4.50 (s, 2H), 4.45 (s, 2H), 3.83 (s, 2H), 2.24 (s, 6H). MS (APCI+) *m*/*z* 467.3 (M+H)⁺.

### Example 32: 2-(4-chloro-3-fluorophenoxy)-N-(3-{2-[(3-chlorophenyl)methoxy]acetamido}-bicyclo[1.1.1]pentan-1-yl)acetamide (Compound 131)

The title compound was prepared according to the method described in Example 29 replacing (6-methylpyridin-2-yl) methanol with (3-chlorophenyl)methanol (45 mg, 0.31 mmol). ¹H NMR (500 MHz, DMSO-*d*₆) *δ* ppm 7.51 - 7.28 (m, 5H), 7.04 (dd, J = 11.3, 2.8 Hz, 1H), 6.84 (ddd, J = 8.9, 2.9, 1.2 Hz, 1H), 4.51 (s, 2H), 4.45 (s, 2H), 3.85 (s, 2H), 2.24 (s, 6H). MS (APCI+) *m*/*z* 467.3 (M+H)⁺.

### Example 33: 2-(4-chloro-3-fluorophenoxy)-N-(3-{2-[2-(3-fluorophenyl)ethoxy]acetamido}-bicyclo[1.1.1]pentan-1-yl)acetamide (Compound 132)

The title compound was prepared according to the method described in Example 29 replacing (6-methylpyridin-2-yl) methanol with 2-(3-fluorophenyl)ethanol (45 mg, 0.31 mmol). ¹H NMR (500 MHz, DMSO-*d*₆) *δ* ppm 7.47 (t, J = 8.9 Hz, 1H), 7.32 (td, J = 8.1, 6.4 Hz, 1H), 7.12 - 6.97 (m, 4H), 6.84 (ddd, J = 9.0, 2.9, 1.2 Hz, 1H), 4.45 (s, 2H), 3.80 (s, 2H), 3.64 (t, J = 6.7 Hz, 2H), 2.85 (t, J = 6.6 Hz, 2H), 2.22 (s, 6H). MS (APCI+) *m*/*z* 465.3 (M+H)⁺.

### Example 34: 2-(4-chloro-3-fluorophenoxy)-N-(3-{2-[2-(3-chlorophenyl)ethoxy]acetamido}-bicyclo[1.1.1]pentan-1-yl)acetamide (Compound 133)

The title compound was prepared according to the method described in Example 29 replacing (6-methylpyridin-2-yl) methanol with 2-(3-chlorophenyl)ethanol (49 mg, 0.31 mmol). ¹H NMR (500 MHz, DMSO-*d*₆) *δ* ppm 7.48 (d, J = 8.9 Hz, 1H), 7.32 (dd, J = 4.8, 2.9 Hz, 2H), 7.32 - 7.22 (m, 2H), 7.09 - 7.03 (m, 1H), 6.84 (ddd, J = 8.9, 2.9, 1.2 Hz, 1H), 4.45 (s, 2H), 3.79 (s, 2H), 3.64 (d, J = 6.7 Hz, 2H), 2.85 (d, J = 6.6 Hz, 1H), 2.22 (s, 6H). MS (APCI+) *m*/*z* 481.3 (M+H)⁺.

### Example 35: 2-(4-chloro-3-fluorophenoxy)-N-(3-{2-[2-(4-fluorophenyl)ethoxy]acetamido}-bicyclo[1.1.1]pentan-1-yl)acetamide (Compound 134)

The title compound was prepared according to the method described in Example 29 replacing (6-methylpyridin-2-yl) methanol with 2-(4-fluorophenyl)ethanol (44 mg, 0.31 mmol). ¹H NMR (500 MHz, DMSO-*d*₆) *δ* ppm 7.48 (d, J = 8.8 Hz, 1H), 7.35 - 7.25 (m, 2H), 7.17 - 7.09 (m, 1H), 7.08 - 7.02 (m, 1H), 6.91 - 6.83 (m, 1H), 4.45 (s, 2H), 3.80 (s, 2H), 3.62 (d, J = 6.8 Hz, 2H), 2.83 (d, J = 6.7 Hz, 1H), 2.21 (s, 6H). MS (APCI+) *m*/*z* 465.3 (M+H)⁺.

### Example 36: 2-(4-chloro-3-fluorophenoxy)-N-(3-{2-[2-(4-chlorophenyl)ethoxy]acetamido}-bicyclo[1.1.1]pentan-1-yl)acetamide (Compound 135)

The title compound was prepared according to the method described in Example 29 replacing (6-methylpyridin-2-yl) methanol with 2-(4-chlorophenyl)ethanol (49 mg, 0.31 mmol). ¹H NMR (500 MHz, DMSO-*d*₆) *δ* ppm 7.48 (d, J = 8.9 Hz, 1H), 7.33 (d, J = 2.1 Hz, 1H), 7.31 - 7.26 (m, 2H), 7.05 (d, J = 2.8 Hz, 1H), 6.89 - 6.83 (m, 1H), 4.45 (s, 2H), 3.79 (s, 2H), 3.62 (d, J = 6.7 Hz, 2H), 2.83 (d, J = 6.7 Hz, 2H), 2.21 (s, 6H). MS (APCI+) *m*/*z* 481.3 (M+H)⁺.

### Example 37: 2-(4-chloro-3-fluorophenoxy)-N-(3-{2-[2-(3-methylphenyl)ethoxy]acetamido}-bicyclo[1.1.1]pentan-1-yl)acetamide (Compound 136)

The title compound was prepared according to the method described in Example 29 replacing (6-methylpyridin-2-yl) methanol with 2-(*m*-tolyl)ethanol (43 mg, 0.31 mmol). ¹H NMR (500 MHz, DMSO-*d*₆) *δ* ppm 7.48 (d, J = 8.9 Hz, 1H), 7.17 (d, J = 7.5 Hz, 1H), 7.09 - 7.01 (m, 4H), 6.91 - 6.83 (m, 1H), 4.45 (s, 2H), 3.79 (s, 2H), 3.62 (d, J = 7.0 Hz, 2H), 2.79 (d, J = 6.9 Hz, 2H), 2.26 (d, J = 0.7 Hz, 3H), 2.21 (s, 6H). MS (APCI+) *m*/*z* 461.3 (M+H)⁺.

### Example 38: 2-(4-chloro-3-fluorophenoxy)-N-(3-{2-[2-(4-methylphenyl)ethoxy]acetamido}-bicyclo[1.1.1]pentan-1-yl)acetamide (Compound 137)

The title compound was prepared according to the method described in Example 29 replacing (6-methylpyridin-2-yl) methanol with 2-(*p*-tolyl)ethanol (43 mg, 0.31 mmol). ¹H NMR (500 MHz, DMSO-*d*₆) *δ* ppm 7.48 (d, J = 8.9 Hz, 1H), 7.11 (dd, J = 8.7, 6.3 Hz, 4H), 7.08 - 7.03 (m, 1H), 6.88 - 6.83 (m, 1H), 4.45 (s, 2H), 3.80 (s, 2H), 3.59 (t, J = 6.9 Hz, 2H), 2.78 (d, J = 6.9 Hz, 2H), 2.25 (s, 3H), 2.23 (s, 1H), 2.20 (s, 6H). MS (APCI+) *m*/*z* 461.3 (M+H)⁺.

### Example 39: 2-(4-chloro-3-fluorophenoxy)-N-(3-{2-[(3-cyanophenyl)methoxy]acetamido}-bicyclo[1.1.1]pentan-1-yl)acetamide (Compound 138)

The title compound was prepared according to the method described in Example 29 replacing (6-methylpyridin-2-yl) methanol with 3-(hydroxymethyl)benzonitrile (42 mg, 0.31 mmol). ¹H NMR (500 MHz, DMSO-*d*₆) *δ* ppm 7.82 (td, J = 1.7, 0.7 Hz, 1H), 7.76 (d, J = 1.3 Hz, 1H), 7.75 - 7.70 (m, 1H), 7.57 (td, J = 7.7, 0.5 Hz, 1H), 7.48 (d, J = 8.9 Hz, 1H), 7.08 - 7.03 (m, 1H), 6.85 (dd, J = 2.9, 1.2 Hz, 1H), 6.83 (dd, J = 2.9, 1.2 Hz, 1H), 4.57 (s, 2H), 4.45 (s, 2H), 3.88 (s, 2H), 2.24 (s, 6H). MS (APCI+) *m*/*z* 458.3 (M+H)⁺.

### Example 40: 2-(4-chloro-3-fluorophenoxy)-N-(3-{2-[(4-cyanophenyl)methoxy]acetamido}-bicyclo[1.1.1]pentan-1-yl)acetamide (Compound 139)

The title compound was prepared according to the method described in Example 29 replacing (6-methylpyridin-2-yl) methanol with 4-(hydroxymethyl)benzonitrile (42 mg, 0.31 mmol). ¹H NMR (500 MHz, DMSO-*d*₆) *δ* ppm 7.87 - 7.79 (m, 2H), 7.57 (h, J = 1.2 Hz, 2H), 7.48 (d, J = 8.9 Hz, 1H), 7.04 (dd, J = 11.3, 2.9 Hz, 1H), 6.92 - 6.83 (m, 1H), 4.61 (s, 2H), 4.45 (s, 2H), 3.88 (s, 2H), 2.24 (s, 6H). MS (APCI+) *m*/*z* 458.3 (M+H)⁺.

### Example 41: 2-(4-chloro-3-fluorophenoxy)-N-(3-{2-[(3-methylphenyl)methoxy]acetamido}-bicyclo[1.1.1]pentan-1-yl)acetamide (Compound 140)

The title compound was prepared according to the method described in Example 29 replacing (6-methylpyridin-2-yl) methanol with m-tolylmethanol (38 mg, 0.31 mmol). ¹H NMR (500 MHz, DMSO-*d*₆) *δ* ppm 7.48 (d, J = 8.9 Hz, 1H), 7.24 (d, J = 7.5 Hz, 1H), 7.19 - 7.11 (m, 3H), 7.05 (d, J = 2.9 Hz, 1H), 6.88 - 6.83 (m, 1H), 4.46 (s, 2H), 3.81 (s, 2H), 2.29 (d, J = 0.7 Hz, 3H), 2.24 (s, 6H). MS (APCI+) *m*/*z* 447.3 (M+H)⁺.

### Example 42: 2-(4-chloro-3-fluorophenoxy)-N-[3-(2-{[3-(dimethylamino)phenyl]methoxy}-)bicyclo[1.1.1]pentan-1-yl]acetamide (Compound 141)

The title compound was prepared according to the method described in Example 29 replacing (6-methylpyridin-2-yl) methanol with (3-(dimethylamino)phenyl)methanol (47 mg, 0.31 mmol). ¹H NMR (400 MHz, DMSO-*d*₆) *δ* ppm 7.47 (t, J = 8.9 Hz, 1H), 7.16 (t, J = 7.8 Hz, 1H), 7.04 (dd, J = 11.3, 2.8 Hz, 1H), 6.85 (ddd, J = 9.0, 2.9, 1.2 Hz, 1H), 6.71 - 6.59 (m, 3H), 4.45 (d, J = 1.7 Hz, 4H), 3.80 (s, 2H), 2.87 (s, 6H), 2.24 (s, 6H). MS (APCI+) *m*/*z* 476.3 (M+H)⁺.

### Example 43: 2-(4-chloro-3-fluorophenoxy)-N-(3-{2-[(4-fluorophenyl)methoxy]acetamido}-bicyclo[1.1.1]pentan-1-yl)acetamide (Compound 142)

The title compound was prepared according to the method described in Example 29 replacing (6-methylpyridin-2-yl) methanol with (4-fluorophenyl)methanol (40 mg, 0.31 mmol). ¹H NMR (400 MHz, DMSO-*d*₆) *δ* ppm 7.47 (t, J = 8.9 Hz, 1H), 7.45 - 7.39 (m, 2H), 7.23 - 7.14 (m, 2H), 7.04 (dd, J = 11.3, 2.9 Hz, 1H), 6.85 (ddd, J = 9.0, 2.9, 1.2 Hz, 1H), 4.49 (s, 2H), 4.46 (s, 2H), 3.83 (s, 2H), 2.25 (s, 6H; ). MS (APCI+) *m*/*z* 451.2 (M+H)⁺.

### Example 44: 2-(4-chloro-3-fluorophenoxy)-N-[3-(2-{2-[4-(dimethylamino)phenyl]ethoxy}-acetamido)bicyclo[1.1.1]pentan-1-yl]acetamide (Compound 143)

The title compound was prepared according to the method described in Example 29 replacing (6-methylpyridin-2-yl) methanol with 2-(4-(dimethylamino)phenyl)ethanol (52 mg, 0.31 mmol). ¹H NMR (400 MHz, DMSO-*d*₆) *δ* ppm 7.48 (t, J = 8.9 Hz, 1H), 7.08 - 7.02 (m, 3H), 6.85 (ddd, J = 9.1, 2.9, 1.2 Hz, 1H), 6.73 - 6.64 (m, 2H), 4.45 (s, 2H), 3.79 (s, 2H), 3.56 (t, J = 7.0 Hz, 2H), 2.83 (s, 6H), 2.71 (t, J = 7.0 Hz, 2H), 2.21 (s, 6H). MS (APCI+) *m*/*z* 490.3 (M+H)⁺.

### Example 45: 2-(4-chloro-3-fluorophenoxy)-N-(3-{2-[(4-methylphenyl)methoxy]acetamido}-bicyclo[1.1.1]pentan-1-yl)acetamide (Compound 144)

The title compound was prepared according to the method described in Example 29 replacing (6-methylpyridin-2-yl) methanol with p-tolylmethanol (39 mg, 0.31 mmol). ¹H NMR (400 MHz, DMSO-*d*₆) *δ* ppm 7.47 (t, J = 8.9 Hz, 1H), 7.27 - 7.23 (m, 1H), 7.18 (s, 1H), 7.08 - 7.03 (m, 1H), 6.88 - 6.84 (m, 1H), 4.46 (d, J = 3.1 Hz, 4H), 3.80 (s, 2H), 2.29 (s, 3H), 2.24 (s, 6H). MS (APCI+) m/z 447.2 (M+H)⁺.

### Example 46: 2-(4-chloro-3-fluorophenoxy)-N-[3-(2-{2-[3-(dimethylamino)phenyl]ethoxy}-acetamido)bicyclo[1.1.1]pentan-1-yl]acetamide (Compound 145)

The title compound was prepared according to the method described in Example 29 replacing (6-methylpyridin-2-yl) methanol with 2-(3-(dimethylamino)phenyl)ethanol (52 mg, 0.31 mmol). ¹H NMR (400 MHz, DMSO-*d*₆) *δ* ppm 7.48 (t, J = 8.9 Hz, 1H), 7.10 (d, J = 7.7 Hz, 1H), 7.08 - 7.02 (m, 1H), 6.89 - 6.84 (m, 1H), 6.62 - 6.49 (m, 3H), 4.45 (s, 2H), 3.80 (s, 2H), 3.62 (t, J = 6.9 Hz, 2H), 2.86 (s, 6H), 2.77 (t, J = 6.9 Hz, 2H), 2.21 (s, 6H). MS (APCI+) *m*/*z* 490.3 (M+H)⁺.

### Example 47: 2-(4-chloro-3-fluorophenoxy)-N-{3-[2-(3-chlorophenoxy)acetamido]-bicyclo[1.1.1]pentan-1-yl}acetamide (Compound 146)

The title compound was prepared using the method described in Example 27E by replacing Example 27B with 2-(3-chlorophenoxy)acetic acid (0.022 g, 0.1 mmol). ¹H NMR (501 MHz, DMSO-*d*₆) *δ* ppm 8.71 (s, 1H), 8.70 (s, 1H), 7.49 (t, *J =* 8.9 Hz, 1H), 7.32 (t, *J =* 8.2 Hz, 1H), 7.10 - 7.04 (m, 1H), 7.02 (ddd, *J* = 7.9*,* 2.0, 0.9 Hz, 2H), 6.93 (ddd, *J =* 8.3, 2.4, 0.9 Hz, 1H), 6.85 (ddd, *J =* 8.9, 2.9, 1.2 Hz, 1H), 4.48 (s, 2H), 4.46 (s, 2H), 2.27 (s, 6H). MS (APCI) *m*/*z* 454.3 (M+H)⁺.

### Example 48: N-{3-[2-(4-chloro-3-fluorophenoxy)acetamido]bicyclo[1.1.1]pentan-1-yl}-3-(3-chlorophenoxy)propanamide (Compound 147)

The title compound was prepared using the method described in Example 27E by replacing Example 27B with 3-(3-chlorophenoxy)propanoic acid (0.024 g, 0.1 mmol). ¹H NMR (501 MHz, DMSO-*d*₆) *δ* ppm 8.69 (s, 1H), 8.57 (s, 1H), 7.49 (t, J = 8.9 Hz, 1H), 7.29 (t, J = 8.4 Hz, 1H), 7.07 (dd, J = 11.4, 2.9 Hz, 1H), 7.03 - 6.94 (m, 2H), 6.87 (dddd, J = 23.3, 9.0, 2.6, 1.1 Hz, 2H), 4.47 (s, 2H), 4.17 (t, J = 6.2 Hz, 2H), 2.23 (s, 6H). MS (APCI) *m*/*z* 468.3 (M+H)⁺.

### Example 49: N-{3-[2-(4-chloro-3-fluorophenoxy)acetamido]bicyclo[1.1.1]pentan-1-yl}-4-(3-chlorophenoxy)butanamide (Compound 148)

The title compound was prepared using the method described in Example 27E by replacing Example 27B with 4-(3-chlorophenoxy)butanoic acid (0.028 g, 0.1 mmol). ¹H NMR (400 MHz, DMSO-*d*₆) *δ* ppm 8.68 (s, 1H), 8.42 (s, 1H), 7.49 (t, J = 8.9 Hz, 1H), 7.34 - 7.26 (m, 2H), 7.07 (dd, J = 11.4, 2.8 Hz, 1H), 6.98 (dd, J = 7.0, 1.2 Hz, 1H), 6.94 - 6.81 (m, 2H), 4.47 (s, 2H), 3.97 (t, J = 6.4 Hz, 2H), 2.21 (s, 2H), 2.18 (s, 6H), 1.89 (t, J = 6.9 Hz, 2H). MS (APCI) *m*/*z* 482.3 (M+H)⁺.

### Example 50: 2-(3,4-dichlorophenoxy)-N-(3-{2-[2-(4-fluorophenyl)ethoxy]acetamido}-bicyclo[1.1.1]pentan-1-yl)acetamide (Compound 149)

### Example 50A: 2-chloro-N-(3-(2-(3,4-dichlorophenoxy)acetamido)bicyclo[1.1.1]pentan-1-yl)acetamide

The title compound was prepared using the method described in Example 28A by replacing Example 27D with Example 22B (1.118 g, 3.31 mmol) to provide the title compound.

### Example 50B: 2-(3,4-dichlorophenoxy)-N-(3-{2-[2-(4-fluorophenyl)ethoxy]acetamido}-bicyclo[1.1.1]pentan-1-yl)acetamide

A solution of 2-(4-fluorophenyl)ethanol (54 mg, 0.39 mmol) in dimethylformamide (0.3 mL)was treated with sodium hydride (18 mg, 0.71 mmol) and stirred for 30 minutes. A solution of Example 50A (22.5 mg, 0.060 mmol) in *N*,*N*-dimethylformamide (0.250 mL) was added to the mixture. The reaction mixture was stirred for 1 hour, filtered and concentrated to dryness. The residue was dissolved in 1:1 dimethyl sulfoxide/methanol and purified by reverse phase HPLC (2-coupled C8 5 µm 100 Å columns 30 mm × 75 mm each, flow rate of 50 mL/minute, 5-90% gradient of acetonitrile in buffer (0.1% trifluoroacetic acid in water)) to provide the title compound. ¹H NMR (400 MHz, DMSO-*d*₆) *δ* ppm 7.53 (d, J = 8.9 Hz, 1H), 7.33 - 7.28 (m, 1H), 7.25 (dd, J = 11.2, 2.6 Hz, 2H), 7.16 - 7.07 (m, 2H), 7.04 - 6.97 (m, 1H), 4.46 (s, 2H), 3.80 (s, 2H), 3.63 (d, J = 6.8 Hz, 2H), 2.83 (t, J = 6.8 Hz, 2H), 2.22 (s, 6H). MS (APCI+) *m*/*z* 481.2 (M+H)⁺.

### Example 51: 2-(3,4-dichlorophenoxy)-N-(3-{2-[2-(3-fluorophenyl)ethoxy]acetamido}-bicyclo[1.1.1]pentan-1-yl)acetamide (Compound 150)

The title compound was prepared according to the method described in Example 50B replacing 2-(4-fluorophenyl)ethanol with 2-(3-fluorophenyl)ethanol (54 mg, 0.39 mmol). ¹H NMR (400 MHz, DMSO-*d*₆) *δ* ppm 7.53 (d, J = 8.9 Hz, 1H), 7.40 - 7.30 (m, 1H), 7.24 (d, J = 2.9 Hz, 1H), 7.14 - 7.07 (m, 2H), 7.07 - 6.96 (m, 2H), 4.46 (s, 2H), 3.80 (s, 2H), 3.65 (t, J = 6.7 Hz, 2H), 2.86 (t, J = 6.6 Hz, 2H), 2.23 (s, 6H). MS (APCI+) *m*/*z* 481.2 (M+H)⁺.

### Example 52: 2-[2-(3-chlorophenyl)ethoxy]-N-{3-[2-(3,4-dichlorophenoxy)acetamido]-bicyclo[1.1.1]pentan-1-yl}acetamide (Compound 151)

The title compound was prepared according to the method described in Example 50B replacing 2-(4-fluorophenyl)ethanol with 2-(3-chlorophenyl)ethanol (60 mg, 0.39 mmol). ¹H NMR (400 MHz, DMSO-*d*₆) *δ* ppm 7.53 (d, J = 8.9 Hz, 1H), 7.37 - 7.27 (m, 2H), 7.29 - 7.19 (m, 3H), 6.98 (dd, J = 9.0, 2.9 Hz, 1H), 4.46 (s, 2H), 3.80 (s, 2H), 3.65 (t, J = 6.6 Hz, 2H), 2.85 (t, J = 6.6 Hz, 2H), 2.23 (s, 6H). MS (APCI+) *m*/*z* 497.1 (M+H)⁺.

### Example 53: 2-(3,4-dichlorophenoxy)-N-(3-{2-[2-(3-methylphenyl)ethoxy]-acetamido}bicyclo[1.1.1]pentan-1-yl)acetamide (Compound 152)

The title compound was prepared according to the method described in Example 50B replacing 2-(4-fluorophenyl)ethanol with 2-(*m*-tolyl)ethanol (52 mg, 0.39 mmol). ¹H NMR (400 MHz, DMSO-*d*₆) *δ* ppm 7.53 (d, J = 9.0 Hz, 1H), 7.24 (d, J = 2.9 Hz, 1H), 7.17 (t, J = 7.5 Hz, 1H), 7.08 - 6.96 (m, 4H), 4.46 (s, 2H), 3.80 (s, 2H), 3.62 (t, J = 6.9 Hz, 2H), 2.80 (t, J = 6.9 Hz, 2H), 2.27 (s, 3H), 2.22 (s, 6H). MS (APCI+) *m*/*z* 477.2 (M+H)⁺.

### Example 54: 2-(3,4-dichlorophenoxy)-N-(3-{2-[(6-methylpyridin-2-yl)methoxy]acetamido}bicyclo[1.1.1]pentan-1-yl)acetamide (Compound 153)

The title compound was prepared according to the method described in Example 50B replacing 2-(4-fluorophenyl)ethanol with (6-methylpyridin-2-yl)methanol (47 mg, 0.39 mmol). ¹H NMR (400 MHz, DMSO-*d*₆) *δ* ppm 8.24 (t, J = 7.9 Hz, 1H), 7.72 (s, 1H), 7.71 - 7.64 (m, 1H), 7.54 (s, 1H), 7.24 (d, J = 2.9 Hz, 1H), 6.98 (dd, J = 9.0, 2.9 Hz, 1H), 4.79 (s, 2H), 4.47 (s, 2H), 4.03 (s, 2H), 3.16 (s, 1H), 2.66 (s, 3H), 2.27 (s, 7H). MS (APCI+) *m*/*z* 464.2 (M+H)⁺.

### Example 55: 2-(3,4-dichlorophenoxy)-N-(3-{2-[2-(4-methylphenyl)ethoxy]acetamido}-bicyclo[1.1.1]pentan-1-yl)acetamide (Compound 154)

The title compound was prepared according to the method described in Example 50B replacing 2-(4-fluorophenyl)ethanol with 2-(p-tolyl)ethanol (53 mg, 0.39 mmol). ¹H NMR (400 MHz, DMSO-*d*₆) *δ* ppm 7.54 (s, 1H), 7.24 (d, J = 2.9 Hz, 1H), 7.16 - 7.07 (m, 4H), 6.98 (dd, J = 8.9, 3.0 Hz, 1H), 4.46 (s, 2H), 3.79 (s, 2H), 3.60 (t, J = 6.9 Hz, 2H), 2.79 (t, J = 6.9 Hz, 2H), 2.26 (s, 3H), 2.21 (s, 6H). MS (APCI+) *m*/*z* 477.3 (M+H)⁺.

### Example 56: 2-[2-(4-chlorophenyl)ethoxy]-N-{3-[2-(3,4-dichlorophenoxy)acetamido]bicyclo-[1.1.1]pentan-1-yl}acetamide (Compound 155)

The title compound was prepared according to the method described in Example 50B replacing 2-(4-fluorophenyl)ethanol with 2-(4-chlorophenyl)ethanol (61 mg, 0.39 mmol). ¹H NMR (400 MHz, DMSO-*d*₆) *δ* ppm 7.53 (d, J = 8.9 Hz, 1H), 7.34 (d, J = 6.3 Hz, 2H), 7.29 (d, J = 2.3 Hz, 2H), 7.24 (s, 1H), 7.01 - 6.97 (m, 1H), 4.46 (s, 2H), 3.79 (s, 2H), 3.63 (t, J = 6.7 Hz, 2H), 2.83 (t, J = 6.7 Hz, 2H), 2.22 (s, 6H). MS (APCI+) *m*/*z* 497.1 (M+H)⁺.

### Example 57: 2-[(4-chlorophenyl)methoxy]-N-{3-[2-(3,4-dichlorophenoxy)acetamido]-bicyclo[1.1.1]pentan-1-yl}acetamide (Compound 156)

The title compound was prepared according to the method described in Example 50B replacing 2-(4-fluorophenyl)ethanol with (4-chlorophenyl)methanol (55 mg, 0.39 mmol). ¹H NMR (400 MHz, DMSO-*d*₆) *δ* ppm 7.53 (d, J = 9.0 Hz, 1H), 7.45 - 7.35 (m, 4H), 7.24 (d, J = 2.9 Hz, 1H), 6.98 (dd, J = 9.0, 2.9 Hz, 1H), 4.49 (d, J = 16.5 Hz, 4H), 3.84 (s, 2H), 2.25 (s, 6H). MS (APCI+) *m*/*z* 483.2 (M+H)⁺.

### Example 58: 2-(3,4-dichlorophenoxy)-N-(3-{2-[(4-fluorophenyl)methoxy]acetamido}-bicyclo[1.1.1]pentan-1-yl)acetamide (Compound 157)

The title compound was prepared according to the method described in Example 50B replacing 2-(4-fluorophenyl)ethanol with (4-fluorophenyl)methanol (49 mg, 0.39 mmol). ¹H NMR (400 MHz, DMSO-*d*₆) *δ* ppm 7.53 (d, J = 9.0 Hz, 1H), 7.45 - 7.38 (m, 2H), 7.24 (d, J = 2.9 Hz, 1H), 7.22 - 7.14 (m, 2H), 6.98 (dd, J = 8.9, 2.9 Hz, 1H), 4.49 (s, 2H), 4.46 (s, 2H), 3.83 (s, 2H), 2.24 (s, 6H). MS (APCI+) *m*/*z* 467.2 (M+H)⁺.

### Example 59: 2-[(3-chlorophenyl)methoxy]-N-{3-[2-(3,4-dichlorophenoxy)acetamido]-bicyclo[1.1.1]pentan-1-yl}acetamide (Compound 158)

The title compound was prepared according to the method described in Example 50B replacing 2-(4-fluorophenyl)ethanol with (3-chlorophenyl)methanol (55 mg, 0.39 mmol). ¹H NMR (400 MHz, DMSO-*d*₆) *δ* ppm 7.54 (s, 1H), 7.49 - 7.32 (m, 3H), 7.24 (d, J = 2.9 Hz, 1H), 6.98 (dd, J = 9.0, 2.9 Hz, 1H), 4.52 (s, 2H), 4.47 (s, 2H), 3.86 (s, 2H), 2.25 (s, 6H). MS (APCI+) *m*/*z* 483.1 (M+H)⁺.

### Example 60: 2-[(4-cyanophenyl)methoxy]-N-{3-[2-(3,4-dichlorophenoxy)acetamido]-bicyclo[1.1.1]pentan-1-yl}acetamide (Compound 159)

The title compound was prepared according to the method described in Example 50B replacing 2-(4-fluorophenyl)ethanol with 4-(hydroxymethyl)benzonitrile (52 mg, 0.39 mmol). ¹H NMR (400 MHz, DMSO-*d*₆) *δ* ppm 7.87 - 7.78 (m, 2H), 7.65 - 7.50 (m, 3H), 7.24 (d, J = 2.9 Hz, 1H), 6.98 (dd, J = 9.0, 2.9 Hz, 1H), 4.62 (s, 2H), 4.47 (s, 2H), 3.89 (s, 2H), 2.25 (s, 6H). MS (APCI+) m/z 474.2 (M+H)⁺.

### Example 61: 2-(3,4-dichlorophenoxy)-N-(3-{2-[(4-methylphenyl)methoxy]-acetamido}bicyclo[1.1.1]pentan-1-yl)acetamide (Compound 160)

The title compound was prepared according to the method described in Example 50B replacing 2-(4-fluorophenyl)ethanol with *p*-tolylmethanol (47 mg, 0.39 mmol). ¹H NMR (400 MHz, DMSO-*d*₆) *δ* ppm 7.53 (d, J = 9.0 Hz, 1H), 7.24 (dd, J = 5.5, 2.5 Hz, 3H), 7.17 (d, J = 7.8 Hz, 2H), 6.98 (dd, J = 9.0, 2.9 Hz, 1H), 4.46 (s, 4H), 3.80 (s, 2H), 2.29 (s, 3H), 2.24 (s, 6H). MS (APCI+) *m*/*z* 463.2 (M+H)⁺.

### Example 62: 2-[(3-cyanophenyl)methoxy]-N-{3-[2-(3,4-dichlorophenoxy)acetamido]bicyclo[1.1.1]pentan-1-yl}acetamide (Compound 161)

The title compound was prepared according to the method described in Example 50B replacing 2-(4-fluorophenyl)ethanol with 3-(hydroxymethyl)benzonitrile (52 mg, 0.39 mmol). ¹H NMR (400 MHz, DMSO-*d*₆) *δ* ppm 7.83 (d, J = 1.5 Hz, 1H), 7.80 - 7.70 (m, 2H), 7.63 - 7.50 (m, 2H), 7.24 (d, J = 2.9 Hz, 1H), 6.98 (dd, J = 9.0, 2.9 Hz, 1H), 4.58 (s, 2H), 4.47 (s, 2H), 3.89 (s, 2H), 2.25 (s, 6H). MS (APCI+) *m*/*z* 474.2 (M+H)⁺.

### Example 63: 2-(3,4-dichlorophenoxy)-N-(3-{2-[(3-methylphenyl)methoxy]-acetamido}bicyclo[1.1.1]pentan-1-yl)acetamide (Compound 162)

The title compound was prepared according to the method described in Example 50B replacing 2-(4-fluorophenyl)ethanol with *m*-tolylmethanol (47 mg, 0.39 mmol). ¹H NMR (400 MHz, DMSO-*d*₆) *δ* ppm 7.53 (d, J = 9.0 Hz, 1H), 7.32 - 7.23 (m, 2H), 7.14 (dd, J = 14.7, 6.7 Hz, 3H), 6.98 (dd, J = 8.9, 2.9 Hz, 1H), 4.47 (d, J = 4.1 Hz, 4H), 3.82 (s, 2H), 2.30 (s, 3H), 2.25 (s, 6H). MS (APCI+) m/z 463.2 (M+H)⁺.

### Example 64: N-{3-[2-(4-chloro-3-fluorophenoxy)acetamido]bicyclo[1.1.1]pentan-1-yl}-3-[(3-chlorophenyl)methoxy]propanamide (Compound 163)

### Example 64A: ethyl 3-((3-chlorobenzyl)oxy)propanoate

A solution of (3-chlorophenyl)methanol (1 g, 7.01 mmol) in tetrahydrofuran (20 mL) was cooled in an ice bath and treated with sodium hydride (0.365 g, 9.12 mmol), after stirring for 1 hour at 0 °C, ethyl 3-bromopropanoate (1.021 mL, 9.12 mmol) was added. The reaction mixture was stirred at 25 °C for 30 minutes, quenched with aqueous saturated ammonium chloride solution and extracted with ethyl acetate. The organic layer was washed with brine (3 x 300 mL), dried (Na₂SO₄), filtered, and concentrated under reduced pressure. The residue was purified by column chromatography on silica gel with 0-30% ethyl acetate in heptane to provide the title compound as a white solid.

### Example 64B: 3-((3-chlorobenzyl)oxy)propanoic acid

A solution of Example 64A (0.51 g, 2.101 mmol) in tetrahydrofuran (7 mL) was treated with sodium hydroxide (10.51 mL, 10.51 mmol) and stirred at 25 °C for 18 hours. The reaction mixture was neutralized with 6 N aqueous HCl. The precipitate was washed with water and dried in a vacuum oven to provide the title compound (0.408 g, 90%)

### Example 64C: N-{3-[2-(4-chloro-3-fluorophenoxy)acetamido]bicyclo[1.1.1]pentan-1-yl}-3-[(3-chlorophenyl)methoxy]propanamide

The title compound was prepared using the method described in Example 27E by replacing Example 27B with Example 64B (0.032 g, 0.1 mmol). ¹H NMR (500 MHz, DMSO-*d₆) δ* ppm 8.68 (s, 1H), 8.47 (s, 1H), 7.49 (t, *J =* 8.9 Hz, 1H), 7.43 - 7.31 (m, 3H), 7.25 (d, *J =* 7.3 Hz, 1H), 7.07 (dd, *J =* 11.3, 2.8 Hz, 1H), 6.91 - 6.82 (m, 1H), 4.47 (s, 2H), 4.46 (s, 2H), 3.62 (t, *J =* 6.3 Hz, 2H), 2.32 (t, *J =* 6.3 Hz, 2H), 2.22 (s, 6H). MS (APCI) *m*/*z* 482 (M+H)⁺.

### Example 65: (3-chlorophenyl)methyl {3-[2-(4-chloro-3-fluorophenoxy)-acetamido]bicyclo[1.1.1]pentan-1-yl}carbamate (Compound 164)

Example 27D (0.1 g, 0.311 mmol) was added to a solution of bis(trichloromethyl) carbonate (0.102 g, 0.342 mmol) in tetrahydrofuran (3 mL) and stirred for 15 minutes. The above solution (1 mL) was added to (3-chlorophenyl)methanol (71.3 mg, 0.500 mmol). The reaction mixture was stirred at 25 °C for 72 hours, concentrated and purified by HPLC (Phenomenex^{®} Luna^{®} C18(2) 5 µm 100 Å AXIA^{™} column 250 mm × 21.2 mm, flow rate 25 mL/minute, 10-80% gradient of acetonitrile in buffer (0.1% trifluoroacetic acid in water)) to provide the title compound (0.027g, 59%). ¹H NMR (501 MHz, DMSO-*d*₆) *δ* ppm 8.69 (s, 1H), 8.06 (s, 1H), 7.49 (t, *J =* 8.9 Hz, 1H), 7.45 - 7.35 (m, 3H), 7.31 (dd, *J =* 7.2*,* 1.9 Hz, 1H), 7.07 (dd, *J =* 11.3, 2.9 Hz, 1H), 6.85 (ddd, *J* = 9.0, 2.9, 1.2 Hz, 1H), 5.00 (s, 2H), 4.47 (s, 2H), 2.18 (s, 6H). MS (APCI) *m*/*z* 454 (M+H)⁺.

### Example 66: 2-(3-chlorophenyl)ethyl {3-[2-(4-chloro-3-fluorophenoxy)-acetamido]bicyclo[1.1.1]pentan-1-yl}carbamate (Compound 165)

The title compound was prepared using the method described in Example 65 by replacing (3-chlorophenyl)methanol with 2-(3-chlorophenyl)ethan-1-ol (0.078 g, 0.5 mmol) (0.029 g, 61%). ¹H NMR (500 MHz, DMSO-*d*₆) *δ* ppm 8.67 (s, 1H), 7.86 (s, 1H), 7.49 (t, *J =* 8.9 Hz, 1H), 7.35 - 7.30 (m, 2H), 7.29 - 7.25 (m, 1H), 7.22 (d, *J =* 7.5 Hz, 1H), 7.06 (dd, *J =* 11.4, 2.8 Hz, 1H), 6.92 - 6.79 (m, 1H), 4.46 (s, 2H), 4.14 (s, 2H), 2.87 (s, 2H), 2.15 (s, 6H). MS (APCI) *m*/*z* 468 (M+H)⁺.

### Example 67: 3-(3-chlorophenyl)propyl {3-[2-(4-chloro-3-fluorophenoxy)acetamido]-bicyclo[1.1.1]pentan-1-yl}carbamate (Compound 166)

The title compound was prepared using the method described in Example 65 by replacing (3-chlorophenyl)methanol with 3-(3-chlorophenyl)propanan-1-ol (0.085 g, 0.5 mmol) (0.031 g, 65%). ¹H NMR (501 MHz, DMSO-*d*₆) *δ* ppm 8.68 (s, 1H), 7.86 (s, 1H), 7.49 (t, *J =* 8.9 Hz, 1H), 7.32 (t, J= 7.8 Hz, 1H), 7.28 (d, *J* = 1.8 Hz, 1H), 7.27 - 7.23 (m, 1H), 7.17 (dt, *J* = 7.6, 1.3 Hz, 1H), 7.07 (dd, *J =* 11.4, 2.8 Hz, 1H), 6.85 (ddd, *J* = 9.0, 2.9, 1.2 Hz, 1H), 4.47 (s, 2H), 3.91 (s, 2H), 2.68 - 2.59 (m, 2H), 2.17 (s, 6H), 1.84 (s, 2H). MS (APCI) *m*/*z* 482 (M+H)⁺.

### Example 68: 3-[(3-chlorophenyl)methoxy]-N-{3-[2-(3,4-dichlorophenoxy)acetamido]-bicyclo[1.1.1]pentan-1-yl}propanamide (Compound 167)

To a solution of Example 64B (9 mg, 0.042 mmol) in *N*,*N*-dimethylformamide (0.3 mL) was added *N*,*N*-diisopropylethylamine (0.017 mL, 0.0.096 mmol), 1-[bis(dimethylamino)methylene]-1*H*-1,2,3-triazolo[4,5-*b*]pyridinium 3-oxid hexafluorophosphate (16 mg, 0.042 mmol) and Example 22B (13 mg, 0.039 mmol). The reaction was stirred at room temperature for 18 hours. The crude reaction was purified by HPLC (Phenomenex^{®} Luna^{®} C18(2) 5 µm 100 Å AXIA^{™} column 250 mm × 21.2 mm, flow rate 25 mL/minute, 10-80% gradient of acetonitrile in buffer (0.1% trifluoroacetic acid in water)) to provide the title compound (14.2 mg, 74%). ¹H NMR (500 MHz, DMSO-*d*₆) *δ* ppm 8.69 (s, 1H), 8.47 (s, 1H), 7.54 (d, *J* = 8.9 Hz, 1H), 7.42 - 7.31 (m, 3H), 7.29 - 7.23 (m, 2H), 6.98 (dd, *J =* 8.9, 2.9 Hz, 1H), 4.48 (s, 2H), 4.46 (s, 2H), 3.62 (t, *J =* 6.3 Hz, 2H), 2.32 (t, *J =* 6.3 Hz, 2H), 2.22 (s, 6H). MS (APCI) *m*/*z* 499 (M+H)⁺.

### Example 69: 4-(3-chlorophenoxy)-N-{3-[2-(3,4-dichlorophenoxy)acetamido]-bicyclo[1.1.1]pentan-1-yl}butanamide (Compound 168)

The title compound was prepared using the method described in Example 68 by replacing Example 64B with 4-(3-chlorophenoxy)butanoic acid (0.009 g, 0.042 mmol) to provide the title compound (14.3 mg, 75%). ¹H NMR (500 MHz, DMSO-*d*₆) *δ* ppm 8.68 (s, 1H), 8.43 (s, 1H), 7.54 (d, *J* = 8.9 Hz, 1H), 7.29 (t, *J =* 8.3 Hz, 1H), 7.26 (d, *J =* 2.9 Hz, 1H), 7.03 - 6.95 (m, 3H), 6.95 - 6.84 (m, 1H), 4.48 (s, 2H), 3.97 (t, *J =* 6.4 Hz, 2H), 2.21 (s, 6H), 2.19 (d, *J =* 7.4 Hz, 2H), 1.89 (t, *J =* 6.9 Hz, 2H). MS (APCI) *m*/*z* 499 (M+H)⁺.

### Example 70: 3-(3-chlorophenoxy)-N-{3-[2-(3,4-dichlorophenoxy)acetamido]bicyclo-[1.1.1]pentan-1-yl}propanamide (Compound 169)

The title compound was prepared using the method described in Example 68 by replacing Example 64B with 3-(3-chlorophenoxy)propanoic acid (0.008 g, 0.042 mmol) to provide the title compound (14.2 mg, 76%). ¹H NMR (500 MHz, DMSO-*d*₆) *δ* ppm 8.69 (s, 1H), 8.57 (s, 1H), 7.54 (d, *J =* 8.9 Hz, 1H), 7.29 (t, *J =* 8.4 Hz, 1H), 7.26 (d, *J =* 2.9 Hz, 1H), 7.04 - 6.95 (m, 3H), 6.94 - 6.85 (m, 1H), 4.48 (s, 2H), 4.17 (t, *J =* 6.2 Hz, 2H), 2.23 (s, 6H); ). MS (APCI) *m*/*z* 485 (M+H)⁺.

### Example 71: 2-(3,4-dichlorophenoxy)-N-[3-(2-{2-[3-(dimethylamino)phenyl]ethoxy}-acetamido)bicyclo[1.1.1]pentan-1-yl]acetamide (Compound 170)

The title compound was prepared according to the method described in Example 50B replacing 2-(4-fluorophenyl)ethanol with 2-(3-(dimethylamino)phenyl)ethanol (63 mg, 0.39 mmol). ¹H NMR (400 MHz, DMSO-*d*₆) *δ* ppm 7.54 (d, J = 8.9 Hz, 1H), 7.25 (d, J = 2.9 Hz, 1H), 7.10 (t, J = 7.8 Hz, 1H), 6.99 (dd, J = 9.0, 2.9 Hz, 1H), 6.63 - 6.51 (m, 3H), 4.48 (s, 2H), 3.81 (s, 2H), 3.64 (t, J = 6.9 Hz, 1H), 2.87 (s, 6H), 2.78 (t, J = 6.9 Hz, 2H), 2.23 (s, 6H). MS (APCI+) *m*/*z* 506.3 (M+H)⁺.

### Example 72: 2-(3-chlorophenoxy)-N-{3-[2-(3,4-dichlorophenoxy)acetamido]-bicyclo[1.1.1]pentan-1-yl}acetamide (Compound 171)

### Example 72A: 2-chloro-N-(3-(2-(3,4-dichlorophenoxy)acetamido)bicyclo[1.1.1]pentan-1-yl)acetamide

The title compound was prepared using the method described in Example 28A by replacing Example 27D with Example 22B (1.118 g, 3.31 mmol) to provide the title compound.

### Example 72B: 2-(3-chlorophenoxy)-N-{3-[2-(3,4-dichlorophenoxy)acetamido]-bicyclo[1.1.1]pentan-1-yl}acetamide

A solution of Example 72A (37.8 mg, 0.1 mmol) in acetonitrile (1 mL) was added to 3-chlorophenol (0.021 mL, 0.200 mmol) and potassium carbonate (27.6 mg, 0.200 mmol). The reaction mixture was stirred at 80 °C for 2hours, concentrated and purified by HPLC (Phenomenex^{®} Luna^{®} C18(2) 5 µm 100 Å AXIA^{™} column 250 mm × 21.2 mm, flow rate 25 mL/minute, 10-80% gradient of acetonitrile in buffer (0.1% trifluoroacetic acid in water)) to provide the title compound (0.033 g, 70%). ¹H NMR (501 MHz, DMSO-*d*₆) *δ* ppm 8.71 (s, 1H), 8.70 (s, 1H), 7.55 (d, *J =* 8.9 Hz, 1H), 7.32 (t, *J =* 8.1 Hz, 1H), 7.26 (d, *J =* 2.9 Hz, 1H), 7.05 (t, *J* = 2.2 Hz, 1H), 7.02 (ddd, *J =* 7.9, 2.0, 0.9 Hz, 1H), 6.99 (dd, *J =* 8.9, 2.9 Hz, 1H), 6.93 (ddd, *J* = 8.4, 2.4, 0.9 Hz, 1H), 4.49 (s, 2H), 4.47 (s, 2H), 2.27 (s, 6H). MS (APCI) *m*/*z* 471 (M+H)⁺. **Example 73: (3-chlorophenyl)methyl** {3-[2-(3,4-dichlorophenoxy)acetamido]-**bicyclo[1.1.1]pentan-1-yl}carbamate (Compound 172)**

Example 22B (0.1 g, 0.296 mmol) was added to a solution of bis(trichloromethyl) carbonate (0.097 g, 0.326 mmol) in tetrahydrofuran (1 mL) and stirred for 15 minutes. This solution (0.25 mL) was added to (3-chlorophenyl)methanol (0.052 g, 0.368 mmol). The reaction mixture was stirred at 25 °C for 18 hours, concentrated and purified by HPLC (Phenomenex^{®} Luna^{®} C18(2) 5 µm 100 Å AXIA^{™} column 250 mm × 21.2 mm, flow rate 25 mL/minute, 10-80% gradient of acetonitrile in buffer (0.1% trifluoroacetic acid in water)) to provide the title compound (0.016 g, 45%). ¹H NMR (501 MHz, DMSO-*d*₆) *δ* ppm 8.69 (s, 1H), 8.06 (s, 1H), 7.54 (d, *J =* 9.0 Hz, 1H), 7.45 - 7.36 (m, 3H), 7.30 (d, *J =* 7.1 Hz, 1H), 7.26 (d, *J =* 2.9 Hz, 1H), 6.98 (dd, *J =* 8.9, 2.9 Hz, 1H), 5.00 (s, 2H), 4.48 (s, 2H), 2.18 (s, 6H). MS (APCI) *m*/*z* 471 (M+H)⁺.

### Example 74: 2-(3-chlorophenyl)ethyl {3-[2-(3,4-dichlorophenoxy)acetamido]-bicyclo[1.1.1]pentan-1-yl}carbamate (Compound 173)

The title compound was prepared using the method described in Example 73 by replacing (3-chlorophenyl)methanol with 2-(3-chlorophenyl)ethan-1-ol (0.058 g, 0.386 mmol) (0.019 g, 53%). ¹H NMR (501 MHz, DMSO-*d*₆) *δ* ppm 8.65 (s, 1H), 7.84 (s, 1H), 7.52 (d, *J =* 8.9 Hz, 1H), 7.33 - 7.29 (m, 2H), 7.26 (ddd, *J =* 8.1, 2.1, 1.2 Hz, 1H), 7.24 (d, *J =* 2.9 Hz, 1H), 7.20 (dt, *J* = 7.5, 1.4 Hz, 1H), 6.96 (dd, *J =* 8.9, 2.9 Hz, 1H), 4.46 (s, 2H), 4.13 (s, 2H), 2.85 (s, 2H), 2.13 (s, 6H). MS (APCI) *m*/*z* 485 (M+H)⁺.

### Example 75: 3-(3-chlorophenyl)propyl {3-[2-(3,4-dichlorophenoxy)acetamido]-bicyclo[1.1.1]pentan-1-yl}carbamate (Compound 174)

The title compound was prepared using the method described in Example 73 by replacing (3-chlorophenyl)methanol with 3-(3-chlorophenyl)propanan-1-ol (0.063 g, 0.386 mmol) (0.0195 g, 53%). ¹H NMR (500 MHz, DMSO-*d*₆) *δ* ppm 8.70 (s, 1H), 7.88 (s, 1H), 7.54 (d, *J =* 8.9 Hz, 1H), 7.32 (t, *J = 7.7* Hz, 1H), 7.28 (t, *J =* 1.8 Hz, 1H), 7.27 - 7.23 (m, 2H), 7.18 (d, *J =* 7.6 Hz, 1H), 6.98 (dd, *J =* 8.9, 2.9 Hz, 1H), 4.48 (s, 2H), 3.91 (s, 2H), 2.70 - 2.58 (m, 2H), 2.17 (s, 6H), 1.84 (s, 2H). MS (APCI) *m*/*z* 499 (M+H)⁺.

### Example 76: 2-(4-chloro-3-fluorophenoxy)-N-[3-(2-{[6-(trifluoromethyl)pyridin-3-yl]oxy}acetamido)bicyclo[1.1.1]pentan-1-yl]acetamide (Compound 175)

### Example 76A: tert-butyl 2-((6-(trifluoromethyl)pyridin-3-yl)oxy)acetate

The title compound was prepared according to the method described in Example 27A replacing 4-chloro-3-methoxyphenol with 6-(trifluoromethyl)pyridin-3-ol (0.8 g, 4.91 mmol). MS (APCI) *m*/*z* 278 (M+H)⁺.

### Example 76B: 2-((6-(trifluoromethyl)pyridin-3-yl)oxy)acetic acid

The title compound was prepared according to the method described in Example 27B replacing *tert*-butyl 2-(4-chloro-3-methoxyphenoxy)acetate with Example 76A (1.32 g, 4.76 mmol). MS (APCI) *m*/*z* 222 (M+H)⁺.

### Example 76C: 2-(4-chloro-3-fluorophenoxy)-N-[3-(2-{[6-(trifluoromethyl)pyridin-3-yl]oxy}acetamido)bicyclo[1.1.1]pentan-1-yl]acetamide

To a solution of Example 76B (7.57 g, 34.2 mmol) and Example 27D (8.85 g, 31.1 mmol) in *N,N*-dimethylformamide (100 mL) was added *N,N*-diisopropylethylamine (13.59 mL, 78.0 mmol) and 2-(7-aza-1*H*-benzotriazole-1-yl)-1,1,3,3-tetramethyluronium hexafluorophosphate (13.02 g, 34.20 mmol). The reaction mixture was stirred for 2 hours at 25 °C. The mixture was diluted with water (500 mL), and the resulting mixture was extracted with ethyl acetate (3 x 400 mL). The combined organic layer was washed with brine (3 x 100 mL), dried (Na₂SO₄), filtered, and concentrated under reduced pressure. The residue was taken into dichloromethane (200 mL), warmed to reflux to dissolve all solids and left at 25 °C for 18 hours to form a solid. The solid was collected by filtration and washed with small amount of dichloromethane to provide the title compound (9.33 g, yield 61.4%). ¹H NMR (400 MHz, DMSO-*d*₆) *δ* ppm 8.81 (s, 1H), 8.72 (s, 1H), 8.47 (d, *J =* 2.8 Hz, 1H), 7.86 (d, *J =* 8.7 Hz, 1H), 7.57 (dd, *J =* 8.8, 2.8 Hz, 1H), 7.49 (t, *J =* 8.9 Hz, 1H), 7.07 (dd, *J =* 11.3, 2.8 Hz, 1H), 6.85 (dd, *J=* 9.0, 2.8 Hz, 1H), 4.67 (s, 2H), 4.48 (s, 2H), 2.27 (s, 6H). MS (APCI) *m*/*z* 489 (M+H)⁺.

### Example 77: N,N'-(bicyclo[2.1.1]hexane-1,4-diyl)bis[2-(4-chlorophenoxy)acetamide] (Compound 176)

Benzyl (4-aminobicyclo[2.1.1]hexan-1-yl)carbamate hydrochloride (MacroChem, 30 mg, 0.106 mmol) was added to trifluoroacetic acid (1 mL, 12.98 mmol) and stirred at 80 °C for 90 minutes. The reaction mixture was cooled to ambient temperature and concentrated in vacuo. To the resulting residue was added *N,N*-dimethylformamide (2.1 mL), triethylamine (0.104 mL, 0.743 mmol), 4-chlorophenoxyacetic acid ( Aldrich, 50 mg, 0.265 mmol) and 1-[bis(dimethylamino)methylene]-1*H*-1,2,3-triazolo[4,5-b]pyridinium 3-oxid hexafluorophosphate (121 mg, 0.318 mmol, HATU) in sequential order. The reaction mixture was then stirred at ambient temperature for 1 hour. The resulting solution was filtered through a glass microfiber frit and purified by preparative HPLC [Waters XBridge^{™} C18 5 µm OBD^{™} column, 30 x 100 mm, flow rate 40 mL/minute, 20-100% gradient of acetonitrile in buffer (0.1% trifluoroacetic acid)] to give the title compound (33mg, 0.073 mmol, 68% yield). ¹H NMR (400 MHz, DMSO-*d*₆) *δ* ppm 8.45 (s, 2H), 7.37 - 7.31 (m, 4H), 7.00 - 6.94 (m, 4H), 4.43 (s, 4H), 2.10 - 2.03 (m, 2H), 1.84 - 1.76 (m, 6H). MS (APCI) *m*/*z* 449 (M+H)⁺.

### Example 78 : N,N'-(bicyclo[2.1.1]hexane-1,4-diyl)bis[2-(4-chloro-3-fluorophenoxy)acetamide] (Compound 177)

The title compound was prepared as described in Example 77, substituting 2-(4-chloro-3-fluorophenoxy)acetic acid (commercially available from Aldlab Chemicals) for 4-chlorophenoxyacetic acid. ¹H NMR (400 MHz, DMSO-*d*₆) *δ* ppm 8.48 (s, 2H), 7.49 (t, J = 8.9 Hz, 2H), 7.06 (dd, J = 11.4, 2.9 Hz, 2H), 6.85 (ddd, J = 9.0, 2.8, 1.2 Hz, 2H), 4.48 (s, 4H), 2.12 - 2.04 (m, 2H), 1.85 - 1.76 (m, 6H). MS (APCI) *m*/*z* 485 (M+H)⁺.

### Example 79: N,N'-(bicyclo[3.1.1]heptane-1,5-diyl)bis[2-(4-chlorophenoxy)acetamide] (Compound 178)

The title compound was prepared as described in Example 77, substituting benzyl (5-aminobicyclo[3.1.1]heptan-1-yl)carbamate hydrochloride (commercially available from Curpys) for benzyl (4-aminobicyclo[2.1.1]hexan-1-yl)carbamate hydrochloride. ¹H NMR (400 MHz, DMSO-*d*₆) *δ* ppm 8.22 (s, 2H), 7.38 - 7.29 (m, 4H), 7.00 - 6.92 (m, 4H), 4.40 (s, 4H), 2.20 - 2.12 (m, 4H), 1.78 (dd, J = 25.2, 6.9 Hz, 6H). MS (APCI) m/z 463 (M+H)⁺.

### Example 80: N,N'-(bicyclo[3.1.1]heptane-1,5-diyl)bis[2-(4-chloro-3-fluorophenoxy)acetamide] (Compound 179)

The title compound was prepared as described in Example 77, substituting benzyl (5-aminobicyclo[3.1.1]heptan-1-yl)carbamate hydrochloride (commercially available from Curpys) for benzyl (4-aminobicyclo[2.1.1]hexan-1-yl)carbamate hydrochloride and 2-(4-chloro-3-fluorophenoxy)acetic acid (commercially available from Aldlab Chemicals) for 4-chlorophenoxyacetic acid. ¹H NMR (400 MHz, DMSO-*d*₆) *δ* ppm 8.25 (s, 2H), 7.48 (t, J = 8.8 Hz, 2H), 7.04 (dd, J = 11.4, 2.9 Hz, 2H), 6.83 (ddd, J = 8.9, 2.9, 1.2 Hz, 2H), 4.44 (s, 4H), 2.21 - 2.12 (m, 4H), 1.87 - 1.68 (m, 6H). MS (ESI⁺) *m*/*z* 463 (M+H)⁺.

### Example 81: benzyl {4-[2-(4-chloro-3-fluorophenoxy)acetamido]bicyclo[2.1.1]hexan-1-yl}carbamate (Compound 180)

*N,N*-Dimethylformamide (9.9 mL), triethylamine (0.97 mL, 6.93 mmol) and 1-[bis(dimethylamino)methylene]-1*H*-1,2,3-triazolo[4,5-b]pyridinium 3-oxid hexafluorophosphate (0.489 g, 1.29 mmol, HATU) were added to a mixture of benzyl (4-aminobicyclo[2.1.1]hexan-1-yl)carbamate hydrochloride (MacroChem, 0.28 g, 0.99 mmol) and 2-(4-chloro-3-fluorophenoxy)acetic acid (Aldlab Chemicals, 0.223 g, 1.09 mmol) in sequential order. The reaction mixture was then stirred at ambient temperature for 1 hour. The resulting solution was filtered through a glass microfiber frit and purified by preparative HPLC [Waters XBridge^{™} C18 5 µm OBD^{™} column, 30 x 100 mm, flow rate 40 mL/minute, 20-100% gradient of acetonitrile in buffer (0.025 M aqueous ammonium bicarbonate, adjusted to pH 10 with ammonium hydroxide)] to give the title compound (0.36 g, 0.83 mmol, 84% yield). ¹H NMR (400 MHz, DMSO-*d*₆) *δ* ppm 8.45 (s, 1H), 7.77 (s, 1H), 7.49 (t, J = 8.9 Hz, 1H), 7.39 - 7.28 (m, 5H), 7.06 (dd, J = 11.4, 2.8 Hz, 1H), 6.84 (ddd, J = 9.0, 2.9, 1.2 Hz, 1H), 4.99 (s, 2H), 4.47 (s, 2H), 2.14 - 1.95 (m, 2H), 1.83 - 1.65 (m, 6H). MS (DCI) *m*/*z* 450 (M+NH₄)⁺.

### Example 82: benzyl {5-[2-(4-chloro-3-fluorophenoxy)acetamido]bicyclo[3.1.1]heptan-1-yl}carbamate (Compound 181)

The title compound was prepared as described in Example 81, substituting benzyl (5-aminobicyclo[3.1.1]heptan-1-yl)carbamate hydrochloride (commercially available from Curpys) for benzyl (4-aminobicyclo[2.1.1]hexan-1-yl)carbamate hydrochloride. ¹H NMR (501 MHz, DMSO-*d*₆) *δ* ppm 8.25 (s, 1H), 7.52 - 7.45 (m, 2H), 7.38 - 7.28 (m, 5H), 7.05 (dd, J = 11.4, 2.9 Hz, 1H), 6.83 (ddd, J = 8.9, 2.9, 1.2 Hz, 1H), 4.97 (s, 2H), 4.44 (s, 2H), 2.25 - 2.14 (m, 2H), 2.08 - 2.01 (m, 2H), 1.83 - 1.69 (m, 6H). MS (DCI) *m*/*z* 464 (M+NH₄)⁺.

### Example 83: 2-(4-chloro-3-fluorophenoxy)-N-(3-{2-[(pyrazolo[1,5-a]pyrimidin-5-yl)oxy]acetamidolbicyclo[1.1.1]pentan-1-yl)acetamide (Compound 182)

### Example 83A : tert-butyl 2-(pyrazolo[1,5-a]pyrimidin-5-yloxy)acetate

To a solution of pyrazolo[1,5-a]pyrimidin-5-ol (Ark Pharm, 0.25 g, 1.85 mmol) and *tert-*butyl bromoacetate (Combi-Blocks, 0.41 mL, 2.78 mmol) in *N,N-*dimethylformamide (5.0 mL) was added potassium bis(trimethylsilyl)amide (Aldrich, 1.0 M solution in tetrahydrofuran, 3.33 mL). After stirring at ambient temperature for 10 minutes, 30 grams of silica gel was added, and the resulting suspension was concentrated under reduced pressure to a free flowing powder, and the powder was directly purified via flash chromatography (SiO₂, 25-100% ethyl acetate in heptane) to give the title compound (0.26 g, 1.04 mmol, 56% yield). MS (ESI⁺) *m*/*z 250* (M+H)⁺.

### Example 83B: 2-(pyrazolo[1,5-a]pyrimidin-5-yloxy)acetic acid

Trifluoroacetic acid (2.0 mL) was added to the product of Example 83A (0.25g, 1.0 mmol). The resulting mixture was stirred at ambient temperature for 18 hours and then concentrated in vacuo to give the title compound (0.2 g, 1.0 mmol, 100% yield). MS (ESI⁺) *m*/*z* 194 (M+H)⁺.

### Example 83C: 2-(4-chloro-3-fluorophenoxy)-N-(3-{2-[(pyrazolo[1,5-a]pyrimidin-5-yl)oxy]acetamido}bicyclo[1.1.1]pentan-1-yl)acetamide

The product of Example 9A (35 mg, 0.091 mmol) was dissolved in trifluoroacetic acid (1.0 mL, 12.98 mmol) and stirred at ambient temperature for 1 hour. The resulting solution was concentrated under reduced pressure and to the residue was added the product of Example 83B (19.3 mg, 0.10 mmol), N,N-dimethylformamide (3.0 mL), triethylamine (0.076 mL, 0.546 mmol) and 1-[bis(dimethylamino)methylene]-1H-1,2,3-triazolo[4,5-b]pyridinium 3-oxid hexafluorophosphate (48 mg, 0.13 mmol, HATU) in sequential order. After stirring at ambient temperature for 1 hour, the reaction mixture was filtered through a glass microfiber frit and purified by preparative HPLC [Waters XBridge^{™} C18 5 µm OBD^{™} column, 50 x 100 mm, flow rate 90 mL/minute, 5-100% gradient of acetonitrile in buffer (0.1% trifluoroacetic acid)] to give the title compound (31 mg, 0.067 mmol, 74.1% yield). ¹H NMR (400 MHz, DMSO-*d*₆) *δ* ppm 8.84 (s, 1H), 8.71 (s, 1H), 8.54 (d, J = 7.9 Hz, 1H), 7.80 (d, J = 2.1 Hz, 1H), 7.49 (t, J = 8.9 Hz, 1H), 7.07 (dd, J = 11.3, 2.9 Hz, 1H), 6.84 (ddd, J = 9.0, 2.9, 1.2 Hz, 1H), 6.11 - 6.04 (m, 2H), 4.51 (s, 2H), 4.47 (s, 2H), 2.23 (s, 6H). MS (ESI⁺) m/z 460 (M+H)⁺.

### Example 84: 2-(4-chloro-3-fluorophenoxy)-N-{3-[2-(3,4-difluorophenoxy)acetamido]-bicyclo[1.1.1]pentan-1-yl}acetamide (Compound 183)

The title compound was prepared as described in Example 83C, substituting 2-(3,4-difluorophenoxy)acetic acid (commercially available from Combi-Blocks) for the product of Example 83B. ¹H NMR (400 MHz, DMSO-*d*₆) *δ* ppm 8.72 (s, 1H), 8.70 (s, 1H), 7.49 (t, J = 8.9 Hz, 1H), 7.37 (dt, J = 10.6, 9.3 Hz, 1H), 7.13 - 7.05 (m, 2H), 6.85 (ddd, J = 9.0, 2.9, 1.2 Hz, 1H), 6.80 (dtd, J = 9.2, 3.2, 1.7 Hz, 1H), 4.48 (s, 2H), 4.44 (s, 2H), 2.27 (s, 6H). MS (ESI⁺) *m*/*z* 455 (M+H)⁺.

### Example 85: 2-(4-chloro-3-fluorophenoxy)-N-(3-{2-[(3-methyl-1H-indazol-6-yl)oxy]acetamidolbicyclo[1.1.1]pentan-1-yl)acetamide (Compound 184)

### Example 85A: tert-butyl 2-((3-methyl-1H-indazol-6-yl)oxy)acetate

The title compound was prepared as described in Example 83A, substituting 3-methyl-1*H*-indazol-6-ol (commercially available from Ark Pharm) for pyrazolo[1,5-*a*]pyrimidin-5-ol. MS (ESI⁺) *m*/*z* 263 (M+H)⁺.

### Example 85B : 2-((3-methyl-1H-indazol-6-yl)oxy)acetic acid, trifluoroacetic acid

The title compound was prepared as described in Example 83B, substituting the product of Example 85A for to the product of Example 83A. MS (APCI) *m*/*z* 207 (M+H)⁺.

### Example 85C: 2-(4-chloro-3-fluorophenoxy)-N-(3-{2-[(3-methyl-1H-indazol-6-yl)oxy]acetamido}bicyclo[1.1.1]pentan-1-yl)acetamide

The title compound was prepared using the coupling method described in Example 83C, substituting the product of Example 85B for the product of Example 9A and was purified by preparative HPLC [Waters XBridge^{™} C18 5 µm OBD^{™} column, 30 x 100 mm, flow rate 40 mL/minute, 5-100% gradient of acetonitrile in buffer (carbonic acid buffer prepared by sparging carbon dioxide gas bubbles through deionized water for 15 minutes immediately before use)]. ¹H NMR (500 MHz, DMSO-*d*₆) *δ* ppm 12.39 (s, 1H), 8.71 (s, 1H), 8.71 (s, 1H), 7.56 (d, J = 8.6 Hz, 1H), 7.49 (t, J = 8.9 Hz, 1H), 7.06 (dd, J = 11.4, 2.8 Hz, 1H), 6.85 (ddd, J = 9.0, 2.9, 1.2 Hz, 1H), 6.82 - 6.77 (m, 2H), 4.47 (s, 2H), 4.46 (s, 2H), 2.41 (s, 3H), 2.27 (s, 6H). MS (ESI⁺) *m*/*z* 473 (M+H)⁺.

### Example 86: N,N'-(bicyclo[1.1.1]pentane-1,3-diyl)bis[2-(3,4-difluorophenoxy)acetamide] (Compound 185)

*tert-Butyl* (3-aminobicyclo[1.1.1]pentan-1-yl)carbamate hydrochloride (Combi-Blocks, 20 mg, 0.085 mmol) was combined with dichloromethane (1.0 mL) and stirred at ambient temperature. Trifluoroacetic acid (1.0 mL, 12.98 mmol) was added, and the resulting solution was stirred at ambient temperature for 20 minutes and then concentrated in vacuo. The resulting residue was taken up in N,N-dimethylformamide (2.0 mL). Triethylamine (0.059 mL, 0.43 mmol), 2-(3,4-difluorophenoxy)acetic acid (Combi-Blocks, 35 mg, 0.187 mmol) and 1-[bis(dimethylamino)methylene]-1H-1,2,3-triazolo[4,5-b]pyridinium 3-oxid hexafluorophosphate (78 mg, 0.204 mmol, HATU) were added in sequential order. After stirring at ambient temperature for 20 minutes, the resulting mixture was filtered through a glass microfiber frit and purified by preparative HPLC [custom packed YMC TriArt^{™} C18 Hybrid 20 µm column, 30 x 150 mm, flow rate 70 mL/minute, 5-100% gradient of acetonitrile in buffer (0.1% trifluoroacetic acid)] to give the title compound (31mg, 0.071 mmol, 83% yield). ¹H NMR (400 MHz, DMSO-*d*₆) *δ* ppm 8.69 (s, 2H), 7.37 (dt, J = 10.7, 9.3 Hz, 2H), 7.09 (ddd, J = 12.6, 6.8, 3.0 Hz, 2H), 6.80 (dtd, J = 9.2, 3.3, 1.7 Hz, 2H), 4.44 (s, 4H), 2.27 (s, 6H). MS (ESI⁺) *m*/*z* 439 (M+H)⁺.

### Example 87 N,N'-(bicyclo[1.1.1]pentane-1,3-diyl)bis[2-(4-chloro-3-fluorophenoxy)acetamide] (Compound 186)

The title compound was prepared as described in Example 86, substituting 2-(4-chloro-3-fluorophenoxy)acetic acid (commercially available from Aldlab Chemicals) for 2-(3,4-difluorophenoxy)acetic acid. ¹H NMR (400 MHz, DMSO-*d*₆) *δ* ppm 8.72 (s, 2H), 7.49 (t, J = 8.9 Hz, 2H), 7.07 (dd, J = 11.4, 2.9 Hz, 2H), 6.85 (ddd, J = 9.0, 2.8, 1.2 Hz, 2H), 4.48 (s, 4H), 2.27 (s, 6H). MS (ESI⁻) *m*/*z* 468 (M-H)⁻.

### Example 88: 2-(4-chloro-3-fluorophenoxy)-N-(4-{2-[(1H-indazol-6-yl)oxy]acetamido}bicyclo[2.1.1]hexan-1-yl)acetamide (Compound 187)

### Example 88A: tert-butyl 2-((1H-indazol-5-yl)oxy)acetate

The title compound was prepared as described in Example 83A, substituting 6-hydroxy-1H-indazole (commercially available from Aldrich) for pyrazolo[1,5-*a*]pyrimidin-5-ol. MS (ESI⁺) *m*/*z* 249 (M+H)⁺.

### Example 88B: 2-((1H-indazol-6-yl)oxy)acetic acid, trifluoroacetic acid

The title compound was prepared as described in Example 83B, substituting the product of Example 88A for the product of Example 83A. MS (ESI⁺) *m*/*z* 193 (M+H)⁺.

### Example 88C: N-(4-aminobicyclo[2.1.1]hexan-1-yl)-2-(4-chloro-3-fluorophenoxy)acetamide, trifluoroacetic acid

The product of Example 81 (110 mg, 0.254 mmol) was dissolved in trifluoroacetic acid (2.0 mL, 26.0 mmol) and stirred at 80 °C in a sealed tube for 3 hours. The reaction mixture was cooled to ambient temperature and then concentrated *in vacuo.* The resulting residue was taken up in methanol (3.0 mL) and was filtered through a glass microfiber frit and purified by preparative HPLC [custom packed YMC TriArt^{™} C18 Hybrid 20 µm column, 50 x 150 mm, flow rate 130 mL/minute, 3-60% gradient of acetonitrile in buffer (0.1% trifluoroacetic acid)] to give the title compound (95 mg, 0.230 mmol, 91% yield). MS (ESI⁺) *m*/*z* 299 (M+H)⁺.

### Example 88D: 2-(4-chloro-3-fluorophenoxy)-N-(4-{2-[(1H-indazol-6-yl)oxy]acetamido}bicyclo[2.1.1]hexan-1-yl)acetamide

The title compound was prepared as described in Example 81, substituting the product of Example 88C for benzyl (4-aminobicyclo[2.1.1]hexan-1-yl)carbamate hydrochloride and the product of Example 88B for 2-(4-chloro-3-fluorophenoxy)acetic acid. ¹H NMR (400 MHz, DMSO-*d*₆) *δ* ppm 12.81 (br s, 1H), 8.49 (d, J = 3.2 Hz, 2H), 7.94 (s, 1H), 7.64 (d, J = 8.8 Hz, 1H), 7.49 (t, J = 8.9 Hz, 1H), 7.07 (dd, J = 11.4, 2.9 Hz, 1H), 6.92 - 6.81 (m, 3H), 4.49 (s, 2H), 4.48 (s, 2H), 2.11 - 2.06 (m, 2H), 1.85 - 1.78 (m, 6H). MS (ESI⁺) *m*/*z* 473 (M+H)⁺.

### Example 89: 2-(4-chloro-3-fluorophenoxy)-N-[4-(2-{[6-(trifluoromethyl)pyridin-3-yl]oxy}acetamido)bicyclo[2.1.1]hexan-1-yl]acetamide (Compound 188)

The title compound was prepared as described in Example 81, substituting the product of Example 88C for benzyl (4-aminobicyclo[2.1.1]hexan-1-yl)carbamate hydrochloride and the product of Example 76B for 2-(4-chloro-3-fluorophenoxy)acetic acid. ¹H NMR (400 MHz, DMSO-*d*₆) *δ* ppm 8.58 (s, 1H), 8.48 (s, 1H), 8.46 (d, J = 2.9 Hz, 1H), 7.88 - 7.85 (m, 1H), 7.56 (dd, J = 8.7, 2.9 Hz, 1H), 7.49 (t, J = 8.9 Hz, 1H), 7.07 (dd, J = 11.4, 2.8 Hz, 1H), 6.85 (ddd, J = 9.0, 2.8, 1.2 Hz, 1H), 4.66 (s, 2H), 4.48 (s, 2H), 2.12 - 2.05 (m, 2H), 1.83 - 1.76 (m, 6H). MS (ESI⁺) *m*/*z* 502 (M+H)⁺.

### Example 90: N-(2-{[tert-butyl(dimethyl)silyl]oxy}ethyl)-2-(4-chloro-3-fluorophenoxy)-N-{3-[2-(4-chloro-3-fluorophenoxy)acetamido]bicyclo[1.1.1]pentan-1-yl}acetamide (Compound 189)

### Example 90A: N-{3-[(2-{[tert-butyl(dimethyl)silyl]oxy}ethyl)amino]bicyclo[1.1.1]pentan-1-yl}-2-(4-chloro-3-fluorophenoxy)acetamide

To a mixture of the product of Example 27D (240 mg, 0.76 mmol) and K₂CO₃ (524 mg, 3.79 mmol) was added *N,N*-dimethylformamide (4.0 mL) followed by (2-bromoethoxy)-tert-butyldimethylsilane (0.488 mL, 2.28 mmol). The reaction mixture was stirred at 47 °C for 16 hours, filtered through a glass microfiber frit and purified by preparative HPLC [custom packed YMC TriArt^{™} C18 Hybrid 20 µm column, 30 x 150 mm, flow rate 90 mL/minute, 5-100% gradient of acetonitrile in buffer (0.025 M aqueous ammonium bicarbonate, adjusted to pH 10 with ammonium hydroxide)] to give the title compound (0.14 g, 0.32 mmol, 42% yield). ¹H NMR (501 MHz, DMSO-*d*₆) *δ* ppm 8.60 (s, 1H), 7.48 (t, J = 8.9 Hz, 1H), 7.06 (dd, J = 11.4, 2.8 Hz, 1H), 6.84 (ddd, J = 9.0, 2.8, 1.2 Hz, 1H), 4.45 (s, 2H), 3.57 (t, J = 6.3 Hz, 2H), 2.56 (t, J = 6.3 Hz, 2H), 2.27 (br s, 1H), 1.96 - 1.91 (m, 6H), 0.87 - 0.84 (m, 9H), 0.03 (s, 6H). MS (ESI⁺) *m*/*z* 443 (M+H)⁺.

### Example 90B N-(2-{[tert-butyl(dimethyl)silyl]oxy}ethyl)-2-(4-chloro-3-fluorophenoxy)-N-{3-[2-(4-chloro-3-fluorophenoxy)acetamido]bicyclo[1.1.1]pentan-1-yl}acetamide

The title compound was prepared as described in Example 81, substituting the product of Example 90A for benzyl (4-aminobicyclo[2.1.1]hexan-1-yl)carbamate hydrochloride. ¹H NMR (400 MHz, DMSO-*d*₆, 120 °C) *δ* ppm 8.25 (s, 1H), 7.43 - 7.34 (m, 2H), 6.97 (dd, J = 11.3, 2.8 Hz, 1H), 6.90 (dd, J = 11.3, 2.8 Hz, 1H), 6.83 (ddd, J = 9.0, 2.9, 1.2 Hz, 1H), 6.77 (ddd, J = 8.9, 2.9, 1.2 Hz, 1H), 4.80 (s, 2H), 4.45 (s, 2H), 3.68 (t, J = 6.0 Hz, 2H), 3.43 (t, J = 6.0 Hz, 2H), 2.38 (s, 6H), 0.87 (s, 9H), 0.04 (s, 6H). MS (ESI⁺) *m*/*z* 629 (M+H)⁺.

### Example 91: 2-(4-chloro-3-fluorophenoxy)-N-{3-[2-(4-chloro-3-fluorophenoxy)acetamido]-bicyclo[1.1.1]pentan-1-yl}-N-(2-hydroxyethyl)acetamide (Compound 190)

The product of Example 90 (40 mg, 0.064 mmol) was dissolved in tetrahydrofuran (1.5 mL) and stirred at ambient temperature. Tetra-n-butylammonium fluoride (Aldrich, 1 M solution in tetrahydrofuran, 0.32 mL) was added in one portion. After stirring at ambient temperature for 30 minutes, the reaction mixture was concentrated in vacuo. The residue was taken up in methanol (3.0 mL), filtered through a glass microfiber frit and purified by preparative HPLC [Waters XBridge^{™} C18 5 µm OBD^{™} column, 30 x 100 mm, flow rate 40 mL/minute, 5-100% gradient of acetonitrile in buffer (carbonic acid buffer prepared by sparging carbon dioxide gas bubbles through deionized water for 15 minutes immediately before use)] to give the title compound (16 mg, 0.031 mmol, 48.9% yield). ¹H NMR (501 MHz, DMSO-*d*₆) *δ* ppm 8.83 - 8.71 (m, 1H), 7.49 (t, J = 8.9 Hz, 1H), 7.45 (t, J = 8.9 Hz, 1H), 7.07 (dd, J = 11.4, 2.9 Hz, 1H), 7.01 (dd, J = 11.5, 2.9 Hz, 1H), 6.85 (ddd, J = 9.0, 2.9, 1.2 Hz, 1H), 6.79 (d, J = 8.9 Hz, 1H), 5.21 - 4.80 (m, 3H), 4.48 (s, 2H), 3.58 - 3.49 (m, 2H), 3.40 - 3.33 (m, 2H), 2.45 - 2.28 (m, 6H). MS (ESI⁺) *m*/*z* 515 (M+H)⁺.

### Example 92: N-{3-[2-(4-chloro-3-fluorophenoxy)acetamido]bicyclo[1.1.1]pentan-1-yl}-N-(2-hydroxyethyl)-2-[[6-(trifluoromethyl)pyridin-3-yl]oxylacetamide (Compound 191)

### Example 92A =N-(2-((tert-butyldimethylsilyl)oxy)ethyl)-N-(3-(2-(4-chloro-3-fluorophenoxy)acetamido)bicyclo[1.1.1]pentan-1-yl)-2-((6-(trifluoromethyl)pyridin-3-yl)oxy)acetamide

The title compound was prepared as described in Example 81, substituting the product of Example 90A for benzyl (4-aminobicyclo[2.1.1]hexan-1-yl)carbamate hydrochloride and the product of Example 76B for 2-(4-chloro-3-fluorophenoxy)acetic acid. MS (ESI⁺) *m*/*z* 646 (M+H)⁺.

### Example 92B N-{3-[2-(4-chloro-3-fluorophenoxy)acetamido]bicyclo[1.1.1]pentan-1-yl}-N-(2-hydroxyethyl)-2-{[6-(trifluoromethyl)pyridin-3-yl]oxy}acetamide

The title compound was prepared as described in Example 91, substituting the product of Example 92A for the product of Example 90. ¹H NMR (501 MHz, DMSO-*d*₆) *δ* ppm 8.80 and 8.72 (two s, 1H, amide rotamers), 8.40 - 8.36 (m, 1H), 7.83 - 7.78 (m, 1H), 7.51 - 7.43 (m, 2H), 7.05 (dd, J = 11.4, 2.9 Hz, 1H), 6.86 - 6.80 (m, 1H), 5.20 - 4.99 (m, 3H), 4.52 - 4.41 (m, 2H), 3.60 - 3.48 (m, 2H), 3.44 - 3.30 (m, 2H), 2.46 - 2.24 (m, 6H). MS (ESI⁺) *m*/*z* 515 (M+H)⁺.

### Example 93: benzyl {3-[2-(4-chloro-3-fluorophenoxy)acetamido]bicyclo[1.1.1]pentan-1-yl}carbamate (Compound 192)

Benzyl chloroformate (Aldrich, 50% solution in toluene, 40 mg, 0.12 mmol) was added to a solution of the product of Example 27D (30mg, 0.11 mmol) and Hunig's Base (0.055 mL, 0.32 mmol) in dichloromethane (3.0 mL), and the reaction mixture was stirred at ambient temperature for 1 hour and then concentrated in vacuo. The resulting residue was taken up in methanol (3 mL), filtered through a glass microfiber frit and purified by preparative HPLC [Waters XBridge^{™} C18 5 µm OBD^{™} column, 50 x 100 mm, flow rate 90 mL/minute, 5-100% gradient of acetonitrile in buffer (0.025 M aqueous ammonium bicarbonate, adjusted to pH 10 with ammonium hydroxide)] to give the title compound (27 mg, 0.064 mmol, 61% yield). ¹H NMR (400 MHz, DMSO-*d*₆) *δ* ppm 8.69 (s, 1H), 7.99 (br s, 1H), 7.49 (t, J = 8.9 Hz, 1H), 7.40 - 7.27 (m, 5H), 7.07 (dd, J = 11.4, 2.8 Hz, 1H), 6.85 (ddd, J = 8.9, 2.8, 1.1 Hz, 1H), 5.00 (s, 2H), 4.47 (s, 2H), 2.18 (br s, 6H). MS (ESI⁻) *m*/*z* 417 (M-H)⁻.

### Example 94: benzyl {4-[2-(3,4-dichlorophenoxy)acetamido]bicyclo[2.1.1]hexan-1-yl}carbamate (Compound 193)

The title compound was prepared as described in Example 81, substituting 2-(3,4-dichlorophenoxy)acetic acid (commercially available from Aldrich) for 2-(4-chloro-3-fluorophenoxy)acetic acid. ¹H NMR (500 MHz, DMSO-*d*₆) *δ* ppm 8.47 (s, 1H), 7.79 (br s, 1H), 7.54 (d, J = 8.9 Hz, 1H), 7.40 - 7.29 (m, 5H), 7.25 (d, J = 2.9 Hz, 1H), 6.98 (dd, J = 9.0, 2.9 Hz, 1H), 4.99 (s, 2H), 4.48 (s, 2H), 2.11 - 2.00 (m, 2H), 1.80 - 1.67 (m, 6H). MS (ESI⁻) *m*/*z* 447 (M-H)⁻.

### Example 95: benzyl {5-[2-(3,4-dichlorophenoxy)acetamido]bicyclo[3.1.1]heptan-1-yl}carbamate (Compound 194)

The title compound was prepared as described in Example 81, substituting benzyl (5-aminobicyclo[3.1.1]heptan-1-yl)carbamate hydrochloride (commercially available from Curpys) for benzyl (4-aminobicyclo[2.1.1]hexan-1-yl)carbamate hydrochloride and 2-(3,4-dichlorophenoxy)acetic acid (commercially available from Aldrich) for 2-(4-chloro-3-fluorophenoxy)acetic acid. ¹H NMR (500 MHz, DMSO-*d*₆) *δ* ppm 8.25 (s, 1H), 7.53 (d, J = 8.9 Hz, 1H), 7.49 (br s, 1H), 7.38 - 7.27 (m, 5H), 7.23 (d, J = 2.9 Hz, 1H), 6.96 (dd, J = 9.0, 2.9 Hz, 1H), 4.97 (s, 2H), 4.45 (s, 2H), 2.24 - 2.13 (m, 2H), 2.06 - 2.01 (m, 2H), 1.82 - 1.67 (m, 6H). MS (ESI⁻) *m*/*z* 461 (M-H)⁻.

### Example 96 2-(3,4-dichlorophenoxy)-N-(4-{2-[(4-fluoro-1H-indazol-6-yl)oxy]acetamido}-bicyclo[2.1.1]hexan-1-yl)acetamide (Compound 195)

### Example 96A: N-(4-aminobicyclo[2.1.1]hexan-1-yl)-2-(3,4-dichlorophenoxy)acetamide

The title compound was prepared as described in Example 88C, substituting the product of Example 94 for the product of Example 81 and purified by preparative HPLC [Waters XBridge^{™} C18 5 µm OBD^{™} column, 30 x 100 mm, flow rate 40 mL/minute, 5-100% gradient of acetonitrile in buffer (0.025 M aqueous ammonium bicarbonate, adjusted to pH 10 with ammonium hydroxide)]. MS (ESI⁺) *m*/*z* 315 (M+H)⁺.

### Example 96B : 2-(3,4-dichlorophenoxy)-N-(4-{2-[(4-fluoro-1H-indazol-6-yl)oxy]acetamido)bicyclo[2.1.1]hexan-1-yl)acetamide

The title compound was prepared as described in Example 81, substituting the product of Example 12B for 2-(4-chloro-3-fluorophenoxy)acetic acid, the product of Example 96A for benzyl (4-aminobicyclo[2.1.1]hexan-1-yl)carbamate hydrochloride and Hunig's base for triethylamine. ¹H NMR (400 MHz, DMSO-*d*₆) *δ* ppm 13.08 (br s, 1H), 8.51 (s, 1H), 8.49 (s, 1H), 8.05 (s, 1H), 7.54 (d, J = 8.9 Hz, 1H), 7.25 (d, J = 2.9 Hz, 1H), 6.98 (dd, J = 8.9, 2.9 Hz, 1H), 6.77 (br s, 1H), 6.70 - 6.66 (m, 1H), 4.51 (s, 2H), 4.49 (s, 2H), 2.11 - 2.06 (m, 2H), 1.85 - 1.78 (m, 6H). MS (ESI⁺) *m*/*z* 507 (M+H)⁺.

### Example 97: 2-(3,4-dichlorophenoxy)-N-[4-(2-{[6-(trifluoromethyl)pyridin-3-yl]oxy}acetamido)bicyclo[2.1.1]hexan-1-yl]acetamide (Compound 196)

The title compound was prepared as described in Example 81, substituting the product of Example 96A for benzyl (4-aminobicyclo[2.1.1]hexan-1-yl)carbamate hydrochloride and the product of Example 76B for 2-(4-chloro-3-fluorophenoxy)acetic acid. ¹H NMR (501 MHz, DMSO-*d*₆) *δ* ppm 8.58 (s, 1H), 8.48 (s, 1H), 8.46 (d, J = 2.9 Hz, 1H), 7.86 (d, J = 8.7 Hz, 1H), 7.58 = 7.55 (m, 1H), 7.54 (d, J = 8.9 Hz, 1H), 7.25 (d, J = 2.9 Hz, 1H), 6.98 (dd, J = 9.0, 2.9 Hz, 1H), 4.66 (s, 2H), 4.49 (s, 2H), 2.11 - 2.06 (m, 2H), 1.83 - 1.79 (m, 6H). MS (ESI⁺) *m*/*z* 518 (M+H)⁺.

### Example 98: N-{3-[2-(3-chloro-4-fluorophenoxy)acetamido]bicyclo[1.1.1]pentan-1-yl}-2-(3,4-dichlorophenoxy)-N-methylacetamide (Compound 197)

### Example 98A

To a solution of the product of Example 22A (220 mg, 0.55 mmol) in *N,N-*dimethylacetamide (3.5 mL) was added sodium hydride (60% dispersion in mineral oil, 26 mg, 0.66 mmol) in one portion. Tetrahydrofuran (2.0 mL) was then added. After stirring at ambient temperature for 5 minutes, methyl iodide (0.051 mL, 0.82 mmol) was added in one portion. After stirring for another 10 minutes, methanol (2.0 mL) was added, and the resulting solution was concentrated under reduced pressure to about 3 mL, filtered through a glass microfiber frit and purified by preparative HPLC [Waters XBridge^{™} C18 5 µm OBD^{™} column, 50 x 100 mm, flow rate 90 mL/minute, 35-75% gradient of acetonitrile in buffer (0.1% trifluoroacetic acid)] to give the title compound (0.08 g, 0.19 mmol, 35% yield). ¹H NMR (400 MHz, DMSO-*d*₆, 120 °C) *δ* ppm 7.44 (d, J = 8.9 Hz, 1H), 7.14 (d, J = 2.9 Hz, 1H), 6.97 - 6.88 (m, 2H), 4.75 (s, 2H), 2.86 (s, 3H), 2.25 (s, 6H), 1.39 (d, J = 0.6 Hz, 9H). MS (ESI⁺) *m*/*z* 415 (M+H)⁺.

### Example 98B: N-{3-[2-(3-chloro-4-fluorophenoxy)acetamido]bicyclo[1.1.1]pentan-1-yl}-2-(3,4-dichlorophenoxy)-N-methylacetamide

The title compound was prepared as described in Example 83C, substituting the product of Example 98A for the product of Example 9A and 2-(4-chloro-3-fluorophenoxy)acetic acid (commercially available from Aldlab Chemicals) for the product of Example 83B. ¹H NMR (400 MHz, DMSO-*d*₆, 120 °C) *δ* ppm 8.25 (s, 1H), 7.46 - 7.37 (m, 2H), 7.14 (d, J = 2.9 Hz, 1H), 6.98 (dd, J = 11.3, 2.8 Hz, 1H), 6.92 (dd, J = 8.9, 2.9 Hz, 1H), 6.83 (ddd, J = 9.0, 2.9, 1.3 Hz, 1H), 4.76 (s, 2H), 4.45 (s, 2H), 2.88 (s, 3H), 2.37 (br s, 6H). MS (ESI⁺) m/z 501/503 (M+H)⁺.

### Example 99: 2-(3,4-dichlorophenoxy)-N-{3-[2-(3,4-dichlorophenoxy)acetamido]-bicyclo[1.1.1]pentan-1-yl}-N-methylacetamide (Compound 198)

The title compound was prepared as described in Example 83C, substituting the product of Example 98A for the product of Example 9A and 2-(3,4-dichlorophenoxy)acetic acid (commercially available from Aldrich) for the product of Example 83B. ¹H NMR (400 MHz, DMSO-*d*₆, 120 °C) *δ* ppm 8.27 (s, 1H), 7.49 - 7.40 (m, 2H), 7.20 (d, J = 2.9 Hz, 1H), 7.15 (d, J = 3.0 Hz, 1H), 6.96 (dd, J = 8.9, 2.9 Hz, 1H), 6.92 (dd, J = 8.9, 2.9 Hz, 1H), 4.77 (s, 2H), 4.46 (s, 2H), 2.88 (s, 3H), 2.37 (br s, 6H). MS (ESI⁺) *m*/*z* 519/517 (M+H)⁺.

### Example 100: N-{3-[2-(4-chlorophenoxy)acetamido]bicyclo[1.1.1]pentan-1-yl}-2-(3,4-dichlorophenoxy)-N-methylacetamide (Compound 199)

The title compound was prepared as described in Example 83C, substituting the product of Example 98A for the product of Example 9A and 2-(4-chlorophenoxy)acetic acid (commercially available from Aldrich) for the product of Example 83B. ¹H NMR (400 MHz, DMSO-*d*₆, 120 °C) *δ* ppm 8.21 (s, 1H), 7.44 (d. J = 9.0 Hz, 1H), 7.30 - 7.26 (m, 2H), 7.14 (d, J = 2.9 Hz, 1H), 6.99 - 6.94 (m, 2H), 6.92 (dd, J = 8.9, 2.9 Hz, 1H), 4.76 (s, 2H), 4.41 (s, 2H), 2.88 (s, 3H), 2.37 (br s, 6H). MS (ESI⁺) *m*/*z* 483/485 (M+H)⁺.

### Example 101: N²-(4-chlorophenyl)-N-{3-[2-(3,4-dichlorophenoxy)acetamido]-bicyclo[1.1.1]pentan-1-yl}-N²-methylglycinamide (Compound 200)

The title compound was prepared as described in Example 83C, substituting the product of Example 22A for the product of Example 9A and 2-((4-chlorophenyl)(methyl)amino)acetic acid hydrochloride (commercially available from Enamine) for the product of Example 83B. ¹H NMR (400 MHz, DMSO-*d*₆), *δ* ppm 8.69 (s, 1H), 8.53 (s, 1H), 7.54 (d, J = 8.9 Hz, 1H), 7.25 (d, J = 2.9 Hz, 1H), 7.20 - 7.14 (m, 2H), 6.98 (dd, J = 8.9, 2.9 Hz, 1H), 6.62 - 6.56 (m, 2H), 4.47 (s, 2H), 3.85 (s, 2H), 2.94 (s, 3H), 2.21 (br s, 6H). MS (ESI⁺) *m*/*z* 482/484 (M+H)⁺.

### Example 102: N-{3-[2-(4-chloro-3-fluorophenoxy)acetamido]bicyclo[1.1.1]pentan-1-yl}-N²-(4-chlorophenyl)-N²-methylglycinamide (Compound 201)

The title compound was prepared as described in Example 81, substituting 2-((4-chlorophenyl)(methyl)amino)acetic acid hydrochloride (commercially available from Enamine) for 2-(4-chloro-3-fluorophenoxy)acetic acid and the product of Example 9B for benzyl (4-aminobicyclo[2.1.1]hexan-1-yl)carbamate hydrochloride. ¹H NMR (400 MHz, DMSO-*d*₆, 120 °C) *δ* ppm 8.17 (s, 1H), 8.07 (s, 1H), 7.46 - 7.36 (m, 5H), 6.98 (dd, J = 11.3, 2.8 Hz, 1H), 6.83 (ddd, J = 9.0, 2.9, 1.3 Hz, 1H), 4.43 (s, 2H), 3.88 (s, 2H), 2.92 (s, 3H), 2.25 (br s, 6H). MS (ESI⁺) *m*/*z* 494 (M+H)⁺.

### Example 103: N²-(4-chlorophenyl)-N-{4-[2-(3,4-dichlorophenoxy)acetamido]-bicyclo[2.1.1]hexan-1-yl}-N²-methylglycinamide (Compound 202)

The title compound was prepared as described in Example 81, substituting 2-((4-chlorophenyl)(methyl)amino)acetic acid hydrochloride (commercially available from Enamine) for 2-(4-chloro-3-fluorophenoxy)acetic acid and the product of Example 96A for benzyl (4-aminobicyclo[2.1.1]hexan-1-yl)carbamate hydrochloride. ¹H NMR (501 MHz, DMSO-*d*₆) *δ* ppm 8.43 (s, 1H), 8.29 (s, 1H), 7.52 (d, J = 8.9 Hz, 1H), 7.22 (d, J = 2.9 Hz, 1H), 7.17 - 7.12 (m, 2H), 6.95 (dd, J = 8.9, 2.9 Hz, 1H), 6.61 - 6.56 (m, 2H), 4.46 (s, 2H), 3.83 (s, 2H), 2.93 (s, 3H), 2.04 - 1.99 (m, 2H), 1.77 - 1.71 (m, 6H). MS (ESI⁺) *m*/*z* 496/498 (M+H)⁺.

### Example 104: N-{3-[2-(4-chloro-3-fluorophenoxy)acetamido]bicyclo[1.1.1]pentan-1-yl}-N²-(4-chlorophenyl)glycinamide (Compound 203)

The title compound was prepared as described in Example 81, substituting the product of Example 9B for benzyl (4-aminobicyclo[2.1.1]hexan-1-yl)carbamate hydrochloride and 2-((4-chlorophenyl)amino)acetic acid (commercially available for Enamine) for 2-(4-chloro-3-fluorophenoxy)acetic acid. ¹H NMR (400 MHz, DMSO-*d*₆) *δ* ppm 8.65 (s, 1H), 8.48 (s, 1H), 7.45 (t, J = 8.8 Hz, 1H), 7.09 - 7.00 (m, 3H), 6.81 (ddd, J = 9.0, 2.9, 1.2 Hz, 1H), 6.52 - 6.46 (m, 2H), 5.97 (t, J = 5.9 Hz, 1H), 4.43 (s, 2H), 3.54 (d, J = 5.9 Hz, 2H), 2.19 (br s, 6H). MS (ESI⁺) *m*/*z* 452 (M+H)⁺.

### Example 105: 4-chloro-N-[2-({3-[2-(4-chloro-3-fluorophenoxy)acetamido]-bicyclo[1.1.1]pentan-1-yl}amino)-2-oxoethyl]-N-methylbenzamide (Compound 204)

### Example 105A: methyl 2-(4-chloro-N-methylbenzamido)acetate

4-Chlorobenzoic acid (0.617 g, 3.94 mmol), sarcosine methyl ester hydrochloride (Ark Pharm, 0.55 g, 3.94 mmol) and triethylamine (1.65 mL, 11.8 mmol) were combined with *N,N-*dimethylacetamide (8 mL), and the mixture was stirred at ambient temperature. 1-[Bis(dimethylamino)methylene]-1*H*-1,2,3-triazolo[4,5-*b*]pyridinium 3-oxid hexafluorophosphate (1.798 g, 4.73 mmol, HATU) was added in one portion. After stirring for 1 hour, dimethyl sulfoxide (2 mL) was added, and the resulting solution was filtered through a glass microfiber frit and purified by preparative HPLC [Waters XBridge^{™} C18 5 µm OBD^{™} column, 50 x 100 mm, flow rate 90 mL/minute, 5-45% gradient of acetonitrile in buffer (0.1% trifluoroacetic acid)] to give the title compound (0.30 g, 1.24 mmol, 32% yield). MS (ESI⁺) *m*/*z* 242 (M+H)⁺.

### Example 105B: 2-(4-chloro-N-methylbenzamido)acetic acid

The product of Example 105A (0.46 g, 1.738 mmol) was dissolved in ethanol (30 mL), aqueous sodium hydroxide (2.5 M, 10 mL) was added, and the resulting mixture was stirred at ambient temperature for 20 minutes. The mixture was partitioned between dichloromethane (2 x 100 mL) and aqueous citric acid (10 weight %, 100 mL). The organic layers were combined, dried over anhydrous sodium sulfate, filtered, and concentrated in vacuo to give the title compound. MS (ESI⁻) *m*/*z* 226 (M-H)⁻.

### Example 105C: 4-chloro-N-[2-({3-[2-(4-chloro-3-fluorophenoxy)acetamido]bicyclo-[1.1.1]pentan-1-yl}amino)-2-oxoethyl]-N-methylbenzamide

The title compound was prepared as described in Example 81, substituting the product of Example 9B for benzyl (4-aminobicyclo[2.1.1]hexan-1-yl)carbamate hydrochloride and the product of Example 105B for 2-(4-chloro-3-fluorophenoxy)acetic acid. ¹H NMR (400 MHz, DMSO-*d*₆, 120 °C) *δ* ppm 8.17 (s, 1H), 8.07 (s, 1H), 7.46 - 7.36 (m, 5H), 6.98 (dd, J = 11.3, 2.8 Hz, 1H), 6.83 (ddd, J = 9.0, 2.9, 1.3 Hz, 1H), 4.43 (s, 2H), 3.88 (s, 2H), 2.92 (s, 3H), 2.25 (br s, 6H). MS (ESI⁺) *m*/*z* 494 (M+H)⁺.

### Example 106: N²-acetyl-N-{3-[2-(4-chloro-3-fluorophenoxy)acetamido]bicyclo-[1.1.1]pentan-1-yl}-N²-(4-chlorophenyl)glycinamide (Compound 205)

Acetyl chloride (7.9 µL, 0.11 mmol) was added to a solution of the product of Example 104 (25mg, 0.055 mmol) in pyridine (1.0 mL). After stirring at ambient temperature for 1 hour, methanol (1 mL) was added, and the resulting solution was concentrated in vacuo. The residue was taken up in N,N-dimethylformamide (2 mL), filtered through a glass microfiber frit and purified by preparative HPLC [Waters XBridge^{™} C18 5 µm OBD^{™} column, 50 x 100 mm, flow rate 90 mL/minute, 5-100% gradient of acetonitrile in buffer (0.1% trifluoroacetic acid)] to give the title compound (24 mg, 0.049 mmol, 88% yield). ¹H NMR (400 MHz, DMSO-*d*₆, 120 °C) *δ* ppm 8.19 (s, 1H), 8.06 (s, 1H), 7.46 - 7.36 (m, 5H), 7.00 (dd, J = 11.3, 2.9 Hz, 1H), 6.85 (ddd, J = 8.9, 2.9, 1.3 Hz, 1H), 4.46 (s, 2H), 4.16 (s, 2H), 2.25 (br s, 6H), 1.86 (s, 3H). MS (ESI⁺) *m*/*z* 494 (M+H)⁺.

### Example 107: 2-(4-chloro-3-fluorophenoxy)-N-(3-{2-[(6-methylpyridin-3-yl)oxy]acetamidolbicyclo[1.1.1]pentan-1-yl)acetamide (Compound 206)

### Example 107A: 2-((6-methylpyridin-3-yl)oxy)acetic acid, 2.5 sodium hydroxide

Ethyl 2-[(6-methylpyridin-3-yl)oxy]acetate (Aldrich-CPR, 1.0 g, 5.12 mmol) was dissolved in ethanol (15 mL) and aqueous sodium hydroxide (2.5 M, 5.12 mL) was added in one portion. The resulting mixture was stirred at ambient temperature for 20 minutes and then concentrated in vacuo to provide the title compound as a sodium salt with 2.5 equivalent sodium hydroxide excipient (1.4 g, 5.12 mmol, quantitative). MS (ESI⁺) *m*/*z* 168 (M+H)⁺.

### Example 107B: 2-(4-chloro-3-fluorophenoxy)-N-(3-{2-[(6-methylpyridin-3-yl)oxy]acetamido}bicyclo[1.1.1]pentan-1-yl)acetamide

*N,N*-Dimethylformamide (1.0 mL), pyridine (1.0 mL, 12.4 mmol) and 1-[bis(dimethylamino)methylene]-1*H*-1,2,3-triazolo[4,5-*b*]pyridinium 3-oxid hexafluorophosphate (100 mg, 0.26 mmol, HATU) were added to a mixture of the product of Example 107A (73 mg, 0.241 mmol) and the product of Example 9B (84 mg, 0.21 mmol) in sequential order. The reaction was then stirred at ambient temperature for 18 hours, filtered through a glass microfiber frit and purified by preparative HPLC [Waters XBridge^{™} C18 5 µm OBD^{™} column, 30 x 100 mm, flow rate 40 mL/minute, 5-100% gradient of acetonitrile in buffer (0.025 M aqueous ammonium bicarbonate, adjusted to pH 10 with ammonium hydroxide)] to give the title compound (35 mg, 0.08 mmol, 38% yield). ¹H NMR (400 MHz, DMSO-*d*₆) *δ* ppm 8.71 (s, 2H), 8.16 (d, J = 3.0 Hz, 1H), 7.49 (t, J = 8.9 Hz, 1H), 7.29 - 7.24 (m, 1H), 7.20 - 7.15 (m, 1H), 7.07 (dd, J = 11.4, 2.8 Hz, 1H), 6.85 (ddd, J = 9.0, 2.8, 1.2 Hz, 1H), 4.48 (s, 2H), 4.47 (s, 2H), 2.39 (s, 3H), 2.26 (s, 6H). MS (ESI⁺) *m*/*z* 434 (M+H)⁺.

### Example 108: 2-(benzyloxy)-N-{3-[2-(4-chloro-3-fluorophenoxy)acetamido]-bicyclo[1.1.1]pentan-1-yl}acetamide (Compound 207)

The title compound was prepared as described in Example 107B, substituting benzyloxyacetic acid (commercially available from Aldrich) for the product of Example 107A. ¹H NMR (500 MHz, DMSO-*d*₆) *δ* ppm 8.69 (s, 1H), 8.35 (s, 1H), 7.49 (t, J = 8.9 Hz, 1H), 7.38 - 7.32 (m, 4H), 7.30 (ddd, J = 7.7, 5.0, 3.8 Hz, 1H), 7.07 (dd, J = 11.3, 2.9 Hz, 1H), 6.85 (ddd, J = 8.9, 2.9, 1.1 Hz, 1H), 4.52 (s, 2H), 4.47 (s, 2H), 3.84 (s, 2H), 2.24 (br s, 6H). MS (ESI⁺) *m*/*z* 433 (M+H)⁺.

### Example 109: 2-(benzyloxy)-N-{3-[2-(4-chlorophenoxy)acetamido]bicyclo[1.1.1]pentan-1-yl}acetamide (Compound 208)

### Example 109A: tert-butyl (3-(2-(4-chlorophenoxy)acetamido)bicyclo[1.1.1]pentan-1-yl)carbamate

To a solution of *tert-butyl* (3-aminobicyclo[1.1.1]pentan-1-yl)carbamate (PharmaBlock,1.1 g, 5.55 mmol) in tetrahydrofuran (40 mL) was added triethylamine (2.32 mL, 16.64 mmol) followed by 4-chlorophenoxyacetyl chloride (Aldrich, 0.866 mL, 5.55 mmol). The mixture was allowed to stir at ambient temperature for 4 hours, and then the resulting solids were isolated via filtration to give the title compound (2.0 g, 5.45 mmol, 98% yield). MS (ESI⁺) *m*/*z* 384 (M+NH₄)⁺.

### Example 109B: N-(3-aminobicyclo[1.1.1]pentan-1-yl)-2-(4-chlorophenoxy)acetamide, trifluoroacetate

To a solution of the product of Example 109A (2.0 g, 5.45 mmol) in dichloromethane (25 mL) at ambient temperature was added trifluoroacetic acid (8.40 mL, 109 mmol). The mixture was allowed to stir at ambient temperature for 2 hours and was concentrated in vacuo. The resulting residue was treated with ether/heptane to give the title compound as a solid (1.5 g, 3.94 mmol, 72% yield). MS (ESI⁺) *m*/*z* 302 (M+NH₄)⁺.

### Example 109C: 2-(benzyloxy)-N-{3-[2-(4-chlorophenoxy)acetamido]bicyclo[1.1.1]pentan-1-yl}acetamide

The title compound was prepared as described in Example 107B, substituting benzyloxyacetic acid (commercially available from Aldrich) for the product of Example 107A and the product of Example 109B for the product of Example 9B. ¹H NMR (500 MHz, DMSO-*d*₆) *δ* ppm 8.67 (s, 1H), 8.34 (s, 1H), 7.40 - 7.27 (m, 7H), 6.99 - 6.93 (m, 2H), 4.52 (s, 2H), 4.42 (s, 2H), 3.84 (s, 2H), 2.24 (br s, 6H). MS (ESI⁺) *m*/*z* 415 (M+H)⁺.

### Example 110: N,N'-(bicyclo[3.1.1]heptane-1,5-diyl)bis[2-(3,4-dichlorophenoxy)acetamide (Compound 209)

### Example 110A: benzyl (5-(2-(3,4-dichlorophenoxy)acetamido)bicyclo[3.1.1]heptan-1-yl)carbamate

The title compound was prepared as described in Example 81, substituting 2-(3,4-dichlorophenoxy)acetic acid (commercially available from Aldrich) for 2-(4-chloro-3-fluorophenoxy)acetic acid and benzyl (5-aminobicyclo[3.1.1]heptan-1-yl)carbamate hydrochloride (commercially available from Curpys) for benzyl (4-aminobicyclo[2.1.1]hexan-1-yl)carbamate hydrochloride. ¹H NMR (500 MHz, DMSO-*d*₆) *δ* ppm 8.25 (s, 1H), 7.53 (d, J = 8.9 Hz, 1H), 7.49 (br s, 1H), 7.38 - 7.27 (m, 5H), 7.23 (d, J = 2.9 Hz, 1H), 6.96 (dd, J = 9.0, 2.9 Hz, 1H), 4.97 (s, 2H), 4.45 (s, 2H), 2.24 - 2.13 (m, 2H), 2.06 - 2.01 (m, 2H), 1.82 - 1.67 (m, 6H). MS (ESI⁺) *m*/*z* 480 (M+NH₄)⁺.

### Example 110B: N,N'-(bicyclo[3.1.1]heptane-1,5-diyl)bis[2-(3,4-dichlorophenoxy)acetamide

The product of Example 110A (25 mg, 0.054 mmol) was stirred in trifluoroacetic acid (0.5 mL, 6.49 mmol) at 75 °C for 2 hours. The reaction mixture was cooled to ambient temperature and then concentrated in vacuo. To the resulting residue was added *N,N-*dimethylformamide (2.0 mL), 2-(3,4-dichlorophenoxy)acetic acid (commercially available from Aldrich, 17.9 mg, 0.081 mmol), triethylamine (38 µL, 0.27 mmol) and 1-[bis(dimethylamino)methylene]-1H-1,2,3-triazolo[4,5-b]pyridinium 3-oxid hexafluorophosphate (30.8 mg, 0.081 mmol, HATU) in sequential order. The reaction mixture was stirred at ambient temperature for 1 hour. The resulting solution was filtered through a glass microfiber frit and purified by preparative HPLC [Waters XBridge^{™} C18 5 µm OBD^{™} column, 30 x 100 mm, flow rate 40 mL/minute, 5-100% gradient of acetonitrile in buffer (0.1% trifluoroacetic acid)] to give the title compound (16 mg, 0.03 mmol, 56% yield). ¹H NMR (400 MHz, DMSO-*d*₆) *δ* ppm 8.26 (s, 2H), 7.54 (d, J = 8.9 Hz, 2H), 7.23 (d, J = 2.9 Hz, 2H), 6.96 (dd, J = 8.9, 2.9 Hz, 2H), 4.46 (s, 4H), 2.21 - 2.12 (m, 4H), 1.86 - 1.69 (m, 6H). MS (ESI⁺) *m*/*z* 415 (M+H)⁺.

### Example 111: 2-(4-chloro-3-fluorophenoxy)-N-{3-[2-(6-methylpyridin-3-yl)acetamido]bicyclo[1.1.1]pentan-1-yl}acetamide (Compound 210)

The title compound was prepared as described in Example 107B, substituting 2-(6-methylpyridin-3-yl)acetic acid (commercially available from Enamine) for the product of Example 107A. ¹H NMR (501 MHz, DMSO-*d*₆) *δ* ppm 8.69 (s, 1H), 8.68 (s, 1H), 8.27 (d, J = 2.3 Hz, 1H), 7.53 - 7.46 (m, 2H), 7.20 - 7.15 (m, 1H), 7.06 (dd, J = 11.4, 2.8 Hz, 1H), 6.84 (ddd, J = 9.0, 2.9, 1.2 Hz, 1H), 4.46 (s, 2H), 3.35 (s, 2H), 2.42 (s, 3H), 2.21 (br s, 6H). MS (ESI⁺) *m*/*z* 418 (M+H)⁺.

### Example 112: 4-{[2-({3-[2-(4-chloro-3-fluorophenoxy)acetamido]bicyclo[1.1.1]pentan-1-yl}amino)-2-oxoethoxy]methyl}benzoic acid (Compound 211)

### Example 112A: N-(3-aminobicyclo[1.1.1]pentan-1-yl)-2-(4-chloro-3-fluorophenoxy)acetamide hydrochloride

A mixture of Example 9A (1.20 g, 3.12 mmol) and 4 N HCl (in dioxane, 4.68 mL, 18.71 mmol) in dioxane (10 mL) was stirred overnight. The solids were filtered, washed with ethyl acetate, and vacuum oven-dried to give the title compound (0.985 g, 98%). MS (ESI⁺) *m*/*z* 284.9 (M+H)⁺.

### Example 112B: 2-(4-chloro-3-fluorophenoxy)-N-(3-(2-hydroxyacetamido)bicyclo[1.1.1]pentan-1-yl)acetamide

A mixture of Example 112A (0.475 g, 1.479 mmol), 2-hydroxyacetic acid (0.193 g, 1.775 mmol) 70% in water, 1-[bis(dimethylamino)methylene]-1H-1,2,3-triazolo[4,5-b]pyridinium 3-oxid hexafluorophosphate (HATU, 0.675 g, 1.775 mmol), and triethylamine (0.618 mL, 4.44 mmol) in tetrahydrofuran (8 mL) was stirred overnight. The reaction mixture was then treated with water and brine and extracted with ethyl acetate (2x). The combined organic layers were dried over MgSO₄, filtered, and concentrated. The residue was purified on a 40 g silica gel column using a Biotage^{®} Isolera^{™} One flash system eluting with heptanes/ethyl acetate (1:9) to ethyl acetate to give the title compound (0.323 g, 64%). MS (ESI⁺) *m*/*z* 342.9 (M+H)⁺.

### Example 112C: methyl 4-((2-((3-(2-(4-chloro-3-fluorophenoxy)acetamido)bicyclo[1.1.1]pentan-1-yl)amino)-2-oxoethoxy)methyl)benzoate

A mixture of Example 112B (100.0 mg, 0.292 mmol) and 60% sodium hydride in mineral oil (12.84 mg, 0.321 mmol) in tetrahydrofuran (3.5 mL) was stirred for 10 minutes. Methyl 4-(bromomethyl)benzoate (73.5 mg, 0.321 mmol) was added. The reaction mixture was stirred overnight. The reaction was quenched with brine and extracted with ethyl acetate (2x). The combined organic layers were dried over MgSO₄, filtered, and concentrated. The residue was purified on a 12 g silica gel column using a Biotage^{®} Isolera^{™} One flash system eluting with heptanes/ethyl acetate (4:6 to 1:9) to give the title compound (55.1 mg, 39%). MS (APCI⁺) *m*/*z* 491.1 (M+H)⁺.

### Example 112D: 4-{[2-({3-[2-(4-chloro-3-fluorophenoxy)acetamido]bicyclo[1.1.1]pentan-1-yl}amino)-2-oxoethoxy]methyl}benzoic acid

A solution of Example 112C (53.0 mg, 0.108 mmol) in tetrahydrofuran (1.2 mL) and methanol (0.8 mL) was treated with a solution of LiOH (7.76 mg, 0.324 mmol) in water (0.6 mL). The mixture was stirred for 6 hours and concentrated. The residue was purified by reverse-phase HPLC performed on a Zorbax Rx-C18 column (250 x 21.2 mm, 7 µm particle size) using a gradient of 10% to 95% acetonitrile : 0.1% aqueous trifluoroacetic acid over 30 minutes at a flow rate of 18 mL/minute to provide the title compound (22.7 mg, 44%). ¹H NMR (400 MHz, DMSO-*d*₆) *δ* ppm 8.68 (s, 1H), 8.39 (s, 1H), 7.97 - 7.85 (m, 2H), 7.54 - 7.39 (m, 3H), 7.05 (dd, J = 11.4, 2.9 Hz, 1H), 6.83 (ddd, J = 9.0, 2.8, 1.2 Hz, 1H), 4.59 (s, 2H), 4.45 (s, 2H), 3.87 (s, 2H), 2.23 (s, 6H). MS (ESI⁺) *m*/*z* 476.9 (M+H)⁺.

### Example 113: 3-{[2-({3-[2-(4-chloro-3-fluorophenoxy)acetamido]bicyclo[1.1.1]pentan-1-yl}amino)-2-oxoethoxy]methyl}benzoic acid (Compound 212)

### Example 113A: methyl 3-((2-((3-(2-(4-chloro-3-fluorophenoxy)acetamido)bicyclo[1.1.1]pentan-1-yl)amino)-2-oxoethoxy)methyl)benzoate

A mixture of Example 112B (100.0 mg, 0.292 mmol) and 60% sodium hydride in mineral oil (12.84 mg, 0.321 mmol) in tetrahydrofuran (4 mL) was stirred for 10 minutes. Methyl 3-(bromomethyl)benzoate (73.5 mg, 0.321 mmol) was added. The reaction mixture was stirred overnight. The reaction was quenched with brine and extracted with ethyl acetate (twice). The combined organic layers were dried over MgSO₄, filtered, concentrated. The residue was purified on a 12 g silica gel column using a Biotage^{®} Isolera^{™} One flash system eluting with heptanes/ethyl acetate (4:6 to 1:9) to give the title compound (46.1 mg, 32%). MS (APCI⁺) *m*/*z* 491.1 (M+H)⁺.

### Example 113B: 3-{[2-({3-[2-(4-chloro-3-fluorophenoxy)acetamido]bicyclo[1.1.1]pentan-1-yl}amino)-2-oxoethoxy]methyl}benzoic acid

A solution of Example 113A (45.0 mg, 0.092 mmol) in tetrahydrofuran (1.2 mL) and methanol (0.8 mL) was treated with a solution of LiOH (6.59 mg, 0.275 mmol) in water (0.6 mL). The mixture was stirred for 6 hours and then concentrated. The residue was purified by HPLC (see protocol in Example 112D) to provide the title compound (11.8 mg, 27%). ¹H NMR (501 MHz, DMSO-*d*₆) *δ* ppm 8.69 (s, 1H), 8.39 (s, 1H), 7.91 (d, J = 1.9 Hz, 1H), 7.86 (dt, J = 7.7, 1.5 Hz, 1H), 7.60 (dt, J = 7.7, 1.5 Hz, 1H), 7.53 - 7.42 (m, 2H), 7.05 (dd, J = 11.4, 2.8 Hz, 1H), 6.83 (ddd, J = 9.0, 2.9, 1.2 Hz, 1H), 4.57 (s, 2H), 4.45 (s, 2H), 3.86 (s, 2H), 2.23 (s, 6H). MS (ESI⁺) *m*/*z* 476.9 (M+H)⁺.

### Example 114: 2-(4-chloro-3-fluorophenoxy)-N-(3-{2-[(5-chloropyridin-2-yl)oxy]acetamido}bicyclo[1.1.1]pentan-1-yl)acetamide (Compound 213)

To a mixture of Example 112B (50 mg, 0.146 mmol), and 5-chloro-2-fluoropyridine (0.018 mL, 0.175 mmol) in dimethylformamide (1.5 mL) was added 60% NaH in mineral oil (7.58 mg, 0.190 mmol). The mixture was stirred for 2 hours. The reaction was quenched with water and brine and extracted with ethyl acetate (twice). The combined organic layers were dried over MgSO₄ and concentrated. The residue was purified by HPLC (see protocol in Example 112D) to provide the title compound as a trifluoroacetic acid salt (23.9 mg, 28.8%). ¹H NMR (400 MHz, DMSO-*d*₆) *δ* ppm 8.68 (s, 1H), 8.62 (s, 1H), 8.16 (d, J = 2.7 Hz, 1H), 7.82 (dd, J = 8.8, 2.7 Hz, 1H), 7.47 (t, J = 8.9 Hz, 1H), 7.05 (dd, J = 11.4, 2.8 Hz, 1H), 6.93 (d, J = 8.8 Hz, 1H), 6.83 (ddd, J = 9.0, 2.8, 1.2 Hz, 1H), 4.64 (s, 2H), 4.45 (s, 2H), 2.22 (s, 6H). MS (ESI⁺) *m*/*z* 454.0 (M+H)⁺.

### Example 115: 2-(4-chloro-3-fluorophenoxy)-N-[3-(2-{[5-(trifluoromethyl)pyridin-2-yl]oxy}acetamido)bicyclo[1.1.1]pentan-1-yl]acetamide (Compound 214)

A mixture of Example 112B (65 mg, 0.190 mmol), 2-fluoro-5-(trifluoromethyl)pyridine (0.027 mL, 0.228 mmol), and 60% NaH in mineral oil (25.03 mg, 0.626 mmol) in dimethylformamide (2 mL) was stirred for 2.5 hours. The reaction was quenched with water and brine and extracted with ethyl acetate (twice). The combined organic layers were dried over MgSO₄ and concentrated. The residue was purified by HPLC (see protocol in Example 112D) to provide the title compound as a trifluoroacetic acid salt (10.3 mg, 9%). ¹H NMR (400 MHz, DMSO-*d*₆) *δ* ppm 8.67 (s, 2H), (s, 1H), 8.07 (dd, J = 8.8, 2.6 Hz, 1H), 7.45 (t, J = 8.9 Hz, 1H), 7.11 - 6.95 (m, 2H), 6.81 (ddd, J = 9.0, 2.9, 1.2 Hz, 1H), 4.74 (s, 2H), 4.44 (s, 2H), 2.21 (s, 6H). MS (APCI⁺) *m*/*z* 487.8 (M+H)⁺.

### Example 116: 2-(4-chloro-3-fluorophenoxy)-N-(3-{2-[(5-cyanopyridin-2-yl)oxy]acetamidolbicyclo[1.1.1]pentan-1-yl)acetamide (Compound 215)

A mixture of Example 112B (80.2 mg, 0.234 mmol), 6-fluoronicotinonitrile (51.4 mg, 0.421 mmol), and cesium carbonate (152 mg, 0.468 mmol) in dimethylformamide (4 mL) was stirred for 5 hours. The reaction was quenched with water and brine and extracted with ethyl acetate (twice). The combined organic layers were dried over MgSO₄, filtered, and concentrated. The residue was purified by HPLC (see protocol in Example 112D) to provide the title compound as a trifluoroacetic acid salt (68.7 mg, 53%). ¹H NMR (400 MHz, DMSO-*d*₆) *δ* ppm 8.77 - 8.55 (m, 3H), 8.15 (dd, J = 8.7, 2.3 Hz, 1H), 7.45 (t, J = 8.9 Hz, 1H), 7.04 (ddd, J = 9.7, 6.6, 1.8 Hz, 2H), 6.81 (ddd, J = 8.9, 2.8, 1.2 Hz, 1H), 4.74 (s, 2H), 4.43 (s, 2H), 2.20 (s, 6H). MS (ESI⁺) *m*/*z* 444.9 (M+H)⁺.

### Example 117: 2-(4-chloro-3-fluorophenoxy)-N-(3-{2-[(5-chloro-4-methylpyridin-2-yl)oxy]acetamidolbicyclo[1.1.1]pentan-1-yl)acetamide (Compound 216)

A mixture of Example 112B (65 mg, 0.190 mmol), 5-chloro-2-fluoro-4-methylpyridine (0.026 mL, 0.228 mmol), and 60% NaH in mineral oil (25.03 mg, 0.626 mmol) in dimethylformamide (2 mL) was stirred for 1.5 hours. The reaction was quenched with water and brine and extracted with ethyl acetate (twice). The combined organic layers were dried over MgSO₄, filtered, and concentrated. The residue was purified by HPLC (see protocol in Example 112D) to provide the title compound as a trifluoroacetic acid salt (7.0 mg, 6.3%). ¹H NMR (400 MHz, DMSO-*d*₆) *δ* ppm 8.68 (s, 1H), 8.60 (s, 1H), 8.09 (s, 1H), 7.47 (t, J = 8.9 Hz, 1H), 7.05 (dd, J = 11.4, 2.8 Hz, 1H), 6.91 (s, 1H), 6.83 (dd, J = 9.0, 2.8 Hz, 1H), 4.63 (s, 2H), 4.45 (s, 2H), 2.30 (s, 3H), 2.21 (s, 6H). MS (APCI⁺) *m*/*z* 468.1 (M+H)⁺.

### Example 118: 2-(4-chloro-3-fluorophenoxy)-N-(3-{2-[(3-methyl-1,2,4-oxadiazol-5-yl)methoxy]acetamido}bicyclo[1.1.1]pentan-1-yl)acetamide (Compound 217)

### Example 118A: methyl [2-(13-[2-(4-chloro-3-fluorophenoxy)acetamido]bicyclo[1.1.1]pentan-1-yl}amino)-2-oxoethoxy]acetate

To a mixture of Example 112B (600.0 mg, 1.751 mmol) and methyl 2-bromoacetate (0.282 mL, 2.98 mmol) in tetrahydrofuran (20 mL) was added cesium carbonate (1711 mg, 5.25 mmol). The reaction mixture was stirred overnight. The reaction mixture was treated with brine and extracted with ethyl acetate (twice). The combined organic layers were dried over MgSO₄, filtered, and concentrated. The residue was purified on a 40 g silica gel column using a Biotage^{®} Isolera^{™} One flash system eluting with heptanes/ethyl acetate (5:95) to give the title compound (0.310 g. 43%). ¹H NMR (400 MHz, DMSO-*d*₆) *δ* ppm 8.66 (s, 1H), 8.34 (s, 1H), 7.46 (t, J = 8.9 Hz, 1H), 7.03 (dd, J = 11.4, 2.8 Hz, 1H), 6.81 (ddd, J = 8.9, 2.8, 1.2 Hz, 1H), 4.44 (s, 2H), 4.16 (s, 2H), 3.88 (s, 2H), 3.63 (s, 3H), 2.21 (s, 6H). MS (ESI⁺) *m*/*z* 415.0 (M+H)⁺.

### Example 118B: [2-({3-[2-(4-chloro-3-fluorophenoxy)acetamido]bicyclo[1.1.1]pentan-1-yl}amino)-2-oxoethoxy]acetic acid

A solution of Example 118A (0.345 g, 0.832 mmol) in tetrahydrofuran (4.5 mL) and methanol (3 mL) was treated with a solution of LiOH (0.060 g, 2.495 mmol) in water (2 mL). The mixture was stirred for 6 hours and concentrated until most solvent was evaporated. The remaining light suspension was treated with 5% citric acid until pH=3. The resulting suspension was filtered. The collected solid was washed with water and vacuum oven-dried to give the title compound. ¹H NMR (500 MHz, DMSO-*d*₆) *δ* ppm 8.69 (s, 1H), 8.43 (s, 1H), 7.49 (t, J = 8.9 Hz, 1H), 7.06 (dd, J = 11.4, 2.8 Hz, 1H), 6.84 (ddd, J = 9.0, 2.8, 1.2 Hz, 1H), 4.47 (s, 2H), 4.09 (s, 2H), 3.91 (s, 2H), 2.24 (s, 6H). MS (ESI⁺) *m*/*z* 401.0 (M+H)⁺.

### Example 118C: 2-(4-chloro-3-fluorophenoxy)-N-(3-{2-[(3-methyl-1,2,4-oxadiazol-5-yl)methoxy]acetamido}bicyclo[1.1.1]pentan-1-yl)acetamide

A mixture of Example 118B (71.5 mg, 0.178 mmol), N-hydroxyacetimidamide (15.86 mg, 0.214 mmol), 1-[bis(dimethylamino)methylene]-1H-1,2,3-triazolo[4,5-b]pyridinium 3-oxid hexafluorophosphate (HATU, 81 mg, 0.214 mmol), and triethylamine (0.037 mL, 0.268 mmol) in acetonitrile (6 mL) was stirred for 6 hours. To the reaction mixture was added 10 mg of 4Å molecular sieves, and the reaction was heated to 81 °C overnight. The reaction mixture was filtered, and the filtrate was concentrated. The concentrate was treated with brine and extracted with ethyl acetate (twice). The combined organic layers were concentrated, and the residue was purified by HPLC (see protocol in Example 112D) to provide the title compound (42.5 mg, 54%). ¹H NMR (500 MHz, DMSO-*d*₆) *δ* ppm 8.72 (s, 1H), 8.51 (s, 1H), 7.50 (t, J = 8.9 Hz, 1H), 7.08 (dd, J = 11.4, 2.8 Hz, 1H), 6.86 (ddd, J = 9.0, 2.9, 1.1 Hz, 1H), 4.85 (s, 2H), 4.48 (s, 2H), 4.01 (s, 2H), 2.36 (s, 3H), 2.25 (s, 6H). MS (ESI⁺) *m*/*z* 439.0 (M+H)⁺.

### Example 119: 2-(4-chloro-3-fluorophenoxy)-N-(3-{2-[(5-oxo-4,5-dihydro-1,3,4-oxadiazol-2-yl)methoxy]acetamido}bicyclo[1.1.1]pentan-1-yl)acetamide (Compound 218)

### Example 119A: 2-(4-chloro-3-fluorophenoxy)-N-{3-[2-(2-hydrazinyl-2-oxoethoxy)acetamidobicyclo[1.1.1]pentan-1-yl}acetamide

A mixture of Example 118A (0.245 g, 0.591 mmol) and hydrazine monohydrate (0.046 mL, 1.477 mmol) in ethanol was heated at 80 °C for 3 hours. The reaction mixture was concentrated. The concentrate was purified on a 12 g silica gel column using a Biotage^{®} Isolera^{™} One flash system eluting with methanol/ethyl acetate (1:9) to give the title compound. ¹H NMR (400 MHz, DMSO-*d*₆) *δ* ppm 9.32 (s, 1H), 8.70 (s, 1H), 8.59 (s, 1H), 7.48 (t, J = 8.9 Hz, 1H), 7.05 (dd, J = 11.4, 2.8 Hz, 1H), 6.83 (ddd, J = 9.0, 2.9, 1.2 Hz, 1H), 4.46 (s, 2H), 4.28 (d, J = 4.0 Hz, 2H), 3.93 (s, 2H), 3.85 (s, 2H), 2.26 (s, 6H). MS (ESI⁺) *m*/*z* 415.0 (M+H)⁺.

### Example 119B: 2-(4-chloro-3-fluorophenoxy)-N-(3-{2-[(5-oxo-4,5-dihydro-1,3,4-oxadiazol-2-yl)methoxy]acetamido}bicyclo[1.1.1]pentan-1-yl)acetamide

A mixture of Example 119A (50.0 mg, 0.121 mmol) and 1,1'-carbonyldiimidazole (23.45 mg, 0.145 mmol) in 1,4-dioxane (1.5 mL) was heated at reflux for 45 minutes. The reaction mixture was concentrated, and the residue was purified by HPLC (see protocol in Example 112D) to provide the title compound (34.2 mg, 64%). ¹H NMR (400 MHz, DMSO-*d*₆) *δ* ppm 12.37 (s, 1H), 8.67 (s, 1H), 8.40 (s, 1H), 7.47 (t, J = 8.9 Hz, 1H), 7.05 (dd, J = 11.4, 2.9 Hz, 1H), 6.83 (ddd, J = 9.0, 2.9, 1.2 Hz, 1H), 4.45 (s, 2H), 4.40 (s, 2H), 3.90 (s, 2H), 2.22 (s, 6H). MS (ESI⁺) *m*/*z* 440.9 (M+H)⁺.

### Example 120: 2-(4-chloro-3-fluorophenoxy)-N-(3-{2-[4-(pentafluoro-λ⁶-sulfanyl)phenoxy]acetamido}bicyclo[1.1.1]pentan-1-yl)acetamide (Compound 219)

A mixture of Example 28A (60.0 mg, 0.166 mmol), 4-(pentafluorothio)phenol (54.9 mg, 0.249 mmol), and potassium carbonate (45.9 mg, 0.332 mmol) in acetone (2 mL) was heated at 120 °C in a Biotage^{®} Initiator microwave reactor for 20 minutes. The reaction mixture was concentrated. The residue was treated with brine and extracted with ethyl acetate. The organic layer was concentrated, and the residue was purified by HPLC (see protocol in Example 112D) to provide the title compound (54.1 mg, 60%). ¹H NMR (400 MHz, DMSO-*d*₆) *δ* ppm 8.75 (s, 1H), 8.70 (s, 1H), 7.82 (d, J = 9.3 Hz, 2H), 7.47 (t, J = 8.9 Hz, 1H), 7.12 - 7.00 (m, 3H), 6.83 (ddd, J = 9.0, 2.9, 1.2 Hz, 1H), 4.54 (s, 2H), 4.46 (s, 2H), 2.25 (s, 6H). MS (ESI⁺) *m*/*z* 544.8 (M+H)⁺.

### Example 121: 2-(4-chloro-3-fluorophenoxy)-N-(3-{2-[(2,6-dimethylpyridin-4-yl)oxy]acetamido}bicyclo[1.1.1]pentan-1-yl)acetamide (Compound 220)

A mixture of Example 28A (65.0 mg, 0.180 mmol), 2,6-dimethylpyridin-4-ol (33.2 mg, 0.270 mmol), potassium carbonate (49.7 mg, 0.360 mmol), and potassium iodide (2.091 mg, 0.013 mmol) in acetone (2.5 mL) was heated at 140 °C in a Biotage^{®} Initiator microwave reactor for 40 minutes. The reaction mixture was concentrated. The residue was treated with brine and extracted with ethyl acetate. The organic layer was concentrated, and the residue was purified by HPLC (see protocol in Example 112D) to provide the title compound as a trifluoroacetic acid salt (53.2 mg, 53%). ¹H NMR (400 MHz, DMSO-*d*₆) *δ* ppm 8.88 (s, 1H), 8.72 (s, 1H), 7.47 (t, J = 8.9 Hz, 1H), 7.26 (s, 2H), 7.05 (dd, J = 11.4, 2.9 Hz, 1H), 6.83 (ddd, J = 9.0, 2.9, 1.2 Hz, 1H), 4.79 (s, 2H), 4.46 (s, 2H), 2.57 (s, 6H), 2.25 (s, 6H). MS (ESI⁺) *m*/*z* 448.1 (M+H)⁺.

### Example 122: 2-[(6-tert-butylpyridin-3-yl)oxy]-N-{3-[2-(4-chloro-3-fluorophenoxy)acetamido]bicyclo[1.1.1]pentan-1-yl}acetamide (Compound 221)

A mixture of Example 28A (65.0 mg, 0.180 mmol), 6-(*tert*-butyl)pyridin-3-ol (40.8 mg, 0.270 mmol), and potassium carbonate (49.7 mg, 0.360 mmol) in acetone (2 mL) was heated at 120 °C in a Biotage^{®} Initiator microwave reactor for 30 minutes. The reaction mixture was concentrated. The residue was treated with brine and extracted with ethyl acetate. The organic layer was concentrated, and the residue was purified by HPLC (see protocol in Example 112D) to provide the title compound as a trifluoroacetic acid salt (49.8 mg, 47%). ¹H NMR (400 MHz, DMSO-*d*₆) *δ* ppm 8.72 (d, J = 15.7 Hz, 2H), 8.26 (t, J = 1.8 Hz, 1H), 7.55 - 7.37 (m, 3H), 7.05 (dd, J = 11.3, 2.8 Hz, 1H), 6.83 (ddd, J = 8.9, 3.0, 1.2 Hz, 1H), 4.54 (s, 2H), 4.46 **(s,** 2H), 2.25 (s, 6H), 1.30 (s, 9H). MS (ESI⁺) *m*/*z* 476.2 (M+H)⁺.

### Example 123: 2-(4-chloro-3-fluorophenoxy)-N-[3-(2-{[5-chloro-6-(trifluoromethyl)pyridin-3-yl]oxy}acetamido)bicyclo[1.1.1]pentan-1-yl]acetamide (Compound 222)

A mixture of Example 28A (70.0 mg, 0.194 mmol), 5-chloro-6-(trifluoromethyl)pyridin-3-ol (57.4 mg, 0.291 mmol), and potassium carbonate (53.6 mg, 0.388 mmol) in acetone (2.5 mL) was heated at 120 °C in a Biotage^{®} Initiator microwave reactor for 20 minutes. The reaction mixture was concentrated. The residue was treated with brine and extracted with ethyl acetate. The organic layer was concentrated, and the residue was purified by HPLC (see protocol in Example 112D) to provide the title compound (32.9 mg, 47%). ¹H NMR (500 MHz, DMSO-*d*₆) *δ* ppm 8.80 (s, 1H), 8.71 (s, 1H), 8.39 (d, J = 2.5 Hz, 1H), 7.85 (d, J = 2.4 Hz, 1H), 7.47 (t, J = 8.9 Hz, 1H), 7.05 (dd, J = 11.4, 2.9 Hz, 1H), 6.83 (ddd, J = 9.0, 2.9, 1.2 Hz, 1H), 4.71 (s, 2H), 4.46 (s, 2H), 2.26 (s, 6H). MS (ESI⁺) *m*/*z* 522.0 (M+H)⁺.

### Example 124: methyl 2-chloro-5-[2-({3-[2-(4-chloro-3-fluorophenoxy)acetamido]-bicyclo[1.1.1]pentan-1-yl}amino)-2-oxoethoxy]benzoate (Compound 223)

### Example 124A: 2-(4-chloro-3-(methoxycarbonyl)phenoxy)acetic acid

The title compound was prepared as described in Example 9C and Example 9D, except substituting methyl 2-chloro-5-hydroxybenzoate for 6-hydroxy-1H-indazole. ¹H NMR (400 MHz, DMSO-*d*₆) *δ* ppm 7.43 (d, *J* = 8.9 Hz, 1H), 7.25 (d, *J* = 3.1 Hz, 1H), 7.10 (dd, *J* = 8.9, 3.2 Hz, 1H), 4.71 (s, 2H), 3.81 (s, 3H).

### Example 124B: methyl 2-chloro-5-[2-({3-[2-(4-chloro-3-fluorophenoxy)acetamido]-bicyclo[1.1.1]pentan-1-yl}amino)-2-oxoethoxy]benzoate

To a mixture of Example 9B (0.16 g, 0.401 mmol), Example 124A (0.123 g, 0.502 mmol) and *N*-ethyl-*N*-isopropylpropan-2-amine (0.350 mL, 2.006 mmol) in *N,N-*dimethylformamide (5.0 mL), 2-(3*H*-[1,2,3]triazolo[4,5-b]pyridin-3-yl)-1,1,3,3-tetramethylisouronium hexafluorophosphate(V) (0.229 g, 0.602 mmol) was added, and the reaction mixture was stirred at ambient temperature for 16 hours. Volatiles were removed under high vacuum, and the residue was purified by HPLC (10-95% acetonitrile in 0.1% trifluoroacetic acid/water on Phenomenex^{®} C18 5µm column) to give 171 mg of the title compound as a light yellow solid. ¹H NMR (400 MHz, DMSO-*d*₆) *δ* ppm 8.71 (d, *J* = 11.6 Hz, 2H), 7.52 7.42 (m, 2H), 7.37 (d, *J* = 3.1 Hz, 1H), 7.15 (dd, *J* = 8.9, 3.1 Hz, 1H), 7.05 (dd, *J =* 11.3, 2.9 Hz, 1H), 6.83 (ddd, *J* = 9.0, 2.9, 1.2 Hz, 1H), 4.47 (d, *J* = 6.6 Hz, 4H), 3.84 (s, 3H), 2.25 (s, 6H). MS (ESI+) *m*/*z* 510.9 (M+H)⁺.

### Example 125: 2-(4-chloro-3-fluorophenoxy)-N-(3-{2-[4-chloro-3-(hydroxymethyl)phenoxy]-acetamido}bicyclo[1.1.1]pentan-1-yl)acetamide (Compound 224)

A mixture of Example 124 (0.13 g, 0.254 mmol) and lithium tetrahydroborate (0.055 g, 2.54 mmol) in tetrahydrofuran (5.0 mL) was stirred at 40 °C for 24 hours. The mixture was concentrated under high vacuum, and the residue was purified by HPLC (10-95% acetonitrile in 0.1% trifluoroacetic acid/water on a Phenomenex^{®} C18 5 µm column) to give 66 mg of the title compound as a light yellow solid. ¹H NMR (400 MHz, DMSO-*d*₆) *δ* ppm 8.68 (d, *J* = 2.8 Hz, 2H), 7.45 (t, *J* = 8.9 Hz, 1H), 7.26 (d, *J* = 8.7 Hz, 1H), 7.12 (d, *J* = 3.1 Hz, 1H), 7.03 (dd, *J* = 11.4, 2.9 Hz, 1H), 6.85 - 6.77 (m, 2H), 5.40 (t, *J* = 5.6 Hz, 1H), 4.51 - 4.41 (m, 4H), 4.38 (s, 2H), 2.23 (s, 6H). MS (ESI+) *m*/*z* 482.9 (M+H)⁺.

### Example 126: 2-(4-chloro-3-fluorophenoxy)-N-(3-{2-[(6-chloropyridin-3-yl)oxy]acetamidolbicyclo[1.1.1]pentan-1-yl)acetamide (Compound 225)

The title compound was prepared as described in Example 124, except substituting 2-((6-chloropridin-3-yl)oxy)acetic acid for 2-(4-chloro-3-(methoxycarbonyl)phenoxy)acetic acid. ¹H NMR (400 MHz, DMSO-*d*₆) *δ* ppm 8.71 (d, *J* = 14.8 Hz, 2H), 8.08 (dd, *J* = 2.8, 0.9 Hz, 1H), 7.49 - 7.36 (m, 3H), 7.03 (dd, *J* = 11.4, 2.8 Hz, 1H), 6.81 (ddd, *J* = 9.0, 2.9, 1.2 Hz, 1H), 4.51 (s, 2H), 4.43 (s, 2H), 2.23 (s, 6H). MS (ESI+) *m*/*z* 454.0 (M+H)⁺.

### Example 127: 2-(4-chloro-3-fluorophenoxy)-N-(3-{2-[(6-chloro-5-methylpyridin-3-yl)oxy]acetamidolbicyclo[1.1.1]pentan-1-yl)acetamide (Compound 226)

A mixture of Example 28A (0.06 g, 0.166 mmol), 6-chloro-5-methylpyridin-3-ol (0.048 g, 0.332 mmol), potassium carbonate (0.046 g, 0.332 mmol) and potassium iodide (1.930 mg, 0.012 mmol) in acetone (2.0 mL) was stirred at 140 °C in a Biotage^{®} Initiator microwave reactor (0-450 W) for 45 minutes. The suspension was filtered, and the filter was washed with methanol. The filtrate was concentrated, and the residue was purified by HPLC (10-85% acetonitrile in 0.1% trifluoroacetic acid/water on a Phenomenex^{®} C18 5 µm column) to give 59 mg of the title compound as a white solid. ¹H NMR (400 MHz, DMSO-*d*₆) *δ* ppm 8.70 (s, 1H), 8.68 (s, 1H), 7.93 (d, *J* = 3.0 Hz, 1H), 7.50 7.41 (m, 2H), 7.03 (dd, *J* = 11.4, 2.9 Hz, 1H), 6.81 (ddd, *J =* 9.0, 2.9, 1.1 Hz, 1H), 5.71 (s, 1H), 4.49 (s, 2H), 4.44 (s, 2H), 2.27 (s, 3H), 2.23 (s, 6H). MS (ESI-) *m*/*z* 466.0 (M-H)⁻.

### Example 128: 2-(4-chloro-3-fluorophenoxy)-N-(3-{2-[(6-chloro-5-fluoropyridin-3-yl)oxy]acetamidolbicyclo[1.1.1]pentan-1-yl)acetamide (Compound 227)

The title compound was prepared as described in Example 127, except substituting 6-chloro-5-fluoropridin-3-ol for 6-chloro-5-methylpyridin-3-ol. ¹H NMR (400 MHz, DMSO-*d*₆) *δ* ppm 8.73 (s, 1H), 8.68 (s, 1H), 8.03 (d, *J* = 2.5 Hz, 1H), 7.67 (dd, *J* = 10.2, 2.6 Hz, 1H), 7.46 (t, *J* = 8.9 Hz, 1H), 7.03 (dd, *J* = 11.3, 2.9 Hz, 1H), 6.81 (ddd, *J* = 9.0, 2.9, 1.1 Hz, 1H), 4.57 (s, 2H), 4.44 (s, 2H), 2.23 (s, 6H). MS (ESI-) *m*/*z* 470.0 (M-H)⁻.

### Example 129: 2-(3-amino-4-chlorophenoxy)-N-{3-[2-(4-chloro-3-fluorophenoxy)acetamido]-bicyclo[1.1.1]pentan-1-yl}acetamide (Compound 228)

### Example 129A: 2-(4-chloro-3-nitrophenoxy)acetic acid

To a solution of 4-chloro-3-nitrophenol (2.2 g, 12.68 mmol) in *N,N*-dimethylformamide (25.0 mL) at ambient temperature was added potassium carbonate (3.50 g, 25.4 mmol) and *tert-*butyl bromoacetate (2.138 mL, 14.58 mmol). This mixture was warmed to 65 °C and allowed to stir for 1.5 hours. The mixture was allowed to cool to ambient temperature and was partitioned between ethyl acetate (50 mL) and H₂O (50 mL). The layers were separated, and the aqueous layer was extracted with ethyl acetate (3 x 15 mL). The combined organic layers were dried over magnesium sulfate and filtered. The filtrate was concentrated under reduced pressure. The residue was purified via column chromatography (SiO₂, 0-10% ethyl acetate/heptanes) to give 3.8 g of *tert*-butyl 2-(4-chloro-3-nitrophenoxy)acetate . To a mixture of *tert*-butyl 2-(4-chloro-3-nitrophenoxy)acetate (3.65 g, 12.68 mmol) in methanol (30 mL) and water (10 mL) was added NaOH (12.68 mL, 63.4 mmol) (5 M solution in water). This mixture was allowed to stir at ambient temperature for 2 hours, and was concentrated under reduced pressure to give a white solid which was dissolved in water. The pH was adjusted to ~1 with 1 N HCl, and the resulting white solid was isolated via filtration to give the title compound (2.0 g, 8.64 mmol, 68.1% yield). ¹H NMR (400 MHz, DMSO-*d*₆) *δ* ppm 7.64 7.56 (m, 2H), 7.22 (dd, *J* = 9.0, 3.0 Hz, 1H), 4.70 (s, 2H).

### Example 129B: 2-(4-chloro-3-fluorophenoxy)-N-(3-(2-(4-chloro-3-nitrophenoxy)-acetamido)bicyclo[1.1.1]pentan-1-yl)acetamide

The title compound was prepared as described in Example 124B, except substituting Example 129A for 2-(4-chloro-3-(methoxycarbonyl)phenoxy)acetic acid. ¹H NMR (400 MHz, DMSO-*d*₆) *δ* ppm 8.73 (s, 1H), 8.68 (s, 1H), 7.69 - 7.57 (m, 2H), 7.45 (t, *J* = 8.9 Hz, 1H), 7.27 (dd, *J* = 9.0, 3.0 Hz, 1H), 7.03 (dd, *J* = 11.4, 2.9 Hz, 1H), 6.81 (ddd, *J* = 8.9, 2.9, 1.2 Hz, 1H), 4.54 (s, 2H), 4.44 (s, 2H), 2.23 (s, 6H).

### Example 129C: 2-(3-amino-4-chlorophenoxy)-N-{3-[2-(4-chloro-3-fluorophenoxy)acetamido]-bicyclo[1.1.1]pentan-1-yl}acetamide

To a mixture of Example 129B (0.19 g, 0.381 mmol) in tetrahydrofuran (20 mL) was added Ra-Ni 2800, water slurry (0.4 g, 3.41 mmol) in a 50 mL pressure bottle, and the reaction vessel was shaken under hydrogen (50 psi) and ambient temperature for 5 hours. The suspension was filtered, and the filtrate was concentrated. The residue was purified by HPLC (10-85% acetonitrile in 0.1% trifluoroacetic acid/water on Phenomenex^{®} C18 5 µm column) to give 72 mg of the title compound as a white solid. ¹H NMR (400 MHz, DMSO-*d*₆) *δ* ppm 8.67 (s, 1H), 8.60 (s, 1H), 7.46 (t, *J* = 8.8 Hz, 1H), 7.08 - 6.99 (m, 2H), 6.82 (ddd, *J* = 9.0, 2.8, 1.2 Hz, 1H), 6.34 (d, *J* = 2.9 Hz, 1H), 6.12 (dd, *J* = 8.7, 2.9 Hz, 1H), 5.29 (s, 2H), 4.44 (s, 2H), 4.27 (s, 2H), 2.23 (s, 6H). MS (ESI+) *m*/*z* 467.9 (M+H)⁺.

### Example 130: 2-[(2-amino-6-methylpyridin-3-yl)oxy]-N-{3-[2-(4-chloro-3-fluorophenoxy)acetamido]bicyclo[1.1.1]pentan-1-yl}acetamide (Compound 229)

The title compound was prepared as described in Example 127, except substituting 2-amino-6-methylpridin-3-ol for 6-chloro-5-methylpyridin-3-ol. ¹H NMR (400 MHz, DMSO-*d*₆) *δ* ppm 14.26 (s, 1H), 8.80 (s, 1H), 8.72 (s, 1H), 8.18 (s, 2H), 7.46 *(t, J* = 8.9 Hz, 1H), 7.37 (d, *J* = 8.1 Hz, 1H), 7.04 (dd, *J* = 11.4, 2.9 Hz, 1H), 6.82 (ddd, *J* = 9.0, 2.9, 1.2 Hz, 1H), 6.59 (dd, *J* = 8.1, 1.0 Hz, 1H), 4.54 (s, 2H), 4.45 (s, 2H), 2.32 (s, 3H), 2.28 (s, 6H). MS (ESI+) *m*/*z* 449.1 (M+H)⁺.

### Example 131: 2-(4-chloro-3-fluorophenoxy)-N-[3-(2-{[5-(trifluoromethyl)pyridin-3-yl]oxy}acetamido)bicyclo[1.1.1]pentan-1-yl]acetamide (Compound 230)

The title compound was prepared as described in Example 127, except substituting 5-(trifluoromethyl)pyridin-3-ol for 6-chloro-5-methylpyridin-3-ol. ¹H NMR (400 MHz, DMSO-*d*₆) *δ* ppm 8.78 (s, 1H), 8.71 (s, 1H), 8.58 (dd, *J* = 15.7, 2.2 Hz, 4H), 7.73 (t, *J* = 2.3 Hz, 1H), 7.47 (t, *J* = 8.8 Hz, 1H), 7.05 (dd, *J* = 11.4, 2.8 Hz, 1H), 6.83 (ddd, *J* = 9.0, 2.9, 1.2 Hz, 1H), 4.66 (s, 2H), 4.46 (s, 2H), 2.25 (s, 6H). MS (ESI+) *m*/*z* 488.0 (M+H)⁺.

### Example 132: 2-chloro-5-[2-({3-[2-(4-chloro-3-fluorophenoxy)acetamido]-bicyclo[1.1.1]pentan-1-yl}amino)-2-oxoethoxy]pyridine-3-carboxylic acid (Compound 231)

The title compound was prepared as described in Example 127, except substituting 2-chloro-5-hydroxynicotinic acid for 6-chloro-5-methylpyridin-3-ol. ¹H NMR (400 MHz, DMSO-*d*₆) *δ* ppm 10.66 (s, 1H), 8.71 (d, *J* = 19.7 Hz, 2H), 8.11 (d, *J* = 3.0 Hz, 1H), 7.68 (d, *J* = 3.0 Hz, 1H), 7.46 (t, *J* = 8.9 Hz, 1H), 7.04 (dd, *J* = 11.4, 2.9 Hz, 1H), 6.82 (ddd, *J* = 9.0, 2.9, 1.2 Hz, 1H), 4.66 (s, 2H), 4.44 (s, 2H), 2.22 (s, 6H). MS (ESI+) *m*/*z* 497.9 (M+H)⁺.

### Example 133: 2-(4-chloro-3-fluorophenoxy)-N-(3-{2-[(2,6-dimethylpyridin-3-yl)oxy]acetamidolbicyclo[1.1.1]pentan-1-yl)acetamide (Compound 232)

The title compound was prepared as described in Example 127, except substituting 2,6-dimethylpyridin-3-ol for 6-chloro-5-methylpyridin-3-ol. ¹H NMR (501 MHz, DMSO-*d*₆) *δ* ppm 8.73 (d, *J* = 13.7 Hz, 2H), 7.81 (d, *J* = 8.7 Hz, 1H), 7.57 - 7.44 (m, 2H), 7.05 (dd, *J* = 11.4, 2.8 Hz, 1H), 6.83 (ddd, *J* = 8.9, 2.9, 1.2 Hz, 1H), 4.69 (s, 2H), 4.46 (s, 2H), 2.54 (s, 6H), 2.24 (s, 6H). MS (ESI+) m/z 448.1 (M+H)⁺.

### Example 134: 2-(4-chloro-3-fluorophenoxy)-N-[3-(2-{[2-(trifluoromethyl)pyridin-4-yl]oxy}acetamido)bicyclo[1.1.1]pentan-1-yl]acetamide (Compound 233)

The title compound was prepared as described in Example 127, except substituting 2-(trifluoromethyl)pyridin-4-ol for 6-chloro-5-methylpyridin-3-ol. ¹H NMR (400 MHz, DMSO-*d*₆) *δ* ppm 8.79 (s, 1H), 8.71 (s, 1H), 8.57 (d, *J* = 5.7 Hz, 1H), 7.52 - 7.40 (m, 2H), 7.24 (dd, *J* = 5.8, 2.4 Hz, 1H), 7.05 (dd, *J* = 11.4, 2.9 Hz, 1H), 6.83 (ddd, *J* = 8.9, 2.8, 1.2 Hz, 1H), 4.67 (s, 2H), 4.46 (s, 2H), 2.25 (s, 6H). MS (ESI+) *m*/*z* 488.1 (M+H)⁺.

### Example 135: 2-(4-chloro-3-fluorophenoxy)-N-(3-{2-[(5,6-dimethylpyridin-3-yl)oxy]acetamidolbicyclo[1.1.1]pentan-1-yl)acetamide (Compound 234)

The title compound was prepared as described in Example 127, except substituting 5,6-dimethylpyridin-3-ol for 6-chloro-5-methylpyridin-3-ol. ¹H NMR (400 MHz, DMSO-*d*₆) *δ* ppm 8.80 (s, 1H), 8.72 (s, 1H), 8.29 (d, *J* = 2.7 Hz, 1H), 7.91 (d, *J* = 2.6 Hz, 1H), 7.47 (t, *J* = 8.9 Hz, 1H), 7.05 (dd, *J* = 11.3, 2.8 Hz, 1H), 6.83 (dd, *J* = 9.0, 2.7 Hz, 1H), 4.65 (s, 2H), 4.46 (s, 2H), 2.52 (s, 3H), 2.35 (s, 3H), 2.25 (s, 6H). MS (ESI+) *m*/*z* 448.1 (M+H)⁺.

### Example 136: 2-[(6-acetylpyridin-3-yl)oxy]-N-{3-[2-(4-chloro-3-fluorophenoxy)acetamido]0-bicyclo[1.1.1]pentan-1-yl}acetamide (Compound 235)

A 2.5 mL microwave vial was charged with Example 28A (35 mg, 1 equivalent, 0.096 mmol), K₂CO₃ (27mg, 0.19 mmol), 1-(5-hydroxypyridin-2-yl)ethanone (27 mg, 0.19 mmol) and potassium iodide (1,2 mg, 0.07 equivalent, 0.05 mmol). To this mixture was added acetone (1.5 mL). The resulting mixture was heated in a Biotage^{®} Initiator microwave for 45 minutes at 140 °C (0-450 W). Upon completion, the mixture was then filtered, and the filtrate was concentrated to dryness. The residue was dissolved in 1:1 dimethyl sulfoxide/methanol and purified by preparative reverse phase HPLC on a Phenomenex^{®} Luna^{®} C8(2) 5 µm 100 Å AXIA^{™} column (30 mm × 150mm). A gradient of acetonitrile (A) and 0.1% trifluoroacetic acid in water (B) was used, at a flow rate of 50 mL/minute (0-0.5 minutes 5% A, 0.5-8.5 minutes linear gradient 5-100% A, 8.7-10.7 minutes 100% A, 10.7 -11.0 minutes linear gradient 100-5% A) to afford the title compound. ¹H NMR (400 MHz, DMSO-*d*₆) *δ* ppm 8.39 (d, *J* = 2.9 Hz, 1H), 7.96 (d, *J =* 8.8 Hz, 1H), 7.53 - 7.45 (m, 2H), 7.05 (dd, *J* = 11.3, 2.9 Hz, 1H), 6.85 (ddd, *J* = 9.0, 2.8, 1.2 Hz, 1H), 4.64 (s, 2H), 4.46 (s, 2H), 2.58 (s, 3H), 2.28 (s, 6H). MS (APCI) *m*/*z* 462.3 (M+H)⁺.

### Example 137: 2-[(2-amino-6-chloropyridin-3-yl)oxy]-N-{3-[2-(4-chloro-3-fluorophenoxy)acetamido]bicyclo[1.1.1]pentan-1-yl}acetamide (Compound 236)

The title compound was prepared as described in Example 136, except substituting 2-amino-6-chloropyridin-3-ol for1-(5-hydroxypyridin-2-yl)ethanone. ¹H NMR (400 MHz, DMSO-*d*₆) *δ* ppm 7.48 (t, *J =* 8.9 Hz, 1H), 7.12 - 6.99 (m, 2H), 6.86 (ddd, *J =* 8.9, 2.9, 1.1 Hz, 1H), 6.48 (d, *J =* 8.1 Hz, 1H), 4.47 (s, 2H), 4.40 (s, 2H), 2.31 (s, 6H). MS (APCI) *m*/*z* 469.2 (M+H)⁺.

### Example 138: 2-(4-chloro-3-fluorophenoxy)-N-(3-{2-[(2-chloro-6-methylpyridin-3-yl)oxy]acetamidolbicyclo[1.1.1]pentan-1-yl)acetamide (Compound 237)

The title compound was prepared as described in Example 136, except substituting 2-chloro-6-methylpyridin-3-ol for 1-(5-hydroxypyridin-2-yl)ethanone. ¹H NMR (400 MHz, DMSO-*d*₆) *δ* ppm 7.48 (t, *J =* 8.9 Hz, 1H), 7.12 - 6.99 (m, 2H), 6.86 (ddd, *J =* 8.9, 2.9, 1.1 Hz, 1H), 6.48 (d, *J =* 8.1 Hz, 1H), 4.47 (s, 2H), 4.40 (s, 2H), 2.31 (s, 6H). MS (APCI) *m*/*z* 469.2 (M+H)⁺.

### Example 139: 2-(4-chloro-3-fluorophenoxy)-N-(3-{2-[(6-cyanopyridin-3-yl)oxy]acetamidolbicyclo[1.1.1]pentan-1-yl)acetamide (Compound 238)

The title compound was prepared as described in Example 136, except substituting 5-hydroxypicolinonitrile for 1-(5-hydroxypyridin-2-yl)ethanone. ¹H NMR (400 MHz, DMSO-*d*₆) *δ* ppm 8.45 (d, *J = 2.9* Hz, 1H), 7.97 (d, *J =* 8.7 Hz, 1H), 7.56 - 7.45 (m, 2H), 7.05 (dd, *J =* 11.3, 2.8 Hz, 1H), 6.85 (ddd, *J* = 9.0, 2.9, 1.2 Hz, 1H), 4.65 (s, 2H), 4.46 (s, 2H), 2.27 (s, 6H). MS (APCI) *m*/*z* 445.2 (M+H)⁺.

### Example 140: 2-(4-chloro-3-fluorophenoxy)-N-(3-{2-[(2-ethyl-6-methylpyridin-3-yl)oxy]acetamidolbicyclo[1.1.1]pentan-1-yl)acetamide (Compound 239)

The title compound was prepared as described in Example 136, except substituting 2-ethyl-6-methylpyridin-3-ol for 1-(5-hydroxypyridin-2-yl)ethanone. ¹H NMR (400 MHz, DMSO-*d*₆) *δ* ppm 7.95 (d, *J =* 8.8 Hz, 1H), 7.64 (d, *J =* 8.9 Hz, 1H), 7.47 (t, *J =* 8.9 Hz, 1H), 7.04 (dd, *J =* 11.3, 2.9 Hz, 1H), 6.85 (ddd, *J* = 9.0, 2.9, 1.2 Hz, 1H), 4.75 (s, 2H), 4.46 (s, 2H), 2.96 (q, *J =* 7.5 Hz, 2H), 2.60 (s, 3H), 2.26 (s, 6H), 1.23 (t, *J =* 7.6 Hz, 3H). MS (APCI) *m*/*z* 462.3 (M+H)⁺.

### Example 141: 2-(4-chloro-3-fluorophenoxy)-N-(3-{2-[(5-chloro-6-fluoropyridin-3-yl)oxy]acetamidolbicyclo[1.1.1]pentan-1-yl)acetamide (Compound 240)

The title compound was prepared as described in Example 136, except substituting 5-chloro-6-fluoropyridin-3-ol for 1-(5-hydroxypyridin-2-yl)ethanone. ¹H NMR (400 MHz, DMSO-*d*₆) *δ* ppm 7.92 - 7.84 (m, 2H), 7.48 (t, *J =* 8.9 Hz, 1H), 7.05 (dd, *J =* 11.3, 2.8 Hz, 1H), 6.89 - 6.84 (m, 1H), 4.56 (s, 2H), 4.46 (s, 2H), 2.27 (s, 6H). MS (APCI) *m*/*z* 472.2 (M+H)⁺.

### Example 142 2-(4-chloro-3-fluorophenoxy)-N-(3-{2-[(6-methoxypyridin-3-yl)oxy]acetamidolbicyclo[1.1.1]pentan-1-yl)acetamide (Compound 241)

The title compound was prepared as described in Example 136, except substituting 6-methoxypyridin-3-ol for 1-(5-hydroxypyridin-2-yl)ethanone. ¹H NMR (400 MHz, DMSO-*d*₆) *δ* ppm 7.83 (dd, *J = 3.1,* 0.7 Hz, 1H), 7.49 (d, *J =* 8.8 Hz, 1H), 7.45 - 7.41 (m, 1H), 7.05 (dd, *J* = 11.3, 2.8 Hz, 1H), 6.85 (ddd, *J =* 9.0*,* 2.8, 1.2 Hz, 1H), 6.78 (dd, *J =* 9.0, 0.6 Hz, 1H), 4.46 (s, 2H), 4.42 (s, 2H), 3.78 (s, 3H), 2.27 (s, 6H). MS (APCI) *m*/*z* 450.2 (M+H)⁺.

### Example 143: 2-(4-chloro-3-fluorophenoxy)-N-(3-{2-[(6-cyano-5-methylpyridin-3-yl)oxy]acetamidolbicyclo[1.1.1]pentan-1-yl)acetamide (Compound 242)

The title compound was prepared as described in Example 136, except substituting 5-hydroxy-3-methylpicolinonitrile for 1-(5-hydroxypyridin-2-yl)ethanone. ¹H NMR (400 MHz, DMSO-*d*₆) *δ* ppm 8.28 (d, *J =* 2.7 Hz, 1H), 7.53 - 7.44 (m, 2H), 7.05 (dd, *J =* 11.3, 2.8 Hz, 1H), 6.85 (ddd, *J =* 8.9, 2.8, 1.2 Hz, 1H), 4.62 (s, 2H), 4.46 (s, 2H), 2.46 (s, 3H), 2.27 (s, 6H). MS (APCI) *m*/*z* 459.1 (M+H)⁺.

### Example 144: 2-(4-chloro-3-fluorophenoxy)-N-(3-{2-[(6-chloro-4-methylpyridin-3-yl)oxy]acetamido}bicyclo[1.1.1]pentan-1-yl)acetamide (Compound 243)

The title compound was prepared as described in Example 136, except substituting 6-chloro-4-methylpyridin-3-ol for 1-(5-hydroxypyridin-2-yl)ethanone. ¹H NMR (400 MHz, DMSO-*d*₆) *δ* ppm 7.88 (s, 1H), 7.47 (t, *J =* 8.9 Hz, 1H), 7.34 (d, *J =* 0.9 Hz, 1H), 7.05 (dd, *J* = 11.3, 2.9 Hz, 1H), 6.88 - 6.80 (m, 1H), 4.58 (s, 2H), 4.46 (s, 2H), 2.27 (s, 6H), 2.23 (d, *J =* 0.8 Hz, 3H). MS (APCI) *m*/*z* 468.2 (M+H)⁺.

### Example 145: 2-(4-chloro-3-fluorophenoxy)-N-(3-{2-[(6-fluoro-5-methylpyridin-3-yl)oxy]acetamidolbicyclo[1.1.1]pentan-1-yl)acetamide (Compound 244)

The title compound was prepared as described in Example 136, except substituting 6-fluoro-5-methylpyridin-3-ol for 1-(5-hydroxypyridin-2-yl)ethanone. ¹H NMR (400 MHz, DMSO-*d*₆) *δ* ppm 7.68 (s, 1H), 7.55 - 7.43 (m, 2H), 7.05 (dd, *J =* 11.3, 2.8 Hz, 1H), 6.85 (ddd, *J* = 9.0, 2.9, 1.2 Hz, 1H), 4.47 (d, *J* = 7.4 Hz, 4H), 2.27 (s, 6H), 2.21 (dt, *J =* 1.4, 0.7 Hz, 3H). MS (APCI) *m*/*z* 452.2 (M+H)⁺.

### Example 146: 2-(4-chloro-3-fluorophenoxy)-N-(3-{2-[(2-fluoro-6-methylpyridin-3-yl)oxy]acetamidolbicyclo[1.1.1]pentan-1-yl)acetamide (Compound 245)

The title compound was prepared as described in Example 136, except substituting 2-fluoro-6-methylpyridin-3-ol for 1-(5-hydroxypyridin-2-yl)ethanone. ¹H NMR (400 MHz, DMSO-*d*₆) *δ* ppm 7.48 (d, *J =* 8.9 Hz, 1H), 7.45 - 7.37 (m, 1H), 7.10 (d, *J =* 8.1 Hz, 1H), 7.08 - 7.03 (m, 1H), 6.85 (ddd, *J =* 9.0, 2.9, 1.2 Hz, 1H), 4.53 (s, 2H), 4.46 (s, 2H), 2.33 (s, 3H), 2.25 (s, 6H). MS (APCI) *m*/*z* 452.2 (M+H)⁺.

### Example 147: 2-(4-chloro-3-fluorophenoxy)-N-(3-{2-[(6-fluoro-5-methoxypyridin-3-yl)oxy]acetamidolbicyclo[1.1.1]pentan-1-yl)acetamide (Compound 246)

The title compound was prepared as described in Example 136, except substituting 6-fluoro-5-methoxypyridin-3-ol for 1-(5-hydroxypyridin-2-yl)ethanone. ¹H NMR (400 MHz, DMSO-*d*₆) *δ* ppm 7.48 (t, *J =* 8.9 Hz, 1H), 7.37 (t, *J =* 2.6 Hz, 1H), 7.30 (dd, *J =* 8.7, 2.6 Hz, 1H), 7.05 (dd, *J =* 11.3, 2.8 Hz, 1H), 6.85 (ddd, *J =* 8.9, 2.9, 1.2 Hz, 1H), 4.51 (s, 2H), 4.46 (s, 2H), 3.86 (s, 3H), 2.28 (s, 6H). MS (APCI) *m*/*z* 468.2 (M+H)⁺.

### Example 148: 2-(4-chloro-3-fluorophenoxy)-N-(3-{2-[(6-fluoro-4-methylpyridin-3-yl)oxy]acetamidolbicyclo[1.1.1]pentan-1-yl)acetamide (Compound 247)

The title compound was prepared as described in Example 136, except substituting 6-fluoro-4-methylpyridin-3-ol for 1-(5-hydroxypyridin-2-yl)ethanone. ¹H NMR (400 MHz, DMSO-*d*₆) *δ* ppm 7.66 (d, *J =* 1.6 Hz, 1H), 7.48 (t, *J =* 8.9 Hz, 1H), 7.12 - 6.99 (m, 2H), 6.90 - 6.82(m, 1H), 4.54 (s, 2H), 4.46 (s, 2H), 2.27 (s, 9H). MS (APCI) *m*/*z* 452.2 (M+H)⁺.

### Example 149: 2-(4-chloro-3-fluorophenoxy)-N-[3-(2-{[6-(propan-2-yl)pyridin-3-yl]oxy}acetamido)bicyclo[1.1.1]pentan-1-yl]acetamide (Compound 248)

The title compound was prepared as described in Example 136, except substituting 6-isopropylpyridin-3-ol for 1-(5-hydroxypyridin-2-yl)ethanone. ¹H NMR (400 MHz, DMSO-*d*₆) *δ* ppm 8.40 (d, *J =* 2.9 Hz, 1H), 7.99 (dd, *J =* 9.0, 2.9 Hz, 1H), 7.79 (d, *J =* 9.0 Hz, 1H), 7.48 (t, *J* = 8.9 Hz, 1H), 7.04 (dd, *J =* 11.3, 2.8 Hz, 1H), 6.85 (ddd, *J =* 9.0, 2.9, 1.3 Hz, 1H), 4.67 (s, 2H), 4.46 (s, 2H), 3.29 - 3.16 (m, 1H), 2.28 (s, 6H), 1.29 (d, *J =* 7.0 Hz, 6H). MS (APCI) *m*/*z* 462.3 (M+H)⁺.

### Example 150: 2-(4-chloro-3-fluorophenoxy)-N-(3-{2-[(6-fluoro-2-methylpyridin-3-yl)oxy]acetamidolbicyclo[1.1.1]pentan-1-yl)acetamide (Compound 249)

The title compound was prepared as described in Example 136, except substituting 6-fluoro-2-methylpyridin-3-ol for 1-(5-hydroxypyridin-2-yl)ethanone. ¹H NMR (400 MHz, DMSO-*d*₆) *δ* ppm 7.49 (d, *J =* 8.8 Hz, 1H), 7.46 - 7.38 (m, 1H), 6.98 - 6.90 (m, 1H), 6.85 (ddd, *J=* 8.9, 2.8, 1.2 Hz, 1H), 4.50 (s, 2H), 4.46 (s, 2H), 2.35 (s, 3H), 2.26 (s, 6H). MS (APCI) *m*/*z* 452.2 (M+H)⁺.

### Example 151: 2-[(6-amino-5-chloropyridin-3-yl)oxy]-N-{3-[2-(4-chloro-3-fluorophenoxy)acetamido]bicyclo[1.1.1]pentan-1-yl}acetamide (Compound 250)

The title compound was prepared as described in Example 136, except substituting 6-amino-5-chloropyridin-3-ol for 1-(5-hydroxypyridin-2-yl)ethanone. ¹H NMR (400 MHz, DMSO-*d*₆) *δ* ppm 7.71 (d, *J =* 2.6 Hz, 1H), 7.64 (d, *J =* 2.6 Hz, 1H), 7.48 (t, *J =* 8.9 Hz, 1H), 7.05 (dd, *J=* 11.3, 2.9 Hz, 1H), 6.85 (ddd, *J =* 9.0, 2.8, 1.2 Hz, 1H), 4.46 (s, 2H), 4.40 (s, 2H), 2.27 (s, 6H). MS (APCI) *m*/*z* 469.2 (M+H)⁺.

### Example 152: 2-(4-chloro-3-fluorophenoxy)-N-(3-{2-[(6-cyclopropylpyridin-3-yl)oxy]acetamidoIbicyclo[1.1.1]pentan-1-yl)acetamide (Compound 251)

The title compound was prepared as described in Example 136, except substituting 6-cyclopropylpyridin-3-ol for 1-(5-hydroxypyridin-2-yl)ethanone. ¹H NMR (400 MHz, DMSO-*d*₆) *δ* ppm 8.28 (d, *J =* 2.9 Hz, 1H), 7.82 (dd, *J =* 9.0, 2.9 Hz, 1H), 7.54 - 7.42 (m, 2H), 7.04 (dd, *J=* 11.3, 2.8 Hz, 1H), 6.85 (ddd, *J =* 9.0*,* 2.9, 1.3 Hz, 1H), 4.61 (s, 2H), 4.46 (s, 2H), 2.27 (s, 6H), 2.26 - 2.18 (m, 1H), 1.22 - 1.15 (m, 2H), 1.04 - 0.94 (m, 2H). MS (APCI) *m*/*z* 460.3 (M+H)⁺.

### Example 153: 2-(4-chloro-3-fluorophenoxy)-N-(3-{2-[(6-methoxy-2-methylpyridin-3-yl)oxy]acetamidolbicyclo[1.1.1]pentan-1-yl)acetamide (Compound 252)

The title compound was prepared as described in Example 136, except substituting 6-methoxy-2-methylpyridin-3-ol for 1-(5-hydroxypyridin-2-yl)ethanone. ¹H NMR (400 MHz, DMSO-*d*₆) *δ* ppm 7.47 (t, *J =* 8.8 Hz, 1H), 7.32 (d, *J =* 8.9 Hz, 1H), 7.05 (dd, *J =* 11.3, 2.8 Hz, 1H), 6.85 (ddd, *J* = 9.0, 2.8, 1.2 Hz, 1H), 6.63 (dd, *J =* 8.8, 0.8 Hz, 1H), 4.46 (s, 2H), 4.40 (s, 2H), 3.78 (s,3H), 2.35 (s, 3H), 2.26 (s, 6H). MS (APCI) *m*/*z* 464.2 (M+H)⁺.

### Example 154: 2-(4-chloro-3-fluorophenoxy)-N-(3-{2-[(6-methoxy-5-methylpyridin-3-yl)oxy]acetamidolbicyclo[1.1.1]pentan-1-yl)acetamide (Compound 253)

The title compound was prepared as described in Example 136, except substituting 6-methoxy-5-methylpyridin-3-ol for 1-(5-hydroxypyridin-2-yl)ethanone. ¹H NMR (400 MHz, DMSO-*d*₆) *δ* ppm 7.64 (dd, *J =* 3.0, 0.8 Hz, 1H), 7.48 (t, *J =* 8.9 Hz, 1H), 7.29 (dd, *J =* 3.0, 0.9 Hz, 1H), 7.05 (dd, *J =* 11.3, 2.8 Hz, 1H), 6.85 (ddd, *J =* 9.0*,* 2.9, 1.2 Hz, 1H), 4.46 (s, 2H), 4.40 (s, 2H), 3.81 (s, 3H), 2.27 (s, 6H), 2.11 (t, *J =* 0.8 Hz, 3H). MS (APCI) m/z 464.2 (M+H)⁺.

### Example 155: 2-(4-chloro-3-fluorophenoxy)-N-(3-{2-[(1H-pyrrolo[3,2-b]pyridin-6-yl)oxy]acetamidolbicyclo[1.1.1]pentan-1-yl)acetamide(Compound 254)

The title compound was prepared as described in Example 136, except substituting 1*H-*pyrrolo[3,2-b]pyridin-6-ol for 1-(5-hydroxypyridin-2-yl)ethanone. ¹H NMR (400 MHz, DMSO-*d*₆) *δ* ppm 8.49 (d, *J =* 2.3 Hz, 1H), 8.18 (dd, *J =* 2.3, 0.9 Hz, 1H), 8.02 (d, *J =* 3.3 Hz, 1H), 7.48 (t, *J = 8.9* Hz, 1H), 7.04 (dt, *J =* 11.4, 3.6 Hz, 1H), 6.85 (ddd, *J* = 9.0, 2.9, 1.2 Hz, 1H), 6.76 (dd, *J =* 3.3, 0.9 Hz, 1H), 4.69 (s, 2H), 4.46 (d, *J* = 5.4 Hz, 2H), 2.27 (d, *J =* 12.5 Hz, 6H). MS (APCI) *m*/*z* 459.2 (M+H)⁺.

### Example 156: 2-(4-chloro-3-fluorophenoxy)-N-(3-[2-[(furo[3,2-b]pyridin-6-yl)oxy]acetamidolbicyclo[1.1.1]pentan-1-yl)acetamide (Compound 255)

The title compound was prepared as described in Example 136, except substituting furo[3,2-b]pyridin-6-ol for 1-(5-hydroxypyridin-2-yl)ethanone. ¹H NMR (400 MHz, DMSO-*d*₆) *δ* ppm 8.37 (d, *J =* 2.4 Hz, 1H), 8.17 (d, *J =* 2*.*3 Hz, 1H), 7.76 (dd, *J =* 2.5, 1.0 Hz, 1H), 7.47 (t, *J* = 8.9 Hz, 1H), 7.10 7.02 (m, 2H), 6.85 (ddd, *J =* 8.9, 2.9, 1.2 Hz, 1H), 4.58 (s, 2H), 4.46 (s, 2H), 2.28 (s, 6H). MS (APCI) *m*/*z* 460.2 (M+H)⁺.

### Example 157: 2-(4-chloro-3-fluorophenoxy)-N-[3-(2-{[6-(trifluoromethyl)pyridazin-3-yl]oxy}acetamido)bicyclo[1.1.1]pentan-1-yl]acetamide (Compound 201)

A mixture of Example 112B (0.06 g, 0.175 mmol) and 3-chloro-6-(trifluoromethyl)pyridazine (0.038 g, 0.210 mmol) in *N,N*-dimethylformamide (1.5 mL) was added sodium hydride (8.75 mg, 0.219 mmol), and the reaction mixture was stirred at room temperature for 1.5 hours. The mixture was then concentrated under high vacuum, and the residue was purified by HPLC (10-85% acetonitrile in 0.1% trifluoroacetic acid/water at 25 mL/minute on a Phenomenex^{®} Luna^{®} C18 5 µm 100 Å AXIA^{™} column (250 mm × 21.2 mm)) to give 26.5 mg of the title compound as a white solid. ¹H NMR (400 MHz, DMSO-*d*₆) *δ* ppm 8.77 (s, 1H), 8.67 (s, 1H), 8.15 (d, *J* = 9.3 Hz, 1H), 7.55 (d, *J* = 9.2 Hz, 1H), 7.46 (t, *J* = 8.9 Hz, 1H), 7.03 (dd, *J* = 11.4, 2.8 Hz, 1H), 6.81 (ddd, *J* = 9.0, 2.9, 1.2 Hz, 1H), 4.94 (s, 2H), 4.44 (s, 2H), 2.22 (s, 6H). MS (ESI+) *m*/*z* 488.9 (M+H)^{+.}

### Example 158: 2-(4-chloro-3-fluorophenoxy)-N-[3-(2-{[6-(methanesulfonyl)pyridin-3-yl]oxy}acetamido)bicyclo[1.1.1]pentan-1-yl]acetamide (Compound 257)

A mixture of Example 112B (0.06 g, 0.175 mmol), 5-chloro-2-(methylsulfonyl)pyridine (0.034 g, 0.175 mmol), and cesium carbonate (0.057 g, 0.175 mmol) in N-methyl-2-pyrrolidone (0.5 mL) was irradiated in a Biotage^{®} Initiator microwave reactor at 120 °C (0-450 W) for 0.5 hours. The reaction mixture was concentrated under high vacuum, and the residue was purified by HPLC (10-85% acetonitrile in 0.1% trifluoroacetic acid/water at 25 mL/minute on a Phenomenex^{®} Luna^{®} C18 5 µm 100 Å AXIA^{™} column (250 mm × 21.2 mm)) to give 28 mg of the title compound as a white solid. ¹H NMR (400 MHz, DMSO-*d*₆) *δ* ppm 8.68 (d, *J* = 7.0 Hz, 2H), 8.60 (d, *J* = 2.6 Hz, 1H), 8.18 (dd, *J* = 8.8, 2.6 Hz, 1H), 7.46 (t, *J* = 8.9 Hz, 1H), 7.12 6.99 (m, 2H), 6.81 (ddd, *J* = 8.9, 2.8, 1.2 Hz, 1H), 4.77 (s, 2H), 4.44 (s, 2H), 3.23 (s, 3H), 2.21 (s, 6H). MS (ESI+) *m*/*z* 431.0 (M+H)⁺.

### Example 159: 2-(4-chloro-3-fluorophenoxy)-N-(3-{2-[(2,2-difluoro-2H-1,3-benzodioxol-5-yl)oxy]acetamidolbicyclo[1.1.1]pentan-1-yl)acetamide (Compound 258)

### Example 159A 2,2-difluorobenzo[d][1,3]dioxol-5-ol

To a cold solution of 5-bromo-2,2-difluorobenzo[d][1,3]dioxole (5.75 mL, 42.2 mmol) in tetrahydrofuran (80 mL) was added a 2.0 M solution of isopropylmagnesium chloride in tetrahydrofuran (28.1 mL, 56.1 mmol) within 5-10 minutes while maintaining the temperature in the range of 10-20 °C. The reaction mixture was stirred at the same temperature for another 15 minutes and then allowed to attain room temperature with continued overnight stirring. The reaction mixture was cooled with an ice bath, triisopropyl borate (12.74 mL, 54.9 mmol) was added dropwise over 2 minutes, and stirring at room temperature was continued for 30 minutes. The reaction mixture was cooled to 10 °C and 10% H₂SO₄ solution (50 mL) was added slowly which resulted in a slight exotherm to 20 °C. After stirring for 15 minutes, the mixture was partitioned between water and ethyl acetate, and the combined organic extracts were washed with saturated NaHCO₃ solution. The organic layer was separated, dried over magnesium sulfate, filtered, and concentrated. The residue was dissolved in 100 mL of *tert-butyl* methyl ether and cooled to 0 °C. 30% Hydrogen peroxide solution in water (5.39 mL, 52.7 mmol) was added slowly, followed by water (60 mL), and the mixture was stirred overnight while warming up to ambient temperature. The reaction mixture was diluted with ethyl acetate and washed twice with sodium thiosulfate solution and brine. The organic layer was dried with magnesium sulfate and filtered. The filtrate was concentrated, and the residue was purified on silica gel (0-50% ethyl acetate in heptane) to give 6.43 g of the title compound as an amber oil. ¹H NMR (400 MHz, DMSO-*d*₆) *δ* ppm 9.75 (s, 1H), 7.12 (d, *J* = 8.7 Hz, 1H), 6.75 (d, *J* = 2.4 Hz, 1H), 6.52 (dd, *J* = 8.7, 2.5 Hz, 1H). MS (ESI-) *m*/*z* 173.1 (M-H)⁻.

### Example 159B 2-(4-chloro-3-fluorophenoxy)-N-(3-{2-[(2,2-difluoro-2H-1,3-benzodioxol-5-yl)oxy]acetamido}bicyclo[1.1.1]pentan-1-yl)acetamide

The title compound was prepared as described in Example 127, except substituting 2,2-difluorobenzo[*d*][1,3]dioxol-5-ol (Example 159A) for 6-chloro-5-methylpyridin-3-ol. ¹H NMR (400 MHz, DMSO-*d*₆) *δ* ppm 8.67 (d, *J* = 9.1 Hz, 2H), 7.46 (t, *J* = 8.9 Hz, 1H), 7.29 (d, *J* = 8.9 Hz, 1H), 7.10 (d, *J* = 2.6 Hz, 1H), 7.04 (dd, *J* = 11.4, 2.8 Hz, 1H), 6.82 (ddd, *J* = 9.0, 2.9, 1.2 Hz, 1H), 6.73 (dd, *J* = 8.9, 2.6 Hz, 1H), 4.44 (s, 2H), 4.41 (s, 2H), 2.24 (s, 6H). MS (ESI+) *m*/*z* 498.9 (M+H)⁺.

### Example 160: 2-{[6-(aminomethyl)pyridin-3-yl]oxy}-N-{3-[2-(4-chloro-3-fluorophenoxy)acetamido]bicyclo[1.1.1]pentan-1-yl}acetamide (Compound 259)

A mixture of Example 28A (0.038 g, 0.089 mmol), *tert-butyl* ((5-hydroxypyridin-2-yl)methyl)carbamate (0.04 g, 0.178 mmol), potassium carbonate (0.025 g, 0.178 mmol) and potassium iodide (1.036 mg, 6.24 µmol) in acetone (1.5 mL) was stirred at 140 °C (0-450 W) in a Biotage^{®} Initiator microwave reactor for 45 minutes. The reaction mixture was concentrated. A mixture of the residue, 4 N HCl in dioxane (1.1 mL), and dichloromethane (5 mL) was stirred at ambient temperature overnight. After concentration, the residue was purified by HPLC (10-85% acetonitrile in 0.1% trifluoroacetic acid/water at 25 mL/minute on a Phenomenex^{®} Luna^{®} C18 5 µm 100 Å AXIA^{™} column (250 mm × 21.2 mm)) to give 48 mg of the title compound as a solid. ¹H NMR (500 MHz, DMSO-*d*₆) *δ* ppm 8.80 (s, 1H), 8.75 (s, 1H), 8.34 (dd, *J* = 2.7, 0.8 Hz, 1H), 8.31 - 8.16 (m, 3H), 7.54 - 7.40 (m, 3H), 7.08 (dd, *J* = 11.3, 2.8 Hz, 1H), 6.86 (ddd, *J* = 9.0, 2.9, 1.2 Hz, 1H), 4.58 (s, 2H), 4.48 (s, 2H), 4.12 (q, *J* = 5.8 Hz, 2H), 2.27 (s, 6H). MS (ESI+) m/z 449.1 (M+H)⁺.

### Example 161: 2-(4-chloro-3-fluorophenoxy)-N-{3-[2-(4-chloro-3-iodophenoxy)acetamido]-bicyclo[1.1.1]pentan-1-yl}acetamide (Compound 260)

### Example 161A: 2-(4-chloro-3-iodophenoxy)acetic acid

The title compound was prepared as described in Example 129A, except substituting methyl 4-chloro-3-iodophenol for 4-chloro-3-nitrophenol. ¹H NMR (400 MHz, DMSO-*d*₆) *δ* ppm 12.97 (s, 1H), 7.46 7.38 (m, 2H), 6.95 (dd, *J* = 8.9, 3.0 Hz, 1H), 4.68 (s, 2H).

### Example 161B: 2-(4-chloro-3-fluorophenoxy)-N-{3-[2-(4-chloro-3-iodophenoxy)acetamido]bicyclo-[1.1.1]pentan-1-yl}acetamide

The title compound was prepared as described in Example 124B, except substituting Example 161A for 2-(4-chloro-3-(methoxycarbonyl)phenoxy)acetic acid. ¹H NMR (500 MHz, DMSO-*d*₆) *δ* ppm 8.73 (d, *J* = 6.2 Hz, 2H), 7.56 7.46 (m, 3H), 7.08 (dd, *J* = 11.4, 2.9 Hz, 1H), 7.02 (dd, *J* = 8.9, 3.0 Hz, 1H), 6.86 (ddd, *J* = 9.0, 2.9, 1.1 Hz, 1H), 4.48 (d, *J* = 11.4 Hz, 4H), 2.28 (s, 6H). MS (ESI+) *m*/*z* 578.8 (M+H)⁺.

### Example 162: 2-[4-chloro-3-(3,5-dimethyl-1,2-oxazol-4-yl)phenoxy]-N-{3-[2-(4-chloro-3-fluorophenoxy)acetamido]bicyclo[1.1.1]pentan-1-yl}acetamide (Compound 261)

A mixture of Example 161B (0.1 g, 0.173 mmol), (3,5-dimethylisoxazol-4-yl)boronic acid (0.036 g, 0.259 mmol), and sodium carbonate (0.173 mL, 0.345 mmol) in *N,N-*dimethylformamide (1.5 mL) was degassed and purged with argon. Then bis(triphenylphosphine)palladium(II) chloride (0.012 g, 0.017 mmol) was added, and the mixture was sparged with argon for 5 minutes. The vessel was sealed and then heated in a Biotage^{®} Initiator microwave reactor at 145 °C (0-450 W) for 60 minutes. The suspension was cooled and filtered, and the filtrate was concentrated under vacuum. The residue was purified by HPLC (15∼95% acetonitrile in 0.1% trifluoroacetic acid/water at 25 mL/minute on a Phenomenex^{®} Luna^{®} C18 5 µm 100 Å AXIA^{™} column (250 mm × 21.2 mm)) to give 62 mg of the title compound as an off-white solid. ¹H NMR (500 MHz, DMSO-*d*₆) *δ* ppm 8.73 (s, 1H), 8.72 (s, 1H), 7.56 - 7.46 (m, 2H), 7.11 - 7.04 (m, 2H), 6.98 (d, *J* = 3.0 Hz, 1H), 6.90 - 6.83 (m, 1H), 4.50 (s, 2H), 4.49 (s, 2H), 2.28 (s, 3H), 2.27 (s, 6H), 2.10 (s, 3H). MS (ESI+) *m*/*z* 548.0 (M+H)⁺.

### Example 163: 2-[4-chloro-3-(cyanomethyl)phenoxy]-N-{3-[2-(4-chloro-3-fluorophenoxy)acetamido]bicyclo[1.1.1]pentan-1-yl}acetamide (Compound 262)

The title compound was obtained as a byproduct in the procedure described in Example 162. ¹H NMR (501 MHz, DMSO-*d*₆) *δ* ppm 8.71 (d, *J* = 15.1 Hz, 2H), 7.51 - 7.40 (m, 2H), 7.15 (d, *J* = 3.0 Hz, 1H), 7.05 (dd, *J* = 11.4, 2.9 Hz, 1H), 6.96 (dd, *J* = 8.9, 3.1 Hz, 1H), 6.83 (ddd, *J* = 8.9, 2.9, 1.2 Hz, 1H), 4.45 (d, *J* = 7.8 Hz, 4H), 4.03 (s, 2H), 2.25 (s, 6H). MS (ESI+) *m*/*z* 492.0 (M+H)⁺.

### Example 164: 2-(4-chloro-3-fluorophenoxy)-N-[3-(2-{[6-(1-hydroxyethyl)pyridin-3-yl]oxy}acetamido)bicyclo[1.1.1]pentan-1-yl]acetamide (Compound 263)

A mixture of Example 28A (0.1 g, 0.235 mmol), 1-(5-hydroxypyridin-2-yl)ethanone (0.065 g, 0.471 mmol), potassium carbonate (0.065 g, 0.471 mmol) and potassium iodide (2.73 mg, 0.016 mmol) in acetone (2.0 mL) was stirred at 140 °C (0-450 W) in a Biotage^{®} Initiator microwave reactor for 45 minutes. The suspension was filtered, and the filtrate was concentrated. This residue and NaBH₄ (0.089 g, 2.35 mmol) in methanol was stirred at ambient temperature overnight. The reaction mixture was concentrated, and the residue was purified by HPLC (10-85% acetonitrile in 0.1% trifluoroacetic acid/water at 25 mL/minute on a Phenomenex^{®} Luna^{®} C18 5 µm 100 Å AXIA^{™} column (250 mm × 21.2 mm)) to give 59 mg of the title compound as a white solid. ¹H NMR (400 MHz, DMSO-*d*₆) *δ* ppm 8.74 (d, *J* = 26.6 Hz, 2H), 8.30 (d, *J* = 2.8 Hz, 1H), 7.81 - 7.60 (m, 2H), 7.47 (t, *J* = 8.9 Hz, 1H), 7.05 (dd, *J =* 11.4, 2.8 Hz, 1H), 6.83 (ddd, *J* = 9.0, 2.9, 1.2 Hz, 1H), 4.85 (q, *J* = 6.5 Hz, 1H), 4.61 (s, 2H), 4.46 (s, 2H), 2.25 (s, 6H), 1.37 (d, *J* = 6.5 Hz, 3H). MS (ESI+) *m*/*z* 464.0 (M+H)⁺.

### Example 165: methyl 5-[2-({3-[2-(4-chloro-3-fluorophenoxy)acetamido]bicyclo-[1.1.1]pentan-1-yl}amino)-2-oxoethoxy]pyridine-2-carboxylate (Compound 264)

The title compound was prepared as described in Example 127, except substituting methyl 5-hydroxypicolinate for 6-chloro-5-methylpyridin-3-ol. ¹H NMR (400 MHz, DMSO-*d*₆) *δ* ppm 8.80 (s, 1H), 8.69 (s, 1H), 8.53 (d, *J* = 5.7 Hz, 1H), 7.62 (d, *J* = 2.6 Hz, 1H), 7.46 (t, *J =* 8.9 Hz, 1H), 7.23 (dd, *J* = 5.8, 2.6 Hz, 1H), 7.04 (dd, *J* = 11.4, 2.9 Hz, 1H), 6.82 (ddd, *J* = 9.0, 2.8, 1.2 Hz, 1H), 4.64 (s, 2H), 4.45 (s, 2H), 3.86 (s, 3H), 2.24 (s, 6H). MS (ESI+) *m*/*z* 431.0 (M+H)⁺.

### Example 166: 2-[(2,2-difluoro-2H-1,3-benzodioxol-5-yl)oxy]-N-[3-(2-{[2-(trifluoromethyl)pyridin-4-yl]oxy}acetamido)bicyclo[1.1.1]pentan-1-yl]acetamide (Compound 265)

### Example 166A: 2-((2,2-difluorobenzo[d][1,3]dioxol-5-yl)oxy)acetic acid

The title compound was prepared as described in Example 129A, except substituting Example 159A for 4-chloro-3-nitrophenol. ¹H NMR (400 MHz, DMSO-*d*₆) *δ* ppm 13.10 (s, 1H), 7.30 (d, *J* = 8.9 Hz, 1H), 7.13 (d, *J* = 2.6 Hz, 1H), 6.73 (dd, *J* = 8.9, 2.6 Hz, 1H), 4.69 (s, 2H).

### Example 166B: N-(3-aminobicyclo[1.1.1]pentan-1-yl)-2-((2,2-difluorobenzo[d][1,3]dioxol-5-yl)oxy)acetamide hydrochloride

The title compound was prepared as described in Example 9A and Example 9B, except substituting 2-((2,2-difluorobenzo[*d*][1,3]dioxol-5-yl)oxy)acetic acid (Example 166A) for 2-(4-chloro-3-fluorophenoxy)acetic acid. ¹H NMR (400 MHz, DMSO-*d*₆) *δ* ppm 9.01 (s, 3H), 8.88 (s, 1H), 7.29 (d, *J* = 8.9 Hz, 1H), 7.11 (d, *J* = 2.5 Hz, 1H), 6.73 (dd, *J* = 8.9, 2.6 Hz, 1H), 4.44 (s, 2H), 2.21 (s, 6H).

### Example 166C: 2-chloro-N-(3-(2-((2,2-difluorobenzo[d][1,3]dioxol-5-yl)oxy)acetamido)bicyclo[1.1.1]pentan-1-yl)acetamide

The title compound was prepared as described in Example 28A, except substituting Example 166B for Example 27D. ¹H NMR (400 MHz, DMSO-*d*₆) *δ* ppm 8.81 (s, 1H), 8.68 (s, 1H), 7.30 (d, *J* = 8.9 Hz, 1H), 7.12 (d, *J* = 2.6 Hz, 1H), 6.75 (dd, *J* = 8.8, 2.6 Hz, 1H), 4.42 (s, 2H), 3.97 (s, 2H), 2.23 (s, 6H). MS (ESI+) *m*/*z* 388.9 (M+H)⁺.

### Example 166D: 2-[(2,2-difluoro-2H-1,3-benzodioxol-5-yl)oxy]-N-[3-(2-([2-(trifluoromethyl)pyridin-4-yl]oxy}acetamido)bicyclo[1.1.1]pentan-1-yl]acetamide

The title compound was prepared as described in Example 127, except substituting Example 166C for Example 28A and 2-(trifluoromethyl)pyridin-4-ol for 6-chloro-5-methylpyridin-3-ol. ¹H NMR (501 MHz, DMSO-*d*₆) *δ* ppm 8.79 (s, 1H), 8.68 (s, 1H), 8.57 (d, *J* = 5.7 Hz, 1H), 7.43 (d, *J* = 2.4 Hz, 1H), 7.30 (d, *J* = 8.9 Hz, 1H), 7.24 (dd, *J* = 5.7, 2.5 Hz, 1H), 7.12 (d, *J* = 2.5 Hz, 1H), 6.75 (dd, *J* = 8.9, 2.6 Hz, 1H), 4.67 (s, 2H), 4.42 (s, 2H), 2.25 (s, 6H). MS (ESI+) *m*/*z* 516.0 (M+H)⁺.

### Example 167: 2-[(2,2-difluoro-2H-1,3-benzodioxol-5-yl)oxy]-N-[3-(2-1[6-(trifluoromethyl)-pyridin-3-yl]oxy}acetamido)bicyclo[1.1.1]pentan-1-yl]acetamide (Compound 266)

The title compound was prepared as described in Example 127, except substituting Example 166C for Example 28A and 6-(trifluoromethyl)pyridin-3-ol for 6-chloro-5-methylpyridin-3-ol. ¹H NMR (400 MHz, DMSO-*d*₆) *δ* ppm 8.77 (s, 1H), 8.66 (s, 1H), 8.43 (d, *J* = 2.9 Hz, 1H), 7.83 (d, *J* = 8.8 Hz, 1H), 7.53 (dd, *J* = 8.7, 2.9 Hz, 1H), 7.28 (d, *J* = 8.9 Hz, 1H), 7.10 (d, *J* = 2.6 Hz, 1H), 6.73 (dd, *J* = 8.9, 2.6 Hz, 1H), 4.63 (s, 2H), 4.40 (s, 2H), 2.24 (s, 6H). MS (ESI+) *m*/*z* .516.0 (M+H)⁺.

### Example 168: 2-[(6-cyanopyridin-3-yl)oxy]-N-(3-[2-[(2,2-difluoro-2H-1,3-benzodioxol-5-yl)oxy]acetamidolbicyclo[1.1.1]pentan-1-yl)acetamide (Compound 267)

The title compound was prepared as described in Example 127, except substituting Example 166C for Example 28A and 5-hydroxypicolinonitrile for 6-chloro-5-methylpyridin-3-ol. ¹H NMR (400 MHz, DMSO-*d*₆) *δ* ppm 8.77 (s, 1H), 8.66 (s, 1H), 8.43 (d, *J* = 2.9 Hz, 1H), 7.97 (d, *J* = 8.7 Hz, 1H), 7.51 (dd, *J* = 8.7, 2.9 Hz, 1H), 7.28 (d, *J* = 8.9 Hz, 1H), 7.10 (d, *J =* 2.6 Hz, 1H), 6.73 (dd, *J* = 8.9, 2.6 Hz, 1H), 4.64 (s, 2H), 4.40 (s, 2H), 2.23 (s, 6H). MS (ESI-) *m*/*z* 470.9 (M-H)⁻.

### Example 169: 2-[(6-cyclopropylpyridin-3-yl)oxy]-N-(3-{2-[(2,2-difluoro-2H-1,3-benzodioxol-5-yl)oxy]acetamido}bicyclo[1.1.1]pentan-1-yl)acetamide (Compound 268)

The title compound was prepared as described in Example 127, except substituting Example 166C for Example 28A and 6-cyclopropylpyridin-3-ol for 6-chloro-5-methylpyridin-3-ol. ¹H NMR (400 MHz, DMSO-*d*₆) *δ* ppm 8.69 (d, *J* = 20.8 Hz, 2H), 8.21 (d, *J* = 2.9 Hz, 1H), 7.53 (dd, *J* = 8.9, 2.9 Hz, 1H), 7.30 (dd, *J* = 15.3, 8.9 Hz, 2H), 7.10 (d, *J* = 2.6 Hz, 1H), 6.73 (dd, *J* = 8.9, 2.6 Hz, 1H), 4.52 (s, 2H), 4.40 (s, 2H), 2.23 (s, 6H), 2.10 (tt, *J* = 8.2, 4.9 Hz, 1H), 1.04 0.92 (m, 2H), 0.88 (dt, *J* = 4.8, 3.1 Hz, 2H). MS (ESI+) *m*/*z* 488.1 (M+H)⁺.

### Example 170: 2-[(2,2-difluoro-2H-1,3-benzodioxol-5-yl)oxy]-N-[3-(2-1[5-(trifluoromethyl)pyridin-3-yl]oxy}acetamido)bicyclo[1.1.1]pentan-1-yl]acetamide (Compound 269)

The title compound was prepared as described in Example 127, except substituting Example 166C for Example 28A and 5-(trifluoromethyl)pyridin-3-ol for 6-chloro-5-methylpyridin-3-ol. ¹H NMR (400 MHz, DMSO-*d*₆) *δ* ppm 8.76 (s, 1H), 8.66 (s, 1H), 8.56 (dd, *J* = 15.2, 2.2 Hz, 2H), 7.71 (t, *J* = 2.3 Hz, 1H), 7.29 (d, *J* = 8.9 Hz, 1H), 7.10 (d, *J* = 2.5 Hz, 1H), 6.73 (dd, *J* = 8.9, 2.6 Hz, 1H), 4.64 (s, 2H), 4.41 (s, 2H), 2.24 (s, 6H). MS (ESI+) *m*/*z* 516.0 (M+H)⁺.

### Example 171: 2-[(2-cyanopyridin-4-yl)oxy]-N-(3-{2-[(2,2-difluoro-2H-1,3-benzodioxol-5-yl)oxy]acetamido}bicyclo[1.1.1]pentan-1-yl)acetamide (Compound 270)

The title compound was prepared as described in Example 127, except substituting Example 166C for Example 28A and 4-hydroxypicolinonitrile for 6-chloro-5-methylpyridin-3-ol. ¹H NMR (400 MHz, DMSO-*d*₆) *δ* ppm 8.76 (s, 1H), 8.66 (s, 1H), 8.52 (d, *J* = 5.8 Hz, 1H), 7.65 (d, *J* = 2.5 Hz, 1H), 7.33 7.21 (m, 2H), 7.10 (d, *J* = 2.5 Hz, 1H), 6.73 (dd, *J* = 8.9, 2.6 Hz, 1H), 4.64 (s, 2H), 4.40 (s, 2H), 2.23 (s, 6H). MS (ESI+) *m*/*z* 473.0 (M+H)⁺.

### Example 172: 2-[(2,2-difluoro-2H-1,3-benzodioxol-5-yl)oxy]-N-(3-{2-[(6-methoxypyridin-3-yl)oxy]acetamidolbicyclo[1.1.1]pentan-1-yl)acetamide (Compound 271)

The title compound was prepared as described in Example 127, except substituting Example 166C for Example 28A and 6-methoxypyridin-3-ol for 6-chloro-5-methylpyridin-3-ol. ¹H NMR (400 MHz, DMSO-*d*₆) *δ* ppm 8.65 (d, *J* = 1.6 Hz, 2H), 7.81 (d, *J* = 3.0 Hz, 1H), 7.38 (dd, *J* = 8.9, 3.1 Hz, 1H), 7.29 (d, *J* = 8.9 Hz, 1H), 7.10 (d, *J* = 2.6 Hz, 1H), 6.78 6.69 (m, 2H), 4.40 (d, *J* = 3.2 Hz, 4H), 3.75 (s, 3H), 2.24 (s, 6H). MS (ESI+) *m*/*z* 478.1 (M+H)⁺.

### Example 173: N,N'-(2-oxobicyclo[2.2.2]octane-1,4-diyl)bis[2-(4-chloro-3-fluorophenoxy)acetamide] (Compound 272)

### Example 173A: ethyl 1,4-dioxaspiro[4.5]decane-8-carboxylate

A mixture of ethyl 4-oxocyclohexanecarboxylate (11.70 mL, 73.4 mmol), ethane-1,2-diol (12.29 mL, 220 mmol), and *p*-toluenesulfonic acid monohydrate (1.397 g, 7.34 mmol) in toluene (200) was stirred at 120 °C with Dean-Stark trap apparatus for 180 minutes. The reaction mixture was neutralized with *N*-ethyl-*N*-isopropylpropan-2-amine and concentrated. The residue was purified on silica gel (0-30% ethyl acetate in heptane) to give 12.77 g of the title compound as a clear oil. ¹H NMR (400 MHz, DMSO-*d*₆) *δ* ppm 4.01 (q, *J* = 7.1 Hz, 2H), 3.81 (s, 4H), 2.32 (tt, *J* = 10.4, 3.8 Hz, 1H), 1.83 1.71 (m, 2H), 1.66 1.57 (m, 1H), 1.62 1.38 (m, 5H), 1.13 (t, *J* = 7.1 Hz, 3H).

### Example 173B: ethyl 8-acetyl-1,4-dioxaspiro[4.5]decane-8-carboxylate

To a solution of diisopropylamine (5.19 mL, 36.4 mmol) in 25 mL of tetrahydrofuran at 0 °C was added n-butyllithium (14.56 mL, 2.5 M in hexane) slowly below 5 °C. After stirring for 30 minutes, the solution was cooled to -78 °C under nitrogen, a solution of ethyl 1,4-dioxaspiro[4.5]decane-8-carboxylate (6.0 g, 28.0 mmol) in tetrahydrofuran (3 mL) was added slowly, and the mixture was stirred for 30 minutes at the same temperature. Then acetyl chloride (2.59 mL, 36.4 mmol) was added slowly to maintain the temperature below -60 °C, and the mixture was stirred at -70 °C for 2 hours. The reaction was quenched with saturated aqueous NH₄Cl solution, and the aqueous mixture was extracted with ethyl acetate. The organic layer was washed with brine, dried over magnesium sulfate and filtered. The filtrate was concentrated, and the residue was purified on silica gel (0-70% ethyl acetate in heptane) to give 6.78 g of the title compound. ¹H NMR (500 MHz, DMSO-*d*₆) *δ* ppm 4.19 4.11 (m, 2H), 3.85 (s, 4H), 2.13 (s, 3H), 2.10 2.01 (m, 2H), 1.90 (ddd, *J* = 13.9, 9.6, 4.6 Hz, 2H), 1.54 (th, *J* = 13.6, 4.7 Hz, 4H), 1.18 (dd, *J* = 7.6, 6.5 Hz, 3H).

### Example 173C: ethyl 1-acetyl-4-oxocyclohexanecarboxylate

A mixture of Example 173B (6.5 g, 25.4 mmol) and HCl (21.13 mL, 127 mmol) in acetone (60 mL) was stirred at ambient temperature overnight. The mixture was concentrated, and the residue was taken up in dichloromethane. The organic layer was washed with brine, dried over magnesium sulfate and filtered. The filtrate was concentrated to give 5.46 g of the title compound that was used without further purification. ¹H NMR (400 MHz, DMSO-*d*₆) *δ* ppm 4.16 (q, *J* = 7.1 Hz, 2H), 2.17 (s, 3H), 2.35 2.07 (m, 8H), 1.17 (t, *J* = 7.1 Hz, 3H).

### Example 173D: ethyl 4-(benzylamino)-2-oxobicyclo[2.2.2]octane-1-carboxylate

A mixture of ethyl 1-acetyl-4-oxocyclohexanecarboxylate (Example 173C, 9.7 g, 45.7 mmol), benzylamine (14.98 mL, 137 mmol), and p-toluenesulfonic acid monohydrate (0.087 g, 0.457 mmol) in toluene (100 mL) was stirred at 130 °C in a Dean-Stark trap apparatus overnight. The mixture was concentrated, and the residue was stirred with a mixture of 50 mL of ethyl acetate and 100 mL of 3 N aqueous HCl for 30 minutes. The precipitate was collected by filtration, washed with a mixture of ethyl acetate/heptane, and air-dried to give 11.3 g of the title compound as a hydrochloride salt. The filtrate was neutralized with 6 N aqueous NaOH and extracted with ethyl acetate (100 mL x 2). The organic layer was washed with brine, dried over magnesium sulfate and filtered. The filtrate was concentrated, and the residue was purified on silica gel (0-70% ethyl acetate in heptane) to give another 0.77 g of the title compound as yellow solid. ¹H NMR (400 MHz, DMSO-*d*₆) *δ* ppm 9.73 (t, *J* = 6.2 Hz, 2H), 7.87 7.12 (m, 5H), 4.09 (m, 4H), 2.88 (s, 2H), 2.08 (dt, *J* = 20.7, 13.4 Hz, 6H), 1.16 (t, *J* = 7.1 Hz, 3H). MS (ESI+) *m*/*z* 302.1 (M+H)⁺.

### Example 173E: ethyl 4-amino-2-oxobicyclo[2.2.2]octane-1-carboxylate hydrochloride

To a mixture of ethyl 4-(benzylamino)-2-oxobicyclo[2.2.2]octane-1-carboxylate hydrochloride (Example 173D, 11.2 g, 33.2 mmol) in tetrahydrofuran (110 mL) in a 50 mL pressure bottle was added 20% Pd(OH)₂/C, wet (2.2 g, 1.598 mmol), and the reaction mixture was shaken at 50 °C under 50 psi of hydrogen for 22 hours. The reaction mixture was cooled to ambient temperature and filtered washing the solids with 1000 mL of methanol. The filtrate was concentrated to give 7.9 g of the title compound as a light yellow solid. ¹H NMR (400 MHz, DMSO-*d*₆) *δ* ppm 8.46 (s, 3H), 4.07 (q, *J* = 7.1 Hz, 2H), 2.62 (s, 2H), 2.17 2.05 (m, 2H), 2.04 1.78 (m, 6H), 1.14 (t, *J* = 7.1 Hz, 3H).

### Example 173F: ethyl 4-(2-(4-chloro-3-fluorophenoxy)acetamido)-2-oxobicyclo[2.2.2]octane-1-carboxylate

To a suspension of Example 173E (7.8 g, 31.5 mmol), *N*-ethyl-*N*-isopropylpropan-2-amine (22.00 mL, 126 mmol) and 2-(4-chloro-3-fluorophenoxy)acetic acid (7.41 g, 36.2 mmol) in N,N-dimethylformamide (200 mL), 2-(3H-[1,2,3]triazolo[4,5-b]pyridin-3-yl)-1,1,3,3-tetramethylisouronium hexafluorophosphate(V) (14.97 g, 39.4 mmol) was added, and the resulting brown solution was stirred at ambient temperature for 16 hours. Water was added, and the mixture was stirred for 15 minutes. The precipitate was collected by filtration, washed with water, and air-dried to give 12.1 g of the title compound as an off-white solid. ¹H NMR (400 MHz, DMSO-*d*₆) *δ* ppm 7.87 (s, 1H), 7.45 (t, *J* = 8.9 Hz, 1H), 7.00 (dd, *J* = 11.4, 2.9 Hz, 1H), 6.79 (ddd, *J* = 8.9, 2.9, 1.2 Hz, 1H), 4.45 (s, 2H), 4.06 (q, *J* = 7.1 Hz, 2H), 2.73 (s, 2H), 2.07 (m, 1H), 2.01 - 1.84 (m, 6H), 1.14 (t, *J* = 7.1 Hz, 3H). MS (ESI+) *m*/*z* 398.0 (M+H)⁺.

### Example 173G: 4-(2-(4-chloro-3-fluorophenoxy)acetamido)-2-oxobicyclo[2.2.2]octane-1-carboxylic acid

A suspension of Example 173F (11.37 g, 28.6 mmol) and sodium hydroxide (7.15 mL, 57.2 mmol) (8 M solution) in methanol (100 mL) was stirred at ambient temperature for 16 hours. The reaction mixture was concentrated, and the residue was acidified with 1 N aqueous HCl. The precipitate was collected by filtration and dried in vacuum oven to give 9.9 g of the title compound as a white solid. ¹H NMR (400 MHz, DMSO-*d*₆) *δ* ppm 12.49 (s, 1H), 7.86 (s, 1H), 7.45 (t, *J* = 8.9 Hz, 1H), 7.00 (dd, *J* = 11.4, 2.9 Hz, 1H), 6.83 - 6.74 (m, 1H), 4.45 (s, 2H), 2.71 (s, 2H), 2.01 - 1.81 (m, 7H). MS (ESI-) *m*/*z* 368.1 (M-H)⁻.

### Example 173H: tert-butyl (4-(2-(4-chloro-3-fluorophenoxy)acetamido)-2-oxobicyclo[2.2.2]octan-1-yl)carbamate

A mixture of Example 173G (0.33 g, 0.892 mmol) diphenylphosphoryl azide (0.193 mL, 0.892 mmol) and triethylamine (0.124 mL, 0.892 mmol) in toluene (3 mL) was heated at 110 °C for about 45 minutes. To the resulting yellow solution, *tert*-butanol (0.427 mL, 4.46 mmol) was added, and the reaction mixture was heated at about 110 °C for about 16 hours. The mixture was concentrated, and the residue was partitioned between saturated NaHCO₃ and ethyl acetate. The organic layer was washed with brine, dried over magnesium sulfate and filtered. The filtrate was concentrated, and the residue was purified on silica gel (10-100% ethyl acetate in heptane) to give 106 mg of the title compound as a white solid.

### Example 173I: N,N'-(2-oxobicyclo[2.2.2]octane-1,4-diyl)bis[2-(4-chloro-3-fluorophenoxy)acetamide]

A mixture of Example 173H (0.1 g, 0.227 mmol) and 4 N hydrogen chloride in dioxane (4.0 mL, 16.00 mmol) was stirred ambient temperature for about 45 minutes. The mixture was then concentrated, and the residue was used without further purification. A mixture of this residue, 2-(4-chloro-3-fluorophenoxy)acetic acid (0.058 g, 0.284 mmol) and N-ethyl-N-isopropylpropan-2-amine (0.198 mL, 1.134 mmol) in *N,N*-dimethylformamide (2.000 mL) was treated with 2-(3*H*-[1,2,3]triazolo[4,5-*b*]pyridin-3-yl)-1,1,3,3-tetramethylisouronium hexafluorophosphate(V) (0.129 g, 0.340 mmol), and the reaction mixture was stirred at ambient temperature overnight. The mixture was concentrated under high vacuum, and the residue was purified by HPLC (20-95% acetonitrile in 0.1% trifluoroacetic acid/water at 25 mL/minute on a Phenomenex^{®} Luna^{®} C18 5 µm 100 Å AXIA^{™} column (250 mm × 21.2 mm)) to give 96 mg of the title compound as a white solid. ¹H NMR (400 MHz, DMSO-*d*₆) *δ* ppm 7.88 (s, 1H), 7.67 (s, 1H), 7.45 (td, *J* = 8.9, 2.4 Hz, 2H), 7.03 (ddd, *J* = 15.1, 11.4, 2.8 Hz, 2H), 6.80 (dddd, *J =* 10.3, 9.0, 2.9, 1.2 Hz, 2H), 4.53 (s, 2H), 4.45 (s, 2H), 2.83 (s, 2H), 2.45 2.33 (m, 2H), 2.08 1.91 (m, 2H), 2.01 (s, 2H), 1.80 (td, *J* = 11.8, 4.5 Hz, 2H). MS (ESI+) *m*/*z* 527.0 (M+H)⁺.

### Example 174: N,N'-(2-hydroxybicyclo[2.2.2]octane-1,4-diyl)bis[2-(4-chloro-3-fluorophenoxy)acetamide] (Compound 273)

A mixture of Example 173 (65 mg, 0.123 mmol) and sodium tetrahydroborate (23.32 mg, 0.616 mmol) in a mixture of methanol/dichloromethane (1.0 mL, 1:1) was stirred at ambient temperature for 1 hour. The mixture was concentrated, and the residue was purified by HPLC (20~95% acetonitrile in 0.1% trifluoroacetic acid/water at 25 mL/minute on a Phenomenex^{®} Luna^{®} C18 5 µm 100 Å AXIA^{™} column (250 mm × 21.2 mm)) to give 54 mg of the title compound as a light brown solid. ¹H NMR (400 MHz, DMSO-*d*₆) *δ* ppm 7.49 7.39 (m, 3H), 7.22 (s, 1H), 7.00 (ddd, *J* = 12.5, 11.4, 2.9 Hz, 2H), 6.78 (dddd, *J* = 9.0, 7.9, 2.9, 1.2 Hz, 2H), 5.04 (d, *J* = 4.4 Hz, 1H), 4.43 (s, 2H), 4.40 (s, 2H), 4.00 (dt, *J* = 8.7, 3.7 Hz, 1H), 2.23 (ddd, *J* = 12.2, 9.5, 2.3 Hz, 1H), 2.03 (ddd, *J* = 12.3, 10.5, 4.7 Hz, 1H), 1.88 (t, *J* = 12.9 Hz, 2H), 1.86 1.78 (m, 1H), 1.75 (ddd, *J* = 12.9, 7.8, 2.4 Hz, 4H). MS (ESI+) *m*/*z* 528.9 (M+H)⁺.

### Example 175: N,N'-(bicyclo[1.1.1]pentane-1,3-diyl)bis{2-[(2,2-difluoro-2H-1,3-benzodioxol-5-yl)oxy]acetamide} (Compound 274)

The title compound was prepared as described in Example 124B, except substituting Example 166B for Example 9B and Example 166A for 2-(4-chloro-3-(methoxycarbonyl)phenoxy)acetic acid. ¹H NMR (400 MHz, DMSO-*d*₆) *δ* ppm 8.65 (s, 2H), 7.28 (d, *J* = 8.8 Hz, 2H), 7.10 (d, *J* = 2.6 Hz, 2H), 6.73 (dd, *J* = 8.9, 2.6 Hz, 2H), 4.41 (s, 4H), 2.24 (s, 6H). MS (ESI+) *m*/*z* 526.8 (M+H)⁺.

### Example 176: methyl 2-chloro-5-{2-[(3-{2-[(2,2-difluoro-2H-1,3-benzodioxol-5-yl)oxy]acetamido}bicyclo[1.1.1]pentan-1-yl)amino]-2-oxoethoxy}benzoate (Compound 275)

The title compound was prepared as described in Example 124B, except substituting Example 166B for Example 9B. ¹H NMR (400 MHz, DMSO-*d*₆) *δ* ppm 8.73 (s, 1H), 8.67 (s, 1H), 7.47 (d, *J* = 8.8 Hz, 1H), 7.37 (d, *J* = 3.0 Hz, 1H), 7.30 (d, *J* = 8.8 Hz, 1H), 7.19 7.09 (m, 2H), 6.75 (dd, *J* = 8.9, 2.5 Hz, 1H), 4.48 (s, 2H), 4.42 (s, 2H), 3.84 (s, 3H), 2.26 (s, 6H). MS (ESI-) *m*/*z* 536.8 (M-H)⁻.

### Example 177: 2-[4-chloro-3-(hydroxymethyl)phenoxy]-N-(3-{2-[(2,2-difluoro-2H-1,3-benzodioxol-5-yl)oxy]acetamido}bicyclo[1.1.1]pentan-1-yl)acetamide (Compound 276)

A mixture of Example 176 (0.07, 0.130 mmol) and lithium tetrahydroborate (0.028 g, 1.299 mmol) in tetrahydrofuran (2.0 mL) was stirred at 40 °C for 24 hours. The reaction mixture was concentrated under high vacuum, and the residue was purified by HPLC (10-95% acetonitrile in 0.1% trifluoroacetic acid/water at 25 mL/minute on a Phenomenex^{®} Luna^{®} C18 5 µm 100 Å AXIA^{™} column (250 mm × 21.2 mm)) to give 23 mg of the title compound as a light yellow solid. ¹H NMR (500 MHz, DMSO-*d*₆) *δ* ppm 8.70 (d, *J* = 16.9 Hz, 2H), 7.31 (dd, *J* = 10.7, 8.8 Hz, 2H), 7.15 (dd, *J* = 14.7, 2.9 Hz, 2H), 6.84 (dd, *J* = 8.7, 3.2 Hz, 1H), 6.77 (dd, *J* = 8.8, 2.6 Hz, 1H), 5.41 (s, 1H), 4.51 (s, 2H), 4.43 (d, *J* = 8.4 Hz, 4H), 2.27 (s, 6H). MS (ESI-) *m*/*z* 509.0 (M-H)⁻.

### Example 178: 2-(4-chloro-3-fluorophenoxy)-N-(3-{2-[(2-cyanopyridin-4-yl)oxy]acetamidolbicyclo[1.1.1]pentan-1-yl)acetamide (Compound 277)

The title compound was prepared as described in Example 127, except substituting 4-hydroxypicolinonitrile for 6-chloro-5-methylpyridin-3-ol. ¹H NMR (400 MHz, DMSO-*d*₆) *δ* ppm 8.76 (s, 1H), 8.68 (s, 1H), 8.56 8.49 (m, 1H), 7.68 7.63 (m, 1H), 7.46 (t, *J =* 8.9 Hz, 1H), 7.25 (dd, *J* = 5.8, 2.6 Hz, 1H), 7.04 (dd, *J* = 11.4, 2.8 Hz, 1H), 6.82 (ddd, *J* = 9.0, 2.9, 1.2 Hz, 1H), 4.64 (s, 2H), 4.44 (s, 2H), 2.23 (s, 6H). MS (ESI+) *m*/*z* 445.0 (M+H)⁺.

### Example 179: 2-[(2,2-difluoro-2H-1,3-benzodioxol-5-yl)oxy]-N-(3-{2-[4-(pentafluoro-λ⁶-sulfanyl)phenoxy]acetamido}bicyclo[1.1.1]pentan-1-yl)acetamide (Compound 278)

The title compound was prepared as described in Example 127, except substituting Example 166C for Example 28A and 4-(pentafluorothiol)phenol for 6-chloro-5-methylpyridin-3-ol. ¹H NMR (400 MHz, DMSO-*d*₆) *δ* ppm 8.73 (s, 1H), 8.65 (s, 1H), 7.85 - 7.76 (m, 2H), 7.29 (d, *J* = 8.9 Hz, 1H), 7.13 - 7.04 (m, 3H), 6.73 (dd, *J* = 8.9, 2.6 Hz, 1H), 4.53 (s, 2H), 4.40 (s, 2H), 2.24 (s, 6H). MS (ESI+) *m*/*z* 572.9 (M+H)⁺.

### Example 180: 2-(4-chloro-3-fluorophenoxy)-N-[3-(2-{[6-(difluoromethoxy)pyridin-3-yl]methoxy}acetamido)bicyclo[1.1.1]pentan-1-yl]acetamide (Compound 279)

A 2.5 mL microwave vial was charged with Example 112B (35 mg, 1 equivalent, 0.102 mmol), Cs₂CO₃ (66 mg, 0.2 mmol), 5-(chloromethyl)-2-(difluoromethoxy)pyridine (39 mg, 0.2 mmol) and potassium iodide (1.2 mg, 0.07 equivalent, 0.07 mmol). Acetone (1.5 mL) was added. The resulting mixture was heated in a Biotage^{®} Initiator microwave reactor for 45 minutes at 140 °C (0-450 W). Upon completion, the mixture was then filtered and concentrated to dryness. The residue was dissolved in 1:1 dimethyl sulfoxide/methanol and purified by preparative reverse phase HPLC on a Phenomenex^{®} Luna^{®} C8(2) 5 µm 100 Å AXIA^{™} column (30 mm × 150mm). A gradient of acetonitrile (A) and 0.1% trifluoroacetic acid in water (B) was used, at a flow rate of 50 mL/minute (0-0.5 minutes 5% A, 0.5-8.5 minutes linear gradient 5-100% A, 8.7-10.7 minutes 100% A, 10.7 -11.0 minutes linear gradient 100-5% A) to afford the title compound. ¹H NMR (400 MHz, DMSO-*d*₆) *δ* ppm 8.32 - 8.21 (m, 1H), 7.93 (dd, *J =* 8.5, 2.4 Hz, 1H), 7.66 (s, 1H), 7.54 - 7.43 (m, 1H), 7.11 - 6.99 (m, 2H), 6.84 (ddd, *J* = 9.0, 2.9, 1.2 Hz, 1H), 4.53 (s, 2H), 4.45 (s, 2H), 3.86 (s, 2H), 2.24 (s, 6H). MS (APCI) *m*/*z* 500.1 (M+H)⁺.

### Example 181: 2-(4-chloro-3-fluorophenoxy)-N-[3-(2-{[6-(1H-pyrazol-1-yl)pyridin-3-yl]methoxy}acetamido)bicyclo[1.1.1]pentan-1-yl]acetamide (Compound 280)

The title compound was prepared as described in Example 180, except substituting 5-(chloromethyl)-2-(1H-pyrazol-1-yl)pyridine for 5-(chloromethyl)-2-(difluoromethoxy)pyridine. ¹H NMR (400 MHz, DMSO-*d*₆) *δ* ppm 8.60 (dd, *J =* 2.6, 0.7 Hz, 1H), 8.51 - 8.45 (m, 1H), 8.00 (dd, *J* = 8.4, 2.3 Hz, 1H), 7.91 (dd, *J* = 8.4, 0.8 Hz, 1H), 7.82 (dd, *J* = 1.7, 0.7 Hz, 1H), 7.47 (t, *J* = 8.9 Hz, 1H), 7.04 (dd, *J* = 11.3, 2.9 Hz, 1H), 6.89 - 6.83 (m, 1H), 6.58 (dd, *J* = 2.6, 1.7 Hz, 1H), 4.59 (s, 2H), 4.45 (s, 1H), 3.90 (s, 2H), 2.25 (s, 6H). MS (APCI) *m*/*z* 500.2 (M+H)⁺.

### Example 182: 2-(4-chloro-3-fluorophenoxy)-N-(3-{2-[(6-methoxypyridin-3-yl)methoxy]acetamido}bicyclo[1.1.1]pentan-1-yl)acetamide (Compound 281)

The title compound was prepared as described in Example 180, except substituting 5-(chloromethyl)-2-methoxypyridine for 5-(chloromethyl)-2-(difluoromethoxy)pyridine. ¹H NMR (400 MHz, DMSO-*d*₆) *δ* ppm 8.13 (dd, *J* = 2.4, 0.7 Hz, 1H), 7.73 (dd, *J* = 8.5, 2.4 Hz, 1H), 7.47 (t, *J =* 8.9 Hz, 1H), 7.04 (dd, *J* = 11.3, 2.9 Hz, 1H), 6.87 - 6.79 (m, 2H), 4.46 (d, 4H), 3.84 (s, 3H), 3.82 (s, 2H), 2.23 (s, 6H). MS (APCI) *m*/*z* 464.2 (M+H)⁺.

### Example 183: 2-(4-chloro-3-fluorophenoxy)-N-(3-{2-[(2,6-dimethylpyridin-4-yl)methoxy]acetamido}bicyclo[1.1.1]pentan-1-yl)acetamide (Compound 282)

The title compound was prepared as described in Example 180, except substituting 4-(chloromethyl)-2,6-dimethylpyridine for 5-(chloromethyl)-2-(difluoromethoxy)pyridine. ¹H NMR (400 MHz, DMSO-*d*₆) *δ* ppm 7.66 (s, 2H), 7.48 (t, *J =* 8.9 Hz, 1H), 7.04 (dd, *J =* 11.4, 2.9 Hz, 1H), 6.85 (ddd, *J* = 9.0, 2.9, 1.2 Hz, 1H), 4.76 (s, 2H), 4.46 (s, 2H), 4.00 (s, 2H), 2.66 (s, 6H), 2.27 (s, 6H). MS (APCI) *m*/*z* 462.2 (M+H)⁺.

### Example 184: 2-(4-chloro-3-fluorophenoxy)-N-(3-{2-[(6-cyanopyridin-3-yl)methoxy]acetamido}bicyclo[1.1.1]pentan-1-yl)acetamide (Compound 283)

The title compound was prepared as described in Example 180, except substituting 5-(chloromethyl)picolinonitrile hydrochloride for 5-(chloromethyl)-2-(difluoromethoxy)pyridine. ¹H NMR (400 MHz, DMSO-*d*₆) *δ* ppm 8.82 - 8.74 (m, 1H), 8.02 (qd, *J =* 8.0, 1.5 Hz, 1H), 7.47 (t, *J =* 8.9 Hz, 1H), 7.04 (dd, *J =* 11.4, 2.8 Hz, 1H), 6.89 - 6.81 (m, 1H), 4.67 (s, 2H), 4.45 (s, 2H), 3.93 (s, 2H), 2.25 (s, 6H). MS (APCI) *m*/*z* 459.1 (M+H)⁺.

### Example 185: 2-(4-chloro-3-fluorophenoxy)-N-[3-(2-{[6-(trifluoromethyl)pyridin-3-yl]methoxy}acetamido)bicyclo[1.1.1]pentan-1-yl]acetamide (Compound 284)

The title compound was prepared as described in Example 180, except substituting 5-(chloromethyl)-2-(trifluoromethyl)pyridine for 5-(chloromethyl)-2-(difluoromethoxy)pyridine. ¹H NMR (400 MHz, DMSO-*d*₆) *δ* ppm 8.76 (d, *J =* 1.9 Hz, 1H), 8.08 (ddd, *J =* 8.1, 2.0, 0.9 Hz, 1H), 7.90 (dd, *J =* 8.1, 0.9 Hz, 1H), 7.47 (t, *J =* 8.9 Hz, 1H), 7.04 (dd, *J =* 11.3, 2.9 Hz, 1H), 6.85 (ddd, *J =* 8.9, 2.8, 1.2 Hz, 1H), 4.68 (s, 2H), 4.46 (s, 2H), 3.94 (s, 2H), 2.25 (s, 6H). MS (APCI) *m*/*z* 502.1(M+H)⁺.

### Example 186: 2-[(5-cyclopropylpyrazin-2-yl)oxy]-N-{3-[2-(3,4-dichlorophenoxy)acetamido]-bicyclo[1.1.1]pentan-1-yl}acetamide (Compound 285)

### Example 186A: ethyl 2-((5-cyclopropylpyrazin-2-yl)oxy)acetate

To ethyl 2-hydroxyacetate (1.475 g, 14 mmol) in tetrahydrofuran (40 mL) at room temperature was added potassium tert-butoxide (20 mL, 1 M in tetrahydrofuran, 20 mmol). After 5 minutes, 2-bromo-5-cyclopropylpyrazine (1.752 g, 8.8 mmol) in tetrahydrofuran (5 mL) was added. The mixture was stirred at room temperature for 2 days. The reaction was quenched by addition of water (20 mL), and then extracted with ethyl acetate (100 mL). The organic phase was concentrated to give 1.97 g of ethyl 2-((5-cyclopropylpyrazin-2-yl)oxy)acetate as a solid. LC/MS (ESI+) *m*/*z* 223 (M+H)⁺.

### Example 186B: 2-((5-cyclopropylpyrazin-2-yl)oxy)acetic acid

To a solution of ethyl 2-((5-cyclopropylpyrazin-2-yl)oxy)acetate (1.96 g, 8.8 mmol) in methanol (8 mL) was added 2 M aqueous potassium hydroxide solution (11 mL). The mixture was stirred at room temperature for 2 hours and was concentrated. The aqueous mixture was then extracted with ethyl acetate (80 mL). Then aqueous phase was acidified with 2 N aqueous HCl solution to pH ~ 3, and then extracted with ethyl acetate (100 mL x 2). The combined organic phase was dried over Na₂SO₄. The organic phase was filtered and concentrated to give 0.6 g of the title compound as a solid. ¹H NMR (400 MHz, DMSO-*d*₆) *δ* ppm 8.22 (s, 1H), 8.12 (s, 1H), 4.83 (s, 2H), 2.13 (m, 1H), 0.96 (m, 2H), 0.80 (m, 2H). MS (ESI+) *m*/*z* 195 (M+H)⁺.

### Example 186C: 2-[(5-cyclopropylpyrazin-2-yl)oxy]-N-{3-[2-(3,4-dichlorophenoxy)acetamido]-bicyclo[1.1.1]pentan-1-yl}acetamide

To a mixture of N-(3-aminobicyclo[1.1.1]pentan-1-yl)-2-(3,4-dichlorophenoxy)acetamide hydrochloride (0.169 g, 0.5 mmol, Example 6C) and 2-((5-cyclopropylpyrazin-2-yl)oxy)acetic acid (Example 186B, 0.097 g, 0.5 mmol) in *N,N-*dimethylformamide (2 mL) was added N,N-diisopropylethylamine (0.194 g, 1.5 mmol), followed by 1-[bis(dimethylamino)methylene]-1H-1,2,3-triazolo[4,5-b]pyridinium 3-oxid hexafluorophosphate (0.209 g, 0.55 mmol, HATU). The mixture was stirred at room temperature for 3 hours, and then diluted with dichloromethane (60 mL). The mixture was washed with water (50 mL x 2) and 0.02 M aqueous Na₂CO₃ solution (50 mL x 2), dried over Na₂SO₄, filtered and concentrated to give 0.24 g of a solid residue. The solid was dissolved in ethyl acetate/methanol (1:1, 3 mL), and purified by flash column chromatography on silica gel (80 g) eluted with heptane and ethyl acetate (10 to 60%) to give 81 mg of the title compound (34% yield) as a white solid. ¹H NMR (400 MHz, DMSO-*d*₆) *δ* ppm 8.72 (s, 1H), 8.63 (s, 1H), 8.20 (s, 1H), 8.16 (s, 1H), 7.55 (d, J = 8, 1H), 7.24 (d, J = 2, 1H), 6.98 (dd, J = 8, 2, 1H), 4.68 (s, 2H), 4.48 (s, 2H), 2.23 (s, 6H), 2.13 (m, 1H), 0.96 (m, 2H), 0.82 (m, 2H). MS (ESI+) *m*/*z* 477 (M+H)⁺.

### Example 187: 2-(4-chloro-3-fluorophenoxy)-N-(3-{[2-(3,4-dichlorophenyl)-2-oxoethyl]amino}bicyclo[1.1.1]pentan-1-yl)acetamide (Compound 286)

A mixture of *N*-(3-aminobicyclo[1.1.1]pentan-1-yl)-2-(4-chloro-3-fluorophenoxy)acetamide hydrochloride (Example 112A, 0.096 g, 0.3 mmol), 2-bromo-1-(3,4-dichlorophenyl)ethanone (0.096 g, 0.36 mmol) and N,N-diisopropylethylamine (0.116 g, 0.9 mmol) in N,N-dimethylformamide (0.6 mL) was stirred at room temperature for 1 hour. The reaction mixture was diluted with ethyl acetate (40 mL) and washed with 0.1 M aqueous Na₂HPO₃ solution (60 mL x3). The organic phase was dried over Na₂SO₄, filtered and concentrated to give 0.16 g of solid residue. The solid was dissolved in ethyl acetate (1.5 mL) and was purified by flash column chromatography on silica gel (80 g) eluting with heptane and ethyl acetate (70 to 100%) to give 40 mg of the title compound (28% yield) as a white solid. ¹H NMR (400 MHz, DMSO-*d*₆) *δ* ppm 8.60 (s, 1H), 8.18 (d, J = 2, 1H), 7.94 (dd, J = 8, 2, 1H), 7.82 (d, J = 8, 1H), 7.48 (t, J = 8, 1H), 7.07 (dd, J =8, 2, 1H), 6.85 (dd, J = 8, 2, 1H), 4.47 (s, 2H), 4.07 (s, 2H), 1.97 (s, 6H). MS (ESI+) *m*/*z* 471 (M+H)⁺.

### Example 188: N-(3-{[2-(4-chlorophenyl)-2-oxoethyl]amino}bicyclo[1.1.1]pentan-1-yl)-2-(3,4-dichlorophenoxy)acetamide (Compound 287)

To N-(3-aminobicyclo[1.1.1]pentan-1-yl)-2-(3,4-dichlorophenoxy)acetamide hydrochloride (0.338 g, 1 mmol, Example 6C), and *N,N*-diisopropylethylamine (0.45 g, 3.5 mmol) in *N,N*-dimethylformamide (1 mL) was added 2-chloro-1-(4-chlorophenyl)ethanone (0.208 g, 1.1 mmol) in N,N-dimethylformamide (0.5 mL). The mixture was stirred at 45 °C for 2 hours. The reaction mixture was diluted with ethyl acetate (100 mL) and washed with 0.1 M aqueous Na₂HPO₃ solution (80 mL x 3). The organic phase was dried over Na₂SO₄, filtered and concentrated to give 0.46 g of a residue. The residue was dissolved in ethyl acetate (2 mL) and purified by flash column chromatography on silica gel (80 g) eluted with heptane and ethyl acetate (70 to 100%) to give 286 mg the title compound (63% yield) as a white solid. ¹H NMR (400 MHz, DMSO-*d*₆) *δ* ppm 8.60 (s, 1H), 8.00 (d, J = 8, 2H), 7.60 (d, J = 8, 2H), 7.54 (d, J = 8, 1H), 7.25 (d, J = 2, 1H), 6.98 (dd, J = 8, 2, 1H), 4.47 (s, 2H), 4.04 (s, 2H), 2.84 (m, 1H), 1.96 (s, 6H). MS (ESI+) *m*/*z* 453 (M+H)⁺.

### Example 189: N-(3-{[2-(4-chlorophenyl)-2-hydroxyethyl]amino}bicyclo[1.1.1]pentan-1-yl)-2-(3,4-dichlorophenoxy)acetamide (Compound 288)

A mixture of *N*-(3-{[2-(4-chlorophenyl)-2-oxoethyl]amino}bicyclo[1.1.1]pentan-1-yl)-2-(3,4-dichlorophenoxy)acetamide (Example 188, 0.254 g, 0.56 mmol) and sodium borohydride (0.085 g, 2.24 mmol) in methanol (2 mL) was stirred at room temperature overnight. 1 N aqueous HCl (1.5 mL) was added. The mixture was stirred at room temperature for 1 hour and was basified with 2 M aqueous sodium carbonate solution to pH ~ 10. The mixture was extracted with ethyl acetate (25 mL x 3). The organic phase was dried over Na₂SO₄, filtered and concentrated to give 0.26 g of a residue. The residue was dissolved in ethyl acetate (2 mL) and purified by flash column chromatography on silica gel (40 g) eluted with ethyl acetate and methanol (0 to 8%) to give 182 mg of the title compound as a white solid. ¹H NMR (400 MHz, DMSO-*d*₆) *δ* ppm 8.60 (s, 1H), 7.54 (d, J = 8, 1H), 7.36 (m, 4H), 7.25 (t, J = 2, 1H), 6.98 (dd, J =8, 2, 1H), 5.35 (d, J = 5, 1H), 4.57 (m, 1H), 4.46 (s, 2H), 2.58 (d, J = 8, 2H), 2.36 (m, 1H), 1.95 (m, 6H). MS (ESI+) *m*/*z* 455 (M+H)⁺.

### Example 190: 2-(4-chloro-3-fluorophenoxy)-N-(3-{[2-(4-chlorophenyl)-2-oxoethyl]amino}bicyclo[1.1.1]pentan-1-yl)acetamide (Compound 289)

To N-(3-aminobicyclo[1.1.1]pentan-1-yl)-2-(4-chloro-3-fluorophenoxy)acetamide hydrochloride (Example 112A, 0.642 g, 2 mmol), and *N,N*-diisopropylethylamine (0.9 g, 7 mmol) in *N,N*-dimethylformamide (2 mL) was added 2-chloro-1-(4-chlorophenyl)ethanone (0.416 g, 2.2 mmol) in N,N-dimethylformamide (1 mL). The mixture was stirred at 45 °C for 1.5 hours. The reaction mixture was diluted with ethyl acetate (100 mL) and washed with 0.1 M aqueous Na₂HPO₃ solution (80 mL x 3). The organic phase was dried over Na₂SO₄, filtered and concentrated to give 0.88 g of a residue. The residue was dissolved in ethyl acetate (2 mL) and purified by flash column chromatography on silica gel (80 g) eluted with heptane and ethyl acetate (70 to 100%) to give 359 mg of the title compound (41% yield) as a white solid. ¹H NMR (400 MHz, DMSO-*d*₆) *δ* ppm 8.60 (s, 1H), 8.00 (d, J = 8, 2H), 7.60 (d, J = 8, 2H), 7.49 (d, t = 8, 1H), 7.06 (dd, J = 8, 2, 1H), 6.84 (m, 1H), 4.46 (s, 2H), 4.05 (s, 2H), 2.84 (m, 1H), 1.96 (s, 6H). MS (ESI+) *m*/*z* 437 (M+H)⁺.

### Example 191: 2-(4-chloro-3-fluorophenoxy)-N-(3-{[2-(4-chlorophenyl)-2-hydroxyethyl]amino}bicyclo[1.1.1]pentan-1-yl)acetamide (Compound 290)

A mixture of 2-(4-chloro-3-fluorophenoxy)-*N*-(3-{[2-(4-chlorophenyl)-2-oxoethyl]amino}bicyclo[1.1.1]pentan-1-yl)acetamide (0.341 g, 0.78 mmol, Example 190) and sodium borohydride (0.118 g, 3.12 mmol) in methanol (5 mL) was stirred at room temperature overnight. 1 N aqueous HCl (1.5 mL) was added. The mixture was stirred at room temperature for 1 hour and was basified with 2 M aqueous sodium carbonate to pH ~ 10. The mixture was extracted with ethyl acetate (50 mL x 2). The organic phase was dried over Na₂SO₄, filtered and concentrated to give 0.36 g of a residue. The residue was dissolved in ethyl acetate (2 mL) and purified by flash column chromatography on silica gel (40 g) eluting with ethyl acetate and methanol (0 to 9%) to give 310 mg of the title compound (90% yield) as a white solid. ¹H NMR (400 MHz, DMSO-*d*₆) *δ* ppm 8.60 (s, 1H), 7.48 (t, J = 8, 1H), 7.36 (m, 4H), 7.06 (dd, J =8, 2, 1H), 6.84 (m, 1H), 5.36 (d, J = 5, 1H), 4.58 (m, 1H), 4.45 (s, 2H), 2.59 (d, J = 8, 2H), 2.35 (m, 1H), 1.95 (m, 6H). MS (ESI+) *m*/*z* 439 (M+H)⁺.

### Example 192: 2-(4-chloro-3-fluorophenoxy)-N-(3-{[2-(4-chloro-3-fluorophenyl)-2-oxoethyl]amino}bicyclo[1.1.1]pentan-1-yl)acetamide (Compound 291)

To N-(3-aminobicyclo[1.1.1]pentan-1-yl)-2-(4-chloro-3-fluorophenoxy)acetamide hydrochloride (Example 112A, 0.321 g, 1. mmol), and N,N-diisopropylethylamine (0.45 g, 3.5 mmol) in *N,N*-dimethylformamide (1 mL) was added 2-chloro-1-(4-chloro-3-fluorophenyl)ethanone (0.23 g, 1.1 mmol) in *N*,*N*-dimethylformamide (0.5 mL). The mixture was stirred at room temperature for 5 hours. The reaction mixture was diluted with ethyl acetate (80 mL) and then washed with 2 M aqueous sodium carbonate solution (50 mL x 3). The organic phase was dried over Na₂SO₄, filtered and concentrated to give 0.46 g of a residue. The residue was dissolved in ethyl acetate (2 mL) and purified by flash column chromatography on silica gel (80 g) eluting with heptane and ethyl acetate (70 to 100%) to give 164 mg of the title compound (36% yield) as a white solid. ¹H NMR (500 MHz, DMSO-*d*₆) *δ* ppm 8.60 (s, 1H), 7.97 (dd, J = 8, 2, 1H), 7.83 (dd, J = 8, 2, 1H), 7.76 (d, t = 8, 1H), 7.47 (d, t = 8, 1H), 7.04 (dd, J = 8, 2, 1H), 6.83 (m, 1H), 4.44 (s, 2H), 4.05 (s, 2H), 2.85 (m, 1H), 1.95 (s, 6H). MS (ESI+) *m*/*z* 455 (M+H)⁺.

### Example 193: 2-(4-chloro-3-fluorophenoxy)-N-(3-{[2-(4-chloro-3-fluorophenyl)-2-hydroxyethyl]amino}bicyclo[1.1.1]pentan-1-yl)acetamide (Compound 292)

A mixture of 2-(4-chloro-3-fluorophenoxy)-N-(3-{ [2-(4-chloro-3-fluorophenyl)-2-oxoethyl]amino}bicyclo[1.1.1]pentan-1-yl)acetamide (Example 192, 0.15 g, 0.33 mmol) and sodium borohydride (0.050 g, 1.32 mmol) in methanol (5 mL) was stirred at room temperature overnight. 1 N aqueous HCl (1 mL) was added. The mixture was stirred at room temperature for 1 hour and then was basified with 2 M aqueous sodium carbonate solution to pH ~ 10. The mixture was extracted with ethyl acetate (50 mL x 2). The organic phase was dried over Na₂SO₄, filtered and concentrated to give 0.36 g of a residue. The residue was dissolved in ethyl acetate (2 mL), and purified by flash column chromatography on silica gel (40 g) eluting with ethyl acetate and methanol (0 to 9%) to give 127 mg of the title compound (84% yield) as a white solid. ¹H NMR (400 MHz, DMSO-*d*₆) *δ* ppm 8.60 (s, 1H), 7.50 (m, 2H), 7.35 (dd, J =8, 1, 1H), 7.21 (dd, J =7, 1, 1H), 7.06 (dd, J = 8, 2, 1H), 6.85 (m, 1H), 5.48 (d, J = 5, 1H), 4.58 (m, 1H), 4.45 (s, 2H), 2.61 (d, J = 8, 2H), 2.43 (m, 1H), 1.95 (m, 6H). MS (ESI+) *m*/*z* 457 (M+H)⁺.

### Example 194: N-{3-[2-(4-chloro-3-fluorophenoxy)acetamido]bicyclo[1.1.1]pentan-1-yl}-3-(4-chlorophenyl)-3-oxopropanamide (Compound 293)

### Example 194A: 3-(4-chlorophenyl)-3-oxopropanoic acid

A 100 mL round bottom flask equipped with a magnetic stir bar was charged with KOH (1.35 g, 24.1 mmol). Water (28.2 mL) was added, and as the solution was stirred at ambient temperature, methyl-3-(4-chlorophenyl)-3-oxopropanoate (3 g, 14.11 mmol) was added. The reaction mixture was stirred at ambient temperature for 44 hours. The basic aqueous reaction mixture was washed twice with methyl *tert-butyl* ether (2 × 10 mL), then was chilled in an ice bath and treated slowly with 1 N aqueous HCl. The resulting white precipitate was collected by filtration and rinsed with water to give the title compound (209 mg, 1.05 mmol, 7.4% yield.). ¹H NMR (501 MHz, DMSO-*d*₆) *δ* ppm 12.72 (s, 1H), 7.96 (d, J = 8.7 Hz, 2H), 7.60 (d, J = 8.7 Hz, 2H), 4.04 (s, 2H).

### Example 194B: N-{3-[2-(4-chloro-3-fluorophenoxy)acetamido]bicyclo[1.1.1]pentan-1-yl}-3-(4-chlorophenyl)-3-oxopropanamide

A 4 mL vial, equipped with a magnetic stir bar, was charged with the product of Example 194A (87 mg, 0.44 mmol), the product of Example 112A (128 mg, 0.40 mmol), and (1-cyano-2-ethoxy-2-oxoethylidenaminooxy)dimethylamino-morpholino-carbenium hexafluorophosphate (COMU, 206 mg, 0.48 mmol). The vial was sealed with a septum screw cap and the contents were placed under a dry nitrogen atmosphere. N,N-Dimethylformamide (DMF) (2 mL) was introduced via syringe to give a solution that was stirred at ambient temperature as *N,N-*diisopropylethylamine (0.21 mL, 1.20 mmol) was added dropwise via syringe. When the addition was complete, the reaction mixture was stirred at ambient temperature for 20.5 hours. The reaction mixture was partitioned between dilute aqueous citric acid (5 mL) and ethyl acetate (5 mL). The organic layer was washed twice with brine (2 × 5 mL), then dried over anhydrous MgSO₄ and filtered. The filtrate was concentrated under reduced pressure to give a yellow oil which was stirred with hot water. The water was decanted away, and the residue was treated with methyl *tert-butyl* ether to give a pale yellow solid which was isolated by filtration and combined with additional material as described below. The filtrate was dried over anhydrous MgSO₄, filtered and concentrated. The residue was purified by preparative HPLC (Phenomenex^{®} Luna^{®} C8(2) 5 µm 100Å AXIA^{™} column (30 mm × 75 mm); a gradient of acetonitrile (A) and 0.1% trifluoroacetic acid in water (B) was used, at a flow rate of 50 mL/minute (0-1.0 minute 5% A, 1.0-8.5 minutes linear gradient 5-100% A, 8.5-11.5 minutes 100% A, 11.5-12.0 minutes linear gradient 95-5% A) to give additional solids. Solids were combined to give the title compound (136 mg, 0.29 mmol; 73% yield). ¹H NMR (400 MHz, CDCl₃) *δ* ppm 7.93 (d, J = 8.6 Hz, 2H), 7.48 (d, J = 8.6 Hz, 2H), 7.32 (t, J = 8.6 Hz, 1H), 6.83 (s, 1H), 6.76 (dd, J = 10.3, 3.0 Hz, 1H), 6.67 (ddd, J = 8.8, 2.9, 1.3 Hz, 1H), 4.39 (s, 2H), 3.89 (s, 2H), 2.48 (s, 6H); MS (ESI⁺) *m*/*z* 465 (M+H)⁺.

### Example 195: (2E)-N-{3-[2-(4-chloro-3-fluorophenoxy)acetamido]bicyclo[1.1.1]pentan-1-yl}-3-(4-chlorophenyl)prop-2-enamide (Compound 294)

The title compound was prepared using the methodologies described above. ¹H NMR (400 MHz, DMSO-*d*₆) *δ* ppm 8.74 (s, 1H), 8.73 (s, 1H), 7.57 (d, J = 8.6 Hz, 2H), 7.53 - 7.45 (m, 3H), 7.39 (d, J = 15.8 Hz, 1H), 7.08 (dd, J = 11.4, 2.8 Hz, 1H), 6.86 (ddd, J = 9.0, 2.9, 1.2 Hz, 1H), 6.54 (d, J = 15.9 Hz, 1H), 4.49 (s, 2H), 2.29 (s, 6H); MS (ESI⁺) *m*/*z* 449 (M+H)⁺.

### Example 196: 2-(3,4-dichlorophenoxy)-N-(3-{2-[(6-methylpyridin-3-yl)oxy]acetamidolbicyclo[1.1.1]pentan-1-yl)acetamide (Compound 295)

The reaction and purification conditions described in Example 107B substituting the product of Example 6C for the product of Example 9B gave the title compound. ¹H NMR (400 MHz, DMSO-*d*₆) *δ* ppm 8.71 (br s, 2H), 8.16 (d, J = 3.0 Hz, 1H), 7.55 (d, J = 8.9 Hz, 1H), 7.29 - 7.23 (m, 2H), 7.19 - 7.14 (m, 1H), 6.99 (dd, J = 8.9, 2.9 Hz, 1H), 4.49 (s, 2H), 4.47 (s, 2H), 2.39 (s, 3H), 2.26 (br s, 6H); MS (ESI⁺) *m*/*z* 450 (M+H)⁺.

### Example 197: 2-{[2,6-bis(trifluoromethyl)pyridin-4-yl]oxy}-N-{3-[2-(4-chloro-3-fluorophenoxy)acetamido]bicyclo[1.1.1]pentan-1-yl}acetamide (Compound 296)

A mixture of Example 28A (60.0 mg, 0.166 mmol), 2,6-bis(trifluoromethyl)pyridin-4-ol (57.6 mg, 0.25 mmol), and potassium carbonate (45.9 mg, 0.33 mmol) in acetone (2.5 mL) was heated at 130 °C in a Biotage^{®} Initiator microwave reactor for 20 minutes. The reaction mixture was concentrated under reduced pressure. The residue was treated with brine and extracted with ethyl acetate (2×). The combined organic layers were concentrated under reduced pressure, and the residue was purified by reverse-phase HPLC (see protocol in Example 112D) to provide the title compound (35 mg, 0.063 mmol, 38% yield). ¹H NMR (400 MHz, DMSO-*d*₆) *δ* ppm 8.82 (s, 1H), 8.71 (s, 1H), 7.78 (s, 2H), 7.48 (t, J = 8.9 Hz, 1H), 7.05 (dd, J = 11.4, 2.8 Hz, 1H), 6.83 (ddd, J = 9.0, 2.9, 1.2 Hz, 1H), 4.84 (s, 2H), 4.46 (s, 2H), 2.26 (s, 6H); MS (ESI⁺) *m*/*z* 556.0 (M+H)⁺.

### Example 198: N,N'-[(2S)-2-hydroxybicyclo[2.2.2]octane-1,4-diyl]bis[2-(4-chloro-3-fluorophenoxy)acetamide] (Compound 297)

### Example 198A: ethyl 1,4-dioxaspiro[4.5]decane-8-carboxylate

A mixture of ethyl 4-oxocyclohexanecarboxylate (11.70 mL, 73.4 mmol), ethane-1,2-diol (12.29 mL, 220 mmol), and *p*-toluenesulfonic acid monohydrate (1.397 g, 7.34 mmol) in toluene (200 mL) was stirred at 120 °C with a Dean-Stark trap apparatus for 180 minutes. The reaction mixture was neutralized with *N*-ethyl-*N*-isopropylpropan-2-amine and then concentrated. The residue was purified on silica gel (0-30% ethyl acetate in heptane) to give 12.77 g of the title compound as a clear oil. ¹H NMR (400 MHz, DMSO-*d*₆) *δ* ppm 4.01 (q, *J* = 7.1 Hz, 2H), 3.81 (s, 4H), 2.32 (tt, *J* = 10.4, 3.8 Hz, 1H), 1.83 - 1.71 (m, 2H), 1.66 - 1.57 (m, 1H), 1.62 - 1.38 (m, 5H), 1.13 (t, *J* = 7.1 Hz, 3H).

### Example 198B: ethyl 8-acetyl-1,4-dioxaspiro[4.5]decane-8-carboxylate

To a solution of diisopropylamine (5.19 mL, 36.4 mmol) in tetrahydrofuran (25 mL) at 0 °C was added *n*-butyllithium slowly below 5 °C. After stirring for 30 minutes, the solution was cooled to -78 °C under nitrogen, and a solution of Example 198A (6.0 g, 28.0 mmol) in tetrahydrofuran (3 mL) was added slowly, and the resultant mixture was stirred for 30 minutes at the same temperature. Then acetyl chloride (2.59 mL, 36.4 mmol) was added slowly to maintain the temperature below -60 °C, and the mixture was stirred at -70 °C for 2 hours. The reaction was quenched with saturated NH₄Cl solution, and the aqueous phase was extracted with ethyl acetate. The organic layer was washed with brine, dried over magnesium sulfate and filtered. The filtrate was concentrated, and the residue was purified on silica gel (0-70% ethyl acetate in heptane) to give 6.78 g of the title compound as a clear oil. ¹H NMR (500 MHz, DMSO-*d*₆) *δ* ppm 4.19 - 4.11 (m, 2H), 3.85 (s, 4H), 2.13 (s, 3H), 2.10 - 2.01 (m, 2H), 1.90 (ddd, *J* = 13.9, 9.6, 4.6 Hz, 2H), 1.54 (th, *J* = 13.6, 4.7 Hz, 4H), 1.18 (dd, *J* = 7.6, 6.5 Hz, 3H).

### Example 198C: ethyl 1-acetyl-4-oxocyclohexane-1-carboxylate

A mixture of Example 198B (6.5 g, 25.4 mmol) and HCl (21.13 mL, 127 mmol) in acetone (60 mL) was stirred at ambient temperature overnight. Volatiles were removed under reduced pressure, and the residue was partitioned between water and dichloromethane. The organic layer was washed with brine, dried over magnesium sulfate and filtered. The filtrate was concentrated to give 5.46 g of the title compound as a clear oil, used without further purification. ¹H NMR (400 MHz, DMSO-*d*₆) *δ* ppm 4.16 (q, *J* = 7.1 Hz, 2H), 2.17 (s, 3H), 2.35 2.07 (m, 8H), 1.17 (t, *J* = 7.1 Hz, 3H).

### Example 198D: ethyl 4-(benzylamino)-2-oxobicyclo[2.2.2]octane-1-carboxylate

A mixture of Example 198C (9.7 g, 45.7 mmol), benzylamine (14.98 mL, 137 mmol), and p-toluenesulfonic acid monohydrate (0.087 g, 0.457 mmol) in toluene (100 mL) was stirred at 130 °C with Dean-Stark trap apparatus overnight. The mixture was concentrated, and the residue was stirred with a mixture of ethyl acetate (50 mL) and 3 N HCl (100 mL) for 30 minutes. The precipitate was collected by filtration, washed with mixture of ethyl acetate/heptane, air-dried to give 11.3 g of title compound as a HCl salt. The filtrate was neutralized with 6 N NaOH and extracted with ethyl acetate (100 mL × 2). The organic layer was washed with brine, dried over magnesium sulfate and filtered. The residue was purified on silica gel (0-70% ethyl acetate in heptane) to give another 0.77 g of the title compound as yellow solid. ¹H NMR (400 MHz, DMSO-*d*₆) *δ* ppm 9.73 (t, *J* = 6.2 Hz, 2H), 7.87 - 7.12 (m, 5H), 4.09 (m, 4H), 2.88 (s, 2H), 2.08 (dt, *J* = 20.7, 13.4 Hz, 6H), 1.16 (t, *J* = 7.1 Hz, 3H); MS (ESI⁺) *m*/*z* 302.1 (M+H)⁺.

### Example 198E: ethyl 4-amino-2-oxobicyclo[2.2.2]octane-1-carboxylate, hydrochloric acid

To a mixture of Example 198D (11.2 g, 33.2 mmol) in tetrahydrofuran (110 mL) in a 50 mL pressure bottle was added 20% Pd(OH)₂/C, wet (2.2 g, 1.598 mmol), and the reaction was shaken at 50 °C under 50 psi of hydrogen for 22 hours. The reaction mixture was cooled to ambient temperature, solids were removed by filtration and washed with methanol (1 L). The filtrate and wash were concentrated to give 7.9 g of the title compound as a light yellow solid. ¹H NMR (400 MHz, DMSO-*d*₆) *δ* ppm 8.46 (s, 3H), 4.07 (q, *J* = 7.1 Hz, 2H), 2.62 (s, 2H), 2.17 - 2.05 (m, 2H), 2.04 - 1.78 (m, 6H), 1.14 (t, *J* = 7.1 Hz, 3H).

### Example 198F: ethyl 4-[2-(4-chloro-3-fluorophenoxy)acetamido]-2-oxobicyclo[2.2.2]octane-1-carboxylate

To a suspension of Example 198E (7.8 g, 31.5 mmol), *N*-ethyl-*N*-isopropylpropan-2-amine (22.00 mL, 126 mmol) and 2-(4-chloro-3-fluorophenoxy)acetic acid (7.41 g, 36.2 mmol) in N,N-dimethylformamide (200 mL), 2-(3H-[1,2,3]triazolo[4,5-b]pyridin-3-yl)-1,1,3,3-tetramethylisouronium hexafluorophosphate(V) (14.97 g, 39.4 mmol) was added, and the resulting brown solution was stirred at ambient temperature for 16 hours. Water was added, and the mixture was stirred for 15 minutes. The precipitate was collected by filtration, washed with water, and air-dried to give 12.1 g of the title compound as an off-white solid. ¹H NMR (400 MHz, DMSO-*d*₆) *δ* ppm 7.87 (s, 1H), 7.45 (t, *J* = 8.9 Hz, 1H), 7.00 (dd, *J* = 11.4, 2.9 Hz, 1H), 6.79 (ddd, *J* = 8.9, 2.9, 1.2 Hz, 1H), 4.45 (s, 2H), 4.06 (q, *J* = 7.1 Hz, 2H), 2.73 (s, 2H), 2.07 (m, 1H), 2.01 - 1.84 (m, 6H), 1.14 (t, *J* = 7.1 Hz, 3H); MS (ESI⁺) *m*/*z* 398.0 (M+H)⁺.

### Example 198G: 4-[2-(4-chloro-3-fluorophenoxy)acetamido]-2-oxobicyclo[2.2.2]octane-1-carboxylic acid

A suspension of Example 198F (11.37 g, 28.6 mmol) and sodium hydroxide (7.15 mL, 57.2 mmol, 8 M solution) in methanol (100 mL) was stirred at ambient temperature for 16 hours. Volatiles were removed, and the residue was acidified with 1 N HCl. The precipitate was collected by filtration and dried in vacuum oven to give 9.9 g of the title compound as a white solid. ¹H NMR (400 MHz, DMSO-*d*₆) *δ* ppm 12.49 (s, 1H), 7.86 (s, 1H), 7.45 (t, *J* = 8.9 Hz, 1H), 7.00 (dd, *J* = 11.4, 2.9 Hz, 1H), 6.83 - 6.74 (m, 1H), 4.45 (s, 2H), 2.71 (s, 2H), 2.01 - 1.81 (m, 7H); MS (ESI⁻) *m*/*z* 368.1 (M-H)⁻.

### Example 198H: N-(4-amino-3-oxobicyclo[2.2.2]octan-1-yl)-2-(4-chloro-3-fluorophenoxy)acetamide

A mixture of Example 198G (3.24 g, 8.76 mmol), diphenylphosphoryl azide (2.84 mL, 13.14 mmol), and triethylamine (3.66 mL, 26.3 mmol) in toluene (100 mL) was heated at 110 °C for 2 hours. The solution was cooled to ambient temperature and poured into 150 mL of 3 N HCl solution. The mixture was stirred for 16 hours to give a suspension. The precipitate was filtered, washed with ethyl acetate, and air-dried to give the title compound (1.63 g) as an HCl salt as a white solid. The filtrate was then basified with solid sodium bicarbonate and extracted with ethyl acetate. The organic layer was washed with brine, dried over magnesium sulfate and filtered. The filtrate was concentrated and purified on silica gel (0-10% methanol/dichloromethane) to give the title compound (0.6 g) as the free base. ¹H NMR (400 MHz, DMSO-*d*₆) *δ* ppm 8.49 (s, 3H), 8.08 (s, 1H), 7.45 (t, J = 8.9 Hz, 1H), 7.01 (dd, J = 11.4, 2.8 Hz, 1H), 6.79 (ddd, J = 9.0, 2.9, 1.2 Hz, 1H), 4.48 (s, 2H), 2.90 (s, 2H), 2.12 - 1.79 (m, 8H).

### Example 198I: N-(4-amino-3-hydroxybicyclo[2.2.2]octan-1-yl)-2-(4-chloro-3-fluorophenoxy)acetamide hydrochloride

A mixture of Example 198H (2.5 g, 6.63 mmol) and sodium borohydride (1.254 g, 33.1 mmol) in a 1:1 mixture of methanol/dichloromethane (50 mL) was stirred for 24 hours. Volatiles were removed, and the residue was partitioned between water and dichloromethane. The organic fraction was separated, dried (MgSO₄), and concentrated. The residue was then treated with 4 N HCl in dioxane. The suspension was sonicated and concentrated. The residue was dried under vacuum to give 2.82 g of the title compound as a light yellow solid. ¹H NMR (400 MHz, DMSO-*d*₆) *δ* ppm 7.97 (s, 3H), 7.72 (s, 1H), 7.40 (t, *J =* 8.9 Hz, 1H), 6.95 (dd, *J =* 11.4, 2.8 Hz, 1H), 6.74 (ddd, *J* = 9.0, 2.9, 1.1 Hz, 1H), 5.64 (s, 1H), 4.41 (s, 2H), 3.83 (d, *J* = 9.1 Hz, 1H), 2.24 (td, *J =* 10.8, 9.9, 5.3 Hz, 1H), 1.96 - 1.51 (m, 9H); MS (ESI⁺) *m*/*z* 343.0 (M+H)⁺.

### Example 198J: N,N'-(2-oxobicyclo[2.2.2]octane-1,4-diyl)bis[2-(4-chloro-3-fluorophenoxy)acetamide]

A mixture of Example 198H (0.5 g, 1.325 mmol), 2-(4-chloro-3-fluorophenoxy)acetic acid (0.339 g, 1.657 mmol) and *N*-ethyl-*N*-isopropylpropan-2-amine (1.157 mL, 6.63 mmol) in N,N-dimethylformamide (20 mL) was treated with 2-(3H-[1,2,3]triazolo[4,5-b]pyridin-3-yl)-1,1,3,3-tetramethylisouronium hexafluorophosphate(V) (0.756 g, 1.988 mmol), and the reaction mixture was stirred at ambient temperature for 30 minutes to see a complete conversion. Water was added, and the resultant mixture was stirred for 15 minutes. The precipitate was collected by filtration, washed with water, and dried in vacuum oven at 50 °C for 2 hours to give 0.64 g of the title compound as a white solid. ¹H NMR (400 MHz, DMSO-*d*₆) *δ* ppm 7.88 (s, 1H), 7.67 (s, 1H), 7.45 (td, J = 8.9, 2.4 Hz, 2H), 7.03 (ddd, J = 15.0, 11.4, 2.8 Hz, 2H), 6.80 (dddd, J = 10.3, 8.9, 2.9, 1.2 Hz, 2H), 4.53 (s, 2H), 4.45 (s, 2H), 2.83 (s, 2H), 2.45 - 2.33 (m, 2H), 2.10 - 1.90 (m, 4H), 1.81 (td, J = 11.6, 6.3 Hz, 2H); MS (ESI⁺) *m*/*z* 527.0 (M+H)⁺.

### Example 198K: N,N'-(2-hydroxybicyclo[2.2.2]octane-1,4-diyl)bis[2-(4-chloro-3-fluorophenoxy)acetamide]

To a solution of Example 198J (0.63 g, 1.195 mmol) in dichloromethane (10 mL) and methanol (10 mL), sodium borohydride (0.226 g, 5.97 mmol) was added portionwise, and the mixture was stirred at ambient temperature for 4 hours. Volatiles were removed, and the residue was triturated with dichloromethane/methanol to give 0.32 g of the title compound as a white solid. The filtrate was concentrated, and the residue was purified on silica gel (10-100% ethyl acetate in heptane) to give 0.21 g of the title compound. ¹H NMR (400 MHz, DMSO-*d*₆) *δ* ppm 7.49 - 7.39 (m, 3H), 7.22 (s, 1H), 7.00 (ddd, *J =* 12.4, 11.4, 2.8 Hz, 2H), 6.78 (tdd, *J* = 9.1*,* 2.9, 1.2 Hz, 2H), 5.04 (s, 1H), 4.41 (d, *J* = 13.3 Hz, 4H), 4.00 (dd, *J* = 9.6, 3.1 Hz, 1H), 2.23 (ddd, *J* = 12.1, 9.4, 2.2 Hz, 1H), 2.09 - 1.97 (m, 1H), 1.93 - 1.80 (m, 2H), 1.84 - 1.68 (m, 6H); MS (ESI⁺) *m*/*z* 529.1 (M+H)⁺.

### Example 198L: N,N'-[(2S)-2-hydroxybicyclo[2.2.2]octane-1,4-diyl]bis[2-(4-chloro-3-fluorophenoxy)acetamide]

The title compound was isolated by chiral preparative SFC (Supercritical Fluid Chromatography) of Example 198K as the second peak eluted off the column. Preparative SFC was performed on a THAR/Waters SFC 80 system running under SuperChrom^{™} software control. The preparative SFC system was equipped with an 8-way preparative column switcher, CO₂ pump, modifier pump, automated back pressure regulator (ABPR), UV detector, and 6-position fraction collector. The mobile phase was comprised of supercritical CO₂ supplied by a Dewar of bone-dry non-certified CO₂ pressurized to 350 psi with a modifier of methanol at a flow rate of 70 g/minute. The column was at ambient temperature, and the backpressure regulator was set to maintain 100 bar. The sample was dissolved in a mixture of methanol/dichloromethane (1:1) at a concentration of 10 mg/mL. The sample was loaded into the modifier stream in 1 mL (10 mg) injections. The mobile phase was held isocratically at 30% methanol:CO₂. Fraction collection was time triggered. The instrument was fitted with a Chiralpak^{®} AD-H column with dimensions 21 mm i.d. × 250 mm length with 5 µm particles. ¹H NMR (400 MHz, DMSO-*d*₆) *δ* ppm 7.49 - 7.39 (m, 3H), 7.23 (s, 1H), 7.00 (ddd, *J =* 12.4, 11.4, 2.9 Hz, 2H), 6.78 (dddd, *J =* 9.0, 8.0, 2.9, 1.2 Hz, 2H), 5.05 (s, 1H), 4.41 (d, *J =* 13.5 Hz, 4H), 4.00 (dd, *J* = 9.4, 3.0 Hz, 1H), 2.23 (ddd, *J =* 12.3, 9.4, 2.3 Hz, 1H), 2.03 (ddd, *J =* 12.3, 10.5, 4.7 Hz, 1H), 1.89 (d, *J =* 10.7 Hz, 2H), 1.87 - 1.76 (m, 1H), 1.74 (ddd, *J =* 12.6, 6.7, 2.4 Hz, 5H); MS (ESI⁺) *m*/*z* 529.1 (M+H)⁺.

### Example 199: N,N'-[(2R)-2-hydroxybicyclo[2.2.2]octane-1,4-diyl]bis[2-(4-chloro-3-fluorophenoxy)acetamide] (Compound 298)

The title compound was isolated using the chiral preparative SFC described in Example 198 as the first peak eluted off the column. ¹H NMR (400 MHz, DMSO-*d*₆) *δ* ppm 7.53 - 7.43 (m, 3H), 7.28 (s, 1H), 7.04 (ddd, *J =* 12.3, 11.5, 2.9 Hz, 2H), 6.82 (tdd, *J =* 9.0, 2.9, 1.2 Hz, 2H), 5.10 (s, 1H), 4.45 (d, *J =* 13.5 Hz, 4H), 4.04 (dd, *J =* 9.6, 3.1 Hz, 1H), 2.27 (ddd, *J =* 12.2, 9.4, 2.2 Hz, 1H), 2.07 (ddd, *J =* 12.2, 10.4, 4.7 Hz, 1H), 1.96 - 1.72 (m, 8H); MS (ESI⁺) *m*/*z* 529.1 (M+H)⁺.

### Example 200: N,N'-(2-methoxybicyclo[2.2.2]octane-1,4-diyl)bis[2-(4-chloro-3-fluorophenoxy)acetamide] (Compound 299)

To a suspension of Example 198K (0.05 g, 0.094 mmol) and potassium hydroxide (6.62 mg, 0.118 mmol) in dimethyl sulfoxide (1.0 mL) at 0 °C was added iodomethane (7.38 µL, 0.118 mmol), and the mixture was stirred at ambient temperature for 30 minutes. Water was added, and the mixture was extracted with dichloromethane. The organic layer was dried over magnesium sulfate and concentrated. The residue was purified by HPLC (performed on a Phenomenex^{®} Luna^{®} C18(2) 5 µm 100Å AXIA^{™} column (250 mm × 21.2 mm) or Phenomenex^{®} Luna^{®} C18(2) 10 µm 100Å AXIA^{™} column (250 mm × 50 mm). A gradient of acetonitrile (A) and 0.1% trifluoroacetic acid in water (B) was used, at a flow rate of 25 mL/minute. A linear gradient was used from about 5% of A to about 95% of A over about 10 minutes. Detection method was UV at wave lengths of 218 nM and 254 nM) to give 18 mg of the title compound as a white solid. ¹H NMR (400 MHz, CDCl₃) *δ* ppm 7.32 (td, J = 8.6, 3.7 Hz, 2H), 6.78 - 6.70 (m, 2H), 6.75 - 6.61 (m, 3H), 6.14 (s, 1H), 4.35 (d, J = 5.7 Hz, 4H), 3.73 (ddd, J = 9.2, 3.8, 1.6 Hz, 1H), 3.34 (s, 3H), 2.65 - 2.45 (m, 3H), 2.17 - 1.81 (m, 7H); MS (ESI⁻) *m*/*z* 541.2 (M-H)⁻.

### Example 201: N,N'-[2-(dimethylamino)bicyclo[2.2.2]octane-1,4-diyl]bis[2-(4-chloro-3-fluorophenoxy)acetamide] (Compound 300)

A mixture of Example 198J (100 mg, 0.190 mmol), dimethylamine (0.119 mL, 0.237 mmol) and tetraisopropoxytitanium(IV) (0.167 mL, 0.569 mmol) in dichloroethane (2.0 mL) was stirred at 85 °C in a microwave vial for 8 hours. The vial was cooled to ambient temperature and sodium triacetoxyborohydride (201 mg, 0.948 mmol) was added, and the mixture was stirred at 85 °C for 16 hours. Volatiles were removed, and the residue was purified by HPLC (10-95% acetonitrile in 0.1% trifluoroacetic acid/water over 15 minutes at 25 mL/minute on a Phenomenex^{®} C18 5 µm (250 mm × 21.2 mm) column) to give 6 mg of the titled compound as a solid. ¹H NMR (400 MHz, DMSO-*d*₆) *δ* ppm 8.95 (s, 1H), 7.98 (s, 1H), 7.86 (s, 1H), 7.54 (td, *J =* 8.9, 4.6 Hz, 2H), 7.10 (td, *J =* 11.3, 2.8 Hz, 2H), 6.88 (td, *J =* 8.6, 2.7 Hz, 2H), 4.66 - 4.50 (m, 4H), 4.34 (d, *J =* 9.4 Hz, 1H), 2.84 (dd, *J =* 21.6, 4.7 Hz, 6H), 2.37 (td, *J =* 11.9, 10.4, 2.8 Hz, 1H), 2.32 - 2.08 (m, 4H), 2.12 - 2.00 (m, 1H), 1.97 - 1.74 (m, 3H), 1.52 (ddd, *J =* 22.1, 14.1, 8.1 Hz, 1H); MS (ESI⁺) *m*/*z* 556.1 (M+H)⁺.

### Example 202: 2-(4-chloro-3-fluorophenoxy)-N-[3-hydroxy-4-(2-{[6-(trifluoromethyl)pyridin-3-yl]oxy}acetamido)bicyclo[2.2.2]octan-1-yl]acetamide (Compound 301)

A mixture of Example 198I (0.1 g, 0.219 mmol), 2-((6-(trifluoromethyl)pyridin-3-yl)oxy)acetic acid (0.073 g, 0.328 mmol) and *N*-ethyl-*N*-isopropylpropan-2-amine (0.153 mL, 0.876 mmol) in N,N-dimethylformamide (2.0 mL) was treated with 2-(3H-[1,2,3]triazolo[4,5-*b*]pyridin-3-yl)-1,1,3,3-tetramethylisouronium hexafluorophosphate(V) (0.125 g, 0.328 mmol), and the reaction mixture was stirred at ambient temperature overnight. Volatiles were removed under high vacuum, and the residue was purified by HPLC (10-95% acetonitrile in 0.1% trifluoroacetic acid/water over 15 minutes at 25 mL/minute on a Phenomenex^{®} C18 5 µm (250 mm × 21.2 mm) column) to give 47 mg of the title compound as a solid. ¹H NMR (400 MHz, DMSO-*d*₆) *δ* ppm 8.40 (d, J = 2.9 Hz, 1H), 7.81 (d, J = 8.8 Hz, 1H), 7.55 - 7.40 (m, 3H), 7.36 (s, 1H), 6.99 (dd, J = 11.4, 2.8 Hz, 1H), 6.77 (ddd, J = 9.0, 3.0, 1.2 Hz, 1H), 5.00 (s, 1H), 4.61 (s, 2H), 4.40 (s, 2H), 4.05 (d, J = 9.3 Hz, 1H), 2.23 (dd, J = 13.2, 9.5 Hz, 1H), 2.04 - 1.85 (m, 4H), 1.81 - 1.68 (m, 5H); MS (ESI⁺) *m*/*z* 546.0 (M+H)⁺.

### Example 203: 2-(4-chloro-3-fluorophenoxy)-N-[(3S)-3-hydroxy-4-(2-{[6-(trifluoromethyl)pyridin-3-yl]oxy}acetamido)bicyclo[2.2.2]octan-1-yl]acetamide (Compound 302)

The title compound was isolated by chiral preparative SFC of Example 202 as the second peak eluted off the column using the methodologies described in Example 198. ¹H NMR (400 MHz, DMSO-*d*₆) *δ* ppm 8.40 (d, J = 2.9 Hz, 1H), 7.81 (d, J = 8.7 Hz, 1H), 7.55 - 7.40 (m, 3H), 7.36 (s, 1H), 6.99 (dd, J = 11.4, 2.9 Hz, 1H), 6.77 (ddd, J = 9.0, 2.9, 1.2 Hz, 1H), 5.00 (d, J = 4.3 Hz, 1H), 4.61 (s, 2H), 4.40 (s, 2H), 4.04 (dt, J = 8.1, 3.2 Hz, 1H), 2.23 (dd, J = 13.1, 9.6 Hz, 1H), 2.04 - 1.81 (m, 4H), 1.86 1.68 (m, 5H); MS (ESI⁺) *m*/*z* 545.9 (M+H)⁺.

### Example 204: 2-(4-chloro-3-fluorophenoxy)-N-[(3R)-3-hydroxy-4-(2-{[6-(trifluoromethyl)pyridin-3-yl]oxy}acetamido)bicyclo[2.2.2]octan-1-yl]acetamide (Compound 303)

The title compound was isolated by chiral preparative SFC of Example 202 as the first peak eluted off the column using the methodologies described in Example 198. ¹H NMR (400 MHz, DMSO-*d*₆) *δ* ppm 8.40 (d, J = 2.9 Hz, 1H), 7.81 (d, J = 8.7 Hz, 1H), 7.55 7.36 (m, 4H), 6.98 (dd, J = 11.4, 2.8 Hz, 1H), 6.77 (ddd, J = 8.9, 2.9, 1.2 Hz, 1H), 5.03 (s, 1H), 4.61 (s, 2H), 4.40 (s, 2H), 4.04 (d, J = 9.0 Hz, 1H), 2.23 (dd, J = 13.2, 9.6 Hz, 1H), 2.04 - 1.81 (m, 4H), 1.83 - 1.68 (m, 5H); MS (ESI⁺) *m*/*z* 546.0 (M+H)⁺.

### Example 205: benzyl [2-({3-[2-(4-chloro-3-fluorophenoxy)acetamido]bicyclo[1.1.1]pentan-1-yl}amino)-2-oxoethyl]carbamate (Compound 304)

The reaction and purification conditions described in Example 107B substituting ((benzyloxy)carbonyl)glycine (Aldrich) for the product of Example 107A gave the title compound. ¹H NMR (400 MHz, DMSO-*d*₆) *δ* ppm 8.69 (s, 1H), 8.47 (s, 1H), 7.49 (t, J = 8.9 Hz, 1H), 7.40 - 7.27 (m, 6H), 7.07 (dd, J = 11.4, 2.9 Hz, 1H), 6.85 (ddd, J = 8.9, 2.9, 1.2 Hz, 1H), 5.03 (s, 2H), 4.47 (s, 2H), 3.55 (d, J = 6.2 Hz, 2H), 2.22 (br s, 6H); MS (ESI⁺) *m*/*z* 476 (M+H)⁺.

### Example 206: 2-(4-chloro-3-fluorophenoxy)-N-(4-{2-[(2,2-difluoro-2H-1,3-benzodioxol-5-yl)oxy]acetamido}-3-hydroxybicyclo[2.2.2]octan-1-yl)acetamide (Compound 305)

### Example 206A: 2,2-difluoro-2H-1,3-benzodioxol-5-ol

To a cold solution of 5-bromo-2,2-difluorobenzo[d][1,3]dioxole (5.75 mL, 42.2 mmol) in tetrahydrofuran (80 mL) was added a 2.0 M solution of isopropylmagnesium chloride in tetrahydrofuran (28.1 mL, 56.1 mmol) within 5-7 minutes by keeping the temperature around 10-20 °C. The reaction mixture was stirred at the same temperature for 15 minutes and then was allowed to attain ambient temperature for overnight. The reaction mixture was cooled with an ice bath, and triisopropyl borate (12.74 mL, 54.9 mmol) was added dropwise over 2 minutes. The reaction mixture was stirred at ambient temperature for 30 minutes. Then the reaction mixture was cooled to 10 °C, and 10% sulfuric acid solution (50 mL) was added slowly to the reaction mixture resulting in a slight exotherm up to 20 °C. The reaction mixture was stirred at ambient temperature for 15 minutes and transferred to separating funnel. Some water was added to dissolve salts. The aqueous layer was separated and washed with ethyl acetate. The combined organic fractions were washed with a saturated aqueous solution of sodium bicarbonate. The organic extract was separated, dried over anhydrous sodium sulfate, filtered, and concentrated. The residue was then dissolved in *tert-butyl* methyl ether (100 mL), cooled to 0 °C and 30% hydrogen peroxide solution in water (5.39 mL, 52.7 mmol) was added slowly to the reaction mixture followed by water (60 mL). The mixture was stirred overnight while warming up to ambient temperature. The reaction mixture was diluted with ethyl acetate and washed twice with 10% sodium thiosulfate solution and brine. The organic layer was dried with magnesium sulfate and filtered. The filtrate was concentrated, and the residue was purified on silica gel (0-50% ethyl acetate in heptane) to give 6.43 g of the title compound as an amber oil. ¹H NMR (400 MHz, DMSO-*d*₆) *δ* ppm 9.79 (s, 1H), 7.16 (d, *J =* 8.7 Hz, 1H), 6.79 (d, *J =* 2.4 Hz, 1H), 6.56 (dd, *J =* 8.7, 2.5 Hz, 1H).

### Example 206B: 2-((2,2-difluorobenzo[d][1,3]dioxol-5-yl)oxy)acetic acid

To a solution of Example 206A in N,N-dimethylformamide (30 mL) at ambient temperature was added potassium carbonate (4.76 g, 34.5 mmol) and *tert-butyl* bromoacetate (2.91 mL, 19.82 mmol). This mixture was warmed to 65 °C and was allowed to stir for 1.5 hours. The mixture was allowed to cool to ambient temperature and was diluted with ethyl acetate (50 mL) and water (50 mL). The layers were separated, and the aqueous layer was extracted with ethyl acetate (3 × 15 mL). The combined organic extracts were dried over anhydrous Na₂SO₄, filtered, concentrated under reduced pressure to give *tert-*butyl [(2,2-difluoro-2H-1,3-benzodioxol-5-yl)oxy]acetate which was used without further purification. This crude was dissolved in methanol (60 mL) and water (20.00 mL) and treated with 5 M sodium hydroxide solution (17.35 mL, 87 mmol). This reaction mixture was allowed to stir at ambient temperature for 2 hours. Volatiles were removed under reduced pressure, and the residue was acidified with 1 N HCl solution. The resulting precipitate was collected by filtration, air-dried to give the title compound (3.28 g, 14.13 mmol, 81 % yield) as a white solid. ¹H NMR (400 MHz, DMSO-*d*₆) *δ* ppm 13.10 (s, 1H), 7.30 (d, J = 8.9 Hz, 1H), 7.13 (d, J = 2.6 Hz, 1H), 6.73 (dd, J = 8.9, 2.6 Hz, 1H), 4.69 (s, 2H); MS (ESI⁻) *m*/*z* 231.0 (M-H)⁻.

### Example 206C: 2-(4-chloro-3-fluorophenoxy)-N-(4-{2-[(2,2-difluoro-2H-1,3-benzodioxol-5-yl)oxy]acetamido}-3-hydroxybicyclo[2.2.2]octan-1-yl)acetamide

The title compound was prepared using the methodologies described in Example 202 substituting Example 206B for 2-((6-(trifluoromethyl)pyridin-3-yl)oxy)acetic acid. ¹H NMR (501 MHz, DMSO-*d*₆) *δ* ppm 7.49 - 7.40 (m, 2H), 7.28 (d, J = 8.9 Hz, 1H), 7.19 (s, 1H), 7.09 (d, J = 2.6 Hz, 1H), 6.99 (dd, J = 11.4, 2.8 Hz, 1H), 6.81 - 6.67 (m, 2H), 4.39 (d, J = 2.6 Hz, 4H), 3.99 (dd, J = 9.7, 3.1 Hz, 1H), 2.24 (ddd, J = 12.4, 9.5, 2.4 Hz, 1H), 2.12 - 2.00 (m, 1H), 1.96 - 1.69 (m, 8H); MS (ESI⁺) *m*/*z* 557.0 (M+H)⁺.

### Example 207: 2-(4-chloro-3-fluorophenoxy)-N-[3-hydroxy-4-(2-{[5-(trifluoromethyl)pyridin-3-yl]oxy}acetamido)bicyclo[2.2.2]octan-1-yl]acetamide (Compound 306)

### Example 207A: 2-chloro-N-[4-[2-(4-chloro-3-fluorophenoxy)acetamido]-2-oxobicyclo[2.2.2]octan-1-yljacetamide

To a mixture of Example 198H (0.6 g, 1.591 mmol) and *N*-ethyl-*N*-isopropylpropan-2-amine (1.389 mL, 7.95 mmol) in tetrahydrofuran (10.0 mL), 2-chloroacetyl chloride (0.198 g, 1.750 mmol) was added dropwise, and the mixture was stirred at ambient temperature overnight. Water was added, and the mixture was extracted with ethyl acetate. The organic layer was washed with brine, dried over magnesium sulfate and filtered. The filtrate was concentrated, and the residue was purified on silica gel (0-75%ethyl acetate in heptane) to afford 0.43 g of the title compound as a white solid. ¹H NMR (400 MHz, DMSO-*d*₆) *δ* ppm 7.86 (d, J = 13.7 Hz, 2H), 7.45 (t, J = 8.9 Hz, 1H), 7.01 (dd, J = 11.4, 2.9 Hz, 1H), 6.79 (ddd, J = 9.0, 2.9, 1.2 Hz, 1H), 4.45 (s, 2H), 4.09 (s, 2H), 2.82 (s, 2H), 2.37 - 1.75 (m, 8H); MS (ESI⁺) *m*/*z* 417.0 (M+H)⁺.

### Example 207B: 2-(4-chloro-3-fluorophenoxy)-N-[3-hydroxy-4-(2-{[5-(trifluoromethyl)pyridin-3-yl]oxy}acetamido)bicyclo[2.2.2]octan-1-yl]acetamide

A mixture of Example 207A (0.043 g, 0.103 mmol), 5-(trifluoromethyl)pyridin-3-ol (0.034 g, 0.206 mmol), potassium carbonate (0.028 g, 0.206 mmol) and potassium iodide (1.198 mg, 7.21 µmol) in acetone (1.0 mL) was stirred at 140 °C in a 2 mL microwave via for 45 minutes. The cooled suspension was filtered, and the crude material was purified by HPLC (10-85% acetonitrile in 0.1% trifluoroacetic acid/water at 25 mL/minute on Phenomenex^{®} C18 5 µm column) to give 44 mg of 2-(4-chloro-3-fluorophenoxy)-N-[3-oxo-4-(2-{ [5-(trifluoromethyl)pyridin-3-yl]oxy}acetamido)bicyclo[2.2.2]octan-1-yl]acetamide as a light yellow solid. The solid was dissolved in a mixture of methanol/dichloromethane (1:1, 2 mL) and treated with sodium borohydride (0.019 g, 0.515 mmol). The reaction mixture was stirred at ambient temperature for 1 hour. Water was added, and the mixture was extracted with dichloromethane. The organic layer was separated, dried (MgSO₄), and concentrated to give 32 mg of the title compound as a light brown solid. ¹H NMR (400 MHz, DMSO-*d*₆) *δ* ppm 8.58 - 8.50 (m, 2H), 7.68 (s, 1H), 7.49 - 7.39 (m, 2H), 7.35 (s, 1H), 6.98 (dd, J = 11.4, 2.8 Hz, 1H), 6.77 (ddd, J = 9.0, 2.9, 1.2 Hz, 1H), 5.02 (s, 1H), 4.63 (s, 2H), 4.40 (s, 2H), 4.03 (dd, J = 9.8, 3.1 Hz, 1H), 2.29 - 2.18 (m, 1H), 2.04 - 1.76 (m, 4H), 1.80 - 1.68 (m, 5H); MS (ESI⁺) *m*/*z* 546.1 (M+H)⁺.

### Example 208: (2S)-N-{3-[2-(4-chloro-3-fluorophenoxy)acetamido]bicyclo[1.1.1]pentan-1-yl}-2-(4-chlorophenyl)-2-hydroxyacetamide (Compound 307)

The title compound was prepared using the methodologies described above. ¹H NMR (400 MHz, CDCl₃) *δ* ppm 7.35 (s, 4H), 7.32 (t, J = 8.6 Hz, 1H), 6.85 (s, 1H), 6.77 - 6.71 (m, 2H), 6.66 (ddd, J = 8.9, 2.9, 1.3 Hz, 1H), 5.00 (d, J = 3.2 Hz, 1H), 4.37 (s, 2H), 3.54 (d, J = 3.6 Hz, 1H), 2.45 (s, 6H); MS (ESI⁺) *m*/*z* 453 (M+H)⁺.

### Example 209: 2-(4-chloro-3-fluorophenoxy)-N-[3-hydroxy-4-(2-{[2-(trifluoromethyl)pyridin-4-yl]oxy}acetamido)bicyclo[2.2.2]octan-1-yl]acetamide (Compound 308)

The title compound was prepared using the methodologies described in Example 207 substituting 2-(trifluoromethyl)pyridin-4-ol for 5-(trifluoromethyl)pyridin-3-ol. ¹H NMR (501 MHz, DMSO-*d*₆) *δ* ppm 8.53 (d, J = 5.7 Hz, 1H), 7.49 - 7.39 (m, 2H), 7.43 - 7.34 (m, 2H), 7.19 (dd, J = 5.8, 2.5 Hz, 1H), 6.98 (dd, J = 11.4, 2.8 Hz, 1H), 6.77 (dt, J = 8.7, 1.8 Hz, 1H), 4.64 (s, 2H), 4.40 (s, 2H), 4.05 (dd, J = 9.5, 3.0 Hz, 1H), 2.23 (dd, J = 13.2, 9.3 Hz, 1H), 2.02 - 1.74 (m, 9H); MS (ESI⁺) *m*/*z* 546.2 (M+H)⁺.

### Example 210: 2-(4-chloro-3-fluorophenoxy)-N-[3-(2-{[3-oxo-2-(2,2,2-trifluoroethyl)-2,3-dihydropyridazin-4-yl]oxy}acetamido)bicyclo[1.1.1]pentan-1-yl]acetamide (Compound 309)

### Examples 210A and B: tert-butyl {[5-chloro-3-oxo-2-(2,2,2-trifluoroethyl)-2,3-dihydropyridazin-4-yl]oxy}acetate (A) and tert-butyl {[5-chloro-6-oxo-1-(2,2,2-trifluoroethyl)-1,6-dihydropyridazin-4-yl]oxy}acetate (B)

A 100 mL round bottom flask, equipped with a magnetic stir bar, was charged with *tert-*butyl 2-hydroxyacetate (1.07 g, 8.10 mmol) and 4,5-dichloro-2-(2,2,2-trifluoroethyl)pyridazin-3(2*H*)-one (FCH Group; CAS: 97137-16-1; 2 g, 8.10 mmol). The flask contents were placed under a dry nitrogen atmosphere and tetrahydrofuran (THF) (16 mL) was introduced via syringe. The resulting solution was stirred at ambient temperature as lithium bis(trimethylsilyl)amide (1.0 M in THF; 8.10 mL, 8.10 mmol) was added dropwise. The reaction mixture was stirred at ambient temperature for 65 hours. The reaction mixture was diluted with ethyl acetate (30 mL) and washed with dilute aqueous citric acid (2 × 10 mL) and with brine (1 × 10 mL). The organic layer was dried over anhydrous MgSO₄, filtered and concentrated under reduced pressure to give a crude mixture that was purified via column chromatography (SiO₂, 10-35% ethyl acetate/heptanes) to give the earlier eluting title compound A (577 mg, 1.7 mmol, 21% yield) and the later eluting title compound B (768 mg, 2.2 mmol, 28% yield). Title compound A: ¹H NMR (400 MHz, CDCl₃) *δ* ppm 7.77 (s, 1H), 5.21 (s, 2H), 4.71 (q, J = 8.3 Hz, 2H), 1.45 (s, 9H); MS (ESI⁺) *m*/*z* 343 (M+H)⁺. Title Compound B: ¹H NMR (400 MHz, CDCl₃) *δ* ppm 7.68 (s, 1H), 4.80 (q, 8.3 Hz, 2H), 4.80 (s, 2H), 1.50 (s, 9H); MS (ESI⁺) *m*/*z* 343 (M+H)⁺.

### Example 210C: tert-butyl {[3-oxo-2-(2,2,2-trifluoroethyl)-2,3-dihydropyridazin-4-yl]oxy}acetate

The product of Example 210A (575 mg, 1.7 mmol) and tetrahydrofuran (THF) (14 mL) were added to 5% Pd/C (wet, 131 mg, 0.55 mmol) and triethylamine (0.47 mL, 3.4 mmol) in a 20 mL vessel. The mixture was stirred for 22 hours at 50 psi hydrogen and 25 °C. The mixture was then filtered and concentrated under reduced pressure to give the title compound (397 mg, 1.3 mmol, 77% yield). ¹H NMR (400 MHz, CDCl₃) *δ* ppm 7.70 (d, J = 4.8 Hz, 1H), 6.36 (d, J = 4.8 Hz, 1H), 4.80 (q, J = 8.4 Hz, 2H), 4.69 (s, 2H), 1.48 (s, 9H); MS (ESI⁺) *m*/*z* 326 (M+NH₄)⁺.

### Example 210D: {[3-oxo-2-(2,2,2-trifluoroethyl)-2,3-dihydropyridazin-4-yl]oxy}acetic acid

A 100 mL round bottom flask, equipped with a magnetic stir bar, was charged with the product of Example 210C (360 mg, 1.17 mmol) and 1,2-dichloroethane (40 mL). The resulting solution was stirred at ambient temperature as trifluoroacetic acid (0.41 mL, 5.32 mmol) was added. The reaction mixture was then stirred at 75 °C for 40 minutes, then the heat was removed, and the mixture was allowed to stir at ambient temperature for 16 hours. Some starting material remained, so additional trifluoroacetic acid (0.41 mL, 5.32 mmol) was added, and the reaction mixture was stirred at 70 °C for 7.5 hours. The mixture was concentrated under reduced pressure to give the title compound (268 mg, 1.06 mmol, 91% yield. ¹H NMR (400 MHz, DMSO-*d*₆) *δ* ppm 13.30 (s, 1H), 7.87 (d, J = 4.9 Hz, 1H), 6.78 (d, J = 5.0 Hz, 1H), 4.94 (q, J = 9.1 Hz, 2H), 4.84 (s, 2H); MS (ESI⁺) *m*/*z* 270 (M+NH₄)⁺.

### Example 210E: 2-(4-chloro-3-fluorophenoxy)-N-[3-(2-{[3-oxo-2-(2,2,2-trifluoroethyl)-2,3-dihydropyridazin-4-yl]oxy}acetamido)bicyclo[1.1.1]pentan-1-yl]acetamide

A 4 mL vial, equipped with a magnetic stir bar, was charged with the product of Example 210D (43.2 mg, 0.17 mmol). The vial was sealed with a septum screw cap and the contents were placed under a dry nitrogen atmosphere. Dichloromethane (1.0 mL) was introduced via syringe, and the resulting solution was stirred at ambient temperature as oxalyl chloride (0.027 mL, 0.31 mmol) was added via syringe followed by one drop of N,N-dimethylformamide (~0.05 mL). The reaction mixture was stirred at ambient temperature for 30 minutes, then volatiles were removed under reduced pressure. The residue was treated with the product of Example 112A (50 mg, 0.16 mmol), and the vial was resealed. The contents were again placed under a dry nitrogen atmosphere, and dichloromethane (3 mL) was added via syringe. This suspension was stirred at ambient temperature while triethylamine (0.065 mL, 0.47 mmol) was added dropwise. When the addition was complete, the reaction mixture was stirred at ambient temperature for 2.25 hours. Volatiles were removed under reduced pressure, and the residue was partitioned between dilute aqueous citric acid (10 mL) and ethyl acetate (10 mL). The organic layer was washed with saturated aqueous sodium bicarbonate (10 mL), then dried over anhydrous MgSO₄, filtered and concentrated under reduced pressure. The crude residue was purified via column chromatography (SiO₂, 100% CH₂Cl₂ to 3% CH₃OH in CH₂Cl₂) to give the title compound. ¹H NMR (501 MHz, CDCl₃) *δ* ppm 7.77 (d, J = 4.8 Hz, 1H), 7.45 (s, 1H), 7.33 (t, J = 8.6 Hz, 1H), 6.85 (s, 1H), 6.76 (dd, J = 10.3, 2.9 Hz, 1H), 6.68 (ddd, J = 8.9, 2.9, 1.3 Hz, 1H), 6.48 (d, J = 4.9 Hz, 1H), 4.81 (q, J = 8.3 Hz, 2H), 4.44 (s, 2H), 4.40 (s, 2H), 2.52 (s, 6H); MS (ESI⁺) *m*/*z* 519 (M+H)⁺.

### Example 211: 2-(4-chloro-3-fluorophenoxy)-N-[3-(2-{[6-oxo-1-(2,2,2-trifluoroethyl)-1,6-dihydropyridazin-4-yl]oxy}acetamido)bicyclo[1.1.1]pentan-1-yl]acetamide (Compound 310)

### Example 211A: tert-butyl {[6-oxo-1-(2,2,2-trifluoroethyl)-1,6-dihydropyridazin-4-yl]oxy}acetate

The product of Example 210B was processed as described in Example 210C to give the title compound. ¹H NMR (400 MHz, CDCl₃) *δ* ppm 7.70 (d, J = 2.9 Hz, 1H), 6.03 (d, J = 2.9 Hz, 1H), 4.72 (q, J = 8.4 Hz, 2H), 4.49 (s, 2H), 1.51 (s, 9H); MS (ESI⁺) *m*/*z* 309 (M+H)⁺.

### Example 211B: {[6-oxo-1-(2,2,2-trifluoroethyl)-1,6-dihydropyridazin-4-yl]oxy}acetic acid

The product of Example 211A was processed as described for Example 210D to give the title compound (415 mg, 1.65 mmol, 93% yield). ¹H NMR (400 MHz, DMSO-*d*₆) *δ* ppm 13.33 (s, 1H), 7.96 (d, J = 2.8 Hz, 1H), 6.37 (d, J = 2.8 Hz, 1H), 4.89 (q, J = 9.1 Hz, 2H), 4.84 (s, 2H); MS (ESI⁺) *m*/*z* 270 (M+NH₄)⁺.

### Example 211C: 2-(4-chloro-3-fluorophenoxy)-N-[3-(2-{[6-oxo-1-(2,2,2-trifluoroethyl)-1,6-dihydropyridazin-4-yl]oxy}acetamido)bicyclo[1.1.1]pentan-1-yl]acetamide

The product of Example 211B was processed as described for Example 210E to give the title compound (48.5 mg, 0.093 mmol, 60% yield). ¹H NMR (400 MHz, CDCl₃) *δ* ppm 7.72 (d, J = 2.9 Hz, 1H), 7.33 (t, J = 8.6 Hz, 1H), 6.87 (s, 1H), 6.76 (dd, J = 10.2, 2.9 Hz, 1H), 6.69 (s, 1H), 6.68 (ddd, J = 10.2, 2.9, 1.3 Hz, 1H), 6.17 (d, J = 2.9 Hz, 1H), 4.73 (q, J = 8.4 Hz, 2H), 4.42 (s, 2H), 4.41 (s, 2H), 2.54 (s, 6H); MS (ESI⁺) *m*/*z* 519 (M+H)⁺.

### Example 212: 2-(4-chloro-3-fluorophenoxy)-N-(4-{2-[(6-cyclopropylpyridin-3-yl)oxy]acetamido}-3-hydroxybicyclo[2.2.2]octan-1-yl)acetamide (Compound 311)

The title compound was prepared using the methodologies described in Example 207 substituting 6-cyclopropylpyridin-3-ol for 5-(trifluoromethyl)pyridin-3-ol. ¹H NMR (501 MHz, DMSO-*d*₆) *δ* ppm 8.27 (s, 1H), 8.19 (d, J = 2.9 Hz, 1H), 7.62 (dd, J = 8.9, 2.9 Hz, 1H), 7.51 (s, 1H), 7.46 - 7.30 (m, 3H), 6.95 (dd, J = 11.4, 2.9 Hz, 1H), 6.76 (ddd, J = 9.0, 2.9, 1.2 Hz, 1H), 4.52 (s, 2H), 4.37 (s, 2H), 4.03 (dd, J = 9.5, 3.1 Hz, 1H), 2.28 - 2.06 (m, 2H), 1.96 (ddt, J = 17.8, 10.6, 5.6 Hz, 1H), 1.88 (s, 2H), 1.88 - 1.73 (m, 2H), 1.77 - 1.66 (m, 4H), 1.04 (dt, J = 8.4, 3.3 Hz, 2H), 0.94 - 0.85 (m, 2H); MS (ESI⁺) m/z 518.2 (M+H)⁺.

### Example 213: N-{3-[2-(4-chloro-3-fluorophenoxy)acetamido]bicyclo[1.1.1]pentan-1-yl}-3-(4-chlorophenyl)-2-oxopropanamide (Compound 312)

The title compound was prepared using the methodologies described above. ¹H NMR (400 MHz, CDCl₃) *δ* ppm 7.37 - 7.28 (m, 4H), 7.17 (d, J = 8.4 Hz, 2H), 6.86 (s, 1H), 6.76 (dd, J = 10.3, 2.9 Hz, 1H), 6.68 (ddd, J = 8.9, 2.9, 1.3 Hz, 1H), 4.40 (s, 2H), 4.16 (s, 2H), 2.52 (s, 6H); MS (ESI⁺) *m*/*z* 465 (M+H)⁺.

### Example 214: 2-(4-chloro-3-fluorophenoxy)-N-[2-hydroxy-4-(2-{[6-(trifluoromethyl)pyridin-3-yl]oxy}acetamido)bicyclo[2.2.2]octan-1-yl]acetamide (Compound 313)

### Example 214A: ethyl 4-{[(4-methoxyphenyl)methyl]amino}-2-oxobicyclo[2.2.2]octane-1-carboxylate

The title compound was prepared using the methodologies described in Example 198D substituting 4-methoxybenzylamine for benzylamine. ¹H NMR (400 MHz, DMSO-*d*₆) *δ* ppm 9.67 (s, 2H), 7.56 - 7.47 (m, 2H), 6.98 - 6.90 (m, 2H), 4.13 - 3.96 (m, 4H), 3.73 (s, 3H), 2.87 (s, 2H), 2.16 - 1.93 (m, 8H), 1.15 (t, J = 7.1 Hz, 3H).

### Example 214B: 4-{[(4-methoxyphenyl)methyl]amino}-2-oxobicyclo[2.2.2]octane-1-carboxylic acid

The title compound was prepared using the methodologies described in Example 198G substituting 214A for 198F. ¹H NMR (400 MHz, DMSO-*d*₆) *δ* ppm 9.65 (s, 1H), 7.53 - 7.46 (m, 2H), 6.98 - 6.90 (m, 2H), 4.00 (s, 2H), 2.82 (s, 2H), 2.13 - 1.94 (m, 8H); MS (ESI⁺) *m*/*z* 303.8 (M+H)⁺.

### Example 214C: 1-amino-4-{[(4-methoxyphenyl)methyl]amino}bicyclo[2.2.2]octan-2-one

The title compound was prepared using the methodologies described in Example 198H substituting 214B for 198G. ¹H NMR (400 MHz, DMSO-*d*₆) *δ* ppm 9.25 (s, 2H), 8.55 - 8.50 (m, 3H), 7.44 - 7.35 (m, 2H), 7.02 - 6.93 (m, 2H), 4.05 (s, 1H), 3.73 (s, 2H), 2.94 (s, 2H), 2.19 - 2.03 (m, 6H), 1.91 (t, *J =* 10.2 Hz, 2H).

### Example 214D: 2-(4-chloro-3-fluorophenoxy)-N-(4-{[(4-methoxyphenyl)methyl]amino}-2-oxobicyclo[2.2.2]octan-1-yl)acetamide

The title compound was prepared using the methodologies described in Example 198F substituting 214C for 198E. ¹H NMR (400 MHz, DMSO-*d*₆) *δ* ppm 7.64 (s, 1H), 7.46 (t, J = 8.9 Hz, 1H), 7.24 - 7.16 (m, 2H), 7.05 (dd, J = 11.4, 2.8 Hz, 1H), 6.86 - 6.78 (m, 3H), 4.52 (s, 2H), 3.68 (s, 3H), 3.55 (s, 2H), 2.43 (s, 2H), 2.43 - 2.32 (m, 2H), 1.82 - 1.65 (m, 4H); MS (ESI⁺) *m*/*z* 460.9 (M+H)⁺.

### Example 214E: N-(4-amino-2-oxobicyclo[2.2.2]octan-1-yl)-2-(4-chloro-3-fluorophenoxy)acetamide

A mixture of Example 214D (1.24 g, 2.69 mmol), ceric ammonium nitrate (6.64 g, 12.11 mmol) and 2,2,2-trifluoroacetic acid (15.0 mL, 195 mmol) in water (15.0 mL) and acetonitrile (2.0 mL) was stirred at 0 °C for 1 hour and then warmed up to room temperature and stirred for another 3 hours. The reaction mixture was diluted with water and washed with ethyl acetate. The aqueous phase was partitioned between ammonium hydroxide and ethyl acetate. The organic layer was dried over magnesium sulfate and filtered. The filtrate was concentrated, and the residue was purified by HPLC (10-80% acetonitrile in 0.1% trifluoroacetic acid/water at 25 mL/minute on a Phenomenex^{®} C18 5 µm column (250 mm × 21.2 mm)) to give 1.14 g of the titled compound as a white solid. ¹H NMR (400 MHz, DMSO-*d*₆) *δ* ppm 8.24 (s, 3H), 7.76 (s, 1H), 7.45 (t, J = 8.9 Hz, 1H), 7.03 (dd, J = 11.3, 2.9 Hz, 1H), 6.81 (ddd, J = 9.0, 2.9, 1.2 Hz, 1H), 4.54 (s, 2H), 2.65 (s, 2H), 2.35 (dd, J = 13.4, 9.8 Hz, 2H), 2.04 - 1.78 (m, 6H); MS (ESI⁺) *m*/*z* 341.2 (M+H)⁺.

### Example 214F: N-(4-amino-2-hydroxybicyclo[2.2.2]octan-1-yl)-2-(4-chloro-3-fluorophenoxy)acetamide, trifluoroacetate

The title compound was prepared using the methodologies described in Example 198I substituting 214E for 198H. ¹H NMR (400 MHz, DMSO-*d*₆) *δ* ppm 7.91 (s, 3H), 7.48 (t, J = 8.9 Hz, 1H), 7.38 (s, 1H), 7.05 (dd, J = 11.4, 2.9 Hz, 1H), 6.87 - 6.74 (m, 1H), 4.48 (s, 2H), 4.14 (dd, J = 9.7, 2.6 Hz, 1H), 2.21 - 1.52 (m, 10H); MS (ESI⁺) *m*/*z* 343.1 (M+H)⁺.

### Example 214G: 2-(4-chloro-3-fluorophenoxy)-N-[2-hydroxy-4-(2-{[6-(trifluoromethyl)pyridin-3-yl]oxy}acetamido)bicyclo[2.2.2]octan-1-yl]acetamide

To a mixture of Example 214F (0.055 g, 0.121 mmol), 2-((6-(trifluoromethyl)pyridin-3-yl)oxy)acetic acid (0.031 g, 0.139 mmol) and *N*-ethyl-*N*-isopropylpropan-2-amine (0.053 mL, 0.302 mmol) in N,N-dimethylformamide (1.5 mL), 2-(3H-[1,2,3]triazolo[4,5-b]pyridin-3-yl)-1,1,3,3-tetramethylisouronium hexafluorophosphate(V) (0.069 g, 0.181 mmol) was added. The mixture was stirred at ambient temperature for 1 hour. Volatiles were removed, and the residue was purified by HPLC (10-95% acetonitrile in 0.1% trifluoroacetic acid/water at 25 mL/minute on a Phenomenex^{®} C18 5 µm column (250 mm × 21.2 mm)) to give 36 mg of the title compound as a white solid. ¹H NMR (400 MHz, DMSO-*d*₆) *δ* ppm 8.39 (d, J = 2.8 Hz, 1H), 7.82 (d, J = 8.8 Hz, 1H), 7.61 (s, 1H), 7.51 - 7.40 (m, 2H), 7.22 (s, 1H), 7.02 (dd, J = 11.4, 2.9 Hz, 1H), 6.79 (ddd, J = 8.9, 2.8, 1.2 Hz, 1H), 5.04 (s, 1H), 4.58 (s, 2H), 4.43 (s, 2H), 4.01 (d, J = 9.0 Hz, 1H), 2.24 (ddd, J = 12.1, 9.3, 2.1 Hz, 1H), 2.03 (ddd, J = 12.4, 10.6, 4.6 Hz, 1H), 1.91 - 1.69 (m, 8H); MS (ESI⁺) *m*/*z* 546.1 (M+H)⁺.

### Example 215: 2-(4-chloro-3-fluorophenoxy)-N-(2-hydroxy-4-{[(4-methoxyphenyl)methyl]amino}bicyclo[2.2.2]octan-1-yl)acetamide (Compound 314)

A mixture of Example 214D (0.042 g, 0.091 mmol) and sodium borohydride (0.017 g, 0.456 mmol) in a mixture of methanol/dichloromethane was stirred for 1 hour. Volatiles were removed, and the residue was purified by HPLC (10-85% acetonitrile in 0.1% trifluoroacetic acid/water at 25 mL/minute on a Phenomenex^{®} C18 5 µm column (250 mm × 21.2 mm)) to give 39 mg of the title compound as a white solid. ¹H NMR (400 MHz, DMSO-*d*₆) *δ* ppm 8.63 (s, 2H), 7.50 - 7.32 (m, 4H), 7.07 6.92 (m, 3H), 6.80 (ddd, J = 9.0, 2.9, 1.2 Hz, 1H), 5.32 (d, J = 4.3 Hz, 1H), 4.45 (s, 2H), 4.20 - 4.11 (m, 1H), 3.95 (brs, 2H), 3.73 (s, 3H), 2.30 - 1.64 (m, 10H); MS (ESI⁺) *m*/*z* 463.0 (M+H)⁺.

### Example 216: (2R)-N-{3-[2-(4-chloro-3-fluorophenoxy)acetamido]bicyclo[1.1.1]pentan-1-yl}-2-(4-chlorophenyl)-2-hydroxyacetamide (Compound 315)

The title compound was prepared using the methodologies described above. ¹H NMR (400 MHz, CDCl₃) *δ* ppm 7.34 (s, 4H), 7.30 (t, J = 8.5 Hz, 1H), 6.87 (s, 1H), 6.84 (s, 1H), 6.74 (dd, J = 10.2, 2.9 Hz, 1H), 6.65 (ddd, J = 8.9, 2.9, 1.3 Hz, 1H), 4.98 (d, J = 3.6 Hz, 1H), 4.35 (s, 2H), 3.74 (t, J = 3.4 Hz, 1H), 2.44 (s, 6H); MS (ESI⁺) *m*/*z* 453 (M+H)⁺.

### Example 217: 2-(4-chloro-3-fluorophenoxy)-N-[3-oxo-4-(2-{[3-oxo-2-(2,2,2-trifluoroethyl)-2,3-dihydropyridazin-4-yl]oxy}acetamido)bicyclo[2.2.2]octan-1-yl]acetamide (Compound 316)

A mixture of Example 198H (0.090 g, 0.239 mmol), Example 210D (0.066 g, 0.262 mmol), *N*-[(dimethylamino)-1H-1,2,3-triazolo-[4,5-p]pyridin-1-ylmethylene]-N-methylmethanaminium hexafluorophosphate N-oxide (HATU, 0.109 g, 0.286 mmol), and triethylamine (0.133 mL, 0.954 mmol) in tetrahydrofuran (4 mL) was stirred for 16 hours. The reaction mixture was treated with saturated, aqueous NaHCO₃ and brine and extracted with ethyl acetate (2×). The combined organic layers were dried over anhydrous MgSO₄, filtered, and concentrated under reduced pressure. The residue was purified on a 12 g silica gel column using a Biotage^{®} Isolera^{™} One flash system eluting with 100% ethyl acetate to provide the title compound (0.10 g, 0.17 mmol, 74% yield). ¹H NMR (500 MHz, DMSO-*d*₆) *δ* ppm 7.95 -7.85 (m, 2H), 7.82 (s, 1H), 7.47 (t, J = 8.9 Hz, 1H), 7.02 (dd, J = 11.4, 2.9 Hz, 1H), 6.80 (ddd, J = 9.1, 2.9, 1.2 Hz, 1H), 6.73 (d, J = 5.0 Hz, 1H), 4.93 (q, J = 9.0 Hz, 2H), 4.64 (s, 2H), 4.47 (s, 2H), 2.85 (s, 2H), 2.47 - 2.35 (m, 2H), 2.13 - 1.92 (m, 4H), 1.83 (dt, J = 13.5, 6.8 Hz, 2H); MS (ESI⁺) *m*/*z* 575.0 (M+H)⁺.

### Example 218: 2-(4-chloro-3-fluorophenoxy)-N-[3-oxo-4-(2-{[6-oxo-1-(2,2,2-trifluoroethyl)-1,6-dihydropyridazin-4-yl]oxy}acetamido)bicyclo[2.2.2]octan-1-yl]acetamide (Compound 317)

The reaction described in Example 217 substituting Example 211B for Example 210D gave the title compound. ¹H NMR (500 MHz, DMSO-*d*₆) *δ* ppm 7.95 - 7.87 (m, 2H), 7.82 (s, 1H), 7.47 (t, J = 8.9 Hz, 1H), 7.02 (dd, J = 11.4, 2.8 Hz, 1H), 6.80 (ddd, J = 9.0, 2.9, 1.2 Hz, 1H), 6.28 (d, J = 2.8 Hz, 1H), 4.85 (q, J = 9.1 Hz, 2H), 4.65 (s, 2H), 4.47 (s, 2H), 2.83 (d, J = 1.4 Hz, 2H), 2.37 - 2.22 (m, 2H), 2.11 - 1.94 (m, 4H), 1.90 (dd, J = 11.7, 4.3 Hz, 2H); MS (ESI⁺) *m*/*z* 575.4 (M+H)⁺.

### Example 219: 2-(4-chloro-3-fluorophenoxy)-N-[(3R)-3-hydroxy-4-(2-{[2-(trifluoromethyl)pyridin-4-yl]oxy}acetamido)bicyclo[2.2.2]octan-1-yl]acetamide (Compound 318)

The title compound was isolated by chiral preparative HPLC of Example 209 as the first peak eluted off the column. The gradient was 25-36% B in 19 minute then step to and hold at 50% B for 5 minute (20 mL/minute flow rate). Mobile phase B was HPLC grade ethanol and mobile phase A was HPLC grade heptane with 0. 2% diethylamine added. The chromatography used a Daicel Chiralpak^{®} IC column, 21 × 250 mm column (5 µm particles). ¹H NMR (400 MHz, DMSO-*d*₆) *δ* ppm 8.53 (d, J = 5.7 Hz, 1H), 7.50 - 7.39 (m, 2H), 7.38 (s, 2H), 7.19 (dd, J = 5.8, 2.5 Hz, 1H), 6.98 (dd, J = 11.4, 2.8 Hz, 1H), 6.81 - 6.73 (m, 1H), 4.64 (s, 2H), 4.39 (s, 2H), 4.05 (dd, J = 9.5, 3.0 Hz, 1H), 2.23 (dd, J = 13.4, 9.5 Hz, 1H), 2.07 - 1.86 (m, 4H), 1.75 (tdd, J = 11.6, 7.3, 2.9 Hz, 5H); MS (ESI⁺) *m*/*z* 546.1 (M+H)⁺. X-ray crystallography confirmed the assigned stereochemistry.

### Example 220: 2-(4-chloro-3-fluorophenoxy)-N-[(3S)-3-hydroxy-4-(2-{[2-(trifluoromethyl)pyridin-4-yl]oxy}acetamido)bicyclo[2.2.2]octan-1-yl]acetamide (Compound 319)

The title compound was isolated by chiral preparative HPLC of Example 209 as the second peak eluted off the column using methodologies described in Example 219. ¹H NMR (400 MHz, DMSO-*d*₆) *δ* ppm 8.53 (d, J = 5.7 Hz, 1H), 7.50 - 7.35 (m, 4H), 7.19 (dd, J = 5.8, 2.5 Hz, 1H), 6.98 (dd, J = 11.4, 2.8 Hz, 1H), 6.77 (dd, J = 9.1, 2.6 Hz, 1H), 4.64 (s, 2H), 4.39 (s, 2H), 4.05 (dd, J = 9.6, 3.0 Hz, 1H), 2.22 (dd, J = 13.2, 9.5 Hz, 1H), 2.01 - 1.66 (m, 9H); MS (ESI⁺) *m*/*z* 546.1 (M+H)⁺.

### Example 221: 2-(4-chloro-3-fluorophenoxy)-N-(4-{2-[4-(dimethylamino)phenyl]acetamido}-3-hydroxybicyclo[2.2.2]octan-1-yl)acetamide (Compound 320)

A 4 mL vial was charged with a stir bar, a 500 µL solution of Example 198I (47.74 mg, 0.13 mmol) in N,N-dimethylacetamide, a 395.7 µL of a 0.35 mmol pre-weighed vial with a solution of 2-(4-(dimethylamino)phenyl)acetic acid (25.2 mg, 0.14 mmol) in 1000 µL of *N,N-*dimethylacetamide, a 500 µL solution of 2-(3H-[1,2,3]triazolo[4,5-b]pyridin-3-yl)-1,1,3,3-tetramethylisouronium hexafluorophosphate(V) (57.4 mg, 0.15 mmol) in *N,N-*dimethylacetamide, and triethylamine (53.01 µL, 0.38 mmol). This was capped and placed to stir at room temperature for 1 hour. Upon completion the mixture was concentrated to dryness, and the residue was purified by reverse phase HPLC (Phenomenex^{®} Luna^{®} C8(2) 5 µm 100Å AXIA^{™} column (50 mm × 30 mm). A gradient of acetonitrile (A) and 0.1% CF₃CO₂H in H₂O (B) was used at a flow rate of 40 mL/minute (0-0.5 minute 5% A, 0.5-6.5 minutes linear gradient 5-100% A, 6.5-8.5 minutes 100% A, 8.5-9.0 minutes linear gradient 100-5% A, 9.0-10.0 minutes 5% A). Detection methods are diode array (DAD) under positive APCI ionization conditions.) to yield the title compound. ¹H NMR (400 MHz, DMSO-*d*₆) *δ* ppm 7.47 (td, *J =* 8.9, 1.1 Hz, 1H), 7.43 - 7.32 (m, 3H), 7.36 - 7.26 (m, 1H), 7.00 (dd, *J* = 11.4, 2.9 Hz, 1H), 6.81 (ddd, *J =* 9.0, 3.0, 1.3 Hz, 1H), 4.43 (d, *J* = 6.5 Hz, 2H), 4.06 (dd, *J* = 8.6, 1.9 Hz, 1H), 3.50 - 3.34 (m, 2H), 3.10 (d, *J* = 5.9 Hz, 6H), 2.26 (ddd, *J* = 12.4, 9.2, 2.6 Hz, 1H), 2.01 - 1.71 (m, 9H); MS (ESI⁺) *m*/*z* 504.1 (M+H)⁺.

### Example 222: 2-(4-chloro-3-fluorophenoxy)-N-(3-hydroxy-4-{2-[3-(trifluoromethyl)phenyl]acetamido }bicyclo[2.2.2]octan-1-yl)acetamide (Compound 321)

The title compound was prepared using the methodologies described in Example 221 substituting 2-(3-(trifluoromethyl)phenyl)acetic acid for 2-(4-(dimethylamino)phenyl)acetic acid. ¹H NMR (400 MHz, DMSO-*d*₆) *δ* ppm 7.64 - 7.51 (m, 4H), 7.47 (t, *J =* 8.9 Hz, 1H), 7.00 (dd, *J* = 11.4, 2.8 Hz, 1H), 6.81 (ddd, *J =* 9.0, 2.9, 1.2 Hz, 1H), 4.42 (s, 2H), 4.13 - 4.05 (m, 1H), 3.52 (d, *J* = 4.0 Hz, 2H), 2.26 (ddd, *J =* 13.2, 9.3, 2.7 Hz, 1H), 2.04 - 1.87 (m, 3H), 1.88 - 1.74 (m, 2H), 1.74 (s, 3H), 1.76 - 1.64 (m, 3H); MS (ESI⁺) *m*/*z* 529.1 (M+H)⁺.

### Example 223: 2-(4-chloro-3-fluorophenoxy)-N-(3-hydroxy-4-{2-[4-(trifluoromethyl)phenyl]acetamido }bicyclo[2.2.2]octan-1-yl)acetamide (Compound 322)

The title compound was prepared using the methodologies described in Example 221 substituting 2-(4-(trifluoromethyl)phenyl)acetic acid for 2-(4-(dimethylamino)phenyl)acetic acid. ¹H NMR (400 MHz, DMSO-*d*₆) *δ* ppm 7.69 - 7.54 (m, 2H), 7.52 - 7.41 (m, 3H), 7.00 (dd, *J* = 11.4, 2.9 Hz, 1H), 6.81 (ddd, *J* = 9.0, 2.9, 1.2 Hz, 1H), 4.42 (s, 2H), 4.09 (d, *J =* 8.4 Hz, 1H), 3.55 - 3.41 (m, 2H), 2.26 (ddd, *J =* 12.6, 9.2, 2.6 Hz, 1H), 2.08 - 1.89 (m, 2H), 1.94 - 1.81 (m, 1H), 1.84 - 1.76 (m, 1H), 1.80 - 1.64 (m, 5H); MS (ESI⁺) *m*/*z* 529.1 (M+H)⁺.

### Example 224: 2-(2H-1,3-benzodioxol-5-yl)-N-{4-[2-(4-chloro-3-fluorophenoxy)acetamido]-2-hydroxybicyclo[2.2.2]octan-1-yl}acetamide (Compound 323)

The title compound was prepared using the methodologies described in Example 221 substituting 2-(benzo[d][1,3]dioxol-5-yl)acetic acid for 2-(4-(dimethylamino)phenyl)acetic acid. ¹H NMR (400 MHz, DMSO-*d*₆) *δ* ppm 7.47 (t, *J =* 8.9 Hz, 1H), 7.00 (dd, *J =* 11.4, 2.9 Hz, 1H), 6.85 - 6.77 (m, 3H), 6.70 (dd, *J* = 7.9*,* 1.8 Hz, 1H), 5.96 (s, 2H), 4.42 (s, 2H), 4.07 - 4.00 (m, 1H), 3.37 - 3.21 (m, 2H), 2.26 (ddd, *J* = 13.2, 9.1, 2.5 Hz, 1H), 2.03 - 1.88 (m, 2H), 1.87 - 1.70 (m, 7H); MS (ESI⁺) *m*/*z* 505.1 (M+H)⁺.

### Example 225: 2-(4-chloro-3-fluorophenoxy)-N-{3-hydroxy-4-[2-(pyridin-3-yl)acetamido]bicyclo[2.2.2]octan-1-yl}acetamide (Compound 324)

The title compound was prepared using the methodologies described in Example 221 substituting 2-(pyridin-3-yl)acetic acid for 2-(4-(dimethylamino)phenyl)acetic acid. ¹H NMR (400 MHz, DMSO-*d*₆) *δ* ppm 8.78 - 8.66 (m, 2H), 8.39 (dt, *J =* 8.1, 1.7 Hz, 1H), 8.01 - 7.91 (m, 1H), 7.47 (td, *J =* 8.9, 1.4 Hz, 1H), 7.04 - 6.96 (m, 1H), 6.81 (ddd, *J* = 9.0, 2.9, 1.2 Hz, 1H), 4.43 (d, *J =* 7.0 Hz, 2H), 4.15 (d, *J =* 8.4 Hz, 1H), 3.70 (s, 2H), 2.27 (ddd, *J =* 12.8, 9.4, 2.8 Hz, 1H), 2.04 (d, *J =* 16.5 Hz, 1H), 2.01 - 1.76 (m, 6H), 1.71 (ddt, *J =* 15.4, 12.0, 6.1 Hz, 2H); MS (ESI⁺) *m*/*z* 462.1 (M+H)⁺.

### Example 226: 2-(4-chloro-3-fluorophenoxy)-N-(3-hydroxy-4-{2-[4-(methanesulfonyl)phenyl]acetamido}bicyclo[2.2.2]octan-1-yl)acetamide (Compound 325)

The title compound was prepared using the methodologies described in Example 221 substituting 2-(4-(methanesulfonyl)phenyl)acetic acid for 2-(4-(dimethylamino)phenyl)acetic acid. ¹H NMR (400 MHz, DMSO-*d*₆) *δ* ppm 7.89 - 7.78 (m, 2H), 7.59 - 7.42 (m, 3H), 7.00 (dd, *J =* 11.4, 2.9 Hz, 1H), 6.81 (ddd, *J =* 8.9, 2.9, 1.2 Hz, 1H), 4.42 (s, 2H), 4.10 (dt, *J =* 9.0, 2.2 Hz, 1H), 3.57 - 3.48 (m, 2H), 3.18 (s, 3H), 2.26 (ddd, *J =* 13.1, 9.3, 2.6 Hz, 1H), 2.04 - 1.78 (m, 4H), 1.81 - 1.64 (m, 5H); MS (ESI⁺) *m*/*z* 539.1 (M+H)⁺.

### Example 227: 2-(4-chloro-3-fluorophenoxy)-N-(3-hydroxy-4-{2-[4-(trifluoromethoxy)phenyl]acetamido}bicyclo[2.2.2]octan-1-yl)acetamide (Compound 326)

The title compound was prepared using the methodologies described in Example 221 substituting 2-(4-(trifluoromethoxy)phenyl)acetic acid for 2-(4-(dimethylamino)phenyl)acetic acid. ¹H NMR (400 MHz, DMSO-*d*₆) *δ* ppm 7.47 (t, *J =* 8.9 Hz, 1H), 7.40 - 7.33 (m, 2H), 7.37 - 7.24 (m, 2H), 7.00 (dd, *J =* 11.4, 2.9 Hz, 1H), 6.81 (ddd, *J =* 9.0, 2.9, 1.2 Hz, 1H), 4.42 (s, 2H), 4.08 (dt, *J =* 8.8, 2.1 Hz, 1H), 3.51 - 3.38 (m, 2H), 2.26 (ddd, *J =* 13.1, 9.2, 2.7 Hz, 1H), 2.08 - 1.89 (m, 2H), 1.93 - 1.73 (m, 2H), 1.76 - 1.64 (m, 5H); MS (ESI⁺) *m*/*z* 545.1 (M+H)⁺.

### Example 228: 2-(4-chloro-3-fluorophenoxy)-N-(3-hydroxy-4-{2-[3-(trifluoromethoxy)phenyl]acetamido}bicyclo[2.2.2]octan-1-yl)acetamide (Compound 327)

The title compound was prepared using the methodologies described in Example 221 substituting 2-(3-(trifluoromethoxy)phenyl)acetic acid for 2-(4-(dimethylamino)phenyl)acetic acid. ¹H NMR (400 MHz, DMSO-*d*₆) *δ* ppm 7.58 (d, *J =* 12.2 Hz, 1H), 7.52 - 7.38 (m, 2H), 7.31 - 7.17 (m, 3H), 7.00 (dd, *J* = 11.4, 2.8 Hz, 1H), 6.81 (ddd, *J* = 9.0, 2.9, 1.2 Hz, 1H), 4.42 (s, 2H), 4.08 (d, *J* = 8.7 Hz, 1H), 3.54 - 3.40 (m, 2H), 2.26 (ddd, *J =* 12.4, 9.2, 2.7 Hz, 1H), 2.06 - 1.66 (m, 9H); MS (ESI⁺) *m*/*z* 545.1 (M+H)⁺.

### Example 229: 2-(4-chloro-3-fluorophenoxy)-N-{3-hydroxy-4-[2-(pyridin-4-yl)acetamido]bicyclo[2.2.2]octan-1-yl}acetamide (Compound 328)

The title compound was prepared using the methodologies described in Example 221 substituting 2-(pyridin-4-yl)acetic acid for 2-(4-(dimethylamino)phenyl)acetic acid. ¹H NMR (400 MHz, DMSO-*d*₆) *δ* ppm 8.76 (d, *J =* 6.0 Hz, 2H), 7.92 - 7.85 (m, 2H), 7.47 (t, *J =* 8.9 Hz, 1H), 7.00 (dd, *J* = 11.4, 2.9 Hz, 1H), 6.81 (ddd, *J =* 8.9, 2.9, 1.2 Hz, 1H), 4.42 (s, 2H), 4.15 (d, *J* = 8.7 Hz, 1H), 3.77 (s, 2H), 2.28 (ddd, *J* = 12.5, 9.4, 2.7 Hz, 1H), 1.97 (q, *J =* 10.9, 10.5 Hz, 2H), 1.81 (td, *J* = 19.2, 16.3, 11.6 Hz, 5H), 1.77 - 1.65 (m, 2H); MS (ESI⁺) *m*/*z* 462.1 (M+H)⁺.

### Example 230: 2-(4-chloro-3-fluorophenoxy)-N-{4-[2-(3,4-difluorophenoxy)acetamido]-2-hydroxybicyclo[2.2.2]octan-1-yl}acetamide (Compound 329)

The title compound was prepared using the methodologies described in Example 214 substituting 2-(3,4-difluorophenoxy)acetic acid for 2-((6-(trifluoromethyl)pyridin-3-yl)oxy)acetic acid. ¹H NMR (400 MHz, DMSO-*d*₆) *δ* ppm 7.39 (m, 2H), 7.31 (dt, J = 10.5, 9.3 Hz, 1H), 7.23 (s, 1H), 7.05 - 6.94 (m, 2H), 6.83 - 6.66 (m, 2H), 5.06 (d, J = 4.4 Hz, 1H), 4.42 (s, 2H), 4.35 (s, 2H), 4.06 (m, 1H), 2.23 (ddd, J = 12.4, 9.6, 2.2 Hz, 1H), 2.08 1.96 (m, 1H), 1.89 (d, J = 10.9 Hz, 2H), 1.87 - 1.68 (m, 6H); MS (ESI⁺) *m*/*z* 5131 (M+H)⁺.

### Example 231: 2-(4-chloro-3-fluorophenoxy)-N-{4-[2-(4-fluorophenoxy)acetamido]-2-hydroxybicyclo[2.2.2]octan-1-yl}acetamide (Compound 330)

The title compound was prepared using the methodologies described in Example 214 substituting 2-(4-fluorophenoxy)acetic acid for 2-((6-(trifluoromethyl)pyridin-3-yl)oxy)acetic acid. ¹H NMR (400 MHz, DMSO-*d₆*) *δ* ppm 7.49 - 7.36 (m, 2H), 7.23 (s, 1H), 7.14 - 6.97 (m, 3H), 6.93 - 6.85 (m, 2H), 6.79 (ddd, J = 9.0, 2.9, 1.2 Hz, 1H), 5.04 (d, J = 4.4 Hz, 1H), 4.43 (s, 2H), 4.32 (s, 2H), 4.10 - 3.95 (m, 1H), 2.24 (ddd, J = 12.1, 9.4, 2.2 Hz, 1H), 2.09 - 1.97 (m, 1H), 1.93 - 1.69 (m, 8H); MS (ESI⁺) *m*/*z* 495.0 (M+H)⁺.

### Example 232: 2-(4-chloro-3-fluorophenoxy)-N-(4-{[2-(4-fluorophenoxy)ethyl]amino}-3-hydroxybicyclo[2.2.2]octan-1-yl)acetamide (Compound 331)

To a mixture of Example 198I (0.05 g, 0.146 mmol) and 2-(4-fluorophenoxy)acetaldehyde (0.027 g, 0.175 mmol) in buffer (1.5 mL, pH = 4)), sodium cyanoborohydride (0.014 g, 0.219 mmol) was added, and the mixture was stirred at ambient temperature for 16 hours. Solvent was removed under high vacuum, and the residue was purified by HPLC (10-95% acetonitrile in 0.1% trifluoroacetic acid/water at 25 mL/minute on a Phenomenex^{®} C18 5 µm column (250 mm × 21.2 mm)) to give 37 mg of the title compound as a white solid. ¹H NMR (501 MHz, DMSO-*d*₆) *δ* ppm 8.45 (m, 2H), 7.68 (s, 1H), 7.46 (t, J = 8.9 Hz, 1H), 7.14 (t, J = 8.8 Hz, 2H), 7.03 - 6.93 (m, 3H), 6.79 (dd, J = 9.0, 2.7 Hz, 1H), 5.74 (s, 1H), 4.44 (s, 2H), 4.16 (s, 1H), 4.08 - 4.02 (m, 1H), 3.26 (tt, J = 8.7, 5.0 Hz, 2H), 2.34 (td, J = 9.8, 4.8 Hz, 1H), 2.12 - 1.94 (m, 2H), 1.89 - 1.78 (m, 5H), 1.80 - 1.66 (m, 2H); MS (ESI⁺) *m*/*z* 481.2 (M+H)⁺.

### Example 233: 2-(4-chloro-3-fluorophenoxy)-N-[3-(2-{[6-(pentafluoro-λ⁶-sulfanyl)pyridin-3-yl]oxy}acetamido)bicyclo[1.1.1]pentan-1-yl]acetamide (Compound 332)

### Example 233A: [6-(pentafluoro-λ⁶-sulfanyl)pyridin-3-yl]boronic acid

To a stirred solution of 5-bromo-2-(pentafluoro-λ⁶-sulfanyl)pyridine (500 mg, 1.67 mmol) and triisopropyl borate (157 mg, 8.36 mmol) in tetrahydrofuran (20 mL) in a perfluoroalkoxy (PFA) tube was added 2.5 M *n*-butyllithium (1.0 mL, 2.51 mmol) dropwise at -78 °C under N₂. The mixture was stirred for 30 minutes at -78 °C. The mixture was quenched with saturated aqueous NH₄Cl solution at -78 °C. The mixture was extracted with ethyl acetate (2×). The combined organic layers were washed with brine (100 mL), dried over anhydrous Na₂SO₄, and concentrated under reduced pressure. The residue was treated with dichloromethane and the resultant solids were collected by filtration. The filter cake was dried under high vacuum to provide the title compound (272 mg, 1.09 mmol, 65% yield). MS (ESI⁺) *m*/*z* 250 (M+H)⁺.

### Example 233B: 6-(pentafluoro-λ⁶-sulfanyl)pyridin-3-ol

To a stirred solution of Example 233A (1.2 g, 4.58 mmol) and triethylamine (6.38 mL, 45.8 mmol) in ethanol (100 mL) and water (10 mL, 555 mmol) was added iodosobenzene diacetate (7.37 g, 22.89 mmol) at 20 °C, and the mixture was allowed to stir for 12 hours at 20 °C. The mixture was concentrated under reduced pressure. The residue was diluted with water (250 mL) and extracted with dichloromethane (3 × 100 mL). The combined organic layers were concentrated under reduced pressure, and the residue was purified by preparative HPLC performed on a Phenomenex^{®} Luna^{®} C18 column (500 × 50 mm, 10 µm particle size) using a gradient of 25% to 55% acetonitrile/0.09% aqueous trifluoroacetic acid over 20 minutes at a flow rate of 80 mL/minute. The desired HPLC fractions were extracted with dichloromethane (3 × 100 mL), and the combined organic layers were concentrated under reduced pressure to provide the title compound (820 mg, 3.7 mmol, yield, 80% yield). MS (ESI⁺) *m*/*z* 222 (M+H)⁺.

### Example 233C: 2-(4-chloro-3-fluorophenoxy)-N-{3-(2-{[6-(pentafluoro-λ⁶-sulfanyl)pyridin-3-yl]oxy}acetamido)bicyclo[1.1.1]pentan-1-yl)acetamide

A mixture of the product of Example 28A (80.0 mg, 0.221 mmol), Example 233B (73.5 mg, 0.332 mmol), KI (1.84 mg, 0.011 mmol), and potassium carbonate (61.2 mg, 0.44 mmol) in acetone (3 mL) was heated at 140 °C in a Biotage^{®} Initiator microwave reactor for 30 minutes. The reaction mixture was filtered. The filtrate was concentrated, and the residue was purified by reverse-phase HPLC (see protocol in Example 112D) to provide the title compound (59.6 mg, 0.11 mmol, 49% yield). ¹H NMR (500 MHz, DMSO-*d₆*) *δ* ppm 8.80 (s, 1H), 8.70 (s, 1H), 8.29 (d, J = 3.0 Hz, 1H), 7.97 (d, J = 9.1 Hz, 1H), 7.63 (dd, J = 9.2, 2.9 Hz, 1H), 7.47 (t, J = 8.9 Hz, 1H), 7.05 (dd, J = 11.3, 2.8 Hz, 1H), 6.83 (ddd, J = 9.0, 2.9, 1.2 Hz, 1H), 4.68 (s, 2H), 4.46 (s, 2H), 2.25 (s, 6H); MS (ESI⁺) *m*/*z* 546.0 (M+H)⁺.

### Example 234: 2-(4-chloro-3-fluorophenoxy)-N-[3-hydroxy-4-(2-{[3-oxo-2-(2,2,2-trifluoroethyl)-2,3-dihydropyridazin-4-yl]oxy}acetamido)bicyclo[2.2.2]octan-1-yl]acetamide (Compound 333)

To a solution of Example 217 (88.0 mg, 0.153 mmol) in CH₂Cl₂ (1.5 mL) and methanol (1.5 mL) was added sodium borohydride (6.95 mg, 0.184 mmol). The reaction mixture was stirred for 1.5 hours. The solution was treated with brine and saturated aqueous NaHCO₃ and extracted with CH₂Cl₂ (2×). The combined organic fractions were concentrated under reduced pressure, and the residue was purified by reverse-phase HPLC (see protocol in Example 112D) to provide the title compound (54.5 mg, 0.094 mmol, 62% yield). ¹H NMR (400 MHz, DMSO-*d₆) δ* ppm 7.87 (d, J = 4.9 Hz, 1H), 7.57 - 7.42 (m, 2H), 7.39 (s, 1H), 7.02 (dd, J = 11.4, 2.8 Hz, 1H), 6.81 (ddd, J = 8.9, 2.9, 1.2 Hz, 1H), 6.71 (d, J = 5.0 Hz, 1H), 4.94 (q, J = 9.1 Hz, 2H), 4.56 (s, 2H), 4.43 (s, 2H), 4.08 (dd, J = 9.6, 3.0 Hz, 1H), 2.33 - 2.18 (m, 1H). 2.05 - 1.69 (m, 10H); MS (ESI⁺) *m*/*z* 577.0 (M+H)⁺.

### Example 235: 2-(4-chloro-3-fluorophenoxy)-N-[3-hydroxy-4-(2-{[6-oxo-1-(2,2,2-trifluoroethyl)-1,6-dihydropyridazin-4-yl]oxy}acetamido)bicyclo[2.2.2]octan-1-yl]acetamide (Compound 334)

The reaction described in Example 234 substituting Example 218 for Example 217 gave the title compound. ¹H NMR (400 MHz, DMSO-*d₆*) *δ* ppm 7.93 (d, J = 2.8 Hz, 1H), 7.54 - 7.44 (m, 3H), 7.41 (s, 1H), 7.02 (dd, J = 11.4, 2.9 Hz, 1H), 6.81 (ddd, J = 9.0, 2.9, 1.2 Hz, 1H), 6.29 (d, J = 2.8 Hz, 1H), 4.87 (q, J = 9.1 Hz, 2H), 4.56 (s, 2H), 4.43 (s, 2H), 4.11 (dd, J = 9.5, 2.9 Hz, 1H), 2.26 (ddd, J = 12.4, 9.5, 2.3 Hz, 1H), 2.04 - 1.68 (m, 10H); MS (ESI⁺) *m*/*z* 577.1 (M+H)⁺.

### Example 236: 2-(4-chloro-3-fluorophenoxy)-N-(4-{[2-(3,4-dichlorophenoxy)ethyl]amino}-3-hydroxybicyclo[2.2.2]octan-1-yl)acetamide (Compound 335)

The title compound was prepared using the methodologies described in Example 232 substituting 2-(3,4-dichlorophenoxy)acetaldehyde for 2-(4-fluorophenoxy)acetaldehyde. ¹H NMR (400 MHz, DMSO-*d*₆) *δ* ppm 8.53 (s, 1H), 8.47 (s, 1H), 7.67 (s, 1H), 7.54 (d, J = 8.9 Hz, 1H), 7.45 (t, J = 8.9 Hz, 1H), 7.24 (d, J = 2.9 Hz, 1H), 6.98 (dq, J = 8.9, 3.0 Hz, 2H), 6.77 (ddd, J = 9.0, 2.9, 1.2 Hz, 1H), 5.73 (d, J = 4.7 Hz, 1H), 4.42 (s, 2H), 4.21 (t, J = 5.1 Hz, 2H), 4.03 (d, J = 9.3 Hz, 1H), 3.24 (m, 2H), 2.33 (ddd, J = 13.0, 9.6, 2.9 Hz, 1H), 2.11 - 1.91 (m, 2H), 1.89 - 1.73 (m, 6H), 1.77 - 1.63 (m, 1H); MS (ESI⁺) *m*/*z* 531.2 (M+H)⁺.

### Example 237: 2-(4-chloro-3-fluorophenoxy)-N-(4-{[2-(4-chlorophenoxy)ethyl]amino}-3-hydroxybicyclo[2.2.2]octan-1-yl)acetamide (Compound 336)

The title compound was prepared using the methodologies described in Example 232 substituting 2-(4-chlorophenoxy)acetaldehyde for 2-(4-fluorophenoxy)acetaldehyde. ¹H NMR (400 MHz, DMSO-*d₆*) *δ* ppm 8.62 - 8.47 (m, 2H), 7.70 (s, 1H), 7.48 (t, *J =* 8.9 Hz, 1H), 7.38 (d, *J =* 6.7 Hz, 1H), 7.39 - 7.25 (m, 1H), 7.07 - 6.89 (m, 3H), 6.81 (dt, *J =* 8.9, 2.0 Hz, 1H), 5.76 (s, 1H), 4.46 (s, 2H), 4.20 (t, *J =* 5.2 Hz, 2H), 4.07 (d, *J =* 8.9 Hz, 1H), 3.32 - 3.25 (m, 2H), 2.36 (ddd, *J =* 12.8, 9.4, 2.8 Hz, 1H), 2.09 (td, *J =* 11.7, 9.9, 5.0 Hz, 1H), 2.06 - 1.94 (m, 1H), 1.95 - 1.83 (m, 4H), 1.81 (dd, *J =* 14.5, 6.2 Hz, 2H), 1.80 - 1.67 (m, 1H); MS (ESI⁺) *m*/*z* 597.2 (M+H)⁺.

### Example 238: 2-(4-chloro-3-fluorophenoxy)-N-(4-{[2-(3,4-dichlorophenoxy)ethyl]amino}-2-hydroxybicyclo[2.2.2]octan-1-yl)acetamide (Compound 337)

The title compound was prepared using the methodologies described in Example 232 substituting the product of Example 214F for the product of Example 198I and substituting 2-(3,4-dichlorophenoxy)acetaldehyde for 2-(4-fluorophenoxy)acetaldehyde. ¹H NMR (400 MHz, DMSO-*d*₆) *δ* ppm 8.79 (t, *J =* 6.1 Hz, 2H), 7.58 (d, *J =* 8.9 Hz, 1H), 7.55 - 7.40 (m, 2H), 7.31 - 7.20 (m, 1H), 7.04 (ddd, *J =* 11.9, 10.2, 2.9 Hz, 2H), 6.83 (dt, *J =* 9.0, 2.0 Hz, 1H), 5.36 (s, 1H), 4.49 (s, 2H), 4.20 (dt, *J =* 22.4, 3.8 Hz, 3H), 3.28 (t, *J =* 6.0 Hz, 2H), 2.27 (ddd, *J =* 12.3, 9.3, 2.4 Hz, 1H), 2.14 - 2.00 (m, 1H), 1.99 (td, *J =* 8.7, 3.0 Hz, 1H), 1.85 (dq, *J =* 15.2, 9.2, 7.2 Hz, 4H), 1.82 - 1.70 (m, 1H), 1.65 (dt, *J =* 12.9, 3.0 Hz, 1H); MS (ESI⁺) *m*/*z* 531.2 (M+H)⁺.

### Example 239: 2-(4-chloro-3-fluorophenoxy)-N-[3-hydroxy-3-methyl-4-(2-{[6-(trifluoromethyl)pyridin-3-yl]oxy}acetamido)bicyclo[2.2.2]octan-1-yl]acetamide (Compound 338)

### Example 239A: tert-butyl {4-[2-(4-chloro-3-fluorophenoxy)acetamido]-2-hydroxy-2-methylbicyclo[2.2.2]octan-1-yl}carbamate

To a solution of the product of Example 173H (300.0 mg, 0.680 mmol) in tetrahydrofuran (15 mL) at -78 °C was added 1.5 M methyllithium lithium bromide complex in diethyl ether (2.3 mL). The reaction mixture was stirred at -78 °C for 1 hour and then was quenched with brine. The mixture was extracted with ethyl acetate (2×). The combined organic layers were dried over anhydrous MgSO₄, filtered, and concentrated under reduced pressure. The residue was purified on a 25 g silica gel column using a Biotage^{®} Isolera^{™} One flash system eluting with heptanes/ethyl acetate (1:1) to provide the title compound (0.23 g, 0.50 mmol, 75% yield). MS (ESI⁺) *m*/*z* 455.0 (M+H)⁺.

### Example 239B: N-(4-amino-3-hydroxy-3-methylbicyclo[2.2.2]octan-1-yl)-2-(4-chloro-3-fluorophenoxy)acetamide, hydrochloric acid

A mixture of the product of Example 239A (0.225 g, 0.49 mmol) and trifluoroacetic acid (0.379 mL, 4.92 mmol) in CH₂Cl₂ (5 mL) was stirred for 5 hours. The reaction mixture was concentrated under reduced pressure, and the residue was dissolved in methanol (2 mL). The solution was treated with 2 M HCl (2 mL) in ether, and the mixture was stirred for 15 minutes. The solution was concentrated under reduced pressure to give the title compound (0.161 g, 0.41 mmol, 83% yield). MS (ESI⁺) *m*/*z* 357.1 (M+H)⁺.

### Example 239C: 2-(4-chloro-3-fluorophenoxy)-N-[3-hydroxy-3-methyl-4-(2-{[6-(trifluoromethyl)pyridin-3-yl]oxy}acetamido)bicyclo[2.2.2]octan-1-yl]acetamide

A mixture of the product of Example 239B (78.0 mg, 0.20 mmol), Example 301B (48.2 mg, 0.22 mmol), *N*-[(dimethylamino)-1*H*-1,2,3-triazolo-[4,5-b]pyridin-1-ylmethylene]-N-methylmethanaminium hexafluorophosphate *N*-oxide (HATU, 90 mg, 0.24 mmol), and triethylamine (0.11 mL, 0.79 mmol) in tetrahydrofuran (3 mL) was stirred for 2 hours. The reaction mixture was treated with saturated aqueous NaHCO₃ and brine and extracted with ethyl acetate (2×). The combined organic layers were dried over anhydrous MgSO₄, filtered, and concentrated under reduced pressure. The residue was purified by reverse-phase HPLC (see protocol in Example 112D) to provide the title compound (46.4 mg, 0.083 mmol, 42% yield). ¹H NMR (400 MHz, DMSO-*d*₆) *δ* ppm 8.44 (d, J = 2.9 Hz, 1H), 7.87 (d, J = 8.7 Hz, 1H), 7.65 - 7.41 (m, 4H), 7.02 (dd, J = 11.4, 2.9 Hz, 1H), 6.80 (ddd, J = 9.0, 2.9, 1.2 Hz, 1H), 5.53 (s, brd,1H), 4.76 (s, 2H), 4.43 (s, 2H), 2.23 (tt, J = 11.5, 3.1 Hz, 1H), 2.04 (dddt, J = 13.6, 11.2, 5.9, 2.6 Hz, 2H), 1.97 - 1.69 (m, 6H), 1.62 (td, J = 11.5, 6.0 Hz, 1H), 1.10 (s, 3H); MS (ESI⁺) *m*/*z* 557.9 (M+H)⁺.

### Example 240: N,N'-(2-hydroxy-2-methylbicyclo[2.2.2]octane-1,4-diyl)bis[2-(4-chloro-3-fluorophenoxy)acetamide] (Compound 339)

The reaction described in Example 239C substituting 2-(4-chloro-3-fluorophenoxy)acetic acid for Example 301B gave the title compound. ¹H NMR (400 MHz, DMSO-*d*₆) *δ* ppm 7.58 - 7.41 (m, 6H), 7.03 (td, J = 11.7, 2.8 Hz, 2H), 6.81 (dddd, J = 9.0, 7.9, 2.8, 1.2 Hz, 2H), 5.57 (s, 1H), 4.58 (s, 2H), 4.43 (s, 2H), 2.21 (dd, J = 13.2, 9.9 Hz, 1H), 2.15 - 1.97 (m, 2H), 1.97 - 1.69 (m, 6H), 1.62 (td, J = 11.5, 5.8 Hz, 1H), 1.09 (s, 3H); MS (ESI⁺) *m*/*z* 540.9 (M+H)⁺.

### Example 241: 2-(4-chloro-3-fluorophenoxy)-N-(2-hydroxy-4-{2-[4-(trifluoromethoxy)phenyl]acetamido}bicyclo[2.2.2]octan-1-yl)acetamide (Compound 340)

The title compound was prepared using the methodologies described in Example 214 substituting 2-(4-(trifluoromethoxy)phenyl)acetic acid for 2-((6-(trifluoromethyl)pyridin-3-yl)oxy)acetic acid. ¹H NMR (400 MHz, DMSO-*d₆*) *δ* ppm 7.68 (s, 1H), 7.48 (t, *J =* 8.9 Hz, 1H), 7.38 - 7.29 (m, 2H), 7.31 - 7.22 (m, 3H), 7.05 (dd, *J =* 11.4, 2.9 Hz, 1H), 6.82 (ddd, *J =* 9.0, 2.9, 1.2 Hz, 1H), 4.46 (s, 2H), 4.06 - 3.97 (m, 1H), 2.24 (ddd, *J =* 12.4, 9.5, 2.4 Hz, 1H), 2.05 (ddd, *J* = 12.3, 10.3, 5.1 Hz, 1H), 1.94 - 1.83 (m, 2H), 1.87 - 1.68 (m, 6H); MS (ESI⁺) *m*/*z 545.0* (M+H)⁺.

### Example 242: 2-(4-chloro-3-fluorophenoxy)-N-(3-{2-[4-(trifluoromethyl)phenyl]acetamido}bicyclo[1.1.1]pentan-1-yl)acetamide (Compound 341)

A mixture of the product of Example 112A (70.0 mg, 0.22 mmol), 2-(4-(trifluoromethyl)phenyl)acetic acid (48.9 mg, 0.24 mmol), *N*-[(dimethylamino)-1*H*-1,2,3-triazolo-[4,5-*b*]pyridin-1-ylmethylene]-N-methylmethanaminium hexafluorophosphate N-oxide (HATU, 99 mg, 0.262 mmol), and triethylamine (0.122 mL, 0.872 mmol) in tetrahydrofuran (2 mL) was stirred for 2 hours. The reaction mixture was treated with saturated aqueous NaHCO₃ and brine and extracted with ethyl acetate (2×). The combined organic layers were dried over anhydrous MgSO₄, filtered, and concentrated under reduced pressure. The residue was purified by reverse-phase HPLC (see protocol in Example 112D) to provide the title compound (57.6 mg, 0.122 mmol, 56% yield). ¹H NMR (400 MHz, DMSO-*d*₆) *δ* ppm 8.71 (s, 1H), 8.67 (s, 1H), 7.62 (d, J = 7.9 Hz, 2H), 7.53 - 7.35 (m, 3H), 7.03 (dd, J = 11.4, 2.8 Hz, 1H), 6.81 (ddd, J = 9.0, 2.9, 1.2 Hz, 1H), 4.43 (s, 2H), 3.45 (s, 2H), 2.18 (s, 6H); MS (ESI⁺) *m*/*z* 471.0 (M+H)⁺.

### Example 243: 2-(4-chloro-3-fluorophenoxy)-N-(3-{2-[6-(trifluoromethyl)pyridin-3-yl]acetamido}bicyclo[1.1.1]pentan-1-yl)acetamide (Compound 342)

The reaction described in Example 242 substituting 2-(6-(trifluoromethyl)pyridin-3-yl)acetic acid for 2-(4-(trifluoromethyl)phenyl)acetic acid gave the title compound. ¹H NMR (400 MHz, DMSO-*d*₆) *δ* ppm 8.78 (s, 1H), 8.67 (s, 1H), 8.57 (d, J = 2.0 Hz, 1H), 7.94 - 7.86 (m, 1H), 7.81 (dd, J = 8.1, 0.9 Hz, 1H), 7.45 (t, J = 8.9 Hz, 1H), 7.02 (dd, J = 11.4, 2.8 Hz, 1H), 6.81 (ddd, J = 9.0, 2.9, 1.2 Hz, 1H), 4.43 (s, 2H), 3.53 (s, 2H), 2.19 (s, 6H); MS (ESI⁺) *m*/*z* 472.2 (M+H)⁺.

### Example 244: 2-(4-chloro-3-fluorophenoxy)-N-(4-{[(4-cyanophenyl)methyl]amino}-3-hydroxybicyclo[2.2.2]octan-1-yl)acetamide (Compound 343)

The title compound was prepared using the methodologies described in Example 232 substituting 2-(4-cyanophenyl)acetaldehyde for 2-(4-fluorophenoxy)acetaldehyde. ¹H NMR (400 MHz, DMSO-*d₆*) *δ* ppm 8.90 (s, 2H), 8.85 (d, *J =* 2.0 Hz, 1H), 8.77 (d, *J =* 10.0 Hz, 1H), 8.20 (dd, *J =* 8.1, 2.0 Hz, 1H), 8.03 (d, *J =* 8.1 Hz, 1H), 7.72 (s, 1H), 7.49 (t, *J =* 8.9 Hz, 1H), 7.03 (dd, *J =* 11.4, 2.9 Hz, 1H), 6.82 (ddd, *J =* 8.9, 2.9, 1.2 Hz, 1H), 5.85 (s, 1H), 4.47 (s, 2H), 4.25 (s, 2H), 4.15 (d, *J =* 9.2 Hz, 1H), 2.40 (td, *J =* 10.4, 9.5, 5.3 Hz, 1H), 2.13 (td, *J =* 11.5, 4.5 Hz, 1H), 2.08 - 1.97 (m, 1H), 1.92 (s, 6H), 1.76 - 1.65 (m, 1H); MS (ESI⁺) *m*/*z* 458.2 (M+H)⁺.

### Example 245: 1,4-bis[2-(4-chloro-3-fluorophenoxy)acetamido]bicyclo[2.2.2]octan-2-yl methanesulfonate (Compound 344)

To a solution of Example 198K (0.32 g, 0.605 mmol) in dichloromethane (4.0 mL) was added triethylamine (0.169 mL, 1.209 mmol) followed by methanesulfonyl chloride (0.061 mL, 0.786 mmol). The mixture was allowed to stir at ambient temperature for 16 hours. The mixture was concentrated under reduced pressure and stirred with water for 30 minutes. The precipitate was collected and air-dried to give 186 mg of the title compound as a white solid. The aqueous filtrate was extracted with dichloromethane, and the organic fraction was dried (MgSO₄) and concentrated. The residue was purified by HPLC (10-95% acetonitrile in 0.1% trifluoroacetic acid/water at 25 mL/minute on a Phenomenex^{®} C18 5 µm column (21.2 × 250 mm)) to give 120 mg of the title compound as a white solid. ¹H NMR (400 MHz, DMSO-*d₆*) *δ* ppm 7.70 (d, *J*= 3.9 Hz, 2H), 7.47 (td, *J =* 8.9, 4.0 Hz, 2H), 7.03 (dd, *J =* 11.4, 2.9 Hz, 2H), 6.82 (ddt, *J =* 8.9, 2.7, 1.3 Hz, 2H), 5.41 - 5.33 (m, 1H), 4.54 - 4.41 (m, 4H), 3.11 (s, 3H), 2.19 (dt, *J =* 14.6, 2.3 Hz, 1H), 2.10 - 1.97 (m, 1H), 1.98 - 1.68 (m, 8H); MS (ESI⁺) *m*/*z* 623.8 [M+NH₄]⁺.

### Example 246: 2-(4-chloro-3-fluorophenoxy)-N-{4-[2-(4-fluorophenoxy)acetamido]-3-hydroxybicyclo[2.2.2]octan-1-yl}acetamide (Compound 345)

The title compound was prepared using the methodologies described in Example 202 substituting 2-(4-fluorophenoxy)acetic acid for 2-((6-(trifluoromethyl)pyridin-3-yl)oxy)acetic acid. ¹H NMR (501 MHz, DMSO-*d₆*) *δ* ppm 7.49 (dd, *J =* 17.3, 8.4 Hz, 2H), 7.21 (s, 1H), 7.18 - 7.07 (m, 2H), 7.02 (dd, *J =* 11.4, 2.9 Hz, 1H), 7.00 - 6.90 (m, 2H), 6.81 (ddd, *J =* 9.0, 2.9, 1.2 Hz, 1H), 5.15 (d, *J =* 4.5 Hz, 1H), 4.41 (d, *J =* 17.4 Hz, 4H), 4.05 - 3.96 (m, 1H), 2.28 (ddd, *J* = 12.5, 9.5, 2.4 Hz, 1H), 2.14 (td, *J =* 10.0, 9.2, 5.7 Hz, 1H), 1.93 (s, 1H), 1.93 - 1.73 (m, 8H); MS (ESI⁺) *m*/*z* 495.1 (M+H)⁺.

### Example 247: (2E)-N-{3-[2-(4-chloro-3-fluorophenoxy)acetamido]bicyclo[1.1.1]pentan-1-yl}-3-[4-(trifluoromethyl)phenyl]prop-2-enamide (Compound 346)

The reaction described in Example 242 substituting (*E*)-3-(4-(trifluoromethyl)phenyl)acrylic acid for 2-(4-(trifluoromethyl)phenyl)acetic acid gave the title compound. ¹H NMR (400 MHz, DMSO-*d*₆) *δ* ppm 8.80 (s, 1H), 8.71 (s, 1H), 7.73 (s, 4H), 7.54 - 7.36 (m, 2H), 7.04 (dd, J = 11.4, 2.8 Hz, 1H), 6.82 (ddd, J = 9.0, 2.9, 1.2 Hz, 1H), 6.63 (d, J = 15.9 Hz, 1H), 4.45 (s, 2H), 2.26 (s, 6H); MS (ESI⁺) *m*/*z* 483.1 (M+H)⁺.

### Example 248: (2E)-N-{3-[2-(4-chloro-3-fluorophenoxy)acetamido]bicyclo[1.1.1]pentan-1-yl}-3-(4-methoxyphenyl)prop-2-enamide (Compound 347)

The reaction described in Example 242 substituting (E)-3-(4-methoxyphenyl)acrylic acid for 2-(4-(trifluoromethyl)phenyl)acetic acid gave the title compound. ¹H NMR (400 MHz, DMSO-*d*₆) *δ* ppm 8.69 (s, 1H), 8.59 (s, 1H), 7.53 - 7.40 (m, 3H), 7.31 (d, J = 15.7 Hz, 1H), 7.04 (dd, J = 11.3, 2.9 Hz, 1H), 6.98 - 6.89 (m, 2H), 6.82 (ddd, J = 9.0, 2.9, 1.2 Hz, 1H), 6.36 (d, J = 15.8 Hz, 1H), 4.45 (s, 2H), 3.75 (s, 3H), 2.24 (s, 6H); MS (ESI⁺) *m*/*z* 445.1 (M+H)⁺.

### Example 249: (2E)-N-{3-[2-(4-chloro-3-fluorophenoxy)acetamido]bicyclo[1.1.1]pentan-1-yl}-3-(3,4-dichlorophenyl)prop-2-enamide (Compound 348)

The reaction described in Example 242 substituting (*E*)-3-(3,4-dichlorophenyl)acrylic acid for 2-(4-(trifluoromethyl)phenyl)acetic acid gave the title compound. ¹H NMR (400 MHz, DMSO-*d*₆) *δ* ppm 8.72 (d, J = 9.4 Hz, 2H), 7.79 (d, J = 2.0 Hz, 1H), 7.63 (d, J = 8.4 Hz, 1H), 7.55 - 7.41 (m, 2H), 7.34 (d, J = 15.8 Hz, 1H), 7.04 (dd, J = 11.4, 2.9 Hz, 1H), 6.82 (ddd, J = 9.0, 2.9, 1.2 Hz, 1H), 6.56 (d, J = 15.8 Hz, 1H), 4.45 (s, 2H), 2.25 (s, 6H); MS (ESI⁺) *m*/*z* 483.0 (M+H)⁺.

### Example 250: (2E)-N-{3-[2-(4-chloro-3-fluorophenoxy)acetamido]bicyclo[1.1.1]pentan-1-yl}-3-(pyridin-2-yl)prop-2-enamide (Compound 349)

A mixture of Example 112A (65.0 mg, 0.20 mmol), (*E*)-3-(pyridin-2-yl)acrylic acid (30.2 mg, 0.20 mmol), *N*-[(dimethylamino)-1*H*-1,2,3-triazolo-[4,5-*b*]pyridin-1-ylmethylene]-*N-*methylmethanaminium hexafluorophosphate *N*-oxide (HATU, 92 mg, 0.24 mmol), and triethylamine (0.11 mL, 0.81 mmol) in tetrahydrofuran (2 mL) was stirred for 5 hours. Water (10 mL) was added to the suspension, and the mixture was stirred for 30 minutes. The resulting solids were collected by filtration, washed with water (10 mL) and ether (10 mL), and vacuum oven-dried to provide the title compound (68.8 mg, 0.165 mmol, 82% yield). ¹H NMR (400 MHz, DMSO-*d*₆) *δ* ppm 8.85 (s, 1H), 8.72 (s, 1H), 8.62 - 8.54 (m, 1H), 7.81 (td, J = 7.7, 1.8 Hz, 1H), 7.59 - 7.36 (m, 3H), 7.34 (ddd, J = 7.6, 4.7, 1.1 Hz, 1H), 7.06 (dd, J = 11.3, 2.8 Hz, 1H), 6.96 (d, J = 15.4 Hz, 1H), 6.84 (ddd, J = 9.0, 2.9, 1.2 Hz, 1H), 4.47 (s, 2H), 2.28 (s, 6H); MS (ESI⁺) *m*/*z* 416.2 (M+H)⁺.

### Example 251: (2E)-N-{3-[2-(4-chloro-3-fluorophenoxy)acetamido]bicyclo[1.1.1]pentan-1-yl}-3-(3-chlorophenyl)prop-2-enamide (Compound 350)

The reaction described in Example 242 substituting (*E*)-3-(3-chlorophenyl)acrylic acid for 2-(4-(trifluoromethyl)phenyl)acetic acid gave the title compound. ¹H NMR (400 MHz, DMSO-*d₆*) *δ* ppm 8.72 (d, J = 2.7 Hz, 2H), 7.59 (q, J = 1.4 Hz, 1H), 7.54 - 7.28 (m, 5H), 7.06 (dd, J = 11.4, 2.9 Hz, 1H), 6.84 (ddd, J = 8.9, 2.9, 1.2 Hz, 1H), 6.57 (d, J = 15.8 Hz, 1H), 4.47 (s, 2H), 2.27 (s, 6H); MS (ESI⁺) *m*/*z* 449.1 (M+H)⁺.

### Example 252: (2E)-N-{3-[2-(4-chloro-3-fluorophenoxy)acetamido]bicyclo[1.1.1]pentan-1-yl}-3-[6-(trifluoromethyl)pyridin-3-yl]prop-2-enamide (Compound 351)

The reaction described in Example 242 substituting (*E*)-3-(6-(trifluoromethyl)pyridin-3-yl)acrylic acid for 2-(4-(trifluoromethyl)phenyl)acetic acid gave the title compound. ¹H NMR (400 MHz, DMSO-*d*₆) *δ* ppm 8.98 - 8.84 (m, 2H), 8.73 (s, 1H), 8.21 (dd, J = 8.2, 2.1 Hz, 1H), 7.93 (d, J = 8.2 Hz, 1H), 7.58 - 7.37 (m, 2H), 7.06 (dd, J = 11.4, 2.8 Hz, 1H), 6.84 (ddd, J = 8.9, 2.8, 1.2 Hz, 1H), 6.75 (d, J = 15.9 Hz, 1H), 4.47 (s, 2H), 2.28 (s, 6H); MS (ESI⁺) *m*/*z* 484.1 (M+H)⁺.

### Example 253: (2E)-N-{3-[2-(4-chloro-3-fluorophenoxy)acetamido]bicyclo[1.1.1]pentan-1-yl}-3-(pyridin-3-yl)prop-2-enamide (Compound 352)

The reaction described in Example 242 substituting (E)-3-(pyridin-3-yl)acrylic acid for 2-(4-(trifluoromethyl)phenyl)acetic acid gave the title compound as a trifluoroacetic acid salt. ¹H NMR (500 MHz, DMSO-*d₆*) *δ* ppm 8.86 (d, J = 5.6 Hz, 2H), 8.74 (s, 1H), 8.65 (dd, J = 5.0, 1.5 Hz, 1H), 8.20 (dt, J = 8.0, 1.9 Hz, 1H), 7.65 (dd, J = 8.0, 5.0 Hz, 1H), 7.54 - 7.40 (m, 2H), 7.07 (dd, J = 11.3, 2.8 Hz, 1H), 6.85 (dt, J = 8.8, 1.8 Hz, 1H), 6.70 (d, J = 15.9 Hz, 1H), 4.48 (s, 2H), 2.29 (s, 6H); MS (ESI⁺) *m*/*z* 416.1 (M+H)⁺.

### Example 254: 2-(4-chloro-3-fluorophenoxy)-N-{(3R)-4-[2-(4-fluorophenoxy)acetamido]-3-hydroxybicyclo[2.2.2]octan-1-yl}acetamide (Compound 353)

The title compound was isolated by chiral preparative SFC of Example 246 as the first peak eluted off the column using the methodologies described in Example 198. ¹H NMR (501 MHz, DMSO-*d*₆) *δ* ppm 7.47 (dd, J = 17.2, 8.4 Hz, 2H), 7.19 (s, 1H), 7.15 - 7.06 (m, 2H), 7.00 (dd, J = 11.4, 2.9 Hz, 1H), 6.97 - 6.89 (m, 2H), 6.79 (ddd, J = 9.0, 2.9, 1.2 Hz, 1H), 5.12 (d, J = 4.2 Hz, 1H), 4.43 - 4.32 (m, 4H), 3.98 (d, J = 9.5 Hz, 1H), 2.25 (ddd, J = 12.6, 9.5, 2.8 Hz, 1H), 2.18 - 2.06 (m, 1H), 1.91 - 1.71 (m, 8H); MS (ESI⁺) *m*/*z* 495.2 (M+H)⁺.

### Example 255: 2-(4-chloro-3-fluorophenoxy)-N-{(3S)-4-[2-(4-fluorophenoxy)acetamido]-3-hydroxybicyclo[2.2.2]octan-1-yl}acetamide (Compound 354)

The title compound was isolated by chiral preparative SFC of Example 246 as the second peak eluted off the column using the methodologies described in Example 198. ¹H NMR (501 MHz, DMSO-*d*₆) *δ* ppm 7.47 (dd, J = 18.0, 9.1 Hz, 2H), 7.19 (s, 1H), 7.15 - 7.04 (m, 2H), 7.00 (dd, J = 11.4, 2.8 Hz, 1H), 6.99 - 6.89 (m, 2H), 6.79 (ddd, J = 9.0, 2.9, 1.2 Hz, 1H), 5.13 (s, 1H), 4.43 - 4.32 (m, 4H), 3.98 (dd, J = 10.0, 3.2 Hz, 1H), 2.26 (ddd, J = 12.5, 9.4, 2.8 Hz, 1H), 2.12 (tt, J = 9.1, 5.0 Hz, 1H), 1.96 - 1.75 (m, 8H); MS (ESI⁺) *m*/*z* 495.1 (M+H)⁺.

### Example 256: 2-(4-chloro-3-fluorophenoxy)-N-[4-(2-{[5-(trifluoromethyl)pyridin-3-yl]oxy}acetamido)bicyclo[2.1.1]hexan-1-yl]acetamide (Compound 355)

### Example 256A: {[5-(trifluoromethyl)pyridin-3-yl]oxy}acetic acid

The reaction and purification conditions described in Example 11A and Example 11B substituting 5-(trifluoromethyl)pyridin-3-ol (Aldrich) for 5-hydroxy-3-methylbenzo[*d*]isoxazole gave the title compound. MS (ESI⁺) *m*/*z* 278 (M+H)⁺.

### Example 256B: 2-(4-chloro-3-fluorophenoxy)-N-[4-(2-{[5-(trifluoromethyl)pyridin-3-yl]oxy]acetamido)bicyclo[2.1.1]hexan-1-yl]acetamide

The reaction and purification conditions described in Example 81, substituting the product of Example 256A for 2-(4-chloro-3-fluorophenoxy)acetic acid and the product of Example 88C for benzyl (4-aminobicyclo[2.1.1]hexan-1-yl)carbamate hydrochloride gave the title compound. ¹H NMR (400 MHz, DMSO-*d*₆) *δ* ppm 8.61 (d, J = 2.8 Hz, 1H), 8.58 - 8.55 (m, 2H), 8.48 (s, 1H), 7.73 (t, J = 2.4 Hz, 1H), 7.49 (t, J = 8.9 Hz, 1H), 7.06 (dd, J = 11.4, 2.9 Hz, 1H), 6.85 (ddd, J = 8.9, 2.9, 1.2 Hz, 1H), 4.68 (s, 2H), 4.48 (s, 2H), 2.10 - 2.04 (m, 2H), 1.84 - 1.77 (m, 6H); MS (ESI⁺) *m*/*z* 502 (M+H)⁺.

### Example 257: 2-(4-chloro-3-fluorophenoxy)-N-(2-hydroxy-4-{2-[5-(trifluoromethyl)pyridin-2-yl]acetamido}bicyclo[2.2.2]octan-1-yl)acetamide (Compound 356)

The title compound was prepared using the methodologies described in Example 214 substituting 2-(5-(trifluoromethyl)pyridin-2-ylacetic acid for 2-((6-(trifluoromethyl)pyridin-3-yl)oxy)acetic acid. ¹H NMR (400 MHz, DMSO-*d*₆) *δ* ppm 8.85 (d, *J =* 2.3 Hz, 1H), 8.14 (dd, *J* = 8.3, 2.5 Hz, 1H), 7.78 (s, 1H), 7.57 - 7.43 (m, 2H), 7.25 (s, 1H), 7.05 (dd, *J =* 11.4, 2.8 Hz, 1H), 6.82 (dd, *J =* 9.1, 3.0 Hz, 1H), 4.46 (s, 2H), 4.02 (dd, *J =* 9.5, 3.1 Hz, 1H), 3.69 (s, 2H), 2.26 (ddd, *J =* 12.2, 9.5, 2.2 Hz, 1H), 2.06 (ddd, *J =* 12.2, 10.5, 4.8 Hz, 1H), 1.95 - 1.85 (m, 2H), 1.88 - 1.80 (m, 1H), 1.84 - 1.69 (m, 6H).; MS (ESI⁺) *m*/*z* 530.1 (M+H)⁺.

### Example 258: N,N'-(2-aminobicyclo[2.2.2]octane-1,4-diyl)bis[2-(4-chloro-3-fluorophenoxy)acetamide] (Compound 357)

### Example 258A: N,N'-(2-azidobicyclo[2.2.2]octane-1,4-diyl)bis[2-(4-chloro-3-fluorophenoxy)acetamide]

A mixture of Example 245 (0.25 g, 0.412 mmol) and sodium azide (0.134 g, 2.058 mmol) in *N*,*N*-dimethylformamide (2.5 mL) in a microwave reactor vial was heated in a microwave oven (Biotage^{®} Initiator, 450 W) at 120 °C for 45 minutes. The reaction mixture was cooled to ambient temperature and partitioned between water and ethyl acetate. The organic layer was washed with brine, dried over magnesium sulfate and filtered. The filtrate was concentrated, and the residue was purified by HPLC (10-95% acetonitrile in 0.1% trifluoroacetic acid/water at 25 mL/minute on a Phenomenex^{®} C18 5 µm column (250 × 21.2 mm)) to give 34 mg of the title compound as a white solid. ¹H NMR (501 MHz, DMSO-*d*₆) *δ* ppm 7.73 (s, 1H), 7.62 (s, 1H), 7.47 (td, *J =* 8.9, 5.3 Hz, 2H), 7.02 (dd, *J =* 11.4, 2.8 Hz, 2H), 6.81 (dddd, *J =* 8.5, 5.5, 2.9, 1.2 Hz, 2H), 4.66 (ddd, *J =* 10.3, 3.7, 1.8 Hz, 1H), 4.54 - 4.42 (m, 4H), 2.40 - 2.31 (m, 1H), 1.99 - 1.59 (m, 9H).

### Example 258B: N,N'-(2-aminobicyclo[2.2.2]octane-1,4-diyl)bis[2-(4-chloro-3-fluorophenoxy)acetamide] trifluoroacetate

A mixture of Example 258A (32 mg, 0.058 mmol) and Raney^{®}-nickel 2800, water slurry (85.1 mg, 0.652 mmol) in tetrahydrofuran (6 mL) in a 50 mL pressure bottle was shaken for 16 hours under 50 psi of hydrogen at ambient temperature. After filtration, the filtrate was concentrate, and the residue was purified by HPLC (10-95% acetonitrile in 0.1% trifluoroacetic acid/water at 25 mL/minute on a Phenomenex^{®} C18 5 µm column (250 × 21.2 mm)) to give 27 mg of the title compound as a white solid. ¹H NMR (400 MHz, methanol-*d*₄) *δ* ppm 7.64 (s, 1H), 7.60 (s, 1H), 7.36 (td, J = 8.7, 5.8 Hz, 2H), 6.89 (ddd, J = 16.3, 10.9, 2.8 Hz, 2H), 6.79 (tq, J = 8.8, 3.0, 2.4 Hz, 2H), 4.56 - 4.41 (m, 4H), 4.33 (dd, J = 10.7, 5.6 Hz, 1H), 2.58 -2.41 1.69 (m, 10H); MS (ESI⁺) *m*/*z* 528.1 (M+H)⁺.

### Example 259: N,N'-(2-acetamidobicyclo[2.2.2]octane-1,4-diyl)bis[2-(4-chloro-3-fluorophenoxy)acetamide] (Compound 358)

To a solution of Example 258 (0.018 g, 0.028 mmol) and pyridine (0.011 mL, 0.140 mmol) in dichloromethane (1.0 mL), acetic anhydride (7.93 µl, 0.084 mmol) was added, and the mixture was stirred at ambient temperature for 1 hour. The mixture was concentrated, and the residue was purified by HPLC (10-95% acetonitrile in 0.1% trifluoroacetic acid/water at 25 mL/minute on a Phenomenex^{®} C18 5 µm column (250 × 21.2 mm)) to give 12 mg of the title compound as a white solid. ¹H NMR (400 MHz, DMSO-*d*₆) *δ* ppm 8.12 (d, J = 7.8 Hz, 1H), 7.75 (s, 1H), 7.54 (s, 1H), 7.45 (td, J = 8.9, 6.9 Hz, 2H), 7.01 (ddd, J = 15.7, 11.3, 2.9 Hz, 2H), 6.80 (ddt, J = 24.1, 9.0, 1.9 Hz, 2H), 4.44 - 4.23 (m, 4H), 4.07 (s, 1H), 2.57 - 2.47 (m, 1H), 2.45 - 2.27 (m, 2H), 2.02 (t, J = 12.2 Hz, 1H), 1.85 - 1.56 (m, 9H); MS (ESI⁺) *m*/*z* 570.2 (M+H)⁺.

### Example 260: 2-(3,4-difluorophenoxy)-N-[3-hydroxy-4-(2-{[6-(trifluoromethyl)pyridin-3-yl]oxy}acetamido)bicyclo[2.2.2]octan-1-yl]acetamide (Compound 359)

### Example 260A: N-(4-amino-3-oxobicyclo[2.2.2]octan-1-yl)-2-(3,4-difluorophenoxy)acetamide

The title compound was prepared using the methodologies described in Example 198F - 198H substituting 2-(3,4-difluorophenoxy)acetic acid for 2-(4-chloro-3-fluorophenoxy)acetic acid. ¹H NMR (400 MHz, DMSO-*d*₆) *δ* ppm 7.78 (s, 1H), 7.40 - 7.28 (m, 1H), 7.08 - 6.98 (m, 1H), 6.76 (br s, 1H), 6.76 - 6.68(m, 1H), 4.42 (s, 2H), 2.71 (s, 2H), 2.04 - 1.88 (m, 4H), 1.82 - 1.68 (m, 2H), 1.58 - 1.44 (m, 4H); MS (ESI⁺) *m*/*z* 325.0 (M+H)⁺.

### Example 260 B: 2-(3,4-difluorophenoxy)-N-[3-hydroxy-4-(2-{[6-(trifluoromethyl)pyridin-3-yl]oxy}acetamido)bicyclo[2. 2.2]octan-1-yl]acetamide

A mixture of Example 260A (0.1 g, 0.308 mmol), 2-((6-(trifluoromethyl)pyridin-3-yl)oxy)acetic acid (0.085 g, 0.385 mmol) and *N*-ethyl-*N*-isopropylpropan-2-amine (0.135 mL, 0.771 mmol) in dimethylformamide (DMF) (3.0 mL) was treated with 2-(3*H*-[1,2,3]triazolo[4,5-*b*]pyridin-3-yl)-1,1,3,3-tetramethylisouronium hexafluorophosphate(V) (0.176 g, 0.463 mmol), and the reaction mixture was stirred at ambient temperature for 16 hours. Volatiles were removed under high vacuum, and the residue was dissolved in dichloromethane/methanol (1:1, 2 mL) and treated with sodium borohydride (0.058 g, 1.542 mmol) for 1 hour at ambient temperature. The reaction mixture was concentrated, and the residue was purified by HPLC (10-95% acetonitrile in 0.1% trifluoroacetic acid/water linear gradient on Phenomenex^{®} C18 5 µm column (25 mm × 21.2 mm) at a flow rate of 25 mL/minute) to give 85 mg of the title compound as a white solid. ¹H NMR (400 MHz, DMSO-*d*₆) *δ* ppm 8.40 (d, J = 2.8 Hz, 1H), 7.81 (d, J = 8.7 Hz, 1H), 7.51 (dd, J = 8.7, 2.9 Hz, 1H), 7.43 (s, 1H), 7.39 - 7.25 (m, 2H), 7.00 (ddd, J = 12.7, 6.7, 3.1 Hz, 1H), 6.71 (dq, J = 8.6, 3.0 Hz, 1H), 4.93 (s, 1H), 4.61 (s, 2H), 4.36 (s, 2H), 4.05 (dd, J = 9.5, 3.0 Hz, 1H), 2.23 (dd, J = 13.1, 9.5 Hz, 1H), 2.03 - 1.78 (m, 4H), 1.82 - 1.67 (m, 5H); MS (ESI⁺) *m*/*z* 530.2 (M+H)⁺.

### Example 261: 2-(1,2-benzoxazol-3-yl)-N-{3-[2-(4-chloro-3-fluorophenoxy)acetamido]bicyclo[1.1.1]pentan-1-yl}acetamide (Compound 360)

The title compound was prepared using the methodologies described above. ¹H NMR (400 MHz, DMSO-*d₆*/D₂O) *δ* ppm 7.84 (d, *J =* 7.9 Hz, 1H), 7.75 - 7.62 (m, 2H), 7.48 (t, *J =* 8.9 Hz, 1H), 7.41 (t, *J* = 7.4 Hz, 1H), 7.05 (dd, *J =* 11.3, 2.9 Hz, 1H), 6.86 (dd, *J* = 9.0, 2.8 Hz, 1H), 4.46 (s, 2H), 3.88 (s, 2H), 2.26 (s, 6H); MS (APCI) *m*/*z* 444 (M+H)⁺.

### Example 262: 2-(4-chloro-3-fluorophenoxy)-N-(3-{2-[3-(trifluoromethoxy)phenyl]acetamido}bicyclo[1.1.1]pentan-1-yl)acetamide (Compound 361)

The title compound was prepared using the methodologies described above. ¹H NMR (400 MHz, DMSO-*d₆*/D₂O) *δ* ppm 7.54 - 7.40 (m, 2H), 7.32 - 7.19 (m, 3H), 7.05 (dd, *J =* 11.2, 2.8 Hz, 1H), 6.86 (d, *J =* 11.2 Hz, 1H), 4.46 (s, 2H), 3.44 (s, 2H), 2.23 (s, 6H); MS (APCI) *m*/*z* 487 (M+H)⁺.

### Example 263: 2-[(2,2-difluoro-2H-1,3-benzodioxol-5-yl)oxy]-N-{4-[2-(3,4-difluorophenoxy)acetamido]-2-hydroxybicyclo[2.2.2]octan-1-yl}acetamide (Compound 362)

The title compound was prepared using the methodologies described in Example 260 substituting Example 206B for 2-((6-(trifluoromethyl)pyridin-3-yl)oxy)acetic acid. ¹H NMR (501 MHz, DMSO-*d*₆) *δ* ppm 7.45 (s, 1H), 7.38 - 7.26 (m, 2H), 7.21 (s, 1H), 7.10 (d, J = 2.6 Hz, 1H), 7.02 (ddd, J = 12.7, 6.7, 3.1 Hz, 1H), 6.73 (ddd, J = 8.9, 4.1, 2.1 Hz, 2H), 5.09 (d, J = 4.5 Hz, 1H), 4.39 (d, J = 16.2 Hz, 4H), 4.02 (dt, J = 8.9, 4.0 Hz, 1H), 2.26 (ddd, J = 12.5, 9.4, 2.4 Hz, 1H), 2.08 (ddd, J = 12.3, 10.7, 4.6 Hz, 1H), 1.97 - 1.75 (m, 10H); MS (ESI⁺) *m*/*z* 541.1 (M+H)⁺.

### Example 264: N,N'-(2-hydroxybicyclo[2.2.2]octane-1,4-diyl)bis[2-(3,4-difluorophenoxy)acetamide] (Compound 363)

### Example 264A: N-(4-amino-3-hydroxybicyclo[2.2.2]octan-1-yl)-2-(3,4-difluorophenoxy)acetamide hydrochloride

The title compound was prepared using the methodologies described in Examples 198F-198I substituting 2-(3,4-difluorophenoxy)acetic acid for 2-(4-chloro-3-fluorophenoxy)acetic acid. ¹H NMR (500 MHz, DMSO-*d*₆) *δ* ppm 8.00 (s, 3H), 7.74 (s, 1H), 7.35 (dt, *J =* 10.6, 9.3 Hz, 1H), 7.04 (ddd, *J =* 12.7, 6.7, 3.1 Hz, 1H), 6.75 (dtd, *J =* 8.5, 3.3, 1.6 Hz, 1H), 5.62 (s, 1H), 4.43 (s, 2H), 3.85 (dt, *J =* 9.3, 2.4 Hz, 1H), 2.32 (ddd, *J =* 12.9, 9.5, 3.0 Hz, 1H), 2.08 - 1.92 (m, 2H), 1.85 (tt, *J =* 13.6, 6.9 Hz, 5H), 1.68 (ddt, *J =* 11.5, 7.2, 3.5 Hz, 1H), 1.59 (ddt, *J =* 14.4, 10.3, 2.2 Hz, 1H); MS (ESI⁺) *m*/*z* 327.3 (M+H)⁺.

### Example 264B: N,N'-(2-hydroxybicyclo[2.2.2]octane-1,4-diyl)bis[2-(3,4-difluorophenoxy)acetamide]

The title compound was prepared using the methodologies described in Example 202 substituting Example 264A for 198I and substituting 2-(3,4-difluorophenoxy)acetic acid for 2-((6-(trifluoromethyl)pyridin-3-yl)oxy)acetic acid. ¹H NMR (400 MHz, DMSO-*d*₆) *δ* ppm 7.43 (s, 1H), 7.32 (td, J = 9.4, 7.7 Hz, 2H), 7.19 (s, 1H), 7.02 (tdd, J = 12.3, 6.7, 3.0 Hz, 2H), 6.73 (tt, J = 9.2, 2.6 Hz, 2H), 5.06 (d, J = 4.3 Hz, 1H), 4.37 (d, J = 13.3 Hz, 4H), 4.09 - 3.95 (m, 1H), 2.23 (td, J = 10.4, 9.8, 4.8 Hz, 1H), 2.11 - 1.99 (m, 1H), 1.93 - 1.87 (m, 1H), 1.88 (s, 1H), 1.76 (tt, J = 9.9, 7.0 Hz, 6H); MS (ESI⁺) *m*/*z* 497.1 (M+H)⁺.

### Example 265: 2-(3,4-difluorophenoxy)-N-{4-[2-(4-fluorophenoxy)acetamido]-3-hydroxybicyclo[2.2.2]octan-1-yl}acetamide (Compound 364)

The title compound was prepared using the methodologies described in Example 264 substituting 2-(4-fluorophenoxy)acetic acid for 2-(3,4-difluorophenoxy)acetic acid. ¹H NMR (400 MHz, DMSO-*d*₆) *δ* ppm 7.43 (s, 1H), 7.32 (q, J = 9.7 Hz, 1H), 7.17 (s, 1H), 7.15 - 6.86 (m, 5H), 6.72 (ddt, J = 10.8, 4.8, 2.2 Hz, 1H), 5.10 (d, J = 4.5 Hz, 1H), 4.36 (d, J = 2.1 Hz, 4H), 3.96 (dt, J = 8.9, 3.6 Hz, 1H), 2.24 (ddd, J = 12.5, 9.5, 2.5 Hz, 1H), 2.09 (td, J = 12.6, 11.9, 8.4 Hz, 1H), 1.88 - 1.70 (m, 8H); MS (ESI⁺) *m*/*z* 479.2 (M+H)⁺.

### Example 266: N,N'-(2-fluorobicyclo[2.2.2]octane-1,4-diyl)bis[2-(4-chloro-3-fluorophenoxy)acetamide] (Compound 365)

### Example 266A: ethyl 4-[2-(4-chloro-3-fluorophenoxy)acetamido]-2-hydroxybicyclo[2.2.2]octane-1-carboxylate

To a solution of the product of Example 173F (350 mg, 0.88 mmol) in CH₂Cl₂ (5 mL) and methanol (5 mL) was added sodium borohydride (36.6 mg, 0.97 mmol). The reaction mixture was stirred for 1.5 hours. The solution was treated with brine and saturated aqueous NaHCO₃ and extracted with CH₂Cl₂ (2×). The combined organic layers were dried over anhydrous MgSO₄, filtered, and concentrated under reduced pressure. The residue was purified on a 12 g silica gel column using a Biotage^{®} Isolera^{™} One flash system eluting with heptanes/ethyl acetate (5:5 to 4:6) to provide the title compound (0.223 g, 0.56 mmol, 63% yield). MS (ESI⁺) *m*/*z* 399.9 (M+H)⁺.

### Example 266B: ethyl 4-[2-(4-chloro-3-fluorophenoxy)acetamido]-2-fluorobicyclo[2.2.2]octane-1-carboxylate

To a solution of the product of Example 266A (185.0 mg, 0.46 mmol) in CH₂Cl₂ (10 mL) at 0 °C was added (diethylamino)sulfur trifluoride (DAST, 0.12 mL, 0.93 mmol). After 1 hour, the reaction was allowed to warm to ambient temperature and was stirred for 5 hours. The reaction mixture was quenched with saturated aqueous NaHCO₃ (10 mL) and extracted with CH₂Cl₂ (2 × 10 mL). The combined organic layers were dried over anhydrous MgSO₄, filtered, and concentrated under reduced pressure. The residue was purified on a 12 g silica gel column using a Biotage^{®} Isolera^{™} One flash system eluting with heptanes/ethyl acetate (6:4) to provide the title compound (0.124 g, 0.31 mmol, 67% yield). MS (ESI⁺) *m*/*z* 402.2 (M+H)⁺.

### Example 266C: 4-[2-(4-chloro-3-fluorophenoxy)acetamido]-2-fluorobicyclo[2.2.2]octane-1-carboxylic acid

To a solution of Example 266B (0.12 g, 0.30 mmol) in methanol (1.5 mL) and tetrahydrofuran (1.5 mL) was added a solution of lithium hydroxide (0.021 g, 0.90 mmol) in water (0.5 mL). The mixture was allowed to stir for 16 hours. Volatiles were removed under reduced pressure. The remaining solution was diluted with water (1 mL) and treated with 2.5 N HCl until a white suspension appeared. The suspension was filtered, and the collected solids were washed with water and vacuum oven-dried to provide the title compound (88.9 mg, 0.24 mmol, 80% yield). MS (ESI⁺) *m*/*z* 374.1 (M+H)⁺.

### Example 266D: N-(4-amino-3-fluorobicyclo[2.2.2]octan-1-yl)-2-(4-chloro-3-fluorophenoxy)acetamide, trifluoroacetic acid

To a suspension of the product of Example 266C (85.0 mg, 0.227 mmol) in toluene (2 mL) were added triethylamine (0.063 mL, 0.46 mmol) and diphenylphosphoryl azide (0.074 mL, 0.34 mmol). The mixture was heated at 110 °C for 1 hour. After allowing the mixture to cool to ambient temperature, the reaction mixture was treated with 3 N HCl (2 mL) followed by stirring for 16 hours. The layers were separated, and the aqueous layer was purified by reverse-phase HPLC (see protocol in Example 112D) to provide the title compound (17.4 mg, 0.050 mmol, 17% yield). MS (ESI⁺) *m*/*z* 345.1 (M+H)⁺.

### Example 266E: N,N'-(2-fluorobicyclo[2.2.2]octane-1,4-diyl)bis[2-(4-chloro-3-fluorophenoxy)acetamide]

The reaction described in Example 242 substituting Example 266D for Example 112A and substituting 2-(4-chloro-3-fluorophenoxy)acetic acid for 2-(4-(trifluoromethyl)phenyl)acetic acid gave the title compound. ¹H NMR (400 MHz, DMSO-*d*₆) *δ* ppm 7.71 (d, J = 16.7 Hz, 2H), 7.53 - 7.42 (m, 2H), 7.03 (dt, J = 11.4, 2.7 Hz, 2H), 6.81 (ddd, J = 8.9, 2.8, 1.2 Hz, 2H), 5.30 (dd, J = 54.2, 8.5 Hz, 1H), 4.50 (s, 2H), 4.45 (s, 2H), 2.46 - 1.61 (m, 10H); MS (ESI⁺) *m*/*z* 531.0 (M+H)⁺.

### Example 267: 2-(4-chloro-3-fluorophenoxy)-N-[3-fluoro-4-(2-{[6-(trifluoromethyl)pyridin-3-yl]oxy}acetamido)bicyclo[2.2.2]octan-1-yl]acetamide (Compound 366)

The reaction described in Example 242 substituting Example 266D for Example 112A and substituting Example 301B for 2-(4-(trifluoromethyl)phenyl)acetic acid gave the title compound. ¹H NMR (400 MHz, DMSO-*d*₆) *δ* ppm 8.43 (d, J = 2.8 Hz, 1H), 7.91 - 7.80 (m, 2H), 7.67 (s, 1H), 7.57 - 7.42 (m, 2H), 7.03 (dd, J = 11.4, 2.9 Hz, 1H), 6.81 (ddd, J = 9.0, 2.9, 1.2 Hz, 1H), 5.41 - 5.19 (m, 1H), 4.68 (s, 2H), 4.45 (s, 2H), 2.45 - 1.69 (m, 10H); MS (ESI⁺) *m*/*z* 548.3 (M+H)⁺.

### Example 268: (2E,2'E)-N,N'-(bicyclo[1.1.1]pentane-1,3-diyl)bis[3-(3,4-dichlorophenyl)prop-2-enamide] (Compound 367)

A mixture of bicyclo[1.1.1]pentane-1,3-diamine dihydrochloride (30.0 mg, 0.175 mmol), (E)-3-(3,4-dichlorophenyl)acrylic acid (91 mg, 0.42 mmol), N-[(dimethylamino)-1H-1,2,3-triazolo-[4,5-b]pyridin-1-ylmethylene]-N-methylmethanaminium hexafluorophosphate N-oxide (HATU, 160 mg, 0.42 mmol), and triethylamine (0.15 mL, 1.05 mmol) in tetrahydrofuran (3 mL) was allowed to stir for 16 hours. The suspension was filtered, and the collected solids were washed with ethyl acetate and water, and vacuum oven-dried to provide the title compound (60.2 mg, 0.12 mmol, 69% yield). ¹H NMR (500 MHz, DMSO-*d* ) *δ* ppm 8.76 (s, 2H), 7.83 (d, J = 2.0 Hz, 2H), 7.67 (d, J = 8.4 Hz, 2H), 7.55 (dd, J = 8.5, 2.0 Hz, 2H), 7.38 (d, J = 15.8 Hz, 2H), 6.61 (d, J = 15.8 Hz, 2H), 2.31 (s, 6H); MS (ESI⁺) *m*/*z* 496.9 (M+H)⁺.

### Example 269: 2-(4-chloro-3-fluorophenoxy)-N-{3-[2-(4-chlorophenoxy)acetamido]bicyclo[1.1.1]pentan-1-yl}acetamide (Compound 368)

To a solution of the product of Example 9B (40mg, 0.100 mmol) in dichloromethane (0.33 mL) and water (0.17 mL) were added 2-(4-chlorophenoxy)acetyl chloride (0.017 mL, 0.110 mmol) and sodium hydroxide (12.0 mg, 0.301 mmol) at ambient temperature. The reaction mixture was stirred 1 hour at ambient temperature. Water was added to the reaction mixture, and a white solid formed. The solids were isolated via filtration, washed with water and purified with preparative HPLC [Waters XBridge^{™} C18 5 µm OBD^{™} column, 30 × 100 mm, flow rate 40 mL/minute, 5-100% gradient of acetonitrile in buffer (0.1 % trifluoroacetic acid)] to give the title compound (30 mg, 0.066 mmol, 66% yield). ¹H NMR (501 MHz, DMSO-*d*₆) *δ* ppm 8.71 (s, 1H), 8.70 (s, 1H), 7.49 (t, J = 8.9 Hz, 1H), 7.34 (d, J = 8.9 Hz, 2H), 7.07 (dd, J = 11.4, 2.9 Hz, 1H), 6.97 (d, J = 9.0 Hz, 2H), 6.85 (ddd, J = 9.0, 2.9, 1.2 Hz, 1H), 4.47 (s, 2H), 4.43 (s, 2H), 2.26 (s, 6H); MS (ESI⁺) *m*/*z* 455 (M+H)⁺.

### Example 270: 2-(4-chloro-3-fluorophenoxy)-N-[3-(2-{[1-methyl-3-(trifluoromethyl)-1H-pyrazol-5-yl]oxy}acetamido)bicyclo[1.1.1]pentan-1-yl]acetamide (Compound 369)

To a solution of Example 28A (50 mg, 0.138 mmol) in acetonitrile (0.772 mL) were added 1-methyl-3-(trifluoromethyl)-1*H*-pyrazol-5-ol (25.3 mg, 0.152 mmol) and potassium carbonate (57.4 mg, 0.415 mmol). The mixture was stirred at 70 °C for 1 hour. After 1 hour, additional 1-methyl-3-(trifluoromethyl)-1*H*-pyrazol-5-ol (25.3 mg, 0.152 mmol) was added, and the mixture was stirred for an additional 1 hour. The reaction mixture was filtered, and the filtrate was concentrated under reduced pressure. The residue was purified by preparative HPLC [Waters XBridge^{™} C18 5 µm OBD^{™} column, 30 × 100 mm, flow rate 40 mL/minute, 5-100% gradient of acetonitrile in buffer (0.1 % trifluoroacetic acid)] to give the title compound (55 mg, 0.112 mmol, 81% yield). ¹H NMR (400 MHz, DMSO-*d*₆) *δ* ppm 8.76 (s, 1H), 8.72 (s, 1H), 7.49 (t, J = 8.9 Hz, 1H), 7.07 (dd, J = 11.4, 2.8 Hz, 1H), 6.85 (ddd, J = 9.0, 2.9, 1.2 Hz, 1H), 6.16 (s, 1H), 4.60 (s, 2H), 4.47 (s, 2H), 3.70 (s, 3H), 2.27 (s, 6H); MS (ESI⁺) *m*/*z* 491 (M+H)⁺.

### Example 271: (2E,2'E)-N,N'-(bicyclo[1.1.1]pentane-1,3-diyl)bis{3-[4-(trifluoromethyl)phenyl]prop-2-enamide} (Compound 370)

The reaction described in Example 268 substituting (*E*)-3-(4-(trifluoromethyl)phenyl)acrylic acid for (*E*)-3-(3,4-dichlorophenyl)acrylic acid gave the title compound. ¹H NMR (400 MHz, DMSO-*d*₆) *δ* ppm 8.86 (s, 2H), 7.77 (s, 8H), 7.48 (d, J = 15.8 Hz, 2H), 6.68 (d, J = 15.8 Hz, 2H), 2.33 (s, 6H); MS (ESI⁺) *m*/*z* 495.1 (M+H)⁺.

### Example 272: 2-(4-chloro-3-fluorophenoxy)-N-(3-{2-[(1-cyclopropyl-1H-pyrazol-5-yl)oxy]acetamidolbicyclo[1.1.1]pentan-1-yl)acetamide (Compound 371)

The reaction and purification conditions described in Example 270 substituting 1-cyclopropyl-1*H*-pyrazol-5-ol for 1-methyl-3-(trifluoromethyl)-1*H*-pyrazol-5-ol gave the title compound. ¹H NMR (400 MHz, DMSO-*d*₆) *δ* ppm 8.70 (s, 1H), 8.57 (s, 1H), 7.55 (d, J = 2.4 Hz, 1H), 7.49 (t, J = 8.9 Hz, 1H), 7.07 (dd, J = 11.4, 2.8 Hz, 1H), 6.85 (ddd, J = 9.0, 2.9, 1.2 Hz, 1H), 5.64 (d, J = 2.3 Hz, 1H), 4.47 (s, 2H), 4.42 (s, 2H), 3.49 (tt, J = 7.3, 3.5 Hz, 1H), 2.24 (s, 6H), 0.95 - 0.83 (m, 4H); MS (ESI⁺) *m*/*z* 449 (M+H)⁺.

### Example 273: 2-(4-chloro-3-fluorophenoxy)-N-[(3R)-3-fluoro-4-(2-{[6-(trifluoromethyl)pyridin-3-yl]oxy}acetamido)bicyclo[2.2.2]octan-1-yl]acetamide (Compound 372)

Example 267 was purified by chiral SFC (supercritical fluid chromatography) using a Chiralpak^{®} AD-H column (21 × 250 mm, ambient temperature) eluting with 30% CH₃OH buffered with 0.1% diethylamine (flow rate 70 g/minute, back pressure 101 bar) in CO₂ to give the title compound (first enantiomer eluted out of the column). Absolute stereochemistry assignment was arbitrary. ¹H NMR (400 MHz, DMSO-*d*₆) *δ* ppm 8.43 (d, J = 2.9 Hz, 1H), 7.92 - 7.77 (m, 2H), 7.67 (s, 1H), 7.57 - 7.41 (m, 2H), 7.03 (dd, J = 11.4, 2.9 Hz, 1H), 6.81 (ddd, J = 9.0, 2.9, 1.2 Hz, 1H), 5.41 - 5.15 (m, 1H), 4.68 (s, 2H), 4.45 (s, 2H), 2.45 - 1.58 (m, 10H). MS (ESI⁺) *m*/*z* 548.3 (M+H)⁺.

### Example 274: 2-(4-chloro-3-fluorophenoxy)-N-[(3S)-3-fluoro-4-(2-{[6-(trifluoromethyl)pyridin-3-yl]oxy}acetamido)bicyclo[2.2.2]octan-1-yl]acetamide (Compound 373)

Example 267 was purified by chiral SFC (supercritical fluid chromatography) using a Chiralpak^{®} AD-H column (21 × 250 mm, ambient temperature) eluting with 30% CH₃OH buffered with 0.1% diethylamine (flow rate 70 g/minute, back pressure 101 bar) in CO₂ to give the title compound (second enantiomer eluted out of the column). Absolute stereochemistry assignment was arbitrary. ¹H NMR (400 MHz, DMSO-*d*₆) *δ* ppm 8.43 (d, J = 2.9 Hz, 1H), 7.85 (d, J = 8.4 Hz, 2H), 7.67 (s, 1H), 7.58 - 7.40 (m, 2H), 7.03 (dd, J = 11.4, 2.9 Hz, 1H), 6.81 (ddd, J = 9.0, 2.9, 1.1 Hz, 1H), 5.30 (dd, J = 54.2, 8.6 Hz, 1H), 4.68 (s, 2H), 4.45 (s, 2H), 2.46 - 1.54 (m, 10H). MS (ESI⁺) *m*/*z* 548.3 (M+H)⁺.

### Example 275: 2-(4-chloro-3-fluorophenoxy)-N-(3-{2-[(1,3,5-trimethyl-1H-pyrazol-4-yl)oxy]acetamidolbicyclo[1.1.1]pentan-1-yl)acetamide (Compound 374)

The reaction and purification conditions described in Example 270 substituting 1,3,5-trimethyl-1*H*-pyrazol-4-ol for 1-methyl-3-(trifluoromethyl)-1*H*-pyrazol-5-ol gave the title compound. ¹H NMR (400 MHz, DMSO-*d₆*) *δ* ppm 8.72 (s, 1H), 8.64 (s, 1H), 7.49 (t, J = 8.9 Hz, 1H), 7.07 (dd, J = 11.3, 2.9 Hz, 1H), 6.86 (ddd, J = 8.9, 2.9, 1.2 Hz, 1H), 4.49 (s, 2H), 4.14 (s, 2H), 3.58 (s, 3H), 2.27 (s, 6H), 2.14 (s, 3H), 2.06 (s, 3H); MS (ESI⁺) *m*/*z* 451 (M+H)⁺.

### Example 276: 2-(4-chloro-3-fluorophenoxy)-N-{(2S)-4-[2-(4-chloro-3-fluorophenoxy)acetamido]-2-hydroxybicyclo[2.2.2]octan-1-yl}-N-methylacetamide (Compound 375)

### Example 276A: N-[(3S)-4-amino-3-hydroxybicyclo[2.2.2]octan-1-yl]-2-(4-chloro-3-fluorophenoxy)acetamide trifluoroacetate

The title compound was isolated by chiral preparative SFC of Example 198I as the second peak eluted off the column, followed by reverse phase HPLC purification to give product as a trifluoroacetic acid salt. The preparative SFC (Supercritical Fluid Chromatography) was performed on a Thar 200 preparative SFC (SFC-5) system using a Chiralpak^{®} IC, 300 ×5 0 mm I.D., 10 µm column. The column was at 38 °C, and the backpressure regulator was set to maintain 100 bar. The mobile phase A is CO₂ and B is isopropanol (0.1% ammonium hydroxide). The chromatography was performed isocratically at 45% of mobile phase B at a flow rate of 200 mL/minute. Fraction collection was time triggered with UV monitor wavelength set at 220 nm. Preparative HPLC was performed on a Gilson 281 semi-preparative HPLC system using a Phenomenex^{®} Luna^{®} C18(2) 10 µm 100Å AXIA^{™} column (250 mm × 80 mm) column. A gradient of acetonitrile (A) and 0.075% trifluoroacetic acid in water (B) was used, at a flow rate of 80 mL/minute. A linear gradient was used from about 30% of A to about 100% of A over about 30 minutes. Detection method was UV at wave length of 220 nM and 254 nM. ¹H NMR (400 MHz, methanol-*d*₄) *δ* ppm 7.36 (t, *J*=8.77 Hz, 1H), 6.89 (dd, *J*=10.74, 2.85 Hz, 1H), 6.79 (br d, *J*=9.21 Hz, 1H), 4.43 (s, 2H), 3.94 (br d, *J*=8.33 Hz, 1H), 2.55 (br t, *J*=12.50 Hz, 1H), 2.35 - 1.84 (m, 8H), 1.83 - 1.58 (m, 2H); MS (ESI⁺) *m*/*z* 343.0 (M+H)⁺.

### Example 276B: 2-(4-chloro-3-fluorophenoxy)-N-[(3S)-3-hydroxy-4-(methylamino)bicyclo[2.2.2]octan-1-yl]acetamide

A mixture of Example 276A (0.2 g, 0.438 mmol), formaldehyde (0.049 mL, 0.657 mmol) and acetic acid (0.100 mL, 1.751 mmol) in water (1.0 mL) was treated with zinc (0.057 g, 0.876 mmol), and the reaction was stirred at ambient temperature for 16 hours. Volatiles were removed under reduced pressure, and the residue was dissolved with a 1:1 mixture of methanol/dimethyl sulfoxide and filtered. The filtrate was purified by HPLC (5-70% acetonitrile in 0.1% trifluoroacetic acid/water at 80 mL/minute on a Phenomenex^{®} C18 10 µm column (25 × 80 mm)) to give 136 mg of the title compound as a white glassy solid. ¹H NMR (400 MHz, DMSO-*d*₆) *δ* ppm 7.40 (t, J = 8.9 Hz, 1H), 6.94 (dd, J = 11.3, 2.8 Hz, 1H), 6.75 (ddd, J = 9.0, 2.8, 1.2 Hz, 1H), 4.37 (s, 2H), 3.89 (ddd, J = 8.7, 3.4, 1.7 Hz, 1H), 2.35 (s, 3H), 2.39 - 2.23 (m, 1H), 2.02 - 1.85 (m, 2H), 1.89 - 1.70 (m, 5H), 1.74 - 1.44 (m, 2H); MS (ESI⁺) *m*/*z* 357.2 (M+H)⁺.

### Example 276C: 2-(4-chloro-3-fluorophenoxy)-N-{(2S)-4-[2-(4-chloro-3-fluorophenoxy)acetamidol-2-hydroxybicyclo[2.2.2]octan-1-yl]-N-methylacetamide

A mixture of Example 276B (65 mg, 0.138 mmol), 2-(4-chloro-3-fluorophenoxy)acetic acid (35.3 mg, 0.173 mmol) and *N*-ethyl-*N*-isopropylpropan-2-amine (0.121 mL, 0.690 mmol) in N,N-dimethylformamide(1.5 mL) was treated with 2-(3H-[1,2,3]triazolo[4,5-b]pyridin-3-yl)-1,1,3,3-tetramethylisouronium hexafluorophosphate(V) (79 mg, 0.207 mmol), and the reaction was stirred at ambient temperature for 16 hours. Solvent was removed, and the residue was purified by HPLC (20-100% acetonitrile in 0.1% trifluoroacetic acid/water at 25 mL/minute on a Phenomenex^{®} C18 5 µm column (250 × 21.2 mm)) three times to give 15 mg of the title compound as a white solid. ¹H NMR (400 MHz, DMSO-*d*₆) *δ* ppm 1H NMR (400 MHz, DMSO-d6) d 8.77 - 8.68 (m, 1H), 8.67 (s, 1H), 7.75 (s, 1H), 7.45 (td, J = 8.8, 6.4 Hz, 2H), 7.11 (dd, J = 11.4, 2.9 Hz, 1H), 6.98 (dd, J = 11.4, 2.9 Hz, 1H), 6.85 (ddd, J = 9.0, 2.8, 1.2 Hz, 1H), 6.77 (ddd, J = 8.9, 2.9, 1.2 Hz, 1H), 5.18 (d, J = 8.9 Hz, 1H), 4.92 (d, J = 16.7 Hz, 1H), 4.82 (d, J = 16.7 Hz, 1H), 4.42 (s, 2H), 2.55 - 2.40 (m, 5H), 1.98 - 1.82 (m, 6H), 1.82 (d, J = 6.0 Hz, 2H); MS (ESI⁺) *m*/*z* 543.2 (M+H)⁺.

### Example 277: (2E)-N-{3-[2-(4-chloro-3-fluorophenoxy)acetamido]bicyclo[1.1.1]pentan-1-yl}-3-[5-(trifluoromethoxy)pyridin-2-yl]prop-2-enamide (Compound 376)

### Example 277A: (E)-ethyl 3-(5-(trifluoromethoxy)pyridin-2-yl)acrylate

Dimethoxyethane (5 mL) and water (0.1 mL) were added to a mixture of (E)-ethyl 3-(4,4,5,5-tetramethyl-1,3,2-dioxaborolan-2-yl)acrylate (Frontier, 0.684 g, 3.02 mmol), potassium carbonate (0.87 g, 6.30 mmol), [1,1'-bis(diphenylphosphino)ferrocene]dichloropalladium(II) (0.21 g, 0.25 mmol) and 2-bromo-5-(trifluoromethoxy)pyridine (Ark Pharm, 0.61g, 2.52 mmol) in a microwave tube. The tube was sealed and degassed three times with a nitrogen back flush each time. The tube was then heated in a Biotage^{®} Initiator+ microwave reactor and irradiated at 110 °C for 30 minutes. The seal was opened, and the layers were separated. The organic layer was filtered through a glass microfiber frit and concentrated in vacuo. The residue was purified by preparative HPLC [YMC TriArt^{™} C18 Hybrid 20 µm column, 25 × 150 mm, flow rate 80 mL/minute, 5-100% gradient of acetonitrile in buffer (0.025 M aqueous ammonium bicarbonate, adjusted to pH 10 with ammonium hydroxide)] to give the title compound (0.37 g, 1.42 mmol, 56% yield). MS (ESI⁺) *m*/*z* 262 (M+H)⁺.

### Example 277B: (E)-3-(5-(trifluoromethoxy)pyridin-2-yl)acrylic acid

The reaction and purification conditions described in Example 105B substituting the product of Example 277A for the product of Example 105A gave the title compound. MS (ESI⁺) *m*/*z* 234 (M+H)⁺.

### Example 277C: (2E)-N-{3-[2-(4-chloro-3-fluorophenoxy)acetamido]bicyclo[1.1.1]pentan-1-yl}-3-[5-(trifluoromethoxy)pyridin-2-yl]prop-2-enamide

The reaction and purification conditions described in Example 81, substituting the product of Example 277B for 2-(4-chloro-3-fluorophenoxy)acetic acid and the product of Example 27D for benzyl (4-aminobicyclo[2.1.1]hexan-1-yl)carbamate hydrochloride gave the title compound. ¹H NMR (500 MHz, DMSO-*d*₆) *δ* ppm 8.95 (s, 1H), 8.76 (s, 1H), 8.68 (d, J = 2.8 Hz, 1H), 7.96 - 7.91 (m, 1H), 7.77 - 7.73 (m, 1H), 7.52 - 7.44 (m, 2H), 7.08 (dd, J = 11.4, 2.9 Hz, 1H), 6.98 (d, J = 15.4 Hz, 1H), 6.86 (ddd, J = 8.9, 2.8, 1.2 Hz, 1H), 4.49 (s, 2H), 2.30 (s, 6H); MS (DCI) *m*/*z* 517 (M+NH₄)⁺.

### Example 278: N-{(2S)-4-[2-(4-chloro-3-fluorophenoxy)acetamido]-2-hydroxybicyclo[2.2.2]octan-1-yl}-N-methyl-2-{[6-(trifluoromethyl)pyridin-3-yl]oxy}acetamide (Compound 377)

The title compound was prepared using the methodologies described in Example 276 substituting 2-((6-(trifluoromethyl)pyridin-3-yl)oxy)acetic acid for 2-(4-chloro-3-fluorophenoxy)acetic acid. ¹H NMR (400 MHz, DMSO-*d*₆) *δ* ppm 8.75 (s, 1H), 8.66 (dd, J = 11.9, 5.7 Hz, 1H), 8.48 (d, J = 2.9 Hz, 1H), 7.83 (d, J = 8.7 Hz, 1H), 7.75 (s, 1H), 7.63 (dd, J = 8.7, 2.9 Hz, 1H), 7.44 (t, J = 8.9 Hz, 1H), 6.98 (dd, J = 11.4, 2.9 Hz, 1H), 6.77 (ddd, J = 8.9, 2.9, 1.1 Hz, 1H), 5.20 (d, J = 9.1 Hz, 1H), 5.10 (d, J = 16.7 Hz, 1H), 4.98 (d, J = 16.7 Hz, 1H), 4.42 (s, 2H), 2.56 - 2.47 (m, 1H), 2.44 (s, 3H), 1.86 (m, 9H); MS (ESI⁺) *m*/*z* 560.2 (M+H)⁺.

### Example 279: 2-(4-chloro-3-fluorophenoxy)-N-[3-(2-{[2-(difluoromethyl)pyridin-4-yl]oxy}acetamido)bicyclo[1.1.1]pentan-1-yl]acetamide (Compound 378)

A mixture of Example 28A (65.0 mg, 0.180 mmol), 2-(difluoromethyl)pyridin-4-ol (39.2 mg, 0.27 mmol), potassium carbonate (49.7 mg, 0.36 mmol), and potassium iodide (2.09 mg, 0.013 mmol) in acetone (2.5 mL) was heated at 140 °C in a Biotage^{®} Initiator microwave reactor for 40 minutes. The reaction mixture was filtered, and the filtrate was concentrated. The residue was purified by reverse-phase HPLC (see protocol in Example 112D) to provide the title compound (68.4 mg, 0.145 mmol, 81% yield). ¹H NMR (400 MHz, DMSO-*d*₆) *δ* ppm 8.82 (s, 1H), 8.74 (s, 1H), 8.51 (d, J = 5.7 Hz, 1H), 7.49 (t, J = 8.9 Hz, 1H), 7.25 (d, J = 2.5 Hz, 1H), 7.17 - 6.98 (m, 2.3H), 6.94 - 6.70 (m, 1.8H), 4.64 (s, 2H), 4.48 (s, 2H), 2.27 (s, 6H); MS (ESI⁺) *m*/*z* 470.2 (M+H)⁺.

### Example 280: 2-[(2,2-difluoro-2H-1,3-benzodioxol-5-yl)oxy]-N-{3-[2-(3,4-difluorophenoxy)acetamido]bicyclo[1.1.1]pentan-1-yl}acetamide (Compound 379)

### Example 280A: [(2,2-difluoro-2H-1,3-benzodioxol-5-yl)oxy]acetic acid

The reaction and purification conditions described in Example 11A and Example 11B, substituting the product of Example 159A for 5-hydroxy-3-methylbenzo[d]isoxazole gave the title compound. MS (ESI⁺) *m*/*z* 233 (M+H)⁺.

### Example 280B: tert-butyl (3-{2-[(2,2-difluoro-2H-1,3-benzodioxol-5-yl)oxy]acetamido}bicyclo[1.1.1]pentan-1-yl)carbamate

The reaction and purification conditions described in Example 11C substituting the product of Example 280A for the product of Example 11B, and *tert*-butyl (3-aminobicyclo[1.1.1]pentan-1-yl)carbamate for the product of Example 9B gave the title compound. MS (ESI⁺) *m*/*z* 435 [M+Na]⁺.

### Example 280C: N-(3-aminobicyclo[1.1.1]pentan-1-yl)-2-[(2,2-difluoro-2H-1,3-benzodioxol-5-yl)oxy]acetamide

The reaction and purification conditions described in Example 9D substituting the product of Example 280B for the product of Example 9C gave the title compound. MS (ESI⁺) *m*/*z* 313 (M+H)⁺.

### Example 280D: 2-[(2,2-difluoro-2H-1,3-benzodioxol-5-yl)oxy]-N-{3-[2-(3,4-difluorophenoxy)acetamido]bicyclo[1.1.1]pentan-1-yl)acetamide

The reaction and purification conditions described in Example 81, substituting 2-(3,4-difluorophenoxy)acetic acid (Combi-Blocks) for 2-(4-chloro-3-fluorophenoxy)acetic acid and the product of Example 280C for benzyl (4-aminobicyclo[2.1.1]hexan-1-yl)carbamate hydrochloride gave the title compound. ¹H NMR (400 MHz, DMSO-*d*₆) *δ* ppm 8.70 (s, 2H), 7.42 - 7.29 (m, 2H), 7.14 (d, J = 2.6 Hz, 1H), 7.09 (ddd, J = 12.6, 6.8, 3.0 Hz, 1H), 6.82 - 6.75 (m, 2H), 4.44 (s, 4H), 2.27 (s, 6H); MS (ESI⁺) *m*/*z* 483 (M+H)⁺.

### Example 281: 2-(4-chloro-3-fluorophenoxy)-N-(4-{2-[(2,2-difluoro-2H-1,3-benzodioxol-5-yl)oxy]acetamido}bicyclo[2.1.1]hexan-1-yl)acetamide (Compound 380)

The reaction and purification conditions described in Example 81, substituting the product of Example 280A for 2-(4-chloro-3-fluorophenoxy)acetic acid and the product of Example 88C for benzyl (4-aminobicyclo[2.1.1]hexan-1-yl)carbamate hydrochloride gave the title compound. ¹H NMR (400 MHz, DMSO-*d*₆) *δ* ppm 8.46 (s, 1H), 8.43 (s, 1H), 7.47 (t, J = 8.9 Hz, 1H), 7.30 (d, J = 8.9 Hz, 1H), 7.11 (d, J = 2.5 Hz, 1H), 7.05 (dd, J = 11.4, 2.8 Hz, 1H), 6.83 (ddd, J = 9.0, 2.9, 1.2 Hz, 1H), 6.75 (dd, J = 8.9, 2.6 Hz, 1H), 4.46 (s, 2H), 4.42 (s, 2H), 2.08 - 2.03 (m, 2H), 1.86 - 1.72 (m, 6H); MS (ESI⁺) *m*/*z* 530 (M+NH₄)⁺.

### Example 282: 2-(4-chloro-3-fluorophenoxy)-N-(3-{2-[(1-methyl-1H-pyrazol-3-yl)oxy]acetamidolbicyclo[1.1.1]pentan-1-yl)acetamide (Compound 381)

### Example 282A: tert-butyl 2-((1-methyl-1H-pyrazol-3-yl)oxy)acetate

To a solution of *tert*-butyl 2-bromoacetate (0.90 mL, 6.12 mmol) in acetonitrile (10 mL) were added 1-methyl-1*H*-pyrazol-3-ol (400 mg, 4.08 mmol) and potassium carbonate (1.69 g, 12.2 mmol). The mixture was stirred at 80 °C for 1 hour. The reaction mixture was quenched with 6 N HCl (3 mL) and water (20mL). The layers were separated, and the aqueous layer was extracted with ethyl acetate (3 × 40 mL). The combined organic layers was dried over anhydrous MgSO₄, filtered and concentrated under reduced pressure. The resulting material was purified by flash column chromatography (SiO₂, 0-100% ethyl acetate/heptane) to give the title compound (450 mg, 2.12 mmol, 52% yield). ¹H NMR (500 MHz, DMSO-*d*₆) *δ* ppm 7.45 (d, J = 2.3 Hz, 1H), 5.61 (d, J = 2.3 Hz, 1H), 4.56 (s, 2H), 3.63 (s, 3H), 1.41 (s, 9H); MS (ESI⁺) *m*/*z* 213 (M+H)⁺.

### Example 282B: 2-((1-methyl-1H-pyrazol-3-yl)oxy)acetic acid

To a solution of the product of Example 282A(400mg, 1.89 mmol) in dichloromethane (10 mL) was added trifluoroacetic acid (1.45 mL, 18.9 mmol). The mixture was stirred at ambient temperature for 2 hours. Water was added, and then the mixture was concentrated under reduced pressure to give the title compound (200mg, 1.28 mmol, 68% yield). ¹H NMR (400 MHz, DMSO-*d*₆) *δ* ppm 7.45 (d, J = 2.3 Hz, 1H), 6.66 (s, 1H), 5.63 (d, J = 2.3 Hz, 1H), 4.61 (s, 2H), 3.65 (s, 3H); MS (ESI+) *m*/*z* 157 (M+H)⁺.

### Example 282C: 2-(4-chloro-3-fluorophenoxy)-N-(3-(2-((1-methyl-1H-pyrazol-3-yl)oxy)acetamido)bicyclo[1.1.1]pentan-1-yl)acetamide

To a solution of the product of Example 9B (40 mg, 0.100 mmol) in *N,N-*dimethylformamide (0.80 mL) were added the product of example 282B (17.2 mg, 0.110 mmol), N-[(dimethylamino)-1*H*-1,2,3-triazolo-[4,5-b]pyridin-1-ylmethylene]-*N*-methylmethanaminium hexafluorophosphate *N*-oxide (HATU, 42.0 mg, 0.110 mmol), and *N*,*N*-diisopropylethylamine (0.053 mL, 0.301 mmol) at ambient temperature. The reaction mixture was stirred 1 hour at ambient temperature, and then was purified with preparative HPLC [Waters XBridge^{™} C18 5 µm OBD^{™} column, 30 × 100 mm, flow rate 40 mL/minute, 5-100% gradient of acetonitrile in buffer (0.1 % trifluoroacetic acid)] to give the title compound (32 mg, 0.076 mmol, 75% yield). ¹H NMR (400 MHz, DMSO-*d*₆) *δ* ppm 8.67 (s, 1H), 8.54 (s, 1H), 7.51 - 7.40 (m, 2H), 7.03 (dd, J = 11.4, 2.9 Hz, 1H), 6.81 (ddd, J = 8.9, 2.8, 1.1 Hz, 1H), 5.60 (d, J = 2.3 Hz, 1H), 4.43 (s, 2H), 4.38 (s, 2H), 3.61 (s, 3H), 2.21 (s, 6H); MS (ESI⁺) *m*/*z* 423 (M+H)⁺.

### Example 283: 2-(4-chloro-3-fluorophenoxy)-N-[(3S)-4-(2-{[5-(difluoromethyl)pyrazin-2-yl]oxy}acetamido)-3-hydroxybicyclo[2.2.2]octan-1-yl]acetamide (Compound 382)

### Example 283A: {[5-(difluoromethyl)pyrazin-2-yl]oxy}acetic acid

The title compound was prepared using the methodologies described in Example 206B substituting 5-(difluoromethyl)pyrazin-2-ol for Example 206A. ¹H NMR (400 MHz, DMSO-*d*₆) *δ* ppm 8.17 - 8.08 (m, 2H), 7.01 (*J =* 52.0 Hz, 1H), 4.68 (s, 2H); MS (ESI⁺) *m*/*z* 205.1 (M+H)⁺.

### Example 283B: 2-(4-chloro-3-fluorophenoxy)-N-[(3S)-4-(2-{[5-(difluoromethyl)pyrazin-2-yl]oxy}acetamido)-3-hydroxybicyclo[2.2.2]octan-1-yl]acetamide

The title compound was prepared using the methodologies described in Example 202 substituting Example 276A for Example 198I and substituting Example 283A for 2-((6-(trifluoromethyl)pyridin-3-yl)oxy)acetic acid. ¹H NMR (400 MHz, DMSO-*d*₆) *δ* ppm 8.04 (s, 1H), 7.92 (d, J = 1.5 Hz, 1H), 7.72 (s, 1H), 7.50 - 7.39 (m, 2H), 6.98 - 6.72 (m, 3H), 4.57 (d, J = 15.6 Hz, 1H), 4.49 (d, J = 15.6 Hz, 1H), 4.39 (s, 2H), 4.06 (dd, J = 9.6, 2.8 Hz, 1H), 2.21 (ddd, J = 12.6, 9.4, 2.6 Hz, 1H), 1.98 - 1.62 (m, 9H); MS (ESI⁺) *m*/*z* 529.1 (M+H)⁺.

### Example 284: 2-[(2,2-difluoro-2H-1,3-benzodioxol-5-yl)oxy]-N-[3-(2-{[5-(difluoromethyl)pyrazin-2-yl]oxy}acetamido)bicyclo[1.1.1]pentan-1-yl]acetamide (Compound 383)

A mixture of Example 280C (75 mg, 0.215 mmol), Example 283A (43.9 mg, 0.215 mmol), *N*-ethyl-*N*-isopropylpropan-2-amine (0.188 mL, 1.075 mmol), *N*,*N*-dimethylformamide (1.5 mL), and 2-(3*H*-[1,2,3]triazolo[4,5-*b*]pyridin-3-yl)-1,1,3,3-tetramethylisouronium hexafluorophosphate(V) (123 mg, 0.323 mmol) was stirred overnight. The reaction mixture was concentrated, and the residue was purified by HPLC (10-85% acetonitrile in 0.1% trifluoroacetic acid/water at 25 mL/minute on a Phenomenex^{®} C18 5 µm column (250 × 21.2 mm)) to give 85 mg of the title compound as a white solid. ¹H NMR (400 MHz, DMSO-*d*₆) *δ* ppm 8.93 (s, 1H), 8.68 (s, 1H), 8.05 (s, 1H), 7.99 (d, J = 1.5 Hz, 1H), 7.28 (d, J = 8.8 Hz, 1H), 7.09 (d, J = 2.5 Hz, 1H), 6.82 - 6.66 (m, 2H), 4.52 (s, 2H), 4.39 (s, 2H), 2.20 (s, 6H); MS (ESI⁺) *m*/*z* 499.0 (M+H)⁺.

### Example 285: 2-(4-chloro-3-fluorophenoxy)-N-[3-(2-{[5-(difluoromethyl)pyrazin-2-yl]oxy}acetamido)bicyclo[1.1.1]pentan-1-yl]acetamide (Compound 384)

The title compound was prepared using the methodologies described in Example 284 substituting Example 9B for Example 280C ¹H NMR (400 MHz, DMSO-*d*₆) *δ* ppm 8.94 (s, 1H), 8.70 (s, 1H), 8.05 (s, 1H), 7.98 (t, J = 1.4 Hz, 1H), 7.45 (t, J = 8.9 Hz, 1H), 7.03 (dd, J = 11.3, 2.8 Hz, 1H), 6.82 (s, 1H), 6.85 - 6.77 (m, 1H), 4.52 (s, 2H), 4.43 (s, 2H), 2.20 (s, 6H); MS (ESI⁺) *m*/*z* 471.0 (M+H)⁺.

### Example 286: 2-(4-chloro-3-fluorophenoxy)-N-(3-{2-[(1,3-dimethyl-1H-pyrazol-5-yl)oxy]acetamido}bicyclo[1.1.1]pentan-1-yl)acetamide (Compound 385)

The reaction and purification conditions described in Example 282 substituting 1,3-dimethyl-1*H*-pyrazol-5-ol for 1-methyl-1*H*-pyrazol-3-ol gave the title compound. ¹H NMR (400 MHz, DMSO-*d*₆) *δ* ppm 8.69 (s, 1H), 8.67 (s, 1H), 7.46 (t, J = 8.9 Hz, 1H), 7.03 (dd, J = 11.4, 2.8 Hz, 1H), 6.81 (ddd, J = 9.0, 2.9, 1.2 Hz, 1H), 5.34 (s, 1H), 4.44 (s, 2H), 4.39 (s, 2H), 3.47 (s, 3H), 2.23 (s, 6H), 1.99 (s, 3H); MS (ESI⁺) *m*/*z* 437 (M+H)⁺.

### Example 287: N-[2-({3-[2-(4-chloro-3-fluorophenoxy)acetamido]bicyclo[1.1.1]pentan-1-yl}amino)-2-oxoethyl]-5-(trifluoromethoxy)pyridine-2-carboxamide (Compound 386)

### Example 287A: tert-butyl [2-({3-[2-(4-chloro-3-fluorophenoxy)acetamido]bicyclo[1.1.1]pentan-1-yl)amino)-2-oxoethyl)carbamate

The reaction and purification conditions described in Example 81, substituting *N*-(*tert-*butoxycarbonyl)glycine for 2-(4-chloro-3-fluorophenoxy)acetic acid and the product of Example 27D for benzyl (4-aminobicyclo[2.1.1]hexan-1-yl)carbamate hydrochloride gave the title compound. MS (ESI⁻) *m*/*z* 440 (M-H)⁻.

### Example 287B: 2-amino-N-{3-[2-(4-chloro-3-fluorophenoxy)acetamido]bicyclo[1.1.1]pentan-1-yl}acetamide

The reaction and purification conditions described in Example 83B, substituting the product of Example 287A for the product of Example 83A gave the title compound. MS (ESI⁺) *m*/*z* 342 (M+H)⁺.

### Example 287C: N-[2-({3-[2-(4-chloro-3-fluorophenoxy)acetamido]bicyclo[1.1.1]pentan-1-yl}amino)-2-oxoethyl]-5-(trifluoromethoxy)pyridine-2-carboxamide

The reaction and purification conditions described in Example 81, substituting 5-(trifluoromethoxy)picolinic acid (Ark Pharm) for 2-(4-chloro-3-fluorophenoxy)acetic acid and the product of Example 287B for benzyl (4-aminobicyclo[2.1.1]hexan-1-yl)carbamate hydrochloride gave the title compound. ¹H NMR (400 MHz, DMSO-*d*₆) *δ* ppm 8.83 (t, J = 5.9 Hz, 1H), 8.74 (d, J = 2.7 Hz, 1H), 8.68 (s, 1H), 8.58 (s, 1H), 8.18 - 8.13 (m, 1H), 8.10 - 8.04 (m, 1H), 7.47 (t, J = 8.9 Hz, 1H), 7.05 (dd, J = 11.4, 2.8 Hz, 1H), 6.83 (ddd, J = 9.0, 2.8, 1.2 Hz, 1H), 4.45 (s, 2H), 3.85 (d, J = 6.0 Hz, 2H), 2.22 (s, 6H); MS (ESI⁺) *m*/*z* 531 (M+H)⁺.

### Example 288: N,N'-(2-cyanobicyclo[2.2.2]octane-1,4-diyl)bis[2-(4-chloro-3-fluorophenoxy)acetamide] (Compound 387)

To a solution of Example 245 (0.43 g, 0.708 mmol) in N,N-dimethylformamide (3.0 mL) was added tetrabutylammonium cyanide (0.248 g, 0.920 mmol), and the mixture was stirred at 90 °C for 16 hours. The mixture was cooled to ambient temperature and partition between water and ethyl acetate. The organic layer was washed with brine, dried over magnesium sulfate and filtered. The filtrate was concentrated, and the residue was purified by HPLC (10-95% acetonitrile in 0.1% trifluoroacetic acid/water at 25 mL/minute on a Phenomenex^{®} C18 5 µm column (250 × 21.2 mm)) to give 213 mg of the title compound as a white solid. ¹H NMR (400 MHz, DMSO-*d*₆) *δ* ppm 8.73 (s, 1H), 8.27 (s, 2H), 7.58 (tdd, J = 8.9, 4.1, 2.6 Hz, 2H), 7.18 - 7.07 (m, 2H), 6.92 (ddp, J = 8.3, 4.6, 1.4 Hz, 2H), 5.04 (s, 5H), 4.78 - 4.64 (m, 2H), 4.57 (s, 2H), 4.56 (s, 1H), 4.27 (s, 1H), 2.29 (t, J = 9.2 Hz, 1H), 2.15 (d, J = 12.0 Hz, 1H), 2.13 - 1.94 (m, 2H), 1.92 - 1.70 (m, 6H); MS (ESI⁺) *m*/*z* 554.9 (M+NH₄)⁺.

### Example 289: 2-(4-chloro-3-fluorophenoxy)-N-(3-{2-[4-(difluoromethyl)phenoxylacetamidolbicyclo[1.1.1]pentan-1-yl)acetamide (Compound 388)

The reaction and purification conditions described in Example 27E substituting 2-(4-(difluoromethyl)phenoxy)acetic acid for 2-(4-chloro-3-methoxyphenoxy)acetic acid gave the titled compound (35 mg, 0.075 mmol, 83% yield). ¹H NMR (501 MHz, DMSO-*d*₆) *δ* ppm 8.76 (s, 1H), 8.74 (s, 1H), 7.52 (d, J = 8 Hz, 2H), 7.50 (t, J = 8 Hz, 1H), 7.08 (dd, J = 9, 3 Hz, 1H), 7.06 (d, J = 8 Hz, 2H), 6.96 (t, J = 60 Hz, 1H), 6.85 (br d, J = 8 Hz, 1H), 4.50 (s, 2H), 4.48 (s, 2H), 2.27 (s, 6H); MS (ESI⁺) *m*/*z* 469 (M+H)⁺.

### Example 290: N,N'-(bicyclo[1.1.1]pentane-1,3-diyl)bis{2-[4-(difluoromethyl)phenoxy]acetamide} (Compound 389)

### Example 290A: bicyclo[1.1.1]pentane-1,3-diamine bis(2,2,2-trifluoroacetate)

A mixture of *tert*-butyl (3-aminobicyclo[1.1.1]pentan-1-yl)carbamate (0.026 g, 0.13 mmol) and 2,2,2-trifluoroacetic acid (0.222 g, 1.95 mmol) in dichloromethane (0.3 mL) was stirred at ambient temperature for 3 hours, and then the mixture was concentrated under reduced pressure to give the title compound (42 mg, 99%yield) which was used without purification or characterization.

### Example 290B: methyl 2-(4-(difluoromethyl)phenoxy)acetate

A mixture of 4-(difluoromethyl)phenol (221 mg, 1.53 mmol), methyl 2-bromoacetate (351 mg, 2.30 mmol) and potassium carbonate (486 mg, 3.52 mmol) in acetone (1.5 mL) was stirred at 60 °C for 2 hours. Ethyl acetate (5 mL) was added, and the solution was filtered. The filtrate was concentrated under reduced pressure, and the residue was purified by flash column chromatography on silica gel (40g) eluted with 5-40% ethyl acetate/heptane to give the title compound (97 mg, 29% yield). ¹H NMR (400 MHz, DMSO-*d*₆) *δ* ppm 7.50 (d, J = 8 Hz, 2H), 7.05 (d, J = 8 Hz, 2H), 6.96 (t, J = 56 Hz, 1H), 4.87 (s, 2H), 3.70 (s, 3H).

### Example 290C: 2-(4-(difluoromethyl)phenoxy)acetic acid

To the product of Example 290B (90 mg, 0.416 mmol) in methanol (3 mL) was added 4 N aqueous sodium hydroxide solution (1.77 mL, 7.08 mmol). The mixture was stirred at ambient temperature for 16 hours. The mixture was acidified with 1 N aqueous HCl solution to pH-6. The resulting mixture was extracted with ethyl acetate (2 × 50 mL). The combined organic fractions were dried over anhydrous Na₂SO₄ and concentrated under reduced pressure to give the title compound (70 mg .83% yield). ¹H NMR (400 MHz, DMSO-*d*₆) *δ* ppm 7.45 (d, J = 8 Hz, 2H), 6.92 (d, J = 8 Hz, 2H), 6.91 (t, J = 56 Hz, 1H), 4.26 (s, 2H).

### Example 290D: N,N'-(bicyclo[1.1.1]pentane-1,3-diyl)bis{2-[4-(difluoromethyl)phenoxy]acetamide}

The product of Example 290C (40.4 mg, 0.20 mmol) and *N*-[(dimethylamino)-1*H*-1,2,3-triazolo-[4,5-b]pyridin-1-ylmethylene]-*N*-methylmethanaminium hexafluorophosphate *N*-oxide (HATU, 84 mg, 0.220 mmol) in *N*,*N*-dimethylformamide (1 mL) was stirred at ambient temperature for 5 minutes, then the product of Example 290A (32.6 mg, 0.1 mmol) and *N*-ethyl-*N*-isopropylpropan-2-amine (103 mg, 0.80 mmol) in *N*,*N*-dimethylformamide (0.5 mL) was added. The mixture was stirred at ambient temperature for 0.5 hour and then was purified by preparative HPLC [Waters XBridge^{™} C18 5 µm OBD^{™} column, 50 × 100 mm, flow rate 90 mL/minute, 5-100% gradient of acetonitrile in buffer (0.1 % aqueous trifluoroacetic acid)] to give the title compound (35 mg, 75% yield). ¹H NMR (400 MHz, DMSO-*d*₆) *δ* ppm 8.73 (s, 2H), 7.52 (d, J = 8 Hz, 4H), 7.06 (d, J = 8 Hz, 4H), 6.96 (t, J = 56 Hz, 2H), 4.50 (s, 4H), 2.27 (s, 6H); MS (ESI⁻) *m*/*z* 465 (M-H)⁻.

### Example 291: 1,4-bis[2-(4-chloro-3-fluorophenoxy)acetamido]bicyclo[2.2.2]octane-2-carboxylic acid (Compound 390)

A mixture of Example 288 (40 mg, 0.074 mmol), concentrated hydrochloric acid (1.0 mL, 32.9 mmol) and acetic acid (1.0 mL, 17.47 mmol) was heated in a microwave vial at 110 °C for 0.5 hours. The solution was concentrated under vacuum, and the residue was purified by HPLC (10-95% acetonitrile in 0.1% trifluoroacetic acid/water at 25 mL/minute on a Phenomenex^{®} C18 5 µm column (250 × 21.2 mm)) to give 4.5 mg of the title compound as a white solid. ¹H NMR (400 MHz, DMSO-*d*₆) *δ* ppm 12.30 (s, 1H), 8.14 (s, 1H), 7.94 (s, 1H), 7.43 (dt, J = 11.9, 8.9 Hz, 2H), 6.98 (td, J = 11.5, 2.9 Hz, 2H), 6.78 (dt, J = 9.2, 3.1 Hz, 2H), 4.56 (s, 2H), 4.43 (s, 2H), 2.42 (d, J = 5.4 Hz, 1H), 2.05 - 1.53 (m, 9H); MS (ESI⁺) *m*/*z* 557.0 (M+H)⁺.

### Example 292: N,N'-(bicyclo[2.1.1]hexane-1,4-diyl)bis{2-[(2,2-difluoro-2H-1,3-benzodioxol-5-yl)oxy]acetamide} (Compound 391)

The reaction and purification conditions described in Example 83C substituting the product of Example 280A (2 equivalents) for the product of Example 83B, and *tert*-butyl 4-aminobicyclo[2.1.1]hex-1-ylcarbamate (Enamine) for the product of Example 9A gave the title compound. ¹H NMR (501 MHz, DMSO-*d*₆) *δ* ppm 8.45 (s, 2H), 7.32 (d, J = 8.9 Hz, 2H), 7.14 (d, J = 2.5 Hz, 2H), 6.77 (dd, J = 8.9, 2.5 Hz, 2H), 4.44 (s, 4H), 2.12 - 2.05 (m, 2H), 1.85 - 1.77 (m, 6H); MS (ESI⁺) *m*/*z* 558 (M+NH₄)⁺.

### Example 293: 2-(4-chloro-3-fluorophenoxy)-N-(3-{2-[3-(trifluoromethoxy)phenoxy]acetamido}bicyclo[1.1.1]pentan-1-yl)acetamide (Compound 392)

The reaction and purification conditions described in Example 282 substituting 3-(trifluoromethoxy)phenol for 1-methyl-1*H*-pyrazol-3-ol gave the title compound. ¹H NMR (400 MHz, DMSO-*d*₆) *δ* ppm8.71 (s, 1H), 8.69 (s, 1H), 7.46 (t, J = 8.9 Hz, 1H), 7.39 (t, J = 8.6 Hz, 1H), 7.04 (dd, J = 11.4, 2.9 Hz, 1H), 6.96 (dd, J = 8.2, 2.2 Hz, 1H), 6.92 (dd, J = 5.1, 2.2 Hz, 2H), 6.82 (ddd, J = 8.9, 2.8, 1.2 Hz, 1H), 4.45 (s, 2H), 4.44 (s, 2H), 2.23 (s, 6H); MS (ESI⁺) *m*/*z* 520 (M+NH₄)⁺.

### Example 294: 2-(4-chloro-3-fluorophenoxy)-N-(3-{2-[4-(trifluoromethoxy)phenoxy]acetamido}bicyclo[1.1.1]pentan-1-yl)acetamide (Compound 393)

The reaction and purification conditions described in Example 282 substituting 4-(trifluoromethoxy)phenol for 1-methyl-1*H*-pyrazol-3-ol gave the title compound. ¹H NMR (400 MHz, DMSO-*d*₆) *δ* ppm 8.69 (s, 2H), 7.46 (t, J = 8.9 Hz, 1H), 7.30 - 7.23 (m, 2H), 7.08 - 6.97 (m, 3H), 6.82 (ddd, J = 9.0, 2.9, 1.2 Hz, 1H), 4.44 (s, 2H), 4.43 (s, 2H), 2.23 (s, 6H); MS (ESI⁺) *m*/*z* 520 (M+NH₄)⁺.

### Example 295: 2-(4-chloro-3-fluorophenoxy)-N-[(3S)-4-{2-[(5,6-dimethylpyridin-3-yl)oxy]acetamido}-3-hydroxybicyclo[2.2.2]octan-1-yl]acetamide (Compound 394)

### Example 295A: 2-chloro-N-{(2S)-4-[2-(4-chloro-3-fluorophenoxy)acetamido]-2-hydroxybicyclo[2.2.2]octan-1-yl}acetamide

A mixture of Example 276A (0.50 g, 1.1 mmol), 2-chloroacetic acid (0.114 g, 1.20 mmol), *N*-[(dimethylamino)-1*H*-1,2,3-triazolo-[4,5-*b*]pyridin-1-ylmethylene]-*N-*methylmethanaminium hexafluorophosphate *N*-oxide (HATU, 0.50 g, 1.31 mmol), and triethylamine (0.46 mL, 3.28 mmol) in tetrahydrofuran (10 mL) was stirred for 16 hours. The reaction mixture was treated with water and brine and extracted with ethyl acetate (2×). The combined organic layers were dried over anhydrous MgSO₄, filtered, and concentrated under reduced pressure. The residue was purified on an 80 g silica gel column using the Biotage^{®} Isolera^{™} One flash system eluting with ethyl acetate/heptanes (8:2 to 9:1) to provide the title compound (0.261 g, 0.62 mmol, 57% yield). MS (ESI⁺) *m*/*z* 419.1 (M+H)⁺.

### Example 295B: 2-(4-chloro-3-fluorophenoxy)-N-[(3S)-4-{2-[(5,6-dimethylpyridin-3-yl)oxy]acetamido}-3-hydroxybicyclo[2.2.2]octan-1-yl]acetamide

A mixture of Example 295A (55.0 mg, 0.13 mmol), 5,6-dimethylpyridin-3-ol (24.2 mg, 0.197 mmol), potassium carbonate (36.3 mg, 0.26 mmol), and potassium iodide (1.524 mg, 9.18 µmol) in acetone (2.5 mL) was heated at 140 °C in a Biotage^{®} Initiator microwave reactor for 40 minutes. The reaction mixture was filtered, and the filtrate was concentrated. The residue was purified by reverse-phase HPLC (see protocol in Example 112D) to provide the title compound as a trifluoroacetic acid salt (24.4 mg, 0.048 mmol, 30% yield). ¹H NMR (400 MHz, DMSO-*d*₆) *δ* ppm 8.30 (d, J = 2.8 Hz, 1H), 7.93 (d, J = 2.4 Hz, 1H), 7.57 - 7.36 (m, 3H), 7.02 (dd, J = 11.4, 2.9 Hz, 1H), 6.81 (ddd, J = 9.0, 2.9, 1.2 Hz, 1H), 4.66 (s, 2H), 4.44 (s, 2H), 4.09 (dd, J = 9.6, 3.1 Hz, 1H), 2.53 (s, 3H), 2.36 (s, 3H), 2.31 - 2.22 (m, 1H), 2.01-1.75 (m, 9H); MS (ESI⁺) *m*/*z* 506.2 (M+H)⁺.

### Example 296: 2-(4-chloro-3-fluorophenoxy)-N-[(3S)-4-{2-[(2,6-dimethylpyridin-4-yl)oxy]acetamido}-3-hydroxybicyclo[2.2.2]octan-1-yl]acetamide (Compound 395)

The reaction described in Example 295B substituting 2,6-dimethylpyridin-4-ol for 5,6-dimethylpyridin-3-ol gave the title compound. ¹H NMR (400 MHz, DMSO-*d*₆) *δ* ppm 7.55 (d, J = 6.3 Hz, 2H), 7.48 (t, J = 8.9 Hz, 1H), 7.24 (s, 2H), 7.02 (dd, J = 11.4, 2.9 Hz, 1H), 6.80 (ddd, J = 8.9, 2.9, 1.2 Hz, 1H), 4.80 (s, 2H), 4.44 (s, 2H), 4.12 (dd, J = 9.7, 3.0 Hz, 1H), 2.58 (s, 6H), 2.26 (ddd, J = 12.5, 9.4, 2.4 Hz, 1H), 2.06 - 1.67 (m, 9H); MS (ESI⁺) *m*/*z* 506.2 (M+H)⁺.

### Example 297: 2-(4-chloro-3-fluorophenoxy)-N-(3-{2-[3-(difluoromethoxy)phenoxy]acetamido}bicyclo[1.1.1]pentan-1-yl)acetamide (Compound 396)

The reaction and purification conditions described in Example 282 substituting 3-(difluoromethoxy)phenol for 1-methyl-1*H*-pyrazol-3-ol gave the title compound. ¹H NMR (400 MHz, DMSO-*d*₆) *δ* ppm 8.71 - 8.65 (m, 2H), 7.46 (t, J = 8.9 Hz, 1H), 7.34 - 7.27 (m, 1H), 7.19 (t, J = 74.1 Hz, 1H), 7.04 (dd, J = 11.4, 2.9 Hz, 1H), 6.85 - 6.77 (m, 2H), 6.77 - 6.71 (m, 2H), 4.44 (s, 2H), 4.42 (s, 2H), 2.24 (s, 6H); MS (ESI⁺) *m*/*z* 502 (M+NH₄)⁺.

### Example 298: 2-(4-chloro-3-fluorophenoxy)-N-(3-{2-[4-(difluoromethoxy)phenoxy]acetamido}bicyclo[1.1.1]pentan-1-yl)acetamide (Compound 397)

The reaction and purification conditions described in Example 282 substituting 4-(difluoromethoxy)phenol for 1-methyl-1*H*-pyrazol-3-ol gave the title compound. ¹H NMR (400 MHz, DMSO-*d₆*) *δ* ppm 8.68 (s, 1H), 8.66 (s, 1H), 7.46 (t, J = 8.9 Hz, 1H), 7.25 - 6.86 (m, 6H), 6.84 - 6.79 (m, 1H), 4.44 (s, 2H), 4.39 (s, 2H), 2.23 (s, 6H); MS (ESI⁺) *m*/*z* 502 (M+NH₄)⁺.

### Example 299: 2-(4-chloro-3-fluorophenoxy)-N-[3-(2-phenoxyacetamido)bicyclo[1.1.1]pentan-1-yl]acetamide (Compound 398)

To a solution of 27D in DMF (0.8 mL) were added 2-phenoxyacetic acid (17.6 mg, 0.116 mmol), N-[(dimethylamino)-1*H*-1,2,3-triazolo-[4,5-*b*]pyridin-1-ylmethylene]-*N-*methylmethanaminium hexafluorophosphate *N*-oxide (HATU, 44.1 mg, 0.116 mmol), and *N*,*N-*diisopropylethylamine (0.055 mL, 0.316 mmol) at ambient temperature. The reaction mixture was stirred for 16 hours and then water was added. The resulting solids were isolated via filtration to give the title compound (18 mg, 0.043 mmol, 41% yield). ¹H NMR (400 MHz, DMSO-*d*₆) *δ* ppm 8.68 (s, 1H), 8.65 (s, 1H), 7.46 (t, J = 8.8 Hz, 1H), 7.27 (t, J = 7.9 Hz, 2H), 7.04 (dd, J = 11.3, 2.9 Hz, 1H), 6.96 - 6.88 (m, 3H), 6.85 - 6.78 (m, 1H), 4.44 (s, 2H), 4.38 (s, 2H), 2.24 (s, 6H); MS (ESI⁺) *m*/*z* 436 (M+NH₄)⁺.

### Example 300: 2-(4-chloro-3-fluorophenoxy)-N-[3-(2-{[6-(difluoromethyl)pyridin-3-yl]oxy}acetamido)bicyclo[1.1.1]pentan-1-yl]acetamide (Compound 399)

The reaction and purification conditions described in Example 282 substituting 6-(difluoromethyl)pyridin-3-ol for 1-methyl-1H-pyrazol-3-ol gave the title compound. ¹H NMR (400 MHz, DMSO-*d*₆) *δ* ppm 8.76 (s, 1H), 8.69 (s, 1H), 8.35 (d, J = 2.8 Hz, 1H), 7.62 (d, J = 8.7 Hz, 1H), 7.50 - 7.42 (m, 2H), 7.03 (dd, J = 11.4, 2.8 Hz, 1H), 6.86 (t, J = 55.2 Hz, 1H), 6.82 (ddd, J = 8.9, 2.8, 1.2 Hz, 1H), 4.58 (s, 2H), 4.44 (s, 2H), 2.23 (s, 6H); ¹⁹F NMR (376 MHz, DMSO-*d*₆) *δ* ppm -113.20, -114.12; MS (ESI⁺) *m*/*z* 436 (M+NH₄)⁺.

### Example 301: N,N'-(bicyclo[1.1.1]pentane-1,3-diyl)bis(2-{[6-(trifluoromethyl)pyridin-3-yl]oxy}acetamide) (Compound 400)

### Example 301A: tert-butyl 2-((6-(trifluoromethyl)pyridin-3-yl)oxy)acetate

A mixture of 6-(trifluoromethyl)pyridin-3-ol (Combi-Blocks, 10 g, 60.1 mmol), potassium carbonate (16.6 g, 120 mmol) and *tert*-butyl bromoacetate (9.25 mL, 63.1 mmol) in *N*,*N*-dimethylformamide (100 mL) was warmed to 65 °C and was allowed to stir for 16 hours. The mixture was cooled to ambient temperature and quenched with saturated, aqueous NaHCO₃ (40 mL) and diluted with ethyl acetate (40 mL) and water (20 mL). The layers were separated, and the aqueous layer was extracted with ethyl acetate (3 × 15 mL). The combined organic layers were dried over anhydrous sodium sulfate and concentrated under reduced pressure. The resulting residue was purified via column chromatography (SiO₂, 15-25% ethyl acetate/heptanes) to give the title compound (16.2 g, 58.4 mmol, 97% yield). MS (ESI⁺) *m*/*z* 278 (M+H)⁺.

### Example 301B: 2-((6-(trifluoromethyl)pyridin-3-yl)oxy)acetic acid

To a solution of the product of Example 301A (16.2 g, 58.4 mmol) in dichloromethane (100 mL) at ambient temperature was added trifluoroacetic acid (45.0 mL, 584 mmol). This mixture was allowed to stir at ambient temperature for 4 hours and then was concentrated under reduced pressure and azeotroped with toluene to give solids which were re-precipitated from ethyl acetate/heptanes to give the title compound (12.3 g, 55.4 mmol, 95% yield). MS (DCI) *m*/*z* 239 (M+NH₄)⁺.

### Example 301C: tert-butyl [3-(2-{[6-(trifluoromethyl)pyridin-3-yl]oxy)acetamido)bicyclo[1.1.1]pentan-1-yl]carbamate

The reaction and purification conditions described in Example 81, substituting the product of Example 301B for 2-(4-chloro-3-fluorophenoxy)acetic acid and *tert*-butyl (3-aminobicyclo[1.1.1]pentan-1-yl)carbamate (Pharmablock) for benzyl (4-aminobicyclo[2.1.1]hexan-1-yl)carbamate hydrochloride gave the title compound. MS (ESI⁺) *m*/*z* 402 (M+H)⁺.

### Example 301D: N,N'-(bicyclo[1.1.1]pentane-1,3-diyl)bis(2-{[6-(trifluoromethyl)pyridin-3-yl]oxy}acetamide)

The reaction and purification conditions described in Example 83C, substituting the product of Example 301B for the product of Example 83B, and the product of Example 301C for the product of Example 9A gave the title compound. ¹H NMR (501 MHz, DMSO-*d*₆) *δ* ppm 8.82 (s, 2H), 8.46 (d, J = 2.8 Hz, 2H), 7.87 (d, J = 8.7 Hz, 2H), 7.57 (dd, J = 8.8, 2.9 Hz, 2H), 4.66 (s, 4H), 2.28 (s, 6H); MS (ESI⁺) *m*/*z* 505 (M+H)⁺.

### Example 302: 2-(3,4-dichlorophenoxy)-N-(3-{2-[(pyridin-2-yl)oxy]acetamidolbicyclo[1.1.1]pentan-1-yl)acetamide (Compound 401)

The title compound was prepared using the methodologies described above. ¹H NMR (400 MHz, DMSO-*d*₆) *δ* ppm 8.70 (s, 1H), 8.61 (s, 1H), 8.14 - 8.09 (m, 1H), 7.73 (ddd, J = 8.9, 7.2, 2.0 Hz, 1H), 7.54 (d, J = 8.9 Hz, 1H), 7.26 (d, J = 2.9 Hz, 1H), 7.02 - 6.96 (m, 2H), 6.89 (d, J = 8.3 Hz, 1H), 4.66 (s, 2H), 4.48 (s, 2H), 2.24 (s, 6H); MS (ESI⁺) *m*/*z* 436 (M+H)⁺.

### Example 303: 2-(4-chloro-3-fluorophenoxy)-N-(3-{2-[(pyridin-2-yl)oxy]acetamido}bicyclo[1.1.1]pentan-1-yl)acetamide (Compound 402)

The title compound was prepared using the methodologies described above. ¹H NMR (400 MHz, DMSO-*d*₆) *δ* ppm 8.70 (s, 1H), 8.61 (s, 1H), 8.12 (ddd, J = 5.0, 2.1, 0.8 Hz, 1H), 7.73 (ddd, J = 8.4, 7.1, 2.0 Hz, 1H), 7.49 (t, J = 8.9 Hz, 1H), 7.07 (dd, J = 11.4, 2.8 Hz, 1H), 7.00 (ddd, J = 7.1, 5.0, 0.9 Hz, 1H), 6.89 (dt, J = 8.2. 0.9 Hz, 1H), 6.85 (ddd, J = 8.9, 2.8, 1.2 Hz, 1H), 4.66 (s, 2H), 4.47 (s, 2H), 2.24 (s, 6H); MS (ESI⁺) *m*/*z* 420 (M+H)⁺.

### Example 304: 2-(4-chloro-3-fluorophenoxy)-N-(4-{2-[(pyridin-3-yl)oxy]acetamido}bicyclo[2.1.1]hexan-1-yl)acetamide (Compound 403)

The title compound was prepared using the methodologies described above. ¹H NMR (400 MHz, DMSO-*d*₆) *δ* ppm 8.50 (s, 1H), 8.48 (s, 1H), 8.31 (dd, J = 2.8, 1.0 Hz, 1H), 8.19 (dd, J = 4.0, 1.8 Hz, 1H), 7.49 (t, J = 8.9 Hz, 1H), 7.38 - 7.30 (m, 2H), 7.07 (dd, J = 11.4, 2.9 Hz, 1H), 6.85 (ddd, J = 9.1, 2.9, 1.2 Hz, 1H), 4.52 (s, 2H), 4.48 (s, 2H), 2.11 - 2.04 (m, 2H), 1.84 - 1.77 (m, 6H); MS (ESI⁺) *m*/*z* 434 (M+H)⁺.

### Example 305: 2-(4-chloro-3-fluorophenoxy)-N-(4-{2-[(pyridin-2-yl)oxy]acetamido}bicyclo[2.1.1]hexan-1-yl)acetamide (Compound 404)

The title compound was prepared using the methodologies described above. ¹H NMR (400 MHz, DMSO-*d*₆) *δ* ppm 8.46 (s, 1H), 8.37 (s, 1H), 8.12 (ddd, J = 5.0, 2.0, 0.8 Hz, 1H), 7.73 (ddd, J = 8.4, 7.1, 2.0 Hz, 1H), 7.49 (t, J = 8.8 Hz, 1H), 7.06 (dd, J = 11.4, 2.9 Hz, 1H), 6.99 (ddd, J = 7.1, 5.0, 1.0 Hz, 1H), 6.89 (dt, J = 8.4. 0.9 Hz, 1H), 6.85 (ddd, J = 9.0, 2.9, 1.2 Hz, 1H), 4.66 (s, 2H), 4.47 (s, 2H), 2.09 - 2.03 (m, 2H), 1.85 - 1.72 (m, 6H); MS (ESI⁺) *m*/*z* 434 (M+H)⁺.

### Example 306: 2-(3,4-dichlorophenoxy)-N-(3-{2-[(pyridin-3-yl)oxy]acetamidolbicyclo[1.1.1]pentan-1-yl)acetamide (Compound 405)

The title compound was prepared using the methodologies described above. ¹H NMR (400 MHz, DMSO-*d*₆) *δ* ppm 8.74 (s, 1H), 8.71 (s, 1H), 8.31 (dd, J = 2.8, 1.0 Hz, 1H), 8.19 (dd, J = 4.2, 1.8 Hz, 1H), 7.55 (d, J = 9.0 Hz, 1H), 7.39 - 7.30 (m, 2H), 7.26 (d, J = 2.9 Hz, 1H), 6.99 (dd, J = 8.9, 2.9 Hz, 1H), 4.52 (s, 2H), 4.49 (s, 2H), 2.27 (s, 6H); MS (ESI⁺) *m*/*z* 436 (M+H)⁺.

### Example 307: 2-(4-chloro-3-fluorophenoxy)-N-{3-[2-(2,4,6-trifluorophenoxy)acetamido]bicyclo[1.1.1]pentan-1-yl}acetamide (Compound 406)

The reaction and purification conditions described in Example 270 substituting 2,4,6-trifluorophenol for 1-methyl-3-(trifluoromethyl)-1*H*-pyrazol-5-ol gave the title compound. ¹H NMR (501 MHz, DMSO-*d*₆) *δ* ppm 8.70 (s, 1H), 8.68 (s, 1H), 7.47 (t, J = 8.9 Hz, 1H), 7.23 (td, J = 9.0, 0.9 Hz, 2H), 7.05 (dd, J = 11.4, 2.9 Hz, 1H), 6.83 (ddd, J = 9.0, 2.9, 1.2 Hz, 1H), 4.46 (s, 2H), 4.44 (s, 2H), 2.23 (s, 6H); MS (ESI⁺) *m*/*z* 473 (M+H)⁺.

### Example 308: 2-(4-chloro-3-fluorophenoxy)-N-(3-{2-[4-(2-hydroxypropan-2-yl)phenoxy]acetamido}bicyclo[1.1.1]pentan-1-yl)acetamide (Compound 407)

The reaction and purification conditions described in Example 270 substituting 4-(2-hydroxypropan-2-yl)phenol for 1-methyl-3-(trifluoromethyl)-1*H*-pyrazol-5-ol gave the title compound. ¹H NMR (501 MHz, DMSO-*d*₆) *δ* ppm 8.70 (s, 1H), 8.64 (s, 1H), 7.47 (t, J = 8.9 Hz, 1H), 7.38 - 7.32 (m, 2H), 7.05 (dd, J = 11.4, 2.8 Hz, 1H), 6.87 - 6.81 (m, 3H), 4.46 (s, 2H), 4.37 (s, 2H), 2.25 (s, 6H), 1.37 (s, 6H); MS (ESI⁺) *m*/*z* 458 (M-OH)⁺.

### Example 309: 2-(4-chloro-3-fluorophenoxy)-N-{3-[2-(4-fluorophenoxy)acetamido]bicyclo[1.1.1]pentan-1-yl}acetamide (Compound 408)

The reaction and purification conditions described in Example 299 substituting 2-(4-fluorophenoxy)acetic acid for 2-phenoxyacetic acid gave the title compound. ¹H NMR (400 MHz, DMSO-*d*₆) *δ* ppm 8.67 (s, 1H), 8.64 (s, 1H), 7.46 (t, J = 8.9 Hz, 1H), 7.13 - 7.06 (m, 2H), 7.04 (dd, J = 11.4, 2.8 Hz, 1H), 6.96 - 6.90 (m, 2H), 6.82 (ddd, J = 8.9, 2.8, 1.2 Hz, 1H), 4.44 (s, 2H), 4.37 (s, 2H), 2.23 (s, 6H); MS (ESI⁺) *m*/*z* 477 (M+NH₄)⁺.

### Example 310: 2-(4-chloro-3-fluorophenoxy)-N-{3-[2-(2,4-difluorophenoxy)acetamido]bicyclo[1.1.1]pentan-1-yl}acetamide (Compound 409)

The reaction and purification conditions described in Example 299 substituting 2-(2,4-difluorophenoxy)acetic acid for 2-phenoxyacetic acid gave the title compound. ¹H NMR (400 MHz, DMSO-*d*₆) *δ* ppm 8.67 (s, 1H), 8.65 (s, 1H), 7.46 (t, J = 8.9 Hz, 1H), 7.26 (ddd, J = 11.7, 8.8, 3.0 Hz, 1H), 7.11 - 7.01 (m, 2H), 6.97 (dddd, J = 9.2, 8.2, 3.0, 1.6 Hz, 1H), 6.81 (ddd, J = 8.9, 2.9, 1.2 Hz, 1H), 4.47 (s, 2H), 4.44 (s, 2H). 2.22 (s, 6H); MS (ESI⁺) *m*/*z* 495 (M+NH₄)⁺.

### Example 311: 2-(4-chloro-3-fluorophenoxy)-N-{3-[2-(3-fluorophenoxy)acetamido]bicyclo[1.1.1]pentan-1-yl}acetamide (Compound 410)

The reaction and purification conditions described in Example 299 substituting 2-(3-fluorophenoxy)acetic acid for 2-phenoxyacetic acid gave the title compound. ¹H NMR (501 MHz, DMSO-*d*₆) *δ* ppm 8.70 (s, 1H), 8.68 (s, 1H), 7.47 (t, J = 8.9 Hz, 1H), 7.34 - 7.28 (m, 1H), 7.05 (dd, J = 11.4, 2.9 Hz, 1H), 6.85 - 6.75 (m, 4H), 4.46 (s, 2H), 4.43 (s, 2H), 2.25 (s, 6H); MS (ESI⁺) *m*/*z* 478 (M+CH₃CN)⁺.

### Example 312: 2-(4-chloro-3-fluorophenoxy)-N-{3-[2-(2-fluorophenoxy)acetamido]bicyclo[1.1.1]pentan-1-yl}acetamide (Compound 411)

The reaction and purification conditions described in Example 299 substituting 2-(2-fluorophenoxy)acetic acid for 2-phenoxyacetic acid gave the title compound. ¹H NMR (501 MHz, DMSO-*d*₆) *δ* ppm 8.69 (s, 1H), 8.67 (s, 1H), 7.47 (t, J = 8.9 Hz, 1H), 7.20 (ddd, J = 11.8, 8.1, 1.6 Hz, 1H), 7.10 (dddd, J = 8.2, 7.4, 1.6, 0.9 Hz, 1H), 7.07 - 7.00 (m, 2H), 6.95 (dddd, J = 8.1, 7.4, 4.6, 1.7 Hz, 1H), 6.83 (ddd, J = 9.0, 2.9, 1.2 Hz, 1H), 4.50 (s, 2H), 4.46 (s, 2H), 2.24 (s, 6H); MS (ESI⁺) *m*/*z* 478 (M+CH₃CN)⁺.

### Example 313: 2-(4-chloro-3-fluorophenoxy)-N-{3-[2-(4-chloro-2-methoxyphenoxy)acetamido]bicyclo[1.1.1]pentan-1-yl}acetamide (Compound 412)

The reaction and purification conditions described in Example 299 substituting 2-(4-chloro-2-methoxyphenoxy)acetic acid for 2-phenoxyacetic acid gave the title compound. ¹H NMR (501 MHz, DMSO-*d*₆) *δ* ppm 8.69 (s, 1H), 8.66 (s, 1H), 7.47 (t, J = 8.9 Hz, 1H), 7.34 (d, J = 2.7 Hz, 1H), 7.23 (dd, J = 8.7, 2.8 Hz, 1H), 7.05 (dd, J = 11.4, 2.8 Hz, 1H), 6.87 (d, J = 8.7 Hz, 1H), 6.83 (ddd, J = 9.0, 2.9, 1.2 Hz, 1H), 4.52 (s, 3H), 4.48 (s, 2H), 4.45 (s, 2H), 2.23 (s, 6H); MS (ESI⁺) *m*/*z* 484 (M+H)⁺.

### Example 314: 2-(4-chloro-3-fluorophenoxy)-N-(3-{2-[4-(1,1,1-trifluoro-2-hydroxypropan-2-yl)phenoxy]acetamido}bicyclo[1.1.1]pentan-1-yl)acetamide (Compound 413)

The reaction and purification conditions described in Example 270 substituting 4-(1,1,1-trifluoro-2-hydroxypropan-2-yl)phenol for 1-methyl-3-(trifluoromethyl)-1*H*-pyrazol-5-ol gave the title compound. ¹H NMR (400 MHz, DMSO-*d*₆) *δ* ppm 8.68 (s, 1H), 8.66 (s, 1H), 7.50 - 7.42 (m, 3H), 7.03 (dd, J = 11.4, 2.8 Hz, 1H), 6.95 - 6.88 (m, 2H), 6.82 (ddd, J = 9.0, 2.9, 1.2 Hz, 1H), 6.44 (s, 1H), 4.44 (s, 2H), 4.41 (s, 2H), 2.24 (s, 6H), 1.62 (s, 3H); MS (ESI⁺) *m*/*z* 530 (M+H)⁺.

### Example 315: 2-(4-chloro-3-fluorophenoxy)-N-{3-[2-(2,4,5-trifluorophenoxy)acetamido]bicyclo[1.1.1]pentan-1-yl}acetamide (Compound 414)

The reaction and purification conditions described in Example 270 substituting 2,4,5-trifluorophenol for 1-methyl-3-(trifluoromethyl)-1*H*-pyrazol-5-ol gave the title compound. ¹H NMR (400 MHz, DMSO-*d*₆) *δ* ppm 8.68 (s, 1H), 8.67 (s, 1H), 7.57 (td, J = 10.9, 7.7 Hz, 1H), 7.45 (t, J = 8.9 Hz, 1H), 7.29 (dt, J = 12.2, 7.9 Hz, 1H), 7.03 (dd, J = 11.4, 2.9 Hz, 1H), 6.87 - 6.76 (m, 1H), 4.52 (s, 2H), 4.44 (s, 2H), 2.22 (s, 6H); MS (ESI⁺) *m*/*z* 472 (M+H)⁺.

### Example 316: 2-(4-chloro-3-fluorophenoxy)-N-(3-{2-[3-fluoro-4-(trifluoromethoxy)phenoxy]acetamido}bicyclo[1.1.1]pentan-1-yl)acetamide (Compound 415)

The reaction and purification conditions described in Example 270 substituting 3-fluoro-4-(trifluoromethoxy)phenol for 1-methyl-3-(trifluoromethyl)-1*H*-pyrazol-5-ol gave the title compound. ¹H NMR (400 MHz, DMSO-*d*₆) *δ* ppm 8.71 (s, 1H), 8.70 (s, 1H), 7.50 - 7.45 (m, 2H), 7.11 (dd, J = 12.3, 2.9 Hz, 1H), 7.05 (dd, J = 11.3, 2.9 Hz, 1H), 6.87 (ddd, J = 9.2, 3.0, 1.5 Hz, 1H), 6.83 (ddd, J = 8.9, 2.8, 1.2 Hz, 1H), 4.49 (s, 2H), 4.46 (s, 2H), 2.25 (s, 6H); MS (ESI⁺) *m*/*z* 520 (M+H)⁺.

### Example 317: 2-(4-chloro-3-fluorophenoxy)-N-{3-[2-(3,4,5-trifluorophenoxy)acetamido]bicyclo[1.1.1]pentan-1-yl}acetamide (Compound 416)

The reaction and purification conditions described in Example 299 substituting 2-(3,4,5-trifluorophenoxy)acetic acid for 2-phenoxyacetic acid gave the title compound. ¹H NMR (400 MHz, DMSO-*d*₆) *δ* ppm 8.69 (s, 1H), 8.67 (s, 1H), 7.46 (t, J = 8.9 Hz, 1H), 7.03 (dd, J = 11.4, 2.8 Hz, 1H), 7.00 - 6.90 (m, 2H), 6.82 (ddd, J = 9.0, 2.9, 1.2 Hz, 1H), 4.44 (s, 2H), 4.43 (s, 2H), 2.23 (s, 6H); MS (ESI⁺) *m*/*z* 472 (M+H)⁺.

### Example 318: (2S)-1,4-bis[2-(4-chloro-3-fluorophenoxy)acetamido]bicyclo[2.2.2]octane-2-carboxylic acid (Compound 417)

### Example 318A: N-[(3R)-4-amino-3-hydroxybicyclo[2.2.2]octan-1-yl]-2-(4-chloro-3-fluorophenoxy)acetamide trifluoroacetate

The title compound was isolated by chiral preparative SFC of Example 198I as the first peak eluted off the column, followed by reverse phase HPLC to give the product as a trifluoroacetic acid salt using the methodologies described in Example 276. ¹H NMR (400 MHz, methanol-*d*₄) *δ* ppm 7.36 (t, *J*=8.77 Hz, 1H), 6.89 (dd, *J*=10.74, 2.85 Hz, 1H), 6.83 -6.74 (m, 1H),, 4.43 (s, 2H), 3.94 (br d, *J*=8.33 Hz, 1H), 2.55 (br t, *J*=12.50 Hz, 1H), 2.32 - 1.86 (m, 8H), 1.82 - 1.58 (m, 2H); MS (ESI⁺) *m*/*z* 343.0 (M+H)⁺.

### Example 318B: N,N'-[(2R)-2-hydroxybicyclo[2.2.2]octane-1,4-diyl]bis[2-(4-chloro-3-fluorophenoxy)acetamide]

The title compound was prepared using the methodologies described in Example 198J substituting Example 318A for Example 198H. ¹H NMR (400 MHz, DMSO-*d*₆) *δ* ppm 7.57 - 7.36 (m, 3H), 7.26 (s, 1H), 7.04 (td, *J =* 11.9, 2.8 Hz, 2H), 6.82 (td, *J =* 8.5, 2.8 Hz, 2H), 5.07 (d, *J* = 4.3 Hz, 1H), 4.45 (d, *J =* 13.2 Hz, 4H), 4.04 (dt, *J =* 8.9, 3.8 Hz, 1H), 2.27 (t, *J =* 11.2 Hz, 1H), 2.06 (dt, *J =* 11.3, 7.1 Hz, 1H), 1.93 (d, *J =* 10.8 Hz, 2H), 1.89 - 1.73 (m, 6H); MS (ESI⁺) *m*/*z* 529.1 (M+H)⁺.

### Example 318C: (2R)-1,4-bis[2-(4-chloro-3-fluorophenoxy)acetamido]bicyclo[2.2.2]octan-2-yl methanesulfonate

The title compound was prepared using the methodologies described in Example 245 substituting Example 318B for Example 198K. ¹H NMR (400 MHz, DMSO-*d*₆) *δ* ppm 7.95 (s, 1H), 7.69 (d, *J =* 4.1 Hz, 2H), 7.47 (td, *J =* 8.9, 4.0 Hz, 2H), 7.03 (dd, *J =* 11.4, 2.8 Hz, 2H), 6.82 (ddt, *J =* 9.0, 2.9, 1.4 Hz, 2H), 5.37 (dd, *J =* 9.4, 2.0 Hz, 1H), 4.54 - 4.41 (m, 4H), 3.11 (s, 3H), 2.19 (dt, *J =* 14.6, 2.3 Hz, 1H), 2.09 - 1.69 (m, 9H); MS (ESI⁺) *m*/*z* 343.0 (M+H)⁺.

### Example 318D: N,N'-[(2S)-2-cyanobicyclo[2.2.2]octane-1,4-diyl]bis[2-(4-chloro-3-fluorophenoxy)acetamide]

The title compound was prepared using the methodologies described in Example 288 substituting Example 318C for Example 245. ¹H NMR (400 MHz, DMSO-*d*₆) *δ* ppm 8.60 (d, *J* = 1.7 Hz, 1H), 8.05 (s, 1H), 7.54 - 7.40 (m, 2H), 7.11 - 6.97 (m, 2H), 6.83 (dddd, *J =* 9.0, 6.3, 2.9, 1.4 Hz, 2H), 4.56 - 4.42 (m, 4H), 2.26 - 1.81 (m, 5H), 1.84 - 1.61 (m, 5H); MS (ESI⁺) *m*/*z* 554.9 (M+NH₄)⁺.

### Example 318E: (S)-1,4-bis(2-(4-chloro-3-fluorophenoxy)acetamido)bicyclo[2.2.2]octane-2-carboxylic acid

The title compound was prepared using the methodologies described in Example 291 substituting Example 318D for Example 288. ¹H NMR (400 MHz, DMSO-*d*₆) *δ* ppm 12.43 (s, 1H), 8.28 (d, J = 4.6 Hz, 1H), 8.07 (s, 1H), 7.62 - 7.49 (m, 2H), 7.10 (ddd, J = 19.1, 11.2, 2.8 Hz, 2H), 6.90 (td, J = 10.8, 10.0, 2.7 Hz, 2H), 4.69 (s, 1H), 4.63 (d, J = 3.5 Hz, 1H), 4.55 (d, J = 7.3 Hz, 2H), 2.68 (dd, J = 14.0, 5.2 Hz, 1H), 2.55 (d, J = 5.5 Hz, 1H), 2.48 (d, J = 5.2 Hz, 1H), 2.19 - 1.85 (m, 7H); MS (ESI⁺) *m*/*z* 557.0 (M+H)⁺.

### Example 319: (2R)-2-(4-chloro-3-fluorophenoxy)-N-{3-[2-(4-chloro-3-fluorophenoxy)acetamido]bicyclo[1.1.1]pentan-1-yl}-3-hydroxypropanamide (Compound 418)

### Example 319A: (S)-methyl 3-(benzyloxy)-2-hydroxypropanoate

To a solution of (S)-3-(benzyloxy)-2-hydroxypropanoic acid (1.66 g, 8.46 mmol) in methanol (42.3 mL) were added concentrated sulfuric acid (2.5 mL, 46.5 mmol) and trimethylorthoformate (4.2 mL, 38.1 mmol). The reaction mixture was stirred at reflux for 16 hours and then was cooled to ambient temperature and filtered. The filtrate was concentrated under reduced pressure. The residue was suspended in a small volume of water and neutralized with half-saturated sodium bicarbonate. The product was extracted into ethyl acetate (3 × 50 mL), and the combined organic extracts were dried over anhydrous MgSO₄ and concentrated under reduced pressure. The residue was purified via flash column chromatography (SiO₂, 0-30% then 30%-100% ethyl acetate in heptane) to give the title compound (1.2 g, 5.71 mmol, 68% yield). ¹H NMR (400 MHz, DMSO-*d*₆) *δ* ppm 7.38 - 7.19 (m, 6H), 5.56 (d, J = 6.2 Hz, 1H), 4.57 - 4.38 (m, 3H), 4.23 (dt, J = 6.3, 4.7 Hz, 1H), 3.61 (s, 4H), 3.58 (d, J = 4.7 Hz, 3H); MS (ESI⁺) *m*/*z* 228 (M+NH₄)⁺.

### Example 319B: (R)-methyl 3-(benzyloxy)-2-(4-chloro-3-fluorophenoxy)propanoate

To a solution of triphenylphosphine (1.5 g, 5.71 mmol) in tetrahydrofuran (7 mL) at 0 °C, was added di-tert-butyl (E)-diazene-1,2-dicarboxylate (2.15 mL, 10.3 mmol). The reaction mixture was stirred for 5 minutes, and then a solution of 4-chloro-3-fluorophenol (0.84 g, 5.71 mmol), Example 319A (1.2 g, 5.71 mmol) and triethylamine (1.43 mL, 10.3 mmol) in tetrahydrofuran (7 mL) was added. The reaction mixture was allowed to warm to ambient temperature and was stirred for 48 hours. The reaction mixture was concentrated under reduced pressure and purified by flash chromatography (SiO₂, 5-100% ethyl acetate in hexanes). The material was carried on without characterization.

### Example 319C: (R)-3-(benzyloxy)-2-(4-chloro-3-fluorophenoxy)propanoic acid

To a solution of Example 319B (500 mg, 1.48 mmol) in tetrahydrofuran (4 mL) were added lithium hydroxide (141 mg, 5.90 mmol) and water (1.0 mL). The mixture was stirred at ambient temperature for 3 hours. After 3 hours, the reaction mixture was diluted with water (10 mL) and ethyl acetate (10 mL). The aqueous layer was washed with ethyl acetate. The aqueous layer was then acidified with 2 N HCl (aq.) to pH=3 and then was extracted with CH₂Cl₂ (3 × 20 mL). The combined organic fractions were dried over anhydrous MgSO₄, and concentrated under reduced pressure. The residue was purified by preparative HPLC [Waters XBridge^{™} C18 5 µm OBD^{™} column, 30 × 100 mm, flow rate 40 mL/minute, 5-100% gradient of acetonitrile in buffer (0.1 % trifluoroacetic acid)] to give the title compound (45 mg, 0.139 mmol, 9% yield). ¹H NMR (400 MHz, DMSO-*d*₆) *δ* ppm 7.43 (t, J = 8.9 Hz, 1H), 7.37 - 7.19 (m, 5H), 7.03 (dd, J = 11.4, 2.9 Hz, 1H), 6.79 (ddd, J = 9.1, 2.9, 1.2 Hz, 1H), 5.09 (dd, J = 5.2, 3.0 Hz, 1H), 4.61 - 4.45 (m, 2H), 3.93 - 3.79 (m, 2H).

### Example 319D: (2R)-3-(benzyloxy)-2-(4-chloro-3-fluorophenoxy)-N-{3-[2-(4-chloro-3-fluorophenoxy)acetamido]bicyclo[1.1.1]pentan-1-yl}propanamide

The reaction and purification conditions described in Example 299 substituting Example 319C for 2-phenoxyacetic acid gave the title compound. MS (ESI⁺) *m*/*z* 591 (M+H)⁺.

### Example 319E: (2R)-2-(4-chloro-3-fluorophenoxy)-N-{3-[2-(4-chloro-3-fluorophenoxy)acetamido]bicyclo[1.1.1]pentan-1-yl}-3-hydroxypropanamide

To a 10 mL pressure tube was added a solution of the product of Example 319D (45 mg, 0.076 mmol) in methanol (1 mL) 5% Pd/C (wet JM#9) (25 mg, 0.104 mmol) and 4 M HCl in dioxane (0.038 mL, 0.15 mmol). The reaction mixture was stirred for 4 hours under 50 psi of hydrogen without external heating. The reaction mixture was filtered and the filtrate was concentrated under reduced pressure. The residue was purified by preparative HPLC [Waters XBridge^{™} C18 5 µm OBD^{™} column, 30 × 100 mm, flow rate 40 mL/minute, 5-100% gradient of acetonitrile in buffer (0.1 % trifluoroacetic acid)] to give the title compound (25 mg, 0.050 mmol, 66% yield). ¹H NMR (400 MHz, DMSO-*d*₆) *δ* ppm 8.70 (s, 1H), 8.70 (s, 1H), 7.49 (t, J = 8.9 Hz, 2H), 7.05 (ddd, J = 11.4, 10.5, 2.8 Hz, 2H), 6.88 - 6.80 (m, 2H), 4.61 (dd, J = 5.6, 4.0 Hz, 1H), 4.47 (s, 2H), 3.76 - 3.68 (m, 2H), 2.23 (s, 6H); MS (ESI⁺) *m*/*z* 518 (M+NH₄)⁺.

### Example 320: N,N'-[(2S)-2-(2H-tetrazol-5-yl)bicyclo[2.2.2]octane-1,4-diyl]bis[2-(4-chloro-3-fluorophenoxy)acetamide] (Compound 419)

A mixture of Example 318 (100 mg, 0.186 mmol), sodium azide (121 mg, 1.857 mmol) and ammonium chloride (99 mg, 1.857 mmol) was heated in a microwave vial at 110 °C for 16 hours. The reaction mixture was cooled to ambient temperature and partitioned between with water and dichloromethane. The organic layer was dried and concentrated. The residue was purified by HPLC (30-100% acetonitrile in 0.1% trifluoroacetic acid/water at 25 mL/minute on a Phenomenex^{®} C18 5 µm column (250 × 21.2 mm)) to give 45 mg of the title compound as a white solid. ¹H NMR (400 MHz, DMSO-*d*₆) *δ* ppm 16.10 (s, 1H), 8.49 (d, J = 3.6 Hz, 1H), 8.05 - 7.97 (m, 1H), 7.54 - 7.40 (m, 2H), 7.00 (dddd, J = 22.3, 16.5, 11.3, 2.8 Hz, 2H), 6.88 - 6.70 (m, 2H), 4.58 (d, J = 15.9 Hz, 1H), 4.51 (d, J = 9.9 Hz, 2H), 4.42 (s, 1H), 2.89 (t, J = 5.8 Hz, 2H), 2.11 - 1.68 (m, 7H), 1.42 - 1.23 (m, 1H); MS (ESI⁺) *m*/*z* 581.1 (M+H)⁺.

### Example 321: N,N'-[(2S)-2-(3-methyl-1,2,4-oxadiazol-5-yl)bicyclo[2.2.2]octane-1,4-diyl]bis[2-(4-chloro-3-fluorophenoxy)acetamide] (Compound 420)

A mixture of Example 318 (80 mg, 0.144 mmol), *N*-hydroxyacetimidamide (13.29 mg, 0.179 mmol) and *N*-ethyl-*N*'-(3-dimethylaminopropyl)carbodiimide hydrochloride (41.3 mg, 0.215 mmol) in dichloromethane (5 mL) was stirred at ambient temperature overnight. The mixture was concentrated, and the residue was dissolved in tetrahydrofuran (5 mL), treated with tetrabutylammonium fluoride (1.435 mL, 1.435 mmol, 1 M in tetrahydrofuran) and stirred at 50 °C for 2 hours. The mixture was partitioned between water and dichloromethane. The organic layer was dried, filtered and concentrated. The residue was purified by HPLC (30-100% acetonitrile in 0.1% trifluoroacetic acid/water at 25 mL/minute on a Phenomenex^{®} C18 5 µm column (250 × 21.2 mm)) to give 45 mg of the title compound as a white solid. ¹H NMR (400 MHz, DMSO-*d*₆) *δ* ppm 8.72 (d, *J =* 9.8 Hz, 1H), 8.05 (d, *J =* 18.1 Hz, 1H), 7.55 - 7.43 (m, 2H), 7.10 - 6.92 (m, 2H), 6.91 - 6.74 (m, 2H), 4.72 - 4.59 (m, 1H), 4.58 (d, *J =* 7.3 Hz, 1H), 4.49 (d, *J =* 17.8 Hz, 2H), 4.12 (s, 1H), 2.32 (d, *J =* 9.7 Hz, 3H), 2.23 - 2.06 (m, 1H), 2.06 - 1.89 (m, 2H), 1.93 - 1.75 (m, 7H); MS (ESI⁺) *m*/*z* 595.0 (M+H)⁺.

### Example 322: 2-(4-chloro-3-fluorophenoxy)-N-{3-[2-(4-chloro-3-fluorophenoxy)acetamido]bicyclo[1.1.1]pentan-1-yl}-3-methoxypropanamide (Compound 421)

### Example 322A: methyl 2-(4-chloro-3-fluorophenoxy)-3-methoxypropanoate

To a solution of methyl 2-bromo-3-methoxypropanoate (0.33 mL, 2.46 mmol) in acetonitrile (5 mL) was added 4-chloro-3-fluorophenol (300 mg, 2.047 mmol) and potassium carbonate (849 mg, 6.14 mmol). The reaction mixture was stirred at 70 °C for 3 hours. The reaction mixture was filtered, and the filtrate was concentrated under reduced pressure. The crude material was carried on without characterization.

### Example 322B: 2-(4-chloro-3-fluorophenoxy)-3-methoxypropanoic acid

To a solution of the product of Example 322A (300 mg, 1.14 mmol) in tetrahydrofuran (4 mL) was added lithium hydroxide (109 mg, 4.57 mmol) and water (1.0 mL). The mixture was stirred at ambient temperature for 18 hours. The reaction mixture was diluted with water (10 mL) and ethyl acetate (20 mL). The aqueous layer was washed with ethyl acetate and then was acidified with 2 N HCl (aqueous) to pH=3. The solution was extracted with CH₂Cl₂ (3 × 20mL). The combined organic fractions were filtered, dried over anhydrous MgSO₄ and concentrated under reduced pressure. MS (ESI⁺) *m*/*z* 266 (M+NH₄)⁺.

### Example 322C: 2-(4-chloro-3-fluorophenoxy)-N-{3-[2-(4-chloro-3-fluorophenoxy)acetamido]bicyclo[1.1.1]pentan-1-yl}-3-methoxypropanamide

The reaction and purification conditions described in Example 299 substituting Example 322B for 2-phenoxyacetic acid gave the title compound. ¹H NMR (501 MHz, DMSO-*d*₆) *δ* ppm 8.78 (s, 1H), 8.71 (s, 1H), 7.49 (td, J = 8.9, 1.6 Hz, 2H), 7.08 (t, J = 2.7 Hz, 1H), 7.06 (t, J = 2.7 Hz, 1H), 6.86 (ddd, J = 2.8, 1.8, 1.1 Hz, 1H), 6.84 (ddd, J = 2.9, 1.8, 1.1 Hz, 1H), 4.81 (dd, J = 5.8, 3.3 Hz, 1H), 4.48 (s, 2H), 3.69 (qd, J = 11.0, 4.6 Hz, 2H), 3.28 (s, 3H), 2.24 (s, 6H); MS (ESI⁺) *m*/*z* 551 (M+H)⁺.

### Example 323: 2-(3,4-dichlorophenoxy)-N-(3-{2-[4-(2-hydroxypropan-2-yl)phenoxy]acetamido}bicyclo[1.1.1]pentan-1-yl)acetamide (Compound 422)

A mixture of the product of Example 50A (40.0 mg, 0.106 mmol), 4-(2-hydroxypropan-2-yl)phenol (32.2 mg, 0.212 mmol), potassium carbonate (43.9 mg, 0.318 mmol), and potassium iodide (1.231 mg, 7.41 µmol) in acetonitrile (0.5mL) was heated at 70 °C for 3 hours. The reaction mixture was filtered, and the collected solid was washed with acetonitrile (2 mL) a couple of times. The combined filtrate and washes were concentrated, and the residue was purified by preparative HPLC [Waters XBridge^{™} C18 5 µm OBD^{™} column, 30 × 100 mm, flow rate 40 mL/minute, 5-100% gradient of acetonitrile in buffer (0.1 % aqueous trifluoroacetic acid)] to give the title compound (9 mg, 0.018 mmol, 17.2% yield). ¹H NMR (400 MHz, DMSO-*d*₆) *δ* ppm 8.67 (s, 1H), 8.62 (s, 1H), 7.51 (d, J = 8.9 Hz, 1H), 7.35 - 7.31 (m, 2H), 7.23 (d, J = 2.9 Hz, 1H), 6.95 (dd, J = 8.9, 2.9 Hz, 1H), 6.85 - 6.80 (m, 2H), 4.86 (s, 1H), 4.45 (s, 2H), 4.35 (s, 2H), 2.23 (s, 6H), 1.36 (s, 6H); MS (ESI⁺) *m*/*z* 494 (M+H)⁺.

### Example 324: 2-(4-chloro-3-fluorophenoxy)-N-{2-hydroxy-4-[2-(3-methyl-1,2-oxazol-5-yl)acetamido]bicyclo[2.2.2]octan-1-yl}acetamide (Compound 423)

The title compound was prepared using the methodologies described in Example 214 substituting 2-(3-methylisoxazol-5-yl)acetic acid for 2-((6-(trifluoromethyl)pyridin-3-yl)oxy)acetic acid. ¹H NMR (400 MHz, DMSO-*d*₆) *δ* ppm 7.71 (s, 1H), 7.45 (t, J = 8.9 Hz, 1H), 7.21 (s, 1H), 7.01 (dd, J = 11.4, 2.9 Hz, 1H), 6.79 (dd, J = 9.1, 2.9 Hz, 1H), 6.08 (s, 1H), 4.43 (s, 2H), 3.99 (dd, J = 9.6, 3.0 Hz, 1H), 3.51 (s, 2H), 2.21 (ddd, J = 12.5, 9.5, 2.5 Hz, 1H), 2.15 (s, 3H), 2.03 (ddd, J = 12.5, 10.6, 5.0 Hz, 1H), 1.87 (d, J = 7.8 Hz, 2H), 1.87 - 1.76 (m, 1H), 1.80 - 1.65 (m, 5H); MS (ESI⁺) *m*/*z* 466.1 (M+H)⁺.

### Example 325: 1-[2-({(2S)-4-[2-(4-chloro-3-fluorophenoxy)acetamido]-2-hydroxybicyclo[2.2.2]octan-1-yl}amino)ethyl]-3-methyl-1H-pyrazole-5-carboxylic acid (Compound 424)

### Example 325A: methyl 1-(2-bromoethyl)-3-methyl-1H-pyrazole-5-carboxylate

A mixture of methyl 5-methyl-1*H*-pyrazole-3-carboxylate (140 mg, 1 mmol), 1,2-dibromoethane (939 mg, 5.00 mmol) and potassium carbonate (276 mg, 2.00 mmol) was stirred in acetonitrile (10 mL) at reflux for 3 hours. The reaction mixture was filtered, and the filtrate was concentrated in vacuo. The residue was diluted with ethyl acetate (50 mL) and washed with water (2 × 20 mL). The organic phase was dried over anhydrous Na₂SO₄ and concentrated in vacuo. The crude product was purified by flash column chromatography on silica gel (40g) eluted with 20 to 60% ethyl acetate in heptane to give the title compound (0.080 g, 0.32 mmol, 32% yield). ¹H NMR (400 MHz, CDCl₃) *δ* ppm 6.64 (s, 1H), 4.87 (t, J = 7 Hz, 2H), 3.87 (s, 3H), 3.68 (t, J = 7 Hz, 2H), 2.28 (s, 3H); MS (ESI⁺) *m*/*z* 248 (M+H)⁺.

### Example 325B: methyl 1-[2-({(2S)-4-[2-(4-chloro-3-fluorophenoxy)acetamido]-2-hydroxybicyclo[2.2.2]octan-1-yl}amino)ethyl]-3-methyl-1H-pyrazole-5-carboxylate

A mixture of product of Example 276A (120 mg, 0.26 mmol), the product of Example 325A (40 mg, 0.16 mmol), potassium iodide (26.9 mg, 0.16 mmol) and N-ethyl-N-isopropylpropan-2-amine (73.2 mg, 0.57 mmol) in dimethyl sulfoxide (0.1 mL) was stirred at 120 °C for 8 hours. The reaction mixture was cooled to ambient temperature and concentrated under reduced pressure to give the title compound (0.082 g, 0.16 mmol, 100% yield). MS (ESI⁺) *m*/*z* 509 (M+H)⁺.

### Example 325C: 1-[2-({(2S)-4-[2-(4-chloro-3-fluorophenoxy)acetamido]-2-hydroxybicyclo[2.2.2]octan-1-yl)amino)ethyl]-3-methyl-1H-pyrazole-5-carboxylic acid

To the product of Example 325B in methanol (1 mL) was added 3 N NaOH solution (0.27 mL). The mixture was stirred at ambient temperature for 18 hours. The resulting solution was filtered through a glass microfiber frit and purified by preparative HPLC [Waters XBridge^{™} C18 5 µm OBD^{™} column, 30 × 100 mm, flow rate 40 mL/minute, 5-100% gradient of acetonitrile in buffer (0.1 % trifluoroacetic acid)] to give the title compound (0.035 g, 0.057 mmol, 36% yield). ¹H NMR (501 MHz, DMSO-*d*₆) *δ* ppm 8.60 (br s, 2H), 7.67 (s, 1H), 7.48 (t, J = 8 Hz, 1H), 7.03 (dd, J = 9, 3 Hz, 1H), 6.81 (br d, J = 8 Hz, 1H), 6.65 (s, 1H), 5.73 (br s, 1H), 4.69 (t, J = 7 Hz, 2H), 4.45 (s, 2H), 4.00 (m, 1H), 3.50 (t, J = 7 Hz, 2H), 2.34 (m, 1H), 2.19 (s, 3H), 2.00 (m, 2H), 1.65 - 1.90 (m, 7H); MS (ESI⁺) *m*/*z* 495 (M+H)⁺.

### Example 326: (2E)-N-{4-[2-(4-chloro-3-fluorophenoxy)acetamido]bicyclo[2.1.1]hexan-1-yl}-3-[5-(trifluoromethoxy)pyridin-2-yl]prop-2-enamide (Compound 425)

The reaction and purification conditions described in Example 81, substituting the product of Example 277B for 2-(4-chloro-3-fluorophenoxy)acetic acid and the product of Example 88C for benzyl (4-aminobicyclo[2.1.1]hexan-1-yl)carbamate hydrochloride gave the title compound. ¹H NMR (400 MHz, DMSO-*d*₆) *δ* ppm 8.73 (s, 1H), 8.68 (d, J = 2.8 Hz, 1H), 8.50 (s, 1H), 7.93 (ddd, J = 8.6, 2.8, 1.2 Hz, 1H), 7.73 (d, J = 8.5 Hz, 1H), 7.52 - 7.42 (m, 2H), 7.07 (dd, J = 11.4, 2.9 Hz, 1H), 7.03 (d, J = 15.4 Hz, 1H), 6.86 (ddd, J = 9.0, 2.9, 1.2 Hz, 1H), 4.49 (s, 2H), 2.19 - 2.11 (m, 2H), 1.90 - 1.77 (m, 6H); MS (ESI⁺) *m*/*z* 514 (M+NH)⁺.

### Example 327: 2-(4-chloro-3-fluorophenoxy)-N-[3-({[4-(trifluoromethyl)phenyl]methanesulfonyl}amino)bicyclo[1.1.1]pentan-1-yl]acetamide (Compound 426)

To a solution of Example 27D (50.0 mg, 0.176 mmol) and (4-(trifluoromethyl)phenyl)methanesulfonyl chloride (45.4 mg, 0.176 mmol) in *N,N-*dimethylformamide (1.5 mL) was added triethylamine (0.061 mL, 0.439 mmol). The mixture was stirred for 3 hours and then treated with water. The precipitate was collected by filtration, and the precipitate was subsequently purified by reverse-phase HPLC (see protocol in Example 112D) to provide the title compound (24.5 mg, 88%). ¹H NMR (400 MHz, DMSO-*d*₆) *δ* ppm 8.76 (s, 1H), 8.20 (s, 1H), 7.77 (d, J = 8.1 Hz, 2H), 7.59 (d, J = 8.0 Hz, 2H), 7.50 (t, J = 8.9 Hz, 1H), 7.07 (dd, J = 11.4, 2.8 Hz, 1H), 6.85 (ddd, J = 9.0, 2.9, 1.2 Hz, 1H), 4.49 (s, 2H), 4.42 (s, 2H), 2.19 (s, 6H); MS (ESI⁺) *m*/*z* 507.0 (M+H)⁺.

### Example 328: 2-(4-chloro-3-fluorophenoxy)-N-(3-{[(4-chlorophenyl)methanesulfonyl]amino}bicyclo[1.1.1]pentan-1-yl)acetamide (Compound 427)

The reaction described in Example 327 substituting (5-chloropyridin-2-yl)methanesulfonyl chloride for (4-(trifluoromethyl)phenyl)methanesulfonyl chloride gave the title compound. ¹H NMR (400 MHz, DMSO-*d*₆) *δ* ppm 8.73 (s, 1H), 8.11 (s, 1H), 7.56 - 7.42 (m, 3H), 7.42 - 7.32 (m, 2H), 7.06 (dd, J = 11.4, 2.9 Hz, 1H), 6.85 (ddd, J = 9.1, 2.9, 1.2 Hz, 1H), 4.48 (s, 2H), 4.30 (s, 2H), 2.16 (s, 6H); MS (ESI⁺) *m*/*z* 473.0 (M+H)⁺.

### Example 329: 2-(4-chloro-3-fluorophenoxy)-N-[3-({[3-(trifluoromethyl)phenyl]methanesulfonyl}amino)bicyclo[1.1.1]pentan-1-yl]acetamide (Compound 428)

The reaction described in Example 327 substituting (3-(trifluoromethyl)phenyl)methanesulfonyl chloride for (4-(trifluoromethyl)phenyl)methanesulfonyl chloride gave the title compound. ¹H NMR (400 MHz, DMSO-*d*₆) *δ* ppm 8.72 (s, 1H), 8.18 (s, 1H), 7.78 - 7.60 (m, 4H), 7.49 (t, J = 8.9 Hz, 1H), 7.06 (dd, J = 11.4, 2.9 Hz, 1H), 6.85 (ddd, J = 8.9, 2.8, 1.2 Hz, 1H), 4.48 (s, 2H), 4.44 (s, 2H), 2.15 (s, 6H); MS (ESI⁺) *m*/*z* 507.1 (M+H)⁺.

### Example 330: 2-(4-chloro-3-fluorophenoxy)-N-[(35)-3-hydroxy-4-({[4-(trifluoromethyl)phenyl]methanesulfonyl}amino)bicyclo[2.2.2]octan-1-yl]acetamide (Compound 429)

### Example 330A: N-[(3S)-4-amino-3-hydroxybicyclo[2.2.2]octan-1-yl]-2-(4-chloro-3-fluorophenoxy)acetamide hydrochloride

A mixture of Example 276A (4.5 g, 9.85 mmol) and hydrogen chloride (4 N in 1,4-dioxane, 10.0 mL, 40.0 mmol) in ether (100 mL) was stirred at room temperature for 16 hours. Volatiles were removed, and the residue was triturated with CH₂Cl₂/CH₃OH/hexane to give the title compound (3.2 g, 86%). MS (ESI⁺) *m*/*z* 343.2 (M+H)⁺.

### Example 330B: 2-(4-chloro-3-fluorophenoxy)-N-[(3S)-3-hydroxy-4-({[4-(trifluoromethyl)phenyl]methanesulfonyl}amino)bicyclo[2.2.2]octan-1-yl]acetamide

To a solution of Example 330A (60.0 mg, 0.158 mmol) and (4-(trifluoromethyl)phenyl)methanesulfonyl chloride (40.9 mg, 0.158 mmol) in *N,N-*dimethylformamide (2 mL) was added triethylamine (0.055 mL, 0.396 mmol). The mixture was stirred at room temperature for 3 hours and at 45 °C for 3 hours. More sulfonyl chloride was added (40.9 mg). The reaction mixture was stirred for 3 hours, treated with brine, and extracted with ethyl acetate (2×). The combined organic layers were dried over MgSO₄, filtered, and concentrated. The residue was purified by reverse-phase HPLC (see protocol in Example 112D) to provide the title compound (20.0 mg, 22%). ¹H NMR (400 MHz, DMSO-*d*₆) *δ* ppm 7.73 (d, J = 8.1 Hz, 2H), 7.64 (d, J = 8.0 Hz, 2H), 7.49 (dd, J = 18.1, 9.2 Hz, 2H), 7.03 (dd, J = 11.4, 2.8 Hz, 1H), 6.81 (dd, J = 8.9, 2.8 Hz, 1H), 6.53 (s, 1H), 4.50 - 4.39 (m, 4H), 4.06 - 3.94 (m, 1H), 2.31 (ddd, J = 12.3, 9.4, 2.4 Hz, 1H), 2.11 - 1.66 (m, 9H); MS (ESI⁺) *m*/*z* 581.9 (M+NH₄)⁺.

### Example 331: 2-(4-chloro-3-fluorophenoxy)-N-[(3S)-3-hydroxy-4-({[3-(trifluoromethyl)phenyl]methanesulfonyl}amino)bicyclo[2.2.2]octan-1-yl]acetamide (Compound 430)

The reaction described in Example 330B substituting (3-(trifluoromethyl)phenyl)methanesulfonyl chloride for (4-(trifluoromethyl)phenyl)methanesulfonyl chloride gave the title compound. ¹H NMR (400 MHz, DMSO-*d*₆) *δ* ppm 7.79 (s, 1H), 7.71 (t, J = 8.3 Hz, 2H), 7.60 (t, J = 7.7 Hz, 1H), 7.49 (dd, J = 17.7, 8.8 Hz, 2H), 7.03 (dd, J = 11.4, 2.8 Hz, 1H), 6.81 (dd, J = 9.1, 2.9 Hz, 1H), 6.53 (s, 1H), 4.53 - 4.39 (m, 4H), 4.00 (dd, J = 9.6, 2.3 Hz, 1H), 2.30 (ddd, J = 12.5, 9.4, 2.4 Hz, 1H), 2.11 - 1.63 (m, 9H); MS (ESI⁺) *m*/*z* 581.9 (M+NH₄)⁺.

### Example 332: 2-(4-chloro-3-fluorophenoxy)-N-(3-{[(4-chlorophenyl)carbamoyl]amino}bicyclo[1.1.1]pentan-1-yl)acetamide (Compound 431)

4-Chlorophenyl isocyanate (35 mg, 0.23 mmol) was added to a pyridine (5.0 mL) solution of the product of Example 27D (65 mg, 0.23 mmol). After stirring at ambient temperature for 30 minutes, the reaction mixture was concentrated in vacuo, taken up in *N,N-*dimethylformamide (3 mL), filtered through a glass microfiber frit, and purified by preparative HPLC [YMC TriArt^{™} C18 Hybrid 20 µm column, 25 × 150 mm, flow rate 80 mL/minute, 5-100% gradient of acetonitrile in buffer (0.025 M aqueous ammonium bicarbonate, adjusted to pH 10 with ammonium hydroxide)] to give the title compound (76 mg, 0.17 mmol, 76% yield). ¹H NMR (500 MHz, DMSO-*d*₆) *δ* ppm 8.71 (s, 1H), 8.46 (s, 1H), 7.50 (t, J = 8.9 Hz, 1H), 7.42 - 7.36 (m, 2H), 7.28 - 7.22 (m, 2H), 7.07 (dd, J = 11.4, 2.8 Hz, 1H), 6.89 - 6.82 (m, 2H), 4.48 (s, 2H), 2.23 (s, 6H); MS (ESI⁺) *m*/*z* 438 (M+H)⁺.

### Example 333: 2-(4-chloro-3-fluorophenoxy)-N-(3-{[(4-chloro-2-hydroxyphenyl)carbamoyl]amino}bicyclo[1.1.1]pentan-1-yl)acetamide (Compound 432)

A mixture of the product of Example 9B (35 mg, 0.088 mmol), 2,6-dichlorobenzo[d]oxazole (17.33 mg, 0.092 mmol) and *N*-ethyl-*N*-isopropylpropan-2-amine (56.7 mg, 0.439 mmol) in dimethyl sulfoxide (0.2 mL) was stirred at 100 °C for 1.5 hours. The resulting solution was filtered through a glass microfiber frit and purified by preparative HPLC [Waters XBridge^{™} C18 5 µm OBD^{™} column, 30 × 100 mm, flow rate 40 mL/minute, 5-100% gradient of acetonitrile in buffer (0.1 % trifluoroacetic acid)] to give the title compound (11 mg, 0.024 mmol, 28% yield). ¹H NMR (400 MHz, DMSO-*d*₆) *δ* ppm 10.36 (s, 1H), 8.72 (s, 1H), 7.94 (d, J = 8 Hz, 1H), 7.82 (s, 1H), 7.51 (s, 1H), 7.50 (t, J = 8 Hz, 1H), 7.08 (dd, J = 9, 3 Hz, 1H), 6.86 (br d, J = 8 Hz, 1H), 6.79 (d, J = 2 Hz, 1H), 6.74 (dd, J = 8, 3Hz, 1H), 4.48 (s, 2H), 2.22 (s, 6H); MS (ESI⁺) *m*/*z* 454 (M+H)⁺.

### Example 334: 4-methylphenyl {4-[2-(4-chloro-3-fluorophenoxy)acetamido]-2-hydroxybicyclo[2.2.2]octan-1-yl}carbamate (Compound 433)

To a mixture of the product of Example 198H (30 mg, 0.088 mmol) and *N*-ethyl-*N-*isopropylpropan-2-amine (0.038 mL, 0.220 mmol) in tetrahydrofuran (1.0 mL) was added*p-*tolyl carbonochloridate (0.019 mL, 0.13 mmol), and the clear solution was stirred at ambient temperature for 16 hours. Solvent was removed under vacuum, and the residue was dissolved in a mixture of dichloromethane/methanol (1:1, 2 mL) and treated with sodium borohydride (16.7 mg, 0.44 mmol) for 1 hour at ambient temperature. The mixture was concentrated under reduced pressure and purified by HPLC (20-100% acetonitrile in 0.1% trifluoroacetic acid/water on a Phenomenex^{®} C18 5µm (250 mm × 21.2 mm) column at a flowrate of 25 mL/minute) to give the title compound (28 mg, 67% yield). ¹H NMR (400 MHz, DMSO-*d*₆) *δ* ppm 7.53 - 7.43 (m, 2H), 7.15 (d, J = 8.3 Hz, 2H), 7.10 - 6.98 (m, 2H), 7.00 - 6.92 (m, 2H), 6.81 (ddd, J = 8.9, 2.9, 1.2 Hz, 1H), 4.89 (s, 1H), 4.43 (s, 2H), 4.08 (dd, J = 9.8, 2.8 Hz, 1H), 2.28 (s, 3H), 2.25 (m, 1H), 1.99 - 1.68 (m, 9H); MS (ESI⁺) *m*/*z* 477.2 (M+H)⁺.

### Example 335: 4-chlorophenyl {3-[2-(4-chloro-3-fluorophenoxy)acetamido]bicyclo[1.1.1]pentan-1-yl}carbamate (Compound 434)

To a suspension of Example 112A (65.0 mg, 0.202 mmol) and triethylamine (0.071 mL, 0.506 mmol) in tetrahydrofuran (2 mL) was added 4-chlorophenyl carbonochloridate (0.031 mL, 0.223 mmol). The mixture was stirred for 2 hours, quenched with saturated NaHCO₃ and brine, and extracted with ethyl acetate (2×). The combined organic layers were concentrated, and the residue was purified by reverse-phase HPLC (see protocol in Example 112D) to provide the title compound (19.1 mg, 21%). ¹H NMR (400 MHz, DMSO-*d*₆) *δ* ppm 8.73 (s, 1H), 8.57 (s, 1H), 7.49 (t, J = 8.9 Hz, 1H), 7.46 - 7.38 (m, 2H), 7.15 (d, J = 8.8 Hz, 2H), 7.07 (dd, J = 11.4, 2.8 Hz, 1H), 6.85 (ddd, J = 9.1, 2.9, 1.2 Hz, 1H), 4.48 (s, 2H), 2.24 (s, 6H); MS (ESI⁺) *m*/*z* 439.1 (M+H)⁺.

### Example 336: 4-methylphenyl {3-[2-(4-chloro-3-fluorophenoxy)acetamido]bicyclo[1.1.1]pentan-1-yl}carbamate (Compound 435)

The reaction described in Example 335 substituting *p*-tolyl carbonochloridate for 4-chlorophenyl carbonochloridate gave the title compound. ¹H NMR (400 MHz, DMSO-*d*₆) *δ* ppm 8.72 (s, 1H), 8.43 (s, 1H), 7.49 (t, J = 8.9 Hz, 1H), 7.16 (d, J = 8.1 Hz, 2H), 7.07 (dd, J = 11.4, 2.9 Hz, 1H), 6.96 (d, J = 8.3 Hz, 2H), 6.85 (ddd, J = 9.0, 2.8, 1.2 Hz, 1H), 4.48 (s, 2H), 2.28 (s, 3H), 2.23 (s, 6H); MS (ESI⁺) *m*/*z* 419.1 (M+H)⁺.

### Example 337: 4-chlorophenyl {4-[2-(4-chloro-3-fluorophenoxy)acetamido]-2-hydroxybicyclo[2.2.2]octan-1-yl}carbamate (Compound 436)

The title compound was prepared using the methodologies described in Example 334 substituting 4-chlorophenyl carbonochloridate for p-tolyl carbonochloridate. ¹H NMR (400 MHz, DMSO-*d*₆) *δ* ppm 7.54 - 7.43 (m, 2H), 7.46 - 7.37 (m, 2H), 7.23 (s, 1H), 7.17 - 7.08 (m, 2H), 7.02 (dd, *J* = 11.4, 2.8 Hz, 1H), 6.81 (dt, *J* = 8.8, 1.8 Hz, 1H), 4.87 (s, 1H), 4.43 (s, 2H), 4.11 (dt, *J* = 9.3, 2.4 Hz, 1H), 2.28 (ddd, *J* = 12.7, 9.5, 2.7 Hz, 1H), 2.04 - 1.66 (m, 9H); MS (ESI⁺) *m*/*z* 497.0 (M+H)⁺.

### Example 338: 2-(4-chloro-3-fluorophenoxy)-N-{(2S)-4-[2-(4-chlorophenoxy)acetamido]-2-hydroxybicyclo[2.2.2]octan-1-yl}acetamide (Compound 437)

### Example 338A: N-(4-amino-2-hydroxybicyclo[2.2.2]octan-1-yl)-2-(4-chloro-3-fluorophenoxy)acetamide, hydrochloric acid

A mixture of Example 214E (7g, 15.39 mmol) and NaBH₄ (0.582 g, 15.39 mmol) in a mixture of methanol (200mL) and methylene chloride (200 mL) was stirred at 20 °C for 12 hours. The solution was concentrated, and the residue was purified by preparative HPLC (5-100% acetonitrile in water with 0.05% HCl on a SNAP C18 (20-35 µm, 800 g) column at a flow rate of 200 mL/minute) to provide the title compound (5.0 g, 83%). MS (ESI⁺) *m*/*z* 343.1 (M+H)⁺.

### Example 338B: N-[(2S)-4-amino-2-hydroxybicyclo[2.2.2]octan-1-yl]-2-(4-chloro-3-fluorophenoxy)acetamide

The title compound was isolated by chiral preparative SFC of Example 338A (see protocol in Example 276A) as the first peak eluted off the column. The chirality of its enantiomer (the second peak eluted off the column) was confirmed by X-ray crystallography. ¹H NMR (400 MHz, methanol-*d*₄) *δ* ppm 7.36 (t, *J*=8.71 Hz, 1H), 6.91 (dd, *J*=10.91, 2.76 Hz, 1H), 6.80 (dd, *J*=8.82, 1.32 Hz, 1H), 4.45 (s, 2H), 4.28 (br d, J = 8.60 Hz, 1H), 2.22 - 1.98 (m, 4H), 1.90 (td, J = 11.74, 4.30 Hz, 1H), 1.82 - 1.49 (m, 8H); MS (ESI⁺) *m*/*z* 343.1 (M+H)⁺.

### Example 338C: 2-(4-chloro-3-fluorophenoxy)-N-{(2S)-4-[2-(4-chlorophenoxy)acetamido]-2-hydroxybicyclo[2.2.2]octan-1-yl}acetamide

A mixture of Example 338B (40 mg, 0.117 mmol), 2-(4-chlorophenoxy)acetic acid (25.04 mg, 0.134 mmol) and *N*-ethyl-*N*-isopropylpropan-2-amine (0.071 mL, 0.408 mmol) in N,N-dimethylformamide (1.5 mL) was treated with 2-(3*H*-[1,2,3]triazolo[4,5-*b*]pyridin-3-yl)-1,1,3,3-tetramethylisouronium hexafluorophosphate(V) (66.6 mg, 0.175 mmol), and the reaction was stirred at ambient temperature for 30 minutes. Solvent was removed under high vacuum, and the residue was purified by HPLC (20-100% acetonitrile in 0.1% trifluoroacetic acid/water on a Phenomenex^{®} C18 10µm (250 mm × 50 mm) column at a flow rate of 50 mL/minute) to give 63 mg of the title column as a white solid. ¹H NMR (400 MHz, DMSO-*d*₆) □ ppm 7.53 - 7.43 (m, 2H), 7.37 - 7.28 (m, 2H), 7.25 (s, 1H), 7.05 (dd, J = 11.4, 2.8 Hz, 1H), 6.99 - 6.89 (m, 2H), 6.83 (ddd, J = 9.0, 2.9, 1.2 Hz, 1H), 5.10 - 5.04 (m, 1H), 4.43 (d, J = 31.5 Hz, 4H), 4.03 (d, J = 9.0 Hz, 1H), 2.27 (ddd, J = 12.2, 9.4, 2.2 Hz, 1H), 2.07 (ddd, J = 12.3, 10.5, 4.8 Hz, 1H), 1.97 - 1.87 (m, 2H), 1.90 - 1.80 (m, 3H), 1.78 (ddd, J = 13.3, 6.8, 2.5 Hz, 4H); MS (ESI⁺) *m*/*z 511.1* (M+H)⁺.

### Example 339: 2-(4-chloro-3-fluorophenoxy)-N-{(2S)-4-[2-(3,4-dichlorophenoxy)acetamido]-2-hydroxybicyclo[2.2.2]octan-1-yl}acetamide (Compound 438)

The title compound was prepared using the methodologies described in Example 338C substituting 2-(3,4-dichlorophenoxy)acetic acid for 2-(4-chlorophenoxy)acetic acid. ¹H NMR (400 MHz, DMSO-*d*₆) □ ppm 7.56 - 7.43 (m, 3H), 7.28 - 7.18 (m, 2H), 7.05 (dd, J = 11.4, 2.8 Hz, 1H), 6.94 (dd, J = 9.0, 2.9 Hz, 1H), 6.83 (ddd, J = 9.0, 2.9, 1.2 Hz, 1H), 5.07 (d, J = 4.4 Hz, 1H), 4.45 (d, J = 8.8 Hz, 4H), 4.04 (dt, J = 8.7, 3.6 Hz, 1H), 2.27 (ddd, J = 12.3, 9.4, 2.2 Hz, 1H), 2.07 (ddd, J = 12.4, 10.6, 4.7 Hz, 1H), 1.93 (d, J = 11.1 Hz, 2H), 1.91 - 1.72 (m, 6H); MS (ESI⁺) *m*/*z* 542.9 (M-H)⁻.

### Example 340: N-{(2S)-4-[2-(4-chloro-3-fluorophenoxy)acetamido]-2-hydroxybicyclo[2.2.2]octan-1-yl}-2-(4-chloro-3-methylphenoxy)-2-methylpropanamide (Compound 439)

The title compound was prepared using the methodologies described in Example 338C substituting Example 276A for Example 338B and 2-(4-chloro-3-methylphenoxy)-2-methylpropanoic acid for 2-(4-chlorophenoxy)acetic acid. ¹H NMR (400 MHz, DMSO-*d*₆) □ ppm 7.53 7.43 (m, 2H), 7.30 (d, J = 8.7 Hz, 1H), 7.10 (s, 1H), 7.02 (dd, J = 11.4, 2.8 Hz, 1H), 6.91 (d, J = 2.9 Hz, 1H), 6.87 6.70 (m, 2H), 5.19 (s, 1H), 4.43 (s, 2H), 3.88 3.79 (m, 1H), 2.50 2.37 (m, 1H), 2.34 2.23 (m, 1H), 2.27 (s, 3H), 2.08 1.93 (m, 1H), 1.97 1.86 (m, 1H), 1.89 1.72 (m, 6H), 1.37 (d, J = 5.3 Hz, 6H); MS (ESI⁺) *m*/*z* 553.3 (M+H)⁺.

### Example 341: N,N'-(bicyclo[2.2.1]heptane-1,4-diyl)bis[2-(4-chloro-3-fluorophenoxy)acetamide] (Compound 440)

### Example 341A: bicyclo[2.2.1]heptane-1,4-dicarboxylic acid

A mixture of dimethyl bicyclo[2.2.1]heptane-1,4-dicarboxylate (1.0 g, 4.71 mmol) and lithium hydroxide monohydrate (0.593 g, 14.13 mmol) in methanol (20 mL) and water (40 mL) was stirred at ambient temperature for 3 days. Volatiles were removed under vacuum, and the residue was acidified with 1 N HCl solution. The white suspension was then extracted with ethyl acetate (100 mL). The organic layer was dried over magnesium sulfate, filtered and concentrated to give 0.85 g of the title compound as a white solid that was used without further purification. ¹H NMR (400 MHz, DMSO-*d*₆) □ ppm 12.16 (s, 2H), 2.01 - 1.83 (m, 4H), 1.73 (d, J = 1.5 Hz, 2H), 1.66 - 1.52 (m, 4H).

### Example 341B: bicyclo[2.2.1]heptane-1,4-diamine dihydrochloride

A mixture of Example 341A (0.87 g, 4.72 mmol), 2,4,6-tripropyl-1,3,5,2,4,6-trioxatriphosphinane 2,4,6-trioxide (7.03 mL of 50% solution in ethyl acetate, 11.81 mmol) and triethylamine (2.96 mL, 21.26 mmol) in toluene (10.0 mL) was treated with azidotrimethylsilane (1.553 mL, 11.81 mmol), and the reaction mixture was stirred at ambient temperature for 2 hours. Volatiles were removed, and the residue was heated at 90 °C for 2 hours. The reaction vessel was cooled to ambient temperature, and 3 N HCl (23.62 mL, 70.9 mmol) was added carefully followed by stirring at 50 °C overnight. The solution was concentrated, and the residue was triturated with acetonitrile. The precipitate was collected by filtration and air-dried to give 0.38 g of the title compound as an off-white solid. ¹H NMR (400 MHz, DMSO-*d*₆) □ ppm 8.82 (s, 6H), 1.99 1.70 (m, 10H).

### Example 341C: N,N'-(bicyclo[2.2.1]heptane-1,4-diyl)bis[2-(4-chloro-3-fluorophenoxy)acetamide]

A mixture of bicyclo[2.2.1]heptane-1,4-diamine dihydrochloride (0.05 g, 0.251 mmol), 2-(3-chloro-4-fluorophenoxy)acetic acid (0.094 g, 0.459 mmol) and *N*-ethyl-*N*-isopropylpropan-2-amine (0.321 mL, 1.838 mmol) in *N,N*-dimethylformamide (1.5 mL) was treated with 2-(3*H-*[1,2,3]triazolo[4,5-*b*]pyridin-3-yl)-1,1,3,3-tetramethylisouronium hexafluorophosphate(V) (0.210 g, 0.551 mmol), and the reaction mixture was stirred at ambient temperature for 30 minutes. Volatiles were removed, and the residue was purified by HPLC (20-100% acetonitrile in 0.1% trifluoroacetic acid/water on a Phenomenex C18 10 µm (250 mm × 50 mm) column at a flow rate of 50 mL/minute) to give 80 mg of the title compound as a white solid. ¹H NMR (400 MHz, DMSO-*d*₆) □ ppm 8.10 (s, 2H), 7.45 (t, J = 8.9 Hz, 2H), 7.01 (dd, J = 11.4, 2.8 Hz, 2H), 6.80 (ddd, J = 8.9, 2.9, 1.2 Hz, 2H), 4.44 (s, 4H), 2.00 (s, 2H), 1.85 (d, J = 6.3 Hz, 4H), 1.85 1.68 (m, 4H); MS (ESI⁺) *m*/*z* 515.9 (M+NH₄)⁺.

### Example 342: 1,4-bis[2-(4-chloro-3-fluorophenoxy)acetamido]bicyclo[2.2.2]octane-2-carboxamide (Compound 441)

A 20 mL vial, equipped with a magnetic stir bar, was charged with hydrogen chloride (12 N, 4 mL, 132 mmol), acetic acid (4 mL, 69.9 mmol) and the product of Example 288 (0.5 g, 0.929 mmol). The reaction was heated at 50 °C for 6 hours and then poured into water. The precipitate was collected by filtration. The precipitate was purified by preparative HPLC (10-95% acetonitrile in 0.1% trifluoroacetic acid/water at 25 mL/minute on a Phenomenex^{®} C18 5 µm column (250 mm × 21.2 mm)) to give the title compound as a white solid (41.5 mg, 8 % yield). ¹H NMR (501 MHz, DMSO-*d6*) *δ* ppm 7.91 (s, 1H), 7.77 (s, 1H), 7.47 (q, J = 8.8 Hz, 2H), 7.09 (s, 1H), 7.04 (ddd, J = 11.4, 5.4, 2.8 Hz, 2H), 6.83 (dddd, J = 9.0, 8.0, 2.9, 1.2 Hz, 3H), 4.61 (d, J = 2.8 Hz, 2H), 4.46 (s, 2H), 1.99 (dd, J = 14.2, 8.7 Hz, 3H), 1.86 (q, J = 9.1, 8.5 Hz, 1H), 1.77 - 1.65 (m, 1H), 1.71 (s, 4H), 1.66 - 1.54 (m, 1H); MS (APCI⁺) *m*/*z* 557.2 (M+H)⁺.

### Example 343: N,N'-[(2S)-2-hydroxybicyclo[2.2.2]octane-1,4-diyl]bis[2-(4-methylphenoxy)acetamide] (Compound 442)

### Example 343A: 4-(benzylamino)-2-oxobicyclo[2.2.2]octane-1-carboxylic acid, hydrochloric acid

A mixture of 198D (HCl salt, 20.7 g, 61.3 mmol) and 25% aqueous sodium hydroxide (49.0 mL, 306 mmol) in methanol (200 mL) and water (200 mL) was stirred for 24 hours at ambient temperature. Volatiles were removed, and the residue was acidified with 1 N HCl. The precipitate was collected by filtration, washed with water, and air-dried to give 16.4 g (86%) of the title compound as a yellow solid. ¹H NMR (400 MHz, DMSO-*d*₆) *δ* ppm 12.70 (s, 1H), 9.67 (s, 2H), 7.62 (dd, J = 7.5, 2.0 Hz, 2H), 7.43 (d, J = 6.6 Hz, 3H), 4.13 (s, 2H), 2.87 (s, 2H), 2.08 (tdq, J = 14.4, 10.8, 5.8, 5.0 Hz, 8H).

### Example 343B: 1-amino-4-(benzylamino)bicyclo[2.2.2]octan-2-one, hydrochloric acid

To a suspension of Example 343A (10.01 g, 32.3 mmol) in toluene (100 mL) was added a 50% ethyl acetate solution of 2,4,6-tripropyl-1,3,5,2,4,6-trioxatriphosphinane 2,4,6-trioxide (22 mL, 37.0 mmol), trimethylsilyl azide (TMS-N₃) (5.0 mL, 37.7 mmol), and triethylamine (11.5 mL, 83 mmol). The mixture was stirred for 30 minutes at room temperature, heated for 2 hours at 85 °C, and then 3 N aqueous hydrochloric acid (86 mL, 258 mmol) was added. The mixture was stirred at 85 °C for 90 minutes and then concentrated. The concentrate was stirred with acetonitrile (150 mL) to precipitate a white solid, which was collected by filtration, washed with acetonitrile (30 mL) and CH₂Cl₂ (25 mL), and vacuum-dried to provide the title compound as an HCl salt (6.244 g, 60.9 % yield). MS (APCI⁺) *m*/*z* 245.0 (M+H)⁺.

### Example 343C: tert-butyl (S)-(4-(benzylamino)-2-hydroxybicyclo[2.2.2]octan-1-yl)carbamate

Example 343B (2.50 g), MgSO₄ (1M, 200 µL) and nicotinamide adenine dinucleotide phosphate (NADPH, 50 mg) were mixed in 50 mL of potassium phosphate buffer (120 mM, pH = 7.0) and 25 mL of isopropanol. To this solution was added Codexis KRED P02C2 enzyme (200 mg) dissolved in 25 mL of the same potassium phosphate buffer. The reaction was stirred overnight. The cloudy, aqueous solution was adjusted to pH > 11 with 50% weight/weight aqueous sodium hydroxide. To this was added 2.58 g (11.58 mmol, 1.5 eq) of di-tert-butyl dicarbonate in 100 mL of ethyl acetate. The biphasic solution was stirred for two hours and monitored as the reaction proceeded. The aqueous layer was routinely checked to maintain pH > 10. At 2 hours, an additional 0.42 mg (0.25 eq) di-tert-butyl dicarbonate was added, and the reaction was continued for an additional hour. The two layers were separated. The aqueous layer was extracted with ethyl acetate (50 mL × 2). The organic layers were combined, washed with brine (30 mL), and concentrated in vacuo. The residue was precipitated in ethyl acetate/hexanes to provide the title compound (1.30 g, 48%). MS (APCI⁺) *m*/*z* 347.4 (M+H)⁺.

### Example 343D: (S)-1,4-diaminobicyclo[2.2.2]octan-2-ol dihydrochloride

Methanol (13.17 mL) and 4 M HCl in dioxane (2.3 mL, 9.20 mmol) was added to Example 343C (1.10 g, 3.17 mmol) and 20% Pd(OH)₂/carbon (0.441 g, 0.321 mmol, 51% in water) in a 50 mL pressure bottle. The mixture was shaken under 60 psi of hydrogen at 40 °C for 16 hours. The reactor was cooled and left shaking for a total of 19.3 hours. The reactor was vented. The reaction mixture was filtered and concentrated. The concentrate was triturated with ethanol. The solid was collected by filtration and vacuum oven-dried to provide the title compound (0.628 g, 86%). This material was used in the next step without further purifications. MS (DCI⁺) *m*/*z* 157.0 (M+H)⁺.

### Example 343E: N,N'-[(2S)-2-hydroxybicyclo[2.2.2]octane-1,4-diyl]bis[2-(4-methylphenoxy)acetamide] (Compound 442)

A mixture of Example 343D (40.0 mg, 0.175 mmol), 1-[bis(dimethylamino)methylene]-1*H*-1,2,3-triazolo[4,5-*b*]pyridinium 3-oxid hexafluorophosphate (HATU, 173 mg, 0.454 mmol), 2-(*p*-tolyloxy)acetic acid (75 mg, 0.454 mmol), and triethylamine (0.122 mL, 0.873 mmol) in tetrahydrofuran (3 mL) was stirred for 3 hours. The reaction was quenched with brine and extracted with ethyl acetate (2×). The combined organic layers were dried over MgSO₄, filtered, and concentrated. The concentrate was dissolved in tetrahydrofuran (1 mL) and methanol (0.6 mL) and treated with 1 N NaOH (0.4 mL). The mixture was stirred for 1 hour, concentrated, diluted with water and brine, and extracted with ethyl acetate (2×). The combined organic layers were dried over MgSO₄ and concentrated. The residue was purified by reverse-phase HPLC (see protocol in Example 112D) to provide the title compound (37.2 mg, 47%). ¹H NMR (500 MHz, DMSO-*d*₆) *δ* ppm 7.35 (s, 1H), 7.17 (s, 1H), 7.12 - 7.03 (m, 4H), 6.85 - 6.72 (m, 4H), 5.15 (d, J = 4.6 Hz, 1H), 4.35 (s, 2H), 4.32 (s, 2H), 4.03 - 3.90 (m, 1H), 2.33 - 2.09 (m, 8H), 1.96 - 1.73 (m, 8H); MS (ESI⁺) *m*/*z* 453.2 (M+H)⁺.

### Example 344: N,N'-[(2S)-2-hydroxybicyclo[2.2.2]octane-1,4-diyl]bis[2-(3,4-dimethylphenoxy)acetamide] (Compound 443)

The reaction described in Example 343E substituting 2-(3,4-dimethylphenoxy)acetic acid for 2-(p-tolyloxy)acetic acid gave the title compound. ¹H NMR (400 MHz, DMSO-*d*₆) *δ* ppm 7.32 (s, 1H), 7.15 (s, 1H), 7.02 (dd, J = 8.4, 4.5 Hz, 2H), 6.73 (dd, J = 8.9, 2.7 Hz, 2H), 6.63 (ddd, J = 10.7, 8.3, 2.8 Hz, 2H), 5.15 (d, J = 4.7 Hz, 1H), 4.33 (d, J = 1.4 Hz, 2H), 4.30 (s, 2H), 3.97 (dt, J = 8.8, 3.8 Hz, 1H), 2.34 - 2.12 (m, 14H), 2.00 - 1.67 (m, 8H); MS (ESI⁺) *m*/*z* 481.3 (M+H)⁺.

### Example 345: 2-(4-chloro-3-fluorophenoxy)-N-{(3S)-4-[2-(4-chloro-3-fluorophenoxy)acetamido]-3-hydroxybicyclo[2.2.2]octan-1-yl}-N-methylacetamide (Compound 444)

### Example 345A: (S)-tert-butyl (4-(benzyl(methyl)amino)-2-hydroxybicyclo[2.2.2]octan-1-yl)carbamate

To a mixture of the product of Example 343C (1.00 g, 2.89 mmol) in CH₂Cl₂ (25 mL) was added acetic acid (0.496 mL, 8.66 mmol), formaldehyde (37% in water, 0.901 mL, 11.55 mmol), and macroporous cyanoborohydride resin (2.32 g, 5.77 mmol, reagent on solid support from Biotage^{®}, 2.49 mmol/g). The reaction mixture was stirred for 3 hours, filtered, and concentrated. The residue was taken up in ethyl acetate and washed with saturated NaHCO₃ and brine. The organic layer was dried over MgSO₄, filtered, and concentrated. The residue was purified on a 40 g silica column using the Biotage^{®} Isolera^{™} One flash system eluting with CH₂Cl₂/CH₃OH (95:5) to provide the title compound (0.599 g, 58%). MS (ESI⁺) *m*/*z* 361.3 (M+H)⁺.

### Example 345B: (S)-1-amino-4-(benzyl(methyl)amino)bicyclo[2.2.2]octan-2-ol, 2 hydrochloric acid

A mixture of Example 345A (0.520 g, 1.442 mmol) and trifluoroacetic acid (1.11 mL, 14.42 mmol) in CH₂Cl₂ (8 mL) was stirred for 6 hours. The reaction mixture was concentrated, and the residue dissolved in CH₃OH (5 mL). To the resulting solution was added 2 N HCl in ether (4 mL), and the mixture was stirred for 15 minutes and then concentrated. The concentrate was suspended in ether, and the mixture was stirred for 15 minutes. The solid was collected by filtration, washed with ether, and vacuum oven-dried to provide title compound (0.415 g, 86%). MS (ESI⁺) *m*/*z* 261.3 (M+H)⁺.

### Example 345C: N-{(2S)-4-[benzyl(methyl)amino]-2-hydroxybicyclo[2.2.2]octan-1-yl}-2-(4-chloro-3-fluorophenoxy)acetamide

A mixture of Example 345B (0.409 g, 1.227 mmol), 1-[bis(dimethylamino)methylene]-1*H*-1,2,3-triazolo[4,5-*b*]pyridinium 3-oxid hexafluorophosphate (0.980 g, 2.58 mmol, HATU), 2-(4-chloro-3-fluorophenoxy)acetic acid (0.527 g, 2.58 mmol), and triethylamine (0.684 mL, 4.91 mmol) in *N,N-*dimethylformamide (6 mL) was stirred for 4 hours. The reaction mixture was quenched with brine and extracted with ethyl acetate (2×). The combined organic layers were dried over MgSO₄, filtered and concentrated. The concentrate was dissolved in CH₃OH (2 mL) and tetrahydrofuran (2 mL) and treated with 2.5 M sodium hydroxide (1.96 mL, 4.91 mmol). The mixture was stirred for 2 hours, quenched with brine, and extracted with ethyl acetate (2×). The combined organic layers were washed with water, dried over MgSO₄, filtered, and concentrated. The residue was purified on a 12 g silica column using the Biotage^{®} Isolera^{™} One flash system eluting with heptanes/ethyl acetate (1:9) to provide the title compound (0.205g, 37%). MS (ESI⁺) *m*/*z* 447.2 (M+H)⁺.

### Example 345D: 2-(4-chloro-3-fluorophenoxy)-N-[(2S)-2-hydroxy-4-(methylamino)bicyclo[2.2.2]octan-1-yl]acetamide, hydrochloric acid

To the product of Example 345C (150 mg, 0.336 mmol) in methanol (3 mL) and 4 M HCl in dioxane (0.252 mL, 1.007 mmol) in a 20 mL Barnstead Hastelloy C reactor was added 20% Pd(OH)₂/carbon (65 mg, 0.047 mmol, 51% in water). The reactor was purged with argon. The mixture was stirred at 1600 RPM under 50 psi of hydrogen at 25 °C. The reactor was vented after 1.6 hours. The reaction mixture was filtered, and the filtrate was concentrated to provide the title compound (0.125 g, 95%) that was used in the nest step without further purifications. MS (ESI⁺) *m*/*z* 357.2 (M+H)⁺.

### Example 345E: 2-(4-chloro-3-fluorophenoxy)-N-{(3S)-4-[2-(4-chloro-3-fluorophenoxy)acetamido]-3-hydroxybicyclo[2.2.2]octan-1-yl}-N-methylacetamide (Compound 444)

A mixture of the product of Example 345D (30.0 mg, 0.076 mmol), 2-(4-chloro-3-fluorophenoxy)acetic acid (18.73 mg, 0.092 mmol), 1-[bis(dimethylamino)methylene]-1*H*-1,2,3-triazolo[4,5-*b*]pyridinium 3-oxid hexafluorophosphate, (HATU, 34.8 mg, 0.092 mmol), and triethylamine (0.032 mL, 0.229 mmol) in *N,N*-dimethylformamide (2.5 mL) was stirred for 1.5 hours. The reaction was quenched with brine, and the mixture was extracted with ethyl acetate (2×). The combined organic layers were dried over MgSO₄, filtered, and concentrated. The residue was purified by reverse-phase HPLC (see protocol in Example 112D) to provide the title compound (18.7 mg, 45%). ¹H NMR (400 MHz, DMSO-*d*₆) *δ* ppm 7.46 (dt, J = 16.0, 8.9 Hz, 2H), 7.26 (s, 1H), 7.05 (dd, J = 11.4, 2.9 Hz, 1H), 6.98 (dd, J = 11.6, 2.8 Hz, 1H), 6.83 (ddd, J = 9.0, 2.9, 1.2 Hz, 1H), 6.76 (ddd, J = 9.0, 2.9, 1.2 Hz, 1H), 5.08 (d, J = 4.3 Hz, 1H), 4.78 (s, 2H), 4.46 (s, 2H), 4.02 (dt, J = 8.7, 3.9 Hz, 1H), 2.80 (s, 3H), 2.41 (ddd, J = 12.4, 9.5, 2.5 Hz, 1H), 2.13 - 1.72 (m, 9H); MS (ESI⁺) *m*/*z* 543.0 (M+H)⁺.

### Example 346: (2S)-1,4-bis[2-(4-chloro-3-fluorophenoxy)acetamido]bicyclo[2.2.2]octan-2-yl acetate (Compound 445)

To a suspension of Example 198K (7.80 g, 14.7 mmol), *N,N-*dimethylpyridin-4-amine (1.88 g, 15.4 mmol), and triethylamine (2.70 mL, 19.4 mmol) in CH₂Cl₂ (80 mL) at 0 °C was added acetyl chloride (1.35 mL, 19.0 mmol). The ice-bath was removed and the mixture was stirred for 30 minutes. The material was concentrated under reduced pressure, and the residue was diluted with ethyl acetate. The mixture was washed sequentially with 1 N HCl (40 mL), water (25 mL), saturated aqueous NaHCO₃ (25 mL), and brine (25 mL). The organic layer was dried over anhydrous Na₂SO₄, and adsorbed onto silica. Purification by column chromatography (SiO₂, 25-30% ethyl acetate/dichloromethane) gave 7.78 g of racemic title compound, 1,4-bis[2-(4-chloro-3-fluorophenoxy)acetamido]bicyclo[2.2.2]octan-2-yl acetate. This material was then purified by chiral SFC (supercritical fluid chromatography) using a Chiralpak^{®} AD-H column eluting with 40% CH₃OH in CO₂ to give the title compound (first enantiomer eluted out of the column (2.97 g, 5.2 mmol, 35% yield). ¹H NMR (501 MHz, DMSO-*d*₆) *δ* ppm 7.60 (s, 1H), 7.55 (s, 1H), 7.47 (td, *J =* 8.9, 2.0 Hz, 2H), 7.00 (ddd, *J =* 16.6, 11.4, 2.9 Hz, 2H), 6.79 (dddd, *J* = 8.6, 7.1, 2.8, 1.2 Hz, 2H), 5.28 (dd, *J =* 9.6, 2.4 Hz, 1H), 4.49 - 4.39 (m, 4H), 2.39 (ddd, *J =* 14.0, 9.4, 2.9 Hz, 1H), 2.26 - 2.17 (m, 1H), 2.05 - 1.99 (m, 1H), 1.98 (s, 3H), 1.93 (dd, J= 9.1, 6.9 Hz, 2H), 1.90 - 1.75 (m, 5H); MS (ESI⁺) *m*/*z* 571 (M+H)⁺.

### Example 347: 2-(4-chloro-3-fluorophenoxy)-N-[3-(2-phenylacetamido)bicyclo[1.1.1]pentan-1-yl]acetamide (Compound 446)

The reaction and purification conditions described in Example 81, substituting phenylacetic acid for 2-(4-chloro-3-fluorophenoxy)acetic acid and the product of Example 27D for benzyl (4-aminobicyclo[2.1.1]hexan-1-yl)carbamate hydrochloride gave the title compound. ¹H NMR (501 MHz, DMSO-*d*₆) *δ* ppm 8.68 (s, 1H), 8.64 (s, 1H), 7.49 (t, J = 8.9 Hz, 1H), 7.32 - 7.26 (m, 2H), 7.25 - 7.19 (m, 3H), 7.06 (dd, J = 11.4, 2.9 Hz, 1H), 6.84 (ddd, J = 8.9, 2.8, 1.2 Hz, 1H), 4.46 (s, 2H), 3.35 (s, 2H), 2.21 (s, 6H); MS (ESI⁺) *m*/*z* 403 (M+H)⁺.

### Example 348: 2-(3,4-dichlorophenoxy)-N-{3-[2-(pyridin-2-yl)acetamido]bicyclo[1.1.1]pentan-1-yl}acetamide (Compound 447)

The reaction and purification conditions described in Example 81, substituting 2-pyridylacetic acid hydrochloride for 2-(4-chloro-3-fluorophenoxy)acetic acid and the product of Example 6C for benzyl (4-aminobicyclo[2.1.1]hexan-1-yl)carbamate hydrochloride gave the title compound. ¹H NMR (400 MHz, DMSO-*d*₆) *δ* ppm 8.70 (s, 1H), 8.69 (s, 1H), 8.46 (ddd, J = 4.8, 1.8, 0.9 Hz, 1H), 7.72 (td, J = 7.7, 1.9 Hz, 1H), 7.54 (d, J = 8.9 Hz, 1H), 7.30 (dt, J = 7.8, 1.1 Hz, 1H), 7.27 - 7.22 (m, 2H), 6.98 (dd, J = 9.0, 2.9 Hz, 1H), 4.48 (s, 2H), 3.56 (s, 2H), 2.22 (s, 6H); MS (ESI⁺) *m*/*z* 420 (M+H)⁺.

### Example 349: 2-(4-chloro-3-fluorophenoxy)-N-{3-[2-(pyridin-2-yl)acetamido]bicyclo[1.1.1]pentan-1-yl}acetamide (Compound 448)

The reaction and purification conditions described in Example 81, substituting 2-pyridylacetic acid hydrochloride for 2-(4-chloro-3-fluorophenoxy)acetic acid and the product of Example 27D for benzyl (4-aminobicyclo[2.1.1]hexan-1-yl)carbamate hydrochloride gave the title compound. ¹H NMR (400 MHz, DMSO-*d*₆) *δ* ppm 8.70 (s, 1H), 8.69 (s, 1H), 8.46 (ddd, J = 4.9, 1.9, 1.0 Hz, 1H), 7.72 (td, J = 7.7, 1.8 Hz, 1H), 7.49 (dd, J = 9.8, 8.1 Hz, 1H), 7.30 (dt, J = 7.8, 1.2 Hz, 1H), 7.24 (ddd, J = 7.6, 4.8, 1.3 Hz, 1H), 7.07 (dd, J = 11.4, 2.8 Hz, 1H), 6.85 (ddd, J = 9.0, 2.8, 1.3 Hz, 1H), 4.47 (s, 2H), 3.56 (s, 2H), 2.22 (s, 6H); MS (ESI⁺) *m*/*z* 404 (M+H)⁺.

### Example 350: (2R)-1,4-bis[2-(4-chloro-3-fluorophenoxy)acetamido]bicyclo[2.2.2]octan-2-yl acetate (Compound 449)

The second eluting enantiomer from the chiral separation described in Example 346 was the title compound (3.23 g, 5.7 mmol, 38% yield). ¹H NMR (501 MHz, DMSO-*d*₆) *δ* ppm 7.60 (s, 1H), 7.55 (s, 1H), 7.47 (td, *J =* 8.9, 2.0 Hz, 2H), 7.00 (ddd, *J =* 16.6, 11.4, 2.9 Hz, 2H), 6.79 (dddd, *J =* 8.6, 7.1, 2.8, 1.2 Hz, 2H), 5.28 (dd, *J =* 9.6, 2.4 Hz, 1H), 4.49 - 4.39 (m, 4H), 2.39 (ddd, *J =* 14.0, 9.4, 2.9 Hz, 1H), 2.26 - 2.17 (m, 1H), 2.05 - 1.99 (m, 1H), 1.98 (s, 3H), 1.93 (dd, *J =* 9.1, 6.9 Hz, 2H), 1.90 - 1.75 (m, 5H); MS (ESI⁺) *m*/*z* 571 (M+H)⁺.

### Example 351: (2R)-1-[2-(4-chloro-3-fluorophenoxy)acetamido]-4-{[2-(4-chloro-3-fluorophenoxy)ethyl]amino}bicyclo[2.2.2]octan-2-yl acetate (Compound 450)

A suspension of Example 350 (0.114 g, 0.2 mmol), indium(III) bromide (0.106 g, 0.300 mmol) and triethylsilane (0.319 mL, 2.000 mmol) in dichloromethane (1 mL) was stirred under nitrogen at 60 °C for 1 hour. The reaction was quenched with water and concentrated under reduced pressure. The residue was purified by preparative HPLC (10-95% acetonitrile in 0.1% trifluoroacetic acid/water at 25 mL/minute on a Phenomenex^{®} C18 5 µm column (250 mm × 21.2 mm)) to give the title compound as a white solid (15 mg, 13% yield). ¹H NMR (400 MHz, DMSO-*d*₆) *δ* ppm8.85 (d, J = 11.2 Hz, 2H), 7.73 (s, 1H), 7.50 (dt, J = 20.1, 8.9 Hz, 2H), 7.11 (dd, J = 11.3, 2.9 Hz, 1H), 6.99 (dd, J = 11.4, 2.9 Hz, 1H), 6.88 (ddd, J = 9.0, 2.9, 1.2 Hz, 1H), 6.79 (ddd, J = 9.0, 2.9, 1.2 Hz, 1H), 5.34 (dd, J = 9.4, 2.1 Hz, 1H), 4.46 (d, J = 2.0 Hz, 2H), 4.21 (t, J = 5.0 Hz, 2H), 3.31 (s, 2H), 2.48 - 2.37 (m, 1H), 2.29 (t, J = 10.0 Hz, 1H), 2.00 (s, 5H), 1.92 - 1.84 (m, 3H), 1.81 (q, J = 11.6 Hz, 1H), 1.72 (dt, J = 13.8, 2.4 Hz, 1H); MS (APCI⁺) *m*/*z* 558.2 (M+H)⁺.

### Example 352: 2-(3,4-dichlorophenoxy)-N-[3-({[(3-fluorophenyl)methyl]carbamoyl}amino)bicyclo[1.1.1]pentan-1-yl]acetamide (Compound 451)

The reaction and purification conditions described in Example 332, substituting 3-fluorobenzyl isocyanate for 4-chlorophenyl isocyanate and the product of Example 6C for the product of Example 27D gave the title compound. ¹H NMR (501 MHz, DMSO-*d*₆) *δ* ppm 8.66 (s, 1H), 7.54 (d, J = 9.0 Hz, 1H), 7.38 - 7.31 (m, 1H), 7.26 (d, J = 2.9 Hz, 1H), 7.09 - 7.01 (m, 3H), 6.98 (dd, J = 9.0, 2.9 Hz, 1H), 6.71 (s, 1H), 6.30 (t, J = 6.1 Hz, 1H), 4.48 (s, 2H), 4.19 (d, J = 6.0 Hz, 2H), 2.18 (s, 6H); MS (ESI⁺) *m*/*z* 452 (M+H)⁺.

### Example 353: 2-(4-chloro-3-fluorophenoxy)-N-(3S)-4-[2-(3,4-dichlorophenoxy)acetamido]-3-hydroxybicyclo[2.2.2]octan-1-yl}acetamide (Compound 452)

The title compound was prepared using the methodologies described in Example 338C substituting Example 276A for Example 338B and 2-(3,4-dichlorophenoxy)acetic acid for 2-(4-chlorophenoxy)acetic acid. ¹H NMR (400 MHz, DMSO-*d*₆) □ ppm 7.57 - 7.43 (m, 3H), 7.29 - 7.18 (m, 2H), 7.02 (dd, J = 11.4, 2.8 Hz, 1H), 6.96 (dd, J = 8.9, 2.9 Hz, 1H), 6.81 (ddd, J = 9.0, 2.9, 1.2 Hz, 1H), 5.08 (d, J = 4.4 Hz, 1H), 4.46 (d, J = 17.4 Hz, 4H), 4.04 (dt, J = 8.9, 4.0 Hz, 1H), 2.27 (ddd, J = 12.4, 9.4, 2.3 Hz, 1H), 2.07 (ddd, J = 12.2, 10.5, 4.6 Hz, 1H), 1.98 - 1.89 (m, 3H), 1.93 - 1.81 (m, 1H), 1.85 - 1.72 (m, 4H); MS (ESI⁺) *m*/*z* 542.9 (M-H)⁻.

### Example 354: N-{(2S)-4-[2-(4-chloro-3-fluorophenoxy)acetamido]-2-hydroxybicyclo[2.2.2]octan-1-yl}-2-(4-chlorophenoxy)-2-methylpropanamide (Compound 453)

The title compound was prepared using the methodologies described in Example 338C substituting Example 276A for Example 338B and 2-(4-chlorophenoxy)-2-methylpropanoic acid for 2-(4-chlorophenoxy)acetic acid. ¹H NMR (400 MHz, DMSO-*d*₆) □ ppm 7.52 - 7.43 (m, 2H), 7.38 - 7.29 (m, 2H), 7.10 (s, 1H), 7.02 (dd, J = 11.4, 2.8 Hz, 1H), 6.96 - 6.87 (m, 2H), 6.80 (ddd, J = 9.0, 2.9, 1.2 Hz, 1H), 4.43 (s, 2H), 3.83 (ddd, J = 9.6, 3.7, 1.6 Hz, 1H), 2.49 - 2.37 (m, 1H), 2.28 (ddd, J = 12.6, 9.5, 2.7 Hz, 1H), 2.07 - 1.94 (m, 1H), 1.91 (dd, J = 15.1, 10.6 Hz, 1H), 1.79 (tdd, J = 13.3, 8.3, 3.0 Hz, 5H), 1.66 - 1.49 (m, 1H), 1.38 (d, J = 5.9 Hz, 6H); MS (ESI⁺) *m*/*z* 539.1 (M-H)⁻.

### Example 355: 2-(4-chloro-3-fluorophenoxy)-N-(3S)-4-[2-(4-chlorophenoxy)acetamido]-3-hydroxybicyclo[2.2.2]octan-1-yl}acetamide (Compound 454)

The title compound was prepared using the methodologies described in Example 338C substituting Example 276A for Example 338B. ¹H NMR (400 MHz, DMSO-*d*₆) □ ppm 7.53 - 7.43 (m, 2H), 7.38 - 7.29 (m, 2H), 7.23 (s, 1H), 7.02 (dd, J = 11.4, 2.9 Hz, 1H), 7.02 - 6.91 (m, 2H), 6.81 (ddd, J = 9.0, 2.9, 1.2 Hz, 1H), 5.11 (d, J = 4.4 Hz, 1H), 4.43 (d, J = 5.6 Hz, 4H), 4.11 - 3.96 (m, 1H), 2.27 (ddd, J = 12.6, 9.5, 2.4 Hz, 1H), 2.17 - 2.03 (m, 1H), 1.99 - 1.82 (m, 3H), 1.86 - 1.72 (m, 5H); MS (ESI⁺) *m*/*z* 511.1 (M+H)⁺.
The compounds in the following table were prepared using the methodologies described above.

| Example | Name (Compound Number) | NMR | MS |
|---|---|---|---|
| Example 356 | *N*-{3-[2-(4-chloro-3-fluorophenoxy)acetamido]bi cyclo[1.1.1]pentan-1-yl}-3-(2,5-dimethoxyphenyl)propanam ide (Compound 455) | | MS (APCI⁺) |
| | | | *m*/*z* 477 (M+H)⁺ |
| Example 357 | 2-(4-chloro-3-fluorophenoxy)-*N*-{3-[2-(3,4-dimethylphenoxy)acetamido ]bicyclo[1.1.1]pentan-1-yl}acetamide (Compound 456) | ¹H NMR (501 MHz, DMSO-*d*_{6_}D₂O) *δ* ppm 7.49 (t, J = 8.8 Hz, 1H), 7.08 - 7.02 (m, 2H), 6.86 (ddd, J = 9.0, 2.9, 1.2 Hz, 1H), 6.77 (d, J = 2.7 Hz, 1H), 6.66 (dd, J = 8.3, 2.8 Hz, 1H), 4.47 (s, 2H), 4.35 (s, 2H), 2.28 (s, 6H), 2.18 (s, 3H), 2.14 (s, 3H) | MS (APCI⁺) |
| | | | *m*/*z* 447 (M+H)⁺ |
| Example 358 | 2-(4-chloro-3-fluorophenoxy)-*N*-(3-{2-[3-(trifluoromethyl)phenyl]acet amido}bicyclo[1.1.1]pentan -1-yl)acetamide (Compound 457) | | MS (APCI⁺) |
| | | | *m*/*z* 471 (M+H)⁺ |
| Example 359 | 2-(4-chloro-3-fluorophenoxy)-*N*-{3-[2-(pyridin-3-yl)acetamido]bicyclo[1.1.1] pentan-1-yl}acetamide (Compound 458) | | MS (APCI⁺) |
| | | | *m*/*z* 404 (M+H)⁺ |
| Example 360 | 2-(4-chloro-3-fluorophenoxy)-*N*-{3-[2-(pyridin-4-yl)acetamido]bicyclo[1.1.1] pentan-1-yl}acetamide (Compound 459) | | MS (APCI⁺) |
| | | | *m*/*z* 404 (M+H)⁺ |
| Example 361 | 2-(4-chloro-3-fluorophenoxy)-*N*-{3-[2-(4-methylphenoxy)acetamido] bicyclo[1.1.1]pentan-1-yl}acetamide (Compound 460) | ¹H NMR (501 MHz, DMSO-*d*_{6_}D₂O) *δ* ppm 7.49 (t, J = 8.9 Hz, 1H), 7.13 - 7.09 (m, 2H), 7.06 (dd, J = 11.3, 2.8 Hz, 1H), 6.89 - 6.83 (m, 3H), 4.47 (s, 2H), 4.37 (s, 2H), 2.28 (s, 6H), 2.24 (s, 3H) | MS (APCI⁺) |
| | | | *m*/*z* 433 (M+H)⁺ |
| Example 362 | *N*-{3-[2-(4-chloro-3-fluorophenoxy)acetamido]bi cyclo[1.1.1]pentan-1-yl}-3-(3-methoxyphenyl)propanamide (Compound 461) | | MS (APCI⁺) |
| | | | *m*/*z* 447 (M+H)⁺ |
| Example 363 | (2*R*)-*N*-{3-[2-(4-chloro-3-fluorophenoxy)acetamido]bi cyclo[1.1.1]pentan-1-yl}-2-hydroxy-4-phenylbutanamide (Compound 462) | | MS (APCI⁺) |
| | | | *m*/*z* 447 (M+H)⁺ |
| Example 364 | 2-(4-chloro-3-fluorophenoxy)-*N*-{(2*S*)-4-[2-(3-ethyl-5-methylphenoxy)acetamido]-2-hydroxybicyclo[2.2.2]octan-1-yl}acetamide (Compound 463) | ¹H NMR (400 MHz, DMSO-*d*₆) *δ* ppm 7.53 - 7.40 (m, 2H), 7.31 (s, 1H), 7.04 (dd, J = 11.3, 2.8 Hz, 1H), 6.84 (ddd, J = 8.9, 2.9, 1.2 Hz, 1H), 6.63 (d, J = 1.5 Hz, 1H), 6.56 (d, J = 8.7 Hz, 2H), 4.46 (s, 2H), 4.30 (d, J = 16.9 Hz, 2H), 4.11 - 4.03 (m, 1H), 2.50 (m, 2H), 2.35 - 2.25 (m, 1H), 2.21 (d, J = 18.2 Hz, 3H), 2.02 (dt, J = 9.4, 6.8 Hz, 1H), 1.91 (dd, J = 21.6, 10.7 Hz, 3H), 1.84 - 1.72 (m, 5H), 1.14 (t, J = 7.6 Hz, 3H) | MS (APCI⁺) |
| | | | *m*/*z* 519.3 (M+H)⁺ |
| Example 365 | 2-(4-chloro-3-fluorophenoxy)-*N*-{(2*S*)-4-[2-(4-ethoxyphenoxy)acetamido]-2-hydroxybicyclo[2.2.2]octan-1-yl}acetamide (Compound 464) | ¹H NMR (400 MHz, DMSO-*d*₆) *δ* ppm 7.48 (t, J = 8.9 Hz, 1H), 7.04 (dd, J = 11.3, 2.8 Hz, 1H), 6.85 (s, 4H), 6.83 (dd, J = 2.9, 1.2 Hz, 1H), 4.46 (s, 2H), 4.29 (s, 2H), 4.07 (dd, J = 9.5, 3.1 Hz, 1H), 3.95 (q, J = 6.9 Hz, 2H), 2.36 - 2.24 (m, 1H), 2.03 (ddd, J = 12.2, 9.9, 5.5 Hz, 1H), 1.97 - 1.71 (m, 8H), 1.29 (t, J = 7.0 Hz, 3H) | MS (APCI⁺) |
| | | | *m*/*z* 521.2 (M+H)⁺ |
| Example 366 | 2-(4-chloro-3-fluorophenoxy)-*N*-{(2*S*)-4-[2-(4-chloro-3-methoxyphenoxy)acetamido ]-2-hydroxybicyclo[2.2.2]octan-1-yl}acetamide (Compound 465) | ¹H NMR (400 MHz, DMSO-*d*₆) *δ* ppm 7.48 (t, J = 8.9 Hz, 1H), 7.30 (d, J = 8.8 Hz, 1H), 7.04 (dd, J = 11.4, 2.9 Hz, 1H), 6.84 (ddd, J = 9.0, 2.9, 1.2 Hz, 1H), 6.72 (d, J = 2.7 Hz, 1H), 6.51 (dd, J = 8.8, 2.7 Hz, 1H), 4.43 (d, J = 24.2 Hz, 4H), 4.07 (dd, J = 9.6, 3.1 Hz, 1H), 3.82 (s, 3H), 2.30 (ddd, J = 12.1, 9.6, 1.8 Hz, 1H), 2.03 (ddd, J = 12.2, 10.0, 5.5 Hz, 1H), 1.91 (dd, J = 21.7, 10.6 Hz, 3H), 1.87 - 1.72 (m, 5H) | MS (APCI⁺) |
| | | | *m*/*z* 541.1 (M+H)⁺ |
| Example 367 | 2-(4-chloro-3-fluorophenoxy)-*N*-[(2*S*)-2-hydroxy-4-{2-[3-(trifluoromethyl)phenoxy]ac etamido }bicyclo[2.2.2]octa n-1-yl]acetamide (Compound 466) | ¹H NMR (400 MHz, DMSO-*d*₆) *δ* ppm 7.58 - 7.42 (m, 2H), 7.36 - 7.28 (m, 1H), 7.26 - 7.19 (m, 2H), 7.13 - 7.00 (m, 1H), 6.84 (ddd, J = 9.0, 2.9, 1.2 Hz, 1H), 4.47 (d, J = 12.3 Hz, 4H), 4.07 (dd, J = 9.6, 3.1 Hz, 1H), 2.35 - 2.24 (m, 1H), 2.03 (ddd, J = 12.2, 10.2, 5.2 Hz, 1H), 1.98 - 1.84 (m, 3H), 1.87 - 1.72 (m, 5H). | MS (APCI⁺) |
| | | | *m*/*z* 545.2 (M+H)⁺ |
| Example 368 | 2-(4-chloro-3-fluorophenoxy)-*N*-{(2*S*)-2-hydroxy-4-[2-(3-methylphenoxy)acetamido] bicyclo[2.2.2]octan-1-yl}acetamide (Compound 467) | ¹H NMR (400 MHz, DMSO-*d*₆) *δ* ppm 7.47 (q, J = 8.4, 7.8 Hz, 1H), 7.17 (t, J = 7.8 Hz, 1H), 7.13 - 7.00 (m, 1H), 6.88 - 6.67 (m, 4H), 4.46 (s, 2H), 4.34 (s, 2H), 4.07 (dd, J = 9.7, 3.1 Hz, 1H), 2.36 - 2.27 (m, 1H), 2.27 (s, 3H), 2.03 (ddd, J = 12.2, 10.0, 5.5 Hz, 1H), 1.98 - 1.84 (m, 3H), 1.87 - 1.72 (m, 5H) | MS (APCI⁺) |
| | | | *m*/*z* 491.2 (M+H)⁺ |
| Example 369 | 2-(4-chloro-3-fluorophenoxy)-*N*-[(2*S*)-2-hydroxy-4-{2-[4-(trifluoromethyl)phenoxy]ac etamido }bicyclo[2.2.2]octa n-1-yl]acetamide (Compound 468) | ¹H NMR (400 MHz, DMSO-*d*₆) *δ* ppm 7.70 - 7.59 (m, 2H), 7.48 (t, J = 8.9 Hz, 1H), 7.16 - 7.00 (m, 3H), 6.84 (ddd, J = 9.0, 2.9, 1.2 Hz, 1H), 4.47 (d, J = 12.3 Hz, 4H), 4.07 (dd, J = 9.5, 3.1 Hz, 1H), 2.36 - 2.25 (m, 1H), 2.03 (ddd, J = 12.2, 10.2, 5.3 Hz, 1H), 1.95 (s, 1H), 1.95 - 1.72 (m, 7H) | MS (APCI⁺) |
| | | | *m*/*z* 545.2 (M+H)⁺ |
| Example 370 | 2-(4-chloro-3-fluorophenoxy)-*N*-{(2*S*)-2-hydroxy-4-[2-(3,4,5-trimethylphenoxy)acetamid o]bicyclo[2.2.2]octan-1-yl}acetamide (Compound 469) | ¹H NMR (400 MHz, DMSO-*d*₆) *δ* ppm 7.48 (t, J = 8.9 Hz, 1H), 7.31 (s, 1H), 7.04 (dd, J = 11.3, 2.8 Hz, 1H), 6.84 (ddd, J = 9.0, 2.9, 1.2 Hz, 1H), 6.57 (s, 2H), 4.46 (s, 2H), 4.28 (s, 2H), 4.07 (dd, J = 9.4, 3.1 Hz, 1H), 2.30 (dd, J = 13.1, 9.8 Hz, 1H), 2.19 (s, 6H), 2.07 - 2.00 (m, 1H), 2.03 (s, 3H), 1.91 (dd, J = 21.4, 10.6 Hz, 3H), 1.84 - 1.72 (m, 5H) | MS (APCI⁺) |
| | | | *m*/*z* 519.2 (M+H)⁺ |
| Example 371 | 2-(4-chloro-3-fluorophenoxy)-*N*-{(2*S*)-4-[2-(3,5-dimethylphenoxy)acetamido ]-2-hydroxybicyclo[2.2.2]octan-1-yl}acetamide (Compound 470) | ¹H NMR (400 MHz, DMSO-*d*₆) *δ* ppm 7.48 (t, J = 8.9 Hz, 1H), 7.40 (s, 1H), 7.31 (s, 1H), 7.04 (dd, J = 11.3, 2.9 Hz, 1H), 6.84 (ddd, J = 9.0, 2.9, 1.2 Hz, 1H), 6.61 (s, 1H), 6.53 (s, 2H), 4.46 (s, 2H), 4.31 (s, 2H), 4.07 (dd, J = 9.6, 3.0 Hz, 1H), 2.35 - 2.25 (m, 1H), 2.22 (s, 6H), 2.08 - 1.97 (m, 1H), 1.97 - 1.84 (m, 3H), 1.84 - 1.72 (m, 5H) | MS (APCI⁺) |
| | | | *m*/*z* 505.2 (M+H)⁺ |
| Example 372 | 2-(4-chloro-3-fluorophenoxy)-*N*-{(2*S*)-4-[2-(3,4-dimethylphenoxy)acetamido ]-2-hydroxybicyclo[2.2.2]octan-1-yl}acetamide (Compound 471) | ¹H NMR (400 MHz, DMSO-*d*₆) *δ* ppm 7.48 (t, J = 8.9 Hz, 1H), 7.39 (s, 1H), 7.31 (s, 1H), 7.08 - 6.99 (m, 2H), 6.84 (ddd, J = 8.9, 2.9, 1.2 Hz, 1H), 6.73 (d, J = 2.7 Hz, 1H), 6.63 (dd, J = 8.3, 2.8 Hz, 1H), 4.46 (s, 2H), 4.30 (s, 2H), 4.07 (dd, J = 9.6, 3.1 Hz, 1H), 2.35 - 2.25 (m, 1H), 2.25 - 2.11 (m, 6H), 2.09 - 1.97 (m, 1H), 1.91 (dd, J = 21.5, 10.5 Hz, 3H), 1.84 - 1.71 (m, 5H) | MS (APCI⁺) |
| | | | *m*/*z* 505.2 (M+H)⁺ |
| Example 373 | 2-(4-chloro-3-fluorophenoxy)-*N*-[(2*S*)-2-hydroxy-4-{2-[(naphthalen-2-yl)oxy]acetamido}bicyclo[2 .2.2]octan-1-yl] acetamide (Compound 472) | ¹H NMR (400 MHz, DMSO-*d*₆) *δ* ppm 7.89 - 7.74 (m, 3H), 7.58 (s, 1H), 7.53 - 7.43 (m, 2H), 7.42 - 7.29 (m, 2H), 7.24 (d, J = 7.6 Hz, 2H), 7.04 (dd, J = 11.3, 2.9 Hz, 1H), 6.84 (ddd, J = 9.0, 2.9, 1.2 Hz, 1H), 4.48 (d, J = 19.6 Hz, 4H), 4.08 (dd, J = 9.6, 3.1 Hz, 1H), 2.38 - 2.27 (m, 1H), 2.10 - 1.74 (m, 9H) | MS (APCI⁺) |
| | | | *m*/*z* 527.2 (M+H)⁺ |
| Example 374 | 2-(4-chloro-3-fluorophenoxy)-*N*-[(2*S*)-2-hydroxy-4-{2-[4-(trifluoromethoxy)phenoxy] acetamido}bicyclo[2.2.2]oct an-1-yl]acetamide (Compound 473) | ¹H NMR (400 MHz, DMSO-*d*₆) *δ* ppm 7.48 (t, J = 8.9 Hz, 1H), 7.34 - 7.24 (m, 2H), 7.08 - 6.97 (m, 3H), 6.84 (ddd, J = 9.0, 2.9, 1.2 Hz, 1H), 4.44 (d, J = 18.6 Hz, 4H), 4.07 (dd, J = 9.4, 3.1 Hz, 1H), 2.36 - 2.25 (m, 1H), 2.03 (ddd, J = 12.0, 10.0, 5.3 Hz, 1H), 1.94 (d, J = 10.0 Hz, 2H), 1.92 - 1.72 (m, 6H) | MS (APCI⁺) |
| | | | *m*/*z* 561.2 (M+H)⁺ |
| Example 375 | 2-(4-chloro-3-fluorophenoxy)-*N*-[(2*S*)-2-hydroxy-4-{2-[4-(propane-1-sulfonyl)phenoxy]acetamid o}bicyclo[2.2.2]octan-1-yl]acetamide (Compound 474) | ¹H NMR (400 MHz, DMSO-*d*₆) *δ* ppm 7.84 - 7.75 (m, 2H), 7.64 (s, 1H), 7.48 (t, J = 8.9 Hz, 1H), 7.31 (s, 1H), 7.18 - 7.09 (m, 2H), 7.04 (dd, J = 11.3, 2.8 Hz, 1H), 6.84 (ddd, J = 8.9, 2.9, 1.2 Hz, 1H), 4.52 (s, 2H), 4.46 (s, 2H), 4.11 - 4.93 (m, 1H), 3.24 - 3.15 (m, 2H), 2.35 - 2.25 (m, 1H), 2.09 - 1.72 (m, 8H), 1.62 - 1.47 (m, 2H), 0.91 (t, J = 7.4 Hz, 3H) | MS (APCI⁺) |
| | | | *m*/*z* 583.2 (M+H)⁺ |
| Example 376 | 2-(4-chloro-3-fluorophenoxy)-*N*-{(2*S*)-2-hydroxy-4-[2-(4-methylphenoxy)acetamido] bicyclo[2.2.2]octan-1-yl}acetamide (Compound 475) | ¹H NMR (400 MHz, DMSO-*d*₆) *δ* ppm 7.48 (t, J = 8.9 Hz, 1H), 7.14 - 7.00 (m, 3H), 6.88 - 6.77 (m, 3H), 4.46 (s, 2H), 4.32 (s, 2H), 4.07 (dd, J = 9.7, 3.1 Hz, 1H), 2.35 - 2.25 (m, 1H), 2.23 (s, 3H), 2.08 - 1.97 (m, 1H), 1.97 - 1.71 (m, 8H) | MS (APCI⁺) |
| | | | *m*/*z* 491.2 (M+H)⁺ |
| Example 377 | 2-(4-chloro-3-fluorophenoxy)-*N*-[(2*S*)-2-hydroxy-4-{2-[4-(methanesulfonyl)phenoxy] acetamido}bicyclo[2.2.2]oct an-1-yl]acetamide (Compound 476) | ¹H NMR (400 MHz, DMSO-*d*₆) *δ* ppm 7.89 - 7.80 (m, 2H), 7.48 (t, J = 8.9 Hz, 1H), 7.17 - 7.09 (m, 2H), 7.04 (dd, J = 11.4, 2.9 Hz, 1H), 6.84 (ddd, J = 9.0, 2.9, 1.2 Hz, 1H), 4.52 (s, 2H), 4.46 (s, 2H), 4.08 (dd, J = 9.6, 3.1 Hz, 1H), 3.15 (s, 3H), 2.31 (ddd, J = 13.4, 9.7, 1.7 Hz, 1H), 2.09 - 1.90 (m, 3H), 1.94 - 1.85 (m, 1H), 1.88 - 1.72 (m, 5H) | MS (APCI⁺) |
| | | | *m*/*z* 555.2 (M+H)⁺ |
| Example 378 | 2-(4-*tert*-butylphenoxy)-*N-*{(3*S*)-4-[2-(4-chloro-3-fluorophenoxy)acetamido]-3-hydroxybicyclo[2.2.2]octan-1-yl}acetamide (Compound 477) | ¹H NMR (400 MHz, DMSO-*d*₆) *δ* ppm 7.53 - 7.42 (m, 1H), 7.34 - 7.25 (m, 2H), 7.04 (dd, J = 11.3, 2.8 Hz, 1H), 6.88 - 6.79 (m, 3H), 4.46 (s, 2H), 4.34 (s, 2H), 4.07 (dd, J = 9.5, 3.1 Hz, 1H), 2.36 - 2.25 (m, 1H), 2.03 (ddd, J = 12.1, 10.1, 5.5 Hz, 1H), 1.91 (dd, J = 22.3, 10.5 Hz, 3H), 1.87 - 1.72 (m, 5H), 1.25 (s, 9H) | MS (APCI⁺) |
| | | | *m*/*z* 533.1 (M+H)⁺ |
| Example 379 | 2-(4-chloro-3-fluorophenoxy)-*N*-[(2*S*)-4-{2-[3,5-di(propan-2-yl)phenoxy]acetamido}-2-hydroxybicyclo[2.2.2]octan-1-yl]acetamide (Compound 478) | ¹H NMR (400 MHz, DMSO-*d*₆) *δ* ppm 7.48 (t, J = 8.9 Hz, 1H), 7.04 (dd, J = 11.3, 2.8 Hz, 1H), 6.84 (ddd, J = 9.0, 2.9, 1.2 Hz, 1H), 6.70 (d, J = 1.5 Hz, 1H), 6.58 (d, J = 1.5 Hz, 2H), 4.46 (s, 2H), 4.35 (s, 2H), 4.07 (dd, J = 9.5, 3.1 Hz, 1H), 2.81 (hept, J = 6.9 Hz, 2H), 2.35 - 2.24 (m, 1H), 2.09 - 1.87 (m, 4H), 1.89 - 1.78 (m, 3H), 1.77 (dt, J = 13.5, 2.7 Hz, 2H), 1.17 (d, J = 6.9 Hz, 12H) | MS (APCI⁺) |
| | | | *m*/*z* 561.3 (M+H)⁺ |
| Example 380 | 2-(4-chloro-3-fluorophenoxy)-*N*-{3-[(phenylmethanesulfonyl)amino]bicyclo[1.1.1]pentan-1-yl}acetamide (Compound 479) | | MS (ESI+) |
| | | | *m*/*z* 439 (M+H)⁺ |
| Example 381 | 2-(4-chloro-3-fluorophenoxy)-*N*-(3-{ [2-(4-fluorophenyl)ethanesulfonyl ]amino}bicyclo[1.1.1]penta n-1-yl)acetamide (Compound 480) | | MS (ESI+) |
| | | | *m*/*z* 471 (M+H)⁺ |
| Example 382 | 2-(4-chloro-3-fluorophenoxy)-*N*-(3-{ [(2-fluorophenyl)methanesulfon yl]amino}bicyclo[1.1.1]pent an-1-yl)acetamide (Compound 481) | | MS (ESI+) |
| | | | *m*/*z* 457 (M+H)⁺ |
| Example 383 | 2-(4-chloro-3-fluorophenoxy)-*N*-(3-{ [(2-methylphenyl)methanesulfo nyl]amino}bicyclo[1.1.1]pe ntan-1-yl)acetamide (Compound 482) | | MS (ESI+) |
| | | | *m*/*z* 453 (M+H)⁺ |
| Example 384 | 2-(4-chloro-3-fluorophenoxy)-*N*-(3-{[(3,4-difluorophenyl)methanesulf onyl]amino}bicyclo[1.1.1]p entan-1-yl)acetamide (Compound 483) | | MS (ESI+) |
| | | | *m*/*z* 475 (M+H)⁺ |
| Example 385 | 2-(4-chloro-3-fluorophenoxy)-*N*-(3-{[(3,5-difluorophenyl)methanesulf onyl]amino}bicyclo[1.1.1]p entan-1-yl)acetamide (Compound 484) | | MS (ESI+) |
| | | | *m*/*z* 475 (M+H)⁺ |
| Example 386 | 2-(4-chloro-3-fluorophenoxy)-*N*-(3-{[2-(2-fluorophenoxy)ethanesulfon yl]amino}bicyclo[1.1.1]pent an-1-yl)acetamide (Compound 485) | ¹H NMR (400 MHz, DMSO-*d*_{6_}D₂O) *δ* ppm 7.59 - 7.43 (m, 4H), 7.38 - 7.24 (m, 1H), 7.06 (dd, J = 11.3, 2.8 Hz, 1H), 6.87 (ddd, J = 9.0, 2.8, 1.2 Hz, 1H), 4.50 (s, 2H), 3.94 (t, J = 7.2 Hz, 2H), 3.42 (t, J = 7.1 Hz, 2H), 2.30 (s, 6H) | MS (ESI+) |
| | | | *m*/*z* 487 (M+H)⁺ |
| Example 387 | 2-(4-chloro-3-fluorophenoxy)-*N*-(3-{[(3-methylphenyl)methanesulfo nyl]amino}bicyclo[1.1.1]pe ntan-1-yl)acetamide (Compound 486) | | MS (ESI+) |
| | | | *m*/*z* 453 (M+H)⁺ |
| Example 388 | 2-(4-chloro-3-fluorophenoxy)-*N-*(3-{[(4-fluorophenyl)methanesulfon yl]amino}bicyclo[1.1.1]pent an-1-yl)acetamide (Compound 487) | | MS (ESI+) |
| | | | *m*/*z* 475 (M+H)⁺ |
| Example 389 | 2-(4-chloro-3-fluorophenoxy)-*N*-{3-[(2-phenoxyethanesulfonyl)ami no]bicyclo[1.1.1]pentan-1-yl}acetamide (Compound 488) | | MS (ESI+) |
| | | | *m*/*z* 469 (M+H)⁺ |
| Example 390 | 2-(4-chloro-3-fluorophenoxy)-*N*-(3-{[2-(4-fluorophenoxy)ethanesulfon yl]amino}bicyclo[1.1.1]pent an-1-yl)acetamide (Compound 489) | ¹H NMR (400 MHz, DMSO-*d*_{6_}D₂O) *δ* ppm 7.49 (t, J = 8.8 Hz, 1H), 7.20 - 7.09 (m, 2H), 7.05 (dd, J = 11.3, 2.8 Hz, 1H), 7.01 - 6.93 (m, 2H), 6.86 (ddd, J = 9.0, 2.9, 1.2 Hz, 1H), 4.47 (s, 2H), 4.29 (t, J = 5.8 Hz, 2H), 3.46 (t, J = 5.8 Hz, 2H), 2.21 (s, 6H) | MS (ESI+) |
| | | | *m*/*z* 487 (M+H)⁺ |
| Example 391 | 2-(4-chloro-3-fluorophenoxy)-*N*-(3-{[(4-cyanophenyl)methanesulfon yl]amino}bicyclo[1.1.1]pent an-1-yl)acetamide (Compound 490) | ¹H NMR (400 MHz, DMSO-*d*_{6_}D₂O) *δ* ppm 7.92 - 7.83 (m, 2H), 7.63 - 7.54 (m, 2H), 7.49 (t, J = 8.9 Hz, 1H), 7.05 (dd, J = 11.3, 2.9 Hz, 1H), 6.86 (ddd, J = 9.0, 2.9, 1.2 Hz, 1H), 4.47 (s, 2H), 4.42 (s, 2H), 2.18 (s, 6H) | MS (ESI+) |
| | | | *m*/*z* 464 (M+H)⁺ |
| Example 392 | 2-(4-chloro-3-fluorophenoxy)-*N*-{3-[2-(3,5-difluorophenyl)acetamido]b icyclo[1.1.1]pentan-1-yl}acetamide (Compound 491) | | MS (APCI⁺) |
| | | | *m*/*z* 439 (M+H)⁺ |
| Example 393 | *N*-[2-({3-[2-(4-chloro-3-fluorophenoxy)acetamido]bi cyclo[1.1.1]pentan-1-yl}amino)-2-oxoethyl]benzamide (Compound 492) | | MS (APCI⁺) |
| | | | *m*/*z* 446 (M+H)⁺ |
| Example 394 | 2-(4-chloro-3-fluorophenoxy)-*N*-[(3*S*)-4-{2-[(4,6-dimethylpyrimidin-5-yl)oxy]acetamido}-3-hydroxybicyclo[2.2.2]octan-1-yl]acetamide (Compound 493) | ¹H NMR (400 MHz, DMSO-*d*₆) *δ* ppm 8.67 (s, 1H), 7.48 (t, J = 8.9 Hz, 1H), 7.01 (dd, J = 11.4, 2.9 Hz, 1H), 6.83 (ddd, J = 8.9, 2.9, 1.2 Hz, 1H), 4.43 (s, 2H), 4.39 - 4.26 (m, 2H), 4.11 - 4.02 (m, 1H), 2.45 (s, 6H), 2.33 (ddd, J = 12.5, 9.4, 2.7 Hz, 1H), 2.22 - 2.10 (m, 1H), 1.99 - 1.85 (m, 4H), 1.82 (ddt, J = 15.6, 12.8, 2.6 Hz, 4H) | MS (APCI⁺) |
| | | | *m*/*z* 507.2 (M+H)⁺ |
| Example 395 | 2-(4-chloro-3-fluorophenoxy)-*N*-{(3*S*)-4-[2-(3-ethyl-5-methylphenoxy)acetamido]-3-hydroxybicyclo[2.2.2]octan-1-yl}acetamide (Compound 494) | ¹H NMR (400 MHz, DMSO-*d*₆) *δ* ppm 7.47 (t, J = 8.9 Hz, 1H), 7.01 (dd, J = 11.4, 2.9 Hz, 1H), 6.82 (ddd, J = 9.0, 2.9, 1.2 Hz, 1H), 6.65 (d, J = 1.4 Hz, 1H), 6.60 - 6.55 (m, 2H), 4.45 - 4.29 (m, 4H), 4.05 - 3.94 (m, 1H), 2.50 (q, J = 7.6 Hz, 2H), 2.31 (ddd, J = 12.5, 9.5, 2.6 Hz, 1H), 2.24 (s, 3H), 2.21 - 2.08 (m, 1H), 1.94 (td, J = 14.0, 13.0, 6.0 Hz, 2H), 1.91 - 1.73 (m, 6H), 1.15 (t, J = 7.6 Hz, 3H) | MS (APCI⁺) |
| | | | *m*/*z* 519.2 (M+H)⁺ |
| Example 396 | 2-(4-chloro-3-fluorophenoxy)-*N*-[(3*S*)-3-hydroxy-4-{2-[(pyridin-3-yl)oxy]acetamido}bicyclo[2.2.2]octan-1-yl]acetamide (Compound 495) | ¹H NMR (400 MHz, DMSO-*d*₆) *δ* ppm 8.57 (d, J = 2.8 Hz, 1H), 8.45 (dd, J = 5.4, 1.1 Hz, 1H), 8.01 (ddd, J = 8.8, 2.9, 1.1 Hz, 1H), 7.86 (dd, J = 8.8, 5.3 Hz, 1H), 7.47 (t, J = 8.9 Hz, 1H), 7.01 (dd, J = 11.4, 2.8 Hz, 1H), 6.82 (ddd, J = 9.0, 2.9, 1.2 Hz, 1H), 4.70 (s, 2H), 4.43 (s, 2H), 4.18 - 4.09 (m, 1H), 2.30 (ddd, J = 13.2, 9.4, 2.2 Hz, 1H), 1.96 (dd, J = 9.6, 4.0 Hz, 4H), 1.89 - 1.71 (m, 5H) | MS (APCI⁺) |
| | | | *m*/*z* 478.2 (M+H)⁺ |
| Example 397 | 2-(4-chloro-3-fluorophenoxy)-*N*-[(3*S*)-4-{2-[3,5-di(propan-2-yl)phenoxy]acetamido}-3-hydroxybicyclo[2.2.2]octan-1-yl]acetamide (Compound 496) | ¹H NMR (400 MHz, DMSO-*d*₆) *δ* ppm 7.47 (t, J = 8.9 Hz, 1H), 7.01 (dd, J = 11.4, 2.9 Hz, 1H), 6.82 (ddd, J = 8.9, 3.0, 1.2 Hz, 1H), 6.72 (d, J = 1.5 Hz, 1H), 6.61 (d, J = 1.5 Hz, 2H), 4.45 - 4.32 (m, 4H), 4.06 - 3.94 (m, 1H), 2.82 (hept, J = 6.9 Hz, 2H), 2.31 (ddd, J = 12.5, 9.4, 2.5 Hz, 1H), 2.13 (td, J = 13.5, 12.0, 8.8 Hz, 1H), 2.00 - 1.72 (m, 8H), 1.17 (d, J = 6.9 Hz, 12H) | MS (APCI⁺) |
| | | | *m*/*z* 561.3 (M+H)⁺ |
| Example 398 | 2-(4-chloro-3-fluorophenoxy)-*N*-[(3*S*)-3-hydroxy-4-(2-{[6-methoxy-2-(methylsulfanyl)pyrimidin-4-yl]oxy}acetamido)bicyclo[2 .2.2]octan-1-yl]acetamide (Compound 497) | ¹H NMR (400 MHz, DMSO-*d*₆) *δ* ppm 7.47 (t, J = 8.9 Hz, 1H), 7.00 (dd, J = 11.4, 2.9 Hz, 1H), 6.82 (ddd, J = 9.0, 2.9, 1.2 Hz, 1H), 5.98 (s, 1H), 4.72 (d, J = 14.6 Hz, 1H), 4.65 (d, J = 14.5 Hz, 1H), 4.42 (s, 2H), 4.08 - 3.99 (m, 1H), 3.87 (s, 3H), 2.48 (s, 3H), 2.28 (ddd, J = 13.2, 9.4, 2.3 Hz, 1H), 2.03 - 1.90 (m, 3H), 1.92 - 1.69 (m, 6H) | MS (APCI⁺) |
| | | | *m*/*z* 555.1 (M+H)⁺ |
| Example 399 | 2-(4-chloro-3-fluorophenoxy)-*N*-[(3*S*)-4-{2-[(4,6-dimethoxypyrimidin-2-yl)oxy]acetamido}-3-hydroxybicyclo[2.2.2]octan-1-yl]acetamide (Compound 498) | ¹H NMR (400 MHz, DMSO-*d*₆) *δ* ppm 7.47 (t, J = 8.9 Hz, 1H), 7.00 (dd, J = 11.4, 2.9 Hz, 1H), 6.82 (ddd, J = 9.0, 2.9, 1.2 Hz, 1H), 5.88 (s, 1H), 4.68 (d, J = 14.6 Hz, 1H), 4.61 (d, J = 14.5 Hz, 1H), 4.42 (s, 2H), 4.07 - 3.94 (m, 1H), 3.74 (s, 12H), 2.33 - 2.23 (m, 1H), 2.05 - 1.89 (m, 3H), 1.92 - 1.69 (m, 6H) | MS (APCI⁺) |
| | | | *m*/*z* 539.2 (M+H)⁺ |
| Example 400 | 2-(4-chloro-3-fluorophenoxy)-*N*-[(3*S*)-3-hydroxy-4-{2-[(6-methyl-1,3-dihydrofuro[3,4-*c*]pyridin-7-yl)oxy]acetamido}bicyclo[2 .2.2]octan-1-yl]acetamide (Compound 499) | ¹H NMR (400 MHz, DMSO-*d*₆) *δ* ppm 8.33 (d, J = 1.0 Hz, 1H), 7.47 (t, J = 8.8 Hz, 1H), 7.01 (dd, J = 11.4, 2.9 Hz, 1H), 6.82 (ddd, J = 8.9, 2.9, 1.2 Hz, 1H), 5.40 - 5.23 (m, 2H), 5.10 (s, 2H), 4.62 (s, 2H), 4.43 (s, 2H), 4.08 (dd, J = 9.6, 3.2 Hz, 1H), 2.59 (s, 3H), 2.36 - 2.24 (m, 1H), 2.08 - 1.85 (m, 4H), 1.89 - 1.72 (m, 5H) | MS (APCI⁺) |
| | | | *m*/*z* 534.1 (M+H)⁺ |
| Example 401 | 2-(4-chloro-3-fluorophenoxy)-*N*-[(3*S*)-3-hydroxy-4-{2-[(pyrimidin-2-yl)oxy]acetamido}bicyclo[2 .2.2]octan-1-yl]acetamide (Compound 500) | ¹H NMR (400 MHz, DMSO-*d*₆) *δ* ppm 8.60 (d, J = 4.8 Hz, 2H), 7.47 (t, J = 8.9 Hz, 1H), 7.18 (t, J = 4.8 Hz, 1H), 7.01 (dd, J = 11.4, 2.9 Hz, 1H), 6.82 (ddd, J = 9.0, 2.9, 1.2 Hz, 1H), 4.78 - 4.64 (m, 2H), 4.42 (s, 2H), 4.07 - 3.94 (m, 1H), 2.33 - 2.23 (m, 1H), 2.01 (ddd, J = 12.1, 10.4, 4.6 Hz, 1H), 1.98 - 1.86 (m, 2H), 1.79 (dddd, J = 20.6, 15.9, 9.1, 3.8 Hz, 6H) | MS (APCI⁺) |
| | | | *m*/*z* 479.2 (M+H)⁺ |
| Example 402 | 2-(4-chloro-3-fluorophenoxy)-*N*-[(3*S*)-3-hydroxy-4-{2-[3-(trifluoromethyl)phenoxy]ac etamido}bicyclo[2.2.2]octan -1-yl]acetamide (Compound 501) | ¹H NMR (400 MHz, DMSO-*d*₆) *δ* ppm 7.59 - 7.50 (m, 1H), 7.47 (t, J = 8.9 Hz, 1H), 7.33 (ddt, J = 7.6, 1.7, 0.9 Hz, 1H), 7.29 - 7.21 (m, 2H), 7.01 (dd, J = 11.3, 2.8 Hz, 1H), 6.82 (ddd, J = 9.0, 2.9, 1.2 Hz, 1H), 4.52 (s, 2H), 4.43 (s, 2H), 4.07 (ddd, J = 9.5, 3.2, 1.1 Hz, 1H), 2.30 (ddd, J = 13.2, 9.5, 2.3 Hz, 1H), 2.05 (ddd, J = 12.1, 10.1, 5.1 Hz, 1H), 1.98 - 1.72 (m, 8H) | MS (APCI⁺) |
| | | | *m*/*z* 545.1 (M+H)⁺ |
| Example 403 | 2-(4-chloro-3-fluorophenoxy)-*N*-{(3*S*)-4-[2-(3,5-dimethylphenoxy)acetamido ]-3-hydroxybicyclo[2.2.2]octan-1-yl}acetamide (Compound 502) | ¹H NMR (400 MHz, DMSO-*d*₆) *δ* ppm 7.47 (t, J = 8.9 Hz, 1H), 7.01 (dd, J = 11.4, 2.9 Hz, 1H), 6.82 (ddd, J = 9.0, 2.9, 1.2 Hz, 1H), 6.62 (dt, J = 1.6, 0.8 Hz, 1H), 6.58 - 6.53 (m, 2H), 4.43 (s, 2H), 4.35 (d, J = 1.7 Hz, 2H), 4.05 - 3.94 (m, 1H), 2.31 (ddd, J = 12.4, 9.4, 2.6 Hz, 1H), 2.23 (s, 6H), 2.20 - 2.08 (m, 1H), 2.00 - 1.88 (m, 2H), 1.91 - 1.73 (m, 6H) | MS (APCI⁺) |
| | | | *m*/*z* 505.2 (M+H)⁺ |
| Example 404 | 2-(4-chloro-3-fluorophenoxy)-*N*-{(3*S*)-3-hydroxy-4-[2-(3,4,5-trimethylphenoxy)acetamid o]bicyclo[2.2.2]octan-1-yl}acetamide (Compound 503) | ¹H NMR (400 MHz, DMSO-*d*₆) *δ* ppm 7.47 (t, J = 8.9 Hz, 1H), 7.01 (dd, J = 11.3, 2.9 Hz, 1H), 6.82 (ddd, J = 9.0, 2.9, 1.2 Hz, 1H), 6.59 (s, 2H), 4.43 (s, 2H), 4.31 (d, J = 2.2 Hz, 2H), 4.00 (dd, J = 9.7, 3.3 Hz, 1H), 2.31 (ddd, J = 12.5, 9.4, 2.6 Hz, 1H), 2.19 (s, 6H), 2.20 - 2.09 (m, 1H), 2.04 (s, 3H), 2.00 - 1.73 (m, 8H) | MS (APCI⁺) |
| | | | *m*/*z* 519.2 (M+H)⁺ |
| Example 405 | 2-(4-chloro-3-fluorophenoxy)-*N*-[(3*S*)-3-hydroxy-4-{2-[4-(methanesulfonyl)phenoxy] acetamido}bicyclo[2.2.2]oct an-1-yl]acetamide (Compound 504) | ¹H NMR (400 MHz, DMSO-*d*₆) *δ* ppm 7.92 - 7.81 (m, 2H), 7.47 (t, J = 8.9 Hz, 1H), 7.22 - 7.11 (m, 2H), 7.01 (dd, J = 11.4, 2.9 Hz, 1H), 6.82 (ddd, J = 9.0, 2.9, 1.2 Hz, 1H), 4.56 (s, 2H), 4.43 (s, 2H), 4.09 (dd, J = 9.6, 3.1 Hz, 1H), 3.15 (s, 3H), 2.35 - 2.24 (m, 1H), 2.09 - 1.87 (m, 4H), 1.91 - 1.72 (m, 5H) | MS (APCI⁺) |
| | | | *m*/*z* 555.2 (M+H)⁺ |
| Example 406 | 2-(4-chloro-3-fluorophenoxy)-*N*-[(3*S*)-3-hydroxy-4-{2-[4-(trifluoromethoxy)phenoxy] acetamido}bicyclo[2.2.2]octan-1-yl]acetamide (Compound 505) | ¹H NMR (400 MHz, DMSO-*d*₆) *δ* ppm 7.47 (t, J = 8.9 Hz, 1H), 7.35 - 7.25 (m, 2H), 7.11 - 6.97 (m, 3H), 6.82 (ddd, J = 8.9, 2.9, 1.1 Hz, 1H), 4.44 (d, J = 7.6 Hz, 4H), 4.10 - 4.02 (m, 1H), 2.30 (ddd, J = 13.1, 9.4, 2.3 Hz, 1H), 2.07 (ddd, J = 12.0, 10.0, 5.2 Hz, 1H), 1.98 - 1.87 (m, 2H), 1.91 - 1.72 (m, 6H) | MS (APCI⁺) |
| | | | *m*/*z* 561.1 (M+H)⁺ |
| Example 407 | 2-(4-*tert*-butylphenoxy)-*N-*{(2*S*)-4-[2-(4-chloro-3-fluorophenoxy)acetamido]-2-hydroxybicyclo[2.2.2]octan-1-yl}acetamide (Compound 506) | ¹H NMR (400 MHz, DMSO-*d*₆) *δ* ppm 7.58 (s, 1H), 7.47 (t, J = 8.9 Hz, 1H), 7.35 - 7.23 (m, 3H), 7.01 (dd, J = 11.3, 2.9 Hz, 1H), 6.91 - 6.78 (m, 3H), 4.45 - 4.30 (m, 4H), 4.07 - 3.98 (m, 1H), 2.31 (ddd, J = 12.4, 9.4, 2.4 Hz, 1H), 2.21 - 2.08 (m, 1H), 1.97 - 1.87 (m, 2H), 1.88 - 1.72 (m, 6H), 1.25 (s, 9H) | MS (APCI⁺) |
| | | | *m*/*z* 533.2 (M+H)⁺ |
| Example 408 | 2-(4-chloro-3-fluorophenoxy)-*N*-[(3*S*)-3-hydroxy-4-{2-[4-(trifluoromethyl)phenoxy]ac etamido}bicyclo[2.2.2]octan -1-yl]acetamide (Compound 507) | ¹H NMR (400 MHz, DMSO-*d*₆) *δ* ppm 7.67 (d, J = 8.6 Hz, 2H), 7.47 (t, J = 8.9 Hz, 1H), 7.12 (d, J = 8.5 Hz, 2H), 7.01 (dd, J = 11.4, 2.9 Hz, 1H), 6.82 (ddd, J = 9.0, 2.9, 1.2 Hz, 1H), 4.52 (s, 2H), 4.43 (s, 2H), 4.12 - 4.03 (m, 1H), 2.30 (ddd, J = 13.2, 9.4, 2.1 Hz, 1H), 2.05 (ddd, J = 12.3, 10.1, 5.4 Hz, 1H), 1.99 - 1.85 (m, 3H), 1.87 - 1.77 (m, 1H), 1.82 (s, 2H), 1.81 - 1.72 (m, 2H) | MS (APCI⁺) |
| | | | *m*/*z* 545.1 (M+H)⁺ |
| Example 409 | 2-(4-chloro-3-fluorophenoxy)-*N*-{(3*S*)-4-[2-(4-ethoxyphenoxy)acetamido]-3-hydroxybicyclo[2.2.2]octan-1-yl}acetamide (Compound 508) | ¹H NMR (400 MHz, DMSO-*d*₆) *δ* ppm 7.47 (t, J = 8.9 Hz, 1H), 7.01 (dd, J = 11.4, 2.9 Hz, 1H), 6.92 - 6.78 (m, 6H), 4.43 (s, 2H), 4.33 (d, J = 1.8 Hz, 2H), 4.05 - 3.90 (m, 4H), 2.31 (ddd, J = 12.5, 9.4, 2.5 Hz, 1H), 2.20 - 2.08 (m, 1H), 1.91 - 1.75 (m, 8H), 1.29 (t, J = 7.0 Hz, 3H) | MS (APCI⁺) |
| | | | *m*/*z* 521.2 (M+H)⁺ |
| Example 410 | 2-(4-chloro-3-fluorophenoxy)-*N*-{(3*S*)-3-hydroxy-4-[2-(3-methylphenoxy)acetamido] bicyclo[2.2.2]octan-1-yl}acetamide (Compound 509) | ¹H NMR (400 MHz, DMSO-*d*₆) *δ* ppm 7.47 (t, J = 8.9 Hz, 1H), 7.18 (t, J = 7.8 Hz, 1H), 7.01 (dd, J = 11.4, 2.9 Hz, 1H), 6.86 - 6.69 (m, 4H), 4.45 - 4.31 (m, 4H), 4.06 - 3.94 (m, 1H), 2.36 - 2.27 (m, 1H), 2.27 (s, 3H), 2.26 - 2.06 (m, 1H), 1.92 (q, J = 6.8 Hz, 2H), 1.81 (qd, J = 14.0, 13.5, 7.0 Hz, 6H) | MS (APCI⁺) |
| | | | *m*/*z* 491.2 (M+H)⁺ |
| Example 411 | 2-(4-chloro-3-fluorophenoxy)-*N*-{(3*S*)-4-[2-(3,4-dimethylphenoxy)acetamido ]-3-hydroxybicyclo[2.2.2]octan-1-yl}acetamide (Compound 510) | ¹H NMR (400 MHz, DMSO-*d*₆) *δ* ppm 7.58 (s, 1H), 7.47 (t, J = 8.9 Hz, 1H), 7.21 (s, 1H), 7.07 - 6.97 (m, 2H), 6.82 (ddd, J = 9.0, 2.9, 1.2 Hz, 1H), 6.75 (d, J = 2.6 Hz, 1H), 6.65 (dd, J = 8.3, 2.8 Hz, 1H), 4.43 (s, 2H), 4.33 (d, J = 2.0 Hz, 2H), 4.05 - 3.96 (m, 1H), 2.30 (ddd, J = 12.6, 9.4, 2.5 Hz, 1H), 2.18 (s, 3H), 2.16 - 2.14 (m, 1H), 2.14 (s, 6H), 2.00 - 1.86 (m, 2H), 1.90 - 1.72 (m, 6H) | MS (APCI⁺) |
| | | | *m*/*z* 505.2 (M+H)⁺ |
| Example 412 | 2-(4-acetylphenoxy)-*N-*{(2*S*)-4-[2-(4-chloro-3-fluorophenoxy)acetamido]-2-hydroxybicyclo[2.2.2]octan-1-yl}acetamide (Compound 511) | ¹H NMR (400 MHz, DMSO-*d*₆) *δ* ppm 7.99 - 7.89 (m, 2H), 7.47 (t, J = 8.9 Hz, 1H), 7.09 - 6.95 (m, 3H), 6.82 (ddd, J = 9.1, 2.9, 1.2 Hz, 1H), 4.48 (d, J = 41.0 Hz, 4H), 4.11 - 4.03 (m, 1H), 2.53 (s, 3H), 2.30 (ddd, J = 13.1, 9.4, 2.2 Hz, 1H), 2.06 (ddd, J = 12.2, 10.2, 5.4 Hz, 1H), 2.00 - 1.84 (m, 3H), 1.87 - 1.72 (m, 5H) | MS (APCI⁺) |
| | | | *m*/*z* 519.2 (M+H)⁺ |
| Example 413 | 2-[(1,3-benzothiazol-2-yl)oxy]-*N*-{(2*S*)-4-[2-(4-chloro-3-fluorophenoxy)acetamido]-2-hydroxybicyclo[2.2.2]octan-1-yl}acetamide (Compound 512) | ¹H NMR (400 MHz, DMSO-*d*₆) *δ* ppm7.64 (dd, J = 7.8, 1.2 Hz, 1H), 7.47 (t, J = 8.9 Hz, 1H), 7.36 (td, J = 7.8, 1.3 Hz, 1H), 7.22 (td, J = 7.7, 1.1 Hz, 1H), 7.14 (dd, J = 8.2, 1.1 Hz, 1H), 7.00 (dd, J = 11.4, 2.8 Hz, 1H), 6.81 (ddd, J = 9.0, 2.9, 1.2 Hz, 1H), 4.60 (d, J = 16.6 Hz, 1H), 4.53 (d, J = 16.6 Hz, 1H), 4.42 (s, 2H), 4.16 - 4.08 (m, 1H), 2.27 (ddd, J = 13.1, 9.3, 2.5 Hz, 1H), 1.97 (dq, J = 11.7, 6.5, 4.0 Hz, 3H), 1.89 - 1.67 (m, 6H) | MS (APCI⁺) |
| | | | *m*/*z* 534.2 (M+H)⁺ |
| Example 414 | 2-(4-chloro-3-fluorophenoxy)-N-[(3S)-4-{2-[(1,5-diphenyl-1*H-*pyrazol-3-yl)oxy]acetamido}-3-hydroxybicyclo[2.2.2]octan-1-yl]acetamide (Compound 513) | ¹H NMR (400 MHz, DMSO-*d*₆) *δ* ppm 7.47 (t, J = 8.9 Hz, 1H), 7.43 - 7.28 (m, 6H), 7.21 (ddt, J = 13.7, 6.0, 1.7 Hz, 4H), 7.01 (dd, J = 11.4, 2.8 Hz, 1H), 6.82 (ddd, J = 9.0, 2.9, 1.2 Hz, 1H), 6.20 (s, 1H), 4.57 (d, J = 1.8 Hz, 2H), 4.43 (s, 2H), 4.10 - 4.02 (m, 1H), 3.96 (s, 0H), 2.30 (ddd, J = 12.5, 9.5, 2.3 Hz, 1H), 2.10 (ddd, J = 11.5, 9.6, 4.8 Hz, 1H), 2.00 - 1.87 (m, 2H), 1.91 - 1.72 (m, 6H) | MS (APCI⁺) |
| | | | *m*/*z* 619.1 (M+H)⁺ |
| Example 415 | 2-(4-chloro-3-fluorophenoxy)-*N*-[(3*S*)-3-hydroxy-4-{2-[(thieno[2,3-*d*]pyrimidin-4-yl)oxy]acetamido}bicyclo[2 .2.2]octan-1-yl] acetamide (Compound 514) | ¹H NMR (400 MHz, DMSO-*d*₆) *δ* ppm 8.64 (s, 1H), 7.85 (d, J = 5.9 Hz, 1H), 7.59 - 7.42 (m, 2H), 7.00 (dd, J = 11.4, 2.8 Hz, 1H), 6.82 (ddd, J = 9.0, 2.9, 1.2 Hz, 1H), 5.03 - 4.88 (m, 2H), 4.42 (s, 2H), 4.08 (dd, J = 9.5, 3.0 Hz, 1H), 2.28 (ddd, J = 12.9, 9.4, 2.1 Hz, 1H), 2.04 - 1.86 (m, 4H), 1.87 - 1.69 (m, 5H) | MS (APCI⁺) |
| | | | *m*/*z* 535.0 (M+H)⁺ |
| Example 416 | 2-[(1,2-benzoxazol-3-yl)oxy]-*N*-{(2*S*)-4-[2-(4-chloro-3-fluorophenoxy)acetamido]-2-hydroxybicyclo[2.2.2]octan-1-yl}acetamide (Compound 515) | ¹H NMR (400 MHz, DMSO-*d*₆) *δ* ppm 7.80 (dt, J = 7.9, 1.1 Hz, 1H), 7.74 - 7.55 (m, 2H), 7.56 - 7.37 (m, 2H), 7.01 (dd, J = 11.4, 2.9 Hz, 1H), 6.82 (ddd, J = 9.0, 2.9, 1.2 Hz, 1H), 4.82 (d, J = 1.8 Hz, 2H), 4.43 (s, 2H), 4.17 - 4.09 (m, 1H), 2.29 (ddd, J = 13.1, 9.4, 2.2 Hz, 1H), 2.02 - 1.92 (m, 4H), 1.91 - 1.70 (m, 5H) | MS (APCI⁺) |
| | | | *m*/*z* 518.1 (M+H)⁺ |
| Example 417 | 2-(4-chloro-3-fluorophenoxy)-*N*-[(3*S*)-4-(2-{[1-(4-chlorophenyl)-1*H*-1,2,4-triazol-3-yl]oxy}acetamido)-3-hydroxybicyclo[2.2.2]octan-1-yl]acetamide (Compound 516) | ¹H NMR (400 MHz, DMSO-*d*₆) *δ* ppm 8.98 (s, 1H), 7.83 - 7.74 (m, 2H), 7.65 - 7.55 (m, 2H), 7.47 (t, J = 8.9 Hz, 1H), 7.01 (dd, J = 11.3, 2.9 Hz, 1H), 6.82 (ddd, J = 8.9, 2.9, 1.1 Hz, 1H), 4.68 (d, J = 2.1 Hz, 2H), 4.42 (s, 2H), 4.06 (dd, J = 9.4, 3.1 Hz, 1H), 3.18 (s, 1H), 2.35 - 2.24 (m, 1H), 2.03 (ddd, J = 12.2, 10.3, 5.1 Hz, 1H), 2.00 - 1.89 (m, 2H), 1.93 - 1.82 (m, 1H), 1.78 (tt, J = 13.4, 5.7 Hz, 5H) | MS (APCI⁺) |
| | | | *m*/*z* 578.1 (M+H)⁺ |
| Example 418 | 2-(4-chloro-3-fluorophenoxy)-*N*-[(3*S*)-3-hydroxy-4-{2-[(naphthalen-2-yl)oxy]acetamido}bicyclo[2 .2.2] octan-1-yl] acetamide (Compound 517) | ¹H NMR (400 MHz, DMSO-*d*₆) *δ* ppm 7.86 (dd, J = 8.6, 4.3 Hz, 2H), 7.80 (d, J = 8.2 Hz, 1H), 7.58 (s, 1H), 7.53 - 7.43 (m, 2H), 7.43 - 7.32 (m, 1H), 7.31 - 7.20 (m, 2H), 7.01 (dd, J = 11.4, 2.8 Hz, 1H), 6.82 (ddd, J = 9.0, 2.9, 1.2 Hz, 1H), 4.54 (d, J = 1.5 Hz, 2H), 4.43 (s, 2H), 4.06 (dd, J = 9.6, 3.1 Hz, 1H), 2.31 (ddd, J = 12.7, 9.3, 2.5 Hz, 1H), 2.19 - 2.03 (m, 1H), 1.92 (dd, J = 18.3, 9.8 Hz, 2H), 1.90 - 1.73 (m, 6H) | MS (APCI⁺) |
| | | | *m*/*z* 527.2 (M+H)⁺ |
| Example 419 | 2-(4-chloro-3-fluorophenoxy)-*N*-[(3*S*)-4-{2-[(6-chloropyridazin-3-yl)oxy]acetamido}-3-hydroxybicyclo[2.2.2]octan-1-yl]acetamide (Compound 518) | ¹H NMR (400 MHz, DMSO-*d*₆) *δ* ppm 7.79 (d, J = 9.2 Hz, 1H), 7.47 (t, J = 8.9 Hz, 1H), 7.39 (d, J = 9.2 Hz, 1H), 7.01 (dd, J = 11.4, 2.8 Hz, 1H), 6.82 (ddd, J = 9.0, 2.9, 1.2 Hz, 1H), 4.91 - 4.73 (m, 2H), 4.42 (s, 2H), 4.13 - 4.04 (m, 1H), 2.28 (ddd, J = 13.3, 9.4, 2.3 Hz, 1H), 2.00 - 1.69 (m, 9H) | MS (APCI⁺) |
| | | | *m*/*z* 513.1 (M+H)⁺ |
| Example 420 | methyl 3-[2-({(2*S*)-4-[2-(4-chloro-3-fluorophenoxy)acetamido]-2-hydroxybicyclo[2.2.2]octan-1-yl}amino)-2-oxoethoxy]-1,2-oxazole-5-carboxylate (Compound 519) | ¹H NMR (400 MHz, DMSO-*d*₆) *δ* ppm 7.47 (t, J = 8.9 Hz, 1H), 7.09 - 6.96 (m, 2H), 6.82 (ddd, J = 9.0, 2.9, 1.2 Hz, 1H), 4.66 (d, J = 1.4 Hz, 2H), 4.42 (s, 2H), 4.15 - 4.07 (m, 1H), 3.89 (s, 3H), 2.28 (ddd, J = 12.5, 9.4, 2.4 Hz, 1H), 2.00 - 1.85 (m, 4H), 1.87 - 1.69 (m, 5H) | MS (APCI⁺) |
| | | | *m*/*z* 526.1 (M+H)⁺ |
| Example 421 | 2-(4-chloro-3-fluorophenoxy)-*N*-[(3*S*)-3-hydroxy-4-{2-[(2-methylquinolin-4-yl)oxy]acetamido}bicyclo[2 .2.2] octan-1-yl] acetamide (Compound 520) | ¹H NMR (400 MHz, DMSO-*d*₆) *δ* ppm 8.41 - 8.33 (m, 1H), 8.09 (dtd, J = 17.1, 8.5, 1.3 Hz, 2H), 7.87 (ddd, J = 8.3, 6.8, 1.4 Hz, 1H), 7.47 (t, J = 8.9 Hz, 1H), 7.34 (s, 1H), 7.01 (dd, J = 11.4, 2.9 Hz, 1H), 6.82 (ddd, J = 9.0, 2.9, 1.2 Hz, 1H), 5.04 (s, 2H), 4.43 (s, 2H), 4.18 (dd, J = 9.7, 3.0 Hz, 1H), 2.84 (s, 3H), 2.31 (ddd, J = 12.3, 9.5, 2.2 Hz, 1H), 2.11 - 1.88 (m, 3H), 1.80 (tdd, J = 12.9, 6.7, 3.1 Hz, 6H) | MS (APCI⁺) |
| | | | *m*/*z* 542.1 (M+H)⁺ |
| Example 422 | 2-(4-chloro-3-fluorophenoxy)-*N*-{(3*S*)-3-hydroxy-4-[2-(4-methylphenoxy)acetamido] bicyclo[2.2.2]octan-1-yl}acetamide (Compound 521) | ¹H NMR (400 MHz, DMSO-*d*₆) *δ* ppm 7.58 (s, 1H), 7.47 (t, J = 8.9 Hz, 1H), 7.22 (s, 1H), 7.15 - 7.06 (m, 2H), 7.01 (dd, J = 11.4, 2.9 Hz, 1H), 6.87 - 6.78 (m, 3H), 4.45 - 4.29 (m, 4H), 4.05 - 3.96 (m, 1H), 2.30 (ddd, J = 12.5, 9.4, 2.5 Hz, 1H), 2.23 (s, 3H), 2.19 - 2.07 (m, 1H), 1.93 (t, J = 12.8 Hz, 2H), 1.87 (d, J = 16.5 Hz, 1H), 1.87 - 1.72 (m, 5H) | MS (APCI⁺) |
| | | | *m*/*z* 491.2 (M+H)⁺ |
| Example 423 | 2-(4-chloro-3-fluorophenoxy)-*N*-[(3*S*)-3-hydroxy-4-{2-[4-(propane-1-sulfonyl)phenoxy] acetamid o}bicyclo[2.2.2]octan-1-yl]acetamide (Compound 522) | ¹H NMR (400 MHz, DMSO-*d*₆) *δ* ppm 7.87 - 7.74 (m, 2H), 7.47 (t, J = 8.9 Hz, 1H), 7.23 - 7.12 (m, 2H), 7.01 (dd, J = 11.4, 2.8 Hz, 1H), 6.82 (ddd, J = 9.0, 2.9, 1.2 Hz, 1H), 4.56 (s, 2H), 4.42 (s, 2H), 4.08 (dd, J = 9.4, 3.1 Hz, 1H), 3.24 - 3.15 (m, 2H), 2.35 - 2.24 (m, 1H), 2.08 - 1.91 (m, 4H), 1.83 - 1.71 (m, 5H), 1.62 - 1.47 (m, 2H), 0.91 (t, J = 7.5 Hz, 3H) | MS (APCI⁺) |
| | | | *m*/*z* 583.3 (M+H)⁺ |
| Example 424 | 2-(4-chloro-3-fluorophenoxy)-*N*-[(3*S*)-4-(2-{[4,6-di(piperidin-1-yl)-1,3,5-triazin-2-yl]oxy}acetamido)-3-hydroxybicyclo[2.2.2]octan-1-yl]acetamide (Compound 523) | ¹H NMR (400 MHz, DMSO-*d*₆) *δ* ppm 7.47 (t, J = 8.9 Hz, 1H), 7.00 (dd, J = 11.4, 2.8 Hz, 1H), 6.81 (ddd, J = 9.0, 2.9, 1.2 Hz, 1H), 4.56 (d, J = 14.4 Hz, 1H), 4.51 - 4.39 (m, 3H), 4.03 - 3.94 (m, 1H), 3.66 (s, 2H), 2.33 - 2.22 (m, 1H), 1.96 (dd, J = 22.7, 8.2 Hz, 3H), 1.78 (qd, J = 16.5, 15.0, 11.6 Hz, 6H), 1.60 (d, J = 6.0 Hz, 5H), 1.47 (s, 8H) | MS (APCI⁺) |
| | | | *m*/*z* 646.2 (M+H)⁺ |
| Example 425 | 2-(4-acetylphenoxy)-*N-*{(3*S*)-4-[2-(4-chloro-3-fluorophenoxy)acetamido]-3-hydroxybicyclo[2.2.2]octan-1-yl}acetamide (Compound 524) | ¹H NMR (400 MHz, DMSO-*d*₆) *δ* ppm 7.97 - 7.89 (m, 2H), 7.48 (t, J = 8.9 Hz, 1H), 7.08 - 6.97 (m, 3H), 6.84 (ddd, J = 9.0, 2.9, 1.2 Hz, 1H), 4.48 (d, J = 14.9 Hz, 4H), 4.07 (dd, J = 9.6, 3.1 Hz, 1H), 2.30 (ddd, J = 12.1, 9.4, 1.6 Hz, 1H), 2.03 (ddd, J = 12.1, 10.0, 5.3 Hz, 1H), 1.91 (dd, J = 21.5, 10.1 Hz, 3H), 1.88 - 1.72 (m, 5H) | MS (APCI⁺) |
| | | | *m*/*z* 519.2 (M+H)⁺ |

### Example 426: N,N'-[(2S)-2-hydroxybicyclo[2.2.2]octane-1,4-diyl]bis[2-(4-chloro-3-methylphenoxy)acetamide] (Compound 525)

A mixture of Example 343D (40.0 mg, 0.175 mmol), (1-[bis(dimethylamino)methylene]-1*H*-1,2,3-triazolo[4,5-*b*]pyridinium 3-oxid hexafluorophosphate) (HATU, 173 mg, 0.454 mmol), 2-(4-chloro-3-methylphenoxy)acetic acid (91 mg, 0.454 mmol), and triethylamine (0.122 mL, 0.873 mmol) in *N,N*-dimethylformamide (2 mL) was stirred for 3 hours. The reaction was quenched with brine and saturated NaHCO₃, and extracted with ethyl acetate (2×). The combined organic layers were washed with brine, dried over MgSO₄, filtered, and concentrated (0.14 g crude). The concentrate was dissolved in tetrahydrofuran (1.7 mL) and methanol (0.8 mL) and treated with a solution of lithium hydroxide (5.02 mg, 0.209 mmol) in water (0.5 mL). The suspension was stirred for 1 hour, diluted with water and brine, and extracted with ethyl acetate (3×). The combined ethyl acetate layers were treated with a small amount of methanol till a clear organic phase was formed. The combined organic layers were concentrated, and the residue was purified by reverse-phase HPLC (see protocol in Example 112D) to provide the title compound (46 mg, 51%). ¹H NMR (400 MHz, DMSO-*d*₆) *δ* ppm 7.42 (s, 1H), 7.30 (dd, J = 8.8, 2.9 Hz, 2H), 7.20 (s, 1H), 6.94 (ddd, J = 9.9, 3.1, 0.8 Hz, 2H), 6.83 - 6.66 (m, 2H), 5.12 (d, J = 4.5 Hz, 1H), 4.40 (s, 2H), 4.37 (s, 2H), 4.06 - 3.94 (m, 1H), 2.30 - 2.24 (m, 7H), 2.19 - 2.09 (m, 1H), 2.00 - 1.68 (m, 8H); MS (ESI⁺) *m*/*z* 521.2 (M+H)⁺.

### Example 427: N,N'-(bicyclo[2.2.1]heptane-1,4-diyl)bis[2-(3,4-dichlorophenoxy)acetamide] (Compound 526)

The title compound was prepared using the methodologies described in Example 341 substituting 2-(3,4-dichlorophenoxy)acetic acid for 2-(3-chloro-4-fluorophenoxy)acetic acid. ¹H NMR (400 MHz, DMSO-*d*₆) *δ* ppm 8.15 (s, 2H), 7.53 (d, J = 8.9 Hz, 2H), 7.23 (d, J = 2.9 Hz, 2H), 6.97 (dd, J = 9.0, 2.9 Hz, 2H), 4.49 (s, 4H), 2.03 (s, 2H), 1.91 1.81 (m, 4H), 1.77 (d, J = 6.4 Hz, 4H); MS (ESI⁺) *m*/*z* 530.9 (M+H)⁺.

### Example 428: N,N'-(bicyclo[2.2.1]heptane-1,4-diyl)bis[2-(4-chlorophenoxy)acetamide]

### (Compound 527)

The title compound was prepared using the methodologies described in Example 341 substituting 2-(4-chlorophenoxy)acetic acid for 2-(3-chloro-4-fluorophenoxy)acetic acid. ¹H NMR (400 MHz, DMSO-*d*₆) *δ* ppm 8.10 (s, 2H), 7.38 7.29 (m, 4H), 7.00 6.89 (m, 4H), 4.43 (s, 4H), 2.03 (s, 2H), 1.88 (d, J = 6.3 Hz, 4H), 1.78 (dd, J = 15.9, 10.3 Hz, 4H); MS (ESI⁺) *m*/*z* 463.1 (M+H)⁺.

### Example 429: 2-(4-chloro-3-fluorophenoxy)-N-{(3S)-4-[2-(4-chloro-3-methoxyphenoxy)acetamido]-3-hydroxybicyclo[2.2.2]octan-1-yl}acetamide (Compound 528)

The title compound was prepared using the methodologies described in Example 338C substituting Example 276A for Example 338B and 2-(4-chloro-3-methoxyphenoxy)acetic acid for 2-(4-chlorophenoxy)acetic acid. ¹H NMR (400 MHz, DMSO-*d*₆) *δ* ppm 7.50 7.40 (m, 2H), 7.27 (d, J = 8.7 Hz, 1H), 7.19 (s, 1H), 6.99 (dd, J = 11.4, 2.9 Hz, 1H), 6.77 (ddd, J = 8.9, 2.9, 1.2 Hz, 1H), 6.69 (d, J = 2.7 Hz, 1H), 6.48 (dd, J = 8.8, 2.8 Hz, 1H), 5.08 (s, 1H), 4.40 (s, 4H), 3.98 (dd, J = 9.6, 3.1 Hz, 1H), 3.80 (s, 3H), 2.24 (ddd, J = 12.4, 9.4, 2.4 Hz, 1H), 2.14 2.00 (m, 1H), 1.96 1.82 (m, 2H), 1.83 1.69 (m, 6H); MS (ESI⁺) *m*/*z* 541.2 (M+H)⁺.

### Example 430: N,N'-(bicyclo[2.2.1]heptane-1,4-diyl)bis{2-[4-(trifluoromethyl)phenoxylacetamide} (Compound 529)

The title compound was prepared using the methodologies described in Example 341 substituting 2-(4-(trifluoromethyl)phenoxy)acetic acid for 2-(3-chloro-4-fluorophenoxy)acetic acid. ¹H NMR (400 MHz, DMSO-*d*₆) *δ* ppm 8.10 (s, 2H), 7.38 7.29 (m, 4H), 7.00 6.89 (m, 4H), 4.43 (s, 4H), 2.03 (s, 2H), 1.88 (d, J = 6.3 Hz, 4H), 1.78 (dd, J = 15.9, 10.3 Hz, 4H); MS (ESI⁺) *m*/*z* 463.1 (M+H)⁺.

### Example 431: N,N'-(bicyclo[2.2.1]heptane-1,4-diyl)bis(2-{[6-(trifluoromethyl)pyridin-3-yl]oxy}acetamide) (Compound 530)

The title compound was prepared using the methodologies described in Example 341 substituting 2-((6-(trifluoromethyl)pyridin-3-yl)oxy)acetic acid for 2-(3-chloro-4-fluorophenoxy)acetic acid. ¹H NMR (400 MHz, DMSO-*d*₆) *δ* ppm 8.19 (s, 2H), 7.70 7.63 (m, 4H), 7.11 (d, J = 8.6 Hz, 4H), 4.54 (s, 2H), 2.04 (s, 2H), 1.93 1.82 (m, 4H), 1.78 (t, J = 6.2 Hz, 4H); MS (ESI⁺) *m*/*z* 547.8 (M+NH₄)⁺.

### Example 432: N,N'-(bicyclo[2.2.1]heptane-1,4-diyl)bis{2-[(2,2-difluoro-2H-1,3-benzodioxol-5-yl)oxy]acetamide} (Compound 531)

The title compound was prepared using the methodologies described in Example 341 substituting 2-((2,2-difluorobenzo[d][1,3]dioxol-5-yl)oxy)acetic acid (Example 166A) for 2-(3-chloro-4-fluorophenoxy)acetic acid. ¹H NMR (400 MHz, DMSO-*d*₆) *δ* ppm 8.10 (s, 2H), 7.31 (d, J = 8.9 Hz, 2H), 7.12 (d, J = 2.5 Hz, 2H), 6.75 (dd, J = 8.9, 2.6 Hz, 2H), 4.44 (s, 4H), 2.05 (s, 2H), 1.89 (d, J = 5.9 Hz, 4H), 1.78 (t, J = 6.2 Hz, 4H); MS (ESI⁺) *m*/*z* 571.8 (M+NH₄)⁺.

### Example 433: 2-(3,4-dichlorophenoxy)-N-{3-[2-(4-fluorophenoxy)acetamido]bicyclo[1.1.1]pentan-1-yl}acetamide (Compound 532)

To a mixture of 2-(4-fluorophenoxy)acetic acid (20.42 mg, 0.120 mmol) and (1-[bis(dimethylamino)methylene]-1*H*-1,2,3-triazolo[4,5-b]pyridinium 3-oxid hexafluorophosphate) (HATU, 45.6 mg, 0.120 mmol) and*N*-(3-aminobicyclo[1.1.1]pentan-1-yl)-2-(3,4-dichlorophenoxy)acetamide, hydrochloric acid (Example 6C, 40.5 mg, 0.12 mmol) was added *N*-ethyl-*N*-isopropylpropan-2-amine (62.0 mg, 0.480 mmol) in *N,N-*dimethylformamide (1 mL). The mixture was stirred at room temperature for 20 minutes, and then water (0.02 mL) was added. The residue was purified by preparative HPLC (Phenomenex^{®} Luna^{®} C18(2) 5 µm 100 Å AXIA^{™} column 250 mm × 21.2 mm, flow rate 25 mL/minute, 5-95% gradient of acetonitrile in buffer (0.1% trifluoroacetic acid in water)) to give the titled compound (39 mg, 0.086 mmol, 72%). ¹H NMR (400 MHz, DMSO-*d*₆) *δ* ppm 7.86 (d, J = 9 Hz, 2H), 7.54 (d, J = 8 Hz, 1H), 7.26 (d, J = 3 Hz, 1H), 7.13 (t, J = 8 Hz, 2H), 6.97 (m, 3H), 4.48 (s, 2H), 4.40 (s, 2H), 2.27 (s, 6H); MS (ESI+) m/z 453 (M+H)⁺.

### Example 434: 2-(3-chloro-4-fluorophenoxy)-N-{3-[2-(3,4-dichlorophenoxy)acetamido]bicyclo[1.1.1]pentan-1-yl}acetamide (Compound 533)

### Example 434A: tert-butyl 2-(3-chloro-4-fluorophenoxy)acetate

To a solution of 3-chloro-4-fluorophenol (2.13 g, 14.5 mmol) in *N,N*-dimethylformamide (14.5 mL) at ambient temperature was added potassium carbonate (4.01 g, 29.0 mmol) and *tert-*butyl bromoacetate (2.45 mL, 16.7 mmol). This mixture was warmed to 65 °C and was stirred for 4 hours. The mixture was cooled to ambient temperature and was filtered through a glass microfiber frit. The filtrate was directly purified by preparative HPLC [YMC TriArt^{™} C18 Hybrid 20 µm column, 50 × 150 mm, flow rate 75 mL/minute, 40-85% gradient of acetonitrile in buffer (0.025 M aqueous ammonium bicarbonate, adjusted to pH 10 with ammonium hydroxide)] to give the title compound (3.1 g, 11.9 mmol, 82% yield). ¹H NMR (400 MHz, CDCl₃) *δ* ppm 7.05 (t, J = 8.8 Hz, 1H), 6.93 (dd, J = 5.9, 3.0 Hz, 1H), 6.76 (dt, J = 9.1, 3.4 Hz, 1H), 4.47 (s, 2H), 1.49 (s, 9H).

### Example 434B: 2-(3-chloro-4-fluorophenoxy)acetic acid

The reaction and isolation conditions described in Example 83B, substituting the product of Example 434A for the product of Example 83A gave the title compound. MS (ESI⁻) m/z 203 (M-H)⁻.

### Example 434C: 2-(3-chloro-4-fluorophenoxy)-N-{3-[2-(3,4-dichlorophenoxy)acetamido]bicyclo[1.1.1]pentan-1-yl}acetamide

The reaction and purification conditions described in Example 81, substituting the product of Example 434B for 2-(4-chloro-3-fluorophenoxy)acetic acid, and the product of Example 6C for benzyl (4-aminobicyclo[2.1.1]hexan-1-yl)carbamate hydrochloride gave the title compound. ¹H NMR (400 MHz, DMSO-*d*₆) *δ* ppm 8.71 (s, 1H), 8.69 (s, 1H), 7.55 (d, J = 8.9 Hz, 1H), 7.35 (t, J = 9.1 Hz, 1H), 7.26 (d, J = 2.9 Hz, 1H), 7.20 (dd, J = 6.1, 3.0 Hz, 1H), 7.01 - 6.94 (m, 2H), 4.49 (s, 2H), 4.45 (s, 2H), 2.27 (s, 6H); MS (ESI⁺) *m*/*z* 487/489 (M+H)⁺.

### Example 435: 2-(4-chloro-3-fluorophenoxy)-N-[3-[2-(3-chloro-4-fluorophenoxy)acetamido]bicyclo[1.1.1]pentan-1-yl}acetamide (Compound 534)

The reaction and purification conditions described in Example 81, substituting the product of Example 434B for 2-(4-chloro-3-fluorophenoxy)acetic acid, and the product of Example 27D for benzyl (4-aminobicyclo[2.1.1]hexan-1-yl)carbamate hydrochloride gave the title compound. ¹H NMR (501 MHz, DMSO-*d*₆) *δ* ppm 8.72 (s, 1H), 8.70 (s, 1H), 7.49 (t, J = 8.9 Hz, 1H), 7.35 (t, J = 9.1 Hz, 1H), 7.20 (dd, J = 6.1, 3.1 Hz, 1H), 7.07 (dd, J = 11.4, 2.8 Hz, 1H), 6.97 (ddd, J = 9.1, 3.8, 3.0 Hz, 1H), 6.85 (ddd, J = 8.9, 2.9, 1.2 Hz, 1H), 4.48 (s, 2H), 4.45 (s, 2H), 2.27 (s, 6H); MS (ESI⁺) *m*/*z* 471 (M+H)⁺.

### Example 436: 2-(3-chloro-4-fluorophenoxy)-N-{4-[2-(4-chloro-3-fluorophenoxy)acetamido]bicyclo[2.1.1]hexan-1-yl}acetamide (Compound 535)

The reaction and purification conditions described in Example 81, substituting the product of Example 434B for 2-(4-chloro-3-fluorophenoxy)acetic acid, and the product of Example 88C for benzyl (4-aminobicyclo[2.1.1]hexan-1-yl)carbamate hydrochloride gave the title compound. ¹H NMR (400 MHz, DMSO-*d*₆) *δ* ppm 8.71 (s, 1H), 8.69 (s, 1H), 7.55 (d, J = 8.9 Hz, 1H), 7.35 (t, J = 9.1 Hz, 1H), 7.26 (d, J = 2.9 Hz, 1H), 7.20 (dd, J = 6.1, 3.0 Hz, 1H), 7.01 - 6.94 (m, 2H), 4.49 (s, 2H), 4.45 (s, 2H), 2.27 (s, 6H); MS (ESI⁺) *m*/*z* 487/489 (M+H)⁺.

### Example 437: N,N'-[(2S)-2-hydroxybicyclo[2.2.2]octane-1,4-diyl]bis{2-[4-(trifluoromethyl)phenoxy]acetamide} (Compound 536)

A mixture of Example 343D (40.0 mg, 0.175 mmol), (1-[bis(dimethylamino)methylene]-1H-1,2,3-triazolo[4,5-b]pyridinium 3-oxid hexafluorophosphate) (HATU, 173 mg, 0.454 mmol), 2-(4-(trifluoromethyl)phenoxy)acetic acid (100 mg, 0.454 mmol), and triethylamine (0.122 mL, 0.873 mmol) in N,N-dimethylformamide (2 mL) was stirred for 3 hours. The reaction was quenched with brine and saturated NaHCO₃, and extracted with ethyl acetate (2×). The combined organic layers were washed with brine, dried over MgSO₄, filtered, concentrated, and purified on a 12 g silica column using the Biotage^{®} Isolera^{™} One flash system eluting with heptanes/ethyl acetate (4:6 to 3:7) to give 43.5 mg of the impure product, which was further purified by reverse-phase HPLC (see protocol in Example 112D) to provide the title compound (24.8 mg, 25%). ¹H NMR (400 MHz, DMSO-*d*₆) *δ* ppm 7.70 - 7.61 (m, 4H), 7.56 (s, 1H), 7.32 (s, 1H), 7.09 (dd, J = 10.6, 8.6 Hz, 4H), 5.09 (s, brd, 1H), 4.53 (s, 2H), 4.50 (s, 2H), 4.04 (dd, J = 9.6, 3.1 Hz, 1H), 2.28 (ddd, J = 12.2, 9.6, 2.2 Hz, 1H), 2.09 (ddd, J = 12.5, 10.6, 4.7 Hz, 1H), 1.99 - 1.70 (m, 8H); MS (ESI⁺) *m*/*z* 561.1 (M+H)⁺.

### Example 438: 2-(4-chloro-3-fluorophenoxy)-N-[(3S)-4-[2-[4-(difluoromethyl)phenoxy]acetamido}-3-hydroxybicyclo[2.2.2]octan-1-yl]acetamide (Compound 537)

To a mixture of *N*-[(3S)-4-amino-3-hydroxybicyclo[2.2.2]octan-1-yl]-2-(4-chloro-3-fluorophenoxy)acetamide trifluoroacetate (Example 276A, 37.0 mg, 0.081 mmol), (1-[bis(dimethylamino)methylene]-1*H*-1,2,3-triazolo[4,5-*b*]pyridinium 3-oxid hexafluorophosphate) (HATU, 30.8 mg, 0.081 mmol) and 2-(4-(difluoromethyl)phenoxy)acetic acid (16.37 mg, 0.081 mmol) was added *N*-ethyl-*N*-isopropylpropan-2-amine (31.4 mg, 0.243 mmol) in N,N-dimethylformamide (1 mL). The mixture was stirred at room temperature for 15 minutes. Then water (0.02 mL) was added to quench the reaction. The residue was purified by preparative HPLC (Phenomenex^{®} Luna^{®} C18(2) 5 µm 100 Å AXIA^{™} column 250 mm × 21.2 mm, flow rate 25 mL/minute, 5-95% gradient of acetonitrile in buffer (0.1% trifluoroacetic acid in water)) to give the title compound (34 mg, 0.065 mmol, 80% yield). ¹H NMR (400 MHz, DMSO-*d*₆) *δ* ppm 7.51 (s, 1H), 7.50 (d, J = 8 Hz, 2H), 7.48 (t, J = 8 Hz, 1H), 7.27 (s, 1H), 7.04 (d, J = 8 Hz, 2H), 7.02 (dd, J = 9, 3 Hz, 1H), 6.95 (t, J = 56 Hz, 1H), 6.81 (br d, J = 8 Hz, 1H), 4.48 (s, 2H), 4.43 (s, 2H), 4.02 (m, 1H), 2.26 (m, 1H), 2.17 (m, 1H), 1.75 - 1.96 (m, 8H); MS (ESI+) *m*/*z* 527 (M+H)⁺.

### Example 439: N,N'-[(2S)-2-hydroxybicyclo[2.2.2]octane-1,4-diyl]bis[2-(3,4-dichlorophenoxy)acetamide] (Compound 538)

A mixture of Example 343D (40.0 mg, 0.175 mmol), (1-cyano-2-ethoxy-2-oxoethylidenaminooxy)dimethylamino-morpholino-carbenium hexafluorophosphate (COMU, 194 mg, 0.454 mmol), 2-(3,4-dichlorophenoxy)acetic acid (100 mg, 0.454 mmol), and triethylamine (0.122 mL, 0.873 mmol) in N,N-dimethylformamide (2.5 mL) was stirred for 3 hours. The reaction was quenched with brine and extracted with ethyl acetate (2×). The combined organic layers were washed with brine, dried over MgSO₄, filtered, and concentrated. The concentrate was dissolved in tetrahydrofuran (1.2 mL) and methanol (0.8 mL) and treated with 1 N NaOH (0.5 mL). The suspension was stirred for 1 hour, diluted with water and brine, and extracted with ethyl acetate (2×). The combined organic layers were dried over MgSO₄ and concentrated, and the residue was purified by reverse-phase HPLC (see protocol in Example 112D) to provide the title compound (28.2 mg, 29%). ¹H NMR (500 MHz, DMSO-*d*₆) *δ* ppm 7.59 - 7.48 (m, 3H), 7.30 - 7.16 (m, 3H), 6.95 (ddd, J = 9.8, 8.9, 2.9 Hz, 2H), 5.08 (d, J = 4.4 Hz, 1H), 4.48 (s, 2H), 4.44 (s, 2H), 4.09 - 3.99 (m, 1H), 2.32 - 2.20 (m, 1H), 2.13 - 2.02 (m, 1H), 1.98 - 1.72 (m, 8H); MS (ESI⁺) *m*/*z* 561.0 (M+H)⁺.

### Example 440: 2-(4-chloro-3-fluorophenoxy)-N-[(2S)-4-{2-[4-(difluoromethyl)phenoxy]acetamidol-2-hydroxybicyclo[2.2.2]octan-1-yl]acetamide (Compound 539)

The title compound was prepared using the methodologies described in Example 338C substituting 2-(4-(difluoromethyl)phenoxy)acetic acid for 2-(4-chlorophenoxy)acetic acid. ¹H NMR (400 MHz, DMSO-*d*₆) *δ* ppm 7.54 - 7.43 (m, 3H), 7.26 (s, 1H), 7.11 - 6.98 (m, 2H), 6.95 (t, J = 56.0 Hz, 1H), 6.87 - 6.78 (m, 1H), 5.05 (s, 1H), 4.46 (d, J = 7.0 Hz, 3H), 4.04 (dd, J = 9.6, 3.1 Hz, 1H), 2.28 (ddd, J = 12.2, 9.5, 2.1 Hz, 1H), 2.13 - 1.96 (m, 1H), 2.00 - 1.73 (m, 8H); MS (ESI⁺) *m*/*z* 527.0 (M+H)⁺.

### Example 441: N,N'-[(2S)-2-hydroxybicyclo[2.2.2]octane-1,4-diyl]bis[2-(4-chlorophenoxy)acetamide]

A mixture of Example 343D (40.0 mg, 0.175 mmol), (1-[bis(dimethylamino)methylene]-1*H*-1,2,3-triazolo[4,5-*b*]pyridinium 3-oxid hexafluorophosphate) (HATU, 173 mg, 0.454 mmol), 2-(4-chlorophenoxy)acetic acid (85 mg, 0.454 mmol), and triethylamine (0.122 mL, 0.873 mmol) in *N,N*-dimethylformamide (2 mL) was stirred for 3 hours. The reaction was quenched with brine and extracted with ethyl acetate (2×). The combined organic layers were washed with brine, dried over MgSO₄, filtered, and concentrated. The concentrate was dissolved in tetrahydrofuran (1.2 mL) and methanol (0.8 mL) and treated with1 N NaOH (0.5 mL). The suspension was stirred for 1 hour, diluted with water and brine, and extracted with ethyl acetate (2×). The combined organic layers were dried over MgSO₄ and concentrated, and the residue was purified by reverse-phase HPLC (see protocol in Example 112D) to provide the title compound (29.8 mg, 35%). ¹H NMR (500 MHz, DMSO-*d*₆) *δ* ppm 7.46 (s, 1H), 7.37 - 7.27 (m, 4H), 7.23 (s, 1H), 7.00 - 6.88 (m, 4H), 5.11 (d, J = 4.5 Hz, 1H), 4.42 (d, J = 1.1 Hz, 2H), 4.39 (s, 2H), 4.06 - 3.97 (m, 1H), 2.27 (ddd, J = 12.5, 9.4, 2.5 Hz, 1H), 2.16 - 2.07 (m, 1H), 1.98 - 1.71 (m, 8H); MS (ESI⁺) *m*/*z* 493.0 (M+H)⁺.

### Example 442: N,N'-[(2S)-2-hydroxybicyclo[2.2.2]octane-1,4-diyl]bis(2-{[6-(trifluoromethyl)pyridin-3-yl]oxy}acetamide)

A mixture of Example 343D (40.0 mg, 0.175 mmol), (1-[bis(dimethylamino)methylene]-1*H*-1,2,3-triazolo[4,5-*b*]pyridinium 3-oxid hexafluorophosphate) (HATU, 173 mg, 0.454 mmol), 2-((6-(trifluoromethyl)pyridin-3-yl)oxy)acetic acid (100 mg, 0.454 mmol), and triethylamine (0.122 mL, 0.873 mmol) in *N,N*-dimethylformamide (2 mL) was stirred for 3 hours. The reaction was quenched with brine and saturated NaHCO₃, and extracted with ethyl acetate (2×). The combined organic layers were washed with brine, dried over MgSO₄, filtered, and concentrated. The concentrate was dissolved in tetrahydrofuran (1.7 mL) and methanol (0.8 mL) and treated with a solution of lithium hydroxide (5.02 mg, 0.209 mmol) in water (0.5 mL). The suspension was stirred for 1 hour, diluted with water and brine, and extracted with ethyl acetate (3×). The combined organic layers were concentrated, and the residue was purified by reverse-phase HPLC (see protocol in Example 112D) to provide the title compound (48.6 mg, 50%). ¹H NMR (400 MHz, DMSO-*d*₆) *δ* ppm 8.43 (dd, J = 6.7, 2.8 Hz, 2H), 7.92 - 7.78 (m, 2H), 7.64 (s, 1H), 7.59 - 7.45 (m, 2H), 7.40 (s, 1H), 5.04 (d, J = 4.3 Hz, 1H), 4.65 (s, 2H), 4.62 (s, 2H), 4.09 (dt, J = 9.7, 3.6 Hz, 1H), 2.33 - 2.21 (m, 1H), 2.06 - 1.70 (m, 9H); MS (ESI⁺) *m*/*z* 563.2 (M+H)⁺.

### Example 443: N,N'-[(2S)-2-hydroxybicyclo[2.2.2]octane-1,4-diyl]bis{2-[(2,2-difluoro-2H-1,3-benzodioxol-5-yl)oxy]acetamide}

The reaction described in Example 442 substituting 2-((2,2-difluorobenzo[d] [1,3]dioxol-5-yl)oxy)acetic acid (Example 166A) for 2-((6-(trifluoromethyl)pyridin-3-yl)oxy)acetic acid gave the title compound. ¹H NMR (400 MHz, DMSO-*d*₆) *δ* ppm 7.45 (s, 1H), 7.31 (dd, J = 8.9, 1.8 Hz, 2H), 7.22 (s, 1H), 7.11 (dd, J = 11.0, 2.6 Hz, 2H), 6.73 (td, J = 8.9, 2.6 Hz, 2H), 5.10 (d, J = 4.4 Hz, 1H), 4.43 (s, 2H), 4.40 (s, 2H), 4.03 (dt, J = 9.3, 3.8 Hz, 1H), 2.28 (ddd, J = 12.7, 9.7, 2.5 Hz, 1H), 2.17 - 2.03 (m, 1H), 2.00 - 1.71 (m, 8H); MS (ESI⁺) *m*/*z* 585.0 (M+H)⁺.

### Example 444: N-{3-[2-(4-chloro-3-fluorophenoxy)acetamido]bicyclo[1.1.1]pentan-1-yl}-4-(3-chlorophenyl)butanamide (Compound 543)

The reaction and purification conditions described in Example 81, substituting 4-(3-chlorophenyl)butanoic acid (ArkPharm) for 2-(4-chloro-3-fluorophenoxy)acetic acid, and the product of Example 27D for benzyl (4-aminobicyclo[2.1.1]hexan-1-yl)carbamate hydrochloride gave the title compound. ¹H NMR (400 MHz, DMSO-*d*₆) *δ* ppm 8.68 (s, 1H), 8.35 (s, 1H), 7.49 (t, J = 8.8 Hz, 1H), 7.34 - 7.20 (m, 3H), 7.15 (dt, J = 7.6, 1.4 Hz, 1H), 7.08 - 7.04 (m, 1H), 6.85 (ddd, J = 8.9, 2.8, 1.2 Hz, 1H), 4.47 (s, 2H), 2.55 (t, J = 7.6 Hz, 2H), 2.20 (s, 6H), 2.07 - 1.99 (m, 2H), 1.81 - 1.71 (m, 2H); MS (ESI⁺) *m*/*z* 465 (M+H)⁺.

### Example 445: 2-(3-chloro-4-fluorophenoxy)-N-{(3S)-4-[2-(3,4-dichlorophenoxy)acetamido]-3-hydroxybicyclo[2.2.2]octan-1-yl}acetamide (Compound 544)

### Example 445A: (S)-tert-butyl (4-(benzylamino)-2-hydroxybicyclo[2.2.2]octan-1-yl)carbamate, hydrochloric acid

Magnesium sulfate (0.196 g) and nicotinamide adenine dinucleotide phosphate (NADPH, 0.200 g) were mixed in 360 mL of potassium phosphate buffer (125 mM, pH = 7.0) and 0.04 L of isopropanol. 60 mL of this solution was reserved and used to dissolve Codexis KRED P02C2 enzyme (400 mg). Example 343B (20.0 g) was added to the 340 mL of remaining buffered solution, and the pH was adjusted to 7.5 with 50% (w/w) NaOH. The reaction was initiated by addition of the enzyme in 60 mL buffered solution. The reaction was stirred overnight at 40 °C. The cloudy, aqueous solution was adjusted to pH > 11 with 50% weight/weight aqueous sodium hydroxide. Diatomaceous earth (20g) was added to the reaction mixture, and then the resultant mixture was stirred for 10 minutes. The reaction was filtered to remove all insoluble material. The aqueous layer was charged back to the reaction vessel and di-tert-butyl dicarbonate (16 g, 1.2 equivalents) in 400 mL of ethyl acetate was charged to the same vessel. The biphasic solution was stirred for two hours. The aqueous layer was routinely checked to maintain pH > 10. At 2 hours, the two layers were separated, and the aqueous layer was charged back to the reaction vessel. The amount of amino alcohol intermediate remaining in the aqueous layer was determined by high performance liquid chromatography (HPLC) and 1.2 equivalents of *di-tert-*butyl dicarbonate was added to the reaction vessel dissolved in 200 mL ethyl acetate. The pH was maintained >10. This reaction proceeded for 2 hours, and the two layers were separated. The organic layers were combined, washed with brine containing 2.5% sodium hydroxide (60 mL), filtered through magnesium sulfate, and concentrated in vacuo. The residual material was taken up in 200 mL methyl *tert-butyl* ether, cooled to 5 °C and 4 N HCl in dioxane (14.0 mL) was slowly added to the solution. The precipitated material was collected by filtration and dried in vacuo to provide the title compound (18.1 g, 75%). MS (APCI⁺) m/z 347.4 (M+H)⁺.

### Example 445B: (S)-tert-butyl (4-amino-2-hydroxybicyclo[2.2.2]octan-1-yl)carbamate hydrochloride

Example 445A (10.01 g, 26.1 mmol) in methanol (84 ml) was added to 20% Pd(OH)₂/C (wet, 0.979 g, 3.56 mmol) in a 300 mL stainless steel reactor. The reactor was purged with argon and stirred at 1200 RPM at 40 °C under 50 psi of hydrogen for 21 hours. The reaction mixture was filtered and concentrated to provide the title compound (7.3 g, 95 % yield). MS (APCI⁺) *m*/*z* 257.3 (M+H)⁺.

### Example 445C: (S)-(9H-fluoren-9-yl)methyl tert-butyl (2-hydroxybicyclo[2.2.2]octane-1,4-diyl)dicarbamate

To a suspension of Example 445B (10.0022 g, 34.2 mmol) and Na₂CO₃ (10.8492 g, 102 mmol) in tetrahydrofuran (100 mL) and water (50 mL) at 0 °C was added (9H-fluoren-9-yl)methyl carbonochloridate (10.6051 g, 41.0 mmol) at once. The mixture was stirred at 0 °C for 5 minutes, allowed to warm to room temperature, stirred for 90 minutes, diluted with ethyl acetate (150 mL), washed with water (150 mL) and brine (50 mL), dried over Na₂SO₄, and concentrated to give the title compound (19.72 g, 41.2 mmol, 121 % yield) that was used in the next step without further purifications. MS (ESI⁺) *m*/*z* 479.2 (M+H)⁺.

### Example 445D: (S)-(9H-fluoren-9-yl)methyl (4-amino-3-hydroxybicyclo[2.2.2]octan-1-yl)carbamate hydrochloride

A 4 M/dioxane solution of hydrogen chloride (17.2 mL, 68.8 mmol) was added to a solution of Example 445C (16.37 g, 34.2 mmol) in methanol (35 mL), and the mixture was heated to 50 °C for 90 minutes, concentrated, and triturated with ethyl acetate (2 × 100 mL). The material was vacuum oven-dried to provide the title compound (13.95 g, 33.6 mmol, 98 % yield) that was used in the next step without further purifications. MS (APCI⁺) *m*/*z* 379.2 (M+H)⁺.

### Example 445E: (S)-(9H-fluoren-9-yl)methyl (4-(2-(3,4-dichlorophenoxy)acetamido)-3-hydroxybicyclo[2.2.2]octan-1-yl)carbamate

A suspension of Example 445D (13.95 g, 33.6 mmol), 2-(3,4-dichlorophenoxy)acetic acid (8.5352 g, 38.6 mmol), and triethylamine (11.8 mL, 85 mmol) in N,N-dimethylformamide (100 mL) at room temperature was stirred for 3 minutes, and (1-[bis(dimethylamino)methylene]-1*H*-1,2,3-triazolo[4,5-*b*]pyridinium 3-oxid hexafluorophosphate) (HATU, 15.9613 g, 42.0 mmol) was then added, and the mixture was stirred for 3.5 hours. The reaction mixture was diluted with ethyl acetate (300 mL), washed with water (250 mL), 0.5 N NaOH (200 mL), and brine (100 mL), and concentrated to give a solid. This material was dissolved in methanol (16 mL) and tetrahydrofuran (48 mL), stirred with 1 N aqueous sodium hydroxide (16 mL, 16.00 mmol) for 30 minutes, concentrated, and diluted with water (100 mL). The solids were collected by filtration and dried (vacuum oven) to provide the title compound (17.52 g, 30.1 mmol, 90 % yield) that was used in the next step without further purification. MS (APCI⁺) *m*/*z* 581.1 (M+H)⁺.

### Example 445F: (S)-N-(4-amino-2-hydroxybicyclo[2.2.2]octan-1-yl)-2-(3,4-dichlorophenoxy)acetamide

To a suspension of Example 445E (17.52 g, 30.1 mmol) in acetonitrile (100 mL) was added diethylamine (31 mL, 300 mmol), and the mixture was stirred for 75 minutes. Then the reaction mixture was concentrated, diluted with water (100 mL), acidified with concentrated HCl (2.5 mL, till pH-3), and extracted with ethyl acetate (2 × 50 mL). The aqueous layer was concentrated, diluted with isopropanol, acetonitrile, and toluene, and then re-concentrated to remove residual water. The residue was dissolved in methanol, filtered to remove some debris, and concentrated. The concentrate was triturated with acetonitrile (45 mL), cooled to 0 °C, collected by filtration, and dried (vacuum oven) to give the crude title compound. This material was diluted with 1 N NaOH (30 mL), extracted with ethyl acetate (2 × 50 mL), washed with brine, dried (Na₂SO₄), and concentrated to give 8.1 g of impure title compound which was adsorbed onto silica and chromatographed (0-20% CH₃OH/CH₂Cl₂, 80 g silica column) to provide the title compound (5.4537 g, 15.18 mmol, 50.4 % yield). MS (APCI⁺) *m*/*z* 359.1 (M+H)⁺.

### Example 445G: 2-(3-chloro-4-fluorophenoxy)-N-{(3S)-4-[2-(3,4-dichlorophenoxy)acetamido]-3-hydroxybicyclo[2.2.2]octan-1-yl}acetamide

The title compound was prepared using the methodologies described in Example 338C substituting Example 445F for Example 338B and 2-(4-chloro-3-fluorophenoxy)acetic acid for 2-(4-chlorophenoxy)acetic acid. ¹H NMR (400 MHz, DMSO-*d*₆) *δ* ppm 7.57 7.45 (m, 2H), 7.34 (t, J = 9.1 Hz, 1H), 7.29 7.20 (m, 2H), 7.15 (dd, J = 6.1, 3.0 Hz, 1H), 7.00 6.88 (m, 2H), 5.08 (d, J = 4.4 Hz, 1H), 4.48 (s, 2H), 4.41 (s, 2H), 4.04 (dt, J = 9.1, 4.1 Hz, 1H), 2.27 (ddd, J = 12.2, 9.5, 2.3 Hz, 1H), 2.13 2.01 (m, 1H), 1.97 1.83 (m, 2H), 1.87 1.72 (m, 6H); MS (ESI⁺) *m*/*z* 545.1 (M+H)⁺.

### Example 446: 2-(4-chloro-3-fluorophenoxy)-N-{(2S)-4-[2-(3-chloro-4-fluorophenoxy)acetamido]-2-hydroxybicyclo[2.2.2]octan-1-yl}acetamide (Compound 545)

The title compound was prepared using the methodologies described in Example 338C substituting 2-(3-chloro-4-fluorophenoxy)acetic acid for 2-(4-chlorophenoxy)acetic acid. ¹H NMR (400 MHz, DMSO-*d*₆) *δ* ppm 7.53 7.43 (m, 2H), 7.34 (t, J = 9.1 Hz, 1H), 7.26 (s, 1H), 7.15 (dd, J = 6.1, 3.1 Hz, 1H), 7.05 (dd, J = 11.3, 2.9 Hz, 1H), 6.93 (dt, J = 9.2, 3.5 Hz, 1H), 6.83 (ddd, J = 9.0, 2.9, 1.2 Hz, 1H), 5.07 (s, 1H), 4.44 (d, J = 22.7 Hz, 4H), 4.04 (dd, J = 9.6, 3.1 Hz, 1H), 2.27 (ddd, J = 12.1, 9.5, 2.2 Hz, 1H), 2.07 (ddd, J = 12.4, 10.6, 4.7 Hz, 1H), 1.97 1.87 (m, 2H), 1.91 1.72 (m, 6H); MS (ESI⁺) *m*/*z* 529.1 (M+H)⁺.

### Example 447: 2-(3-chloro-4-fluorophenoxy)-N-{3-[2-(4-chlorophenoxy)acetamido]bicyclo[1.1.1]pentan-1-yl}acetamide (Compound 546)

The reaction and purification conditions described in Example 81, substituting the product of Example 434B for 2-(4-chloro-3-fluorophenoxy)acetic acid, and the product of Example 14B for benzyl (4-aminobicyclo[2.1.1]hexan-1-yl)carbamate hydrochloride gave the title compound. ¹H NMR (501 MHz, DMSO-*d*₆) *δ* ppm 8.70 (s, 1H), 8.69 (s, 1H), 7.38 - 7.31 (m, 3H), 7.20 (dd, J = 6.1, 3.1 Hz, 1H), 7.00 - 6.94 (m, 3H), 4.45 (s, 2H), 4.43 (s, 2H), 2.27 (s, 6H); MS (ESI⁺) *m*/*z* 453 (M+H)⁺.

### Example 448: N,N'-(bicyclo[1.1.1]pentane-1,3-diyl)bis[2-(3-chloro-4-fluorophenoxy)acetamide] (Compound 547)

*tert*-Butyl (3-aminobicyclo[1.1.1]pentan-1-yl)carbamate (30 mg, 0.15 mmol, PharmaBlock) was dissolved in dichloromethane (1 mL) and trifluoroacetic acid (0.5 mL) was added. The resulting solution was stirred at ambient temperature for 30 minutes and then concentrated under reduced pressure. To the resulting residue was added triethylamine (0.13 mL, 0.91 mmol), *N,N*-dimethylformamide (3 mL), the product of Example 434B (65 mg, 0.32 mmol), and (1-[bis(dimethylamino)methylene]-1*H*-1,2,3-triazolo[4,5-*b*]pyridinium 3-oxid hexafluorophosphate) (HATU, 126 mg, 0.33 mmol) in sequential order. The resulting reaction mixture was stirred at ambient temperature for 30 minutes, filtered through a glass microfiber frit, and purified by preparative HPLC [Waters XBridge^{™} C18 5 µm OBD^{™} column, 30 × 100 mm, flow rate 40 mL/minute, 5-100% gradient of acetonitrile in buffer (0.025 M aqueous ammonium bicarbonate, adjusted to pH 10 with ammonium hydroxide)] to give the title compound (59 mg, 0.13 mmol, 83% yield). ¹H NMR (501 MHz, DMSO-*d*₆) *δ* ppm 8.70 (s, 2H), 7.35 (t, J = 9.1 Hz, 2H), 7.20 (dd, J = 6.1, 3.1 Hz, 2H), 6.97 (ddd, J = 9.2, 3.8, 3.1 Hz, 2H), 4.46 (s, 4H), 2.27 (s, 6H); MS (ESI⁺) *m*/*z* 471 (M+H)⁺.

### Example 449: N,N'-(2,3-dihydroxybicyclo[2.2.2]octane-1,4-diyl)bis[2-(4-chloro-3-fluorophenoxy)acetamide] (Compound 548)

### Example 449A: (R)-O-(1,4-bis(2-(4-chloro-3-fluorophenoxy)acetamido)bicyclo[2.2.2]octan-2-yl) O-(p-tolyl) carbonothioate

To a suspension of Example 199 (0.15 g, 0.28 mmol), *N,N*-dimethylpyridin-4-amine (0.035 g, 0.29 mmol), and triethylamine (0.043 ml, 0.31 mmol) in CH₂Cl₂ (1.1 mL) at ambient temperature was added *O*-*p*-tolyl carbonochloridothioate (0.045 ml, 0.30 mmol). The mixture was stirred for 1 hour and then was heated to reflux briefly to give a solution. The mixture was allowed to cool to 40 °C, and the mixture was allowed to stir for 3 hours. The reaction mixture was diluted with saturated, aqueous NaHCO₃ and extracted with CH₂Cl₂ (3 × 10 mL). The combined organic fractions were dried over anhydrous Na₂SO₄ and concentrated under reduced pressure. The residue was purified via column chromatography (SiO₂, 20% ethyl acetate/CH₂Cl₂) to give the title compound (0.16 g, 0.23 mmol, 81% yield). ¹H NMR (400 MHz, DMSO-*d*₆) *δ* ppm7.75 (s, 1H), 7.69 (s, 1H), 7.46 (dt, *J =* 11.6, 8.9 Hz, 2H), 7.26 - 7.19 (m, 2H), 7.06 - 6.95 (m, 4H), 6.81 (dddd, *J =* 8.5, 5.5, 2.9, 1.2 Hz, 2H), 5.88 (dd, *J =* 9.4*,* 2.1 Hz, 1H), 4.50 (s, 2H), 4.45 (s, 2H), 2.59 - 2.52 (m, 1H), 2.37 (m, 1H), 2.31 (s, 3H), 2.12 - 2.00 (m, 2H), 1.96 - 1.71 (m, 6H).

### Example 449B: N,N'-(bicyclo[2.2.2]oct-2-ene-1,4-diyl)bis(2-(4-chloro-3-fluorophenoxy)acetamide)

A solution of the product of Example 449A (0.15 g, 0.23 mmol) in toluene (0.90 mL) and N,N-dimethylformamide (DMF) (0.10 mL) was microwaved at 170 °C for 20 minutes. The mixture was diluted with ethyl acetate (10 mL) and washed with 1 N NaOH (5 mL) and brine (5 mL). The organic layer was concentrated under reduced pressure, and the residue was purified via column chromatography (SiO₂, 4% CH₃OH/CH₂Cl₂) to give the title compound (0.065 g, 0.13 mmol, 56% yield). ¹H NMR (400 MHz, DMSO-*d*₆) *δ* ppm 8.05 (s, 2H), 7.49 (t, *J =* 8.9 Hz, 2H), 7.06 (dd, *J =* 11.4, 2.9 Hz, 2H), 6.84 (ddd, *J =* 8.9, 2.9, 1.1 Hz, 2H), 6.19 (s, 2H), 4.53 (s, 4H), 1.82 - 1.60 (m, 8H); MS (APCI⁺) *m*/*z* 511 (M+H)⁺.

### Example 449C: N,N'-(2,3-dihydroxybicyclo[2.2.2]octane-1,4-diyl)bis[2-(4-chloro-3-fluorophenoxy)acetamide]

A biphasic suspension of the product of Example 449B (0.027 g, 0.053 mmol) and AD-mix □ (0.08 g, 0.053 mmol) in t-butanol (0.25 mL) and water (0.25 mL) was allowed to stir for 16 hours. Acetone (0.25 mL) was added, and the mixture was stirred for an additional 4 hours. Another aliquot of AD-mix □ (0.078 g, 0.053 mmol), t-butanol (0.25 mL), and water (0.25 mL) were then added, and the mixture was again allowed to stir for 16 hours. The reaction mixture was quenched with NaHSO₃ (165 mg), stirred for 30 minutes and diluted with water. The mixture was extracted with CH₂Cl₂ (3 × 7 mL), and the combined organic fractions were dried over anhydrous Na₂SO₄ and concentrated under reduced pressure to give a mixture of crude product and starting material. This mixture and AD-mix □ (0.08 g, 0.053 mmol) in *t*-butanol (0.25 mL) and water (0.25 mL) was allowed to stir for 72 hours. Additional AD-mix □ (0.08 g, 0.053 mmol) was added, and the mixture was allowed to stir for 16 hours. Citric acid (5.5 mg, 0.029 mmol) was added, and the mixture was allowed to stir for 16 hours. The mixture was quenched with NaHSO₃ (165 mg) and was stirred for 60 minutes. This mixture was diluted with water (10 mL) and extracted with CH₂Cl₂ (3 × 7 mL). The combined organic fractions were dried over anhydrous Na₂SO₄ and concentrated under reduced pressure. The residue was purified via column chromatography (SiO₂, 1.5% CH₃OH/ CH₂Cl₂) to give the title compound (0.0059 g, 10.8 µmol, 20% yield). ¹H NMR (400 MHz, DMSO-*d*₆) *δ* ppm 7.48 (t, *J =* 8.9 Hz, 2H), 7.31 (s, 2H), 7.05 (dd, *J =* 11.4, 2.9 Hz, 2H), 6.82 (ddd, *J =* 9.0, 2.9, 1.2 Hz, 2H), 5.06 (dd, *J =* 3.7, 1.8 Hz, 2H), 4.47 (d, *J =* 1.5 Hz, 4H), 4.04 (s, 2H), 2.06 - 1.88 (m, 4H), 1.84 - 1.69 (m, 4H); MS (ESI⁺) *m*/*z* 545 (M+H)⁺.

### Example 450: N,N'-[(2S)-2-hydroxybicyclo[2.2.2]octane-1,4-diyl]bis[2-(3-fluorophenoxy)acetamide] (Compound 549)

The reaction described in Example 442 substituting 2-(3-fluorophenoxy)acetic acid for 2-((6-(trifluoromethyl)pyridin-3-yl)oxy)acetic acid gave the title compound. ¹H NMR (400 MHz, DMSO-*d*₆) *δ* ppm 7.47 (s, 1H), 7.38 - 7.27 (m, 2H), 7.24 (s, 1H), 6.84 - 6.73 (m, 6H), 5.08 (s, brd, 1H), 4.44 (s, 2H), 4.41 (s, 2H), 4.02 (dd, J = 9.8, 3.1 Hz, 1H), 2.28 (ddd, J = 12.6, 9.5, 2.4 Hz, 1H), 2.17 - 2.06 (m, 1H), 1.98 - 1.74 (m, 8H); MS (ESI⁺) *m*/*z* 461.0 (M+H)⁺.

### Example 451: N,N'-[(2S)-2-hydroxybicyclo[2.2.2]octane-1,4-diyl]bis[2-(4-fluorophenoxy)acetamide] (Compound 550)

The reaction described in Example 442 substituting 2-(4-fluorophenoxy)acetic acid for 2-((6-(trifluoromethyl)pyridin-3-yl)oxy)acetic acid gave the title compound. ¹H NMR (400 MHz, DMSO-*d*₆) *δ* ppm 7.42 (s, 1H), 7.21 (s, 1H), 7.17 - 7.07 (m, 4H), 7.00 - 6.87 (m, 4H), 5.13 (d, J = 4.5 Hz, 1H), 4.39 (s, 2H), 4.36 (s, 2H), 4.00 (dt, J = 8.7, 3.7 Hz, 1H), 2.28 (ddd, J = 12.4, 9.5, 2.5 Hz, 1H), 2.21 - 2.07 (m, 1H), 1.99 - 1.73 (m, 8H); MS (ESI⁺) *m*/*z* 461.1 (M+H)⁺.

### Example 452: N-[3-(benzylamino)bicyclo[1.1.1]pentan-1-yl]-2-(4-chloro-3-fluorophenoxy)acetamide (Compound 551)

The product of Example 27D (80 mg, 0.28 mmol) was dissolved in *N,N-*dimethylformamide (2 mL) and stirred at ambient temperature. Triethylamine (0.039 mL, 0.28 mmol) was added followed by benzyl bromide (0.033 mL, 0.28 mmol). After 1 hour, CH₃OH (1.0 mL) was added, and the resulting solution was filtered through a glass microfiber frit and purified by preparative HPLC [YMC TriArt^{™} C18 Hybrid 5 µm column, 50 × 100 mm, flow rate 70 mL/minute, 5-100% gradient of acetonitrile in buffer (0.025 M aqueous ammonium bicarbonate, adjusted to pH 10 with ammonium hydroxide)] to give the title compound (48 mg, 0.13 mmol, 46% yield). ¹H NMR (400 MHz, DMSO-*d*₆) *δ* ppm 8.54 (s, 1H), 7.53 - 7.44 (m, 1H), 7.39 - 7.33 (m, 4H), 7.33 - 7.25 (m, 4H), 7.23 - 7.16 (m, 2H), 7.02 (dd, J = 11.4, 2.8 Hz, 1H), 6.80 (ddd, J = 9.0, 2.9, 1.2 Hz, 1H), 4.41 (s, 2H), 3.60 (s, 4H), 1.85 (s, 6H); MS (ESI⁺) *m*/*z* 375 (M+H)⁺.

### Example 453: rac-N,N'-[(1R,2S,4R,5S)-2,5-dihydroxybicyclo[2.2.2]octane-1,4-diyl]bis[2-(4-chloro-3-fluorophenoxy)acetamide] (Compound 552)

### Example 453A: rac-(1R,2S,4R,5S)-1,4-bis(2-(4-chloro-3-fluorophenoxy)acetamido)bicyclo[2.2.2]octane-2,5-diyl bis(4-bromobenzoate)

To a suspension of *N,N'*-(2,5-dioxobicyclo[2.2.2]octane-1,4-diyl)bis[2-(4-chloro-3-fluorophenoxy)acetamide] (130 mg, 0.216 mmol, Example 462) in methanol-dichloromethane (1:1, 4 mL) at 0 °C was added sodium borohydride (16.35 mg, 0.432 mmol). After 60 minutes, the suspension became a clear solution. The solution was treated with brine and saturated aqueous NaHCO₃ and extracted with CH₂Cl₂. The organic phase was washed with brine, dried over Na₂SO₄, and concentrated to give a mixture of three diastereomeric diols (115 mg, 98% yield). To a solution of the mixture of the three diastereomeric diols (115 mg, 0,211 mmol) in CH₂Cl₂ (2 mL) at room temperature was added triethylamine (107 mg, 1.054 mmol) followed by 4-bromobenzoyl chloride (111 mg, 0.506 mmol) and N,N-dimethylpyridin-4-amine (5.15 mg, 0.042 mmol). After the reaction mixture was stirred for 2 hours, it was diluted with ethyl acetate, washed sequentially with 0.5 N HCl, saturated NaHCO₃, and brine, dried over Na₂SO₄, and concentrated. The residue was purified by chromatography on silica gel eluting with 0-100% ethyl acetate in heptane to give the title compound (24 mg, 13% yield) as the first diastereomer to elute followed by the other two diastereomers. MS(APCI) *m*/*z:* 913.0 (M+H)⁺.

### Example 453B: rac-N,N'-[(1R,2S,4R,5S)-2,5-dihydroxybicyclo[2.2.2]octane-1,4-diyl]bis[2-(4-chloro-3-fluorophenoxy)acetamide]

To a solution of rac-(1R,25,4R,55)-1,4-bis(2-(4-chloro-3-fluorophenoxy)acetamido)bicyclo[2.2.2]octane-2,5-diyl bis(4-bromobenzoate) (23 mg, 0.025 mmol, Example 453A) in methanol was added potassium carbonate (7 mg, 0.050 mmol). The reaction mixture was stirred for 2 hours. The solution was diluted with CH₂Cl₂. The organic layer was washed with brine, dried over Na₂SO₄, and concentrated. The residue was purified by chromatography on silica gel eluting with 10-100% ethyl acetate in heptane to give the title compound (5 mg, 36% yield). ¹H NMR (400 MHz, CDCl₃) *δ* ppm 7.33 (t, J = 8.6 Hz, 2H), 6.75 (dd, J = 10.2, 2.9 Hz, 2H), 6.67 (ddd, J = 8.9, 2.8, 1.3 Hz, 2H), 6.52 (s, 2H), 5.50 (d, J = 6.1 Hz, 2H), 4.40 (s, 4H), 4.05 - 3.88 (m, 2H), 2.33 (ddd, J = 14.4, 8.3, 1.3 Hz, 2H), 2.19 (dd, J = 14.4, 2.1 Hz, 2H), 2.10 - 1.98 (m, 2H), 1.92 - 1.79 (m, 2H); MS(APCI) *m*/*z* 545.2 (M+H)⁺

### Example 454: rac-N,N'-[(1R,4R,5S)-2-hydroxy-5-methoxybicyclo[2.2.2]octane-1,4-diyl]bis[2-(4-chloro-3-fluorophenoxy)acetamide] (Compound 553)

To a solution of *rac-N,N*-[(1*R,*2*S,*4*R*)-2-methoxy-5-oxobicyclo[2.2.2]octane-1,4-diyl]bis[2-(4-chloro-3-fluorophenoxy)acetamide] (Example 456, 20 mg, 0.036 mmol) in tetrahydrofuran (2 mL) at -78 °C was added lithium tri-sec-butylhydroborate (1 M, 0.04 mL, 0.04 mmol) dropwise. The reaction mixture was stirred at -78 °C for 2 hours. Additional 1 M lithium tri-sec-butylhydroborate (0.08 mL) was added and stirring was continued at -78 °C for 2 hours. The reaction was quenched with 1 M HCl (0.2 mL), diluted with CH₂Cl₂, washed with saturated NaHCO₃ and brine, dried over Na₂SO₄, and concentrated. The residue was purified by silica gel chromatography eluting with 10-100% ethyl acetate in heptane to give some unreacted starting material and the desired alcohols as a mixture of two diastereomers (10 mg, 50% total yield) with 2.5:1 ratio by ¹HNMR. ¹H NMR (400 MHz, CDCl₃) *δ* ppm 7.39 - 7.28 (m, 2H), 6.80 - 6.70 (m, 2H), 6.66 (m, 2H), 6.61 (s, 0.3H), 6.56 (s, 0.7H), 6.53 (s, 0.3H), 6.48 (s, 0.7H), 4.43 - 4.31 (m, 4H), 4.25 - 4.16 (m, 0.8H), 4.01 (m, 0.3H), 3.86 - 3.70 (m, 1.8H), 3.38 (s, 0.9H), 3.33 (s, .2H), 3.03 (d, 0.3H), 2.63 - 2.23 (m, 4H), 2.08 - 1.89 (m, 2H), 1.86-1.75 (m, 1H), 1.68 - 1.61 (m, 1H); MS(APCI) *m*/*z* 559.2 (M+H)⁺.

### Example 455: rac-N,N'-[(1R,4R,5R)-2-hydroxy-5-methoxybicyclo[2.2.2]octane-1,4-diyl]bis[2-(4-chloro-3-fluorophenoxy)acetamide] (Compound 554)

### Example 455A: rac-(1R,4R,5R)-1,4-bis(2-(4-chloro-3-fluorophenoxy)acetamido)-5-methoxybicyclo[2. 2. 2]octan-2-yl 4-bromobenzoate

To a solution of *rac-N,N*-[(1*R,*2*R,*4*R*)-2-methoxy-5-oxobicyclo[2.2.2]octane-1,4-diyl]bis[2-(4-chloro-3-fluorophenoxy)acetamide] (Example 457, 19 mg, 0.034 mmol) in methanol-dichloromethane (1:1, 2 mL) at ambient temperature was added sodium borohydride (1.548 mg, 0.041 mmol). The reaction mixture was stirred for 2 hours. The solution was treated with brine and saturated aqueous NaHCO₃ and extracted with CH₂Cl₂. The extracts were combined, washed with brine, dried over Na₂SO₄, and concentrated. The residue was purified by silica gel chromatography eluting with 0-100% ethyl acetate-heptane to give a mixture of desired alcohols (two diastereomers) and two other by-products. The mixture was dissolved in CH₂Cl₂ (2 mL) at ambient temperature, triethylamine (16.28 mg, 0.161 mmol) was added to the solution followed by 4-bromobenzoyl chloride (14.12 mg, 0.064 mmol) and *N,N-*dimethylpyridin-4-amine (3.93 mg, 0.032 mmol). The reaction mixture was stirred for 2 hours. It was then concentrated, and the residue was purified by preparative HPLC on a Phenomenex^{®} C8 column (Luna^{®} 5µm AXIA^{™} 150 × 30mm) eluted with 5-100% acetonitrile in water with 0.1% trifluoroacetic acid at a flow rate of 50 mL/minute to give the desired benzoate as a mixture of two diastereomers (2:1 ratio by ¹HNMR).

### Example 455B: rac-N,N'-[(1R,4R,5R)-2-hydroxy-5-methoxybicyclo[2.2.2]octane-1,4-diyl]bis[2-(4-chloro-3-fluorophenoxy)acetamide]

To a solution of *rac*-(1*R*,4*R*,5*R*)-1,4-bis(2-(4-chloro-3-fluorophenoxy)acetamido)-5-methoxybicyclo[2.2.2]octan-2-yl 4-bromobenzoate (example 455A) (5 mg, 6.74 µmol) in CH₃OH was added potassium carbonate (1.8 mg, 0.013 mmol). The reaction mixture was stirred for 2 hours. It was then diluted with CH₂Cl₂, washed with brine, dried over Na₂SO₄, and concentrated. The residue was purified by chromatography on silica gel eluting with 10-100% ethyl acetate in heptane to give the title compound as a mixture of two diastereomers (1.7:1 by ¹HNMR). ¹H NMR (400 MHz, CDCl₃) *δ* ppm 7.38 - 7.28 (m, 2H), 6.90 (s, 0.4H), 6.80 - 6.69 (m, 2H), 6.65 (m, 2H), 6.44 (s, 0.6H), 6.43 (s, 0.6H), 6.41 (s 0.4H), 5.13 (m, 0.4 H), 4.44 - 4.32 (m, 4H), 4.17 - 4.09 (m, 0.7H), 4.07 (d, J = 3.0 Hz, 0.7H), 3.92 - 3.78 (m, 1H), 3.35 (2 s, 3H), 3.08 (dd, J = 14.2, 8.7 Hz, 0.4H), 2.90 (m, 0.4H), 2.61 (m, 0.7H), 2.28 (m, 1.4H), 2.14 (dd, J = 14.1, 2.7 Hz, 0.7H), 2.11 - 2.01 (m, 1H), 1.89 (m, 2H), 1.82 - 1.64 (m, 1.7H), 1.49 (m, 0.4H); MS(APCI) *m*/*z* 559.2 (M+H)⁺.

### Example 456: rac-N,N'-[(1R,2S,4R)-2-methoxy-5-oxobicyclo[2.2.2]octane-1,4-diyl]bis[2-(4-chloro-3-fluorophenoxy)acetamide] (Compound 555)

### Example 456A: diethyl dispiro[1,3-dioxane-2,2'-bicyclo[2.2.2]octane-5',2"-[1,3]dioxane]-1',4'-dicarboxylate

A 25 mL round bottom flask, equipped with a magnetic stir bar, was charged with diethyl 2,5-dioxobicyclo[2.2.2]octane-1,4-dicarboxylate (0.8 g, 2.83 mmol), *p*-toluenesulfonic acid monohydrate (0.054 g, 0.283 mmol), 1,3-propanediol (2.156 g, 28.3 mmol), and toluene (20 mL). The flask was fitted with a Dean-Stark trap and condenser and the reaction mixture was heated at reflux for 10 hours. The reaction mixture was allowed to cool, then partitioned between ethyl acetate and saturated aqueous NaHCO₃. The organic layer was washed with brine, then dried (Na₂SO₄), and filtered. The filtrate was concentrated under reduced pressure to give the title compound (0.91 g, 81% yield), which was used in the next step without further purification. ¹H NMR (400 MHz, CDCl₃) *δ* ppm 4.31 - 4.12 (m, 4H), 4.03 (td, J = 12.2, 2.7 Hz, 2H), 3.99 - 3.86 (m, 4H), 3.80 (ddt, J = 11.6, 4.8, 1.7 Hz, 2H), 2.36 - 2.17 (m, 6H), 1.92 (qt, J = 12.6, 5.1 Hz, 2H), 1.53 - 1.42 (m, 2H), 1.30 (q, J = 7.8, 7.1 Hz, 8H).

### Example 456B: diethyl 5-oxospiro[bicyclo[2.2.2]octane-2,2'-[1,3]dioxane]-1,4-dicarboxylate

A mixture of the bis-ketal prepared above (Example 456A, 200 mg, 0.502 mmol) and p-toluenesulfonic acid monohydrate (9.55 mg, 0.050 mmol) in acetone (3 mL) was heated at 50 °C for 1 hour. The reaction was stopped by adding 25 mg of Na₂CO₃, and then the mixture was partitioned between ethyl acetate and saturated aqueous NaHCO₃. The organic layer was washed with brine, then dried (Na₂SO₄), and filtered. The filtrate was concentrated under reduced pressure to give the title compound (170 mg, 100%). ¹H NMR (400 MHz, CDCl₃) *δ* ppm 4.34 - 4.13 (m, 4H), 3.95 (dtd, J = 9.5, 7.6. 2.6 Hz, 3H), 3.86 (ddt, J = 11.8, 5.5, 1.4 Hz, 1H), 3.11 (dd, J = 19.1, 3.1 Hz, 1H), 2.66 - 2.48 (m, 2H), 2.38 - 2.18 (m, 3H), 2.05 - 1.87 (m, 2H), 1.65 (ddd, J = 13.7, 11.4, 5.5 Hz, 1H), 1.40 - 1.32 (m, 1H), 1.29 (td, J = 7.1, 4.5 Hz, 6H).

### Example 456C: diethyl rac-(1R,4R,5S)-5-hydroxyspiro[bicyclo[2.2.2]octane-2,2'-[1,3]dioxane]-1,4-dicarboxylate

To a solution of diethyl 5-oxospiro[bicyclo[2.2.2]octane-2,2'-[1,3]dioxane]-1,4-dicarboxylate (610 mg, 1.792 mmol, Example 456A) in methanol-dichloromethane (1:1, 10 mL) was added sodium borohydride (50.9 mg, 1.344 mmol). The reaction mixture was stirred for 3 hours at 0 °C. The solution was treated with saturated aqueous NaHCO₃ and extracted with CH₂Cl₂. The organic layer was concentrated, and the residue was purified by chromatography on silica gel eluting with 10-100% ethyl acetate in heptane to give, 270 mg (44% yield) of the title compound (*trans*-isomer) and its diastereomer (cis-isomer, first eluted) (136 mg, 22% yield). ¹H NMR (501 MHz, CDCl₃) *δ* ppm 4.29 - 4.15 (m, 4H), 4.06 (dtd, J = 18.9, 12.1, 2.6 Hz, 2H), 3.97 - 3.83 (m, 2H), 2.71 (d, J = 7.2 Hz, 1H), 2.62 (dd, J = 13.9, 2.6 Hz, 1H), 2.32 - 2.20 (m, 2H), 2.11 (dd, J = 14.6, 9.4 Hz, 1H), 2.04 (dd, J = 13.8, 1.4 Hz, 1H), 2.02 - 1.92 (m, 1H), 1.79 (ddd, J = 12.9, 10.7, 3.9 Hz, 1H), 1.66 (tdd, J = 13.2, 5.6, 2.6 Hz, 1H), 1.55 (ddd, J = 13.2, 10.8, 3.9 Hz, 1H), 1.38 (ddt, J = 13.3, 2.7, 1.1 Hz, 1H), 1.29 (td, J = 7.1, 3.2 Hz, 6H). *cis* diastereomer of 456C: ¹H NMR (501 MHz, CDCl₃) *δ* ppm 4.27 - 4.09 (m, 5H), 4.04 - 3.77 (m, 4H), 3.04 - 2.95 (m, 1H), 2.64 (ddd, J = 14.5, 9.3, 3.1 Hz, 1H), 2.34 (d, J = 13.5 Hz, 1H), 2.32 - 2.25 (m, 1H), 2.13 - 2.04 (m, 1H), 2.01 - 1.88 (m, 1H), 1.81 (dd, J = 13.5, 2.7 Hz, 1H), 1.74 - 1.62 (m, 2H), 1.51 (dd, J = 14.4, 2.7 Hz, 1H), 1.36 - 1.31 (m, 1H), 1.31 - 1.23 (m, 6H).

### Example 456D: diethyl rac-(1R,4R,5S)-5-methoxyspiro[bicyclo[2.2.2]octane-2,2'-[1,3]dioxane]-1,4-dicarboxylate

To a solution of diethyl *rac*-(1*R*,4*R*,5*S*)-5-hydroxyspiro[bicyclo[2.2.2]octane-2,2'-[1,3]dioxane]-1,4-dicarboxylate (Example 456C, 180 mg, 0.526 mmol) in tetrahydrofuran (3 mL) was added sodium hydride (27.3 mg, 0.683 mmol) at 0 °C. After 30 minutes at ambient temperature, iodomethane (0.098 mL, 1.577 mmol) was added, and the reaction mixture was stirred for 2 hours at ambient temperature. The solution was treated with saturated aqueous NaHCO₃ and extracted with ethyl acetate. The organic layer was washed with brine, dried over Na₂SO₄, and concentrated to give the title compound. ¹H NMR (400 MHz, CDCl₃) *δ* ppm 4.17 - 3.91 (m, 5H), 3.86 (td, J = 12.2, 2.5 Hz, 1H), 3.73 (ddd, J = 24.2, 11.8, 5.1 Hz, 2H), 3.58 (dd, J = 9.1, 5.2 Hz, 1H), 3.18 (s, 3H), 2.25 - 2.14 (m, 3H), 2.04 (ddd, J = 14.1, 5.2, 2.8 Hz, 1H), 1.97 (dd, J = 14.0, 9.0 Hz, 1H), 1.89 - 1.75 (m, 1H), 1.70 (t, J = 12.1 Hz, 1H), 1.55 (ddd, J = 12.9, 11.2, 6.8 Hz, 1H), 1.34 - 1.23 (m, 1H), 1.21 (s, 1H), 1.15 (q, J = 7.0 Hz, 6H).

### Example 456E: rac-(1R,4R,5S)-1-(ethoxycarbonyl)-5-methoxyspiro[bicyclo[2.2.2]octane-2,2'-[1,3]dioxane]-4-carboxylic acid

To a solution of diethyl rac-(1R,4R,5S)-5-methoxyspiro[bicyclo[2.2.2]octane-2,2'-[1,3]dioxane]-1,4-dicarboxylate (145 mg, 0.407 mmol, Example 456D) in ethanol (3 mL) was added NaOH (2 M) (65.1 mg, 1.627 mmol) and the reaction mixture was stirred at 50 °C for 2 hours and then heated at reflux for 2 hours. The reaction mixture was concentrated, and the residue was dissolved in water (5 mL), acidified with 2 N HCl to pH1-2, and extracted with ethyl acetate. The organic layer was washed with brine, dried over Na₂SO₄, and concentrated to give the title compound. ¹H NMR (400 MHz, DMSO-*d*₆) *δ* ppm 12.18 (s, 1H), 4.14 - 4.00 (m, 2H), 3.95 (td, J = 11.7, 2.4 Hz, 1H), 3.89 - 3.65 (m, 3H), 3.62 (ddd, J = 9.1, 5.1, 1.2 Hz, 1H), 3.18 (d, J = 1.8 Hz, 3H), 2.21 - 1.83 (m, 5H), 1.68 (tdd, J = 12.4, 5.5, 2.9 Hz, 2H), 1.55 - 1.43 (m, 1H), 1.40 - 1.27 (m, 2H), 1.16 (t, J = 7.1 Hz, 3H).

### Example 456F: rac-(1R,4R,5S)-5-methoxyspiro[bicyclo[2.2.2]octane-2, 2'-[1,3]dioxane]-1,4-dicarboxylic acid

To a solution of *rac*-(1*R*,4*R*,5*S*)-1-(ethoxycarbonyl)-5-methoxyspiro[bicyclo[2.2.2]octane-2,2'-[1,3]dioxane]-4-carboxylic acid (134 mg, 0.408 mmol, Example 456E) in ethanol (3 mL) was added NaOH (2 M) (82 mg, 2.040 mmol), and the reaction mixture was heated at reflux for 10 hours. The solution was concentrated in vacuo. The resulting residue was dissolved in water, acidified with 2 N HCl to pH1-2, extracted with ethyl acetate, washed with brine, dried over Na₂SO₄, and concentrated in vacuo to give the title compound. ¹H NMR (400 MHz, DMSO-*d*₆) *δ* ppm 11.97 (s, 2H), 4.01 (qd, J = 13.3, 12.3, 4.8 Hz, 1H), 3.93 - 3.69 (m, 3H), 3.62 (dd, J = 9.3, 4.7 Hz, 1H), 3.18 (s, 3H), 2.18 - 1.96 (m, 4H), 1.86 (ddd, J = 13.9, 4.8, 2.8 Hz, 1H), 1.82 - 1.60 (m, 2H), 1.48 (ddd, J = 12.6, 11.1, 6.5 Hz, 1H), 1.42 - 1.28 (m, 2H).

### Example 456G: rac-(1R,4R,5S)-1,4-diamino-5-methoxybicyclo[2.2.2]octan-2-one bis-hydrochloride

To a suspension of *rac*-(1*R*,4*R*,5*S*)-5-methoxyspiro[bicyclo[2.2.2]octane-2,2'-[1,3]dioxane]-1,4-dicarboxylic acid (120 mg, 0.400 mmol, Example 456F) in toluene (2 mL) was added 2,4,6-tripropyl-1,3,5,2,4,6-trioxatriphosphinane 2,4,6-trioxide (0.595 mL, 0.999 mmol) followed by triethylamine (0.251 mL, 1.798 mmol) and azidotrimethylsilane (0.131 mL, 0.999 mmol). The reaction was stirred at ambient temperature for 120 minutes, and then the mixture was concentrated in vacuo at 23 °C. The mixture was purified by chromatography on silica gel eluting with 0-50% ethyl acetate in heptane to give *rac*-(1*R,*4*R,*5*S*)-5-methoxyspiro[bicyclo[2.2.2]octane-2,2'-[1,3]dioxane]-1,4-dicarbonyl diazide (70 mg, 50% yield). A suspension of *rac*-(1*R*,4*R*,5*S*)-5-methoxyspiro[bicyclo[2.2.2]octane-2,2'-[1,3]dioxane]-1,4-dicarbonyl diazide (68 mg, 0.194 mmol), in toluene (3 mL) was heated at 90 °C for 2 hours. Concentrated hydrogen chloride (0.5 mL, 3.00 mmol) was added, and then the mixture was heated at 90 °C for 2 hours. The solution was evaporated to give the title compound (35 mg, 98% yield) which was used in the next step without further purification.

### Example 456H: rac-N,N'-[(1R,2S,4R)-2-methoxy-5-oxobicyclo[2.2.2]octane-1,4-diyl]bis[2-(4-chloro-3-fluorophenoxy)acetamide]

A mixture of 2-(4-chloro-3-fluorophenoxy)acetic acid (98 mg, 0.480 mmol), *rac-*(1*R*,4*R*,5*S*)-1,4-diamino-5-methoxybicyclo[2.2.2]octan-2-one bis-hydrochloride (34 mg, 0.185 mmol, Example 456G), *N*-ethyl-*N*-isopropylpropan-2-amine (0.193 mL, 1.107 mmol) in dimethylformamide (2 mL) was treated with 2-(3*H*-[1,2,3]triazolo[4,5-*b*]pyridin-3-yl)-1,1,3,3-tetramethylisouronium hexafluorophosphate(V) (281 mg, 0.738 mmol), and the reaction was stirred at ambient temperature for 6 hours. The reaction mixture was diluted with ethyl acetate, washed with water and brine, and dried over Na₂SO₄. Solvent was removed. and the residue was purified by chromatography on silica gel eluting with 30--60% ethyl acetate in heptane to give the title compound (28 mg, 27% yield). ¹H NMR (400 MHz, CDCl₃) *δ* ppm 7.74 (s, 1H), 7.33 (td, J = 8.6, 4.4 Hz, 2H), 6.86 (s, 1H), 6.82 - 6.61 (m, 4H), 4.47 - 4.34 (m, 4H), 3.75 (ddd, J = 9.0, 3.1, 1.5 Hz, 1H), 3.45 (dd, J = 14.0, 9.1 Hz, 1H), 3.33 (s, 3H), 3.24 (dd, J = 19.0, 1.5 Hz, 1H), 3.03 - 2.85 (m, 2H), 2.75 (dd, J = 19.1, 2.9 Hz, 1H), 1.90 (ddt, J = 16.2, 11.8, 3.2 Hz, 1H), 1.66 - 1.58 (m, 2H); MS(APCI) *m*/*z* 557.2 (M+H)⁺.

### Example 457: rac-N,N'-[(1R,2R,4R)-2-methoxy-5-oxobicyclo[2.2.2]octane-1,4-diyl]bis[2-(4-chloro-3-fluorophenoxy)acetamide] (Compound 556)

### Example 457A: rac-(1R,4R,5R)-diethyl 5-hydroxyspiro[bicyclo[2.2.2]octane-2,2'-[1,3]dioxane]-1,4-dicarboxylate

The title compound was prepared and isolated as the *cis* diastereomer of Example 456C.

### Example 457B: rac-(1R,4R,5R)-diethyl 5-methoxyspiro[bicyclo[2.2.2]octane-2,2'-[1,3]dioxane]-1,4-dicarboxylate

To a solution of rac-(1R,4R,5R)-diethyl 5-hydroxyspiro[bicyclo[2.2.2]octane-2,2'-[1,3]dioxane]-1,4-dicarboxylate (130 mg, 0.380 mmol, Example 457A) in tetrahydrofuran (5 mL) was added sodium hydride (19.74 mg, 0.494 mmol). After 30 minutes, iodomethane (0.071 mL, 1.139 mmol) was added, and the reaction mixture was stirred for 3 hours at ambient temperature. The solution was treated with saturated aqueous NaHCO₃ and extracted with ethyl acetate. The organic fraction was washed with brine, dried over Na₂SO₄, and concentrated. The residue was purified by chromatography eluting with 10-70% ethyl acetate in heptane to give 70 mg (52% yield) of the title compound. ¹H NMR (400 MHz, CDCl₃) *δ* ppm 4.11 - 3.94 (m, 4H), 3.83 - 3.58 (m, 5H), 3.13 (d, J = 0.7 Hz, 3H), 2.47 (ddd, J = 14.0, 8.8, 3.0 Hz, 1H), 2.15 - 2.04 (m, 1H), 1.99 - 1.71 (m, 4H), 1.61 - 1.41 (m, 2H), 1.37 (dd, J = 14.1, 3.1 Hz, 1H), 1.20 - 1.15 (m, 1H), 1.12 (t, J = 7.2 Hz, 6H); MS(APCI) *m*/*z* 357.1 (M+H)⁺.

### Example 457C: rac-(1R,4R,5R)-1,4-diamino-5-methoxybicyclo[2.2.2]octan-2-one

To a solution of rac-(1R,4R,5R)-diethyl 5-methoxyspiro[bicyclo[2.2.2]octane-2,2'-[1,3]dioxane]-1,4-dicarboxylate (67 mg, 0.188 mmol, Example 457B) in hydrochloric acid (313 µL, 1.880 mmol) was heated at 100 °C for 3 hours. After cooling, the reaction mixture was dissolved in CH₃CN, and concentrated in vacuo to give the title compound (45 mg, 99%). ¹H NMR (501 MHz, DMSO-*d*₆) *δ* ppm 3.88 (ddd, J = 9.0, 3.0, 1.5 Hz, 1H), 3.24 (s, 3H), 3.23 - 3.16 (m, 1H), 2.49 (d, J = 2.8 Hz, 1H), 2.46 (dd, J = 10.3, 2.9 Hz, 1H), 2.39 (d, J = 18.5 Hz, 1H), 2.12 (dddd, J = 13.0, 11.2, 4.2, 2.1 Hz, 1H), 2.08 - 2.01 (m, 1H), 1.85 (ddd, J = 13.7, 11.4, 5.6 Hz, 1H), 1.74 (dd, J = 14.4, 3.0 Hz, 1H), 1.72 - 1.63 (m, 1H).

### Example 457D: rac-N,N'-[(1R,2R,4R)-2-methoxy-5-oxobicyclo[2.2.2]octane-1,4-diyl]bis[2-(4-chloro-3-fluorophenoxy)acetamide]

The title compound was prepared in 48% yield from the material prepared in Example 457C using the procedure described in Example 456H. ¹H NMR (501 MHz, DMSO-d₆) *δ* ppm 7.81 (s, 1H), 7.73 (s, 1H), 7.49 (td, J = 8.9, 1.8 Hz, 2H), 7.05 (ddd, J = 28.6, 11.4, 2.9 Hz, 2H), 6.84 (dddd, J = 15.6, 9.0, 2.9, 1.2 Hz, 2H), 4.57 (s, 2H), 4.54 (s, 2H), 4.04 (dt, J = 8.4, 2.2 Hz, 1H), 3.28 (s, 3H), 3.07 (dd, J = 18.8, 2.9 Hz, 1H), 2.76 (d, J = 18.8 Hz, 1H), 2.44 (td, J = 12.0, 5.3 Hz, 1H), 2.34 (dd, J = 14.1, 2.8 Hz, 1H), 2.24 (ddd, J = 13.8, 9.0, 2.9 Hz, 1H), 2.19 - 2.08 (m, 1H), 1.86 (td, J = 12.2, 5.3 Hz, 1H), 1.71 (t, J = 12.4 Hz, 1H); MS(APCI) *m*/*z* 557.2 (M+H)⁺.

### Example 458: rac-N,N'-[(2R,5S)-2,5-dihydroxybicyclo[2.2.2]octane-1,4-diyl]bis[2-(4-chloro-3-fluorophenoxy)acetamide] (Compound 557)

To a solution of *N,N'*-(2,5-dioxobicyclo[2.2.2]octane-1,4-diyl)bis[2-(4-chloro-3-fluorophenoxy)acetamide] (44 mg, 0.081 mmol, Example 462) in CH₃OH-CH₂Cl₂ (2 mL) at -10°C was added sodium borohydride (6.1 mg, 0.163 mmol). The reaction mixture was stirred for 2 hours. The solution was quenched with brine and saturated aqueous NaHCO₃ and extracted with CH₂Cl₂. The organic fraction was washed with brine, dried over Na₂SO₄, and concentrated. The residue was purified by chromatography eluting with 0-5% CH₃OH in CH₂Cl₂ to give the title compound (third eluted diastereomer) along with the other two diastereomers: *rac-N,N'-*[(1*R,*2*S,*4*R,*5S)*-*2,5-dihydroxybicyclo[2.2.2]octane-1,4-diyl]bis[2-(4-chloro-3-fluorophenoxy)acetamide] (first eluted diastereomer) and *rac-N,N'*-[(1*S,*2*S*,4*S*,5*S*)-2,5-dihydroxybicyclo[2.2.2]octane-1 ,4-diyl]bis[2-(4-chloro-3-fluorophenoxy)acetamide] (second eluted diastereomer). ¹H NMR (400 MHz, DMSO-*d*₆) *δ* ppm 7.48 (t, J = 8.9 Hz, 2H), 7.26 (s, 2H), 7.05 (dd, J = 11.4, 2.9 Hz, 2H), 6.83 (ddd, J = 8.9, 2.8, 1.2 Hz, 2H), 5.02 (dd, J = 4.5, 2.9 Hz, 2H), 4.46 (s, 4H), 4.14 - 4.03 (m, 1H), 4.02 - 3.93 (m, 1H), 2.41 (dd, J = 13.7, 9.4 Hz, 1H), 2.28 - 2.17 (m, 1H), 2.15 - 2.04 (m, 1H), 1.89 (t, J = 12.4 Hz, 1H), 1.81 - 1.68 (m, 3H), 1.64 (dt, J = 13.2, 6.6 Hz, 1H); MS(APCI) *m*/*z* 545.2 (M+H)⁺

### Example 459: (2S)-1,4-bis[2-(4-chloro-3-fluorophenoxy)acetamido]bicyclo[2.2.2]octane-2-carboxylic acid (Compound 558)

A solution of Example 463 (0.03 g, 0.053 mmol) in tetrahydrofuran (1 mL) was treated with 1 N lithium hydroxide (0.158 mL, 0.158 mmol) and stirred at room temperature for 2 hours. The reaction was concentrated, and the residue was purified by preparative HPLC (Phenomenex^{®} Luna^{®} C18(2) 5 µm 100 Å AXIA^{™} column 250 mm × 21.2 mm, flow rate 25 mL/minute, 10-90% gradient of acetonitrile in buffer (0.1% trifluoroacetic acid in water)) to provide the title compound (18 mg, 62% yield). ¹H NMR (400 MHz, methanol-*d*₄) *δ* ppm 1.97-2.15 (m, 7H), 2.21-2.31 (m, 1H), 2.35-2.47 (m, 2H), 3.48 (br dd, J=11.00, 5.62 Hz, 1H), 4.35-4.50 (m, 4H), 6.79 (br d, J=9.29 Hz, 2H), 6.90 (dt, J=11.25, 2.20 Hz, 2H), 7.36 (td, J=8.68, 1.71 Hz, 2H), 7.46 (s, 1H); MS (APCI) *m*/*z* 558 (M+H)⁺.

### Example 460: (2R)-1,4-bis[2-(4-chloro-3-fluorophenoxy)acetamido]bicyclo[2.2.2]octane-2-carboxylic acid (Compound 559)

A solution of Example 464 (0.08 g, 0.140 mmol) in tetrahydrofuran (1 mL) was treated with 1 N lithium hydroxide (0.420 mL, 0.420 mmol) and stirred at room temperature for 2 hours. The reaction mixture was concentrated, and the residue was purified by preparative HPLC (Phenomenex^{®} Luna^{®} C18(2) 5 µm 100 Å AXIA^{™} column 250 mm × 21.2 mm, flow rate 25 mL/minute, 10-90% gradient of acetonitrile in buffer (0.1% trifluoroacetic acid in water)) to provide the title compound (50 mg, 64% yield). ¹H NMR (400 MHz, methanol-*d*₄) *δ* ppm 1.97-2.15 (m, 7H), 2.21-2.31 (m, 1H), 2.35-2.47 (m, 2H), 3.48 (br dd, J=11.00, 5.62 Hz, 1H), 4.35-4.50 (m, 4H), 6.79 (br d, J=9.29 Hz, 2H), 6.90 (dt, J=11.25, 2.20 Hz, 2H), 7.36 (td, J=8.68, 1.71 Hz, 2H), 7.46 (s, 1H); MS (APCI) *m*/*z* 558 (M+H)⁺.

### Example 461: rac-N,N'-[(1S,2S,4S,5S)-2,5-dihydroxybicyclo[2.2.2]octane-1,4-diyl]bis[2-(4-chloro-3-fluorophenoxy)acetamide] (Compound 560)

To a solution of N,N'-(2,5-dioxobicyclo[2.2.2]octane-1,4-diyl)bis(2-(4-chloro-3-fluorophenoxy)acetamide) (44 mg, 0.081 mmol, Example 462) in CH₃OH-CH₂Cl₂ (2 mL) at -10°C was added sodium borohydride (6.1 mg, 0.163 mmol). The reaction mixture was stirred for 2 hours. The solution was quenched with brine and saturated aqueous NaHCO₃ and extracted with CH₂Cl₂. The organic fraction was washed with brine, dried over Na₂SO₄, and concentrated. The residue was purified by chromatography eluting with 0-5% CH₃OH in CH₂Cl₂ to give the title compound (send eluted diastereomer) along with the other two diastereomers: *rac-N,N'-*[(1*R*,2*S*,4*R*,5*S*)-2,5-dihydroxybicyclo[2.2.2]octane-1,4-diyl]bis[2-(4-chloro-3-fluorophenoxy)acetamide] and *rac-N,N'-*[(2*R,*5*S*)-2,5-dihydroxybicyclo[2.2.2]octane-1,4-diyl]bis[2-(4-chloro-3-fluorophenoxy)acetamide]. ¹H NMR (400 MHz, DMSO-*d*₆) *δ* ppm 7.48 (t, J = 8.9 Hz, 2H), 7.26 (s, 2H), 7.05 (dd, J = 11.4, 2.9 Hz, 2H), 6.82 (ddd, J = 9.0, 2.9, 1.2 Hz, 2H), 5.11 (d, J = 4.2 Hz, 2H), 4.47 (s, 4H), 3.97 - 3.89 (m, 2H), 3.17 (d, J = 5.2 Hz, 0H), 2.28 - 2.17 (m, 2H), 1.93 (dd, J = 13.7, 3.3 Hz, 2H), 1.90 - 1.75 (m, 4H); MS(APCI) m/z 545.2 (M+H)⁺.

### Example 462: N,N'-(2,5-dioxobicyclo[2.2.2]octane-1,4-diyl)bis[2-(4-chloro-3-fluorophenoxy)acetamide] (Compound 561)

### Example 462A: 2,5-dioxobicyclo[2.2.2]octane-1,4-dicarboxylic acid

A solution of diethyl 2,5-dioxobicyclo[2.2.2]octane-1,4-dicarboxylate (2 g, 7.08 mmol) in hydrochloric acid (9.45 mL, 56.7 mmol) was heated at 100 °C for 4 hours. After cooling, the suspension was dissolved in CH₃OH and concentrated to give the title compound (1.6 g, 100% yield). ¹H NMR (501 MHz, DMSO-*d*₆) *δ* ppm 12.96 (s, 2H), 2.93 (dd, J = 19.2, 2.3 Hz, 2H), 2.80 (d, J = 19.1 Hz, 2H), 2.20 (pd, J = 8.1, 2.7 Hz, 2H), 2.15 - 2.03 (m, 2H).

### Example 462B: 2,5-dioxobicyclo[2.2.2]octane-1,4-dicarbonyl azide

To a suspension of 2,5-dioxobicyclo[2.2.2]octane-1,4-dicarboxylic acid (810 mg, 3.58 mmol) in toluene (18 mL) was added 2,4,6-tripropyl-1,3,5,2,4,6-trioxatriphosphinane 2,4,6-trioxide (5.33 mL, 8.95 mmol) followed by triethylamine (2.246 mL, 16.12 mmol) and azidotrimethylsilane (1.177 mL, 8.95 mmol). The reaction mixture was stirred at ambient temperature for 2 hours, and then the reaction mixture was concentrated under reduced pressure. The residue was purified by column chromatography on silica gel column eluted with 0-50% ethyl acetate in heptane to give the title compound (410 mg, 41% yield).

### Example 462C: 1,4-diaminobicyclo[2.2.2]octane-2,5-dione bis-hydrochloride

A suspension of 2,5-dioxobicyclo[2.2.2]octane-1,4-dicarbonyl azide (410 mg, 1.484 mmol) in toluene (12 mL) was heated at 90 °C for 2 hours. Concentrated hydrogen chloride (3 mL, 18.00 mmol) was added, and then the mixture was heated at 90 °C for another 2 hours. The solution was concentrated under reduced pressure to give the title compound (358 mg, 100% yield) which was used in the next step without further purification.

### Example 462D: N,N'-(2,5-dioxobicyclo[2.2.2]octane-1,4-diyl)bis[2-(4-chloro-3-fluorophenoxy)acetamide]

A mixture of 2-(4-chloro-3-fluorophenoxy)acetic acid (150 mg, 0.733 mmol), 1,4-diaminobicyclo[2.2.2]octane-2,5-dione bis-hydrochloride (350 mg, 1.452 mmol), N-ethyl-N-isopropylpropan-2-amine (1.521 mL, 8.71 mmol) in N,N-dimethlformamide (2 mL) was treated with 2-(3*H*-[1,2,3]triazolo[4,5-*b*]pyridin-3-yl)-1,1,3,3-tetramethylisouronium hexafluorophosphate(V) (1.65 g, 4.35 mmol), and the reaction was stirred at ambient temperature for 12 hours. The reaction mixture was diluted with ethyl acetate, washed with water and brine, and dried over Na₂SO₄. Volatiles were removed, and the residue was triturated with CH₃CN to give the titled compound (330 mg). The mother liquor was concentrated and triturated again with CH₃CN to give a second batch of the title compound (130 mg) (total 460 mg, 59% yield). ¹H NMR (400 MHz, CDCl₃) *δ* ppm 7.78 (s, 2H), 7.34 (t, J = 8.6 Hz, 2H), 6.79 (dd, J = 10.2, 2.8 Hz, 2H), 6.72 (ddd, J = 8.8, 2.9, 1.2 Hz, 2H), 4.44 (s, 4H), 4.06 (d, J = 19.7 Hz, 2H), 3.25 - 3.02 (m, 2H), 2.52 (dd, J = 19.6, 2.5 Hz, 2H), 1.98 - 1.75 (m, 2H); MS(APCI) *m*/*z* 541.1 (M+H)⁺.

### Example 463: methyl (2S)-1,4-bis[2-(4-chloro-3-fluorophenoxy)acetamido]bicyclo[2.2.2]octane-2-carboxylate (Compound 562)

Example 470 (200 mg, 0.35 mmol) was subjected to chiral separation by supercritical fluid chromatography (SFC) (ChiralPak^{®} IB, 21 x 250 mm, 5 micron, 20% methanol:CO₂ at 70 g/minute, 100 bar backpressure) to provide the title compound (45 mg, 45% yield). ¹H NMR (400 MHz, DMSO-*d*₆) *δ* ppm 7.57 (d, J = 9.4 Hz, 2H), 7.48 (td, J = 8.9, 1.2 Hz, 2H), 7.01 (td, J = 11.3, 2.9 Hz, 2H), 6.86 - 6.75 (m, 2H), 4.49 - 4.37 (m, 4H), 3.56 (s, 3H), 3.54 - 3.50 (m, 1H), 2.28 - 1.76 (m, 8H), 1.76 - 1.55 (m, 2H); MS (APCI) *m*/*z* 572 (M+H)⁺.

### Example 464: methyl (2R)-1,4-bis[2-(4-chloro-3-fluorophenoxy)acetamido]bicyclo[2.2.2]octane-2-carboxylate(Compound 563)

Example 470 (200 mg, 0.35 mmol) was subjected to chiral separation by supercritical fluid chromatography (SFC) ( ChiralPak IB, 21 x 250 mm, 5 micron, 20% methanol:CO₂ at 70 g/minute, 100 bar backpressure) to provide the title compound (100 mg, 100% yield). ¹H NMR (400 MHz, DMSO-*d*₆) *δ* ppm 7.57 (d, J = 9.4 Hz, 2H), 7.48 (td, J = 8.9, 1.2 Hz, 2H), 7.01 (td, J = 11.3, 2.9 Hz, 2H), 6.86 - 6.75 (m, 2H), 4.49 - 4.37 (m, 4H), 3.56 (s, 3H), 3.54 - 3.50 (m, 1H), 2.28 - 1.76 (m, 8H), 1.76 - 1.55 (m, 2H). MS (APCI) *m*/*z* 572 (M+H)⁺.

### Example 465

### 2-(4-chloro-3-fluorophenoxy)-N-{4-[(4-chlorophenyl)methoxy]-2-oxobicyclo[2.2.2]octan-1-yl}acetamide (Compound 564)

### Example 465A: ethyl 2,4-bis((tert-butyldimethylsilyl)oxy)bicyclo[2.2.2]oct-2-ene-1-carboxylate

To a solution of Example 512A (0.514 g, 2.422 mmol) and triethylamine (1.013 mL, 7.27 mmol) in CH₂Cl₂ (6 mL) was added tert-butyldimethylsilyl trifluoromethanesulfonate (1.669 mL, 7.27 mmol) dropwise at 0 °C. The mixture was stirred at 0 °C for 1 hour and at room temperature for 1.5 hours. The reaction was quenched with water and extracted with CH₂Cl₂ (2×). The combined organic layers were dried over MgSO₄, filtered, and concentrated. The residue was purified on a 25 g silica gel column using the Biotage^{®} Isolera^{™} One flash system eluting with heptanes/ethyl acetate (95:5 to 9:1) to provide the title compound (0.84 g, 83%); MS (ESI⁺) *m*/*z* 441.0 (M+H)⁺.

### Example 465B: 4-((tert-butyldimethylsilyl)oxy)-2-oxobicyclo[2.2.2]octane-1-carboxylic acid

To a solution of Example 465A (0.880 g, 1.997 mmol) in methanol (6 mL) and tetrahydrofuran (8 mL) was added a solution of lithium hydroxide (0.143 g, 5.99 mmol) in water (4 mL). The mixture was stirred for 7 hours. The reaction mixture was concentrated. The remaining residue was diluted with 15 mL of water and treated with 5% citric acid until pH=3. The precipitate was collected by filtration, washed with water, and vacuum oven-dried to provide the title compound (0.450 g, 76%); MS (ESI⁺) *m*/*z* 299.1 (M+H)⁺.

### Example 465C: 4-((tert-butyldimethylsilyl)oxy)-1-isocyanatobicyclo[2.2.2]octan-2-one

To a mixture of Example 465B in tetrahydrofuran (6 mL) was added triethylamine (0.308 mL, 2.211 mmol) followed by 2,4,6-tripropyl-1,3,5,2,4,6-trioxatriphosphorinane-2,4,6-trioxide (0.675 mL, 1.156 mmol) (50 weight% in ethyl acetate) and azidotrimethylsilane (0.153 mL, 1.156 mmol). The mixture was stirred for 3 hrs. The mixture was quenched with brine and extracted with ethyl acetate (2×). The combined organic layers were dried over MgSO₄, filtered, and concentrated. The residue was purified on a 24 g silica gel column using the Biotage^{®} Isolera^{™} One flash system eluting with heptanes/ethyl acetate (9:1) to provide the title compound (0.235 g, 79%); MS (ESI⁺) *m*/*z* 313.1 [M+NH₄]⁺.

### Example 465D: 1-amino-4-hydroxybicyclo[2.2.2]octan-2-one hydrochloric acid

A mixture of Example 465C (0.206 g, 0.697 mmol) and 1 N HCl (3.49 mL, 3.49 mmol) in tetrahydrofuran (4 mL) was heated at 65 °C for 6 hours. The reaction mixture was concentrated to provide the title compound as an HCl salt that was used in the next step without further purifications. MS (APCI⁺) *m*/*z* 156.1 (M+H)⁺.

### Example 465E: 2-(4-chloro-3-fluorophenoxy)-N-(4-hydroxy-2-oxobicyclo[2.2.2]octan-1-yl)acetamide

To a mixture of Example 465D (0.134 g, 0.697 mmol) in *N,N*-dimethylformamide (4 mL) was added triethylamine (0.874 mL, 6.27 mmol), 2-(4-chloro-3-fluorophenoxy)acetic acid (0.157 g, 0.767 mmol) and 1-[bis(dimethylamino)methylene]-1*H*-1,2,3-triazolo[4,5-*b*]pyridinium 3-oxid hexafluorophosphate (HATU, 0.398 g, 1.046 mmol). The mixture was stirred overnight. The reaction mixture was quenched with brine and extracted with ethyl acetate (2×). The combined organic layers were washed with brine, dried over MgSO₄, filtered, and concentrated. The residue was purified on a 12 g silica gel column using the Biotage^{®} Isolera^{™} One flash system eluting with heptanes/ethyl acetate (1:9) to 100% ethyl acetate to provide the title compound (0.177g, 74%). MS (ESI⁺) *m*/*z* 342.1 (M+H)⁺.

### Example 465F: 2-(4-chloro-3-fluorophenoxy)-N-{4-[(4-chlorophenyl)methoxy]-2-oxobicyclo[2.2.2]octan-1-yl}acetamide

To a mixture of Example 465E (65.0 mg, 0.190 mmol), 2,6-di-tert-butylpyridine (0.103 mL, 0.475 mmol), and silver trifluoromethanesulfonate (98 mg, 0.380 mmol) in CH₂Cl₂ (4 mL) was added 1-(bromomethyl)-4-chlorobenzene (78 mg, 0.380 mmol). The reaction mixture was stirred for 4 hours. The reaction mixture was filtered and concentrated. The residue was purified on a silica column (12 g) using the Biotage^{®} Isolera^{™} One flash system eluting with heptane/ethyl acetate (8:2 to 6:4) to provide the title compound (59.3 mg, 67%). ¹H NMR (400 MHz, DMSO-*d*₆) *δ* ppm 7.67 (s, 1H), 7.46 (t, J = 8.9 Hz, 1H), 7.38 - 7.32 (m, 2H), 7.32 - 7.25 (m, 2H), 7.05 (dd, J = 11.4, 2.9 Hz, 1H), 6.82 (ddd, J = 9.0, 2.9, 1.2 Hz, 1H), 4.54 (s, 2H), 4.43 (s, 2H), 2.66 (d, J = 1.4 Hz, 2H), 2.43 - 2.33 (m, 2H), 2.02 - 1.70 (m, 6H); MS (ESI⁺) *m*/*z* 466.0 (M+H)⁺.

### Example 466: 2-(4-chloro-3-fluorophenoxy)-N-{4-[(4-chlorophenyl)methoxy]-2-hydroxybicyclo[2.2.2]octan-1-yl}acetamide (Compound 565)

To a suspension of Example 465F (50.1 mg, 0.107 mmol) in methanol (1 mL) and CH₂Cl₂ (1 mL) was added sodium tetrahydroborate (8.13 mg, 0.215 mmol). The reaction mixture was stirred for 2 hours. The reaction mixture was concentrated, and the residue was purified by reverse-phase HPLC (see protocol in Example 112D) to provide the title compound (35.0 mg, 70%). ¹H NMR (400 MHz, DMSO-*d*₆) *δ* 7.45 (t, J = 8.9 Hz, 1H), 7.38 - 7.30 (m, 2H), 7.30 - 7.21 (m, 3H), 7.02 (dd, J = 11.4, 2.8 Hz, 1H), 6.79 (ddd, J = 9.1, 2.9, 1.2 Hz, 1H), 5.08 (d, J = 4.5 Hz, 1H), 4.43 (s, 2H), 4.33 (s, 2H), 4.06 (dtd, J = 8.4, 5.0, 3.1 Hz, 1H), 2.17 - 1.43 (m, 10H); MS (ESI⁺) m/z 468.1 (M+H)⁺.

### Example 467: N,N'-[2-(2-hydroxypropan-2-yl)bicyclo[2.2.2]octane-1,4-diyl]bis[2-(4-chloro-3-fluorophenoxy)acetamide] (Compound 566)

To a mixture of Example 470 (100 mg, 0.175 mmol) in tetrahydrofuran (3.0 mL) and dichloromethane (30 mL), methylmagnesium bromide (0.350 mL, 1.050 mmol) was added at 0 °C, and the mixture was stirred for another 16 hours at ambient temperature. Then the reaction was quenched with saturated ammonium chloride solution and extracted with ethyl acetate. The organic layer was dried over magnesium sulfate and filtered. The filtrate was concentrated, and the residue was purified by preparative HPLC (40-100% acetonitrile in 0.1% trifluoroacetic acid/water at 50 mL/minute on Phenomenex^{®} C18 10µm 50 mm × 250 mm column) twice to give 48 mg of the title compound. ¹H NMR (400 MHz, DMSO-*d*₆) *δ* ppm 9.75 (s, 1H), 7.60 7.42 (m, 3H), 7.04 (ddd, J = 11.5, 6.4, 2.9 Hz, 2H), 6.82 (td, J = 9.8, 2.7 Hz, 2H), 5.37 (s, 1H), 4.43 (s, 2H), 4.444.30 (m, 2H), 2.83 (td, J = 11.6, 7.4 Hz, 1H), 2.57 (q, J = 9.3, 7.9 Hz, 1H), 2.10 1.92 (m, 3H), 1.91 1.70 (m, 4H), 1.62 1.48 (m, 1H), 1.36 1.21 (m, 1H), 1.17 (s, 3H), 1.11 (s, 3H); MS (ESI⁺) *m*/*z* 559.0 (M+H)⁺.

### Example 468: N-[4-(benzyloxy)-2-hydroxybicyclo[2.2.2]octan-1-yl]-2-(4-chloro-3-fluorophenoxy)acetamide (Compound 567)

### Example 468A: N-(4-(benzyloxy)-2-oxobicyclo[2.2.2]octan-1-yl)-2-(4-chloro-3-fluorophenoxy)acetamide

To a mixture of Example 465E (50.3 mg, 0.147 mmol), 2,6-di-tert-butylpyridine (0.070 mL, 0.324 mmol), and silver trifluoromethanesulfonate (68.1 mg, 0.265 mmol) in CH₂Cl₂ (3 mL) was added (chloromethyl)benzene (0.030 mL, 0.265 mmol). The reaction mixture was stirred for 5.5 hours. The reaction mixture was filtered and concentrated. The residue was purified on a silica column (12 g) using the Biotage^{®} Isolera^{™} One flash system eluting with heptane/ethyl acetate (8:2 to 6:4) to provide the title compound (48.1 mg, 76%). MS (ESI⁺) *m*/*z* 432.2 (M+H)⁺.

### Example 468B: N-[4-(benzyloxy)-2-hydroxybicyclo[2.2.2]octan-1-yl]-2-(4-chloro-3-fluorophenoxy)acetamide

The reaction described in Example 466 substituting Example 468A for Example 465F gave the title compound. ¹H NMR (400 MHz, DMSO-*d*₆) *δ* 7.45 (t, J = 8.9 Hz, 1H), 7.31 - 7.13 (m, 6H), 7.02 (dd, J = 11.4, 2.8 Hz, 1H), 6.80 (ddd, J = 8.9, 2.9, 1.2 Hz, 1H), 5.08 (d, J = 4.4 Hz, 1H), 4.44 (s, 2H), 4.33 (s, 2H), 4.13 - 4.00 (m, 1H), 2.19 - 1.48 (m, 10H); MS (ESI⁺) *m*/*z* 434.1 (M+H)⁺.

### Example 469: N,N'-[2-(hydroxymethyl)bicyclo[2.2.2]octane-1,4-diyl]bis[2-(4-chloro-3-fluorophenoxy)acetamide] (Compound 568)

A suspension of aluminum(III) lithium hydride (0.306 ml, 0.306 mmol) in tetrahydrofuran (0.5 mL) was cooled to 0 °C, treated with Example 470 (0.05 g, 0.088 mmol) in tetrahydrofuran (0.5 mL), and left stirring at room temperature for 20 minutes. The reaction was carefully quenched with water (0.2 mL), extracted with dichloromethane (1 mL), dried over a pad of diatomaceous earth, and concentrated. The residue was purified by preparative HPLC (Phenomenex^{®} Luna^{®} C18(2) 5 µm 100 Å AXIA^{™} column 250 mm × 21.2 mm, flow rate 25 mL/minute, 10-90% gradient of acetonitrile in buffer (0.1% trifluoroacetic acid in water)) to provide the title compound (27 mg, 56.8% yield). ¹H NMR (400 MHz, DMSO-*d*₆) *δ* ppm 8.39 (s, 1H), 7.48 (td, J = 8.7, 3.8 Hz, 3H), 7.02 (dt, J = 11.3, 3.0 Hz, 2H), 6.80 (ddd, J = 9.0, 2.9, 1.2 Hz, 2H), 5.15 (s, 1H), 4.42 (q, J = 7.3 Hz, 4H), 3.57 (t, J = 9.6 Hz, 1H), 3.40 - 3.34 (m, 1H), 2.45 (q, J = 6.0 Hz, 1H), 2.27 - 2.09 (m, 2H), 2.09 - 1.88 (m, 2H), 1.88 - 1.69 (m, 5H), 1.45 (ddd, J = 12.8, 7.0, 2.7 Hz, 1H); MS (APCI) *m*/*z* 544 (M+H)⁺.

### Example 470: methyl 1,4-bis[2-(4-chloro-3-fluorophenoxy)acetamido]bicyclo[2.2.2]octane-2-carboxylate (Compound 569)

### Example 470A: ethyl 4-(2-(4-chloro-3-fluorophenoxy)acetamido)-2-cyanobicyclo[2.2.2]octane-1-carboxylate

To a solution of Example 198F (19.5 g, 46.6 mmol), toluene-4-sulfonylmethyl isocyanide (27.3 g, 140 mmol) and ethanol (4.62 mL, 79 mmol) in dimethoxyethane (500 mL) was added potassium tert-butoxide (26.1 g, 233 mmol) at -50 °C under N₂. The mixture was stirred at -50 °C for 3 hours. The reaction was quenched with saturated aqueous ammonium chloride (1500 mL) and extracted with ethyl acetate (3 × 500 mL). The combined organic layers were washed with brine (300 mL), dried over anhydrous sodium sulfate and concentrated under reduced pressure. The residue was purified by column chromatography on silica gel (petroleum ether:ethyl acetate=3:1) to give the title compound (16.5 g, 69.3% yield). MS (ESI) *m*/*z* 409 (M+H)⁺.

### Example 470B: 4-(2-(4-chloro-3-fluorophenoxy)acetamido)-2-cyanobicyclo[2.2.2]octane-1-carboxylic acid

To a mixture of Example 470A (16.5 g, 32.3 mmol) in tetrahydrofuran (200 mL), methanol (50 mL) and water (50 mL) was added sodium hydroxide (2.58 g, 64.6 mmol) at 20 °C, and the reaction mixture was stirred at 20 °C for 12 hours. The reaction mixture was concentrated under reduced pressure. The residue was diluted with water (200 mL), acidified with HCl (1 N) to pH=1. The resulting precipitate was collected by filtration and dried under high vacuum to give the title compound (13.5 g, 100% yield). ¹H NMR (400 MHz, DMSO-*d*₆) *δ* ppm 1.67-2.11 (m, 9H), 2.23-2.38 (m, 1H), 3.36-3.44 (m, 1H), 4.46 (s, 2H), 6.76-6.86 (m, 1H), 7.03 (dd, J=11.36, 2.76 Hz, 1H), 7.48 (t, J=8.93 Hz, 1H), 7.73 (s, 1H), 12.85 (br s, 1H).

### Example 470C: tert-butyl (4-(2-(4-chloro-3-fluorophenoxy)acetamido)-2-cyanobicyclo[2.2.2]octan-1-yl)carbamate

To a solution of Example 470B (12 g, 22.57 mmol) in t-butanol (200 mL) were added triethylamine (13.31 mL, 96 mmol) and diphenylphosphoryl azide (17.53 g, 63.7 mmol) at 20 °C, and the reaction mixture was stirred at 80 °C under N₂ for 12 hours. After being cooled to 20 °C, the resulting solution was concentrated under reduced pressure. The residue was purified by column chromatography on silica gel (petroleum ether:ethyl acetate=3:1) to give the title compound (12 g, 70.9 % yield). ¹H NMR: (400 MHz, DMSO-*d*₆) *δ* ppm 1.38 (s, 8H), 1.47-2.39 (m, 11H), 3.80-3.97 (m, 1H), 4.44 (s, 2H), 6.80 (dt, *J*=8.93, 1.41 Hz, 1H), 7.02 (dd, *J*=11.37, 2.81 Hz, 2H), 7.48 (t, *J*=8.93 Hz, 1H), 7.65 (s, 1H).

### Example 470D: methyl 1-amino-4-(2-(4-chloro-3-fluorophenoxy)acetamido)bicyclo[2.2.2]octane-2-carboxylate, hydrochloric acid

HCl (20 mL, 658 mmol) was bubbled into a solution Example 470C (1 g, 1.881 mmol) in methanol (20 mL) at -50 °C for 0.5 hours. The reaction was stirred at 20 °C for 2 hours, concentrated under reduced pressure and diluted with water (20 mL). Lyophilization provided the title compound (1 g, 76% yield). MS (ESI) *m*/*z* 385 (M+H)⁺.

### Example 470E: methyl 1,4-bis[2-(4-chloro-3-fluorophenoxy)acetamido]bicyclo[2.2.2]octane-2-carboxylate

A suspension of Example 470D (0.8 g, 1.899 mmol), *N*-ethyl-*N*-isopropylpropan-2-amine (0.829 ml, 4.75 mmol) and 2-(4-chloro-3-fluorophenoxy)acetic acid (0.388 g, 1.899 mmol) in *N,N*-dimethylformamide (8 mL) was treated with 2-(3*H*-[1,2,3]triazolo[4,5-*b*]pyridin-3-yl)-1,1,3,3-tetramethylisouronium hexafluorophosphate(V) (0.794 g, 2.089 mmol) and stirred at 20 °C for 20 hours. The reaction was concentrated and purified by preparative HPLC (Phenomenex^{®} Luna^{®} C18(2) 5 µm 100 Å AXIA^{™} column 250 mm × 21.2 mm, flow rate 25 mL/minute, 10-90% gradient of acetonitrile in buffer (0.1% trifluoroacetic acid in water)) to provide the title compound (0.82 g, 76%). ¹H NMR (400 MHz, DMSO-*d*₆) *δ* ppm 7.57 (d, J = 9.4 Hz, 2H), 7.48 (td, J = 8.9, 1.2 Hz, 2H), 7.01 (td, J = 11.3, 2.9 Hz, 2H), 6.86 - 6.75 (m, 2H), 4.49 - 4.37 (m, 4H), 3.56 (s, 3H), 3.54 - 3.50 (m, 1H), 2.28 - 1.76 (m, 8H), 1.76 - 1.55 (m, 2H); MS (APCI⁺) *m*/*z* 572 (M+H)⁺.

### Example 471: 2-(2-bromo-4-chloro-5-fluorophenoxy)-N-{2-bromo-4-[2-(4-chloro-3-fluorophenoxy)acetamido]-3-oxobicyclo[2.2.2]octan-1-yl}acetamide (Compound 570)

The title compound was prepared as a minor product in the synthesis of Example 473. ¹H NMR (501 MHz, CDCl₃) *δ* ppm 7.68 - 7.59 (m, 2H), 7.32 (t, J = 8.6 Hz, 1H), 6.82 - 6.76 (m, 2H), 6.76 - 6.69 (m, 2H), 5.54 (d, J = 2.0 Hz, 1H), 4.49 - 4.46 (m, 0H), 4.45 (d, J = 1.1 Hz, 2H), 4.44 - 4.41 (m, 0H), 4.40 (s, 2H), 3.22 - 3.00 (m, 2H), 2.84 (dddd, J = 17.2, 11.7, 5.5, 3.5 Hz, 1H), 2.39 - 2.17 (m, 2H), 2.17 - 2.06 (m, 1H), 1.86 - 1.63 (m, 2H); MS(APCI) *m*/*z* 684.8 (M+H)⁺.

### Example 472: rac-N,N'-[(2S,3R)-2-bromo-3-hydroxybicyclo[2.2.2]octane-1,4-diyl]bis[2-(4-chloro-3-fluorophenoxy)acetamide] (Compound 571)

To a solution of *N,N*'-(2-bromo-3-oxobicyclo[2.2.2]octane-1,4-diyl)bis[2-(4-chloro-3-fluorophenoxy)acetamide] (Example 473, 20 mg, 0.033 mmol) in CH₃OH-CH₂Cl₂ (3 mL) was added sodium borohydride (1.2 mg, 0.033 mmol). The reaction mixture was stirred for 0.5 hours at ambient temperature. The mixture was then treated with saturated aqueous NaHCO₃ and extracted with CH₂Cl₂. The organic fraction was washed with brine, dried over Na₂SO₄, and concentrated. The residue was purified by chromatography eluting with 10-100% ethyl acetate in heptane to give the title compound (18 mg, 90% yield). ¹H NMR (400 MHz, CDCl₃) *δ* ppm 7.33 (td, J = 8.6, 2.3 Hz, 2H), 6.76 (ddd, J = 10.1, 7.2, 2.9 Hz, 2H), 6.68 (td, J = 9.2, 8.4, 2.9 Hz, 3H), 6.35 (s, 1H), 5.40 (dd, J = 8.2, 2.2 Hz, 1H), 4.47 - 4.30 (m, 4H), 4.22 - 4.09 (m, 1H), 3.19 (d, J = 4.6 Hz, 1H), 2.59 (t, J = 12.2 Hz, 1H), 2.44 (td, J = 12.3, 4.6 Hz, 1H), 2.24 - 2.12 (m, 1H), 2.12 - 1.93 (m, 4H), 1.91 - 1.78 (m, 1H); MS(APCI) *m*/*z* 609.1 (M+H)⁺.

### Example 473: N,N'-(2-bromo-3-oxobicyclo[2.2.2]octane-1,4-diyl)bis[2-(4-chloro-3-fluorophenoxy)acetamide] (Compound 572)

To a solution of *N,N'*-(2-oxobicyclo[2.2.2]octane-1,4-diyl)bis[2-(4-chloro-3-fluorophenoxy)acetamide] (Example 173, 300 mg, 0.569 mmol) in acetic acid (3 mL) at 80 °C was added bromine (95 mg, 0.597 mmol) in acetic acid (0.5 mL) slowly. The reaction mixture was stirred at 80 °C for 6 hours. The mixture was then diluted with ethyl acetate, washed with water and brine, and dried over Na₂SO₄. Volatiles were removed in vacuo, and the residue was purified by chromatography on silica gel eluting with 0-100% ethyl acetate in heptane to give the title compound (240 mg, 70% yield), and the corresponding di-brominated product (15 mg, 4%, Example 471). ¹H NMR (400 MHz, CDCl₃) *δ* ppm 7.64 (s, 1H), 7.33 (td, J = 8.6, 5.8 Hz, 2H), 6.77 (dd, J = 10.2, 2.9 Hz, 2H), 6.70 (dddd, J = 8.7, 5.8, 2.9, 1.3 Hz, 2H), 6.32 (s, 1H), 5.59 (d, J = 2.0 Hz, 1H), 4.50 - 4.34 (m, 4H), 3.21 - 3.00 (m, 2H), 2.84 (m, 1H), 2.27 (ddd, J = 13.7, 12.2, 4.5 Hz, 1H), 2.22 - 2.12 (m, 1H), 2.12 - 1.99 (m, 1H), 1.82 - 1.62 (m, 2H), MS(APCI) *m*/*z* 607.1 (M+H)⁺.

### Example 474: N,N'-(bicyclo[3.2.1]octane-1,4-diyl)bis[2-(4-chloro-3-fluorophenoxy)acetamide] (Compound 573)

### Example 474A: N,N'-(bicyclo[3.2.1]oct-3-ene-1,4-diyl)bis(2-(4-chloro-3-fluorophenoxy)acetamide)

To a solution of Example 245 (0.25 g, 0.412 mmol) in N,N-dimethylformamide (2.5 mL) was added sodium azide (0.134 g, 2.058 mmol), and the reaction mixture was allowed to stir at 90 °C for 16 hours, then at 120 °C under microwave irradiation for 45 minutes. The reaction mixture was cooled to ambient temperature and partitioned between water and ethyl acetate. The organic layer was washed with brine, dried over magnesium sulfate and filtered. The filtrate was concentrated, and the residue was purified by preparative reverse phase HPLC (10-95% acetonitrile in 0.1% trifluoroacetic acid/water at 25 mL/minute on Phenomenex^{®} C18 5µm 250 mm x 21.2 mm column) to give 33 mg of the title compound as a by-product. ¹H NMR (400 MHz, DMSO-*d*₆) *δ* ppm 9.04 (s, 1H), 8.04 (s, 1H), 7.45 (td, J = 8.9, 2.5 Hz, 2H), 7.02 (ddd, J = 11.4, 6.5, 2.9 Hz, 2H), 6.80 (ddt, J = 8.9, 2.7, 1.3 Hz, 2H), 5.80 (t, J = 3.7 Hz, 1H), 4.56 (s, 2H), 4.45 (s, 2H), 2.58 2.49 (m, 1H), 2.32 (dd, J = 16.8, 4.7 Hz, 1H), 1.99 1.79 (m, 6H), 1.83 1.70 (m, 1H); MS (ESI⁺) *m*/*z* 511.1 (M+H)⁺.

### Example 474B: N,N'-(bicyclo[3.2.1]octane-1,4-diyl)bis[2-(4-chloro-3-fluorophenoxy)acetamide]

A mixture of Example 474A (16 mg, 0.031 mmol) in tetrahydrofuran (3.5 mL) and 5% Pt/C wet, RD0585 (5mg, 10.53 µmol) in a 10 mL pressure tube was stirred under 50 psi of hydrogen and at ambient temperature for 2 hours. The suspension was filtered, and the filtrate was concentrated. The residue was purified by preparative reverse phase HPLC (40-100% acetonitrile in 0.1% trifluoroacetic acid/water at 59 mL/minute on Phenomenex^{®} C18 10 µm 250 mm x 50 mm column) to give10 mg of the title compound. ¹H NMR (400 MHz, DMSO-*d*₆) *δ* ppm 7.99 - 7.88 (m, 2H), 7.48 (t, J = 8.9 Hz, 2H), 7.04 (dt, J = 11.4, 3.1 Hz, 2H), 6.83 (dtd, J = 9.0, 2.9, 1.1 Hz, 2H), 4.50 (s, 2H), 4.46 (s, 2H), 3.71 (dddd, J = 10.5, 7.9, 5.3, 2.4 Hz, 1H), 2.12 (d, J = 12.2 Hz, 1H), 1.95 - 1.38 (m, 9H); MS (ESI⁺) *m*/*z* 513.1 (M+H)⁺.

### Example 475: 2-(4-chloro-3-fluorophenoxy)-N-{(2S)-4-[2-(4-chloro-3-hydroxyphenoxy)acetamido]-2-hydroxybicyclo[2.2.2]octan-1-yl}acetamide (Compound 574)

### Example 475A: tert-butyl 2-(3-bromo-4-chlorophenoxy)acetate

To a mixture of 3-bromo-4-chlorophenol (0.872 g, 4.20 mmol) and *tert*-butyl 2-bromoacetate (0.931 mL, 6.31 mmol) in *N,N*-dimethylformamide (8 mL) was added cesium carbonate (4.11 g, 12.61 mmol). The reaction mixture was stirred at 60 °C overnight. After cooling, the suspension was filtered and rinsed with ethyl acetate. The filtrate was treated with brine and washed with ethyl acetate (2×). The combined organic layers were dried over MgSO₄, filtered, and concentrated. The residue was purified on a 40 g silica column using the Biotage^{®} Isolera^{™} One flash system eluting with heptanes/ethyl acetate (85:15) to provide the title compound (0.910 g, 67%); MS (DCI⁺) *m*/*z* 340.0 (M+NH₄)⁺.

### Example 475B: tert-butyl 2-(4-chloro-3-(4,4,5,5-tetramethyl-1,3,2-dioxaborolan-2-yl)phenoxy)acetate

A mixture of Example 475A (0.777 g, 2.416 mmol), bis(pinacolato)diboron (0.736 g, 2.90 mmol), potassium acetate (0.474 g, 4.83 mmol), and [1,1'-bis(diphenylphosphino)ferrocene]dichloropalladium(II), complex with dichloromethane (0.079 g, 0.097 mmol) in 1,4-dioxane (15 mL) was degassed and heated to 100 °C overnight. The mixture was concentrated. The residue was treated with brine and extracted with ethyl acetate (2×). The combined organic layers were dried over MgSO₄, filtered, and concentrated. The residue was purified on a 40 g silica column using the Biotage^{®} Isolera^{™} One flash system eluting with heptanes/ethyl acetate (95:5 to 90:10) to provide the title compound (0.477 g, 54%). MS (ESI⁺) *m*/*z* 386.0 (M+NH₄)⁺.

### Example 475C: tert-butyl 2-(4-chloro-3-hydroxyphenoxy)acetate

A solution of Example 475B (0.373 g, 1.012 mmol) in acetone (5 mL) was treated with a solution of Oxone^{®} (potassium peroxomonosulfate, 0.871 g, 1.416 mmol) in water (5 mL) at 0 °C. The reaction mixture was stirred at 0 °C for 1 hour and at room temperature for 2 hours. The reaction was quenched with aqueous Na₂S₂O₃ and the mixture was concentrated. The resulting suspension was treated with water and extracted with ethyl acetate (2×). The combined organic layers were dried over MgSO4, filtered, and concentrated. The residue was purified on a 25 g silica gel column using the Biotage^{®} Isolera^{™} One flash system eluting with heptanes/ethyl acetate (85:15 to 80:20) to provide the title compound (0.225 g, 86%); MS (DCI⁺) *m*/*z* 276.0 (M+NH₄)⁺.

### Example 475D: 2-(4-chloro-3-hydroxyphenoxy)acetic acid

A mixture of Example 475C (0.220 g, 0.850 mmol) and trifluoroacetic acid (1.31 mL, 17.01 mmol) in CH₂Cl₂ (6 mL) was stirred overnight. The resulting suspension was filtered, rinsed with CH₂Cl₂, and vacuum oven-dried to provide the title compound (0.148 g, 86%); MS (DCI⁺) *m*/*z* 220.0 [M+NH₄]⁺.

### Example 475E: 2-(4-chloro-3-fluorophenoxy)-N-{(2S)-4-[2-(4-chloro-3-hydroxyphenoxy)acetamido]-2-hydroxybicyclo[2.2.2]octan-1-yl}acetamide

A mixture of Example 338B (50.0 mg, 0.146 mmol), triethylamine (0.026 mL, 0.190 mmol), Example 475D (29.6 mg, 0.146 mmol), and (1-[bis(dimethylamino)methylene]-1*H-*1,2,3-triazolo[4,5-b]pyridinium 3-oxid hexafluorophosphate) (HATU, 66.6 mg, 0.175 mmol) in N,N-dimethylformamide (2 mL) was stirred for 4 hrs. The reaction mixture was quenched with brine and saturated NaHCO₃ and extracted with ethyl acetate (2×). The combined organic layers were washed with brine, dried over MgSO₄, filtered, and concentrated. The residue was purified by reverse-phase HPLC (see protocol in Example 112D) to provide the title compound (28.8 mg, 37%). ¹H NMR (400 MHz, DMSO-*d*₆) *δ* ppm 10.17 (s, 1H), 7.48 (t, J = 8.9 Hz, 1H), 7.41 (s, 1H), 7.26 (s, 1H), 7.19 (d, J = 8.8 Hz, 1H), 7.05 (dd, J = 11.4, 2.9 Hz, 1H), 6.89 - 6.75 (m, 1H), 6.51 (d, J = 2.9 Hz, 1H), 6.36 (dd, J = 8.8, 2.9 Hz, 1H), 5.08 (s, brd, 1H), 4.46 (s, 2H), 4.31 (s, 2H), 4.04 (dd, J = 9.6, 3.1 Hz, 1H), 2.27 (ddd, J = 12.1, 9.6, 2.2 Hz, 1H), 2.07 (ddd, J = 12.3, 10.4, 4.9 Hz, 1H), 1.99 - 1.66 (m, 8H); MS (ESI⁺) *m*/*z* 527.1 (M+H)⁺.

### Example 476: 2-(4-chloro-3-fluorophenoxy)-N-[(2S)-2-hydroxy-4-{2-[(5-methyl-1,3-thiazol-2-yl)oxy]acetamido}bicyclo[2.2.2]octan-1-yl]acetamide (Compound 575)

### Example 476A: (S)-2-chloro-N-(4-(2-(4-chloro-3-fluorophenoxy)acetamido)-3-hydroxybicyclo[2.2.2]octan-1-yl)acetamide

The title compound was prepared using the methodologies described in Example 295A substituting Example 338B for Example 276A. ¹H NMR (400 MHz, DMSO-*d*₆) *δ* ppm 7.79 (s, 1H), 7.54 (t, J = 8.9 Hz, 1H), 7.32 (s, 1H), 7.11 (dd, J = 11.4, 2.9 Hz, 1H), 6.89 (ddd, J = 8.9, 2.9, 1.2 Hz, 1H), 5.14 (d, J = 4.4 Hz, 1H), 4.53 (s, 2H), 4.10 (dt, J = 8.8, 3.7 Hz, 1H), 3.99 (s, 2H), 2.30 (ddd, J = 12.6, 9.4, 2.4 Hz, 1H), 2.12 (ddd, J = 12.3, 10.4, 5.0 Hz, 1H), 2.01 1.75 (m, 8H); MS (ESI⁺) *m*/*z* 419.0 (M+H)⁺.

### Example 476B: 2-(4-chloro-3-fluorophenoxy)-N-[(2S)-2-hydroxy-4-{2-[(5-methyl-1,3-thiazol-2-yl)oxy]acetamido}bicyclo[2.2.2]octan-1-yl]acetamide

The title compound was prepared using the methodologies described in Example 295B substituting Example 476A for Example 295A and 5-methylthiazol-2-ol for 5,6-dimethylpyridin-3-ol. ¹H NMR (400 MHz, DMSO-*d*₆) *δ* ppm 7.68 (s, 1H), 7.42 (t, J = 8.9 Hz, 1H), 7.23 (s, 1H), 6.98 (dd, J = 11.4, 2.9 Hz, 1H), 6.81 - 6.73 (m, 1H), 6.52 (d, J = 1.7 Hz, 1H), 5.14 (d, J = 4.3 Hz, 1H), 4.69 (s, 1H), 4.39 (s, 2H), 4.11 (s, 2H), 2.20 (ddd, J = 12.4, 9.5, 2.3 Hz, 1H), 2.03 (d, J = 1.4 Hz, 3H), 1.96 (ddd, J = 12.5, 10.1, 5.5 Hz, 1H), 1.90 - 1.74 (m, 3H), 1.70 (dd, J = 14.3, 9.2 Hz, 4H), 1.65 (dd, J = 13.2, 2.8 Hz, 1H); MS (ESI⁺) *m*/*z* 498.1 (M+H)⁺.

### Example 477: 2-(4-chloro-3-fluorophenoxy)-N-[(2S)-4-({[(4-chloro-3-fluorophenyl)methyl]carbamoyl}amino)-2-hydroxybicyclo[2.2.2]octan-1-yl]acetamide (Compound 576)

### Example 477A: (S)-2,5-dioxopyrrolidin-1-yl (4-(2-(4-chloro-3-fluorophenoxy)acetamido)-3-hydroxybicyclo[2.2.2]octan-1-yl)carbamate

A mixture of Example 338B (200 mg, 0.583 mmol), bis(2,5-dioxopyrrolidin-1-yl) carbonate (179 mg, 0.700 mmol) and pyridine (0.071 mL, 0.875 mmol) in acetonitrile (3.0 mL) was stirred at room temperature for 2 days. The mixture was removed under high vacuum to give 180 mg (64%) of the title compound, which was used without further purifications. MS (APCI⁺) *m*/*z* 484.2 (M+H)⁺.

### Example 477B: 2-(4-chloro-3-fluorophenoxy)-N-[(2S)-4-({[(4-chloro-3-fluorophenyl)methyl]carbamoyl}amino)-2-hydroxybicyclo[2.2.2]octan-1-yl]acetamide

A mixture of Example 477A (61.7 mg, 0.128 mmol), (4-chloro-3-fluorophenyl)methanamine (0.019 mL, 0.153 mmol), and triethylamine (0.021 mL, 0.153 mmol) in N,N-dimethylformamide (1.5 mL) was heated at 50 °C for 3 hrs. After cooling, the reaction mixture was treated with brine and extracted with ethyl acetate (2×). The combined organic layers were dried over MgSO₄, filtered, and concentrated. The residue was purified by reverse-phase HPLC (see protocol in Example 112D) to provide the title compound (23.7 mg, 35%). ¹H NMR (400 MHz, DMSO-*d*₆) *δ* ppm 7.50 (dt, J = 14.5, 8.4 Hz, 2H), 7.26 - 7.15 (m, 2H), 7.11 - 7.00 (m, 2H), 6.83 (ddd, J = 8.9, 2.8, 1.1 Hz, 1H), 6.18 (t, J = 6.1 Hz, 1H), 5.74 (s, 1H), 5.02 (d, J = 4.4 Hz, 1H), 4.46 (s, 2H), 4.14 (d, J = 6.1 Hz, 2H), 4.01 (dt, J = 8.5, 3.8 Hz, 1H), 2.23 (ddd, J = 12.6, 9.4, 2.8 Hz, 1H), 2.12 - 1.98 (m, 1H), 1.95 - 1.63 (m, 8H); MS (ESI⁺) *m*/*z* 528.2 (M+H)⁺.

### Example 478: 2-(4-chloro-3-fluorophenoxy)-N-[(2S)-2-hydroxy-4-{2-[(1,3-thiazol-2-yl)oxy]acetamido}bicyclo[2.2.2]octan-1-yl]acetamide (Compound 577)

The title compound was prepared using the methodologies described in Example 295B substituting Example 476A for Example 295A and thiazol-2-ol for 5,6-dimethylpyridin-3-ol. ¹H NMR (400 MHz, DMSO-*d*₆) *δ* ppm 7.73 (s, 1H), 7.48 (t, J = 8.9 Hz, 1H), 7.25 (s, 1H), 7.05 (dd, J = 11.4, 2.8 Hz, 1H), 6.89 (d, J = 5.3 Hz, 1H), 6.82 (dd, J = 8.8, 2.7 Hz, 1H), 6.37 (d, J = 5.3 Hz, 1H), 4.46 (s, 2H), 4.23 (s, 2H), 4.03 (dd, J = 9.5, 3.1 Hz, 1H), 2.24 (ddd, J = 12.5, 9.5, 2.4 Hz, 1H), 2.06 (ddd, J = 12.3, 10.5, 5.0 Hz, 1H), 1.93 - 1.67 (m, 8H); MS (ESI⁺) *m*/*z* 484.2 (M+H)⁺.

### Example 479: 2-(4-chloro-3-fluorophenoxy)-N-[(2S)-2-hydroxy-4-{2-[(4,5,6,7-tetrahydro-1,3-benzothiazol-2-yl)oxy]acetamido}bicyclo[2.2.2]octan-1-yl]acetamide (Compound 578)

The title compound was prepared using the methodologies described in Example 295B substituting Example 476A for Example 295A and 4,5,6,7-tetrahydrobenzo[*d*]thiazol-2-ol for 5,6-dimethylpyridin-3-ol. ¹H NMR (400 MHz, DMSO-*d*₆) *δ* ppm 7.75 (s, 1H), 7.48 (t, J = 8.9 Hz, 1H), 7.25 (s, 1H), 7.05 (dd, J = 11.3, 2.9 Hz, 1H), 6.82 (dt, J = 9.2, 1.8 Hz, 1H), 4.46 (s, 2H), 4.16 (s, 2H), 4.07 - 3.94 (m, 1H), 2.34 (p, J = 3.5, 2.9 Hz, 2H), 2.24 (ddt, J = 9.5, 6.5, 2.6 Hz, 3H), 2.05 (ddd, J = 12.2, 10.3, 5.0 Hz, 1H), 1.96 - 1.67 (m, 12H); MS (ESI⁺) *m*/*z* 484.2 (M+H)⁺.

### Example 480: rac-N,N'-[(1S,2R,4R)-2-hydroxybicyclo[2.2.1]heptane-1,4-diyl]bis[2-(4-chloro-3-fluorophenoxy)acetamide] (Compound 579)

### Example 480A: rac-(1R,2S,4S)-1,4-bis(2-(4-chloro-3-fluorophenoxy)acetamido)bicyclo[2.2.1]heptan-2-yl 4-nitrobenzoate

A solution of *rac*-*N,N*'-[(1*S*,2*S*,4*R*)-2-hydroxybicyclo[2.2.1]heptane-1,4-diyl]bis[2-(4-chloro-3-fluorophenoxy)acetamide] (Example 486, 59 mg, 0.114 mmol), triphenylphosphine (45.0 mg, 0.172 mmol), and 4-nitrobenzoic acid (28.7 mg, 0.172 mmol) in toluene (2 mL) was treated with diethyl azodicarboxylate (74.8 mg, 0.172 mmol). The mixture was then heated at 110 °C for 8 hours. The reaction mixture was concentrated and purified by chromatography on silica gel eluting with 0-100% ethyl acetate/heptane to give the title compound (20 mg, 26% yield). ¹H NMR (400 MHz, CDCl₃) *δ* ppm 8.34 - 8.18 (m, 2H), 8.13 - 8.03 (m, 2H), 7.33 (t, J = 8.6 Hz, 1H), 7.15 (t, J = 8.6 Hz, 1H), 7.08 (s, 1H), 6.80 - 6.70 (m, 2H), 6.68 (ddd, J = 8.9, 2.9, 1.3 Hz, 1H), 6.61 (dd, J = 10.2, 2.8 Hz, 1H), 6.53 (ddd, J = 8.9, 2.9, 1.2 Hz, 1H), 5.33 (dd, J = 7.1, 2.2 Hz, 1H), 4.46 - 4.24 (m, 4H), 2.57 (s, 2H), 2.51 (dd, J = 13.9, 7.2 Hz, 1H), 2.44 - 2.26 (m, 2H), 2.10 - 1.80 (m, 3H).

### Example 480B: rac-N,N'-[(1S,2R,4R)-2-hydroxybicyclo[2.2.1]heptane-1,4-diyl]bis[2-(4-chloro-3-fluorophenoxy)acetamide]

To a solution of rac-(1R,2S,4S)-1,4-bis(2-(4-chloro-3-fluorophenoxy)acetamido)bicyclo[2.2.1]heptan-2-yl 4-nitrobenzoate (Example 480A, 20 mg, 0.030 mmol) in CH₃OH (2 mL) was added potassium carbonate (8.32 mg, 0.060 mmol). The reaction mixture was stirred for 2 hours. The mixture was then diluted with CH₂Cl₂, washed with brine, dried over Na₂SO₄, and concentrated. The residue was purified by chromatography on silica gel eluting with 10-100% ethyl acetate in heptane to give the title compound (12 mg, 77% yield). ¹H NMR (400 MHz, CDCl₃) *δ* ppm 7.32 (td, J = 8.6, 3.3 Hz, 2H), 6.93 (s, 1H), 6.76 (ddd, J = 10.3, 5.6, 2.9 Hz, 2H), 6.68 (dddd, J = 9.1, 5.1, 2.9, 1.3 Hz, 2H), 6.61 (s, 1H), 4.45 - 4.36 (m, 4H), 4.18 (td, J = 5.6, 2.3 Hz, 1H), 3.11 (d, J = 5.2 Hz, 1H), 2.41 - 2.27 (m, 2H), 2.25 - 2.13 (m, 2H), 2.13 - 2.01 (m, 1H), 1.96 (ddd, J = 12.9, 3.7, 2.6 Hz, 1H), 1.82 - 1.65 (m, 2H); MS(APCI) *m*/*z* 515.2 (M+H)⁺.

### Example 481: 2-(4-chlorophenoxy)-N-(3-{2-[(2,2-difluoro-2H-1,3-benzodioxol-5-yl)oxy]acetamidolbicyclo[1.1.1]pentan-1-yl)acetamide (Compound 580)

The reaction and purification conditions described in Example 81, substituting 2-(4-chlorophenoxy)acetic acid (Aldrich) for 2-(4-chloro-3-fluorophenoxy)acetic acid, and the product of Example 280C for benzyl (4-aminobicyclo[2.1.1]hexan-1-yl)carbamate hydrochloride gave the title compound. ¹H NMR (400 MHz, DMSO-*d*₆) *δ* ppm 8.70 (s, 1H), 8.69 (s, 1H), 7.38 - 7.30 (m, 3H), 7.14 (d, J = 2.6 Hz, 1H), 7.00 - 6.94 (m, 2H), 6.77 (dd, J = 8.9, 2.6 Hz, 1H), 4.44 (s, 2H), 4.43 (s, 2H), 2.27 (s, 6H), 1.86 - 1.72 (m, 6H); MS (APCI⁺) *m*/*z* 481 (M+H)⁺.

### Example 482: 2-(3,4-dichlorophenoxy)-N-{(2S)-4-[2-(4-fluorophenoxy)acetamido]-2-hydroxybicyclo[2.2.2]octan-1-yl}acetamide (Compound 581)

The title compound was prepared using the methodologies described in Example 338C substituting Example 445E for Example 338B and 2-(4-fluorophenoxy)acetic acid for 2-(4-chlorophenoxy)acetic acid. ¹H NMR (400 MHz, DMSO-*d*₆) *δ* ppm 7.53 (d, J = 9.0 Hz, 1H), 7.41 (s, 1H), 7.29 7.21 (m, 2H), 7.19 7.06 (m, 2H), 7.00 6.87 (m, 3H), 5.07 (s, 1H), 4.48 (s, 2H), 4.36 (s, 2H), 4.04 (dd, J = 9.7, 3.1 Hz, 1H), 2.27 (ddd, J = 12.2, 9.5, 2.2 Hz, 1H), 2.13 2.01 (m, 1H), 1.98 1.89 (m, 2H), 1.92 1.75 (m, 5H), 1.76 (dd, J = 5.7, 2.4 Hz, 1H); MS (ESI⁺) *m*/*z* 511.1 (M+H)⁺.

### Example 483: 2-(4-chlorophenoxy)-N-{(3S)-4-[2-(3,4-dichlorophenoxy)acetamido]-3-hydroxybicyclo[2.2.2]octan-1-yl}acetamide (Compound 582)

The title compound was prepared using the methodologies described in Example 338C substituting Example 445E for Example 338B. ¹H NMR (400 MHz, DMSO-*d*₆) *δ* ppm 7.53 (d, J = 8.9 Hz, 1H), 7.46 (s, 1H), 7.37 - 7.28 (m, 2H), 7.29 - 7.21 (m, 2H), 7.00 - 6.88 (m, 3H), 5.07 (d, J = 4.3 Hz, 1H), 4.48 (s, 2H), 4.39 (s, 2H), 4.07 - 4.00 (m, 1H), 2.27 (ddd, J = 12.3, 9.5, 2.3 Hz, 1H), 2.07 (ddd, J = 12.1, 10.4, 4.6 Hz, 1H), 1.97 - 1.85 (m, 2H), 1.80 (tdd, J = 12.8, 9.3, 6.4 Hz, 6H); MS (ESI⁺) *m*/*z* 527.0 (M+H)⁺.

### Example 484: 2-(3,4-dichlorophenoxy)-N-[(2S)-2-hydroxy-4-(2-{[6-(trifluoromethyl)pyridin-3-yl]oxy}acetamido)bicyclo[2.2.2]octan-1-yl]acetamide (Compound 583)

The title compound was prepared using the methodologies described in Example 338C substituting Example 445E for Example 338B and 2-((6-(trifluoromethyl)pyridin-3-yl)oxy)acetic acid for 2-(4-chlorophenoxy)acetic acid. ¹H NMR (400 MHz, DMSO-*d*₆) *δ* ppm 8.42 (d, J = 2.9 Hz, 1H), 7.85 (d, J = 8.8 Hz, 1H), 7.64 (s, 1H), 7.52 (dd, J = 11.0, 8.7 Hz, 2H), 7.29 - 7.20 (m, 2H), 6.96 (dd, J = 8.9, 2.9 Hz, 1H), 5.08 (d, J = 4.3 Hz, 1H), 4.62 (s, 2H), 4.48 (s, 2H), 4.04 (d, J = 9.5 Hz, 1H), 2.33 - 2.22 (m, 1H), 2.13 - 2.01 (m, 1H), 1.93 (d, J = 11.2 Hz, 2H), 1.90 - 1.78 (m, 4H), 1.77 (dd, J = 12.4, 2.9 Hz, 2H); MS (ESI⁺) *m*/*z* 562.2 (M+H)⁺.

### Example 485: 2-(3,4-dichlorophenoxy)-N-{3-[2-(3,4-difluorophenoxy)acetamido]bicyclo[1.1.1]pentan-1-yl}acetamide (Compound 584)

The reaction and purification conditions described in Example 81, substituting 2-(3,4-difluorophenoxy)acetic acid (CombiBlocks) for 2-(4-chloro-3-fluorophenoxy)acetic acid, and the product of Example 6C for benzyl (4-aminobicyclo[2.1.1]hexan-1-yl)carbamate hydrochloride gave the title compound. ¹H NMR (501 MHz, DMSO-*d*₆) *δ* ppm 8.72 (s, 1H), 8.70 (s, 1H), 7.55 (d, J = 8.9 Hz, 1H), 7.37 (dt, J = 10.6, 9.3 Hz, 1H), 7.26 (d, J = 2.9 Hz, 1H), 7.09 (ddd, J = 12.6, 6.7, 3.0 Hz, 1H), 6.99 (dd, J = 9.0, 2.9 Hz, 1H), 6.80 (dtd, J = 9.2, 3.3, 1.7 Hz, 1H), 4.49 (s, 2H), 4.44 (s, 2H), 2.27 (s, 6H); MS (ESI⁺) *m*/*z* 488 (M+NH₄)⁺.

### Example 486: rac-N,N'-[(1S,2S,4R)-2-hydroxybicyclo[2.2.1]heptane-1,4-diyl]bis[2-(4-chloro-3-fluorophenoxy)acetamide] (Compound 585)

### Example 486A: dimethyl 2-oxobicyclo[2.2.1]heptane-1,4-dicarboxylate

A mixture of dimethyl bicyclo[2.2.1]heptane-1,4-dicarboxylate (1.5 g, 7.07 mmol), chromium(VI) oxide (4.24 g, 42.4 mmol) in acetic acid (15 mL) was heated at 90 °C for 14 hours. The reaction mixture was diluted with ethyl acetate, washed with water, saturated NaHCO₃ and brine, dried (Na₂SO₄), and concentrated. The residue was purified by chromatography eluting with 10-60% ethyl acetate in heptane to give the title compound (400 mg, 25% yield). ¹H NMR (400 MHz, CDCl₃) *δ* ppm 3.78 (d, J = 5.2 Hz, 6H), 2.67 (dd, J = 18.0, 2.5 Hz, 1H), 2.44 - 2.32 (m, 2H), 2.32 - 2.16 (m, 3H), 1.98 - 1.78 (m, 2H).

### Example 486B: rac-(1R,2R,4S)-dimethyl 2-hydroxybicyclo[2.2.1]heptane-1,4-dicarboxylate

To a solution of dimethyl 2-oxobicyclo[2.2.1]heptane-1,4-dicarboxylate (516 mg, 2.281 mmol, Example 486A) in tetrahydrofuran (10 mL) at -78°C was added 1 N lithium tri-*sec-*butylhydroborate (2.74 mL, 2.74 mmol) dropwise. The reaction mixture was stirred at -78 °C for 0.5 hour before it was quenched with 0.75 mL of 1 M HCl. The reaction mixture was diluted with CH₂Cl₂, washed with saturated NaHCO₃ and brine, dried over Na₂SO₄, and concentrated. The residue was purified by chromatography on silica gel eluting with 10-100% ethyl acetate in heptane to give the titled compound (440 mg, 85% yield). ¹H NMR (501 MHz, CDCl₃) *δ* ppm 4.48 (ddt, J = 10.6, 4.1, 2.1 Hz, 1H), 3.72 (s, 3H), 3.69 (s, 3H), 2.46 - 2.35 (m, 2H), 2.32 (d, J = 2.6 Hz, 1H), 2.13 - 2.06 (m, 1H), 2.06 - 2.01 (m, 1H), 1.91 (dt, J = 10.2, 2.3 Hz, 1H), 1.85 (ddd, J = 12.4, 4.4, 1.9 Hz, 1H), 1.82 - 1.73 (m, 1H), 1.42 (dt, J = 13.1, 3.7 Hz, 1H).

### Example 486C: rac-(1R,2R,4S)-dimethyl 2-((tert-butyldimethylsilyl)oxy)bicyclo[2.2.1]heptane-1,4-dicarboxylate

To a solution of rac-(1R,2R,4S)-dimethyl 2-hydroxybicyclo[2.2.1]heptane-1,4-dicarboxylate (155 mg, 0.679 mmol, Example 486B) in N,N-dimethylformamide (5 mL) was added imidazole (185 mg, 2.72 mmol) followed by tert-butyldimethylsilyl chloride (307 mg, 2.037 mmol) and 4-(dimethylamino)pyridine (8.30 mg, 0.068 mmol). The solution was stirred at ambient temperature for 3 hours. The reaction mixture was diluted with ethyl acetate, washed with water and brine, dried over Na₂SO₄, and concentrated. The residue was purified by silica gel chromatography eluting with 0-50% ethyl acetate in heptane to give the titled compound (210 mg, 90%). ¹H NMR (400 MHz, CDCl₃) *δ* ppm 4.55 (ddd, J = 10.1, 3.5, 1.7 Hz, 1H), 3.68 (d, J = 0.9 Hz, 5H), 2.50 - 2.28 (m, 2H), 2.12 - 1.68 (m, 5H), 1.35 (dt, J = 12.7, 3.3 Hz, 1H), 0.88 (s, 9H), 0.02 (s, 3H), 0.00 (s, 3H).

### Example 486D: rac-(1R,2R,4S)-2-((tert-butyldimethylsilyl)oxy)bicyclo[2.2.1]heptane-1,4-dicarboxylic acid

To a solution of rac-(1R,2R,4S)-dimethyl 2-((*tert-*butyldimethylsilyl)oxy)bicyclo[2.2.1]heptane-1,4-dicarboxylate (100 mg, 0.292 mmol, Example 486C) in methanol (1 mL) and tetrahydrofuran (THF, 1.5 mL) was added a solution of lithium hydroxide (28 mg, 1.168 mmol) in water (1 mL). The mixture was stirred for 8 hours. The reaction mixture was diluted with 5 mL of water and acidified with 1 N HCl to pH=2. The mixture was extracted with ethyl acetate, washed with brine, dried over Na₂SO₄, and concentrated to give the titled compound (90 mg, 98% yield), which was used in the next step without further purification. ¹H NMR (500 MHz, CDCl₃) *δ* ppm 4.59 (ddd, J = 10.1, 3.5, 1.6 Hz, 1H), 3.68 (d, J = 4.7 Hz, 0H), 2.47 - 2.36 (m, 2H), 2.09 (ddd, J = 12.4, 10.0, 6.3 Hz, 1H), 2.00 (d, J = 2.1 Hz, 2H), 1.99 - 1.88 (m, 1H), 1.85 - 1.74 (m, 1H), 1.39 (dt, J = 12.7, 2.9 Hz, 1H), 0.88 (s, 9H), 0.04 (s, 3H), 0.03 (s, 3H). (The two carboxylic acid protons shown as very broad peaks were not integrated.)

### Example 486E: rac-(1R,2R,4S)-2-((tert-butyldimethylsilyl)oxy)bicyclo[2.2.1]heptane-1,4-diamine

A mixture of *rac-*(1*R*,2*R*,4*S*)-2-((*tert*-butyldimethylsilyl)oxy)bicyclo[2.2.1]heptane-1,4-dicarboxylic acid (72 mg, 0.229 mmol, Example 486D), triethylamine (62.8 mg, .621 mmol) and diphenylphosphoryl azide (151 mg, 0.550 mmol) in toluene (2 mL) was stirred at 90 °C for 4 hours. The reaction mixture was then cooled down to 0 °C and sodium trimethylsilanolate (103 mg, 0.916 mmol) was added, and mixture was stirred at ambient temperature for 1 hour before it was quenched with 1 mL of 5% citric acid. The reaction mixture was extracted with ether. The aqueous layer was basified with 1 N NaOH to pH10-11 and then extracted with dichloromethane. The dichloromethane extracts were washed with brine, dried over Na₂SO₄, and concentrated to give the title compound (40 mg), which was used in the next step without further purification. ¹H NMR (400 MHz, CDCl₃) *δ* ppm 3.80 (d, J = 9.5 Hz, 1H), 2.18 - 1.98 (m, 2H), 1.73 (dq, J = 23.9, 9.4, 7.5 Hz, 3H), 1.62 - 1.50 (m, 1H), 1.34 - 1.30 (m, 1H), 0.92 - 0.83 (m, 1H), 0.81 (s, 9H), -0.08 (s, 3H), -0.16 (s, 3H).

### Example 486F: rac-N,N'-((1R,2R,4S)-2-((tert-butyldimethylsilyl)oxy)bicyclo[2.2.1]heptane-1,4-diyl)bis(2-(4-chloro-3-fluorophenoxy)acetamide)

A mixture of 2-(4-chloro-3-fluorophenoxy)acetic acid (96 mg, 0.468 mmol), *rac-*(1R,2R,4S)-2-((tert-butyldimethylsilyl)oxy)bicyclo[2.2.1]heptane-1,4-diamine (40 mg, 156 mmol, Example 486E), *N*-ethyl-*N*-isopropylpropan-2-amine (0.109 mL, 0.624 mmol) in *N,N-*dimethylformamide (DMF) (2 mL) was treated with 2-(3*H*-[1,2,3]triazolo[4,5-b]pyridin-3-yl)-1,1,3,3-tetramethylisouronium hexafluorophosphate(V) (237 mg, 0.624 mmol), and the reaction mixture was stirred at ambient temperature for 12 hours. The reaction mixture was diluted with ethyl acetate, washed with water and brine, and dried over Na₂SO₄. Volatiles were removed under reduced pressure, and the residue was purified by chromatography on silica gel eluting with 0-100% ethyl acetate in heptane to give the desired product (25 mg, 26% yield). ¹H NMR (400 MHz, CDCl₃) *δ* ppm 7.37 - 7.29 (m, 2H), 6.79 - 6.69 (m, 2H), 6.69 - 6.63 (m, 2H), 6.56 (s, 1H), 6.51 (s, 1H), 4.44 (ddd, J = 10.4, 3.7, 1.7 Hz, 1H), 4.40 (s, 2H), 4.39 (s, 2H), 2.44 - 2.37 (m, 3H), 2.36 - 2.27 (m, 1H), 2.04 - 1.81 (m, 3H), 1.63 (ddt, J = 12.4, 3.3, 1.5 Hz, 1H), 0.89 (s, 9H), 0.06 (s, 3H), 0.04 (s, 3H); MS(ESI) *m*/*z* 629.1 (M+H)⁺

### Example 486G: rac-N,N'-[(1S,2S,4R)-2-hydroxybicyclo[2.2.1]heptane-l,4-diyl]bis[2-(4-chloro-3-fluorophenoxy)acetamide]

A solution of *rac-N,N*-((1*R,*2*R,*4*S*)-2-((*tert-*butyldimethylsilyl)oxy)bicyclo[2.2.1]heptane-1,4-diyl)bis(2-(4-chloro-3-fluorophenoxy)acetamide) (21 mg, 0.033 mmol, Example 486F) in tetrahydrofuran (2 mL) was treated with tetrabutylammonium fluoride (0.040 mL, 0.040 mmol) and then was left stirring at ambient temperature for 2 hours. The reaction mixture was concentrated and purified by chromatography on silica gel (0-85% ethyl acetate/heptane) to give the title compound (13 mg, 76% yield). ¹H NMR (400 MHz, CDCl₃) *δ* ppm 7.38 - 7.34 (m, 1H), 7.34 - 7.29 (m, 1H), 6.90 (s, 1H), 6.77 (d, J = 2.9 Hz, 1H), 6.74 (d, J = 2.9 Hz, 1H), 6.68 (dt, J = 2.8, 1.3 Hz, 1H), 6.66 (dt, J = 2.8, 1.3 Hz, 1H), 6.48 (s, 1H), 4.51 - 4.39 (m, 2H), 4.38 (s, 2H), 4.37 - 4.31 (m, 1H), 2.86 - 2.73 (m, 1H), 2.57 - 2.46 (m, 1H), 2.33 (dd, J = 9.0, 3.5 Hz, 1H), 2.27 - 2.13 (m, 2H), 1.97 - 1.84 (m, 1H), 1.66 (dt, J = 12.3, 3.4 Hz, 1H), 1.56 - 1.45 (m, 1H); MS(APCI) *m*/*z* 515.4 (M+H)⁺.

### Example 487: 2-(4-chloro-3-fluorophenoxy)-N-{(2S)-4-[2-(3,4-difluorophenoxy)acetamido]-2-hydroxybicyclo[2.2.2]octan-1-yl}acetamide (Compound 586)

The title compound was prepared using the methodologies described in Example 338C substituting 2-(3,4-difluorophenoxy)acetic acid for 2-(4-chlorophenoxy)acetic acid. ¹H NMR (400 MHz, DMSO-*d*₆) *δ* ppm 7.53 7.43 (m, 2H), 7.35 (dt, J = 10.6, 9.3 Hz, 1H), 7.26 (s, 1H), 7.10 6.99 (m, 2H), 6.83 (ddd, J = 8.9, 2.9, 1.2 Hz, 1H), 6.75 (dtd, J = 8.6, 3.3, 1.7 Hz, 1H), 5.07 (s, 1H), 4.47 (s, 2H), 4.39 (s, 2H), 4.04 (dd, J = 10.0, 3.1 Hz, 1H), 3.17 (s, 0H), 2.27 (ddd, J = 12.3, 9.5, 2.2 Hz, 1H), 2.07 (ddd, J = 12.3, 10.5, 4.7 Hz, 1H), 1.98 1.87 (m, 2H), 1.91 1.78 (m, 3H), 1.82 1.72 (m, 3H); MS (ESI⁺) *m*/*z* 513.2 (M+H)⁺.

### Example 488: 2-(4-chloro-3-fluorophenoxy)-N-{(3S)-4-[2-(3,4-difluorophenoxy)acetamido]-3-hydroxybicyclo[2.2.2]octan-1-yl}acetamide (Compound 587)

The title compound was prepared using the methodologies described in Example 338C substituting Example 276A for Example 338B and 2-(3,4-difluorophenoxy)acetic acid for 2-(4-chlorophenoxy)acetic acid. ¹H NMR (400 MHz, DMSO-*d*₆) *δ* ppm 7.53 - 7.43 (m, 2H), 7.35 (dt, J = 10.6, 9.3 Hz, 1H), 7.22 (s, 1H), 7.12 - 6.98 (m, 2H), 6.79 (dddd, J = 14.1, 8.2, 3.0, 1.4 Hz, 2H), 5.09 (d, J = 4.4 Hz, 1H), 4.43 (d, J = 2.1 Hz, 4H), 4.03 (dt, J = 8.4, 3.6 Hz, 1H), 2.27 (ddd, J = 12.3, 9.4, 2.3 Hz, 1H), 2.15 - 2.03 (m, 1H), 1.97 - 1.86 (m, 2H), 1.91 - 1.72 (m, 6H); MS (ESI⁺) *m*/*z* 513.0 (M+H)⁺.

### Example 489: N,N'-[(2S)-2-hydroxybicyclo[2.2.2]octane-1,4-diyl]bis[2-(3-chloro-4-fluorophenoxy)acetamide] (Compound 588)

The reaction described in Example 442 substituting 2-(3-chloro-4-fluorophenoxy)acetic acid for 2-((6-(trifluoromethyl)pyridin-3-yl)oxy)acetic acid gave the title compound. ¹H NMR (500 MHz, DMSO-*d*₆) *δ* ppm 7.48 (s, 1H), 7.34 (td, J = 9.1, 2.4 Hz, 2H), 7.24 (s, 1H), 7.16 (ddd, J = 14.7, 6.1, 3.1 Hz, 2H), 6.94 (dddd, J = 10.1, 9.2, 3.8, 3.1 Hz, 2H), 5.10 (d, J = 4.4 Hz, 1H), 4.44 (s, 2H), 4.41 (s, 2H), 4.03 (dt, J = 8.4, 3.4 Hz, 1H), 2.27 (ddd, J = 12.5, 9.4, 2.5 Hz, 1H), 2.09 (ddd, J = 12.3, 10.7, 4.5 Hz, 1H), 2.00 - 1.69 (m, 8H); MS (ESI⁺) *m*/*z* 529.1 (M+H)⁺.

### Example 490: 2-(4-chloro-3-fluorophenoxy)-N-[(2S)-2-hydroxy-4-(2-{[6-(trifluoromethyl)pyridin-3-yl]oxy}acetamido)bicyclo[2.2.2]octan-1-yl]acetamide (Compound 589)

The title compound was prepared using the methodologies described in Example 338C substituting 2-((6-(trifluoromethyl)pyridin-3-yl)oxy)acetic acid for 2-(4-chlorophenoxy)acetic acid. ¹H NMR (400 MHz, DMSO-*d*₆) *δ* ppm 7.85 (d, J = 8.7 Hz, 1H), 7.65 (s, 1H), 7.57 7.43 (m, 2H), 7.26 (s, 1H), 7.05 (dd, J = 11.4, 2.8 Hz, 1H), 6.83 (ddd, J = 9.0, 2.9, 1.2 Hz, 1H), 5.08 (s, 1H), 4.62 (s, 2H), 4.47 (s, 2H), 4.04 (dd, J = 9.4, 3.1 Hz, 1H), 2.28 (ddd, J = 12.0, 9.6, 2.2 Hz, 1H), 2.07 (ddd, J = 12.3, 10.5, 4.7 Hz, 1H), 1.98 1.73 (m, 8H); MS (ESI⁺) *m*/*z* 546.1 (M+H)⁺.

### Example 491: 2-(3-chloro-4-fluorophenoxy)-N-{3-[2-(3,4-difluorophenoxy)acetamido]bicyclo[1.1.1]pentan-1-yl}acetamide (Compound 590)

### Example 491A: N-(3-aminobicyclo[1.1.1]pentan-1-yl)-2-(3,4-difluorophenoxy)acetamide

(1-[Bis(dimethylamino)methylene]-1*H*-1,2,3-triazolo[4,5-*b*]pyridinium 3-oxid hexafluorophosphate) (HATU, 823 mg, 2.16 mmol) was added to an *N,N*-dimethylformamide (3 mL) solution of *tert*-butyl (3-aminobicyclo[1.1.1]pentan-1-yl)carbamate (330 mg, 1.66 mmol, CombiBlocks), 2-(3,4-difluorophenoxy)acetic acid (329 mg, 1.75 mmol, CombiBlocks) and triethylamine (0.58 mL, 4.16 mmol) in one portion. After stirring at ambient temperature for 1 hour, the resulting solution was concentrated under reduced pressure, and the residue was taken up in dichloromethane (5 mL). The reaction mixture was chilled to 0 °C and trifluoroacetic acid (2 mL) was slowly added over 1 minute. The resulting solution was stirred at ambient temperature for 1 hour, concentered under reduced pressure, and directly purified by preparative HPLC [YMC TriArt^{™} C18 Hybrid 20 µm column, 25 × 150 mm, flow rate 80 mL/minute, 5-100% gradient of acetonitrile in buffer (0.025 M aqueous ammonium bicarbonate, adjusted to pH 10 with ammonium hydroxide)] to give the title compound (0.45 g, 1.68 mmol, 100% yield). MS (ESI⁺) *m*/*z* 269 (M+H)⁺.

### Example 491B: 2-(3-chloro-4-fluorophenoxy)-N-{3-[2-(3,4-difluorophenoxy)acetamido]bicyclo[1.1.1]pentan-1-yl}acetamide

The reaction and purification conditions described in Example 81, substituting the product of Example 434B for 2-(4-chloro-3-fluorophenoxy)acetic acid, and the product of Example 491A for benzyl (4-aminobicyclo[2.1.1]hexan-1-yl)carbamate hydrochloride gave the title compound. ¹H NMR (400 MHz, DMSO-*d*₆) *δ* ppm 8.70 (s, 2H), 7.42 - 7.31 (m, 2H), 7.20 (dd, J = 6.1, 3.1 Hz, 1H), 7.09 (ddd, J = 12.6, 6.7, 3.0 Hz, 1H), 6.97 (ddd, J = 9.1, 3.8, 3.0 Hz, 1H), 6.80 (dtd, J = 9.2, 3.3, 1.7 Hz, 1H), 4.46 (s, 2H), 4.44 (s, 2H), 2.27 (s, 6H); MS (ESI⁺) *m*/*z* 472 (M+NH₄)⁺.

### Example 492: 2-(3-chloro-4-fluorophenoxy)-N-(3-{2-[(2,2-difluoro-2H-1,3-benzodioxol-5-yl)oxy]acetamidolbicyclo[1.1.1]pentan-1-yl)acetamide (Compound 591)

The reaction and purification conditions described in Example 81, substituting the product of Example 434B for 2-(4-chloro-3-fluorophenoxy)acetic acid, and the product of Example 166B for benzyl (4-aminobicyclo[2.1.1]hexan-1-yl)carbamate hydrochloride gave the title compound. ¹H NMR (400 MHz, DMSO-*d*₆) *δ* ppm 8.69 (s, 2H), 7.38 - 7.30 (m, 2H), 7.20 (dd, J = 6.1, 3.0 Hz, 1H), 7.14 (d, J = 2.5 Hz, 1H), 6.97 (ddd, J = 9.1, 3.8, 3.1 Hz, 1H), 6.77 (dd, J = 8.9, 2.6 Hz, 1H), 4.46 (s, 2H), 4.44 (s, 2H), 2.27 (s, 6H); MS (ESI⁺) *m*/*z* 516 (M+NH₄)⁺.

### Example 493: N,N'-[(2S)-2-hydroxybicyclo[2.2.2]octane-1,4-diyl]bis{2-[4-chloro-3-(hydroxymethyl)phenoxy]acetamide} (Compound 592)

To a solution of Example 495 (0.100 g, 0.164 mmol) in tetrahydrofuran (2 mL) was added 2 M lithium borohydride (0.820 mL, 1.641 mmol) in tetrahydrofuran. The mixture was heated at 40 °C for 3 hours. After cooling, the reaction was quenched with brine and saturated NaHCO₃, and extracted with ethyl acetate (2×). The combined organic layers were washed with brine, dried over MgSO₄, filtered, concentrated. The residue was purified by reverse-phase HPLC (see protocol in Example 112D) to provide the title compound (40.3 mg, 44%). ¹H NMR (400 MHz, DMSO-*d*₆) *δ* ppm 7.47 (s, 1H), 7.29 (dd, J = 8.7, 4.2 Hz, 2H), 7.25 (s, 1H), 7.12 (dd, J = 4.6, 3.2 Hz, 2H), 6.82 (ddd, J = 12.1, 8.7, 3.2 Hz, 2H), 5.55 - 5.03 (m, brd, 3H), 4.51 (d, J = 1.5 Hz, 4H), 4.41 (s, 2H), 4.38 (s, 2H), 4.01 (dd, J = 9.6, 3.2 Hz, 1H), 2.28 (ddd, J = 12.5, 9.5, 2.5 Hz, 1H), 2.14 (td, J = 9.9, 9.0, 5.5 Hz, 1H), 1.99 - 1.71 (m, 8H); MS (ESI⁺) *m*/*z* 553.1 (M+H)⁺.

### Example 494: 3,3'-{[(2S)-2-hydroxybicyclo[2.2.2]octane-1,4-diyl]bis[azanediyl(2-oxoethane-2,1-diyl)oxy]}bis(6-chlorobenzoic acid) (Compound 593)

### Example 494A: (S)-dimethyl 5,5'-((((2-(2-(4-chloro-3-(methoxycarbonyl)phenoxy)acetoxy)bicyclo[2.2.2]octane-1,4-diyl)bis(azanediyl))bis(2-oxoethane-2,1-diyl))bis(oxy))bis(2-chlorobenzoate)

The title compound was isolated in the same step of Example 495 (72.3 mg, 25%) as the less polar compound relative to Example 495. MS (ESI⁺) *m*/*z* 835.0 (M+H)⁺.

### Example 494B: 3,3'-{[(2S)-2-hydroxybicyclo[2.2.2]octane-1,4-diyl]bis[azanediyl(2-oxoethane-2,1-diyl)oxy]}bis(6-chlorobenzoic acid)

A solution of Example 494A (70.0 mg, 0.084 mmol) in tetrahydrofuran (1.5 mL) and methanol (1.0 mL) was treated with a solution of lithium hydroxide (20.05 mg, 0.837 mmol) in water (1.0 mL). The mixture was stirred for 3 hours. The reaction was quenched with brine and 5% citric acid, and extracted with ethyl acetate (2×). The combined organic layers were washed with brine, dried over MgSO₄, filtered, and concentrated. The residue was purified by reverse-phase HPLC (see protocol in Example 112D) to provide the title compound (25.4 mg, 52%). ¹H NMR (400 MHz, DMSO-*d*₆) *δ* ppm 7.53 (s, 1H), 7.44 (dd, J = 8.9, 3.2 Hz, 2H), 7.35 - 7.24 (m, 3H), 7.09 (td, J = 9.3, 3.2 Hz, 2H), 4.47 (s, 2H), 4.44 (s, 2H), 4.02 (dd, J = 9.6, 3.1 Hz, 1H), 2.27 (ddd, J = 12.3, 9.4, 2.3 Hz, 1H), 2.16 - 2.01 (m, 1H), 2.00 - 1.69 (m, 8H); MS (ESI⁺) *m*/*z* 598.0 (M+NH₄)⁺.

### Example 495: dimethyl 3,3'-{[(2S)-2-hydroxybicyclo[2.2.2]octane-1,4-diyl]bis[azanediyl(2-oxoethane-2,1-diyl)oxy]}bis(6-chlorobenzoate) (Compound 594)

A mixture of Example 343D (80.0 mg, 0.349 mmol), (1-[bis(dimethylamino)methylene]-1H-1,2,3-triazolo[4,5-b]pyridinium 3-oxid hexafluorophosphate) (HATU, 345 mg, 0.908 mmol), 2-(4-chloro-3-(methoxycarbonyl)phenoxy)acetic acid (222 mg, 0.908 mmol), and triethylamine (0.243 mL, 1.746 mmol) in N,N-dimethylformamide (5 mL) was stirred for 3 hours. The reaction was quenched with brine and saturated NaHCO₃, and extracted with ethyl acetate (2×). The combined organic layers were washed with brine, dried over MgSO₄, filtered, and concentrated. The residue was purified on a 25 g silica gel column using the Biotage^{®} Isolera^{™} One flash system eluting with heptanes/ethyl acetate (3:7 to 1:9) to give 115 mg (54%) of the title compound as the more polar compound relative to Example 494A. ¹H NMR (500 MHz, DMSO-*d*₆) *δ* ppm 7.54 (s, 1H), 7.48 (dd, J = 8.9, 3.1 Hz, 2H), 7.33 (dd, J = 10.0, 3.1 Hz, 2H), 7.29 (s, 1H), 7.14 (ddd, J = 11.5, 8.9, 3.1 Hz, 2H), 5.08 (d, J = 4.4 Hz, 1H), 4.48 (s, 2H), 4.45 (s, 2H), 4.06 - 4.01 (m, 1H), 3.86 (d, J = 0.5 Hz, 6H), 2.27 (ddd, J = 12.4, 9.4, 2.5 Hz, 1H), 2.09 (ddd, J = 12.5, 10.8, 4.5 Hz, 1H), 1.97 - 1.67 (m, 8H); MS (ESI⁺) *m*/*z* 609.1 (M+H)⁺.

### Example 496: N,N'-[(1R,3s,5S,8r)-bicyclo[3.2.1]octane-3,8-diyl]bis[2-(4-chloro-3-fluorophenoxy)acetamide] (Compound 595)

*tert-*Butyl ((1*R*,3*s*,5*S*,8*r*)-8-aminobicyclo[3.2.1]octan-3-yl)carbamate hydrochloride (52 mg, 0.19 mmol, Curpys Chemicals) was dissolved in dichloromethane (1 mL) and trifluoroacetic acid (1 mL) was added. The resulting solution was stirred at ambient temperature for 30 minutes and then concentrated under reduced pressure. To the resulting residue was added triethylamine (0.16 mL, 1.13 mmol), *N,N*-dimethylformamide (3 mL), 2-(4-chloro-3-fluorophenoxy)acetic acid (81 mg, 0.40 mmol, Aldlab), and (1-[bis(dimethylamino)methylene]-1*H*-1,2,3-triazolo[4,5-b]pyridinium 3-oxid hexafluorophosphate) (HATU, 79 mg, 0.21 mmol) in sequential order. The resulting reaction mixture was stirred at ambient temperature for 30 minutes, filtered through a glass microfiber frit, and purified by preparative HPLC [Waters XBridge^{™} C18 5 µm OBD^{™} column, 30 × 100 mm, flow rate 40 mL/minute, 15-100% gradient of methanol in buffer (0.025 M aqueous ammonium bicarbonate, adjusted to pH 10 with ammonium hydroxide)] to give the title compound (70 mg, 0.14 mmol, 73% yield). ¹H NMR (501 MHz, DMSO-*d*₆) *δ* ppm 7.83 (d, J = 6.4 Hz, 1H), 7.77 (d, J = 8.0 Hz, 1H), 7.49 (td, J = 8.9, 5.8 Hz, 2H), 7.12 (dd, J = 11.4, 2.8 Hz, 1H), 7.08 (dd, J = 11.3, 2.9 Hz, 1H), 6.89 (ddd, J = 9.0, 2.9, 1.2 Hz, 1H), 6.86 (ddd, J = 9.0, 2.9, 1.2 Hz, 1H), 4.63 (s, 2H), 4.52 (s, 2H), 3.95 (tq, J = 12.1, 6.4 Hz, 1H), 3.67 (q, J = 5.2 Hz, 1H), 2.23 - 2.12 (m, 2H), 1.79 - 1.71 (m, 2H), 1.69 - 1.61 (m, 2H), 1.57 - 1.45 (m, 4H); MS (ESI⁺) *m*/*z* 513 (M+H)⁺.

### Example 497: 2-(4-chloro-3-fluorophenoxy)-N-{(3S)-4-[2-(3-chloro-4-fluorophenoxy)acetamido]-3-hydroxybicyclo[2.2.2]octan-1-yl}acetamide (Compound 596)

The title compound was prepared using the methodologies described in Example 338C substituting Example 276A for Example 338B and 2-(3-chloro-4-fluorophenoxy)acetic acid for 2-(4-chlorophenoxy)acetic acid. ¹H NMR (400 MHz, DMSO-*d*₆) *δ* ppm 7.50 7.39 (m, 2H), 7.30 (t, J = 9.1 Hz, 1H), 7.22 7.10 (m, 2H), 6.98 (dd, J = 11.4, 2.8 Hz, 1H), 6.91 (ddd, J = 9.2, 3.8, 3.1 Hz, 1H), 6.77 (ddd, J = 9.0, 2.9, 1.2 Hz, 1H), 5.06 (d, J = 4.4 Hz, 1H), 4.40 (d, J = 3.5 Hz, 4H), 4.09 3.95 (m, 1H), 2.24 (ddd, J = 12.4, 9.7, 2.6 Hz, 1H), 2.11 1.99 (m, 1H), 1.93 1.69 (m, 8H); MS (ESI⁺) *m*/*z* 529.0 (M+H)⁺.

### Example 498: methyl 2-chloro-5-[2-({(3S)-4-[2-(4-chloro-3-fluorophenoxy)acetamido]-3-hydroxybicyclo[2.2.2]octan-1-yl}amino)-2-oxoethoxy]benzoate (Compound 597)

The title compound was prepared using the methodologies described in Example 338C substituting Example 124A for 2-(4-chlorophenoxy)acetic acid. ¹H NMR (400 MHz, DMSO-*d*₆) *δ* ppm 7.51 (s, 1H), 7.50 7.40 (m, 2H), 7.28 (d, J = 3.1 Hz, 1H), 7.22 (s, 1H), 7.09 (dd, J = 8.9, 3.1 Hz, 1H), 7.01 (dd, J = 11.4, 2.9 Hz, 1H), 6.79 (ddd, J = 9.0, 2.9, 1.2 Hz, 1H), 5.01 (s, 1H), 4.42 (d, J = 5.3 Hz, 4H), 4.00 (dd, J = 9.6, 3.1 Hz, 1H), 3.82 (s, 3H), 2.24 (ddd, J = 12.3, 9.5, 2.2 Hz, 1H), 2.03 (ddd, J = 12.3, 10.5, 4.8 Hz, 1H), 1.90 (d, J = 11.7 Hz, 2H), 1.89 1.68 (m, 6H); MS (ESI⁺) *m*/*z* 569.1 (M+H)⁺.

### Example 499: 2-(4-chloro-3-fluorophenoxy)-N-[(2S)-4-{2-[4-chloro-3-(hydroxymethyl)phenoxy]acetamido}-2-hydroxybicyclo[2.2.2]octan-1-yl]acetamide (Compound 598)

The title compound was prepared using the methodologies described in Example 125 substituting Example 498 for Example 124. ¹H NMR (400 MHz, DMSO-*d*₆) *δ* ppm 7.53 7.44 (m, 2H), 7.32 7.23 (m, 2H), 7.14 7.01 (m, 2H), 6.87 6.76 (m, 2H), 5.07 (s, 2H), 4.48 (d, J = 15.1 Hz, 4H), 4.38 (s, 2H), 4.04 (dd, J = 9.6, 3.1 Hz, 1H), 2.27 (ddd, J = 13.1, 9.5, 2.1 Hz, 1H), 2.07 (ddd, J = 12.2, 10.5, 4.7 Hz, 1H), 2.00 1.83 (m, 3H), 1.80 (tdt, J = 10.0, 6.2, 3.0 Hz, 5H); MS (ESI⁺) *m*/*z* 541.3 (M+H)⁺.

### Example 500: N,N'-(2-methyl-3-oxo-2-azabicyclo[2.2.2]octane-1,4-diyl)bis[2-(3,4-dichlorophenoxy)acetamide] (Compound 599)

To a solution of NaH (12.83 mg, 0.321 mmol) in *N,N*-dimethylformamide (5.0 mL) was added Example 501D (120 mg, 0.214 mmol), and the reaction was stirred at ambient temperature for 30 minutes. Then iodomethane (0.020 mL, 0.321 mmol) was added. After two hours, water was added, and the aqueous phase was extracted with dichloromethane. The organic layer was dried over magnesium sulfate and filtered. The filtrate was concentrated, and the residue was purified by reverse phase preparative HPLC (30-100% acetonitrile in 0.1% trifluoroacetic acid/water at 50 mL/minute on a Phenomenex^{®} C18 10 µm 250 mm x 50 mm column) to give 78 mg of the title compound. ¹H NMR (400 MHz, DMSO-*d*₆) *δ* ppm 8.51 (s, 1H), 7.87 (d, J = 1.3 Hz, 1H), 7.55 - 7.43 (m, 2H), 7.21 (ddd, J = 15.2, 3.0, 1.8 Hz, 2H), 7.00 - 6.88 (m, 2H), 4.65 (s, 2H), 4.56 (s, 2H), 2.77 (s, 3H), 2.72 - 2.57 (m, 2H), 2.40 (td, J = 11.8, 3.9 Hz, 2H), 1.80 (td, J = 11.9, 4.2 Hz, 2H), 1.50 - 1.40 (m, 2H); MS (ESI⁺) *m*/*z* 576.1 (M+H)⁺.

### Example 501: N,N'-(3-oxo-2-azabicyclo[2.2.2]octane-1,4-diyl)bis[2-(3,4-dichlorophenoxy)acetamide] (Compound 600)

### Example 501A: methyl 8-amino-1,4-dioxaspiro[4.5]decane-8-carboxylate

To a suspension of 8-amino-1,4-dioxaspiro[4.5]decane-8-carboxylic acid (2.62 g, 13.02 mmol, ArkPharm) in methanol (7.5 mL) and dichloromethane (30 mL), trimethylsilyldiazomethane (9.77 mL, 19.53 mmol) was added at 0 °C, and the mixture was stirred at ambient temperature overnight. The reaction was quenched with acetic acid (2.0 mL) and stirred for 5 minutes. The reaction mixture was partitioned between saturated NaHCO₃ and dichloromethane. The organic layer was dried over magnesium sulfate and filtered. The filtrate was concentrated to give 2.97 g of the title compound which was used without further purification. ¹H NMR (400 MHz, DMSO-*d*₆) *δ* ppm 3.78 (d, J = 0.8 Hz, 4H), 3.56 (s, 3H), 1.88 - 1.75 (m, 2H), 1.75 (d, J = 3.9 Hz, 2H), 1.76 - 1.64 (m, 2H),1.53 - 1.42 (m, 2H), 1.44 - 1.34 (m, 2H); MS (ESI⁺) *m*/*z* 216.1 (M+H)⁺.

### Example 501B: methyl 8-(2-(3,4-dichlorophenoxy)acetamido)-1,4-dioxaspiro[4.5]decane-8-carboxylate

A mixture of Example 501A (1.4 g, 5.53 mmol), 2-(3,4-dichlorophenoxy)acetic acid (1.528 g, 6.91 mmol) and *N*-ethyl-*N*-isopropylpropan-2-amine (2.414 mL, 13.82 mmol) in *N,N-*dimethylformamide (10.0 mL) was treated with 2-(3*H*-[1,2,3]triazolo[4,5-*b*]pyridin-3-yl)-1,1,3,3-tetramethylisouronium hexafluorophosphate(V) (3.15 g, 8.29 mmol), and the reaction mixture was stirred at ambient temperature for 2 hours. The mixture was partitioned between water and dichloromethane. The organic layer was washed with brine, dried over magnesium sulfate and filtered. The filtrate was concentrated and purified by revers phase preparative HPLC (30-100% acetonitrile in 0.1% trifluoroacetic aced/water at 50 mL/minute on Phenomenex^{®} C18 10 µm 250 mm x 50 mm column) to give 1.6 g of the title compound. ¹H NMR (400 MHz, DMSO-*d*₆) *δ* ppm 8.31 (s, 1H), 7.50 (d, J = 8.9 Hz, 1H), 7.14 (d, J = 2.9 Hz, 1H), 6.91 (dd, J = 9.0, 2.9 Hz, 1H), 4.59 (s, 2H), 3.82 (s, 3H), 3.52 (s, 3H), 2.06 - 1.95 (m, 2H), 1.88 (td, J = 13.4, 12.6, 4.5 Hz, 2H), 1.67 - 1.48 (m, 4H); MS (ESI⁺) *m*/*z* 418.0 (M+H)⁺.

### Example 501C: N-(1-amino-3-oxo-2-azabicyclo[2.2.2]octan-4-yl)-2-(3,4-dichlorophenoxy)acetamide

A mixture of Example 501B (0.8 g, 1.913 mmol) and hydrogen chloride (5.0 mL, 5.00 mmol, 1 N solution in water) in acetone (5.0 mL) was stirred at 60 °C for 2 hours. The mixture was concentrated, and water was added. The precipitate was collected by filtration and air-dried to give intermediate methyl 1-(2-(3,4-dichlorophenoxy)acetamido)-4-oxocyclohexane-1-carboxylate (0.67 g). This solid was dissolved in methanol (10 mL) and treated with 7 N ammonia solution in methanol (10.0 mL, 70.0 mmol). The reaction mixture was stirred at ambient temperature overnight. The precipitate was collected by filtration, washed with water, and air-dried to give 0.55 g of the title compound. ¹H NMR (400 MHz, DMSO-*d*₆) *δ* ppm 8.11 (s, 1H), 7.82 (s, 1H), 7.55 (d, J = 8.9 Hz, 1H), 7.27 (d, J = 2.9 Hz, 1H), 6.99 (dd, J = 9.0, 2.9 Hz, 1H), 4.59 (s, 2H), 2.66 (ddd, J = 12.5, 10.7, 4.1 Hz, 2H), 2.26 (s, 2H), 1.77 (td, J = 11.5, 4.2 Hz, 2H), 1.60 (td, J = 11.3, 4.2 Hz, 2H), 1.48 - 1.35 (m, 2H); MS (ESI⁺) *m*/*z* 358.1 (M+H)⁺.

### Example 501D: N,N'-(3-oxo-2-azabicyclo[2.2.2]octane-1,4-diyl)bis[2-(3,4-dichlorophenoxy)acetamide]

A mixture of Example 501C (150 mg, 0.419 mmol), 2-(3,4-dichlorophenoxy)acetic acid (116 mg, 0.523 mmol) and *N*-ethyl-*N*-isopropylpropan-2-amine (0.219 mL, 1.256 mmol) in *N,N-*dimethylformamide (3.0 mL) was treated with 2-(3*H*-[1,2,3]triazolo[4,5-*b*]pyridin-3-yl)-1,1,3,3-tetramethylisouronium hexafluorophosphate(V) (239 mg, 0.628 mmol), and the reaction mixture was stirred at ambient temperature for 30 minutes. The mixture was partitioned between water and dichloromethane. The organic layer was washed with brine, dried over magnesium sulfate and filtered. The filtrate was concentrated, and the residue was purified by preparative reverse phase HPLC (30-100% acetonitrile in 0.1% trifluoroacetic acid/water at 50 mL/minute on Phenomenex^{®} C18 10µm 250 mm x 50 mm column) to give180 mg of the title compound. ¹H NMR (400 MHz, DMSO-*d*₆) *δ* ppm 8.77 (s, 1H), 8.35 (s, 1H), 7.85 (s, 1H), 7.54 (dd, J = 9.8, 8.9 Hz, 2H), 7.28 (t, J = 2.8 Hz, 2H), 7.00 (dt, J = 8.9, 2.8 Hz, 2H), 4.60 (s, 2H), 4.53 (s, 2H), 2.76 - 2.64 (m, 2H), 2.32 - 2.20 (m, 2H), 1.91 (td, J = 11.5, 4.2 Hz, 2H), 1.58 - 1.47 (m, 2H); MS (ESI⁺) *m*/*z* 562.2 (M+H)⁺.

### Example 502: 2-(4-chloro-3-fluorophenoxy)-N-{4-[2-(3,4-dichlorophenoxy)acetamido]-3-oxo-2-azabicyclo[2.2.2]octan-1-yl}acetamide (Compound 601)

The title compound was prepared using the methodologies described in Example 501D substituting 2-(4-chloro-3-fluorophenoxy)acetic acid for 2-(3,4-dichlorophenoxy)acetic acid. ¹H NMR (400 MHz, DMSO-*d*₆) *δ* ppm 8.77 (s, 1H), 8.36 (s, 1H), 7.85 (s, 1H), 7.56 (d, J = 8.9 Hz, 1H), 7.48 (t, J = 8.9 Hz, 1H), 7.28 (d, J = 2.9 Hz, 1H), 7.08 (dd, J = 11.4, 2.9 Hz, 1H), 7.00 (dd, J = 9.0, 2.9 Hz, 1H), 6.86 (ddd, J = 9.0, 2.9, 1.2 Hz, 1H), 4.60 (s, 2H), 4.52 (s, 2H), 2.76 - 2.64 (m, 2H), 2.26 (dt, J = 11.5, 7.1 Hz, 2H), 1.91 (td, J = 11.4, 4.2 Hz, 2H), 1.57 - 1.47 (m, 2H); MS (ESI⁺) *m*/*z* 544.1 (M+H)⁺.

### Example 503: 2-(4-chloro-3-fluorophenoxy)-N-{1-[2-(3,4-dichlorophenoxy)acetamido]-3-oxo-2-azabicyclo[2.2.2]octan-4-yl}acetamide (Compound 602)

### Example 503A: N-(1-amino-3-oxo-2-azabicyclo[2.2.2]octan-4-yl)-2-(4-chloro-3-fluorophenoxy)acetamide

The title compound was prepared using the methodologies described in Example 501A-501C substituting 2-(4-chloro-3-fluorophenoxy)acetic acid for 2-(3,4-dichlorophenoxy)acetic acid in Example 501B. ¹H NMR (400 MHz, DMSO-*d*₆) *δ* ppm 8.11 (s, 1H), 7.82 (s, 1H), 7.50 (t, J = 8.9 Hz, 1H), 7.11 (dd, J = 11.3, 2.9 Hz, 1H), 6.86 (ddd, J = 9.0, 2.9, 1.2 Hz, 1H), 4.58 (s, 2H), 2.67 (ddd, J = 12.5, 10.7, 4.1 Hz, 2H), 1.77 (td, J = 11.5, 4.3 Hz, 2H), 1.67 1.54 (m, 2H), 1.41 (dtd, J = 12.8, 6.7, 6.2, 2.9 Hz, 2H); MS (ESI⁺) *m*/*z* 359.2 (M+H)⁺.

### Example 503B: 2-(4-chloro-3-fluorophenoxy)-N-{1-[2-(3,4-dichlorophenoxy)acetamido]-3-oxo-2-azabicyclo[2.2.2]octan-4-yl}acetamide

The title compound was prepared using the methodologies described in Example 501D substituting Example 503A for Example 501C. ¹H NMR (400 MHz, DMSO-*d*₆) *δ* ppm 8.83 (s, 1H), 8.41 (s, 1H), 7.90 (s, 1H), 7.63 - 7.52 (m, 2H), 7.33 (d, J = 2.9 Hz, 1H), 7.17 (dd, J = 11.3, 2.9 Hz, 1H), 7.06 (dd, J = 9.0, 2.9 Hz, 1H), 6.93 (ddd, J = 9.1, 2.9, 1.2 Hz, 1H), 4.65 (s, 2H), 4.59 (s, 2H), 2.82 - 2.70 (m, 2H), 2.32 (td, J = 11.5, 4.1 Hz, 2H), 1.97 (td, J = 11.4, 4.1 Hz, 2H), 1.64 - 1.53 (m, 2H); MS (ESI⁺) *m*/*z* 544.1 (M+H)⁺.

### Example 504: N,N'-(3-oxo-2-azabicyclo[2.2.2]octane-1,4-diyl)bis[2-(4-chloro-3-fluorophenoxy)acetamide] (Compound 603)

The title compound was prepared using the methodologies described in Example 501D substituting Example 503A for Example 501C and 2-(4-chloro-3-fluorophenoxy)acetic acid for 2-(3,4-dichlorophenoxy)acetic acid. ¹H NMR (400 MHz, DMSO-*d*₆) *δ* ppm 8.30 (s, 1H), 8.00 (d, J = 7.7 Hz, 1H), 7.50 (td, J = 8.9, 6.6 Hz, 2H), 7.10 - 6.95 (m, 2H), 6.89 - 6.77 (m, 2H), 4.61 (s, 2H), 4.51 (s, 2H), 3.68 - 3.54 (m, 1H), 3.55 (s, 3H), 2.16 (d, J = 13.4 Hz, 2H), 1.79 - 1.62 (m, 4H), 1.47 (qd, J = 13.7, 13.3, 3.7 Hz, 2H).; MS (ESI⁺) *m*/*z* 528.1 (M+H)⁺.

### Example 505: 2-(3,4-dichlorophenoxy)-N-(1-{2-[(2,2-difluoro-2H-1,3-benzodioxol-5-yl)oxy]acetamido}-3-oxo-2-azabicyclo[2.2.2]octan-4-yl)acetamide (Compound 604)

The title compound was prepared using the methodologies described in Example 501D substituting 2-((2,2-difluorobenzo[d][1,3]dioxol-5-yl)oxy)acetic acid (Example 166A) for 2-(3,4-dichlorophenoxy)acetic acid. ¹H NMR (400 MHz, DMSO-*d*₆) *δ* ppm 8.77 (s, 1H), 8.34 (s, 1H), 7.85 (s, 1H), 7.56 (d, J = 8.9 Hz, 1H), 7.34 - 7.25 (m, 2H), 7.14 (d, J = 2.5 Hz, 1H), 7.00 (dd, J = 8.9, 3.0 Hz, 1H), 6.77 (dd, J = 8.9, 2.6 Hz, 1H), 4.60 (s, 2H), 4.49 (s, 2H), 2.69 (ddd, J = 12.6, 10.8, 4.1 Hz, 2H), 2.26 (td, J = 11.4, 4.0 Hz, 2H), 1.91 (td, J = 11.5, 4.2 Hz, 2H), 1.58 - 1.47 (m, 2H); MS (ESI⁺) *m*/*z* 572.2 (M+H)⁺.

### Example 506: N,N'-(2-methyl-3-oxo-2-azabicyclo[2.2.2]octane-1,4-diyl)bis[2-(4-chloro-3-fluorophenoxy)acetamide] (Compound 605)

The title compound was prepared using the methodologies described in Example 500 substituting Example 504 for Example 501D. ¹H NMR (400 MHz, DMSO-*d*₆) *δ* ppm 8.54 (s, 1H), 7.91 (s, 1H), 7.50 (td, J = 8.9, 3.9 Hz, 2H), 7.09 (ddd, J = 17.8, 11.3, 2.9 Hz, 2H), 6.86 (dddd, J = 10.2, 9.0, 2.9, 1.2 Hz, 2H), 4.68 (s, 2H), 4.59 (s, 2H), 2.81 (s, 3H), 2.76 2.63 (m, 2H), 2.44 (td, J = 11.5, 4.0 Hz, 2H), 1.84 (td, J = 12.0, 4.2 Hz, 2H), 1.50 (dd, J = 11.1, 4.2 Hz, 2H); MS (ESI⁺) *m*/*z* 542.1 (M+H)⁺.

### Example 507: 2-(4-chloro-3-fluorophenoxy)-N-{(2S)-4-[2-(5-chloropyridin-2-yl)acetamido]-2-hydroxybicyclo[2.2.2]octan-1-yl}acetamide (Compound 606)

The title compound was prepared using the methodologies described in Example 338C substituting 2-(5-chloropyridin-2-yl)acetic acid for 2-(4-chlorophenoxy)acetic acid. ¹H NMR (400 MHz, DMSO-*d*₆) *δ* ppm 8.52 (d, J = 2.6 Hz, 1H), 7.86 (dd, J = 8.4, 2.6 Hz, 1H), 7.71 (s, 1H), 7.48 (t, J = 8.9 Hz, 1H), 7.35 (d, J = 8.4 Hz, 1H), 7.24 (s, 1H), 7.05 (dd, J = 11.4, 2.8 Hz, 1H), 6.82 (ddd, J = 9.0, 2.9, 1.2 Hz, 1H), 4.46 (s, 2H), 4.02 (ddd, J = 9.5, 3.3, 1.2 Hz, 1H), 2.30 - 2.21 (m, 1H), 2.06 (ddd, J = 12.7, 10.9, 4.8 Hz, 1H), 1.96 - 1.70 (m, 8H); MS (ESI⁺) *m*/*z* 496.2 (M+H)⁺.

### Example 508: 2-(4-chloro-3-fluorophenoxy)-N-[(2S)-2-hydroxy-4-{2-[(pyridin-3-yl)oxy]acetamido}bicyclo[2.2.2]octan-1-yl]acetamide (Compound 607)

The title compound isolated as a trifluoroacetic acid salt was prepared using the methodologies described in Example 338C substituting 2-(pyridin-3-yloxy)acetic acid for 2-(4-chlorophenoxy)acetic acid. ¹H NMR (400 MHz, DMSO-*d*₆) *δ* ppm 8.52 (d, J = 2.8 Hz, 1H), 8.43 - 8.37 (m, 1H), 7.82 (ddd, J = 8.7, 2.9, 1.2 Hz, 1H), 7.73 (dd, J = 8.7, 5.1 Hz, 1H), 7.65 (s, 1H), 7.48 (t, J = 8.9 Hz, 1H), 7.28 (s, 1H), 7.05 (dd, J = 11.4, 2.9 Hz, 1H), 6.83 (ddd, J = 9.0, 2.9, 1.2 Hz, 1H), 4.63 (2H), 4.47 (s, 2H), 4.05 (dd, J = 9.5, 3.1 Hz, 1H), 2.28 (ddd, J = 12.1, 9.5, 2.1 Hz, 1H), 2.07 (ddd, J = 12.4, 10.6, 4.6 Hz, 1H), 1.98 -1.73 (m, 8H); MS (ESI⁺) *m*/*z* 496.2 (M+H)⁺.

### Example 509: 2-(4-chloro-3-fluorophenoxy)-N-{(2S)-4-[2-(4-chloro-3-fluorophenyl)acetamido]-2-hydroxybicyclo[2.2.2]octan-1-yl}acetamide (Compound 608)

The title compound was prepared using the methodologies described in Example 338C substituting 2-(4-chloro-3-fluorophenyl)acetic acid for 2-(4-chlorophenoxy)acetic acid. ¹H NMR (400 MHz, DMSO-*d*₆) *δ* ppm 7.65 (s, 1H), 7.44 (td, J = 8.5, 6.4 Hz, 2H), 7.27 - 7.16 (m, 2H), 7.02 (ddd, J = 14.2, 9.8, 2.4 Hz, 2H), 6.78 (dd, J = 8.8, 2.5 Hz, 1H), 4.42 (s, 2H), 3.98 (dd, J = 9.6, 3.1 Hz, 1H), 2.20 (ddd, J = 12.4, 9.5, 2.4 Hz, 1H), 2.00 (ddd, J = 12.4, 10.2, 5.2 Hz, 1H), 1.89 - 1.63 (m, 8H); MS (ESI⁺) *m*/*z* 513.2 (M+H)⁺.

### Example 510: 2-(4-chloro-3-fluorophenoxy)-N-(3-{2-[(4-chlorophenyl)(2-hydroxyethyl)amino]acetamido}bicyclo[1.1.1]pentan-1-yl)acetamide (Compound 609)

To a mixture of the product of Example 104 (37 mg, 0.082 mmol) and 1,4-dioxane-2,5-diol (17 mg, 0.14 mmol, Aldrich) in a buffer solution (3.6 weight % sodium acetate trihydrate and 2.4 weight % acetic acid in methanol, 0.8 mL) was added sodium cyanoborohydride (12.9 mg, 0.21 mmol) portionwise over a period of 1 minute followed by one drop of trifluoroacetic acid and one drop of water. The resulting reaction mixture was left stirring at 30 °C for 18 hours. Additional 1,4-dioxane-2,5-diol (17 mg, 0.15 mmol) and sodium cyanoborohydride (12.9 mg, 0.21 mmol) were added followed by trifluoroacetic acid (0.1 mL). The resulting solution was stirred at 30 °C for 10 minutes and was directly purified by preparative HPLC [YMC TriArt^{™} C18 Hybrid 5 µm column, 50 × 100 mm, flow rate 140 mL/minute, 5-100% gradient of acetonitrile in buffer (0.025 M aqueous ammonium bicarbonate, adjusted to pH 10 with ammonium hydroxide)] to give the title compound (34 mg, 0.07 mmol, 83% yield). ¹H NMR (400 MHz, DMSO-*d*₆) *δ* ppm 8.74 (s, 1H), 8.67 (s, 1H), 7.45 (t, J = 8.9 Hz, 1H), 7.16 - 7.09 (m, 2H), 7.03 (dd, J = 11.4, 2.9 Hz, 1H), 6.81 (ddd, J = 9.0, 2.9, 1.2 Hz, 1H), 6.56 - 6.49 (m, 2H), 4.99 (br s, 1H), 4.43 (s, 2H), 3.83 (s, 2H), 3.52 (d, J = 5.6 Hz, 2H), 3.41 (t, J = 5.7 Hz, 2H), 2.18 (s, 6H); MS (APCI⁺) *m*/*z* 496 (M+H)⁺.

### Example 511: 2-(4-chloro-3-fluorophenoxy)-N-(3-{[(3-ethyl-1,2-oxazol-5-yl)methoxy]methyl}bicyclo[1.1.1]pentan-1-yl)acetamide (Compound 610)

### Example 511A: tert-butyl (3-(((3-ethylisoxazol-5-yl)methoxy)methyl)bicyclo[1.1.1]pentan-1-yl)carbamate

To a solution of *tert-*butyl (3-(hydroxymethyl)bicyclo[1.1.1]pentan-1-yl)carbamate (0.24 g, 1.13 mmol) in tetrahydrofuran (3 mL) at 0 °C was added sodium hydride (0.068 g, 1.69 mmol). The ice-water bath was removed, and the mixture was allowed to stir for 15 minutes. The mixture was then cooled again to 0 °C, and the 5-(chloromethyl)-3-ethylisoxazole (0.16 mL, 1.24 mmol) was added. The mixture was allowed to stir at 0 °C for 20 minutes, then the ice-water bath was removed, and the mixture was allowed to stir for 2 hours. The material was quenched with saturated, aqueous NaHCO₃ (5 mL) and diluted with ethyl acetate (10 mL). The layers were separated, and the aqueous layer was extracted with ethyl acetate (3 × 5 mL). The combined organic fractions were dried over anhydrous Na₂SO₄, filtered, and concentrated under reduced pressure. The residue was purified via column chromatography (SiO₂, 10% ethyl acetate/heptanes to 50% ethyl acetate/heptanes) to give the title compound (45 mg, 0.14 mmol, 12% yield). MS (ESI⁺) *m*/*z* 267.1 (M-*t*-Bu)⁺.

### Example 511B: 3-(((3-ethylisoxazol-5-yl)methoxy)methyl)bicyclo[1.1.1]pentan-1-amine, 2 trifluoroacetic Acid

To a solution of the product of Example 511A (45 mg, 0.14 mmol) in CH₂Cl₂ (2 mL) was added trifluoroacetic acid (1.1 mL, 14 mmol). The mixture was allowed to stir at ambient temperature for 30 minutes. The mixture was concentrated under reduced pressure to give the title compound which was carried on without purification. MS (ESI⁺) *m*/*z* 223 (M+H)⁺.

### Example 511C: 2-(4-chloro-3-fluorophenoxy)-N-(3-{[(3-ethyl-1,2-oxazol-5-yl)methoxy]methyl}bicyclo[1.1.1]pentan-1-yl)acetamide

To a solution of 2-(4-chloro-3-fluorophenoxy)acetic acid (0.037 g, 0.18 mmol) and the product of Example 511B (0.031 g, 0.14 mmol) in *N,N*-dimethylformamide (1.5 mL) was added triethylamine (0.1 mL, 0.70 mmol) followed by 2-(3*H*-[1,2,3]triazolo[4,5-*b*]pyridin-3-yl)-1,1,3,3-tetramethylisouronium hexafluorophosphate(V) (HATU, 0.056 g, 0.18 mmol). The mixture was allowed to stir at ambient temperature for 16 hours and then was quenched with saturated, aqueous NH₄Cl (20 mL), diluted with CH₂Cl₂ (25 mL), and the layers were separated. The aqueous layer was extracted with CH₂Cl₂ (3 × 5 mL), and the combined organic fractions were dried over anhydrous Na₂SO₄, filtered, and concentrated under reduced pressure. The residue was purified by column chromatography (SiO₂, 10% ethyl acetate/heptanes to 80% ethyl acetate/heptanes) to give the title compound (0.035 g, 0.086 mmol, 61% yield). ¹H NMR (400 MHz, DMSO-*d*₆) *δ* ppm 8.61 (s, 1H), 7.45 (t, J = 8.9 Hz, 1H), 7.03 (dd, J = 11.4, 2.8 Hz, 1H), 6.81 (ddd, J = 9.0, 2.8, 1.2 Hz, 1H), 6.36 (s, 1H), 4.52 (s, 2H), 4.42 (s, 2H), 3.52 (s, 2H), 2.58 (q, J = 7.6 Hz, 2H), 1.90 (s, 6H), 1.15 (t, J = 7.6 Hz, 3H); MS (ESI⁺) *m*/*z* 409.1 (M+H)⁺.

### Example 512: 2-(4-chloro-3-fluorophenoxy)-N-{4-[2-(4-chlorophenyl)ethoxy]-2-hydroxybicyclo[2.2.2]octan-1-yl}acetamide (Compound 611)

### Example 512A: ethyl 4-hydroxy-2-oxobicyclo[2.2.2]octane-1-carboxylate

To a solution of ethyl 1-acetyl-4-oxocyclohexanecarboxylate (5.0 g, 23.56 mmol) in ethanol (75 mL), sodium ethoxide (1.759 mL, 4.71 mmol) was added to give a brown solution. After 3 hours stirring at ambient temperature, the reaction mixture was concentrated, and the residue was partitioned between saturated citric acid and ethyl acetate. The organic layer was washed with brine, dried over magnesium sulfate and filtered. The filtrate was concentrated, and the residue was purified on silica gel (0-70% ethyl acetate in heptane) to give 2.5 g of the title compound. ¹H NMR (400 MHz, DMSO-*d*₆) *δ* ppm 4.93 (s, 1H), 4.04 (q, J = 7.1 Hz, 2H), 2.33 (t, J = 1.5 Hz, 2H), 2.11 - 1.92 (m, 2H), 1.86 (ddd, J = 13.8, 11.0, 5.1 Hz, 2H), 1.68 - 1.53 (m, 2H), 1.13 (t, J = 7.1 Hz, 3H); MS (ESI⁺) *m*/*z* 213.1 (M+H)⁺.

### Example 512B: ethyl 4-(4-chlorophenethoxy)-2-oxobicyclo[2.2.2]octane-1-carboxylate

To a mixture of Example 512A (0.52 g, 2.450 mmol), 2,6-di-*tert*-butylpyridine (1.854 mL, 8.58 mmol) and silver trifluoromethanesulfonate (1.889 g, 7.35 mmol) in dichloromethane (75 mL), 1-(2-bromoethyl)-4-chlorobenzene (1.141 mL, 7.84 mmol) was added, and the reaction mixture was stirred at ambient temperature for 4 days. The dark suspension was filtered to remove salt. The filtrate was concentrated, and the residue was purified by preparative reverse phase HPLC (35-100% acetonitrile in 0.1% trifluoroacetic acid/water at 50 mL/minute on a Phenomenex^{®} C18 10 µm 250 mm x 50 mm column) to give 0.61 g of the title compound. ¹H NMR (400 MHz, DMSO-*d*₆) *δ* ppm 7.37 7.19 (m, 4H), 4.04 (q, J = 7.1 Hz, 2H), 3.52 (t, J = 6.8 Hz, 2H), 2.69 (t, J = 6.8 Hz, 2H), 2.42 (s, 2H), 2.08 1.95 (m, 2H), 1.93 1.79 (m, 2H), 1.68 (t, J = 8.2 Hz, 4H), 1.12 (t, J = 7.1 Hz, 3H).

### Example 512C: 4-(4-chlorophenethoxy)-2-oxobicyclo[2.2.2]octane-1-carboxylic acid

A suspension of ethyl 4-(4-chlorophenethoxy)-2-oxobicyclo[2.2.2]octane-1-carboxylate (0.61 g, 1.565 mmol, Example 512B) and sodium hydroxide (3.91 mL, 31.3 mmol) (8 M solution) in methanol (25 mL) was stirred at ambient temperature for 16 hours. The reaction mixture was concentrated, and the residue was acidified with 1 N HCl. The precipitate was collected by filtration and dried in vacuum oven at 50 °C overnight to give 0.33 g of the title compound. ¹H NMR (400 MHz, DMSO-*d*₆) *δ* ppm 12.38 (s, 1H), 7.32 7.19 (m, 4H), 3.52 (t, J = 6.8 Hz, 2H), 2.69 (t, J = 6.8 Hz, 2H), 2.39 (s, 2H), 2.04 1.91 (m, 42H), 1.84 (dq, J = 14.0, 8.1, 7.2 Hz, 2H), 1.66 (t, J = 8.2 Hz, 4H); MS (ESI⁺) *m*/*z* 323.1 (M+H)⁺.

### Example 512D: 1-amino-4-(4-chlorophenethoxy)bicyclo[2.2.2]octan-2-one, Hydrochloric Acid

A mixture of 4-(4-chlorophenethoxy)-2-oxobicyclo[2.2.2]octane-1-carboxylic acid (0.33 g, 1.022 mmol, Example 512C), diphenylphosphoryl azide (0.331 mL, 1.534 mmol) and triethylamine (0.427 mL, 3.07 mmol) in toluene (12 mL) was heated at 110 °C for 2 hours. The solution was cooled to ambient temperature and stirred with 20 mL of 3 N HCl solution overnight. The suspension was filtered to give 0.31 g of the title compound as the hydrochloride salt which was used without further purification. ¹H NMR (400 MHz, DMSO-*d*₆) *δ* ppm 8.35 (s, 3H), 7.37 7.31 (m, 2H), 7.30 7.20 (m, 2H), 3.58 (t, J = 6.8 Hz, 2H), 2.74 (t, J = 6.7 Hz, 2H), 2.63 (s, 2H), 2.04 1.94 (m, 1H), 1.83 (dddd, J = 31.7, 19.9, 11.6, 5.5 Hz, 7H); MS (ESI⁺) *m*/*z* 294.1 (M+H)⁺.

### Example 512E: 2-(4-chloro-3-fluorophenoxy)-N-{4-[2-(4-chlorophenyl)ethoxy]-2-hydroxybicyclo[2.2.2]octan-1-yl}acetamide

A mixture of Example 512D (0.06 g, 0.127 mmol), 2-(4-chloro-3-fluorophenoxy)acetic acid (0.033 g, 0.159 mmol) and *N*-ethyl-*N*-isopropylpropan-2-amine (0.111 mL, 0.636 mmol) in *N,N*-dimethylformamide (1.5 mL) was treated with 2-(3*H*-[1,2,3]triazolo[4,5-*b*]pyridin-3-yl)-1,1,3,3-tetramethylisouronium hexafluorophosphate(V) (0.073 g, 0.191 mmol), and the reaction was stirred at ambient temperature for 16 hours. The reaction was concentrated under reduced pressure, and the residue was dissolved in a mixture of methanol and dichloromethane (5.0 mL 1:1) and treated with sodium borohydride (0.024 g, 0.636 mmol) for 2 hours at ambient temperature. The reaction mixture was concentrated, and the residue was purified by preparative reverse phase HPLC (20-100% acetonitrile in 0.1% trifluoroacetic acid/water at 25 mL/minute on a Phenomenex^{®} C18 5µm (250 mm x 21.2 mm) column) to give 45 mg of the title compound. ¹H NMR (400 MHz, DMSO-*d*₆) *δ* ppm 7.43 (t, J = 8.9 Hz, 1H), 7.31 - 7.16 (m, 5H), 7.00 (dd, J = 11.4, 2.9 Hz, 1H), 6.78 (ddd, J = 9.0, 2.9, 1.2 Hz, 1H), 5.04 (d, J = 4.4 Hz, 1H), 4.41 (s, 2H), 4.01 (ddd, J = 11.3, 5.9, 3.2 Hz, 1H), 3.42 (t, J = 6.8 Hz, 2H), 2.64 (t, J = 6.7 Hz, 2H), 2.05 - 1.90 (m, 2H), 1.94 - 1.80 (m, 2H), 1.85 - 1.70 (m, 1H), 1.63 - 1.33 (m, 5H); MS (ESI⁺) *m*/*z* 428.1 (M+H)⁺.

### Example 513: 2-(4-chloro-3-fluorophenoxy)-N-[3-(phenoxymethyl)bicyclo[1.1.1]pentan-1-yl]acetamide (Compound 612)

### Example 513A: tert-butyl (3-(phenoxymethyl)bicyclo[1.1.1]pentan-1-yl)carbamate

A vial (2 mL) was charged with phenol (20.8 mg, 0.22 mmol), *tert*-butyl (3-(hydroxymethyl)bicyclo[1.1.1]pentan-1-yl)carbamate (44.5 mg, 0.21 mmol, ArkPharm), tetrahydrofuran (0.3 mL) and triphenylphosphine (58.0 mg, 0.22 mmol). The vial was sealed and was sonicated in a sonicator (VWR model 75T) for several minutes to give a clear solution. While still sonicating, diisopropyl azodicarboxylate (0.044 mL, 0.22 mmol) was added dropwise to the reaction mixture over a period of 1 minute. The reaction mixture was sonicated for another 15 minutes. The resulting solution was directly purified by preparative HPLC [YMC TriArt^{™} C18 Hybrid 20 µm column, 25 × 150 mm, flow rate 80 mL/minute, 20-100% gradient of acetonitrile in buffer (0.025 M aqueous ammonium bicarbonate, adjusted to pH 10 with ammonium hydroxide)] to give the title compound (33mg, 0.114 mmol, 54.7% yield). MS (DCI⁺) *m*/*z* 307 (M+NH₄)⁺.

### Example 513B: 3-(phenoxymethyl)bicyclo[1.1.1]pentan-1-amine, trifluoroacetic acid

The product of Example 513A (30 mg, 0.104 mmol) was dissolved in dichloromethane (0.5 mL) and stirred at ambient temperature. Trifluoroacetic acid (1.0 mL) was added in one portion. After stirring at ambient temperature for 10 minutes, the resulting solution was concentrated under reduced pressure to give the title compound (33 mg, 0.11 mmol, 100% yield). MS (DCI⁺) *m*/*z* 190 (M+H)⁺.

### Example 513C: 2-(4-chloro-3-fluorophenoxy)-N-[3-(phenoxymethyl)bicyclo[1.1.1]pentan-1-yl]acetamide

The reaction and purification conditions described in Example 81, substituting the product of Example 513B for benzyl (4-aminobicyclo[2.1.1]hexan-1-yl)carbamate hydrochloride gave the title compound. ¹H NMR (400 MHz, DMSO-*d*₆) *δ* ppm 8.68 (s, 1H), 7.49 (t, J = 8.9 Hz, 1H), 7.30 - 7.23 (m, 2H), 7.07 (dd, J = 11.4, 2.8 Hz, 1H), 6.95 - 6.89 (m, 3H), 6.85 (ddd, J = 8.9, 2.8, 1.2 Hz, 1H), 4.47 (s, 2H), 4.09 (s, 2H), 2.02 (s, 6H); MS (ESI⁺) *m*/*z* 376 (M+H)⁺.

### Example 514: 2-(4-chloro-3-fluorophenoxy)-N-[3-({[5-(difluoromethyl)pyridin-2-yl]oxy}methyl)bicyclo[1.1.1]pentan-1-yl]acetamide (Compound 613)

### Example 514A: tert-butyl (3-(((5-(difluoromethyl)pyridin-2-yl)oxy)methyl)bicyclo[1.1.1]pentan-1-yl)carbamate

To a solution of 2-chloro-5-(difluoromethyl)pyridine (0.051 mL, 0.43 mmol) and *tert-*butyl (3-(hydroxymethyl)bicyclo[1.1.1]pentan-1-yl)carbamate (0.1 g, 0.47 mmol) in tetrahydrofuran (4 mL) at 0 °C was added potassium bis(trimethylsilyl)amide (0.85 mL, 0.85 mmol) (1 M in tetrahydrofuran) dropwise over 3 minutes. The material was allowed to warm to ambient temperature and was allowed to stir for 3 hours. The material was quenched with saturated, aqueous NaHCO₃ (5 mL) and diluted with ethyl acetate (5 mL). The layers were separated, and the aqueous layer was extracted with ethyl acetate (3 × 3 mL). The combined organic fractions were dried over anhydrous Na₂SO₄, filtered, and concentrated under reduced pressure. The residue was purified via column chromatography (SiO₂, 2% ethyl acetate/heptanes to 40% ethyl acetate/heptanes) to give the title compound (85 mg, 0.25 mmol, 59% yield). MS (ESI⁺) *m*/*z* 285.0(M-*t*-Bu)⁺.

### Example 514B: 3-(((5-(difluoromethyl)pyridin-2-yl)oxy)methyl)bicyclo[1.1.1]pentan-1-amine, 2 trifluoroacetic acid

To a solution of the product of Example 517A (85 mg, 0.25 mmol) in CH₂Cl₂ (3 mL) at ambient temperature was added trifluoroacetic acid (0.36 mL, 5 mmol). This mixture was allowed to stir at ambient temperature for 2 hours and then was concentrated under reduced pressure and azeotroped with toluene to give the title compound (117 mg, 0.25 mmol, 100 % yield) which was carried on without characterization.

### Example 514C: 2-(4-chloro-3-fluorophenoxy)-N-[3-({[5-(difluoromethyl)pyridin-2-yl]oxy}methyl)bicyclo[1.1.1]pentan-1-yl]acetamide

To a solution of 2-(4-chloro-3-fluorophenoxy)acetic acid (0.061 g, 0.300 mmol) and the product of Example 517B (0.117 g, 0.25 mmol) in *N,N*-dimethylformamide (3 mL) was added triethylamine (0.17 mL, 1.25 mmol) followed by 2-(3*H*-[1,2,3]triazolo[4,5-*b*]pyridin-3-yl)-1,1,3,3-tetramethylisouronium hexafluorophosphate(V) (HATU, 0.10 g, 0.26 mmol). The mixture was allowed to stir at ambient temperature for 16 hours then was quenched with saturated, aqueous NH₄Cl (20 mL), diluted with CH₂Cl₂ (25 mL), and the layers were separated. The aqueous layer was extracted with CH₂Cl₂ (3 × 5 mL), and the combined organic fractions were dried over anhydrous Na₂SO₄, filtered, and concentrated under reduced pressure. The residue was purified by column chromatography (SiO₂, 10% ethyl acetate/heptanes to 80% ethyl acetate/heptanes) to give the title compound (34.3 mg, 0.080 mmol, 32% yield).). ¹H NMR (500 MHz, DMSO-*d*₆) *δ* ppm 8.67 (s, 1H), 8.35 (d, J = 2.1 Hz, 1H), 7.89 (dd, J = 8.7, 2.5 Hz, 1H), 7.48 (t, J = 8.9 Hz, 1H), 7.17 - 6.91 (m, 3H), 6.83 (ddd, J = 9.0, 2.9, 1.2 Hz, 1H), 4.45 (s, 2H), 4.43 (s, 2H), 1.98 (s, 6H); MS (ESI⁺) *m*/*z* 427 (M+H)⁺.

### Example 515: 2-(4-chlorophenoxy)-N-{(3S)-4-[2-(4-fluorophenoxy)acetamido]-3-hydroxybicyclo[2.2.2]octan-1-yl}acetamide (Compound 614)

### Example 515A: (S)-tert-butyl (4-(2-(4-chlorophenoxy)acetamido)-2-hydroxybicyclo[2.2.2]octan-1-yl)carbamate

A mixture of Example 445B (0.200 g, 0.683 mmol), 1-[bis(dimethylamino)methylene]-1*H*-1,2,3-triazolo[4,5-*b*]pyridinium 3-oxid hexafluorophosphate (HATU, 0.312 g, 0.820 mmol), 2-(4-chlorophenoxy)acetic acid (0.140 g, 0.751 mmol), and triethylamine (0.238 mL, 1.708 mmol) in *N,N*-dimethylformamide (3 mL) was stirred for 3 hours. The reaction mixture was quenched with brine and extracted with ethyl acetate (2×). The combined organic layers were washed with brine, dried over MgSO₄, filtered, and concentrated. The residue was purified on a 12 g silica gel column using the Biotage^{®} Isolera^{™} One flash system eluted with heptanes/ethyl acetate (5:5 to 4:6) to provide the title compound (0.192 g, 66%). MS (ESI⁺) *m*/*z* 423.1 (M+H)⁺.

### Example 515B: (S)-N-(4-amino-3-hydroxybicyclo[2.2.2]octan-1-yl)-2-(4-chlorophenoxy)acetamide, hydrochloric acid

A mixture of Example 515A (0.185 g, 0.435 mmol) and trifluoroacetic acid (0.335 mL, 4.35 mmol) in CH₂Cl₂ (4 mL) was stirred for 5 hours. The reaction mixture was concentrated, and the residue was dissolved in methanol (2 mL). The solution was treated with 2 M HCl (2 mL) in ether and stirred for 15 minutes. The solution was concentrated to provide the title compound (0.157 g, 100%). This material was used in the next step without further purification. MS (ESI⁺) *m*/*z* 325.3 (M+H)⁺.

### Example 515C: 2-(4-chlorophenoxy)-N-{(3S)-4-[2-(4-fluorophenoxy)acetamido]-3-hydroxybicyclo[2.2.2]octan-1-yl}acetamide

A mixture of Example 515B (50.0 mg, 0.138 mmol), 1-[bis(dimethylamino)methylene]-1*H*-1,2,3-triazolo[4,5-*b*]pyridinium 3-oxid hexafluorophosphate) (HATU, 63.2 mg, 0.166 mmol), 2-(4-fluorophenoxy)acetic acid (25.9 mg, 0.152 mmol), and triethylamine (0.048 mL, 0.346 mmol) in *N,N*-dimethylformamide (2 mL) was stirred for 3 hours. The reaction mixture was quenched with brine and extracted with ethyl acetate (2×). The combined organic layers were washed with brine, dried over MgSO₄, filtered, and concentrated. The residue was purified by HPLC performed on a Phenomenex^{®} Luna^{®}-C18 column (250 × 30 mm, 10 µm particle size) using a gradient of 20% to 100% acetonitrile:0.1% aqueous trifluoroacetic acid over 26 minutes at a flow rate of 50 mL/minute to provide the title compound (34.7 mg, 53%). ¹H NMR (400 MHz, DMSO-*d*₆) *δ* ppm 7.46 (s, 1H), 7.37 - 7.29 (m, 2H), 7.20 (s, 1H), 7.17 - 7.06 (m, 2H), 7.00 - 6.87 (m, 4H), 5.13 (d, J = 4.5 Hz, 1H), 4.39 (s, 4H), 4.00 (dt, J = 8.9, 3.8 Hz, 1H), 2.28 (ddd, J = 12.5, 9.5, 2.5 Hz, 1H), 2.14 (td, J = 9.9, 9.0, 5.9 Hz, 1H), 1.97 - 1.67 (m, 8H); MS (ESI⁺) *m*/*z* 477.2 (M+H)⁺.

### Example 516: N,N'-[(2S)-2-hydroxybicyclo[2.2.2]octane-1,4-diyl]bis[2-(3,4-difluorophenoxy)acetamide] (Compound 615)

A mixture of Example 343D (0.040 g, 0.175 mmol), 1-[bis(dimethylamino)methylene]-1*H*-1,2,3-triazolo[4,5-*b*]pyridinium 3-oxid hexafluorophosphate) (HATU, 0.173 g, 0.454 mmol), 2-(3,4-difluorophenoxy)acetic acid (0.085 g, 0.454 mmol), and triethylamine (0.122 mL, 0.873 mmol) in *N,N*-dimethylformamide (3 mL) was stirred for 3 hours. The reaction mixture was quenched with brine and extracted with ethyl acetate (2×). The combined organic layers were washed with brine, dried over MgSO₄, filtered, and concentrated. The residue was purified by HPLC (see protocol in Example 515C) to provide the title compound (47.5 mg, 55%). ¹H NMR (500 MHz, DMSO-*d*₆) *δ* ppm 7.47 (s, 1H), 7.35 (dtd, J = 11.5, 9.3, 2.3 Hz, 2H), 7.24 (s, 1H), 7.05 (dddd, J = 15.3, 12.6, 6.7, 3.0 Hz, 2H), 6.76 (ddtd, J = 10.9, 9.1, 3.2, 1.6 Hz, 2H), 5.10 (d, J = 4.4 Hz, 1H), 4.43 (s, 2H), 4.40 (s, 2H), 4.03 (d, J = 9.1 Hz, 1H), 2.28 (ddd, J = 12.6, 9.4, 2.5 Hz, 1H), 2.09 (ddd, J = 12.5, 10.7, 4.6 Hz, 1H), 1.99 - 1.71 (m, 8H); MS (ESI⁺) *m*/*z* 497.1 (M+H)⁺.

### Example 517: 2-(4-chlorophenoxy)-N-{(2S)-4-[2-(4-fluorophenoxy)acetamido]-2-hydroxybicyclo[2.2.2]octan-1-yl}acetamide (Compound 616)

### Example 517A: (S)-tert-butyl (4-(2-(4-fluorophenoxy)acetamido)-2-hydroxybicyclo[2.2.2]octan-1-yl)carbamate

The reaction described in Example 515A substituting 2-(4-fluorophenoxy)acetic acid for 2-(4-chlorophenoxy)acetic acid gave the title compound. MS (ESI⁺) *m*/*z* 407.1 (M+H)⁺.

### Example 517B: (S)-N-(4-amino-3-hydroxybicyclo[2.2.2]octan-1-yl)-2-(4-fluorophenoxy)acetamide, hydrochloric acid

The reaction described in Example 515B substituting Example 517A for Example 515A gave the title compound. MS (ESI⁺) *m*/*z* 309.3 (M+H)⁺.

### Example 517C: 2-(4-chlorophenoxy)-N-{(2S)-4-[2-(4-fluorophenoxy)acetamido]-2-hydroxybicyclo[2.2.2]octan-1-yl}acetamide

The reaction described in Example 515C substituting Example 517B and 2-(4-chlorophenoxy)acetic acid for Example 515B and 2-(4-fluorophenoxy)acetic acid, respectively, gave the title compound. ¹H NMR (400 MHz, DMSO-*d*₆) *δ* ppm 7.41 (s, 1H), 7.37 - 7.30 (m, 2H), 7.23 (s, 1H), 7.17 - 7.05 (m, 2H), 6.99 - 6.86 (m, 4H), 5.11 (d, J = 4.5 Hz, 1H), 4.42 (s, 2H), 4.36 (s, 2H), 4.01 (dt, J = 8.3, 3.6 Hz, 1H), 2.28 (ddd, J = 12.4, 9.4, 2.4 Hz, 1H), 2.17 - 2.04 (m, 1H), 1.98 - 1.71 (m, 8H); MS (ESI⁺) *m*/*z* 477.2 (M+H)⁺.

### Example 518: 2-(3,4-difluorophenoxy)-N-{(2S)-4-[2-(4-fluorophenoxy)acetamido]-2-hydroxybicyclo[2.2.2]octan-1-yl}acetamide (Compound 617)

A mixture of Example 517B (60.0 mg, 0.174 mmol), 1-[bis(dimethylamino)methylene]-1*H*-1,2,3-triazolo[4,5-*b*]pyridinium 3-oxid hexafluorophosphate (HATU, 79 mg, 0.209 mmol), 2-(3,4-difluorophenoxy)acetic acid (36.0 mg, 0.191 mmol), and triethylamine (0.061 mL, 0.435 mmol) in *N,N*-dimethylformamide (2 mL) was stirred for 3 hours. The reaction mixture was concentrated. The residue was treated with tetrahydrofuran (1 mL), and a solution of lithium hydroxide (16.67 mg, 0.696 mmol) in water (1 mL). The mixture was stirred for 1 hour. The reaction mixture was quenched with brine and extracted with ethyl acetate (2×). The combined organic layers were washed with brine, dried over MgSO₄, filtered, and concentrated. The residue was purified by HPLC (see protocol in Example 515C) to provide the title compound (44.8 mg, 54%). ¹H NMR (400 MHz, DMSO-*d*₆) *δ* ppm 7.45 - 7.29 (m, 2H), 7.23 (s, 1H), 7.17 - 7.02 (m, 3H), 6.98 - 6.89 (m, 2H), 6.77 (dtd, J = 8.7, 3.3, 1.7 Hz, 1H), 5.09 (s, brd, 1H), 4.43 (s, 2H), 4.36 (s, 2H), 4.03 (dd, J = 9.7, 3.1 Hz, 1H), 2.28 (ddd, J = 12.3, 9.5, 2.3 Hz, 1H), 2.16 - 2.03 (m, 1H), 1.99 - 1.69 (m, 8H); MS (ESI⁺) *m*/*z* 479.1 (M+H)⁺.

### Example 519: 2-(4-chlorophenoxy)-N-{(3S)-4-[2-(3,4-difluorophenoxy)acetamido]-3-hydroxybicyclo[2.2.2]octan-1-yl}acetamide (Compound 618)

The reaction described in Example 518 substituting Example 515B for Example 517B gave the title compound. ¹H NMR (501 MHz, DMSO-*d*₆) *δ* ppm 7.46 (s, 1H), 7.41 - 7.28 (m, 3H), 7.23 (s, 1H), 7.07 (ddd, J = 12.6, 6.7, 3.1 Hz, 1H), 6.97 - 6.88 (m, 2H), 6.77 (dtd, J = 9.2, 3.2, 1.7 Hz, 1H), 5.09 (s, brd, 1H), 4.43 (s, 2H), 4.39 (s, 2H), 4.03 (dd, J = 9.8, 3.1 Hz, 1H), 2.27 (ddd, J = 12.5, 9.4, 2.4 Hz, 1H), 2.09 (ddd, J = 12.4, 10.7, 4.7 Hz, 1H), 1.97 - 1.69 (m, 8H); MS (ESI⁺) *m*/*z* 495.2 (M+H)⁺.

### Example 520: 2-(4-chloro-3-fluorophenoxy)-N-{(2S)-4-[2-(4-fluorophenoxy)acetamido]-2-hydroxybicyclo[2.2.2]octan-1-yl}acetamide (Compound 619)

A mixture of Example 338B (50.0 mg, 0.146 mmol), 1-[bis(dimethylamino)methylene]-1*H*-1,2,3-triazolo[4,5-*b*]pyridinium 3-oxid hexafluorophosphate (HATU, 66.6 mg, 0.175 mmol), 2-(4-fluorophenoxy)acetic acid (27.3 mg, 0.160 mmol), and triethylamine (0.051 mL, 0.365 mmol) in *N,N*-dimethylformamide (2 mL) was stirred for 3 hours. The reaction mixture was concentrated. The residue was treated with tetrahydrofuran (1mL) and a solution of lithium hydroxide (13.97 mg, 0.583 mmol) in water (1 mL). The mixture was stirred for 2 hours. The reaction mixture was quenched with brine and extracted with ethyl acetate (2×). The combined organic layers were washed with brine, dried over MgSO₄, filtered, and concentrated. The residue was purified by HPLC (see protocol in Example 515C) to provide the title compound (43.4, 60%). ¹H NMR (500 MHz, DMSO-*d*₆) *δ* ppm 7.48 (t, J = 8.9 Hz, 1H), 7.41 (s, 1H), 7.26 (s, 1H), 7.15 - 7.08 (m, 2H), 7.05 (dd, J = 11.4, 2.9 Hz, 1H), 6.97 - 6.88 (m, 2H), 6.83 (ddd, J = 9.0, 2.9, 1.1 Hz, 1H), 5.06 (s, brd, 1H), 4.47 (s, 2H), 4.36 (s, 2H), 4.04 (dd, J = 9.7, 3.2 Hz, 1H), 2.27 (ddd, J = 12.3, 9.5, 2.3 Hz, 1H), 2.07 (ddd, J = 12.9, 11.0, 4.7 Hz, 1H), 1.98 - 1.72 (m, 8H); MS (ESI⁺) *m*/*z* 495.2 (M+H)⁺.

### Example 521: N,N'-[(1R,2S,4R,5S)-bicyclo[2.2.1]heptane-2,5-diyl]bis[2-(4-chloro-3-fluorophenoxy)acetamide] (Compound 620)

The title compound was isolated by chiral preparative SFC (Supercritical Fluid Chromatography) of Example 529D as the second peak eluted off the column. Preparative SFC was performed on a THAR/Waters SFC 80 system running under SuperChrom^{™} software control. The preparative SFC system was equipped with an 8-way preparative column switcher, CO₂ pump, modifier pump, automated back pressure regulator (ABPR), UV detector, and 6-position fraction collector. The mobile phase was comprised of supercritical CO₂ supplied by a Dewar of bone-dry non-certified CO₂ pressurized to 350 psi with a modifier of methanol at a flow rate of 80 g/minute. The column was at ambient temperature, and the backpressure regulator was set to maintain 100 bar. The sample was dissolved in a mixture of methanol/dichloromethane (1:1) at a concentration of 6.75 mg/mL. The sample was loaded into the modifier stream in 1 mL injections. The mobile phase was held isocratically at 55 % methanol: CO₂. Fraction collection was time triggered. The instrument was fitted with a Chiralcel^{®} OZ-H column with dimensions 30 mm i.d. × 250 mm length with 5 µm particles. The absolute stereochemistry assignment was arbitrary. ¹H NMR (400 MHz, DMSO-*d*₆) *δ* ppm 7.91 (d, J = 6.9 Hz, 2H), 7.48 (t, J = 8.9 Hz, 2H), 7.05 (dd, J = 11.4, 2.9 Hz, 2H), 6.84 (ddd, J = 9.0, 2.9, 1.2 Hz, 2H), 4.49 (s, 4H), 3.53 (td, J = 7.6, 3.5 Hz, 2H), 2.10 (d, J = 4.6 Hz, 2H), 1.60 (dd, J = 13.3, 8.1 Hz, 2H), 1.45 - 1.28 (m, 4H); MS (ESI⁺) *m*/*z* 499.2 (M+H)⁺.

### Example 522: N,N'-[(1S,2R,4S,5R)-bicyclo[2.2.1]heptane-2,5-diyl]bis[2-(4-chloro-3-fluorophenoxy)acetamide] (Compound 621)

The title compound was isolated by chiral preparative SFC (Supercritical Fluid Chromatography) of Example 529D as the first peak eluted off the column. The absolute stereochemistry assignment is arbitrary. ¹H NMR (501 MHz, DMSO-*d*₆) *δ* ppm 7.92 (d, J = 6.9 Hz, 2H), 7.48 (t, J = 8.9 Hz, 2H), 7.05 (dd, J = 11.4, 2.8 Hz, 2H), 6.84 (ddd, J = 9.0, 2.9, 1.2 Hz, 2H), 4.49 (d, J = 1.1 Hz, 4H), 3.53 (td, J = 7.6, 3.6 Hz, 2H), 2.23 - 1.99 (m, 2H), 1.60 (dd, J = 13.3, 8.1 Hz, 2H), 1.45 - 1.28 (m, 4H); MS (ESI⁺) *m*/*z* 499.3 (M+H)⁺.

### Example 523: methyl 5-[2-({(3S)-4-[2-(4-chloro-3-fluorophenoxy)acetamido]-3-hydroxybicyclo[2.2.2]octan-1-yl}amino)-2-oxoethoxy]pyrimidine-2-carboxylate (Compound 622)

The title compound was synthesized as a byproduct of the reaction described in Example 524 using the reaction conditions described in Example 538. ¹H NMR (400 MHz, DMSO-*d*₆) *δ* ppm 8.61 (s, 2H), 7.70 (s, 1H), 7.48 (t, J = 8.9 Hz, 1H), 7.26 (s, 1H), 7.05 (dd, J = 11.4, 2.8 Hz, 1H), 6.83 (ddd, J = 9.0, 3.0, 1.2 Hz, 1H), 4.72 (s, 2H), 4.46 (s, 2H), 4.04 (dd, J = 9.5, 3.0 Hz, 1H), 3.88 (s, 3H), 2.28 (ddd, J = 12.4, 9.5, 2.2 Hz, 1H), 2.07 (ddd, J = 12.3, 10.5, 4.8 Hz, 1H), 1.97 1.90 (m, 2H), 1.90 1.82 (m, 1H), 1.86 1.73 (m, 5H); MS (ESI⁺) *m*/*z* 537.3 (M+H)⁺.

### Example 524: 2-(4-chloro-3-fluorophenoxy)-N-[(2S)-4-{2-[(2-cyanopyrimidin-5-yl)oxy]acetamido}-2-hydroxybicyclo[2.2.2]octan-1-yl]acetamide (Compound 623)

The title compound was synthesized using the procedure described in Example 538 substituting 5-hydroxypyrimidine-2-carbonitrile for 2-methoxypyrimidin-5-ol. ¹H NMR (400 MHz, DMSO-*d*₆) *δ* ppm 8.68 (s, 2H), 7.72 (s, 1H), 7.58 7.42 (m, 1H), 7.27 (s, 1H), 7.05 (dd, J = 11.4, 2.8 Hz, 1H), 6.83 (ddd, J = 9.0, 2.9, 1.2 Hz, 1H), 4.76 (s, 2H), 4.46 (s, 2H), 4.04 (dd, J = 9.6, 3.1 Hz, 1H), 2.27 (ddd, J = 12.5, 9.5, 2.3 Hz, 1H), 2.06 (ddd, J = 12.3, 10.6, 4.7 Hz, 1H), 1.83 (s, 1H), 1.96 1.72 (m, 7H); MS (ESI⁺) *m*/*z* 504.3 (M+H)⁺.

### Example 525: 2-(4-chloro-3-fluorophenoxy)-N-[(2S)-4-{2-[(2-ethoxypyrimidin-5-yl)oxy]acetamido}-2-hydroxybicyclo[2.2.2]octan-1-yl]acetamide (Compound 624)

### Example 525A: 5-bromo-2-ethoxypyrimidine

To a solution of 5-bromo-2-chloropyrimidine (0.500 g, 2.58 mmol) in tetrahydrofuran (10 mL) was added ethanol (1.207 mL, 20.68 mmol) and potassium carbonate (2.86 g, 20.68 mmol). The mixture was heated to 50 °C overnight followed by 60 °C overnight, and at 65 °C for one more day. The suspension was quenched with water and extracted with ethyl acetate (2×). The combined organic layers were washed with brine, dried over MgSO₄, filtered, and concentrated. The residue was purified on a 40 g column using the Biotage^{®} Isolera^{™} One flash system eluted with heptanes/ethyl acetate (95:5 to 85:15) to provide the title compound (0.336g, 64%). MS (DCI⁺) *m*/*z* 203.1 (M+H)⁺.

### Example 525B: 2-ethoxy-5-(4,4, 5, 5-tetramethyl-1,3, 2-dioxaborolan-2-yl)pyrimidine

A mixture of Example 525A (325.0 mg, 1.601 mmol), bis(pinacolato)diboron (813 mg, 3.20 mmol), potassium acetate (471 mg, 4.80 mmol), and [1,1'-bis(diphenylphosphino)ferrocene]dichloropalladium(II), complex with dichloromethane (PdCl₂(dppf)-CH₂Cl₂, 52.3 mg, 0.064 mmol) in dioxane (12 mL) was degassed and heated to 100 °C overnight. The solvent was evaporated. The residue was treated with brine and extracted with ethyl acetate (2×). The combined organic layers were dried over MgSO₄, filtered, and concentrated. The residue was purified on a 40 g column using the Biotage^{®} Isolera^{™} One flash system eluting with heptanes/ethyl acetate (4:6 to 5:5) to provide the title compound (0.343 g, 86%). MS (ESI⁺) *m*/*z* 251.3 (M+H)⁺.

### Example 525C: 2-ethoxypyrimidin-5-ol

A solution of Example 525B (0.230 g, 0.920 mmol) in acetone (5 mL) was treated with a solution of Oxone^{®} (potassium peroxomonosulfate) (0.791 g, 1.287 mmol) in water (5 mL) at 0 °C. The reaction mixture was stirred at 0 °C for 1 hour and at room temperature for 2.5 hours. The reaction vial was left uncapped overnight to concentrate the reaction mixture. The remaining solution was treated with aqueous Na₂S₂O₃ and extracted with ethyl acetate (2×). The combined organic layers were washed with water, dried over MgSO₄, filtered, and concentrated. The residue was purified by HPLC (see protocol in Example 529D, 10 to 90% acetonitrile) to provide the title compound (44.6 mg, 35%). MS (DCI⁺) *m*/*z* 141.1 (M+H)⁺.

*Example 525D: 2-(4-chloro-3-fluorophenoxy)-N-[(2S)-4-{2-[(2-ethoxypyrimidin-5-yl)oxy]acetamido}-2-hydroxybicyclo[2.2.2]octan-1-yl]acetamide trifluoroacetic acid saltA* mixture of Example 476A (70.0 mg, 0.167 mmol), Example 525C (35.1 mg, 0.250 mmol), potassium carbonate (46.1 mg, 0.334 mmol) and potassium iodide (1.386 mg, 8.35 µmol) in acetone (2.5 mL) was heated at 140 °C in a Biotage^{®} Initiator microwave reactor for 40 minutes. The reaction mixture was filtered. The filtrate was concentrated, and the residue was purified by HPLC (see protocol in Example 529D) to provide the title compound (59.4 mg, 68%). ¹H NMR (400 MHz, DMSO-*d*₆) *δ* ppm 8.30 (s, 2H), 7.54 (s, 1H), 7.48 (t, J = 8.9 Hz, 1H), 7.27 (s, 1H), 7.05 (dd, J = 11.4, 2.8 Hz, 1H), 6.89 - 6.69 (m, 1H), 4.47 (d, J = 5.2 Hz, 4H), 4.28 (q, J = 7.0 Hz, 2H), 4.04 (dd, J = 9.6, 3.1 Hz, 1H), 2.27 (ddd, J = 12.3, 9.5, 2.3 Hz, 1H), 2.07 (ddd, J = 12.4, 10.5, 4.8 Hz, 1H), 1.99 - 1.69 (m, 8H), 1.31 (t, J = 7.0 Hz, 3H); MS (ESI⁺) *m*/*z* 523.2 (M+H)⁺.

### Example 526: rac-N,N'-[(1R,3R,4R,5R)-3-hydroxybicyclo[3.2.1]octane-1,4-diyl]bis[2-(4-chloro-3-fluorophenoxy)acetamide] (Compound 625)

### Example 526A: N,N'-((1S,5R)-bicyclo[3.2.1]oct-3-ene-1,4-diyl)bis(2-(4-chloro-3-fluorophenoxy)acetamide)

To a suspension of Example 198 (0.4002 g, 0.756 mmol) and pyridine (0.40 mL, 4.96 mmol) at ambient temperature was added a 1 M solution of trifluoromethanesulfonic anhydride in CH₂Cl₂ (1.0 mL, 1.000 mmol) and the mixture was stirred for 30 minutes. Then the mixture was diluted with ethyl acetate, washed with water and brine, and concentrated. The residue was chromatographed on silica gel (15% ethyl acetate/dichloromethane) to give the title compound (0.2917 g, 0.570 mmol, 75 % yield). ¹H NMR (400 MHz, DMSO-*d*₆) *δ* ppm 9.07 (s, 1H), 8.07 (s, 1H), 7.48 (td, J = 8.9, 2.4 Hz, 2H), 7.06 (ddd, J = 11.4, 6.4, 2.9 Hz, 2H), 6.83 (ddt, J = 9.0, 2.8, 1.4 Hz, 2H), 5.83 (t, J = 3.9 Hz, 1H), 4.59 (d, J = 1.1 Hz, 2H), 4.48 (s, 2H), 2.63 - 2.53 (m, 1H), 2.35 (dd, J = 17.0, 4.6 Hz, 1H), 2.05 - 1.70 (m, 6H); LC/MS (APCI⁺) *m*/*z* 511 (M+H)⁺.

### Example 526B: N,N'-[(1R,3R,4R,5R)-3-hydroxybicyclo[3.2.1]octane-1,4-diyl]bis[2-(4-chloro-3-fluorophenoxy)acetamide]

To a solution of Example 526A (0.0970 g, 0.190 mmol) in tetrahydrofuran (THF) (1.0 mL) at -78 °C was added borane dimethyl sulfide complex (0.027 mL, 0.284 mmol), and the mixture was stirred for 15 minutes, allowed to warm to ambient temperature, and stirred for 4 hours. The reaction mixture was then cooled to 0 °C, quenched with ethanol (0.32 mL), 1 N NaOH (0.32 mL), and 30% H₂O₂ (0.32 mL), stirred for 5 minutes, allowed to warm, and stirred for 1 hour. The mixture was then diluted with ethyl acetate, washed with water and brine, dried (Na₂SO₄), and concentrated. The residue was chromatographed on silica gel (5% isopropanol/dichloromethane) to give the title compound (0.0335 g, 0.063 mmol, 33.4 % yield). ¹H NMR (501 MHz, DMSO-*d*₆) *δ* ppm 8.05 (s, 1H), 8.00 (d, J = 7.2 Hz, 1H), 7.48 (td, J = 8.9, 1.6 Hz, 2H), 7.05 (ddd, J = 12.2, 11.4, 2.8 Hz, 2H), 6.84 (dddd, J = 14.0, 9.0, 2.9, 1.2 Hz, 2H), 4.66 (d, J = 5.8 Hz, 1H), 4.57 - 4.49 (m, 2H), 4.46 (s, 2H), 3.57 - 3.44 (m, 2H), 2.22 (ddd, J = 12.2, 5.8, 2.8 Hz, 1H), 2.13 - 2.07 (m, 1H), 1.89 - 1.81 (m, 1H), 1.80 - 1.75 (m, 1H), 1.73 - 1.58 (m, 3H), 1.58 - 1.50 (m, 2H).; MS (ESI⁻) *m*/*z* 526.9 [M-H]⁻.

### Example 527: N,N'-[(2S)-2-hydroxybicyclo[2.2.2]octane-1,4-diyl]bis{2-[(2-methoxypyrimidin-5-yl)oxy]acetamide} (Compound 626)

### Example 527A: 2-((2-methoxypyrimidin-5-yl)oxy)acetic acid

To a solution of 2-methoxypyrimidin-5-ol (6.1 g, 48.4 mmol) in *N,N*-dimethylformamide (50 mL) at ambient temperature was added potassium carbonate (13.37 g, 97 mmol) and *tert-*butyl bromoacetate (8.16 mL, 55.6 mmol). This suspension was then stirred at 65 °C for 1.5 hours. The reaction mixture was cooled to ambient temperature and was diluted with ethyl acetate (50 mL) and H₂O (50 mL). The aqueous layer was extracted with ethyl acetate (3 × 100 mL). The combined organic layers were dried over anhydrous Na₂SO₄ and filtered. The filtrate was concentrated under reduced pressure to give *tert-*butyl 2-((2-methoxypyrimidin-5-yl)oxy)acetate as a crude oil, which was used without further purification. This crude was dissolved in methanol (90 mL) and water (30.0 mL), to this mixture was added NaOH (48.0 mL, 240 mmol) (5 M aqueous solution). This reaction mixture was stirred at ambient temperature for 12 hours. The mixture was concentrated under reduced pressure to give a solid. The crude solid was then dissolved in water (200 mL) and the pH was adjusted to ~1 with 1 N aqueous HCl. The resulting precipitate was isolated by filtration, washed with water and air-dried to give the title compound (5.9 g, 32.0 mmol, 66.7 % yield). ¹H NMR (501 MHz, DMSO-*d*₆) *δ* ppm 8.37 (s, 2H), 4.79 (s, 2H), 3.87 (s, 3H).

### Example 527B: (S)-1,4-diaminobicyclo[2.2.2]octan-2-ol, 2 Hydrochloric Acid

A solution of Example 445B (0.3 g, 1.025 mmol) and 4 N HCl in dioxane (2.56 mL, 10.25 mmol) in methanol (3.0 mL) was stirred at ambient temperature for 48 hours. The mixture was concentrated under high vacuum to give 0.21 g of the title compound carried on without further purification. ¹H NMR (501 MHz, DMSO-*d*₆) *δ* ppm 8.27 (s, 3H), 8.02 (s, 3H), 5.81 (d, J = 4.6 Hz, 1H), 3.89 3.82 (m, 1H), 2.21 (ddd, J = 12.8, 9.5, 2.8 Hz, 1H), 2.01 (dq, J = 15.2, 4.5 Hz, 1H), 1.91 1.54 (m, 8H).

### Example 527C: N,N'-[(2S)-2-hydroxybicyclo[2.2.2]octane-1,4-diyl]bis{2-[(2-methoxypyrimidin-5-yl)oxy]acetamide}

To a solution of Example 527A (68.4 mg, 0.438 mmol), Example 527B (50.0 mg, 0.194 mmol), and N,N-diisopropylethylamine (0.204 mL, 1.167 mmol) in *N,N*-dimethylformamide (2.5 mL) at ambient temperature was added (1-[bis(dimethylamino)methylene]-1*H*-1,2,3-triazolo[4,5-*b*]pyridinium 3-oxid hexafluorophosphate) (HATU, 185 mg, 0.486 mmol) portionwise, and the mixture was stirred at the same temperature for 30 minutes. The mixture was concentrated under high vacuum, and the residue was purified by HPLC (Phenomenex^{®} Luna^{®} C18(2) 10 µm 100Å AXIA^{™} column (250 mm × 50 mm). A 30-100% gradient of acetonitrile (A) and 0.1% trifluoroacetic acid in water (B) was used over 25 minutes, at a flow rate of 50 mL/minute) to give 78 mg of the title compound. ¹H NMR (400 MHz, DMSO-*d*₆) *δ* ppm 8.33 (d, J = 12.4 Hz, 4H), 7.54 (s, 1H), 7.30 (s, 1H), 4.50 (d, J = 15.7 Hz, 4H), 4.07 (dd, J = 9.7, 3.2 Hz, 1H), 3.86 (d, J = 1.2 Hz, 6H), 2.27 (ddd, J = 12.2, 9.5, 2.3 Hz, 1H), 2.04 (ddd, J = 12.5, 10.8, 4.9 Hz, 1H), 1.97 1.85 (m, 3H), 1.88 1.74 (m, 5H); MS (ESI⁺) *m*/*z* 489.5 (M+H)⁺.

### Example 528: 2-(4-chloro-3-fluorophenoxy)-N-[(2S)-2-hydroxy-4-(2-{[2-(trifluoromethyl)pyrimidin-5-yl]oxy}acetamido)bicyclo[2.2.2]octan-1-yl]acetamide (Compound 627)

A mixture of Example 476A (0.05 g, 0.119 mmol), 2-(trifluoromethyl)ypyrimidin-5-ol (0.039 g, 0.239 mmol), potassium carbonate (0.033 g, 0.239 mmol) and potassium iodide (1.386 mg, 8.35 µmol) in acetone (1.0 mL) was heated at 140 °C for 45 minutes in Biotage^{®} Initiator microwave reactor for 45 minutes. The mixture was concentrated, and the residue was purified by HPLC (Phenomenex^{®} Luna^{®} C18(2) 10 µm 100Å AXIA^{™} column (250 mm × 50 mm). A 30-100% gradient of acetonitrile (A) and 0.1% trifluoroacetic acid in water (B) was used over 25 minutes, at a flow rate of 50 mL/minute) to give 47 mg of the title compound. ¹H NMR (400 MHz, DMSO-*d*₆) *δ* ppm 8.70 (s, 2H), 7.71 (s, 1H), 7.48 (t, J = 8.9 Hz, 1H), 7.26 (s, 1H), 7.05 (dd, J = 11.4, 2.9 Hz, 1H), 6.83 (dt, J = 9.2, 1.9 Hz, 1H), 5.06 (s, 1H), 4.75 (s, 2H), 4.47 (s, 2H), 4.05 (dd, J = 9.5, 3.1 Hz, 1H), 2.28 (ddd, J = 12.0, 9.5, 2.2 Hz, 1H), 2.12 2.01 (m, 1H), 2.01 1.79 (m, 5H), 1.82 1.73 (m, 3H); MS (ESI⁺) *m*/*z* 547.2 (M+H)⁺.

### Example 529: rac-N,N'-[(1R,2S,4R,5S)-bicyclo[2.2.1]heptane-2,5-diyl]bis[2-(4-chloro-3-fluorophenoxy)acetamide] (Compound 628)

### Example 529A: dimethyl rac-(1R,2S,4R,5S)-bicyclo[2.2.1]heptane-2,5-dicarboxylate

To a mixture of bicyclo[3.3.1]nonane-2,6-dione (1.556 g, 9.20 mmol) in methanol (10 mL) was added a solution of thallium(iii) nitrate trihydrate (12.27 g, 27.6 mmol) in methanol (10 mL) at 20 °C. The mixture was stirred at 20 °C for 12 hours. The resulting mixture was filtered, and the filtrate was concentrated under reduced pressure. The residue was diluted with water (10 mL) and extracted with ethyl acetate (20 mL) The organic phase was dried with Na₂SO₄ and concentrated under reduced pressure to give the title compound (1.4 g, yield 57.4%) that was used in the next step without further purifications. ¹H NMR (400 MHz, CDCl₃) *δ* ppm 1.55 (dd, J=12.78, 8.99 Hz, 2 H) 1.73 - 1.81 (m, 1 H) 1.90 - 1.96 (m, 2 H) 2.35 (dd, J=8.93, 5.50 Hz, 2 H) 2.55 - 2.62 (m, 2 H) 2.78 - 2.96 (m, 1 H) 3.64 - 3.70 (m, 6 H).

### Example 529B: rac-(1R,2S,4R,5S)-bicyclo[2.2.1]heptane-2,5-dicarboxylic acid

To a mixture of Example 529A (1.4 g, 5.28 mmol) in tetrahydrofuran (15 mL) was added a solution of lithium hydroxide monohydrate (0.664 g, 15.83 mmol) in water (8 mL) at 20 °C dropwise. The mixture was stirred at 20 °C for 12 hours. The solution was concentrated under reduced pressure. The residue was diluted with water (10 mL), adjusted to pH = 3 by aqueous 1 M HCl, and extracted with ethyl acetate (2 × 20 mL). The combined organic phases were concentrated under reduced pressure to give the title compound (1 g, yield 82%) that was used in the next step without further purifications. ¹H NMR (400 MHz, CD₃OD) *δ* ppm 1.35 - 1.43 (m, 2 H) 1.50 - 1.63 (m, 2 H) 1.82 - 1.94 (m, 2 H) 2.36 (dd, J=8.99, 5.48 Hz, 2 H) 2.54 (d, J=4.82 Hz, 2 H).

### Example 529C: rac-(1R,2S,4R,5S)-bicyclo[2.2.1]heptane-2,5-diamine

To a solution of Example 529B (300 mg, 1.303 mmol) in toluene (10 mL) was added triethylamine (0.509 mL, 3.65 mmol) and diphenyl phosphorazidate (861 mg, 3.13 mmol) in order at 20 °C under N₂. The mixture was stirred for 3 hours at 90 °C under N₂ and concentrated under reduced pressure at 45°C. The resulting residue was diluted with aqueous hydrochloric acid (3 N, 10 mL) and stirred for 12 hours at 90 °C. The reaction solution was concentrated under reduced pressure at 45°C, and the residue was purified by preparative HPLC performed on a Phenomenex^{®} Luna^{®}-C18 column (100 × 30 mm, 5 µm particle size) using a gradient of 1% to 100% acetonitrile:0.075% aqueous trifluoroacetic acid over 6 minutes at a flow rate of 25 mL/minute to provide 50 mg of the product as a trifluoroacetic acid (TFA) salt. The TFA salt was treated with HCl in ethyl acetate (4 N, 10 mL), and the mixture was filtered. The solid was collected and dried by high vacuum to provide the title compound as an HCl salt (49.2 mg, yield 18.01%). ¹H NMR (400 MHz, DMSO-*d*₆) *δ* ppm 1.42 (br d, J=13.23 Hz, 2 H) 1.66 - 1.72 (m, 2 H) 1.73 (s, 2 H) 2.38 (br d, J=4.85 Hz, 2 H) 3.02 (br s, 2 H) 8.04 (br s, 6 H).

### Example 529D: rac-N,N'-[(1R,2S,4R,5S)-bicyclo[2.2.1]heptane-2,5-diyl]bis[2-(4-chloro-3-fluorophenoxy)acetamide]

A mixture of Example 529C (0.035 g, 0.176 mmol), triethylamine (0.122 ml, 0.879 mmol), 2-(4-chloro-3-fluorophenoxy)acetic acid (0.079 g, 0.387 mmol) and HATU (1-[bis(dimethylamino)methylene]-1*H*-1,2,3-triazolo[4,5-*b*]pyridinium 3-oxid hexafluorophosphate) (0.147 g, 0.387 mmol) in dimethylformamide (DMF) (2 ml) was stirred for 5 hours. The reaction mixture was quenched with water and brine and extracted with ethyl acetate (2×). The combined organic layers were concentrated, and the residue was purified by HPLC performed on a Phenomenex^{®} Luna^{®} C18 column (250 × 30 mm, 10 µm particle size) using a gradient of 20% to 100% acetonitrile:0.1% aqueous trifluoroacetic acid over 26 minutes at a flow rate of 50 mL/minute to provide the title compound (57.5 mg, 58%). ¹H NMR (400 MHz, DMSO-*d*₆) *δ* ppm 8.07 (s, 1H), 7.94 (d, J = 2.6 Hz, 1H), 7.49 (t, J = 8.9 Hz, 1H), 7.33 - 7.22 (m, 2H), 7.06 (dd, J = 11.4, 2.8 Hz, 1H), 6.90 - 6.78 (m, 1H), 4.48 (s, 2H), 4.45 (d, J = 5.0 Hz, 1H), 4.08 (dd, J = 9.6, 3.1 Hz, 1H), 2.36 (ddd, J = 12.3, 9.4, 2.2 Hz, 1H), 2.15 - 1.77 (m, 9H); MS (ESI⁺) *m*/*z* 488.2 (M+H)⁺.

### Example 530: 2-(4-chloro-3-fluorophenoxy)-N-[(2S)-2-hydroxy-4-(2-{[2-(2,2,2-trifluoroethoxy)pyrimidin-5-yl]oxy}acetamido)bicyclo[2.2.2]octan-1-yl]acetamide (Compound 629)

The title compound was synthesized using the procedure described in Example 538 substituting 2-(2,2,2-trifluoroethoxypyrimidin-5-ol for 2-methoxypyrimidin-5-ol. ¹H NMR (400 MHz, DMSO-*d*₆) *δ* ppm 8.38 (s, 2H), 7.57 (s, 1H), 7.48 (t, J = 8.9 Hz, 1H), 7.26 (s, 1H), 7.05 (dd, J = 11.4, 2.9 Hz, 1H), 6.83 (dd, J = 8.9, 2.9 Hz, 1H), 4.97 (q, J = 9.0 Hz, 2H), 4.53 (s, 2H), 4.46 (s, 2H), 4.04 (dd, J = 9.6, 3.1 Hz, 1H), 2.27 (ddd, J = 12.2, 9.5, 2.3 Hz, 1H), 2.07 (ddd, J = 12.5, 10.6, 4.8 Hz, 1H), 1.93 (d, J = 10.4 Hz, 2H), 1.85 (s, 1H), 1.90 1.82 (m, 1H), 1.78 (ddd, J = 13.1, 6.2, 4.0 Hz, 5H); MS (ESI⁺) *m*/*z* 577.2 (M+H)⁺.

### Example 531: 2-(4-chloro-3-fluorophenoxy)-N-[(2S)-2-hydroxy-4-(2-{[5-(trifluoromethyl)pyrimidin-2-yl]oxy}acetamido)bicyclo[2.2.2]octan-1-yl]acetamide (Compound 630)

The title compound was synthesized using the procedure described in Example 538 substituting 5-trifluoromethylpyrimidin-2-ol for 2-methoxypyrimidin-5-ol. ¹H NMR (400 MHz, DMSO-*d*₆) *δ* ppm 8.71 (s, 2H), 7.72 (s, 1H), 7.48 (t, J = 8.9 Hz, 1H), 7.27 (s, 1H), 7.05 (dd, J = 11.4, 2.8 Hz, 1H), 6.86 6.80 (m, 1H), 4.75 (s, 2H), 4.47 (s, 2H), 4.05 (dd, J = 9.5, 3.2 Hz, 1H), 2.28 (ddd, J = 12.4, 9.5, 2.3 Hz, 1H), 2.06 (ddd, J = 12.8, 11.0, 4.7 Hz, 1H), 1.94 (dp, J = 10.9, 3.0, 2.5 Hz, 2H), 1.88 (ddd, J = 14.7, 7.5, 2.0 Hz, 1H), 1.86 1.73 (m, 5H); MS (ESI⁺) *m*/*z* 547.3 (M+H)⁺.

### Example 532: 2-(4-fluorophenoxy)-N-[(2S)-2-hydroxy-4-{2-[(2-methoxypyrimidin-5-yl)oxy]acetamido}bicyclo[2.2.2]octan-1-yl]acetamide (Compound 631)

### Example 532A: (S)-tert-butyl (2-hydroxy-4-(2-((2-methoxypyrimidin-5-yl)oxy)acetamido)bicyclo[2.2.2]octan-1-yl)carbamate

A mixture of Example 445B (1.52 g, 5.19 mmol), 2-chloroacetic acid (0.515 g, 5.45 mmol) and *N*-ethyl-*N*-isopropylpropan-2-amine (3.17 mL, 18.17 mmol) in *N,N-*dimethylformamide (30 mL) was treated with 2-(3*H*-[1,2,3]triazolo[4,5-*b*]pyridin-3-yl)-1,1,3,3-tetramethylisouronium hexafluorophosphate(V) (2.467 g, 6.49 mmol), and the reaction was stirred at ambient temperature for 30 minutes. The reaction was quenched with saturated ammonium chloride solution and extracted with dichloromethane. The organic layer was washed with brine, dried over magnesium sulfate and filtered. The filtrate was concentrated to give 2.51 g of crude as an oil, which used without further purification. To a mixture of this crude (0.5 g, 1.202 mol) in acetone (5.0 mL) in a 20 mL of microwave reaction vial was added 2-methoxypyrimidin-5-ol (0.303 g, 2.404 mmol), potassium carbonate (0.332 g, 2.404 mmol) and potassium iodide (0.014 g, 0.084 mmol), and the reaction mixture was heated at 140 °C in Biotage^{®} Initiator microwave reactor for 45 minutes. The mixture was filtered, and the filtrate was concentrated and purified on silica gel (0-10% methanol/dichloromethane) to give 0.38 g of the title compound. ¹H NMR (400 MHz, DMSO-*d*₆) *δ* ppm 8.32 (s, 2H), 7.50 (s, 1H), 6.02 (s, 1H), 4.48 (s, 2H), 3.88 (d, J = 2.4 Hz, 1H), 3.86 (s, 3H), 3.82 (s, 1H), 2.25 (ddd, J = 12.3, 9.4, 2.5 Hz, 1H), 2.04 (ddd, J = 12.4, 10.0, 4.5 Hz, 1H), 1.95 1.60 (m, 8H), 1.36 (s, 9H); MS (ESI⁺) *m*/*z* 423.2 (M+H)⁺.

### Example 532B: (S)-N-(4-amino-3-hydroxybicyclo[2.2.2]octan-1-yl)-2-((2-methoxypyrimidin-5-yl)oxy)acetamide, Trifluoroacetic Acid

A mixture of Example 532A (0.38 g, 0.899 mmol) and 2,2,2-trifluoroacetic acid (5.0 mL, 64.9 mmol) in dichloromethane (10 mL) was stirred at ambient temperature for 16 hours. The reaction was concentrated, and the residue was purified by HPLC (Phenomenex^{®} Luna^{®} C18(2) 10 µm 100Å AXIA^{™} column (250 mm × 50 mm), 5-75% gradient of acetonitrile (A) and 0.1% trifluoroacetic acid in water (B) was used over 25 minutes, at a flow rate of 50 mL/minute) to give 300 mg of the title compound. ¹H NMR (400 MHz, DMSO-*d*₆) *δ* ppm 8.32 (s, 2H), 7.78 (s, 3H), 7.70 (s, 1H), 4.50 (s, 2H), 3.99 3.95 (m, 1H), 3.86 (s, 3H), 3.84 3.77 (m, 2H), 2.33 (ddd, J = 12.9, 9.5, 3.0 Hz, 1H), 2.09 1.93 (m, 2H), 1.92 1.75 (m, 5H), 1.63 (q, J = 10.6, 10.1 Hz, 1H), 1.59 1.50 (m, 1H); MS (ESI⁺) *m*/*z* 323.4 (M+H)⁺.

### Example 532C: 2-(4-fluorophenoxy)-N-{(2S)-2-hydroxy-4-{2-[(2-methoxypyrimidin-5-yl)oxy]acetamido}bicyclo[2.2.2]octan-1-yl]acetamide

To a mixture of Example 532B (60 mg, 0.137 mmol), 2-(4-fluorophenoxy)acetic acid (24.56 mg, 0.144 mmol), and *N*-ethyl-*N*-isopropylpropan-2-amine (0.084 mL, 0.481 mmol) in *N,N*-dimethylformamide (1.0 mL), 2-(3*H*-[1,2,3]triazolo[4,5-*b*]pyridin-3-yl)-1,1,3,3-tetramethylisouronium hexafluorophosphate(V) (65.3 mg, 0.172 mmol) was added, and the mixture was stirred at ambient temperature for 1.5 hours. The reaction was concentrated, and the residue was purified by HPLC (Phenomenex^{®} Luna^{®} C18(2) 10 µm 100Å AXIA^{™} column (250 mm × 50 mm), 25-95% gradient of acetonitrile (A) and 0.1% trifluoroacetic acid in water (B) was used over 25 minutes, at a flow rate of 50 mL/minute) to give 48 mg of the t. ¹H NMR (400 MHz, DMSO-*d*₆) *δ* ppm 8.28 (s, 2H), 7.50 (s, 1H), 7.17 (s, 1H), 7.14 - 7.03 (m, 2H), 6.91 (ddd, J = 11.5, 5.7, 3.5 Hz, 2H), 4.45 (s, 2H), 4.35 (s, 2H), 4.00 - 3.91 (m, 1H), 3.82 (s, 3H), 2.24 (ddd, J = 12.5, 9.5, 2.6 Hz, 1H), 2.10 (td, J = 9.9, 9.0, 5.9 Hz, 1H), 1.96 - 1.83 (m, 2H), 1.76 (ddt, J = 13.4, 10.1, 3.9 Hz, 6H).; MS (ESI⁺) *m*/*z* 475.2 (M+H)⁺.

### Example 533: 2-[(2,2-difluoro-2H-1,3-benzodioxol-5-yl)oxy]-N-[(2S)-2-hydroxy-4-{2-[(2-methoxypyrimidin-5-yl)oxy]acetamido}bicyclo[2.2.2]octan-1-yl]acetamide (Compound 632)

The title compound was synthesized using the procedure described in Example 532C substituting 2-((2,2-difluorobenzo[d][1,3]dioxol-5-yl)oxy)acetic acid for2-(4-fluorophenoxy)acetic acid. ¹H NMR (400 MHz, DMSO-*d*₆) d 8.32 (s, 2H), 7.54 (s, 1H), 7.31 (d, J = 8.9 Hz, 1H), 7.22 (s, 1H), 7.12 (d, J = 2.6 Hz, 1H), 6.74 (dd, J = 8.9, 2.6 Hz, 1H), 4.49 (s, 2H), 4.43 (s, 2H), 4.03 (dd, J = 9.3, 3.2 Hz, 1H), 3.86 (s, 3H), 2.28 (ddd, J = 12.5, 9.5, 2.4 Hz, 1H), 2.15 2.04 (m, 1H), 1.92 (ddd, J = 11.2, 7.4, 4.7 Hz, 2H), 1.89 1.73 (m, 6H); MS (ESI⁺) *m*/*z* 537.3 (M+H)⁺.

### Example 534: 2-[3-fluoro-4-(trifluoromethyl)phenoxy]-N-[(2S)-2-hydroxy-4-{2-[(2-methoxypyrimidin-5-yl)oxy]acetamido}bicyclo[2.2.2]octan-1-yl]acetamide (Compound 633)

The title compound was synthesized using the procedure described in Example 532C substituting Example 574A for 2-(4-fluorophenoxy)acetic acid. ¹H NMR (400 MHz, DMSO-*d*₆) *δ* ppm 8.28 (s, 2H), 7.65 (t, J = 8.8 Hz, 1H), 7.50 (s, 1H), 7.30 (s, 1H), 7.06 (dd, J = 13.1, 2.4 Hz, 1H), 6.90 (dd, J = 8.8, 2.4 Hz, 1H), 4.53 (s, 2H), 4.45 (s, 2H), 4.03 (dd, J = 9.5, 3.1 Hz, 1H), 3.82 (s, 3H), 2.28 2.18 (m, 1H), 1.99 (ddd, J = 12.5, 10.6, 5.0 Hz, 1H), 1.94 1.82 (m, 3H), 1.84 1.68 (m, 5H); MS (ESI⁺) *m*/*z* 543.2 (M+H)⁺.

### Example 535: 2-(3,4-difluorophenoxy)-N-[(2S)-2-hydroxy-4-{2-[(2-methoxypyrimidin-5-yl)oxy]acetamido}bicyclo[2.2.2]octan-1-yl]acetamide (Compound 634)

The title compound was synthesized using the procedure described in Example 532C substituting 2-(3,4-difluorophenoxy)acetic acid for 2-(4-fluorophenoxy)acetic acid. ¹H NMR (400 MHz, DMSO-*d*₆) *δ* ppm 8.32 (s, 2H), 7.54 (s, 1H), 7.35 (dt, J = 10.7, 9.3 Hz, 1H), 7.23 (s, 1H), 7.07 (ddd, J = 12.6, 6.7, 3.1 Hz, 1H), 6.77 (dtd, J = 8.7, 3.2, 1.6 Hz, 1H), 4.46 (d, J = 29.4 Hz, 4H), 4.03 (dd, J = 9.8, 3.2 Hz, 1H), 3.86 (s, 3H), 2.27 (ddd, J = 12.5, 9.4, 2.5 Hz, 1H), 2.09 (ddd, J = 12.5, 10.7, 4.7 Hz, 1H), 1.98 1.86 (m, 2H), 1.89 1.79 (m, 2H), 1.78 (ddd, J = 12.7, 6.8, 2.5 Hz, 4H); MS (ESI⁺) *m*/*z* 493.3 (M+H)⁺.

### Example 536: 2-(4-chlorophenoxy)-N-[(2S)-2-hydroxy-4-{2-[(2-methoxypyrimidin-5-yl)oxy]acetamido}bicyclo[2.2.2]octan-1-yl]acetamide (Compound 635)

The title compound was synthesized using the procedure described in Example 532C substituting 2-(4-chlorophenoxy)acetic acid for 2-(4-fluorophenoxy)acetic acid. ¹H NMR (400 MHz, DMSO-*d*₆) *δ* ppm 8.32 (s, 2H), 7.54 (s, 1H), 7.37 7.30 (m, 2H), 7.23 (s, 1H), 7.00 6.92 (m, 2H), 4.48 (s, 2H), 4.42 (s, 2H), 4.04 3.98 (m, 1H), 3.86 (s, 3H), 2.27 (ddd, J = 12.5, 9.4, 2.6 Hz, 1H), 2.16 2.07 (m, 1H), 1.98 1.86 (m, 2H), 1.83 (tt, J = 4.2, 2.0 Hz, 1H), 1.81 (s, 3H), 1.81 1.74 (m, 2H); MS (ESI⁺) *m*/*z* 491.4 (M+H)⁺.

### Example 537: 2-(4-chloro-3-fluorophenoxy)-N-[(2S)-4-(2-{[2-(cyclobutyloxy)pyrimidin-5-yl]oxy}acetamido)-2-hydroxybicyclo[2.2.2]octan-1-yl]acetamide (Compound 636)

The title compound was synthesized using the procedure described in Example 538 substituting 2-cyclobutoxypyrimidin-5-ol for 2-methoxypyrimidin-5-ol. ¹H NMR (400 MHz, DMSO-*d*₆) *δ* ppm 8.25 (s, 2H), 7.49 (s, 1H), 7.44 (t, J = 8.9 Hz, 1H), 7.22 (s, 1H), 7.01 (dd, J = 11.4, 2.8 Hz, 1H), 6.79 (dd, J = 9.0, 3.1 Hz, 1H), 5.00 (h, J = 7.8, 7.4 Hz, 1H), 4.43 (d, J = 3.4 Hz, 4H), 4.00 (dd, J = 9.5, 3.1 Hz, 1H), 2.34 (dddt, J = 9.6, 7.3, 5.4, 2.6 Hz, 2H), 2.23 (ddd, J = 12.3, 9.4, 2.3 Hz, 1H), 2.08 1.98 (m, 2H), 2.02 1.95 (m, 1H), 1.98 1.77 (m, 4H), 1.74 (ddt, J = 16.6, 6.3, 2.9 Hz, 6H), 1.66 1.52 (m, 1H); MS (ESI⁺) *m*/*z* 549.4 (M+H)⁺.

### Example 538: 2-(4-chloro-3-fluorophenoxy)-N-[(2S)-2-hydroxy-4-{2-[(2-methoxypyrimidin-5-yl)oxy]acetamido}bicyclo[2.2.2]octan-1-yl]acetamide (Compound 637)

A mixture of Example 476A (0.05 g, 0.119 mmol), 2-methoxypyrimidin-5-ol (0.030 g, 0.239 mmol), potassium carbonate (0.033 g, 0.239 mmol) and potassium iodide (1.386 mg, 8.35 µmol) in acetone (1.0 mL) was heated at 140 °C in Biotage^{®} Initiator microwave reactor for 45 minutes. Volatiles were removed, and the residue was purified by HPLC (Phenomenex^{®} Luna^{®} C18(2) 10 µm 100Å AXIA^{™} column (250 mm × 50 mm). A 30-100% gradient of acetonitrile (A) and 0.1% trifluoroacetic acid in water (B) was used over 25 minutes, at a flow rate of 50 mL/minute) to give 51.5 mg of the title compound. ¹H NMR (400 MHz, DMSO-*d*₆) *δ* ppm 8.32 (s, 2H), 7.53 (s, 1H), 7.48 (t, J = 8.9 Hz, 1H), 7.26 (s, 1H), 7.05 (dd, J = 11.4, 2.8 Hz, 1H), 6.83 (ddd, J = 8.9, 2.9, 1.2 Hz, 1H), 4.47 (d, J = 7.9 Hz, 4H), 4.04 (dd, J = 9.4, 3.1 Hz, 1H), 3.86 (s, 3H), 2.27 (ddd, J = 12.3, 9.4, 2.3 Hz, 1H), 2.07 (ddd, J = 12.4, 10.5, 4.7 Hz, 1H), 1.97 1.88 (m, 2H), 1.90 1.80 (m, 1H), 1.78 (ddt, J = 13.0, 6.0, 2.6 Hz, 5H); MS (ESI⁺) *m*/*z* 509.3 (M+H)⁺.

### Example 539: rac-N,N'-[(1R,3S,4R,5R)-3-hydroxybicyclo[3.2.1]octane-1,4-diyl]bis[2-(4-chloro-3-fluorophenoxy)acetamide] (Compound 638)

To a solution of Example 553 (0.0161 g, 0.031 mmol) in methanol (0.30 mL) at 0 °C was added NaBH₄ (1.2 mg, 0.032 mmol), and the mixture was stirred for 1 hour. The reaction mixture was quenched with acetone, diluted with ethyl acetate, washed with 1 N HCl and brine, dried (Na₂SO₄), and concentrated. The residue was chromatographed on silica gel (3% isopropanol/dichloromethane) to give the title compound (0.0094 g, 0.018 mmol, 58.2 % yield). ¹H NMR (400 MHz, DMSO-*d*₆) *δ* ppm 7.97 (s, 1H), 7.66 (d, J = 8.0 Hz, 1H), 7.48 (td, J = 8.9, 4.2 Hz, 2H), 7.06 (ddd, J = 20.0, 11.4, 2.9 Hz, 2H), 6.84 (dddd, J = 10.0, 8.9, 2.8, 1.2 Hz, 2H), 5.14 (d, J = 3.8 Hz, 1H), 4.68 - 4.55 (m, 2H), 4.46 (s, 2H), 3.89 - 3.75 (m, 2H), 2.45 - 2.35 (m, 1H), 2.24 - 2.09 (m, 2H), 1.99 - 1.85 (m, 2H), 1.85 - 1.69 (m, 2H), 1.67 - 1.54 (m, 1H), 1.53 - 1.42 (m, 1H); MS (ESI⁺) *m*/*z* 529.4 (M+H)⁺.

### Example 540: 2-(4-chloro-3-fluorophenoxy)-N-[(3S)-3-hydroxy-4-{2-[(1,2-oxazol-3-yl)oxy]acetamido}bicyclo[2.2.2]octan-1-yl]acetamide (Compound 639)

### Example 540A: (S)-tert-butyl (4-(2-(4-chloro-3-fluorophenoxy)acetamido)-2-hydroxybicyclo[2.2.2]octan-1-yl)carbamate

The title compound was prepared as described in Example 81, substituting the product of Example 659D for benzyl (4-aminobicyclo[2.1.1]hexan-1-yl)carbamate hydrochloride. MS (ESI⁺) *m*/*z* 387 (M-tBu+H)⁺.

### Example 540B: 2-(4-chloro-3-fluorophenoxy)-N-[(3S)-3-hydroxy-4-{2-[(1,2-oxazol-3-yl)oxy]acetamido}bicyclo[2.2.2]octan-1-yl]acetamide (Compound 639)

The reaction and purification conditions described in Example 83C substituting potassium 2-(isoxazol-3-yloxy)acetate (commercially available from Enamine) for the product of Example 83B, and the product of Example 540A for the product of Example 9A gave the title compound. ¹H NMR (400 MHz, DMSO-*d*₆) *δ* ppm 8.66 (d, J = 1.8 Hz, 1H), 7.52 - 7.44 (m, 2H), 7.33 (s, 1H), 7.02 (dd, J = 11.4, 2.9 Hz, 1H), 6.81 (ddd, J = 9.0, 2.9, 1.2 Hz, 1H), 6.35 (d, J = 1.8 Hz, 1H), 5.00 (s, 1H), 4.62 - 4.54 (m, 2H), 4.43 (s, 2H), 4.06 (d, J = 9.2 Hz, 1H), 2.30 - 2.20 (m, 1H), 2.06 - 1.70 (m, 9H); MS (APCI⁺) *m*/*z* 468 (M+H)⁺.

### Example 542: 2-(4-chloro-3-fluorophenoxy)-N-(3-{[2-(4-chlorophenoxy)-2-methylpropyl]amino}bicyclo[1.1.1]pentan-1-yl)acetamide (Compound 641)

To a mixture of Example 9B (0.040 g, 0.140 mmol) and 2-(4-chlorophenoxy)-2-methylpropanal (0.042 g, 0.21 mmol) in methanolic pH4 buffer (2.4 weight% sodium acetate·trihydrate, 3.6 weight % acetic acid in methanol, 1.4 mL) at 0 °C was added sodium cyanoborohydride (0.019 g, 0.31 mmol). The reaction mixture was allowed to warm to ambient temperature, stirred for 3 hours, and then was concentrated in vacuo. The residue was purified by preparative HPLC (Waters XBridge^{™} C18 5 µm OBD column, 30 × 100 mm, flow rate 40 mL/minute, 5-100% gradient of acetonitrile in 0.1% trifluoroacetic acid/water) followed by repurification by normal phase column chromatography (ethyl acetate/heptanes, 1-100%) gave the title compound (0.050 g, 0.11 mmol, 76% yield). ¹H NMR (501 MHz, DMSO-*d*₆) *δ* ppm 8.91 (s, 1H), 7.51 (t, J = 8.9 Hz, 1H), 7.42 - 7.36 (m, 2H), 7.11 - 7.05 (m, 3H), 6.87 (ddd, J = 9.0, 2.9, 1.2 Hz, 1H), 4.52 (s, 2H), 3.12 (s, 2H), 2.29 (s, 6H), 1.29 (s, 6H); MS 467 (M+H)⁺.

### Example 543: 5-[2-({(3S)-4-[2-(4-chloro-3-fluorophenoxy)acetamido]-3-hydroxybicyclo[2.2.2]octan-1-yl}amino)-2-oxoethoxy]pyrimidine-2-carboxylic acid (Compound 642)

A mixture of Example 476A (75.0 mg, 0.179 mmol), methyl 5-hydroxypyrimidine-2-carboxylate (41.4 mg, 0.268 mmol), potassium iodide (1.485 mg, 8.94 µmol), and potassium carbonate (49.4 mg, 0.358 mmol) in acetone (2.5 mL) was heated at 140 °C in a Biotage^{®} Initiator microwave reactor for 40 minutes. The suspension was filtered and the solid rinsed with methanol. The filter cake was dissolved in water and purified by HPLC (see protocol in Example 529D) to provide the title compound (11.2 mg, 12%). ¹H NMR (400 MHz, DMSO-*d*₆) *δ* ppm 8.59 (s, 2H), 7.69 (s, 1H), 7.48 (t, J = 8.9 Hz, 1H), 7.27 (s, 1H), 7.05 (dd, J = 11.4, 2.8 Hz, 1H), 6.83 (dd, J = 9.1, 2.8 Hz, 1H), 5.10 (d, J = 4.4 Hz, 1H), 4.71 (s, 2H), 4.46 (s, 2H), 4.05 (d, J = 9.5 Hz, 1H), 2.28 (ddd, J = 12.2, 9.4, 2.2 Hz, 1H), 2.07 (ddd, J = 12.3, 10.5, 4.8 Hz, 1H), 2.00 - 1.69 (m, 8H); MS (APCI⁺) *m*/*z* 523.2 (M+H)⁺.

### Example 544: N,N'-[2-(2-hydroxyethyl)bicyclo[2.2.2]octane-1,4-diyl]bis[2-(4-chloro-3-fluorophenoxy)acetamide] (Compound 643)

A solution of Example 573 (20.0 mg, 0.033 mmol) in tetrahydrofuran (THF) (1 mL) at 0 °C was treated with 1 M LAH (lithium aluminum hydride) (0.100 mL, 0.100 mmol) in ether. The reaction was warmed to room temperature and stirred for 2 hours. The reaction mixture was quenched with 20% Na/K tartrate at 0 °C and extracted with ethyl acetate (2×). The combined organic layers were washed with 20% Na/K tartrate and concentrated. The residue was purified by HPLC (see protocol in Example 529D) to provide the title compound (0.4 mg, 2%). ¹H NMR (501 MHz, DMSO-*d*₆) *δ* ppm 7.55 - 7.42 (m, 3H), 7.36 (s, 1H), 7.02 (dd, J = 11.4, 2.9 Hz, 2H), 6.81 (ddd, J = 9.0, 2.9, 1.2 Hz, 2H), 4.52 - 4.35 (m, 5H), 3.48-3.29 (m, 2H), 2.45 - 2.22 (m, 2H), 2.04 (ddd, J = 12.9, 10.6, 2.5 Hz, 1H), 1.95 - 1.50 (m, 9H), 1.31 - 1.16 (m, 1H); MS (APCI⁺) *m*/*z* 557.2 (M+H)⁺.

### Example 545: N,N'-[2-hydroxy-2-(hydroxymethyl)bicyclo[2.2.2]octane-1,4-diyl]bis[2-(4-chloro-3-fluorophenoxy)acetamide] (Compound 644)

A solution of Example 555 (0.0210 g, 0.039 mmol) and tris(((trifluoromethyl)sulfonyl)oxy)scandium (0.019 g, 0.039 mmol) in ClCH₂CH₂Cl (0.20 mL) was stirred at ambient temperature for 20 hours. The reaction mixture was then diluted with ethyl acetate, washed with water and brine, dried (Na₂SO₄), and concentrated. The residue was chromatographed on silica gel (4% methanol/dichloromethane) to give the title compound (0.0058 g, 10.37 µmol, 26.7 % yield). ¹H NMR (500 MHz, DMSO-*d*₆) *δ* ppm 8.15 (s, 1H), 7.56 (s, 1H), 7.47 (td, J = 8.9, 6.0 Hz, 2H), 7.02 (ddt, J = 11.4, 10.2, 3.4 Hz, 2H), 6.81 (tdd, J = 9.1, 2.9, 1.2 Hz, 2H), 5.19 (s, 1H), 5.12 (t, J = 5.4 Hz, 1H), 4.49 - 4.39 (m, 4H), 3.33 - 3.28 (m, 1H), 2.22 - 2.08 (m, 2H), 2.08 - 1.98 (m, 2H), 1.95 - 1.87 (m, 1H), 1.86 - 1.70 (m, 4H) ; LC/MS (APCI⁺) *m*/*z* 559.4 (M+H)⁺.

### Example 546: 2-(4-chloro-3-fluorophenoxy)-N-[(2S)-2-hydroxy-4-{2-[(pyrazolo[1,5-a]pyridin-5-yl)oxy]acetamido}bicyclo[2.2.2]octan-1-yl]acetamide (Compound 645)

A mixture of Example 476A (0.05 g, 0.119 mmol), pyrazolo[1,5-a]pyridin-5-ol (0.032 g, 0.239 mmol), potassium carbonate (0.033 g, 0.239 mmol) and potassium iodide (1.386 mg, 8.35 µmol) in acetone (1.0 mL) was heated at 140 °C for 45 minutes in Biotage^{®} Initiator microwave reactor. The mixture was concentrated, and the residue was purified by HPLC (Phenomenex^{®} Luna^{®} C18(2) 10 µm 100Å AXIA^{™} column (250 mm × 50 mm). A 30-100% gradient of acetonitrile (A) and 0.1% trifluoroacetic acid in water (B) was used over 25 minutes, at a flow rate of 50 mL/minute) to give 35 mg of the title compound. ¹H NMR (501 MHz, DMSO-*d*₆) *δ* ppm 8.51 (d, J = 7.6 Hz, 1H), 7.85 (d, J = 2.2 Hz, 1H), 7.52 (s, 1H), 7.46 (t, J = 8.8 Hz, 1H), 7.24 (s, 1H), 7.03 (dd, J = 11.4, 2.8 Hz, 1H), 6.89 (d. J = 2.7 Hz, 1H), 6.81 (dd, J = 9.0, 2.8 Hz, 1H), 6.61 (dd, J = 7.6, 2.7 Hz, 1H), 6.37 (d, J = 2.1 Hz, 1H), 4.43 (d, J = 8.1 Hz, 4H), 4.02 (dd, J = 9.5, 3.2 Hz, 1H), 2.27 (ddd, J = 12.2, 9.5, 2.3 Hz, 1H), 2.09 2.00 (m, 1H), 1.98 1.90 (m, 1H), 1.93 1.87 (m, 1H), 1.90 1.72 (m, 6H); MS (ESI⁺) *m*/*z* 517.1 (M+H)⁺.

### Example 547: 2-(4-chloro-3-fluorophenoxy)-N-{(2S)-4-[2-(5-fluoro-2-oxopyrimidin-1(2H)-yl)acetamido]-2-hydroxybicyclo[2.2.2]octan-1-yl}acetamide (Compound 646)

A mixture of Example 476A (0.05 g, 0.119 mmol), 5-fluoropyrimidin-2-ol (0.027 g, 0.239 mmol), potassium carbonate (0.033 g, 0.239 mmol) and potassium iodide (1.386 mg, 8.35 µmol) in acetone (1.0 mL) was heated at 140 °C for 45 minutes in Biotage^{®} Initiator microwave reactor. The mixture was concentrated, and the residue was purified by HPLC (Phenomenex^{®} Luna^{®} C18(2) 10 µm 100Å AXIA^{™} column (250 mm × 50 mm). A 30-100% gradient of acetonitrile (A) and 0.1% trifluoroacetic acid in water (B) was used over 25 minutes, at a flow rate of 50 mL/minute) to give 23.0 mg of the title compound. ¹H NMR (400 MHz, DMSO-*d*₆) *δ* ppm 8.81 (d, J = 3.8 Hz, 1H), 8.39 (t, J = 4.0 Hz, 1H), 7.88 (s, 1H), 7.48 (t, J = 8.9 Hz, 1H), 7.26 (s, 1H), 7.05 (dd, J = 11.3, 2.8 Hz, 1H), 6.83 (dd, J = 9.1, 2.7 Hz, 1H), 4.46 (s, 2H), 4.40 (s, 2H), 4.03 (dd, J = 9.6, 3.4 Hz, 1H), 2.26 (ddd, J = 12.5, 9.4, 2.5 Hz, 1H), 2.06 (ddd, J = 12.5, 10.7, 5.0 Hz, 1H), 1.96 - 1.69 (m, 8H); MS (ESI⁺) *m*/*z* 497.0 (M+H)⁺.

### Example 548: 2-(4-chloro-3-fluorophenoxy)-N-[(2S)-4-{[(5-fluoro-1H-benzimidazol-2-yl)methyl]amino}-2-hydroxybicyclo[2.2.2]octan-1-yl]acetamide (Compound 647)

### Example 548A: (S)-(9H-fluoren-9-yl)methyl (4-amino-3-hydroxybicyclo[2.2.2]octan-1-yl)carbamate, Hydrochloric Acid

To a suspension of Example 445A (2.26 g, 7.72 mmol) and sodium carbonate (2.454 g, 23.16 mmol) in tetrahydrofuran (20.0 mL) and water (10.00 mL) at 0 °C was added 9*H*-fluoren-9-yl)methyl carbonochloridate (2.396 g, 9.26 mmol) at once. The mixture was stirred at 0 °C for 5 minutes, then allowed to warm up to ambient temperature and stir for 16 hours. The reaction mixture was diluted with ethyl acetate (100 mL), washed with water (100 mL) and brine (100 mL), dried over magnesium sulfate, and filtered. The filtrate was concentrated to give a solid. To a solution of the solid in methanol (15 mL) was added 4 N HCl in dioxane (9.65 mL, 38.6 mmol) followed by stirring at 50 °C for 2 hours. The mixture was concentrated, and the residue was triturated with ethyl acetate to give 3.46 g of the title compound. ¹H NMR (400 MHz, DMSO-*d*₆) *δ* ppm 7.87 (dd, J = 15.3, 7.6 Hz, 5H), 7.82 (s, 1H), 7.68 (dd, J = 10.5, 7.5 Hz, 3H), 7.46 7.36 (m, 3H), 7.32 (dtd, J = 13.4, 7.5, 1.2 Hz, 3H), 7.14 (s, 1H), 5.57 (s, 1H), 4.21 (s, 3H), 3.81 (s, 1H), 3.57 (s, 3H), 2.25 (s, 1H), 2.01 (s, 1H), 1.89 (s, 2H), 1.78 (s, 7H), 1.64 (s, 1H), 1.55 (s, 1H); MS (ESI⁺) *m*/*z* 379.4 (M+H)⁺.

### Example 548B: (S)-(9H-fluoren-9-yl)methyl (4-(2-(4-chloro-3-fluorophenoxy)acetamido)-3-hydroxybicyclo[2.2.2]octan-1-yl)carbamate

To a mixture of Example 548A (2.85 g, 7.53 mmol), 2-(4-chloro-3-fluorophenoxy)acetic acid (1.772 g, 8.66 mmol), and *N*-ethyl-*N*-isopropylpropan-2-amine (4.60 mL, 26.4 mmol) in *N,N*-dimethylformamide (35 mL), 2-(3*H*-[1,2,3]triazolo[4,5-b]pyridin-3-yl)-1,1,3,3-tetramethylisouronium hexafluorophosphate(V) (3.58 g, 9.41 mmol) was added, and the mixture was stirred at ambient temperature for 30 minutes. Water was added, and the suspension was stirred overnight. The precipitate was filtered, washed with water, and air-dried to afford 1.2 g of the title compound. ¹H NMR (400 MHz, DMSO-*d*₆) *δ* ppm 7.88 (dd, *J =* 7.5, 1.1 Hz, 3H), 7.70 (d, *J =* 7.4 Hz, 3H), 7.53 - 7.29 (m, 7H), 7.24 (s, 1H), 7.06 (dd, *J =* 11.4, 2.8 Hz, 2H), 6.83 (ddd, *J =* 9.0, 2.9, 1.2 Hz, 1H), 5.06 (s, 1H), 4.46 (s, 3H), 4.20 (s, 2H), 2.89 (s, 1H), 2.76 - 2.67 (m, 3H), 2.21 (s, 1H), 2.06 (d, *J =* 8.3 Hz, 1H), 1.88 (s, 2H), 1.74 (s, 2H); MS (ESI⁺) *m*/*z* 565.2 (M+H)⁺.

### Example 548C: (S)-N-(4-amino-2-hydroxybicyclo[2.2.2]octan-1-yl)-2-(4-chloro-3-fluorophenoxy)acetamide, Hydrochloric Acid

To a solution of Example 548B (57.6 g, 102 mmol) in acetonitrile (300 mL) was added diethylamine (100 mL, 967 mmol), and the mixture was stirred at ambient temperature for 75 minutes. The reaction mixture was concentrated, triturated with hot heptane (75 mL) leaving an orange gum which was stirred with hot *tert-*butyl methyl ether (50 mL) and cooled to 0 °C. The mixture was filtered, rinsed with *tert-*butyl methyl ether (30 mL), re-precipitated from ethyl acetate (125 mL), and dried to give a white powder. This material was dissolved in 0.33 M aqueous HCl (257 mL) and washed with ethyl acetate (2×100 mL). The aqueous layer was concentrated, and triturated with *tert-*butyl methyl ether (2x50 mL) to give the title compound. The filtrate was concentrated, diluted with acetonitrile (100 mL), washed with heptane (4×40 mL) to remove nonpolar by-products, combined with the mother liquor from the ethyl acetate precipitation, and concentrated to give 18g of gummy solid, which was chromatographed (10% methanol/dichloromethane, 1.5% triethylamine) to give additional impure title compound. This material was dissolved in isopropanol (~50 mL), stirred with 4 M HCl in dioxane (4 mL), and concentrated to give a residue, which was dissolved in water (50 mL), washed with ethyl acetate (2×25 mL), and concentrated to give additional title compound which was combined with the initially-obtained material. The combined batches of title compound were stirred with hot isopropanol (76 mL), diluted with hot acetonitrile (170 mL), filtered, concentrated, and azeotroped with toluene to give the title compound as a white solid (30.8185 g, 81 mmol, 80 % yield). ¹H NMR (400 MHz, DMSO-*d*₆) *δ* ppm 8.11 (s, 2H), 7.73 - 7.41 (m, 2H), 7.06 (dd, *J* = 11.4, 2.8 Hz, 1H), 6.83 (ddd, *J* = 9.0, 2.9, 1.2 Hz, 1H), 5.33 (s, 1H), 4.48 (d, *J =* 1.2 Hz, 2H), 4.16 - 4.06 (m, 1H), 3.14 (d, *J =* 24.1 Hz, 1H), 2.38 - 2.02 (m, 3H), 2.01 - 1.53 (m, 7H); MS (ESI⁺) *m*/*z* 343.3 (M+H)⁺.

### Example 548D: 2-(4-chloro-3-fluorophenoxy)-N-[(2S)-4-{[(5-fluoro-1H-benzimidazol-2-yl)methyl]amino}-2-hydroxybicyclo[2.2.2]octan-1-yl]acetamide, Trifluoroacetic Acid

To a mixture of Example 548C (51 mg, 0.134 mmol) and 5-fluoro-1*H-*benzo*[*d]imidazole-2-carbaldehyde (55.2 mg, 0.336 mmol) in buffer solution((2.4 weight % sodium acetate trihydrate and 3.6 weight % acetic acid in methanol, 2.0 mL), sodium cyanotrihydroborate (25.4 mg, 0.403 mmol) was added, and the mixture was stirred at ambient temperature for 16 hours. The reaction mixture was concentrated under high vacuum and the residue was purified by HPLC (Phenomenex^{®} Luna^{®} C18(2) 10 µm 100Å AXIA^{™} column (250 mm × 50 mm). A 10-95% gradient of acetonitrile (A) and 0.1% trifluoroacetic acid in water (B) was used over 25 minutes, at a flow rate of 50 mL/minute) to give 16 mg of the title compound. ¹H NMR (400 MHz, DMSO-*d*₆) *δ* ppm 9.40 (s, 2H), 7.64 (dd, J = 8.8, 4.9 Hz, 1H), 7.53 7.41 (m, 2H), 7.41 (s, 1H), 7.17 7.02 (m, 2H), 6.88 6.77 (m, 1H), 4.49 (s, 2H), 4.36 (s, 2H), 4.20 (dd, J = 9.5, 2.8 Hz, 1H), 2.38 2.27 (m, 1H), 2.07 (dd, J = 11.1, 4.8 Hz, 1H), 2.01 (d, J = 10.6 Hz, 1H), 1.99 1.85 (m, 5H), 1.81 (s, 1H), 1.72 (dt, J = 12.9, 3.0 Hz, 1H); MS (ESI⁺) *m*/*z* 491.4 (M+H)⁺.

### Example 549: 2-(4-chloro-3-fluorophenoxy)-N-[(2S)-4-{[(5,6-difluoro-1H-benzimidazol-2-yl)methyl]amino}-2-hydroxybicyclo[2.2.2]octan-1-yl]acetamide (Compound 648)

The title compound was synthesized using the procedure described in Example 548D substituting 5,6-difluoro-1*H*-benzo[*d*]imidazole-2-carbaldehyde for 5-fluoro-1*H-*benzo[*d*]imidazole-2-carbaldehyde. ¹H NMR (400 MHz, DMSO-*d*₆) *δ* ppm 9.42 (s, 2H), 7.91 (dd, J = 14.8, 6.8 Hz, 1H), 7.79 7.69 (m, 1H), 7.49 (t, J = 8.8 Hz, 1H), 7.41 (s, 1H), 7.06 (dd, J = 11.4, 2.8 Hz, 1H), 6.84 (ddd, J = 9.0, 2.9, 1.2 Hz, 1H), 5.34 (s, 1H), 4.49 (s, 2H), 4.37 (s, 2H), 4.19 (d, J = 8.5 Hz, 1H), 2.32 (t, J = 11.1 Hz, 1H), 2.13 1.98 (m, 3H), 1.89 (s, 4H), 1.80 (d, J = 10.1 Hz, 1H), 1.71 (d, J = 12.8 Hz, 1H); MS (ESI⁺) *m*/*z* 509.3 (M+H)⁺.

### Example 550: rac-2-(4-chloro-3-fluorophenoxy)-N-[(1R,3R,4R,5R)-4-{[2-(4-chloro-3-fluorophenoxy)ethyl]amino}-3-hydroxybicyclo[3.2.1]octan-1-yl]acetamide (Compound 649)

To a solution of Example 526A (0.0604 g, 0.118 mmol) in tetrahydrofuran (0.60 mL) at 0 °C was added borane dimethyl sulfide complex (0.037 mL, 0.390 mmol), and the mixture was stirred overnight gradually warming to ambient temperature. The mixture was cooled to 0 °C, quenched with methanol (0.3 mL), 1 N NaOH (1.1 mL), and 30% H₂O₂ (0.4 mL), stirred for 5 minutes, allowed to warm for 30 minutes, and heated to 50 °C for 1 hour. The mixture was diluted with ethyl acetate, washed with water and brine, dried (Na₂SO₄), and concentrated. The residue was chromatographed on silica gel (5% methanol/dichloromethane) and rechromatographed on silica gel (66% acetonitrile/dichloromethane - 7% isopropanol/dichloromethane) to give the title compound (0.0083 g, 0.016 mmol, 13.63 % yield). ¹H NMR (500 MHz, CDCl₃) *δ* ppm 7.32 (dd, J = 10.1, 7.2 Hz, 1H), 7.29 - 7.23 (m, 1H), 6.78 - 6.69 (m, 2H), 6.66 (tdd, J = 9.6, 2.9, 1.2 Hz, 2H), 6.52 (s, 1H), 4.37 (s, 2H), 4.07 - 3.99 (m, 2H), 3.41 (ddd, J = 10.7, 8.9, 6.2 Hz, 1H), 3.16 (ddd, J = 12.7, 6.7, 4.5 Hz, 1H), 2.86 (ddd, J = 12.7, 5.4, 4.2 Hz, 1H), 2.45 - 2.31 (m, 3H), 2.09 - 2.01 (m, 1H), 2.01 - 1.92 (m, 1H), 1.92 - 1.55 (m, 5H+water); MS (ESI⁺) *m*/*z* 515.0 (M+H)⁺.

### Example 551: 3-(4-chloro-3-fluorophenoxy)-N-{(2S)-4-[2-(4-chloro-3-fluorophenoxy)acetamido]-2-hydroxybicyclo[2.2.2]octan-1-yl}propanamide (Compound 650)

The title compound was prepared using the methodologies described above. ¹H NMR (501 MHz, DMSO-*d*₆) *δ* ppm 7.50 - 7.43 (m, 3H), 7.38 (s, 1H), 7.06 - 7.00 (m, 2H), 6.82 - 6.79 (m, 2H), 5.14 (d, J = 3.4 Hz, 1H), 4.43 (s, 2H), 4.20 - 4.11 (m, 2H), 4.08 - 4.03 (m, 1H), 2.56 - 2.52 (m, 2H), 2.28 - 2.21 (m, 1H), 2.02 - 1.64 (m, 9H); MS (APCI⁺) *m*/*z* 543 (M+H)⁺.

### Example 552: 2-(4-chloro-3-fluorophenoxy)-N-[(2S)-2-hydroxy-4-{2-[(6-methoxypyridin-3-yl)oxy]acetamido}bicyclo[2.2.2]octan-1-yl]acetamide (Compound 651)

The title compound was synthesized using the procedure described in Example 538 substituting 6-methoxypyridin-3-ol for 2-methoxypyrimidin-5-ol. ¹H NMR (400 MHz, DMSO-*d*₆) *δ* ppm 7.81 (d, J = 3.0 Hz, 1H), 7.53 7.43 (m, 2H), 7.38 (dd, J = 9.0, 3.1 Hz, 1H), 7.26 (s, 1H), 7.05 (dd, J = 11.4, 2.8 Hz, 1H), 6.83 (dd, J = 8.9, 3.0 Hz, 1H), 6.77 (d, J = 9.0 Hz, 1H), 4.42 (d, J = 33.7 Hz, 4H), 4.04 (dd, J = 9.5, 3.1 Hz, 1H), 3.79 (s, 2H), 2.28 (ddd, J = 12.0, 9.4, 2.2 Hz, 1H), 2.07 (ddd, J = 12.2, 10.4, 4.8 Hz, 1H), 1.97 1.73 (m, 3H); MS (ESI⁺) *m*/*z* 508.2 (M+H)⁺.

### Example 553: N,N'-[(1R,4R,5R)-3-oxobicyclo[3.2.1]octane-1,4-diyl]bis[2-(4-chloro-3-fluorophenoxy)acetamide] (Compound 652)

To a suspension of Example 550 (0.0605 g, 0.114 mmol) in CH₂Cl₂ (0.60 mL) at ambient temperature was added Dess-Martin periodinane (0.0995 g, 0.235 mmol), and the mixture was stirred for 90 minutes. Then the mixture was quenched with isopropanol, diluted with ethyl acetate, washed with saturated Na₂S₂O₃, 1 N NaOH, and brine, dried (Na₂SO₄), and concentrated. The residue was chromatographed on silica gel (50% ethyl acetate/dichloromethane) to give the title compound (0.0450 g, 0.085 mmol, 74.7 % yield). ¹H NMR (400 MHz, CDCl₃) *δ* ppm 7.49 (d, J = 5.3 Hz, 1H), 7.33 (td, J = 8.6, 4.1 Hz, 2H), 6.83 - 6.69 (m, 3H), 6.67 (ddd, J = 7.1, 3.5, 1.3 Hz, 2H), 4.54 - 4.46 (m, 3H), 4.40 (s, 2H), 3.33 (d, J = 14.5 Hz, 1H), 3.05 - 2.96 (m, 1H), 2.83 - 2.70 (m, 2H), 2.11 (ddd, J = 12.1, 5.7, 3.5 Hz, 1H), 2.06 - 1.96 (m, 1H), 1.86 - 1.74 (m, 2H), 1.37 (td, J = 12.1, 10.8, 6.9 Hz, 1H); MS (ESI⁺) *m*/*z* 527.2 (M+H)⁺.

### Example 554: rac-N,N'-[(1S,4S,5R)-bicyclo[3.2.1]octane-1,4-diyl]bis[2-(4-chloro-3-fluorophenoxy)acetamide] (Compound 653)

Example 526A ( 0.0318 g, 0.062 mmol) and ethyl acetate (2 mL) were added to 5% Pt/C wet, RD0585 (0.030 g, 0.062 mmol) in a 20 mL Barnstead Hast C reactor and stirred for 1.1 hours under hydrogen (50 psi) at 25 °C. The mixture was filtered, and the filtrate was concentrated to give the title compound (0.0098 g, 0.019 mmol, 30.7 % yield). ¹H NMR (400 MHz, DMSO-*d*₆) *δ* ppm 7.97 (s, 1H), 7.91 (d, J = 7.7 Hz, 1H), 7.47 (td, J = 8.9, 0.8 Hz, 2H), 7.03 (dt, J = 11.4, 3.1 Hz, 2H), 6.82 (dtd, J = 9.0, 2.9, 1.2 Hz, 2H), 4.50 (s, 2H), 4.46 (s, 2H), 3.71 (brs, 1H), 2.12 (brs, 1H), 1.94 - 1.82 (m, 3H), 1.78 - 1.54 (m, 6H), 1.54 - 1.40 (m, 1H); MS (ESI⁺) *m*/*z* 513.4 (M+H)⁺.

### Example 555: N,N'-(spiro[bicyclo[2.2.2]octane-2,2'-oxirane]-1,4-diyl)bis[2-(4-chloro-3-fluorophenoxy)acetamide] (Compound 654)

To a suspension of trimethylsulfoxonium iodide (0.0966 g, 0.439 mmol) in *N,N-*dimethylformamide (0.50 mL) was added a 1 M solution of potassium 2-methylpropan-2-olate in tetrahydrofuran (0.65 mL, 0.650 mmol). The mixture was stirred 30 minutes, and solid *N,N'*-(2-oxobicyclo[2.2.2]octane-1,4-diyl)bis(2-(4-chloro-3-fluorophenoxy)acetamide) (Example 599A, 0.1151 g, 0.218 mmol) was added. The mixture was stirred overnight, diluted with ethyl acetate, washed with water and brine, dried (Na₂SO₄), and concentrated. The residue was chromatographed on silica gel (15% ethyl acetate/dichloromethane) to give the title compound (0.0784 g, 0.145 mmol, 66.3 % yield). ¹H NMR (501 MHz, DMSO-*d*₆) *δ* ppm 7.70 (s, 1H), 7.47 (td, J = 8.9, 2.7 Hz, 2H), 7.01 (ddd, J = 11.4, 3.7, 2.9 Hz, 2H), 6.94 (s, 1H), 6.80 (dddd, J = 8.3, 7.0, 2.9, 1.1 Hz, 2H), 4.47 (s, 2H), 4.44 (s, 2H), 2.70 (d, J = 4.4 Hz, 1H), 2.63 (d, J = 4.4 Hz, 1H), 2.41 - 2.31 (m, 2H), 2.15 - 2.01 (m, 3H), 1.97 - 1.81 (m, 5H); MS (ESI⁺) *m*/*z* 541.4 (M+H)⁺.

### Example 556: 2-(4-chloro-3-fluorophenoxy)-N-(4-{2-[(2-methoxypyrimidin-5-yl)oxy]acetamido}-2-oxobicyclo[2.2.2]octan-1-yl)acetamide (Compound 655)

A suspension of Example 538 (0.12 g, 0.236 mmol) and Dess-Martin periodinane (1,1,1-tris(acetyloxy)-1,1-dihydro-1,2-benzodioxol-3-(1*H*)-one) (0.200 g, 0.472 mmol) in dichloromethane (4 mL) was stirred at ambient temperature for 2 hours. Then the reaction was quenched with isopropanol (~0.3 mL), saturated Na₂S₂O₃ aqueous solution (2 mL), and the mixture was stirred for 5 minutes. The precipitate was collected by filtration, washed with methanol and air-dried to give the title compound (0.1 g, 0.197 mmol, 84 % yield). ¹H NMR (400 MHz, DMSO-*d*₆) *δ* ppm 8.34 (s, 2H), 7.95 (s, 1H), 7.71 (s, 1H), 7.49 (t, J = 8.9 Hz, 1H), 7.08 (dd, J = 11.3, 2.9 Hz, 1H), 6.85 (ddd, J = 9.0, 2.9, 1.2 Hz, 1H), 4.55 (d, J = 9.9 Hz, 4H), 3.86 (s, 3H), 2.87 (s, 2H), 2.48 2.37 (m, 2H), 2.14 1.95 (m, 4H), 1.86 (s, 1H), 1.90 1.80 (m, 1H); MS (ESI⁺) *m*/*z* 507.4 (M+H)⁺.

### Example 557: 2-(4-chloro-3-fluorophenoxy)-N-[(3S)-4-{2-[(4-chlorophenyl)(methyl)amino]acetamido}-3-hydroxybicyclo[2.2.2]octan-1-yl]acetamide (Compound 656)

The reaction and purification conditions described in Example 83C substituting 2-((4-chlorophenyl)(methyl)amino)acetic acid hydrochloride (commercially available from Enamine) for the product of Example 83B and the product of Example 540A for Example 9A gave the title compound. ¹H NMR (400 MHz, methanol-*d*₄) *δ* ppm 7.34 (t, J = 8.7 Hz, 1H), 7.16 - 7.11 (m, 2H), 6.87 (dd, J = 11.0, 2.9 Hz, 1H), 6.76 (ddd, J = 8.9, 2.9, 1.3 Hz, 1H), 6.69 - 6.62 (m, 2H), 4.38 (s, 2H), 4.12 (ddd, J = 9.5, 3.3, 1.5 Hz, 1H), 3.88 - 3.74 (m, 2H), 2.98 (s, 3H), 2.44 (ddd, J = 13.4, 9.4, 2.8 Hz, 1H), 2.15 - 1.78 (m, 9H); MS (APCI⁺) *m*/*z* 524 (M+H)⁺.

### Example 558: 2-(4-chloroanilino)-N-{(2S)-4-[2-(4-chloro-3-fluorophenoxy)acetamido]-2-hydroxybicyclo[2.2.2]octan-1-yl}acetamide (Compound 657)

The reaction and purification conditions described in Example 83C substituting 2-((4-chlorophenyl)amino)acetic acid (commercially available from Enamine) for the product of Example 83B and the product of Example 540A for Example 9A gave the title compound. ¹H NMR (400 MHz, DMSO-*d*₆) *δ* ppm 7.51 - 7.44 (m, 2H), 7.22 (s, 1H), 7.13 - 7.08 (m, 2H), 7.01 (dd, J = 11.4, 2.8 Hz, 1H), 6.80 (ddd, J = 9.0, 2.9, 1.2 Hz, 1H), 6.56 - 6.50 (m, 2H), 6.08 (t, J = 5.8 Hz, 1H), 5.10 (d, J = 4.1 Hz, 1H), 4.42 (s, 2H), 3.96 - 3.89 (m, 1H), 3.60 - 3.48 (m, 2H), 2.30 - 2.20 (m, 1H), 2.12 - 2.01 (m, 1H), 1.96 - 1.69 (m, 8H); MS (ESI⁺) *m*/*z* 510 (M+H)⁺.

### Example 559: 2-(4-chloro-3-fluorophenoxy)-N-[(3S)-3-hydroxy-4-{2-[(1-methyl-1H-pyrazol-4-yl)oxy]acetamido}bicyclo[2.2.2]octan-1-yl]acetamide (Compound 658)

The title compound was prepared using the methodologies described above. ¹H NMR (500 MHz, DMSO-*d*₆) *δ* ppm 7.53 (s, 1H), 7.49 (t, J = 8.9 Hz, 1H), 7.45 (d, J = 0.9 Hz, 1H), 7.19 (d, J = 0.9 Hz, 1H), 7.11 (s, 1H), 7.03 (dd, J = 11.4, 2.8 Hz, 1H), 6.81 (ddd, J = 9.0, 2.9, 1.2 Hz, 1H), 5.21 - 5.15 (m, 1H), 4.44 (s, 2H), 4.26 - 4.16 (m, 2H), 4.01 - 3.95 (m, 1H), 3.73 (s, 3H), 2.28 (ddd, J = 12.7, 9.4, 2.9 Hz, 1H), 2.23 - 2.13 (m, 1H), 1.99 - 1.73 (m, 8H); MS (APCI⁺) *m*/*z* 481 (M+H)⁺.

### Example 560: 2-(4-chloro-3-fluorophenoxy)-N-{(3S)-4-[2-(3-fluorophenoxy)acetamido]-3-hydroxybicyclo[2.2.2]octan-1-yl}acetamide (Compound 659)

The title compound was prepared using the methodologies described above. ¹H NMR (400 MHz, DMSO-*d*₆) *δ* ppm 7.51 (s, 1H), 7.48 (t, J = 8.9 Hz, 1H), 7.36 - 7.28 (m, 1H), 7.24 (s, 1H), 7.02 (dd, J = 11.4, 2.9 Hz, 1H), 6.84 - 6.76 (m, 4H), 5.12 (d, J = 4.2 Hz, 1H), 4.44 (s, 2H), 4.43 (s, 2H), 4.05 - 3.99 (m, 1H), 2.28 (ddd, J = 12.6, 9.5, 2.4 Hz, 1H), 2.10 (ddd, J = 13.3, 9.2, 2.7 Hz, 1H), 1.97 - 1.70 (m, 8H); MS (APCI⁺) *m*/*z* 495 (M+H)⁺.

### Example 561: 2-(4-chloro-3-fluorophenoxy)-N-{(3S)-4-[2-(3-chlorophenoxy)acetamido]-3-hydroxybicyclo[2.2.2]octan-1-yl}acetamide (Compound 660)

The title compound was prepared using the methodologies described above. ¹H NMR (501 MHz, DMSO-*d*₆) *δ* ppm 7.51 (s, 1H), 7.48 (t, J = 8.9 Hz, 1H), 7.34 - 7.29 (m, 1H), 7.25 (s, 1H), 7.04 - 7.00 (m, 3H), 6.91 (ddd, J = 8.4, 2.4, 0.9 Hz, 1H), 6.81 (ddd, J = 9.0, 2.9, 1.1 Hz, 1H), 5.11 (d, J = 4.0 Hz, 1H), 4.45 (s, 2H), 4.43 (s, 2H), 4.05 - 3.99 (m, 1H), 2.27 (ddd, J = 12.5, 9.4, 2.6 Hz, 1H), 2.14 - 2.06 (m, 1H), 1.99 - 1.74 (m, 8H); MS (APCI⁺) *m*/*z* 511 (M+H)⁺.

### Example 562: N-[(2S)-2-hydroxy-4-{2-[(pyridin-2-yl)oxy]acetamido}bicyclo[2.2.2]octan-1-yl]-2-[(1-methyl-1H-pyrazol-4-yl)oxy]acetamide (Compound 661)

The title compound was prepared using the methodologies described above. ¹H NMR (400 MHz, DMSO-*d*₆) *δ* ppm 8.11 (ddd, J = 5.0, 2.0, 0.8 Hz, 1H), 7.72 (ddd, J = 8.4, 7.1, 2.0 Hz, 1H), 7.44 (d, J = 0.9 Hz, 1H), 7.40 (s, 1H), 7.18 (d, J = 0.9 Hz, 1H), 7.08 (s, 1H), 6.98 (ddd, J = 7.1, 5.0, 0.9 Hz, 1H), 6.87 (dt, J = 8.4, 0.9 Hz, 1H), 5.14 (d, J = 4.6 Hz, 1H), 4.61 (s, 2H), 4.26 - 4.14 (m, 2H), 4.00 - 3.92 (m, 1H), 3.72 (s, 3H), 2.32 - 2.10 (m, 2H), 1.95 - 1.84 (m, 2H), 1.87 - 1.71 (m, 6H); MS (APCI⁺) *m*/*z* 430 (M+H)⁺.

### Example 563: 2-(3-chlorophenoxy)-N-[(2S)-2-hydroxy-4-{2-[(pyridin-2-yl)oxy]acetamido}bicyclo[2.2.2]octan-1-yl]acetamide (Compound 662)

The title compound was prepared using the methodologies described above. ¹H NMR (400 MHz, DMSO-*d*₆) *δ* ppm 8.10 (ddd, J = 5.0, 2.1, 0.8 Hz, 1H), 7.71 (ddd, J = 8.7, 7.1, 2.0 Hz, 1H), 7.41 (s, 1H), 7.34 - 7.28 (m, 1H), 7.24 (s, 1H), 7.05 - 6.96 (m, 3H), 6.93 - 6.89 (m, 1H), 6.89 - 6.85 (m, 1H), 5.16 - 5.06 (m, 1H), 4.60 (s, 2H), 4.50 - 4.39 (m, 2H), 4.03 - 3.95 (m, 1H), 2.26 (ddd, J = 12.5, 9.5, 2.4 Hz, 1H), 2.14 - 2.04 (m, 1H), 1.96 - 1.69 (m, 8H); MS (APCI⁺) *m*/*z* 460 (M+H)⁺.

### Example 564: 2-(4-chloro-3-fluorophenoxy)-N-[(2S)-2-hydroxy-4-{2-[(pyridin-2-yl)oxy]acetamido}bicyclo[2.2.2]octan-1-yl]acetamide (Compound 663)

The title compound was prepared using the methodologies described above. ¹H NMR (501 MHz, DMSO-*d*₆) *δ* ppm 8.11 (ddd, J = 5.0, 2.0, 0.8 Hz, 1H), 7.72 (ddd, J = 8.4, 7.1, 2.0 Hz, 1H), 7.48 (t, J = 8.9 Hz, 1H), 7.40 (s, 1H), 7.25 (s, 1H), 7.05 (dd, J = 11.4, 2.9 Hz, 1H), 6.98 (ddd, J = 7.1, 5.0, 0.9 Hz, 1H), 6.89 - 6.85 (m, 1H), 6.83 (ddd, J = 8.9, 2.9, 1.1 Hz, 1H), 5.10 - 5.04 (m, 1H), 4.61 (s, 2H), 4.50 - 4.42 (m, 2H), 4.05 - 3.99 (m, 1H), 2.26 (ddd, J = 12.4, 9.5, 2.4 Hz, 1H), 2.10 - 2.01 (m, 1H), 1.94 - 1.69 (m, 8H); MS (APCI⁺) *m*/*z* 478 (M+H)⁺.

### Example 565: 2-(3,4-dichlorophenoxy)-N-[(2S)-2-hydroxy-4-{2-[(5-methyl-1,2-oxazol-3-yl)oxy]acetamido}bicyclo[2.2.2]octan-1-yl]acetamide (Compound 664)

The title compound was prepared using the methodologies described above. ¹H NMR (501 MHz, DMSO-*d*₆) *δ* ppm 7.52 - 7.49 (m, 2H), 7.24 (s, 1H), 7.21 (d, J = 2.9 Hz, 1H), 6.94 (dd, J = 9.0, 2.9 Hz, 1H), 5.95 (q, J = 0.8 Hz, 1H), 5.06 (d, J = 4.3 Hz, 1H), 4.46 (s, 2H), 4.45 (s, 2H), 4.03 - 3.98 (m, 1H), 2.28 (d, J = 0.9 Hz, 3H), 2.23 (ddd, J = 12.5, 9.4, 2.5 Hz, 1H), 2.07 - 1.99 (m, 1H), 1.93 - 1.70 (m, 8H); MS (APCI⁺) *m*/*z* 498 (M+H)⁺.

### Example 566: 2-(4-chloro-3-fluorophenoxy)-N-[(2S)-2-hydroxy-4-{2-[(5-methyl-1,2-oxazol-3-yl)oxy]acetamido}bicyclo[2.2.2]octan-1-yl]acetamide (Compound 665)

The title compound was prepared using the methodologies described above. ¹H NMR (400 MHz, DMSO-*d*₆) *δ* ppm 7.53 (s, 1H), 7.48 (t, J = 8.9 Hz, 1H), 7.26 (s, 1H), 7.05 (dd, J = 11.4, 2.8 Hz, 1H), 6.83 (ddd, J = 9.0, 2.9, 1.2 Hz, 1H), 5.99 - 5.97 (m, 1H), 5.10 - 5.05 (m, 1H), 4.48 (s, 2H), 4.46 (s, 2H), 4.06 - 3.99 (m, 1H), 2.31 - 2.30 (m, 3H), 2.29 - 2.21 (m, 1H), 2.11 - 1.99 (m, 1H), 1.97 - 1.72 (m, 8H); MS (APCI⁺) *m*/*z* 482 (M+H)⁺.

### Example 567: 2-(4-chloro-3-fluorophenoxy)-N-[(2S)-2-hydroxy-4-{2-[(6-methoxypyrimidin-4-yl)oxy]acetamido}bicyclo[2.2.2]octan-1-yl]acetamide (Compound 666)

The title compound was synthesized using the procedure described in Example 538 substituting 6-methoxypyrimidin-4-ol for 2-methoxypyrimidin-5-ol. ¹H NMR (400 MHz, DMSO-*d*₆) *δ* ppm 8.22 (s, 1H), 7.85 (s, 1H), 7.48 (t, J = 8.9 Hz, 1H), 7.25 (s, 1H), 7.05 (dd, J = 11.4, 2.9 Hz, 1H), 6.82 (ddd, J = 9.0, 2.9, 1.2 Hz, 1H), 5.59 (s, 1H), 4.45 (d, J = 12.4 Hz, 4H), 4.02 (d, J = 7.7 Hz, 1H), 3.79 (s, 3H), 2.24 (t, J = 11.4 Hz, 1H), 1.90 (d, J = 6.2 Hz, 2H), 1.81 (dd, J = 26.2, 14.5 Hz, 1H), 1.77 (s, 5H), 1.71 (d, J = 13.2 Hz, 1H); MS (ESI⁺) *m*/*z* 509.2 (M+H)⁺.

### Example 568: 2-(4-chloro-3-fluorophenoxy)-N-[(2S)-2-hydroxy-4-{2-[(5-methoxypyridin-2-yl)oxy]acetamido}bicyclo[2.2.2]octan-1-yl]acetamide (Compound 667)

The title compound was synthesized using the procedure described in Example 538 substituting 5-methoxypyridin-2-ol for 2-methoxypyrimidin-5-ol. ¹H NMR (400 MHz, DMSO-*d*₆) *δ* ppm 7.72 (s, 1H), 7.48 (t, J = 8.9 Hz, 1H), 7.29 (dd, J = 9.8, 3.2 Hz, 1H), 7.26 7.20 (m, 2H), 7.05 (dd, J = 11.4, 2.9 Hz, 1H), 6.83 (dt, J = 8.9, 2.0 Hz, 1H), 6.32 (d, J = 9.8 Hz, 1H), 4.46 (s, 2H), 4.39 (s, 2H), 4.02 (dd, J = 9.6, 3.2 Hz, 1H), 3.61 (s, 3H), 2.25 (ddd, J = 12.6, 9.5, 2.4 Hz, 1H), 2.06 (ddd, J = 12.4, 10.4, 4.9 Hz, 1H), 1.93 1.83 (m, 2H), 1.85 1.72 (m, 5H), 1.76 1.68 (m, 1H); MS (ESI⁺) *m*/*z* 508.4 (M+H)⁺.

### Example 569: N-{3-[2-(4-chloro-3-fluorophenoxy)acetamido]bicyclo[1.1.1]pentan-1-yl}-1-(4-chlorophenyl)cyclopropane-1-carboxamide (Compound 668)

To a mixture of Example 9B (0.020 g, 0.070 mmol) and 1-(4-chlorophenyl)cyclopropanecarboxylic acid (0.015 g, 0.077 mmol) in N,N-dimethylformamide (0.40 mL) was added triethylamine (0.040 mL, 0.28 mmol) followed by HATU (1-[bis(dimethylamino)methylene]-1*H*-1,2,3-triazolo[4,5-*b*]pyridinium 3-oxid hexafluorophosphate) (0.029 g, 0.077 mmol). This mixture was allowed to stir at ambient temperature overnight, was diluted with *N,N*-dimethylformamide/water (3:1, 1.2 mL), filtered, and purified by preparative HPLC [Waters XBridge^{™} C18 5 µm OBD column, 30 × 100 mm, flow rate 40 mL/minute, 5-100% gradient of acetonitrile in buffer (0.025 M aqueous ammonium bicarbonate, adjusted to pH 10 with ammonium hydroxide)] to give the title compound (0.027 g, 0.058 mmol, 83% yield). ¹H NMR (501 MHz, DMSO-*d*₆) *δ* ppm 8.65 (s, 1H), 7.51 (s, 1H), 7.48 (t, J = 8.9 Hz, 1H), 7.40 - 7.34 (m, 2H), 7.34 - 7.28 (m, 2H), 7.06 (dd, J = 11.3, 2.9 Hz, 1H), 6.84 (ddd, J = 8.9, 2.8, 1.1 Hz, 1H), 4.45 (s, 2H), 2.17 (s, 6H), 1.32 (q, J = 3.9 Hz, 2H), 0.93 (q, J = 4.0 Hz, 2H); MS (ESI⁺) *m*/*z* 463 (M+H)⁺.

### Example 570: (2E)-{1,4-bis[2-(4-chloro-3-fluorophenoxy)acetamido]bicyclo[2.2.2]octan-2-ylidene}acetic acid (Compound 669)

To a solution of Example 573 (40.0 mg, 0.067 mmol) in tetrahydrofuran (THF) (1 mL) and methanol (0.8 mL) was added a solution of lithium hydroxide monohydrate (8.43 mg, 0.201 mmol) in water (0.6 mL). The mixture was stirred for 5 hours. The reaction mixture was concentrated, and the residue was purified by HPLC (see protocol in Example 529D) to provide the title compound (13.4 mg, 35%). ¹H NMR (400 MHz, pyridine-*d*₅) *δ* ppm 8.26 (s, 1H), 8.11 (s, 1H), 7.34 (dt, J = 22.9, 8.7 Hz, 2H), 7.06 - 6.91 (m, 2H), 6.79 (dddd, J = 34.1, 8.9, 3.0, 1.2 Hz, 2H), 6.39 (t, J = 2.5 Hz, 1H), 4.78 (s, 2H), 4.67 (s, 2H), 3.92 (d, J = 2.5 Hz, 2H), 2.70 (td, J = 11.4, 10.9, 5.2 Hz, 2H), 2.30 (pd, J = 11.9, 11.4, 5.0 Hz, 4H), 2.15 - 1.95 (m, 2H); MS (ESI⁺) *m*/*z* 567.0 (M+H)⁺.

### Example 571: N,N'-(2-hydroxybicyclo[3.2.1]octane-1,5-diyl)bis[2-(4-chloro-3-fluorophenoxy)acetamide] (Compound 670)

To a solution of Example 572F (0.11 g, 0.209 mmol) in methanol (2.0 mL) and dichloromethane (2 mL) at ambient temperature was added sodium borohydride (0.020 g, 0.521 mmol), and the mixture was stirred for 0.5 hour. The volatiles were removed under vacuum. The residue was purified by HPLC (Phenomenex^{®} Luna^{®} C18(2) 10 µm 100Å AXIA^{™} column (250 mm × 50 mm). A 30-100% gradient of acetonitrile (A) and 0.1% trifluoroacetic acid in water (B) was used over 25 minutes, at a flow rate of 50 mL/minute) to give 76 mg of the title compound. ¹H NMR (400 MHz, DMSO-*d*₆) *δ* ppm 7.93 (d, J = 10.0 Hz, 1H), 7.53 (s, 1H), 7.44 (td, J = 8.9, 1.8 Hz, 2H), 7.01 (td, J = 11.5, 2.8 Hz, 2H), 6.79 (td, J = 8.6, 2.9 Hz, 2H), 4.51 4.38 (m, 4H), 3.79 (dd, J = 10.5, 5.8 Hz, 1H), 2.35 (dd, J = 11.2, 2.9 Hz, 1H), 2.03 (d, J = 11.2 Hz, 1H), 1.96 1.86 (m, 1H), 1.81 (q, J = 6.0, 4.9 Hz, 2H), 1.75 (dd, J = 11.2, 4.9 Hz, 2H), 1.75 1.58 (m, 1H), 1.61 1.45 (m, 1H), 1.37 1.17 (m, 1H); MS (ESI⁺) *m*/*z* 529.4 (M+H)⁺.

### Example 572: N,N'-(2-oxobicyclo[3.2.1]octane-1,5-diyl)bis[2-(4-chloro-3-fluorophenoxy)acetamide] (Compound 671)

### Example 572A: 8-((trimethylsilyl)ethynyl)-1,4-dioxaspiro[4.5]decan-8-ol

A solution of *n*-butyllithium (154 mL, 2.5 M in hexane, 384 mmol) was added to a solution of ethynyltrimethylsilane (37.7 g, 384 mmol) in tetrahydrofuran (800 mL) at -70°C under a nitrogen atmosphere dropwise. A solution of 1,4-dioxaspiro[4.5]decan-8-one (50 g, 320 mmol) in tetrahydrofuran (800 mL) was then added dropwise at -78°C with stirring for 0.5 hour. The resulting solution was stirred at the same temperature for 1.5 hours. The mixture was quenched by saturated aqueous solution of NH₄Cl and extracted with ethyl acetate (3 × 200 mL). The organic layers were combined and concentrated under reduced pressure at 40 °C to give the title compound (62.7 g, yield 77%). ¹H NMR (400 MHz, CDCl₃) *δ* ppm 3.94 (s, 4 H), 1.84 - 2.06 (m, 5 H), 1.74 - 1.82 (m, 4 H) 0.12 - 0.19 (m, 9 H).

### Example 572B: 8-ethynyl-1,4-dioxaspiro[4.5]decan-8-ol

To a solution of Example 572A (200 g, 629 mmol) in methanol (1.2 L) was added K₂CO₃ (174 g, 1258 mmol) at 0 °C, and the resulting mixture was stirred at ambient temperature for 2 hours. The solution was concentrated under reduced pressure at 40 °C, and the residue was diluted with water (100 mL), and extracted with ethyl acetate (3 × 200 mL). The combined organic layers were washed with brine (3 × 30 mL), dried over Na₂SO₄, and concentrated under reduced pressure at 45 °C. The residue was purified by flash column on silica gel (petroleum ether: ethyl acetate =4:1) to give the title compound (130 g, yield 91%). ¹H NMR (400 MHz, CDCl₃) *δ* ppm 3.94 (s, 4 H), 2.45 - 2.50 (m, 1 H) 2.33 (br s, 1 H), 1.89 - 2.01 (m, 4 H) 1.74 - 1.83 (m, 4 H).

### Example 572C: 1-(8-hydroxy-1,4-dioxaspiro[4.5]decan-8-yl)ethanone

To a solution of Example 572B (20 g, 88 mmol) in dichloromethane (100 mL) was added 20 g silica-supported (HgSO₄/H₂SO₄), 0.1 g of silica-supported (HgSO₄/H₂SO₄) (2.54 mmol sulfuric acid, 0.46 mmol HgSO₄ per gram of reagent, 5 mol%), and the mixture was stirred at 40 °C for 12 hours. The mixture was filtered, and the filtrate was concentrated under reduced pressure at 40 °C to give (17 g, yield 77%) of the title compound, which was used directly in the next step.

### Example 572D: 4-(benzylamino)-1-hydroxybicyclo[2.2.2]octan-2-one

To a solution of Example 572C (5 g, 25.6 mmol) in toluene(200 mL) was added benzylamine (8.23 g, 77mmol) and 4-methylbenzenesulfonic acid hydrate (0.049 g, 0.256mmol) at 20 °C in order, and the reaction mixture was stirred for 12 hours at 130 °C with a Dean-Stark trap. Volatiles were removed, and the residue was stirred with a mixture of ethyl acetate (50 mL) and aqueous HCl (150 mL, 3 N) for 30 minutes at 20 °C. The precipitate was collected by filtration, washed with a mixture of ethyl acetate/petroleum ether (1:3), dried under high vacuum to give the title compound (5 g, yield 63.7%). ¹H NMR (400 MHz, DMSO-*d*₆) *δ* ppm 7.26 - 7.39 (m, 4 H), 7.22 (br d, *J*=6.48 Hz, 1 H), 4.89 (br s, 1 H), 3.69 (br s, 2 H), 2.64 - 2.70 (m, 1 H), 2.31 - 2.47 (m, 3 H), 1.71 - 1.91 (m, 4 H), 1.71 - 1.93 (m, 1 H), 1.55 - 1.68 (m, 2 H).

### Example 572E: 4-amino-1-hydroxybicyclo[2.2.2]octan-2-one

To a solution of Example 572D (10 g, 35.5 mmol) in tetrahydrofuran (200 mL) was added palladium hydroxide on carbon (2.492 g, 17.74 mmol) under argon at 25 °C. The mixture was stirred at 25 °C under hydrogen (45 psi) for 12 hours. The reaction mixture was filtered through a pad of diatomaceous earth, and the cake was washed with methanol (500 mL). The filtrate was concentrated under reduced pressure at 45 °C. The residue was purified by preparative HPLC (Preparative HPLC was performed on a Gilson 281 semi-preparative HPLC system using a Phenomenex^{®} Luna^{®} C18(2) 10 um 100Å AXIA^{™} column (250 mm × 80 mm) column. A gradient of acetonitrile (A) and 0.075% trifluoroacetic acid in water (B) is used, at a flow rate of 80 mL/minute. A linear gradient is used from about 30% of A to about 100% of A over about 30 minutes. Detection method is UV at wave length of 220 nM and 254 nM) to give the title compound (3.5 g, yield 57.1%). ¹H NMR (400 MHz, DMSO-*d*₆) *δ* ppm 8.49 (br s, 3 H), 2.62 - 2.72 (m, 2 H), 2.00 - 2.09 (m, 2 H), 1.89 - 1.98 (m, 2 H), 1.84 (td, J=12.35, 4.40 Hz, 2 H), 1.63 - 1.73 (m, 2 H); MS (ESI⁺) *m*/*z* 156.1 (M+H)⁺.

### Example 572F: N,N'-(2-oxobicyclo[3.2.1]octane-1,5-diyl)bis[2-(4-chloro-3-fluorophenoxy)acetamide]

To a mixture of Example 572E (1.0 g, 5.22 mmol), 2-(4-chloro-3-fluorophenoxy)acetic acid (1.228 g, 6.00 mmol), and *N,N*-diisopropylethylamine (3.65 mL, 20.87 mmol) in *N,N-*dimethylformamide (15 mL) was added 1-[bis(dimethylamino)methylene]-1*H*-1,2,3-triazolo[4,5-*b*]pyridinium 3-oxid hexafluorophosphate (2.480 g, 6.52 mmol) portionwise, and the mixture was stirred at ambient temperature for 30 minutes. Then the mixture was diluted with dichloromethane (100 mL), washed with saturated ammonium chloride (100 mL), and concentrated. The residue was purified on silica gel (0-10% methanol/dichloromethane) to give 120 mg of the title compound as a byproduct. ¹H NMR (400 MHz, DMSO-*d*₆) *δ* ppm 7.66 (d, J = 17.6 Hz, 2H), 7.39 (td, J = 8.8, 2.1 Hz, 2H), 6.96 (ddd, J = 11.1, 7.9, 2.8 Hz, 2H), 6.82 (dddd, J = 9.0, 6.6, 2.9, 1.2 Hz, 2H), 4.49 (s, 2H), 4.44 (s, 2H), 3.19 (s, 1H), 2.81 2.59 (m, 2H), 2.40 2.09 (m, 8H), 2.09 1.90 (m, 2H); MS (ESI⁺) *m*/*z* 527.3 (M+H)⁺.

### Example 573: ethyl (2E)-{1,4-bis[2-(4-chloro-3-fluorophenoxy)acetamido]bicyclo[2.2.2]octan-2-ylidene}acetate (Compound 672)

To a solution of triethyl phosphonoacetate (0.532 mL, 2.67 mmol) in tetrahydrofuran (THF) (4 mL) was added 1 M (THF) LHMDS (lithium bis(trimethylsilyl)amide) (2.67 mL, 2.67 mmol) at 0 °C, and the reaction mixture was stirred at 0 °C for 1 hour. The solution was then added to a solution of Example 599A (128.0 mg, 0.243 mmol) in THF (6 mL) at -78 °C. The solution was allowed to warm to room temperature and stirred overnight. The reaction was quenched with brine and extracted with ethyl acetate (2×). The combined organic layers were washed with brine, dried over MgSO₄, filtered, concentrated. The residue was purified on a 12 g column using the Biotage^{®} Isolera^{™} One flash system eluting with heptanes/ethyl acetate (4:6 to 3:7) to give slightly impure product, which was further purified by HPLC (see protocol in Example 529D) to provide the title compound (88.8 mg, 61%). ¹H NMR (400 MHz, Pyridine-d5) δ ppm 8.30 (s, 1H), 8.12 (s, 1H), 7.34 (dtd, J = 17.5, 8.8, 1.0 Hz, 2H), 6.97 (ddd, J = 25.6, 11.0, 2.8 Hz, 2H), 6.79 (dddd, J = 31.0, 8.9, 2.9, 1.2 Hz, 2H), 6.15 (q, J = 1.9, 1.4 Hz, 1H), 4.80 (s, 2H), 4.67 (s, 2H), 4.12 (qd, J = 7.2, 1.2 Hz, 2H), 3.79 (d, J = 2.6 Hz, 2H), 2.68 (qd, J = 8.3, 7.5, 2.5 Hz, 2H), 2.28 (dq, J = 9.2, 5.8, 3.5 Hz, 4H), 2.01 (td, J = 10.9, 9.7, 6.2 Hz, 2H), 1.13 (td, J = 7.1, 1.0 Hz, 3H); MS (ESI⁺) *m*/*z* 614.1 (M+H)⁺.

### Example 574: N-[(2S)-4-{[(5,6-dimethyl-1H-benzimidazol-2-yl)methyl]amino}-2-hydroxybicyclo[2.2.2]octan-1-yl]-2-[3-fluoro-4-(trifluoromethyl)phenoxy]acetamide (Compound 673)

### Example 574A: 2-(3-fluoro-4-(trifluoromethyl)phenoxy)acetic acid

To a solution of 3-fluoro-4-(trifluoromethyl)phenol (0.9 g, 5.00 mmol) in *N,N-*dimethylformamide (10.0 mL) at ambient temperature was added potassium carbonate (1.381 g, 9.99 mmol) and *tert-*butyl bromoacetate (0.843 mL, 5.75 mmol). This mixture was stirred at 65 °C for 16 hours, allowed to cool to ambient temperature and was diluted with ethyl acetate (50 mL) and H₂O (50 mL). The layers were separated, and the aqueous layer was extracted with ethyl acetate (3 × 15 mL). The combined organic layers were dried over anhydrous Na₂SO₄, filtered, and concentrated under reduced pressure to give the ester (1.18 g, 4.96 mmol, 99 % yield). This ester was dissolved in methanol/water (3:1, 20 mL) and treated with 12.5 N NaOH aqueous solution (4.00 mL, 50.0 mmol). The mixture was stirred at ambient temperature overnight. The mixture was concentrated, and the residue was acidified with 1 N aqueous HCl. The precipitate was collected by filtration and air-dried to give 1.18 g of the title compound. ¹H NMR (400 MHz, DMSO-*d*₆) *δ* ppm 7.68 (t, J = 8.8 Hz, 1H), 7.14 (dd, J = 13.1, 2.4 Hz, 1H), 6.95 (dd, J = 8.9, 2.4 Hz, 1H), 4.84 (s, 2H); MS (ESI⁺) *m*/*z* 236.9 (M+H)⁺.

### Example 574B: (S)-(9H-fluoren-9-yl)methyl (4-(2-(3-fluoro-4-(trifluoromethyl)phenoxy)acetamido)-3-hydroxybicyclo[2.2.2]octan-1-yl)carbamate

The title compound was synthesized using the same procedure described in Example 548B substituting Example 574A for 2-(4-chloro-3-fluorophenoxy)acetic acid. ¹H NMR (501 MHz, DMSO-*d*₆) *δ* ppm 7.99 - 7.84 (m, 2H), 7.74 - 7.64 (m, 3H), 7.46 - 7.28 (m, 4H), 7.15 - 6.90 (m, 2H), 5.03 (s, 1H), 4.57 (s, 2H), 4.37 (t, J = 5.1 Hz, 1H), 4.13 (d, J = 69.9 Hz, 3H), 3.45 (qd, J = 7.0, 5.1 Hz, 1H), 2.89 (s, 1H), 2.73 (s, 1H), 2.25 - 1.47 (m, 8H); MS (ESI⁺) *m*/*z* 599.2 (M+H)⁺.

### Example 574C: (S)-N-(4-amino-2-hydroxybicyclo[2.2.2]octan-1-yl)-2-(3-fluoro-4-(trifluoromethyl)phenoxy)acetamide, Trifluoroacetic Acid

The title compound was synthesized using the same procedure described in Example 548C substituting Example 574B for Example 548B. ¹H NMR (501 MHz, DMSO-*d*₆) *δ* ppm 7.94 (s, 2H), 7.82 - 7.62 (m, 1H), 7.54 - 7.35 (m, 1H), 7.09 (dd, *J =* 13.1, 2.4 Hz, 1H), 6.94 (dd, *J =* 8.8, 2.4 Hz, 1H), 4.58 (s, 3H), 4.40 (s, 1H), 3.97 (s, 2H), 2.38 - 1.51 (m, 10H); MS (ESI⁺) *m*/*z* 377.1 (M+H)⁺.

### Example 574D: (S)-N-(4-(((5,6-dimethyl-1H-benzo[d]imidazol-2-yl)methyl)amino)-2-hydr^{o}ₓybicyclo[2.2.2]octan-1-yl)-2-(3-fluoro-4-(trifluoromethyl)phenoxy)acetamide, Trifluoroacetic Acid

The title compound was synthesized using the same procedure described in Example 548D substituting Example 574C for Example 548C (51 mg, 0.134 mmol) and 5,6-dimethyl-1*H-*benzo[*d*]imidazole-2-carbaldehyde for 5-fluoro-1*H*-benzo[*d*]imidazole-2-carbaldehyde. ¹H NMR (501 MHz, DMSO-*d*₆) *δ* ppm 7.69 (t, J = 8.8 Hz, 1H), 7.50 (s, 1H), 7.40 (s, 2H), 7.11 (dd, J = 13.1, 2.4 Hz, 1H), 6.95 (dd, J = 8.8, 2.4 Hz, 1H), 4.59 (s, 2H), 4.32 (s, 2H), 4.22 (dd, J = 9.4, 2.8 Hz, 1H), 2.32 (s, 6H), 2.04 (q, J = 7.9 Hz, 3H), 1.97 - 1.84 (m, 4H), 1.84 - 1.67 (m, 3H); MS (ESI⁺) *m*/*z* 535.3 (M+H)⁺.

### Example 576: 2-(4-chloro-3-fluorophenoxy)-N-[(2S)-2-hydroxy-4-{2-[(5-methoxypyridin-3-yl)oxy]acetamido}bicyclo[2.2.2]octan-1-yl]acetamide (Compound 675)

The title compound was synthesized using the procedure described in Example 538 substituting 5-methoxypyridin-3-ol for 2-methoxypyrimidin-5-ol. ¹H NMR (400 MHz, DMSO-*d*₆) *δ* ppm 7.87 (dd, J = 15.6, 2.4 Hz, 1H), 7.46 (dd, J = 19.0, 10.1 Hz, 2H), 7.26 (s, 1H), 7.02 (dd, J = 11.4, 2.9 Hz, 1H), 6.91 (q, J = 2.4 Hz, 1H), 6.79 (ddd, J = 9.0, 2.9, 1.3 Hz, 1H), 4.43 (d, J = 3.2 Hz, 4H), 4.00 (dd, J = 9.7, 3.1 Hz, 1H), 3.77 (s, 3H), 2.29 2.19 (m, 1H), 2.08 1.98 (m, 1H), 1.93 1.80 (m, 2H), 1.78 (s, 4H), 1.83 1.69 (m, 2H); MS (ESI⁺) *m*/*z* 508.3 (M+H)⁺.

### Example 577: N,N'-[2-(2-methoxyethyl)-3-oxo-2-azabicyclo[2.2.2]octane-1,4-diyl]bis[2-(4-chloro-3-fluorophenoxy)acetamide] (Compound 676)

To a solution Example 504 (60.0 mg, 0.114 mmol) in dimethylformamide (DMF) (1.8 mL) was added 60% sodium hydride in mineral oil (6.81 mg, 0.170 mmol) and 1-bromo-2-methoxyethane (0.016 mL, 0.170 mmol). The reaction mixture was stirred overnight. The reaction was treated with methanol and concentrated. The concentrate was purified by HPLC (see protocol in Example 529D) to provide the title compound (48.1 mg, 72%). ¹H NMR (500 MHz, DMSO-*d*₆) *δ* ppm 8.52 (s, 1H), 7.91 (s, 1H), 7.50 (q, J = 9.0 Hz, 2H), 7.11 (dd, J = 11.3, 2.9 Hz, 1H), 7.05 (dd, J = 11.3, 2.8 Hz, 1H), 6.85 (dddd, J = 21.5, 9.0, 2.9, 1.2 Hz, 2H), 4.67 (s, 2H), 4.59 (s, 2H), 3.45 (t, J = 6.2 Hz, 2H), 3.37-3.34 (m, 2H), 3.22 (s, 3H), 2.71 (td, J = 11.9, 10.1, 5.4 Hz, 2H), 2.49 - 2.32 (m, 2H), 1.88 (td, J = 11.8, 4.3 Hz, 2H), 1.48 (q, J = 8.4, 5.8 Hz, 2H); MS (ESI⁺) *m*/*z* 586.3 (M+H)⁺.

### Example 578: N,N'-(3-oxo-2-propyl-2-azabicyclo[2.2.2]octane-1,4-diyl)bis[2-(4-chloro-3-fluorophenoxy)acetamide] (Compound 677)

To a solution of Example 504 (60.0 mg, 0.114 mmol) in dimethylformamide (DMF) (1.8 mL) was added 60% sodium hydride in mineral oil (6.81 mg, 0.170 mmol) and 1-bromopropane (0.015 mL, 0.170 mmol). The reaction mixture was stirred overnight. The reaction was treated with methanol and concentrated. The concentrate was purified by HPLC (see protocol in Example529D) to provide the title compound (45.8 mg, 71%). ¹H NMR (500 MHz, DMSO-*d*₆) *δ* ppm 8.62 (s, 1H), 7.94 (s, 1H), 7.51 (td, J = 8.9, 4.0 Hz, 2H), 7.12 (dd, J = 11.3, 2.9 Hz, 1H), 7.05 (dd, J = 11.3, 2.9 Hz, 1H), 6.95 - 6.79 (m, 2H), 4.69 (s, 2H), 4.59 (s, 2H), 3.23 (dd, J = 8.8, 6.3 Hz, 2H), 2.73 (td, J = 11.9, 10.2, 5.4 Hz, 2H), 2.45 (td, J = 11.5, 4.2 Hz, 2H), 1.88 (td, J = 11.8, 4.3 Hz, 2H), 1.47 (tt, J = 14.7, 7.1 Hz, 4H), 0.76 (t, J = 7.4 Hz, 3H); MS (ESI⁺) *m*/*z* 570.1 (M+H)⁺.

### Example 580: N,N'-[2-(2-hydroxyethyl)-3-oxo-2-azabicyclo[2.2.2]octane-1,4-diyl]bis[2-(4-chloro-3-fluorophenoxy)acetamide] (Compound 679)

To a solution of Example 504 (100 mg, 0.189 mmol) in *N*,*N*-dimethylformamide (2.0 mL) was added NaH (11.36 mg, 60% in mineral oil, 0.284 mmol), and the reaction was stirred at ambient temperature for 15 minutes. Then (2-bromoethoxy)(*tert*-butyl)dimethylsilane (0.061 mL, 0.284 mmol) was added, and the reaction mixture was stirred for 16 hours. Methanol (0.5 mL) was added to quench the reaction, volatiles were removed, and the residue was purified by HPLC (Phenomenex^{®} Luna^{®} C18(2) 10 µm 100Å AXIA^{™} column (250 mm × 50 mm). A 40-100% gradient of acetonitrile (A) and 0.1% trifluoroacetic acid in water (B) was used over 25 minutes, at a flow rate of 50 mL/minute) to give 68 mg of silyl ether. A solution of the silyl ether from the previous step (84 mg, 0.123 mmol) and hydrogen chloride (5.0 mL, 6.25 mmol) in methanol (5.0 mL) was stirred at ambient temperature overnight. The mixture was concentrated, and the residue was purified by HPLC (Phenomenex^{®} Luna^{®} C18(2) 10 µm 100Å AXIA^{™} column (250 mm × 50 mm). A 30-100% gradient of acetonitrile (A) and 0.1% trifluoroacetic acid in water (B) was used over 25 minutes, at a flow rate of 50 mL/minute) to give 32 mg of the title compound. ¹H NMR (501 MHz, DMSO-*d*₆) *δ* ppm 8.54 (s, 1H), 7.92 (s, 1H), 7.54 - 7.41 (m, 2H), 7.14 - 7.00 (m, 2H), 6.85 (ddd, J = 20.0, 8.9, 2.9 Hz, 2H), 4.67 (s, 2H), 4.59 (s, 2H), 3.44 - 3.36 (m, 4H), 2.70 (td, J = 11.9, 10.1, 5.4 Hz, 2H), 2.43 (td, J = 11.4, 4.2 Hz, 2H), 1.89 (td, J = 11.8, 4.3 Hz, 2H), 1.49 (tt, J = 11.7, 9.9, 3.8 Hz, 2H); MS (+ESI) *m*/*z* 572.4 (M+H)⁺.

### Example 581: 2-(3,4-dichlorophenoxy)-N-{1-[2-(3,4-difluorophenoxy)acetamido]-3-oxo-2-azabicyclo[2.2.2]octan-4-yl}acetamide (Compound 680)

The title compound was synthesized using the procedure described in Example 501D substituting 2-(3,4-difluorophenoxy)acetic acid for 2-(3,4-dichlorophenoxy)acetic acid. ¹H NMR (501 MHz, DMSO-*d*₆) *δ* ppm 8.77 (s, 1H), 8.33 (s, 1H), 7.85 (s, 1H), 7.56 (d, J = 8.9 Hz, 1H), 7.35 (dt, J = 10.6, 9.2 Hz, 1H), 7.28 (d, J = 2.9 Hz, 1H), 7.10 (ddd, J = 12.6, 6.7, 3.1 Hz, 1H), 7.00 (dd, J = 8.9, 3.0 Hz, 1H), 6.80 (dtd, J = 9.1, 3.3, 1.6 Hz, 1H), 4.60 (s, 2H), 4.49 (s, 2H), 2.75 - 2.65 (m, 2H), 2.31 - 2.22 (m, 2H), 1.91 (td, J = 11.5, 4.3 Hz, 2H), 1.53 (dq, J = 12.3, 8.0, 5.8 Hz, 2H); MS (+ESI) *m*/*z* 528.3 (M+H)⁺.

### Example 585: {[2-({3-[2-(4-chloro-3-fluorophenoxy)acetamido]bicyclo[1.1.1]pentan-1-yl}amino)-2-oxoethyl](4-chlorophenyl)amino}acetic acid (Compound 684)

Trifluoroacetic acid (0.5 mL) was added directly to the product of Example 586 (16 mg, 0.028 mmol) and stirred at 40 °C for 1hour. The reaction mixture was concentrated under reduced pressure. The resulting residue was taken up in methanol (2 mL), filtered through a glass microfiber frit and purified by HPLC [Waters XBridge^{™} C18 5 µm OBD column, 50 × 100 mm, flow rate 90 mL/minute, 40-100% gradient of methanol in buffer (0.1% trifluoroacetic acid)] to give the title compound (10 mg, 0.016 mmol, 57 % yield). ¹H NMR (400 MHz, DMSO-*d*₆/D₂O) *δ* ppm 7.47 (t, J = 8.9 Hz, 1H), 7.25 - 7.19 (m, 2H), 7.04 (dd, J = 11.3, 2.9 Hz, 1H), 6.84 (ddd, J = 9.0, 3.0, 1.2 Hz, 1H), 6.48 - 6.42 (m, 2H), 4.44 (s, 2H), 4.18 (s, 2H), 3.95 (s, 2H), 2.22 (s, 6H); MS (APCI⁺) *m*/*z* 510 (M+H)⁺.

### Example 586: tert-butyl {[2-({3-[2-(4-chloro-3-fluorophenoxy)acetamido]bicyclo[1.1.1]pentan-1-yl}amino)-2-oxoethyl](4-chlorophenyl)amino}acetate (Compound 685)

The title compound was prepared as described in Example 582C, substituting the product of Example 104 for the product of Example 582B, and *tert*-butyl bromoacetate (Matrix) for 4-(bromomethyl)-1-chloro-2-fluorobenzene. ¹H NMR (501 MHz, DMSO-*d*₆) *δ* ppm 8.68 (s, 1H), 8.61 (s, 1H), 7.47 (t, J = 8.9 Hz, 1H), 7.22 - 7.17 (m, 2H), 7.04 (dd, J = 11.3, 2.9 Hz, 1H), 6.82 (ddd, J = 9.0, 2.9, 1.2 Hz, 1H), 6.48 - 6.41 (m, 2H), 4.44 (s, 2H), 4.11 (s, 2H), 3.86 (s, 2H), 2.19 (s, 6H), 1.39 (s, 9H); MS (APCI⁺) *m*/*z* 566 (M+H)⁺.

### Example 587: ({3-[2-(4-chloro-3-fluorophenoxy)acetamido]bicyclo[1.1.1]pentan-1-yl}[2-(4-chloro-3-fluorophenoxy)ethyl]amino)acetic acid (Compound 686)

The title compound was prepared as described in Example 585, substituting the product of Example 589 for the product of Example 586. ¹H NMR (400 MHz, DMSO-*d*₆, 120 °C) *δ* ppm 8.05 (s, 1H), 7.48 - 7.37 (m, 2H), 7.02 - 6.97 (m, 2H), 6.87 - 6.82 (m, 2H), 4.49 (s, 2H), 4.27 (t, J = 5.3 Hz, 2H), 3.80 (s, 2H), 3.36 - 3.31 (m, 2H), 3.02 - 2.95 (m, 6H); MS (APCI⁺) *m*/*z* 515 (M+H)⁺.

### Example 588: ({3-[2-(4-chloro-3-fluorophenoxy)acetamido]bicyclo[1.1.1]pentan-1-yl}[3-(4-chloro-3-fluorophenoxy)propyl]amino)acetic acid (Compound 687)

### Example 588A: tert-butyl 2-((3-(2-(4-chloro-3-fluorophenoxy)acetamido)bicyclo[1.1.1]pentan-1-yl)amino)acetate

Preparative HPLC purification of the crude reaction mixture in Example 579A also afforded this title compound as an earlier eluting fraction (0.76 g, 1.91 mmol, 64% yield). ¹H NMR (400 MHz, DMSO-*d*₆/D₂O) *δ* ppm 7.46 (t, J = 8.9 Hz, 1H), 7.02 (dd, J = 11.3, 2.8 Hz, 1H), 6.83 (ddd, J = 9.0, 2.9, 1.2 Hz, 1H), 4.43 (s, 2H), 3.13 (s, 2H), 1.92 (s, 6H), 1.40 (s, 9H); MS (APCI⁺) *m*/*z* 399 (M+H)⁺.

### Example 588B: 4-(3-bromopropoxy)-1-chloro-2-fluorobenzene

Potassium carbonate (1.36 g, 9.83 mmol) and 4-chloro-3-fluorophenol (1.2 g, 8.19 mmol, Pharmablock), were combined with *N,N*-dimethylformamide (10 mL) and stirred at ambient temperature. 1,3-Dibromopropane (1.25 mL, 12.3 mmol, Alfa) was added in one portion. The resulting mixture was stirred at 55 °C for 1 hour and then at 65 °C for 1 hour. The reaction mixture was cooled to ambient temperature and filtered through a pad of diatomaceous earth. The resulting solution was directly purified by preparative HPLC [YMC TriArt^{™} C18 Hybrid 5 µm column, 50 × 100 mm, flow rate 140 mL/minute, 5-100% gradient of acetonitrile in buffer (carbonic acid buffer prepared by sparging carbon dioxide gas bubbles through deionized water for 15 minutes immediately before use)] to give the title compound (0.8 g, 3.0 mmol, 37 % yield). ¹H NMR (400 MHz, DMSO-*d*₆) *δ* ppm 7.47 (t, J = 8.9 Hz, 1H), 7.09 (dd, J = 11.5, 2.9 Hz, 1H), 6.85 (ddd, J = 9.0, 2.9, 1.2 Hz, 1H), 4.10 (t, J = 6.0 Hz, 2H), 3.65 (t, J = 6.5 Hz, 2H), 2.24 (p, J = 6.3 Hz, 2H); MS (DCI⁺) *m*/*z* 350 (M+NH₄)⁺.

### Example 588C: tert-butyl ({3-[2-(4-chloro-3-fluorophenoxy)acetamido]bicyclo[1.1.1]pentan-1-yl}[3-(4-chloro-3-fluorophenoxy)propyl]amino)acetate

The title compound was prepared as described in Example 582C, substituting the product of Example 588A for the product of Example 582B, and the product of Example 588B for 4-(bromomethyl)-1-chloro-2-fluorobenzene. ¹H NMR (400 MHz, CDCl₃) *δ* ppm 7.33 (t, J = 8.6 Hz, 1H), 7.27 - 7.22 (m, 1H), 6.79 (s, 1H), 6.78 - 6.70 (m, 2H), 6.70 - 6.62 (m, 2H), 4.39 (s, 2H), 4.00 (t, J = 6.1 Hz, 2H), 3.29 (s, 2H), 2.88 (t, J = 6.9 Hz, 2H), 2.18 (s, 6H), 1.90 (p, J = 6.5 Hz, 2H), 1.47 (s, 9H); MS (ESI⁺) *m*/*z* 585 (M+H)⁺.

### Example 588D: ({3-[2-(4-chloro-3-fluorophenoxy)acetamido]bicyclo[1.1.1]pentan-1-yl}[3-(4-chloro-3-fluorophenoxy)propyl]amino)acetic acid (Compound 687)

The title compound was prepared as described in Example 585, substituting the product of Example 588C for the product of Example 586. ¹H NMR (400 MHz, methanol-*d₄*) *δ* ppm 7.37 (t, J = 8.7 Hz, 1H), 7.34 (t, J = 8.7 Hz, 1H), 6.92 (dd, J = 10.9, 2.8 Hz, 1H), 6.87 (dd, J = 11.1, 2.8 Hz, 1H), 6.81 (ddd, J = 8.9, 2.9, 1.3 Hz, 1H), 6.78 (ddd, J = 8.9, 2.8, 1.2 Hz, 1H), 4.50 (s, 2H), 4.09 (t, J = 5.8 Hz, 2H), 3.94 (s, 2H), 3.30 - 3.26 (m, 2H), 2.42 (s, 6H), 2.21 - 2.11 (m, 2H); MS (APCI⁺) *m*/*z* 529 (M+H)⁺.

### Example 589: tert-butyl ({3-[2-(4-chloro-3-fluorophenoxy)acetamido]bicyclo[1.1.1]pentan-1-yl}[2-(4-chloro-3-fluorophenoxy)ethyl]amino)acetate (Compound 688)

The title compound was prepared using the methodologies described above. ¹H NMR (400 MHz, CDCl₃) *δ* ppm 7.33 (t, J = 8.6 Hz, 1H), 7.26 (t, J = 8.7 Hz, 1H), 6.81 (s, 1H), 6.78 - 6.61 (m, 4H), 4.40 (s, 2H), 4.00 (t, J = 5.9 Hz, 2H), 3.42 (s, 2H), 3.16 (t, J = 5.9 Hz, 2H), 2.22 (s, 6H), 1.47 (s, 9H); MS (ESI⁺) *m*/*z* 571 (M+H)⁺.

### Example 590: 2-(4-chloro-3-fluorophenoxy)-N-(3-oxo-1-{2-[(2,2,3,3-tetrafluoro-2,3-dihydro-1,4-benzodioxin-6-yl)oxy]acetamido}-2-azabicyclo[2.2.2]octan-4-yl)acetamide (Compound 689)

The title compound was prepared using the methodologies described in Example 501D substituting Example 503A for Example 501C and 2,2,3,3-tetrafluoro-2,3-dihydrobenzo[*b*][1,4]dioxin-6-ol for 2-(3,4-dichlorophenoxy)acetic acid. ¹H NMR (501 MHz, DMSO-*d*₆) *δ* ppm 8.79 (s, 1H), 8.36 (s, 1H), 7.85 (s, 1H), 7.51 (t, J = 8.8 Hz, 1H), 7.40 (d, J = 9.1 Hz, 1H), 7.15 - 7.07 (m, 2H), 6.94 (dd, J = 9.2, 2.9 Hz, 1H), 6.87 (ddd, J = 8.9, 2.9, 1.2 Hz, 1H), 4.59 (s, 2H), 4.53 (s, 2H), 2.76 - 2.65 (m, 2H), 2.27 (td, J = 11.6, 4.6 Hz, 2H), 1.91 (td, J = 11.5, 4.2 Hz, 2H), 1.57 - 1.47 (m, 2H).; MS (+ESI) *m*/*z* 606.2 (M+H)⁺.

### Example 591: 2-(4-chloro-3-fluorophenoxy)-N-(1-{2-[(2-methoxypyrimidin-5-yl)oxy]acetamido}-3-oxo-2-azabicyclo[2.2.2]octan-4-yl)acetamide (Compound 690)

### Example 591A: 2-chloro-N-(4-(2-(4-chloro-3-fluorophenoxy)acetamido)-3-oxo-2-azabicyclo[2.2.2]octan-1-yl)acetamide

A mixture of Example 503A, 2-chloroacetic acid (58.8 mg, 0.623 mmol) and *N*-ethyl-*N-*isopropylpropan-2-amine (0.331 mL, 1.895 mmol) in *N,N*-dimethylformamide (1.5 mL) was treated with 2-(3*H*-[1,2,3]triazolo[4,5-*b*]pyridin-3-yl)-1,1,3,3-tetramethylisouronium hexafluorophosphate(V) (257 mg, 0.677 mmol), and the reaction was stirred at ambient temperature for 30 minutes. The mixture was concentrated, and the residue was purified by HPLC (Phenomenex^{®} Luna^{®} C18(2) 10 µm 100Å AXIA^{™} column (250 mm × 50 mm). A 30-100% gradient of acetonitrile (A) and 0.1% trifluoroacetic acid in water (B) was used over 25 minutes, at a flow rate of 50 mL/minute) to give 85 mg of the title compound. ¹H NMR (400 MHz, DMSO-*d*₆) *δ* ppm 8.65 (s, 1H), 8.53 (s, 1H), 7.80 (s, 1H), 7.47 (t, J = 8.9 Hz, 1H), 7.07 (dd, J = 11.3, 2.9 Hz, 1H), 6.83 (ddd, J = 8.9, 2.9, 1.2 Hz, 1H), 4.54 (s, 2H), 4.00 (s, 2H), 2.70 - 2.59 (m, 2H), 2.20 (td, J = 11.4, 4.1 Hz, 2H), 1.81 (td, J = 11.5, 4.2 Hz, 2H), 1.53 - 1.43 (m, 2H); MS (+ESI) *m*/*z* 418.2 (M+H)⁺.

### Example 591B: 2-(4-chloro-3-fluorophenoxy)-N-(1-{2-[(2-methoxypyrimidin-5-yl)oxy]acetamido}-3-oxo-2-azabicyclo[2.2.2]octan-4-yl)acetamide

The title compound was synthesized using the same procedure described in Example 538 substituting Example 591A for 476A. ¹H NMR (400 MHz, DMSO-*d*₆) *δ* ppm 8.79 (s, 1H), 8.36 (s, 1H), 7.85 (s, 1H), 7.51 (t, J = 8.8 Hz, 1H), 7.40 (d, J = 9.1 Hz, 1H), 7.15 - 7.07 (m, 2H), 6.94 (dd, J = 9.2, 2.9 Hz, 1H), 6.87 (ddd, J = 8.9, 2.9, 1.2 Hz, 1H), 4.59 (s, 2H), 4.53 (s, 2H), 2.76 - 2.65 (m, 2H), 2.27 (td, J = 11.6, 4.6 Hz, 2H), 1.91 (td, J = 11.5, 4.2 Hz, 2H), 1.57 - 1.47 (m, 2H); MS (+ESI) *m*/*z* 508.3 (M+H)⁺.

### Example 592: 2-(4-chloro-3-fluorophenoxy)-N-(3-oxo-1-{2-[(3H-[1,2,3]triazolo[4,5-b]pyridin-3-yl)oxy]acetamido}-2-azabicyclo[2.2.2]octan-4-yl)acetamide (Compound 691)

The title compound was isolated as a byproduct from the same reaction described in Example 591A. ¹H NMR (400 MHz, DMSO-*d*₆) *δ* ppm 8.91 (dd, J = 4.4, 1.4 Hz, 1H), 8.79 (s, 1H), 8.77 - 8.67 (m, 2H), 7.91 (s, 1H), 7.68 (dd, J = 8.4, 4.5 Hz, 1H), 7.57 (t, J = 8.8 Hz, 1H), 7.18 (dd, J = 11.3, 2.9 Hz, 1H), 6.94 (ddd, J = 9.0, 2.9, 1.2 Hz, 1H), 5.15 (s, 2H), 4.65 (s, 2H), 2.75 (td, J = 11.8, 3.7 Hz, 2H), 2.34 (td, J = 11.4, 3.9 Hz, 2H), 1.95 (td, J = 11.3, 4.2 Hz, 2H), 1.65 - 1.55 (m, 2H); MS (+ESI) *m*/*z* 518.3 (M+H)⁺.

### Example 593: 2-(4-chloro-3-fluorophenoxy)-N-(3-{[3-(4-chloro-3-fluorophenoxy)propyl](2-hydroxyethyl)amino}bicyclo[1.1.1]pentan-1-yl)acetamide (Compound 692)

### Example 593A: N-(3-((2-((tert-butyldimethylsilyl)oxy)ethyl)(3-(4-chloro-3-fluorophenoxy)propyl)amino)bicyclo[1.1.1]pentan-1-yl)-2-(4-chloro-3-fluorophenoxy)acetamide

Potassium carbonate (62.4 mg, 0.45 mmol) and the product of Example 90A (50 mg, 0.11 mmol) were combined with *N,N*-dimethylformamide (2.0 mL) and stirred at ambient temperature. The product of Example 588B (91 mg, 0.34 mmol) was added in one portion. The resulting mixture was stirred at 30 °C for 72 hours. The reaction mixture was filtered through a glass microfiber frit and the resulting solution was directly purified by preparative HPLC [YMC TriArt^{™} C18 Hybrid 5 µm column, 50 × 100 mm, flow rate 140 mL/minute, 50-100% gradient of acetonitrile in buffer (0.025 M aqueous ammonium bicarbonate, adjusted to pH 10 with ammonium hydroxide)] to give the title compound (27 mg, 0.034 mmol, 38 % yield). ¹H NMR (501 MHz, DMSO-*d*₆) *δ* ppm 8.64 (s, 1H), 7.49 (t, J = 8.9 Hz, 1H), 7.45 (t, J = 8.9 Hz, 1H), 7.06 (dd, J = 11.4, 2.9 Hz, 1H), 7.03 (dd, J = 11.6, 2.8 Hz, 1H), 6.84 (ddd, J = 9.0, 2.9, 1.2 Hz, 1H), 6.80 (ddd, J = 9.0, 2.9, 1.2 Hz, 1H), 4.46 (s, 2H), 4.01 (t, J = 6.1 Hz, 2H), 3.53 (t, J = 6.7 Hz, 2H), 2.63 (t, J = 6.8 Hz, 2H), 2.57 (t, J = 6.7 Hz, 2H), 1.98 (s, 6H), 1.78 (p, J = 6.5 Hz, 2H), 0.84 (s, 9H), 0.00 (s, 6H); MS (APCI⁺) *m*/*z* 629 (M+H)⁺.

### Example 593B: 2-(4-chloro-3-fluorophenoxy)-N-(3-{[3-(4-chloro-3-fluorophenoxy)propyl](2-hydroxyethyl)amino}bicyclo[1.1.1]pentan-1-yl)acetamide (Compound 692)

The title compound was prepared as described in Example 91, substituting the product of Example 593A for the product of Example 90, and the pH10 aqueous buffer (25 mM ammonium bicarbonate adjust to pH10) for the carbonic acid buffer for preparative HPLC. ¹H NMR (400 MHz, DMSO-*d₆*) *δ* ppm (contained 0.25 equivalent (10 weight% ) Bu₄NOH) 8.63 (s, 1H), 7.47 (t, J = 8.9 Hz, 1H), 7.43 (t, J = 8.9 Hz, 1H), 7.05 (dd, J = 6.6, 2.9 Hz, 1H), 7.02 (dd, J = 6.7, 2.8 Hz, 1H), 6.85 - 6.78 (m, 2H), 4.45 (s, 2H), 4.00 (t, J = 6.1 Hz, 2H), 3.48 - 3.38 (m, 2H), 2.60 (t, J = 6.9 Hz, 2H), 2.52 (t, J = 6.8 Hz, 2H), 1.96 (s, 6H), 1.77 (p, J = 6.5 Hz, 2H) 0.25 equivalent Bu₄NOH, 3.18 - 3.11 (m, 2H) 0.25 equivalent Bu₄NOH, 1.61 - 1.49 (m, 2H) 0.25 equivalent Bu₄NOH, 1.30 (h, J = 7.3 Hz, 2H) 0.25 equivalent Bu₄NOH, 0.92 (t, J = 7.3 Hz, 3H) 0.25 equivalent Bu₄NOH; MS (APCI⁺) *m*/*z* 515 (M+H)⁺.

### Example 594: (2R)-2-({(3S)-4-[2-(4-chloro-3-fluorophenoxy)acetamido]-3-hydroxybicyclo[2.2.2]octan-1-yl}amino)-4-(4-chlorophenoxy)butanoic acid (Compound 693)

The title compound was isolated by chiral preparative SFC (Supercritical Fluid Chromatography) of Example 607D as the second peak eluted off the column. Preparative SFC was performed on a THAR/Waters SFC 80 system running under SuperChrom^{™} software control. The preparative SFC system was equipped with an 8-way preparative column switcher, CO₂ pump, modifier pump, automated back pressure regulator (ABPR), UV detector, and 6-position fraction collector. The mobile phase was comprised of supercritical CO₂ supplied by a Dewar of bone-dry non-certified CO₂ pressurized to 350 psi with a modifier of methanol at a flow rate of 80 g/minute. The column temperature was at 34 °C, and the backpressure regulator was set to maintain 100 bar. The sample was dissolved methanol at a concentration of 5 mg/mL. The sample was loaded into the modifier stream in 2 mL injections. The mobile phase was held isocratically at 50 % methanol (with 0.1 % diethylamine):CO₂. Fraction collection was time triggered. The instrument was fitted with a Whelk-O^{®} column with dimensions 30 mm i.d. × 250 mm length with 5 µm particles. The absolute stereochemistry assignment is arbitrary. ¹H NMR (500 MHz, DMSO-*d*₆) *δ* ppm 7.47 (t, J = 8.9 Hz, 1H), 7.40 - 7.28 (m, 3H), 7.04 (dd, J = 11.4, 2.9 Hz, 1H), 7.02 - 6.92 (m, 2H), 6.82 (dd, J = 9.0, 2.8 Hz, 1H), 5.19 (s, brd, 1H), 4.47 (d, J = 13.9 Hz, 2H), 4.06 (dtd, J = 22.5, 11.8, 10.8, 5.0 Hz, 3H), 2.14 - 1.41 (m, 12H); MS (ESI⁺) *m*/*z* 555.3 (M+H)⁺.

### Example 595: (2S)-2-({(3S)-4-[2-(4-chloro-3-fluorophenoxy)acetamido]-3-hydroxybicyclo[2.2.2]octan-1-yl}amino)-4-(4-chlorophenoxy)butanoic acid (Compound 694)

The title compound was isolated by chiral preparative SFC (Supercritical Fluid Chromatography) of Example 607D as the first peak eluted off the column. The absolute stereochemistry assignment is arbitrary. ¹H NMR (400 MHz, DMSO-*d*₆) *δ* ppm 7.47 (t, J = 8.8 Hz, 1H), 7.38 - 7.24 (m, 3H), 7.04 (dd, J = 11.4, 2.9 Hz, 1H), 7.02 - 6.91 (m, 2H), 6.82 (ddd, J = 9.0, 2.9, 1.2 Hz, 1H), 5.17 (s, brd, 1H), 4.45 (s, 2H), 4.06 (qt, J = 10.8, 5.1 Hz, 3H), 2.19 - 1.31 (m, 12H); MS (ESI⁺) *m*/*z* 555.2 (M+H)⁺.

### Example 596: 2-(4-chloro-3-fluorophenoxy)-N-[(2S)-2-hydroxy-4-{2-[(2-methyl-3-oxo-2,3-dihydro-1H-isoindol-5-yl)oxy]acetamido}bicyclo[2.2.2]octan-1-yl]acetamide (Compound 695)

### Example 596A: tert-butyl 2-((2-methyl-3-oxoisoindolin-5-yl)oxy)acetate

To a solution of 6-hydroxy-2-methylisoindolin-1-one (0.50 g, 3.1 mmol) in *N,N-*dimethylformamide (3.6 mL) at ambient temperature was added potassium carbonate (0.847 g, 6.13 mmol) and *tert*-butyl bromoacetate (0.52 mL, 3.5 mmol). This mixture was warmed to 70 °C and was allowed to stir for 2.5 hours. Then the mixture was allowed to cool to ambient temperature and was diluted with ethyl acetate (50 mL) and water (50 mL). The layers were separated, and the aqueous layer was extracted with ethyl acetate (3 × 15 mL). The combined organic layers were dried over anhydrous Na₂SO₄, filtered, and concentrated under reduced pressure to give the title compound (0.85 g, 3.1 mmol, quantitative yield), which was carried forward without purification. ¹H NMR (501 MHz, DMSO-*d*₆) *δ* ppm 7.48 (d, J = 8.3 Hz, 1H), 7.14 (dd, J = 8.3, 2.5 Hz, 1H), 7.10 (d, J = 2.4 Hz, 1H), 4.74 (s, 2H), 4.37 (s, 2H), 3.06 (s, 3H), 1.42 (s, 9H); MS (ESI⁺) *m*/*z* 278 (M+H)⁺.

### Example 596B: 2-((2-methyl-3-oxoisoindolin-5-yl)oxy)acetic acid

To a solution of Example 596A (0.85 g, 3.1 mmol) in dichloromethane (4.60 mL) was added trifluoroacetic acid (0.24 mL, 3.1 mmol), and the resulting mixture was stirred at ambient temperature for 1 hour and then was concentrated to afford the title compound, which was carried forward without purification. MS (ESI⁺) *m*/*z* 222 (M+H)⁺.

### Example 596C: (S)-tert-butyl (2-hydroxy-4-(2-((2-methyl-3-oxoisoindolin-5-yl)oxy)acetamido)bicyclo[2.2.2]octan-1-yl)carbamate

The methodologies described in Example 569 with the following modifications gave the title compound: (1) substituting Example 445B for Example 9B, (2) substituting Example 596B for 1-(4-chlorophenyl)cyclopropanecarboxylic acid, and (3) purifying by preparative HPLC (Waters XBridge^{™} C18 5 µm OBD column, 30 × 100 mm, flow rate 40 mL/minute, 5-100% gradient of acetonitrile in 0.1% trifluoroacetic acid/water). MS (ESI⁺) *m*/*z* 460 (M+H)⁺.

### Example 596D: (S)-N-(4-amino-3-hydroxybicyclo[2.2.2]octan-1-yl)-2-((2-methyl-3-oxoisoindolin-5-yl)oxy)acetamide

To a solution of Example 596C (0.15 g, 0.33 mmol) in dichloromethane (0.450 mL) was added trifluoroacetic acid (0.10 mL, 1.3 mmol), and the resulting mixture was stirred at ambient temperature overnight and concentrated to afford the title compound, which was carried forward without purification. MS (ESI⁺) *m*/*z* 360 (M+H)⁺.

### Example 596E: 2-(4-chloro-3-fluorophenoxy)-N-[(2S)-2-hydroxy-4-{2-[(2-methyl-3-oxo-2,3-dihydro-1H-isoindol-5-yl)oxy]acetamido}bicyclo[2.2.2]octan-1-yl]acetamide

The methodologies described in Example 569 with the following modifications gave the title compound: (1) substituting 2-(4-chloro-3-fluorophenoxy)acetic acid for 1-(4-chlorophenyl)cyclopropanecarboxylic acid, (2) substituting Example 596D for Example 9B, (3) additionally stirring the reaction mixture with LiOH (1 N aqueous, 5 mL) for 2 hours prior to purification to cleave any esterification product that was formed, and (4) purifying by preparative HPLC (Waters XBridge^{™} C18 5 µm OBD column, 30 × 100 mm, flow rate 40 mL/minute, 5-100% gradient of acetonitrile in 0.1% trifluoroacetic acid/water) . ¹H NMR (400 MHz, CDCl₃) *δ* ppm 7.41 - 7.29 (m, 3H), 7.10 (dd, J = 8.3, 2.4 Hz, 1H), 6.75 (dd, J = 10.1, 2.9 Hz, 1H), 6.70 - 6.63 (m, 1H), 6.38 (s, 1H), 6.28 (s, 1H), 4.46 - 4.38 (m, 4H), 4.35 (s, 2H), 4.18 (d, J = 8.8 Hz, 1H), 3.22 (s, 3H), 2.57 (s, 1H), 2.47 (t, J = 11.9 Hz, 1H), 2.15 - 2.06 (m, 4H), 1.84 (dd, J = 24.5, 13.1 Hz, 2H), 1.72 - 1.62 (m, 1H), 1.44 - 1.27 (m, 1H), 1.26 (s, 1H); MS (ESI⁺) *m*/*z* 546 (M+H)⁺.

### Example 597: 2-(4-chloro-3-fluorophenoxy)-N-[(2S)-4-{[3-(4-chlorophenoxy)propyl](methyl)amino}-2-hydroxybicyclo[2.2.2]octan-1-yl]acetamide (Compound 696)

### Example 597A: (S)-tert-butyl (4-((3-(4-chlorophenoxy)propyl)amino)-2-hydroxybicyclo[2.2.2]octan-1-yl)carbamate

Cesium carbonate (1.0 g, 3.07 mmol) and the product of Example 445B (300 mg, 1.03 mmol) were combined with N,N-dimethylformamide (5.0 mL) and stirred at ambient temperature. 1-(3-Bromopropoxy)-4-chlorobenzene (281 mg, 1.13 mmol, Enamine) was added in one portion. The resulting mixture was stirred at 35 °C for 18 hours. The reaction mixture was filtered through a pad of diatomaceous earth, and the resulting solution was directly purified by preparative HPLC [YMC TriArt^{™} C18 Hybrid 5 µm column, 50 × 100 mm, flow rate 140 mL/minute, 5-100% gradient of acetonitrile in buffer (0.025 M aqueous ammonium bicarbonate, adjusted to pH 10 with ammonium hydroxide)] to give the title compound (190 mg, 0.45 mmol, 44 % yield). MS (ESI⁺) *m*/*z* 425 (M+H)⁺.

### Example 597B: (S)-tert-butyl (4-((3-(4-chlorophenoxy)propyl)amino)-2-hydroxybicyclo[2.2.2]octan-1-yl)carbamate

The product of Example 597A (140 mg, 0.33 mmol), trifluoroacetic acid (0.18 mL), and formaldehyde (37 weight% in H₂O, 0.073 mL) were combined with methanol (2 mL) and stirred at ambient temperature. Sodium cyanoborohydride (145 mg, 2.31 mmol) was added in one portion. The resulting reaction mixture was left stirring at ambient temperature for 1 hour and additional formaldehyde (37 weight% in H₂O, 0.15 mL), sodium cyanoborohydride (145 mg, 2.31 mmol) and trifluoroacetic acid (0.18 mL) were added. The resulting mixture was stirred at ambient temperature for one more hour, filtered through a glass microfiber frit and purified by preparative HPLC [Waters XBridge^{™} C18 5 µm OBD^{™} column, 30 x 100 mm, flow rate 40 mL/minute, 5-100% gradient of acetonitrile in buffer (0.025 M aqueous ammonium bicarbonate, adjusted to pH 10 with ammonium hydroxide)] to give the title compound (110 mg, 0.25 mmol, 76% yield). (APCI⁺) *m*/*z* 439 (M+H)⁺.

### Example 597C: 2-(4-chloro-3-fluorophenoxy)-N-[(2S)-4-{[3-(4-chlorophenoxy)propyl](methyl)amino}-2-hydroxybicyclo[2.2.2]octan-1-yl]acetamide

The title compound was prepared as described in Example 83C, substituting 2-(4-chloro-3-fluorophenoxy)acetic acid (commercially available from Combi-Blocks) for the product of Example 83B, and the product of Example 597B for the product of Example 9A. ¹H NMR (501 MHz, DMSO-*d*₆) *δ* ppm 7.48 (t, J = 8.9 Hz, 1H), 7.34 - 7.27 (m, 2H), 7.24 (s, 1H), 7.05 (dd, J = 11.4, 2.9 Hz, 1H), 6.97 - 6.90 (m, 2H), 6.82 (ddd, J = 8.9, 2.9, 1.2 Hz, 1H), 5.01 (d, J = 3.9 Hz, 1H), 4.45 (s, 2H), 3.99 - 3.92 (m, 3H), 2.48 - 2.37 (m, 2H), 2.08 (s, 3H), 2.00 (ddd, J = 12.7, 10.8, 4.7 Hz, 1H), 1.95 - 1.81 (m, 2H), 1.81 - 1.69 (m, 4H), 1.63 - 1.55 (m, 1H), 1.53 - 1.34 (m, 4H); MS (APCI⁺) *m*/*z* 525 (M+H)⁺.

### Example 598: N,N'-(bicyclo[1.1.1]pentane-1,3-diyl)bis[3-(4-chloro-3-fluorophenoxy)propanamide] (Compound 697)

The title compound was prepared using the methodologies described above. ¹H NMR (400 MHz, DMSO-*d₆*) *δ* ppm 8.52 (s, 2H), 7.41 (t, J = 8.9 Hz, 2H), 7.01 (dd, J = 11.5, 2.9 Hz, 2H), 6.77 (ddd, J = 9.0, 2.8, 1.1 Hz, 2H), 4.13 (t, J = 6.1 Hz, 4H), 2.48 - 2.41 (m, 4H), 2.15 (s, 6H); MS (APCI⁺) *m*/*z* 499 (M+H)⁺.

### Example 599: 3-[{1,4-bis[2-(4-chloro-3-fluorophenoxy)acetamido]bicyclo[2.2.2]octan-2-yl}(methyl)amino]propanoic acid (Compound 698)

### Example 599A: N,N'-(2-oxobicyclo[2.2.2]octane-1,4-diyl)bis(2-(4-chloro-3-fluorophenoxy)acetamide)

A suspension of Example 198 (1 g, 1.9 mmol) and Dess-Martin periodinane (1,1,1-tris(acetyloxy)-1,1-dihydro-1,2-benzodioxol-3-(1*H*)-one) (1.60 g, 3.78 mmol) in dichloromethane (7.6 mL) was stirred for 2 hours. To the reaction mixture, additional Dess-Martin periodinane (1,1,1-tris(acetyloxy)-1,1-dihydro-1,2-benzodioxol-3-(1*H*)-one) (1.60 g, 3.78 mmol) was added, and the mixture stirred for 2 hours. The reaction mixture was then quenched with Na₂SO₃ (2 mL, saturated aqueous), stirred for 5 minutes, extracted with dichloromethane (2 × 10 mL), and concentrated. The residue was chromatographed on a normal phase column (25% ethyl acetate/dichloromethane) to give the title compound (0.85 g, 1.6 mmol, 85% yield). ). MS (ESI⁺) *m*/*z* 527 (M+H)⁺.

### Example 599B: N,N'-(2-(methylamino)bicyclo[2.2.2]octane-1,4-diyl)bis(2-(4-chloro-3-fluorophenoxy)acetamide)

A mixture of Example 599A (0.080 g, 0.15 mmol), methylamine hydrochloride (0.031 g, 0.46 mmol), zinc chloride (0.23 mL, 0.46 mmol, 1.9 M in 2-methyltetrahydrofuran) and sodium cyanoborohydride (0.048 g, 0.76 mmol) in methanol (1 mL) was stirred at 50 °C for 3 days. The reaction mixture was then concentrated, and the residue was purified by preparative HPLC (Waters XBridge^{™} C18 5 µm OBD column, 30 × 100 mm, flow rate 40 mL/minute, 5-100% gradient of acetonitrile in 0.1% trifluoroacetic acid/water) to give the title compound (0.068 g, 0.13 mmol, 87% yield). ¹H NMR (400 MHz, DMSO-*d*₆) *δ* ppm 10.51 (s, 1H), 7.89 (s, 1H), 7.76 (d, J = 6.7 Hz, 1H), 7.59 (t, J = 8.8 Hz, 1H), 7.49 (t, J = 8.8 Hz, 2H), 7.26 (dd, J = 11.0, 3.0 Hz, 1H), 7.04 (dd, J = 11.5, 2.9 Hz, 2H), 6.87 - 6.79 (m, 2H), 5.29 (d, J = 16.5 Hz, 1H), 5.19 (d, J = 16.3 Hz, 1H), 4.49 (t, J = 9.2 Hz, 4H), 4.05 (d, J = 8.7 Hz, 2H), 3.04 (s, 3H), 2.57 (s, 1H), 2.06 (d, J = 8.2 Hz, 1H), 1.91 (s, 1H), 1.81 (d, J = 11.8 Hz, 2H), 1.72 (s, 1H); MS (ESI⁺) *m*/*z* 542 (M+H)⁺.

### Example 599C: tert-butyl 3-((1,4-bis(2-(4-chloro-3-fluorophenoxy)acetamido)bicyclo[2.2.2]octan-2-yl)(methyl)amino)propanoate

To a solution of Example 599B (0.057 g, 0.11 mmol) in N,N-dimethylformamide (0.75 mL) and acetone (0.75 mL) was added potassium carbonate (0.044 g, 0.32 mmol), sodium iodide (0.008 g, 0.053 mmol), and *tert*-butyl 3-bromopropanoate (0.050 mL, 0.32 mmol). This reaction mixture was allowed to stir at 80 °C overnight. Additional potassium carbonate (0.044 g, 0.32 mmol), sodium iodide (0.008 g, 0.053 mmol), and *tert*-butyl 3-bromopropanoate (0.050 mL, 0.32 mmol) were added, and the mixture continued to stir for 2 days at 80 °C. Additional sodium iodide (0.008 g, 0.053 mmol) and *tert*-butyl 3-bromopropanoate (0.050 mL, 0.32 mmol) were added, and the mixture continued to stir for 4 days at 80 °C. Then the reaction mixture was cooled to ambient temperature, concentrated, diluted with *N,N*-dimethylformamide/water (3:1, 1.2 mL), filtered, and purified by preparative HPLC (Waters XBridge^{™} C18 5 µm OBD column, 30 × 100 mm, flow rate 40 mL/minute, 5-100% gradient of acetonitrile in 0.1% trifluoroacetic acid/water) to give the title compound (0.0093 g, 0.014 mmol, 13% yield). MS (ESI⁺) *m*/*z* 670 (M+H)⁺.

### Example 599D: 3-[{1,4-bis[2-(4-chloro-3-fluorophenoxy)acetamido]bicyclo[2.2.2]octan-2-yl}(methyl)amino]propanoic acid

The methodologies described in Example 596D substituting Example 599C for Example 596C, with shortened reaction time at 4 hours instead of overnight, and purification by preparative HPLC (Waters XBridge^{™} C18 5 µm OBD column, 30 × 100 mm, flow rate 40 mL/minute, 5-100% gradient of acetonitrile in 0.1% trifluoroacetic acid/water) gave the title compound. ¹H NMR (400 MHz, CDCl₃) *δ* ppm 7.33 (dd, J = 18.1, 9.4 Hz, 2H), 6.74 (d, J = 9.4 Hz, 2H), 6.66 (d, J = 8.9 Hz, 2H), 4.69 (s, 1H), 4.38 (d, J = 16.8 Hz, 4H), 3.04 (s, 2H), 3.02 (s, 3H), 2.95 (s, 2H), 1.94 - 1.50 (m, 7H), 0.88 - 0.81 (m, 10H); MS (ESI⁺) *m*/*z* 614 (M+H)⁺.

### Example 600: 2-(4-chloro-3-fluorophenoxy)-N-[(2S)-4-{[2-(4-chlorophenoxy)-1-cyanoethyl]amino}-2-hydroxybicyclo[2.2.2]octan-1-yl]acetamide (Compound 699)

To a solution of Example 338B (0.060 g, 0.175 mmol) in ethanol (EtOH) (3 mL) was added 2-(4-chlorophenoxy)acetaldehyde (0.036 g, 0.210 mmol), followed by a gradual addition of trimethylsilyl cyanide (0.030 mL, 0.228 mmol) and a final addition of ammonium chloride (2.81 mg, 0.053 mmol) in one portion. The resulting mixture was heated at 70 °C overnight. The mixture was concentrated, and the residue was purified by HPLC (see protocol in Example 529D) to provide the title compound (8.4 mg, 7.5%). ¹H NMR (400 MHz, DMSO-*d*₆) *δ* ppm 7.48 (t, J = 8.9 Hz, 1H), 7.37 (d, J = 8.9 Hz, 2H), 7.30 (d, J = 3.7 Hz, 1H), 7.12 - 6.96 (m, 3H), 6.83 (dd, J = 9.0, 2.9 Hz, 1H), 4.48-4.43 (m, 3H), 4.24 - 4.03 (m, 3H), 2.22 - 1.41 (m, 10H); MS (ESI⁺) *m*/*z* 522.3 (M+H)⁺.

### Example 601: [{1,4-bis[2-(4-chloro-3-fluorophenoxy)acetamido]bicyclo[2.2.2]octan-2-yl}(methyl)amino]acetic acid (Compound 700)

### Example 601A: tert-butyl 2-((1,4-bis(2-(4-chloro-3-fluorophenoxy)acetamido)bicyclo[2.2.2]octan-2-yl)(methyl)amino)acetate

The methodologies described in Example 599C substituting *tert*-butyl bromoacetate for *tert*-butyl 3-bromopropanoate with a shortened reaction time (overnight) and only the initial addition of the reactants gave the title compound. ¹H NMR (400 MHz, DMSO-*d*₆, diagnostic peaks) *δ* ppm 7.90 (s, 1H), 7.66 (s, 1H), 7.48 (td, J = 8.9, 5.5 Hz, 2H), 7.17 - 7.08 (m, 1H), 7.06 - 6.98 (m, 1H), 6.92 - 6.85 (m, 1H), 6.82 (dd, J = 8.8, 2.8 Hz, 1H), 4.47 (d, J = 14.8 Hz, 4H), 4.02 (d, J = 14.6 Hz, 5H), 2.41 (s, 4H), 2.18 (s, 3H), 2.18 - 2.04 (m, 1H), 1.97 (s, 2H), 1.81 (s, 3H), 1.75 (d, J = 12.7 Hz, 1H), 1.65 (s, 1H), 1.41 (s, 9H); MS (ESI⁺) *m*/*z* 656 (M+H)⁺.

### Example 601B: [{1,4-bis[2-(4-chloro-3-fluorophenoxy)acetamido]bicyclo[2.2.2]octan-2-yl}(methyl)amino]acetic acid

To a solution of Example 601A (0.032 g, 0.043 mmol) in dichloromethane (0.07 mL) was added trifluoroacetic acid (0.01 mL), and the resulting mixture was stirred at ambient temperature for 7 hours. The reaction mixture was concentrated and purified by preparative HPLC (Waters XBridge^{™} C18 5 µm OBD column, 30 × 100 mm, flow rate 40 mL/minute, 5-100% gradient of acetonitrile in 0.1% trifluoroacetic acid/water) to give the title compound (0.009 g, 0.014 mmol, 33% yield). ¹H NMR (400 MHz, CDCl₃) *δ* ppm 7.32 (d, J = 8.3 Hz, 2H), 6.70 (d, J = 21.5 Hz, 4H), 6.23 (s, 1H), 4.45 (m, 2H), 4.35 (m, 2H), 2.66 (s, 3H), 2.14 (m, 2H), 1.29 (m, 1H), 1.26 (m, 7H), 0.84 (m, 10H); MS (ESI⁺) *m*/*z* 600 (M+H)⁺.

### Example 602: N,N'-(2-{[3-(hydroxymethyl)oxetan-3-yl]amino}bicyclo[2.2.2]octane-1,4-diyl)bis[2-(4-chloro-3-fluorophenoxy)acetamide] (Compound 701)

The methodologies described for Example 599B substituting (3-aminooxetan-3-yl)methanol for methylamine hydrochloride gave the title compound. ¹H NMR (501 MHz, DMSO-*d*₆) *δ* ppm 7.73 (d, J = 3.8 Hz, 2H), 7.48 (td, J = 8.9, 1.4 Hz, 2H), 7.12 - 7.04 (m, 1H), 7.06 - 6.98 (m, 1H), 6.88 - 6.78 (m, 2H), 4.73 (s, 1H), 4.59 (d, J = 14.8 Hz, 1H), 4.56 - 4.48 (m, 2H), 4.46 (s, 2H), 4.48 - 4.42 (m, 1H), 4.33 (d, J = 7.3 Hz, 1H), 3.83 - 3.94 (m, 2H), 2.34 (d, J = 10.9 Hz, 1H), 2.24 - 2.18 (m, 2H), 2.12 (m, 1H), 1.89 (m, 1H), 1.84 (d, J = 10.8 Hz, 2H), 1.79 (d, J = 12.9 Hz, 1H), 1.62 (m, 1H); MS (ESI⁺) *m*/*z* 614 (M+H)⁺.

### Example 604: 2-(4-chloro-3-fluorophenoxy)-N-[(3S)-4-{[3-(4-chloro-3-fluorophenoxy)propyl]amino}-3-hydroxybicyclo[2.2.2]octan-1-yl]acetamide (Compound 703)

The title compound was prepared using the methodologies described above. ¹H NMR (501 MHz, DMSO-*d₆*) *δ* ppm 7.48 (t, J = 8.2 Hz, 1H), 7.47 - 7.41 (m, 1H), 7.43 (s, 1H), 7.07 - 6.99 (m, 2H), 6.81 (m, J = 9.0, 4.0, 2.9, 1.1 Hz, 2H), 4.46 (d, J = 3.6 Hz, 1H), 4.42 (s, 2H), 4.05 - 3.99 (m, 2H), 3.69 - 3.64 (m, 1H), 2.59 - 2.51 (m, 2H), 1.92 - 1.68 (m, 7H), 1.64 (td, J = 11.8, 5.7 Hz, 1H), 1.55 - 1.48 (m, 1H), 1.45 - 1.36 (m, 3H); MS (APCI⁺) *m*/*z* 529 (M+H)⁺.

### Example 605: N-(3-{bis[3-(4-chloro-3-fluorophenoxy)propyl]amino}bicyclo[1.1.1]pentan-1-yl)-2-(4-chloro-3-fluorophenoxy)acetamide (Compound 704)

Cesium carbonate (40 mg, 0.12 mmol) and the product of Example 27D (35 mg, 0.12 mmol) were combined with *N,N*-dimethylformamide (1.0 mL) and stirred at ambient temperature. The product of Example 588B (36 mg, 0.14 mmol) was added in one portion. The resulting mixture was stirred at ambient temperature for 3 hours and then at 60 °C for 18 hours. The reaction mixture was cooled to ambient temperature and filtered through a pad of diatomaceous earth. The resulting solution was directly purified by preparative HPLC [Waters XBridge^{™} C18 5 µm OBD^{™} column, 30 x 100 mm, flow rate 40 mL/minute, 5-100% gradient of acetonitrile in buffer (0.1 % trifluoroacetic acid)] to give the title compound (5 mg, 6.5 □mol, 5 % yield) as the trifluoroacetic acid salt. ¹H NMR (400 MHz, methanol-*d₄*) *δ* ppm 7.26 (t, J = 8.7 Hz, 1H), 7.21 (t, J = 8.8 Hz, 2H), 6.79 (dd, J = 11.1, 2.8 Hz, 1H), 6.73 (dd, J = 11.1, 2.8 Hz, 2H), 6.69 (ddd, J = 8.9, 2.9, 1.3 Hz, 1H), 6.63 (ddd, J = 8.9, 2.9, 1.3 Hz, 2H), 4.11 - 3.84 (m, 6H), 3.51 (t, J = 7.2 Hz, 2H), 3.15 - 3.06 (m, 2H), 2.47 (br s, 6H), 2.12 - 1.89 (m, 4H); MS (ESI⁺) *m*/*z* 657 (M+H)⁺.

### Example 606: 2-(4-chloro-3-fluorophenoxy)-N-(3-{[3-(4-chloro-3-fluorophenoxy)propyl]amino}bicyclo[1.1.1]pentan-1-yl)acetamide (Compound 705)

The preparative HPLC purification in Example 605 also afforded factions containing this title compound. These fractions were combined and concentrated under reduced pressure. The resulting residue was taken up in methanol, filtered through a glass microfiber frit and purified by preparative HPLC [Waters XBridge^{™} C18 5 µm OBD^{™} column, 30 × 100 mm, flow rate 40 mL/minute, 5-100% gradient of acetonitrile in buffer (0.025 M aqueous ammonium bicarbonate, adjusted to pH 10 with ammonium hydroxide)] to give the title compound (5 mg, 10.6 µmol, 9 % yield). ¹H NMR (400 MHz, DMSO-*d*₆/D₂O) *δ* ppm 7.46 (t, J = 8.9 Hz, 1H), 7.43 (t, J = 8.9 Hz, 1H), 7.07 - 6.97 (m, 2H), 6.88 - 6.77 (m, 2H), 4.43 (s, 2H), 4.00 (t, J = 6.4 Hz, 2H), 2.56 (t, J = 7.0 Hz, 2H), 1.95 (s, 6H), 1.80 (p, J = 6.7 Hz, 2H); MS (ESI⁺) *m*/*z* 471 (M+H)⁺.

### Example 607: 2-({(3S)-4-[2-(4-chloro-3-fluorophenoxy)acetamido]-3-hydroxybicyclo[2.2.2]octan-1-yl}amino)-4-(4-chlorophenoxy)butanoic acid (Compound 706)

### Example 607A: ethyl 4-(4-chlorophenoxy)butanoate

A mixture of 4-chlorophenol (10 g, 78 mmol) and ethyl 4-bromobutanoate (22.8 g, 117 mmol) in ethanol (100 mL) with K₂CO₃ (16.1 g, 117 mmol) was refluxed for 12 hours. The reaction mixture was cooled and filtered. The filtrate was concentrated under reduced pressure. To the residue was added ethyl acetate (100 mL) and H₂O (70 mL). The two phases were separated, and the aqueous phase was extracted with ethyl acetate (3 × 40 mL). The combined organic phases were combined, dried over anhydrous Na₂SO₄, filtered and concentrated under reduced pressure. The residue was purified by column chromatography (SiO₂, petroleum ether:ethyl acetate = 100:1 to 5:1) to give the title compound (10 g, 39.1 mmol, 50% yield). ¹H NMR (400MHz, DMSO-*d*₆) *δ* ppm = 7.33 - 7.27 (m, 2H), 6.96 - 6.90 (m, 2H), 4.06 (q, J=7.1 Hz, 2H), 3.97 (t, J=6.3 Hz, 2H), 2.44 (t, J=7.3 Hz, 2H), 1.95 (quin, J=6.8 Hz, 2H), 1.17 (t, J=7.1 Hz, 3H).

### Example 607B: ethyl 2-bromo-4-(4-chlorophenoxy)butanoate

To a stirred solution of lithium diisopropylamide (0.494 mL, 0.989 mmol, 2 M in *n-*hexane) in anhydrous tetrahydrofuran (2 mL) was added chlorotrimethylsilane (0.188 mL, 1.48 mmol) at -70°C followed by Example 607A (0.2 g, 0.824 mmol) in tetrahydrofuran (0.5 mL). The reaction mixture was stirred at -70°C for 2 hours and 1-bromopyrrolidine-2,5-dione (0.147 g, 0.824 mmol) was added at -70°C. The mixture was then stirred at 15 °C for 1.5 hours. To the mixture was added ethyl acetate (4 mL) and H₂O (4 mL). The two phases were separated, and the aqueous phase was extracted with ethyl acetate (3 × 4 mL). The combined organic phases were dried over anhydrous Na₂SO₄, filtered, and concentrated under reduced pressure. The residue was purified via prep-thin layer chromatography (TLC( (SiO₂, petroleum ether: ethyl acetate = 10:1) to give the tile compound (0.15 g, 0.42 mmol, 50% yield). ¹H NMR (400MHz, CDCl₃) *δ* ppm 7.26 - 7.22 (m, 2H), 6.86 - 6.80 (m, 2H), 4.56 (dd, J=5.7, 8.8 Hz, 1H), 4.26 (ttd, J=3.6, 7.1, 10.7 Hz, 2H), 4.15 - 4.04 (m, 2H), 2.63 - 2.52 (m, 1H), 2.45 - 2.35 (m, 1H), 1.31 (t, J=7.2 Hz, 3H).

### Example 607C: ethyl 2-(1(3S)-4-[2-(4-chloro-3-fluorophenoxy)acetamido]-3-hydroxybicyclo[2.2.2]octan-1-yl}amino)-4-(4-chlorophenoxy)butanoate

A mixture of Example 338B (0.200 g, 0.583 mmol), Example 607B (0.281 g, 0.875 mmol), and triethylamine (0.163 mL, 1.167 mmol) in dimethylformamide (DMF) (4 mL) was stirred for 4 days. The reaction was quenched with brine and extracted with ethyl acetate (2×). The combined organic layers were washed with brine, dried over MgSO₄, filtered, and concentrated. The residue was purified on a 24 g column using the Biotage^{®} Isolera^{™} One flash system eluting with heptanes/ethyl acetate (5:5 to 2:8) to provide the title compound (90.6 mg, 27%). MS (ESI⁺) *m*/*z* 583.3 (M+H)⁺.

### Example 607D: 2-({(3S)-4-[2-(4-chloro-3-fluorophenoxy)acetamido]-3-hydroxybicyclo[2.2.2]octan-1-yl}amino)-4-(4-chlorophenoxy)butanoic acid

A solution of Example 607C (88.0 mg, 0.151 mmol) in tetrahydrofuran (THF) (1.5 ml) and methanol (MeOH) (1.2 ml) was treated with a solution of lithium hydroxide monohydrate (18.99 mg, 0.452 mmol) in water (0.8 ml). The mixture was stirred at 35 °C for 2 hours. Most of the solvent was evaporated (bath temperature at 35°C). The residue was treated with ethyl acetate and 5% citric acid. The suspension was filtered. The solid was rinsed with water and ethyl acetate, and vacuum oven-dried (T= 45 °C) to provide the title compound (43.0 mg, 51%). ¹H NMR (400 MHz, DMSO-*d*₆) *δ* ppm 7.47 (t, J = 8.9 Hz, 1H), 7.39 - 7.24 (m, 3H), 7.04 (dd, J = 11.4, 2.9 Hz, 1H), 7.01 - 6.90 (m, 2H), 6.82 (ddd, J = 9.0, 2.9, 1.2 Hz, 1H), 5.15 (s, brd, 1H), 4.45 (s, 2H), 4.14-4.00 (m, 3H), 3.48-3.39. (m, 1H), 2.17 - 1.33 (m, 12H); MS (ESI⁺) *m*/*z* 555.3 (M+H)⁺.

### Example 608: N,N'-{2-[(2-hydroxyethyl)amino]bicyclo[2.2.2]octane-1,4-diyl}bis[2-(4-chloro-3-fluorophenoxy)acetamide] (Compound 707)

The methodologies described for Example 599B substituting 2-aminoethanol for methylamine hydrochloride and, although resulting in incomplete conversion, the reaction was modified with shortened reaction time (overnight) to give the title compound. ¹H NMR (501 MHz, CDCl₃) *δ* ppm 7.68 (s, 1H), 7.37 - 7.31 (m, 2H), 6.81 - 6.64 (m, 4H), 6.29 (d, J = 13.2 Hz, 1H), 4.39 (d, J = 12.2 Hz, 5H), 3.16 - 3.06 (m, 4H), 2.28 (t, J = 12.2 Hz, 2H), 2.03 (t, J = 11.8 Hz, 2H), 1.27 (d, J = 14.9 Hz, 3H), 0.87 (dd, J = 17.7, 6.6 Hz, 3H), 0.81 (s, 3H); MS (ESI⁺) *m*/*z* 572 (M+H)⁺.

### Example 610: 2-[(4-fluoro-1H-indazol-6-yl)oxy]-N-[(3S)-3-hydroxy-4-{2-[(1H-indazol-6-yl)oxy]acetamido}bicyclo[2.2.2]octan-1-yl]acetamide (Compound 709)

The title compound was prepared using the methodologies described above. ¹H NMR (400 MHz, DMSO-*d*₆) *δ* ppm 13.17 (s, 1H), 12.84 (s, 1H), 8.04 (s, 1H), 7.94 (s, 1H), 7.64 (d, J = 8.8 Hz, 1H), 7.53 (s, 1H), 7.27 (s, 1H), 6.93 - 6.87 (m, 1H), 6.80 (dd, J = 8.8, 2.1 Hz, 1H), 6.76 - 6.70 (m, 1H), 6.64 (dd, J = 11.7, 1.8 Hz, 1H), 5.16 (d, J = 4.2 Hz, 1H), 4.53 - 4.42 (m, 4H), 4.05 - 3.94 (m, 1H), 2.36 - 2.25 (m, 1H), 2.25 - 2.13 (m, 1H), 2.01 - 1.73 (m, 8H); MS (ESI⁺) *m*/*z* 523 (M+H)⁺.

### Example 611: N,N'-[(2S)-2-hydroxybicyclo[2.2.2]octane-1,4-diyl]bis{2-[(4-fluoro-1H-indazol-6-yl)oxy]acetamide} (Compound 710)

The title compound was prepared using the methodologies described above. ¹H NMR (400 MHz, DMSO-*d₆*) *δ* ppm 13.17 (br s, 2H), 8.05 (d, J = 3.5 Hz, 2H), 7.52 (s, 1H), 7.28 (s, 1H), 6.75 (dt, J = 16.5, 1.4 Hz, 2H), 6.68 - 6.61 (m, 2H), 5.13 (d, J = 4.2 Hz, 1H), 4.50 (s, 2H), 4.47 (s, 2H), 4.06 - 3.99 (m, 1H), 2.29 (td, J = 10.4, 9.5, 5.1 Hz, 1H), 2.19 - 2.07 (m, 1H), 2.02 - 1.72 (m, 8H); MS (ESI⁺) *m*/*z* 541 (M+H)⁺.

### Example 612: N,N'-{2-[(2-hydroxyethyl)(methyl)amino]bicyclo[2.2.2]octane-1,4-diyl}bis[2-(4-chloro-3-fluorophenoxy)acetamide] (Compound 711)

The methodologies described for Example 599B substituting 2-(methylamino)ethanol for methylamine hydrochloride gave the title compound. ¹H NMR (500 MHz, CDCl₃) *δ* ppm 7.32 (td, J = 8.6, 4.6 Hz, 2H), 6.7 - 6.70 (m, 3H), 6.65 (dd, J = 8.7, 2.8 Hz, 2H), 6.15 (s, 1H), 4.76 (d, J = 11.7 Hz, 1H), 4.56 (d, J = 11.7 Hz, 1H), 4.40 - 4.30 (m, 4H), 4.10 (d, J = 9.4 Hz, 1H), 2.70 - 2.61 (m, 1H), 2.52 (ddd, J = 12.6, 9.0, 3.1 Hz, 1H), 2.39 - 2.27 (m, 1H), 2.15 (s, 3H), 2.05 (m, 1H), 1.93 - 1.81 (m, 2H), 0.84 (m, 7H); MS (ESI⁺) *m*/*z* 589 (M+H)⁺.

### Example 613: N-{(3S)-4-[2-(4-chloro-3-fluorophenoxy)acetamido]-3-hydroxybicyclo[2.2.2]octan-1-yl}-3-(4-chlorophenyl)-3-(methylamino)propanamide (Compound 712)

### Example 613A: (S)-(9H-fluoren-9-yl)methyl tert-butyl (2-hydroxybicyclo[2.2.2]octane-1,4-diyl)dicarbamate

To a suspension of Example 445B (1.99 g, 6.83 mmol) and Na₂CO₃ (2.171 g, 20.48 mmol) in tetrahydrofuran (20 mL) and water (10 mL) at 0 °C was added solid (9*H*-fluoren-9-yl)methyl carbonochloridate (2.12 g, 8.19 mmol) at once. The reaction mixture was stirred at 0 °C for 5 minutes, allowed to warm to ambient temperature, stirred for 2 hours, diluted with ethyl acetate (30 mL), washed with water (30 mL) and brine, dried (Na₂SO₄), and concentrated to give the title compound (3.83 g, 8.00 mmol, 117 % yield), which was carried forward without purification. MS (ESI⁺) *m*/*z* 379 (M-C(O)OC(CH₃)₃+H)⁺.

### Example 613B: (S)-(9H-fluoren-9-yl)methyl (4-amino-3-hydroxybicyclo[2.2.2]octan-1-yl)carbamate hydrochloride

A solution of HCl (51 mL, 204 mmol, 4 M in dioxane) was added to a solution of Example 613A (48.8 g, 102 mmol) in methanol (100 mL), and the reaction mixture was heated to 50 °C for 90 minutes. Then the mixture was concentrated, and the residue was triturated with ethyl acetate (3 × 100 mL). The solid was vacuum oven dried to give the title compound (45.06 g, 109 mmol, 106 % yield), which was carried forward without further purification. MS (ESI⁺) *m*/*z* 379 (M+H)⁺.

### Example 613C: (S)-(9H-fluoren-9-yl)methyl (4-(2-(4-chloro-3-fluorophenoxy)acetamido)-3-hydroxybicyclo[2.2.2]octan-1-yl)carbamate

A suspension of Example 613B (5.64 g, 13.6 mmol), 2-(4-chloro-3-fluorophenoxy)acetic acid (3.198 g, 15.63 mmol), and triethylamine (4.75 ml, 34.1 mmol) in *N,N*-dimethylformamide (30 mL) at ambient temperature was stirred for 3 minutes, and HATU (1-[bis(dimethylamino)methylene]-1*H*-1,2,3-triazolo[4,5-b]pyridinium 3-oxid hexafluorophosphate) (6.459 g, 16.99 mmol) was then added. The reaction mixture was stirred for 60 minutes, diluted with ethyl acetate (100 mL), washed with 0.5 N NaOH (100 mL), water (125 mL), and brine (50 mL), and concentrated to give a residue. This material was dissolved in methanol (4 mL) and tetrahydrofuran (4 mL), and stirred with 1 N aqueous NaOH (4.1 mL, 4.1 mmol) for 20 minutes, concentrated to remove most of the volatiles, diluted with ethyl acetate (75 mL), washed with water (50 mL) and brine (50 mL), dried (Na₂SO₄), and concentrated to give the title compound (8.1 g, 14 mmol, 105 % yield), which was carried forward without purification. MS (ESI⁺) *m*/*z* 565 (M+H)⁺.

### Example 613D: (S)-N-(4-amino-2-hydroxybicyclo[2.2.2]octan-1-yl)-2-(4-chloro-3-fluorophenoxy)acetamide

To a solution of Example 613C (7.68 g, 13.6 mmol) in acetonitrile (40 mL) was added diethylamine (14 mL, 135 mmol), and the reaction mixture was stirred for 90 minutes, concentrated, diluted with water (25 mL), acidified with 1 N HCl (15 mL), and washed with ethyl acetate (2 × 20 mL). The aqueous layer was basified with 50% aqueous NaOH (1 mL) and extracted with dichloromethane (3 × 25 mL). The combined organic fractions were dried (Na₂SO₄) and concentrated. The residue was triturated with methyl *tert*-butyl ether (2 × 10 mL), and dried (vacuum oven) to give the title compound (3.63 g, 10.6 mmol, 78% yield). MS (ESI⁺) *m*/*z* 384 (M+CH₃CN)⁺.

### Example 613E: (S)-N-(4-(2-(4-chloro-3-fluorophenoxy)acetamido)-3-hydroxybicyclo[2.2.2]octan-1-yl)-3-(4-chlorophenyl)-3-oxopropanamide

A suspension of Example 613D (0.0550 g, 0.160 mmol) and ethyl 3-(4-chlorophenyl)-3-oxopropanoate (0.0422 g, 0.186 mmol) in toluene (0.65 mL) was stirred at 100 °C overnight and then concentrated. The residue was chromatographed (dichloromethane - 5% methanol/dichloromethane) and re-chromatographed (66-80% ethyl acetate/dichloromethane) to give the title compound (0.031 g, 0.058 mmol, 36 % yield). MS (ESI⁺) *m*/*z* 523 (M+H)⁺.

### Example 613F: N-{(3S)-4-[2-(4-chloro-3-fluorophenoxy)acetamido]-3-hydroxybicyclo[2.2.2]octan-1-yl}-3-(4-chlorophenyl)-3-(methylamino)propanamide

The methodologies described for Example 599B substituting Example 613E for Example 599A and with shortened reaction time (overnight) gave the title compound. ¹H NMR (500 MHz, DMSO-*d*₆) *δ* ppm 8.94 - 8.89 (m, 2H), 7.64 (s, 1H), 7.57 - 7.51 (m, 2H), 7.51 - 7.44 (m, 2H), 7.48 - 7.42 (m, 1H), 7.24 (d, J = 1.6 Hz, 1H), 7.04 (dd, J = 11.4, 2.8 Hz, 1H), 6.81 (ddd, J = 9.0, 2.9, 1.2 Hz, 1H), 5.03 (s, 1H), 4.55 - 4.48 (m, 1H), 4.45 (s, 2H), 3.98 (d, J = 9.4 Hz, 1H), 2.82 - 2.68 (m, 2H), 2.43 (t, J = 5.3 Hz, 3H), 2.18 - 1.93 (m, 2H), 1.91 - 1.52 (m, 8H); MS (ESI⁺) *m*/*z* 538 (M+H)⁺.

### Example 614: N-{(3S)-4-[2-(4-chloro-3-fluorophenoxy)acetamido]-3-hydroxybicyclo[2.2.2]octan-1-yl}-3-(4-chlorophenyl)-3-[(oxetan-3-yl)amino]propanamide (Compound 713)

The methodologies described in Example 599B with the following modifications provided the title compound: (1) substituting Example 613E for Example 599A, (2) substituting oxetan-3-amine for methylamine hydrochloride, and (3) with shortened reaction time (overnight) gave the title compound. ¹H NMR (400 MHz, DMSO-*d*₆) *δ* ppm 9.73 (s, 2H), 7.64 (s, 1H), 7.56 - 7.48 (m, 2H), 7.48 (d, J = 9.0 Hz, 2H), 7.45 (s, 1H), 7.23 (s, 1H), 7.03 (dd, J = 11.4, 2.9 Hz, 1H), 6.81 (dd, J = 9.2, 2.9 Hz, 1H), 4.70 - 4.59 (m, 2H), 4.54 (s, 1H), 4.44 (s, 2H), 4.31 (dd, J = 14.8, 7.1 Hz, 3H), 3.97 (d, J = 9.3 Hz, 1H), 2.77 (t, J = 5.8 Hz, 2H), 2.22 - 1.93 (m, 1H), 1.83 (d, J = 10.9 Hz, 1H), 1.78 - 1.71 (m, 4H), 1.59 (q, J = 12.4, 10.5 Hz, 4H), 1.24 (s, 2H); MS (ESI⁺) *m*/*z* 580 (M+H)⁺.

### Example 615: N,N'-{2-[(2,2,2-trifluoroethyl)amino]bicyclo[2.2.2]octane-1,4-diyl}bis[2-(4-chloro-3-fluorophenoxy)acetamide] (Compound 714)

The methodologies described for Example 599B substituting 2,2,2-trifluoroethanamine for methylamine hydrochloride and, although resulting in incomplete conversion, the reaction was modified with a shortened reaction time (overnight) to give the title compound. ¹H NMR (400 MHz, DMSO-*d*₆) *δ* ppm 7.54 (d, J = 2.4 Hz, 2H), 7.49 (q, J = 9.1 Hz, 2H), 7.07 - 6.94 (m, 2H), 6.81 (ddt, J = 8.9, 6.4, 1.9 Hz, 2H), 4.45 (d, J = 8.3 Hz, 4H), 3.33 - 3.19 (m, 1H), 3.09 (d, J = 7.4 Hz, 1H), 2.41 - 2.32 (m, 2H), 2.00 - 1.88 (m, 3H), 1.91 - 1.72 (m, 6H); MS (ESI⁺) *m*/*z* 610 (M+H)⁺.

### Example 616: N,N'-[2-(3,3-difluoroazetidin-1-yl)bicyclo[2.2.2]octane-1,4-diyl]bis[2-(4-chloro-3-fluorophenoxy)acetamide] (Compound 715)

The methodologies described for Example 599B substituting 3,3-difluoroazetidine hydrochloride for methylamine hydrochloride and with shortened reaction time (overnight) gave the title compound. ¹H NMR (400 MHz, DMSO-*d*₆) *δ* ppm 7.54 - 7.41 (m, 5H), 7.05 - 6.99 (m, 2H), 6.81 (dddd, J = 8.8, 4.3, 3.0, 1.2 Hz, 2H), 4.47 (s, 2H), 4.42 (s, 2H), 3.54 (dq, J = 34.0, 11.4 Hz, 4H), 2.09 - 2.00 (m, 2H), 1.93 (s, 2H), 1.85 - 1.72 (m, 3H), 1.57 (d, J = 12.0 Hz, 2H), 1.54 - 1.39 (m, 2H); MS (ESI⁺) *m*/*z* 604 (M+H)⁺.

### Example 617: N,N'-[2-(methoxymethyl)bicyclo[2.2.2]octane-1,4-diyl]bis[2-(4-chloro-3-fluorophenoxy)acetamide] (Compound 716)

### Example 617A: methyl 1,4-bis(2-(4-chloro-3-fluorophenoxy)acetamido)bicyclo[2.2.2]octane-2-carboxylate

To a mixture of Example 470D (250 mg, 0.650 mmol), 2-(4-chloro-3-fluorophenoxy)acetic acid (166 mg, 0.812 mmol), and *N*-ethyl-*N*-isopropylpropan-2-amine (0.397 mL, 2.274 mmol) in *N,N*-dimethylformamide (5.0 mL), 2-(3*H*-[1,2,3]triazolo[4,5-*b*]pyridin-3-yl)-1,1,3,3-tetramethylisouronium hexafluorophosphate(V) (371 mg, 0.974 mmol) was added, and the mixture was stirred at ambient temperature for 30 minutes. *N,N-*Dimethylformamide was removed under high vacuum, and the residue was purified by HPLC (Phenomenex^{®} Luna^{®} C18(2) 10 µm 100Å AXIA^{™} column (250 mm × 50 mm). A 30-100% gradient of acetonitrile (A) and 0.1% trifluoroacetic acid in water (B) was used over 25 minutes, at a flow rate of 50 mL/minute) to give 300 mg of the title compound. ¹H NMR (400 MHz, DMSO-*d*₆) *δ* ppm 7.56 (d, J = 9.3 Hz, 2H), 7.48 (td, J = 8.9, 1.2 Hz, 2H), 7.01 (td, J = 11.3, 2.8 Hz, 2H), 6.80 (dtd, J = 9.0, 3.0, 1.2 Hz, 2H), 4.50 - 4.36 (m, 4H), 3.56 (s, 3H), 2.19 (dd, J = 12.8, 5.9 Hz, 1H), 2.13 - 2.02 (m, 2H), 2.06 - 1.84 (m, 5H), 1.74 - 1.56 (m, 2H); MS (+ESI) *m*/*z* 571.1 (M+H)⁺.

### Example 617B: N,N'-(2-(hydroxymethyl)bicyclo[2.2.2]octane-1,4-diyl)bis(2-(4-chloro-3-fluorophenoxy)acetamide)

To a solution of Example 617A (300 mg, 0.525 mmol) in tetrahydrofuran (10.0 mL) was added aluminum(III) lithium hydride (1.838 mL, 1.838 mmol) slowly at 0 °C, and the reaction was stirred at ambient temperature for 60 minutes. Water was added, and the mixture was extracted with dichloromethane (2 × 50 mL). The organic fractions were dried over magnesium sulfate and filtered. The filtrate was concentrated, and the residue was purified by HPLC (Phenomenex^{®} Luna^{®} C18(2) 10 µm 100Å AXIA^{™} column (250 mm × 50 mm). A 30-100% gradient of acetonitrile (A) and 0.1% trifluoroacetic acid in water (B) was used over 25 minutes, at a flow rate of 50 mL/minute) to give 100 mg of the title compound. ¹H NMR (400 MHz, DMSO-*d*₆) *δ* ppm 8.39 (s, 1H), 7.53 - 7.43 (m, 3H), 7.02 (dt, J = 11.3, 3.1 Hz, 2H), 6.80 (dd, J = 9.0, 2.8 Hz, 2H), 5.10 (s, 1H), 4.42 (d, J = 7.2 Hz, 3H), 4.37 (d, J = 14.6 Hz, 1H), 3.57 (dd, J = 10.8, 8.3 Hz, 1H), 2.54 (s, 0H), 2.44 (p, J = 5.8 Hz, 1H), 2.23 - 2.11 (m, 1H), 1.99 (t, J = 11.9 Hz, 1H), 1.91 (dd, J = 11.9, 6.0 Hz, 1H), 1.84 (d, J = 10.1 Hz, 3H), 1.74 (dd, J = 15.5, 10.8 Hz, 2H), 1.45 (ddd, J = 12.8, 7.0, 2.7 Hz, 1H); MS (+ESI) *m*/*z* 545.3 (M+H)⁺.

### Example 617C: N,N'-(2-(methoxymethyl)bicyclo[2.2.2]octane-1,4-diyl)bis(2-(4-chloro-3-fluorophenoxy)acetamide)

To a solution of Example 617B (0.098 g, 0.180 mmol) in dichloromethane (2.5 mL) was added a few drops of (diethyloxonio)trifluoroborate (2.285 µl, 0.018 mmol) followed by addition of (diazomethyl)trimethylsilane (0.135 mL, 0.271 mmol) dropwise at 0°C, and the reaction was stirred at ambient temperature for 30 minutes. Another 1.5 equivalents of diazomethyl)trimethylsilane was added with continued stirring overnight. Water was added, and the mixture was extracted with dichloromethane (2×20 mL). The organic layers were combined, dried over magnesium sulfate and filtered. The filtrate was concentrated, and the residue was purified by HPLC (Phenomenex^{®} Luna^{®} C18(2) 10 µm 100Å AXIA^{™} column (250 mm × 50 mm). A 35-100% gradient of acetonitrile (A) and 0.1% trifluoroacetic acid in water (B) was used over 25 minutes, at a flow rate of 50 mL/minute) to give 12 mg of the title compound. ¹H NMR (400 MHz, DMSO-*d*₆) *δ* ppm 7.72 (s, 1H), 7.48 (q, J = 8.6 Hz, 3H), 7.02 (dt, J = 11.4, 2.5 Hz, 2H), 6.81 (dtd, J = 9.0, 3.1, 1.2 Hz, 2H), 4.43 (d, J = 7.3 Hz, 4H), 3.44 - 3.37 (m, 2H), 3.20 (s, 3H), 3.24 - 3.16 (m, 1H), 2.17 - 2.06 (m, 1H), 2.06 - 1.92 (m, 3H), 1.84 (tdd, J = 19.6, 10.9, 4.5 Hz, 5H), 1.56 (ddd, J = 12.9, 6.7, 2.1 Hz, 1H); MS (+ESI) *m*/*z* 557.0 (M+H)⁺.

### Example 618: 1,4-bis[2-(4-chloro-3-fluorophenoxy)acetamido]bicyclo[2.2.2]oct-2-ene-2-carboxylic acid (Compound 717)

To a solution of Example 619 (0.0094 g, 0.017 mmol) in tetrahydrofuran (0.040 mL) and methanol (0.020 mL) was added 1 N aqueous sodium hydroxide (0.020 mL, 0.020 mmol), and the mixture was stirred for 3 hours. Then the mixture was diluted with water, washed with dichloromethane, acidified with 1 N aqueous HCl (0.040 mL), extracted with dichloromethane, dried (Na₂SO₄), and concentrated to give the title compound (0.0056 g, 10.08 µmol, 61.1 % yield). ¹H NMR (400 MHz, DMSO-*d*₆) *δ* ppm 9.64 (s, 1H), 8.25 (s, 1H), 7.50 (td, J = 8.9, 5.0 Hz, 2H), 7.26 (s, 1H), 7.07 (dd, J = 11.3, 2.9 Hz, 2H), 6.86 (dt, J = 8.2, 3.5 Hz, 2H), 4.56 (s, 2H), 4.50 (s, 2H), 2.49 - 2.40 (m, 2H, buried), 1.81 (td, J = 10.9, 4.4 Hz, 2H), 1.70 (td, J = 10.5, 3.8 Hz, 2H), 1.48 (dd, J = 11.9, 4.6 Hz, 2H); MS (ESI⁺) *m*/*z* 555.1 (M+H)⁺.

### Example 619: methyl 1,4-bis[2-(4-chloro-3-fluorophenoxy)acetamido]bicyclo[2.2.2]oct-2-ene-2-carboxylate (Compound 718)

### Example 619A: 1,4-bis(2-(4-chloro-3-fluorophenoxy)acetamido)bicyclo[2.2.2]oct-2-en-2-yl trifluoromethanesulfonate

To a suspension of Example 599A (0.1478 g, 0.280 mmol) and 1,1,1-trifluoro-N-phenyl-N-((trifluoromethyl)sulfonyl)methanesulfonamide (0.2017 g, 0.565 mmol) in tetrahydrofuran (0.56 mL) at 0 °C was added dropwise a 1 M solution of lithium bis(trimethylsilyl)amide in tetrahydrofuran (0.56 mL, 0.560 mmol), and the mixture was stirred at 0 °C for 10 minutes, and at ambient temperature for 1 hour. The mixture was diluted with more tetrahydrofuran (0.56 mL), heated to 60 °C for 15 hours, diluted with ethyl acetate, washed with 1 N aqueous NaOH and brine, dried (Na₂SO₄), and concentrated. The residue was chromatographed on silica gel (5% ethyl acetate/CH₂Cl₂) to give the title compound (0.070 g, 0.106 mmol, 37.9 % yield). ¹H NMR (501 MHz, DMSO-*d*₆) *δ* ppm 8.42 (s, 1H), 8.35 (s, 1H), 7.48 (dt, J = 11.3, 8.8 Hz, 2H), 7.04 (ddd, J = 16.9, 11.3, 2.9 Hz, 2H), 6.84 (dddd, J = 10.2, 8.9, 2.9, 1.2 Hz, 2H), 6.27 (s, 1H), 4.56 (d, J = 1.1 Hz, 4H), 2.22 - 2.09 (m, 2H), 1.81 (dtd, J = 23.2, 11.4, 5.4 Hz, 4H), 1.67 (td, J = 10.6, 10.0, 4.2 Hz, 2H).; MS (ESI⁻) *m*/*z* 656.7 (M+H)⁻.

### Example 619B: methyl 1,4-bis[2-(4-chloro-3-fluorophenoxy)acetamido]bicyclo[2.2.2]oct-2-ene-2-carboxylate

A solution of Example 619A (0.0256 g, 0.039 mmol) and [1,1'-bis(diphenylphosphino)ferrocene]dichloropalladium(II) (PdCl₂(dppf), 1.420 mg, 1.941 µmol) in methanol (2 mL) and triethylamine (6.49 µL, 0.047 mmol) was degassed with nitrogen, pressurized with carbon monoxide (50 psi), and heated to 40 °C for 16 hours. The reaction mixture was concentrated, and the residue was chromatographed on silica gel (10% ethyl acetate/dichloromethane) to give the title compound (0.0140 g, 0.025 mmol, 63.3 % yield). ¹H NMR (501 MHz, DMSO-*d*₆) *δ* ppm 8.70 (s, 1H), 8.27 (s, 1H), 7.49 (td, J = 8.9, 1.4 Hz, 2H), 7.13 (s, 1H), 7.07 (ddd, J = 11.4, 10.5, 2.9 Hz, 2H), 6.86 (ddd, J = 9.0, 2.9, 1.2 Hz, 2H), 4.56 (s, 2H), 4.52 (s, 2H), 3.61 (s, 3H), 2.04 - 1.97 (m, 2H), 1.84 - 1.78 (m, 2H), 1.77 - 1.68 (m, 4H); MS (ESI⁺) *m*/*z* 569.0 (M+H)⁺.

### Example 620: N,N'-[2-(3-hydroxyazetidin-1-yl)bicyclo[2.2.2]octane-1,4-diyl]bis[2-(4-chloro-3-fluorophenoxy)acetamide] (Compound 719)

The methodologies described for Example 599B substituting azetidin-3-ol for methylamine hydrochloride and, although resulting in incomplete conversion, the reaction was modified with a shortened reaction time (3 hours) to give the title compound. ¹H NMR (400 MHz, DMSO-*d*₆) *δ* ppm 7.87 (s, 1H), 7.75 (s, 1H), 7.54 - 7.43 (m, 2H), 7.11 (s, 1H), 7.03 (ddd, J = 11.3, 5.4, 2.9 Hz, 2H), 6.81 (dd, J = 7.6, 4.6 Hz, 1H), 4.56 (d, J = 1.9 Hz, 2H), 4.45 (s, 2H), 4.33 (m, 1H), 3.98 (m, 2H), 3.69 (m, 2H), 2.07 (d, J = 10.3 Hz, 2H), 1.96 - 1.65 (m, 6H), 1.48 (s, 2H); MS (ESI⁺) *m*/*z* 586 (M+H)⁺.

### Example 621: 2-[(4-fluoro-1H-indazol-6-yl)oxy]-N-[(3S)-3-hydroxy-4-(2-{[6-(trifluoromethyl)pyridin-3-yl]oxy}acetamido)bicyclo[2.2.2]octan-1-yl]acetamide (Compound 720)

The title compound was prepared using the methodologies described above. ¹H NMR (501 MHz, DMSO-*d₆*) *δ* ppm 13.17 (s, 1H), 8.44 (d, J = 2.8 Hz, 1H), 8.04 (s, 1H), 7.85 (d, J = 8.7 Hz, 1H), 7.55 (dd, J = 8.7, 2.9 Hz, 1H), 7.52 (s, 1H), 7.40 (s, 1H), 6.75 - 6.70 (m, 1H), 6.64 (dd, J = 11.6, 1.8 Hz, 1H), 5.05 (d, J = 4.1 Hz, 1H), 4.65 (s, 2H), 4.47 (s, 2H), 4.11 - 4.06 (m, 1H), 2.28 (ddd, J = 12.0, 9.6, 2.1 Hz, 1H), 2.06 - 1.73 (m, 9H); MS (ESI⁺) *m*/*z* 552 (M+H)⁺.

### Example 622: 2-[(4-fluoro-1H-indazol-6-yl)oxy]-N-{(3S)-4-[2-(4-fluorophenoxy)acetamido]-3-hydroxybicyclo[2.2.2]octan-1-yl}acetamide (Compound 721)

The title compound was prepared using the methodologies described above. ¹H NMR (400 MHz, DMSO-*d₆*) *δ* ppm 13.17 (s, 1H), 8.04 (s, 1H), 7.52 (s, 1H), 7.21 (s, 1H), 7.17 - 7.08 (m, 2H), 6.99 - 6.91 (m, 2H), 6.76 - 6.71 (m, 1H), 6.64 (dd, J = 11.6, 1.8 Hz, 1H), 5.14 (d, J = 4.3 Hz, 1H), 4.47 (s, 2H), 4.39 (s, 2H), 4.05 - 3.95 (m, 1H), 2.29 (td, J = 10.8, 9.7, 5.4 Hz, 1H), 2.20 - 2.09 (m, 1H), 2.03 - 1.72 (m, 8H); MS (APCI⁺) *m*/*z* 501 (M+H)⁺.

### Example 623: N,N'-{2-[(oxetan-3-yl)amino]bicyclo[2.2.2]octane-1,4-diyl}bis[2-(4-chloro-3-fluorophenoxy)acetamide] (Compound 722)

The methodologies described for Example 599B substituting oxetan-3-amine for methylamine hydrochloride and, although resulting in incomplete conversion, the reaction was modified with a shortened reaction time (overnight) to give the title compound. ¹H NMR (400 MHz, DMSO-*d*₆) *δ* ppm 7.78 (d, J = 12.6 Hz, 2H), 7.53 - 7.44 (m, 2H), 7.10 (dd, J = 11.3, 3.0 Hz, 1H), 7.03 (dd, J = 11.4, 2.9 Hz, 1H), 6.87 (dd, J = 8.7, 2.8 Hz, 1H), 6.82 (ddd, J = 9.2, 2.9, 1.2 Hz, 1H), 4.78 - 4.65 (m, 3H), 4.63 - 4.50 (m, 2H), 4.50 (s, 1H), 4.46 (s, 2H), 4.15 (s, 1H), 3.71 (m, 1H), 2.29 (m, 2H), 2.15 (m, 2H), 1.92 (m, 2H), 1.80 (d, J = 7.7 Hz, 3H), 1.60 (s, 1H) ; MS (ESI⁺) *m*/*z* 586 (M+H)⁺.

### Example 624: N,N'-(2-propylbicyclo[2.2.2]octane-1,4-diyl)bis[2-(4-chloro-3-fluorophenoxy)acetamide] (Compound 723)

### Example 624A: N,N'-(2-(3-hydroxyprop-1-yn-1-yl)bicyclo[2.2.2]oct-2-ene-1,4-diyl)bis(2-(4-chloro-3-fluorophenoxy)acetamide)

A suspension of Example 619A (.0363 g, 0.055 mmol), bis(triphenylphosphine)palladium(II) chloride (PdCl₂(PPh₃)₂, 2.3 mg, 3.28 µmol), copper(I) iodide (2.5 mg, 0.013 mmol), prop-2-yn-1-ol (5 µl, 0.085 mmol) and triethylamine (0.080 mL, 0.574 mmol) in tetrahydrofuran (0.080 mL) was stirred at 60 °C for 40 minutes. The reaction mixture was then concentrated, and the residue was chromatographed on silica gel (30% ethyl acetate/dichloromethane) to give the title compound (0.0191 g, 0.034 mmol, 61.4 % yield). ¹H NMR (400 MHz, DMSO-*d*₆) *δ* ppm 8.15 (s, 1H), 7.79 (s, 1H), 7.49 (t, J = 8.9 Hz, 2H), 7.09 (ddd, J = 18.5, 11.4, 2.8 Hz, 2H), 6.87 (dddd, J = 17.9, 8.9, 2.8, 1.1 Hz, 2H), 6.57 (s, 1H), 5.26 (t, J = 5.8 Hz, 1H), 4.55 (d, J = 7.4 Hz, 4H), 4.19 (d, J = 5.8 Hz, 2H), 2.06 - 1.97 (m, 2H), 1.87 - 1.64 (m, 6H); LC/MS (APCI⁺) *m*/*z* 565 (M+H)⁺.

### Example 624B: N,N'-(2-propylbicyclo[2.2.2]octane-1,4-diyl)bis[2-(4-chloro-3-fluorophenoxy)acetamide]

A mixture of Example 624A in methanol (1.0 mL) was added to 5% Pd/C (wet JM#9) (10.0 mg, 0.042 mmol) in a 20 mL RS10 Hast C reactor, and a 4 M solution of HCl in dioxane (34 µL, 0.136 mmol) was added. The reactor was purged with argon, and the mixture was stirred at 1200 RPM under 45 psi of hydrogen at 25 °C overnight. The reaction mixture was filtered and concentrated. The residue was chromatographed on silica gel (15-30% ethyl acetate/dichloromethane) to give the title compound (0.0040 g, 7.20 µmol, 21.43 % yield). ¹H NMR (501 MHz, CDCl₃) *δ* ppm 7.32 (td, J = 8.6, 1.6 Hz, 2H), 6.74 (dd, J = 10.3, 2.8 Hz, 2H), 6.65 (dtd, J = 8.9, 2.7, 1.2 Hz, 2H), 6.09 (s, 1H), 5.99 (s, 1H), 4.35 (d, J = 2.1 Hz, 2H), 4.32 (s, 2H), 2.51 - 2.40 (m, 2H), 2.19 - 2.10 (m, 1H), 2.10 - 1.97 (m, 4H), 1.95 - 1.86 (m, 2H), 1.82 (td, J = 11.2, 4.5 Hz, 1H), 1.68 (ddd, J = 12.7, 5.9, 2.8 Hz, 1H), 1.50 - 1.40 (m, 1H), 1.33 - 1.08 (m, 3H), 0.89 (t, J = 7.1 Hz, 3H); MS (ESI⁺) *m*/*z* 555.3 (M+H)⁺.

### Example 625: 2-[(4-fluoro-1H-indazol-6-yl)oxy]-N-[(3S)-4-{2-[3-fluoro-4-(trifluoromethoxy)phenoxy]acetamido}-3-hydroxybicyclo[2.2.2]octan-1-yl]acetamide (Compound 724)

The title compound was prepared using the methodologies described above. ¹H NMR (400 MHz, DMSO-*d₆*) *δ* ppm 13.17 (s, 1H), 8.04 (s, 1H), 7.52 (s, 1H), 7.51 - 7.45 (m, 1H), 7.29 (s, 1H), 7.11 (dd, J = 12.4, 3.0 Hz, 1H), 6.86 (ddd, J = 9.2, 3.0, 1.5 Hz, 1H), 6.74 - 6.71 (m, 1H), 6.64 (dd, J = 11.6, 1.8 Hz, 1H), 5.09 (d, J = 4.2 Hz, 1H), 4.49 (s, 2H), 4.47 (s, 2H), 4.11 - 4.02 (m, 1H), 2.36 - 2.26 (m, 1H), 2.10 - 1.73 (m, 9H); MS (ESI⁺) *m*/*z* 585 (M+H)⁺.

### Example 626: 2-(4-chlorophenoxy)-N-[(2S)-4-{2-[(4-fluoro-1H-indazol-6-yl)oxy]acetamido}-2-hydroxybicyclo[2.2.2]octan-1-yl]acetamide (Compound 725)

The title compound was prepared using the methodologies described above. ¹H NMR (400 MHz, DMSO-*d₆*) *δ* ppm 13.17 (s, 1H), 8.04 (s, 1H), 7.52 (s, 1H), 7.37 - 7.30 (m, 2H), 7.24 (s, 1H), 6.99 - 6.90 (m, 2H), 6.74 - 6.72 (m, 1H), 6.64 (dd, J = 11.7, 1.8 Hz, 1H), 5.13 (d, J = 4.3 Hz, 1H), 4.47 (s, 2H), 4.42 (s, 2H), 4.06 - 3.98 (m, 1H), 2.34 - 2.24 (m, 1H), 2.18 - 2.08 (m, 1H), 2.03 - 1.74 (m, 8H); MS (APCI⁺) *m*/*z* 517 (M+H)⁺.

### Example 628: 2-(4-chloro-3-fluorophenoxy)-N-[(2S)-4-{2-[(4-fluoro-1H-indazol-6-yl)oxy]acetamido}-2-hydroxybicyclo[2.2.2]octan-1-yl]acetamide (Compound 727)

The title compound was prepared using the methodologies described above. ¹H NMR (400 MHz, DMSO-*d₆*) *δ* ppm 13.17 (s, 1H), 8.04 (s, 1H), 7.52 (s, 1H), 7.48 (t, J = 8.9 Hz, 1H), 7.26 (s, 1H), 7.05 (dd, J = 11.4, 2.8 Hz, 1H), 6.83 (ddd, J = 9.0, 2.9, 1.2 Hz, 1H), 6.74 - 6.71 (m, 1H), 6.64 (dd, J = 11.6, 1.8 Hz, 1H), 5.08 (d, J = 4.3 Hz, 1H), 4.47 (br s, 4H), 4.08 - 4.01 (m, 1H), 2.32 - 2.23 (m, 1H), 2.12 - 2.02 (m, 1H), 2.02 - 1.75 (m, 8H); MS (APCI⁺) *m*/*z* 535 (M+H)⁺.

### Example 629: 2-(3,4-difluorophenoxy)-N-[(2S)-4-{2-[(4-fluoro-1H-indazol-6-yl)oxy]acetamido}-2-hydroxybicyclo[2.2.2]octan-1-yl]acetamide (Compound 728)

The title compound was prepared using the methodologies described above. ¹H NMR (501 MHz, DMSO-*d₆*) *δ* ppm 13.17 (s, 1H), 8.04 (s, 1H), 7.52 (s, 1H), 7.36 (dt, J = 10.6, 9.3 Hz, 1H), 7.23 (s, 1H), 7.07 (ddd, J = 12.6, 6.7, 3.1 Hz, 1H), 6.77 (dtd, J = 9.1, 3.3, 1.7 Hz, 1H), 6.73 - 6.71 (m, 1H), 6.64 (dd, J = 11.7, 1.8 Hz, 1H), 5.10 (d, J = 4.4 Hz, 1H), 4.47 (s, 2H), 4.43 (s, 2H), 4.06 - 4.01 (m, 1H), 2.32 - 2.23 (m, 1H), 2.15 - 2.05 (m, 1H), 2.01 - 1.75 (m, 8H); MS (ESI⁺) *m*/*z* 519 (M+H)⁺.

### Example 630: 3-(4-chloro-3-fluorophenoxy)-N-{4-[2-(4-chlorophenoxy)acetamido]bicyclo[2.2.2]octan-1-yl}propanamide (Compound 729)

The title compound was prepared using the methodologies described above. ¹H NMR (501 MHz, DMSO-*d₆*) *δ* ppm 7.48 (s, 1H), 7.45 (t, J = 8.9 Hz, 1H), 7.41 (s, 1H), 7.36 - 7.31 (m, 2H), 7.04 (dd, J = 11.5, 2.8 Hz, 1H), 6.95 - 6.90 (m, 2H), 6.79 (ddd, J = 8.9, 2.8, 1.2 Hz, 1H), 4.38 (s, 2H), 4.14 (t, J = 6.1 Hz, 2H), 2.47 (t, J = 6.1 Hz, 2H), 1.92 - 1.80 (m, 12H); MS (APCI⁺) *m*/*z* 509 (M+H)⁺.

### Example 631: 3-(4-chloro-3-fluorophenoxy)-N-{3-[2-(4-chloro-3-fluorophenoxy)acetamido]bicyclo[1.1.1]pentan-1-yl}propanamide (Compound 730)

The title compound was prepared using the methodologies described above. ¹H NMR (500 MHz, DMSO-*d₆*) *δ* ppm 8.71 (s, 1H), 8.58 (s, 1H), 7.49 (t, J = 8.9 Hz, 1H), 7.45 (t, J = 8.9 Hz, 1H), 7.09 - 7.03 (m, 2H), 6.85 (ddd, J = 8.9, 2.9, 1.2 Hz, 1H), 6.81 (ddd, J = 9.0, 2.9, 1.2 Hz, 1H), 4.47 (s, 2H), 4.17 (t, J = 6.1 Hz, 2H), 2.53 - 2.50 (m, 2H), 2.22 (s, 6H); MS (APCI⁺) *m*/*z* 485 (M+H)⁺.

### Example 632: 2-(3-fluoro-4-methylphenoxy)-N-{(3S)-4-[2-(4-fluorophenoxy)acetamido]-3-hydroxybicyclo[2.2.2]octan-1-yl}acetamide (Compound 731)

The title compound was prepared using the methodologies described above. ¹H NMR (501 MHz, DMSO-*d₆*) *δ* ppm 7.43 (s, 1H), 7.20 (s, 1H), 7.16 (t, J = 8.9 Hz, 1H), 7.15 - 7.10 (m, 2H), 6.98 - 6.92 (m, 2H), 6.73 (dd, J = 11.8, 2.6 Hz, 1H), 6.67 (dd, J = 8.4, 2.6 Hz, 1H), 5.13 (d, J = 4.3 Hz, 1H), 4.40 - 4.38 (m, 2H), 4.36 (s, 2H), 4.02 - 3.96 (m, 1H), 2.28 (ddd, J = 12.7, 9.5, 2.8 Hz, 1H), 2.19 - 2.09 (m, 4H), 1.99 - 1.74 (m, 8H); MS (ESI⁺) *m*/*z* 475 (M+H)⁺.

### Example 633: 2-(4-chlorophenoxy)-N-{(2S)-4-[2-(3-fluoro-4-methylphenoxy)acetamido]-2-hydroxybicyclo[2.2.2]octan-1-yl}acetamide (Compound 732)

The title compound was prepared using the methodologies described above. ¹H NMR (501 MHz, DMSO-*d₆*) *δ* ppm 7.43 (s, 1H), 7.36 - 7.31 (m, 2H), 7.23 (s, 1H), 7.16 (t, J = 8.7 Hz, 1H), 6.97 - 6.93 (m, 2H), 6.73 (dd, J = 11.8, 2.6 Hz, 1H), 6.67 (dd, J = 8.4, 2.6 Hz, 1H), 5.11 (d, J = 4.2 Hz, 1H), 4.47 - 4.39 (m, 2H), 4.36 (s, 2H), 4.04 - 3.98 (m, 1H), 2.27 (ddd, J = 12.4, 9.4, 2.6 Hz, 1H), 2.14 (d, J = 1.8 Hz, 3H), 2.13 - 2.06 (m, 1H), 1.98 - 1.74 (m, 8H); MS (APCI⁺) *m*/*z* 491 (M+H)⁺.

### Example 634: 2-(3-fluoro-4-methylphenoxy)-N-[(3S)-4-{2-[3-fluoro-4-(trifluoromethoxy)phenoxy]acetamido}-3-hydroxybicyclo[2.2.2]octan-1-yl]acetamide (Compound 733)

The title compound was prepared using the methodologies described above. ¹H NMR (400 MHz, DMSO-*d₆*) *δ* ppm 7.49 (td, J = 9.1, 1.2 Hz, 1H), 7.43 (s, 1H), 7.28 (s, 1H), 7.23 - 7.14 (m, 1H), 7.12 (dd, J = 12.4, 3.0 Hz, 1H), 6.86 (ddd, J = 9.2, 3.0, 1.5 Hz, 1H), 6.73 (dd, J = 11.9, 2.5 Hz, 1H), 6.67 (dd, J = 8.4, 2.6 Hz, 1H), 5.07 (d, J = 4.3 Hz, 1H), 4.49 (s, 2H), 4.36 (s, 2H), 4.09 - 4.00 (m, 1H), 2.27 (ddd, J = 12.2, 9.6, 2.2 Hz, 1H), 2.14 (d, J = 1.8 Hz, 3H), 2.10 - 2.01 (m, 1H), 1.97 - 1.71 (m, 8H); MS (ESI⁺) *m*/*z* 559 (M+H)⁺.

### Example 635: 2-(3,4-difluorophenoxy)-N-{(2S)-4-[2-(3-fluoro-4-methylphenoxy)acetamido]-2-hydroxybicyclo[2.2.2]octan-1-yl}acetamide (Compound 734)

The title compound was prepared using the methodologies described above. ¹H NMR (400 MHz, DMSO-*d₆*) *δ* ppm 7.43 (s, 1H), 7.35 (dt, J = 10.6, 9.3 Hz, 1H), 7.23 (s, 1H), 7.20 - 7.13 (m, 1H), 7.07 (ddd, J = 12.6, 6.7, 3.0 Hz, 1H), 6.80 - 6.75 (m, 1H), 6.73 (dd, J = 11.9, 2.6 Hz, 1H), 6.67 (dd, J = 8.4, 2.6 Hz, 1H), 5.10 (d. J = 4.1 Hz, 1H), 4.43 (s, 2H), 4.36 (s, 2H), 4.08 - 3.99 (m, 1H), 2.27 (ddd, J = 12.4, 9.4, 2.4 Hz, 1H), 2.14 (d, J = 1.8 Hz, 3H), 2.12 - 2.05 (m, 1H), 1.98 - 1.71 (m, 8H); MS (ESI⁺) *m*/*z* 493 (M+H)⁺.

### Example 636: 2-[(4-fluoro-1H-indazol-6-yl)oxy]-N-{(2S)-4-[2-(3-fluoro-4-methylphenoxy)acetamido]-2-hydroxybicyclo[2.2.2]octan-1-yl}acetamide (Compound 735)

The title compound was prepared using the methodologies described above. ¹H NMR (400 MHz, DMSO-*d₆*) *δ* ppm 13.17 (s, 1H), 8.05 (s, 1H), 7.43 (s, 1H), 7.27 (s, 1H), 7.16 (t, J = 8.8 Hz, 1H), 6.78 - 6.76 (m, 1H), 6.73 (dd, J = 11.8, 2.6 Hz, 1H), 6.69 - 6.62 (m, 2H), 5.13 (d, J = 4.2 Hz, 1H), 4.50 (s, 2H), 4.36 (s, 2H), 4.07 - 4.00 (m, 1H), 2.34 - 2.24 (m, 1H), 2.18 - 2.08 (m, 4H), 1.99 - 1.73 (m, 8H); MS (ESI⁺) *m*/*z* 515 (M+H)⁺.

### Example 637: 2-(3-fluoro-4-methylphenoxy)-N-[(3S)-3-hydroxy-4-{2-[4-(trifluoromethoxy)phenoxy]acetamido}bicyclo[2.2.2]octan-1-yl]acetamide (Compound 736)

The title compound was prepared using the methodologies described above. ¹H NMR (400 MHz, DMSO-*d₆*) *δ* ppm 7.43 (s, 1H), 7.33 - 7.24 (m, 3H), 7.16 (t, J = 8.8 Hz, 1H), 7.05 - 6.99 (m, 2H), 6.73 (dd, J = 11.8, 2.6 Hz, 1H), 6.67 (dd, J = 8.4, 2.6 Hz, 1H), 5.11 (d, J = 4.1 Hz, 1H), 4.45 (s, 2H), 4.36 (s, 2H), 4.05 - 3.97 (m, 1H), 2.32 - 2.22 (m, 1H), 2.16 - 2.13 (m, 3H), 2.13 - 2.06 (m, 1H), 1.98 - 1.73 (m, 8H); MS (ESI⁺) *m*/*z* 541 (M+H)⁺.

### Example 638: 2-(4-chloro-3-fluorophenoxy)-N-{(2S)-4-[2-(3-fluoro-4-methylphenoxy)acetamido]-2-hydroxybicyclo[2.2.2]octan-1-yl}acetamide (Compound 737)

The title compound was prepared using the methodologies described above. ¹H NMR (400 MHz, DMSO-*d₆*) *δ* ppm 7.48 (t, J = 8.9 Hz, 1H), 7.43 (s, 1H), 7.26 (s, 1H), 7.19 - 7.14 (m, 1H), 7.05 (dd, J = 11.4, 2.9 Hz, 1H), 6.83 (ddd, J = 9.0, 2.9, 1.2 Hz, 1H), 6.73 (dd, J = 11.9, 2.6 Hz, 1H), 6.67 (dd, J = 8.4, 2.6 Hz, 1H), 5.10 - 5.05 (m, 1H), 4.46 (s, 2H), 4.36 (s, 2H), 4.07 - 4.01 (m, 1H), 2.27 (ddd, J = 12.4, 9.4, 2.4 Hz, 1H), 2.14 (d, J = 1.8 Hz, 3H), 2.10 - 2.03 (m, 1H), 1.96 - 1.70 (m, 8H); MS (ESI⁺) *m*/*z* 509 (M+H)⁺.

### Example 639: 2-(4-chloro-3-fluorophenoxy)-N-[(3S)-4-{2-[(4-fluoro-1H-indazol-6-yl)oxy]acetamido}-3-hydroxybicyclo[2.2.2]octan-1-yl]acetamide (Compound 738)

The title compound was prepared using the methodologies described above. ¹H NMR (400 MHz, DMSO-*d₆*) *δ* ppm 13.18 (s, 1H), 8.05 (s, 1H), 7.51 (s, 1H), 7.48 (t, J = 8.9 Hz, 1H), 7.28 (s, 1H), 7.02 (dd, J = 11.4, 2.9 Hz, 1H), 6.81 (ddd, J = 9.0, 2.9, 1.2 Hz, 1H), 6.78 - 6.76 (m, 1H), 6.65 (dd, J = 11.6, 1.7 Hz, 1H), 5.13 (d, J = 4.2 Hz, 1H), 4.50 (s, 2H), 4.43 (s, 2H), 4.07 - 3.99 (m, 1H), 2.33 - 2.21 (m, 1H), 2.17 - 2.08 (m, 1H), 1.99 - 1.69 (m, 8H); MS (ESI⁺) *m*/*z* 535 (M+H)⁺.

### Example 640: 2-(4-chlorophenoxy)-N-(4-{2-[(4-fluoro-1H-indazol-6-yl)oxy]acetamido}bicyclo[2.2.2]octan-1-yl)acetamide (Compound 739)

The title compound was prepared using the methodologies described above. ¹H NMR (400 MHz, DMSO-*d₆*) *δ* ppm 13.16 (s, 1H), 8.04 (s, 1H), 7.48 (s, 1H), 7.43 (s, 1H), 7.35 - 7.29 (m, 2H), 6.96 - 6.89 (m, 2H), 6.75 - 6.71 (m, 1H), 6.64 (dd, J = 11.6, 1.8 Hz, 1H), 4.46 (s, 2H), 4.38 (s, 2H), 1.91 (s, 12H); MS (ESI⁺) *m*/*z* 501 (M+H)⁺.

### Example 641: 2-(4-chlorophenoxy)-N-{4-[2-(3,4-difluorophenoxy)acetamido]bicyclo[2.2.2]octan-1-yl}acetamide (Compound 740)

The title compound was prepared using the methodologies described above. ¹H NMR (501 MHz, DMSO-*d₆*) *δ* ppm 7.41 (s, 1H), 7.40 (s, 1H), 7.36 - 7.29 (m, 3H), 7.02 (ddd, J = 12.6, 6.7, 3.0 Hz, 1H), 6.93 - 6.88 (m, 2H), 6.76 - 6.70 (m, 1H), 4.37 (s, 2H), 4.36 (s, 2H), 1.88 (s, 12H); MS (ESI⁺) *m*/*z* 479 (M+H)⁺.

### Example 642: 2-(4-chlorophenoxy)-N-(4-{2-[3-fluoro-4-(trifluoromethoxy)phenoxy]acetamido}bicyclo[2.2.2]octan-1-yl)acetamide (Compound 741)

The title compound was prepared using the methodologies described above. ¹H NMR (501 MHz, DMSO-*d₆*) *δ* ppm 7.50 - 7.44 (m, 2H), 7.40 (s, 1H), 7.33 - 7.28 (m, 2H), 7.06 (dd, J = 12.4, 2.9 Hz, 1H), 6.93 - 6.88 (m, 2H), 6.82 (ddd, J = 9.2, 2.9, 1.4 Hz, 1H), 4.44 (s, 2H), 4.36 (s, 2H), 1.88 (s, 12H); MS (ESI⁺) *m*/*z* 545 (M+H)⁺.

### Example 643: 2-(4-chlorophenoxy)-N-(4-{2-[(2,2-difluoro-2H-1,3-benzodioxol-5-yl)oxy]acetamido}bicyclo[2.2.2]octan-1-yl)acetamide (Compound 742)

The title compound was prepared using the methodologies described above. ¹H NMR (501 MHz, DMSO-*d₆*) *δ* ppm 7.71 (s, 1H), 7.70 (s, 1H), 7.63 - 7.58 (m, 3H), 7.38 (d, J = 2.6 Hz, 1H), 7.23 - 7.19 (m, 2H), 7.00 (dd, J = 8.9, 2.6 Hz, 1H), 4.68 (s, 2H), 4.67 (s, 2H), 2.19 (br s, 12H); MS (ESI⁺) *m*/*z* 523 (M+H)⁺.

### Example 644: 2-(3-chloro-4-fluorophenoxy)-N-{4-[2-(4-chlorophenoxy)acetamido]bicyclo[2.2.2]octan-1-yl}acetamide (Compound 743)

The title compound was prepared using the methodologies described above. ¹H NMR (400 MHz, DMSO-*d₆*) *δ* ppm 7.44 (s, 1H), 7.42 (s, 1H), 7.37 - 7.30 (m, 3H), 7.15 (dd, J = 6.1, 3.1 Hz, 1H), 6.96 - 6.89 (m, 3H), 4.40 (s, 2H), 4.38 (s, 2H), 1.90 (s, 12H); MS (ESI⁺) *m*/*z* 495 (M+H)⁺.

### Example 645: N,N'-(bicyclo[2.2.2]octane-1,4-diyl)bis[2-(4-chlorophenoxy)acetamide] (Compound 744)

The title compound was prepared using the methodologies described above. ¹H NMR (400 MHz, DMSO-*d₆*) *δ* ppm 7.42 (s, 2H), 7.35 - 7.30 (m, 4H), 6.96 - 6.89 (m, 4H), 4.38 (s, 4H), 1.90 (s, 12H); MS (ESI⁺) *m*/*z* 477 (M+H)⁺.

### Example 646: rac-N,N'-[(2R,3R)-2,3-dihydroxybicyclo[2.2.2]octane-1,4-diyl]bis[2-(4-chloro-3-fluorophenoxy)acetamide] (Compound 745)

### Example 646A: N,N'-(2-((triethylsilyl)oxy)bicyclo[2.2.2]oct-2-ene-1,4-diyl)bis(2-(4-chloro-3-fluorophenoxy)acetamide)

To a suspension of Example 599A (0.1021 g, 0.194 mmol) and triethylamine (0.13 mL, 0.933 mmol) in CH₂Cl₂ (1.0 mL) at -78 °C was added dropwise triethylsilyl trifluoromethanesulfonate (0.13 mL, 0.575 mmol), and the mixture was stirred for 30 minutes. The mixture was then allowed to warm and stirred for an additional 30 minutes. The mixture was then diluted with ethyl acetate, washed with saturated NaHCO₃ and brine, dried (Na₂SO₄), concentrated. The residue was chromatographed on silica gel (10% ethyl acetate/dichloromethane, fast) to give impure title compound, which was used without further purification (0.1240 g, 0.193 mmol, 100 % yield). LC/MS (APCI⁺) *m*/*z* 641 (M+H)⁺.

### Example 646B: rac- N,N'-(2-hydroxy-3-oxobicyclo[2.2.2]octane-1,4-diyl)bis(2-(4-chloro-3-fluorophenoxy)acetamide)

To a suspension of Example 646A (0.1240 g, 0.193 mmol) and NaHCO₃ (0.0404 g, 0.481 mmol) in CH₂Cl₂ (0.80 mL) at 0 °C was added 3-chloroperoxybenzoic acid (0.0676 g, 0.392 mmol), and the mixture was stirred for 1 hour. The mixture was then diluted with ethyl acetate, quenched with saturated aqueous Na₂SO₃ (0.5 mL), washed with saturated NaHCO₃ and brine, dried (Na₂SO₄), and concentrated to give a mixture of products.

This material was stirred in methanol (2 mL) with NH₄F (0.0219 g, 0.591 mmol), briefly heated to reflux, stirred for 2 hours, and heated to 60 °C for 1 hour. Another aliquot of NH₄F (0.0219 g, 0.591 mmol) was added, and the mixture was stirred for 1 hour. It was then concentrated, diluted with ethyl acetate, washed with saturated NaHCO₃ and brine, dried (Na₂SO₄), and concentrated. The residue was chromatographed on silica gel (33% ethyl acetate/dichloromethane) to give impure title compound (0.0431 g, 0.079 mmol, 41.0 % yield). ¹H NMR (501 MHz, DMSO-*d*₆) *δ* ppm 7.81 (s, 1H), 7.57 (s, 1H), 7.48 (td, J = 8.8, 3.8 Hz, 2H), 7.10 - 7.02 (m, 2H), 6.84 (dddd, J = 8.7, 7.2, 2.9, 1.1 Hz, 2H), 6.04 (d, J = 5.8 Hz, 1H), 4.56 (s, 2H), 4.54 - 4.47 (m, 2H), 4.46 - 4.43 (m, 1H), 2.38 - 2.27 (m, 2H), 2.18 - 2.06 (m, 3H), 1.99 - 1.81 (m, 2H), 1.75 - 1.65 (m, 1H); LC/MS (APCI⁺) *m*/*z* 543 (M+H)⁺.

### Example 646C: rac-N,N'-[(2R,3R)-2,3-dihydroxybicyclo[2.2.2]octane-1,4-diyl]bis[2-(4-chloro-3-fluorophenoxy)acetamide]

A solution of Bu₄NBH₄ (0.0212 g, 0.082 mmol) in acetic acid (0.22 mL) was stirred for ~3 minutes, cooled to 0 °C, and a solution of Example 646B (0.0360 g, 0.066 mmol) in acetic acid (0.22 mL) and acetonitrile (0.22 mL) was added dropwise. The mixture was stirred for 2 hours, allowed to warm for 15 minutes, quenched with saturated Rochelle's salt, carefully neutralized with saturated NaHCO₃, and extracted with dichloromethane (3 × 10 mL). The combined organic fractions were dried (Na₂SO₄) and concentrated. The residue was chromatographed on silica gel (25-50% acetonitrile/dichloromethane, flushed with 15% methanol/dichloromethane) to give the title compound (0.0186 g, 0.034 mmol, 51.5 % yield). ¹H NMR (400 MHz, DMSO-*d*₆) *δ* ppm 7.48 (t, J = 8.9 Hz, 2H), 7.28 (s, 2H), 7.05 (dd, J = 11.4, 2.9 Hz, 2H), 6.83 (ddd, J = 8.9, 2.9, 1.2 Hz, 2H), 5.24 (d, J = 4.6 Hz, 2H), 4.46 (s, 4H), 3.79 (d, J = 4.3 Hz, 2H), 2.02 - 1.86 (m, 4H), 1.85 - 1.67 (m, 4H); MS (ESI⁺) *m*/*z* 545.1 (M+H)⁺.

### Example 647: N-{(2S)-2-hydroxy-4-[2-(4-methylphenoxy)acetamido]bicyclo[2.2.2]octan-1-yl}-2-[(3-methyl-1H-indazol-6-yl)oxy]acetamide (Compound 746)

The title compound was prepared using the methodologies described above. ¹H NMR (400 MHz, DMSO-*d₆*) *δ* ppm 12.40 (s, 1H), 7.57 (d, J = 8.8 Hz, 1H), 7.36 (s, 1H), 7.24 (s, 1H), 7.08 (d, J = 8.2 Hz, 2H), 6.83 - 6.77 (m, 3H), 6.75 (dd, J = 8.7, 2.1 Hz, 1H), 5.21 - 5.09 (m, 1H), 4.45 (s, 2H), 4.32 (s, 2H), 4.03 - 3.96 (m, 1H), 2.42 (s, 3H), 2.35 - 2.14 (m, 2H), 2.22 (s, 3H), 1.99 - 1.73 (m, 8H); MS (ESI⁺) *m*/*z* 493 (M+H)⁺.

### Example 648: 2-[3-fluoro-4-(trifluoromethoxy)phenoxy]-N-[(3S)-3-hydroxy-4-{2-[(3-methyl-1H-indazol-6-yl)oxy]acetamido}bicyclo[2.2.2]octan-1-yl]acetamide (Compound 747)

The title compound was prepared using the methodologies described above. ¹H NMR (400 MHz, DMSO-*d₆*) *δ* ppm 12.40 (s, 1H), 7.57 (d, J = 8.7 Hz, 1H), 7.53 (s, 1H), 7.51 - 7.45 (m, 1H), 7.25 (s, 1H), 7.08 (dd, J = 12.4, 2.9 Hz, 1H), 6.84 (ddd, J = 9.2, 3.1, 1.5 Hz, 1H), 6.81 (d, J = 2.1 Hz, 1H), 6.75 (dd, J = 8.7, 2.1 Hz, 1H), 5.19 - 5.12 (m, 1H), 4.46 (br s, J = 2.6 Hz, 4H), 4.03 - 3.96 (m, 1H), 2.42 (s, 3H), 2.35 - 2.13 (m, 2H), 2.00 - 1.68 (m, 8H); MS (ESI⁺) *m*/*z* 581 (M+H)⁺.

### Example 649: 2-(4-chloro-3-fluoroanilino)-N-{(2S)-4-[2-(4-chloro-3-fluorophenoxy)acetamido]-2-hydroxybicyclo[2.2.2]octan-1-yl}acetamide (Compound 748)

The title compound was prepared using the methodologies described above. ¹H NMR (400 MHz, DMSO-*d₆*) *δ* ppm 7.49 (s, 1H), 7.47 (t, J = 8.9 Hz, 1H), 7.25 (s, 1H), 7.20 (t, J = 8.8 Hz, 1H), 7.02 (dd, J = 11.4, 2.9 Hz, 1H), 6.80 (ddd, J = 9.0, 2.9, 1.2 Hz, 1H), 6.48 (dd, J = 12.5, 2.6 Hz, 1H), 6.42 - 6.35 (m, 2H), 5.05 (d, J = 4.1 Hz, 1H), 4.42 (s, 2H), 4.02 - 3.92 (m, 1H), 3.59 (d, J = 5.8 Hz, 2H), 2.30 - 2.19 (m, 1H), 2.07 - 1.97 (m, 1H), 1.95 - 1.67 (m, 8H); MS (ESI⁺) *m*/*z* 528 (M+H)⁺.

### Example 650: 2-(4-chloro-3-fluorophenoxy)-N-[(3S)-3-hydroxy-4-{2-[(3-methyl-1H-indazol-6-yl)oxy]acetamido}bicyclo[2.2.2]octan-1-yl]acetamide (Compound 749)

The title compound was prepared using the methodologies described above. ¹H NMR (400 MHz, DMSO-*d₆*) *δ* ppm 12.40 (s, 1H), 7.57 (d, J = 8.8 Hz, 1H), 7.53 - 7.40 (m, 2H), 7.25 (s, 1H), 7.06 - 6.99 (m, 1H), 6.83 - 6.78 (m, 2H), 6.75 (dd, J = 8.7, 2.1 Hz, 1H), 5.17 (s, 1H), 4.50 - 4.39 (m, 4H), 4.03 - 3.97 (m, 1H), 2.42 (s, 3H), 2.36 - 2.10 (m, 2H), 2.01 - 1.64 (m, 8H); MS (ESI⁺) *m*/*z* 531 (M+H)⁺.

### Example 651: 2-(4-chloro-3-fluorophenoxy)-N-[(3S)-4-{2-[3-fluoro-4-(trifluoromethoxy)phenoxy]acetamido}-3-hydroxybicyclo[2.2.2]octan-1-yl]acetamide (Compound 750)

The title compound was prepared using the methodologies described above. ¹H NMR (400 MHz, DMSO-*d₆*) *δ* ppm 7.54 - 7.44 (m, 3H), 7.29 (s, 1H), 7.11 (dd, J = 12.4, 3.0 Hz, 1H), 7.02 (dd, J = 11.4, 2.9 Hz, 1H), 6.86 (ddd, J = 9.2, 3.0, 1.5 Hz, 1H), 6.81 (ddd, J = 9.0, 2.9, 1.2 Hz, 1H), 5.08 (s, 1H), 4.49 (s, 2H), 4.43 (s, 2H), 4.10 - 4.00 (m, 1H), 2.32 - 2.22 (m, 1H), 2.10 - 2.00 (m, 1H), 1.98 - 1.71 (m, 8H); MS (ESI⁺) *m*/*z* 579 (M+H)⁺.

### Example 652: 2-(4-chloro-3-fluorophenoxy)-N-[(2S)-4-{2-[4-fluoro-2-(methanesulfonyl)phenoxy]acetamido}-2-hydroxybicyclo[2.2.2]octan-1-yl]acetamide (Compound 751)

### Example 652A: tert-butyl 2-(4-fluoro-2-(methylsulfonyl)phenoxy)acetate

The methodologies described in Example 596A substituting 4-fluoro-2-(methylsulfonyl)phenol for 6-hydroxy-2-methylisoindolin-1-one gave the title compound. MS (ESI⁺) *m*/*z* 249 (M-*t*Bu+H)⁺.

### Example 652B: 2-(4-fluoro-2-(methylsulfonyl)phenoxy)acetic acid

To a solution of Example 652A (0.320 g, 1.05 mmol) in methanol (0.53 mL) and water (0.53 mL) was added NaOH (1.05 mL, 5.26 mmol, 5 M aqueous). The reaction mixture stirred at ambient temperature overnight, was concentrated, acidified with 1 N HCl, and concentrated again to give the title compound (0.26 g, 1.05 mmol, quantitative yield), which was carried forward without purification. ¹H NMR (400 MHz, DMSO-*d*₆) *δ* ppm 8.49 (s, 1H), 7.48 (dq, J = 8.9, 4.9, 4.1 Hz, 2H), 7.11 (dd, J = 8.9, 4.0 Hz, 1H), 4.37 (s, 2H), 2.54 (s, 3H).

### Example 652C: (S)-tert-butyl (4-(2-(4-fluoro-2-(methylsulfonyl)phenoxy)acetamido)-2-hydroxybicyclo[2.2.2]octan-1-yl)carbamate

The methodologies described in Example 569 substituting Example 445B for Example 9B and substituting Example 652B for 1-(4-chlorophenyl)cyclopropanecarboxylic acid gave the title compound. ¹H NMR (400 MHz, DMSO-*d*₆) *δ* ppm 7.67 - 7.51 (m, 2H), 7.39 (s, 1H), 7.26 (dd, J = 9.0, 4.0 Hz, 1H), 6.03 (s, 1H), 4.91 (d, J = 4.2 Hz, 1H), 4.64 (s, 2H), 3.88 (d, J = 9.5 Hz, 1H), 3.35 (s, 3H), 2.25 (t, J = 11.5 Hz, 1H), 2.11 - 1.95 (m, 1H), 1.95 - 1.59 (m, 8H), 1.36 (s, 9H); MS (ESI⁺) *m*/*z* 487 (M+H)⁺.

### Example 652D: (S)-N-(4-amino-3-hydroxybicyclo[2.2.2]octan-1-yl)-2-(4-fluoro-2-(methylsulfonyl)phenoxy)acetamide

The methodologies described in Example 596D substituting Example 652C for Example 596C with shorter reaction time (1 hour) gave the title compound. MS (ESI⁺) *m*/*z* 387 (M+H)⁺.

### Example 652E: 2-(4-chloro-3-fluorophenoxy)-N-[(2S)-4-{2-[4-fluoro-2-(methanesulfonyl)phenoxy]acetamido}-2-hydroxybicyclo[2.2.2]octan-1-yl]acetamide

The methodologies described in Example 569 substituting 2-(4-chloro-3-fluorophenoxy)acetic acid for 1-(4-chlorophenyl)cyclopropanecarboxylic acid and substituting Example 652D for Example 9B gave the title compound. ¹H NMR (400 MHz, DMSO-*d*₆) *δ* ppm 7.6 - 7.49 (m, 2H), 7.44 (t, J = 8.9 Hz, 1H), 7.38 (s, 1H), 7.30 - 7.13 (m, 2H), 7.02 (dd, J = 11.4, 2.9 Hz, 1H), 6.79 (ddd, J = 8.9, 2.9, 1.1 Hz, 1H), 5.06 (d, J = 4.4 Hz, 1H), 4.61 (s, 2H), 4.43 (s, 2H), 4.1 - 3.92 (m, 1H), 3.32 (s, 3H), 2.24 (dd, J = 13.0, 9.8 Hz, 1H), 2.10 - 1.97 (m, 1H), 1.95 - 1.67 (m, 8H); MS (ESI⁺) *m*/*z* 573 (M+H)⁺.

### Example 653: 2-(4-chloro-3-fluorophenoxy)-N-[(2S)-4-{2-[(6-chloro-4-methyl-3-oxo-3,4-dihydro-2H-1,4-benzoxazin-8-yl)oxy]acetamido}-2-hydroxybicyclo[2.2.2]octan-1-yl]acetamide (Compound 752)

### ethyl 2-((6-chloro-3-oxo-3,4-dihydro-2H-benzo[b][1,4]oxazin-8-yl)oxy)acetate

Following the procedure reported by Combs et al. (J. Med. Chem. 1990, 33, 380), 6-chloro-3-oxo-3,4-dihydro-2*H*-benzo[*b*][1,4]oxazin-8-yl benzoate is formed by reacting 3-amino-5-chloro-2-hydroxyphenyl benzoate with chloroacetyl chloride. Following a benzoyl group deprotection, the phenol is reacted with ethyl 2-bromoacetate as described in Example 596A to form the title compound.

### Example 653A: ethyl 2-((6-chloro-4-methyl-3-oxo-3,4-dihydro-2H-benzo[b][1,4]oxazin-8-yl)oxy)acetate

To a solution of ethyl 2-((6-chloro-3-oxo-3,4-dihydro-2*H*-benzo[*b*][1,4]oxazin-8-yl)oxy)acetate (0.10 g, 0.35 mmol) in dichloromethane (2 mL) was added sodium hydride (0.017 g, 0.420 mmol, 60 weight% dispersion in mineral oil), and after 10 minutes, iodomethane (0.03 mL, 0.42 mmol) was added. This mixture was allowed to stir at ambient temperature overnight, was diluted with water (5 mL), and extracted with dichloromethane (3 × 10 mL). The combined organic fractions were dried (Na₂SO₄), filtered, and concentrated to give the title compound (0.10 g, 0.35 mmol, quantitative yield), which was carried forward without purification. ¹H NMR (400 MHz, CDCl₃) *δ* ppm 6.68 (d, J = 2.2 Hz, 1H), 6.62 (d, J = 2.2 Hz, 1H), 4.67 (d, J = 7.8 Hz, 4H), 4.28 (q, J = 7.2 Hz, 2H), 3.33 (s, 3H), 1.30 (t, J = 7.1 Hz, 3H). MS (ESI⁺) *m*/*z* 300 (M+H)⁺.

### Example 653B: 2-((6-chloro-4-methyl-3-oxo-3,4-dihydro-2H-benzo[b][1,4]oxazin-8-yl)oxy)acetic acid

The methodologies described in Example 652B substituting Example 653A for Example 652A gave the title compound. ¹H NMR (501 MHz, DMSO-*d*₆) *δ* ppm 6.16 (s, 1H), 6.10 (s, 1H), 4.12 - 4.02 (m, 2H), 3.97 (s, 2H), 3.46 (s, 3H).

### Example 653C: (S)-tert-butyl (4-(2-((6-chloro-4-methyl-3-oxo-3,4-dihydro-2H-benzo[b][1,4]oxazin-8-yl)oxy)acetamido)-2-hydroxybicyclo[2.2.2]octan-1-yl)carbamate

The methodologies described in Example 569 substituting Example 445B for Example 9B and substituting Example 653B for 1-(4-chlorophenyl)cyclopropanecarboxylic acid gave the title compound. ¹H NMR (400 MHz, DMSO-*d*₆) *δ* ppm 7.39 (s, 1H), 6.87 (d, J = 2.2 Hz, 1H), 6.74 (d, J = 2.3 Hz, 1H), 5.99 (s, 1H), 4.61 (s, 2H), 4.41 (s, 2H), 3.84 (d, J = 8.7 Hz, 1H), 3.22 (s, 3H), 2.86 (s, 3H), 2.21 (t, J = 11.7 Hz, 1H), 1.99 (s, 1H), 1.77 - 1.63 (m, 8H), 1.33 (d, J = 1.5 Hz, 9H); MS (ESI⁺) *m*/*z* 410 (M-C(O)OC(CH₃)₃+H)⁺.

### Example 653D: (S)-N-(4-amino-3-hydroxybicyclo[2.2.2]octan-1-yl)-2-((6-chloro-4-methyl-3-oxo-3,4-dihydro-2H-benzo[b][1,4]oxazin-8-yl)oxy)acetamide

The methodologies described in Example 596D substituting Example 653C for Example 596C with shorter reaction time (1 hour) gave the title compound. MS (ESI⁺) *m*/*z* 410 (M+H)⁺.

### Example 653E: 2-(4-chloro-3-fluorophenoxy)-N-[(2S)-4-{2-[(6-chloro-4-methyl-3-oxo-3,4-dihydro-2H-1,4-benzoxazin-8-yl)oxy]acetamido}-2-hydroxybicyclo[2.2.2]octan-1-yl]acetamide

The methodologies described in Example 569 substituting 2-(4-chloro-3-fluorophenoxy)acetic acid for 1-(4-chlorophenyl)cyclopropanecarboxylic acid and substituting Example 653D for Example 9B gave the title compound. ¹H NMR (501 MHz, DMSO-*d*₆, diagnostic peaks) *δ* ppm 7.5 - 7.42 (m, 2H), 7.26 (s, 1H), 7.05 (d, J = 8.8 Hz, 1H), 6.91 (s, 1H), 6.84 (m, 1H), 6.78 (s, 1H), 5.08 (m, 1H), 4.65 (s, 2H), 4.46 (s, 2H), 4.45 (s, 2H), 4.03 (m, 1H), 1.93 (m, 2H), 1.78 (m, 8H); MS (ESI⁺) *m*/*z* 596 (M+H)⁺.

### Example 655: 2-(4-chloro-3-fluorophenoxy)-N-[(2S)-4-{2-[(6-chloro-3-oxo-3,4-dihydro-2H-1,4-benzoxazin-8-yl)oxy]acetamido}-2-hydroxybicyclo[2.2.2]octan-1-yl]acetamide (Compound 754)

### Example 655A: 2-((6-chloro-3-oxo-3,4-dihydro-2H-benzo[b][1,4]oxazin-8-yl)oxy)acetic acid

The methodologies described in Example 652B substituting ethyl 2-((6-chloro-3-oxo-3,4-dihydro-2*H*-benzo[*b*][1,4]oxazin-8-yl)oxy)acetate for Example 652A gave the title compound. ¹H NMR (501 MHz, DMSO-*d*₆) *δ* ppm 10.86 (s, 1H), 6.67 (d, J = 2.3 Hz, 1H), 6.62 (d, J = 2.3 Hz, 1H), 4.73 (s, 2H), 4.57 (s, 2H).

### Example 655B: (S)-tert-butyl (4-(2-((6-chloro-3-oxo-3,4-dihydro-2H-benzo[b][1,4]oxazin-8-yl)oxy)acetamido)-2-hydroxybicyclo[2.2.2]octan-1-yl)carbamate

The methodologies described in Example 569 substituting Example 445B for Example 9B and substituting Example 655A for 1-(4-chlorophenyl)cyclopropanecarboxylic acid gave the title compound. MS (ESI⁺) *m*/*z* 496 (M+H)⁺.

### Example 655C: (S)-N-(4-amino-3-hydroxybicyclo[2.2.2]octan-1-yl)-2-((6-chloro-3-oxo-3,4-dihydro-2H-benzo[b][1,4]oxazin-8-yl)oxy)acetamide

The methodologies described in Example 596D substituting Example 655B for Example 596C with shorter reaction time (2.5 hours) gave the title compound. MS (ESI⁺) *m*/*z* 396 (M+H)⁺.

### Example 655D: 2-(4-chloro-3-fluorophenoxy)-N-[(2S)-4-{2-[(6-chloro-3-oxo-3,4-dihydro-2H-1,4-benzoxazin-8-yl)oxy]acetamido}-2-hydroxybicyclo[2.2.2]octan-1-yl]acetamide

The methodologies described in Example 569 substituting 2-(4-chloro-3-fluorophenoxy)acetic acid for 1-(4-chlorophenyl)cyclopropanecarboxylic acid and substituting Example 655C for Example 9B gave the title compound. ¹H NMR (400 MHz, DMSO-*d*₆) *δ* ppm 10.78 (s, 1H), 7.52 - 7.39 (m, 2H), 7.26 (s, 1H). 7.05 (dd, J = 11.4, 2.9 Hz, 1H), 6.83 (d, J = 7.2 Hz, 1H), 6.65 (d, J = 2.3 Hz, 1H), 6.56 (d, J = 2.3 Hz, 1H), 5.08 (d, J = 4.3 Hz, 1H), 4.58 (s, 2H), 4.46 (s, 2H), 4.43 (s, 2H), 4.03 (s, 1H), 2.25 (d, J = 11.7 Hz, 1H), 2.07 (s, 1H), 1.95 - 1.68 (m, 8H); MS (ESI⁺) *m*/*z* 582 (M+H)⁺.

### Example 656: 2-(4-chloro-3-fluorophenoxy)-N-{(2S)-4-[2-(4-fluoro-2-methylphenoxy)acetamido]-2-hydroxybicyclo[2.2.2]octan-1-yl}acetamide (Compound 755)

### Example 656A: tert-butyl 2-(4-fluoro-2-methylphenoxy)acetate

The methodologies described in Example 596A substituting 4-fluoro-2-methylphenol for 6-hydroxy-2-methylisoindolin-1-one gave the title compound. ¹H NMR (501 MHz, DMSO-*d*₆) *δ* ppm 7.02 (ddd, J = 9.2, 3.2, 0.9 Hz, 1H), 6.96 - 6.88 (m, 1H), 6.82 (dd, J = 9.0, 4.6 Hz, 1H), 4.65 (s, 2H), 2.20 (s, 3H), 1.42 (s, 9H); MS (ESI⁺) *m*/*z* 240 (M+H)⁺.

### Example 656B: 2-(4-fluoro-2-methylphenoxy)acetic acid

The methodologies described in Example 652B substituting Example 656A for Example 652A gave the title compound. ¹H NMR (400 MHz, DMSO-*d*₆) *δ* ppm 8.46 (s, 1H), 6.95 (dd, J = 9.3, 3.2 Hz, 1H), 6.86 (td, J = 8.7, 3.2 Hz, 1H), 6.71 (dd, J = 9.0, 4.8 Hz, 1H), 4.18 (s, 2H), 2.17 (s, 3H), 1.12 (s, 1H).

### Example 656C: (S)-tert-butyl (4-(2-(4-fluoro-2-methylphenoxy)acetamido)-2-hydroxybicyclo[2.2.2]octan-1-yl)carbamate

The methodologies described in Example 569 substituting Example 445B for Example 9B and substituting Example 656B for 1-(4-chlorophenyl)cyclopropanecarboxylic acid gave the title compound. ¹H NMR (400 MHz, DMSO-*d*₆) *δ* ppm 7.27 (s, 1H), 7.02 (ddd, J = 9.3, 3.2, 0.9 Hz, 1H), 6.98 - 6.89 (m, 1H), 6.78 (dd, J = 9.0, 4.6 Hz, 1H), 6.02 (s, 1H), 4.90 (d, J = 4.0 Hz, 1H), 4.36 (s, 2H), 3.87 (dd, J = 9.0, 4.1 Hz, 1H), 2.25 (ddd, J = 12.3, 9.4, 2.5 Hz, 1H), 2.19 (s, 3H), 2.04 (ddd, J = 12.3, 10.1, 5.0 Hz, 1H), 1.95 - 1.58 (m, 8H); MS (ESI⁺) *m*/*z* 323 (M-C(O)OC(CH₃)₃+H)⁺.

### Example 656D: (S)-N-(4-amino-3-hydroxybicyclo[2.2.2]octan-1-yl)-2-(4-fluoro-2-methylphenoxy)acetamide

The methodologies described in Example 596D substituting Example 656C for Example 596C with shorter reaction time (3 hours) gave the title compound. MS (ESI⁺) *m*/*z* 323 (M+H)⁺.

### Example 656: 2-(4-chloro-3-fluorophenoxy)-N-{(2S)-4-[2-(4-fluoro-2-methylphenoxy)acetamido]-2-hydroxybicyclo[2.2.2]octan-1-yl}acetamide

The methodologies described in Example 569 substituting 2-(4-chloro-3-fluorophenoxy)acetic acid for 1-(4-chlorophenyl)cyclopropanecarboxylic acid and substituting Example 656D for Example 9B, gave the title compound. ¹H NMR (501 MHz, DMSO-*d*₆) *δ* ppm 7.48 (t, J = 8.9 Hz, 1H), 7.31 (s, 1H), 7.26 (s, 1H), 7.04 (ddd, J = 12.1, 10.3, 2.9 Hz, 2H), 6.94 (td, J = 8.7, 3.3 Hz, 1H), 6.83 (ddd, J = 9.0, 2.9, 1.2 Hz, 1H), 6.78 (dd, J = 9.0, 4.7 Hz, 1H), 5.08 (d, J = 4.3 Hz, 1H), 4.46 (s, 2H), 4.37 (s, 2H), 4.09 - 3.98 (m, 1H), 2.33 - 2.23 (m, 1H), 2.19 (s, 3H), 2.07 (td, J = 11.7, 4.7 Hz, 1H), 1.93 (d, J = 10.7 Hz, 2H), 1.90 - 1.73 (m, 6H); MS (ESI⁺) *m*/*z* 509 (M+H)⁺.

### Example 658: 2-(4-chloro-3-fluorophenoxy)-N-[(2S)-4-{2-[4-chloro-3-(methoxymethyl)phenoxy]acetamido}-2-hydroxybicyclo[2.2.2]octan-1-yl]acetamide (Compound 757)

The methodologies described in Example 653A substituting Example 499 for ethyl 2-((6-chloro-3-oxo-3,4-dihydro-2*H*-benzo[*b*][1,4]oxazin-8-yl)oxy)acetate and diluting with *N,N-*dimethylformamide/water (1.2 mL, 3:1) to purify by preparative HPLC [Waters XBridge^{™} C18 5 µm OBD column, 30 × 100 mm, flow rate 40 mL/minute, 5-100% gradient of acetonitrile in buffer (0.025 M aqueous ammonium bicarbonate, adjusted to pH 10 with ammonium hydroxide)] gave the title compound. ¹H NMR (500 MHz, CDCl₃) *δ* ppm 7.33 (t, J = 8.6 Hz, 1H), 7.28 (s, 1H), 7.06 (d, J = 3.1 Hz, 1H), 6.78 - 6.71 (m, 2H), 6.66 (ddd, J = 8.8, 2.9, 1.3 Hz, 1H), 6.35 (s, 1H), 6.25 (s, 1H), 4.79 (d, J = 2.0 Hz, 1H), 4.51 (s, 2H), 4.40 (d, J = 2.4 Hz, 2H), 4.35 (s, 2H), 4.18 (d, J = 8.9 Hz, 1H), 3.49 (s, 3H), 2.57 (ddd, J = 12.3, 8.8, 3.0 Hz, 1H), 2.48 (t, J = 11.8 Hz, 1H), 2.11 (td, J = 11.7, 8.8 Hz, 2H), 2.04 (d, J = 26.6 Hz, 1H), 2.01 - 1.88 (m, 2H), 1.88 - 1.77 (m, 2H), 1.65 (dd, J = 12.0, 6.7 Hz, 1H); MS (ESI⁺) *m*/*z* 555 (M+H)⁺.

### Example 659: 2-(4-chloro-3-fluorophenoxy)-N-[(2S)-4-{2-[(2,3-dihydro-1,4-benzodioxin-6-yl)oxy]acetamido}-2-hydroxybicyclo[2.2.2]octan-1-yl]acetamide (Compound 758)

### Example 659A: tert-butyl 2-((2,3-dihydrobenzo[b][1,4]dioxin-6-yl)oxy)acetate

To a solution of 2,3-dihydrobenzo[*b*][1,4]dioxin-6-ol (1 g, 6.57 mmol) in *N,N-*dimethylformamide (20 mL) at ambient temperature was added potassium carbonate (1.82 g, 13.2 mmol) and *tert*-butyl bromoacetate (1.11 mL, 7.56 mmol). This mixture was warmed to 65 °C and was allowed to stir for 3 hours. The mixture was then allowed to cool to ambient temperature and was stirred for 20 hours. The mixture was diluted with ethyl acetate (20 mL) and H₂O (20 mL). The layers were separated and the aqueous layer was extracted with ethyl acetate (3 × 10 mL). The combined organic fractions were dried over anhydrous Na₂SO₄, filtered, and concentrated under reduced pressure. The residue was purified via column chromatography (SiO₂, 50% ethyl acetate/heptanes) to give the title compound (1.5 g, 5.63 mmol, 86% yield). MS (ESI⁺) *m*/*z* 211.1 (M-*t*-Bu+H)⁺.

### Example 659B: 2-((2,3-dihydrobenzo[b][1,4]dioxin-6-yl)oxy)acetic acid

To a mixture of the product of Example 659A (1.5 g, 5.63 mmol) in methanol (15 mL) and water (5.00 mL) was added aqueous NaOH (5 M, 5.63 mL, 28.2 mmol). This mixture was allowed to stir at ambient temperature for 1.5 hours and then was concentrated under reduced pressure to give a residue which was dissolved in water. The pH was adjusted to ~1 with concentrated HCl, and the resulting solid was isolated via filtration to give the title compound (1.01 g, 4.81 mmol, 85% yield). MS (ESI⁺) *m*/*z* 211.3 (M+H)⁺.

### Example 659C: (S)-tert-butyl (4-(benzylamino)-2-hydroxybicyclo[2.2.2]octan-1-yl)carbamate, hydrochloric acid (Alternative preparation of Example 445A)

Water (3.24 L) was charged to a 6 L jacketed reaction vessel, followed by magnesium chloride heptahydrate (1.46 g, 7.1 mmol), NADP⁺ (1.8 g, 2.3 mmol) and monopotassium phosphate (6.93 g, 526 mmol). All components were dissolved before the pH was adjusted to pH 7.5 with 50% weight/weight NaOH to make the desired buffer (200 mL reserved). The product of Example 343B (180 g, 569 mmol) was added to the buffer, and the pH was again adjusted to pH 7.5 with 50% weight/weight NaOH. Finally, isopropanol (360 mL, 10% volume/volume) was added to the reaction followed by enzyme (3.6 g, KRED P2C02, Codexis, Redwood City, CA) that was solubilized in the reserved 200 mL of buffer. This reaction was allowed to proceed at 40 °C for 16 hours with the pH being held between 7.5 and 8.0. Upon completion of the reaction, the buffer was adjusted to pH 12 and held at this point for 30 minutes. The reaction was filtered through diatomaceous earth to remove the catalyst. Di-*tert-*butyl dicarbonate (20.7 g, 98 mmol,1.25 equivalents) was added to 3.6 L of ethyl acetate, and the solution was charged to the reaction vessel containing the filtered aqueous fraction. This reaction was allowed to proceed at 30 °C for 2.5 hours with moderate stirring. After 2.5 hours, the two layers were separated, and the aqueous fraction was assayed for remaining amino-alcohol product. Di-*tert*-butyl dicarbonate (1.25 equivalents with respect to the remaining amino-alcohol) was added to 2.0 L of ethyl acetate and charged to the reaction vessel. The reaction was allowed to proceed at 30 °C for 2.5 hours, after which the organic and aqueous layers were separated. The combined organic layers were washed with 2.5% NaOH (560 mL) and dried over Na₂SO₄. The ethyl acetate was removed in vacuo, and the residue was taken up in methyl *tert*-butyl ether (MTBE) (1.8 L) to provide the product in solution at 0.2 M. This reaction was fitted with an overhead stirrer, cooled to 0 °C and 4 N HCl in dioxane (169 mL, 1.5 equivalents with respect to product) was slowly added. The title compound precipitated out of solution and, after stirring for 5 minutes at 0 °C, and was collected by filtration. The white solid was washed with methyl *tert*-butyl ether (MTBE) (100 mL) and dried under vacuum overnight to provide the title compound (160 g, 418 mmol, 79% yield). MS (APCI⁺) *m*/*z* 347.4 (M+H)⁺.

### Example 659D: (S)-tert-butyl (4-amino-2-hydroxybicyclo[2.2.2]octan-1-yl)carbamate hydrochloride (Alternative preparation of Example 445B)

The product of Example 659C (163.7 g, 427 mmol) in methanol (1375 mL) was added to 20% Pd(OH)₂/C, wet (16 g, 58 mmol) in a 2 L Hastelloy C reactor. The reactor was purged with argon and was stirred under 50 psi of hydrogen at 40 °C. The reaction mixture was allowed to stir for 11.3 hours after reaching 38 °C. The reactor was vented and materials were filtered through 45 µm nylon filter to remove catalyst and concentrated under reduced pressure to give the title compound (121.49 g, 415 mmol, 97% yield). ¹H NMR (400 MHz, DMSO-*d*₆) *δ* ppm 8.14 (s, 3H), 6.12 (s, 1H), 5.09 (d, J = 4.2 Hz, 1H), 3.90 (dt, J = 9.4, 3.0 Hz, 1H), 2.11 (ddd, J = 12.8, 9.5, 3.0 Hz, 1H), 2.06 - 1.94 (m, 1H), 1.88 - 1.50 (m, 7H), 1.32 (s, 9H).

### Example 659E: (S)-tert-butyl (4-(2-((2,3-dihydrobenzo[b][1,4]dioxin-6-yl)oxy)acetamido)-2-hydroxybicyclo[2.2.2]octan-1-yl)carbamate

To a solution of the product of Example 659D (0.13 g, 0.44 mmol) and the product of Example 659B (0.121 g, 0.58 mmol) in *N,N*-dimethylformamide (3 mL) at ambient temperature was added triethylamine (0.25 mL, 1.78 mmol). The mixture was allowed to stir for 5 minutes, and then 2-(3*H*-[1,2,3]triazolo[4,5-*b*]pyridin-3-yl)-1,1,3,3-tetramethylisouronium hexafluorophosphate(V) (HATU, 0.219 g, 0.577 mmol) was added portion-wise over 3 minutes. The mixture was allowed to stir for 2 hours, then NaOH (10%, 0.2 mL) was added, and the mixture was allowed to stir for 20 minutes. The mixture was diluted with ethyl acetate (5 mL) and water (5 mL), and the layers were separated. The aqueous layer was extracted with ethyl acetate (3 × 3 mL). The combined organic fractions were dried over anhydrous Na₂SO₄, filtered, and concentrated under reduced pressure. The residue was purified via column chromatography (SiO₂, 10% methanol/ethyl acetate) to give the title compound (0.2 g, 0.446 mmol, 100% yield). MS (ESI⁺) *m*/*z* 393.1 (M-*t*-Bu+H)⁺.

### Example 659F: (S)-N-(4-amino-3-hydroxybicyclo[2.2.2]octan-1-yl)-2-((2,3-dihydrobenzo[b][1,4]dioxin-6-yl)oxy)acetamide, trifluoroacetic acid

To a solution of the product of Example 659E (0.2 g, 0.446 mmol) in CH₂Cl₂ (4 mL) was added trifluoroacetic acid (1.031 mL, 13.4 mmol). This mixture was allowed to stir at ambient temperature for 2 hours and then was concentrated under reduced pressure to give the title compound (0.3 g, 0.520 mmol, quantitative). MS (ESI⁺) *m*/*z* 349.3 (M+H)⁺.

### Example 659G: 2-(4-chloro-3-fluorophenoxy)-N-[(2S)-4-{2-[(2,3-dihydro-1,4-benzodioxin-6-yl)oxy]acetamido}-2-hydroxybicyclo[2.2.2]octan-1-yl]acetamide

To a solution of the product of Example 659F (0.09 g, 0.156 mmol) in *N,N-*dimethylformamide (2 mL) was added 2-(4-chloro-3-fluorophenoxy)acetic acid (0.035 g, 0.172 mmol) and triethylamine (0.087 mL, 0.625 mmol) followed by 2-(3*H*-[1,2,3]triazolo[4,5-b]pyridin-3-yl)-1,1,3,3-tetramethylisouronium hexafluorophosphate(V) (HATU, 0.071 g, 0.187 mmol). This mixture was allowed to stir at ambient temperature for 16 hours. The mixture was then directly purified by preparative HPLC [Waters XBridge^{™} C18 5 µm OBD^{™} column, 30 × 100 mm, flow rate 40 mL/minute, 5-100% gradient of acetonitrile in buffer (0.025 M aqueous ammonium bicarbonate, adjusted to pH 10 with ammonium hydroxide)] to give the title compound (0.050 g, 0.093 mmol, 60% yield). ¹H NMR (500 MHz, DMSO-*d*₆) *δ* ppm 7.48 (t, J = 8.9 Hz, 1H), 7.35 (s, 1H), 7.27 (s, 1H), 7.06 (dd, J = 11.4, 2.9 Hz, 1H), 6.83 (ddd, J = 9.0, 2.9, 1.2 Hz, 1H), 6.75 (d, J = 8.8 Hz, 1H), 6.44 (d, J = 2.9 Hz, 1H), 6.40 (dd, J = 8.8, 2.9 Hz, 1H), 5.08 (d, J = 4.4 Hz, 1H), 4.47 (s, 2H), 4.27 (s, 2H), 4.23 - 4.13 (m, 4H), 4.04 (dq, J = 8.6, 3.0 Hz, 1H), 2.27 (ddd, J = 12.4, 9.5, 2.3 Hz, 1H), 2.11 - 2.02 (m, 1H), 1.98 - 1.70 (m, 8H); MS (ESI⁺) *m*/*z* 535.2 (M+H)⁺.

### Example 660: 2-(4-chlorophenoxy)-N-[(2S)-4-{2-[3-fluoro-4-(trifluoromethoxy)phenoxy]acetamidol-2-hydroxybicyclo[2.2.2]octan-1-yl]acetamide (Compound 759)

The title compound was prepared using the methodologies described above. ¹H NMR (500 MHz, DMSO-*d₆*) *δ* ppm 7.54 (s, 1H), 7.52 - 7.45 (m, 1H), 7.37 - 7.30 (m, 2H), 7.25 (s, 1H), 7.09 (dd, J = 12.4, 2.9 Hz, 1H), 6.99 - 6.92 (m, 2H), 6.84 (ddd, J = 9.2, 3.0, 1.4 Hz, 1H), 5.13 (d, J = 4.4 Hz, 1H), 4.46 (s, 2H), 4.42 (d, J = 1.0 Hz, 2H), 4.05 - 3.98 (m, 1H), 2.28 (ddd, J = 12.4, 9.5, 2.5 Hz, 1H), 2.16 - 2.06 (m, 1H), 1.98 - 1.88 (m, 2H), 1.88 - 1.74 (m, 6H); MS (APCI⁺) *m*/*z* 560 (M+H)⁺.

### Example 661: 2-[(2,2-difluoro-2H-1,3-benzodioxol-5-yl)oxy]-N-[(2S)-4-{2-[3-fluoro-4-(trifluoromethoxy)phenoxy]acetamidol-2-hydroxybicyclo[2.2.2]octan-1-yl]acetamide (Compound 760)

The title compound was prepared using the methodologies described above. ¹H NMR (400 MHz, DMSO-*d₆*) *δ* ppm 7.53 (s, 1H), 7.51 - 7.44 (m, 1H), 7.31 (d, J = 8.9 Hz, 1H), 7.23 (s, 1H), 7.12 (d, J = 2.6 Hz, 1H), 7.08 (dd, J = 12.4, 3.0 Hz, 1H), 6.84 (ddd, J = 9.2, 3.0, 1.5 Hz, 1H), 6.75 (dd, J = 8.9, 2.6 Hz, 1H), 5.12 (d, J = 4.0 Hz, 1H), 4.46 (s, 2H), 4.43 (s, 2H), 4.07 - 4.00 (m, 1H), 2.34 - 2.23 (m, 1H), 2.16 - 2.04 (m, 1H), 1.99 - 1.71 (m, 8H); MS (APCI⁺) *m*/*z* 607 (M+H)⁺.

### Example 662: 2-(4-chloro-3-fluorophenoxy)-N-[(2S)-4-{2-[3-fluoro-4-(trifluoromethoxy)phenoxy]acetamidol-2-hydroxybicyclo[2.2.2]octan-1-yl]acetamide (Compound 761)

The title compound was prepared using the methodologies described above. ¹H NMR (400 MHz, DMSO-*d₆*) *δ* ppm 7.53 (s, 1H), 7.51 - 7.45 (m, 2H), 7.26 (s, 1H), 7.12 - 7.02 (m, 2H), 6.87 - 6.80 (m, 2H), 5.08 (d, J = 4.2 Hz, 1H), 4.47 (s, 2H), 4.46 (s, 2H), 4.08 - 4.01 (m, 1H), 2.32 - 2.22 (m, 1H), 2.11 = 2.02 (m, 1H), 1.99 - 1.66 (m, 8H); MS (APCI⁺) *m*/*z* 579 (M+H)⁺.

### Example 663: 2-(4-fluorophenoxy)-N-[(2S)-4-{2-[3-fluoro-4-(trifluoromethoxy)phenoxy]acetamido}-2-hydroxybicyclo[2.2.2]octan-1-yl]acetamide (Compound 762)

The title compound was prepared using the methodologies described above. ¹H NMR (501 MHz, DMSO-*d₆*) *δ* ppm 7.53 (s, 1H), 7.48 (td, J = 9.1, 1.2 Hz, 1H), 7.21 (s, 1H), 7.15 - 7.10 (m, 2H), 7.08 (dd, J = 12.4, 3.0 Hz, 1H), 6.98 - 6.93 (m, 2H), 6.84 (ddd, J = 9.2, 3.0, 1.4 Hz, 1H), 5.14 (d, J = 4.3 Hz, 1H), 4.46 (s, 2H), 4.39 (d, J = 1.0 Hz, 2H), 4.03 - 3.98 (m, 1H), 2.32 - 2.25 (m, 1H), 2.19 - 2.09 (m, 1H), 1.99 - 1.72 (m, 8H); MS (APCI⁺) *m*/*z* 545 (M+H)⁺.

### Example 664: 2-(3,4-difluorophenoxy)-N-[(2S)-4-{2-[3-fluoro-4-(trifluoromethoxy)phenoxy]acetamidol-2-hydroxybicyclo[2.2.2]octan-1-yl]acetamide (Compound 763)

The title compound was prepared using the methodologies described above. ¹H NMR (501 MHz, DMSO-*d₆*) *δ* ppm 7.53 (s, 1H), 7.51 - 7.46 (m, 1H), 7.36 (dt, J = 10.6, 9.3 Hz, 1H), 7.23 (s, 1H), 7.11 - 7.04 (m, 2H), 6.84 (ddd, J = 9.2, 3.0, 1.4 Hz, 1H), 6.77 (dtd, J = 9.1, 3.3, 1.7 Hz, 1H), 5.10 (d, J = 4.4 Hz, 1H), 4.46 (s, 2H), 4.43 (s, 2H), 4.06 - 4.00 (m, 1H), 2.28 (ddd, J = 12.4, 9.4, 2.4 Hz, 1H), 2.09 (ddd, J = 12.4, 10.8, 4.7 Hz, 1H), 1.98 - 1.74 (m, 8H); MS (APCI⁺) *m*/*z* 563 (M+H)⁺.

### Example 665: 2-[3-fluoro-4-(trifluoromethoxy)phenoxy]-N-[(35)-3-hydroxy-4-{2-[4-(trifluoromethoxy)phenoxy]acetamido}bicyclo[2.2.2]octan-1-yl]acetamide (Compound 764)

The title compound was prepared using the methodologies described above. ¹H NMR (400 MHz, DMSO-*d₆*) *δ* ppm 7.53 (s, 1H), 7.51 - 7.45 (m, 1H), 7.33 - 7.23 (m, 3H), 7.08 (dd, J = 12.4, 3.0 Hz, 1H), 7.05 - 6.99 (m, 2H), 6.84 (ddd, J = 9.1, 2.9, 1.4 Hz, 1H), 5.11 (d, J = 4.1 Hz, 1H), 4.46 (s, 2H), 4.45 (s, 2H), 4.08 - 3.99 (m, 1H), 2.34 - 2.22 (m, 1H), 2.15 - 2.07 (m, 1H), 1.98 - 1.71 (m, 9H); MS (APCI⁺) *m*/*z* 611 (M+H)⁺.

### Example 666: 2-[3-fluoro-4-(methanesulfonyl)phenoxy]-N-[(3S)-3-hydroxy-4-{2-[4-(trifluoromethoxy)phenoxy]acetamido}bicyclo[2.2.2]octan-1-yl]acetamide (Compound 765)

The title compound was prepared using the methodologies described above. ¹H NMR (400 MHz, DMSO-*d₆*) *δ* ppm 7.76 (t, J = 8.7 Hz, 1H), 7.62 (s, 1H), 7.33 - 7.24 (m, 3H), 7.07 (dd, J = 12.4, 2.4 Hz, 1H), 7.04 - 6.99 (m, 2H), 6.96 (dd, J = 8.9, 2.4 Hz, 1H), 5.12 (d, J = 4.0 Hz, 1H), 4.56 (s, 2H), 4.45 (s, 2H), 4.07 - 3.97 (m, 1H), 3.26 (s, 3H), 2.28 (ddd, J = 12.4, 9.5, 2.3 Hz, 1H), 2.16 - 2.06 (m, 1H), 1.99 - 1.73 (m, 8H); MS (APCI⁺) *m*/*z* 605 (M+H)⁺.

### Example 667: 2-[(2,2-difluoro-2H-1,3-benzodioxol-5-yl)oxy]-N-[(2S)-4-{2-[3-fluoro-4-(methanesulfonyl)phenoxy]acetamido}-2-hydroxybicyclo[2.2.2]octan-1-yl]acetamide (Compound 766)

The title compound was prepared using the methodologies described above. ¹H NMR (501 MHz, DMSO-*d₆*) *δ* ppm 7.76 (t, J = 8.6 Hz, 1H), 7.62 (s, 1H), 7.31 (d, J = 8.8 Hz, 1H), 7.23 (s, 1H), 7.12 (d, J = 2.6 Hz, 1H), 7.07 (dd, J = 12.3, 2.4 Hz, 1H), 6.96 (dd, J = 8.9, 2.4 Hz, 1H), 6.74 (dd, J = 8.9, 2.6 Hz, 1H), 5.11 (d, J = 4.1 Hz, 1H), 4.56 (s, 2H), 4.43 (s, 2H), 4.06 - 4.00 (m, 1H), 3.26 (s, 3H), 2.28 (ddd, J = 12.4, 9.5, 2.4 Hz, 1H), 2.14 - 2.06 (m, 1H), 1.98 - 1.72 (m, 8H); MS (APCI⁺) *m*/*z* 601 (M+H)⁺.

### Example 668: 2-[3-fluoro-4-(methanesulfonyl)phenoxy]-N-{(3S)-4-[2-(4-fluorophenoxy)acetamido]-3-hydroxybicyclo[2.2.2]octan-1-yl}acetamide (Compound 767)

The title compound was prepared using the methodologies described above. ¹H NMR (400 MHz, DMSO-*d₆*) *δ* ppm 7.76 (t, J = 8.7 Hz, 1H), 7.62 (s, 1H), 7.21 (s, 1H), 7.16 - 7.09 (m, 2H), 7.07 (dd, J = 12.4, 2.4 Hz, 1H), 6.98 - 6.92 (m, 3H), 5.14 (d, J = 3.0 Hz, 1H), 4.56 (s, 2H), 4.39 (s, 2H), 4.04 - 3.97 (m, 1H), 3.26 (s, 3H), 2.33 - 2.24 (m, 1H), 2.20 - 2.07 (m, 1H), 1.99 - 1.72 (m, 8H); MS (APCI⁺) *m*/*z* 539 (M+H)⁺.

### Example 669: 2-(3,4-difluorophenoxy)-N-[(2S)-4-{2-[3-fluoro-4-(methanesulfonyl)phenoxy]acetamido}-2-hydroxybicyclo[2.2.2]octan-1-yl]acetamide (Compound 768)

The title compound was prepared using the methodologies described above. ¹H NMR (501 MHz, DMSO-*d₆*) *δ* ppm 1.72-1.99 (m, 8H), 2.04-2.13 (m, 1H), 2.24-2.31 (m, 1H), 3.26 (s, 3H), 3.98-4.07 (m, 1H), 4.43 (s, 2H), 4.56 (s, 2H), 5.10 (d, J = 4.3Hz, 1H), 6.74-6.80 (m, 1H), 6.96 (dd, J = 8.9, 2.4Hz, 1H), 7.04-7.09 (m, 2H), 7.23 (s, 1H), 7.32-7.39 (m, 1H), 7.62 (s, 1H), 7.76 (t, J = 8.7Hz, 1H); MS (APCI⁺) *m*/*z* 557 (M+H)⁺.

### Example 670: 2-(4-chloro-3-fluorophenoxy)-N-[(2S)-4-{2-[3-fluoro-4-(methanesulfonyl)phenoxy]acetamido}-2-hydroxybicyclo[2.2.2]octan-1-yl]acetamide (Compound 769)

The title compound was prepared using the methodologies described above. ¹H NMR (501 MHz, DMSO-*d₆*) *δ* ppm 7.77 (t, J = 8.7 Hz, 1H), 7.62 (s, 1H), 7.48 (t, J = 8.9 Hz, 1H), 7.26 (s, 1H), 7.09 - 7.03 (m, 2H), 6.96 (dd, J = 8.9, 2.4 Hz, 1H), 6.83 (ddd, J = 8.9, 2.9, 1.1 Hz, 1H), 5.09 (d, J = 4.0 Hz, 1H), 4.57 (s, 2H), 4.47 (s, 2H), 4.07 - 4.01 (m, 1H), 3.26 (s, 3H), 2.32 - 2.24 (m, 1H), 2.10 - 2.03 (m, 1H), 1.98 - 1.73 (m, 8H); MS (APCI⁺) *m*/*z* 573 (M+H)⁺.

### Example 674: 2-[(2,2-difluoro-2H-1,3-benzodioxol-5-yl)oxy]-N-[(2S)-4-{2-[3-fluoro-4-(trifluoromethyl)phenoxy]acetamido}-2-hydroxybicyclo[2.2.2]octan-1-yl]acetamide (Compound 773)

### Example 674A: (S)-N-(4-amino-3-hydroxybicyclo[2.2.2]octan-1-yl)-2-(3-fluoro-4-(trifluoromethyl)phenoxy)acetamide, Trifluoroacetic Acid

To a mixture of Example 445B (0.5 g, 1.708 mmol), Example 574A (0.468 g, 1.964 mmol), and *N*-ethyl-*N*-isopropylpropan-2-amine (1.044 mL, 5.98 mmol) in *N,N-*dimethylformamide (10.0 mL), 2-(3*H*-[1,2,3]triazolo[4,5-b]pyridin-3-yl)-1,1,3,3-tetramethylisouronium hexafluorophosphate(V) (2.67 mL, 2.135 mmol) was added, and the mixture was stirred at ambient temperature for 16 hours. Volatiles were removed under high vacuum, and the residue was dissolved in 20 mL of tetrahydrofuran and treated with 20 mL of 1 N aqueous LiOH for 1 hour. The mixture was concentrated, and the residue was suspended in dichloromethane (10 mL) and treated with trifluoroacetic acid (1.97 mL, 25.6 mmol) for 45 minutes. The mixture was concentrated under vacuum, and the residue was purified by HPLC (Phenomenex^{®} Luna^{®} C18(2) 10 µm 100Å AXIA^{™} column (250 mm × 50 mm). A 30-100% gradient of acetonitrile (A) and 0.1% trifluoroacetic acid in water (B) was used over 25 minutes, at a flow rate of 50 mL/minute) to give 670 mg of the title compound. ¹H NMR (400 MHz, DMSO-*d*₆) *δ* ppm 8.25 (s, 1H), 7.69 (d, J = 15.2 Hz, 4H), 7.60 (t, J = 8.8 Hz, 1H), 6.97 (dd, J = 13.1, 2.4 Hz, 1H), 6.84 (dd, J = 8.8, 2.3 Hz, 1H), 4.48 (d, J = 5.8 Hz, 2H), 3.73 (d, J = 8.1 Hz, 1H), 2.26 (ddd, J = 12.9, 9.5, 2.9 Hz, 1H), 1.96 (ddd, J = 13.7, 8.6, 4.9 Hz, 1H), 1.93 - 1.80 (m, 1H), 1.75 (ddt, J = 21.5, 10.7, 5.9 Hz, 5H), 1.61 - 1.42 (m, 2H); MS (+ESI) *m*/*z* 377.1 (M+H)⁺.

### Example 674B: (S)-2-((2,2-difluorobenzo[d][1,3]dioxol-5-yl)oxy)-N-(4-(2-(3-fluoro-4-(trifluoromethyl)phenoxy)acetamido)-2-hydroxybicyclo[2.2.2]octan-1-yl)acetamide

To a mixture of Example 674A (50 mg, 0.102 mmol), 2-((2,2-difluorobenzo[*d*][1,3]dioxol-5-yl)oxy)acetic acid (26.0 mg, 0.112 mmol), and *N*-ethyl-*N-*isopropylpropan-2-amine (0.089 mL, 0.510 mmol) in *N,N*-dimethylformamide (2.0 mL), 2-(3*H-*[1,2,3]triazolo[4,5-*b*]pyridin-3-yl)-1,1,3,3-tetramethylisouronium hexafluorophosphate(V) (0.510 mL, 0.127 mmol) was added and the mixture was stirred at ambient temperature for 120 minutes. The mixture was concentrated, and the residue was purified by prep HPLC (Phenomenex^{®} Luna^{®} C18(2) 10 µm 100Å AXIA^{™} column (250 mm × 50 mm). A 20-95% gradient of acetonitrile (A) and 0.1% trifluoroacetic acid in water (B) was used over 25 minutes, at a flow rate of 50 mL/minute) to give 41 mg of the title compound. ¹H NMR (400 MHz, DMSO-*d*₆) *δ* ppm 7.67 (t, J = 8.8 Hz, 1H), 7.57 (s, 1H), 7.29 (d, J = 8.9 Hz, 1H), 7.20 (s, 1H), 7.10 (d, J = 2.5 Hz, 1H), 7.04 (dd, J = 13.1, 2.4 Hz, 1H), 6.89 (dd, J = 8.8, 2.3 Hz, 1H), 6.72 (dd, J = 8.9, 2.6 Hz, 1H), 5.05 (s, 1H), 4.52 (s, 2H), 4.41 (s, 2H), 4.01 (dd, J = 9.5, 3.2 Hz, 1H), 2.26 (ddd, J = 12.5, 9.5, 2.4 Hz, 1H), 2.12 - 2.03 (m, 1H), 1.97 - 1.84 (m, 2H), 1.87 - 1.71 (m, 6H).; MS (+ESI) *m*/*z* = 591.1 (M+H)⁺.

### Example 675: 2-(4-chlorophenoxy)-N-[(2S)-4-{2-[3-(difluoromethyl)-4-fluorophenoxylacetamidol-2-hydroxybicyclo[2.2.2]octan-1-yl]acetamide (Compound 774)

### Example 675A: 3-(difluoromethyl)-4-fluorophenol

To a solution of 5-((*tert*-butyldimethylsilyl)oxy)-2-fluorobenzaldehyde (0.5 g, 2 mmol) in dichloromethane (8.6 mL) was added DAST (diethylaminosulfur trifluoride) (0.65 mL, 4.9 mmol). This reaction mixture was stirred at ambient temperature overnight and then diluted with NaHCO₃ (saturated aqueous) until the pH was 8-9. Then the layers were separated, and the organic layer was dried (Na₂SO₄) and concentrated to give the title compound (0.3 g, 1.9 mmol, 92% yield), which was carried forward without additional purification.

### Example 675B: tert-butyl 2-(3-(difluoromethyl)-4-fluorophenoxy)acetate

The methodologies described in Example 596A substituting Example 675A for 6-hydroxy-2-methylisoindolin-1-one gave the title compound. ¹H NMR (400 MHz, DMSO-*d*₆) *δ* ppm 7.38 - 7.32 (m, 1H), 7.21 - 7.13 (m, 2H), 7.02 - 6.95 (m, 1H), 4.76 (s, 2H), 1.46 (s, 9H).

### Example 675C: 2-(3-(difluoromethyl)-4-fluorophenoxy)acetic acid

The methodologies described in Example 652B substituting Example 675B for Example 652A gave the title compound. ¹H NMR 1H NMR (400 MHz, DMSO-*d*₆) *δ* ppm 10.17 (d, J = 12.2 Hz, 1H), 7.34 - 7.27 (m, 1H), 7.16 - 7.12 (m, 2H), 6.99 - 6.97 (m, 1H), 4.74 (s, 2H).

### Example 675D: (S)-tert-butyl (4-(2-(3-(difluoromethyl)-4-fluorophenoxy)acetamido)-2-hydroxybicyclo[2.2.2]octan-1-yl)carbamate

The methodologies described in Example 569 substituting Example 445B for Example 9B and substituting Example 675C for 1-(4-chlorophenyl)cyclopropanecarboxylic acid gave the title compound. MS (ESI⁺) *m*/*z* 459 (M+H)⁺.

### Example 675E: (S)-N-(4-amino-3-hydroxybicyclo[2.2.2]octan-1-yl)-2-(3-(difluoromethyl)-4-fluorophenoxy)acetamide

The methodologies described in Example 596D substituting Example 675D for Example 596C with shorter reaction time (2.5 hours) gave the title compound. MS (ESI⁺) *m*/*z* 359 (M+H)⁺.

### Example 675F: 2-(4-chlorophenoxy)-N-[(2S)-4-{2-[3-(difluoromethyl)-4-fluorophenoxy]acetamido}-2-hydroxybicyclo[2.2.2]octan-1-yl]acetamide

To a solution of Example 675E (0.028 g, 0.078 mmol) in *N*,*N*-dimethylformamide (0.44 mL) was added triethylamine (0.08 mL, 0.6 mmol) followed by 2-(4-chlorophenoxy)acetyl chloride (0.01 mL, 0.08 mmol). This mixture was allowed to stir at ambient temperature overnight, was diluted with *N*,*N*-dimethylformamide (0.5 mL), and purified by preparative HPLC [Waters XBridge^{™} C18 5 µm OBD column, 30 × 100 mm, flow rate 40 mL/minute, 5-100% gradient of acetonitrile in buffer (0.025 M aqueous ammonium bicarbonate, adjusted to pH 10 with ammonium hydroxide)] to give the title compound (0.020 g, 0.038 mmol, 49% yield). ¹H NMR (501 MHz, DMSO-*d*₆) *δ* ppm 7.52 (s, 1H), 7.34 (d, J = 8.9 Hz, 2H), 7.27 - 7.21 (m, 2H), 7.16 - 7.09 (m, 2H), 6.96 (d, J = 8.7 Hz, 2H), 6.78 (d, J = 9.0 Hz, 1H), 4.43 (s, 2H), 4.42 (s, 2H), 4.02 (d, J = 8.7 Hz, 1H), 2.30 - 2.27 (m, 1H), 2.14 - 2.09 (m, 1H), 1.95 - 1.91 (m, 1H), 1.86 - 1.77 (m, 7H); MS (ESI⁺) *m*/*z* 527 (M+H)⁺.

### Example 676: 2-[3-(difluoromethyl)-4-fluorophenoxy]-N-{(3S)-4-[2-(3,4-difluorophenoxy)acetamido]-3-hydroxybicyclo[2.2.2]octan-1-yl}acetamide (Compound 775)

The methodologies described in Example 569 substituting 2-(3,4-difluorophenoxy)acetic acid for 1-(4-chlorophenyl)cyclopropanecarboxylic acid and substituting Example 675E for Example 9B gave the title compound. ¹H NMR (400 MHz, DMSO-*d*₆) *δ* ppm 7.51 (s, 1H), 7.41 - 7.26 (m, 2H), 7.24 (s, 1H), 7.18 - 7.01 (m, 2H), 6.77 (d, J = 9.1 Hz, 1H), 6.62 (s, 1H), 5.10 (s, 1H), 4.43 (s, 4H), 4.04 (s, 1H), 2.30 - 2.24 (m, 1H), 2.12 - 2.06 (m, 1H), 1.94 - 1.92 (m, 1H), 1.88 - 1.57 (m, 7H); MS (ESI⁺) *m*/*z* 529 (M+H)⁺.

### Example 677: 2-(4-chloro-3-fluorophenoxy)-N-[(2S)-2-hydroxy-4-{2-[2-(methoxymethyl)phenoxy]acetamido}bicyclo[2.2.2]octan-1-yl]acetamide (Compound 776)

### Example 677A: tert-butyl 2-(2-(methoxymethyl)phenoxy)acetate

The methodologies described in Example 596A substituting 2-(methoxymethyl)phenol for 6-hydroxy-2-methylisoindolin-1-one gave the title compound. ¹H NMR (400 MHz, DMSO-*d*₆) *δ* ppm 7.41 - 7.34 (m, 1H), 7.34 - 7.25 (m, 1H), 7.01 (td, J = 7.4, 1.0 Hz, 1H), 6.92 (dd, J = 8.3, 1.0 Hz, 1H), 4.73 (s, 2H), 4.51 (s, 2H), 3.38 (s, 3H), 1.47 (s, 9H).

### Example 677B: 2-(2-(methoxymethyl)phenoxy)acetic acid

The methodologies described in Example 652B substituting Example 677A for Example 652A gave the title compound. ¹H NMR (400 MHz, DMSO-*d*₆) *δ* ppm 7.27 (dd, J = 7.5, 1.7 Hz, 1H), 7.19 (td, J = 7.8, 1.8 Hz, 1H), 6.91 (td, J = 7.5, 1.0 Hz, 1H), 6.83 (dd, J = 8.2, 1.0 Hz, 1H), 4.66 (s, 2H), 4.41 (s, 2H), 3.28 (s, 3H).

### Example 677C: (S)-tert-butyl (2-hydroxy-4-(2-(2-(methoxymethyl)phenoxy)acetamido)bicyclo[2.2.2]octan-1-yl)carbamate

The methodologies described in Example 569 substituting Example 445B for Example 9B and substituting Example 677B for 1-(4-chlorophenyl)cyclopropanecarboxylic acid gave the title compound. MS (ESI⁺) *m*/*z* 435 (M+H)⁺.

### Example 677D: (S)-N-(4-amino-3-hydroxybicyclo[2.2.2]octan-1-yl)-2-(2-(methoxymethyl)phenoxy)acetamide

The methodologies described in Example 596D substituting Example 677C for Example 596C with shorter reaction time (1.5 hours) gave the title compound. MS (ESI⁺) *m*/*z* 335 (M+H)⁺.

### Example 677E: 2-(4-chloro-3-fluorophenoxy)-N-[(2S)-2-hydroxy-4-[2-[2-(methoxymethyl)phenoxy]acetamido}bicyclo[2.2.2]octan-1-yl]acetamide

The methodologies described in Example 569 with the following modifications gave the title compound: (1) substituting 2-(4-chloro-3-fluorophenoxy)acetic acid for 1-(4-chlorophenyl)cyclopropanecarboxylic acid, (2) substituting Example 677D for Example 9B, and (3) additionally stirring the reaction mixture with LiOH (1 N aqueous, 5 mL) for 2 hours prior to purification to cleave any esterification product that was formed. ¹H NMR (500 MHz, DMSO-*d*₆) *δ* ppm 7.48 (t, J = 8.9 Hz, 1H), 7.32 - 7.22 (m, 4H), 7.05 (dd, J = 11.4, 2.9 Hz, 1H), 6.99 - 6.92 (m, 1H), 6.92 - 6.87 (m, 1H), 6.86 - 6.80 (m, 1H), 5.09 (d, J = 4.4 Hz, 1H), 4.46 (s, 2H), 4.45 (s, 2H), 4.43 (s, 2H), 4.07 - 4.01 (m, 1H), 3.30 (s, 3H), 2.30 - 2.20 (m, 1H), 2.07 - 2.02 (m, 1H), 1.96 - 1.72 (m, 8H); MS (ESI⁺) *m*/*z* 521 (M+H)⁺.

### Example 678: 2-(4-chloro-3-fluorophenoxy)-N-[(2S)-2-hydroxy-4-{2-[3-(methoxymethyl)phenoxy]acetamido}bicyclo[2.2.2]octan-1-yl]acetamide (Compound 777)

### Example 678A: tert-butyl 2-(3-(methoxymethyl)phenoxy)acetate

The methodologies described in Example 596A substituting 3-(methoxymethyl)phenol for 6-hydroxy-2-methylisoindolin-1-one gave the title compound. ¹H NMR (400 MHz, DMSO-*d*₆) *δ* ppm 7.31 (t, J = 7.8 Hz, 1H), 6.99 - 6.92 (m, 1H), 6.92 - 6.82 (m, 2H), 4.69 (s, 2H), 4.43 (s, 2H), 3.33 (s, 3H), 1.48 (s, 9H).

### Example 678B: 2-(3-(methoxymethyl)phenoxy)acetic acid

The methodologies described in Example 652B substituting Example 678A for Example 652A gave the title compound. ¹H NMR (501 MHz, DMSO-*d*₆) *δ* ppm 7.25 (t, J = 7.8 Hz, 1H), 6.93 - 6.79 (m, 3H), 4.66 (s, 2H), 4.37 (s, 2H), 3.28 (s, 3H).

### Example 678C: (S)-tert-butyl (2-hydroxy-4-(2-(3-(methoxymethyl)phenoxy)acetamido)bicyclo[2.2.2]octan-1-yl)carbamate

The methodologies described in Example 569 substituting Example 445B for Example 9B and substituting Example 678B for 1-(4-chlorophenyl)cyclopropanecarboxylic acid gave the title compound. MS (ESI⁺) *m*/*z* 435 (M+H)⁺.

### Example 678D: (S)-N-(4-amino-3-hydroxybicyclo[2.2.2]octan-1-yl)-2-(3-(methoxymethyl)phenoxy)acetamide

The methodologies described in Example 596D substituting Example 678C for Example 596C with shorter reaction time (1.5 hours) gave the title compound. MS (ESI⁺) *m*/*z* 335 (M+H)⁺.

### Example 678: 2-(4-chloro-3-fluorophenoxy)-N-[(2S)-2-hydroxy-4-{2-[3-(methoxymethyl)phenoxy]acetamido}bicyclo[2.2.2]octan-1-yl]acetamide

The methodologies described in Example 569 substituting 2-(4-chloro-3-fluorophenoxy)acetic acid for 1-(4-chlorophenyl)cyclopropanecarboxylic acid and substituting Example 678D for Example 9B gave the title compound. ¹H NMR (400 MHz, DMSO-*d*₆) *δ* ppm 7.48 (t, J = 8.9 Hz, 1H), 7.41 (s, 1H), 7.30 - 7.21 (m, 2H), 7.05 (dd, J = 11.4, 2.9 Hz, 1H), 6.91 - 6.85 (m, 2H), 6.82 (ddt, J = 8.8, 3.3, 1.7 Hz, 2H), 5.07 (d, J = 4.4 Hz, 1H), 4.46 (s, 2H), 4.37 (s, 4H), 4.11 - 3.99 (m, 1H), 3.28 (s, 3H), 2.38 - 2.20 (m, 1H), 2.16 - 2.00 (m, 1H), 1.97 - 1.68 (m, 8H); MS (ESI⁺) *m*/*z* 521 (M+H)⁺.

### Example 679: 2-(3,4-difluorophenoxy)-N-{(2S)-4-[2-(4-ethylphenoxy)acetamido]-2-hydroxybicyclo[2.2.2]octan-1-yl}acetamide (Compound 778)

### Example 679A: (S)-tert-butyl (4-(2-(4-ethylphenoxy)acetamido)-2-hydroxybicyclo[2.2.2]octan-1-yl)carbamate

To a solution of the product of Example 659D (0.266 g, 0.908 mmol) in *N,N-*dimethylformamide (4 mL) at ambient temperature was added triethylamine (0.506 mL, 3.63 mmol) followed by (4-ethylphenoxy)acetyl chloride (0.199 g, 1 mmol). The mixture was allowed to stir at ambient temperature for one hour, and then the mixture was purified directly by column chromatography (SiO₂, 10% methanol/ethyl acetate) to give the title compound (0.29 g, 0.693 mmol, 76% yield). MS (ESI⁺) *m*/*z* 363 (M-*t*-Bu+H)⁺.

### Example 679B: (S)-N-(4-amino-3-hydroxybicyclo[2.2.2]octan-1-yl)-2-(4-ethylphenoxy)acetamide, trifluoroacetate

To a solution of the product of Example 679A (0.29 g, 0.693 mmol) in CH₂Cl₂ (5 mL) was added trifluoroacetic acid (1.60 mL, 20.8 mmol). This mixture was allowed to stir at ambient temperature for 2 hours, and then was concentrated under reduced pressure to give the title compound (0.38 g, 0.695 mmol, 100% yield). MS (ESI⁺) *m*/*z* 319.4 (M+H)⁺.

### Example 679C: 2-(3,4-difluorophenoxy)-N-{(2S)-4-[2-(4-ethylphenoxy)acetamido]-2-hydroxybicyclo[2.2.2]octan-1-yl}acetamide

To a solution of the product of Example 679B (0.055 g, 0.10 mmol) in *N,N-*dimethylformamide (1.5 mL) was added (3,4-difluorophenoxy)acetic acid (0.021 g, 0.11 mmol) and triethylamine (0.056 mL, 0.40 mmol) followed by 2-(3H-[1,2,3]triazolo[4,5-*b*]pyridin-3-yl)-1,1,3,3-tetramethylisouronium hexafluorophosphate(V) (HATU, 0.046 g, 0.121 mmol). This mixture was allowed to stir at ambient temperature for 16 hours. The mixture was then directly purified by preparative HPLC [Waters XBridge^{™} C18 5 µm OBD^{™} column, 30 × 100 mm, flow rate 40 mL/minute, 5-100% gradient of acetonitrile in buffer (0.025 M aqueous ammonium bicarbonate, adjusted to pH 10 with ammonium hydroxide)] to give the title compound (0.025 g, 0.051 mmol, 51% yield). ¹H NMR (501 MHz, DMSO-*d*₆) *δ* ppm 7.38 - 7.29 (m, 2H), 7.21 (s, 1H), 7.09 (d, J = 8.5 Hz, 2H), 7.05 (ddd, J = 12.6, 6.8, 3.1 Hz, 1H), 6.82 - 6.78 (m, 2H), 6.75 (dtd, J = 9.1, 3.3, 1.6 Hz, 1H), 5.07 (d, J = 4.4 Hz, 1H), 4.41 (s, 2H), 4.31 (s, 2H), 4.06 - 3.97 (m, 1H), 2.54 - 2.49 (m, 2H), 2.26 (ddd, J = 12.4, 9.4, 2.4 Hz, 1H), 2.07 (ddd, J = 12.1, 10.5, 4.6 Hz, 1H), 1.95 - 1.87 (m, 2H), 1.85 - 1.72 (m, 6H), 1.12 (t, J = 7.6 Hz, 3H); MS (ESI⁺) *m*/*z* 489.2 (M+H)⁺.

### Example 680: N-{3-[2-(4-chloro-3-fluorophenoxy)acetamido]bicyclo[1.1.1]pentan-1-yl}-2-(4-fluorophenyl)-2-(2-hydroxyethoxy)acetamide (Compound 779)

### Example 680A: N-(3-(2-(4-chloro-3-fluorophenoxy)acetamido)bicyclo[1.1.1]pentan-1-yl)-2-(4-fluorophenyl)-2-hydroxyacetamide

The methodologies described in Example 569 substituting 2-(4-fluorophenyl)-2-hydroxyacetic acid for 1-(4-chlorophenyl)cyclopropanecarboxylic acid gave the title compound. ¹H NMR (400 MHz, DMSO-*d*₆) *δ* ppm 8.68 (s, 1H), 8.54 (s, 1H), 7.55 - 7.35 (m, 3H), 7.22 - 7.10 (m, 2H), 7.06 (dd, J = 11.4, 2.8 Hz, 1H), 6.84 (ddd, J = 9.0, 2.9, 1.2 Hz, 1H), 6.17 (d, J = 4.8 Hz, 1H), 4.86 (d, J = 4.8 Hz, 1H), 4.47 (s, 2H), 2.22 (s, 6H); MS (ESI⁺) *m*/*z* 437 (M+H)⁺.

### Example 680B: N-{3-[2-(4-chloro-3-fluorophenoxy)acetamido]bicyclo[1.1.1]pentan-1-yl}-2-(4-fluorophenyl)-2-(2-hydroxyethoxy)acetamide

The methodologies described in Example 599C with the following modifications gave the title compound: (1) substituting Example 680A for Example 599B, (2) substituting 2-bromoethanol for *tert*-butyl 3-bromopropanoate, (3) without *N*,*N*-dimethylformamide, (4) at 65 °C, and (5) purifying by preparative HPLC [Waters XBridge^{™} C18 5 µm OBD column, 30 × 100 mm, flow rate 40 mL/minute, 5-100% gradient of acetonitrile in buffer (0.025 M aqueous ammonium bicarbonate, adjusted to pH 10 with ammonium hydroxide)]. ¹H NMR (501 MHz, DMSO-*d*₆) *δ* ppm 8.70 (s, 1H), 8.60 (s, 1H), 7.49 (t, J = 8.9 Hz, 1H), 7.43 - 7.37 (m, 2H), 7.22 - 7.15 (m, 2H), 7.07 (dd, J = 11.4, 2.8 Hz, 1H), 6.85 (ddd, J = 9.0, 2.8, 1.1 Hz, 1H), 4.80 (t, J = 5.7 Hz, 1H), 4.75 (d, J = 8.8 Hz, 1H), 4.46 (s, 2H), 3.55 (p, J = 5.6, 5.0 Hz, 2H), 3.45 - 3.36 (m, 2H), 2.23 (d, J = 3.7 Hz, 6H); MS (ESI⁺) *m*/*z* 481 (M+H)⁺.

### Example 681: 2-[(2,2-difluoro-2H-1,3-benzodioxol-5-yl)oxy]-N-[(2S)-2-hydroxy-4-{2-[4-(trifluoromethoxy)phenoxy]acetamido}bicyclo[2.2.2]octan-1-yl]acetamide (Compound 780)

The title compound was prepared using the methodologies described above. ¹H NMR (400 MHz, DMSO-*d₆*) *δ* ppm 7.48 (s, 1H), 7.34 - 7.27 (m, 3H), 7.23 (s, 1H), 7.12 (d, J = 2.5 Hz, 1H), 7.02 - 6.97 (m, 2H), 6.74 (dd, J = 8.9, 2.6 Hz, 1H), 5.10 (s, 1H), 4.43 (s, 2H), 4.42 (s, 2H), 4.06 - 3.99 (m, 1H), 2.32 - 2.23 (m, 1H), 2.16 - 2.05 (m, 1H), 1.98 - 1.71 (m, 8H); MS (APCI⁺) *m*/*z* 589 (M+H)⁺.

### Example 682: 2-(4-fluorophenoxy)-N-[(2S)-2-hydroxy-4-{2-[4-(trifluoromethoxy)phenoxy]acetamido}bicyclo[2.2.2]octan-1-yl]acetamide (Compound 781)

The title compound was prepared using the methodologies described above. ¹H NMR (400 MHz, DMSO-*d₆*) *δ* ppm 7.48 (s, 1H), 7.32 - 7.26 (m, 2H), 7.21 (s, 1H), 7.16 - 7.08 (m, 2H), 7.02 - 6.91 (m, 4H), 5.21 - 5.04 (m, 1H), 4.42 (s, 2H), 4.39 (s, 2H), 4.03 - 3.97 (m, 1H), 2.33 - 2.23 (m, 1H), 2.20 - 2.08 (m, 1H), 2.00 - 1.71 (m, 8H); MS (APCI⁺) *m*/*z* 527 (M+H)⁺.

### Example 683: 2-(4-chloro-3-fluorophenoxy)-N-{(2S)-4-[2-(4-ethylphenoxy)acetamido]-2-hydroxybicyclo[2.2.2]octan-1-yl}acetamide (Compound 782)

To a solution of the product of Example 679B (0.045 g, 0.082 mmol) in *N,N-*dimethylformamide (1.5 mL) was added 2-(4-chloro-3-fluorophenoxy)acetic acid (0.018 g, 0.086 mmol) and triethylamine (0.046 mL, 0.329 mmol) followed by 2-(3*H*-[1,2,3]triazolo[4,5-*b*]pyridin-3-yl)-1,1,3,3-tetramethylisouronium hexafluorophosphate(V) (HATU, 0.038 g, 0.099 mmol). This mixture was allowed to stir at ambient temperature for 16 hours. The mixture was then directly purified by preparative HPLC [Waters XBridge^{™} C18 5 µm OBD^{™} column, 30 × 100 mm, flow rate 40 mL/minute, 5-100% gradient of acetonitrile in buffer (0.1 % trifluoroacetic acid)] to give the title compound (0.03 g, 0.059 mmol, 72% yield). ¹H NMR (501 MHz, DMSO-*d*₆) *δ* ppm 7.46 (t, J = 8.9 Hz, 1H), 7.34 (s, 1H), 7.24 (s, 1H), 7.12 - 7.06 (m, 2H), 7.03 (dd, J = 11.4, 2.9 Hz, 1H), 6.83 - 6.77 (m, 3H), 5.05 (d, J = 4.4 Hz, 1H), 4.44 (s, 2H), 4.31 (s, 2H), 4.06 - 3.98 (m, 1H), 2.55 - 2.49 (m, 2H), 2.26 (ddd, J = 12.3, 9.4, 2.3 Hz, 1H), 2.09 - 2.01 (m, 1H), 1.96 - 1.72 (m, 8H), 1.12 (t, J = 7.6 Hz, 3H); MS (ESI⁺) *m*/*z* 505.1 (M+H)⁺.

### Example 684: 2-(4-chlorophenoxy)-N-[(2S)-2-hydroxy-4-{2-[4-(trifluoromethoxy)phenoxy]acetamido}bicyclo[2.2.2]octan-1-yl]acetamide (Compound 783)

The title compound was prepared using the methodologies described above. ¹H NMR (400 MHz, DMSO-*d₆*) *δ* ppm 7.48 (s, 1H), 7.37 - 7.27 (m, 4H), 7.23 (s, 1H), 7.02 - 6.93 (m, 4H), 5.18 - 5.08 (m, 1H), 4.42 (s, 4H), 4.05 - 3.97 (m, 1H), 2.32 - 2.24 (m, 1H), 2.17 - 2.07 (m, 1H), 1.99 - 1.71 (m, 8H); MS (APCI⁺) *m*/*z* 543 (M+H)⁺.

### Example 685: 2-(3,4-difluorophenoxy)-N-[(2S)-2-hydroxy-4-{2-[4-(trifluoromethoxy)phenoxy]acetamido}bicyclo[2.2.2]octan-1-yl]acetamide (Compound 784)

The title compound was prepared using the methodologies described above. ¹H NMR (501 MHz, DMSO-*d₆*) *δ* ppm 7.48 (s, 1H), 7.36 (dt, J = 10.7, 9.4 Hz, 1H), 7.31 - 7.27 (m, 2H), 7.23 (s, 1H), 7.07 (ddd, J = 12.6, 6.7, 3.0 Hz, 1H), 7.02 - 6.97 (m, 2H), 6.79 - 6.75 (m, 1H), 5.09 (d, J = 4.3 Hz, 1H), 4.43 (s, 2H), 4.42 (s, 2H), 4.06 - 4.00 (m, 1H), 2.31 - 2.24 (m, 1H), 2.13 - 2.05 (m, 1H), 1.98 - 1.73 (m, 8H); MS (APCI⁺) *m*/*z* 545 (M+H)⁺.

### Example 686: N,N'-[(2S)-2-hydroxybicyclo[2.2.2]octane-1,4-diyl]bis{2-[4-(trifluoromethoxy)phenoxy]acetamide} (Compound 785)

The title compound was prepared using the methodologies described above. ¹H NMR (400 MHz, DMSO-*d₆*) *δ* ppm 7.48 (s, 1H), 7.34 - 7.24 (m, 5H), 7.05 - 6.96 (m, 4H), 5.11 (d, J = 4.1 Hz, 1H), 4.45 (s, 2H), 4.42 (s, 2H), 4.05 - 3.99 (m, 1H), 2.31 - 2.22 (m, 1H), 2.16 - 2.06 (m, 1H), 1.98 - 1.73 (m, 8H); MS (APCI⁺) *m*/*z* 593 (M+H)⁺.

### Example 687: 2-[(2,2-difluoro-2H-1,3-benzodioxol-5-yl)oxy]-N-[(3S)-3-hydroxy-4-{2-[4-(trifluoromethoxy)phenoxy]acetamido}bicyclo[2.2.2]octan-1-yl]acetamide (Compound 786)

The title compound was prepared using the methodologies described above. ¹H NMR (400 MHz, DMSO-*d₆*) *δ* ppm 7.45 (s, 1H), 7.34 - 7.27 (m, 3H), 7.26 (s, 1H), 7.09 (d, J = 2.5 Hz, 1H), 7.05 - 6.99 (m, 2H), 6.72 (dd, J = 8.9, 2.6 Hz, 1H), 5.11 (d, J = 4.4 Hz, 1H), 4.45 (s, 2H), 4.39 (s, 2H), 4.07 - 3.99 (m, 1H), 2.35 - 2.22 (m, 1H), 2.18 - 2.06 (m, 1H), 1.99 - 1.70 (m, 8H); MS (ESI⁺) *m*/*z* 589 (M+H)⁺.

### Example 688: 2-(4-chlorophenoxy)-N-{(2S)-2-hydroxy-4-[2-(4-methylphenoxy)acetamido]bicyclo[2.2.2]octan-1-yl}acetamide (Compound 787)

The title compound was prepared using the methodologies described above. ¹H NMR (400 MHz, DMSO-*d₆*) *δ* ppm 7.37 - 7.31 (m, 3H), 7.22 (s, 1H), 7.10 - 7.05 (m, 2H), 6.98 - 6.93 (m, 2H), 6.82 - 6.77 (m, 2H), 5.10 (d, J = 4.4 Hz, 1H), 4.42 (s, 2H), 4.32 (s, 2H), 4.04 - 3.96 (m, 1H), 2.33 - 2.24 (m, 1H), 2.22 (s, 3H), 2.16 - 2.05 (m, 1H), 1.98 - 1.73 (m, 8H); MS (ESI⁺) *m*/*z* 473 (M+H)⁺.

### Example 689: 2-(3,4-difluorophenoxy)-N-{(2S)-2-hydroxy-4-[2-(4-methylphenoxy)acetamido]bicyclo[2.2.2]octan-1-yl}acetamide (Compound 788)

The title compound was prepared using the methodologies described above. ¹H NMR (501 MHz, DMSO-*d₆*) *δ* ppm 7.39 - 7.32 (m, 2H), 7.22 (s, 1H), 7.10 - 7.04 (m, 3H), 6.82 - 6.75 (m, 3H), 5.09 (d, J = 4.4 Hz, 1H), 4.43 (s, 2H), 4.32 (s, 2H), 4.06 - 4.00 (m, 1H), 2.28 (ddd, J = 12.4, 9.6, 2.6 Hz, 1H), 2.22 (s, 3H), 2.13 - 2.05 (m, 1H), 1.99 - 1.71 (m, 8H); MS (ESI⁺) *m*/*z* 475 (M+H)⁺.

### Example 690: 2-(4-chloro-3-fluorophenoxy)-N-(3-{2-[4-chloro-2-(2-hydroxyacetamido)phenoxy]acetamido}bicyclo[1.1.1]pentan-1-yl)acetamide (Compound 789)

To a solution of Example 701 (0.023 g, 0.042 mmol) in CH₂Cl₂ (0.42 mL) was added boron tribromide (0.085 mL, 0.085 mmol, 1 M in CH₂Cl₂) in an ice bath. The reaction mixture was allowed to warm to ambient temperature overnight. The mixture was diluted with water (1 mL), dichloromethane (2 mL), ethyl acetate (2 mL), and methanol (3 mL), and the layers were separated. The aqueous layer was extracted with methanol and ethyl acetate (2 × 10 mL, 1:1), the layers were separated, and the combined organic layers were dried (Na₂SO₄), filtered, and concentrated. The residue was diluted with *N*,*N*-dimethylformamide/water (1.2 mL, 3:1) and purified by preparative HPLC [Waters XBridge^{™} C18 5 µm OBD column, 30 × 100 mm, flow rate 40 mL/minute, 5-100% gradient of acetonitrile in buffer (0.025 M aqueous ammonium bicarbonate, adjusted to pH 10 with ammonium hydroxide)] to yield the title compound (0.006 g, 0.011 mmol, 26% yield). ¹H NMR (400 MHz, DMSO-*d*₆) *δ* ppm 9.38 (s, 1H), 8.72 (d, J = 4.5 Hz, 2H), 8.24 (d, J = 2.6 Hz, 1H), 7.49 (t, J = 8.9 Hz, 1H), 7.17 - 7.02 (m, 2H), 6.97 (d, J = 8.8 Hz, 1H), 6.85 (d, J = 6.8 Hz, 1H), 6.10 - 6.00 (m, 1H), 4.59 (s, 2H), 4.47 (s, 2H), 4.03 (d, J = 5.5 Hz, 2H), 2.25 (s, 6H); MS (ESI⁺) *m*/*z* 526 (M+H)⁺.

### Example 691: 2-(4-chloro-3-fluorophenoxy)-N-[(2S)-4-{2-[3-(difluoromethyl)-4-fluorophenoxy]acetamido}-2-hydroxybicyclo[2.2.2]octan-1-yl]acetamide (Compound 790)

The methodologies described in Example 569 substituting 2-(4-chloro-3-fluorophenoxy)acetic acid for 1-(4-chlorophenyl)cyclopropanecarboxylic acid and substituting Example 675E for Example 9B gave the title compound. ¹1H NMR (501 MHz, DMSO-*d*₆) *δ* ppm 7.51 - 7.43 (m, 2H), 7.33 - 7.22 (m, 2H), 7.15 - 7.07 (m, 2H), 7.05 - 7.00 (m, 1H), 6.80 (ddd, J = 9.0, 2.9, 1.2 Hz, 1H), 5.06 (d, J = 4.3 Hz, 1H), 4.44 (s, 2H), 4.41 (s, 2H), 4.02 (dd, J = 9.3, 4.3 Hz, 1H), 2.24 (ddd, J = 12.2, 9.4, 2.3 Hz, 1H), 2.11 - 1.99 (m, 1H), 1.96 - 1.70 (m, 8H); MS (ESI⁺) *m*/*z* 545 (M+H)⁺.

### Example 692: 2-(4-chlorophenyl)-2-(2-hydroxyacetamido)ethyl {3-[2-(4-chloro-3-fluorophenoxy)acetamido]bicyclo[1.1.1]pentan-1-yl}carbamate (Compound 791)

### Example 692A: 2-((tert-butoxycarbonyl)amino)-2-(4-chlorophenyl)ethyl (3-(2-(4-chloro-3-fluorophenoxy)acetamido)bicyclo[1.1.1]pentan-1-yl)carbamate

To a solution of *tert-*butyl (1-(4-chlorophenyl)-2-hydroxyethyl)carbamate (0.030 g, 0.11 mmol), Example 9B (0.030 g, 0.11 mmol), and 4-nitrophenyl carbonochloridate (0.032 g, 0.16 mmol) in acetonitrile (1.5 mL) was added Hunig's Base (0.037 mL, 0.21 mmol) and pyridine (0.04 mL, 0.5 mmol). The reaction mixture was stirred for 30 minutes at 115 °C in a Biotage^{®} Initiator microwave reactor and then concentrated. The residue was purified by preparative HPLC [Waters XBridge^{™} C18 5 µm OBD column, 30 × 100 mm, flow rate 40 mL/minute, 5-100% gradient of acetonitrile in buffer (0.025 M aqueous ammonium bicarbonate, adjusted to pH 10 with ammonium hydroxide)] to give the title compound (0.013 g, 0.022 mmol, 21% yield). ¹H NMR (400 MHz, DMSO-*d*₆) *δ* ppm 8.68 (s, 1H), 7.93 (s, 1H), 7.49 (t, J = 8.9 Hz, 1H), 7.42 - 7.29 (m, 4H), 7.06 (dd, J = 11.5, 2.7 Hz, 1H), 6.84 (dd, J = 8.7, 2.6 Hz, 1H), 4.72 (s, 1H), 4.46 (s, 2H), 3.99 (d, J = 7.5 Hz, 2H), 2.11 (s, 6H), 1.35 (s, 9H); MS (ESI⁺) *m*/*z* 482 (M-Boc+H)⁺.

### Example 692B: 2-amino-2-(4-chlorophenyl)ethyl (3-(2-(4-chloro-3-fluorophenoxy)acetamido)bicyclo[1.1.1]pentan-1-yl)carbamate

The methodologies described in Example 596D substituting Example 692A for Example 596C with shorter reaction time (1.5 hours) gave the title compound. MS (ESI⁺) *m*/*z* 482 (M+H)⁺.

### Example 692C: 2-(2-((tert-butyldimethylsilyl)oxy)acetamido)-2-(4-chlorophenyl)ethyl (3-(2-(4-chloro-3-fluorophenoxy)acetamido)bicyclo[1.1.1]pentan-1-yl)carbamate

The methodologies described in Example 569 substituting potassium 2-((*tert*butyldimethylsilyl)oxy)acetate for 1-(4-chlorophenyl)cyclopropanecarboxylic acid, substituting Example 692B for Example 9B, and without purification gave the title compound. MS (ESI⁺) *m*/*z* 654 (M+H)⁺.

### Example 692D: 2-(4-chlorophenyl)-2-(2-hydroxyacetamido)ethyl {3-[2-(4-chloro-3-fluorophenoxy)acetamido]bicyclo[1.1.1]pentan-1-yl}carbamate

To a solution of Example 692C (0.021 g, 0.032 mmol) in methanol (0.1 mL) was added acetyl chloride (0.3 µL, 0.005 mmol). The reaction mixture was stirred at ambient temperature for 2 hours, then was diluted with CH₂Cl₂ (5 mL), and was neutralized with NaHCO₃ (saturated aqueous, 1 mL). The organic layer was separated and then concentrated in vacuo. The residue was diluted with *N*,*N*-dimethylformamide (1 mL), and purified by preparative HPLC [Waters XBridge^{™} C18 5 µm OBD column, 30 × 100 mm, flow rate 40 mL/minute, 5-100% gradient of acetonitrile in buffer (0.025 M aqueous ammonium bicarbonate, adjusted to pH 10 with ammonium hydroxide)] to give the title compound (0.006 g, 0.011mmol, 34% yield). ¹H NMR (400 MHz, DMSO-*d*₆) *δ* ppm 8.64 (s, 1H), 8.28 (d, J = 8.5 Hz, 1H), 7.92 (s, 1H), 7.46 (t, J = 8.9 Hz, 1H), 7.36 (s, 4H), 7.03 (dd, J = 11.4, 2.8 Hz, 1H), 6.81 (dt, J = 8.9, 1.7 Hz, 1H), 5.51 (t, J = 5.8 Hz, 1H), 5.06 (s, 1H), 4.43 (s, 2H), 4.22 - 4.04 (m, 2H), 3.80 (d, J = 4.9 Hz, 2H), 2.12 (s, 6H); MS (ESI⁺) *m*/*z* 540 (M+H)⁺.

### Example 693: 2-(4-chloro-3-fluorophenoxy)-N-[(2S)-2-hydroxy-4-{2-[(6-methoxypyridin-2-yl)oxy]acetamido}bicyclo[2.2.2]octan-1-yl]acetamide (Compound 792)

The title compound was synthesized using the procedure described in Example 538 substituting 6-methoxypyridin-2-ol for 2-methoxypyrimidin-5-ol. ¹H NMR (400 MHz, DMSO-*d*₆) *δ* ppm 7.81 (d, J = 3.1 Hz, 1H), 7.53 7.42 (m, 2H), 7.38 (dd, J = 9.0, 3.2 Hz, 1H), 7.26 (s, 1H), 7.05 (dd, J = 11.4, 2.9 Hz, 1H), 6.83 (ddd, J = 9.0, 2.9, 1.2 Hz, 1H), 6.76 (d, J = 9.0 Hz, 1H), 4.47 (s, 2H), 4.38 (s, 2H), 4.04 (dd, J = 9.6, 3.1 Hz, 1H), 3.79 (s, 3H), 2.28 (ddd, J = 12.1, 9.0, 1.9 Hz, 1H), 2.07 (ddd, J = 12.2, 10.3, 4.6 Hz, 1H), 1.98 1.89 (m, 2H), 1.94 1.73 (m, 6H); MS (ESI⁺) *m*/*z* 508.2 (M+H)⁺.

### Example 694: 2-(4-chloro-3-fluorophenoxy)-N-{(2S)-4-[2-(4-chloro-2-methoxyphenoxy)acetamido]-2-hydroxybicyclo[2.2.2]octan-1-yl}acetamide (Compound 793)

The title compound was synthesized using the procedure described in Example 538 substituting 4-chloro-2-methoxyphenol for 2-methoxypyrimidin-5-ol. ¹H NMR (400 MHz, DMSO-*d*₆) *δ* ppm 7.48 (t, J = 8.9 Hz, 1H), 7.33 (s, 1H), 7.26 (s, 1H), 7.09 - 6.99 (m, 2H), 7.02 - 6.86 (m, 2H), 6.83 (ddd, J = 9.0, 2.9, 1.2 Hz, 1H), 5.01 (s, 1H), 4.42 (d, J = 37.2 Hz, 4H), 4.08 - 4.00 (m, 1H), 3.80 (s, 3H), 2.27 (ddd, J = 12.5, 9.4, 2.4 Hz, 1H), 2.07 (ddd, J = 11.9, 10.0, 5.2 Hz, 1H), 1.96 - 1.71 (m, 8H); MS (ESI⁺) *m*/*z* 541.1 (M+H)⁺.

### Example 698: 2-(4-chloro-3-fluorophenoxy)-N-[3-(2-{4-chloro-2-[(2-hydroxyethyl)amino]phenoxy}acetamido)bicyclo[1.1.1]pentan-1-yl]acetamide (Compound 797)

### Example 698A: 2-(2-((2-((tert-butyldimethylsilyl)oxy)ethyl)amino)-4-chlorophenoxy)acetic acid

The methodologies described in Example 596A with the following modifications gave the title compound: (1) substituting (2-bromoethoxy)(*tert*-butyl)dimethylsilane for *tert-*butyl bromoacetate, (2) substituting Example 701B for 6-hydroxy-2-methylisoindolin-1-one, (3) with methanol instead of *N,N*-dimethylformamide, and (4) at 65 °C. ¹H NMR (400 MHz, DMSO-*d*₆) *δ* ppm 6.56 (d, J = 8.4 Hz, 1H), 6.47 (d, J = 2.5 Hz, 1H), 6.40 (dd, J = 8.5, 2.5 Hz, 1H), 5.91 (t, J = 6.1 Hz, 1H), 4.04 (s, 2H), 3.12 (q, J = 6.1, Hz, 4H), 0.83 (s, 9H), 0.01 (s, 6H); MS (ESI⁺) *m*/*z* 359 (M+H)⁺.

### Example 698B: 2-(2-((2-((tert-butyldimethylsilyl)oxy)ethyl)amino)-4-chlorophenoxy)-N-(3-(2-(4-chloro-3-fluorophenoxy)acetamido)bicyclo[1.1.1]pentan-1-yl)acetamide

The methodologies described in Example 569 substituting Example 698A for 1-(4-chlorophenyl)cyclopropanecarboxylic acid gave the title compound. MS (ESI⁺) *m*/*z* 626 (M+H)⁺.

### Example 698C: 2-(4-chloro-3-fluorophenoxy)-N-[3-(2-{4-chloro-2-[(2-hydroxyethyl)amino]phenoxy}acetamido)bicyclo[1.1.1]pentan-1-yl]acetamide

To a solution of Example 698B (0.0084 g, 0.013 mmol) in tetrahydrofuran (0.08 mL) was added TBAF (tetra-*N*-butylammonium fluoride) (0.01 mL, 0.01 mmol). This reaction mixture was allowed to stir at ambient temperature for 1 hour and then was concentrated. The residue was dissolved in heptanes and filtered through a plug of silica and diatomaceous earth. Then the silica plug was rinsed with dichloromethane to collect the impure title compound which was further purified by normal phase column chromatography (ethyl acetate/ heptanes, 0-100%) to give the title compound (0.0034 g, 0.0066 mmol, 51% yield). ¹H NMR (501 MHz, DMSO-*d*₆) *δ* ppm 8.71 (d, J = 9.3 Hz, 2H), 7.50 (t, J = 8.9 Hz, 1H), 7.07 (d, J = 11.3 Hz, 1H), 6.85 (d, J = 9.9 Hz, 1H), 6.72 (d, J = 8.6 Hz, 1H), 6.56 (s, 1H), 6.50 (d, J = 8.7 Hz, 1H), 5.50 (s, 1H), 4.76 (t, J = 5.5 Hz, 1H), 4.48 (s, 2H), 4.41 (s, 2H), 3.56 (q, J = 5.8 Hz, 2H), 3.16 (q, J = 5.9 Hz, 2H), 2.29 (s, 6H); MS (ESI⁺) *m*/*z* 512 (M+H)⁺.

### Example 699: 2-[(2,2-difluoro-2H-1,3-benzodioxol-5-yl)oxy]-N-{(3S)-4-[2-(4-fluorophenoxy)acetamido]-3-hydroxybicyclo[2.2.2]octan-1-yl}acetamide (Compound 798)

The title compound was prepared using the methodologies described above. ¹H NMR (400 MHz, DMSO-*d₆*) *δ* ppm 7.45 (s, 1H), 7.31 (d, J = 8.9 Hz, 1H), 7.20 (s, 1H), 7.16 - 7.07 (m, 3H), 6.98 - 6.92 (m, 2H), 6.72 (dd, J = 8.9, 2.6 Hz, 1H), 5.13 (d, J = 4.3 Hz, 1H), 4.39 (s, 2H), 4.39 (s, 2H), 4.04 - 3.97 (m, 1H), 2.28 (ddd, J = 12.5, 9.4, 2.5 Hz, 1H), 2.20 - 2.10 (m, 1H), 1.98 - 1.73 (m, 8H); MS (ESI⁺) *m*/*z* 523 (M+H)⁺.

### Example 700: 2-(4-chlorophenoxy)-N-[(2S)-4-{2-[(2,2-difluoro-2H-1,3-benzodioxol-5-yl)oxy]acetamido}-2-hydroxybicyclo[2.2.2]octan-1-yl]acetamide (Compound 799)

The title compound was prepared using the methodologies described above. ¹H NMR (400 MHz, DMSO-*d₆*) *δ* ppm 7.45 (s, 1H), 7.36 - 7.29 (m, 3H), 7.23 (s, 1H), 7.09 (d, J = 2.6 Hz, 1H), 6.98 - 6.93 (m, 2H), 6.72 (dd, J = 8.9, 2.6 Hz, 1H), 5.11 (d, J = 3.4 Hz, 1H), 4.42 (s, 2H), 4.39 (s, 2H), 4.05 - 3.98 (m, 1H), 2.33 - 2.22 (m, 1H), 2.17 - 2.05 (m, 1H), 1.98 - 1.72 (m, 8H); MS (ESI⁺) *m*/*z* 539 (M+H)⁺.

### Example 701: 2-(4-chloro-3-fluorophenoxy)-N-(3-{2-[4-chloro-2-(2-methoxyacetamido)phenoxy]acetamido}bicyclo[1.1.1]pentan-1-yl)acetamide (Compound 800)

### Example 701A: tert-butyl 2-(4-chloro-2-nitrophenoxy)acetate

The methodologies described in Example 596A substituting 4-chloro-2-nitrophenol for 6-hydroxy-2-methylisoindolin-1-one gave the title compound. ¹H NMR (400 MHz, DMSO-*d*₆) *δ* ppm 8.11 - 7.90 (m, 1H), 7.71 (dd, J = 9.0, 2.7 Hz, 1H), 7.31 (d, J = 9.1 Hz, 1H), 4.92 (s, 2H), 1.40 (s, 9H).

### Example 701B: tert-butyl 2-(2-amino-4-chlorophenoxy)acetate

To a solution of Example 701A (1.304 g, 4.53 mmol) in acetone (15 mL) and water (3 mL) was added ammonium chloride (3.64 g, 68.0 mmol), followed by zinc dust (4.45 g, 68.0 mmol). This reaction mixture was allowed to stir at ambient temperature for 1.5 hours and then was filtered through diatomaceous earth with ethyl acetate. The filtrate was concentrated under reduced pressure to give the title compound (1.14 g, 4.44 mmol, 97% yield) which was carried forward without further purification. ¹H NMR (400 MHz, DMSO-*d*₆) *δ* ppm 6.73 - 6.64 (m, 2H), 6.46 (dd, J = 8.5, 2.6 Hz, 1H), 5.04 (s, 2H), 4.59 (s, 2H), 1.43 (s, 9H); MS (ESI⁺) *m*/*z* 202 (M-C(O)OC(CH₃)₃+H)⁺.

### Example 701C: tert-butyl 2-(4-chloro-2-(2-methoxyacetamido)phenoxy)acetate

To a solution of Example 701B (0.070 g, 0.27 mmol) in dichloromethane (1.5 mL) was added triethylamine (0.05 mL, 0.33 mmol) and methoxyacetyl chloride (0.03 mL, 0.3 mmol). This mixture was allowed to stir at ambient temperature for 1 hour, was diluted with water (1mL), and extracted with dichloromethane (3 × 5 mL). The combined organic layers were dried (Na₂SO₄), filtered, and concentrated to give the title compound (0.070 g, 0.27 mmol, quantitative yield), which was carried forward without further purification. ¹H NMR (501 MHz, DMSO-*d*₆) *δ* ppm 9.10 (s, 1H), 8.27 (d, J = 2.6 Hz, 1H), 7.12 (dd, J = 8.8, 2.6 Hz, 1H), 7.06 (d, J = 8.8 Hz, 1H), 4.80 (s, 2H), 4.06 (s, 2H), 3.42 (s, 3H), 1.42 (s, 9H); MS (ESI⁺) *m*/*z* 274 (M-C(O)OC(CH₃)₃+H)⁺.

### Example 701D: 2-(4-chloro-2-(2-methoxyacetamido)phenoxy)acetic acid

The methodologies described in Example 652B substituting Example 701C for Example 652A gave the title compound. MS (ESI⁺) *m*/*z* 274 (M+H)⁺.

### Example 701E: 2-(4-chloro-3-fluorophenoxy)-N-(3-{2-[4-chloro-2-(2-methoxyacetamido)phenoxy]acetamido}bicyclo[1.1.1]pentan-1-yl)acetamide

The methodologies described in Example 569 Example 701D for 1-(4-chlorophenyl)cyclopropanecarboxylic acid gave the title compound. ¹H NMR (400 MHz, DMSO-*d*₆) *δ* ppm 9.32 (s, 1H), 8.79 (s, 1H), 8.73 (s, 1H), 8.11 (d, J = 2.7 Hz, 1H), 7.49 (t, J = 8.9 Hz, 1H), 7.14 (dd, J = 8.8, 2.6 Hz, 1H), 7.07 (dd, J = 11.5, 2.8 Hz, 1H), 7.00 (d, J = 8.8 Hz, 1H), 6.85 (d, J = 8.9 Hz, 1H), 4.56 (s, 2H), 4.47 (d, J = 5.0 Hz, 2H), 4.05 (s, 2H), 3.78 (s, 3H), 2.26 (s, 6H); MS (ESI⁺) *m*/*z* 540 (M+H)⁺.

### Example 705: 2-[(2,2-difluoro-2H-1,3-benzodioxol-5-yl)oxy]-N-{(3S)-4-[2-(3,4-difluorophenoxy)acetamido]-3-hydroxybicyclo[2.2.2]octan-1-yl}acetamide (Compound 804)

The title compound was prepared using the methodologies described above. ¹H NMR (400 MHz, DMSO-*d₆*) *δ* ppm 7.45 (s, 1H), 7.40 - 7.28 (m, 2H), 7.23 (s, 1H), 7.09 (d, J = 2.6 Hz, 1H), 7.09 - 7.03 (m, 1H), 6.81 - 6.75 (m, 1H), 6.72 (dd, J = 8.9, 2.6 Hz, 1H), 5.09 (d, J = 4.0 Hz, 1H), 4.43 (s, 2H), 4.40 (s, 2H), 4.07 - 3.99 (m, 1H), 2.34 - 2.22 (m, 1H), 2.16 - 2.04 (m, 1H), 1.98 - 1.72 (m, 8H); MS (APCI⁺) *m*/*z* 541 (M+H)⁺.

### Example 706: 2-(4-chloro-3-fluorophenoxy)-N-(3-{2-[2-(2-hydroxyethoxy)phenoxy]acetamido}bicyclo[1.1.1]pentan-1-yl)acetamide (Compound 805)

### Example 706A: di-tert-butyl 2,2'-(1,2-phenylenebis(oxy))diacetate

The methodologies described in Example 596A substituting pyrocatechol for 6-hydroxy-2-methylisoindolin-1-one gave the title compound. ¹H NMR (400 MHz, DMSO-*d*₆) *δ* ppm 6.89 (s, 4H), 4.64 (s, 4H), 1.42 (s, 18H).

### Example 706B: tert-butyl 2-(2-(2-hydroxyethoxy)phenoxy)acetate

To a cooled (0 °C) solution of Example 706A (0.073 g, 0.22 mmol) in tetrahydrofuran (2 mL) was added DIBAL-H (diisobutylaluminum hydride) (0.32 mL, 0.32 mmol, 1.0 M in toluene). This reaction mixture was allowed to stir and gradually warm to ambient temperature over 3 hours. Then to the reaction mixture was added more DIBAL (0.3 mL, 0.32 mmol, 1.0 M in toluene), and after continuing to stir for 18 hours, the reaction mixture was quenched with saturated aqueous Rochelle's salt (1 mL) and extracted with dichloromethane (3 × 10 mL). The combined organic layers were dried (Na₂SO₄), filtered, and concentrated to give the title compound (0.058 g, 0.22 mmol, quantitative yield), which was carried forward without purification. ¹H NMR (400 MHz, DMSO-*d*₆) *δ* ppm 7.02 - 6.94 (m, 1H), 6.92 - 6.82 (m, 3H), 4.80 (q, J = 5.5 Hz, 1H), 4.64 (d, J = 6.2 Hz, 2H), 3.98 (q, J = 5.2, 4.7 Hz, 2H), 3.70 (p, J = 5.0 Hz, 2H), 1.42 (d, J = 1.3 Hz, 9H).

### Example 706C: tert-butyl 2-(2-(2-((tert-butyldimethylsilyl)oxy)ethoxy)phenoxy)acetate

A mixture of Example 706B (0.059 g, 0.22 mmol) and imidazole (0.053 g, 0.77 mmol) was treated with a solution of TBS-Cl (*tert*-butyldimethylchlorosilane) (0.073 g, 0.49 mmol) in N,N-dimethylformamide (0.63 mL). The resulting homogeneous solution was stirred at ambient temperature for 4 hours and then was partitioned between brine (15 mL) and (1:1) hexanes/Et₂O (3 × 30 mL). The organic layer was cooled and washed with cold 1 N HCl (5 mL), dried (Na₂SO₄), filtered, and concentrated in vacuo to give the title compound (0.083 g, 0.22 mmol, 98% yield), which was carried forward without further purification. ¹H NMR (501 MHz, DMSO-*d*₆) *δ* ppm 7.02 - 6.79 (m, 4H), 4.61 (d, J = 22.5 Hz, 2H), 4.00 (ddd, J = 16.3, 5.6, 4.4 Hz, 2H), 3.90 (q, J = 4.8 Hz, 2H), 1.46 - 1.37 (m, 9H), 0.86 (d, J = 1.2 Hz, 9H), 0.06 (d, J = 0.9 Hz, 6H); MS (ESI⁺) *m*/*z* 327 (M-C(O)OC(CH₃)₃+H)⁺.

### Example 706D: 2-(2-(2-((tert-butyldimethylsilyl)oxy)ethoxy)phenoxy)acetic acid

The methodologies described in Example 652B substituting Example 706C for Example 652A gave the title compound. ¹H NMR (400 MHz, DMSO-*d*₆) *δ* ppm 7.02 - 6.80 (m, 4H), 3.99 (t, J = 5.0 Hz, 3H), 3.89 (dd, J = 5.6, 4.3 Hz, 3H), 0.85 (s, 9H), 0.05 (s, 6H).

### Example 706E: 2-(4-chloro-3-fluorophenoxy)-N-(3-{2-[2-(2-hydroxyethoxy)phenoxy]acetamido}bicyclo[1.1.1]pentan-1-yl)acetamide

The methodologies described in Example 569 substituting Example 706D for 1-(4-chlorophenyl)cyclopropanecarboxylic acid gave the title compound. ¹H NMR (400 MHz, DMSO-*d*₆) *δ* ppm 8.71 (s, 1H), 8.51 (s, 1H), 7.49 (t, J = 8.8 Hz, 1H), 7.13 - 6.99 (m, 2H), 6.99 - 6.80 (m, 4H), 4.89 (s, 1H), 4.47 (d, J = 5.1 Hz, 2H), 4.42 (s, 2H), 4.00 (t, J = 5.0 Hz, 2H), 3.75 - 3.70 (m, 2H), 2.27 (s, 6H) ; MS (ESI⁺) *m*/*z* 479 (M+H)⁺.

### Example 708: N-{(3S)-4-[2-(4-chloro-3-fluorophenoxy)acetamido]-3-hydroxybicyclo[2.2.2]octan-1-yl}-3-(4-chlorophenyl)-3-oxopropanamide (Compound 807)

A suspension of Example 548C (0.0550 g, 0.160 mmol) and ethyl 3-(4-chlorophenyl)-3-oxopropanoate (0.0422 g, 0.186 mmol) in toluene (0.65 mL) was stirred at 100 °C overnight, concentrated, and chromatographed on silica gel (0-5% methanol/dichloromethane) and rechromatographed on silica gel (66-80% ethyl acetate/dichloromethane) to give the title compound as a ~0.55:1 mixture of tautomers by ¹H NMR (0.0305 g, 0.058 mmol, 36.3 % yield). ¹H NMR (500 MHz, DMSO-*d*₆) *δ* ppm 7.98 - 7.91 (m, 2H), 7.80 (s, 0.55H), 7.75 (s, 1H), 7.67 - 7.62 (d, J = 8.4 Hz, 1.1H), 7.63 - 7.58 (m, 2H), 7.53 (d, J = 8.4 Hz, 1.1H), 7.51 - 7.45 (m, 1.55H), 7.29 (s, 0.55H), 7.25 (s, 1H), 7.08 - 7.02 (m, 1.55H), 6.86 - 6.78 (m, 1.55H), 5.76 (s, 0.55H), 5.12 (d, J = 4.3 Hz, 0.55H), 5.06 (d, J = 4.4 Hz, 1H), 4.50 - 4.42 (m, 3.10H), 4.16 - 3.97 (m, 1.55H), 3.81 (s, 2H), 2.31 (t, J = 11.4 Hz, 0.55H), 2.24 (td, J = 11.3, 10.5, 5.3 Hz, 1H), 2.13 - 1.67 (m, 14H); MS (ESI⁺) *m*/*z* 523.2 (M+H)⁺.

### Example 711: (3S)-3-amino-N-{3-[2-(4-chloro-3-fluorophenoxy)acetamido]bicyclo[1.1.1]pentan-1-yl}-4-phenylbutanamide (Compound 810)

The methodologies described in Example 569 substituting (S)-3-amino-4-phenylbutanoic acid for 1-(4-chlorophenyl)cyclopropanecarboxylic acid gave the title compound. ¹H NMR (400 MHz, DMSO-*d*₆) *δ* ppm 8.70 (s, 2H), 7.89 (s, 2H), 7.49 (t, J = 8.9 Hz, 1H), 7.35 (dd, J = 8.1, 6.6 Hz, 2H), 7.28 (t, J = 7.3 Hz, 1H), 7.24 - 7.21 (m, 2H), 7.06 (dd, J = 11.4, 2.8 Hz, 1H), 6.88 - 6.80 (m, 1H), 4.47 (s, 2H), 2.96 (dd, J = 13.6, 5.6 Hz, 2H), 2.74 (d, J = 8.6 Hz, 1H), 2.31 (d, J = 7.7 Hz, 2H), 2.21 (s, 6H); MS (ESI⁺) *m*/*z* 446 (M+H)⁺.

### Example 715: 2-(4-chloro-3-fluorophenoxy)-N-{(2S)-4-[2-(4-fluoro-3-methylphenoxy)acetamido]-2-hydroxybicyclo[2.2.2]octan-1-yl}acetamide (Compound 814)

The title compound was prepared using the methodologies described above. ¹H NMR (501 MHz, DMSO-*d₆*) *δ* ppm 7.48 (t, J = 8.9 Hz, 1H), 7.37 (s, 1H), 7.26 (s, 1H), 7.07 - 7.02 (m, 2H), 6.85 - 6.81 (m, 2H), 6.73 (dt, J = 8.9, 3.6 Hz, 1H), 5.08 (s, 1H), 4.50 - 4.42 (m, 2H), 4.33 (s, 2H), 4.06 - 4.00 (m, 1H), 2.27 (ddd, J = 12.4, 9.5, 2.3 Hz, 1H), 2.19 (d, J = 1.9 Hz, 3H), 2.12 - 2.01 (m, 1H), 1.96 - 1.74 (m, 8H); MS (ESI⁺) *m*/*z* 509 (M+H)⁺.

### Example 716: 2-(3,4-difluorophenoxy)-N-{(2S)-4-[2-(4-fluoro-3-methylphenoxy)acetamido]-2-hydroxybicyclo[2.2.2]octan-1-yl}acetamide (Compound 815)

The title compound was prepared using the methodologies described above. ¹H NMR (400 MHz, DMSO-*d₆*) *δ* ppm 7.41 - 7.31 (m, 2H), 7.23 (s, 1H), 7.10 - 7.01 (m, 2H), 6.84 (dd, J = 6.4, 3.1 Hz, 1H), 6.80 - 6.70 (m, 2H), 5.09 (d, J = 4.2 Hz, 1H), 4.43 (s, 2H), 4.33 (s, 2H), 4.07 - 3.99 (m, 1H), 2.32 - 2.23 (m, 1H), 2.19 (d, J = 2.0 Hz, 3H), 2.13 - 2.03 (m, 1H), 1.97 - 1.72 (m, 8H); MS (ESI⁺) *m*/*z* 493 (M+H)⁺.

### Example 717: 2-(4-chlorophenoxy)-N-{(2S)-4-[2-(4-fluoro-3-methylphenoxy)acetamido]-2-hydroxybicyclo[2.2.2]octan-1-yl}acetamide (Compound 816)

The title compound was prepared using the methodologies described above. ¹H NMR (400 MHz, DMSO-*d₆*) *δ* ppm 7.37 (s, 1H), 7.36 - 7.30 (m, 2H), 7.23 (s, 1H), 7.04 (t, J = 9.2 Hz, 1H), 6.98 - 6.93 (m, 2H), 6.84 (ddd, J = 6.4, 3.2, 0.8 Hz, 1H), 6.73 (dt, J = 9.0, 3.6 Hz, 1H), 5.11 (s, 1H), 4.46 - 4.37 (m, 2H), 4.33 (s, 2H), 4.04 - 3.98 (m, 1H), 2.28 (ddd, J = 12.3, 9.4, 2.4 Hz, 1H), 2.19 (d, J = 1.9 Hz, 3H), 2.15 - 2.08 (m, 1H), 1.98 - 1.71 (m, 8H); MS (ESI⁺) *m*/*z* 491 (M+H)⁺.

### Example 718: 2-(4-fluoro-3-methylphenoxy)-N-{(3S)-4-[2-(4-fluorophenoxy)acetamido]-3-hydroxybicyclo[2.2.2]octan-1-yl}acetamide (Compound 817)

The title compound was prepared using the methodologies described above. ¹H NMR (501 MHz, DMSO-*d₆*) *δ* ppm 7.37 (s, 1H), 7.21 (s, 1H), 7.15 - 7.09 (m, 2H), 7.04 (t, J = 9.1 Hz, 1H), 6.98 - 6.92 (m, 2H), 6.86 - 6.82 (m, 1H), 6.73 (dt, J = 8.9, 3.6 Hz, 1H), 5.13 (s, 1H), 4.43 - 4.35 (m, 2H), 4.33 (s, 2H), 4.02 - 3.97 (m, 1H), 2.28 (ddd, J = 12.6, 9.5, 2.8 Hz, 1H), 2.20 - 2.18 (m, 3H), 2.16 - 2.11 (m, 1H), 1.98 - 1.74 (m, 8H); MS (ESI⁺) *m*/*z* 475 (M+H)⁺.

### Example 719: 2-(3,4-difluorophenoxy)-N-[(2S)-4-{2-[4-fluoro-3-(methanesulfonyl)phenoxy]acetamido}-2-hydroxybicyclo[2.2.2]octan-1-yl]acetamide (Compound 818)

### Example 719A: tert-butyl 2-(4-fluoro-3-(methylsulfonyl)phenoxy)acetate

The methodologies described in Example 596A substituting 4-fluoro-3-(methylsulfonyl)phenol for 6-hydroxy-2-methylisoindolin-1-one gave the title compound. ¹H NMR (400 MHz, DMSO-*d*₆) *δ* ppm 7.48 (t, J = 9.3 Hz, 1H), 7.39 - 7.30 (m, 1H), 7.25 (dd, J = 5.5, 3.2 Hz, 1H), 4.77 (s, 2H), 3.33 - 3.31 (m, 3H), 1.43 (s, 9H).

### Example 719B: 2-(4-fluoro-3-(methylsulfonyl)phenoxy)acetic acid

The methodologies described in Example 652B substituting Example 719A for Example 652A gave the title compound. ¹H NMR (400 MHz, DMSO-*d*₆) *δ* ppm 8.28 (s, 1H), 7.42 (t, J = 9.2 Hz, 1H), 7.31 - 7.10 (m, 2H), 4.45 (s, 2H), 3.30 (s, 3H).

### Example 719C: (S)-tert-butyl (4-(2-(4-fluoro-3-(methylsulfonyl)phenoxy)acetamido)-2-hydroxybicyclo[2.2.2]octan-1-yl)carbamate

The methodologies described in Example 569 with substituting Example 445B for Example 9B and substituting Example 719B for 1-(4-chlorophenyl)cyclopropanecarboxylic acid gave the title compound. MS (ESI⁺) *m*/*z* 487 (M+H)⁺.

### Example 719D: (S)-N-(4-amino-3-hydroxybicyclo[2.2.2]octan-1-yl)-2-(4-fluoro-3-(methylsulfonyl)phenoxy)acetamide

The methodologies described in Example 596D substituting Example 719C for Example 596C gave the title compound. MS (ESI⁺) *m*/*z* 387 (M+H)⁺.

### Example 719E: 2-(3,4-difluorophenoxy)-N-[(2S)-4-{2-[4-fluoro-3-(methanesulfonyl)phenoxy]acetamido}-2-hydroxybicyclo[2.2.2]octan-1-yl]acetamide

The methodologies described in Example 569 substituting 2-(3,4-difluorophenoxy)acetic acid for 1-(4-chlorophenyl)cyclopropanecarboxylic acid and substituting Example 719D for Example 9B gave the title compound. ¹H NMR (501 MHz, DMSO-*d*₆) *δ* ppm 7.59 (s, 1H), 7.47 (t, J = 9.2 Hz, 1H), 7.40 - 7.33 (m, 1H), 7.33 - 7.27 (m, 2H), 7.23 (s, 1H), 7.07 (ddd, J = 12.6, 6.7, 3.1 Hz, 1H), 6.77 (ddt, J = 8.8, 4.9, 2.4 Hz, 1H), 5.10 (s, 1H), 4.48 (s, 2H), 4.43 (s, 2H), 4.03 (d, J = 9.1 Hz, 1H), 3.31 (s, 3H), 2.31 - 2.23 (m, 1H), 2.12 - 2.06 (m, 1H), 1.96 - 1.90 (m, 2H), 1.86 - 1.77 (m, 6H); MS (ESI⁺) *m*/*z* 557 (M+H)⁺.

### Example 720: 2-[4-fluoro-3-(methanesulfonyl)phenoxy]-N-{(3S)-4-[2-(4-fluorophenoxy)acetamido]-3-hydroxybicyclo[2.2.2]octan-1-yl}acetamide (Compound 819)

The methodologies described in Example 569 substituting 2-(4-fluorophenoxy)acetic acid for 1-(4-chlorophenyl)cyclopropanecarboxylic acid and substituting Example 719D for Example 9B gave the title compound. ¹H NMR (400 MHz, DMSO-*d*₆) *δ* ppm 7.59 (s, 1H), 7.47 (t, J = 9.2 Hz, 1H), 7.31 (dd, J = 8.7, 4.2 Hz, 2H), 7.20 (s, 1H), 7.12 (t, J = 8.8 Hz, 2H), 7.00 - 6.89 (m, 2H), 5.14 (s, 1H), 4.48 (s, 2H), 4.39 (s, 2H), 4.01 (s, 1H), 3.32 (s, 3H), 2.28 (t, J = 11.5 Hz, 1H), 2.15 (d, J = 11.5 Hz, 1H), 1.96 - 1.88 (m, 2H), 1.85 - 1.77 (m, 6H); MS (ESI⁺) *m*/*z* 539 (M+H)⁺.

### Example 722: N,N'-[(2S)-2-hydroxybicyclo[2.2.2]octane-1,4-diyl]bis{2-[3-fluoro-4-(trifluoromethyl)phenoxy]acetamide} (Compound 821)

To a mixture of Example 574C (75 mg, 0.153 mmol), Example 574A (38.2 mg, 0.161 mmol), and *N*-ethyl-*N*-isopropylpropan-2-amine (0.107 mL, 0.612 mmol) in *N,N-*dimethylformamide (2.5 mL), 2-(3*H*-[1,2,3]triazolo[4,5-*b*]pyridin-3-yl)-1,1,3,3-tetramethylisouronium hexafluorophosphate(V) (0.765 mL, 0.191 mmol) was added, and the mixture was stirred at ambient temperature for 1 hour. *N*,*N*-Dimethylformamide was removed under high vacuum, and the residue was purified by HPLC(Phenomenex^{®} Luna^{®} C18(2) 10 µm 100Å AXIA^{™} column (250 mm × 50 mm). A 30-100% gradient of acetonitrile (A) and 0.1% trifluoroacetic acid in water (B) was used over 25 minutes, at a flow rate of 50 mL/minute) to give 55 mg of the title compound. ¹H NMR (400 MHz, DMSO-*d*₆) *δ* ppm 7.69 (t, J = 8.8 Hz, 2H), 7.59 (s, 1H), 7.34 (s, 1H), 7.08 (td, J = 13.1, 2.4 Hz, 2H), 6.93 (td, J = 8.6, 2.3 Hz, 2H), 5.05 (d, J = 4.3 Hz, 1H), 4.56 (d, J = 13.0 Hz, 4H), 4.07 (dt, J = 7.9, 2.9 Hz, 1H), 2.33 - 2.22 (m, 1H), 2.03 (ddd, J = 12.3, 10.6, 4.9 Hz, 1H), 1.99 - 1.84 (m, 3H), 1.88 - 1.73 (m, 5H); MS (+ESI) *m*/*z* 597.1 (M+H)⁺.

### Example 723: 2-[3-fluoro-4-(trifluoromethyl)phenoxy]-N-[(2S)-4-{2-[4-fluoro-3-(trifluoromethyl)phenoxy]acetamido}-2-hydroxybicyclo[2.2.2]octan-1-yl]acetamide (Compound 822)

### Example 723A: 2-(4-fluoro-3-(trifluoromethyl)phenoxy)acetic acid

The title compound was synthesized using the same procedure described in Example 574A substituting 4-fluoro-3-(trifluoromethyl)phenol for 3-fluoro-4-(trifluoromethyl)phenol. ¹H NMR (400 MHz, DMSO-*d*₆) *δ* ppm 7.40 (t, J = 9.7 Hz, 1H), 7.30 - 7.18 (m, 2H), 4.75 (s, 2H).

### Example 723B: (S)-N-(4-amino-3-hydroxybicyclo[2.2.2]octan-1-yl)-2-(4-fluoro-3-(trifluoromethyl)phenoxy)acetamide, Trifluoroacetic Acid

The title compound was synthesized using the same procedure described in Example 674A substituting Example 723A for Example 574A. ¹H NMR (400 MHz, DMSO-*d*₆) *δ* ppm 7.78 (s, 3H), 7.70 (s, 1H), 7.44 (t, J = 9.6 Hz, 1H), 7.32 - 7.21 (m, 2H), 4.51 (d, J = 5.8 Hz, 2H), 3.85 - 3.77 (m, 1H), 2.33 (ddd, J = 12.9, 9.5, 2.9 Hz, 1H), 2.10 - 1.94 (m, 2H), 1.97 - 1.80 (m, 4H), 1.79 (q, J = 6.8, 5.9 Hz, 1H), 1.69 - 1.49 (m, 2H); MS (+ESI) *m*/*z* 377 (M+H)⁺.

### Example 723C: 2-[3-fluoro-4-(trifluoromethyl)phenoxy]-N-[(2S)-4-12-[4-fluoro-3-(trifluoromethyl)phenoxy]acetamido}-2-hydroxybicyclo[2.2.2]octan-1-yl]acetamide

The title compound was synthesized using the same procedure described in Example 674B substituting Example 723B for Example 674A and Example 574A for 2-((2,2-difluorobenzo[d][1,3]dioxol-5-yl)oxy)acetic acid. ¹H NMR (400 MHz, DMSO-*d*₆) *δ* ppm 7.69 (t, J = 8.8 Hz, 1H), 7.53 (s, 1H), 7.45 (t, J = 9.5 Hz, 1H), 7.33 (s, 1H), 7.26 (dd, J = 8.3, 4.3 Hz, 2H), 7.10 (dd, J = 13.1, 2.4 Hz, 1H), 6.94 (dd, J = 8.9, 2.3 Hz, 1H), 5.05 (s, 1H), 4.57 (s, 2H), 4.48 (s, 2H), 4.07 (d, J = 8.5 Hz, 1H), 2.32 - 2.21 (m, 1H), 2.03 (ddd, J = 12.5, 10.7, 4.9 Hz, 1H), 1.99 - 1.85 (m, 3H), 1.80 (ddd, J = 18.7, 10.4, 4.5 Hz, 5H); MS (ESI⁺) *m*/*z* 597.1 (M+H)⁺.

### Example 724: N,N'-[(2S)-2-hydroxybicyclo[2.2.2]octane-1,4-diyl]bis{2-[4-fluoro-3-(trifluoromethyl)phenoxy]acetamide} (Compound 823)

### Example 724A: (S)-(9H-fluoren-9-yl)methyl (4-(2-(4-fluoro-3-(trifluoromethyl)phenoxy)acetamido)-3-hydroxybicyclo[2.2.2]octan-1-yl)carbamate

The title compound was synthesized using the same procedure described in Example 574B substituting 723A for Example 574A. ¹H NMR (400 MHz, DMSO-*d*₆) *δ* ppm 7.84 (d, J = 7.5 Hz, 2H), 7.66 (d, J = 7.4 Hz, 2H), 7.46 - 7.20 (m, 8H), 6.99 (s, 1H), 4.47 (s, 2H), 4.16 (s, 3H), 3.99 (s, 1H), 2.17 (s, 1H), 2.01 (s, 1H), 1.87 - 1.81 (m, 3H), 1.81 - 1.63 (m, 5H); MS (ESI⁺) *m*/*z* 599.3 (M+H)⁺.

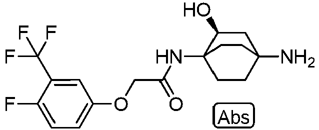

### Example 724B: (S)-N-(4-amino-2-hydroxybicyclo[2.2.2]octan-1-yl)-2-(3-fluoro-4-(trifluoromethyl)phenoxy)acetamide, Trifluoroacetic Acid

The title compound was synthesized using the same procedure described in Example 574C substituting 724A for Example 574B. ¹H NMR (400 MHz, DMSO-*d*₆) *δ* ppm 7.83 (s, 3H), 7.46 - 7.33 (m, 2H), 7.29 - 7.19 (m, 2H), 5.18 (s, 1H), 4.48 (s, 2H), 4.15 - 4.07 (m, 1H), 2.11 (ddd, J = 12.8, 9.4, 2.9 Hz, 1H), 2.07 - 1.89 (m, 3H), 1.92 - 1.75 (m, 1H), 1.68 (ddd, J = 14.2, 10.6, 6.8 Hz, 3H), 1.64 - 1.47 (m, 2H); MS (ESI⁺) *m*/*z* 377.1 (M+H)⁺.

### Example 724C: N,N'-[(2S)-2-hydroxybicyclo[2.2.2]octane-1,4-diyl]bis{2-[4-fluoro-3-(trifluoromethyl)phenoxy]acetamide}

The title compound was synthesized using the same procedure described in Example 674B substituting Example 724B for Example 674A and Example 723A for 2-((2,2-difluorobenzo[*d*][1,3]dioxol-5-yl)oxy)acetic acid. ¹H NMR (400 MHz, DMSO-*d*₆) *δ* ppm 7.54 (s, 1H), 7.50 - 7.40 (m, 2H), 7.28 (qd, J = 8.7, 8.1, 3.3 Hz, 5H), 5.08 (s, 1H), 4.50 (d, J = 12.4 Hz, 4H), 4.04 (dd, J = 9.7, 3.0 Hz, 1H), 2.27 (ddd, J = 12.4, 9.4, 2.4 Hz, 1H), 2.07 (ddd, J = 12.2, 10.5, 4.6 Hz, 1H), 1.98 - 1.87 (m, 2H), 1.90 - 1.81 (m, 1H), 1.85 - 1.73 (m, 5H); MS (ESI⁺) *m*/*z* 597.1 (M+H)⁺.

### Example 725: 2-(4-chloro-3-fluorophenoxy)-N-[(2S)-2-hydroxy-4-(2-{[6-(trifluoromethyl)pyridin-2-yl]oxy}acetamido)bicyclo[2.2.2]octan-1-yl]acetamide (Compound 824)

### Example 725A: tert-butyl 2-((6-(trifluoromethyl)pyridin-2-yl)oxy)acetate

Potassium *tert*-butoxide (1.04 g, 9.29 mmol) was added portion-wise to a solution of *tert-*butyl 2-hydroxyacetate (1.17 g, 8.85 mmol) in tetrahydrofuran (8 mL) over a period of 3 minutes. After stirring at ambient temperature for 10 minutes, a solution of 2-bromo-6-(trifluoromethyl)pyridine (1.0 g, 4.42 mmol, Matrix) in tetrahydrofuran (8 mL) was slowly added. The mixture was stirred at ambient temperature for 1 hour and then partitioned between dichloromethane (2 × 50 mL) and saturated aqueous sodium bicarbonate (100 mL). The organic layers were combined and dried over anhydrous sodium sulfate and concentrated under reduced pressure to give the title compound (1.23 g, 4.44 mmol, 100 % yield). (ESI⁺) *m*/*z* 222 (M-tBu+H)⁺.

### Example 725B: 2-((6-(trifluoromethyl)pyridin-2-yl)oxy)acetic acid

To a solution of the product of Example 725A (1.23 g, 4.44 mmol) in dichloromethane (1 mL) was added trifluoroacetic acid (4.0 mL) in one portion. The resulting reaction mixture was stirred at ambient temperature for 1 hour and then concentrated under reduced pressure to give the title compound (1.0 g, 4.5 mmol, 100 % yield). (ESI⁺) *m*/*z* 222 (M +H)⁺.

### Example 725C: (S)-tert-butyl (2-hydroxy-4-(2-((6-(trifluoromethyl)pyridin-2-yl)oxy)acetamido)bicyclo[2.2.2]octan-1-yl)carbamate

The reaction and purification conditions described in Example 81, substituting the product of Example 725B for 2-(4-chloro-3-fluorophenoxy)acetic acid, and the product of Example 445B for benzyl (4-aminobicyclo[2.1.1]hexan-1-yl)carbamate hydrochloride gave the title compound. ¹H NMR (501 MHz, DMSO-*d*₆) *δ* ppm 8.00 - 7.94 (m, 1H), 7.53 (s, 1H), 7.47 (d, J = 7.3 Hz, 1H), 7.19 (dd, J = 8.4, 0.8 Hz, 1H), 6.01 (s, 1H), 4.87 (d, J = 4.0 Hz, 1H), 4.64 (s, 2H), 3.87 - 3.80 (m, 1H), 2.21 (ddd, J = 12.6, 9.3, 2.7 Hz, 1H), 2.06 - 1.99 (m, 1H), 1.91 - 1.57 (m, 8H), 1.35 (s, 9H); MS (ESI⁺) *m*/*z* 404 (M-tBu+H)⁺.

### Example 725D: 2-(4-chloro-3-fluorophenoxy)-N-[(2S)-2-hydroxy-4-(2-{[6-(trifluoromethyl)pyridin-2-yl]oxy}acetamido)bicyclo[2.2.2]octan-1-yl]acetamide

The title compound was prepared as described in Example 83C, substituting 2-(4-chloro-3-fluorophenoxy)acetic acid for the product of Example 83B, and the product of Example 725C for the product of Example 9A. ¹H NMR (400 MHz, DMSO-*d*₆) *δ* ppm 7.97 (ddd, J = 8.4, 7.4, 0.8 Hz, 1H), 7.57 (s, 1H), 7.51 - 7.45 (m, 2H), 7.24 (s, 1H), 7.19 (d, J = 8.4 Hz, 1H), 7.05 (dd, J = 11.4, 2.9 Hz, 1H), 6.82 (ddd, J = 8.9, 2.8, 1.2 Hz, 1H), 5.05 (br s, 1H), 4.65 (s, 2H), 4.46 (s, 2H), 4.05 - 3.96 (m, 1H), 2.29 - 2.18 (m, 1H), 2.10 - 2.00 (m, 1H), 1.96 - 1.68 (m, 8H); MS (ESI⁺) *m*/*z* 546 (M+H)⁺.

### Example 726: 2-(4-chlorophenoxy)-N-[(2S)-2-hydroxy-4-(2-{[6-(trifluoromethyl)pyridin-2-yl]oxy}acetamido)bicyclo[2.2.2]octan-1-yl]acetamide (Compound 825)

The title compound was prepared using the methodologies described above. ¹H NMR (400 MHz, DMSO-*d₆*) *δ* ppm 8.00 - 7.95 (m, 1H), 7.57 (s, 1H), 7.47 (d, J = 7.3 Hz, 1H), 7.36 - 7.30 (m, 2H), 7.24 - 7.17 (m, 2H), 6.98 - 6.91 (m, 2H), 5.08 (d, J = 3.3 Hz, 1H), 4.65 (s, 2H), 4.41 (s, 2H), 4.03 - 3.93 (m, 1H), 2.24 (ddd, J = 12.6, 9.4, 2.5 Hz, 1H), 2.15 - 2.06 (m, 1H), 1.95 - 1.67 (m, 8H); MS (ESI⁺) *m*/*z* 528 (M+H)⁺.

### Example 727: 2-(4-chloro-3-fluorophenoxy)-N-[(2S)-4-{2-[4-fluoro-3-(methanesulfonyl)phenoxy]acetamido}-2-hydroxybicyclo[2.2.2]octan-1-yl]acetamide (Compound 826)

The methodologies described in Example 569 substituting 2-(4-chloro-3-fluorophenoxy)acetic acid for 1-(4-chlorophenyl)cyclopropanecarboxylic acid and substituting Example 719D for Example 9B gave the title compound. ¹H NMR (501 MHz, DMSO-*d*₆) *δ* ppm 7.59 (s, 1H), 7.47 (q, J = 8.9 Hz, 2H), 7.35 - 7.28 (m, 2H), 7.26 (s, 1H), 7.05 (dd, J = 11.4, 2.9 Hz, 1H), 6.85 - 6.79 (m, 1H), 5.08 (s, 1H), 4.48 (s, 2H), 4.46 (s, 2H), 4.04 (d, J = 9.5 Hz, 1H), 3.24 (s, 3H), 2.27 (t, J = 11.3 Hz, 1H), 2.06 (dt, J = 11.8, 6.5 Hz, 1H), 1.92 (d, J = 10.1 Hz, 2H), 1.89 - 1.73 (m, 6H); MS (ESI⁺) *m*/*z* 573 (M+H)⁺.

### Example 728: N-{3-[2-(4-chloro-3-fluorophenoxy)acetamido]bicyclo[1.1.1]pentan-1-yl}-2-(4-chlorophenoxy)-4-hydroxybutanamide (Compound 827)

To a mixture of Example 9B (0.040 g, 0.14 mmol) and 3-(4-chlorophenoxy)dihydrofuran-2(3*H*)-one (0.025 g, 0.12 mmol) in dichloromethane (0.35 mL) was added trimethylaluminum (0.070 mL, 0.14 mmol). This reaction mixture was allowed to stir at ambient temperature for 2.5 hours, was diluted with aqueous citric acid (1 mL), NaHCO₃ (saturated aqueous, 1 mL), and dichloromethane (10 mL). The layers were separated, and the aqueous layer was extracted with dichloromethane (2 × 10 mL). The combined organic fractions were dried over anhydrous Na₂SO₄, filtered, and concentrated under reduced pressure. The residue was purified by preparative HPLC [Waters XBridge^{™} C18 5 µm OBD column, 30 × 100 mm, flow rate 40 mL/minute, 5-100% gradient of acetonitrile in buffer (0.025 M aqueous ammonium bicarbonate, adjusted to pH 10 with ammonium hydroxide)] to give the title compound (0.030 g, 0.060 mmol, 43% yield). ¹H NMR (400 MHz, DMSO-*d*₆) *δ* ppm 8.72 (s, 1H), 8.69 (s, 1H), 7.49 (t, J = 8.9 Hz, 1H), 7.38 - 7.29 (m, 2H), 7.06 (dd, J = 11.4, 2.8 Hz, 1H), 6.95 - 6.88 (m, 2H), 6.84 (ddd, J = 9.0, 2.9, 1.2 Hz, 1H), 4.62 (t, J = 5.1 Hz, 1H), 4.57 (t, J = 6.4 Hz, 1H), 4.46 (s, 2H), 3.51 (q, J = 6.1 Hz, 2H), 2.21 (s, 6H), 1.89 (q, J = 6.4 Hz, 2H); MS (ESI⁺) *m*/*z* 497 (M+H)⁺.

### Example 729: 2-(4-chloro-3-fluorophenoxy)-N-[(3S)-4-{2-[4-fluoro-3-(trifluoromethyl)phenoxy]acetamido}-3-hydroxybicyclo[2.2.2]octan-1-yl]acetamide (Compound 828)

The title compound was synthesized using the same procedure described in Example 674B substituting Example 724B for Example 674A and 2-(4-chloro-3-fluorophenoxy)acetic acid for 2-((2,2-difluorobenzo[*d*][1,3]dioxol-5-yl)oxy)acetic acid. ¹H NMR (400 MHz, DMSO-*d*₆) *δ* ppm 7.54 - 7.40 (m, 3H), 7.29 (h, J = 3.5 Hz, 3H), 7.02 (dd, J = 11.4, 2.9 Hz, 1H), 6.81 (ddd, J = 9.0, 2.9, 1.2 Hz, 1H), 4.52 (s, 2H), 4.44 (s, 2H), 4.05 (dd, J = 9.6, 3.0 Hz, 1H), 1 2.28 (ddd, J = 12.4, 9.4, 2.3 Hz, 1H), 2.08 (ddd, J = 12.1, 10.4, 4.6 Hz, 1H), 1.98 - 1.87 (m, 2H), 1.92 - 1.72 (m, 6H); MS (ESI⁺) *m*/*z* 563.1 (M+H)⁺.

### Example 730: 2-(3,4-difluorophenoxy)-N-[(3S)-4-{2-[4-fluoro-3-(trifluoromethyl)phenoxy]acetamido}-3-hydroxybicyclo[2.2.2]octan-1-yl]acetamide (Compound 829)

The title compound was synthesized using the same procedure described in Example 674B substituting Example 724B for Example 674A and 2-(3,4-difluorophenoxy)acetic acid for 2-((2,2-difluorobenzo[*d*][1,3]dioxol-5-yl)oxy)acetic acid. ¹H NMR (400 MHz, DMSO-*d*₆) *δ* ppm 7.54 - 7.40 (m, 3H), 7.29 (h, J = 3.5 Hz, 3H), 7.02 (dd, J = 11.4, 2.9 Hz, 1H), 6.81 (ddd, J = 9.0, 2.9, 1.2 Hz, 1H), 4.52 (s, 2H), 4.44 (s, 2H), 4.05 (dd, J = 9.6, 3.0 Hz, 1H), 1 2.28 (ddd, J = 12.4, 9.4, 2.3 Hz, 1H), 2.08 (ddd, J = 12.1, 10.4, 4.6 Hz, 1H), 1.98 - 1.87 (m, 2H), 1.92 - 1.72 (m, 6H); MS (ESI⁺) *m*/*z* 547.2 (M+H)⁺.

### Example 731: 2-(4-chlorophenoxy)-N-[(3S)-4-{2-[4-fluoro-3-(trifluoromethyl)phenoxy]acetamido}-3-hydroxybicyclo[2.2.2]octan-1-yl]acetamide (Compound 830)

The title compound was synthesized using the same procedure described in Example 674B substituting Example 724B for Example 674A and 2-(4-chlorophenoxy)acetic acid for 2-((2,2-difluorobenzo[d][1,3]dioxol-5-yl)oxy)acetic acid. ¹H NMR (400 MHz, DMSO-*d*₆) *δ* ppm 7.54 - 7.40 (m, 2H), 7.37 - 7.24 (m, 5H), 6.98 - 6.89 (m, 2H), 4.51 (s, 2H), 4.39 (s, 2H), 4.04 (dd, J = 9.5, 3.1 Hz, 1H), 2.28 (ddd, J = 12.3, 9.5, 2.3 Hz, 1H), 2.13 - 2.01 (m, 1H), 1.93 (d, J = 9.8 Hz, 2H), 1.89 - 1.81 (m, 2H), 1.78 (ddt, J = 13.3, 6.0, 3.4 Hz, 4H); MS (ESI⁺) *m*/*z* 545.1 (M+H)⁺.

### Example 732: 2-(4-fluorophenoxy)-N-[(3S)-4-{2-[4-fluoro-3-(trifluoromethyl)phenoxy]acetamido}-3-hydroxybicyclo[2.2.2]octan-1-yl]acetamide (Compound 831)

The title compound was synthesized using the same procedure described in Example 674B substituting Example 724B for Example 674A and 2-(4-fluorophenoxy)acetic acid for 2-((2,2-difluorobenzo[d][1,3]dioxol-5-yl)oxy)acetic acid. ¹H NMR (400 MHz, DMSO-*d*₆) *δ* ppm 7.49 - 7.40 (m, 2H), 7.29 (dt, J = 5.9, 3.5 Hz, 3H), 7.16 - 7.07 (m, 2H), 6.97 - 6.89 (m, 2H), 4.51 (s, 2H), 4.36 (s, 2H), 4.04 (dd, J = 9.8, 3.1 Hz, 1H), 2.28 (ddd, J = 12.5, 9.4, 2.4 Hz, 1H), 2.07 (ddd, J = 12.5, 10.8, 4.6 Hz, 1H), 1.94 (dq, J = 10.7, 2.9 Hz, 2H), 1.91 - 1.82 (m, 2H), 1.80 (dddd, J = 16.0, 12.6, 6.7, 3.8 Hz, 4H); MS (ESI⁺) *m*/*z* 529.2 (M+H)⁺.

### Example 733: 2-(4-chloro-3-fluorophenoxy)-N-[(3S)-4-{2-[3-fluoro-4-(trifluoromethyl)phenoxy]acetamido}-3-hydroxybicyclo[2.2.2]octan-1-yl]acetamide (Compound 832)

The title compound was synthesized using the same procedure described in Example 674B substituting Example 574C for Example 674A and 2-(4-chloro-3-fluorophenoxy)acetic acid for 2-((2,2-difluorobenzo[*d*][1,3]dioxol-5-yl)oxy)acetic acid. ¹H NMR (400 MHz, DMSO-*d*₆) *δ* ppm 7.69 (t, J = 8.8 Hz, 1H), 7.53 - 7.43 (m, 2H), 7.34 (s, 1H), 7.10 (dd, J = 13.1, 2.4 Hz, 1H), 7.02 (dd, J = 11.4, 2.9 Hz, 1H), 6.94 (dd, J = 8.8, 2.4 Hz, 1H), 6.81 (ddd, J = 9.0, 2.9, 1.2 Hz, 1H), 5.05 (s, 1H), 4.57 (s, 2H), 4.43 (s, 2H), 4.07 (dd, J = 9.5, 3.1 Hz, 1H), 2.32 - 2.21 (m, 1H), 2.09 - 1.71 (m, 9H); MS (ESI⁺) *m*/*z* 563.2 (M+H)⁺.

### Example 734: 2-(3,4-difluorophenoxy)-N-[(3S)-4-{2-[3-fluoro-4-(trifluoromethyl)phenoxy]acetamido}-3-hydroxybicyclo[2.2.2]octan-1-yl]acetamide (Compound 833)

The title compound was synthesized using the same procedure described in Example 674B substituting Example 574C for Example 674A and 2-(3,4-difluorophenoxy)acetic acid for 2-((2,2-difluorobenzo[d][1,3]dioxol-5-yl)oxy)acetic acid. ¹H NMR (400 MHz, DMSO-*d*₆) *δ* ppm 7.69 (t, J = 8.8 Hz, 1H), 7.47 (s, 1H), 7.40 - 7.30 (m, 2H), 7.13 - 6.96 (m, 2H), 6.94 (dd, J = 8.8, 2.4 Hz, 1H), 6.75 (dtd, J = 9.2, 3.3, 1.6 Hz, 1H), 4.94 (s, 1H), 4.57 (s, 2H), 4.40 (s, 2H), 4.07 (dd, J = 9.6, 3.1 Hz, 1H), 2.27 (ddd, J = 13.4, 9.6, 1.9 Hz, 1H), 2.03 (ddd, J = 12.6, 10.9, 4.8 Hz, 1H), 1.98 - 1.87 (m, 3H), 1.89 - 1.72 (m, 5H).; MS (ESI⁺) *m*/*z* 529.2 (M+H)⁺.

### Example 735: 2-(4-chlorophenoxy)-N-[(3S)-4-{2-[3-fluoro-4-(trifluoromethyl)phenoxy]acetamido}-3-hydroxybicyclo[2.2.2]octan-1-yl]acetamide (Compound 834)

The title compound was synthesized using the same procedure described in Example 674B substituting Example 574C for Example 674A and 2-(4-chlorophenoxy)acetic acid for 2-((2,2-difluorobenzo[*d*][1,3]dioxol-5-yl)oxy)acetic acid. ¹H NMR (400 MHz, DMSO-*d*₆) *δ* ppm 7.69 (t, J = 8.7 Hz, 1H), 7.47 (s, 1H), 7.37 - 7.29 (m, 3H), 7.10 (dd, J = 13.0, 2.4 Hz, 1H), 6.97 - 6.89 (m, 3H), 5.03 (s, 1H), 4.57 (s, 2H), 4.39 (s, 2H), 4.07 (dd, J = 9.6, 3.1 Hz, 1H), 2.27 (ddd, J = 13.5, 9.6, 1.8 Hz, 1H), 2.02 (ddd, J = 12.6, 10.8, 4.8 Hz, 1H), 1.99 - 1.84 (m, 3H), 1.87 - 1.78 (m, 1H), 1.79 (s, 2H), 1.80 - 1.72 (m, 2H); MS (ESI⁺) *m*/*z* 545.2 (M+H)⁺.

### Example 736: 2-(4-fluorophenoxy)-N-[(3S)-4-{2-[3-fluoro-4-(trifluoromethyl)phenoxy]acetamido}-3-hydroxybicyclo[2.2.2]octan-1-yl]acetamide (Compound 835)

The title compound was synthesized using the same procedure described in Example 674B substituting Example 574C for Example 674A and 2-(4-fluorophenoxy)acetic acid for 2-((2,2-difluorobenzo[*d*][1,3]dioxol-5-yl)oxy)acetic acid. ¹H NMR (400 MHz, DMSO-*d*₆) *δ* ppm 7.69 (t, J = 8.8 Hz, 1H), 7.41 (s, 1H), 7.34 (s, 1H), 7.16 - 7.06 (m, 3H), 6.93 (ddt, J = 8.5, 6.5, 3.1 Hz, 3H), 5.04 (s, 1H), 4.57 (s, 2H), 4.36 (s, 2H), 4.07 (dd, J = 9.5, 3.1 Hz, 1H), 2.27 (ddd, J = 13.2, 9.5, 1.9 Hz, 1H), 2.03 (ddd, J = 12.6, 10.9, 4.9 Hz, 1H), 2.00 - 1.87 (m, 3H), 1.89 - 1.73 (m, 5H); MS (ESI⁺) *m*/*z* 529.0 (M+H)⁺.

### Example 737: 2-(4-chlorophenoxy)-N-[3-(2-{[6-(trifluoromethyl)pyridin-2-yl]oxy}acetamido)bicyclo[1.1.1]pentan-1-yl]acetamide (Compound 836)

The title compound was prepared using the methodologies described above. ¹H NMR (501 MHz, DMSO-*d₆*) *δ* ppm 8.70 (s, 1H), 8.68 (s, 1H), 8.02 - 7.96 (m, 1H), 7.50 (d, J = 7.3 Hz, 1H), 7.37 - 7.32 (m, 2H), 7.21 (d, J = 8.4 Hz, 1H), 6.99 - 6.94 (m, 2H), 4.69 (s, 2H), 4.42 (s, 2H), 2.22 (s, 6H); MS (ESI⁺) *m*/*z* 470 (M+H)⁺.

### Example 738: 2-(4-chlorophenoxy)-N-{3-[2-(3,4-difluorophenoxy)acetamido]bicyclo[1.1.1]pentan-1-yl}acetamide (Compound 837)

The title compound was prepared using the methodologies described above. ¹H NMR (400 MHz, DMSO-*d₆*) *δ* ppm 8.70 (s, 1H), 8.69 (s, 1H), 7.41 - 7.31 (m, 3H), 7.09 (ddd, J = 12.6, 6.7, 3.0 Hz, 1H), 7.00 - 6.93 (m, 2H), 6.80 (dtd, J = 9.2, 3.3, 1.7 Hz, 1H), 4.44 (s, 2H), 4.43 (s, 2H), 2.27 (s, 6H); MS (ESI⁺) *m*/*z* 437 (M+H)⁺.

### Example 739: 2-(4-chloro-3-fluorophenoxy)-N-[(2S)-2-hydroxy-4-(2-{[6-(pentafluoro-lambda⁶-sulfanyl)pyridin-3-yl]oxy}acetamido)bicyclo[2.2.2]octan-1-yl]acetamide (Compound 838)

### Example 739A: 5-bromopyridine-2-thiol

To a solution of 5-bromopyridin-2-ol (19 g, 109 mmol) in toluene (500 mL) was added 2,4-bis(4-methoxyphenyl)-1,3,2,4-dithiadiphosphetane 2,4-disulfide (22.08 g, 54.6 mmol) in portions at 20 °C under N₂. The mixture was stirred for 1 hour at 110 °C and cooled to 20 °C. The precipitate was collected by filtration and dried by high vacuum to provide the title compound (17.6 g, yield 84%). ¹H NMR:(400 MHz, DMSO-*d*₆) *δ* ppm 7.18 (d, J=9.26 Hz, 1 H) 7.51 (dd, J=9.26, 1.76 Hz, 1 H) 7.89 (d, J=1.76 Hz, 1 H).

### Example 739B: 1,2-bis(5-bromopyridin-2-yl)disulfane

To a solution of NaOH (7.67 g, 192 mmol) in water (600 mL) was added Example 739A (32 g, 167 mmol) at 20 °C under N₂, and the mixture was stirred for 30 minutes. A solution of potassium hexacyanoferrate (III) (63.1 g, 192 mmol) in water (500 mL) was added dropwise to the mixture. The resulting reaction mixture was stirred for 14 hours at 20 °C. The precipitate was collected by filtration, washed by water and dried by high vacuum to provide the title compound (29 g, yield 88%). ¹H NMR: (400 MHz, DMSO-*d*₆) *δ* ppm 7.60 (d, J=8.82 Hz, 2 H) 8.05 (dd, J=8.60, 2.43 Hz, 2 H) 8.64 (d, J=1.76 Hz, 2 H).

### Example 739C: 5-bromo-2-(chlorotetrafluoro-λ⁶-sulfanyl)pyridine

Anhydrous chlorine gas (dried by concentrated sulfuric acid, 18 g, 254 mmol) was bubbled slowly through a solution of anhydrous potassium fluoride (12.5 g, 215 mmol) in anhydrous acetonitrile (60 mL) at 0 °C. The escape hole was connected to a syringe filled with CaCl₂. The bottle was then resealed with closure and weighed. Example 739B (5 g, 13.22 mmol) was added to the reaction mixture in one portion at 0 °C. The resulting mixture was stirred for 2 hours at 0 °C and 20 hours at 20 °C. The mixture was filtered in a glove box, and the filtrate was concentrated under reduced pressure to provide the title compound (7.5 g, yield 94%) that was used in the next step directly.

### Example 739D: 5-bromo-2-(pentafluoro-λ⁶-sulfanyl)pyridine

A mixture of Example 739C (2.5 g, 8.32 mmol) and silver(I) fluoride (2.111 g, 16.64 mmol) was mixed quickly and thoroughly and heated to 60 °C for 20 hours under N₂ in a PFA sealed PFA tube. The residue was treated with dichloromethane and the solid filtered. The filtrate was concentrated under reduced pressure, and the residue was purified by column chromatography on Al₂O₃ (petroleum ether:dichloromethane=10:1) to provide the title compound (0.83 g, yield 35.1%) that was stored in a PFA bottle. ¹H NMR: (400 MHz, DMSO) δ ppm 7.67 (d, J=8.38 Hz, 1 H) 8.06 (d, J=8.38 Hz, 1 H) 8.66 (d, J=1.76 Hz, 1 H).

### Example 739E: (6-(pentafluoro- λ⁶-sulfanyl)pyridin-3-yl)boronic acid

To a stirred solution of Example 739D (500 mg, 1.672 mmol) and triisopropyl borate (1573 mg, 8.36 mmol) in tetrahydrofuran (20 mL) was added *n*-butyllithium (1.003 mL, 2.5 mmol in n-hexane) dropwise at -78°C under N₂. The mixture was stirred for 30 minutes at -78°C. The reaction was quenched with saturated aqueous NH₄Cl solution at -78°C and extracted with ethyl acetate (3 × 150 mL). The combined organic layers were washed with brine, dried over Na₂SO₄, and concentrated under reduced pressure. The residue was treated with dichloromethane and filtered. The cake was dried by high vacuum to provide the title compound (271 mg, yield 62.5%). ¹H NMR: (400 MHz, CD₃OD) *δ* ppm 7.83 (d, J=8.38 Hz, 1H), 8.31 (br d, J=8.16 Hz, 1H), 8.76 (s, 1H).

### Example 739F: 6-(pentafluoro- λ⁶-sulfanyl)pyridin-3-ol

To a stirred solution of Example 739E (1.2 g, 4.58 mmol) and triethylamine (6.38 mL, 45.8 mmol) in ethanol (100 mL) and water (10 mL, 555 mmol) was added iodosobenzene diacetate (7.37 g, 22.89 mmol) at 20 °C, and the mixture was stirred for 12 hours at 20°C. The reaction mixture was concentrated under reduced pressure. The residue was diluted with water (250 mL) and extracted with dichloromethane (3 × 100 mL). The combined organic layers were concentrated under reduced pressure, and the residue was purified by preparative HPLC performed on a Phenomenex^{®} Luna^{®} C18 column (250 × 50 mm, 10 µm particle size) using a gradient of 25% to 55% acetonitrile:0.09% aqueous trifluoroacetic acid over 20 minutes at a flow rate of 80 mL/minute. The desired HPLC fractions were extracted with dichloromethane (3 × 100 mL), and the combined organic layers were concentrated under reduced pressure to provide the title compound (751 mg, yield 80%). ¹H NMR: (400 MHz, DMSO-*d*₆) *δ* ppm 7.42 (br d, J=8.16 Hz, 1H), 7.82 (br d, J=9.04 Hz, 1H), 8.09 (d, J=2.43 Hz, 1H), 11.12 (s, 1H).

### Example 739G: 2-(4-chloro-3-fluorophenoxy)-N-[(2S)-2-hydroxy-4-(2-{[6-(pentafluoro-lambda⁶-sulfanyl)pyridin-3-yl]oxy}acetamido)bicyclo[2.2.2]octan-1-yl]acetamide:

A mixture of Example 476A (55.0 mg, 0.131 mmol), Example 739F (43.5 mg, 0.197 mmol), potassium iodide (1.089 mg, 6.56 µmol), and potassium carbonate (36.3 mg, 0.262 mmol) in acetone (3 mL) was heated at 140 °C in a Biotage^{®} Initiator microwave reactor for 30 minutes. The reaction mixture was filtered. The filtrate was concentrated, and the residue was purified by HPLC (see protocol in Example 529D) to provide the title compound (52.3 mg, 66%). ¹H NMR (400 MHz, DMSO-*d*₆) *δ* ppm 8.27 (d, J = 3.0 Hz, 1H), 7.97 (d, J = 9.0 Hz, 1H), 7.67 (s, 1H), 7.59 (dd, J = 9.1, 2.9 Hz, 1H), 7.48 (t, J = 8.9 Hz, 1H), 7.26 (s, 1H), 7.05 (dd, J = 11.4, 2.8 Hz, 1H), 6.83 (ddd, J = 9.0, 2.9, 1.2 Hz, 1H), 4.65 (s, 2H), 4.47 (s, 2H), 4.04 (dd, J = 9.5, 3.1 Hz, 1H), 2.28 (ddd, J = 12.1, 9.5, 2.1 Hz, 1H), 2.07 (ddd, J = 12.4, 10.6, 4.7 Hz, 1H), 1.98 - 1.64 (m, 8H): MS (ESI⁺) *m*/*z* 604.2 (M+H)⁺.

### Example 742: 2-(4-chloro-3-methylphenoxy)-N-[(2S)-4-{2-[3-fluoro-4-(trifluoromethyl)phenoxy]acetamido}-2-hydroxybicyclo[2.2.2]octan-1-yl]acetamide (Compound 841)

The title compound was synthesized using the same procedure described in Example 674B substituting 2-(4-chloro-3-methylphenoxy)acetic acid for 2-((2,2-difluorobenzo[*d*][1,3]dioxol-5-yl)oxy)acetic acid. ¹H NMR (400 MHz, DMSO-*d*₆) *δ* ppm 7.69 (t, J = 8.8 Hz, 1H), 7.59 (s, 1H), 7.30 (d, J = 8.8 Hz, 1H), 7.21 (s, 1H), 7.07 (dd, J = 13.1, 2.4 Hz, 1H), 6.97 - 6.88 (m, 2H), 6.79 (dd, J = 8.7, 3.1 Hz, 1H), 4.54 (s, 2H), 4.40 (d, J = 1.1 Hz, 2H), 4.04 - 3.97 (m, 1H), 2.28 (s, 3H), 2.32 - 2.23 (m, 1H), 2.19 - 2.09 (m, 1H), 1.99 - 1.86 (m, 2H), 1.87 - 1.74 (m, 6H); MS (ESI⁺) *m*/*z* 559.2 (M+H)⁺.

### Example 743: 2-(3,4-difluorophenoxy)-N-[(2S)-4-{2-[3-fluoro-4-(trifluoromethyl)phenoxy]acetamido}-2-hydroxybicyclo[2.2.2]octan-1-yl]acetamide (Compound 842)

The title compound was synthesized using the same procedure described in Example 674B substituting 2-(3,4-difluorophenoxy)acetic acid for 2-((2,2-difluorobenzo[*d*][1,3]dioxol-5-yl)oxy)acetic acid. ¹H NMR (400 MHz, DMSO-*d*₆) *δ* ppm 7.69 (t, J = 8.8 Hz, 1H), 7.59 (s, 1H), 7.36 (dt, J = 10.7, 9.4 Hz, 1H), 7.23 (s, 1H), 7.12 - 7.02 (m, 2H), 6.92 (dd, J = 8.9, 2.3 Hz, 1H), 6.77 (ddt, J = 9.1, 3.3, 1.7 Hz, 1H), 4.96 (s, 1H), 4.54 (s, 2H), 4.43 (s, 2H), 4.03 (dd, J = 9.6, 3.1 Hz, 1H), 2.28 (ddd, J = 12.4, 9.5, 2.4 Hz, 1H), 2.15 - 2.03 (m, 1H), 1.98 - 1.83 (m, 2H), 1.80 (tdt, J = 11.4, 6.3, 2.7 Hz, 6H).; MS (ESI⁺) *m*/*z* 547.2 (M+H)⁺.

### Example 744: 2-(4-chlorophenoxy)-N-[(2S)-4-{2-[3-fluoro-4-(trifluoromethyl)phenoxy]acetamido}-2-hydroxybicyclo[2.2.2]octan-1-yl]acetamide (Compound 843)

The title compound was synthesized using the same procedure described in Example 674B substituting 2-(4-chlorophenoxy)acetic acid for 2-((2,2-difluorobenzo[*d*][1,3]dioxol-5-yl)oxy)acetic acid. ¹H NMR (400 MHz, DMSO-*d*₆) *δ* ppm 7.69 (t, J = 8.8 Hz, 1H), 7.59 (s, 1H), 7.38 - 7.29 (m, 2H), 7.23 (s, 1H), 7.07 (dd, J = 13.1, 2.4 Hz, 1H), 7.00 - 6.88 (m, 3H), 4.54 (s, 2H), 4.42 (s, 2H), 4.01 (dd, J = 9.7, 3.2 Hz, 1H), 2.28 (ddd, J = 12.4, 9.5, 2.4 Hz, 1H), 2.18 - 2.03 (m, 1H), 1.93 (ddd, J = 11.7, 5.3, 2.9 Hz, 2H), 1.91 - 1.73 (m, 6H); MS (ESI⁺) *m*/*z* 545.1 (M+H)⁺.

### Example 745: 2-(4-fluorophenoxy)-N-[(2S)-4-{2-[3-fluoro-4-(trifluoromethyl)phenoxy]acetamido}-2-hydroxybicyclo[2.2.2]octan-1-yl]acetamide (Compound 844)

The title compound was synthesized using the same procedure described in Example 674B substituting 2-(4-fluorophenoxy)acetic acid for 2-((2,2-difluorobenzo[*d*][1,3]dioxol-5-yl)oxy)acetic acid. ¹H NMR (400 MHz, DMSO-*d*₆) *δ* ppm 7.69 (t, J = 8.8 Hz, 1H), 7.59 (s, 1H), 7.21 (s, 1H), 7.18 - 7.02 (m, 3H), 7.00 - 6.85 (m, 3H), 4.54 (s, 2H), 4.05 - 3.94 (m, 1H), 2.28 (ddd, J = 12.5, 9.4, 2.5 Hz, 1H), 2.14 (td, J = 9.9, 9.0, 5.8 Hz, 1H), 2.07 - 1.84 (m, 2H), 1.88 - 1.74 (m, 6H); MS (ESI⁺) *m*/*z* 529.2 (M+H)⁺.

### Example 746: 2-(3,4-difluorophenoxy)-N-[(2S)-4-{2-[4-fluoro-3-(trifluoromethyl)phenoxy]acetamido}-2-hydroxybicyclo[2.2.2]octan-1-yl]acetamide (Compound 845)

The title compound was synthesized using the same procedure described in Example 674B substituting Example 723B for Example 674A and 2-(3,4-difluorophenoxy)acetic acid for 2-((2,2-difluorobenzo[d][1,3]dioxol-5-yl)oxy)acetic acid. ¹H NMR (400 MHz, DMSO-*d*₆) *δ* ppm 7.51 (s, 1H), 7.45 - 7.17 (m, 6H), 7.03 (ddd, J = 12.7, 6.7, 3.1 Hz, 1H), 6.78 - 6.69 (m, 1H), 4.44 (s, 2H), 4.39 (s, 2H), 4.00 (dd, J = 9.6, 3.1 Hz, 1H), 2.23 (ddd, J = 12.3, 9.5, 2.3 Hz, 1H), 2.11 - 1.99 (m, 1H), 1.97 - 1.83 (m, 2H), 1.77 (dddt, J = 16.1, 12.6, 6.2, 3.5 Hz, 6H); MS (ESI⁺) *m*/*z* 547.2 (M+H)⁺.

### Example 747: 2-(4-chlorophenoxy)-N-[(2S)-4-{2-[4-fluoro-3-(trifluoromethyl)phenoxy]acetamido}-2-hydroxybicyclo[2.2.2]octan-1-yl]acetamide (Compound 846)

The title compound was synthesized using the same procedure described in Example 674B substituting Example 723B for Example 674A and 2-(4-chlorophenoxy)acetic acid for 2-((2,2-difluorobenzo[*d*][1,3]dioxol-5-yl)oxy)acetic acid. ¹H NMR (400 MHz, DMSO-*d*₆) *δ* ppm 7.51 (s, 1H), 7.40 (t, J = 9.5 Hz, 1H), 7.34 - 7.25 (m, 2H), 7.29 - 7.17 (m, 3H), 6.98 - 6.87 (m, 2H), 4.97 (s, 1H), 4.44 (s, 2H), 4.38 (s, 2H), 3.98 (dd, J = 9.6, 3.1 Hz, 1H), 2.23 (ddd, J = 12.5, 9.4, 2.4 Hz, 1H), 2.13 - 2.02 (m, 1H), 1.96 - 1.69 (m, 8H); MS (ESI⁺) *m*/*z* 547.2 (M+H)⁺.

### Example 748: 2-(4-fluorophenoxy)-N-[(2S)-4-{2-[4-fluoro-3-(trifluoromethyl)phenoxy]acetamido}-2-hydroxybicyclo[2.2.2]octan-1-yl]acetamide (Compound 847)

The title compound was synthesized using the same procedure described in Example 674B substituting Example 723B for Example 674A and 2-(4-fluorophenoxy)acetic acid for 2-((2,2-difluorobenzo[*d*][1,3]dioxol-5-yl)oxy)acetic acid. ¹H NMR (400 MHz, DMSO-*d*₆) *δ* ppm 7.51 (s, 1H), 7.45 - 7.36 (m, 1H), 7.23 (dd, J = 8.4, 4.3 Hz, 2H), 7.17 (s, 1H), 7.14 - 7.02 (m, 2H), 6.98 - 6.86 (m, 2H), 5.11 (s, 1H), 4.44 (s, 2H), 4.35 (s, 2H), 3.97 (dd, J = 9.7, 3.1 Hz, 1H), 2.24 (ddd, J = 12.5, 9.5, 2.5 Hz, 1H), 2.17 - 2.02 (m, 1H), 1.89 (t, J = 8.1 Hz, 2H), 1.87 - 1.69 (m, 6H).; MS (ESI⁺) *m*/*z* 529.3 (M+H)⁺.

### Example 749: 2-(4-chloro-3-fluorophenoxy)-N-[(2S)-4-{2-[4-fluoro-3-(trifluoromethyl)phenoxy]acetamido}-2-hydroxybicyclo[2.2.2]octan-1-yl]acetamide (Compound 848)

The title compound was synthesized using the same procedure described in Example 674B substituting Example 723B for Example 674A and 2-(4-chloro-3-fluorophenoxy)acetic acid for 2-((2,2-difluorobenzo[*d*][1,3]dioxol-5-yl)oxy)acetic acid. ¹H NMR (400 MHz, DMSO-*d*₆) *δ* ppm 7.51 (s, 1H), 7.42 (dt, J = 15.9, 9.2 Hz, 2H), 7.23 (h, J = 3.4 Hz, 3H), 7.01 (dd, J = 11.4, 2.9 Hz, 1H), 6.79 (dd, J = 8.7, 2.7 Hz, 1H), 4.43 (d, J = 6.2 Hz, 4H), 4.00 (dd, J = 9.6, 3.1 Hz, 1H), 2.23 (ddd, J = 12.2, 9.5, 2.2 Hz, 1H), 2.03 (ddd, J = 12.6, 10.7, 4.8 Hz, 1H), 1.89 (d, J = 10.8 Hz, 2H), 1.89 - 1.69 (m, 6H); MS (ESI⁺) *m*/*z* 563.2 (M+H)⁺.

### Example 750: 2-(4-chloro-3-fluorophenoxy)-N-[(2S)-4-{2-[3-fluoro-4-(trifluoromethyl)phenoxy]acetamido}-2-hydroxybicyclo[2.2.2]octan-1-yl]acetamide (Compound 849)

The title compound was synthesized using the same procedure described in Example 538 substituting 3-fluoro-4-(trifluoromethyl)phenol for 2-methoxypyrimidin-5-ol. ¹H NMR (400 MHz, DMSO-*d*₆) *δ* ppm 7.54 (s, 1H), 7.46 (dt, J = 15.7, 9.1 Hz, 2H), 7.31 - 7.22 (m, 3H), 7.05 (dd, J = 11.4, 2.9 Hz, 1H), 6.83 (ddd, J = 9.0, 2.9, 1.2 Hz, 1H), 5.09 (s, 1H), 4.47 (d, J = 6.2 Hz, 4H), 4.04 (dd, J = 9.8, 3.0 Hz, 1H), 2.27 (ddd, J = 12.2, 9.5, 2.2 Hz, 1H), 2.07 (ddd, J = 12.5, 10.7, 4.7 Hz, 1H), 1.97 - 1.89 (m, 2H), 1.87 (s, 1H), 1.80 (tdd, J = 12.2, 7.0, 3.1 Hz, 5H); MS (ESI⁺) *m*/*z* 563.0 (M+H)⁺.
The compounds in the following table were prepared using the methodologies described above.

| **Example** | **Name (Compound Number)** | **NMR** | **MS** |
|---|---|---|---|
| Example 541 | 2-(4-chloro-3-fluorophenoxy)-N-[(3S)-4-{2-[(5-chloropyridin-2-yl)oxy] acetamido }-3-hydroxybicyclo[2.2.2]octan-1-yl]acetamide (Compound 640) | ¹H NMR (400 MHz, DMSO-*d₆*) □ ppm 8.17 (dd, J = 2.7, 0.7 Hz, 1H), 7.83 (dd, J = 8.8, 2.7 Hz, 1H), 7.51 - 7.44 (m, 2H), 7.24 (s, 1H), 7.02 (dd, J = 11.4, 2.9 Hz, 1H), 6.94 (dd, J = 8.8, 0.7 Hz, 1H), 6.80 (ddd, J = 9.0, 2.9, 1.2 Hz, 1H), 5.05 (d, J = 4.0 Hz, 1H), 4.71 - 4.59 (m, 2H), 4.43 (s, 2H), 4.03 - 3.96 (m, 1H), 2.29 - 2.21 (m, 1H), 2.07 - 1.98 (m, 1H), 1.96 - 1.88 (m, 2H), 1.87 - 1.68 (m, 6H) | MS (APCI⁺) *m*/*z* 512 (M+H)⁺ |
| Example 575 | 2-(4-chloro-3-fluorophenoxy)-*N*-{(2*S*)-4-[2-(4-fluorophenoxy)acetamido]-2-hydroxybicyclo[2.2.2]octan-1-yl}acetamide (Compound 674) | ¹H NMR (501 MHz, DMSO-*d*₆) *δ* ppm 7.48 (t, J = 8.9 Hz, 1H), 7.41 (s, 1H), 7.26 (s, 1H), 7.15 - 7.08 (m, 2H), 7.05 (dd, J = 11.4, 2.9 Hz, 1H), 6.97 - 6.88 (m, 2H), 6.83 (ddd, J = 9.0, 2.9, 1.1 Hz, 1H), 5.06 (s, brd, 1H), 4.47 (s, 2H), 4.36 (s, 2H), 4.04 (dd, J = 9.7, 3.2 Hz, 1H), 2.27 (ddd, J = 12.3, 9.5, 2.3 Hz, 1H), 2.07 (ddd, J = 12.9, 11.0, 4.7 Hz, 1H), 1.98 - 1.72 (m, 8H). | (ESI⁺) *m*/*z* 495.2 (M+H)⁺ |
| Example 579 | 2-(4-chlorophenoxy)-*N-*{(3*S*)-4-[2-(3,4-difluorophenoxy)acetamido] -3-hydroxybicyclo[2.2.2]octan-1-yl}acetamide (Compound 678) | ¹H NMR (501 MHz, DMSO-*d*₆) *δ* ppm 7.46 (s, 1H), 7.41 - 7.28 (m, 3H), 7.23 (s, 1H), 7.07 (ddd, J = 12.6, 6.7, 3.1 Hz, 1H), 6.97 - 6.88 (m, 2H), 6.77 (dtd, J = 9.2, 3.2, 1.7 Hz, 1H), 5.09 (s, brd, 1H), 4.43 (s, 2H), 4.39 (s, 2H), 4.03 (dd, J = 9.8, 3.1 Hz, 1H), 2.27 (ddd, J = 12.5, 9.4, 2.4 Hz, 1H), 2.09 (ddd, J = 12.4, 10.7, 4.7 Hz, 1H), 1.97 - 1.69 (m, 8H). | (ESI⁺) *m*/*z* 495.2 (M+H)⁺ |
| Example 582 | 2-(3,4-difluorophenoxy)-*N-*{(2*S*)-4-[2-(4-fluorophenoxy)acetamido]-2-hydroxybicyclo[2.2.2]octan-1-yl}acetamide (Compound 681) | ¹H NMR (400 MHz, DMSO-*d*₆) *δ* ppm 7.45 - 7.29 (m, 2H), 7.23 (s, 1H), 7.17 - 7.02 (m, 3H), 6.98 - 6.89 (m, 2H), 6.77 (dtd, J = 8.7, 3.3, 1.7 Hz, 1H), 5.09 (s, brd, 1H), 4.43 (s, 2H), 4.36 (s, 2H), 4.03 (dd, J = 9.7, 3.1 Hz, 1H), 2.28 (ddd, J = 12.3, 9.5, 2.3 Hz, 1H), 2.16 - 2.03 (m, 1H), 1.99 - 1.69 (m, 8H). | (ESI⁺) *m*/*z* 479.1 (M+H)⁺ |
| Example 583 | 2-(4-chlorophenoxy)-*N-*{(2*S*)-4-[2-(4-fluorophenoxy)acetamido]-2-hydroxybicyclo[2.2.2]octan-1-yl}acetamide (Compound 682) | ¹H NMR (400 MHz, DMSO-d6) δ 7.41 (s, 1H), 7.37 - 7.30 (m, 2H), 7.23 (s, 1H), 7.17 - 7.05 (m, 2H), 6.99 - 6.86 (m, 4H), 5.11 (d, J = 4.5 Hz, 1H), 4.42 (s, 2H), 4.36 (s, 2H), 4.01 (dt, J = 8.3, 3.6 Hz, 1H), 2.28 (ddd, J = 12.4, 9.4, 2.4 Hz, 1H), 2.17 - 2.04 (m, 1H), 1.98 - 1.71 (m, 8H). | (ESI⁺) *m*/*z* 477.2 (M+H)⁺ |
| Example 584 | *N,N'*-[(2*S*)-2-hydroxybicyclo[2.2.2]octane -1,4-diyl]bis[2-(3,4-difluorophenoxy)acetamide] (Compound 683) | ¹H NMR (500 MHz, DMSO-*d*₆) *δ* ppm 7.47 (s, 1H), 7.35 (dtd, J = 11.5, 9.3, 2.3 Hz, 2H), 7.24 (s, 1H), 7.05 (dddd, J = 15.3, 12.6, 6.7, 3.0 Hz, 2H), 6.76 (ddtd, J = 10.9, 9.1, 3.2, 1.6 Hz, 2H), 5.10 (d, J = 4.4 Hz, 1H), 4.43 (s, 2H), 4.40 (s, 2H), 4.03 (d, J = 9.1 Hz, 1H), 2.28 (ddd, J = 12.6, 9.4, 2.5 Hz, 1H), 2.09 (ddd, J = 12.5, 10.7, 4.6 Hz, 1H), 1.99 - 1.71 (m, 8H). | (ESI⁺) *m*/*z* 497.1 (M+H)⁺ |
| Example 603 | 2-(4-chloro-3-fluorophenoxy)-*N*-[(2*S*)-2-hydroxy-4-{2-[(2,2,3,3-tetrafluoro-2,3-dihydro-1,4-benzodioxin-6-yl)oxy]acetamido}bicyclo[2 .2.2]octan-1-yl]acetamide (Compound 702) | ¹H NMR (400 MHz, DMSO-*d*₆) *δ* ppm 7.57 - 7.45 (m, 2H), 7.40 (d, J = 9.2 Hz, 1H), 7.26 (s, 1H), 7.05 (dd, J = 11.1, 2.9 Hz, 2H), 6.90 (dd, J = 9.2, 2.9 Hz, 1H), 6.83 (ddd, J = 9.0, 2.9, 1.2 Hz, 1H), 5.01 (s, brd, 1H), 4.45 (d, J = 11.2 Hz, 4H), 4.04 (dd, J = 9.6, 3.1 Hz, 1H), 2.37 - 2.20 (m, 1H), 2.07 (ddd, J = 12.5, 10.6, 4.7 Hz, 1H), 1.99 - 1.71 (m, 8H). | (ESI⁺) *m*/*z* 607.2 (M+H)⁺. |
| Example 609 | 2-(4-chlorophenoxy)-*N-*{(3*S*)-4-[2-(4-fluorophenoxy)acetamido]-3-hydroxybicyclo[2.2.2]octan-1-yl}acetamide (Compound 708) | ¹H NMR (400 MHz, DMSO-*d*₆) *δ* ppm 7.46 (s, 1H), 7.37 - 7.29 (m, 2H), 7.20 (s, 1H), 7.17 - 7.06 (m, 2H), 7.00 - 6.87 (m, 4H), 5.13 (d, J = 4.5 Hz, 1H), 4.39 (s, 4H), 4.00 (dt, J = 8.9, 3.8 Hz, 1H), 2.28 (ddd, J = 12.5, 9.5, 2.5 Hz, 1H), 2.14 (td, J = 9.9, 9.0, 5.9 Hz, 1H), 1.97 - 1.67 (m, 8H). | (ESI⁺) *m*/*z* 477.2 (M+H)⁺ |
| Example 627 | 2-(4-chloro-3-fluorophenoxy)-*N*-[(2*S*)-4-{2-[(2,2-difluoro-2*H*-1,3-benzodioxol-5-yl)oxy]acetamido}-2-hydroxybicyclo[2.2.2]octan-1-yl]acetamide (Compound 726) | ¹H NMR (501 MHz, DMSO-*d*₆) *δ* ppm 7.54 - 7.42 (m, 2H), 7.31 (d, J = 8.9 Hz, 1H), 7.26 (s, 1H), 7.13 - 7.00 (m, 2H), 6.83 (ddd, J = 9.0, 2.9, 1.2 Hz, 1H), 6.72 (dd, J = 8.9, 2.6 Hz, 1H), 5.07 (s, brd, 1H), 4.47 (s, 2H), 4.40 (s, 2H), 4.04 (dd, J = 9.9, 3.2 Hz, 1H), 2.28 (ddd, J = 12.3, 9.5, 2.3 Hz, 1H), 2.07 (ddd, J = 12.9, 11.0, 4.7 Hz, 1H), 1.98 - 1.70 (m, 8H). | (ESI⁺) *m*/*z* 557.1 (M+H)⁺. |
| Example 654 | 2-(4-chloro-3-fluorophenoxy)-*N*-[(3*S*)-3-hydroxy-4-{2-[(4-methylpyrimidin-2-yl)oxy]acetamido}bicyclo[2 .2.2]octan-1-yl]acetamide (Compound 753) | ¹H NMR (400 MHz, DMSO-*d₆*) □ ppm 8.42 (d, J = 5.0 Hz, 1H), 7.51 - 7.44 (m, 2H), 7.25 (s, 1H), 7.06 - 6.99 (m, 2H), 6.80 (ddd, J = 9.0, 2.9, 1.2 Hz, 1H), 5.06 (d, J = 3.6 Hz, 1H), 4.74 - 4.61 (m, 2H), 4.43 (s, 2H), 4.02 - 3.96 (m, 1H), 2.39 (s, 3H), 2.29 - 2.21 (m, 1H), 2.12 - 2.02 (m, 1H), 1.97 - 1.89 (m, 2H), 1.87 - 1.70 (m, 6H) | MS (APCI⁺) *m*/*z* 493 (M+H)⁺ |
| Example 657 | 2-(3,4-difluorophenoxy)-*N-*{(2*S*)-4- [2-(4-fluoro-3-methoxyphenoxy)acetamido ]-2-hydroxybicyclo[2.2.2]octan-1-yl}acetamide (Compound 756) | ¹H NMR (501 MHz, DMSO-*d*₆) *δ* ppm 7.46 - 7.31 (m, 2H), 7.23 (s, 1H), 7.15 - 6.99 (m, 2H), 6.82 - 6.67 (m, 2H), 6.42 (dt, J = 8.9, 3.1 Hz, 1H), 5.10 (s, brd, 1H), 4.43 (s, 2H), 4.36 (s, 2H), 4.03 (dd, J = 9.8, 3.1 Hz, 1H), 3.81 (s, 3H), 2.28 (ddd, J = 12.4, 9.4, 2.4 Hz, 1H), 2.10 (ddd, J = 12.2, 10.6, 4.5 Hz, 1H), 2.00 - 1.72 (m, 8H). | (ESI⁺) *m*/*z* 509.1 (M+H)⁺. |
| Example 671 | 2-(3,4-difluorophenoxy)-*N-*{(3*S*)-4-[2-(4-fluoro-3-methoxyphenoxy)acetamido ]-3-hydroxybicyclo[2.2.2]octan-1-yl}acetamide (Compound 770) | ¹H NMR (501 MHz, DMSO-d6) δ 7.47 (s, 1H), 7.41 - 7.30 (m, 1H), 7.19 (s, 1H), 7.11 (dd, J = 11.3, 8.9 Hz, 1H), 7.04 (ddd, J = 12.7, 6.8, 3.0 Hz, 1H), 6.75 (dt, J = 6.3, 3.2 Hz, 2H), 6.45 (dt, J = 9.0, 3.2 Hz, 1H), 4.40 (s, 4H), 4.05 - 3.96 (m, 1H), 3.82 (s, 3H), 2.28 (ddd, J = 12.7, 9.4, 2.8 Hz, 1H), 2.15 (tt, J = 9.3, 5.0 Hz, 1H), 2.00 - 1.72 (m, 8H). | (ESI⁺) *m*/*z* 509.1 (M+H)⁺. |
| Example 672 | 2-(4-chloro-3-fluorophenoxy)-*N*-{(3*S*)-4-[2-(4-fluoro-3-methoxyphenoxy)acetamido ]-3-hydroxybicyclo[2.2.2]octan-1-yl}acetamide (Compound 771) | ¹H NMR (400 MHz, DMSO-*d*₆) *δ* ppm 7.55 - 7.40 (m, 2H), 7.20 (s, 1H), 7.11 (dd, J = 11.3, 8.9 Hz, 1H), 7.02 (dd, J = 11.4, 2.9 Hz, 1H), 6.81 (ddd, J = 9.0, 2.9, 1.2 Hz, 1H), 6.76 (dd, J = 7.3, 3.0 Hz, 1H), 6.45 (dt, J = 8.9, 3.2 Hz, 1H), 4.44 (s, 2H), 4.40 (s, 2H), 4.05 - 3.97 (m, 1H), 3.82 (s, 3H), 2.28 (ddd, J = 12.5, 9.4, 2.6 Hz, 1H), 2.14 (td, J = 10.0, 9.1, 5.8 Hz, 1H), 2.01 - 1.72 (m, 8H). | (ESI⁺) *m*/*z* 525.1 (M+H)⁺. |
| Example 673 | 2-(4-chloro-3-fluorophenoxy)-*N*-[(3*S*)-4-{2-[4-fluoro-3-(trifluoromethoxy)phenoxy] acetamido }-3-hydroxybicyclo[2.2.2]octan-1-yl]acetamide (Compound 772) | ¹H NMR (400 MHz, DMSO-d6) δ 7.56 - 7.37 (m, 3H), 7.27 (s, 1H), 7.20 - 7.09 (m, 1H), 7.08 - 6.96 (m, 2H), 6.81 (dd, J = 9.3, 2.8 Hz, 1H), 4.47 (s, 2H), 4.43 (s, 2H), 4.04 (dd, J = 9.5, 3.1 Hz, 1H), 2.27 (ddd, J = 12.3, 9.5, 2.3 Hz, 1H), 2.15 - 2.00 (m, 1H), 1.99 - 1.70 (m, 8H). | (ESI⁺) *m*/*z* 579.3 (M+H)⁺. |
| Example 695 | 2-(4-chloro-3-fluorophenoxy)-*N*-{(2*S*)-4-[2-(4-fluoro-3-methoxyphenoxy)acetamido ]-2-hydroxybicyclo[2.2.2]octan-1-yl}acetamide (Compound 794) | ¹H NMR (400 MHz, DMSO-*d*₆) *δ* ppm 7.48 (t, J = 8.9 Hz, 1H), 7.40 (s, 1H), 7.26 (s, 1H), 7.15 - 6.99 (m, 2H), 6.83 (ddd, J = 8.9, 2.8, 1.2 Hz, 1H), 6.75 (dd, J = 7.4, 2.9 Hz, 1H), 6.42 (dt, J = 8.9, 3.1 Hz, 1H), 5.08 (d, J = 4.4 Hz, 1H), 4.47 (s, 2H), 4.36 (s, 2H), 4.11 - 3.98 (m, 1H), 3.81 (s, 3H), 2.28 (ddd, J = 12.1, 9.5, 2.1 Hz, 1H), 2.07 (ddd, J = 12.1, 10.4, 4.7 Hz, 1H), 1.98 - 1.73 (m, 8H). | (ESI⁺) *m*/*z* 525.2 (M+H)⁺. |
| Example 696 | 2-(4-chloro-3-fluorophenoxy)-*N*-[(3*S*)-4-{2-[3-(difluoromethoxy)-4-fluorophenoxy]acetamido}-3-hydroxybicyclo[2.2.2]octan-1-yl]acetamide (Compound 795) | ¹H NMR (400 MHz, DMSO-*d*₆) *δ* ppm 7.54 - 7.20 (m, 5H), 7.07 - 6.93 (m, 2H), 6.89 - 6.76 (m, 2H), 4.43 (d, J = 1.7 Hz, 4H), 4.03 (dd, J = 9.8, 3.1 Hz, 1H), 2.27 (ddd, J = 12.3, 9.4, 2.4 Hz, 1H), 2.17 - 2.01 (m, 1H), 1.98 - 1.70 (m, 8H). | (ESI⁺) *m*/*z* 561.1 (M+H)⁺. |
| Example 697 | 2-[3-(difluoromethoxy)-4-fluorophenoxy]-N-{(2*S*)-4-[2-(3,4-difluorophenoxy)acetamido] -2-hydroxybicyclo[2.2.2]octan-1-yl} acetamide (Compound 796) | ¹H NMR (400 MHz, DMSO-*d*₆) *δ* ppm 7.46 (s, 1H), 7.44 - 7.20 (m, 4H), 7.10 - 7.00 (m, 1H), 6.97 (dd, J = 6.6, 3.0 Hz, 1H), 6.84 (dt, J = 9.2, 3.3 Hz, 1H), 6.75 (dtd, J = 8.6, 3.3, 1.7 Hz, 1H), 4.43 (s, 2H), 4.40 (s, 2H), 4.03 (dd, J = 9.5, 3.1 Hz, 1H), 2.28 (ddd, J = 12.4, 9.5, 2.4 Hz, 1H), 2.16 - 2.03 (m, 1H), 1.97 - 1.73 (m, 8H). | (ESI⁺) *m*/*z* 545.2 (M+H)⁺. |
| Example 702 | 2-(4-chloro-3-fluorophenoxy)-*N*-[(2*S*)-4-{2-[4-(difluoromethoxy)-3-fluorophenoxy]acetamido}-2-hydroxybicyclo[2.2.2]octan-1-yl]acetamide (Compound 801) | ¹H NMR (400 MHz, DMSO-*d*₆) *δ* ppm 7.53 - 7.44 (m, 2H), 7.35 - 7.22 (m, 2H), 7.14 - 6.88 (m, 3H), 6.80 (dddd, J = 20.0, 9.1, 2.9, 1.3 Hz, 2H), 5.08 (s, brd, 1H), 4.47 (s, 2H), 4.42 (s, 2H), 4.04 (dd, J = 9.7, 3.1 Hz, 1H), 2.27 (ddd, J = 12.3, 9.6, 1.9 Hz, 1H), 2.07 (ddd, J = 12.3, 10.5, 4.7 Hz, 1H), 1.98 - 1.70 (m, 8H). | (ESI⁺) *m*/*z* 561.3 (M+H)⁺ |
| Example 703 | 2-(4-chloro-3-fluorophenoxy)-*N*-[(3*S*)-4-{2-[4-(difluoromethoxy)-3-fluorophenoxy]acetamido}-3-hydroxybicyclo[2.2.2]octan-1-yl]acetamide (Compound 802) | ¹H NMR (400 MHz, DMSO-*d*₆) *δ* ppm 7.56 - 7.40 (m, 2H), 7.34 - 7.21 (m, 2H), 7.15 - 6.91 (m, 3H), 6.80 (dtd, J = 9.1, 2.8, 1.3 Hz, 2H), 5.08 (s, brd, 1H), 4.45 (s, 2H), 4.43 (s, 2H), 4.04 (dd, J = 9.7, 3.1 Hz, 1H), 2.27 (ddd, J = 12.4, 9.5, 2.4 Hz, 1H), 2.08 (ddd, J = 12.3, 10.5, 4.7 Hz, 1H), 1.98 - 1.73 (m, 8H). | (ESI⁺) *m*/*z* 561.2 (M+H)⁺ |
| Example 704 | 2-[4-(difluoromethoxy)-3-fluorophenoxy]-*N*-{(2*S*)-4-[2-(3,4-difluorophenoxy)acetamido] -2-hydroxybicyclo[2.2.2]octan-1-yl} acetamide (Compound 803) | ¹H NMR (400 MHz, DMSO-*d*₆) *δ* ppm 7.46 (s, 1H), 7.42 - 7.22 (m, 3H), 7.14 - 6.89 (m, 3H), 6.84 - 6.70 (m, 2H), 5.07 (s, brd, 1H), 4.45 (s, 2H), 4.40 (s, 2H), 4.04 (dd, J = 9.6, 3.1 Hz, 1H), 2.27 (ddd, J = 12.4, 9.4, 2.4 Hz, 1H), 2.08 (ddd, J = 12.3, 10.4, 4.7 Hz, 1H), 1.98 - 1.69 (m, 8H). | (ESI⁺) *m*/*z* 545.1 (M+H)⁺ |
| Example 707 | 2-(4-chloro-3-fluorophenoxy)-*N*-[(3*S*)-3-hydroxy-4-{2-[(pyridazin-3-yl)oxy]acetamido}bicyclo[2 .2.2]octan-1-yl]acetamide (Compound 806) | ¹H NMR (400 MHz, DMSO-*d*₆) □ ppm 8.90 (dd, J = 4.5, 1.3 Hz, 1H), 7.64 (dd, J = 9.0, 4.4 Hz, 1H), 7.50 - 7.45 (m, 2H), 7.37 (s, 1H), 7.28 (dd, J = 9.0, 1.3 Hz, 1H), 7.02 (dd, J = 11.4, 2.9 Hz, 1H), 6.80 (ddd, J = 9.0, 2.9, 1.2 Hz, 1H), 5.00 (d, J = 4.0 Hz, 1H), 4.91 - 4.77 (m, 2H), 4.43 (s, 2H), 4.08 - 4.01 (m, 1H), 2.25 (dd, J = 13.2, 9.4 Hz, 1H), 2.05 - 1.70 (m, 9H) | MS (APCI⁺) *m*/*z* 479 (M+H)⁺ |
| Example 709 | 2-(4-chloro-3-fluorophenoxy)-*N*-[(2*S*)-4-{ 2-[3-(difluoromethoxy)-4-fluorophenoxy]acetamido }-2-hydroxybicyclo[2.2.2]octan-1-yl]acetamide (Compound 808) | ¹H NMR (400 MHz, DMSO-*d*₆) *δ* ppm 7.52 - 7.44 (m, 2H), 7.32 (dd, J = 10.4, 9.1 Hz, 1H), 7.24 (d, J = 13.0 Hz, 1H), 7.05 (dd, J = 11.3, 3.1 Hz, 1H), 6.95 (dd, J = 6.6, 3.0 Hz, 1H), 6.88 - 6.70 (m, 2H), 5.08 (d, J = 4.4 Hz, 1H), 4.47 (s, 2H), 4.40 (s, 2H), 4.04 (d, J = 9.4 Hz, 1H), 2.28 (td, J = 11.2, 10.6, 5.0 Hz, 1H), 2.14 - 2.00 (m, 1H), 1.99 - 1.69 (m, 8H). | (ESI⁺) *m*/*z* 561.2 (M+H)⁺ |
| Example 710 | 2-(3,4-difluorophenoxy)-*N-*[(3*S*)-3-hydroxy-4-{2-[3-(trifluoromethyl)phenoxy]ac etamido } bicyclo[2.2.2]octan -1-yl]acetamide (Compound 809) | ¹H NMR (400 MHz, DMSO-*d*₆) *δ* ppm 7.53 (t, J = 8.4 Hz, 1H), 7.47 (s, 1H), 7.40 - 7.28 (m, 3H), 7.27 - 7.20 (m, 2H), 7.04 (ddd, J = 12.7, 6.7, 3.0 Hz, 1H), 6.75 (dtd, J = 9.1, 3.3, 1.7 Hz, 1H), 4.99 (s, brd, 1H), 4.53 (s, 2H), 4.40 (s, 2H), 4.04 (dd, J = 9.6, 3.1 Hz, 1H), 2.28 (ddd, J = 12.5, 9.5, 2.4 Hz, 1H), 2.15 - 2.02 (m, 1H), 1.99 - 1.69 (m, 8H). | (ESI⁺) *m*/*z* 529.1 (M+H)⁺ |
| Example 712 | 2-(3,4-difluorophenoxy)-*N-*{(3*S*)-4-[2-(4-fluorophenoxy)acetamido]-3-hydroxybicyclo[2.2.2]octan-1-yl} acetamide (Compound 811) | ¹H NMR (501 MHz, DMSO-*d*₆) *δ* ppm 7.47 (s, 1H), 7.35 (dt, J = 10.6, 9.3 Hz, 1H), 7.21 (s, 1H), 7.17 - 7.09 (m, 2H), 7.04 (ddd, J = 12.7, 6.7, 3.1 Hz, 1H), 7.00 - 6.91 (m, 2H), 6.75 (dtd, J = 9.2, 3.3, 1.6 Hz, 1H), 5.12 (s, brd, 1H), 4.44 - 4.37 (m, 4H), 4.01 (ddd, J = 9.5, 3.3, 1.3 Hz, 1H), 2.28 (ddd, J = 12.7, 9.5, 2.8 Hz, 1H), 2.15 (tt, J = 9.0, 5.1 Hz, 1H), 1.99 - 1.71 (m, 8H). | (ESI⁺) *m*/*z* 479.1 (M+H)⁺ |
| Example 713 | 2-(3,4-difluorophenoxy)-*N-*[(3*S)*-4-{2-[4-fluoro-3-(trifluoromethoxy)phenoxy] acetamido}-3-hydroxybicyclo[2.2.2]octan-1-yl]acetamide (Compound 812) | ¹H NMR (400 MHz, DMSO-*d*₆) *δ* ppm 7.48 - 7.40 (m, 2H), 7.35 (dt, J = 10.6, 9.3 Hz, 1H), 7.27 (s, 1H), 7.14 (ddd, J = 6.4, 3.0, 1.3 Hz, 1H), 7.08 - 6.98 (m, 2H), 6.75 (dtd, J = 9.2, 3.3, 1.7 Hz, 1H), 4.47 (s, 2H), 4.39 (s, 2H), 4.04 (dd, J = 9.6, 3.1 Hz, 1H), 2.27 (ddd, J = 12.3, 9.5, 2.3 Hz, 1H), 2.07 (ddd, J = 12.1, 10.4, 4.6 Hz, 1H), 1.99 - 1.69 (m, 8H). | (ESI⁺) *m*/*z* 563.1 (M+H)⁺ |
| Example 714 | 2-(4-chlorophenoxy)-*N-*{(2*S*)-4-[2-(3,4-difluorophenoxy)acetamido] -2-hydroxybicyclo[2.2.2]octan-1-yl}acetamide (Compound 813) | ¹H NMR (400 MHz, DMSO-*d*₆) *δ* ppm 7.47 (s, 1H), 7.42 - 7.29 (m, 3H), 7.23 (s, 1H), 7.04 (ddd, J = 12.7, 6.7, 3.1 Hz, 1H), 6.99 - 6.90 (m, 2H), 6.82 - 6.65 (m, 1H), 5.10 (s, brd, 1H), 4.42 (s, 2H), 4.40 (s, 2H), 4.02 (dd, J = 9.6, 3.1 Hz, 1H), 2.28 (ddd, J = 12.5, 9.5, 2.4 Hz, 1H), 2.19 - 2.04 (m, 1H), 2.01 - 1.67 (m, 8H). | (ESI⁺) *m*/*z* 495.2 (M+H)⁺ |
| Example 721 | 2-(4-chloro-3-fluorophenoxy)-*N*-[(2*S*)-4-{2-[4-fluoro-3-(trifluoromethoxy)phenoxy] acetamido }-2-hydroxybicyclo[2.2.2]octan-1-yl]acetamide (Compound 820) | ¹H NMR (400 MHz, DMSO-*d*₆) *δ* ppm 7.56 - 7.37 (m, 3H), 7.26 (s, 1H), 7.15 - 7.08 (m, 1H), 7.05 (dd, J = 11.4, 2.9 Hz, 1H), 6.99 (dt, J = 9.2, 3.4 Hz, 1H), 6.83 (dt, J = 8.8, 1.8 Hz, 1H), 4.47 (s, 2H), 4.44 (s, 2H), 4.04 (dd, J = 9.5, 3.1 Hz, 1H), 2.27 (ddd, J = 12.3, 9.4, 2.3 Hz, 1H), 2.07 (ddd, J = 12.6, 10.6, 4.8 Hz, 1H), 1.98 - 1.71 (m, 8H). | (ESI⁺) *m*/*z* 579.0 (M+H)⁺ |
| Example 740 | 2-(4-chloro-3-fluorophenoxy)-*N*-[(2*S*)-2-hydroxy-4-{2-[4-(pentafluoro-lambda⁶-sulfanyl)phenoxy]acetamido }bicyclo[2.2.2]octan-1-yl]acetamide (Compound 839) | ¹H NMR (400 MHz, DMSO-*d*₆) *δ* ppm 7.90 - 7.74 (m, 2H), 7.58 (s, 1H), 7.48 (t, J = 8.9 Hz, 1H), 7.26 (s, 1H), 7.11 - 7.00 (m, 3H), 6.83 (dt, J = 8.6, 1.8 Hz, 1H), 5.04 (s, brd, 1H), 4.52 (s, 2H), 4.47 (s, 2H), 4.04 (dd, J = 9.5, 3.1 Hz, 1H), 2.28 (ddd, J = 12.0, 9.5, 2.2 Hz, 1H), 2.06 (dt, J = 10.5, 6.6 Hz, 1H), 1.99 - 1.63 (m, 8H). | (ESI⁺) *m*/*z* 603.0 (M+H)⁺ |
| Example 741 | 2-(4-chloro-3-fluorophenoxy)-*N*-[(2*S*)-2-hydroxy-4-(2-phenoxyacetamido)bicyclo[ 2.2.2]octan-1-yl]acetamide (Compound 840) | ¹H NMR (400 MHz, DMSO-*d*₆) *δ* ppm 7.48 (t, J = 8.9 Hz, 1H), 7.40 (s, 1H), 7.34 - 7.23 (m, 3H), 7.05 (dd, J = 11.4, 2.9 Hz, 1H), 6.99 - 6.87 (m, 3H), 6.83 (ddd, J = 9.0, 2.9, 1.2 Hz, 1H), 4.47 (s, 2H), 4.37 (s, 2H), 4.04 (dd, J = 9.7, 3.1 Hz, 1H), 2.28 (ddd, J = 12.2, 9.6, 2.2 Hz, 1H), 2.13 - 2.01 (m, 1H), 1.99 - 1.72 (m, 8H). | (ESI⁺) *m*/*z* 477.2 (M+H)⁺ |

### Example 751: Activity of exemplary compounds in an in vitro model of vanishing cell white matter disease (VWMD)

In order to test exemplary compounds of the invention and reference compounds in a cellular context, a stable VWMD cell line was first constructed. The ATF4 reporter was prepared by fusing the human full-length ATF4 5'-UTR (NCBI Accession No. BC022088.2) in front of the firefly luciferase (FLuc) coding sequence lacking the initiator methionine as described in Sidrauski et al (eLife 2013). The construct was used to produce recombinant retroviruses using standard methods and the resulting viral supernatant was used to transduce HEK293T cells, which were then subsequently selected with puromycin to generate a stable cell line.

HEK293T cells carrying the ATF4 luciferase reporter were plated on polylysine coated 384-well plates (Greiner Bio-one) at 30,000 cells per well. Cells were treated the next day with 1 µg/mL tunicamycin and 200 nM of a compound of Formula (I) for 7 hours. Luminescence was measured using One Glo (Promega) as specified by the manufacturer. Cells were maintained in DMEM with L-glutamine supplemented with 10% heat-inactivated FBS (Gibco) and Antibiotic-Antimycotic solution (Gibco).

Table 2 below summarizes the EC₅₀ data obtained using the ATF4-Luc assay for exemplary compounds of the invention. In this table, "A" represents an EC₅₀ of less than 10 nM; "B" an EC₅₀ of between 10 nM and 50 nM; "C" an EC₅₀ of between 50 nM and 250 nM; "D" an EC₅₀ of between 250 nM and 500 nM; "E" an EC₅₀ of between 500 nM and 2 µM; "F" an EC₅₀ of greater than 2 µM; and "G" indicates that data is not available.

**Table 2: EC₅₀ values of exemplary compounds of the invention and reference compounds in the ATF4-Luc assay.**

| **Compound No.** | **ATF4-Luc EC₅₀** |
|---|---|
| **100** | A |
| **101** | B |
| **102** | C |
| **103** | B |
| **104** | D |
| **105** | B |
| **106** | A |
| **107** | C |
| **108** | B |
| **109** | E |
| **110** | E |
| **111** | A |
| **112** | A |
| **113** | C |
| **114** | B |
| **115** | C |
| **116** | A |
| **117** | A |
| **118** | B |
| **119** | C |
| **120** | B |
| **121** | A |
| **122** | A |
| **123** | B |
| **124** | B |
| **125** | A |
| **126** | A |
| **127** | E |
| **128** | E |
| **129** | G |
| **130** | B |
| **131** | B |
| **132** | C |
| **133** | C |
| **134** | C |
| **135** | D |
| **136** | D |
| **137** | E |
| **138** | D |
| **139** | D |
| **140** | C |
| **141** | E |
| **142** | B |
| **143** | F |
| **144** | D |
| **145** | F |
| **146** | A |
| **147** | C |
| **148** | C |
| **149** | B |
| **150** | B |
| **151** | A |
| **152** | C |
| **153** | C |
| **154** | D |
| **155** | B |
| **156** | B |
| **157** | B |
| **158** | A |
| **159** | C |
| **160** | C |
| **161** | C |
| **162** | B |
| **163** | D |
| **164** | E |
| **165** | C |
| **166** | D |
| **167** | E |
| **168** | C |
| **169** | D |
| **170** | F |
| **171** | A |
| **172** | D |
| **173** | C |
| **174** | D |
| **175** | A |
| **176** | B |
| **177** | A |
| **178** | F |
| **179** | F |
| **180** | C |
| **181** | F |
| **182** | F |
| **183** | A |
| **184** | C |
| **185** | B |
| **186** | A |
| **187** | C |
| **188** | C |
| **189** | E |
| **190** | E |
| **191** | E |
| **192** | E |
| **193** | F |
| **194** | F |
| **195** | B |
| **196** | B |
| **197** | C |
| **198** | C |
| **199** | C |
| **200** | F |
| **201** | E |
| **202** | F |
| **203** | C |
| **204** | E |
| **205** | F |
| **206** | C |
| **207** | C |
| **208** | C |
| **209** | F |
| **210** | F |
| **211** | F |
| **212** | F |
| **213** | C |
| **214** | C |
| **215** | C |
| **216** | C |
| **217** | E |
| **218** | E |
| **219** | A |
| **220** | E |
| **221** | E |
| **222** | A |
| **223** | E |
| **224** | C |
| **225** | B |
| **226** | B |
| **227** | A |
| **228** | A |
| **229** | E |
| **230** | B |
| **231** | F |
| **232** | E |
| **233** | B |
| **234** | C |
| **235** | C |
| **236** | B |
| **237** | C |
| **238** | B |
| **239** | F |
| **240** | A |
| **241** | B |
| **242** | B |
| **243** | D |
| **244** | A |
| **245** | B |
| **246** | B |
| **247** | C |
| **248** | C |
| **249** | D |
| **250** | C |
| **251** | B |
| **252** | C |
| **253** | B |
| **254** | D |
| **255** | C |
| **256** | C |
| **257** | D |
| **258** | A |
| **259** | D |
| **260** | A |
| **261** | B |
| **262** | B |
| **263** | E |
| **264** | E |
| **265** | B |
| **266** | A |
| **267** | C |
| **268** | B |
| **269** | B |
| **270** | D |
| **271** | B |
| **272** | A |
| **273** | A |
| **274** | A |
| **275** | E |
| **276** | B |
| **277** | D |
| **278** | B |
| **279** | D |
| **280** | F |
| **281** | E |
| **282** | F |
| **283** | E |
| **284** | B |
| **285** | C |
| **286** | C |
| **287** | C |
| **288** | C |
| **289** | C |
| **290** | E |
| **291** | D |
| **292** | E |
| **293** | D |
| **294** | B |
| **295** | C |
| **296** | F |
| **297** | A |
| **298** | A |
| **299** | B |
| **300** | B |
| **301** | A |
| **302** | A |
| **303** | B |
| **304** | E |
| **305** | A |
| **306** | A |
| **307** | F |
| **308** | A |
| **309** | C |
| **310** | C |
| **311** | A |
| **312** | C |
| **313** | A |
| **314** | F |
| **315** | E |
| **316** | E |
| **317** | F |
| **318** | B |
| **319** | A |
| **320** | E |
| **321** | E |
| **322** | C |
| **323** | F |
| **324** | F |
| **325** | F |
| **326** | C |
| **327** | F |
| **328** | F |
| **329** | A |
| **330** | A |
| **331** | A |
| **332** | B |
| **333** | D |
| **334** | E |
| **335** | A |
| **336** | A |
| **337** | A |
| **338** | B |
| **339** | A |
| **340** | C |
| **341** | C |
| **342** | D |
| **343** | C |
| **344** | A |
| **345** | A |
| **346** | C |
| **347** | D |
| **348** | B |
| **349** | E |
| **350** | C |
| **351** | D |
| **352** | E |
| **353** | A |
| **354** | A |
| **355** | C |
| **356** | B |
| **357** | A |
| **358** | A |
| **359** | B |
| **360** | F |
| **361** | F |
| **362** | A |
| **363** | A |
| **364** | A |
| **365** | A |
| **366** | B |
| **367** | F |
| **368** | A |
| **369** | F |
| **370** | F |
| **371** | D |
| **372** | C |
| **373** | C |
| **374** | F |
| **375** | C |
| **376** | D |
| **377** | E |
| **378** | B |
| **379** | A |
| **380** | B |
| **381** | E |
| **382** | F |
| **383** | F |
| **384** | F |
| **385** | E |
| **386** | F |
| **387** | A |
| **388** | B |
| **389** | D |
| **390** | C |
| **391** | D |
| **392** | A |
| **393** | A |
| **394** | A |
| **395** | E |
| **396** | B |
| **397** | B |
| **398** | B |
| **399** | B |
| **400** | C |
| **401** | B |
| **402** | C |
| **403** | E |
| **404** | E |
| **405** | C |
| **406** | B |
| **407** | C |
| **408** | A |
| **409** | A |
| **410** | A |
| **411** | B |
| **412** | C |
| **413** | D |
| **414** | A |
| **415** | A |
| **416** | A |
| **417** | G |
| **418** | B |
| **419** | C |
| **420** | A |
| **421** | C |
| **422** | D |
| **423** | F |
| **424** | G |
| **425** | E |
| **426** | C |
| **427** | E |
| **428** | E |
| **429** | C |
| **430** | E |
| **431** | C |
| **432** | C |
| **433** | C |
| **434** | D |
| **435** | D |
| **436** | C |
| **437** | A |
| **438** | A |
| **439** | A |
| **440** | A |
| **441** | B |
| **442** | A |
| **443** | A |
| **444** | B |
| **445** | A |
| **446** | F |
| **447** | F |
| **448** | F |
| **449** | B |
| **450** | B |
| **451** | F |
| **452** | A |
| **453** | B |
| **454** | A |
| **455** | F |
| **456** | A |
| **457** | F |
| **458** | F |
| **459** | F |
| **460** | B |
| **461** | F |
| **462** | F |
| **463** | F |
| **464** | B |
| **465** | A |
| **466** | A |
| **467** | A |
| **468** | A |
| **469** | F |
| **470** | B |
| **471** | A |
| **472** | A |
| **473** | A |
| **474** | F |
| **475** | A |
| **476** | C |
| **477** | F |
| **478** | F |
| **479** | F |
| **480** | F |
| **481** | F |
| **482** | F |
| **483** | F |
| **484** | F |
| **485** | E |
| **486** | F |
| **487** | F |
| **488** | F |
| **489** | E |
| **490** | E |
| **491** | F |
| **492** | F |
| **493** | F |
| **494** | D |
| **495** | B |
| **496** | E |
| **497** | E |
| **498** | D |
| **499** | F |
| **500** | F |
| **501** | A |
| **502** | C |
| **503** | B |
| **504** | C |
| ***505*** | A |
| **506** | D |
| **507** | A |
| **508** | B |
| **509** | A |
| **510** | A |
| **511** | B |
| **512** | F |
| **513** | F |
| **514** | B |
| **515** | A |
| **516** | F |
| **517** | A |
| **518** | B |
| **519** | F |
| **520** | B |
| **521** | A |
| **522** | F |
| **523** | F |
| **524** | B |
| **525** | A |
| **526** | B |
| **527** | B |
| **528** | A |
| **529** | B |
| **530** | E |
| **531** | D |
| **532** | A |
| **533** | A |
| **534** | A |
| **535** | B |
| **536** | A |
| **537** | A |
| **538** | A |
| **539** | A |
| **540** | A |
| **541** | B |
| **542** | A |
| **543** | C |
| **544** | A |
| **545** | A |
| **546** | A |
| **547** | A |
| **548** | A |
| **549** | C |
| **550** | A |
| **551** | E |
| **552** | A |
| **553** | A |
| **554** | A |
| **555** | B |
| **556** | B |
| **557** | A |
| **558** | E |
| **559** | E |
| **560** | A |
| **561** | A |
| **562** | A |
| **563** | A |
| **564** | F |
| **565** | E |
| **566** | B |
| **567** | E |
| **568** | A |
| **569** | A |
| **570** | C |
| **571** | B |
| **572** | B |
| **573** | A |
| **574** | A |
| **575** | F |
| **576** | B |
| **577** | F |
| **578** | F |
| **579** | B |
| **580** | A |
| **581** | A |
| **582** | A |
| **583** | A |
| **584** | A |
| **585** | A |
| **586** | A |
| **587** | A |
| **588** | A |
| **589** | A |
| **590** | A |
| **591** | A |
| **592** | E |
| **593** | F |
| **594** | F |
| **595** | F |
| **596** | A |
| **597** | B |
| **598** | A |
| **599** | B |
| **600** | A |
| **601** | A |
| **602** | A |
| **603** | A |
| **604** | B |
| **605** | C |
| **606** | C |
| **607** | C |
| **608** | C |
| **609** | F |
| **610** | F |
| **611** | B |
| **612** | F |
| **613** | E |
| **614** | A |
| **615** | A |
| **616** | A |
| **617** | A |
| **618** | A |
| **619** | A |
| **620** | A |
| **621** | A |
| **622** | E |
| **623** | B |
| **624** | A |
| **625** | A |
| **626** | F |
| **627** | A |
| **628** | A |
| **629** | C |
| **630** | A |
| **631** | C |
| **632** | B |
| **633** | B |
| **634** | C |
| **635** | B |
| **636** | D |
| **637** | A |
| **638** | A |
| **639** | C |
| **640** | A |
| **641** | F |
| **642** | F |
| **643** | A |
| **644** | A |
| **645** | A |
| **646** | E |
| **647** | C |
| **648** | B |
| **649** | A |
| **650** | A |
| **651** | A |
| **652** | B |
| **653** | A |
| **654** | A |
| **655** | B |
| **656** | B |
| **657** | C |
| **658** | C |
| **659** | A |
| **660** | A |
| **661** | F |
| **662** | D |
| **663** | B |
| **664** | B |
| **665** | B |
| **666** | C |
| **667** | F |
| **668** | F |
| **669** | C |
| **670** | E |
| **671** | E |
| **672** | B |
| **673** | C |
| **674** | A |
| **675** | B |
| **676** | D |
| **677** | E |
| **678** | A |
| **679** | C |
| **680** | A |
| **681** | A |
| **682** | A |
| **683** | A |
| **684** | F |
| **685** | F |
| **686** | E |
| **687** | E |
| **688** | C |
| **689** | C |
| **690** | D |
| **691** | F |
| **692** | D |
| **693** | E |
| **694** | D |
| **695** | E |
| **696** | B |
| **697** | F |
| **698** | D |
| **699** | B |
| **700** | D |
| **701** | B |
| **702** | A |
| **703** | A |
| **704** | D |
| **705** | B |
| **706** | D |
| **707** | A |
| **708** | A |
| **709** | A |
| **710** | A |
| **711** | A |
| **712** | E |
| **713** | F |
| **714** | B |
| **715** | B |
| **716** | B |
| **717** | C |
| **718** | A |
| **719** | B |
| **720** | A |
| **721** | A |
| **722** | A |
| **723** | B |
| **724** | A |
| **725** | A |
| **726** | A |
| **727** | A |
| **728** | A |
| **729** | C |
| **730** | B |
| **731** | A |
| **732** | A |
| **733** | A |
| **734** | A |
| **735** | A |
| **736** | A |
| **737** | A |
| **738** | A |
| **739** | A |
| **740** | B |
| **741** | B |
| **742** | A |
| **743** | A |
| **744** | A |
| **745** | A |
| **746** | B |
| **747** | B |
| **748** | B |
| **749** | A |
| **750** | A |
| **751** | F |
| **752** | E |
| **753** | D |
| **754** | F |
| **755** | A |
| **756** | A |
| **757** | B |
| **758** | A |
| **759** | A |
| **760** | A |
| **761** | A |
| **762** | A |
| **763** | A |
| **764** | B |
| **765** | D |
| **766** | C |
| **767** | D |
| **768** | C |
| **769** | B |
| **770** | A |
| **771** | A |
| **772** | A |
| **773** | A |
| **774** | A |
| **775** | A |
| **776** | F |
| **777** | B |
| **778** | B |
| **779** | F |
| **780** | A |
| **781** | A |
| **782** | A |
| **783** | A |
| **784** | A |
| **785** | C |
| **786** | A |
| **787** | A |
| **788** | A |
| **789** | C |
| **790** | A |
| **791** | F |
| **792** | A |
| **793** | A |
| **794** | A |
| **795** | A |
| **796** | B |
| **797** | C |
| **798** | A |
| **799** | A |
| **800** | C |
| **801** | A |
| **802** | A |
| **803** | A |
| **804** | A |
| **805** | E |
| **806** | E |
| **807** | B |
| **808** | A |
| **809** | A |
| **810** | F |
| **811** | A |
| **812** | A |
| **813** | A |
| **814** | A |
| **815** | A |
| **816** | A |
| **817** | A |
| **818** | D |
| **819** | E |
| **820** | A |
| **821** | A |
| **822** | A |
| **823** | A |
| **824** | B |
| **825** | C |
| **826** | C |
| **827** | C |
| **828** | A |
| **829** | A |
| **830** | A |
| **831** | A |
| **832** | A |
| **833** | A |
| **834** | A |
| **835** | A |
| **836** | D |
| **837** | A |
| **838** | A |
| **839** | A |
| **840** | A |
| **841** | A |
| **842** | A |
| **843** | A |
| **844** | A |
| **845** | A |
| **846** | A |
| **847** | A |
| **848** | A |
| **849** | A |
| | |

VWMD mutations were introduced into the genome of the HEK293T ATF4-Fluc stable cell lines by using Gene Art CRISPR nuclease vector with OFP Reporter kit (ThermoFisher; see Table 3 below). Guide RNAs were designed using the CRISPR Design Tool (http://crispr.mit.edu) and ligated into the CRISPR OFP Nuclease Vector. To obtain homology directed repair (HDR) incorporating VWMD point mutations in the genome, 150 bp ssDNA ultramer oligos were synthesized by Integrated DNA Technologies containing specific mutations of interest. In addition to the VWMD mutations, the ssDNA HDR templates contained a silent mutation to the PAM site of the CRISPR gRNA sequence (to avoid further Cas9 cutting) and 75 bp of homology on each side of the mutation.

HEK293T ATF4-Fluc cells were transfected with 500 ng of the CRISPR OFP Nuclease Vector and 1 uL of 10 µM ssDNA HDR template using lipofectamine 3000 (ThermoFisher) or SF Cell Line 4D-nucleofector X Kit (Lonza) according to the manufacturer's instructions. After 2-3 days of recovery, single cells were sorted for positive OFP expression on a FACS Aria II (BD Biosciences) into wells of a 96 well plate and allowed to recover for 1-2 weeks.

The resulting clones were surveyed for CRISPR editing and HDR by harvesting the genomic DNA with the PureLink Genomic DNA kit (ThermoFisher), amplifying a ~500bp locus near the editing site, and sequencing the amplicon. Clones that displayed an ambiguous chromatogram signal near the expected CRISPR editing site were further examined by TA cloning (Invitrogen) and sequencing of the amplicon, yielding the sequence of each allele in the clone. Typical clones obtained were hemizygous for the VWMD point mutation, with one or two alleles harboring the desired mutation, and the remaining alleles knocked out (edited to produce a premature stop codon).

Table 3 below summarizes the **EC₅₀** data obtained using the ATF4-Luc assay for VWMD eIF2B mutant with exemplary compounds of the invention and reference compounds. In this table, "A" represents an EC₅₀ of less than 10 nM; "B" an EC₅₀ of between 10 nM and 50 nM; "C" an EC₅₀ of between 50 nM and 250 nM; "D" an EC₅₀ of between 250 nM and 500 µM; "E" an EC₅₀ of between 500 nM and 2 µM; and "F" an EC₅₀ of greater than 2 µM.

**Table 3: Exemplary eIF2B mutants and corresponding ATF4-Luc EC50 data.**

| **Compound No.** | **ATF4-Luc EC₅₀** | | | | | | |
|---|---|---|---|---|---|---|---|
| | **B1 V183F** | **B3 H341Q** | **B3 I346T** | **B4 R357W** | **B4 R483W** | **B5 R113H** | **B5 R195H** |
| **112** | A | A | A | A | A | A | A |
| **175** | C | B | A | B | A | B | B |
| **217** | F | E | E | E | F | F | E |
| **297** | A | A | A | A | A | A | A |

### EQUIVALENTS AND SCOPE

In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

Furthermore, the invention encompasses all variations, combinations, and permutations in which one or more limitations, elements, clauses, and descriptive terms from one or more of the listed claims are introduced into another claim. For example, any claim that is dependent on another claim can be modified to include one or more limitations found in any other claim that is dependent on the same base claim. Where elements are presented as lists, *e.g.,* in Markush group format, each subgroup of the elements is also disclosed, and any element(s) can be removed from the group. It should it be understood that, in general, where the invention, or aspects of the invention, is/are referred to as comprising particular elements and/or features, certain embodiments of the invention or aspects of the invention consist, or consist essentially of, such elements and/or features. For purposes of simplicity, those embodiments have not been specifically set forth *in haec verba* herein. It is also noted that the terms "comprising" and "containing" are intended to be open and permits the inclusion of additional elements or steps. Where ranges are given, endpoints are included. Furthermore, unless otherwise indicated or otherwise evident from the context and understanding of one of ordinary skill in the art, values that are expressed as ranges can assume any specific value or sub-range within the stated ranges in different embodiments of the invention, to the tenth of the unit of the lower limit of the range, unless the context clearly dictates otherwise.

This application refers to various issued patents, published patent applications, journal articles, and other publications. If there is a conflict between any of the references and the instant specification, the specification shall control. In addition, any particular embodiment of the present invention that falls within the prior art may be explicitly excluded from any one or more of the claims. Because such embodiments are deemed to be known to one of ordinary skill in the art, they may be excluded even if the exclusion is not set forth explicitly herein. Any particular embodiment of the invention can be excluded from any claim, for any reason, whether or not related to the existence of prior art.

Those skilled in the art will recognize or be able to ascertain using no more than routine experimentation many equivalents to the specific embodiments described herein. The scope of the present embodiments described herein is not intended to be limited to the above Description, but rather is as set forth in the appended claims.

## Claims

1. A compound of Formula (II): 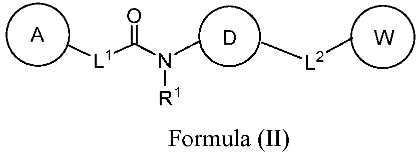 or a pharmaceutically acceptable salt thereof, wherein:
D is selected from the group consisting of: 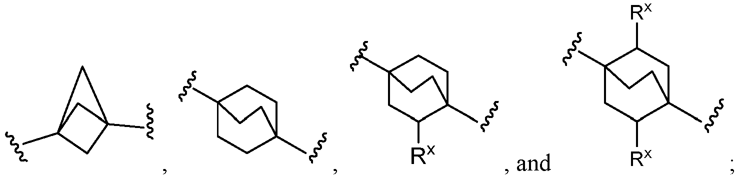
L¹ is C₁-C₆ alkylene, C₂-C₆ alkenylene, 2-7-membered heteroalkylene, -O-, or -NR^{C}-, wherein C₁-C₆ alkylene, C₂-C₆ alkenylene, or 2-7-membered heteroalkylene is optionally substituted with 1-5 R^{X};
L² is -(C₀-C₂ alkylene)-O-(C₀-C₂ alkylene)-;
R¹ is hydrogen, C₁-C₆ alkyl, C₁-C₆ alkoxy-C₂-C₆ alkyl, hydroxy-C₂-C₆ alkyl, or silyloxy-C₂-C₆ alkyl;
R^{N1} is selected from the group consisting of hydrogen, C₁-C₆ alkyl, hydroxy-C₂-C₆ alkyl, halo-C₂-C₆ alkyl, amino-C₂-C₆ alkyl, cyano-C₂-C₆ alkyl, -C(O)NR^{B}R^{C}, -C(O)R^{D}, -C(O)OR^{D}, and -S(O)₂R^{D};
A and W are each independently phenyl or 5-6-membered heteroaryl, wherein each phenyl or 5-6-membered heteroaryl is optionally substituted with 1-5 R^{Y};
each R^{X} is independently selected from the group consisting of C₁-C₆ alkyl, hydroxy-C₁-C₆ alkyl, halo-C₁-C₆ alkyl, amino-C₁-C₆ alkyl, cyano-C₁-C₆ alkyl, C₁-C₆ alkoxy-C₁-C₆ alkyl, oxo, halo, cyano, - OR^{A}, -NR^{B}R^{C}, -NR^{B}C(O)R^{D}, -C(O)NR^{B}R^{C}, -C(O)R^{D}, -C(O)OH, -C(O)OR^{D}, -SR^{E}, -S(O)R^{D}, - S(O)₂R^{D}, -OS(O)R^{D}, -OS(O)₂R^{D}, and G²;
each R^{Y} is independently selected from the group consisting of hydrogen, C₁-C₆ alkyl, hydroxy-C₁-C₆ alkyl, halo-C₁-C₆ alkyl, halo-C₁-C₆ alkoxy, amino-C₁-C₆ alkyl, cyano-C₁-C₆ alkyl, oxo, halo, cyano, -OR^{A}, -NR^{B}R^{C}, -NR^{B}C(O)R^{D}, -C(O)NR^{B}R^{C}, -C(O)R^{D}, -C(O)OH, -C(O)OR^{D}, -S(R^{F})ₘ, - S(O)R^{D}, -S(O)₂R^{D}, and G¹; or
2 R^{Y} groups on adjacent atoms, together with the atoms to which they are attached form a 3-7-membered fused cycloalkyl, heterocyclyl, aryl, or heteroaryl ring optionally substituted with 1-5 R^{X};
each G¹ and G² is independently C₃-C₆ cycloalkyl, 4-7-membered heterocyclyl, aryl, or 5-6-membered heteroaryl, wherein each C₃-C₆ cycloalkyl, 4-7-membered heterocyclyl, aryl, or 5-6-membered heteroaryl is optionally substituted with 1-3 R^{Z};
each R^{Z} is independently selected from the group consisting of C₁-C₆ alkyl, hydroxy-C₁-C₆ alkyl, halo-C₁-C₆ alkyl, halo, cyano, -OR^{A}, -NR^{B}R^{C}, -NR^{B}C(O)R^{D}, -C(O)NR^{B}R^{C}, -C(O)R^{D}, -C(O)OH, - C(O)OR^{D}, and -S(O)₂R^{D};
each R^{A} is independently hydrogen, C₁-C₆ alkyl, halo-C₁-C₆ alkyl, -C(O)NR^{B}R^{C}, -C(O)R^{D}, or - C(O)OR^{D};
each of R^{B} and R^{C} is independently hydrogen or C₁-C₆ alkyl; or
R^{B} and R^{C} together with the atom to which they are attached form a 3-7-membered heterocyclyl ring optionally substituted with 1-3 R^{Z};
each R^{D} is independently C₁-C₆ alkyl, 2-7-membered heteroalkyl, or halo-C₁-C₆ alkyl, wherein each C₁-C₆ alkyl, 2-7-membered heteroalkyl, or halo-C₁-C₆ alkyl is optionally substituted with 1-5 R^{G};
each R^{E} is independently hydrogen, C₁-C₆ alkyl, or halo-C₁-C₆ alkyl;
each R^{F} is independently hydrogen, C₁-C₆ alkyl, or halo;
each R^{Y} is independently aryl or 5-6 membered heteroaryl, wherein each aryl or 5-6 membered heteroaryl is optionally substituted with 1-5 R^{Y};
each R^{H} is independently C₁-C₆ alkyl or halo-C₁-C₆ alkyl;
m is 1 when R^{F} is hydrogen or C₁-C₆ alkyl, 3 when R^{F} is C₁-C₆ alkyl, or 5 when R^{F} is halo; and t is 0 or 1.

2. The compound of claim 1, wherein each R^{X} is independently selected from the group consisting of oxo, -OR^{A} (*e.g.,* -OH or -OCH₃), -C(O)OH, -C(O)OR^{D} (*e.g.,* -C(O)OCH₃), halo, and hydroxy-C₁-C₆ alkyl.

3. The compound of claim 1 or 2, wherein L¹ is CH₂O-* or CH₂OCH₂-*; wherein "-*" indicates the attachment point to A.

4. The compound of any one of claims 1-3, wherein L² is selected from the group consisting of O-*, OCH₂-*, CH₂O-*, OCH₂CH₂-*, CH₂OCH₂-*, and CH₂CH₂O-*; wherein "-*" indicates the attachment point to W.

5. The compound of any one of claims 1-4, wherein R¹ is hydrogen or -CH₃.

6. The compound of any one of claims 1-5, wherein A is selected from the group consisting of:

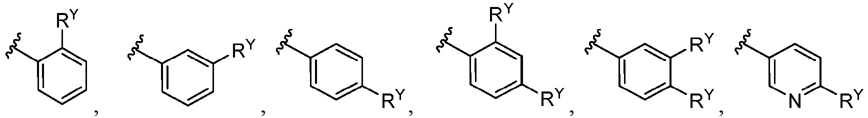

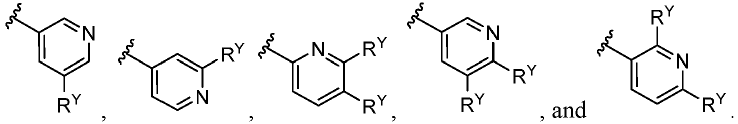

7. The compound of any one of claims 1-6, wherein W is selected from the group consisting of: 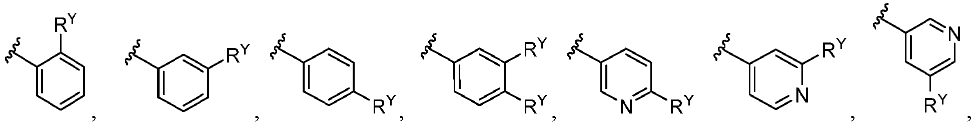 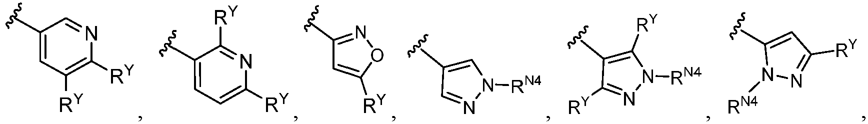 and 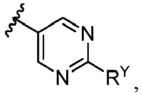 wherein R^{N4} is hydrogen or -CH₃.

8. The compound of any one of claims 1-7, wherein each R^{Y} is independently hydrogen, chloro, fluoro, -CF₃, -CHF₂, -CH₃, -CH₂CH₃, -CH(CH₃)₂, -OCH₃, -OCF₃, -OCH(CH₃)₂, or -CN.

9. A compound of Formula (II-a): 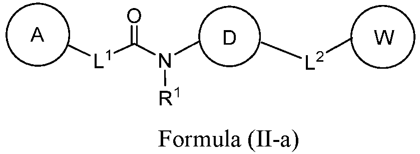 or a pharmaceutically acceptable salt thereof, wherein:
D is bicyclo[1.1.1]pentanyl or bicyclo[2.2.2]octanyl, each of which is optionally substituted with 1-4 R^{X} groups;
L¹ is CH₂O-* or CH₂OCH₂-*, wherein "-*" indicates the attachment point to A;
L² is selected from the group consisting of O-*, OCH₂-*, CH₂O-*, OCH₂CH₂-*, CH₂OCH₂-*, and CH₂CH₂O-*; wherein "-*" indicates the attachment point to W;
A is phenyl or pyridyl, each of which is optionally substituted with 1-5 R^{Y} groups;
W is phenyl, pyridyl, isoxazolyl, or pyrazolyl, each of which is optionally substituted on one or more available carbons with 1-5 R^{Y} groups; and wherein pyrazolyl may be optionally substituted on an available nitrogen with hydrogen or -CH₃;
each R^{X} is independently fluoro, oxo, -OH, -OCH₃, -C(O)OH, or -C(O)OCH₃;
each R^{Y} is independently chloro, fluoro, -CF₃, -CH₃, -CH₂CH₃, -CH(CH₃)₂, -OCH₃, - OCH(CH₃)₂, or -CN; or
2 R^{Y} groups on adjacent atoms, together with the atoms to which they are attached form a furanyl, pyrrolyl, or dioxolanyl ring, each of which is optionally substituted with 1-2 R^{X}; and
R¹ is hydrogen.

10. A compound selected from 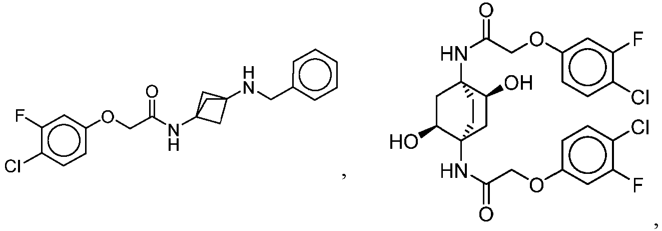 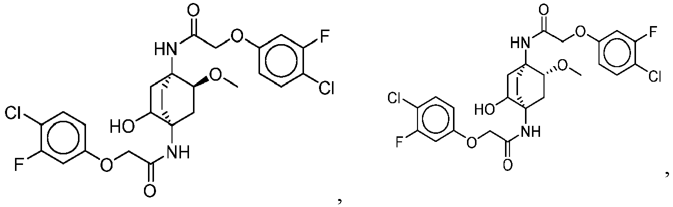 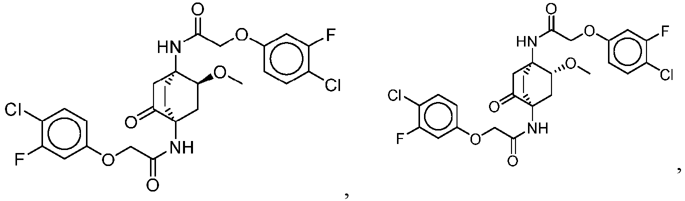 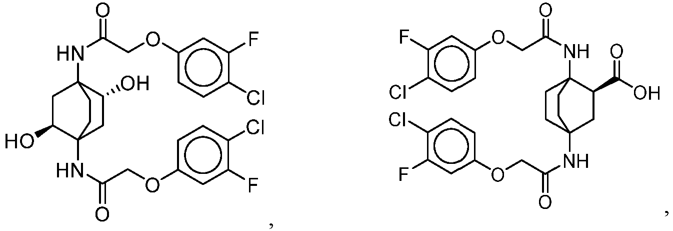 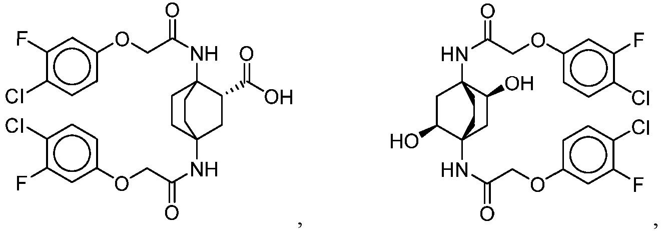 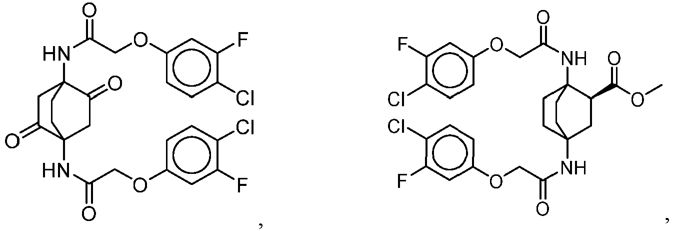 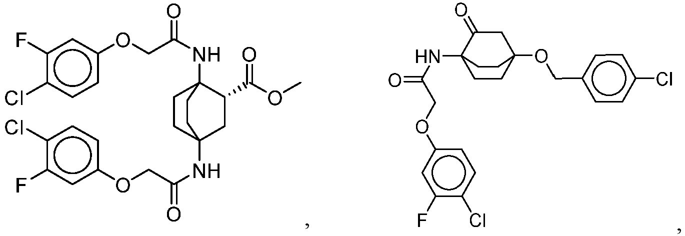 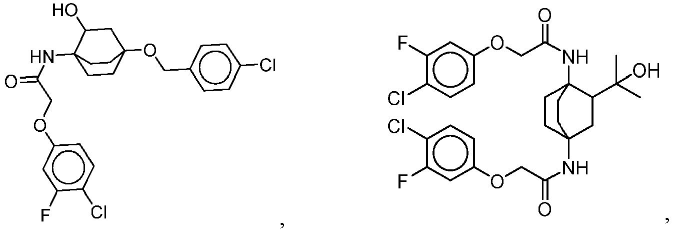 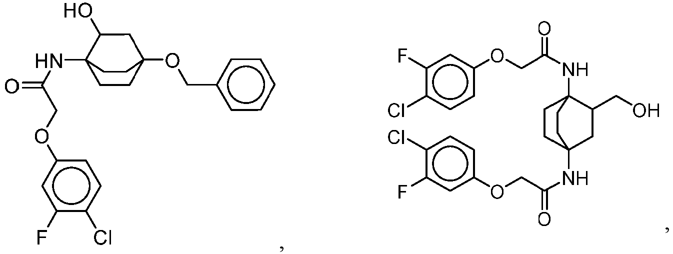 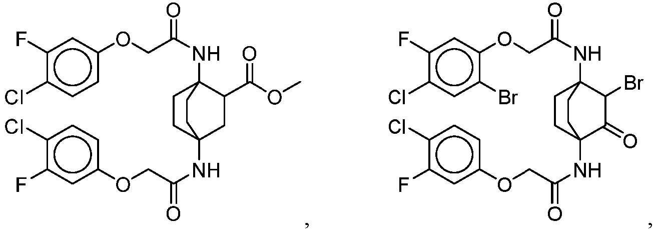 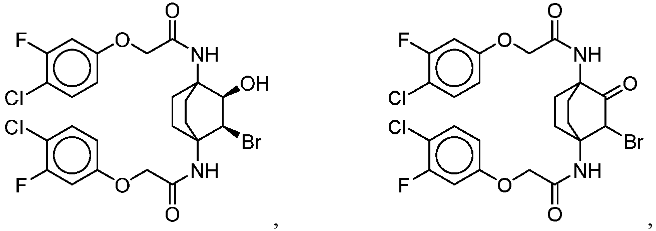 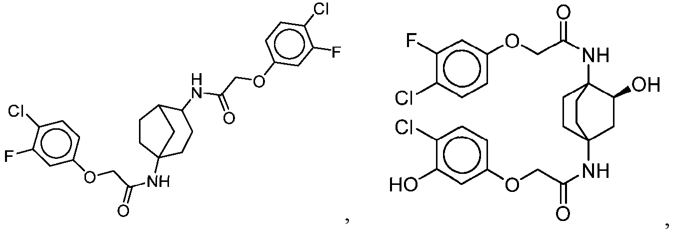 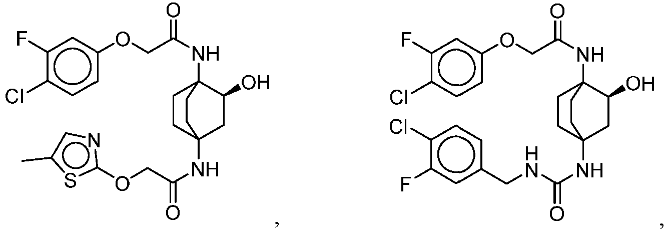 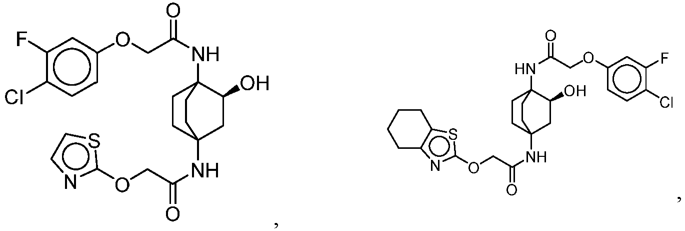 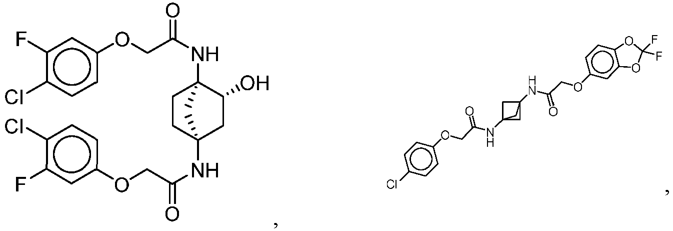 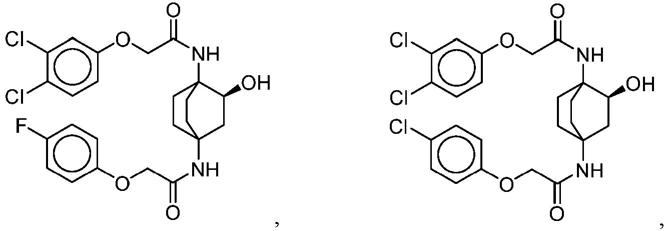 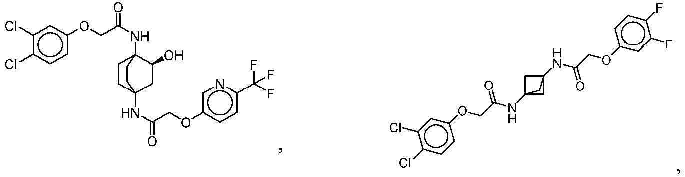 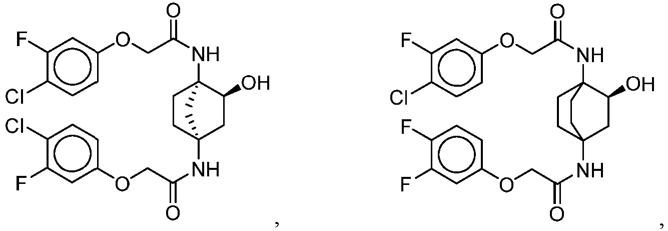 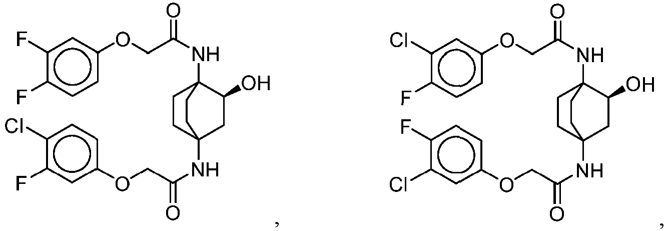 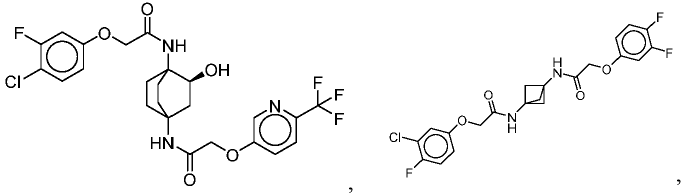 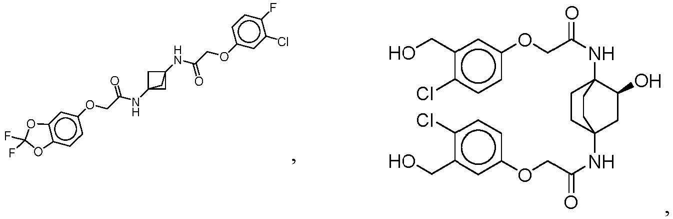 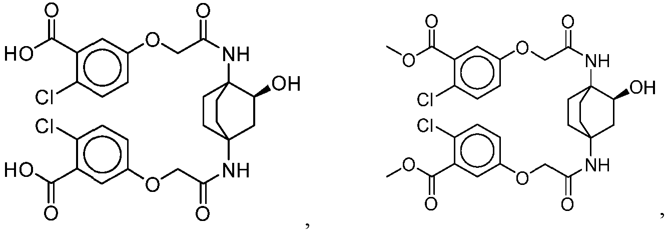 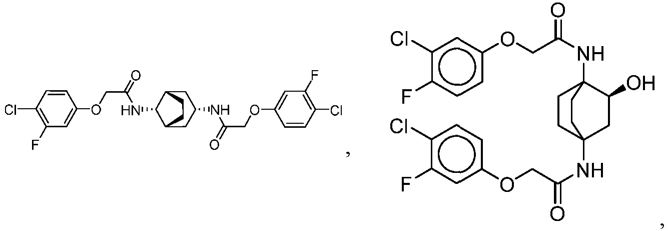 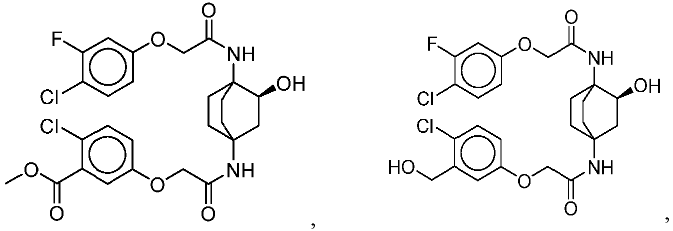 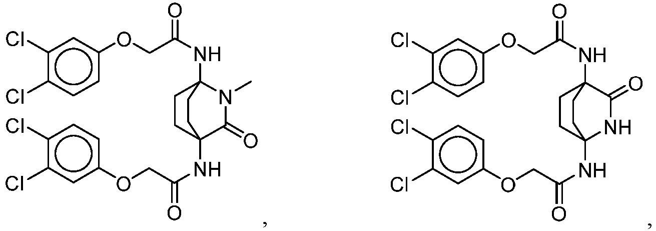 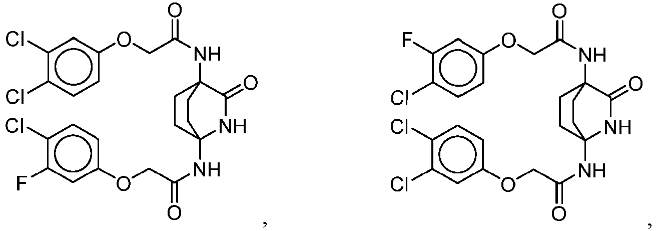 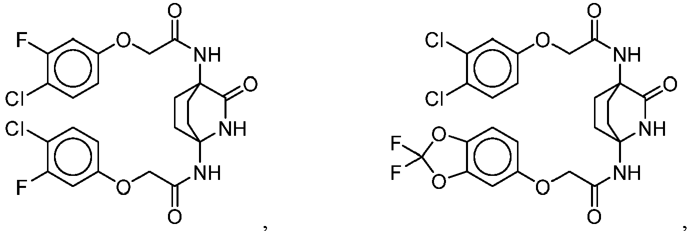 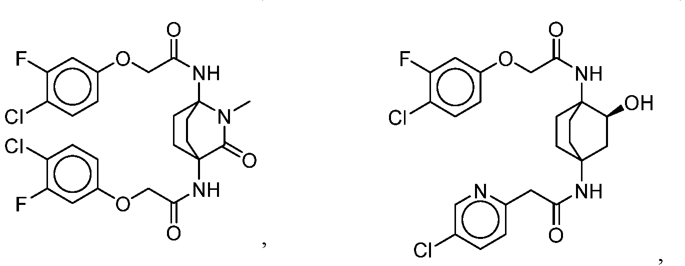 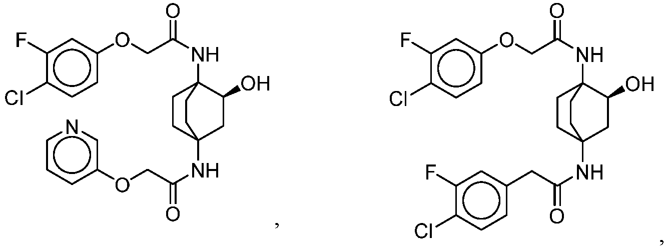 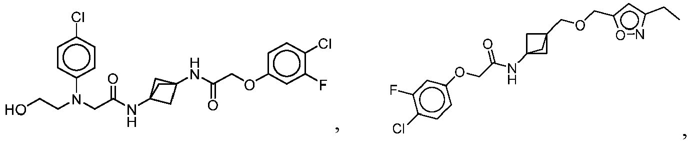 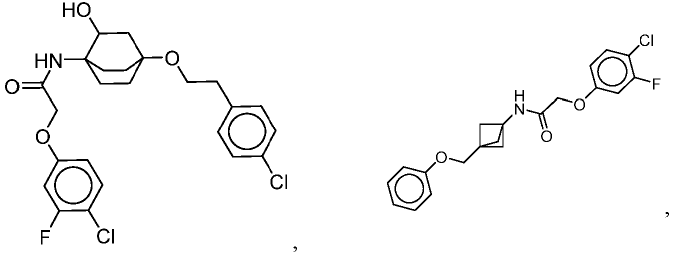 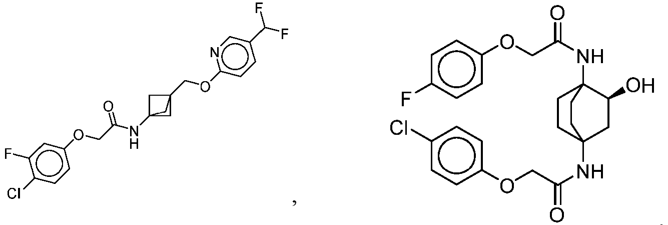 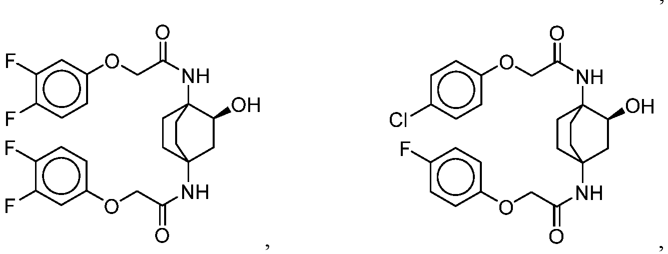 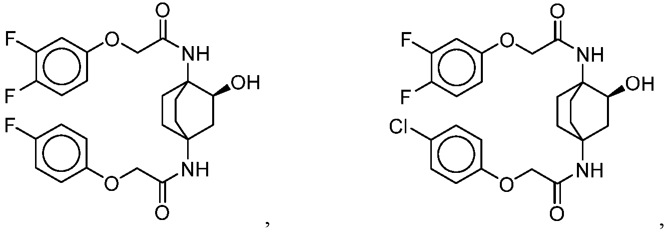 and 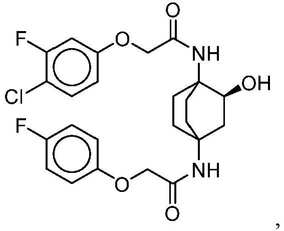 or a pharmaceutically acceptable salt thereof.

## Patentansprüche

1. Verbindung der Formel (II): 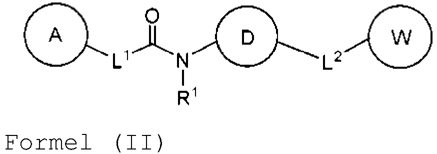 oder ein pharmazeutisch unbedenkliches Salz davon, wobei:
D ausgewählt ist aus der Gruppe bestehend aus: 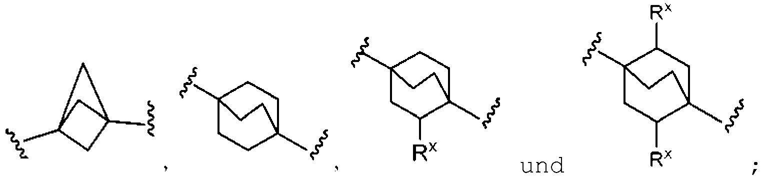
L¹ für C₁-C₆-Alkylen, C₂-C₆-Alkenylen, 2-7-gliedriges Heteroalkylen, -O- oder -NR^{C}- steht, wobei C₁-C₆-Alkylen, C₂-C₆-Alkenylen oder 2-7-gliedriges Heteroalkylen gegebenenfalls durch 1-5 R^{X} substituiert ist;
L² für -(C₀-C₂-Alkylen)-O-(C₀-C₂-alkylen)- steht;
R¹ für Wasserstoff, C₁-C₆-Alkyl, C₁-C₆-Alkoxy-C₂-C₆-alkyl, Hydroxy-C₂-C₆-alkyl oder Silyloxy-C₂-C₆-alkyl steht;
R^{N1} aus der Gruppe bestehend aus Wasserstoff, C₁-C₆-Alkyl, Hydroxy-C₂-C₆-alkyl, Halogen-C₂-C₆-alkyl, Amino-C₂-C₆-alkyl, Cyano-C₂-C₆-alkyl, -C(O)NR^{B}R^{C}, -C(O)R^{D}, -C(O)OR^{D} und -S(O)₂R^{D} ausgewählt ist;
A und W jeweils unabhängig für Phenyl oder 5-6-gliedriges Heteroaryl stehen, wobei jedes Phenyl oder 5-6-gliedriges Heteroaryl gegebenenfalls durch 1-5 R^{Y} substituiert ist;
R^{X} jeweils unabhängig aus der Gruppe bestehend aus C₁-C₆-Alkyl, Hydroxy-C₁-C₆-alkyl, Halogen-C₁-C₆-alkyl, Amino-C₁-C₆-alkyl, Cyano-C₁-C₆-alkyl, C₁-C₆-Alkoxy-C₁-C₆-alkyl, Oxo, Halogen, Cyano, -OR^{A}, -NR^{B}R^{C}, -NR^{B}C(O)R^{B}, -C(O)NR^{B}R^{C}, -C(O)R^{D}, -C(O)OH, -C(O)OR^{D}, -SR^{E}, -S(O)R^{D}, -S(O)₂R^{D}, - OS(O)R^{D}, -OS(O)₂R^{D} und G² ausgewählt ist;
R^{Y} jeweils unabhängig aus der Gruppe bestehend aus Wasserstoff, C₁-C₆-Alkyl, Hydroxy-C₁-C₆-alkyl, Halogen-C₁-C₆-alkyl, Halogen-C₁-C₆-alkoxy, Amino-C₁-C₆-alkyl, Cyano-C₁-C₆-alkyl, Oxo, Halogen, Cyano, -OR^{A}, -NR^{B}R^{C}, -NR^{B}C(O)R^{D}, -C(O)NR^{B}R^{C}, -C(O)R^{D}, -C(O)OH, -C(O)OR^{D}, -S(R^{F})ₘ, -S(O)R^{D}, -S(O)₂R^{D}, und G¹ ausgewählt ist; oder
2 R^{Y}-Gruppen an benachbarten Atomen zusammen mit den Atomen, an die sie gebunden sind, einen 3-7-gliedrigen kondensierten Cycloalkyl-, Heterocyclyl-, Aryl- oder Heteroarylring, der gegebenenfalls durch 1-5 R^{X} substituiert ist, bilden;
G¹ und G² jeweils unabhängig für C₃-C₆-Cycloalkyl, 4-7-gliedriges Heterocyclyl, Aryl oder 5-6-gliedriges Heteroaryl stehen, wobei jedes C₃-C₆-Cycloalkyl, 4-7-gliedrige Heterocyclyl, Aryl oder 5-6-gliedrige Heteroaryl gegebenenfalls durch 1-3 R^{Z} substituiert ist; R^{Z} jeweils unabhängig aus der Gruppe bestehend aus C₁-C₆-Alkyl, Hydroxy-C₁-C₆-alkyl, Halogen-C₁-C₆-alkyl, Halogen, Cyano, -OR^{A}, -NR^{B}R^{C}, -NR^{B}C(O)R^{D}, -C(O)NR^{B}R^{C}, -C(O)R^{D}, - C(O)OH, -C(O)OR^{D} und -S(O)₂R^{D} ausgewählt ist;
R^{A} jeweils unabhängig für Wasserstoff, C₁-C₆-Alkyl, Halogen-C₁-C₆-alkyl, -C(O)NR^{B}R^{C}, -C(O)R^{D} oder -C(O)OR^{D} steht;
R^{B} und R^{C} jeweils unabhängig für Wasserstoff oder C₁-C₆-Alkyl stehen; oder
R^{B} und R^{C} zusammen mit dem Atom, an das sie gebunden sind, einen 3-7-gliedrigen Heterocyclylring, der gegebenenfalls durch 1-3 R^{Z} substituiert ist, bilden;
R^{D} jeweils unabhängig für C₁-C₆-Alkyl, 2-7-gliedriges Heteroalkyl oder Halogen-C₁-C₆-alkyl steht, wobei jedes C₁-C₆-Alkyl, 2-7-gliedrige Heteroalkyl oder Halogen-C₁-C₆-alkyl gegebenenfalls durch 1-5 R^{G} substituiert ist;
R^{E} jeweils unabhängig für Wasserstoff, C₁-C₆-Alkyl oder Halogen-C₁-C₆-alkyl steht;
R^{F} jeweils unabhängig für Wasserstoff, C₁-C₆-Alkyl oder Halogen steht;
R^{G} jeweils unabhängig für Aryl oder 5-6-gliedriges Heteroaryl steht, wobei jedes Aryl oder 5-6-gliedrige Heteroaryl gegebenenfalls durch 1-5 R^{H} substituiert ist;
R^{H} jeweils unabhängig für C₁-C₆-Alkyl oder Halogen-C₁-C₆-alkyl steht;
m für 1 steht, wenn R^{F} für Wasserstoff oder C₁-C₆-Alkyl steht, für 3 steht, wenn R^{F} für C₁-C₆-Alkyl steht, oder für 5 steht, wenn R^{F} für Halogen steht; und
t für 0 oder 1 steht.

2. Verbindung nach Anspruch 1, wobei R^{X} jeweils unabhängig aus der Gruppe bestehend aus Oxo, -OR^{A} (z. B. -OH oder -OCH₃), -C(O)OH, -C(O)OR^{D} (z. B. -C(O)OCH₃), Halogen und Hydroxy-C₁-C₆-alkyl ausgewählt ist.

3. Verbindung nach Anspruch 1 oder 2, wobei L¹ für - CH₂O-* oder -CH₂OCH₂-* steht; wobei "-*" die Bindungsstelle zu A anzeigt.

4. Verbindung nach einem der Ansprüche 1-3, wobei L² aus der Gruppe bestehend aus 0-*, OCH₂-*, CH₂O-*, OCH₂CH₂-*, CH₂OCH₂-* und CH₂CH₂O-* ausgewählt ist; wobei "-*" die Bindungsstelle zu W anzeigt.

5. Verbindung nach einem der Ansprüche 1-4, wobei R¹ für Wasserstoff oder -CH₃ steht.

6. Verbindung nach einem der Ansprüche 1-5, wobei A ausgewählt ist aus der Gruppe bestehend aus:

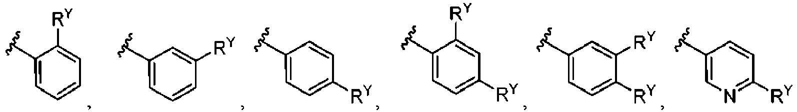

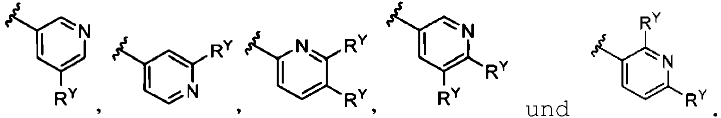

7. Verbindung nach einem der Ansprüche 1-6, wobei W ausgewählt ist aus der Gruppe bestehend aus: 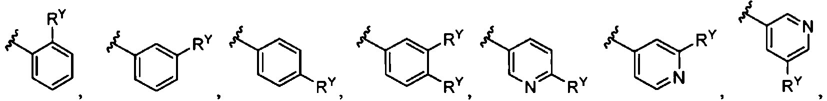 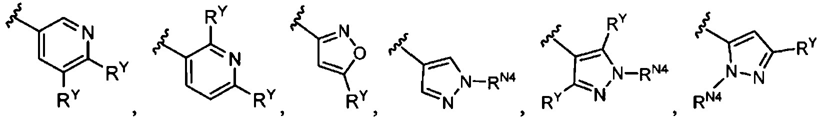 und 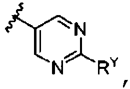 wobei R^{N4} für Wasserstoff oder -CH₃ steht.

8. Verbindung nach einem der Ansprüche 1-7, wobei R^{Y} jeweils unabhängig für Wasserstoff, Chlor, Fluor, -CF₃, -CHF₂, -CH₃, -CH₂CH₃, -CH(CH₃)₂, -OCH₃, -OCF₃, -OCH(CH₃)₂ oder -CN steht.

9. Verbindung der Formel (II-a): 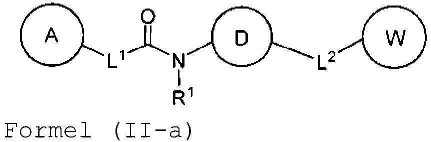 oder ein pharmazeutisch unbedenkliches Salz davon, wobei:
D für Bicyclo[1.1.1]pentanyl oder Bicyclo[2.2.2]octanyl steht, wobei jede dieser Gruppen gegebenenfalls durch 1-4 R^{X}-Gruppen substituiert ist;
L¹ für -CH₂O-* oder -CH₂OCH₂-* steht, wobei "-*" die Bindungsstelle zu A anzeigt;
L² aus der Gruppe bestehend aus O-*, OCH₂-*, CH₂O-*, OCH₂CH₂-*, CH₂OCH₂-* und CH₂CH₂O-* ausgewählt ist; wobei "-*" die Bindungsstelle zu W anzeigt;
A für Phenyl oder Pyridyl steht, wobei jede dieser Gruppen gegebenenfalls durch 1-5 R^{Y}-Gruppen substituiert ist;
W für Phenyl, Pyridyl, Isoxazolyl oder Pyrazolyl steht, wobei jede dieser Gruppen gegebenenfalls an einem oder mehreren verfügbaren Kohlenstoffatomen durch 1-5 R^{Y}-Gruppen substituiert ist und wobei Pyrazolyl gegebenenfalls an einem verfügbaren Stickstoff durch Wasserstoff oder -CH₃ substituiert ist;
R^{X} jeweils unabhängig für Fluor, Oxo, -OH, -OCH₃, -C(O)OH oder -C(O)OCH₃ steht;
R^{Y} jeweils unabhängig für Chlor, Fluor, -CF₃, -CH₃, - CH₂CH₃, -CH(CH₃)₂, -OCH₃, -OCH(CH₃)₂ oder -CN steht; oder
2 R^{Y}-Gruppen an benachbarten Atomen zusammen mit den Atomen, an die sie gebunden sind, einen Furanyl-, Pyrrolyl- oder Dioxolanylring bilden, wobei jeder dieser Ringe gegebenenfalls durch 1-2 R^{X} substituiert ist; und R¹ für Wasserstoff steht.

10. Verbindung, ausgewählt aus 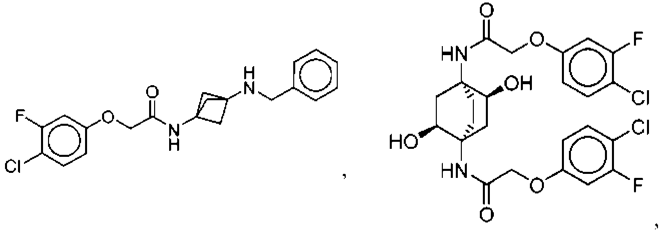 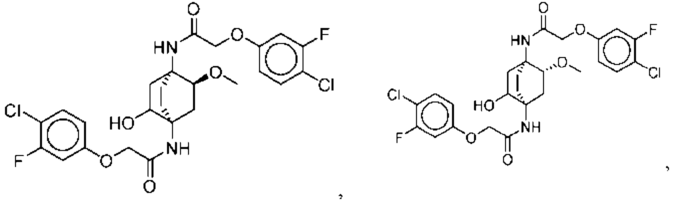 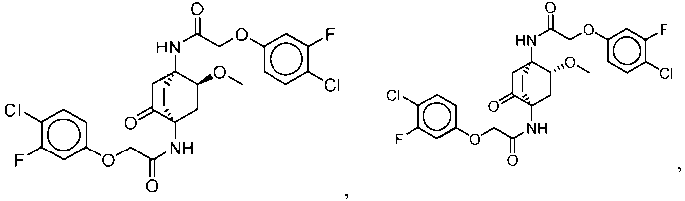 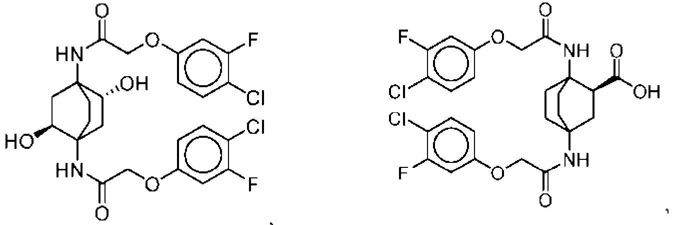 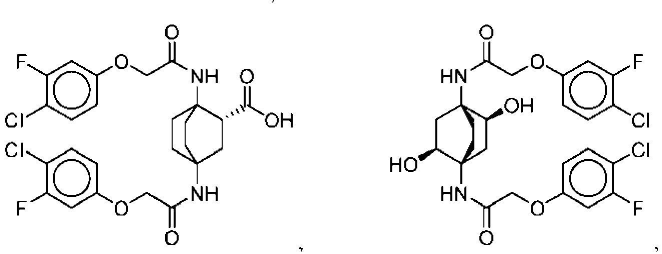 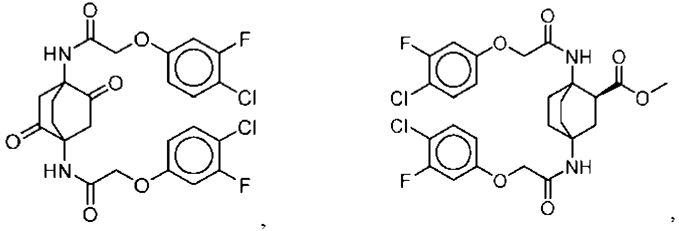 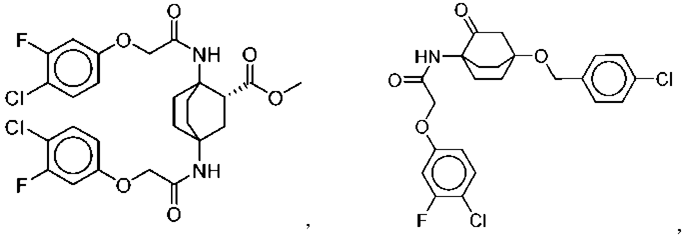 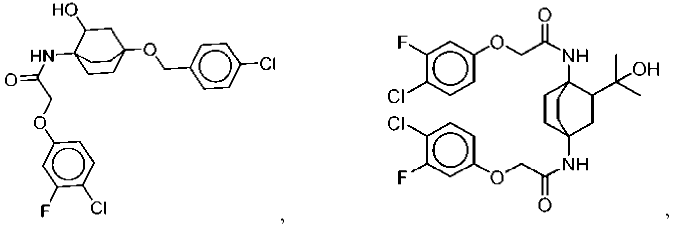 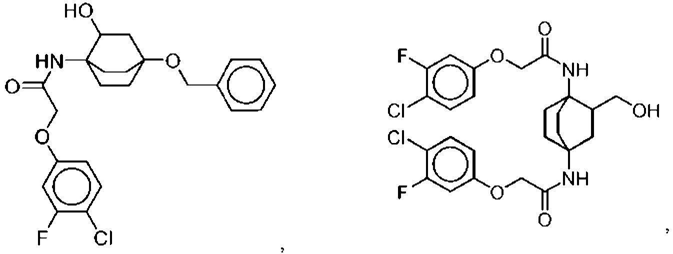 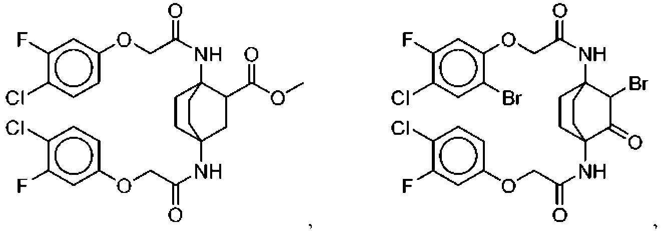 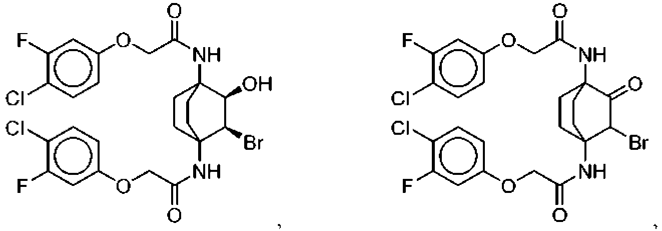 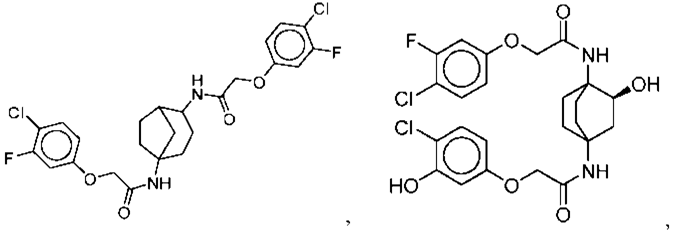 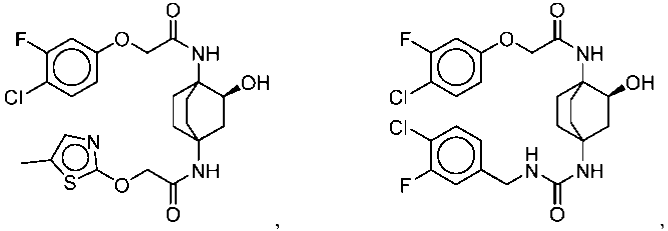 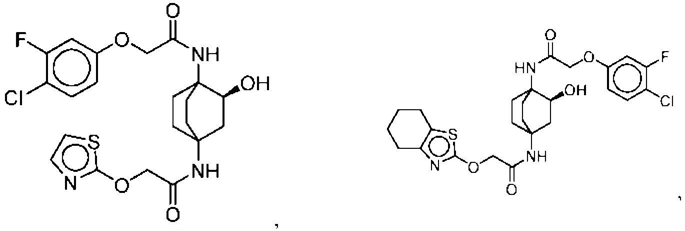 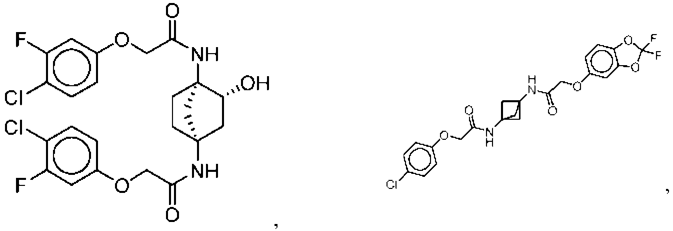 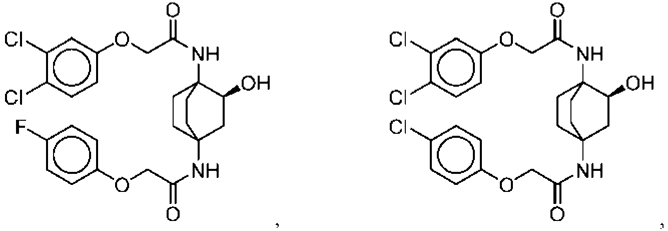 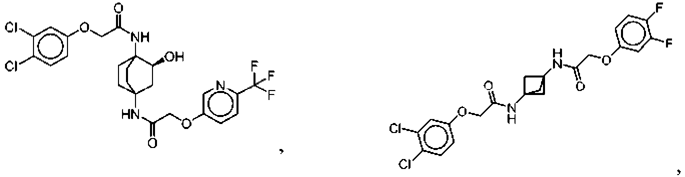 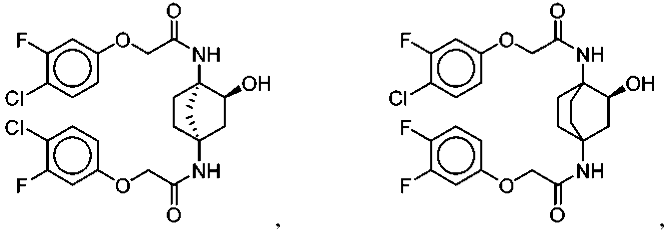 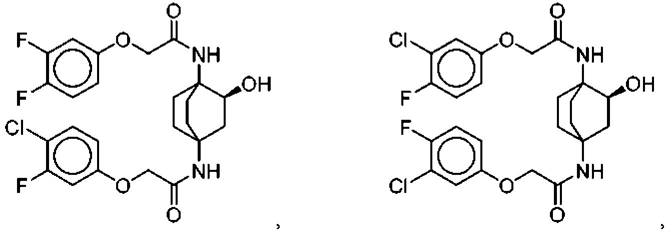 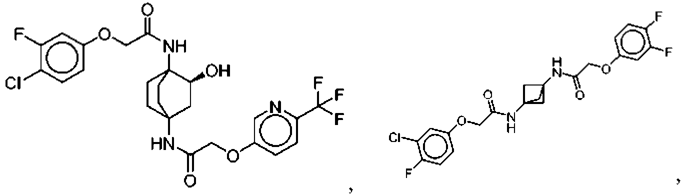 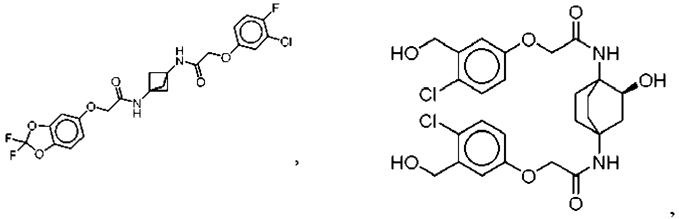 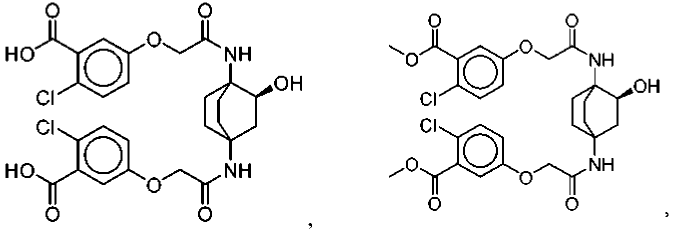 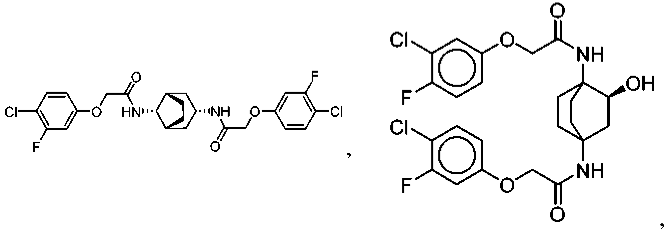 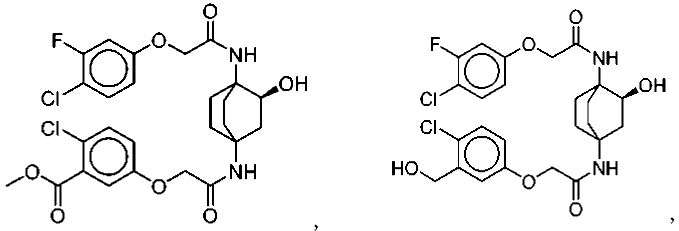 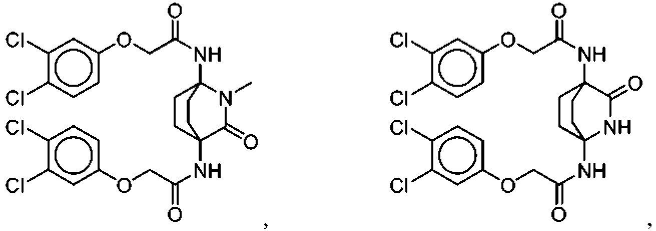 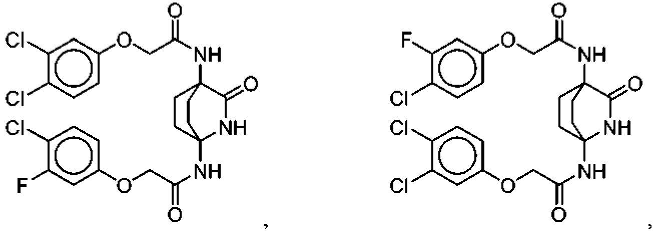 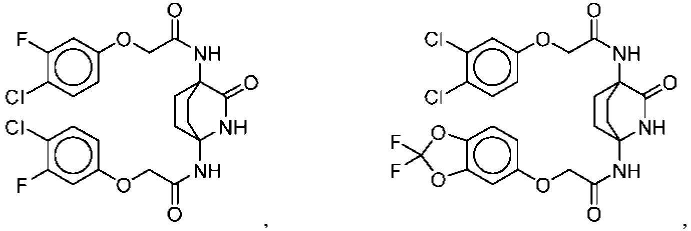 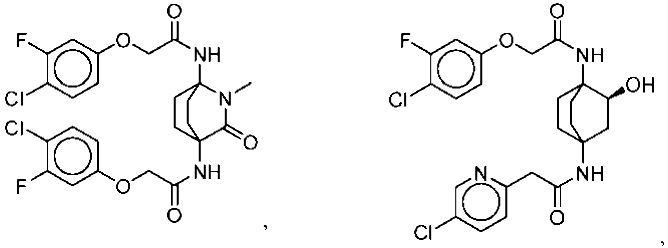 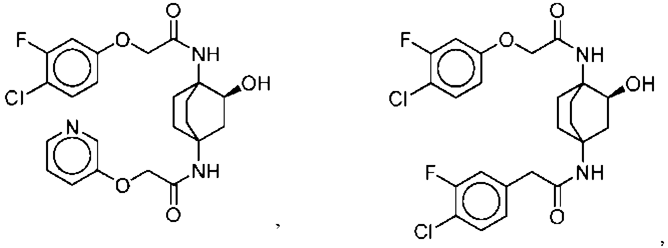 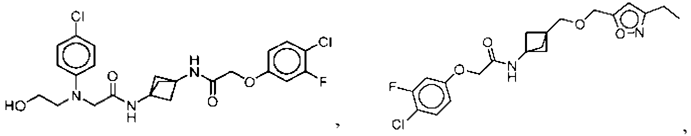 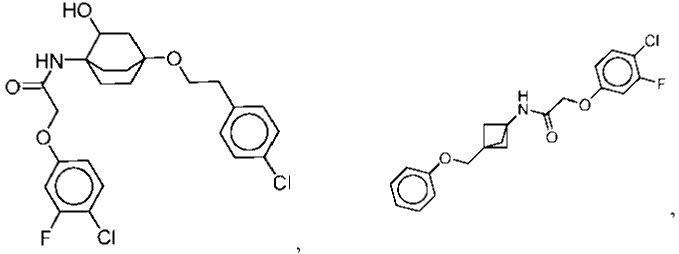 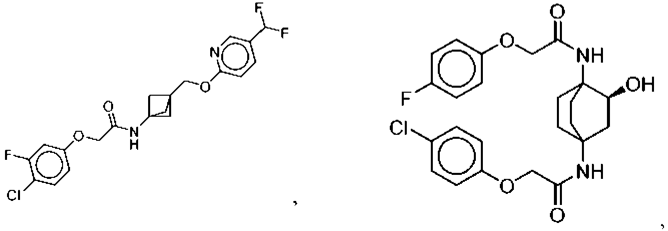 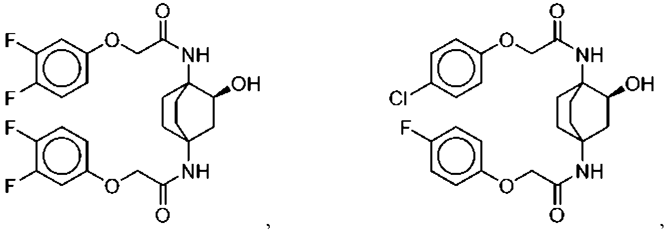 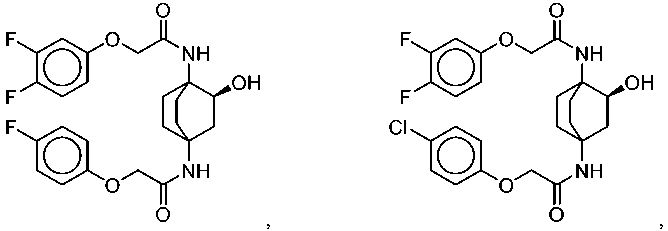 und 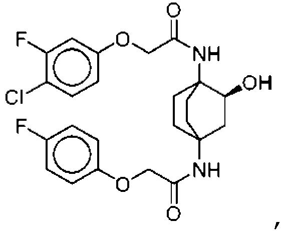 oder ein pharmazeutisch unbedenkliches Salz davon.

## Revendications

1. Composé de Formule (II) : 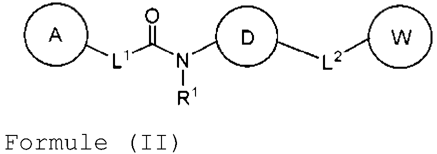 ou sel pharmaceutiquement acceptable correspondant :
D étant choisi dans le groupe constitué par : 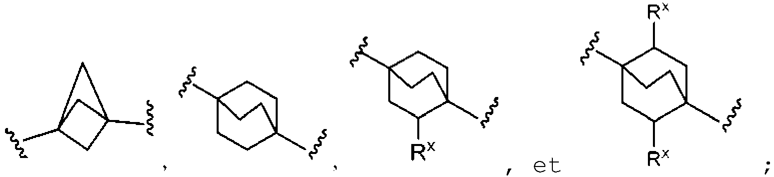
L¹ étant C₁-C₆ alkylène, C₂-C₆ alcénylène, hétéroalkylène à 2 à 7 chaînons, -0-, ou -NR^{C}-, C₁-C₆ alkylène, C₂-C₆ alcénylène, ou hétéroalkylène à 2 à 7 chaînons étant éventuellement substitué par 1-5 R^{X} ;
L² étant -(C₀-C₂ alkylène)-O-(C₀-C₂ alkylène)- ;
R¹ étant hydrogène, C₁-C₆ alkyle, C₁-C₆ alcoxy-C₂-C₆ alkyle, hydroxy-C₂-C₆ alkyle, ou silyloxy-C₂-C₆ alkyle ;
R^{N1} étant choisi dans le groupe constitué d'hydrogène, C₁-C₆ alkyle, hydroxy-C₂-C₆ alkyle, halogéno-C₂-C₆ alkyle, amino-C₂-C₆ alkyle, cyano-C₂-C₆ alkyle, -C(O)NR^{B}R^{C}, - C(O)R^{D}, -C(O)OR^{D} et -S(O)₂R^{D}, ;
A et W étant chacun indépendamment phényle ou hétéroaryle à 5 à 6 chaînons, chaque phényle ou hétéroaryle à 5 à 6 chaînons étant éventuellement substitué par 1-5 R^{Y} ;
chaque R^{X} étant indépendamment choisi dans le groupe constitué par C₁-C₆ alkyle, hydroxy-C₁-C₆ alkyle, halogéno-C₁-C₆ alkyle, amino-C₁-C₆ alkyle, cyano-C₁-C₆ alkyle, C₁-C₆ alcoxy-C₁-C₆ alkyle, oxo, halogéno, cyano, -OR^{A}, -NR^{B}R^{C}, -NR^{B}C(O)R^{D}, -C(O)NR^{B}R^{C}, -C(O)R^{D}, -C(O)OH, - C(O)OR^{D}, -SR^{E}, -S(O)R^{D}, -S(O)₂R^{D}, -OS(O)R^{D}, -OS(O)₂R^{D}, et G² ;
chaque R^{Y} étant indépendamment choisi dans le groupe constitué d'hydrogène, C₁-C₆ alkyle, hydroxy-C₁-C₆ alkyle, halogéno-C₁-C₆ alkyle, halogéno-C₁-C₆ alcoxy, amino-C₁-C₆ alkyle, cyano-C₁-C₆ alkyle, oxo, halogéno, cyano, -OR^{A}, - NR^{B}R^{C}, -NR^{B}C(0) R^{D}, -C (0) NR^{B}R^{C}, -C(O)R^{D}, -C (0) OH, -C(O)OR^{D}, -S(R^{F})ₘ, -S(O)R^{D}, -S(O)₂R^{D} et G¹ ; ou
2 groupes R^{Y} sur des atomes adjacents, conjointement avec les atomes auxquels ils sont fixés formant un cycle cycloalkyle, hétérocyclyle, aryle ou hétéroaryle condensé à 3 à 7 chaînons éventuellement substitué par 1-5 R^{X} ;
chaque G¹ et G² étant indépendamment C₃-C₆ cycloalkyle, hétérocyclyle à 4 à 7 chaînons, aryle, ou hétéroaryle à 5 à 6 chaînons, chaque C₃-C₆ cycloalkyle, hétérocyclyle à 4 à 7 chaînons, aryle, ou hétéroaryle à 5 à 6 chaînons étant éventuellement substitué par 1-3 R^{Z} ;
chaque R^{Z} étant indépendamment choisi dans le groupe constitué de C₁-C₆ alkyle, hydroxy-C₁-C₆ alkyle, halogéno-C₁-C₆ alkyle, halogéno, cyano, -OR^{A}, -NR^{B}R^{C}, -NR^{B}C(O)R^{D}, - C(O)NR^{B}R^{C}, -C(O)R^{D}, -C(O)OH, -C(O)OR^{D} et -S(O)₂R^{D}, ;
chaque R^{A} étant indépendamment hydrogène, C₁-C₆ alkyle, halogéno-C₁-C₆ alkyle, -C(O)NR^{B}R^{C}, -C(O)R^{D} ou -C(O)OR^{D} ;
chacun parmi R^{B} et R^{C} étant indépendamment hydrogène ou C₁-C₆ alkyle ; ou
R^{B} et R^{C} conjointement avec l'atome auquel ils sont fixés formant un cycle hétérocyclyle à 3 à 7 chaînons éventuellement substitué par 1-3 R^{Z} ;
chaque R^{D} étant indépendamment C₁-C₆ alkyle, hétéroalkyle à 2 à 7 chaînons, ou halogéno-C₁-C₆ alkyle, chaque C₁-C₆ alkyle, hétéroalkyle à 2 à 7 chaînons, ou halogéno-C₁-C₆ alkyle étant éventuellement substitué par 1-5 R^{G} ;
chaque R^{E} étant indépendamment hydrogène, C₁-C₆ alkyle ou halogéno-C₁-C₆ alkyle ;
chaque R^{F} étant indépendamment hydrogène, C₁-C₆ alkyle ou halogéno ;
chaque R^{G} étant indépendamment aryle ou hétéroaryle à 5 à 6 chaînons, chaque aryle ou hétéroaryle à 5 à 6 chaînons étant éventuellement substitué par 1-5 R^{H} ;
chaque R^{E} étant indépendamment C₁-C₆ alkyle ou halogéno-C₁-C₆ alkyle ;
m étant 1 lorsque R^{F} est hydrogène ou C₁-C₆ alkyle, 3 lorsque R^{F} est C₁-C₆ alkyle ou 5 lorsque R^{F} est halogéno ; et
t étant 0 ou 1.

2. Composé selon la revendication 1, chaque R^{X} étant indépendamment choisi dans le groupe constitué par oxo, -OR^{A} (par ex., -OH ou -OCH₃), -C(O)OH, -C(O)OR^{D} (par ex., -C(O)OCH₃), halogéno, et hydroxy-C₁-C₆ alkyle.

3. Composé selon la revendication 1 ou 2, L¹ étant CH₂O-* ou CH₂OCH₂-* ; « -* » indiquant le point de fixation à A.

4. Composé selon l'une quelconque des revendications 1-3, L² étant choisi dans le groupe constitué par 0-*, OCH₂-*, CH₂O-*, OCH₂CH₂-*, CH₂OCH₂-*, et CH₂CH₂O-* ; « -* » indiquant le point de fixation à W.

5. Composé selon l'une quelconque des revendications 1-4, R¹ étant hydrogène ou -CH₃.

6. Composé selon l'une quelconque des revendications 1-5, A étant choisi dans le groupe constitué par :

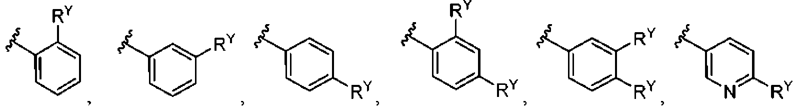

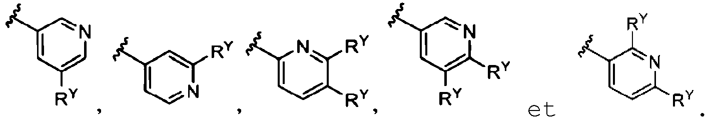

7. Composé selon l'une quelconque des revendications 1-6, W étant choisi dans le groupe constitué par : 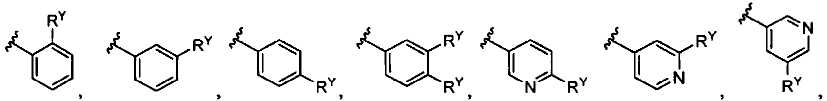 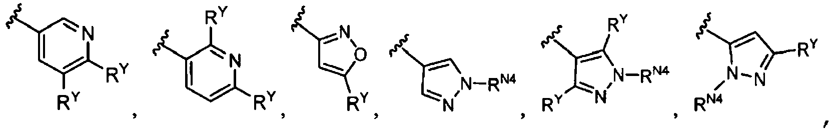 et 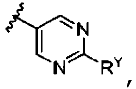 R^{N4} étant hydrogène ou -CH₃.

8. Composé selon l'une quelconque des revendications 1-7, chaque R^{Y} étant indépendamment hydrogène, chloro, fluoro, -CF₃, -CHF₂, -CH₃, -CH₂CH₃, -CH(CH₃)₂, -OCH₃, -OCF₃, -OCH(CH₃)₂, ou -CN.

9. Composé de Formule (II-a) : 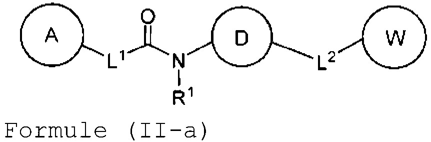 ou sel pharmaceutiquement acceptable correspondant :
D étant bicyclo[1.1.1]pentanyle ou bicyclo[2.2.2]octanyle, chacun desquels étant éventuellement substitué par 1-4 groupes R^{X} ;
L¹ étant -CH₂O-* ou -CH₂OCH₂-*, « -* » indiquant le point de fixation à A ;
L² étant choisi dans le groupe constitué par O-*, OCH₂-*, CH₂O-*, OCH₂CH₂-*, CH₂OCH₂-*, et CH₂CH₂O-* ; « -* » indiquant le point de fixation à W ;
A étant phényle ou pyridinyle, chacun desquels étant éventuellement substitué par 1-5 groupes R^{Y} ;
W étant phényle, pyridinyle, isoxazolyle ou pyrazolyle, chacun desquels étant éventuellement substitué sur un ou plusieurs carbones disponibles par 1-5 groupes R^{Y} ; et
pyrazolyle pouvant être éventuellement substitué sur un azote disponible par hydrogène ou -CH₃ ;
chaque R^{X} étant indépendamment fluoro, oxo, -OH, -OCH₃, - C(O)OH ou -C(O)OCH₃ ;
chaque R^{Y} étant indépendamment chloro, fluoro, -CF₃, - CH₃, -CH₂CH₃, -CH(CH₃)₂, -OCH₃, -OCH(CH₃)₂ ou -CN ; ou
2 groupes R^{Y} sur des atomes adjacents, conjointement avec les atomes auxquels ils sont fixés formant un cycle furanyle, pyrrolyle ou dioxolanyle, chacun desquels étant éventuellement substitué par 1-2 R^{X} ; et R¹ étant hydrogène.

10. Composé choisi parmi 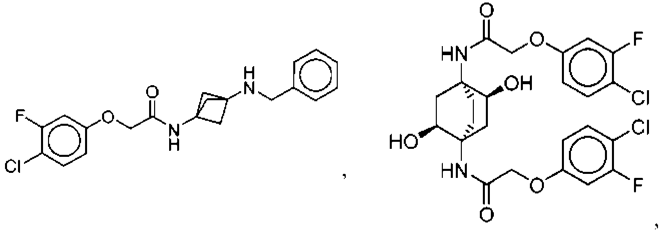 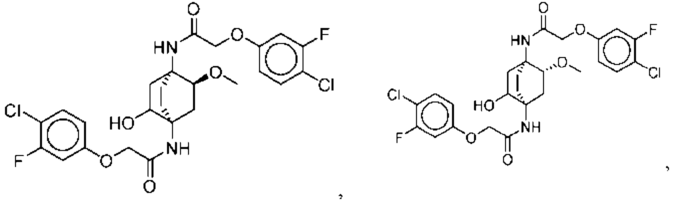 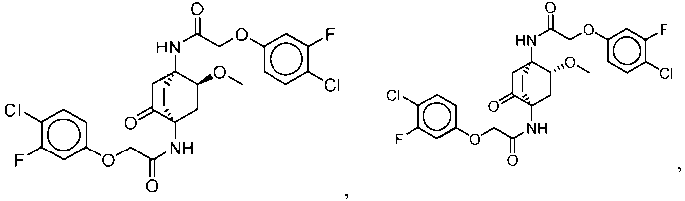 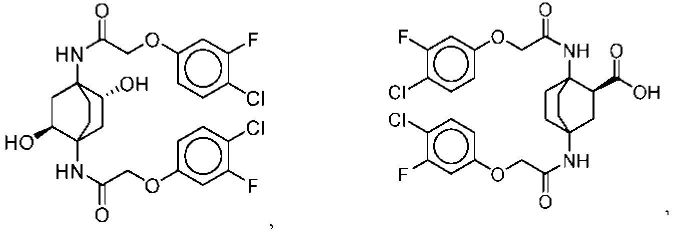 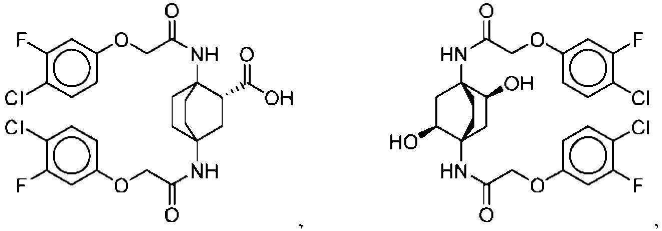 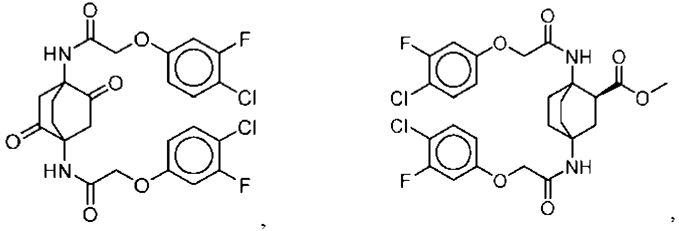 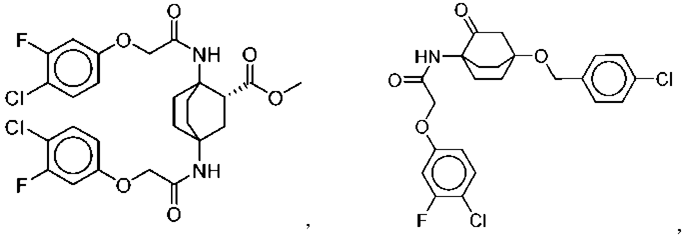 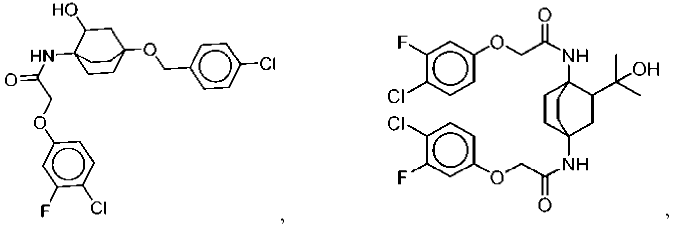 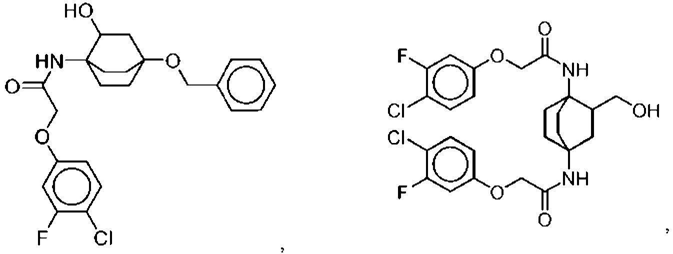 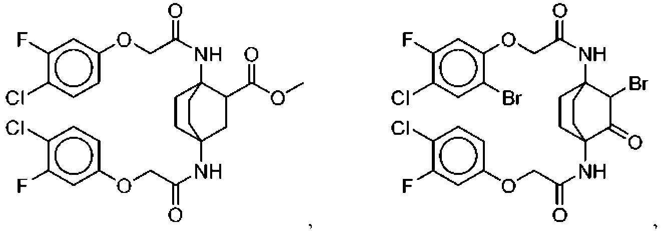 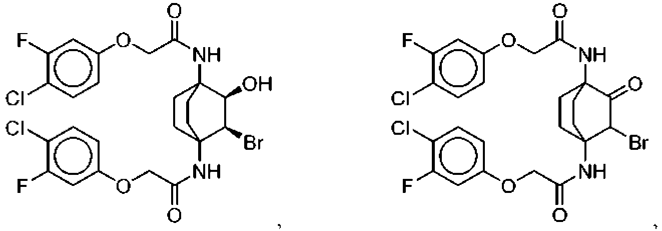 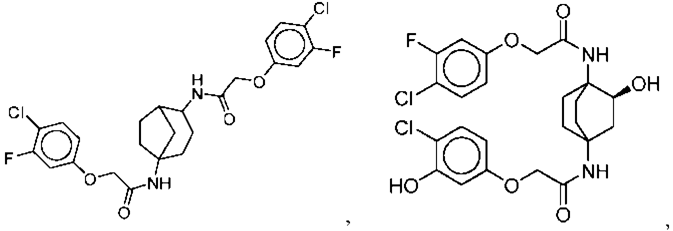 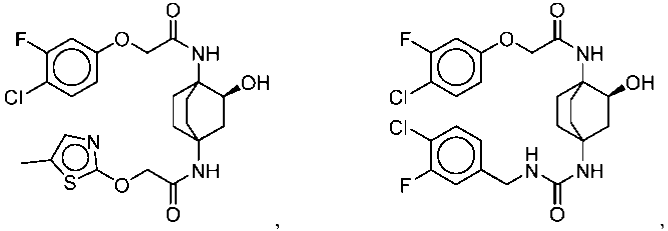 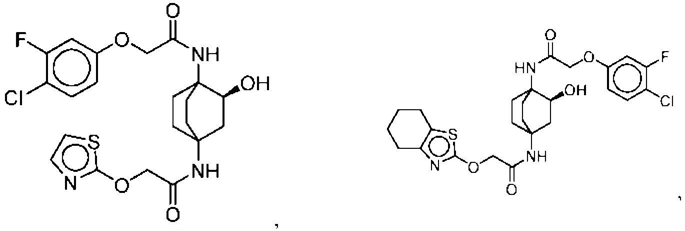 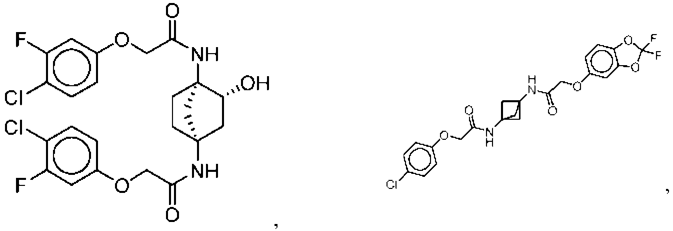 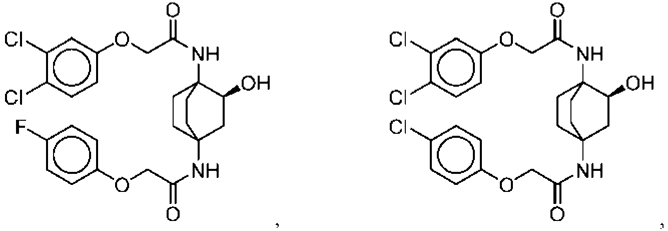 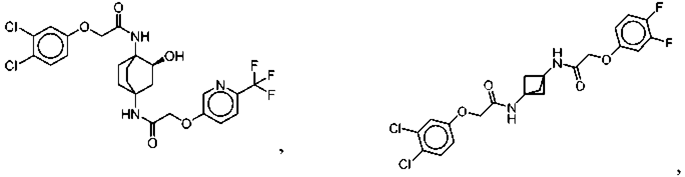 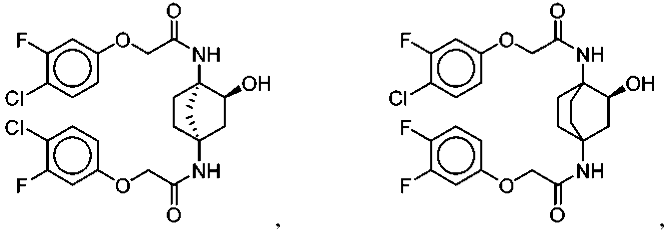 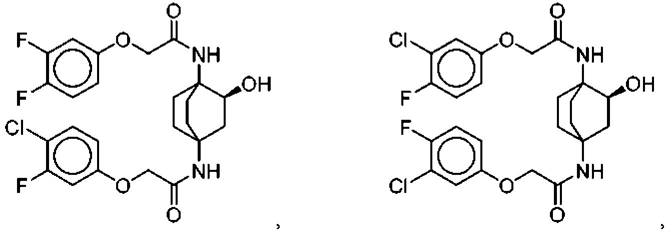 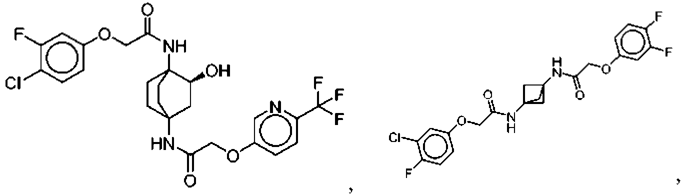 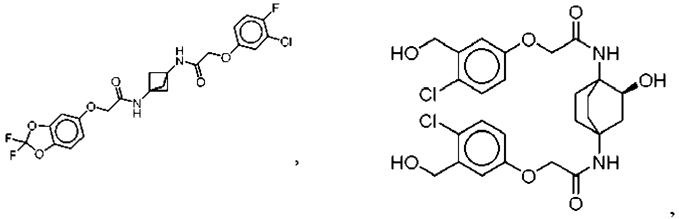 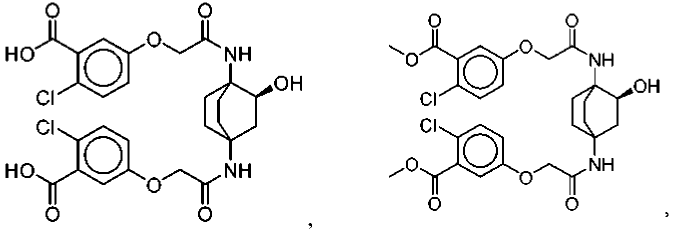 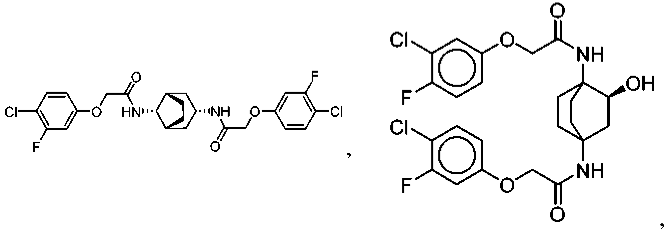 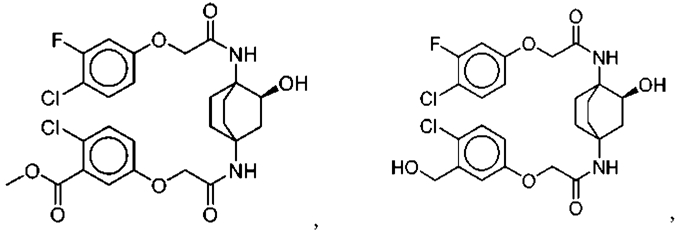 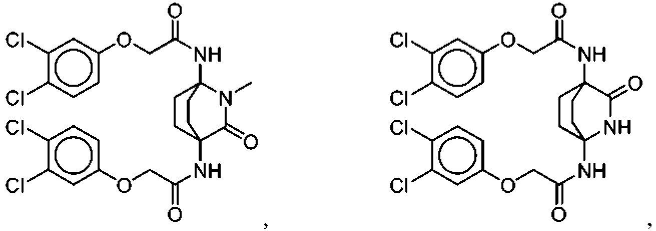 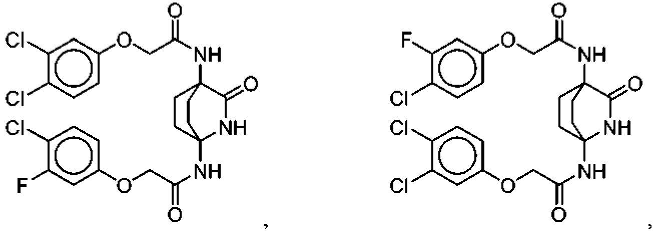 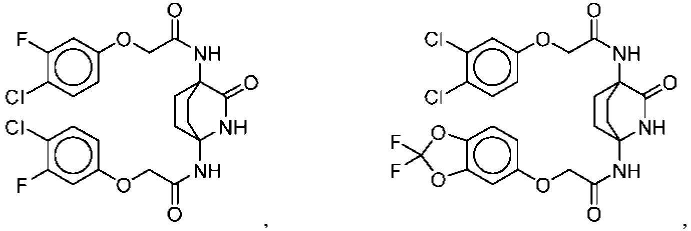 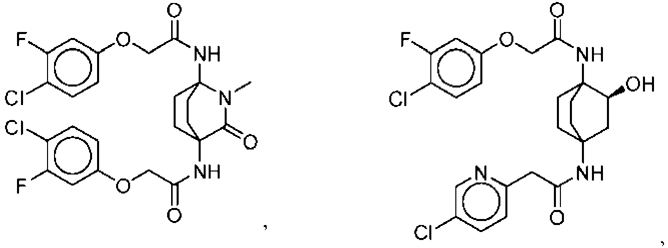 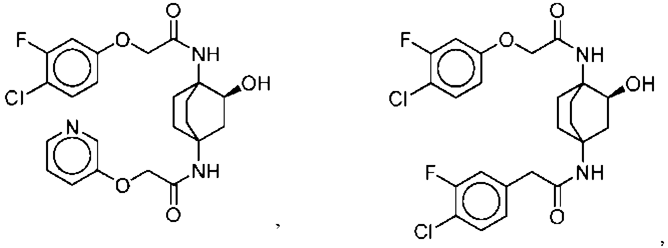 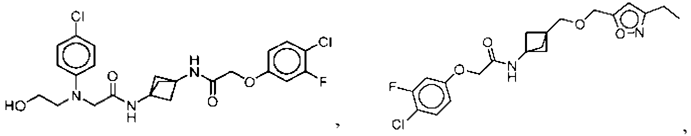 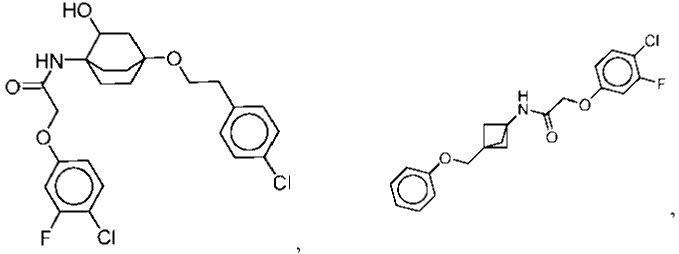 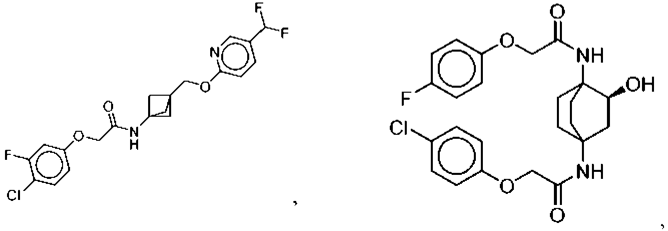 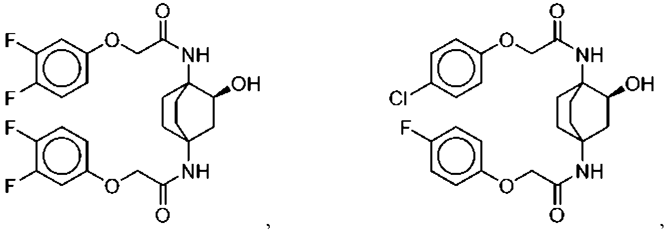 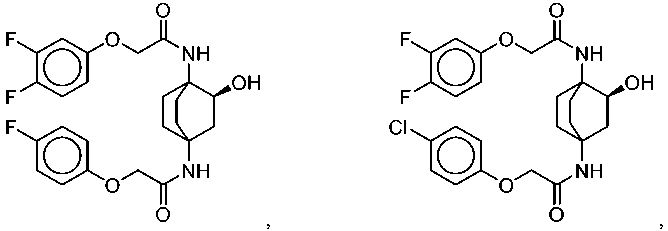 et 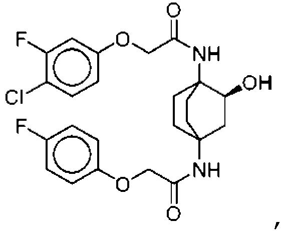 ou sel pharmaceutiquement acceptable de celui-ci.
